# EUROPEAN PATENT APPLICATION

(11) **EP 1 681 350 A2**
(43) Date of publication of application: **19.07.2006**
(21) Application number: 06008722.8
(22) Date of filing: 10.12.1998
(51) Int. Cl.: C12N 15/31, C07K 14/195, A61K 38/00, A61K 38/16, A61K 39/00

(54) **Porphorymonas gingivalis polypeptides and polynucleotides**

(30) Priority: 10.12.1997 AU PP083997; 31.12.1997 AU PP118297; 30.01.1998 AU PP154698; 10.03.1998 AU PP226498; 09.04.1998 AU PP291198; 23.04.1998 AU PP312898; 05.05.1998 AU PP333898; 22.05.1998 AU PP365498; 29.07.1998 AU PP004917; 30.07.1998 AU PP004963; 04.08.1998 AU PP005028
(62) Divisional of application: 98960880.7
(71) Applicant: CSL LIMITED, Parkville, VIC 3052 (AU)
(72) Inventor: Ross, Bruce Carter, Coburg VIC 3058 (AU); Barr, Ian George, Templestowe VIC 3106 (AU); Patterson, Michelle Anne, Laverton VIC 3028 (AU); Agius, Catherine Therese, Box Hill South VIC 3128 (AU); Rothel, Linda Joy, Glen Huntly VIC 3163 (AU); Margetts, Mai Brigid, Moonee Ponds VIC 3039 (AU); Hocking, Dianna Margaret, Flemington VIC 3031 (AU); Webb, Elizabeth Ann, Eltham VIC 3422 (AU)
(74) Representative: Lee, Nicholas John

(57) **Abstract**

The present invention relates to isolated *Porphorymonas gingivalis* polypeptides and nucleotides. The polypeptides include;
an amino acid sequence selected from the group consisting of SEQ. ID. NO. 265 to SEQ. ID. NO. 528, SEQ. ID. NO. 531 and SEQ. ID. NO. 532; or
an amino acid sequence at least 85%, preferably at least 95%, identical to an amino acid sequence selected from the group consisting of SEQ. ID. NO. 265 to SEQ. ID. NO. 528, SEQ. ID. NO. 531 and SEQ. ID. NO. 532; or
at least 40 amino acids having a contiguous sequence of at least 40 amino acids identical to a contiguous amino acid sequence selected from the group consisting of SEQ. ID. NO. 265 to SEQ. ID. NO. 528, SEQ. ID. NO. 531 and SEQ. ID. NO. 532.

## Description

### FIELD OF THE INVENTION

The present invention relates to *P. gingivalis* nucleotide sequences, P. *gingivalis* polypeptides and probes for detection ofP. *gingivalis.* The *P. gingivalis* polypeptides and nucleotides can be used in compositions for use in raising an immune response in a subject against P. *gingivalis* and treating or preventing or reducing the severity of the condition known as periodontitis.

### BACKGROUND OF THE INVENTION

Periodontal diseases are bacterial-associated inflammatory diseases of the supporting tissues of the teeth and range from the relatively mild form of gingivitis, the non-specific, reversible inflammation of gingival tissue to the more aggressive forms of periodontitis which are characterised by the destruction of the tooth's supporting structures. Periodontitis is associated with a subgingival infection of a consortium of specific Gram-negative bacteria that leads to the destruction of the periodontium and is a major public health problem. One bacterium that has attracted considerable interest is P. *gingivalis* as the recovery of this microorganism from adult periodontitis lesions can be up to 50% of the subgingival anaerobically cultivable flora, whereas *P*. *gingivalis* is rarely recovered, and then in low numbers, from healthy sites. A proportional increase in the level of P. *gingivalis* in subgingival plaque has been associated with an increased severity of periodontitis and eradication of the microorganism from the cultivable subgingival microbial population is accompanied by resolution of the disease. The progression of periodontitis lesions in non-human primates has been demonstrated with the subgingival implantation of *P. gingivalis.* These findings in both animals and humans suggest a major role for *P. gingivalis* in the development of adult periodontitis.

*P. gingivalis* is a black-pigmented, anaerobic, asaccharolytic, proteolytic Gram-negative rod that obtains energy from the metabolism of specific amino acids. The microorganism has an absolute growth requirement for iron, preferentially in the form of haeme or its Fe(III) oxidation product haemin and when grown under conditions of excess haemin is highly virulent in experimental animals. A number of virulence factors have been implicated in the pathogenicity of P. *gingivalis* including the capsule, adhesins, cytotoxins and extracellular hydrolytic enzymes.

In order to develop an efficacious and safe vaccine to prevent, eliminate or reduce P. *gingivalis* colonisation it is necessary to identify and produce antigens that are involved in virulence that have utility as immunogens possibly through the generation of specific antibodies. Whilst it is possible to attempt to isolate antigens directly from cultures of P. *gingivalis* this is often difficult For example as mentioned above, P. *gingivalis* is a strict anaerobe and can be difficult to isolate and grow. It is also known that, for a number of organisms, when cultured in *vitro* that many virulence genes are down regulated and the encoded proteins are no longer expressed. If conventional chemistry techniques were applied to purify vaccine candidates potentially important (protective) molecules may not be identified. With DNA sequencing, as the gene is present (but not transcribed) even when the organism is grown in *vitro* it can be identified, cloned and produced as a recombinant DNA protein. Similarly, a protective antigen or therapeutic target may be transiently expressed by the organism *in* vitro or produced in low levels making the identification of these molecules extremely difficult by conventional methods.

With serological identification of therapeutic targets one is limited to those responses which are detectable using standard methods such as Western Blotting or ELISA. The limitation here is the both the level of response that is generated by the animal or human and determining whether this response is protective, damaging or irrelevant. No such limitation is present with a sequencing approach to the identification of potential therapeutic or prophylactic targets.

It is also well known that P. *gingivalis* produces a range of broadly active proteases (University of Melbourne International Patent Application No PCT /AU 96/00673, US Patent Nos 5,475,097 and 5,523,390), which make the identification of intact proteins difficult because of their degradation by these proteases.

### SUMMARY OF THE INVENTION

The present inventors have attempted to isolate P. *gingivalis* nucleotide sequences which can be used for recombinant production of P. *gingivalis* polypeptides and to develop nucleotide probes specific for P. *gingivalis.* The DNA sequences listed below have been selected from a large number of P. *gingivalis* sequences according to their indicative potential as vaccine candidates. This intuitive step involved comparison of the deduced protein sequence from theP. *gingivalis* DNA sequences to the known protein sequence databases. Some of the characteristics used to select useful vaccine candidates include; the expected cellular location, such as outer membrane proteins or secreted proteins, particular functional activities of similar proteins such as those with an enzymatic or proteolytic activity, proteins involved in essential metabolic pathways that when inactivated or blocked may be deleterious or lethal to the organism, proteins that might be expected to play a role in the pathogenesis of the organism eg. red cell lysis, cell agglutination or cell receptors and proteins which are paralogues to proteins with proven vaccine efficacy.

In a first aspect the present invention consists an isolated antigenic *Porphorymonas gingivalis* polypeptide, the polypeptide comprising;
an amino acid sequence selected from the group consisting of SEQ. ID. NO. 265 to SEQ. ID. NO. 528, SEQ. ID. NO. 531 and SEQ. ID. NO. 532; or
an amino acid sequence at least 85%, preferably at least 95%, identical to an amino acid sequence selected from the group consisting of SEQ. ID. NO. 265 to SEQ. ID. NO. 528, SEQ. ID. NO. 531 and SEQ. ID. NO. 532; or
at least 40 amino acids having a contiguous sequence of at least 40 amino acids identical to a contiguous amino acid sequence selected from the group consisting of SEQ. ID. NO. 265 to SEQ. ID. NO. 528, SEQ. ID. NO. 531 and SEQ. ID. NO. 532.

In an embodiment of the present invention the polypeptide comprises;
an amino acid sequence selected from the group consisting of SEQ. ID. NO. 386 to SEQ. ID. NO. 528 and SEQ. ID. NO. 532; or
an amino acid sequence at least 85%, preferably at least 95%, identical to an amino acid sequence selected from the group consisting of SEQ. ID. NO. 386 to SEQ. ID. NO. 528 and SEQ. ID. NO. 532; or
at least 40 amino acids having a contiguous sequence of at least 40 amino acids identical to a contiguous amino acid sequence selected from the group consisting of SEQ. ID. NO. 386 to SEQ. ID. NO. 528 and, SEQ. ID. NO. 532.

As used herein % identity for polypeptides is to be calculated using the alignment algorithm of Needleman and Munsch (9) using a standard protein scoring matrix (Blosum 50).

In a preferred embodiment of the present invention the polypeptide comprises an amino acid sequence selected from the group consisting of SEQ. ID. NO. 386, SEQ. ID. NO. 424; SEQ. ID. NO. 425, SEQ. ID. NO. 434, SEQ. ID. NO. 447, SEQ. ID. NO. 458, SEQ. ID. NO. 475, SEQ. ID. NO. 498, SEQ. ID. NO. 499, SEQ. ID. NO. 500, SEQ. ID. NO. 501, SEQ. ID. NO. 387, SEQ. ID. NO. 400, SEQ. ID. NO. 411, SEQ. ID. NO. 419, SEQ. ID. NO. 420, SEQ. ID. NO. 427, SEQ. ID. NO. 429, SEQ. ID. NO. 433, SEQ. ID NO. 437, SEQ. ID. NO. 438, SEQ. ID. NO. 443, SEQ. ID. NO. 444, SEQ. ID. NO. 448, SEQ. ID. NO. 449, SEQ. ID. NO. 452, SEQ. ID. NO. 455, SEQ. ID. NO. 457, SEQ. ID. NO. 459, SEQ. ID. NO. 461, SEQ. ID. NO. 462, SEQ. ID. NO. 463, SEQ. ID. NO. 467, SEQ. ID. NO. 468, SEQ. ID. NO. 469, SEQ. ID. NO. 482, SEQ. ID. NO. 484, SEQ. ID. NO. 485, SEQ. ID. NO. 494, SEQ. ID. NO. 508, SEQ. ID. NO. 509, SEQ. ID. NO. 510, SEQ. ID. NO. 520, SEQ . ID. NO. 521, SEQ. ID. NO. 522, SEQ. ID. NO. 525, SEQ. ID. NO. 526, SEQ. ID. NO. 528, SEQ. ID. NO. 389, SEQ. ID. NO. 390 and SEQ. ID. NO. 391.

In another preferred embodiment of the present invention the polypeptide comprises an amino acid sequence selected from the group consisting of residue 422 to residue 531 of SEQ. ID. NO. 303, residue 534 to residue 582 of SEQ. ID. NO. 303, residue 127 to residue 232 of SEQ. ID. NO. 301, residue 240 to residue 259 of SEQ. ID. NO. 301, residue 139 to residue 156 of SEQ. ID. NO. 295, residue 160 to residue 178 of SEQ. ID. NO. 295, residue 180 to residue 207 of SEQ. ID. NO. 295, residue 221 to residue 257 of SEQ. ID. NO. 295, residue 259 to residue 323 of SEQ. ID. NO. 295, residue 885 to residue 985 of SEQ. ID. NO. 299, residue 147 to residue 259 of SEQ. ID. NO. 363, residue 140 to residue 252 of SEQ. ID. NO. 344, residue 247 to residue 356 of SEQ. ID. NO. 353, residue 359 to residue 391 of SEQ. ID. NO. 353, residue 120 to residue 254 of SEQ. ID. NO. 300, residue 287 to residue 311 of SEQ. ID. NO. 286, residue 313 to residue 352 of SEQ. ID. NO. 286, residue 354 to residue 401 of SEQ. ID. NO. 286, residue 208 to residue 252 of SEQ. ID. NO. 287, residue 259 to residue 373 of SEQ. ID. NO. 287, residue 5 to residue 120 of SEQ. ID. NO. 293, residue 123 to residue 139 of SEQ. ID. NO. 293, residue 233 to residue 339 of SEQ. ID. NO. 265, residue 67 to residue 228 of SEQ. ID. NO. 278, residue 130 to residue 172 of SEQ. ID. NO. 274, residue 174 to residue 238 of SEQ. ID. NO. 274, residue 99 to residue 112 of SEQ. ID. NO. 274, residue 114 to residue 128 of SEQ. ID. NO. 274, residue 26 to residue 69 of SEQ. ID. NO. 285, residue 71 to residue 128 of SEQ. ID. NO. 285, residue 130 to residue 146 of SEQ. ID. NO. 285, residue 620 to residue 636 of SEQ. ID. NO. 327, residue 638 to residue 775 of SEQ. ID. NO. 327, residue 397 to residue 505 of SEQ. ID. NO. 301, residue 528 to residue 545 of SEQ. ID. NO. 301, residue 556 to residue 612 of SEQ. ID. NO. 301, residue 614 to residue 631 of SEQ. ID. NO. 301, residue 633 to residue 650 of SEQ. ID. NO. 301, residue 553 to residue 687 of SEQ. ID. NO. 299, residue 305 to residue 447 of SEQ. ID. NO. 289, residue 1 to residue 52 of SEQ. ID. NO. 364, residue 65 to residue 74 of SEQ. ID. NO. 364, residue 486 to residue 604 of SEQ. ID. NO. 275, residue 158 to residue 267 of SEQ. ID. NO. 272, residue 270 to residue 282 of SEQ. ID. NO. 272, residue 163 to residue 237 of SEQ . ID. NO. 273, residue 240 to residue 251 of SEQ. ID. NO. 273, residue 213 to residue 344 of SEQ. ID. NO. 282, residue 183 to residue 324 of SEQ. ID. NO. 292, residue 327 to residue 341 of SEQ. ID. NO. 292, residue 352 to residue 372 of SEQ. ID. NO. 292, residue 141 to residue 166 of SEQ. ID. NO. 271, residue 168 to residue 232 of SEQ. ID. NO. 271, residue 1 to residue 13 of SEQ. ID. NO. 302, residue 15 to residue 28 of SEQ. ID. NO. 302, residue 30 to residue 72 of SEQ. ID. NO. 302, residue 476 to residue 529 of SEQ. ID. NO. 277, residue 41 to residue 146 of SEQ. ID. NO. 299, residue 149 to residue 162 of SEQ. ID. NO. 299, residue 166 to residue 177 of SEQ. ID. NO. 299, residue 192 to residue 203 of SEQ. ID. NO. 299, residue 71 to residue 343 of SEQ. ID. NO. 290, residue 346 to residue 363 of SEQ. ID. NO. 290, residue 36 to residue 240 of SEQ. ID. NO. 331, residue 242 to residue 270 of SEQ. ID. NO. 331, residue 1 to residue 192 of SEQ. ID. NO. 375, residue 266 to residue 290 of SEQ. ID. NO. 375, residue 23 to residue 216 of SEQ. ID. NO. 279, residue 220 to residue 270 of SEQ. ID. NO. 279, residue 285 to residue 386 of SEQ. ID. NO. 279, residue 84 to residue 234 of SEQ. ID. NO. 297, residue 248 to residue 259 of SEQ. ID. NO. 297, residue, 261 to residue 269 of SEQ. ID. NO. 297, residue 275 to residue 402 of SEQ. ID. NO. 294, residue 1 to residue 171 of SEQ. ID. NO. 298, residue 403 to residue 417 of SEQ. ID. NO. 307, residue 420 to residue 453 of SEQ. II7. NO. 307, residue 456 to residue 464 of SEQ. ID. NO. 307, residue 468 to residue 690 of SEQ. ID. NO. 307, residue 1 to residue 285 of SEQ. ID. NO. 304, residue 287 to residue 315 of SEQ. ID. NO. 304, residue 318 to residue 336 of.SEQ. ID. NO. 304, residue 255 to residue 269 of SEQ. ID. NO. 342, residue 271 to residue 337 of SEQ. ID. NO. 342, residue 347 to residue 467 of SEQ. ID. NO. 281, residue 116 to residue 136 of SEQ. ID. NO. 375, residue 138 to residue 357 of SEQ. ID. NO. 375, residue 133 to residue 423 of SEQ. ID. NO. 364, residue 141 to residue 299 of SEQ. ID. NO. 305, residue 202 to residue 365 of SEQ. ID. NO. 296, residue 134 to residue 426 of SEQ. ID. NO. 288, residue 1 to residue 218 of SEQ. ID: NO. 276, residue 1 to residue 246 of SEQ. ID. NO. 280, residue 444 to residue 608 of SEQ. ID. NO. 364, residue 10 to residue 686 of SEQ. ID. NO. 283, residue 1 to residue 148 of SEQ. ID. NO. 296, residue 1 to residue 191 of SEQ. ID. NO. 287, residue 193 to residue 204 of SEQ. ID. NO. 287, residue 209 to residue 373 of SEQ. ID. NO. 287, residue 211 to residue 470 of SEQ. ID. NO. 284, residue 472 to residue 482 of SEQ. ID. NO. 284, residue 133 to residue 144 of SEQ. ID. NO. 281, residue 146 to residue 336 of SEQ. ID. NO. 281, residue 1 to residue 264 of SEQ. ID. NO. 303, residue 265 to residue 295 of SEQ. ID. NO. 303, residue 297 to residue 326 of SEQ. ID. NO. 303, residue 328 to residue 338 of SEQ. ID. NO. 303, residue 247 to residue 356 of SEQ. ID. NO. 353, residue 358 to residue 391 of SEQ. ID. NO. 35.3, residue Z57 to residue 288 of SECZ. ID. NO. 298, residue 290 to residue 385 of SEQ. ID. NO. 298, residue 245 to residue 256 of SEQ. ID. NO. 298, residue 422 to residue 802 of SEQ. ID. NO. 303, residue 803 to residue 814 of SEQ. ID. NO. 303, residue 139 to residue 156 of SEQ. ID. NO. 295, residue 160 to residue 340 of SEQ. ID. NO. 295, residue 145 to residue 361 of SEQ. ID. NO. 282, residue 363 to residue 387 of SEQ. ID. NO. 282, residue 398 to residue 471 of SEQ. ID. NO. 282, residue 573 to residue 679 of SEQ. ID. NO. 320, residue 27 to residue 168 of SEQ. ID. NO. 291, residue 170 to residue 183 of SEQ. ID. NO. 291, residue 185 to residue 415 of SEQ. ID. NO. 291, residue 1 to residue 301 of SEQ. ID. NO. 364, residue 114 to residue 702 of SEQ. ID. NO. 337, residue 377 to residue 412 of SEQ. ID. NO. 321, residue 413 to residue 772 of SEQ. ID. NO. 321, residue 14 to residue 454 of SEQ. ID. NO. 265, residue 129 to residue 614 of SEQ. ID. NO. 268, residue 1 to residue 930 of SEQ. ID. NO. 300, residue 932 to residue 1046 of SEQ. ID. NO. 300, residue 1 to residue 301 of SEQ. ID. NO. 364, residue 1 to residue 42 of SEQ. ID. NO. 381, residue 44 to residue 973 of SEQ. ID. NO. 381, residue 1 to residue 93 of SEQ. ID. NO. 358, residue 95 to residue 179 of SEQ. ID. NO. 358, residue 181 to residue 227 of SEQ. ID. NO. 358, residue 114 to residue 702 of SEQ. ID. NO. 337, residue 1 to residue 659 of SEQ. ID. NO. 355, residue 661 to residue 907 of SEQ. ID. NO. 355, residue 1 to residue 131 of SEQ. ID. NO. 370, residue 133 to residue 601 of SEQ. ID. NO. 370, residue 1 to residue 813 of SEQ. ID. NO. 344, residue 377 to residue 412 of SEQ. ID. NO. 321, residue 413 to residue 772 of SEQ. ID. NO. 321, and residue 189 to residue 614 of SEQ. ID. NO. 364.

In a second aspect the present invention consists in a n isolated antigenic *Porphorymonas gingivalis* polypeptide, the polypeptide comprising an amino acid sequence selected from the group consisting of SEQ. ID. NO. 386 to SEQ. ID. NO. 528 and SEQ. ID. NO. 532 less the leader sequence set out in Table 3.

In a third aspect the present invention consists in an isolated DNA molecule, the DNA molecule comprising a nucleotide sequence which encodes the polypeptide of the first aspect the present invention or a sequence which hybridises thereto under stringent conditions.

It is preferred that the isolated DNA molecule comprises a nucleotide sequence selected from the group consisting of SEQ. ID. NO. 1 to SEQ. ID. NO. 264, SEQ. ID. NO. 529 and SEQ. ID. NO. 530.

In a fourth aspect the present invention consists in a recombinant expression vector comprising the DNA molecule of the second aspect of the present invention operably linked to a transcription regulatory element.

The present invention also provides a cell comprising this recombinant expression vector.

In a further aspect the present invention consists in a method for producing a P. *gingivalis* polypeptide comprising culturing the cell under conditions that permit expression of the polypeptide.

In yet a further aspect the present invention provides a composition for use in raising an immune response directed against P. *gingivalis* in a subject, the composition comprising an effective amount of at least one polypeptide of the first aspect of the present invention, or at least one DNA molecule of the second aspect of the present invention, or both,and a pharmaceutically acceptable carrier. It is preferred that the pharmaceutically acceptable carrier is an adjuvant In other aspects the present invention provides methods of treatingP. *gingivalis* infection in subject comprising the administration of the composition to the subject such that treatment of P. *gingivalis* infection occurs. The treatment may be prophylactic or therapeutic.

In yet another aspect the present invention provides an antibody raised against a polypeptide of the first aspect the invention. The antibody may be polyclonal or monoclonal. The present invention also provides compositions including these antibodies. It is preferred that these compositions are adapted for oral use and may be, for example, dentrifices, mouthwashes, etc.

In a still further aspect the present invention provides a nucleotide probe comprising at least 18 nucleotides and having a contiguous sequence of at least 18 nucleotides identical to a contiguous nucleotide sequence selected from the group consisting of SEQ. ID. NO.1 to SEQ. ID. NO. 121, SEQ. ID. NO. 529, and sequences complementary thereto. It is preferred that the probe further comprises a detectable label.

The present invention also provides a method for detecting the presence of P. *gingivalis* nucleic acid in a sample comprising:
(a) contacting a sample with the nucleotide probe under conditions in which a hybrid can form between the probe and a P. *gingivalis* nucleic acid in the sample; and
(b) detecting the hybrid formed in step (a), wherein detection of a hybrid indicates the presence of a P. *gingivalis* nucleic acid in the sample.

### DETAILED DESCRIPTION

### Definitions

A purified or isolated polypeptide or a substantially pure preparation of a polypeptide are used interchangeably herein and, as used herein, mean a polypeptide that has been separated from other proteins, lipids, and nucleic acids with which it naturally occurs. Preferably, the polypeptide is also separated from substances, e.g., antibodies or gel matrix, e.g., polyacrylamide, which are used to purify it Preferably, the polypeptide constitutes at least 10, 20, 50 70, 80 or 95% dry weight of the purified preparation. Preferably, the preparation contains: sufficient polypeptide to allow protein sequencing; at least 1, 10, or 100 mg of the polypeptide.

A purified preparation of cells refers to, in the case of plant or animal cells, an in vitro preparation of cells and not an entire intact plant or animal. In the case of cultured cells or microbial cells, it consists of a preparation of at least 10% and more preferably 50% of the subject cells:

A purified or isolated or a substantially pure nucleic acid, e.g., a substantially pure DNA, (are terms used interchangeably herein) is a nucleic acid which is one or both of the following: not immediately contiguous with both of the coding sequences with which it is immediately contiguous (i.e., one at the 5' end and one at the 3' end) in the naturally occurring genome of the organism from which the nucleic acid is derived; or which is substantially free of a nucleic acid with which it occurs in the organism from which the nucleic acid is derived. The term includes, for example, a recombinant DNA which is incorporated into a vector, e.g., into an autonomously replicating plasmid or virus, or into the genomic DNA of a prokaryote or eukaryote, or which exists as a separate molecule (e.g., a cDNA or a genomic DNA fragment produced by PCR or restriction endonuclease treatment) independent of other DNA sequences. Substantially pure DNA also includes a recombinant DNA which is part of a hybrid gene encoding additional P. *gingivalis* DNA sequence.

A "contig" as used herein is a nucleic acid representing a continuous stretch of genomic sequence of an organism.

An "open reading frame", also referred to herein as ORF, is a region of nucleic acid which encodes a polypeptide. This region may represent a portion of a coding sequence or a total sequence and can be determined from a stop to stop codon or from a start to stop codon.

As used herein, a "coding sequence" is a nucleic acid which is transcribed into messenger RNA and/or translated into a polypeptide when placed under the control of appropriate regulatory sequences. The boundaries of the coding sequence are determined by a translation start codon at the five prime terminus and a translation stop code at the three prime terminus. A coding sequence can include but is not limited to messenger RNA synthetic DNA, and recombinant nucleic acid sequences.

A "complement" of a nucleic acid as used herein refers to an antiparallel or antisense sequence that participates in Watson-Crick base-pairing with the original sequence.

A "gene product" is a protein or structural RNA which is specifically encoded by a gene.

As used herein, the term "probe" refers to a nucleic acid, peptide or other chemical entity which specifically binds to a molecule of interest Probes are often associated with or capable of associating with a label. A label is a chemical moiety capable of detection. Typical labels comprise dyes, radioisotopes, luminescent and chemiluminescent moieties, fluorophores, enzymes, precipitating agents, amplification sequences, and the Like. Similarly, a nucleic acid, peptide or other chemical entity which specifically binds to a molecule of interest and immobilizes such molecule is referred herein as a "capture ligand". Capture ligands are typically associated with or capable of associating with a support such as nitro-cellulose, glass, nylon membranes, beads, particles and the like. The specificity of hybridization is dependent on conditions such as the base pair composition of the nucleotides, and the temperature and salt concentration of the reaction. These conditions are readily discernible to one of ordinary skill in the art using routine experimentation.

Homologous refers to the sequence similarity or sequence identity between two polypeptides or between two nucleic acid molecules. When a position in both of the two compared sequences is occupied by the same base or amino acid monomer subunit, e.g., if a position in each of two DNA molecules is occupied by adenine, then the molecules are homologous at that position. The percent of homology between two sequences is a function of the number of matching or homologous positions shared by the two sequences divided by the number of positions compared x 100.

The terms peptides, proteins, and polypeptides are used interchangeably herein.

An "immunogenic component" as used herein is a moiety, such as an P. *gingivalis* polypeptide, analog or fragment thereof, that is capable of eliciting a humoral and/or cellular immune response in a host animal.

An "antigenic component" as used herein is a moiety, such as P. *gingivalis* polypeptide, analog or fragment thereof, that is capable of binding to a specific antibody with sufficiently high affinity to form a detectable antigen-antibody complex.

As used herein, the term "cell-specific promoter" means a DNA sequence that serves as a promoter, i.e., regulates expression of a selected DNA sequence operably linked to the promoter, and which effects expression of the selected DNA sequence in specific cells of a tissue. The term also covers so-called "leaky" promoters, which regulate expression of a selected DNA primarily in one tissue, but cause expression in other tissues as well.

As used herein, the term "control sequence" refers to a nucleic acid having a base sequence which is recognized by the host organism to effect the expression of encoded sequences to which they are ligated. The nature of such control sequences differs depending upon the host organism; in prokaryotes, such control sequences generally include a promoter, ribosomal binding site, terminators, and in some cases operators; in eukaryotes, generally such control sequences include promoters, terminators and in some instances, enhancers. The term control sequence is intended to include at a minimum, all components whose presence is necessary for expression, and may also include additional components whose presence is advantageous, for example, leader sequences.

As used herein, the term "operably linked" refers to sequences joined or ligated to function in their intended manner. For example, a control sequence is operably linked to coding sequence by ligation in such a way that expression of the coding sequence is achieved under conditions compatible with the control sequence and host cell.

A "sample" as used herein refers to a biological sample, such as, for example, tissue or fluid isolated from an individual (including without limitation plasma. serum, cerebrospinal fluid, lymph, tears, saliva and tissue sections) or from i*n* vitro cell culture constituents, as well as samples from the environment.

The practice of the invention will employ, unless otherwise indicated, conventional techniques of chemistry, molecular biology, microbiology, recombinant DNA, and immunology well known to those skilled in the art. Such techniques are described and explained throughout the literature in sources such as, J. Perbal, A Practical Guide to Molecular Cloning, John Wiley and Sons (1984), J. Sambrook et al., Molecular Cloning: A Laboratory Manual, Cold Spring Harbour Laboratory Press (1989), T.A. Brown (editor), Essential Molecular Biology: A Practical Approach, Volumes 1 and 2, IRL Press (1991), D.M. Glover and B.D. Hames (editors), DNA Cloning: A Practical Approach, Volumes 1-4, IRL Press (1995 and 1996), and F.M. Ausubel et al. (Editors), Current Protocols in Molecular Biology, Greene Pub. Associates and Wiley-Interscience (1988, including all updates until present). The disclosure of these texts are incorporated herein by reference.

### Pharmaceutically Acceptable Carriers

The antibodies, polypeptides and DNA of the present invention can be included in compositions which include a carrier or diluent. These compositions include pharmaceutical compositions where the carrier or diluent will be pharmaceutically acceptable. Pharmaceutically acceptable carriers or diluents include those used in compositions suitable for oral, rectal, nasal, topical (including buccal and sublingual), vaginal, parenteral (including subcutaneous, intramuscular, intravenous, intradermal, intrathecal and epidural) administration. They are non-toxic to recipients at the dosages and concentrations employed. Representative examples of pharmaceutically acceptable carriers or diluents include, but are not limited to; water, isotonic solutions which are preferably buffered at a physiological pH (such as phosphate-buffered saline or Tris-buffered saline) and can also contain one or more of, mannitol, lactose, trehalose, dextrose, glycerol, ethanol or polypeptides (such as human serum albumin). The compositions may conveniently be presented in unit dosage form and may be prepared by any of the methods well known in the art of pharmacy.

As will be well understood by those skilled in the art alterations, may be made to the amino acid sequences set out in the Sequence Listings. These alterations may be deletions, insertions, or substitutions of amino acid residues. The altered polypeptides can be either naturally occurring (that is to say, purified or isolated from a natural source) or synthetic (for example, by performing site-directed metagenesis on the encoding DNA). It is intended that such altered polypeptides which have at least 85%, preferably at least 95% identity with the sequences set out in the Sequence Listing are within the scope of the present invention. Antibodies raised against these altered polypeptides will also bind to the polypeptides having one of the sequences set out in the Sequence Listings. The level of % identity is to be calculated as set out above.

Protein sequences are homologous if they are related by divergence from a common ancestor. Consequently, a species homologue of the protein will be the equivalent protein which occurs naturally in another species. Within any one species a homologue may exist as numerous allelic variants, and these will be considered homologues of the protein. Allelic variants and species homologues can be obtained by following standard techniques known to those skilled in the art.

An allelic variant will be a variant that is naturally occurring within an individual organism.

### Mutants, Variants and Homology - Nucleic Acids

Mutant polynucleotides will possess one or more mutations which are deletions, insertions, or substitutions of nucleotide residues. Mutants can be either naturally occurring (that is to say, isolated from a natural source) or synthetic (for example, by performing site-directed metagenesis on the DNA). It is thus apparent that polynucleotides of the invention can be either naturally occurring or recombinant (that is to say prepared using recombinant DNA techniques).

An allelic variant will be a variant that is naturally occurring within an individual organism.

Nucleotide sequences are homologous if they are related by divergence from a common ancestor. Consequently, a species homologue of the polynucleotide will be the equivalent polynucleotide which occurs naturally in another species. Within any one species a homologue may exist as numerous allelic variants, and these will be considered homologues of the polynucleotide. Allelic variants and species homologues can be obtained by following standard techniques known to those skilled in the art.

### Antibody Production

Antibodies, either polyclonal or monoclonal, which are specific for a polypeptide of the present invention can be produced by a person skilled in the art using standard techniques such as, but not limited to, those described by Harlow et *al.* Antibodies: A Laboratory Manual, Cold Springs Harbor Laboratory Press (1988), and D. Catty (editor), Antibodies: A Practical Approach, IRL Press (1988).

Various procedures known in the art may be used for the production of polyclonal antibodies to epitopes of a protein. For the production of polyclonal antibodies, a number of host animals are acceptable for the generation of antibodies by immunization with one or more injections of a polypeptide preparation, including but not limited to rabbits, mice, rats, etc. Various adjuvants may be used to increase the immunological response in the host animal, depending on the host species, including but not limited to Freund's (complete and incomplete), mineral gels such as aluminium hydroxide, surface active substances such as lysalecithin, pluronic polyols, polyanions, oil emulsions, keyhole lympet hemocyanins, dinitrophenol, and potentially useful human adjuvants such as BCG (bacille Calmette-Guerin) and Corynebacterium parvum.

A monoclonal antibody to an epitope of a protein may be prepared by using any technique which provides for the production of antibody molecules by continuous cell lines in culture. These include but are not limited to the hybridoma technique originally described by Kohler-and Milstein (1975, Nature 256, 493-497), and the more recent human B-cell hybridoma technique (Kesber et al. 1983, Immunology Today 4:72) and EBV-hybridoma technique (Cole et al. 1985, Monoclonal Antibodies and Cancer Therapy, Alan R. Liss, Inc. pp. 77-96). In addition, techniques developed for the production of "chimeric antibodies" by splicing the genes from antibody molecule of appropriate antigen specificity together with genes from a human antibody molecule of appropriate biological activity may be used (Morrison et al. 1984, Proc. Natl. Acad. Sci., 81:885I-6855; Neuberger et al. 1984 Nature 312:604-608; Takeda et al. 1985 Nature 31:452-454). Alternatively, techniques described for the production of single chain antibodies (U.S. Patentw4,946;778) can be adapted to produce 4-specific single chain antibodies.

Recombinant human or humanized versions of monoclonal antibodies are a preferred embodiment for human therapeutic applications. Humanized antibodies may be prepared according to procedures in the literature (e.g. Jones et al. 1986, Nature 321:522-25; Reichman et al. 1988 Nature 332:323-27; Verhoeyen et al. 1988, Science 239:1534-36). The recently described "gene conversion metagenesis" strategy for the production of humanized monoclonal antibody may also be employed in the production of humanized antibodies (Carter et al. 1992 Proc. Natl. Acad. Sci. U.S.A. 89:4285-89). Alternatively, techniques for generating the recombinant phase library of random combinations of heavy and light regions may be used to prepare recombinant antibodies (e.g. Huse et al. 1989 Science 246:1275-81).

Antibody fragments which contain the idiotype of the molecule such as Fu F(ab1) and F(ab2) may be generated by known techniques. For example, such fragments include but are not limited to: the F(ab) E2 fragment which can be produced by pepsin digestion of the intact antibody molecule; the Fab' fragments which can be generated by reducing the disulfide bridges of the F(ab')2 fragment, and the two Fab fragments which can be generated by treating the antibody molecule with papain and a reducing agent. Alternatively, Fab expression libraries may be constructed (Huse et al. 1989, Science 246:1275-1281) to allow rapid and easy identification of monoclonal Fab fragment with the desired specificity to a protein.

### Adjuvants

"Adjuvant" means a composition comprised of one or more substances that enhances the immunogenicity and efficacy of a vaccine composition. Non-limiting examples of suitable adjuvants include squalane and squalene (or other oils of animal origin); block copolymers; detergents such as Tween®-80; Quil® A, mineral oils such as Drakeol or Marcol, vegetable oils such as peanut oil; Corynebacterium-derived adjuvants such as Corynebacterium parvum; Propionibacterium-derived adjuvants such as Propionibacterium acne; Mycobacterium bovis (Bacillus Calmetic and Guerinn or BCG); interleukins such as interleukin 2 and interleukin-12; monokines such as interleukin 1; tumour necrosis factor; interferons such as gamma interferon; combinations such as saponin-aluminium hydroxide or Quil-A aluminium hydroxide; liposomes; ISCOM adjuvant; mycobacterial cell wall extract; synthetic glycopeptides such as muramyl dipeptides or other derivatives; Avridine; Lipid A; dextran sulfate; DEAE-Dextran or DHAE-Dextran with aluminium phosphate; carboxypolymethylene such as Carbopol' EMA; acrylic copolymer emulsions such as Neocryl A640 (e.g. U.S. Pat. No. 5,047,238); vaccinia or animal posvirus proteins; sub-viral particle adjuvants such as cholera toxin, or mixtures thereof.

As used herein, stringent conditions are those that (1) employ low ionic strength and high temperature for washing, for example, 0.015 M NaCl/0.0015 M sodium citrate/0.1% NaDodSO4 at 50°C; (2) employ during hybridisation a denaturing agent such as formamide, for example, 50% (vol/vol) formamide with 0.1% bovine serum albumin, 0.1% Ficoll, 0.1% polyvinylpyrrolidone, 50 mM sodium phosphate buffer at pH 6.5 with 750 mM NaCl, 75 mM sodium citrate at 42°C; or (3) employ 50% formamide, 5 x SSC (0.75 M NaCl, 0.075 M sodium citrate), 50 mM sodium phosphate (pH 6.8), 0.1% sodium pyrophosphate, 5 x Denhardfs solution, sonicated salmon sperm DNA (50 µg/ml), 0.1% SDS and 10% dextran sulfate at 42°C in 0.2 x SSC and 0.1% SDS

As will be understood the present invention includes within its scope DNA vaccination. Further information regarding DNA vaccination may be found in Donnelly et al, Journal of Immunological Methods 176(1994) 145-152, the disclosure of which is incorporated herein by reference.

Throughout this specification the word "comprise", or variations such as "comprises" or "comprising", will be understood to imply the inclusion of a stated element or integer or group of elements or integers but not the exclusion of any other element or integer, or group of elements or integers.

### Preparation of the P. gingivalis library for sequencing.

To determine the DNA sequence of P. *gingivalis* genomic DNA was isolated from P. *gingivalis* strain W50 (ATCC 53978) essentially by the method described by Mamur J. (J. Mol. Biol. **3**, 208-218, 1961). Cloning of DNA fragments was performed essentially as described by Fleischmann *et al.,* (Science; 269, 496-512,1995)(2). Briefly, purified genomic DNA from P. *gingivalis* was nebulized to fragment the DNA and was treated with Bal31 nuclease to create blunt ends then run twice through preparative 1% agarose gels. DNA fragments of 1.6-2.0 kb were excised from the gel and the DNA recovered. This DNA was then ligated to the vector pUC18 (SmaI digested and dephosphorylated; Pharmacia) and electrophoresed through a 1% preparative agarose gel. The fragment comprising linear vector plus one insert was excised, purified and this process repeated to reduce any vector without insert contamination. The recovered vector plus insert DNA was blunt-ended with T4 DNA polymerase, then a final ligation to produce circular DNA was performed. Aliquots of Epicurian Coli Electroporation-Competent Cells (Stratagene) were transformed with the ligated DNA and plated out on SOB agar antibiotic diffusion plates containing X-gal and incubated at 37°C overnight. Colonies with inserts appeared white and those without inserts (vector alone) appeared blue. Plates were stored at 4°C until the white clones were picked and expanded for the extraction of plasmid DNA for sequencing.

### DNA sequencing

Plasmid DNA was prepared by picking bacterial colonies into 1.5ml of LB, TB or SOB broth supplemented with 50-100ug/ml Ampicillin in 96 deep well plates. Plasmid DNA was isolated using the QIAprep Spin or QIAprep 96 Turbo miniprep kits (QIAGEN GmbH, Germany). DNA was eluted into a 96 well gridded array and stored at -20C.

Sequencing reactions were performed using ABI PRISM Dye Terminator and ABI PRISM BIGDye Terminator Cycle Sequencing Ready Reaction kits with AmpliTaq DNA polymerase FS (PE Applied Biosystems, Foster City, CA) using the M13 Universal forward and reverse sequencing primers. Sequence reactions were conducted on either a Perkin-Elmer GeneAmp 9700 (PE Applied Biosystems) or Hybaid PCR Express (Hybaid, UK) thermal cyclers. Sequencing reactions were analysed on ABI PRISM 377 DNA sequencers (PE Applied Biosystems).

The sequences obtained are set out below. The relationship between these sequences is set out in Table 1. The initiation codon was calculated using a combination of sequence homology alignment (FASTA), signal sequence prediction (PSORT, SignalP) or ORF prediction (GeneMark).

**Table 1: Reference table indicating the relationships of each sequence ID to the selected proteins.**

| Protein name | DNA sequence of complete ORF | Amino acid sequence of complete ORF | DNA sequence of protein | Amino acid sequence of protein |
|---|---|---|---|---|
| PG1 | 1 | 265 | 122 | 386 |
| PG10 | 2 | 268 | 123 | 387 |
| PG100 | 3 | 267 | 124 | 388 |
| PG101 | 4 | 268 | | |
| PG102 | 5 | 269 | 125, 126 | 389, 390 |
| PG104 | 6 | 270 | 127 | 391 |
| PG105 | 7 | 271 | 128 | 392 |
| PG106 | 8 | 272 | 129 | 393 |
| PG107 | 9 | 273 | 130, 131, 132 | 394, 395, 398 |
| PG108 | 10 | 274 | 133 | 397 |
| PG109 | 11 | 275 | 134, 135 | 398, 399 |
| PG11 | 12 | 276 | 136 | 400 |
| PG110 | 13 | 277 | 137 | 401 |
| PG111 | 14 | 278 | | |
| PG112 | 15 | 279 | 138, 139 | 402, 403 |
| PG113 | 16 | 280 | 140 | 404 |
| PG114 | 17 | 281 | 141 | 405 |
| PG115 | 18 | 282 | 142 | 406 |
| PG116 | 19 | 283 | 143 | 407 |
| PG117 | 20 | 284 | 144 | 408 |
| PG118 | 21 | 285 | 145 | 409 |
| PG119 | 22 | 286 | 146 | 410 |
| PG12 | 23 | 287 | 147 | 411 |
| PG120 | 24 | 288 | 148 | 412 |
| PG121 | 25 | 289 | 149 | 413 |
| PG122 | 26 | 290 | 150 | 414 |
| PG123 | 27 | 291 | 151 | 415 |
| PG124 | 28 | 292 | 152 | 416 |
| PG125 | 29 | 293 | 153 | 417 |
| PG126 | 30 | 294 | 154 | 418 |
| PG13 | 31 | 295 | 155 | 419 |
| PG14 | 32 | 296 | 156 | 420 |
| PG15 | 33 | 297 | 157 | 421 |
| PG16 | 34 | 298 | 158 | 422 |
| PG18 | 35 | 299 | 159 | 423 |
| PG2 | 36 | 300 | 160, 161 | 424, 425 |
| PG21 | 37 | 301 | 162 | 426 |
| PG22 | 38 | 302 | 163 | 427 |
| PG23 | 39 | 303 | 164 | 428 |
| PG24 | 40 | 304 | 165 | 429 |
| PG25 | 41 | 305 | 166 | 430 |
| PG27 | 42 | 306 | 167 | 431 |
| PG28 | 43 | 307 | 168 | 432 |
| PG29 | 44 | 308 | 169 | 433 |
| PG3 | 45 | 309 | 170 | 434 |
| PG30 | 46 | 310 | 171 | 435 |
| PG31 | 47 | 311 | 172 | 436 |
| PG32 | 48 | 312 | 173 | 437 |
| PG33 | 49 | 313 | 174 | 438 |
| PG34 | 50 | 314 | 175, 176 | 439, 440 |
| PG35 | 51 | 315 | 177 | 441 |
| PG36 | 52 | 316 | 178 | 442 |
| PG37 | 53 | 317 | 179, 180 | 443,444 |
| PG38 | 54 | 318 | 181 | 445 |
| PG39 | 55 | 319 | 182 | 446 |
| PG4 | 56 | 320 | 183 | 447 |
| PG40 | 57 | 321 | 184 | 448 |
| PG41 | 58 | 322 | 185 | 449 |
| PG42 | 59 | 323 | 186 | 450 |
| PG43 | 60 | 324 | 187 | 451 |
| PG44 | 61 | 325 | 188 | 452 |
| PG45 | 62 | 326 | 189 | 453 |
| PG46 | 63 | 327 | 190 | 454 |
| PG47 | 64 | 328 | 191 | 455 |
| PG48 | 65 | 329 | 192 | 456 |
| PG49 | 66 | 330 | 193 | 457 |
| PG5 | 67 | 331 | 194 | 458 |
| PG50 | 68 | 332 | 195 | 459 |
| PG51 | 69 | 333 | 196 | 460 |
| PG52 | 70 | 334 | 197 | 461 |
| PG53 | 71 | 335 | 198 | 462 |
| PG54 | 72 | 336 | 199 | 463 |
| PG55 | 73 | 337 | 200 | 464 |
| PG56 | 74 | 338 | 201,202 | 465,466 |
| PG57 | 75 | 339 | 20.3, 204, 205 | 467, 468, 469 |
| PG58 | 76 | 340 | 206, 207 | 470, 471 |
| PG59 | 77 | 341 | 208, 209, 210 | 472, 473, 474 |
| PG6 | 78 | 342 | 211 | 475 |
| PG60 | 79 | 343 | 212 | 476 |
| PG61 | 80 | 344 | 213 | 477 |
| PG62 | 81 | 345 | 214 | 478 |
| PG63 | 82 | 346 | 215 | 479 |
| PG64 | 83 | 347 | 216 | 480 |
| PG65 | 84 | 348 | 217 | 481 |
| PG66 | 85 | 349 | 218 | 482 |
| PG67 | 86 | 350 | 219 | 483 |
| PG68 | 87 | 351 | 220, 221 | 484, 485 |
| PG69 | 88 | 352 | 222 | 486 |
| PG7 | 89 | 353 | 223 | 487 |
| PG70 | 90 | 354 | 224 | 488 |
| PG71 | 91 | 355 | 225 | 489 |
| PG72 | 92 | 356 | 226 | 490 |
| PG73 | 93 | 357 | 227 | 491 |
| PG74 | 94 | 358 | 228 | 492 |
| PG75 | 95 | 359 | 229 | 493 |
| PG76 | 96 | 360 | 230 | 494 |
| PG77 | 97 | 361 | 231 | 495 |
| PG78 | 98 | 362 | 232 | 496 |
| PG79 | 99 | 363 | 233 | 497 |
| PG8 | 100 | 364 | 234, 235, 236, 237 | 498, 499, 500, 501 |
| PG80 | 101 | 365 | 238 | 502 |
| PG81 | 102 | 366 | 102 | 366 |
| PG82 | 103 | 367 | 239 | 503 |
| PG83 | 104 | 368 | 240 | 504 |
| PG84 | 105 | 369 | 241, 242 | 505, 506 |
| PG85 | 106 | 370 | 243 | 507 |
| PG86 | 107 | 371 | 244, 245 | 508, 509. |
| PG87 | 108 | 372 | 246 | 510 |
| PG88 | 109 | 373 | 247, 248, 249 | 511, 512 ,513 |
| PG89 | 110 | 374 | 250 | 514 |
| PG9 | 111 | 375 | 251, 252, 253 | 515, 516, 517 |
| PG90 | 112 | 376 | 254, 255 | 518, 519 |
| PG91 | 113 | 377 | 256 | 520 |
| PG92 | 114 | 378 | 257 | 521 |
| PG93 | 115 | 379 | 258 | 522 |
| PG94 | 116 | 380 | 259 | 523 |
| PG95 | 117 | 381 | 260 | 524 |
| PG96 | 118 | 382 | 261 | 525 |
| PG97 | 119 | 383 | 262 | 526 |
| PG98 | 120 | 384 | 263 | 527 |
| PG99 | 121 | 385 | 264 | 528 |
| PG127 | 529 | 531 | 530 | 532 |

### DNA sequence analysis

DNA files in FASTA format were converted to GCG format files and imported into a database. The DNA files were translated into amino acid files using the program Flip obtained from ANGIS(Australian Genomic Information Service, University of Sydney, Australia). A series of bioinformatic analyses were performed on the proteins in order to select potential vaccine candidates. The programs used were FASTA homology searching (1), PSORT (2,3), SignalP (4), TopPred (5), and GeneMark (6). The proteins and their bioinformatic results were stored in the custom written database for search and retrieval of proteins with the desired characteristics

The FASTA homology results for these proteins were then examined for any alignment with a protein suggesting surface location or vaccine efficacy. All proteins were searched for homology against a non-redundant bacterial protein database compiled by ANGIS using the FASTA algorithm. The settings used for the FASTA searches were Ktup = 2, gap creation penalty = -12, gap extension penalty = -2, width for deriving alignment in opt = 16 and the Blosum 50 scoring matrix. Individual FASTA search results were examined for significant homology by statistical probability and amino acid alignments. The results are set out in Table 2.

Protein files were then trimmed to the first, second, third, fourth and fifth methionine residues using a protein trimming program (ANGIS). The trimmed proteins were then subjected to PSORT analysis for the detection of signal sequences and the prediction of cell location. Proteins exhibiting a PSORT probability of outer membrane >0.8 were considered to indicate surface localisation. A second signal sequence detection program SignalP was also performed and, in certain instances, this program detected signals not identified with PSORT. All proteins identified by other methods were also analysed by PSORT and SignalP. Previously, the C-terminal amino acid of bacterial outer membrane proteins has been shown to be important for the assembly of the protein on the outer membrane (7). A typical structure definition for outer membrane proteins has been determined as the presence of a signal sequence at the N-terminus and a tyrosine or phenylalanine at the C-terminus. A number of the selected proteins exhibit this characteristic structure. The program TopPred was used to determine the presence and number of membrane spanning domains (MSDs) and the presence of such sequences indicates a preference to be attached to membranes such as the outer membrane. The results of PSORT, SignalP and TopPred analyses with the C-terminal amino acids of the selected proteins are set out in Table 3.

The 70 amino acids from the C-terminus of a number of P. *gingivalis* outer membrane proteins share 50-100% protein sequence identity. These proteins included RGP1, RGP2, KGP, HagA, HagC, HagD, prtH and prtT. This conserved motif may be involved in the attachment or sorting of proteins to the outer membrane. The protein data set was searched using FASTA homology as described above and a number of novel proteins were identified which demonstrate similar motifs at their C-termini. The results are listed in **Table 4**

The TonBIII box is a 30 amino acid motif present within TonB outer membrane receptors in a wide variety of bacteria. The TonBIII box of *P. gingivalis* (8) was used to search the protein data set for homology by FASTA as described above. Those proteins demonstrating significant homology are listed in Table 5.

**Table 2: FASTA protein homology results of complete ORFs against a non-redundant protein database.**

| Protein name | Homology description | Genbank accession number | Length of homolog | Length of *P. gingivalis* protein | FASTA homology results | | |
|---|---|---|---|---|---|---|---|
| | | | | | Identity % | Overlap | E value |
| PG1 | 48kD outer membrane protein, Actinobacillus pleuropneumoniae | U24492 | 449aa | 451aa | 32 | 454aa | 1.40E-42 |
| PG2 | Outer membrane protein (susC), Bacteroides thetaiotaomicron | L49338 | 1038aa | 1017aa, 1014aa | 28 | 1099aa | 4.60E-32 |
| PG3 | Outer membrane porin F adhesin, Pseudomonas fluorescens | U19743 | 317aa, | 223aa | 35 | 187aa | 1 1.10E-10 |
| PG4 | Outer membrane protein A. Escherichia fergusonil | M83352 | 243aa | 672aa | 48 | 88aa | 4.10E-10 |
| PG5 | Adhesin protein (AdcA). Streptococcus pneumoniae | Z71552 | 423aa | 315aa | 25 | 279aa | 9.40E-15 |
| PG6 | Hemolysin A (phyA). Prevotella melaninogenica | U27587 | 332aa | 324aa | 60 | 306aa | 3.00E-74 |
| PG7 | Hemolysin (tlyC), Serpulina hyodysenteriae | X73141 | 266aa | 404aa | 33 | 266aa | 1.40E-24 |
| PG8 | Heme uptake protein A. Bacteriodes fragilis | X97122 | 431aa | 598aa, 550aa, 458aa, 426aa | 79 | 417aa | 6.70E.121 |
| PG9 | Internalin A (in1A), Lysteria monocytogenes | M67471 | 744aa | 1266aa, 1232aa, 1174aa | 38 | 340aa | 7.30E-23 |
| PG10 | Macrophage infectivity potentiator (MIP), Legionella oakridgensis. | U92214 | 234aa | 195aa | 50 | 201aa | 4.70E-31 |
| PG11 | Haemagglutinin (phg), Prevotella intermedia | AF017417 | 309aa | 313aa | 44 | 309aa | 3.80E-44 |
| PG12 | Outer membrane lipoprotein. Haemophilus influenzae | M88502 | 274aa | 271aa | 36 | 254aa | 9.60E-27 |
| PG13 | Ferric receptor (cfrA), Compylobacter coli | U80812 | 696aa | 757aa | 24 | 625aa | 1.20E-18 |
| PG14 | 38kD antigen. Helicobacter pylori | U88810 | 329aa | 331aa | 37 | 328aa | 1.10E.35 |
| PG15 | Outer membrane protein. Erwinia amylovara | X77921 | 377aa | 267aa | 30 | 253aa | 5.40E-08 |
| PG10 | C terminal protease, Bartonella bacilliformis | L37094 | 434aa | 569aa | 38 | 357aa | 3.00E-35 |
| PG18 | Protein-export membrane protein (secD), Helicobacter pylori | AE000652 | 503aa | 981aa | 32 | 611aa | 1.10E-36 |
| PG21 | Surface antigen gene, Methanosarcina mazei | X84710 | 783aa | 821aa | 37 | 331aa | 6.20E-33 |
| PG22 | Alpha-hemolysln gene, Aeromonas hydrophila | L36462 | 85aa | 108aa | 57 | 67aa | 2.60E-14 |
| PG23 | clpA/clpB protease. Bacillus subtilis | D26185 | 810aa | 859aa | 45 | 855aa | 7.10E-122 |
| PG24 | Putative hemolysin, Streptococcus mutans | AF051356 | 445aa | 417aa | 29 | 432aa | 1.80E-29 |
| PG25 | Cysteine protease, Porphyromonas gingivalis | U54891 | 1723aa | 293aa | 42 | 142aa | 1.10E-12 |
| PG27 | TonB linked adhesin, Porphyromonas gingivalis | Y07618 | 1097aa | 312aa | 45 | 360aa | 3.20E-41 |
| PG28 | Cysteine protease / hemagglutinin, Porphyromonas gingivalls | S75942 | 886aa | 843aa | 35 | 838aa | 7.00E-90 |
| PG30 | Putative NlpD lipoprotein, Aquifex aeolicus | AE000754 | 187aa | 337aa | 42 | 142aa | 1.80E-12 |
| PG31 | Hemolysin (tlvC), Serpulina hysdysenteriae | X73141 | 141aa | 151aa | 31 | 123aa | 1.80E-07 |
| PG32 | Major outer membrane protein (oprF), Pseudomonas aeruginosa | M94078 | 350aa | 391aa | 26 | 382aa | 3.40E-07 |
| PG33 | Major outer membrane protein (oprF),Pseudomonas fluorescens | L21200 | 317aa | 385aa | 32 | 183aa | 2.30E-D6 |
| PG34 | Putative membrane protein. Rhodobacter capsulatus | Q07396 | 193aa | 190aa | 46 | 190aa | 2.20E-36 |
| PG35 | Colcin 1 receptor, Escherichia coli | J04229 | 663aa | 833aa | 25 | 590aa | 2.40E-10 |
| PG36 | Outer membrane antigen (oma87), Pasteurella multocida | U80439 | 789aa | 891aa | 21 | 894aa | 3.70E-10 |
| PG37 | Cationic outer membrane protein (ompH), Yersinia enterocolitica | M34854 | 164aa | 174aa, 170aa | 27 | 168aa | 4.30E-07 |
| PG38 | Cationic outer membrane protein (ompH), Yersinia enterocolitica | M34854 | 104aa | 163aa | 23 | 160aa | 5.90E-05 |
| PG30 | Outer membrane protein (susC) Bacteroides thetaiotaomicron | L49338 | 1038aa | 827aa | 24 | 347as | 1.50E-06 |
| PG40 | Heme receptor (Hut A), Vibrio cholera | Q56644 | 693aa | 772aa | 23 | 722aa | 4.90E-09 |
| PG41 | Outer membrane protein (tolC), Escherichia coli | X54049 | 495aa | 462aa | 22 | 436aa | 4.60E-09 |
| PG42 | Neuraminidase, Micromonospora viridifaciens | D01045 | 647aa | 492aa | 32 | 375aa | 2.10E-22 |
| PG43 | Immunoreactive outer membrane protein (omp28). Brucella melitensis | U30815 | 250aa | 245aa | 24 | 178aa | 0.0015 |
| PG44 | Macrophage infectivity potentiator. Legionella lsraelensis | U92208 | 242aa | 276aa | 35 | 219aa | 9.10E-18 |
| PG43 | Outer membrane protein, Neisseria meningitidis | AF021245 | 797aa | 775aa | 21 | 699aa | 0.0034 |
| PG46 | Outer membrane protein 85, Neisseria gonorrhoeae | UB1959 | 792aa | 774aa | 31 | 117aa | 0.00098 |
| PG47 | Outer membrane protein (susC) Bacteroides thetaiotaomicron | L49338 | 1038aa | 867aa | 20 | 962aa | 1.00E-03 |
| PG48 | Immunoglobulin binding surface protein (sir22). | X75750 | 365aa | 431aa | 25 | 269aa | 5.20E-05 |
| PG49 | Streptococcus pyogenes Fimbrillin (orf2). Porphyromonas gingivalis | D42087 | 453aa | 333aa | 23 | 295aa | 0.082 |
| PG50 | Outer membrane protein (susC) Bacteroides thetaiotaomicron | L49338 | 1038aa | 848aa | 26 | 579aa | 1.80E-11 |
| PG51 | PGaA antigen. Porphyromonas gingivalis | X95938 | 202aa | 202aa | 54 | 126aa | 1.20E-25 |
| PG52 | Alkaline protease secretion apparatus (aprF) Pseudomonas aeruginosa | X64558 | 481aa | 455aa | 21 | 427aa | 3.50E-06 |
| PG53 | Protein export protein (tolC), Salmonella enteritidis | U25178 | 491aa | 444aa | 23 | 436aa | 6.20E-11 |
| PG54 | Protease I, Achromobacter lvticus | J5128 | 853aa | 940aa | 24 | 695ea | 1.50E.22 |
| PG55 | Fimbrillin (orf3). Porphyromonas gingivalis | D42067 | 870aa | 670aa | 43 | 688aa | 4.90E-106 |
| PG50 | Cysteine protease Porphyromonas gingivalis | U68468 | 364aa | 1282aa, 1274aa | 25 | 212aa | 0.00012 |
| PG57 | Cysteine protease, Porphyromonas gingivalis | U68468 | 1358aa | 924aa, 922aa, 921aa | 31 | 742aa | 1.40E-23 |
| PG00 | Outer membrane protein 11, Helicobacter pylori | AE000562 | 166aa | 547aa | 25 | 183aa | 2.20E+00 |
| PG01 | Ferric pseudobactin M114 receptor protein (pbuA). Pseudomonas sp. | X73412 | 826aa | 749aa | 22 | 585aa | 1.00E-05 |
| PG66 | Attachment and invasion protein (ail), Salmonella typhimurium | AF007380 | 155aa | 206aa | 21 | 140aa | 1.90E+00 |
| PG08 | Serum opacity factor, Streptococcus pyogenes | U02290 | 1025aa | 1225aa. 1224aa | 24 | 170aa | 2.10E-01 |
| PG69 | Vacuolating cytotoxin (vacA), Helicobacter pylori | U63261 | 160aa | 425aa | 32 | 111aa | 1.20E+oo |
| PG70 | Outer membrane protein, Neisseria gonorrhoea | U52059 | 174aa | 286aa | 22 | 153aa | 8.90E+00 |
| PG71 | Gliding motilityprotein (gldA). Flavobacterium johnsonles | AF007381 | 578aa | 834aa | 23 | 572aa | 3.90E.25 |
| PG75 | Class 3 outer membrane porin (porB), Neisseria meningitidis | U07191 | 3328a | 391aa | 23 | 239aa | 4.60E-01 |
| PG81 | Outer membrane protein (ompA),shigella dvsenteriae | V01344 | 351aa | >235aa | 28 | 186aa | 3.10E-01 |
| PG82 | Outer membrane protein (alkL). Pseudomonas oleovorans | X85936 | 230aa | 434aa | 26 | 136aa | 2.80E+00 |
| PG83 | Gliding motility protein (gldA), Flavobacterium johnsoniae | AF007381 | 578aa | 928aa | 21 | 639aa | 8.50.E-09 |
| PG87 | Hypothetical protein, Mycobacterium tuberculosis | AL021942 | 877aa | 781aa | 29 | 794aa | 2.20E.34 |
| PG89 | NADH-ubiquinone oxidoreductese, Helicobacter pylori | AE000831 | 512aa | 259aa | 24 | 186aa | 3.90E-01 |
| PG91 | Neuraminidase (nanH), Bacteroides fragilis | D28493 | 544aa | 540aa | 24 | 251aa | 1.60E+00 |
| PG02 | Hypothetical protein. Mycobacterium tuberculosis | AL021942 | 877aa | 771aa | 29 | 770aa | 8.00E-30 |
| PG03 | Cytoadherence protein P1. Mvcoplasma pneumonias | X07191 | 219aa | 776aa | 41 | 63aa | 6.90E-01 |
| PG94 | Arginvl endopeptidase. Porphyromonas gingivalis | D26470 | 991aa | 1157aa | 24 | 328aa | 7.60E-08 |
| PG95 | Sensor protein (EVGS). Escherichia colt | D14008 | 1197aa | 961aa | 28 | 511aa | 2.60E-17 |
| **PG105** | Plasma cell membrane glycoprotein. Human | P22413 | 873aa | 449aa | 34 | 404aa | 5.60E-33 |
| **PG106** | Hypothetical secreted protein, Helicobacter pylori | O24951 | 242aa | 248aa | 30 | 252as | 7.80E-22 |
| **PG107** | Cell division ATP binding protein. Mycobacterium leprae | 032883 | 229aa | 246aa, 241aa, 232aa | 46 | 193aa | 1.20E-28 |
| **PG108** | ABC transporter, Archaeoglobus fulgidis | 029244 | 228aa | 219aa | 51 | 219aa | 3.80E-41 |
| **PG109** | Proteinase IV. Escherichia coli | F64936 | 618aa | 595aa, 589aa | 38 | 597aa | 1.10E-57 |
| **PG110** | Preprotein translocase, Staphylococcus aureus | O06446 | 843aa | 523aa | 43 | 521aa | 6.00E-71 |
| **PG111** | ABC transporter, Synechocystis sp. | P73758 | 574aa | >720aa | 40 | 579aa | 1.70E-73 |
| **PG112** | Glycosyl transferase, Erwinia amylovora | Q46634 | 351aa | 375aa, 362aa | 31 | 363aa | 1.60E-32 |
| **PG113** | Heat shock protein (dnak), Treponema pallidum | AE001203 | 635aa | 640aa | 62 | 644aa | 9.10E-138 |
| **PG114** | Dihydrollpamide dehydrogenase, Clostridium magnum | Q59299 | 578aa | 449aa | 37 | 450aa | 3.80E-54 |
| **PG115** | Zinc protease, Escherichia coli | P31828 | 931aa | 941aa | 27 | 890aa | 6.60E-57 |
| **PG116** | Heat shock protein (HTPG). Escherichia coli | P10413 | 624aa | 684aa | 32 | 627aa | 4.60E-48 |
| **PG117** | Transcriptional regulator, Aquifex aeolicus | O66591 | 506aa | 484aa | 39 | 389aa | 2.40E-49 |
| **PG118** | ABC transporter, Bacillus subtilus | H70019 | 251aa | 250aa | 59 | 251aa | 1.50E-60 |
| **PG119** | ATP-dependent protease. Aquifex aeolicus | O66827 | 444aa | 461aa | 46 | 458aa | 1.60E-77 |
| **PG120** | Nitrogen assimilation regulatory protein. Bradyrhizobium sp.: | P10576 | 480aa | 457aa | 49 | 242aa | 3.80E-45 |
| FG121 | Cobalamin synthasis protein, Bacillus megaterium | B1331323 | 367aa | 602aa | 36 | 324aa | 9.20E-37 |
| PG122 | Outer membrane integrity (tolA.), Haemophilus influenzas | P71397 | 819as | 443aa | 37 | 441aa | 1.90E-54 |
| PG123 | Fimbrillin. Porphyromonas gingivalis | D1034032 | 490aa | 479aa | 32 | 480aa | 7.30E-48 |
| PG124 | Heat shock protein (dnaI), Leptospira interrogans | AF007813 | 389aa | 383aa | 48 | 356aa | 2.30E-57 |
| PG125 | Cobalainin biosynthesis protein(CBIK). Salmonella typhimurium | Q05592 | 264aa | 293aa | 37 | 259aa | 3.70E-26 |
| PG120 | ABC-type permease, Pseudomonas aeruginosa | 088878 | 326aa | 356aa | 33 | 333aa | 1.30E-30 |
| PG127 | Endonuclease excision repair protein (uvrB), Pseudomonas aeruginosa | X93486 | 870sa | 878aa | 56 | 675aa | 1.10E-134 |

**Table 3: Results of PSORT, SignalP and TopPred analysis of the proteins. The signal present column indicates the presence of a signal sequence detected with either PSORT or SignalP. The terms in parentheses indicates the type of signal sequence as determined by PSORT. The cell location & probability values are generated by PSORT and represent the probability of the protein being in the cell compartments outer membrane (OM), inner membrane (IM), periplasmic space (PC) or cytoplasm (C). The number of transmembrane domains (TMDs) was determined by TopPred and does not include uncleavable signal sequences.**

| Protein name | Protein seqID number | Protein Length | Signal Present | Methionine in ORF | SignalP cleavage site | PSORT cleavage site | Cell Location & probability | | | | C-terminal Amino Acid | Number of TMD's |
|---|---|---|---|---|---|---|---|---|---|---|---|---|
| | | | | | | | OM | 1M | PS | C | | |
| FG1 | 386 | 451aa | Y | 1 | 24 | 34 | 0 | 0 | 0 | 0.22 | N | 0 |
| PG2 | 424 | 1017aa | Y | 1 | 20 | 20 | 0.94 | 0 | 0.33 | 0 | F | 3 |
| PG2 | 425 | 1014aa | Y | 2 | 17 | 17 | 0.94 | 0 | 0.29 | 0 | F | 3 |
| PG3 | 434 | 223aa | Y(lipoprotein) | 1 | - | 18 | 0.79 | 0.78 | 0 | 0 | K | 3 |
| PG4 | 447 | 672aa | Y(lipoprotein) | 1 | 22 | 22 | 0.79 | 0.7 | 0 | 0 | R | 0 |
| PG5 | 458 | 315aa | Y | 1 | 40 | 35 | 0 | 0.25 | 0 | 0 | R | 0 |
| PG5 | 475 | 324aa | N | 1 | - | - | 0 | 0 | 0 | 0.2 | S | 1 |
| PG7 | 487 | 404aa | N | 1 | 7 | - | 0 | 0.42 | 0 | 0 | E | 3 |
| PG8 | 498 | 598aa | N | 1 | - | - | 0 | 0 | 0 | 0.22 | N | 0 |
| PG8 | 499 | 550aa | N | 2 | - | - | 0 | 0 | 0 | 0.25 | N | 0 |
| PG8 | 500 | 458aa | N | 3 | - | - | 0 | 0 | 0 | 0.34 | N | 0 |
| PG8 | 501 | 426aa | N | 4 | - | - | 0 | 0 | 0 | 0.24 | N | 0 |
| PGD | sis | 1266aa | N | 1 | 7 | - | 0 | 0 | 0 | 0.22 | E | 1 |
| PG9 | 516 | 1232aa | N | 2 | - | - | 0 | 0 | 0 | 0.39 | E | 1 |
| PG9 | 517 | 1174aa | N | 3 | - | - | 0 | 0 | 0 0 | 0.47 | E | 1 |
| PG10 | 387 | 195aa | N | 1 | - | - | 0 | 0 | 0 | 0.11 | 1< | 0 |
| PG11 | 400 | 313aa | Y | 1 | 22 | 26 | 024 | 0 0 | 0.93 | 0 | R | 1 |
| PG12 | 411 | 271aa | Y (lipoprotein) | 3 | 27 | 29 | 0.79 | 0.7 | 0 | 0 | R | 0 |
| PG13 | 419 | 757aa | Y | 1 | 23 | 25 | 0.94 | 0 | 0.29 | 0 | N | 0 |
| PG14 | 420 | 331aa | Y (uncleavable) | 1 | 35 | 26 | 0 | 0.58 | 0 | 0 | K | 1 |
| PG15 | 421 | 267aa | Y | 2 | 24 | 18 | 0 | 0.11 | 0 | 0 | K | 1 |
| PG16 | 422 | 589aa | Y (lipoprotein) | 1 | 24 | is | 0.79 | 0.7 | 0 | 0 | G | 0 |
| PG18 | 423 | 981aa | Y | 1 | 30 | | 0 | 0.56 | 0 | 0 | K | 11 |
| PG21 | 426 | 821aa | Y | 2 | 24 | 27 | 0.34 | 0 | 0.37 | 0 | G | 1 |
| PG22 | 427 | 106aa | Y (uncleavable) | 1 | 41 | 41 | 0 | 0.20 | 0 | 0 | P | 0 |
| PG23 | 428 | 859aa | N | 1 | - | | 0 | 0.12 | 0 | 0 | A | 1 |
| PG24 | 428 | 417aa | Y | 1 | 19 | 19 | 0 | 0.44 | 0 | 0 | N | 3 |
| PG25 | 430 | 293aa | Y | 1 | 27 | 28 | 0.2 | 0 | 0.82 | 0 | R | 0 |
| PG27 | 431 | 312aa | N | 1 | - | - | 0 | 0 0 | 0 | 0.28 | Q | 1 |
| PG28 | 432 | 843aa | Y | 1 | 21 | 21 | 0.93 | 0 | 0.24 | 0 | H | 1 |
| PG29 | 433 | 290aa | Y | 1 | 18 | 16 | 0.28 | 0 | 0.94 | 0 | K | 1 |
| PG30 | 435 | 337aa | Y | 1 | 21 | 21 | 0.24 | 0 | 0.4 | 0 | K | 0 |
| PG31 | 436 | 151aa | N | 1 | - | - | 0 | 0 | 0 | 0.3 | T | 0 |
| PG32 | 437 | 391aa | Y | 1 | 20 | 20 | 0.62 | 0 | 0.13 | 0 | K | 0 |
| PG33 | 438 | 385aa | Y | 1 | 26 | 26 | 0.81 | 0 | 0.31 | 0 | E | 1 |
| PG34 | 439 | 190aa | Y | 1 | - | 13 | 0 | 0.5 | 0 | 0 | A | 5 |
| PG34 | 440 | 186aa | Y (uncleavable) | 2 | - | 47 | 0 | 0.5 | 0 | 0 | A | 4 |
| PG35 | 441 | 833aa | Y | 1 | 22 | 22 | 0.94 | 0 | 0.37 | 0 | F | 1 |
| PG36 | 442 | 891aa | Y (uncleavable) | 1 | - | 40 | 0 | 0.31 | 0 | 0 | F | 2 |
| PG37 | 443 | 174aa | Y (uncleavable) | 1 | 28 | 24 | 0 | 0.35 | 0 | 0 | K | 0 |
| PG37 | 444 | 170aa | Y(uncleavable) | 2 | 24 | 20 | 0 | 0.35 | 0 | 0 | K | 0 |
| PG38 | 445 | 183aa | Y | 1 | 18 | 18 | 0.21 | 0 | 0.93 | 0 | K | 1 |
| PG39 | 446 | 827aa | Y | 1 | 36 | 36 | 0.93 | 0 | 0.25 | 0 | F | 3 |
| PG40 | 448 | 772aa | Y | 2 | 19 | 19 . | 0.94 | 0 | 0.32 | 0 | F | 4 |
| PG41 | 449 | 462aa | Y | 2 | 27 | 27 | 0.25 | 0 | 0.54 | 0 | Q | 2 |
| PG42 | 450 | 492aa | Y | 5 | 30 | - | 0 | 0 | 0.00 | 0.13 | Q | 2 |
| PG43 | 451 | 245aa | Y (uncleavable) | 2 | 28 | 22 | 0 | 0.38 | 0 | 0 | K | 1 |
| PG44 | 452 | 276aa | Y | 1 | 19 | 24 | 0.15 | 0 | 0.89 | 0 | K | 0 |
| PC45 | 453 | 775ee | Y (lipoprotein) | 1 | 19 | 23 | 0.79 | 0.7 | 0 | 0 | F | 4 |
| PG46 | 454 | 774aa | Y | 1 | 27 | 27 | 0.73 | 0 | 0.22 | 0 | F | 2 |
| PG47 | 455 | 887aa | Y | 1 | 24 | 24 | 0.94 | 0 | 0.38 | 0 | F | 2 |
| PG48 | 456 | 431aa | Y | 1 | 24 | 24 | 0 | 0.1 | 0 | 0 | R | 1 |
| PG49 | 457 | 333aa | Y (uncleavable) | 1 | 24 | 18 | 0 | 0.12 | 0 | 0 | 1 | 0 |
| PG50 | 459 | 848aa | Y | 1 | 21 | 21 | 0.94 | 0 | 0.34 | 0 | F | 3 |
| PG51 | 460 | 202aa | Y | 1 | 26 | 25 | 0.2 | 0 | 0.61 | 0 | S | 0 |
| PG52 | 461 | 455aa | Y (uncleavable) | 1 | 23 | 21 | 0 | 0.18 | 0 | 0 | F | 1 |
| PG53 | 462 | 444aa | Y | 1 | 14 | 17 | 0.36 | 0 | 0.22 | 0 | D | 2 |
| PG54 | 463 | 940aa | Y | 1 | 27 | 20 | 0.86 | 0 | 0.25 | 0 | Q | 5 |
| PG55 | 464 | 670aa | Y (lipoprotein) | 1 | 23 | 23 | 0.79 | 0.7 | 0 | 0 | K | 2 |
| PG56 | 465 | 1282aa | Y (uncleavable) | 1 | | 21 | 0 | 0.04 | 0 | 0 | K | 4 |
| PG56 | 466 | 1274aa | 14 | 2 | - | - | 0 | 0 | 0 | 0.27 | K | 5 |
| PG57 | 467 | 925aa | Y | 1 | 28 | 24 | 0.53 | 0 | 0.2 | 0 | P | 3 |
| PG57 | 468 | 922aa | Y | 2 | 25 | 21 | 0.53 | 0 | 0.2 | 0 | P | 3 |
| PG57 | 469 | 921aa | Y | 3 | 24 | 20 | 0.53 | 0 | 0.2 | 0 | P | 3 |
| PG58 | 470 | 593aa | Y | 1 | 24 | 24 | 0.52 | 0 | 0.19 | 0 | F | 1 |
| PG58 | 471 | 589aa | Y | 2 | 20 | 20 | 0.82 | 0 | 0.19 | 0 | F | 1 |
| PG59 | 472 | 346aa | Y | 1 | 37 | | 0 | 0.18 | 0 | 0 | F | 1 |
| PG59 | 473 | 345aa | Y | 2 | 36 | 56 | 0.92 | 0 | 0.15 | 0 | F | 1 |
| PG59 | 474 | 330aa | Y | 3 | 21 | 41 | 0.93 | 0 0 | 0.25 | 0 | F | 1 |
| PG60 | 476 | 547aa | Y | 1 | 28 | 28 | 0.93 | 0 | 0.25 | 0 | F | 0 |
| PG61 | 477 | 749aa | Y | 2 | 21 | 21 | 0.94 | 0 | 0.29 | 0 | F | 3 |
| PG62 | 478 | 494aa | Y | 1 | 21 | 21 | 0.93 | 0 | 0.24 | 0 | F | 2 |
| PG63 | 479 | 294aa | Y | 1 | 20 | 20 | 0.93 | 0 | 0.24 | 0 | F | 1 |
| PC64 | 480 | 204aa | Y | 1 | 20 | 20 | 0.93 | 0 | 0.19 | 0 | F | 1 |
| PGBS | 481 | 243aa | Y | 1 | 18 | 18 | 0.93 | 0 | 0.25 | 0 | F | 1 |
| PG66 | 482 | 206aa | Y | 1 | 21 | 21 | 0.94 | 0 | 0.3 | 0 | F | 1 |
| PG67 | 483 | 950aa | Y | 1 | 28 | 38 | 0.93 | 0 | 0.27 | 0 | Y | 4 |
| PG68 | 484 | 1226aa | Y | 1 | 25 | 25 | 0.91 | 0 | 0.31 | 0 | Y | 0 |
| PC68 | 485 | 1225aa | Y | 2 | 24 | 24 | 0.91 | 0 | 0.31 | 0 | Y | 0 |
| PG69 | 488 | 425aa | Y | 1 | 29 | 29 | 0.93 | 0 | 0.21 | 0 | F | 1 |
| PG70 | 488 | 260aa | Y | 1 | 18 | 24 | 0.93 | 0 | 0.24 | 0 | F | 0 |
| PG71 | 489 | 834aa | Y | 2 | 20 | 20 | 0.94 | 0 | 0.31 | 0 | N | 2 |
| PG72 | 490 | 399aa | Y | 1 | 27 | 27 | 0.94 | 0 | 0.32 | 0 | H | 2 |
| PG73 | 491 | 382aa | Y | 2 | 20 | 20 | 0.94 | 0 | 0.3 | 0 | L | 1 |
| PG74 | 492 | 222aa | Y | 1 | 24 | 24 | 0.94 | 0 | 0.32 | 0 | L | 0 |
| PG75 | 493 | 391aa | Y | 1 | 26 | 26 | 0.94 | 0 | 0.3 | 0 | H | 1 |
| PG76 | 494 | 446aa | Y | 1 | 21 | 22 | 0.94 | 0 | 0.32 | 0 | V | 3 |
| PG77 | 495 | 308aa | Y | 2 | 28 | 28 | 0.94 | 0 | 0.38 | 0 | K | 0 |
| PG78 | 496 | 314aa | Y | 1 | 23 | 23 | 0.94 | 0 | 0.29 | 0 | D | 0 |
| PG79 | 497 | 285aa | Y | 1 | - | 32 | 0.93 | 0 | 0.26 | 0 | G | 2 |
| PG80 | 502 | 240aa | Y | 1 | 19 | 19 | 0.93 | 0 | 0.22 | 0 | N | 2 |
| PG81 | 366 | >235aa | Y | 1 | 28 | 20 | 0.93 | 0 | 0.21 | 0 | Q | 1 |
| PG82 | 503 | 434aa | Y | 1 | 30 | 24 | 0.93 | 0 | 0.2 | 0 | N | 3 |
| PG83 | 504 | 926aa | Y | 1 | 23 | 57 | 0.93 | 0 | 0.21 | 0 | S | 1 |
| PG84 | 505 | 400aa | Y | 1 | 25 | 25 | 0.93 | 0 | 0.25 | 0 | N | 1 |
| PG84 | 506 | 398aa | Y | 2 | 23 | 23 | 0.93 | 0 | 0.25 | 0 | N | 1 |
| PG85 | 507 | 581aa | Y | 1 | 20 | 20 | 0.93 | 0 | 0.46 | 0 | L | 2 |
| PG86 | 508 | 239aa | Y | 1 | 44 | - | 0 | 0 | 0 | 0.12 | H | 0 |
| PG86 | 509 | 211aa | Y | 2 | 16 | 46 | 0.91 | 0 | 0.03 | 0 | H | 0 |
| PG87 | 510 | 781aa | Y | 1 | 26 | 47 | 0.89 | 0 | 0.21 | 0 | N | 2 |
| PG88 | 511 | 271aa | Y | 2 | 28 | 19 | 0.89 | 0 | 0.25 | 0 | P | 0 |
| PG88 | 512 | 270aa | Y | 3 | 27 | 18 | 0.89 | 0 | 0.25 | 0 | P | 0 |
| PG88 | 513 | 267aa | Y | 4 | 24 | 15 | 0.89 | 0 | 0.23 | 0 | P | 0 |
| PG89 | 514 | 259aa | Y | 2 | 23 | 25 | 0.88 | 0 | 0.35 | 0 | N | 1 |
| PG90 | 518 | 229aa | Y | 1 | 22 | 21 | 0.85 | 0 | 0.44 | 0 | K | 0 |
| PG90 | 519 | 228aa | Y | 2 | 21 | 20 | 0.85 | 0 | 0.44 | 0 | K | 0 |
| PG91 | 520 | 540aa | Y | ,1 | 25 | 25 | 0.85 | 0 | 0.30 | 0 | E | 0 |
| PG92 | 521 | 771aa | Y | 2 | 19 | 19 | 0.85 | 0 | 0.3 | 0 | R | 3 |
| PG93 | 522 | 776aa | Y | 1 | 25 | 2S | 0.85 | 0 | 0.37 | 0 | R | 4 |
| PG94 | 523 | 1157aa | Y | 1 | 23 | 29 | 0.8 | 0 | 0.25 | 0 | Q | 5 |
| PG95 | 524 | 981aa | Y (lipoprotein) | 1 | | 19 | 0.79 | 0.87 | 0 | 0 | V | 1 |
| PG96 | 525 | 563aa | Y | 1 | 23 | 23 | 0.40 | 0 | 0.33 | 0 | K | 0 |
| PG97 | 526 | 437aa | Y | 1 | 23 | 23 | 0.32 | 0 | 0.85 | 0 | Q | 0 |
| PG98 | 527 | 318aa | Y (lipoprotein) | 1 | 19 | 19 | 0.79 | 0.7 | 0 | 0 | L | 1 |
| PG99 | 528 | 401aa | Y (uncleavable) | 1 | 22 | 20 | 0 | 0 | 0.3 | 0 | R | 0 |
| PG100 | 388 | 279aa | Y | 1 | 20 | 18 | 0.26 | 0 | 0.54 | 0 | 1 | 0 |
| PG101 | 268 | >157aa | N (ORF incomplete) | | - | - | | | | | R | 1 |
| PG102 | 389 | 562aa | Y | 1 | 29 | 29 | 0.19 | 0 | 0.4 | 0 | S | 3 |
| PG102 | 390 | 558aa | Y | 2 | 25 | 25 | 0.26 | 0 | 0.46 | 0 | S | 3 |
| PG104 | 391 | 391aa | Y | 1 | 17 | 17 | 0.62 | 0 | 0.22 | | R | 0 |
| PG105 | 392 | 449aa | Y | 1 | 22 | 19 | 0.31 | 0 | 0.91 | 0 | P | 3 |
| PG106 | 393 | 246aa | Y | 2 | 41 | 49 | 0 | 0 | 0 | 0.02 | L | 0 |
| PG107 | 394 | 246aa | N | 1 | | - | 0 | 0 | 0 | 0.32 | D | 1 |
| PG107 | 395 | 241aa | N | 2 | | | 0 | 0 | 0 | 0.3 | D | 1 |
| PG107 | 396 | 232aa | N | 3 | | - | 0 | 0 | 0 | 0.21 | D | 1 |
| PC108 | 397 | 219aa | N | 1 | - | - | 0 | 0 | 0 | 0.19 | R | 1 |
| PG109 | 398 | 595aa | Y | 1 | 35 | 37 | 0.26 | 0 | 0.93 | 0 | Y | 3 |
| PG109 | 399 | 589aa | Y | 2 | 29 | 31 | 0.27 | 0 | 0.93 | 0 | Y | 3 |
| PG110 | 401 | >523aa | N | 1 | - | - | 0 | 0 | 0 | 0.38 | incomplete | 0 |
| PG111 | 278 | >720aa | N (ORF incomplete) | - | | - | | | | | G | 1 |
| **PG112** | 402 | 375aa | Y | 1 | - | 43 | 0 | 0.12 | 0 | 0 | N | 1 |
| **PG112** | 403 | 382aa | Y | 2 | - | 30 | 0 | 0 | 0.12 | 0 | N | 1 |
| **PG113** | 404 | 640aa | N | 1 | | - | 0 | 0 | 0 | 0.25 | K | 1 |
| **PG114** | 405 | 499aa | N | 1 | - | - | 0 | 0.12 | 0 | 0 | G | 4 |
| **PC115** | 400 | 941aa | Y | 1 | 23 | 22 | 0.13 | 0 | 0.92 | 0 | Q | 2 |
| **PG116** | 407 | 684aa | N | 1 | - | - | 0 | 0.12 | 0 | 0 | L | 2 |
| PG117 | 408 | 464aa | N | 1 | - | | 0 | 0.19 | 0 | 0 | L | 1 |
| PG118 | 409 | 250aa | N | 1 | - | - | 0 | 0 | 0 | 0.27 | E | 1 |
| PG119 | 410 | 461aa | N | 1 | - | - | 0 | 0.28 | 0 | 0 | E | 2 |
| PG120 | 412 | 457aa | N | 1 | - | - | 0 | 0 | 0 | 0.21 | E | 0 |
| PG121 | 413 | 802aa | N | 1 | - | | 0 | 0 | 0 | 0.31 | E | 3 |
| PG122 | 414 | 443aa | N | 1 | - | - | 0 | 0 | 0 | 0.14 | Q | 4 |
| PG123 | 415 | 479aa | Y | 2 | 22 | 22 | 0.26 | 0 | 0.94 | 0 | K | 0 |
| PG124 | 416 | 383aa | N | 1 | - | - | 0 | 0 | 0 | 0.29 | D | 2 |
| PG125 | 417 | 293aa | Y | 1 | 23 | is | 0.18 | 0 | 0.93 | 0 | R | 1 |
| PC126 | 418 | 356aa | N | 1 | | - | 0 | 0.52 | 0 | 0 | D | 9 |
| PG127 | 532 | 678aa | N | 1 | - | - | 0 | 0 | 0 | 0.28 | A | 2 |

**Table 4: Percentage identity and percentage similarity of various proteins with the 70 amino acids from the C-terminal of the P. gingivalis arginine protease 1 (RGP1), arginine protease 2 (RGP2), and the cysteine protease/hemagglutinin (prtT).**

| **Protein name** | | **Percent identity** | | | | **Percent similarity** | | |
|---|---|---|---|---|---|---|---|---|
| | | **RGP1** | **RGP2** | **prtT** | | **RGP1** | **RGP2** | **prtT** |
| **PG21** | | 17 | 29 | 21 | | 40 | 57 | 49 |
| **PG25** | | 43 | 41 | 9 | | 64 | 73 | 14 |
| **PG27** | | 41 | 33 | 7 | | 73 | 74 | 11 |
| **PG28** | | 21 | 26 | 34 | | 49 | 57 | 74 |
| **PG54** | | 19 | 13 | 16 | | 40 | 43 | 33 |
| **PG57** | | 11 | 14 | 19 | | 20 | 24 | 34 |
| **PG91** | | 31 | 21 | 39 | | 57 | 53 | 74 |
| **PG96** | | 0 | 13 | 20 | | 0 | 24 | 43 |
| **PG97** | | 10 | 26 | 33 | | 14 | 47 | 61 |
| **PG98** | | 16 | 20 | 0 | | 47 | 54 | 0 |
| **PG99** | | 19 | 0 | 26 | | 41 | 0 | 54 |
| **PG100** | | 20 | 21 | 24 | | 39 | 57 | 41 |
| **PG101** | | 11 | 16 | 27 | | 17 | 39 | 60 |
| **PG102** | | 27 | 20 | 31 | | 50 | 61 | 61 |
| PG104 | | 16 | 23 | 26 | | 46 | 44 | 49 |

**Table 5: Percentage identity and percentage similarity of various proteins with the TonBIII box of P. gingivalis.**

| **Protein name** | **Percent identity** | **Percent similarity** |
|---|---|---|
| **PG2** | 46 | 71 |
| **PG13** | 57 | 93 |
| **PG35** | 50 | 96 |
| **PG47** | 39 | 71 |
| **PG50** | 54 | 98 |

**Cloning, expression and purification of recombinant *P*. *gingivalis* genes.**

### PG1

Oligonucleotides to the 5' and 3' regions of the deduced protein were used to amplify the gene of interest from a preparation of P. *gingivalis* W50 genomic DNA using the TaqPlus Precision PCR System (Stratagene) and a PTC-100 (MJ Research) thermal cycler or similar device. The 5' oligonucleotide primer sequence was GCGCCATATGCTGGCCGAACCGGCC, the 3' oligonucleotide primer sequence was GCGCCTCGAGTCAATTCATTTCCTTATAGAG. The PCR fragment was purified, digested with Nde I, Xho I restriction enzymes (Promega) and ligated into the corresponding sites of the plasmid pProEx-1 (Gibco-BRL) and transformed into E. *coli* ER1793 cells (a gift from Elizabeth Raleigh, New England Biolabs). A resulting clone expressing the correct insert was selected and induced with or without 0.1mM IPTG (Promega) for expression of the recombinant protein. Expression of the recombinant protein was determined by SDS-PAGE analysis and Western Blot using the one of the rabbit antisera described above or an anti-hexahistidine antibody (Clontech) that detects the hexahistidine tag that was fused to *the* P. *gingivalis* recombinant protein. PG1 was purified by disruption of the E. *coli* cells by sonication in binding buffer (Novagen) and solubilisation by the addition of sarkosyl (N-Lauroyl sarcosine) to a 1% final concentration. There after the preparation was diluted to 0.1% sarkosyl in binding buffer, bound to a Nickel-nitrilotriacetic acid column (Ni-NTA; Qiagen), after washing bound proteins were eluted with 1M imidazole in elution buffer (Novagen) according to the Qiagen recommendations with 0.1% sarkosyl added to all buffers. Following purification samples were dialysed against 500mM NaCl, 20mM Tris, 0.1% sarkosyl at pH7.4 to remove the imidazole, concentrated as required and stored at 4°C until used. Purity and antigenicity were assessed by SDS-PAGE and Western blot using selected antisera (from those described above) and the protein concentration was determined by the BCA assay (Pierce).

### PG2

The methods used for PG2 were essentially the same as for PG1 with the following exceptions. The 5' oligonucleotide primer sequence was CGCGGTATACATGAAAAGAATGACGC, the 3' oligonucleotide primer sequence was CGCGAGATCTGAAAGACAACTGAATACC and the PCR product was cloned into pGex-stop RBS(IV) (Patent application WO9619496, JC Cox, SE Edwards, I Frazer and EA Webb. Variants of human papilloma virus antigens) using the BstZ 171 and Bgl II restriction sites. 2% sarkosyl was used to solubilise PG2 and 8M urea was added to the solublisation buffer and to all other buffers. Urea was removed from the purified protein by sequential dialysis (4M then 2M then 1M then 0.5M then 0M urea all in 50mM Tris, 500mM NaCl, 0.1% sarkosyl, pH7.4). Purified protein was stored at 4°C until required.

### PG3

The methods used for PG3 were essentially the same as for PG1 with the following exceptions. The 5' oligonucleotide primer sequence was GCGCGTATACATGAAGAAATCAAGTGTAG, the 3' oligonucleotide primer sequence was GCGCAGATCTCTTCAGCGTACCTTGCTGTG and DNA was amplified with Pfu DNA polymerase (Stratagene). The PCR product was cloned directly into pCR-Blunt and transformed into E. *coli* Top10F'(InVitrogen) before subcloning into the expression plasmid pGex-stop RBS(IV) using the Bst Z171 and Bgl II restriction sites and transformed into E. *coli* BL21DE3 (Pharmacia Biotech). The following modifications were made to the purification of PG3 from the PG1 method. Cells expressing the recombinant protein were disrupted by sonication in binding buffer and the insoluble inclusion bodies concentrated by centrifugation. Inclusion bodies were then solubilised in 6M urea (Sigma) in binding buffer and eluted with 6M urea added to the elution buffer. In some instances 6M guanidine hydrochloride (Sigma) was used instead of urea for these steps. Urea (or guanidine hydrochloride when it was substituted) was removed from the purified protein by sequential dialysis against reducing levels of urea (3M then 1.5M then 0.5M then 0M urea all in 50mM Tris, 500mM NaCl, 8% glycerol, pH7.4). Purified protein was stored frozen at -80°C until required. Protein concentration was determined by the Coomassie Plus protein assay (Pierce).

### PG4

The methods used for PG4 were essentially the same as for PG3 with the following exceptions. The 5' oligonucleotide primer sequence was CTTCTGTATACTTACAGCGGACATCATAAAATC, the 3' oligonucleotide primer sequence was TTCCAGGAGGGTACCACGCAACTCTTCTTCGAT and DNA was amplified with the Tth XL PCR kit (Perkin Elmer). The PCR product was cloned into the expression plasmid pGex-stop RBS(IV) using the Bst Z171 and Kpn I restriction sites and transformed into *E*. *coli* ER1793.

### PG5

The methods used for PG5 were essentially the same as for PG3 with the following exceptions. The 5' oligonucleotide primer sequence was TTGCAACATATGATCAGAACGATACTITCA, the 3' oligonucleotide primer sequence was AGCAATCTCGAGCGGTTCATGAGCCAAAGC and DNA was amplified with the Tth XL PCR kit. The PCR product was cloned into, the expression plasmid pET24 (Novagen) using the Nde I and Xho I restriction sites and transformed into *E. coli* BL21 (Pharmacia Biotech). Removal of urea was not proceeded past 1M urea as the protein was insoluble at lower concentrations of urea. Purified protein was stored at 4°C until required.

### PG6

The methods used for PG6 were essentially the same as for PG3 with the following exceptions. The 5' oligonucleotide primer sequence was TAAACATATGTGCCTCGAACCCATAATTGCTCCG, the 3' oligonucleotide primer sequence was CGTCCGCGGAAGCTTTGATCGGCC-ATTGCTACT and DNA was amplified with the Tth XL PCR kit. The PCR product was cloned into the expression plasmid pET24a using the Nde I and Hind III restriction sites and transformed into E. *coli* BL21.

### PG8

The methods used for PG8 were essentially the same as for PG3 with the following exceptions. The 5' oligonucleotide primer sequence was CGCGGTATACATGGAGTTCAAGATTGTG, the 3' oligonucleotide primer sequence was CGCGAGATCTGTITI'CTGAAAGCTTTTC and DNA was amplified with the TaqPlus Precision PCR System. The PCR product was cloned into the expression plasmid pProEx-1 using the Nde I and Xho I restriction sites and transformed into E. *coli* ER1793.

### PG8A

PG8A is a shortened version of PG8 and has the first 173 amino acids removed. The methods used for PG8A were essentially the same as for PG3 with the following exceptions. The 5' oligonucleotide primer sequence was CGCGGTATACATGGAAAACTTAAAGAAC, the 3' oligonucleotide primer sequence was CGCGAGATCTGTTTTCTGAAAGCTTTTC and DNA was amplified with the TaqPlus Precision PCR System. The PCR product was cloned into the expression plasmid pGex-stop RBS(IV) using the Bst Z171 and Bgl II restriction sites and transformed into E. *coli* ER1793. Prior to dialysis of the purified protein EDTA (Sigma) was added to a final concentration of 10mM.

### PG10

The methods used for PG10 were essentially the same as for PG3 with the following exceptions. The 5' oligonucleotide primer sequence was CGCGGATATCATGGATAAAGTGAGGTATGC, the 3' oligonucleotide primer sequence was CGCGAGATCTTTTGTTGATACTCAATAATTC and DNA was amplified with the TaqPlus Precision PCR System. The PCR product was digested with Eco RV and Bgl II and ligated into the expression plasmid pGex-stop RBS(IV) using the Bst Z171 and Bgl II restriction sites and transformed into E. *coli* ER1793.

### FG11

The methods used for PG11 were essentially the same as for PG1 with the following exceptions. The 5' oligonucleotide primer sequence was GCGCGTATACATGAGAGCAAACATTTGGCAGATACTTTCCG, the 3' oligonucleotide primer sequence was GCGCAGATCTGCGCAAGCGCAGTATATCGCC and DNA was amplified with Tli DNA polymerase (Promega). The PCR product was cloned into *pCR-Blunt* and transformed into *E. coli* Top10F"before subcloning into the expression plasmid pGex-stop RBS(IV) using the Bst Z171 and Bgl II restriction sites and transformed into E. *coli* ER1793. PG11 was purified by solubilisation of E. *coli* cells with 2% sarkosyl in binding buffer (Qiagen) which was diluted to 0.1% sarkosyl in binding buffer, bound to a Nickel-nitrilotriacetic acid column (Ni-NTA; Qiagen), after washing bound proteins were eluted with 1M imidazole (0.7% CHAPS (Sigma) in elution buffer; Qiagen) according to the Qiagen recommendations. Following purification samples were dialysed against 500mM NaCl, 20mM Tris, 0.7% CHAPS, 20% glycerol (Sigma) at pH7.4 to remove the imidazole, concentrated as required and stored at 4°C until used.

### PG12

The methods used for PG12 were essentially the same as for PG1 with the following exceptions. The 5' oligonucleotide primer sequence was GCGCGTATACATGAATAGCAGACATCTGACAATCACAATCATTGCCGG, the 3' oligonucleotide primer sequence was GCGCAGATCTGCTGTTCTGTGAGTGCAGTTGTTTAAGTG and DNA was amplified with Tli DNA polymerase. The PCR product was cloned into pCR-Blunt and transformed into E. *coli* Top10F'cells before subcloning into the expression plasmid pGex-stop RBS(IV) using the Bst Z171 and Bgl II restriction sites and transformed into *E. coli* BL21. Purification of the recombinant protein was essentially the same as PG11 except 0.5% DHPC (1,2-Diheptanoyl-*sn*-glycero-3-phosphocholine; Avanti) in 50mM Tris, 50mM NaCl, pH8.0 was used to solubilise the inclusion bodies instead of sarkosyl and the DHPC was diluted to 0.1% before addition to the Ni-NTA and 0.1% DHPC was added to all buffers.

### PG13

The methods used for PG13 were essentially the same as for PG3 with the following exceptions. The 5' oligonucleotide primer sequence was GCGCCATATGCGGACAAAAACTATCITTTTTGCG, the 3' oligonucleotide primer sequence was GCGCCTCGAGGTTGTTGAATCGAATCGCTATTTGAGC and DNA was amplified with Tli DNA polymerase. The PCR product was cloned the expression plasmid pET24b using the Nde I and Xho I restriction sites and transformed into *E. coli* BL21. Purification of the recombinant protein was essentially the same as PG3 using 6M urea and 1% NOG (n-octyl glucoside; Sigma) was added to the dialysis buffer. Removal of urea was not proceeded past 2M urea as the protein was insoluble at lower concentrations of urea. Purified protein was stored at 4°C until required.

### PG14

The methods used for PG12 were essentially the same as for PG1 with the following exceptions. The 5' oligonucleotide primer sequence was GCGCGGCGCCATGACGGACAACAAACAACGTAATATCG, the 3' oligonucleotide primer sequence was GCGCCTCGAGTTACTTGCGTATGATCACGGACATACCC and DNA was amplified with Tli DNA polymerase. The PCR product was cloned the expression plasmid pProEx-1 using the Ehe I and Xho I restriction sites and transformed into *E. coli* BL21. ' Purification of the recombinant protein was essentially the same as PG12.

### PG15

The methods used for PG15 were essentially the same as for PG3 with the following exceptions. The 5' oligonucleotide primer sequence was CAAAAGTATACTAATAAATATCATTCTCAA, the 3' oligonucleotide primer sequence was GCTTATGGTACCTTTGGTCTTATCTATTAT and DNA was amplified with the Tth XL PCR kit The PCR product was cloned into the expression plasmid pGex-stop RBS(IV) using the Bst Z171 and Kpn I restriction sites and transformed into E. *coli* ER1793.

### PG22

The methods used for PG22 were essentially the same as for PG1 with the following exceptions. The 5' oligonucleotide primer sequence was CCCCGGATCCGATGCGACTGATCAAGGC, the 3' oligonucleotide primer sequence was CCCCCTCGAGCGGAACGGGGTCATAGCC and DNA was amplified with the TaqPlus Precision PCR System. The PCR product was cloned into the expression plasmid pET24b using the Bam HI and Xho I restriction sites and transformed into E. *coli* BL21DE3. Once PG22 was purified dialysis was performed in the same manner as for PG1 but in the presence of 1M imidazole.

### PG24

The methods used for PG24 were essentially the same as for PG3 with the following exceptions. The 5' oligonucleotide primer sequence was CGCGGTATACATGAATTACCTGTACATAC, the 3' oligonucleotide primer sequence was CGCGGGATCCGTTCGATTGGTCGTCGATGG and DNA was amplified with the TaqPlus Precision PCR System. The PCR product was digested with Bst Z171 and Bam HI and ligated into the expression plasmid pGex-stop RBS(IV) using the Bst Z171 and Bgl II restriction sites and transformed into *E. coli* ER1793. Due to the low level of expression of PG24 purification was not proceeded with except on small scale.

### PG24A

A modified version of PG24 was also cloned and expressed. PG24A is the same as PG24 with the predicted N-terminal sequence removed. The methods used for PG24A were essentially the same as for PG3 with the following exceptions. The 5' oligonucleotide primer sequence was CGCGCATATGGAGATTGCTTTCCTTTCTTCG, the 3' oligonucleotide primer sequence was CGCGCTCGAGTTAGITCGATTGGTCGTCG and DNA was amplified with the TaqPlus Precision PCR System. The PCR product was cloned into the expression plasmid pProEx-1 using the Nde I and Xho I restriction sites and transformed into E. *coli* ER1793. Purification of the recombinant protein was essentially the same as PG3 except 8M urea was used to solubilise the inclusion bodies and in the buffers used for the Ni-NTA column purification. Urea was removed by sequential dialysis (4M then 2M, then 1M then 0.5M then 0M urea all in 50mM Tris, 500mM NaCl, 8% glycerol, pH7.4). Purified protein was stored frozen at -80°C until required.

### PG29

The methods used for PG29 were essentially the same as for PG3 with the following exceptions. The 5' oligonucleotide primer sequence was GCGCGATATCGCTAGCATGAAAAAGCTATTTCTC, the 3' oligonucleotide primer sequence was GCGCAGATCTCTCGAGTTTGCCATCGGA7TGCGGATTG and DNA was amplified with Pfu DNA polymerase being used. The PCR product was cloned into pCR-Blunt (InVitrogen) and transformed into *E. coli* Top10F'before subcloning into the expression plasmid pGex-stop RBS(IV) using the EcoR V and Bgl II restriction sites and transformed into *E. coli* BL21. 6M urea was used throughout the purification process.

### PG30

The methods used for PG30 were essentially the same as for PG3 with the following exceptions. The predicted N-terminal signal sequence was removed from the recombinant protein. The 5' oligonucleotide primer sequence was TACGGAATTCGTGACCCCCGTCAGAAATGTGCGC, the 3' oligonucleotide primer sequence was CTATGCGGCCGCTrTGATCCTCAAGGCTTTGCCCGG and DNA was amplified with the Tth XL PCR kit. The PCR product was cloned into the expression plasmid pET24a using the Eco RI and Not I restriction sites and transformed into E. *coli* BL21DE3. Expression studies and immunoreactivity studies were carried out on whole E. *coli* lysates of PG30. 10ml cultures of recombinant *E. coli* were grown to an OD of 2.0 (A₆₀₀ₙₘ) in terrific broth and the cells were induced with 0.5mM IPTG and samples taken for analysis at 4 hours post induction. Purification was not done for these studies.

### PG31

The methods used for PG31 were essentially the same as for PG30 with the following exceptions. The 5' oligonucleotide primer sequence was CGGGGAATTCGCAAAAATCAATTTCTATGCTGAA, the 3' oligonucleotide primer sequence was CTATGCGGCCGCTGTATGCAATAGGGAAAGCTCCGA and DNA was amplified with the Tth XL PCR kit. The PCR product was cloned into the expression plasmid pET24a using the Eco RI and Not I restriction sites and transformed into *E.coli* BL21DE3. Expression studies and immunoreactivity studies were carried out on whole E. *coli* lysates. Purification was not done for these studies.

### PG32

The methods used for PG32 were essentially the same as for PG30 with the following exceptions. The predicted N-terminal signal sequence was removed from the recombinant protein. The 5' oligonucleotide primer sequence was CGCAGAATTCCAGGAGAATACTGTACCGGCAACG, the 3' oligonucleotide primer sequence was CTATGCGGCCGCCTTGGAGCGAACGATTACAACAC and DNA was amplified with the Tth XL PCR kit. The PCR product was cloned into the expression plasmid pET24a using the Eco RI and Not I restriction sites and transformed into *E. coli* BL21DE3. Expression studies and immunoreactivity studies were carried out on whole E. *coli* lysates. Purification was not done for these studies.

### PG33

The methods used for PG33 were essentially the same as for PG30 with the following exceptions. The predicted N-terminal signal sequence was removed from the recombinant protein.
The 5' oligonucleotide primer sequence was TGCAGAATTCCAAGAAGCTACTACACAGAACAAA, the 3' oligonucleotide primer sequence was CTATGCGGCCGCTTCCGCTGCAGTCATTACTACAA and DNA was amplified with the Tth XL PCR kit. The PCR product was cloned into the expression plasmid pET24a using the Eco RI and Not I restriction sites and transformed into E. *coli* BL21DE3. Expression studies and immunoreactivity studies were carried out on whole *E. coli* lysates*.* Purification was not done for these studies.

### PG35

The methods used for PG35 were essentially the same as for PG30 with the following exceptions. The 5' oligonucleotide primer sequence was GCGCGAATTCATGAAACAACTAAACATTATCAGC, the 3' oligonucleotide primer sequence was GCGTGCGGCCGCGAAATTGATCTTTGTACCGACGA and DNA was amplified with the Tth XL PCR kit. The PCR product was cloned into the expression plasmid pET24a using the Eco RI and Not I restriction sites and transformed into E. *coli* BL21DE3. Expression studies and immunoreactivity studies were carried out on whole *E*. *coli* lysates. Purification was not done for these studies.

### PG36

The methods used for PG36 were essentially the same as for PG30 with the following exceptions. The 5' oligonucleotide primer sequence was AAAGGAATTCTACAAAAAGATTATTGCCGTAGCA, the 3' oligonucleotide primer sequence was CTATGCGGCCGCGAACTCCTGTCCGAGCACAAAGT and DNA was amplified with the Tth XL PCR kit. The PCR product was cloned into the expression plasmid pET24a using theEco RI and Not I restriction sites and transformed into *E. coli* BL21DE3. Expression studies and immunoreactivity studies were carried out on whole *E. coli* lysates. Purification was not done for these studies.

### PG37

The methods used for PG37 were essentially the same as for PG30 with the following exceptions. The 5' oligonucleotide primer sequence was TGGCGAATTCAAACGGTTTTTGATTTTGATCGGC, the 3' oligonucleotide primer sequence was CTATGCGGCCGCCTTGCTAAAGCCCATCTTGCTCAG and DNA was amplified with the Tth XL PCR kit. The PCR product was cloned into the expression plasmid pET24a using the Eco RI and Not I restriction sites and transformed into E. *coli* BL21DE3. Expression studies and immunoreactivity studies were carried out on whole E. *coli* lysates. Purification was not done for these studies.

### PG38

The methods used for PG38 were essentially the same as for PG30 with the following exceptions. The predicted N-terminal signal sequence was removed from the recombinant protein. The 5' oligonucleotide primer sequence was CCTCGAATTCCAAAAGGTGGCAGTGGTAAACACT, the 3' oligonucleotide primer sequence was CTATGCGGCCGCCTTGATTCCGAGTTTCGGITTTAC and DNA was amplified with the Tth XL PCR kit. The PCR product was cloned into the expression plasmid pET24a using the Eco RI and Not I restriction sites and transformed into E. *coli* BL21DE3. Expression studies and immunoreactivity studies were carried out on whole E. *coli* lysates. Purification was not done for these studies.

### PG39

The methods used for PG39 were essentially the same as for PG30 with the following exceptions. The predicted N-terminal signal sequence was removed from the recombinant protein. The 5' oligonucleotide primer sequence was AGCTGGATCCCAAGGCGTCAGGGTATCGGGCTAT, the 3' oligonucleotide primer sequence was CTATGCGGCCGCGAATTCGACGAGGAGACGCAGGT and DNA was amplified with the Tth XL PCR kit. The PCR product was cloned into the expression plasmid pET24a using the Bam HI and Not I restriction sites and transformed into *E. coli* BL21DE3. Expression studies and immunoreactivity studies were carried out on whole *E. coli* lysates. Purification was not done for these studies.

### PG40

The methods used for PG40 were essentially the same as for PG30 with the following exceptions. The predicted N-terminal signal sequence was removed from the recombinant protein. The 5' oligonucleotide primer sequence was GGCTGAATTCAAGACGGACAACGTCCCGACAGAT, the 3' oligonucleotide primer sequence was CTATGCGGCCGCGAAGTTGACCATAACCTTACCCA and DNA was amplified with the Tth XL PCR kit. The PCR product was cloned into the expression plasmid pET24a using the Eco RI and Not I restriction sites and transformed into E. *coli* BL21DE3. Expression studies and immunoreactivity studies were carried out on whole *E. coli* lysates. Purification was not done for these studies.

### PG41

The methods used for PG41 were essentially the same as for PG30 with the following exceptions. The predicted N-terminal signal sequence was removed from the recombinant protein. The 5' oligonucleotide primer sequence was GACTGAATTCCAAAACGCCTCCGAAACGACGGTA, the 3' oligonucleotide primer sequence was CTATGCGGCCGCTTGTTCGGGAATCCCCATGCCGTT and DNA was amplified with the Tth XL PCR kit. The PCR product was cloned into the expression plasmid pET24a using the Eco RI and Not I restriction sites and transformed into *E. coli* BL21DE3. Expression studies and immunoreactivity studies were carried out on whole E. *coli* lysates. Purification was not done for these studies.

### PG42

The methods used for PG42 were essentially the same as for PG30 with the following exceptions. The 5' oligonucleotide primer sequence was GTTTGAATTCGCAAATAATACTCTTTTGGCGAAG, the 3' oligonucleotide primer sequence was GAGTGCGGCCGCTTTGCCGGACATCGAAGAGATCGTC and DNA was amplified with the Tth XL PCR kit. The PCR product was cloned into the expression plasmid pET24a using the Eco RI and Not I restriction sites and transformed into *E. coli* BL21DE3. Expression studies and immunoreactivity studies were carried out on whole *E. coli* lysates. Purification was not done for these studies.

### PG43

The methods used for PG43 were essentially the same as for PG30 with the following exceptions. The 5' oligonucleotide primer sequence was GCGCGAATTGAAAAAAGAAAAACTTTGC-ATTGCG, the 3' oligonucleotide primer sequence was CTATGCGGCCGCCTTCAAAGCGAAAGAAGCCTTAAC and DNA was amplified with the Tth XL PCR kit. The PCR product was cloned into the expression plasmid pET24a using the Eco RI and Not I restriction sites and transformed into E. *coli* BL21DE3. Expression studies and immunoreactivity studies were carried out on whole *E. coli* lysates. Purification was not done for these studies.

### PG44

The methods used for PG44 were essentially the same as for PG30 with the following exceptions; The predicted N-terminal signal sequence was removed from the recombinant protein. The 5' oligonucleotide primer sequence was AGCCGAATTCTGTAAGAAAAATGCTGACACTACC, the 3' oligonucleotide primer sequence was CTATGCGGCCGCCTTTTTCCCGGGCTTGATCCCGAT and DNA was amplified with the Tth XL PCR kit. The PCR product was cloned into the expression plasmid pET24a using the Eco RI and Not I restriction sites and transformed into *E. coli* BL21DE3. Expression studies and immunoreactivity studies were carried out on whole *E. coli* lysates. Purification was not done for these studies.

### PG45

The methods used for PG45 were essentially the same as for PG30 with the following exceptions. The predicted N-terminal signal sequence was removed from the recombinant protein. The 5' oligonucleotide primer sequence was GACAGGATCCTGG'TCCACCACAAAGAATCTGCCG, the 3' oligonucleotide primer sequence was CTATGCGGCCGCGAAGGGATAGCCGACAGCCAAAT and DNA was amplified with the Tth XL PCR kit. The PCR product was cloned into the expression plasmid pET24a using the Bam HI and Not I restriction sites and transformed into *E. coli* BL21DE3. Expression studies and immunoreactivity studies were carried out on whole *E. coli* lysates. Purification was not done for these studies.

### PG46

The methods used for PG46 were essentially the same as for PG30 with the following exceptions. The predicted N-terminal signal sequence was removed from the recombinant protein. The 5' oligonucleotide primer sequence was CTCGGAATTCCGTTATGTGCCGGACGGTAGCAGA, the 3' oligonucleotide primer sequence was CTATGCGGCCGCGAACGGATAGCCTACTGCAATGT and DNA was amplified with the Tth XL PCR kit. The PCR product was cloned into the expression plasmid pET24a using the Eco RI and Not I restriction sites and transformed into *E. coli* BL21DE3. Expression studies and immunoreactivity studies were carried out on whole E. *coli* lysates. Purification was not done for these studies.

### PG47

The methods used for PG47 were essentially the same as for PG30 with the following exceptions. The predicted N-terminal signal sequence was removed from the recombinant protein. The 5' oligonucleotide primer sequence was CGCCGAATTCCAAACAGTGGTGACCGGTAAGGTGATCGATTCAGAA, the 3' oligonucleotide primer sequence was CTATGCGGCCGCGAAGTTTACACGAATACCGGTAGACCAAGTGCGGCC and DNA was amplified with the Tth XL PCR kit. The PCR product was cloned into the expression plasmid pET24a using the Eco RI and Not I restriction sites and transformed into E. *coli* BL21DE3. Expression studies and immunoreactivity studies were carried out on whole *E. coli* lysates. Purification was not done for these studies.

### PG48

The methods used for PG48 were essentially the same as for PG30 with the following exceptions. The predicted N-terminal signal sequence was removed from the recombinant protein. The 5' oligonucleotide primer sequence was TGCTGAATTCCAAAAATCCAAGCAGGTACAGCGA, the 3' oligonucleotide primer sequence was CTATGCGGCCGCTCGTAACCATAGTCTTGGGTTTTG and DNA was amplified with the Tth XL PCR kit. The PCR product was cloned into the expression plasmid pET24a using the Eco RI and Not I restriction sites and transformed into *E. coli* BL21DE3. Expression studies and immunoreactivity studies were carried out on whole *E. coli* lysates. Purification was not done for these studies.

### PG49

The methods used for PG49 were essentially the same as for PG30 with the following exceptions. The predicted N-terminal signal sequence was removed from the recombinant protein. The 5' oligonucleotide primer sequence was GAACGGATCCAACGAGCCGGTGGAAGACAGATCC, the 3' oligonucleotide primer sequence was GAGTGCGGCCGCTAATCTCGACTTCATACTTGTACCA and DNA was amplified with the Tth XL PCR kit. The PCR product was cloned into the expression plasmid pET24a using the Barn HI and Not I restriction sites and transformed into *E. coli* BL21DE3. Expression studies and immunoreactivity studies were carried out on whole E. *coli* lysates. Purification was not done for these studies.

### PG50

The methods used for PG50 were essentially the same as for PG30 with the following exceptions. The predicted N-terminal signal sequence was removed from the recombinant protein. The 5'oligonucleotide -primer sequence was GCTGGGATCCGCGACAGACACTGAGTTCAAGTAC, the 3' oligonucleotide primer sequence was CTATGCGGCCGCGAACTTCACTACCAAGCCCATGT and DNA was amplified with the Tth XL PCR kit. The PCR product was cloned into the expression plasmid pET24a using the Bam HI and Not I restriction sites and transformed into E. *coli* BL21DE3. Expression studies and immunoreactivity studies were carried out on whole *E. coli* lysates. Purification was not done for these studies.

### PG51

The methods used for PG51 were essentially the same as for PG30 with the following exceptions. The predicted N-terminal signal sequence was removed from the recombinant protein. The 5' oligonucleotide primer sequence was TCTTGAATTCGCGCAAAGTCTTTTCAGCACCGAA, the 3' oligonucleotide primer sequence was CTATGCGGCCGCACTTTTTCGTGGGATCACTCTCTT and DNA was amplified with the Tth XL PCR kit_{'} The PCR product was cloned into the expression plasmid pET24a using the Eco RI and Not I restriction sites and transformed into E. *coli* BL21DE3. Expression studies and immunoreactivity studies were carried out on whole E. *coli* lysates. Purification was not done for these studies.

### PG52

The methods used for PG52 were essentially the same as for PG30 with the following exceptions. The 5' oligonucleotide primer sequence was AGAAGAATTCAAACGGACAATCCTCCTGACGGCA, the 3' oligonucleotide primer sequence was CTATGCGGCCGCGAAGTGTTTGCCCTGATAGAAATC and DNA was amplified with the Tth XL PCR kit The PCR product was cloned into the expression plasmid pET24a using the Eco RI and Not I restriction sites and transformed into E. *coli* BL21DE3. Expression studies and immunoreactivity studies were carried out on whole *E. coli* lysates, Purification was not done for these studies.

### PG53

The methods used for PG53 were essentially the same as for PG30 with the following exceptions. The predicted N-terminal signal sequence was removed from the recombinant protein. The 5' oligonucleotide primer sequence was GGCTGAATTCGCGAATCCCCTTACGGGCCAATCG, the 3' oligonucleotide primer sequence was CTATGCGGCCGCGTCCGAAAGGCAGCCGTAATAGG and DNA was amplified with the Tth XL PCR kit. The PCR product was cloned into the expression plasmid pET24a using the Eco RI and Not I restriction sites and transformed into E. *coli* BL21DE3. Expression studies and immunoreactivity studies were carried out on whole E. *coli* lysates. Purification was not done for these studies.

### PG54

The methods used for PG54 were essentially the same as for PG30 with the following exceptions. The predicted N-terminal signal sequence was removed from the recombinant protein. The 5' oligonucleotide, primer sequence was CGCTGAATTCCAGATTTCGTTCGGAGGGGAACCC, the 3' oligonucleotide primer sequence was CTATGCGGCCGCCTGCTTCACGATGTTTTGGCTCA and DNA was amplified with the Tth XL PCR kit. The PCR product was cloned into the expression plasmid pET24a using the Eco RI and Not I restriction sites and transformed into *E. coli* BL21DE3. Expression studies and immunoreactivity studies were carried out on whole E. *coli* lysates. Purification was not done for these studies.

### PG55

The methods used for PG55 were essentially the same as for PG30 with the following exceptions. The predicted N-terminal signal sequence was removed from the recombinant protein. The 5' oligonucleotide primer sequence was CGAGGGATCCGAGCTCTCTATT'hGCGATGGCGAG; the 3' oligonucleotide primer sequence was GAGTGCGGCCGCTCTTACCTGACTTCTTGTCACGAAT and DNA was amplified with the Tth XL PCR kit. The PCR product was cloned into the expression plasmid pET24a using the Bam HI and Not I restriction sites and transformed into *E. coli* BL21DE3. Expression studies and immunoreactivity studies were carried out on whole *E. coli* lysates. Purification was not done for these studies.

### PG56

The methods used for PG56 were essentially the same as for PG30 with the following exceptions. The 5' oligonucleotide primer sequence was AAATGGATCCCGAAAAATTITGAGCTTTTTGATG, the 3' oligonucleotide primer sequence was CTATGCGGCCGCTTTGATTCGTAATTTTTCCGTATC and DNA was amplified with the Tth XL PCR kit. The PCR product was cloned into the expression plasmid pET24a using the Bam HI and Not I restriction sites and transformed into E. *coli* BL21DE3. Expression studies and immunoreactivity studies were carried out on whole E. *coli* lysates. Purification was not done for these studies.

### PG57

The methods used for PG57 were essentially the same as for PG30 with the following exceptions. The predicted N-terminal signal sequence was removed from the recombinant protein. The 5' oligonucleotide primer sequence was TGCTGGATCCCAAGAGATCTCAGGCATGAATGCA, the 3' oligonucleotide primer sequence was GAGTGCGGCCGCTCGGCCTCTTTATCTCTACCTTTTC and DNA was amplified with the Tth XL PCR kit The PCR product was cloned into the expression plasmid pET24a using the Bam HI and Not I restriction sites and transformed into *E. coli* BL21DE3. Expression studies and immunoreactivity studies were carried out on whole E. *coli* lysates. Purification was not done for these studies.

### PG58

The methods used for PG58 were essentially the same as for PG30 with the following exceptions. The predicted N-terminal signal sequence was removed from the recombinant protein. The 5' oligonucleotide primer sequence was CGGTGAATTCCAAACCCCACGAAATACAGAAACC, the 3' oligonucleotide primer sequence was GAGTGCGGCCGCTGAAAGTCCAGCTAAA.ACCGGCGAA and DNA was amplified with the Tth XL PCR kit The PCR product was cloned into the expression plasmid pET24a using the Eco RI and Not I restriction sites and transformed into E. *coli* BL21DE3. Expression studies and immunoreactivity studies were carried out on whole E. *coli* lysates. Purification was not done for these studies.

### PG59

The methods used for PG59 were essentially the same as for PG30 with the following exceptions. The predicted N-terminal signal sequence was removed from the recombinant protein. The 5' oligonucleotide primer sequence was TGCTGAATTCCAACAAGAGAAGCAGGTGTTTCAT, the 3' oligonucleotide primer sequence was GAGTGCGGCCGCTGAAGATGCTCTTATCGTCCAAACG and DNA was amplified with the Tth XL PCR kit The PCR product was cloned into the expression plasmid pET24a using the Eco RI and Not I restriction sites and transformed into *E. coli* BL21DE3. Expression studies and immunoreactivity studies were carried out on whole *E. coli* lysates. Purification was not done for these studies.

### PG60

The methods used for PG60 were essentially the same as for PG30 with the following exceptions. The predicted N-terminal signal sequence was removed from the recombinant protein. The 5' oligonucleotide primer sequence was GGCGGAATTCCAGATGCTCAATACTCCTTTCGAG, the 3' oligonucleotide primer sequence was GAGTGCGGCCGCTGAAGAGGTAGGAGATATTGCAGAT and DNA was amplified with the Tth XL PCR kit. The PCR product was cloned into the expression plasmid pET24a using the Eco RI and Not I restriction sites and transformed into *E. coli* BL21DE3. Expression studies and immunoreactivity studies were carried out on whole E. *coli* lysates. Purification was not done for these studies.

### PG61

The methods used for PG61 were essentially the same as for PG30 with the following exceptions. The predicted N-terminal signal-sequence was removed from the recombinant protein. The 5' oligonucleotide primer sequence was AGCAGAATTCCCCGTCTCCAACAGCGAGATAGAT, the 3' oligonucleotide primer sequence was GAGTGCGGCCGCTGAAATCGATTGTCAGACTACCCAG and DNA was amplified with the Tth XL PCR kit The PCR product was cloned into the expression plasmid pET24a using the Eco RI and Not I restriction sites and transformed into E. *coli* BL21DE3. Expression studies and immunoreactivity studies were carried out on whole E. *coli* lysates. Purification was not done for these studies.

### PG62

The methods used for PG62 were essentially the same as for PG30 with the following exceptions. The predicted N-terminal signal sequence was removed from the recombinant protein. The 5' oligonucleotide primer sequence was TGCTGAATTCCAGCGGTTTCCGATGGTGCAGGGA, the 3' oligonucleotide primer sequence was GAGTGCGGCCGCTGAAGTGAAATCCGACACGCAGCTG and DNA was amplified with the Tth XL PCR kit. The PCR product was cloned into the expression plasmid pET24a using the Eco RI and Not I restriction sites and transformed into E. *coli* BL21DE3. Expression studies and immunoreactivity studies were carried out on whole *E. coli* lysates. Purification was not done for these studies.

### PG63

The methods used for PG63 were essentially the same as for PG30 with the following exceptions. The predicted N-terminal signal sequence was removed from the recombinant protein. The 5' oligonucleotide primer sequence was GGCAGAATTCCAAGAAGCAAACACTGCATCTGAC, the 3' oligonucleotide primer sequence was GAGTGCGGCCGCTGAAAGTGTACGCAACACCCACGCC and DNA was amplified with the Tth XL PCR kit. The PCR product was cloned into the expression plasmid pET24a using the Eco RI and Not I restriction sites and transformed into *E. coli* BL21DE3. Expression studies and immunoreactivity studies were carried out on whole *E. coli* lysates. Purification was not done for these studies.

### PG64

The methods used for PG64 were essentially the same as for PG30 with the following exceptions. The predicted N-terminal signal sequence was removed from the recombinant protein. The 5' oligonucleotide primer sequence was TGCTGAATTCCAGAGTCGTCCTGCTCTTAGACTG, the 3' oligonucleotide primer sequence was GAGTGCGGCCGCTGAAGCGAACACCGAGACCCACAAA and DNA was amplified with the Tth XL PCR kit. The PCR product was cloned into the expression plasmid pET24a using the Eco RI and Not I restriction sites and transformed into *E. coli* BL21DE3. Expression studies and immunoreactivity studies were carried out on whole *E. coli* lysates. Purification was not done for these studies.

### PG65

The methods used for PG65 were essentially the same as for PG30 with the following exceptions. The predicted N-terminal signal sequence was removed from the recombinant protein. The 5' oligonucleotide primer sequence was GGCCGGATCCATCGGACAAAGCCGCCCGGCACTT, the 3' oligonucleotide primer sequence was GAGTGCGGCCGCTAAAGCGGTAACCTATGCCCACGAA and DNA was amplified with the Tth XL PCR kit. The PCR product was cloned into the expression plasmid pET24a using the Bam HI and Not I restriction sites and transformed into E. *coli* BL21DE3. Expression studies and immunoreactivity studies were carried out on whole *E. coli* lysates. Purification was not done for these studies.

### PG66

The methods used for PG66 were essentially the same as for PG30 with the following exceptions. The predicted N-terminal signal sequence was removed from the recombinant protein. The 5' oligonucleotide primer sequence was GTTTGAATTCCAAGACGTTATCAGACCATGGTCA, the 3' oligonucleotide primer sequence was GAGTGCGGCCGCTAAAATGAGTGGAGAGCGTGGCCAT and DNA was amplified with the Tth XL PCR kit. The PCR product was cloned into the expression plasmid pET24a using the Eco RI and Not I restriction sites and transformed into E. *coli* BL21DE3. Expression studies and immunoreactivity studies were carried out on whole E. *coli* lysates. Purification was not done for these studies.

### PG67

The methods used for PG67 were essentially the same as for PG30 with the following exceptions. The predicted N-terminal signal sequence was removed from the recombinant protein. The 5' oligonucleotide primer sequence was GAACGAGCTCGCGGAACGTCCTATGGCCGGAGCA, the 3' oligonucleotide primer sequence was GAGTGCGGCCGCTATACCAAGTATTCGTGATGGGACG and DNA was amplified with the Tth XL PCR kit The PCR product was cloned into the expression plasmid pET24a using the Sac I and Not I restriction sites and transformed into E. *coli* BL21DE3. Expression studies and immunoreactivity studies were carried out on whole E. *coli* lysates. Purification was not done for these studies.

### PG68

The methods used for PG68 were essentially the same as for PG30 with the following exceptions. The predicted N-terminal signal sequence was removed from the recombinant protein. The 5' oligonucleotide primer sequence was GCTTGCGGCCGCCCTTATGAAAGATITGCAGAT, the 3' oligonucleotide primer sequence was GGTGCTCGAGTATACTCAACAAGCACCTTATGCAC and DNA was amplified with the Tth XL PCR kit The PCR product was cloned into the expression plasmid pET24a using the Not I and Xho I restriction sites and transformed into *E. coli* BL21DE3. Expression studies and immunoreactivity studies were carried out on whole *E. coli* lysates. Purification was not done for these studies.

### PG69

The methods used for PG69 were essentially the same as for PG30 with the following exceptions. The predicted N-terminal signal sequence was removed from the recombinant protein. The 5' oligonucleotide primer sequence was TGCTGAATTCCAGGAAGGGGAGGGGAGTGCCCGA, the 3' oligonucleotide primer sequence was GAGTGCGGCCGCTGAAGCTGTAGCGGGCTTTGAACCA and DNA was amplified with the Tth XL PCR kit. The PCR product was cloned into the expression plasmid pET24a using the Eco RI and Not I restriction sites and transformed into E. *coli* BL21DE3. Expression studies and immunoreactivity studies were carried out on whole *E. coli* lysates. Purification was not done for these studies.

### PG70

The methods used for PG70 were essentially the same as for PG30 with the following exceptions. The predicted N-terminal signal sequence was removed from the recombinant protein. The 5' oligonucleotide primer sequence was CGGTGGATCCTCGCAAATGCTCTTCTCAGAGAAT, the 3' oligonucleotide primer sequence was GAGTGCGGCCGCTAAACGAAATATCGATACCAACATC and DNA was amplified with the Tth XL PCR kit. The PCR product was cloned into the expression plasmid pET24a using the Barn HI and Not I restriction sites and transformed into E. *coli* BL21DE3. Expression studies and immunoreactivity studies were carried out on whole *E. coli* lysates. Purification was not done for these studies.

### PG71

The methods used for PG71 were essentially the same as for PG30 with the following exceptions. The predicted N-terminal signal sequence was removed from the recombinant protein. The 5' oligonucleotide primer sequence was TGCTGAATTCCAGAACAATACCCTCGATGTACAC, the 3' . oligonucleotide primer sequence was GAGTGCGGCCGCTATTGCCGGTAGGATTTCCTTGTCC and DNA was amplified with the Tth XL PCR kit The PCR product was cloned into the expression plasmid pET24a using the Eco RI and Not I restriction sites and transformed into E. *coli* BL21DE3. Expression studies and immunoreactivity studies were carried out on whole E. *coli* lysates. Purification was not done for these studies.

### PG72

The methods used for PG72 were essentially the same as for PG30 with the following exceptions. The predicted N-terminal signal sequence was removed from the recombinant protein. The 5' oligonucleotide primer sequence was TGCTGAATTCGGAGAGCGACTGGAGACGGACAGC, the 3' oligonucleotide primer sequence was GAGTGCGGCCGCTATGATTGCGTTTCAGAAAAGCTAT and DNA was amplified with the Tth XL PCR kit The PCR product was cloned into the expression plasmid pET24a using the Eco RI and Not I restriction sites and transformed TGCTGAATTCGGAGAGCGACTGGAGACGGACAGC into *E. coli* BL21DE3. Expression studies and immunoreactivity studies were carried out on whole *E. coli* lysates. Purification was not done for these studies.

### PG73

The methods used for PG73 were essentially the same as for PG30 with the following exceptions. The predicted N-terminal signal sequence was removed from the recombinant protein. The 5' oligonucleotide primer sequence was CGGTGAATTCCAACAGACAGGACCGGCCGAACGC, the 3' oligonucleotide primer sequence was GAGTGCGGCCGCTTAAGAAAGGTATCTGATAGATCAG and DNA was amplified with the Tth XL PCR kit. The PCR product was cloned into the expression plasmid pET24a using the Eco RI and Not I restriction sites and transformed into *E. coli* BL21DE3. Expression studies and immunoreactivity studies were carried out on whole E. *coli* lysates. Purification was not done for these studies.

### PG74

The methods used for PG74 were essentially the same as for PG30 with the following exceptions. The predicted N-terminal signal sequence was removed from the recombinant protein. The 5' oligonucleotide primer sequence was TGCTGAATTCCAAGAAAATAATACAGAAAAGTCA, the 3' oligonucleotide primer sequence was GAGTGCGGCCGCTGAGGTITAATCCTATGCCAATACT and DNA was amplified with the Tth XL PCR kit. The PCR product was cloned into the expression plasmid pET24a using the Eco RI and Not I restriction sites and transformed into *E. coli* BL21DE3. Expression studies and immunoreactivity studies were carried out on whole *E. coli* lysates. Purification was not done for these studies.

### PG75

The methods used for PG75 were essentially the same as for PG30 with the following exceptions. The predicted N-terminal signal sequence was removed from the recombinant protein. The 5' oligonucleotide primer sequence was GGCGGGATCCGCTCAGGAGCAACTGAATGTGGTA, the 3' oligonucleotide primer sequence was GAGTGCGGCCGCTGTGGAACAAATTGCGCAATCCATC and DNA was amplified with the Tth XL PCR kit. The PCR product was cloned into the expression plasmid pET24a using the Bam HI and Not I restriction sites and transformed into E. *coli* BL21DE3. Expression studies and immunoreactivity studies were carried out on whole E. *coli* lysates. Purification was not done for these studies.

### PG76

The methods used for PG76 were essentially the same as for PG30 with the following exceptions. The predicted N-terminal signal sequence was removed from the recombinant protein. The 5' oligonucleotide primer sequence was AGCAGAATTCGGAAACGCACAGAGCTTTTGGGAA, the 3' oligonucleotide primer sequence was GAGTGCGGCCGCTTACCTGCACCTTATGACTGAATAC and DNA was amplified with the Tth XL PCR kit. The PCR product was cloned into the expression plasmid pET24a using the Eco RI and Not I restriction sites and transformed into *E. coli* BL21DE3. Expression studies and immunoreactivity studies were carried out on whole *E. coli* lysates. Purification was not done for these studies.

### PG77

The methods used for PG77 were essentially the same as for PG30 with the following exceptions. The predicted N-terminal signal sequence was removed from the recombinant protein. The 5' oligonucleotide primer sequence was TGCTGAATTCCAAGAGAAAAAGGATAGTCTCTCT, the 3' oligonucleotide primer sequence was GAGTGCGGCCGCTCTTCTTATCGCCATAGAATACAGG and DNA was amplified with the Tth XL PCR kit. The PCR product was cloned into the expression plasmid pET24a using the Eco RI and Not I restriction sites and transformed into E. *coli* BL21DE3. Expression studies and immunoreactivity studies were carried out on whole E. *coli* lysates. Purification was not done for these studies.

### PG78

The methods used for PG78 were essentially the same as for PG30 with the following exceptions. The predicted N-terminal signal sequence was removed from the recombinant protein. The 5' oligonucleotide primer sequence was GGCAGAATTCCAGGATTCTTCCCACGGTAGCAAT, the 3' oligonucleotide primer sequence was GAGTGCGGCCGCTATCATGATAGTAAAGACTGGTTCT and DNA was amplified with the Tth XL PCR kit. The PCR product was cloned into the expression plasmid pET24a using the Eco RI and Not I restriction sites and transformed into E. *coli* BL21DE3. Expression studies and immunoreactivity studies were carried out on whole E. *coli* lysates. Purification was not done for these studies.

### PG79

The methods used for PG79 were essentially the same as for PG30 with the following exceptions. The 5' oligonucleotide primer sequence was TGCTGAATTCGTAGTGACGCTGCTCGTAATTGTC, the 3' oligonucleotide primer sequence was GAGTGCGGCCGCTGCCGTCCTGCCTTTCTGCCTGACG and DNA was amplified with the Tth XL PCR kit. The PCR product was cloned into the expression plasmid pET24a using the Eco RI and Not I restriction sites and transformed into E. *coli* BL21DE3. Expression studies and immunoreactivity studies were carried out on whole E. *coli* lysates. Purification was not done for these studies.

### PG80

The methods used for PG80 were essentially the same as for PG30 with the following exceptions. The predicted N-terminal signal sequence was removed from the recombinant protein. The 5' oligonucleotide primer sequence was GGCCGAATTCCAAAACGTGCAGTTGCACTACGAT, the 3' oligonucleotide primer sequence was GAGTGCGGCCGCTGTTGAAAGTCCATTTGACCGCAAG and DNA was amplified with the Tth XL PCR kit. The PCR product was cloned into the expression plasmid pET24a using the Eco RI and Not I restriction sites and transformed into E. *coli* BL21DE3. Expression studies and immunoreactivity studies were carried out on whole *E*. *coli* lysates. Purification was not done for these studies.

### PG81

The methods used for PG81 were essentially the same as for PG30 with the following exceptions. The predicted N-terminal signal sequence was removed from the recombinant protein. The 5' oligonucleotide primer sequence was GTTTGAATTCCAGGATTTTCTCTATGAAATAGGA, the 3' oligonucleotide primer sequence was GAGTGCGGCCGCTITGTTTATTACAAAAAGTCTTACG and DNA was amplified with the Tth XL PCR kit. The PCR product was cloned into the expression plasmid pET24a using the Eco RI and Not I restriction sites and transformed into E. *coli* BL21DE3. Expression studies and immunoreactivity studies were carried out on whole E. *coli* lysates. Purification was not done for these studies.

### PG82

The methods used for PG82 were essentially the same as for PG30 with the following exceptions. The predicted N-terminal signal sequence was removed from the recombinant protein. The 5' oligonucleotide primer sequence was GAACGAATTCCAGAACAACAACTTTACCGAGTCG, the 3' oligonucleotide primer sequence was GAGTGCGGCCGCTGTTCAGTTTCAGCTITITAAACCA and DNA was amplified with the Tth XL PCR kit The PCR product was cloned into the expression plasmid pET24a using the Eco RI and Not I restriction sites and transformed into E. *coli* BL21DE3. Expression studies and immunoreactivity studies were carried out on whole *E. coli* lysates. Purification was not done for these studies.

### PG84

The methods used for PG84 were essentially the same as for PG30 with the following exceptions. The predicted N-terminal signal sequence was removed from the recombinant protein. The 5' oligonucleotide primer sequence was TGCTGGATCCCAGAATGATGACATCTTCGAAGAT, the 3' oligonucleotide primer sequence was GAGTGCGGCCGCTATTGCGTCCCCGGCCACTACGTCC and DNA was amplified with the Tth XL PCR kit. The PCR product was cloned into the expression plasmid pET24a using the Bam HI and Not I restriction sites and transformed into E. *coli* BL21DE3. Expression studies and immunoreactivity studies were carried out on whole E. *coli* lysates. Purification was not done for these studies.

### PG85

The methods used for PG85 were essentially the same as for PG30 with the following exceptions. The predicted N-terminal signal sequence was removed from the recombinant protein. The 5' oligonucleotide primer sequence was CGGTGAATTCGTACCAACGGACAGCACGGAATCG, the 3' oligonucleotide primer sequence was GAGTGCGGCCGCTCAGATTGGTGCTATAAGAAAGGTA and DNA was amplified with the Tth XL PCR kit. The PCR product was cloned into the expression plasmid pET24a using the Eco RI and Not I restriction sites and transformed into E. *coli* BL21DE3. Expression studies and immunoreactivity studies were carried out on whole *E. coli* lysates. Purification was not done for these studies.

### PG86

The methods used for PG86 were essentially the same as for PG30 with the following exceptions. The predicted N-terminal signal sequence was removed from the recombinant protein. The 5' oligonucleotide primer sequence was TGCTGGATCCCAAACGCATGATCATCTCATCGAA, the 3' oligonucleotide primer sequence was GAGTGCGGCCGCTGTGGTTCAGGCGGTGGC'rCAAATCT and DNA was amplified with the Tth XL PCR kit. The PCR product was cloned into the expression plasmid pET24a using the Barn HI and Not I restriction sites and transformed into E. *coli* BL21DE3. Expression studies and immunoreactivity studies were carried out on whole *E. coli* lysates. Purification was not done for these studies.

### PG87

The methods used for PG87 were essentially the same as for PG30 with the following exceptions. The predicted N-terminal signal sequence was removed from the recombinant protein. The 5' oligonucleotide primer sequence was GGCGGAATTCCAGAGCTATGTGGACTACGTCGAT, the 3' oligonucleotide primer sequence was GAGTGCGGCCGCTATTACTGTGATTAGCGCGACGCTG and DNA was amplified with the Tth XL PCR kit. The PCR product was cloned into the expression plasmid pET24a using the Eco RI and Not I restriction sites and transformed into E. *coli* BL21DE3. Expression studies and immunoreactivity studies were carried out on whole E. *coli* lysates. Purification was not done for these studies.

### PG88

The methods used for PG88 were essentially the same as for PG30 with the following exceptions. The predicted N-terminal signal sequence was removed from the recombinant protein. The 5' oligonucleotide primer sequence was AGCAGAATTCGCCGAATCGAAGTCTGTCTCTTTC, the 3' oligonucleotide primer sequence was GAGTGCGGCCGCTCGGCAAGTAACGCTTTAGTGGGGA and DNA was amplified with the Tth XL PCR kit The PCR product was cloned into the expression plasmid pET24a using the Eco RI and Not I restriction sites and transformed into *E. coli* BL21DE3. Expression studies and immunoreactivity studies were carried out on whole *E. coli* lysates. Purification was not done for these studies.

### PG89

The methods used for PG89 were essentially the same as for PG30 with the following exceptions. The predicted N-terminal signal sequence was removed from the recombinant protein. The 5' oligonucleotide primer sequence was TGCTGAATTCCAATCGAAGTTAAAGATCAAGAGC, the 3' oligonucleotide primer sequence was GAGTGCGGCCGCTATTAGTCCAAAGACCCACGGTAAA and DNA was amplified with the Tth XL PCR kit The PCR product was cloned into the expression plasmid pET24a using the Eco RI and Not I restriction sites and transformed into *E*. *coli* BL21DE3. Expression studies and immunoreactivity studies were carried out on whole E. *coli* lysates. Purification was not done for these studies.

### PG90

The methods used for PG90 were essentially the same as for PG30 with the following exceptions. The predicted N-terminal signal sequence was removed from the recombinant protein. The 5' oligonucleotide primer sequence was GGCAGAATTCCAAACAACGACGAACAGTAGCCGG, the 3' oligonucleotide primer sequence was GAGTGCGGCCGCTTTTTTGTTGTGATACTGTTTGGGC and DNA was amplified with the Tth XL PCR kit. The PCR product was cloned into the expression plasmid pET24a using the Eco RI and Not I restriction sites and transformed into E. *coli* BL21DE3. Expression studies and immunoreactivity studies were carried out on whole *E. coli* lysates. Purification was not done for these studies.

### PG91

The methods used for PG91 were essentially the same as for PG30 with the following exceptions. The predicted N-terminal signal sequence was removed from the recombinant protein. The 5' oligonucleotide primer sequence was TGCTGAATTCCAGACGATGGGAGGAGATGATGTC, the 3' oligonucleotide primer sequence was GAGTGCGGCCGCTTTCCACGATGAGCTTCTCTACGAA and DNA was amplified with the Tth XL PCR kit The PCR product was cloned into the expression plasmid pET24a using the Eco RI and Not I restriction sites and transformed into E. *coli* BL21DE3. Expression studies and immunoreactivity studies were carried out on whole *E*. *coli* lysates. Purification was not done for these studies.

### PG92

The methods used for PG92 were essentially the same as for PG30 with the following exceptions. The predicted N-terminal signal sequence was removed from the recombinant protein. The 5' oligonucleotide primer sequence was GGCCGAATTCGCCGATGCACAAAGCTCTGTCTCT, the 3' oligonucleotide primer sequence was GAGTGCGGCCGCTTCGAGGACGATTGCTTAGTTCGTA and DNA was amplified with the Tth XL PCR kit. The PCR product was cloned into the expression plasmid pET24a using the Eco RI and Not I restriction sites and transformed into E. *coli* BL21DE3. Expression studies and immunoreactivity studies were carried out on whole *E. coli* lysates. Purification was not done for these studies.

### PG93

The methods used for PG93 were essentially the same as for PG30 with the following exceptions. The predicted N-terminal signal sequence was removed from the recombinant protein. The 5' oligonucleotide primer sequence was GGCCGAGCTCCAAGAGGAAGGTATTTGGAATACC, the 3' oligonucleotide primer sequence was GAGTGCGGCCGCTGCGAATCACTGCGAAGCGAATTAG and DNA was amplified with the Tth XL PCR kit. The PCR product was cloned into the expression plasmid pET24a using the Sac I and Not I restriction sites and transformed into *E. coli* BL21DE3. Expression studies and immunoreactivity studies were carried out on whole E. *coli* lysates. Purification was not done for these studies.

### PG94

The methods used for PG94 were essentially the same as for PG30 with the following exceptions. The predicted N-terminal signal sequence was removed from the recombinant protein. The 5' oligonucleotide primer sequence was GGCCGAGCTCCAAGAGGAAGGTATTTGGAATACC, the 3' oligonucleotide primer sequence was GAGTGCGGCCGCTTTGTCCTACCACGATCATTTTCTT and DNA was amplified with the Tth XL PCR lcit. The PCR product was cloned into the expression plasmid pET24a using the Eco RI and Not I restriction sites and transformed into E. *coli* BL21DE3. Expression studies and immunoreactivity studies were carried out on whole *E. coli* lysates. Purification was not done for these studies.

### PG95

The methods used for PG95 were essentially the same as for PG30 with the following exceptions. The predicted N-terminal signal sequence was removed from the recombinant protein. The 5' oligonucleotide primer sequence was GGCCGAGCTCTGTGGAAAAAAAGAAAAACACTCT, the 3' oligonucleotide primer sequence was GAGTGCGGCCGCTAACTGTCTCCTTGTCGCTCCCCGG and DNA was amplified with the Tth XL PCR kit. The PCR product was cloned into the expression plasmid pET24a using the Sac I and Not I restriction sites and transformed into E. *coli* BL21DE3. Expression studies and immunoreactivity studies were carried out on whole *E. coli* lysates. Purification was not done for these studies.

### PG96

The methods used for PG96 were essentially the same as for PG30 with the following exceptions. The predicted N-terminal signal sequence was removed from the recombinant protein. The 5' oligonucleotide primer sequence was TGCTGAGCTCCAAACGCAAATGCAAGCAGACCGA, the 3' oligonucleotide primer sequence was GAGTGCGGCCGCTTTTGAGAATTTTCATTGTCTCACG and DNA was amplified with the Tth XL PCR kit. The PCR product was cloned into the expression plasmid pET24a using the Sac I and Not I restriction sites and transformed into *E. coli* BL21DE3. Expression studies and immunoreactivity studies were carried out on whole E. *coli* lysates. Purification was not done for these studies.

### PG97

The methods used for PG97 were essentially the same as for PG30 with the following exceptions. The predicted N-terminal signal sequence was removed from the recombinant protein. The 5' oligonucleotide primer sequence was GGCGGGATCCCAGTtTGTTCCGGG TCCCACCACA, the 3' oligonucleotide primer sequence was GAGTGCGGCCGCTCTGTITGATGAGCTTAGTGGTATA and DNA was amplified with the Tth XL PCR kit The PCR product was cloned into the expression plasmid pET24a using the Barn HI and Not I restriction sites and transformed into *E. coli* BL21DE3. Expression studies and immunoreactivity studies were carried out on whole E. *coli* lysates. Purification was not done for these studies.

### PG98

The methods used for PG98 were essentially the same as for PG30 with the following exceptions. The predicted N-terminal signal sequence was removed from the recombinant protein. The 5' oligonucleotide primer sequence was AGCAGAATTCCAAGAAAGAGTCGATGAAAAAGTA, the 3' oligonucleotide primer sequence was GAGTGCGGCCGCTTAGCTGTGTAACATTAAGTTTTTATTGAT and DNA was amplified with the Tth XL PCR kit The PCR product was cloned into the expression plasmid pET24a using the Eco RI and Not I restriction sites and transformed into E. *coli* BL21DE3. Expression studies and immunoreactivity studies were carried out on whole E. *coli* lysates. Purification was not done for these studies.

### PG99

The methods used for PG99 were essentially the same as for PG30 with the following exceptions. The predicted N-tenninal signal sequence was removed from the recombinant protein. The 5' oligonucleotide primer sequence was TGCTGAATTCAAGGACAATTCTTCTTACAAACCT, the 3' oligonucleotide primer sequence was GAGTGCGGCCGCTTCGAATCACGACTTTTCTCACAAt°r and DNA was amplified with the Tth XL PCR kit The PCR product was cloned into the expression plasmid pET24a using the Eco RI and Not I restriction sites and transformed into *E. coli* BL21DE3. Expression studies and immunoreactivity studies were carried out on whole *E. coli* lysates. Purification was not done for these studies.

### PG100

The methods used for PG100 were essentially the same as for PG30 with the following exceptions. The predicted N-terminal signal sequence was removed from the recombinant protein. The 5' oligonucleotide primer sequence was GGCAGAATTCCAGTCTTTGAGCACAATCAAAGTA, the 3' oligonucleotide primer sequence was GAGTGCGGCCGCTGATAGCCAGCTTGATGCTCTTAGC and DNA was amplified with the Tth XL PCR kit The PCR product was cloned into the expression plasmid pET24a using the Eco RI and Not I restriction sites and transformed into *E. coli* BL21DE3. Expression studies and immunoreactivity studies were carried out on whole E. *coli* lysates. Purification was not done for these studies.

### PG101

The methods used for PG101 were essentially the same as for PG30 with the following exceptions. The 5' oligonucleotide primer sequence was TGCTGAATTCAAAGGCAAGGGCGATCTGGTCGGG, the 3' oligonucleotide primer sequence was GAGTGCGGCCGCITCTCTTCTCGMCTTGGCCGAGTA and DNA was amplified with the Tth XL PCR kit The PCR product was cloned into the expression plasmid pET24a using the Eco RI and Not I restriction sites and transformed into *E. coli* BL21DE3. Expression studies and immunoreactivity studies were carried out on whole E. *coli* lysates. Purification was not done for these studies.

### PG102

The methods used for PG102 were essentially the same as for PG30 with the following exceptions. The predicted N-terminal signal sequence was removed from the recombinant protein. The 5' oligonucleotide primer sequence was GGCCGAATTCCAGATGGATATTGGTGGAGACGAT, the 3' oligonucleotide primer sequence was GAGTGCGGCCGCTCTCTACAATGATTTTTTCCACGAA and DNA was amplified with the Tth XL PCR kit The PCR product was cloned into the expression plasmid pET24a using the Eco RI and Not I restriction sites and transformed into *E. coli* BL21DE3. Expression studies and immunoreactivity studies were carried out on whole *E. coli* lysates. Purification was not done for these studies.

### PG104

The methods used for PG104 were essentially the same as for PG30 with the following exceptions. The predicted N-terminal signal sequence was removed from the recombinant protein. The 5' oligonucleotide primer sequence was GAACGGATCCAACGTGTCTGCTCAGTCACCCCGA, the 3' oligonucleotide primer sequence was GAGTGCGGCCGCTTCTGAGCGATACTTTTGCACGTAT and DNA was amplified with the Tth XL PCR kit The PCR product was cloned into the expression plasmid pET24a using the Bam HI and Not I restriction sites and transformed into *E. coli* BL21DE3. Expression studies and immunoreactivity studies were carried out on whole E. *coli* lysates. Purification was not done for these studies.

### Animal antisera and human patient sera.

Various antisera were raised for detecting the expression and refolding of the recombinant *P. gingivalis* proteins. A whole cell antisera was raised by injecting New Zealand White rabbits with 3 doses of sonicated *P. gingivalis* (strain W50) containing approximately 2mg of protein. The first dose was given in Freunds complete adjuvant (FCA) and the second and third doses were given in Freunds incomplete adjuvant (IFA) at 3 week intervals. Doses (1ml) were given intramuscularly into the hind legs and rabbits bled 7 days after the last dose, the blood clotted and serum removed and stored at - 20°C until required. A second rabbit antisera was produced in a similar manner but using a sarkosyl insoluble fraction (each dose was 0.69mg of protein) derived from P. *gingivalis* W50 according to the method of Doidg and Trust T. et al 1994 as the immunogen. A third rabbit antisera was produced in a similar manner to the first only the sarkosyl soluble fraction (1mg of protein per dose) derived fromP. *gingivalis* W50 cells according to the method of Doidg P. and Trust Tj. (1994 Infect Immun 62:4526-33) was used as the immunogen.

A "protected rat serum" pool was also used in these studies and was obtained from rats immunised with formalin killed whole P. *gingivalis* cells in FIA (strain ATCC 33277; 2 doses of 2x10⁹ cells, 3 weeks apart). Rats were then challenged 2 weeks after their last dose with live P. *gingivalis* cells (strain 33277) given orally as previously described (Klaussen B. et al. 1991, Oral Microbiol Immunol 6:193-201) and the serum obtained from these rats 6 weeks after the final challenge inoculation at the time of sacrifice.

Human sera were obtained from adult patients undergoing treatment or assessment for periodontitis at an outpatient clinic. These patients had at least 6 teeth with 6mm attachment loss and had *P. gingivalis* present in their sub-gingival plaque as detected using a P. *gingivalis* specific DNA probe. Sera was pooled from these patients and compared to a pool of sera from periodontally healthy patients.

### Immunization and Murine Lesion Model Protocols

The mouse abscess model was used to assess the efficacy of immunising mice with recombinant P. *gingivalis* proteins in protecting mice from formation of a subcutaneous abscess. This model has been used by others as a predictor of potential vaccines against periodontal disease (Bird PS, et al. 1995 J. Periodontol. 66:351-362. BALB/c mice 6-8 weeks old were immunised by subcutaneously injecting them with 0.1 ml containing either 10 or 20µg of recombinant P. *gingivalis* protein, 20µg of E. *coli* lysate protein, 2 x 10⁹ formalin killed cells of P. *gingivalis* strain 33277 emulsified in incomplete Freund's adjuvant (IFA; Sigma) on day 0. At day 21 mice were re-injected with the same dose and then bled 1 week later and evaluated for antibody levels. At day 35 mice all mice were challenged with approximately 2 x 10⁹ cells of live P. *gingivalis* (ATCC 33277) by subcutaneous injection in the abdomen. Following challenge mice were monitored daily for weight loss and the size of the lesion measured for the next 10 days. Lesion sizes were measured by length and width and expressed as mm². Groups were statistically analysed using a Kruskal-Wallis one-way ANOVA and were also individually examined using the unpaired t test or Mann-Whitney rank sum test using the Instat statistical package.

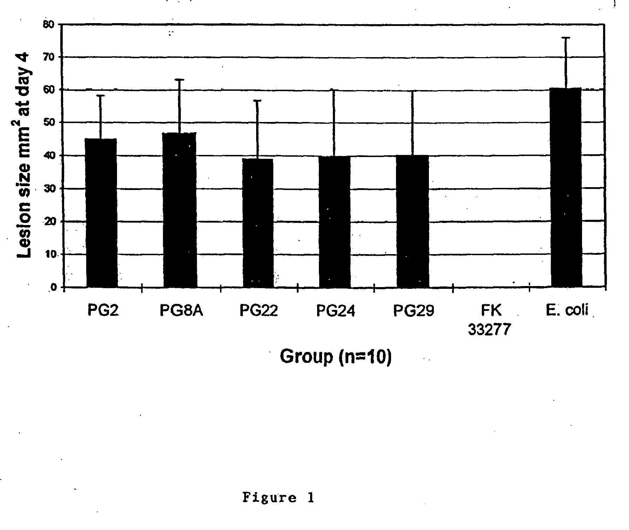

Figure 1 shows the results of one experiment at day 4 after challenge (lesions were at maximum size at this time point). Control mice immunised with *E. coli* lysate showed large lesions while mice immunised with killed cells of P. *gingivalis* strain 33277 were fully protected. This indicates that whole cells provide protection against P. *gingivalis* while E. *coli* protein immunised mice were not protected. Mice given the various PG recombinant proteins showed significant levels of protection for PG2, PG22, PG24 and PG29 (p<0.05 unpaired t test) while PG8A was not quite significantly different (p=0.07) compared to the E. *coli* control group.

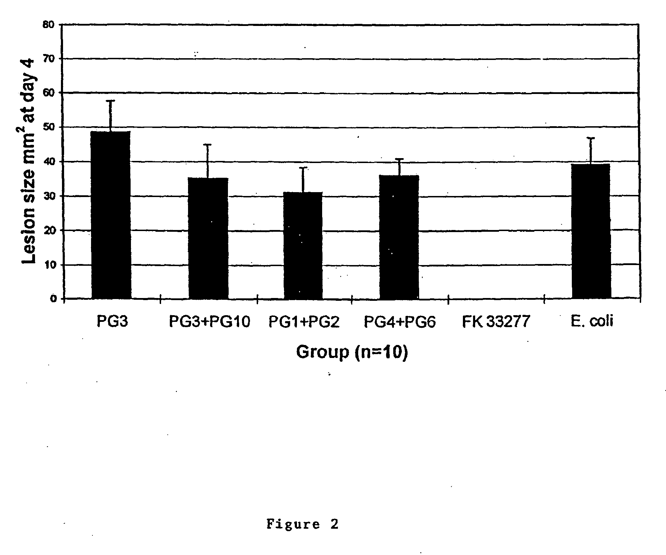

Figure 2 shows the results of a separate experiment using combinations of recombinant proteins. Mice given PG1 + PG2 showed a significant level of protection compared to control mice give E. *coli* lysate (p<0.026 unpaired t test).

### Immunoscreening

Cloned candidates were cultured in 15ml of Terrific broth, induced with IPTG and sampled at 4h post-induction. One ml of culture was removed, pelleted and the cells resuspended in a volume of PBS determined by dividing the OD A₀₀₀ₙₘ of the culture by 8. An aliquot of lysate (100µl) was added to 100µl of 2x sample reducing buffer (125mM Tris pH 6.8, 20% glycerol, 4% SDS, 80mM DTT, 0.03% bromophenol blue) and boiled for 10min. SDS-PAGE was performed according to the method of Laemmli UK. 1970 (Nature 227:680-685) using 4-20% 1.0mm Tris-Glycine gels (Novex) according to the manufacturers recommendations. Proteins were transferred onto Hybond-C Extra nitrocellulose membranes (Amersham) by transblotting and the membranes were then blocked for 2h at room temperature (RT) in 5% skim milk in 20mM Tris,.0.5M NaCl, 0.05% Tween-20, pH 7.5 (TTBS).

Immunoscreening was performed separately with the rabbit *anti-P. gingivalis* whole cell serum, the rat protective serum, a pool of human periodontal patients serum, and in many cases an anti-T7-Tag antibody HRP conjugate (Novagen). Prior to use, the rabbit, rat and human sera were diluted 1/5000, 1/1000 and 1/500 respectively in 5% skim milk in TTBS and absorbed with 100µl (for the rabbit serum) or 250µl (for the rat and human sera) *E. coli* extract (20mg/ml; Promega) for 6h at RT.

Membranes were incubated overnight at RT with the absorbed antisera, or for 1 hr at RT with 1/5000 diluted anti-T7-Teg conjugate. Following 3x10min washes with TTBS, HRP-conjugated anti-rabbit (Silenus), anti-mouse (Silenus) or anti-human (KPL) antibody, diluted 1/5000 in 5% skim milk in TTBS, was added for 1h at RT. Membranes were washed as before, prior to addition of TMB membrane peroxidase substrate (KPL) for detection of immunoreactive proteins. Results of reactivity for the recombinant P. *gingivalis* proteins is shown in Table 7.

In addition some of the sera (pooled sera diluted 1/1000) from the mice immunised with P. *gingivalis* recombinant proteins (prior to challenge) were analysed for their reactivity against Western blots of whole native W50 *P. gingivalis* proteins using similar techniques as those outlined above. PG2, PG8A, PG29 and PG3 all showed bands at a similar molecular weight to that of the recombinant PG protein in the native W50 blot. This indicates that PG proteins are expressed in the W50 strain and that the recombinant proteins have at least some identical immunogenicity to the native proteins.

### m-RNA analysis

### Hot Phenol RNA Extraction

*P. gingivalis* W50 cells (150ml culture) were grown anaerobically to mid log phase (OD A₀₀₀=0.18) mixed with 50% glycerol and stored at -70°C until RNA extraction. Cells were pelleted by centrifugation at 6000g, and resuspended in 8ml ASE (20mM NaOAc, 0.5% SDS,1mM EDTA). An equal volume of 20mM NaOAc(pH 4.5)-saturated phenol was added and mixed by shaking for 30 seconds, incubated at 65°C for 5 minutes, followed by a further 5 second shaking and repeated incubation. After cooling, 2ml chloroform was added and mixed by shaking for 5 seconds, and the mixture spun at 10000g for 10 minutes at 4°C. The top aqueous phase was transferred and re-extracted by repeating the phenol and chloroform steps. The aqueous phase was transferred again and 100U RNase inhibitor (RNAsin; Promega) were added. RNA was precipitated with 3 volumes 100% ethanol at -20°C overnight The RNA precipitate was recovered by centrifugation at 10000g at 4°C for 15 minutes, then washed with 100% ethanol, dried and resuspended in 600*µ*l sterile, deionised, dH₂O with 1*µ*l of fresh RNase inhibitor. RNA was aliquoted and stored at -70°C. The RNA concentration was determined spectrophotometrically. A formaldehyde RNA gel confirmed RNA integrity (Sambrook J. et al. 1989, Molecular Cloning. A laboratory manual. Cold Spring Laboratory Press, New York. 2nd Edition).

### RT-PCR

The isolated RNA was used as a template for Reverse Transcription (RT) to produce cDNA. Varying RNA concentrations were used for the RT as each RNA transcript was potentially present at different levels. Subsequent amplification of the cDNA was performed using Polymerase Chain Reaction (PCR). RT-PCR was performed using GeneAmp® RNA PCR Kit (Perkin Elmer) according to the manufacturer's protocol with the following exception-to the PCR; 35 cycles were performed as follows: Melt phase 95°C for 30 seconds, Anneal phase varied between 50-60°C for 30 seconds, Extension phase 72°C for 1 minute. Amplification was performed in a PTC-100 Programable Thermal Controller (MJ Research Inc.). As a control to demonstrate that the amplified product did not arise from contaminating DNA, Reverse Transcriptase (RTase) was omitted from a parallel tube. The PCR products were examined against DNA markers (GIBCO 1kB ladder) on a 1% agarose gel stained with ethidium bromide.

RT-PCR results are shown in Table 6 using the oligonucleotide primers as used in "Cloning, expression and purification of recombinant P. *gingivalis* genes" section described above,except for the following changes. For PG1 the 3' reverse primer used was GCGCCTCGAGATTCATTTCCTTATAGAG, for PG4 the 5' forward primer was CTTCTTGTCGACTACAGCGGACATCATAAAATC and the 3' reverse primer was TTCCACCTCGAGTTAACGCAACTCTI'CZTCGAT, for PG6 the 5' forward primer was TAAAGAATTCTGCCTCGAACCCATAATTGCTCCG, for PG10 the 5' forward primer was CGCGCATATGGATAAAGTGAGCTATGC and the 3' reverse primer was CGCGCTCGAGTTTGTTGATACTCAATAATTC, for PG13 the 5' forward primer was GCCCGGCGCCATGCGGACAAAAACTATCTTTTTTGCG and the 3' reverse primer was GCCCGGCGCCTTAGTTGTTGAATCGAATCGCTATTTGAGC. Amplification of P. *gingivalis* transcripts is a likely indication that RNA for a specific candidate is present and that the protein is produced. However, where there is no amplification achieved this does not indicate that this gene is never transcribed and may be the result of the culture conditions or the state of the cells when harvested.

**Table 6. Expression of PG m-RNA with in vitro grown P. gingivalis W50. The symbols are + band visible on agarose gel, - no band present on agarose gel, ND not detected.**

| PG # | RNA µg | Annealing temp. °C | RT-PCR | PCR (-RT) | Approx. fragment size bp. | Expected fragment size bp |
|---|---|---|---|---|---|---|
| 1 | 0.15 | 55 | + | - | 1300 | 1362 |
| 2 | 1.0 | 50 | + | - | 3200 | 3051 |
| 3 | 0.15 | 60 | + | - | 720 | 690 |
| 4 | 2.9 | 55 | - | - | N.D. | 2000 |
| 5 | 0.02 | 50 | + | - | 1000 | 947 |
| 6 | 1.0 | 55 | + | - | 1000 | 972 |
| 8A | 0.15 | 50 | + | - | 1200 | 1278 |
| 10 | 0.15 | 55 | + | - | 590 | 585 |
| 11 | 0.10 | 60 | + | - | 960 | 942 |
| 12 | 0.02 | 60 | + | - | 880 | 831 |
| 13 | 1.0 | 50 | + | - | 2150 | 2274 |
| 14 | 0.15 | 60 | + | - | 1050 | 996 |
| 22 | 1.0 | 60 | - | - | N.D. | 228 |
| 24 | 1.0 | 55 | + | + | 1150 | 1194 |
| 29 | 0.15 | 60 | + | - | 880 | 885 |

**Table 7: Immunoblot results of proteins expressed in E.coli against rabbit, rat and human antisera. Deduced MW was calculated from amino acid sequence of the P. gingivalis proteins, some of which had their N-terminal signal sequences removed. Apparent MW was determined from SDS-PAGE gels. The N- and C-terminal tags add approximately 2.5 KDa to the deduced MW of the recombinant proteins. The symbols are + positive, - negative, +/- weak positive, ND not done.**

| **Protein number** | **Deduced MW (KDa)** | **Apparent MW (KDa)** | **Antisera reactivity** | | | |
|---|---|---|---|---|---|---|
| | | | **T7** | **Rabbit** | **Rat** | **Human** |
| PG1 | 47.5 | 63 | ND | - | - | - |
| PG2 | 112.4 | 125.7 | ND | + | - | - |
| PG3 | 22.6 | 18.3 | ND | -^{a} | - | - |
| PG4 | 75 | 90.6 | ND | - | - | - |
| PG5 | 34.9 | 43.8 | ND | - | - | -, |
| PG6 | 36.7 | 47.1 | ND | - | - | - |
| PG8 | 67.5 | 63.1 | ND | - ^{b} | - | - |
| PG8A | 47.7 | 90.6 | ND | - | - | - |
| PG10 | 21.3 | 25.5 | ND | + | - | + |
| PG11 | 36.2 | 42.4 | ND | - | - | - |
| PG12 | 30.7 | 30.6 | ND | - | - | - |
| PG13 | 84.5 | 101 | ND | - | - | - |
| PG14 | 36 | 42.4 | ND | - | + | + |
| PG22 | 8.6 | 11.1 | ND | - | - | - |
| PG24A | 47 | 63.1 | ND | - | - | - |
| PG29 | 31.1 | 40.9 | ND | + | + | + |
| PG30 | 35.1 | 46.9 | + | - | - | - |
| PG31 | 16.7 | - | - | - | - | - |
| PG32 | 41.2 | 59.5 | + | + | + | - |
| PG33 | 39.9 | 52.7 | + | + | + | - |
| PG35 | 92.6 | 116.6 | + | - | - | - |
| PG36 | 98.9 | 120.2 | - | - | - | - |
| PG37 | 18.8 | 23.1 | + | + | - | - - |
| PG38 | 16.1 | 22.9 | + | - | - | - |
| PG39 | 87.9 | 116.6 | + | - | - | - |
| PG40 | 76.6 | 103.1 | + | - | - | - |
| PG41 | 48.3 | 81.1 | + | - | + | + |
| PG42 | 59.3 | 73.9 | + | - | - | - |
| PG43 | 27.1 | 50.3 | + | - | - | - |
| PG44 | 28.6 | 32.3 | + | - | + | - - |
| PG45 | 84 | 100.6 | + | - | - | - |
| PG46 | 83 | 97.7 | + | - | - | - |
| PG47 | 93.7 | 42.5 | + | + | - | + |
| PG48 | 45.2 | 37.9 | + | - | - | - |
| PG49 | 33.3 | 64.1 | + | - | + | - |
| PG50 | 91.9 | 113.2 | + | + | - | - |
| PG51 | 19.6 | 27.2 | + | - | - | - |
| PG52 | 50.4 | 64.4 | + | + | - | + |
| PG53 | 47.4 | 45.4 | + | - | - | + |
| PG54 | 101.4 | 46.7 | + | + | - - | - |
| PG55 | 70.4 | 68.4 | + | - | - | - |
| PG56 | 142.3 | - | - | - | - | - |
| PG57 | 100 . | 134.5 | + | + | + | + |
| PG58 | 63 | 82.9 | + | - | - | - |
| PG59 | 33.3 | 43.6 | + | - | - | - |
| PG60 | 55.6 | 77.8 | + | - | - | - |
| PG61 | 81.5 | 107.3 | + | - | - | - |
| PG62 | 51.9 | 58.4 | + | - | - | - - |
| PG63 | 29.6 | 43.6 | + | - | - | - |
| PG64 | 18.5 | 26.9 | + | - | - | - |
| PG65 | 25.9 | 28.8 | + | - | - | - |
| PG66 | 22.2 | 25.1 | + | + | - | - |
| PG67 | 103.7 | 105 | + | - | - | - |
| PG68 | 133.3 | 30.7 | + | - | + | + |
| PG69 | 44.4 | 50.8 | + | - | - | - |
| PG70 | 25.9 | 30.8 | + | - | - | - |
| PG71 | 88.9 | 105.5 | + | - | - | - |
| PG72 | 40.7 | 49.8 | + | - | - | - |
| PG73 | 40.7 | 29 | +/- | - | - | - |
| PG74 | 22.2 | 32.5 | + | - | - | - |
| PG75 | 40.7 | 46.7 | + | - | - | - |
| PG76 | 48.1 | 55.6 | + | - | - | + |
| PG77 | 29.6 | 36.9 | + | - | - | - |
| PG78 | 33.3 | 35.4 | + | - | - | - |
| PG79 | 33.3 | - | - | - | - | - |
| PG80 | 25.9 | 20.5 | + | - | - | - |
| PG81 | 23 | 25.8 | + | - | - | - |
| PG82 | 44.8 | 48.5 | + | - | - | - |
| PG84 | 41.7 | 52.4 | + | - | - | +/- |
| PG85 | 62.7 | 72.4 | + | - | - | - |
| PG86 | 21.7 | 27.4 | + | - | - - | +/- |
| PG87 | 83 | 91.3 | + | - | - | + |
| PG88 | 27 | 40.1 | + | - | - | - |
| PG89 | 26.2 | 29.4 | + | - | - | - |
| PG90 | 23 | 28.4 | + | - | - | - |
| PG91 | 57.2 | 85.7 | + | + | + | + |
| PG92 | 83.6 | 110.4 | + | - | - | + |
| PG93 | 83.4 | 110.4 | + | - | - | + |
| PG96 | 59.3 | 70.3 | + | + | + | + |
| PG97 | 44.4 | 57.5 | + | - | + | + |
| PG98 | 33.3 | 36 | + | - | - | - |
| PG99 | 40.7 | 55.6 | + | - | + | + |
| PG100 | 29.6 | 10.8 | + | - | - - | - |
| PG101 | 14.8 | 19.7,14.1 | + | - | - | - |
| PG102 | 59.3 | 70.3 | + | - | - | + |
| PG104 | 40.7 | 57.5 | + | - | - | + |

| | | | | | | |
|---|---|---|---|---|---|---|
| a. Positive reaction detected with the rabbit antiserum to sarkosyl insoluble *P. gingivalis* antigen. | | | | | | |
| b. Purified protein demonstrated weak positive reaction with the rabbit antiserum to whole P. *gingivalis.* | | | | | | |

It will be appreciated by persons skilled in the art that numerous variations and/or modifications may be made to the invention as shown in the specific embodiments without departing from the spirit or scope of the invention as broadly described. The present embodiments are, therefore, to be considered in all respects as illustrative and not restrictive.

### References.

1. Lipman DJ, Pearson WR. 1985. Rapid and sensitive protein similarity searches. Science 277:1435-1441.
2. Horton, P. and Nakai, K. (1996). A probabilistic classification system for predicting the cellular localization sites of proteins. Intellig. Syst. Mol. Biol. 4: 109-115.
3. Nakai K, Kanehisa M. 1991. Expert systems for predicting protein localization sites in Gram-negative bacteria. Proteins: Structure, Function, and Genetics 11:95-110.
4. Nielsen H, Engelbrecht J, Brunak S and von Heijne G. 1997. Identification of prokaryotic and eukaryotic signal peptides and prediction of their cleavage sites. Protein Engineering 10, 1-6.
5. Claros MG and G von Heijne. (1994). TopPredII: an improved software for membrane protein structure predictions. Comput. Appl. Biosci. 10: 685-686.
6. Borodovsky M, Rudd KE, and EV Koonin. (1994). Intrinsic and extrinsic approaches for detecting genes in a bacterial genome. Nucleic Acids Res. 22:4756-4767.
7. Struvye M, Moons M, Tommassen J. 1991. Carboxy-terminal phenylalanine is essential for the correct assembly of a bacterial outer membrane protein J. Mol. Biol. 218:141-148.
8. Aduse-Opoku J, Slaney JM, Rangarajan M, Muir J, Young KA, Curtis MA. 1997. The Tla receptor protein of *Porphyromonas gingivalis* W50: a homolog of the RI precursor (PrpRI) is an outer membrane receptor required for growth on low levels of hemin. J. Bacteriol. 179:4778-4788.
9. Needleman SB, Munsch CD. 1970. Ageneral method applicable to the search of similarity in the amino acid sequence of two proteins. J. Molec. Biol. 48: 443-453.

( 2) INFORMATION FOR SEQ ID NO:1

(i) SEQUENCE CHARACTERISTICS:
(A) LENGTH: 1362 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1362

(xi) SEQUENCE DESCRIPTION: SEQ ID NO:1

TTCTGTGTCA TGGCAAAAGT TATAAAAACA AAAAAAGGCC TTGCACTTAA TCTGAAAGGA 60
AAACCGCTGC CCGAGATGCT GGCCGAACCG GCCCAAAGTC CTACTTACGC GGTCGTGCCC 120
GACGATTTTG AAGGTGTTAT CCCCAAGGTG ACGGCTCGTC CGGGGGATAA GGTGCGTGCC 180
GGCTCAGCAC TGATGCACCA CAAGGCATAT CCGGAGATGA AGTTTACAAG TCCGGTTAGC 240
GGCGAAGTGA TCGCGGTGAA TCGCGGTGCC AAGCGCAAGG TGTTGAGCAT CGAGGTGAAA 300
CCGGACGGAC TGAACGAATA CGAGTCATTC CCTGTCGGGG ATCCGTCTGC CCTCTCTGCC 360
GAACAGATCA AGGAGCTTTT ACTGTCGAGC GGTATGTGGG GTTTTATTAA GCAACGTCCT 420
TACGACATAG TGGCTACACC GGATATAGCT CCACGCGACA TTTATATTAC TGCCAACTTT 480
ACTGCACCAT TGGCTCCGGA CTTCGATTTC ATCGTTCGAG GAGAAGAACG CGCCCTGCAG 540
ACTGCCATCG ATGCCTTGGC CAAACTCACG ACAGGAAAGG TGTATGTGGG CCTGAAGCCG 600
GGTTCATCTC TGGGCTTGCA CAATGCAGAA ATCGTAGAAG TACACGGACC TCATCCGGCA 660
GGTAACGTGG GCGTGCTGAT CAATCATACG AAGCCAATCA ATCGGGGCGA AACGGTGTGG 720
ACGCTCAAGG CTACCGACCT GATCGTGATC GGACGTTTCC TGCTTACGGG CAAAGCCGAT 780
TTTACCAGAA TGATTGCCAT GACCGGCTCA GACGCTGCAG CTCACGGATA CGTCCGTATT 840
ATGCCGGGTT GCAATGTCTT TGCTTCCTTC CCCGGCCGAC TGACAATAAA GGAATCTCAC 900
GAGCGTGTGA TCGATGGCAA TGTGCTGACC GGTAAGAAGC TCTGCGAGAA GGAGCCTTTC 960
CTGTCAGCCC GGTGTGACCA GATCACGGTG ATCCCCGAAG GCGACGATGT GGACGAACTC 1020
TTCGGGTGGG CTGCACCCCG TCTCGATCAG TACAGCATGA GCAGAGCTTA TTTCTCTTGG 1080
TTGCAGGGGA AAAACAAAGA GTACGTACTC GATGCCCGGA TCAAGGGTGG CGAACGTGCT 1140
ATGATCATGA GCAACGAGTA TGACCGCGTT TTCCCGATGG ACATCTATCC GGAGTATTTG 1200
CTCAAGGCTA TTATAGCATT CGACATCGAC AAGATGGAGG ACTTAGGCAT ATATGAAGTG 1260
GCTCCGGAGG ACTTTGCCAC TTGCGAATTT GTGGATACAT CCAAGATCGA GCTGCAGCGT 1320
ATCGTTCGCG AGGGCTTGGA TATGCTCTAT AAGGAAATGA AT 1362

(2) INFORMATION FOR SEQ ID NO:2

(i) SEQUENCE CHARACTERISTICS:
(A) LENGTH: 603 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...603

(xi) SEQUENCE DESCRIPTION: SEQ ID NO:2

GAACTAAGCA AGTGTTACAT GGATAAAGTG AGCTATGCTC TGGGATTGAG CATCGGTAAT 60
AATTTCAAGT CTTCGGGCAT CGACAGCGTC GTTATGGATG ATTTCATGCA AGGTCTGTCT 120
GATGTACTGG AAGAAAAAGC CCCTCAGCTC TCCTATGACG AGGCCAAGCG CGAAATAGAG 180
GCGTATTTCA TGGATTTGCA GCAGAAGGCT GTCAAACTGA ACAAAGAGGC CGGAGAAGAA 240

TTCCTCAAGA TAAATGCACA CAAGGAAGGT GTGACGACCT TACCGAGCGG CTTGCAATAC 300
GAAGTCATTA AGATGGGAGA GGGCCCGAAA CCCACCCTTT CGGACACGGT AACCTGTCAT 360
TATCACGGTA CGCTCATCAA CGGTATCGTT TTCGATAGCT CTATGGACAG GGGAGAACCG 420
GCCAGTTTCC CTCTAAGAGG AGTTATAGCC GGCTGCACGG AGATTCTTCA ATTAATGCCT 480
GTAGGATCCA AGTGGAAAGT AACTATACCG AGCGATCTGG CGTATGGAGA TCGTGGTGCC 540
GGCGAACATA TCAAACCGGG TAGTACGCTC ATTTTTATAA TCGAATTATT GAGTATCAAC 600
AAA 603

(2) INFORMATION FOR SEQ ID NO:3

(i) SEQUENCE CHARACTERISTICS:
(A) LENGTH: 837 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...837

(xi) SEQUENCE DESCRIPTION: SEQ ID NO:3

CAAAAAAACA AACGAAAGAT GAAAAAAGCA TTACTTATTG GTGCTGCTCT TTTGGGAGCA 60
GTCAGTTTTG CAAGTGCTCA GTCTTTGAGC ACAATCAAAG TACAGAACAA TTCAGTACAG 120
CAACCTCGTG AGGAAGCCAC TATTCAGGTT TGTGGAGAAT TGGCAGAGCA AGTTGACTGC 180
ATTGGGACAG GTAATTCTGC AATCATAGCC GCTGCAGCGA AATTTGAAAG CGATGATCTC 240
GAAAGCTATG TTGGCTGGGA GATCATGAGT GTTGATTTCT TCCCTGGATA TAAAGCGTGC 300
AAGTACACAT CTGCAGTCTG GGCTGATGAT ATGACCATTT TGGGCCAATC AGAAGATAGT 360
GATCCCGAAA TGCAGACTAT CAACAATCTT GCTCTCAAGA CTAGTGTCAA GATTGAAGCC 420
GGCAAGAATT ACATAGTTGG TTATATTGCT AATACCGCAG GTGGACATCC TATCGGATGT 480
GATCAGGGCC CTGCCGTTGA TGGTTATGGA GATTTGGTTT CTATATCAGA AGATGGTGGT 540
GCTACTTTCC CTCCGTTCGA ATCTCTTCAT CAAGCAGTTC CTACCTTAAA TTACAACATC 600
TATGTCGTTG TTCATTTGAA GAAGGGTGAA GGTGTTGAGG CTGTTCTTAC CAACGACAAG 660
GCTAATGCTT ATGTTCAGAA TGGCGTTATC TATGTAGCCG GAGCTAATGG TCGTCAGGTA 720
TCTCTGTTCG ACATGAACGG TAAGGTTGTT TATACCGGCG TTAGCGAAAC GATTGCAGCT 780
CCTCAGAAGG GCATGTATAT CCTCCGTGTA GGTGCTAAGA GCATCAAGCT CGCTATC 837

(2) INFORMATION FOR SEQ ID NO:4

(i) SEQUENCE CHARACTERISTICS:
(A) LENGTH: 471 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...471

(xi) SEQUENCE DESCRIPTION: SEQ ID NO:4

CGTGnATTCC TGCCGGAGAA AGCTCTCTAT ATCGGCTGCC GCGTGGAGAC GCAAGAGGGG 60
CATGCCGTAG GTTTCGGACT GGATGACGGC CCTGCGATGA AAGGCAAGGG CGATCTGGTC 120
GGGAGCTATC TTCCCGGTGC TGCTCCGATG CCTTTTGTCC CGCTTTCTGA TATTCCGGCT 180
CGTTCGATGG ATGCCAATTT TTATATCTAT TCTCGTATTT CACTGGGTTC GGGCACGCAG 240

GATGTTCTCC AGCATAGGAT GAAAGTGTAT CCCAACCCTG CCACGACGGA GCTGCATGTG 300
GAAGCCCTGT CCGCATGGGT GGGCGAGCAG GCTGCGGTAT ATGATATGCG TGGTCGTCGG 360
GTATCGGCTC GGACGGTGGA TAGCGAGAAG CTGTGCATCG ACATTGCCTC ACTGCCCGTG 420
GGCGTCTATA TGCTGCGCAT CGGCAGCTAC TCGGCCAAGT TCGAGAAGAG A 471

(2) INFORMATION FOR SEQ ID NO:5

(i) SEQUENCE CHARACTERISTICS:
(A) LENGTH: 1686 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1686

(xi) SEQUENCE DESCRIPTION: SEQ ID NO:5

ACAAGAAATG TTTCACTCAT AAAAATGCCA AGAATTATGA AATTAAAAAT TGCACTCAGA 60
CTGCTGCTGG CGACTTTTGC CATAGTTTTA TTTAGCCCTC TGGCCAAGGC CCAGATGGAT 120
ATTGGTGGAG ACGATGTATT GATCGAGACG ATGTCCACCC TATCAGGATA TTCAGAGGAT 180
TTTTATTACA AGATGGCTGT GGCAGACAAT GGATGGATCT ATGTGATGTT GGATTTCTCT 240
CGTATTTATT TTGATGATGT CAGGCTGTAT CGTTCCAAAG ACGGTGGTGC TACTTACCAA 300
AAGTTAGGGT CTTTGGGGTC TTTGGTGCCT TATGACTTCG ATGTCTCGCA TTGCGATTTT 360
ATTGTAACGG GAAAGGATGA AGATGATATC AATGTTTGGA CAGTCATGAC AGCATTCGAA 420
TATGTAGGTG GTACTATTGG CAATGGCGTT TTGCTGATGC ATCGCCATGA TGCAGATATC 480
AATAATACAG AGTGTGTGTA CAAGAAGGAT TTCCCTAATA ATAGACTGAT GGGTGTAGCC 540
ATCGCCTCCA ACTACCGTGC GCCCTCTCCT TACGGTTTGG GGGGCGATCC TTTTGCTCTC 600
GCTGTCGCCG TTAGTGGCTC CGGAAGCGAT CACAGCTTCT TGGACTATAT TTTTTCGTTA 660
GATGCTGGAG TACACTTTGA GCAAAAGCGT ATTTACACAA GACCCCAAAA ACTGACTATC 720
AATAGAGTAG ACCTTTCATT AGGCAGTACA TCTCCTTCTC TTGGATTTAA TACTTGGCCA 780
CTAATGGGAG TCGTATTCGA AATGAATAAG AACCTTGATG GCTTCGACAT TGGTTTCATT 840
TCCAACTTTG TGGACTATGA TCCCCGCTAT GCGTGGTCTG AACCGATAAT AATAGAAGAA 900
GACTGTGGAT GGACTGATTT TAATCCTTTG GGAGCACTAA GTATAGAGAT CCAAATGATG 960
TTGGATGACA ATTCGGATAA TACCGTGGGT GGAGAACGCT CCCATAACTT CCTGATCACT 1020
TACCCGGGCC ATTACGTATA TCCGAAGCAA TCTTTCAATT ATTCTCCCGG ACATACACCG 1080
ACAAAGAAAG ATCTGGTCTT TAAACACTGT ATAGGTATTC CGGCTTTGGC ATACGATAAG 1140
GAAGGCGATC GTTATCTGAC TACTTTTCAA GATCACAATC TAATGAGATA CAGATGGATC 1200
AAATACGATG ACATTAACTC TTTTTATGGT TGGAGTTGGC CATATGTATA TGCAAAAGAA 1260
GCTAAAGATA AAAAGAGGCG CCGTCCGCAA GTAGCACTCA ATCCTACCAA TGGAAAGGCT 1320
TGTTGGGTAT GGCATACTCG CAAGAGCCCA TATGATGAAA CCAAACCACA TCCTACTCCT 1380
GTAATTATTA AACATTTCCT ATGGTCCGAT ACGGAGTGGG TACATGCTCT GGACGTGGGG 1440
GACGTATTGC AGAAGGAGGG TAGCATGAAG CTCTACCCCA ATCCTGCCAA AGAATATGTT 1500
CTGATCAACC TACCCAAAGA AGGGGGGCAC GAGGCAGTCG TATACGACAT GCAGGGCCGA 1560
ATCGTGGAGA AAGTTTCATT TTCAGGGAAA GAATATAAGC TGAATGTGCA GTATCTGTCC 1620
AAAGGTACGT ACATGCTGAA AGTTGTAGCG GATACGGAGT ATTTCGTGGA AAAAATCATT 1680
GTAGAG 1686

(2) INFORMATION FOR SEQ ID NO:6

(i) SEQUENCE CHARACTERISTICS:
(A) LENGTH: 1173 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: UNKNOWN

(vi) ORIGINAL SOURCE:

(A) ORGANISM: PORYPHYROMONAS GINGIVALIS

(ix) FEATURE:
(A) NAME/KEY: misc_feature
(B) LOCATION 1...1173

(xi) SEQUENCE DESCRIPTION: SEQ ID NO:6

CAAATGAAAC GATTACTCCC CTTTCTCCTT TTAGCAGGAC TCGTAGCCGT AGGAAACGTG 60
TCTGCTCAGT CACCCCGAAT CCCTCAAGTG GATGTACACA CTCGCATCGC AAGAAATGCC 120
CGTTATCGAC TGGACAAGAT CAGTGTCCCG GATTCTCGTC AGATATTCGA TTACTTCTAT 180
AAAGAAGAAA CGATACCCAC TAAAATACAA ACGACCACAG GAGGTGCAAT TACAAGCATC 240
GATTCGCTTT TCTATGAAGA CGACAGGTTG GTTCAGGTGC GCTATTTTGA CAATAACCTT 300
GAATTAAAAC AAGCGGAGAA GTATGTATAC GACGGTTCTA AGCTGGTCCT TCGAGAAATT 360
CGCAAGTCGC CGACAGACGA AACGCCAATA AAGAAAGTTA GCTATCACTA TCTCTGTGGC 420
AGCGATATGC CTTTTGAGAT TACGACAGAG ATGAGCGATG GCTATTTTGA AAGCCATACG 480
CTTAACTATC TGAATGGAAA GATTGCCCGA ATAGATATCA TGACTCAACA GAACCCATCG 540
GCCGAATTGA TCGAAACGGG TAGAATGGTA TATGAGTTTG ATGCCAATAA TGATGCTGTA 600
CTGCTTCGTG ACAGTGTATT TCTTCCTCTT CAAAACAAGT GGGTAGAAAT GTTTACTCAC 660
CGTTATACAT ACGACAATAA GCATAATTGT ATTCGTTGGG AACAAGACGA ATTCGGCACC 720
CTCACCCTTG CCAACAACTT CGAATACGAC ACCACTATCC CTCTGTCGTC TGTATTGTTC 780
CCCACGCATG AGGAGTTCTT CCGTCCTCTT CTTCCCAATT TTATGAAGCA TATGCGTACG 840
AAGCAAACGT ATTTCAATAA CTCCGGAGAA GGCTTGTCAG AGGTATGCGA TTACAACTAC 900
TTCTATACCG ATATGCAGGG TAATGCACTG ACCGATGTTG CCGTGAACGA ATCGATCAAG 960
ATTTATCCTC GTCCTGCCAC GGATTTTCTG CGTATAGAAG GTTCGCAACT GCTTCGCCTT 1020
TCGCTATTCG ACATGAACGG GAAGCTCATC AGAGCTACCG AATTGACAGG CGATTTGGCC 1080
ATTATCGGAG TTGCATCTCT TCCGAGAGGC ACTTACATCG CAGAAATAAC TGCTGCAAAC 1140
AGCAAAACCA TACGTGCAAA AGTATCGCTC AGA 1173

(2) INFORMATION FOR SEQ ID NO:7

(i) SEQUENCE CHARACTERISTICS:
(A) LENGTH: 1284 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1284

(xi) SEQUENCE DESCRIPTION: SEQ ID NO:7

AAGAGGAATC CCTTACCTTT GACGGCATCA AATCGAAAGA TTTTTATAAA AATGAGACAG 60
CATTTATCTC TATTTCCTTT TATCTTGTTT CTGCTTCTTG CCTTCTCTTA TGTCGGTTGC 120
AGAACAGTCC GACAAACACC TAAGCAGTCG GAACGGTACG TCGTAGTCCT GTCTTTGGAC 180
GGCTTCCGAC CGGACTATAC CGATCGGGCA CGTACACCGG CGTTGGATCG GATGGCACAG 240
GAGGGATTGA GCGGGTCGCT CCAACCATGC TTCCCCTCGC TTACATTTCC CAATCATTAC 300
AGCATGGCTA CGGGGCTTTA CCCCGATCAT CACGGTATCG TAGCCAATGA GTTTGTGGAT 360
TCGCTACTGG GCATCTTTCG TATATCCGAC CGAAAAGCCG TGGAGACCCC CGGATTTTGG 420
GGCGGCGAGC CGGTTTGGAA TACGGCCGCA CGCCAAGGCA TCCGTACCGG TGTCTACTTT 480
TGGGTAGGAT CCGAAACGGC TGTGAACGGA AATCGGCCGT GGCGGTGGAA AAAATTCTCC 540
TCCACCGTTC CGTTTCGTGA CCGTGCCGAC TCCGTCATCG CGTGGCTCGG ACTGCCCGAA 600
AAGGAGCGAC CGCGCTTGCT CATGTGGTAC ATCGAGGAGC CGGATATGAT CGGACACAGC 660
CAAACGCCCG AAAGCCCGCT GACACTGGCA ATGGTAGAGC GGTTGGACAG TGTGGTCGGC 720
TATTTCCGCA AGCGGTTGGA CTCTCTGCCC ATAGCCGCAC AGACCGACTT CATCATAGTA 780
TCCGATCACG GTATGGCCAC GTACGAAAAT GAGAAATGTG TCAATCTGTC GCATTATCTG 840
CCTGCGGACA GTTTCCTCTA CATGGCCACC GGGGCCTTCA CCCACTTGTA CCCGAAGCCC 900
TCCTATACCG AGCGAGCCTA TGAGATCCTG CGGGCCATTC CACATATATC GGTTTACCGC 960
AAGGGGGAGG TGCCCAAGCG TTTGCGCTGT GGCACCAATC CTCGTTTGGG CGAACTGGTC 1020
GTGATTCCGG ACATAGGCTC CACCGTCTTT TTCGCAATAA ATGAAGACGT TCGTCCGGGA 1080
GCGGCACATG GCTATGACAA CCAAGCACCG GAAATGCGGG CTTTACTCCG GGCTGTCGGA 1140
CCCGATTTCC GTCCGGGCAG TAGGGTGGAA AACCTGCCGA ATATCACCAT CTATCCGCTC 1200
ATATGCAGGC TGTTGGGTAT AGAGCCTGCA CCCAACGATG CGGACGAAAC GTTGCTGAAC 1260
GGCCTGATCC GAGACAAACG ACCA 1284

(2) INFORMATION FOR SEQ ID NO:8

(i) SEQUENCE CHARACTERISTICS:
(A) LENGTH: 846 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...846

(xi) SEQUENCE DESCRIPTION: SEQ ID NO:8

CTTTCCCGTG GAGTTTTTCC CCTGATGTAT GGCCGAAGAG GAAGCATTCG TGCCTCTTCA 60
GGGCATAGGG ACAAAATTTT TAAGAATACA ATTATCAGAT TTATCACAAT GAAAGTAGGT 120
TTGTTCATCC CCTGTTATGT CAATGCAGTG TATCCGGAAG TGGGTATCGC CACGTACAAA 180
CTGCTGAAGA GTTTGGACAT AGATGTCGAC TACCCGATGG ATCAGACATG TTGCGGCCAG 240
CCTATGCCCA ATGCCGGATT CGAACAGAAA GCTCAAAAGC TGGCTTTGCG ATTCGAAGAG 300
CTGTTCGAGT CGTATGATGT AGTCGTAGGG CCATCGGCCA GTTGCGTTGC TTTCGTGAAA 360
GAAAACTATG ATCATATCCT CAGACCGACA GGACATGTCT GCAAGTCGGC AGCCAAGCTT 420
CGGGATATAT GCGAGTTCTT GCACGATGAC CTGAAGATCA CCAGCCTCCC CTCCCGATTC 480
GCCCATAAGG TGAGCCTGCA CAACAGTTGC CACGGTGTGC GCGAACTGCA TCTGTCCACC 540
CCCAGTGAAG TGCACCGACC GTACCACAAC AAGGTGCGCC GGCTATTGGA GATGGTGCAG 600
GGCATAGAGG TATTCGAGCC GAAGCGAATA GACGAATGCT GCGGTTTCGG CGGTATGTAC 660
TCGGTGGAGG AGCCGGAGGT ATCCACCTGT ATGGGGCATG ACAAGGTGCT GGATCACATA 720
TCCACAGGTG CGGAGTACAT CACAGGGCCG GACAGCTCGT GCCTCATGCA TATGCAGGGA 780
GTGATAGACA GAGAGAAATT GCCGATCAAG ACAATTCATG CAGTAGAAAT TTTAGCAGCA 840
AACTTA 846

(2) INFORMATION FOR SEQ ID NO:9

(i) SEQUENCE CHARACTERISTICS:
(A) LENGTH: 753 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...753

(xi) SEQUENCE DESCRIPTION: SEQ ID NO:9

CCTCTGAAAA AACGAATGGA TATTGTAAGT ATGGCCGATA AAGCTCTTGT ACTGGAGATG 60
AGAGATGTGA CGCTCTGTCA GGAGGAAAAC GTCATTTTTC AAAATTTGAA TCTGACCCTT 120
TCCGCCGGAG ACTTCGTCTA TCTGATAGGC TCAGTGGGAT CGGGGAAGAG CACTTTGCTG 180
AAGGCTTTGT ATGCTGAGGT GCCTATCTCT GCCGGTTATG CCCGCGTGAT AGATTATGAT 240
CTGGCAAAGT TGAAACGGAA GCAGTTGCCC TATCTGCGCA GGAATTTGGG CATTGTGTTT 300
CAGGATTTCC AGTTGCTGAA CGGACGTACT GTTGCGGAGA ATTTGGATTT CGTTTTGCGA 360
GCTACGGACT GGAAAAACCG AGCCGATCGC GAGCAGCGTA TCGAGGAGGT TTTGACCCGT 420
GTGGGAATGT CTCGGAAGGC TTATAAGAGA CCGCACGAAC TGTCCGGAGG GGAGCAACAA 480
CGTGTGGGTA TAGCCAGAGC TTTGCTGGCG AAGCCTGCGT TGATCCTGGC CGACGAACCC 540
ACAGGCAACC TCGATTCGGT GACCGGATTG CAGATCGCTT CTCTGCTCTA CGAAATCAGT 600

AAGCAGGGCA CTGCAGTACT TATGAGCACG CACAACAGCA GCCTGCTGTC GCATCTGCCG 660
GCACGGACAT TGGCCGTTCG TAAGAATGGC GATGCCTCCT CTTTGGTCGA GCTGAGTGCA 720
GATGCTGTTT CAAGAAAAAA TACGGAAATA GAT 753

(2) INFORMATION FOR SEQ ID NO:10

(i) SEQUENCE CHARACTERISTICS:
(A) LENGTH: 714 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...714

(xi) SEQUENCE DESCRIPTION: SEQ ID NO:10

ACCAGGCATT GTCCGGCTTG TCGCTCTTCC TTTCACCTCA TAAAAACAAG TAAAACAATG 60
ATTGAAATCA GCAACCTCAC CAAGGTTTTC AGAACAGAAG AAATAGAGAC GGTAGCCCTC 120
GATGGCGTAT CGCTCAAAGT GGACAAAGGC GAATTTATCG CCATAATGGG GCCTTCGGGA 180
TGCGGTAAGT CCACTCTGCT CAATATCCTC GGCCTTCTCG ACAATCCCAC TTCCGGTATC 240
TACAAGCTCG ATGGGGCAGA AGTGGGCAAC CTCCGGGAAA AAGACAGGAC TGCCGTCCGT 300
AAGGGCAATA TCGGCTTCGT ATTCCAGAGC TTCAACCTCA TCGAAGAGAT GACGGTAAGC 360
GAGAACGTGG AGTTGCCGCT CGTCTATCTG GGTGTGAAGG CCTCCGAGCG GAAAGAGCGA 420
GTGGAGGAGG CACTGCGCAA GATGAGCATC AGCCACCGGG CCGGCCACTT CCCCAATCAG 480
CTCTCCGGAG GACAACAGCA GCGCGTGGCT ATCGCCCGTG CCGTGGTGGC CAATCCGAAG 540
CTCATCCTCG CCGATGAACC CACGGGTAAC CTCGACTCCA AAAACGGAGC CGATGTCATG 600
GAACTGCTCA GAGGTCTCAA TCGCGAAGGT GCAACCATCG TCATGGTGAC GCACTCCGAG 660
CACGATGCAC GTAGTGCCGG CCGCATCATC AATCTGTTCG ACGGTAAGAT TCGC 714

(2) INFORMATION FOR SEQ ID NO:11

(i) SEQUENCE CHARACTERISTICS:
(A) LENGTH: 1812 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1812

(xi) SEQUENCE DESCRIPTION: SEQ ID NO:11

AGCACAGAAA CAAATAGCAA ATCTGAAATG AAAGAATTTT TCAAAATGTT TTTCGCCTCG 60
ATCCTCGGGG TTATAACGGC AGGAATCATC TTGTTCTGTA TCTTTCTATT TATCTTTTTC 120
GGCATCGTAG CCGGTATTGC CTCCAAGGCA ACGGGAGGAA CCATTCCGAA GATCGAAGCA 180
AACTCCATCC TACATATANA CAATTCTTCT TTCCCTGAGA TCGTATCGGC CAATCCCTGG 240
AGCATGCTCA CAGGCAAAGA CGAGTCCGTA TCGCTCTCAC AGGCAGTCGA AGCCATCGGC 300
CAAGCCAAAA ATAATCCCAA CATAACCGGT ATCTTCCTCG ATCTGGACAA CCTTTCCGTC 360
GGTATGGCAT CGGCAGAGGA ATTGCGTCGC GCGTTGCAGG ATTTCAAGAT GTCGGGCAAG 420
TTCGTCGTAT CCTATGCCGA CAGATACACC CAAAAGGGTT ACTACCTCTC CAGTATTGCA 480
GACAAACTCT ACCTCAATCC GAAAGGAATG TTGGGGCTTA TCGGGATTGC GACCCAAACA 540
ATGTTCTACA AAGATGCCCT CGACAAATTC GGCGTGAAGA TGGAGATCTT CAAGGTAGGC 600

ACCTACAAGG CAGCCGTAGA GCCATTCATG CTCAACAGGA TGAGCGATGC CAATCGCGAA 660
CAAATCACCA CATACATAAA CGGGCTTTGG GACAAGATCA CATCCGATAT TGCAGAGTCG 720
CGCAAGACGG CAATGGATTC CGTGAAAATG TTTGCCGACA AAGGCGAAAT GTTCGGTCTT 780
GCCGAGAAAG CGGTGGAGAT GAAGCTCGTG GATGAGCTGG CTTACCGTAC CGATGTGGAG 840
AAAGAACTCA AAAAGATGTC CCAACGCGGA GAGAAAGATG AACTTCGGTT CGTATCGCTT 900
TCTCAGGTTC TGGCCAATGG CCCGATGAAC AAAACGAAAG GCAGTCGGAT CGCCGTTCTC 960
TTTGCCGAAG GTGAAATAAC GGAAGAAATA ATAAAGAAGC CGTTCGACAC TGACGGTAGC 1020
TCCATCACAC AAGAACTCGC CAAAGAAATC AAGGCAGCAG CCGATGACGA TGATATCAAA 1080
GCCGTAGTAC TTCGTGTCAA TTCTCCGGGA GGTAGTGCTT TCACTTCCGA ACAGATATGG 1140
AAGCAGGTAG CCGATCTCAA GGCCAAAAAG CCTATCGTGG TCTCCATGGG CGACGTAGCA 1200
GCCTCGGGCG GATACTACAT AGCCTGCGCA GCCAACAGTA TCGTGGCAGA GCATACGACT 1260
CTGACCGGCT CCATCGGCAT ATTCGGCATG TTCCCGAACT TCGCGGGCGT AGCCAAGAAG 1320
ATAGGAGTGA ATATGGACGT CGTACAGACA TCCAAGTATG CAGACTTGGG CAACACCTTC 1380
GCTCCGATGA CGGTCGAAGA TCGTGCCCTC ATCCAACGCT ACATAGAGCA GGGCTACGAC 1440
CTCTTCCTCA CTCGCGTATC GGAAGGCCGC AACCGCACCA AGGCACAGAT CGACAGCATC 1500
GCTCAAGGCC GTGTATGGCT CGGCGACAAA GCTCTTGCAC TCGGTTTGGT GGATGAGCTT 1560
GGAGGTTTGG ACACAGCTAT CAAACGGGCC GCGAAGCTGG CTCAGCTCGG TGGCAACTAC 1620
AGCATAGAGT ATGGCAAGAC CAAGCGCAAC TTCTTCGAAG AGTTGCTCTC CTCATCAGCA 1680
GCGGATATGA AGTCTGCCAT CCTGAGTACC ATTCTCTCCG ATCCGGAAAT AGAAGTTCTG 1740
CGCGAACTCC GCTCCATGCC GCCCCGTCCT TCGGGCATAC AGGCACGTCT CCCCTATTAC 1800
TTCATGCCGT AC 1812

(2) INFORMATION FOR SEQ ID NO:12

(i) SEQUENCE CHARACTERISTICS:
(A) LENGTH: 972 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...972

(xi) SEQUENCE DESCRIPTION: SEQ ID NO:12

CTAACGTTGT TTTGTTGCAA CTATTTCAAA CAGATGAGAG CAAACATTTG GCAGATACTT 60
TCCGTTTCGG TTCTCTTTTT CTTCGGGACA GCGATCGGAC AGGCTCAGAG TCGAAACCGT 120
ACATACGAGG CTTATGTGAA ACAGTACGCC GACGAAGCTA TCCGACAGAT GAGCCGCTAC 180
AATATACCGG CAAGCATCAC CATAGCACAG GCTTTGGTGG AGACAGGAGC CGGAGCCAGT 240
ACACTGGCCA GCGTACACAA CAATCACTTC GGGATCAAAT GCCACAAATC GTGGACGGGC 300
AAGCGCACCT ATCGTACCGA CGATGCGCCG AACGAATGCT TCCGCAGCTA TTCGGCCGCT 360
CGCGAATCGT ATGAAGATCA TTCCCGATTT CTGCTCCAAC CACGCTATCG TCCCCTGTTC 420
AAACTCGACA GAGAAGACTA TCGGGGCTGG GCTACGGGGT TGCAACGCTG TGGCTATGCC 480
ACCAATCGGG GCTATGCCAA TCTGCTGATC AAGATGGTGG AGCTGTATGA GCTATATGCT 540
TTGGATCGCG AGAAGTACCC CTCATGGTTC CACAAGTCTT ACCCCGGGTC CAACAAAAAA 600
TCCCATCAAA CGACCAAGCA GAAGCAGAGC GGACTCAAGC ACGAAGCTTA CTTCAGCTAC 660
GGACTGCTCT ACATCATAGC CAAGCAAGGC GATACCTTCG ATTCTTTGGC CGAAGAGTTC 720
GACATGAGAG CCTCCAAACT GGCCAAATAC AACGATGCTC CCGTGGATTT CCCGATCGAA 780
AAGGGCGATG TGATCTATCT GGAGAAAAAG CACGCATGCT CCATCTCCAA ACACACACAG 840
CACGTAGTGC GTGTGGGCGA TTCGATGCAC AGTATCTCCC AACGCTATGG CATCCGGATG 900
AAGAACCTCT ACAAGCTCAA CGACAAGGAT GGCGAATATA TACCCCAAGA GGGCGATATA 960
CTGCGCTTGC GC 972

(2) INFORMATION FOR SEQ ID NO:13

(i) SEQUENCE CHARACTERISTICS:
(A) LENGTH: 1599 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1599

(xi) SEQUENCE DESCRIPTION: SEQ ID NO:13

AGGATCCCCG ACGAGCAGAC CGGACGTATC ATGGACGGAC GTCGATATTC GGATGGCCTC 60
CATCAGGCTA TCGAAGCCAA AGAGCATGTG AAAGTAGAGG CTGCGACACA GACATTTGCA 120
ACTATCACTT TGCAGAACTA TTTCCGCATG TATCATAAGC TGGCAGGGAT GACCGGTACT 180
GCTGAAACTG AAGCGGGAGA GCTTTGGGAC ATCTACAAAC TGGACGTTGT AGTTATTCCG 240
ACAAACAAGC CTATCGCCCG TAAGGATATG AATGATCGTA TCTATAAGAC GGCACGTGAA 300
AAATATGCAG CAGTTATCGA AGAGATTGTA CGTCTTGTCG AAGAGGGCAG ACCTGTACTT 360
GTCGGTACTA CTTCGGTGGA AATATCCGAA TTGTTGAGCC GTATGTTACG CTTGCGTGGC 420
ATCCAACACA ATGTACTCAA TGCCAAATTG CATCAGAAGG AGGCCGAGAT TGTAGCTCAG 480
GCCGGTCAGA AAGGAACTGT TACCATCGCA ACGAACATGG CCGGTCGTGG TACCGACATC 540
AAGCTCTCTG CCGAGGTTAA GAAAGCCGGG GGTTTGGCTA TCATTGGTAC GGAAAGGCAC 600
GAATCCAGAC GAGTGGACAG ACAGCTTCGT GGTCGTTCCG GCCGTCAGGG TGATCCCGGT 660
TCGTCCATAT TCTATGTTTC CCTTGAAGAT CATCTGATGC GCCTCTTTGC CACAGAAAAG 720
ATTGCATCAT TGATGGATCG TTTAGGTTTC AAGGAAGGAG AAGTGCTCGA AAACAACATG 780
CTGAGTAAGT CCGTGGAGCG TGCTCAAAAG AAGGTGGAAG AGAACAACTT CGGTATCCGT 840
AAACATCTGC TTGAGTACGA TGATGTAATG AATTCGCAGC GTGAAGTCAT TTATACCCGT 900
CGCCGTCATG CTTTGATGGG AGAGCGTATC GGTATGGATG TACTCAATAC CATATACGAC 960
GTATGTAAGG CTCTGATTGA CAATTATGCA GAAGCCAATG ATTTCGAAGG CTTCAAGGAA 1020
GATCTGATGC GTGCACTCGC GATAGAATCT CCTATCACGC AAGAAATATT CAGAGGTAAG 1080
AAAGCAGAAG AGCTGACCGA TATGCTTTTC GATGAAGCTT ACAAGTCTTT CCAACGTAAG 1140
ATGGATCTGA TCGCAGAAGT GGCCCACCCT GTGCTTCATC AGGTATTCGA GACCCAAGCC 1200
GCCGTGTACG AGCGCATTCT AATCCCCATT ACGGATGGTA AACGTGTCTA TAACATAGGA 1260
TGCAATTTGC GTGAAGCGGA TGAAACTCAA GGGAAAAGCA TCATCAAAGA ATTTGAGAAA 1320
GCTATCGTAC TGCATACTAT CGATGAGTCT TGGAAAGAAC ATCTGCGTGA GATGGACGAG 1380
CTTCGTAATT CCGTTCAGAA TGCCAGCTAC GAAAACAAAG ATCCACTACT TATCTATAAA 1440
CTCGAATCTT ACGAACTGTT CCGCAAGATG GTAGAAGCCA TGAACCGTAA GACCGTAGCG 1500
ATCCTAATGC GTGCTCGGAT ACCGGTACCG GAGGCTCCTT CCCAAGAAGA GCTGGAACAC 1560
AGGCGGCAAA TAGAAATCCG ACATGCAACC CAACAACGT 1599

(2) INFORMATION FOR SEQ ID NO:14

(i) SEQUENCE CHARACTERISTICS:
(A) LENGTH: 2160 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2160

(xi) SEQUENCE DESCRIPTION: SEQ ID NO:14

AAATCTTGCC GTGTTATTGG CCAGACGAGG CGATACGGAT GCTGCCCTGT CGGACTACGA 60
CCGTGCCATC AAAGCCTATC CGGAGTTTGC CGATGCCTAT TTCAATCGCG GCCTGCTGTT 120
GCTTTCGCGC GGAAAGGCCA AAGAAGGCAT CGCCGATCTG AGTCGGGCAG GCGAATACGG 180
GCTCTACAAG GCGTACAACA TCATCAAACG AATGAGCACG AAGTCATGAT CTCCGTCAAT 240
AACCTGACTG TCGATTTCGG CACCCGTCTG CTCTTCGATC AGGTATCATT CGTCATCAAC 300
AGGCGCGACC GTATCGCTCT TGTAGGGAAG AACGGTGCCG GCAAGAGTAC GCTGCTCAAG 360
CTGATTGCCG GCATGGAAGA ACCGACATCC GGACACATAG CACGCCCCAA GGGGATCCGC 420
ATAGGCTATC TGCCGCAGGT GATGCGTTTG CAGGACGGAC ACACGGTTTA CGAAGAGGTC 480
GAGCAGGCTT TCAACGATAT TCGCCAAATA GAGGAAGAGA TACGGCGTCT GTCCGATGAA 540
ATGGCCGGAC GTACGGACTA CGAATCGGAT GACTATATCC GACTGATAGA GCATTATACG 600

AATATGAGCG AGACCCTCTC TCTCATGCAG CAGGGCAACT ATCATGCTGC GATCGAACAG 660
ACATTGATCG GTCTGGGCTT CGGCCGAGAG GACTTCCACC GCCCCACAGC CGATTTCAGC 720
GGAGGATGGC GTATGCGGAT AGAGCTGGCC AAACTTCTGC TCCAACGCCC CGAAGTTTTG 780
CTGCTCGACG AGCCGACCAA TCACCTCGAC ATCGAATCCA TCGGCTGGCT GGAGCAGTTC 840
ATCGCCACCA ATGCAGGAGC CGTTATCCTG GTGTCGCACG ACAGGGCATT CATCGACAAT 900
ACCACGACAC GCACTATCGA AATAGAACTG GGACATATAT ACGACTACAA GACCAACTAC 960
AGCCACTATG TGGAGCTGCG CGAAGAGCGG CTGCGACAGC AGATGCGTGC CTACGAGAAT 1020
CAGCAGAAGA TGATCCGCGA TACGGAGGAC TTCATCGAAC GATTCAGATA CAAGGCCACG 1080
AAGTCCGTAC AGGTACAGAG CCGGATCAAA CAGTTGGAGA AAGTAGAGCG CGTGGAGATA 1140
GACGAGCGGG ATCGTTCGGC ATTTCACTTC CGCTTTATCC CGGCACAGCC TTCCGGCAGT 1200
TATCCGCTAA TAGTGGATGA TTTGGCCAAG GCTTATGGCG ATCACCAGGT GTTTTCCGGA 1260
GCTACATACA CCATCGAAAG AGGCGAAAAG GTGGCTTTCG TAGGCAAAAA CGGTGCCGGC 1320
AAAAGTACCA TGGTCAAGTG TATCATGGGA GAGCTGACAG ACTACACCGG CAAGCTCGAA 1380
CTGGGGCACA ACGTGCAGCT GGGCTACTTT GCCCAAAACG AAGCCCAAGA GCTAAGAGGG 1440
GATCTCACGG TATTCGACAC GATAGACCGT GAGGCCGTGG GCGACATCCG TCTGCGCCTG 1500
AACGATTTGC TCGGGGCTTT TCTCTTCGGG GGCGAAGCAT CGGAAAAGAA AGTAAGTGTC 1560
CTGAGTGGAG GAGAACGAGC ACGATTGGCT ATTATCAGGC TTTTGCTACA GCCGGCTAAC 1620
TTCCTTATTC TCGATGAGCC GACCAATCAC CTCGATATGC GCTCGAAGGA TGTACTGAAA 1680
GAGGCGATCA AGAACTTCGA TGGGACTGTC ATCGTAGTAT CTCACGACCG TGAGTTCCTC 1740
GATGGGCTTG TCAGCAAGGT GTATGAATTT GCAGATGGAC AGGTGAACGA ACACCTCGGA 1800
GGTATATACG ACTATCTCCG GACCCGCCGT ATGCAGACGC TGACAGAGCT GGAGCGAACC 1860
ACTACGATCG AAACAAAAAC CACACGGGAG GCTATACCTG AAACGGAAGC CAAAGCGGAC 1920
TACCGTCGGC AAAAGGAGGT AGCCAAACAG CTGCGCACGT TGGAGCGAAC CGTAGCAACC 1980
TGCGAGGAGC GGATCGGAAA ATTGGAGTCG GAATTACAGG CAATAGAGAT GCTACTGCAA 2040
GATCCGAAAC ATGCGACTGA CGCGAATCTG TTCGAGCGAT ACGCCGGCAT GAAACAAGAA 2100
CTCGAAAAGG CCATGGAGGA CTGGGAACAG GCTTCCGAAG CTTTATCCGA AGCCCAAGGA 2160

(2) INFORMATION FOR SEQ ID NO:15

(i) SEQUENCE CHARACTERISTICS:
(A) LENGTH: 1158 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1158

(xi) SEQUENCE DESCRIPTION: SEQ ID NO:15

TATTTGAAGC TATTAATACT ACAAATAGCC TTGATGAATT TCTTAAAAAA AGAACCGTTT 60
AAAATATTCT CTATGATTTA TCTGCTGTTA GATACAATAA CAAACCGTGC CGGTACAGAA 120
CGCGCCGTGA TCAACTTGGC TAACAACCTG CATGCCAATG GTCATCGCGT ATCATTAGTC 180
AGCGTTTGTA CAAAAGAAGG AGAGCCTTCC TTCCAAGTAG AAAAAGGAAT AGAAGTACAC 240
CATCTCGGAA TTAGGCTTTA TGGCAATGCA TTAGCCCGCA AAACAGTATA TTTCAAGGCT 300
TATCGAAGGA TAAAAGCCCT ATACAAGAAG CGTGAACCGG TTTTATTGAT AGGGACTAAT 360
ATTTTTATCA ATACAATTTT GTCTCAGATC AGTAACAGAG GCAGAATATT TACGATCGGA 420
TGCGAACATA TCTCTTATGA TATTGCCCGC CCTATTACAA AACGCATAAG GGGGTTTCTG 480
TATTCAGGGC TTGATGCCGT TGTAGCACTG ACAAAAAGAG ATCAGCAATC GTTCGAGGCA 540
ATCTTACGTG GACGCTCTAA AGCATATGTC ATACCCAATC AAGTTTCATT TACTACAGTC 600
CAAAGAGATG CTACTACTCA CAAACAAATG TTGGCGATTG GCAGGCTTAC CTACCAGAAG 660
GGTTTTGAAT TCATGATAGA AGATGCATCA CGAGTGCTGC GAGAAAGGCC TGATTGGAAG 720
CTTATCATAG TCGGAGATGG CGAAAATGAA TCGATGCTAC GTAAAGAAAT TGCATCTCGC 780
AATATGGAGT CGCAAATAGA AATACATCCA TCTACACCGG AAATTCGCAA ATACTACGAA 840
TCATCTGCTA TTTATCTAAT GACGTCCCGT TTCGAAGGAC TACCAATGGT ACTTCTCGAA 900
GCAGAAGCAT ATGCACTACC TATAATCTCA TACGATTGTC CGACCGGCCC GAGGGAACTG 960
ATCGAAAACG GTCGCAATGG TTTCCTTGTG CCAATGGAAG CACATGAAGA CTTCGCGGAT 1020
AAGTTACGCT TATTGATGGA TGATGAAACT CTTCGTAAGA AAATGGGACA AGAATCAGAG 1080
TTGATGGTCA AATCCTACTC TCCGGCAAAT ATCTATGAAT GTTGGAAGAA ACTATTCGTC 1140
GAAATCGGCT ACATGAAT 1158

(2) INFORMATION FOR SEQ ID NO:16

(i) SEQUENCE CHARACTERISTICS:
(A) LENGTH: 1965 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1965

(xi) SEQUENCE DESCRIPTION: SEQ ID NO:16

ACAACGAAAG AAAACGAGAC AACAACAAAA AACGAATATA GAATTATGGG AAAAATCATT 60
GGAATTGACT TAGGCACAAC GAACTCTTGT GTCTCTGTAT TGGAAGGTAA CGAACCTATC 120
GTTATTACAA ACAGTGAGGG CAAGCGCACA ACGCCCTCGG TAGTGGCTTT TGTGGATGGT 180
GGCGAGCGTA AGGTGGGCGA TCCGGCCAAG CGTCAGGCCA TCACCAATCC GACCAAGACG 240
ATATACTCTA TCAAACGCTT CATGGGCGAA ACTTACGATC AGGTTTCCAG AGAAGTGGAG 300
AGAGTGCCAT TCAAGGTAGT ACGTGGGGAC AATAATACTC CGCGCGTAGA TATAGACGGT 360
CGTCTCTATA CGCCGCAGGA AATTTCGGCC ATGATCCTTC AGAAGATGAA GAAGACGGCC 420
GAAGACTACC TCGGTCAGGA AGTAACGGAG GCCGTGATCA CTGTGCCCGC ATACTTCAAC 480
GACGCTCAAC GTCAGGCAAC GAAAGAAGCA GGAGAGATCG CCGGCCTGAA AGTTCGCCGT 540
ATTGTGAACG AGCCTACGGC AGCTTCTCTG GCCTACGGTC TGGACAAGTC CAATAAGGAT 600
ATGAAGATCG CTGTCTTCGA CTTGGGTGGC GGTACCTTCG ATATCTCTAT CTTGGAATTG 660
GGCGACGGCG TTTTCGAAGT GAAATCGACC AACGGTGATA CGCACCTCGG AGGAGACGAC 720
TTCGACCACG TGATCATTGA CTGGCTGGCA GAAGAGTTCA AGTCTCAGGA AGGTGTGGAT 780
CTTCGCCAGG ATCCTATGGC TATGCAGCGT CTGAAAGAAG CTGCCGAAAA AGCCAAGATA 840
GAGCTCTCCA GCACTTCATC TACGGAGATC AACCTCCCCT ATATCATGCC GGTGAACGGC 900
ATCCCCAAGC ACTTGGTGAT GACGCTTACA AGGGCTAAGT TCGAGCAGTT GGCCGATCGT 960
CTGATTCAGG CATGTGTGGC ACCCTGCGAA ACGGCCTTGA AAGATGCCGG TATGTCACGT 1020
GGCGATATCG ATGAAGTGAT TCTCGTAGGT GGTTCCACAC GTATTCCTGC TATTCAGGAG 1080
ATTGTGGAGA AGATCTTCGG TAAGGCTCCG TCCAAGGGTG TGAATCCCGA CGAAGTGGTA 1140
GCTGTGGGTG CCGCTATTCA AGGCGGTGTT CTGACCGGTG AGGTAAAGGA TGTCTTGCTG 1200
TTGGACGTTA CCCCCTTGTC GCTCGGTATC GAGACTATGG GAGGCGTGAT GACTCGCTTG 1260
ATCGATGCCA ATACCACTAT CCCGACGAAG AAGAGCGAAA TCTTTACCAC AGCAGTGGAC 1320
AATCAACCTT CGGTAGAGAT TCATGTACTT CAGGGTGAGC GTTCTTTGGC TAAGGACAAT 1380
AAGAGCATCG GCCGTTTCAA CTTGGACGGT ATTGCTCCGG CGCCCCGTCA GACACCGCAG 1440
ATCGAAGTAA CGTTTGACAT CGATGCCAAC GGTATCCTGA ATGTAACGGC TCATGACAAA 1500
GCTACCGGCA AGAAGCAGAA TATCCGCATC GAAGCCTCCA GCGGTTTGTC CGATGATGAG 1560
ATCAAGCGCA TGAAGGAAGA GGCGCAGGCC AATGCCGAAG CAGATAAGAA AGAGAAAGAA 1620
CGTATCGACA AGATCAATCA GGCCGACAGC ATGATCTTCC AGACGGAAAA GCAGTTGAAG 1680
GAGTTGGGAG ACAAATTCCC GGCCGACAAG AAGGCTCCGA TCGATACCGC TCTCGACAAA 1740
CTGAAAGAAG CACACAAAGC ACAGGATGTA GCTGCTATCG ATACAGCCAT GGCCGAACTG 1800
CAAACCGCTC TTTCCGCAGC GGGCGAAGAG CTTTACAAGA ATGCCGGAGC AGCCCAAGGT 1860
GGCGCACAAC CCGGTCCGGA CTTCGGCGGT GCTCAAGGTC CCTCTGCCGG TGATCAGCCC 1920
TCTGACGACA AGAACGTCAC AGACGTAGAC TTCGAGGAAG TGAAG 1965

(2) INFORMATION FOR SEQ ID NO:17

(i) SEQUENCE CHARACTERISTICS:
(A) LENGTH: 1401 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature

(B) LOCATION 1...1401

(xi) SEQUENCE DESCRIPTION: SEQ ID NO:17

AAGTGGGCAC GTACAACTAC TTTAAGGATA TCGAACGCAA CAATCTATAA AACTATGCGC 60
TACGACTTAG CTATCATCGG TGGAGGGCCG GCCGGTTATA CGGCTGCCGA ACGTGCTGCC 120
AAAGGTGGCC TGAAAACCCT CCTAATTGAG AAGAATGCTC TCGGTGGTGT ATGCCTCAAC 180
GAAGGATGTA TACCGACCAA GACGCTACTC TACTCGGCCA AAGTGCTACA TCAAATTGCT 240
ACGGCATCTA AATATGCAGT AAGTGGAACG GCCGATGGAC TTGACCTCGG CAAGGTGATT 300
GCCAGAAAAG GTAAAATCAT TCGCAAGCTG ACTGCAGGCA TCCGTTCACG CCTGACAGAG 360
GCCGGAGTAG AGATGGTGAC GGCAGAAGCT ACCGTAACGG GATGCGATGC AGACGGCATC 420
ATCGGCATTA CTGCGGGCGA AGCACAGTAC AAAGCTGCCA ACCTGCTACT ATGTACCGGT 480
TCGGAGACGT TTATTCCACC CATCCCCGGA GTGGAGCAGA CAGAGTATTG GACAAACCGT 540
GAAGCTCTAC AGAACAAAGA GATTCCGACC TCTCTCGTCA TCATCGGTGG TGGAGTGATC 600
GGAATGGAGT TCGCTTCTTT CTTCAACGGT ATCGGTACGC AAGTGCACGT GGTGGAGATG 660
CTGCCGGAAA TACTCAACGG TATCGATCCC GAACATGCAG CTATGCTACG CGCTCACTAT 720
GAAAAAGAAG GAATCAAATT CTACCTCGGG CACAAAGTAA CATCGGTTCG CAACGGAGCT 780
GTTACGGTAG AATACGAAGG AGAAAGCAAA GAGATCGAAG GAGAACGTAT CCTGATGAGT 840
GTGGACGTCG GCCCCGTGCT GCAAGGATTC GAGTCGCTCG GATTGGTGCT TGCCGGCAAA 900
GGTGTAAAGA CTAATGAGAG GATGCAAACT TCCCTGCCCA ATGTCTATGC TGCAGGTGAT 960
ATTACAGGCT TCTCGCTTTT GGCACATACG GCTGTACGGG AAGCAGAGGT AGCAGTAGAT 1020
CAGATTTTGG GCAAAACAGA CGAAACGATG AGCTACCGTG CCGTACCAGG TGTGGTGTAC 1080
ACCAATCCCG AGGTCGCCGG TGTGGGAGAG ACGGAAGAAT CGCTTCGCAA AGCAGGACGT 1140
GCCTACACTG TTCGTCGCCT TCCTATGGCC TTCTCCGGTC GATTTGTAGC AGAAAACGAA 1200
CAAGGCAATG GAGAGTGCAA ACTACTACTT GATGAAGAGA ACCGCTTGAT CGGAGCACAC 1260
CTCATTGGCA ATCCGGCCGG CGAACTCATC GTAACCGCTG CCATGGCCAT CGAGACCGGC 1320
ATGACGGATC GACAAATCGA ACGAATCATA TTCCCTCATC CGACTGTAGG CGAAATCCTA 1380
AAAGAAACTC TCGCCGGAGG T 1401

(2) INFORMATION FOR SEQ ID NO:18

(i) SEQUENCE CHARACTERISTICS:
(A) LENGTH: 2835 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2835

(xi) SEQUENCE DESCRIPTION: SEQ ID NO:18

CCTAAGATAC TTATGGAATT GAAAAGATTT TTATCACTTG GTCTTCTGCT TGTGGGATTC 60
ATTCCGATGA AGCTTTCTGC CCAACACGCT CAGCCACTCC CTACAGATCC GGCTGTTCGT 120
GTCGGTAAGT TGGACAACGG ATTGACTTAT TTCATCCGTC ACAACGAGAA CCCGAAAGAT 180
CGTGCGGATT TCTTTATCGC ACAAAAGGTA GGTTCTATTC TTGAAGAAGA TAGCCAGTCC 240
GGTTTGGCTC ACTTCTTGGA ACACATGGCT TTCAACGGTA CGAAGAACTT CCCCGGTAAG 300
AACTTGATCA ACTATCTCGA AACGATCGGT GTACGTTTCG GTCAGAACCT GAACGCTTCT 360
ACCGGATTCG ACAAGACGGA ATATACGATA ATGGATGTGC CGACTACACG TCAGGGAATC 420
ATCGACTCCT GCTTGCTTAT CCTGCATGAT TGGAGTAACA ATATTACCCT CGACGGGCAT 480
GAGATCGACG AGGAGCGCGG TGTGATCCAG GAAGAGTGGC GTCCTCGTCG CGATGCCAAC 540
CTTCGTATGT TCGAGGCTAT ACTTGCCAAG GCTATGCCGG GTAATAAATA TGCAGAACGC 600
ATGCCCATCG GTCTGATGGA CGTCGTGCTC AACTTCAAGC ATGATGAGCT GCGCAACTAT 660
TATAAGAAAT GGTATCGTCC CGACCTGCAA GGTCTGGTGA TCGTGGGAGA TATCGATGTG 720
GACTATGTGG AGAACAAGAT CAAAGAACTC TTCAAGGACG TTCCTGCTCC CGTGAATCCA 780
GCAGAGCGTA TCTATACGCC GGTAGAGGAC AACGATGAGC CTATCGTAGC CATTGCTACC 840
GATGCTGAGG CTACTACCAC GCAGCTCTCC ATCAGCTTCA AGAGCGACCC CACTCCTCAA 900
GAAGTGCGAG GATCGATATT CGGACTTGTG GAAGACTATA TGAAACAGGT GATCACTACA 960
GCCGTGAATG AGCGTCTGTC CGAGATTACT CACAAGCCTA ACGCTCCTTT CCTCAGTGCA 1020
GGAGCTTTCT TCTCTAACTT CATGTACATC ACCCAGACTA AGGACGCATT CAATTTTGTT 1080
GCCACGGTTC GTGAGGGTGA AGCGGAGAAA GCGATGAACG CATTGGTGGC AGAGATAGAA 1140
AGCCTCCGTC AGTTCGGTAT CACCAAAGGC GAATACGATC GTGCACGCAC GAATGTGCTC 1200
AAGCGATACG AGAATCAATA CAACGAAAGA GACAAGCGTA AGAACAATGC TTATGCCAAT 1260
GAATACTCCA CCTACTTCAC CGATGGCGGC TATATCCCCG GTATTGAGGT GGAATATCAG 1320

ACGGTGAATG CTTTTGCTCC TCAGGTTCCT CTGGAAGCAT TCAATCAGGC TATTGCCCAA 1380
ATGATCGATC CGGTGAAGAA TGCTGTCGTT ACCCTCACCG GTCCTTCAAA GGCTGAAGCC 1440
AAGATTCCGA GCGAAGCAGA CTTCCTCGCT GCTTTCAAAG CTGCTCGTCA GCAGAAAGTA 1500
GAAGCCAAGA AAGACGAAGT CTCCGACCAA AAATTGATGG AGAAAGCTCC TAAGGCCGGA 1560
AAGATCGTTT CCGAGAAGAA AGATCAGAAG TTCGGTACCA CGGAACTTAC CCTTAGCAAT 1620
GGCATCAAAG TATACCTCAA GAAGACCGAT TTCAAATCAA ACGAAATCCT GATGAGTGCT 1680
CTCAGCCCGG GTGGTATCCT CTCCGGAAAG CATGCTCCCA ACCAATCTGT GATGAATTCG 1740
TTCATGAACG TGGGTGGCTT GGGCAACTTC GATGCTATCC AGCTGGATAA GGTGCTGACA 1800
GGTCGCTCTG CTTCCGTATC TCCCTCTTTG TCTCTGCTCA GTGAAGGTCT TTCGGGCAAA 1860
ACGACTGTAG AAGATATGGA AACTTTCTTC CAGTTGATCT ATCTCCAAAT GACTGCTAAC 1920
CGCAAGGATC CCGAAGCGTT CAAGGCCACA CAGGAAAAGT TGTACAATAA CTTGAAAAAT 1980
CAGGAAGCCA ACCCGATGGC TGCGCTTATG GACTCTATCC GTCATACCAT GTACGGCGAT 2040
AATCCGATGA TGAAACCCAT GAAAGCTGCT GACGTGGAGA AAGTAAATTA CGATCAGGTA 2100
ATGGCTTTCT ACAATGAGCG ATTCGCTGAT GCCGGCGACT TTATGTTCTT CTTTATCGGT 2160
AATCTGGATG AAGCCAAGAT GAAGCCATTG ATCGAAACTT ATCTTGCTTC ATTGCCCAAC 2220
CTCAAGCGTG GCGATAAGAT GAATAAGGCT CAGGTACCGG CTGCCCGTTC GGGAAAGATC 2280
GATTGCAAGT TCGAGAAGGA AATGGATACT CCTTCGACTA CTATATTCGA TGTCGTGTCC 2340
GGAAATGTGG AATATACGCT CAAGAACAGT CTCCTGCTGG AAGTCTTCTC AGCCGTAATG 2400
GATCAGGTGT ACACGGCTAC CGTTCGCGAG AAGGAAGGCG GTGCATACAG TGTGGCTGCA 2460
TTCGGCGGTC TCGAGCAATA TCCTCAGCCC AAGGCTCTGA TGCAGATCTA TTTCCCCACG 2520
GATCCTGCTC GTGCCGAGGA AATGAATGCT ATCGTTTTTG CTGAGTTGGA GAAGCTTGCC 2580
AAGGAGGGCC CCAATGTGGA ATACTTTAAG AAGACTATCG AAAACCTGAA TAAGCAGCAC 2640
AAAGAAAGTC TGCGTGAGAA TCGTTTCTGG CTCGAAGCCA TGAAGGCGTC TTTCTTCGAA 2700
GGAAATGACT TCATCACAGA CTACGAATCC GTACTGAACG GTCTTACTCC TGCTGAATTG 2760
CAAAAGTTTG CGGCAGACCT CTTGAAGCAG CAGAATCGGG TTGTTGTCAT GATGGCTCCT 2820
GTTGCAAAGG CTCAA 2835

(2) INFORMATION FOR SEQ ID NO:19

(i) SEQUENCE CHARACTERISTICS:
(A) LENGTH: 2058 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2058

(xi) SEQUENCE DESCRIPTION: SEQ ID NO:19

TACACTATGA GTAAGAAAGG AACAATCGGG GTAACGAGCG ACAATATATT CCCCGTCATC 60
AAAAAATTCC TGTACAGCGA CCATGAGATA TTCCTGCGTG AGATCGTCTC CAATGCCGTG 120
GATGCTACGC AGAAGCTGAA AACGCTTACA TCCGTCGGCG AATTCAAAGG CGAGACGGGT 180
GACCTCCGCG TAACGGTCAG CGTGGATGAA GTGGCACGCA CGATCACGGT CAGCGACCGC 240
GGCGTAGGGA TGACCGAAGA GGAGGTGGAG AAGTACATCA ATCAGATTGC TTTCTCCAGT 300
GCGGAAGAGT TTCTTGAAAA GTACAAAGAC GACAAGGCCG CCATTATCGG CCACTTCGGA 360
CTCGGATTTT ACTCGGCTTT CATGGTGTCC GAGCGAGTGG ACGTGATCAC GCGCTCTTTC 420
CGAGAAGATG CTACGGCGGT GAAATGGAGC TGCGACGGAT CGCCCGAATA CACGCTCGAA 480
CCTGCGGACA AGGCTGACCG TGGCACCGAC ATCGTGATGC ACATCGATGA GGAGAATAGC 540
GAGTTCCTCA AAAAAGAAAA GATAGAGGGG CTCCTCGGCA AATACTGTAA GTTCCTTACC 600
GTGCCGATCA TTTTCGGCAA GAAGCAGGAA TGGAAAGACG GCAAGATGCA AGATACGGAC 660
GAGGACAATC AGATCAACGA CACACATCCT GCCTGGACCA AAAAGCCTGC CGACCTCAAG 720
GACGAAGACT ATAAGGAATT TTACCGTTCG CTCTATCCCA TGTCCGAAGA GCCTCTCTTC 780
TGGATCCACC TCAATGTGGA CTATCCGTTC AATCTGACAG GTATCCTCTA TTTCCCGAAG 840
ATCAAAAACA ACTTGGATCT GCAGCGCAAC AAGATTCAGC TCTACTGCAA TCAGGTTTAC 900
GTCACCGATG AAGTACAGGG TATCGTGCCG GACTTCCTCA CCCTCCTGCA CGGGGTCATC 960
GATTCGCCGG ATATTCCCCT CAACGTATCG CGCTCCTATC TGCAGAGCGA TGCCAATGTG 1020
AAGAAGATCT CGTCTCATAT CACCAAGAAG GTGGCAGACC GTCTGGAAGA AATTTTCAAA 1080
AACGACCGCC CCACATTCGA GGAGAAATGC GATAGTCTGA AGCTCTTCGT CGAATACGGT 1140
ATGCTGACGG ATGAGAAGTT CTATGAGCGT GCAGCCAAAT TCTTCCTTTT CACCGATATG 1200
GACGGACACA AGTACACGTT CGACGAATAC CGAACGCTCG TCGAAGGTGT ACAGACGGAT 1260
AAGGACGGAC AGGTAGTGTA TCTCTATGCT ACGGACAAGC ATGGACAGTA CAGCCACGTG 1320
AAACGTGCAT CCGACAAAGG CTACAGCGTG ATGCTGTTGG ATGGTCAGTT GGATCCGCAT 1380
ATCGTGAGCC TGCTGGAGCA AAAGTTGGAG AAGACACACT TTGTCCGTGT CGATAGCGAT 1440

ACGATCAACA ATCTGATCCG CAAGGAGGAA AGAGCCGAAG TGAAACTGTC CGATACGGAG 1500
CGCGCCACTC TCGTGAAGCT GTTCGAAGCA CGCCTGCCAC GGGACGAGAA GAAGCACTTC 1560
AATGTAGCTT TCGAATCGCT CGGAGCCGAA GGTGAAGCCA TCCTTATCAC ACAAGCCGAA 1620
TTCATGCGCC GTATGCGCGA TATGGCACAG CTGCAGCCGG GAATGAGCTT CTACGGCGAA 1680
CTCCCCGATT CGTACAATCT GGTACTTAAT ACCGATCATC CGCTCATCGA CAGGGTACTC 1740
TCCGGTGAGA AAGAATCGGT AGAGCCTTCG CTCACAGAGC TTAGAGCGAA AATCGCCGAG 1800
CTGAAAGCGG AAGAGGCCAA GCTGCTCGAT GAGGAAAAAG GGAAGAAACC GGAGGAAATC 1860
CCTGTTGCCA CGAAGGAAGC CAAGGAGAAC AACGCCGTCG AACAGGCCAA AACCGAAGGC 1920
AGTATCAACG ATCAACTGAC CAAATATGCT CAGGACAACG AGCTGATAGG TCAGCTCATC 1980
GACTTGGCTC TGCTCGGAAG CGGATTGCTG ACGGGAGAGG CTTTGGCCGA ATTCATTCGT 2040
CGCAGCCAGC GTCTTCTC 2058

(2) INFORMATION FOR SEQ ID NO:20

(i) SEQUENCE CHARACTERISTICS:
(A) LENGTH: 1446 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1446

(xi) SEQUENCE DESCRIPTION: SEQ ID NO:20

GACATTCGAC AGAAAAGACC GTGCTTCAAC GCCAATTTGT ACTTTTACCG TGCAATGGAA 60
AAACTGATCG ATATTTTGGT CGTAGACGAT GATGTGGCAG TCTGTGCCGC ACTGCGTCTG 120
GTGCTCAAGC GAGCGGGCTA TAATCCCGTT ATAGCCAACA GTCCCGACGA AGCTTTGTCC 180
ATAATGCGGA ATCCTGATGG CGGCTGTAAG CCGGCTGTGA TTCTGATGGA TATGAATTTC 240
TCCCTTTCGA CCTCCGGCAG GGAAGGATTG GAACTACTGG AGAAGATGCA GATATTCACT 300
TCCTGCCCTG TCATACTGAT GACGGCTTGG GCTTCGATTC CACTGGCAGT GGAGGGAATG 360
AGGCTTGGAG CTTTCGACTT CATAGGCAAG CCATGGGACA ACGATCGGCT CCTTCGTACC 420
ATAGATACGG CCTTGCATCT GGCTGCTCCC TCAGCTGTGG CGAATCCATC GGAACAGTCT 480
GACAGAGATA CAGCCCGTCA GCCGAAAGCT ACAGTCCAAG AGAATGACCC CTGTGCCCAT 540
ATCATAGGCC GGAGCGATGC CATCTGTAAG ATCAAGGAAC GGATACGCCG CATAGCTCCC 600
ACCCATGCCT CTGTGCTGAT CACGGGCGAG AGCGGTACGG GCAAAGAGTT GATAGCCGAA 660
GCTCTGCACC GTGGGAGCAA ACGAGCCTCA GCCCCATTCG TCAAGGTCAA TTTGGGTGGG 720
ATTCCCGAAA GTTTGTTCGA AAGTGAGCTG TTCGGACATA AGAAAGGAGC TTTTACCAAT 780
GCTTTTTCCG ACAGGAAAGG ACGGTTCGAG CTGGCTGATG GCGGCACGAT CTTTCTGGAC 840
GAAATAGGCG AACTACCGGT CGGCAACCAA GTAAAACTGC TGCGAGTGCT ACAGGAACAG 900
ACATTCGAGC CGTTGGGCGA GAGCGTCTCC CACCGAGTGG ACATCCGTGT GGTATCGGCT 960
ACGAATGCTT CCTTGGAGCG AATGGTAGCC GAAGGACGTT TCAGAGAGGA CCTCTACTAT 1020
CGAATCAACC TGATACATCT GCATCTGCCT CCGCTGCGTG AGCGTCAGGA GGATATACAG 1080
CTGCTGGTGG AAGCCTTCAG TGAAGCCTTT GCCCAATCGA ACGGATTGCC CCATGCCGTT 1140
TGGAGTGCGG AAGCTATGCG ACGTATCTGT GCCATGCCCC TACCGGGCAA TGTACGCGAA 1200
CTGAAAAACG TAGTGGAGCG TACGCTATTG CTCTCGGGAT CGAGAGAAAT CAGTGCCCGG 1260
GATGTGGCTG ACTTCGGTTC GCAGGTGACG GCAGCAGACC ACTCCGACGA ACGGGCTTTG 1320
ACCGACATGG AGGAAGCTGC TATCCGAGAG ACGCTGACTA AATACAACGG CAACGTTAGT 1380
CGTGCTGCAC GAGCCTTGGG ATTGAGCCGG GCAGCTCTTT ACCGGCGAAT GGAGAAATAC 1440
GGACTG 1446

(2) INFORMATION FOR SEQ ID NO:21

(i) SEQUENCE CHARACTERISTICS:
(A) LENGTH: 789 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...789

(xi) SEQUENCE DESCRIPTION: SEQ ID NO:21

AGATCTCTGC AATCATTTCA AAATAAAAAA CACTCAAGTA TGCTTAAGAT AAAGAACCTC 60
CACGCCACAG TACAGGGCAA AGAGATATTG AAAGGAATCA ATCTGGAGAT CAATGCCGGA 120
GAGATTCATG CTATCATGGG GCCGAACGGA TCGGGGAAAA GTACGCTCTC TTCCGTTTTG 180
GTGGGACATC CCTCCTTTGA AGTCACGGAA GGAGAGGTGA CATTCAATGG AATCGACCTG 240
CTCGAACTCG AACCGGAAGA ACGTGCACAC CTCGGACTCT TTCTCAGTTT CCAATATCCG 300
GTCGAGATCC CGGGCGTCAG CATGGTGAAT TTCATGAGGG CAGCTGTCAA TGAACATAGG 360
AAAGCGATCG GAGCAGAACC CGTATCGGCA AGCGACTTCC TCAAGATGAT GCGAGAGAAG 420
CGTGCCATTG TGGAGCTGGA CAACAAATTG GCCAGCCGTT CTGTGAACGA AGGCTTCTCC 480
GGTGGAGAAA AAAAGAGGAA CGAAATCTTC CAAATGGCTA TGCTCGAACC CAAGCTGGCT 540
ATTTTGGACG AAACCGATAG CGGGCTCGAT ATCGACGCTC TCCGCATCGT AGCAGGCGGG 600
GTAAACCGAC TCCGCTCTCC GGAGAATGCT GCTATTGTGA TCACACACTA TCAGCGTTTG 660
CTCGAGTACA TCAAGCCGGA CTTCGTACAC GTCCTTTACA AGGGGCGCAT CGTCAAGTCG 720
GGAGGAGCCG AGCTGGCTCT CACGCTCGAA GAAAAAGGCT ACGACTGGAT CAAGGAAGAG 780
ATAGGAGAA 789

(2) INFORMATION FOR SEQ ID NO:22

(i) SEQUENCE CHARACTERISTICS:
(A) LENGTH: 1386 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1386

(xi) SEQUENCE DESCRIPTION: SEQ ID NO:22

AGCATGGCTA AGGAGAAAAC GATCTACGTC TGCCGTTCGT GCGGAACCAA ATACGCCAAA 60
TGGCAAGGCA ACTGCAATGC CTGTGGAGAG TGGAACTGCA TTGATGAGGA GAAGGTGCCG 120
GCACCGGCAT CGGGCAAGCA TGCAGCCAAG AGTTTTATGC CTCGGGAGCA GGACAACCGG 180
CCAAGACTCT TACAGGATGT GGAGTCCGGC GATGAAGAGC GTATTCGCCT CGGCGATGAA 240
GAGTTCGACC GCGTACTGGG TGGAGGAATT GTCAAAGGAG CATTTGTCCT GCTTGGCGGC 300
GAGCCGGGAA TCGGTAAGTC CACGCTTATC CTCCAGACGG TGCTGCGTCT GCCGCAGTTG 360
CGCACGCTCT ATGTGTCGGG CGAAGAAAGT GCCCGACAAC TGAAGATGCG CGCCGAACGA 420
CTGGGGCAAG CCATGAATGG GTGCTACGTA TACTGCGAAA CGAATATAGA GAGGATACTC 480
TCCCGTGCAG AAGAACTCAC ACCCGATCTC CTCGTGATAG ACTCTATACA GACGGTCTAT 540
ACCGAGGAAA TGGAAAGCTC GGCCGGCAGC GTGGGGCAGA TCCGCGAATG TGCCGCCTTA 600
CTGCTCAAAT ACTGCAAGAC TACGGGTATC CCCGTCATCG TCATCGGACA CATCACCAAA 660
GAAGGTAGCA TAGCCGGACC GAAGGTGCTG GAGCATATAG TGGATACGGT GCTTCTCTTC 720
GACGGGGATA AGCATCATCT CTACCGGATA CTCCGAGGAC AGAAGAACCG CTATGGCAGT 780
ACTTCCGAGC TGGGGATATA CGAGATGCGG CAGGACGGTC TGCGTGGCGT GGAGAATCCG 840
AGCGAACATC TCATCACACG CAATAGGGAA GACCTCAGTG GCATAGCCAT AGCCGTAGCG 900
ATGGAGGGCA TTCGCCCGAT ACTCATCGAA GCGCAGGCTT TGGTCAGCTC GGCCATTTAT 960
GCCAATCCGC AGCGTTCGGC CACGGGCTTC GATATTCGGC GGATGAACAT GCTCTTAGCC 1020
GTACTGGAGA AACGTGCCGG CTTCAAGCTC ATACAGAAGG ATGTGTTTCT GAACATTGCC 1080
GGAGGTATCA AAATAGCCGA TCCGGCTACG GATCTGGCCG TTATCTCGGC AGTGCTGGCG 1140
TCGAGTCTGG ACATCGTTAT CCCGCCGGCC GTATGCATGA CGGGCGAGGT CGGACTCTCC 1200
GGAGAGATAC GTCCCGTGAG CCGCATCGAG CAGCGCATAA CGGAAGCGCG TCGCATAGGG 1260
TTCAAAGAGA TATTGGTACC GGCCGATAAT TTCCGGCAGG AGGATGCCGG CCGCTTCGGT 1320
ATTCGGCTCG TGCCGGTCAG AAAGGTGGAG GAAGCCTTCC GCCATCTGTT CTCGAAAGGA 1380
AGAGAA 1386

(2) INFORMATION FOR SEQ ID NO:23

(i) SEQUENCE CHARACTERISTICS:
(A) LENGTH: 1119 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1119

(xi) SEQUENCE DESCRIPTION: SEQ ID NO:23

GGGTCTTGTG GAAGTAGCCC AGCAATTGGC CGATCAGGGT GTTCGCGTTG TGATCGCCGG 60
ATTGGACATG GACTTTCGAC GTCAGCCTTT CGGACCTATG CCGGGCTTGT GTGCCATAGC 120
CGACTCCGTG ACCAAAGTTC ATGCCGTGTG TGTGGAATGC GGCCGATTGG CCAGCTATTC 180
TTTCCGTCGT GTCCAAGGCG ATCAGCAAGT GATGCTGGCC GAACTGAACG AATACAGTCC 240
CCTCTGCAGA ACCTGCTACA GGAAATGCAG TTCTCCCCCA CAAACAGAAG AAATCCATTC 300
GACAATATGA ATAGCAGACA TCTGACAATC ACAATCATTG CCGGCCTCTC CCTCTTTGTA 360
CTGACATTGG GCGGCTGCTC CGTAGCCCAA CAAGATACGC AGTGGACTCT CGGCGGAAAG 420
CTCTTTACTT CGGCGTGGAT ACAACGTTCG GCCGAATATC AAGCGCTTTG CATTCAGGCA 480
TACAACATCG CTACGGAAAG AGTGGACGCT CTACCGGCAG AACGTAAACA AGGAGATAGG 540
CCTTATGCCA TCGTAACGGA CATAGACGAA ACCATTTTGG ACAATACGCC TAACTCCGTG 600
TATCAGGCTC TCAGGGGCAA GGATTATGAT GAAGAGACTT GGGGGAAATG GTGTGCACAG 660
GCCGATGCCG ACACACTGGC AGGAGCTTTG TCTTTCTTCC TCCATGCAGC GAACAAGGGG 720
ATCGAGGTCT TTTACGTCAC CAACCGCAGA GACAATCTGC GCGAAGCAAC TCTTCAGAAC 780
CTTCAGCGTT ACGGATTCCC CTTTGCCGAT GAAGAACATT TGCTTACGAC CCATGGGCCA 840
TCCGACAAAG AACCCCGTCG GCTCAAAATA CAAGAACAGT ATGAAATAGT ATTGCTCATA 900
GGAGACAACT TGGGCGACTT CCACCACTTC TTCAATACGA AAGAAGAGTC CGGACGCAAA 960
CAGGCTCTGG GCCTGACAGC CGGGGAGTTT GGCCGGCACT TCATCATGCT GCCCAATCCC 1020
AACTACGGAT CTTGGGAACC GGCATGGTAC GGCGGGAAGT ATCCGCCACT GCCCGAAAGA 1080
GACAAAGCAC TTAAACAACT GCACTCACAG AACAGCAGA 1119

(2) INFORMATION FOR SEQ ID NO:24

(i) SEQUENCE CHARACTERISTICS:
(A) LENGTH: 1278 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1278

(xi) SEQUENCE DESCRIPTION: SEQ ID NO:24

CGTTGGGGTT TTTCTAATTT TGTGGCCATG AGCACCAATA TAGATGTACA ACAGATCAAA 60
CAGCGTTTCG GCATCATCGG TAGCAGTCCG CTGATGGAAC ATGCCATACG AGTGGCAGCA 120
CAGGTGGCTC CTACCGACAT GTCCGTCCTC GTGACGGGGG AGAGCGGTTC GGGAAAAGAG 180
TTCTTCCCAC AGATAATCCA CTACTACAGC GCCCGGAAAC ATCATAGCTA CATTGCAGTC 240
AATTGCGGAG CCATCCCCGA AGGAACCATC GATTCCGAGC TGTTCGGACA CCGCAAAGGT 300
TCCTTTACCG GAGCCGTATC GGATCGCAAG GGGTACTTCG AAGAAGCATC CGGCGGCACG 360
ATCTTTCTGG ACGAAGTGGG CGAACTGCCT TTGCCCACGC AGGCGAGGCT GCTGAGGGTG 420

CTGGAGACGG GCGAGTTCAT CCCCGTAGGA GCCAGCCAGT CGCAGAAGAC GGATGTCCGT 480
ATCGTAGCGG CGACGAATGT GAACCTCAAG GAGGCGGTAG CGAACGGGAA GTTCCGGGAA 540
GACCTCTTCT TCCGGCTCAA TACGGTACCG ATCGAGGTGC CTGCGCTGCG TATGCGACCG 600
GACGACGTGC CCTTGCTTTT TCGCCGATTC GCCGCCGACA GCGCCGAGAA GTATCGGATG 660
CCTCCGCTGC GCCTATCGGA CGAAGCCCGT ACCATATTAA TGCGTTACCG CTGGCCCGGC 720
AATGTGCGAG AGCTGCGCAA TATAACCGAC AGGCTGAGCA TCCTGGAGGA GGAGCGGACG 780
GTATCGGCAG AGACCATCAC TCGCTACCTG GACGCTGAGG GGATGCAAGA CCTCCACCCC 840
GTCGTGATCC GACGGAACGA AACGACCGAA GCGGACAAAC AAATCCCCCA TTACGAGCGC 900
GAAATCATCT ACCAGGTGCT ATACGATATG AAGAAAGAGA TAGCCGATTT GAAGGGGATC 960
ATGAACCGCC TGGCGCACCA CGAACAGCCC TCATGGCCTG TAGGGTCGGA CGTCTGGGGC 1020
AACGACGACA AGCGCACCGC AGATCCGAAG TGGGGCGTCA GCACGCACAA GGCCCCCATC 1080
GCGAACGCGG CAGAACCCGT GGAGCCGATA CAGGAAGCCA GCGAATACAC CGAGGATCCG 1140
GTTTCGCTGG AGGAGGTAGA GAAGAAAATG ATTTCCCTTG CATTGGAACG CCACGGCGGA 1200
AGGCGCAAGC AGACAGCCGA GGAACTGAAG ATTTCGGAGC GGACACTATA CCGTAAAATC 1260
AAGGAGTATG GACTGGAA 1278

(2) INFORMATION FOR SEQ ID NO:25

(i) SEQUENCE CHARACTERISTICS:
(A) LENGTH: 1959 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1959

(xi) SEQUENCE DESCRIPTION: SEQ ID NO:25

AAAAATCTTC CGAGGTACGC TCCGGCATTT GGCAGAAATC GTAAAAGGGA ACAACCTGAC 60
CCTGACAACG ATGATAGTCG TGGGAGAAGC CATAGACAAT CGGGAAGGGC TGTCACGGTT 120
GTATGCCGAC GAATTCAAAC ACCTCTTCCG CACATGATCC TACTCTTCGG CGGTACTACG 180
GAAGGCCGTG CCGCAGCTCG CGTGCTGGAT GAAGCGGGAA GTCCGTTTTT CTACTCCACC 240
AAAGGCAATC TGCAAGAGAT CCAGAGTAGC CACGGCCATC GTCTGACAGG AGCCATGACG 300
GTTGCCGACA TGGTTTCGTT TTGTCGGAAA GAAGAGATCC GACTGATCGT GGACGCCGCT 360
CATCCTTTCG CCGAAGAATT GCACGCTTCA GTGGCAGAAG CCTCTGAACA AACAGGTATC 420
CCCGTAGTAA GATACGAGAG ACAATACCCT CCACGCGAAG AAGGTATCGT CTGGTGTGCA 480
AACTACGATA CGGCTGCCGA GCGGATGCTT GGCGATGGCG TGCAGCGTCT GCTGATGCTC 540
ACAGGAGTGA ATACGATCCC CAAGCTGGCT GCTTTCTGGA AAGAGCGCAC CACCTTTTGC 600
CGCATATTGA AGCGAGACGA ATCGGTTGCT TTGGCAGAGA AGAACGGCTT TCCTGCGGAG 660
CGCATCGTTT TCTTCGAACC GCATGCGGAC GAGGAGCTGA TGCAAGCCGT TCGCCCCGAT 720
GCCATTATCA CAAAAGAAAG CGGAGAGAGC GGTTACTTCC GAGAAAAGAT AGAAGCTGCC 780
CGACGGATGG GCATCCGTAT ATATGCCGTC GTACGTCCCC CTTTGCCTCC TTCATTCATT 840
CCCGTAGGCG GGCCTGTCGG TTTGAGACGG GCGGTAGAAC GCCTCGTGCC GGGATTCTTT 900
TCACTCCGAA GCGGATTCAC TACCGGCACC ACAGCTACCG CTGCAGTAGT AGCAGCCATG 960
TACCGATTGA TGGGGCTTGG CTCTCTCGCC GAAGCTCCCG TAGAATTGCC TTCGGGCGAA 1020
ATAGTCAGTC TGCCCATAGC GGAAATTCGA GAGGAAGAAG ATGCTGTCGT ATCCGCAGTC 1080
CTGAAAGATG CAGGTGATGA TCCGGATGTG ACCAATGGCA TGGCGGTATG CGCTACGATC 1140
AGGCTCAATC CCGAACATGA GGAAGTCCGC TTCCTGCAGG GTGAAGGGGT GGGGGTAGTG 1200
ACGCTCCCCG GCCTCGGTCT GGAGGTCGGA GGTCCGGCTA TCAACCTCGT ACCTCGACGA 1260
ATGATGACAG CAGAGGTACG CCGACTCTAT GCGCAGGGAG GTGTGGATAT TACGATTAGC 1320
GTACCCGAAG GCCGAGAGGC TGCTACCCAG ACATTCAATC CCCGACTCGG CATACGGGAC 1380
GGCATCTCTA TTATCGGAAC ATCGGGAGTC GTGAAACCTT TTTCGGCCGA AGCGTTCGTT 1440
GGTGCCATCC GTAAGCAAGT GGGTATTGCC ACCGCCTTGG GAGCCAATCA TATCGTCCTC 1500
AATTCGGGAG CCAAGAGTGA GCGTTATGTA AAAGGAGCCT ATCCGGCACT CATTCCACAG 1560
GCCTTTGTGC AGTATGGCAA TTTCGTCGGC GAATCACTCA GTTGTGTAGC TTCCTTCCCT 1620
TCTGTCCGTT CGGTAACGGT AGGAATCATG CTCGGCAAAG CAGTGAAACT CGCCGAAGGC 1680
TATCTGGATA CGCACAGTAA AAAGGTAGTG ATGAATCGGG ATTTCCTGCA CGAACTGGCT 1740
CGTCAGGCAG GTTGTTCGGA AGACATCCAT GCCATAATAG ACAGCCTGAA TTTGGCTCGT 1800
GAGCTATGGA CTATGCCGAG TGCGGAGGAC AGCGATCGAC TGCTACGAAA GATTGCCGAA 1860
CGATCTTGGG AAACTTGCCG CCCATCGGTA CCATCGGCCG AATTAGAACT CCTGCTGATC 1920
GATGAGTCCG GAGCGATTCG TTTTCGTATC GGTGGAGAA 1959

(2) INFORMATION FOR SEQ ID NO:26

(i) SEQUENCE CHARACTERISTICS:
(A) LENGTH: 1353 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1353

(xi) SEQUENCE DESCRIPTION: SEQ ID NO:26

CAACAAAGAC CACATAGAAT TATTATGTTG AGGACTTTCC GAATCGGTGG TATTCACCCC 60
CCCGAAAACA AGTTGTCGGC AGGCAAGCCC GTAGAGGTGT TGCCTATCCC CTCACAGGTA 120
GTCATCCCTC TTGGTCAGCA CATCGGTGCA CCGGCAACTG CCACGGTCAA GAAAGGGCAT 180
GAAGTTAAGG TCGGGACTAT CATTGCTCAG GCCGGAGGAT TCGTATCAGC TAATATCCAC 240
TCATCTGTGT CGGGTAAGGT GCTGAAGATC GATAACGTAT ACGACTCAAG CGGCTATCCC 300
AAGCCCGCAG TCTTCATTAG CGTAGAAGGT GACGAATGGG AAGAGGGCAT CGATCGCTCA 360
CCAGCCATCG TCAAAGAATG CAATCTGGAT GCAAAAGAAA TCGTAGCCAA AATTTCTGCA 420
GCCGGTATTG TGGGTCTTGG CGGTGCTACC TTCCCTACCC ATGTGAAGCT GTCCCCTCCT 480
CCGGGCAACA AAGCTGAGAT CCTGATCATC AACGCCGTAG AGTGCGAGCC TTATCTGACG 540
AGCGACCATG TCCTTATGCT GGAGCACGGC GAAGAGATCA TGATCGGCGT GAGTATCCTG 600
ATGAAAGCCA TTCAGGTAAA CAAGGCCGTC ATCGGAGTTG AGAATAATAA GAAAGATGCT 660
ATTGCTCACC TCACCAAACT GGCCACTGCA TATCCGGGCA TAGAGGTAAT GCCGTTGAAG 720
GTGCAATATC CTCAAGGCGG TGAGAAGCAG CTGATCGATG CAGTGATCCG CAAGCAGGTA 780
AAAAGCGGTG CCTTGCCTAT CAGCACAGGT GCCGTAGTAC AAAACGTGGG TACGGTATTC 840
GCCGTGTACG AAGCAGTACA GAAGAACAAG CCTCTGGTCG AGCGCATCGT GACGGTTACA 900
GGAAAAAAAC TGTCTCGTCC GTCTAACCTC CTCGTTCGTA TAGGTACTCC TATTGCGGCT 960
TTGATCGAAG CAGCAGGTGG CTTGCCGGAG AATACGGGCA AGATCATCGG CGGAGGTCCG 1020
ATGATGGGAC GCGCTCTGCT GTCACCGGAT GTGCCTGTGA CCAAAGGCAG CTCCGGAGTA 1080
TTGATTCTCG ATAGAGAAGA GGCAGTTCGC AAGCCTATGC GCGACTGTAT CCGATGCGCC 1140
AAGTGCGTCG GAGTGTGTCC GATGGGACTC AATCCGGCTT TCCTTATGCG CGACACCTTA 1200
TATAAGAGCT GGGAAACAGC GGAAAAAGGC AACGTGGTTG ACTGTATCGA ATGCGGTTCG 1260
TGCAGCTTCA CCTGTCCGGC CAACCGTCCT CTGCTGGATT ATATCCGCCA AGCCAAGAAG 1320
ACTGTGATGG GTATCCAAAG AGCACGTAAG CAA 1353

(2) INFORMATION FOR SEQ ID NO:27

(i) SEQUENCE CHARACTERISTICS:
(A) LENGTH: 1467 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGTHAL SOURCE:
(A) ORGANISM: PORYPHYROMONAS GINGIVALIS

(ix) FEATURE:
(A) NAME/KEY: misc_feature
(B) LOCATION 1...1467

(xi) SEQUENCE DESCRIPTION: SEQ ID NO:27

ATGAACTCTC AAAAGAAAGA GGCTTTTAAT ATGAAAAGAA TACAACTAAC TCTTATCGCT 60
CTCTTCGCCG CTGTTGCCGG TTTGGTCGCT CAAAATGCTT ACGAGGGAGT AATTTCATAT 120
AAAATTTCGT TGGACAAAAC CGGAAACAAG CTTGTACTGA ATGGTGCGGC AGATATGAGT 180
AATTTAAAGC TCAAGAGCAC TCAGATGATC ATTGTTACGC CTATTCTTCG TTCAGAAGAT 240

GGTACCAGCC GGGTGGAATT TCCTTCGGTA GTCATTACAG GCCGCAATAG AACAAAAGCT 300
CTCAAGCGTG AAATCGCATT TAGTTCGGCT TTGCCCCAAG CAAAACATGC AGCTCAATAC 360
ATTCGCCGTC ATAATGGGAA GAGCGAGCAG TTTGCTTTTA CAGGAGAACA TGCTTATGCA 420
TCATGGATGA TGGATGCCAA GTTTGTGGTT CGTGAGGAGG TACGAGGTTG TGCTAAATGC 480
CCTGTAGGTC TCTCGAGTAA TATTGTTCCT TTTGATCCAC TCTTCAATCC GGCAGAGGCT 540
CCTTATTTGT TGGCACACAT TACTCCGGCA GAAGAAGTGG AAAAACAGCG AGAGTCCAGC 600
TTCGATGCTT ATATCAACTT CAAAGTCAAT AAGGCAGATG TCCTTCCTGA GTATCGCAAC 660
AATAAGGCGG AGTTAGAGAA AATCAAAGAA TTTGTAAGCA CCGTTAAGGC TAATCCAAAC 720
TATTCGGTCA ATAAAATGAT CATCGAAGGG TTTGCTTCTC CCGAGGCTTC AATAGCCCAC 780
AATAAGGCTT TGTCGGAGCG CCGTGCTAAA AGACTCGCGG AAGAATTGGT GCGTAAGTAT 840
GGCAAAACAT TGCCGAATAT AACCACTGAA TTCGGCGGTG AAGATTGGAA GGGGCTGAAA 900
CTGGCTATCG AAAAGAGTGA TATAGCCGAT CGTGACCGCG TATTGGAGAT AATCAACTCC 960
GATAAATATG CCGATGATGA TGCACGTGAA CAGGCTCTGA AGCAACTTTC GTCTTATCGT 1020
TATATCTTGG ATCAGATCTA TCCGAATTTG CGTCGCAATA CGATAACCAT GGGGTATATC 1080
GTTCGTGATT ATACCCTCGA AGAAGCTCGT GAAATCATTA AGACTGCTCC GAAAGAACTT 1140
AGTGAGGCCG AAATGTACCG TGTGGCAATG TCTTATCCTG AGGGGCACCA AGAGCGTTTG 1200
TTTGCTCTGA ATACGACCCT TAAGTATTTC CCTGAAAGTG TAACGGGCCG AATCAATTTG 1260
GCTGTAGCCG CTTTTAATGG TGGAGACGTT CAACAGGCAA TTGCTCTGTT GAGTCCGATT 1320
CAGACAGAAA AGGGTGTAAG CAATATCCTT GGAGCTGCTT ATGCTCGTAC GGGAGATTTT 1380
GCTCGTGCCG AAACCTTCTT CCGTAAGGCC GTTGCAGAAG GAGATGCAAA TGCGCAGCGC 1440
AACCTCGATA TGCTGCTTGG CAAAAAG 1467

(2) INFORMATION FOR SEQ ID NO:28

(i) SEQUENCE CHARACTERISTICS:
(A) LENGTH: 1152 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1152

(xi) SEQUENCE DESCRIPTION: SEQ ID NO:28

GACATGGCAG AAAAAAGAGA CTATTACGAA GTCCTCGGTG TATCGAAGAA TGCCACCGAC 60
GATGAACTGA AAAAAGCATA TCGCAAGAAG GCTATCCAAT ACCATCCTGA TAAGAACCCC 120
GGTGACAAGG AGGCCGAAGA GCACTTCAAA GAGGTAGCTG AAGCCTACGA CGTATTGAGC 180
GATCCGCAGA AGCGCAGTCA ATATGACCAG TTCGGCCATG CCGGATTGGG CGGAGCTGCC 240
GGTGGAGGTT TCAGCGGAGG CGGTATGTCC ATGGAGGATA TTTTCAGTCG CTTCGGTGAT 300
CTATTCGGTG GGTTCGGCGG TTTCGGCGGA TTCTCCGATA TGGGCGGTGG CAGTCGCAGA 360
CGTGTTCGCA GAGGGTCTGA CCTGCGAGTA CGAGTGAAGC TTTCTTTGGC CGATATAAGT 420
AAAGGTGTGG AGAAGAAAGT GAAGGTAAAA AAGCAGGTAG TGTGCAGCAA ATGTCGTGGC 480
GATGGCACGG AAGAAGCCAA TGGCAAGACT ACCTGCCAGA CCTGCCATGG AACCGGCGTG 540
GTTACACGTG TGAGCAACAC TTTCCTTGGG GCCATGCAGA CCCAGAGCAC TTGTCCCACT 600
TGCCACGGAG AGGGTGAGAT CATCACGAAG CCATGCTCCA AGTGTAAGGG CGAAGGTGTG 660
GAGATCGGCG AAGAGGTGAT CTCATTCCAC ATCCCTGCCG GTGTAGCCGA AGGAATGCAA 720
ATGTCCGTGA ACGGCAAGGG AAATGCCGCG CCCCGAGGAG GCGTGAATGG CGACTTGATA 780
GTCGTGATCG CCGAGGAACC GGATCCGAAT CTGATCCGCA ATGGCAACGA TCTGATATAC 840
AATCTGCTTA TATCCGTTCC GTTGGCTATA AAAGGAGGTA GTGTGGAAGT GCCGACGATA 900
GACGGACGAG CCAAGATCCG CATCGAGGCG GGGACACAAC CCGGCAAGAT GCTGCGTTTG 960
CGCAATAAGG GGTTGCCCAG CGTAAACGGC TATGGCATGG GAGACCAACT GGTGAATGTC 1020
AATGTCTATA TCCCCGAATC GATCGATGCC AAAGATGAGC AGGCTATCGC AGCGATGGAA 1080
AACTCGGACA GCTTCAAACC TACCGATGCT GCTCGTAAGG ATATAGACAA GAAATACAGA 1140
GAGATGCTGG AT 1152

(2) INFORMATION FOR SEQ ID NO:29

(i) SEQUENCE CHARACTERISTICS:
(A) LENGTH: 927 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...927

(xi) SEQUENCE DESCRIPTION: SEQ ID NO:29

CGATTCGATT CAACAACTAA TGTCTCACAA ATTAATTTAA GAACAGAGAT GAAAAAACTG 60
ATTTTAGCGA CTTTGGGACT TATGGCCATT GCCATGCTCT CATGTTCAAG CAACAACAAG 120
GATTTGGAGA ACAAAGGGGA GGCTACTCTT TTGGTAACGT TTGGTAGCTC CTATAAAGCT 180
CCACGCGAAA CCTATGCGAA GATTGAGAAG ACTTTTGCCG CAGCTTATCC CGATCAAAGG 240
ATAAGCTGGA CATACACGTC TTCTATTATC CGAAAGAAAC TGGCTCAGCA GGGTATTTAT 300
ATCGATGCTC CGGATGAGGC TTTGGAGAAA TTGGCTCGTC TGGGTTATAA GAAGATCAAT 360
GTACAGAGTC TTCATGTGAT TCCCGGCCGA GAATATGATG AGATGATCGA CTTTGTCAAT 420
AAGTTTAAGG CAGCACATAG TGATATTACT GTGAAGGTAG GGGCTCCGCT TTTCGATACC 480
GATGAAGATA TGCGCGAGGT GGCAGAGATC TTGCACAAGC GTTTTCAGCA AACGATAGAG 540
AAAGGTGAAG CTATTGTATT CATGGGACAC GGCACCGAGC ATGCTGCCAA TGACAGGTAT 600
GCCCGTATCA ATAAGATCAT GAAGAACTAT AGCAAGTTCA TGATCGTCGG AACCGTCGAG 660
TCCGATCCCT CTATCAATGA TGTTATTGCC GAACTGAAAG AAACCGGTGC CACGGCCGTA 720
ACAATGATGC CGCTGATGAG TGTGGCAGGC GACCATGCTA CGAATGATAT GGCCGGAGAT 780
GAGGACGATA GCTGGAAGAC GTTGCTGACC AATGCCGGCT ACACAGTTTC TATAGACAAG 840
CTGGACAATG GCAATTTCTC AGCTCTTGGA GATATAGAAG AGATCCGGAA TATCTGGCTC 900
AAGCATATGA AAGCCACCTC TGCTCGC 927

(2) INFORMATION FOR SEQ ID NO:30

(i) SEQUENCE CHARACTERISTICS:
(A) LENGTH: 1473 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1473

(xi) SEQUENCE DESCRIPTION: SEQ ID NO:30

CGGAGAGATG CGCGGAGGCA ACTGGTACGC CGTAGGAGGA AAGAGCTATC TGGCACAGCA 60
AATACGCGAT GCCGGAGGAG AGTATTTCCT GAAAGACGAT CAGCGATCCG GTGGTGTTTC 120
CCTCGACTTC GAGACGGTCT ACAGCCGATC CGATTCGGCT CGCTACTGGC GAATCCTCAA 180
CAGCTATCCC GGGGAGTTCG GTTACGAAGC TTTGAAAGCC GAAGACAGCC GATATGCCGA 240
TTTCAGGGCA TTCAAGGAAA AAGGCGTGAT ATACTGCAAC CTGAGGGAAA CAGCCTTCTA 300
CGAACTCATG CCCATGCATC CCGATTGGGT GCTGGCCGAT CTTATCGCTA TCTTGCACCC 360
CGGACTACTT CCCGACCACC AACCGCATTT CTATTATTTG CTCCAATGAC ATCCGTCAGC 420
CACTTACGTA CAATTTCTGT CGCAGGTATC CTGGCTGCGC TGGGAGGGGC TGTACTCATT 480
CTCTTCGGGG TTAATCTCTT CCTCGGCTCG GTGGCTATTC CGATGAGCGA GATCTTCCGA 540
CATCTTTTTT CAGATCGTCC CGAAGGAGGA GAAGCACTCG TGCACTACAA TATCCTATGG 600
AAATCCCGCC TGCCCGAAGC CCTCACGGCT GCTTTTGCCG GCGCAGGTTT ATCCGTTAGT 660
GGCTTGCAGA TGCAGACCGT CTTTCGCAAT CCTTTGGCCG GTCCGTCCGT TCTCGGCATC 720
AGCTCCGGTG CCAGTTTGGG TGTTGCTTTG GTCGTTCTGC TGAGCGGCTC GCTGGGAGGA 780
GTGGCATTGA GTAGCCTGGG TTATATGGGC GAGGTGGCCA TGAATATAGC CGCTGCCGTA 840
GGCTCGCTGG CAGTAATGGG GCTGATCGTT TTTGTCAGCA CCAAGGTGCG CAGCCACGTT 900
ACGCTGCTCA TTATCGGCGT TATGATCGGA TATGTAGCCA CTGCCGTCAT CGGGGTATTC 960
AAGTTTTTCA GTATCGAAGA AGATATTCGG GCATACGTAA TTTGGGGGTT GGGCAGCTTT 1020
TCCCGTGCCA CGGATTCGCA ACTGAGTTTC TTTGCCATTC TGATGTTGAT CTTTATTCCG 1080

GCCGGTATGC TCCTTGTCAA GCAGTTGAAT CTCTTATTGC TGGGAGAAAG CTACGCACGT 1140
AATCTGGGAC TGAATACTCG TCGGGCACGG CTGCTCGTGA TCTCTTCCGC CGGTTTGCTC 1200
ATCGCTACCG TCACGGCCTA TTGCGGTCCC ATCGGCTTTT TGGGGATGGC TGTGCCACAC 1260
TTGGCACGGG TTATCTTTCA CACATCGGAT CATCGGATCC TGATGCCTGC TACCTGTTTG 1320
ATTGGAAGTG CTCTGGCTCT TTTCTGCAAT ATCATTGCTC GTATGCCGGG GTTTGAGGGG 1380
GCTTTGCCCG TCAATTCCGT AACGGCTTTG GTGGGAGCAC CTATTATCGT CACCGTTTTG 1440
TTCCGGCGCA GACGCTTCAA GGAAGAAACC GAC 1473

(2) INFORMATION FOR SEQ ID NO:31

(i) SEQUENCE CHARACTERISTICS:
(A) LENGTH: 2289 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2289

(xi) SEQUENCE DESCRIPTION: SEQ ID NO:31

CATTTTTTAG TAACGATTAT GCGGACAAAA ACTATCTTTT TTGCGATTAT CTCTTTTATT 60
GCTCTATTGT CGTCTTCTCT GTCGGCTCAG AGCAAAGCCG TTTTAACCGG TAGTGTGTCG 120
GATGCCGAAA CCGGAGAGCC TCTTGCCGGT GCTCGAATCG AAGTCAAACA CACCAACATA 180
GTAGCCGGTG CCGATGCCGG CGGACATTTC GAGATCAAGA ACCTGCCGGC AGGGCAGCAT 240
ACTATTATAT GTTCGTTGGG GGGGTATGGA CAGAAAGAGG AGGTGGTTGC CATCGAAGCC 300
GGACAGACCA AAACGATCTC TTTTGCATTG CGACTGCGAA CGAACAACTT GGAGGAAGTC 360
GTCGTTACCG GTACCGGTAC ACGTTACCGC TTGGTCGATG CTCCTGTGGC AACGGAAGTC 420
CTTACCGCTA AGGACATAGC CTCTTTCTCG GCTCCTACTT CCGAGGCCTT ATTGCAGGGG 480
CTGAGTCCGT CTTTTGACTT CGGCCCCAAT CTGATGGGCT CTTTCATGCA GCTGAACGGC 540
CTTAGCAGTA AGTATATCCT CATCCTTATC GATGGTAAGC GTGTGTACGG CGATGTAGGC 600
GGTCAGGCCG ATTTGAGTCG TATTTCTCCT GATCAGATCG AACGGATCGA ACTGGTGAAA 660
GGTGCTTCGA GTTCGCTCTA CGGATCCGAT GCCATCGCCG GGGTAATCAA TGTGATCACA 720
AAAAAGAATA CGAATCGACT GAGTGCATAT ACGTCACATC GCATATCGAA GTACAACGAT 780
CGGCAAACCA ATACTTCGCT CGATATAAAC ATCGGTAAGT TCAGTAGCAA TACCAACTAT 840
TTCTTCTACC ATACGGATGG CTGGCAGAAT AGTCCGTTCG AAATAAAAAA GAAAAAAGGA 900
TCCGGCGAAC CGGTCTTGGA GGAAACGTAT AAGAAAACTT TTCGTGCACA GGAAAATCAG 960
GGTGTAAGCC AATCGCTTTC CTATTATGCA ACTAACAATC TTAGCTTCAG CGGAAATGTG 1020
CAGTACAATA AACGTCAGAT CTTCACTCCG ACTTTTTCCG AAAAGAAGGC CTATGACATG 1080
GATTATCCTG CTTTGACGGC TTCACTCGGT ACGAACTATC TTTTCCCCAA TGGTCTGCAT 1140
ACGCTTTCTT TCGATGCCGT CTACGATCGC TTCCGTTTCG GATATTTGTA TCATGACAAG 1200
GACAGCAGTG AGAGCCTGAT CAACAACCAA GGTCAGACCG AGCAACCCAC ATTCTTTCCG 1260
GGTCAGCTAC GCAATAAAAA CGATCAGATC CGATACACGG CAGAGGCTCG CGGTGTATTT 1320
ACACTGCCTT ATGCGCAGAA ACTGACCGGC GGTTTGGAGT ATTTCCGTGA GGAATTGATC 1380
TCTCCCTATA ATTTGATTAC CGACAAGGCA GATGCTTCCA CGCTCTCTGC TTATGTACAA 1440
GATGAATGGA AACCGCTCGA TTGGTTCAAT ATGACAGCCG GTTTCCGTCT GGTACACCAT 1500
CAGGAGTTCG GTACACGAAT GACGCCTAAG GTATCCATAC TCGCCAAGTA TGGGCCGCTG 1560
AACTTCCGCG CTACGTATGC TAACGGCTAT AAGACTCCCA CGCTGAAAGA GCTTTTTGCA 1620
CGGAACGAAC TCACCACTAT GGGTTCGCAC AATCTCTATC TCGGCAATGC GGATCTTAAG 1680
CCACAGATGT CGGATTATTA TGCTTTGGGC TTGGAGTACA ATCAAGGCCC TATCTCGTTC 1740
AGTGCAACGG TTTATGACAA TGAACTTCGC AATCTGATCT CCTTTATGGA TATACCGACC 1800
TCACCCGAGC ACGAAGCTCA GGGAATCAAG AAAACCAAGC AGTATGCCAA CATAGGAAAA 1860
GCTCGCAGCC GCGGCCTTGA TGTCCTATGT GATGCCTCTA TCGGTTGGGG TATCAAGTTA 1920
GGAGCCGGAT ACAGCCTCGT GGAAGCTAAG AATCTCCAGA CGGATGAGTG GCTGGAAGGA 1980
GCTGCACGTC ATCGTGCCAA TGTGCACGCC GATTGGGTTC ACTACTGGGG TCAGTATAGA 2040
CTTGGCGTGA GCCTTTTCGG CCGTATTCAG AGCGAGCGTT ACTACAAAGA CGGCAATGCT 2100
CCGGACTATA CCTTGTGGCG ACTCGCCACA TCGCATCGTT TCGCTCATTT CCGCCACATC 2160
ATCCTGGATG GAACGCTCGG TATAGACAAC CTGTTTGACT ACGTGGATGA TCGTCCTATG 2220
GGTGTCAATT ATGCTACCGT AACGCCGGGA CGTACTTTCT TTGCTCAAAT AGCGATTCGA 2280
TTCAACAAC 2289

(2) INFORMATION FOR SEQ ID NO:32

(i) SEQUENCE CHARACTERISTICS:
(A) LENGTH: 1095 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1095

(xi) SEQUENCE DESCRIPTION: SEQ ID NO:32

TGCTGCGCAA GGCTTCGGGA CACCTCTCCC CGGAAGAAGT CGTTTCGGAA TGATACCGTT 60
TTTCCTTATT TCGTTATTCA TCTGATCAAA CATATTATCA TTATGACGGA CAACAAACAA 120
CGTAATATCG TATTCCCGGC GTTTCTCCTC TTGCTGGGAG TCATCGCAGT GGTGACGATC 180
GTTGGTTTTT TCATGCTCAG ACCGGCCGAG GAGATTATCC AAGGACAGAT AGAAGTGACC 240
GAATACCGAG TGTCCAGCAA AGTGCCCGGG CGCATCAAGG AACTTAGGGT ATCCGAGGGA 300
CAGCAGGTGC AGGCCGGCGA TACCCTCGCT GTCATCGAAG CCCCCGACGT AGCGGCTAAG 360
ATGGAGCAGG CAAAGGCTGC CGAAGCAGCT GCACAGGCTC AGAACGCCAA GGCTCTCAAA 420
GGAGCACGCA GCGAACAGAT ACAGGCAGCC TATGAGATGT GGCAGAAAGC TCAGGCCGGC 480
GTAGCCATAG CGACCAAGAC ACACCAGCGC GTGCAGAACC TCTATGACCA GGGAGTGGTA 540
CCGGCTCAGA AGTTGGACGA AGCCACTGCC CAGCGCGATG CGGCCATCGC TACGCAAAAA 600
GCGGCCGAAG CCCAGTACAA TATGGCTCGC AACGGTGCCG AACGCGAAGA CAAGCTGGCA 660
GCTTCTGCCC TCGTCGATAG AGCGAGAGGA GCCGTCGCCG AGGTGGAGTC GTACATCAAC 720
GAAACCTACC TCATCGCCCC ACGGGCAGGC GAAGTGTCGG AGATATTCCC CAAAGCCGGC 780
GAACTCGTAG GTACCGGCGC ACCTATCATG AATATCGCCG AGATGGGCGA TATGTGGGCC 840
AGCTTTGCCG TTCGTGAGGA TTTCCTCAGC AGCATGACCA TGGGAGCCGT TCTGGAGACT 900
GTGGTGCCGG CTCTGAATGA AGAAAAAGTA CGCTTCAAGA TCACATTCAT CAAGAACATG 960
GGTACCTATG CTGCCTGGAA AGCGACCAAG ACAACAGGGC AGTACGACCT GAAGACCTTC 1020
GAGGTAAAGG CCACCCTTGC GGATAAAGAC AAGGCACAAA AGCTACGCCC GGGTATGTCC 1080
GTGATCATAC GCAAG 1095

(2) INFORMATION FOR SEQ ID NO:33

(i) SEQUENCE CHARACTERISTICS:
(A) LENGTH: 960 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...960

(xi) SEQUENCE DESCRIPTION: SEQ ID NO:33

CCGCAATCCT CTCCTGATCG AAGAAGCTTC CAAAACGTCA TGAATAAATA TCATTCTCAA 60
AGCGTTTTAG AGGTCGGCAA AATTGGGATT GTGATTATCT TTGCGCCCAT AGTACGGAAT 120
GTACATCAAC AACCCCCTTT TTTAAGCCAT AAATCAATTA TGCGTATTGT CAGTAATTTT 180
TTGTTCGTCT CTTTTTCGGT TTTGCTTTTT GCATCATGCC GTTCCCAGCG AGAAAAGGTC 240
CTTTACCTGC AAGATATCCA AACTTTTAAT CGGGAGATTA TCGCTAAACC ATATGACGTA 300
AAAATTGAGA AGGACGATGT GCTGAACATC CTTGTCACCA GTAGAGACCC GGAGCTTTCA 360
ACGCCCTACA ACCAAGTGTT GACCACTCGT GCACTGGCCC GCAACGGCTA TGGAACGAAC 420
TCGAACGAAG GCTTCCTGGT CGATTCGAAA GGGTACATCA ATTATCCTAT TTTAGGCCAG 480
ATCTATGTAG AGGCCCTTAC TCGTACCGAA CTGGAGAAGG AGATACAGAA GAGGATTATT 540
TCCAGTGGAT TTATCAAGGA TCCTACGGTA ACGGTGCAGC TTCAAAATTT CAAGGTGTCG 600

GTTTTGGGAG AGGTGAATCA TCCGGGTTCG ATGTCGGTAA AAGGAGAGCG AATAACTCTT 660
TTGGAAGCGA TCGGAATGGC CGGAGACCTG ACAATCTATG GTCGCCGCGA TCGGGTTTTT 720
GTGATTAGAG AAACCGATGG GCATCGCGAG GTTTTCCAGA CGGATCTCAG AAAGGCCGAC 780
TTGCTCGCAA GCCCCGTGTA CTATCTGCAT CAGAACGACG TCATCTATGT GGAGCCGAAC 840
GACAAGAAAA CACAGATGAG CGAGATCAAC CAGAATAATA ACGTAAACGT ATGGCTGAGT 900
GTTACCTCCA CTTTGGTATC CATTTCCACG CTGACGATTA CGATAATAGA TAAGACCAAA 960

(2) INFORMATION FOR SEQ ID NO:34

(i) SEQUENCE CHARACTERISTICS:
(A) LENGTH: 1746 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1746

(xi) SEQUENCE DESCRIPTION: SEQ ID NO:34

TCAAAAATAG TACTACGAAA GTTTTGTACC TTAGCGCGCA TGAAAAAGAC CAATCTGTTT 60
TTATCTCTGC TGGTGATCTT TATCACCGGT AGTTTTATGA CTGCCTGTGC ACAGAAGTCC 120
AAGACGAACA AACTCACCGA AGAAGATCGG AGCCGCAATG AGTATGTACA GTCGATGGAT 180
GTGCTTAGCA ATATTATCGG TAACGTCAGG CTGTATTTCG TCGATACCAT AAGTATCAAA 240
CATATGACTC GGCGTGGTAT AGATGCGATG TTGGGCGGGC TTGACCCCTA TACCGAATAC 300
ATTCCTTACG AGGAAATGGA TGAACTGAAA TTGATGACTA CGGGAGAGTA TGCCGGAGTC 360
GGAGCTATCA TATCGCAGCG CCCGGATAGT GCTGTGATTA TCCAGAGACC TATGGAAGGT 420
ATGCCCGCAG ACGAAGCAGG ATTGATAGCA GGCGACCGCA TCCTGACTAT CGATGGGAAA 480
GACTTCCGCA AATCCACCAC ACCGAAAGTA AGCCAAGCAC TGAAAGGGAT AGCCGGTACT 540
GTTGCAAAGG TGACAGTAAT GCGCTATGGC GAAACCAAAC CTCGTACTTT TTCCGTGAAA 600
CGTCAAAAAG TGATTATGAA TTCCGTCACT TACAGCGGAA TGCTCGATGG CTCGATAGGA 660
TATATCCGCT TGAACAACTT TACGGACAAA AGTGCAGAAG AGGTGCGCAC GGCCTTGTTG 720
GATCTTCGTG ACAAACAAGG AGCGAAAGGT CTCATTTTGG ATTTAAGAGG CAATGGTGGC 780
CGACTGATGC AGGCTGCTAT CGAGATAGTC AATCTGTTCG TCCCTAAGGG CAAAGAGGTG 840
GTAACGACCA AAGGTCGCAT TGCAGAGTCG GCGTCCGTAT TTCGCACATT GACTGAACCG 900
ATCGACACGA AACTCCCGAT AGTAGTCCTG ATCGATGGAC AATCGGCATC TTCCTCGGAG 960
ATTGTAGCCG GAGCACTGCA GGATATGGAC AGGGCTGTAC TGATGGGACA AAAGAGCTAT 1020
GGCAAAGGGC TTGTACAAAC GACTCGTCAG CTACCATACA ACGGCGTGAT CAAATTGACT 1080
ACGGCCAAGT ACTACATCCC AAGCGGACGT TGTATTCAGC GTTTGGACTA CAGCCGCACC 1140
AATCGGACAG GTATGGCAAC GGCCATTCCT GACAGTCTGC ACAAAATCTT TTACACTGCT 1200
GCCGGAAGAC GTGTAGAAGA TGCAGGAGGA ATCCTGCCTG ACATCGAGGT CAAACAAGAT 1260
ACAGCTGCGA CATTACTTTA TTATATGGCC ATCAATAATG ACGTTTTCGA TTTCGTCACA 1320
GGTTATGTGC TCAAGCATAA AACGATTGCC AAGCCGGAGG ATTTTTCCAT AACGAACGAG 1380
GACTATGCAG CTTTCTGCAA GATGATGGAA GAAAAGAAAT TTGACTATGA TCGCCAGAGT 1440
GGCAAGATGC TTGACAAACT GGAGGAACTG GCTAAGATAG AAGGCTACCT GCCGGAAGCC 1500
AACTCGGAGC TTAAAGCACT ACGCGAAAAG CTAAAACCCA ACCTGTCGCG TGATCTGCTA 1560
CGATTCAAAA AGGAGATAAC AAACTATCTC AACAATGAGA TTGTCACTCG CTATTATTAT 1620
GAGCGAGGCA GTATCCGCCA GAGTTTGCCG GAAGATAAGG TAGTCAAAGA AGCTATTAAG 1680
CTGCTGAAGG ACCATCCGGA ACAAATTCGA CAGATCCTTG CAGCTCCGAA AGCAGAGAAT 1740
AAAGGG 1746

(2) INFORMATION FOR SEQ ID NO:35

(i) SEQUENCE CHARACTERISTICS:
(A) LENGTH: 2955 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2955

(xi) SEQUENCE DESCRIPTION: SEQ ID NO:35

GTAACAGACA AAATGCAAAA CAAAGGATTT GTGATTGTTA TCACATCGGC TCTGGCCATC 60
ATCTGTGCGT TTTACCTGTC ATTCTCTTTC GTTACGAACC GTTACGAAAA GAAGGCTAAG 120
GCGATGGGCG ATGTTGCCGG AATGGCCTAT CTTGATTCCA TGTCGAATGA GAAGGTCTGG 180
TTCGGCTACA CGCTGAAAGA AGCTCAAGCC CAGCAAATTG GTCTTGGCCT TGACTTAAAG 240
GGGGGTATGA ACGTTATCTT GAAACTTAAC GCAAGCGATC TGCTTCGTAA CCTCTCTAAC 300
AAAAGTTTGG ATCCCAACTT CAACAAAGCT CTGGAGAATG CTGCCAAGAG CACGGAGCAA 360
TCCGACTTCA TCGATATTTT CGTGAAGGAA TATCGCAAGC TCGATCCCAA CGGTCGCTTG 420
GCCGTTATCT TCGGTTCGGG TGACCTTCGC GACCAGATTA CCGCAAAGTC TACGGATGCA 480
GACGTAGTGC GTCTGCTCAA AGAAAAATAT AATAGTGCTG TAGAAGCTTC GTTCAATGTG 540
CTCCGTGCTC GTATCGATGC TTTCGGTGTG GTTGCACCTA ATTTGCAGCG ATTGGAAGGG 600
CAAGGGCGTA TCCTTGTCGA ACTCCCCGGA GTGAAAGACC CTGAGCGTGT TCGTACCCTT 660
TTGCAACGCA GTGCCAACCT ACAGTTCTGG CGTACATACA AATTCGAAGA GGTCAGCGGA 720
GACTTGATCG CTGCCAATGA TCGTCTGAGC GAATTGGCTA TGAACAACAC GGATGCTACC 780
CCGGAAACAG AGCCTGCAAC TACTGACTCT GTAGCTGCAA CAGCCGATTC TGCTGCTGTA 840
CAAGCTGTAG CTGATTCTGC TACTGTAGCA CAAAAAGAGG CCAAGGATGC TACTCGTAAA 900
GACGCACTCT TCTCTCTGCT TACTCCCGTG AATCGTGGCG GTGCAGTAGT GGGTGTGGCT 960
CGTCGTGCTA ATATGGCTCA GATATCTGAA ATGCTCCAGC AAGCTCACGA TCTGAAGGTT 1020
ACACGTGAAG ATGTGCTTTT CCTCTGGGGT GCTAAAGCAA TCGAAGACCC CGAAACCAAA 1080
AAGGAGACCG ACCTCTACGA ACTCTATGCT ATTCGTACCA ATCGTACGGG AGATCCTGAT 1140
TTGGGAGGTG ATGTAGTGAC TTCCGCCAAG AGTGATATCC AAAATGACTT CGGTCGTTCC 1200
GAACCGATCG TTTCGATGAC GATGAATGAA GAAGGTGCTC GTAAATGGGC GCGTATCACA 1260
AAGGATAACG TGGGACGGGC AATCGCTATC GTTTTGGATG GTGTGGTTTA TTCTGCTCCG 1320
AACGTGAATG ATGAGATCAC GGGCGGTCGC TCTCAGATCT CCGGGCACTT CACCGTGGAG 1380
GAGGCCGGTG ACCTTGCCAA CGTACTCAAC TCCGGTAAAA TGGATGCTAC GGTAAGCATC 1440
GAACAGGAAA ACGTGATTGG TCCTACGCTG GGTGCCGAGT CCATTAAAGC CGGATTCTTG 1500
TCGTTCCTGC TCGCTTTGGT TATCCTGATG TGTTACATGT GTCTGGCTTA CGGTTTCTTG 1560
CCGGGTCTTA TCGCAAACGG CGCATTGATT GTAAACAGCT TCTTCACATT GGGCGTATTG 1620
GCTTCTTTCC ATGCCGTGCT GACCCTCTCG GGTATCGCAG GTTTGGTGCT GACGCTGGGT 1680
ATGGCTGTGG ATGCCAACGT ACTTATCTTC GAGCGTATCA AAGAAGAGCT TCGTGCCGGT 1740
AAGACTCCGA TTCGTGCCGT TACGGATGGT TATGGCAACG CTTTCTCTGC CATCTTCGAC 1800
TCGAACGTTA CGACTATTAT TACCGGTATC ATCCTATTCC TCTACGGGAC GGGGCCGATT 1860
CGCGGTTTTG CCACTACGTT GATTATCGGT CTTATCGCTT CTTTCATTAC GGCTGTCTTC 1920
TTGACTCGTA TCGTCTTCGA GAAACTGGCG AAAAAAGGTC GTTTGGATAA GATTACATTC 1980
ACTACGAGCA TTACTCGCAA TCTCCTTGTC AATCCCTCAT ACAACATCTT GGGTAAGCGC 2040
AAGACCGGCT TTATCATTCC GGTGATTATC ATCGTTTTGG GACTTATAGC TTCATTTACA 2100
ATCGGTCTCA ATAGGGGTAT TGAATTCTCC GGAGGACGTA ACTACGTAGT TAAATTCGAC 2160
CAGCCTGTAT CTTCCGAAGC CGTTCGTTCG GCCTTGTCTT CTCCCCTGCA GGAAAAGGTA 2220
TTGGTTACCT CCATCGGTAC TGAAGGGACA GAGGTGCGTA TATCTACGAA CTATAAGATC 2280
CAGGAGGAAA GCGAAGAAAC TGAAGCAGAG ATTACTGACA AATTGTATCA GAGCCTGAAA 2340
GGTTTCTACA CCCAGCAGCC TACTGCTGAT CAGTTCTTGG ACAATATCAT TAGCTCTCAG 2400
AAAGTAAGTC CCAGTATGTC GAGTGACATC ACGAGAGGTG CTATTTGGGC TGTGCTGTTA 2460
TCGATGATCT TCATGGCCAT TTATATTCTG ATTCGCTTCC GTGACATTTC TTTCTCTGCC 2520
GGGGTATTCG TATCTGTGGC CGCTACTACA TTCTGCATTA TTGCTCTGTA TGCGTTGCTG 2580
TGGAAGATTC TGCCCTTCAC CATGGAGATC GATCAGAACT TCATCGCTGC TATTCTGGCT 2640
ATCATCGGTT ACTCGCTCAA TGACACCGTG GTTGTATTTG ACCGTATCCG AGAGACGATG 2700
AAATTGTACC CCAACAGAGA TCGCTATCAG GTGATCAACG ATGCCCTTAA TTCAACATTG 2760
GGTCGAACAT TAAATACGTC TTTGACTACG TTTATCGTTA TGTTGGTAAT CTTCATCTTT 2820
GGAGGTGCTA CGATGCGTAG TTTCACGTTC TCGATCCTGC TCGGTATCGT TATCGGTACA 2880
TACTCTACGC TCTTTGTTGC TACACCCCTT GCCTACGAGA TCCAAAAGCG CAAGCTCAAC 2940
AAAGCAGCTA AGAAA 2955

(2) INFORMATION FOR SEQ ID NO:36

(i) SEQUENCE CHARACTERISTICS:
(A) LENGTH: 3138 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...3138

(xi) SEQUENCE DESCRIPTION: SEQ ID NO:36

GACTTCCCTT GGGTTAGGGT AAAACCGGAG AAGAAAAGAA AACAACATAA CAGTAATAAT 60
TTTAAGTTTA ACGCAAAAGA AAAGTCTATG AAAAGAATGA CGCTATTCTT CCTTTGCTTG 120
CTGACGAGCA TTGGGTGGGC TATGGCCCAG AATAGAACCG TGAAGGGTAC AGTTATCTCC 180
TCCGAGGATA ATGAGCCCCT GATCGGCGCG AATGTCGTGG TTGTCGGAAA CACCACTATC 240
GGTGCTGCAA CCGACTTGGA TGGCAACTTC ACGCTTAGCG TGCCTGCCAA TGCCAAAATG 300
TTGAGAGTGT CCTATTCCGG TATGACTACC AAAGAGGTCG CCATCGCTAA TGTGATGAAG 360
ATCGTACTGG ATCCGGACTC TAAGGTTCTG GAGCAGGTAG TTGTATTGGG TTACGGTACG 420
GGACAGAAAC TCAGCACTGT TTCCGGTTCT GTGGCCAAAG TGTCCAGCGA AAAGCTCGCG 480
GAAAAGCCCG TTGCCAATAT CATGGATGCC CTCCAAGGTC AGGTAGCCGG TATGCAGGTT 540
ATGACTACAT CCGGTGACCC TACTGCCGTC GCTTCTGTGG AGATCCATGG TACAGGGTCG 600
TTGGGCGCAA GCTCTGCACC ATTGTATATC GTGGATGGTA TGCAAACTTC TTTGGATGTT 660
GTGGCTACGA TGAATCCGAA TGATTTTGAA TCTATGTCCG TTTTGAAAGA TGCTTCTGCA 720
ACATCTATTT ATGGAGCTCG TGCTGCAAAC GGAGTCGTTT TCATTCAAAC GAAGAAAGGT 780
AAAATGAGCG AGAGAGGTCG TATTACCTTT AATGCCAGTT ACGGGATTTC TCAAATCCTG 840
AATACTAAGC CCCTTGATAA TATGATGACT GGAGATGAAT TGCTGGATTT TCAGGTGAAG 900
GCAGGTTTTT GGGGGAACAA TCAAACCGTT CAGAAGGTTA AAGATATGAT CCTTGCCGGA 960
GCTGAAGATT TGTATGGCAA TTATGATTCT TTGAAAGATG AGTATGGTAA GACATTGTTC 1020
CCAGTGGATT TTAATCATGA TGCAGACTGG CTCAAGGCTT TGTTTAAAAC AGCACCCACC 1080
AGTCAAGGTG ATATTTCTTT CTCCGGAGGG TCTCAGGGAA CTTCATATTA TGCCTCTATA 1140
GGCTACTTCG ATCAGGAAGG TATGGCTCGT GAACCGGCAA ATTTTAAGCG CTATAGTGGC 1200
CGGCTCAACT TCGAAAGTCG TATCAATGAA TGGCTGAAAG TTGGTGCAAA TTTGTCTGGT 1260
GCGATAGCGA ATAGACGATC TGCCGACTAT TTTGGAAAGT ATTATATGGG GTCAGGTACT 1320
TTCGGTGTGT TAACGATGCC TCGTTATTAT AACCCTTTTG ATGTGAATGG GGATTTAGCA 1380
GATGTCTATT ACATGTATGG AGCTACCAGA CCTTCTATGA CAGAACCGTA CTTCGCAAAA 1440
ATGAGACCGT TCAGTTCCGA ATCACATCAG GCCAATGTAA ATGGTTTCGC CCAGATTACT 1500
CCGATCAAAG GCCTTACTTT AAAGGCACAG GCTGGTGTTG ATATTACTAA TACTCGCACT 1560
TCTTCTAAGA GAATGCCCAA TAATCCGTAT GATTCTACTC CTCTTGGGGA AAGAAGAGAA 1620
AGAGCTTATC GAGATGTTAG CAAGTCTTTT ACAAATACGG CTGAATATAA GTTTTCAATT 1680
GATGAAAAAC ATGATCTTAC AGCATTGATG GGGCATGAAT ATATTGAATA TGAAGGGGAT 1740
GTTATTGGGG CATCTTCTAA AGGATTTGAA AGTGATAAGT TGATGTTACT GAGCCAGGGA 1800
AAAACCGGAA ATAGTTTGTC TTTGCCTGAA CACAGAGTCG CTGAATATGC CTATTTGTCT 1860
TTCTTTAGTC GTTTTAATTA CGGTTTTGAC AAATGGATGT ATATAGATTT CTCTGTTCGT 1920
AATGACCAAT CCTCTCGATT CGGATCCAAT AATAGAAGCG CGTGGTTCTA TTCTGTCGGT 1980
GGAATGTTTG ACATATATAA TAAATTCATT CAAGAAAGTA ATTGGCTCAG TGATCTTCGA 2040
CTGAAAATGA GTTATGGTAC AACGGGTAAC TCGGAGATTG GTAATTACAA CCACCAAGCA 2100
CTCGTTACTG TGAACAATTA TACTGAAGAT GCTATGGGGC TTAGCATTTC TACAGCAGGC 2160
AATCCCGACC TCTCGTGGGA AAAGCAGTCT CAGTTCAACT TCGGTTTGGC TGCAGGGGCT 2220
TTCAATAATC GCTTATCTGC AGAGGTAGAT TTCTATGTCC GCACTACGAA TGATATGTTG 2280
ATTGATGTCC CGATGCCTTA TATCAGTGGT TTCTTCTCAC AGTATCAGAA TGTAGGCTCT 2340
ATGAAAAATA CGGGTGTAGA CCTTTCTCTT AAGGGGACGA TCTACCAAAA TAAGGACTGG 2400
AATGTATATG CTTCTGCGAA TTTCAACTAC AATAGACAGG AAATAACAAA GCTTTTCTTC 2460
GGTCTCAATA AGTACATGTT GCCTAATACC GGTACTATAT GGGAAATTGG GTACCCCAAT 2520
TCGTTCTATA TGGCTGAATA TGCTGGAATC GACAAAAAAA CCGGTAAGCA GTTGTGGTAT 2580
GTTCCTGGTC AAGTCGATGC GGATGGTAAT AAAGTTACAA CAAGCCAGTA CTCAGCTGAC 2640
TTGGAGACAC GAATTGATAA GTCTGTTACT CCTCCTATTA CAGGTGGTTT CTCCTTAGGT 2700
GCTTCTTGGA AAGGACTTTC TTTAGATGCT GATTTTGCCT ACATCGTTGG TAAATGGATG 2760
ATCAATAATG ACCGTTACTT TACAGAGAAT GCAGGTGGAT TGATGCAATT AAATAAAGAT 2820
AAAATGCTAT TGAATGCCTG GACAGAGGAT AATAAAGAAA CAGATGTTCC AAAATTGGGA 2880
CAGTCTCCTC AGTTTGATAC GCATTTGTTG GAGAATGCTT CTTTCCTGCG TTTGAAGAAT 2940
CTCAAACTCA CCTATGTACT CCCCAATAGT CTTTTTGCTG GGCAGAATGT GATTGGTGGA 3000
GCTCGTGTCT ATTTGATGGC GCGCAATCTG TTAACTGTTA CGAAGTATAA AGGCTTTGAC 3060
CCTGAAGCAG GGGGGAATGT GGGAAAAAAT CAATATCCTA ATTCTAAGCA GTACGTTGCG 3120
GGTATTCAGT TGTCTTTC 3138

(2) INFORMATION FOR SEQ ID NO:37

(i) SEQUENCE CHARACTERISTICS:
(A) LENGTH: 2607 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2607

(xi) SEQUENCE DESCRIPTION: SEQ ID NO:37

TGGCATAGGA ATATTTTTAT CTTTGCGAGT ACATTTAGCC CGAAAAATAT GCTCCCACTG 60
CCATACCGTT ATGCAAAAAC CGAGCACCTT TTTCTCGCAA AAGGATACTG CAAGAATCCA 120
ATAACAAACA TAATTATCCT ATTTATGAAG AAAAAGAATT TTTTGCTTCT TGGCATTTTC 180
GTTGCTTTGC TGACTTTCAT CGGCAGCATG CAGGCACAAC AGGCCAAAGA TTATTTCAAC 240
TTTGACGAAC GGGGCGAGGC CTACTTCTCA TTCAAAGTGC CTGATAGGGC CGTTCTACAA 300
GAGCTGGCTC TGATCATGTC CATCGACGAG TTTGACCCCG TAACCAATGA AGCCATTGCC 360
TATGCCAGCG AAGAGGAGTT CGAGGCATTC CTGCGCTATG GGCTCAAGCC TACATTCTTG 420
ACTCCTCCAT CCATGCAGCG CGCTGTCGAG ATGTTCGACT ACCGCTCAGG AGAAAAATAC 480
GAATGGAATG CTTACCCCAC CTATGAAGCC TATATCAGCA TGATGGAAGA GTTCCAAACA 540
AAGTATCCAT CACTTTGTAC TACTTCCGTC ATTGGCAAGT CCGTAAAGGA TCGTAAACTG 600
ATGATTTGCA AGCTGACGTC CTCTGCCAAT ACAGGGAAAA AGCCTCGCGT GCTCTATACT 660
TCTACGATGC ACGGAGACGA AACGACCGGA TATGTGGTAC TGCTCCGACT CATAGACCAT 720
CTGCTGTCGA ACTACGAATC CGATCCGAGG ATTAAGAACA TTCTGGATAA AACGGAAGTA 780
TGGATCTGCC CTTTGACCAA TCCGGACGGA GCATACAGAG CCGGAAACCA CACCCTACAA 840
GGAGCTACTC GCTACAATGC CAACAATGTC GATTTGAACC GTAACTTCAA GGATGATGTA 900
GCCGGTGATC ACCCCGATGG AAAACCTTGG CAGCCGGAGG CAACTGCATT CATGGATTTG 960
GAAGGAAACA CCTCTTTCGT GCTCGGTGCC AATATACATG GAGGAACAGA GGTGGTGAAC 1020
TATCCATGGG ATAATAAAAA AGAAAGACAT GCAGACGATG AGTGGTACAA ACTGATCAGT 1080
CGCAACTACG CAGCCGCTTG TCAGAGTATT TCCGCCAGCT ACATGACCTC CGAAACCAAT 1140
TCGGGAATCA TCAACGGTTC AGACTGGTAT GTAATTCGCG GAAGTCGTCA GGACAATGCA 1200
AATTATTTCC ATCGTCTGCG AGAAATTACC CTTGAAATCA GCAACACGAA GTTGGTGCCG 1260
GCCTCTCAAC TTCCAAAGTA TTGGAATCTG AACAAAGAAT CTCTGCTTGC TCTGATCGAA 1320
GAATCCTTAT ACGGCATCCA TGGTACAGTG ACTTCCGCTG CGAACGGACA GCCTCTCAAA 1380
TGCCAGATCT TGATAGAAAA CCATGACAAG CGCAACTCCG ATGTTTACTC CGATGCTACC 1440
ACAGGCTACT ACGTACGTCC TATCAAAGCC GGCACTTATA CGGTGAAATA CAAAGCCGAG 1500
GGTTATCCTG AGGCAACTCG TACCATTACG ATCAAGGACA AAGAAACCGT CATCATGGAC 1560
ATTGCATTGG GCAACTCGGT TCCTCTGCCT GTACCCGATT TCACAGCTTC TCCTATGACC 1620
ATCTCAGTAG GCGAAAGCGT CCAATTCCAA GATCAAACGA CAAATAACCC CACGAATTGG 1680
GAGTGGACGT TCGAAGGCGG ACAGCCTGCC ATGAGTACAG AGCAGAATCC GCTCGTATCC 1740
TATAGTCATC CCGGTCAGTA CGACGTTACG CTCAAAGTGT GGAATGCAAG TGGTTCCAAC 1800
ACGATTACGA AAGAAAAATT CATCACTGTC AATGCCGTTA TGCCTGTAGC TGAATTCGTC 1860
GGTACCCCGA CGGAAATAGA AGAGGGCCAG ACGGTATCTT TCCAAAACCA ATCCACCAAT 1920
GCCACCAACT ACGTATGGAT ATTCGATGGC GGCACTCCCG CTACCAGTGA AGACGAAAAC 1980
CCGACTGTGC TTTACAGCAA AGCCGGCCAA TACGATGTCA CGCTCAAGGC GATCAGTGCT 2040
TCCGGTGAAA CGGTGAAGAC GAAAGAAAAA TACATCACTG TCAAGAAAGC TCCGGTCCCT 2100
GCTCCGGTAG CCGACTTCGA AGGAACACCT CGAAAAGTAA AGAAAGGCGA GACAGTTACT 2160
TTCAAAGACT TGTCTACGAA CAATCCGACT TCATGGCTTT GGGTGTTCGA AGGCGGCTCT 2220
CCTGCCACCA GCACGGAGCA AAACCCGGTG GTCACCTACA ATGAAACAGG CAAGTACGAT 2280
GTCCAGCTGA CTGCCACCAA CGAGGGCGGA AGCAATGTGA AGAAAGCAGA AGACTACATT 2340
GAGGTTATCC TCGATGACAG TGTCGAGGAC ATAGTGGCAC AGACGGGTAT CGTCATTCGT 2400
CCGCAAAACG GAACGAAGCA GATCCTCATA GAAGCCAACG CTGCTATCAA AGCGATCGTT 2460
CTCTATGACA TCAATGGACG GGTCGTACTC AAAACTACTC CGAATCAGCT CCGCTCGACC 2520
GTAGATCTTT CCATCCTGCC CGAAGGAATC TACACCATCA ATATCAAAAC GGAAAAATCC 2580
GCTCGCACGG AAAAGATCCA TATCGGG 2607

(2) INFORMATION FOR SEQ ID NO:38

(i) SEQUENCE CHARACTERISTICS:
(A) LENGTH: 318 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:

(A) ORGANISM: PORYPHYROMONAS GINGIVALIS

(ix) FEATURE:
(A) NAME/KEY: misc_feature
(B) LOCATION 1...318

(xi) SEQUENCE DESCRIPTION: SEQ ID NO:38

CTTTCGTACA GTGGAGAGAG CGATGCAAAA GAGTCTGATC AGAATTGCCG GAAATGTACC 60
TTCATCGGCT TTGAAAAACG AGTAAATACG ATGCGACTGA TCAAGGCTTT TCTCGTGCAA 120
CTCTTACTGC TCCCCATTTT CTTCTACAAG CGGTTTATAT CGCCGCTTAC ACCGCCTTCA 180
TGCCGGTTTA CCCCCTCATG TTCGTCCTAT GCCATCGAAG CCTTACGTAA ATATGGCCCG 240
GGCAAAGGAC TATTGCTGAG CATCAAGCGT ATTCTCCGCT GTCACCCGTG GGGTGGAAGT 300
GGCTATGACC CCGTTCCG 318

(2) INFORMATION FOR SEQ ID NO:39

(i) SEQUENCE CHARACTERISTICS:
(A) LENGTH: 2583 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2583

(xi) SEQUENCE DESCRIPTION: SEQ ID NO:39

TTAGAGATGG CATACGACTT TACACAAACA TTCCGCAACA GCCTGGAGTA CAGCTATCAG 60
GAAGCAACCC GTCTCGGCGT CGTAGCCGTG ACGCAAGATA TGCTCGTACT CGGTATCATT 120
CGCGACGGAG ACAATGGCGC GATCGACATC ATGCGGCACT ATGGGATCAA CTTGTACGAA 180
CTCAAACGGT TGATCGAGTT GGAAGCCATC GCCGAGAGTT TGCCTGCTTC GCCTGAGGGA 240
TCGCCCATCT TCACCCCTTC GGCTCGGGAG GCTATCGATG ATGCCACAGA CATCTGTGCC 300
GACATGGAGG ACGAGGCCGT CAGCCCGGTC CATCTGTTGC TGAGTATCCT CAACTCGACA 360
CAGGAGAGCT TAGTACAAAA GATATTTATG AAACAAGGTA TAAAATACGA CACCATCCTG 420
TCGGATTACT TCGGACAGCG CAACCCCTCC GAAGGGAAGT CTCCCTCCGA AATGGAGATC 480
CTCGACGGGT ACCAAGACAA CGACTTCGAC GACGAAGAGG ACGAATCCTC TCCGCCTTCC 540
GGGAATAGCG GGACAGGCGG AGGCTCCGGC GACGCCCCCG AACAGAATAC CGGCGGAGGC 600
GATACTACCA CCACGACACG GAGTGGAGGC GACACGCCTG CACTGGACAC CTTCGGCACC 660
GACATCACTG CCATGGCGGC AGCAGGCAAG CTCGACCCGG TAGTGGGTCG GGAGCAGGAG 720
ATCGAAAGGG TGATACAGAT ACTCAGCCGG CGCAAAAAGA ACAATCCGGT GCTCATCGGC 780
GAACCCGGTG TAGGCAAGAG TGCCATCGTG GAAGGACTGG CCGAACGCAT CGTGAACAGG 840
AAGGTGAGCC GTATTCTTTT CGACAAGCGG ATCATCAGCC TCGATTTGGC TCAGATGGTA 900
GCCGGCACCA AATATCGCGG ACAGTTCGAA GAGCGGTTGA AAGCCGTGCT CGATGAGCTG 960
AAGAAGAATC CGCAGATCAT CCTCTTCATC GACGAGATAC ATACCATCGT GGGAGCAGGC 1020
TCTGCAGCCG GATCGATGGA TACGGCCAAT ATGCTCAAAC CCGCTCTTGC CCGTGGACAG 1080
GTACAGTGCA TCGGAGCCAC TACGCTGGAT GAGTATCGTA AGAACATAGA AAAGGACGGA 1140
GCACTCGAAC GCCGCTTCCA GAAGGTGCCG ATAGCCCCCT CGACTGCAGA AGAAACGCTG 1200
ACCATCCTGC AAAACATCAA AGAGAAATAC GAGGACTATC ACGGTGTACG CTATACGGAC 1260
GAAGCGATCA AAGCGGCAGT GGAACTGACC GATCGCTATG TATCCGATCG TTTCTTCCCA 1320
GATAAGGCGA TAGATGCCAT GGACGAGGCC GGCGCGAGCG TCCATATCAC CAATGTGGTG 1380
GCTCCGAAAG AAATCGAGAT ACTGGAGGCC GAATTGGCAT CGGTGCGAGA GAACAAGCTC 1440
TCGGCCGTAA AGGCTCAGAA CTACGAACTG GCTGCCTCCT TCCGCGATCA GGAGCGGCGC 1500
ACTCAGCAGC AGATAGCGGA AGAGAAGAAA AAATGGGAAG AGCAGATGTC CAAGCACCGC 1560
GAGACGGTGG ACGAGAATGT AGTGGCGCAT GTAGTGGCGT TGATGACAGG CGTTCCGGCT 1620
GAGCGGCTGA GCACGGGCGA AGGCGAACGT CTGCGCACGA TGGCAGATGA TCTCAAGACC 1680
AAAGTAGTAG GTCAGGACAC AGCCATCGAA AAGATGGTGC ATGCCATCCA GCGCAATCGT 1740
CTGGGACTTC GCAATGAAAA GAAACCGATC GGTTCTTTCC TTTTCCTCGG CCCCACGGGG 1800
GTAGGCAAGA CCTATTTGGC CAAGAAGCTC GCCGAATACC TGTTCGAGGA TGAGAATGCC 1860
ATGATCAGGG TGGATATGAG CGAGTATATG GAGAAGTTCT CCGTTTCGCG TCTCGTGGGT 1920
GCCCCTCCGG GATATGTGGG CTATGAAGAA GGCGGCCAAC TGACGGAGCG CGTAAGACGC 1980
AAACCCTATT CCGTGGTTCT CTTGGATGAG ATCGAAAAGG CGCATGCCGA TGTCTTCAAT 2040
CTGCTCTTAC AGGTGATGGA CGAAGGTCAG CTGACCGACA GTCTGGGACG GCGCGTGAAT 2100
TTCAAGAACA CCGTGATCAT CATCACCTCC AACGTGGGTA CACGCCAGCT CAAAGACTTC 2160

GGGCAGGGTA TCGGGTTCCG TTCGGAAAAA GACGAGGAAG CGAACAAGGA GCATAGCCGT 2220
TCCGTGATCC AAAAAGCTCT GAACAAGACG TTCAGCCCCG AATTTCTCAA CCGTTTGGAC 2280
GATATCATCC TCTTCGACCA ACTGGGCAAG ACGGAGATTC GCCGGATGGT GGACATAGAG 2340
CTTAAAGCCG TCTTGGCGCG CATCCATCGT GCCGGATACG ACCTCGTCCT TACCGATGAA 2400
GCCAAGGATG TGATAGCGAC GAAGGGATAC GACCTCCaAT ACGGAGCACG ACCGCTCAAG 2460
CGCACACTCC AGAACGAAGT GGAGGATCGC CTCACGGATC TTATCCTCTC CGGACAGATC 2520
GAGAAAGGGC AGACGCTTAC GCTCTCTGCT CGCGATGGCG AGATCATCGT ACAAGAACAA 2580
GCA 2583

(2) INFORMATION FOR SEQ ID NO:40

(i) SEQUENCE CHARACTERISTICS:
(A) LENGTH: 1368 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1368

(xi) SEQUENCE DESCRIPTION: SEQ ID NO:40

AGAGGAGGAC AAATCCGCCG ACACCATACC GATTCGTCAA GGGGATCGGA CAGCAAAGCC 60
TGCCGCTTCT CCTTTCATGC CGAGACCATC GGTTTCTCCA ACCACCAAAG AGCAAAAATG 120
AATTACCTGT ACATACTGAT TACACTTTTA CTCTCCGGCT TTTTTTCCGG TGCTGAGATT 180
GCTTTCCTTT CTTCGGACAA ACTGCGTCTT GAGTTGGACA GGAATAGAGG CGATCTCACA 240
GGCAGAGCGT TAAACTTGCT GTATCGACAT CCGGACCAAC TGGTGACTAC TCTCCTTGTG 300
GGTAATAATA TCGTTTTGGT AGTCTATGGT CTGCTGATGG CGGGATTGCT GGCCGCACCT 360
TTGGCGCAAT GGATTGATAA CGATGCTATG ATCGTCGTTC TCCAATCTGT CTTATCCACT 420
ATCATCATAC TGTTTACCGG GGAATTTCTA CCCAAAGCCA TTTTCAAGAC CAATGCCAAT 480
ATGATGATGA GGGTATTCGC CCTCCCTATC GTAGCGATCT ATTATCTGCT TTATCCTCTG 540
TCTAAACTCT TCACCGGTTT ATCTCGCTCT TTTATTCGTC TGGTGGACAA GAATTATGTG 600
CCTACAACAG TAGGGTTGGG GCGCGTAGAT CTCGATCATT ATTTGGCAGA AAATATGTCC 660
GGAGAAAACG AACAGAACGA CTTGACTACC GAAGTGAAAA TCATCCAGAA TGCGCTGGAT 720
TTTTCCGGTA TTCAGGTGCG AGACTGCATG ATCCCACGCA ATGAGATGAT AGCATGTGAG 780
TTGCAAACGG ATATTGAAGT ACTCAAAACG ACTTTTATCG ATACCGGTTT GTCCAAGATC 840
ATTATCTACA GACAGAACAT AGATGACGTA GTAGGATATA TCCATTCGAG CGAAATGTTT 900
CGTGGGCAAG ACTGGCAAAA ACGTATCAAT ACTACTGTAT TCGTACCCGA AAGCATGTAT 960
GCCAATAAAC TGATGCGACT ACTCATGCAG CGCAAGAAAA GCATTGCGAT CGTCATCGAT 1020
GAACTTGGAG GTACGGCCGG AATGGTCACA TTAGAGGATT TGGTAGAAGA GATTTTCGGT 1080
GACATTGAGG ACGAACACGA CACTCGCAAG ATCATAGCCA AACAGCTCGG CCCTCATACC 1140
TATCTGGTCA GTGGTCGTAT GGAAATAGAT GATGTGAACG AACGTTTTGG GTTGTCCTTG 1200
CCTGAGTCTG ACGACTACCT TACCGTGGCC GGATTTATCC TGAATAGCCA TCAAAATATC 1260
CCACAGGCCA ATGAGGTCGT GGAGATTGCT CCTTATACTT TTACCATTCT CAGATCTTCT 1320
TCCACCAAGA TCGAACTGGT GAAAATGTCC ATCGACGACC AATCGAAC 1368

(2) INFORMATION FOR SEQ ID NO:41

(i) SEQUENCE CHARACTERISTICS:
(A) LENGTH: 897 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...897

(xi) SEQUENCE DESCRIPTION: SEQ ID NO:41

TTAAAAAAGG AGATAACTAT GAAACAGAAC TACTTCAAAA GAGTCTGCTC ACTGCTTTGG 60
CTGGTTTTAC CCATGCTTAT TATGCCATTG GAAGTAGCAG CTCAAGAGAT TATTCCGAAC 120
GAAGAGCTGT TGGAATCATT GACTTTCGTT GCACCGGTTG AGGAGACAGA CGCAATAGAG 180
GCAGAGGTAG AAGCTCTGCA GGAGATAGTC GCTACTGAGG AGATTGCGGA GCAGGCTGTT 240
CGTTCTTATA CCTACACGGT CTATCGTGAT GGCGTGAAGA TTGCTTCAGG ATTGACTGAG 300
CCCACTTTTC TCGATGAAGA TGTTCCTGCC GGCGAACATA CCTACTGCGT AGAAGTACAG 360
TATCAGGGAG GCGTATCCGA CAAAGTATGC GTGGACGTAG AGGTGAAGGA CTTCAAACCG 420
GTTACCAATC TCACCGGAAC TGCTTCCAAT GACGAAGTTT CTTTGGACTG GGACGGTGTG 480
GAAGAGAAAG CTGAAGAGCC GGCAAGTGAT AAAGCAGTCA GCTACAACGT CTACAAGAAT 540
GGAACCTTGA TCGGTAATAC AGCTGAAACT CATTATGTGG AGACCGGTGT AGCCAATGGT 600
ACATACATCT ACGAAGTGGA AGTAAAGTAT CCTGACGGTG TATCTCCGAA GGTGGCTGTA 660
ACCGTGACCG TGACCAACAG CTCATTGAGC AATGTAGATG GACAGGCTCC TTACACATTG 720
CGAGTAGAAG GCAAGAAGAT TATTGCGGAA GCCCATGGTA TGATCACGCT CTACGACATC 780
AACGGACGTA CCGTGGCCGT AGCCCCGAAT CGATTGGAAT ACATGGCGCA AACCGGTTTC 840
TATGCAGTGC GCTTCGATGT GGGGAATAAA CACCATGTAT CGAAAATACA AGTAAGA 897

(2) INFORMATION FOR SEQ ID NO:42

(i) SEQUENCE CHARACTERISTICS:
(A) LENGTH: 1131 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1131

(xi) SEQUENCE DESCRIPTION: SEQ ID NO:42

TTGTTTAATC ATAAAAAATC ATGGTATGAA ACATTTCAAT TTTATCTCGT TGTTTTCCGC 60
TCTGGCTTTA TTCTTTTGTG TGAAAATACC CTTGCACAAC AAAAAACAGA GGAGTTTGCA 120
CCTGTGTCGG ATTTACGTGC AGAAGCGTAC GGCTCTACCG TTTTCCTCCA CTGGACTCCG 180
CCGTATGACA ATCCGATGAT TCCTCTAAGC GAGAGTTTTG AATCAGGTAT TCCAGCTATA 240
TGGAAGACCA TTGACGCAGA TGGCGATGGC TATAATTGGA TGCATTTGAC CAATTTCACG 300
GGACAGAGTG GTCTCTGTGT CTCTTCGGCT TCATACATAG GCGGCGTCGG AGCTTTGACT 360
CCGGACAATT ATCTGATAAC ACCCGAATTA AAACTACCCA CAGACGCGTT GGTGGAAATA 420
ATCTATTGGG TATGTACTCA AGATCTCACT GCTCCATCGG AGCACTATGC CGTTTATTCC 480
TCTTCTACAG GCAATAATGC TGCTGACTTT GTTAATCTCT TATATGAAGA GACTTTGACT 540
GCCAAACGGA TACAATCCCC CGAGTTGATC CGCGGAAATC GGACACAAGG TGTTTGGTAT 600
CAAAGAAAGG TGGTACTCCC TAACGATACT AAATATGTTG CTTTCCGCCA TTTTAATTCC 660
ACGGATAATT TCTGGCTCAA TTTGGATGAA GTATCTATCC TGTATACCCC TCTTCCCCGA 720
AGAGCTCCGT GTCCGCATCC GGGTGGTTAC ACTTATTCTG TATTCCGTGA TGGACAAAAG 780
ATAGCGAGTG GATTGTCGGC ATTGGCATAT ATCGATACGG ATGTACCGTA TGGGACTCAA 840
GACTATTGTG TCCAAGTCAA TTATCTGCAA GGAGACTCGT ATAAAGTCTG CAAAAATATA 900
GTGGTGGCAA ATTCTGCAAA CATCTATGGG GCGGATAAGC CTTTTGCGTT GACCGTGGTT 960
GGCAAGACCA TTGTAGCGAG TGCTTTCAAA GGAGAGATCA CTCTTTATGA CATTCGTGGC 1020
CGGCTGATAG CTTCCGGCTG CGATACGCTT AGGTACAAAG CGGAAAATGG TTTTTACCTC 1080
ATTAAAATAC AGGTAAACGG AACTGTCTAT ACTGAGAAAA TCCAAATCCA A 1131

(2) INFORMATION FOR SEQ ID NO:43

(i) SEQUENCE CHARACTERISTICS:
(A) LENGTH: 2547 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2547

(xi) SEQUENCE DESCRIPTION: SEQ ID NO:43

TTCGGAATAT CACCTTCAAT GAAAAAAAGT TTTCTTTTAG CCATAGTAAT GCTCTTTGGC 60
ATTGCCATGC AGGGACATTC TGCTCCGGTT ACGAAAGAGC GAGCTTTGAG TCTGGCTCGG 120
CTGGCTTTGC GACAGGTATC CTTGCGAATG GGACAAACAG CAGTATCTGA CAAGATTTCC 180
ATCGATTACG TTTATCGGCA AGGAGATGCT GAGAGGGGTA TCACATCACA AGAGGAAGGC 240
TCTCCTGCAT ATTTTTATGT AGCTAATCGT GGAAATAATG AGGGCTATGC TCTTGTAGCA 300
GCAGATGACA GAATACCGAC AATTTTAGCC TATTCACCCA TTGGCCGTTT CGACATGGAC 360
AGTATGCCGG ACAATCTTCG CATGTGGCTA CAAATTTACG ATCAGGAAAT AGGCCTGATA 420
CTTTCCGGAA AAGCTCAGCT CAATGAAGAG ATATTACGTA CCGAGGGCGT ACCGGCTGAA 480
GTACATGCTC TGATGGATAA CGGTCATTTT GCCAACGATC CCATGCGATG GAATCAAGGT 540
TACCCATGGA ACAATAAGGA ACCACTGCTT CCTAATGGCA ATCATGCCTA TACCGGCTGT 600
GTTGCTACTG CTGCAGCACA AATCATGCGC TACCATAGCT GGCCGCTTCA AGGTGAAGGC 660
TCTTTCGATT ATCATGCAGG TTCATTAGTT GGCAACTGGT CCGGCACATT TGGTGAAATG 720
TACGACTGGA TCAATATGCC CGGAAATCCC GACCTTGATA ATCTGACTCA ATCTCAAGTG 780
GATGCCTACG CCACACTGAT GCGTGATGTG AGTGCATCTG TTTCGATGAG TTTTTATGAA 840
AATGGAAGTG GTACGTACAG CGTTTATGTA GTAGGAGCCT TGCGAAACAA CTTTCGCTAC 900
AAGCGTTCAC TGCAGCTACA TGTACGCGCC TTATATACCT CACAGGAGTG GCACGATATG 960
ATCCGCGGGG AACTTGCCTC CGGAAGGCCG GTCTATTATG CAGGGAATAA CCAGAGCATA 1020
GGACATGCTT TCGTTTGCGA TGGTTATGCT TCGGATGGTA CTTTCCATTT CAACTGGGGT 1080
TGGGGAGGTG TTTCCAACGG CTTCTACAAA CTAACACTCC TCTCGCCGAC TTCGTTGGGT 1140
ATCGGAGGTG AGGGAATAGG TTTTACCATT TATCAAGAGA TCATCACCGG TATCGAACCG 1200
GCTAAGACTC CCGCTGAAGC CGGTACAGAT GCCTTGCCGA TCTTGGCACT GAAAGACATA 1260
GAAGCCGAGT ATAAAAGTGA ATCCGGATTG AACGTAGGGT ATTCGATATA TAATACAGGT 1320
GAAGAGCAAT CAAATCTTGA CCTCGGATAC AGATTGAACA AGGCTGACGG AGAAGTCATA 1380
GAGGTGAAAA CTTCATCTAT CAATATCTCT TGGTACGGAT ACGGAGAGCA TCCCGAGAGT 1440
TTCTCATTGG CACCTAATCA GTTGTCACAA GGAATCAACA CCATCACCCT ACTTTATCGT 1500
CGCACAGGCA CCGAACAGTG GGAGCCGGTA CGGCATGCAC AGGGAGGATA TGTCAATAGC 1560
ATTAAAGTAA ATACGACAGA CCCGAACAAT GTCGTAGTCA CGGTAGATAA TAACGAAGGC 1620
AAGCTCAGTA TCGTCCCCAA CAGCTTTGTC GCAGATCTGA ATTCTTATGA ACATAGTACG 1680
ATTACAGTAC AGTTCAATAG CGACAGCCCT GATGAGATCC GTACACCCGT AGCCTTTGCT 1740
CTATCTACAG GAGCTACTGC GGACGATGTA ATATCTTTGG GCTGGGTAAT GGCTGAAGTT 1800
CCGGGCGGTA GCAGCAACTA TCCGGTGGTT TGGTCTAAAG ACGTTCTCAC TCTCTCGGAA 1860
GGCGACTATA CATTGTGGTA TAGATTTTCC ATCAACAACC AAAAGGATGA ATGGAAAAAG 1920
ATCGGAAGCG TGTCAGTAAA AACACCGACA GAGTATACGC ACCCCTTATT CGAAGTGGGC 1980
CATAATCAAA CTTCTACCTA TACGCTGGAT ATGGCACACA ACAGAGTATT GCCCGACTTT 2040
ACACTCAAAA ATCTCGGATT GCCTTTCAAT GGTGAGTTGG TTGTTGTTTT CCGCCAAACA 2100
CAATCCTCAT CGGGGTCTTT ATGGGCAGCT CAAGAAACAG TACATATCAA GCAAGGAGAA 2160
ACTTTCGTAT ATAAACCTGT TGTCGAAGGC CCTATACCTG ATGGATCCTA TCGTGCGACC 2220
CTCCATGCAT TCGTAAACGG ACAACAACAG TTGTACCTCA AGGGGAAAAG GAACTACACG 2280
GTGAAGATCG TCAATGGTAC AGCGGTAGAA GCAATAGAAT CGTCAGAAGA GATCAGAGTA 2340
TTCCCTAATC CGGCACGCGA TTATGTGGAA ATATCGGCAC CTTGCATTCC CCAAGAAACA 2400
TCTATCATTC TTTTCGATCT GTCAGGCAAG ATTGTCATGA AGAATAGTTT ATCAGCGGGG 2460
CATGGCAGAA TGGATGTCAG CCGACTTCCT AATGGGGCCT ACATCCTTAA GGTGGATGGA 2520
TATACGACGA AAATAAATAT AGTGCAC 2547

(2) INFORMATION FOR SEQ ID NO:44

(i) SEQUENCE CHARACTERISTICS:
(A) LENGTH: 885 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:

(A) ORGANISM: PORYPHYROMONAS GINGIVALIS

(ix) FEATURE:
(A) NAME/KEY: misc_feature
(B) LOCATION 1...885

(xi) SEQUENCE DESCRIPTION: SEQ ID NO:44

ACGAAGAAAA CACTGATGAA AAAGCTATTT CTCTCGCTCA CGAGTCTTGT AATGGTCTTC 60
GCTGTTGCAA GTTGCGATAT AATCGACAAG GATCAAACCC TCTTGCCGGC TCCGACCAAT 120
GTGACACCCG ATAATCCGGA TGACAATCCT TCGGAGATCG ACATTACGCA GACGCACACA 180
GAAAAATATG TTTTGGCTGA AGAATTTACC GGCCAAAAAT GTCTCAACTG TCCGAAAGGT 240
CATCGCAAAC TGGCGGCTCT CAAGGAGCAA TACGGTAAGA GATTGACTGT TGTCGGTATA 300
CATGCCGGCC CTGGATCTCT CGTGCCACCT CTTTTCCGTA CAGAAGCCGG AGACGCATAT 360
TATAGCAAGT TCGCCAATAA TACCCCTCTC CCTGCGCTGA TGGTTTCGCG CAAAAAGTTC 420
GGCTCTTCCT ACGTTTATGA TAAGAGCTAC AAAACGTGGG ACGTGCCTAT TGCCGAGCAG 480
ATGGAGCAAA AGGCGAAGAT CAATATCTTT GCCGTGGCCC AATACACCGA TACCCAAAAG 540
ATCAAGGTGA CTGTAAAGGG TAAAATACTG GAGGGGAATA CACTCCCGAA GTCCATGGTT 600
CAGGTGTATC TGTTGGAGGA TAAGCTGATC GCTCCGCAGG TGGATGGCAA TACGACAGTC 660
GAGAATTACG AGCACAATCA CGTGTTGCGT GGAGCCGTTA ATGGTATTTG GGGCGAAGAA 720
TTTGTGAATC TCAAAGATTA TTTGTATACT TACGCCGTTG AACCGCTCTC GGGTATGTCC 780
TTCGTAGCCG AGAATTATTC GATTGTGGCT TTTGTATACG ATGTGCAGAC GTTCGAAGTG 840
TATGACGTTG TGCATGTAAA GATCAATCCG CAATCCGATG GCAAA 885

(2) INFORMATION FOR SEQ ID NO:45

(i) SEQUENCE CHARACTERISTICS:
(A) LENGTH: 690 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...690

(xi) SEQUENCE DESCRIPTION: SEQ ID NO:45

ACGAATAAAA AAGAAGAGAC AATGAAGAAA TCAAGTGTAG TAGCCTCAGT TTTGGCCGTG 60
GCTCTCGTGT TCGCCGGTTG CGGACTGAAC AATATGGCAA AAGGCGGCCT TATCGGCGCC 120
GGAGTAGGAG GTGCCATTGG TGCCGGAGTA GGTAACGTAG CCGGAAATAC GGCTGTCGGT 180
GCCATCGTCG GTACTGCAGT CGGTGGAGCA GCCGGTGCTC TCATCGGAAA GAAGATGGAC 240
AAGCAGAAAA AAGAACTGGA GGCCGCAGTA CCCGATGCTA CGATTCAGAC AGTAAATGAC 300
GGAGAGGCTA TTCTGGTTAC TTTCGATAGC GGTATCCTCT TTGCGACGAA CTCCAGCACT 360
CTGAGTCCCA ACTCACGCAC TGCGCTGACG AAGTTTGCTG CAAACATGAA CAAAAACCCC 420
GACACGGATA TTCGTATCGT AGGCCATACG GACAATACCG GCTCCGACAA GATCAACGAT 480
CCTCTGTCTG AGAGACGTGC AGCCAGCGTA TATTCTTTCC TGAATTCTCA GGGTGTGAGT 540
ATGTCGCGCA TGGCAGCCGA AGGGCGTGGG AGCCATGAAC CGGTTGCAGA CAATAGCACA 600
GTTGCCGGAC GTTCGGCCAA CCGCCGTGTG GAGGTTTATA TCTTGCCGAA TGCCAAGATG 660
ATCGAACAAG CACAGCAAGG TACGCTGAAG 690

(2) INFORMATION FOR SEQ ID NO:46

(i) SEQUENCE CHARACTERISTICS:
(A) LENGTH: 1026 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc feature
(B) LOCATION 1...1026

(xi) SEQUENCE DESCRIPTION: SEQ ID NO:46

AACAGGAACA GAAATATGTC GAAAAAATCG ATCCTTCTGC TTTGCTGTTC GCTGTGCTTC 60
ATTTCTGCTA CGAAGGCTGT GACCCCCGTC AGAAATGTGC GCAATAGCCA AGTGAACAGC 120
AAAGCAAAGA CCGAACGTAC AAAGCCCTCG GACTCTGTAC GGTACATTAG CAACATGATT 180
GCAGATCGGC TGGAGTTCCG CAACAAGATT TCTTCCGAAA AAGAGGTAAG AAAAGCCGAA 240
TATGAAAATC GGCTGGCGAT GGAAGCACTC AATTACCCTG CCATAGATTT ATATGGTGAA 300
GATTCTTGGA GCGAGTATGT AAACCCTTTC GTGGGTGCAG GAACCGATGT CGAAATTCCG 360
AACTCCTATG ACATTGATTG CTCTTCGTTC GTGATGCCCG TCGAAGATAA GCAGGTCACC 420
TCTCAATTTG GCTACCGTCG GCGTTTCGGA CGGATGCACT ATGGTATTGA TCTTTCAGTG 480
AATCGTGGCG ATACGATACG AGCAGCCTTT GACGGGAAAG TTCGTGTACG CAGCTATGAA 540
GCGCGTGGCT ATGGCTACTA CATAGTCTTG CGCCATCCGA ACGGACTGGA GACTGTGTAC 600
GGACACATGA GTCGCCAATT GGTAGACGAG AATCAGATCG TTCGAGCAGG ACAACCGATC 660
GGATTAGGAG GCAGCACGGG TCGAAGCACC GGTCCTCATC TTCACTTCGA GACCCGCTTC 720
ATGGGTATTC CCATCAATCC GAGTACCATT ATAGACTTCG ATAACGGAGT GCCGCTCCGA 780
GACATTTACA CATTCAAACG AGGGAGCAAT TCTCGCTATG CAAAAGCCTC TAAGACTTCT 840
TCTCGCTATG CAAAAAAAGG GAAGAAAGGC AGACAAGCTT CTTCTCCTAT GACCTATAGA 900
ATCAAAAAAG GCGATACTTT GGAAACAATA GCCAAAAGGC ACGGCACTTC TGTTCAGAAA 960
CTCTGTGCTA CCAATGGCAT TGGCAAGAGT AAAATTTTCA CTCCGGGCAA AGCCTTGAGG 1020
ATCAAA 1026

(2) INFORMATION FOR SEQ ID NO:47

(i) SEQUENCE CHARACTERISTICS:
(A) LENGTH: 477 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...477

(xi) SEQUENCE DESCRIPTION: SEQ ID NO:47

CCGAGCAAAA CGATAATTAA GACAATGGCA AAAATCAATT TCTATGCTGA AGGCGTCAGC 60
CTTCCTCGGA TCAGAAGACG GATCGTCGGT AAGTGGATAG CCGAAGTATG CAGCCGATAT 120
GGGAAAGCGG TGGGAGAAAT CTCCTATCTT TTCTGTGATG ACGAATATAT CCTGAAAGCC 180
AATCAGGAAT TTCTCGATCA TGACTACTAC ACCGACATCA TCACCTTCGA TTCCTGCGAA 240
GCGGATACGG TGAATGGCGA CCTGCTTATC AGTCTCGATA CCGTACGCTC GAATGCCCGT 300
GCTCTTGATC TTCGATACGA AGACGAACTG CATCGTGTCA TTATCCACGG CATACTGCAT 360
CTTTGCGGAT TGAAAGACAA GAGCAAAAAG GATGAAGCCC AAATGCGTGC AGCCGAAGAG 420
AAAGCCCTTG TCATGCTGCG AGAAACCATC GGATCGGAGC TTTCCCTATT GCATACA 477

(2) INFORMATION FOR SEQ ID NO:48

(i) SEQUENCE CHARACTERISTICS:
(A) LENGTH: 1185 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1185

(xi) SEQUENCE DESCRIPTION: SEQ ID NO:48

AAAAGTAAAA CTATGAAGGT AAAGTACTTA ATGCTCACAT TGGTTGGAGC AATTGCACTG 60
AACGCAAGTG CACAGGAGAA TACTGTACCG GCAACGGGTC AGTTACCCGC TAAGAATGTT 120
GCTTTTGCTC GCAATAAAGC AGGCAGCAAT TGGTTTGTAA CACTGCAAGG CGGTGTTGCA 180
GCGCAGTTCC TCAATGACAA CAACAACAAA GACCTCATGG ACCGCTTAGG AGCCATAGGT 240
TCTCTTTCTG TCGGAAAGTA TCACAGCCCT TTCTTTGCAA CTCGTTTGCA AATTAACGGA 300
GGTCAAGCCC ACACTTTCCT CGGAAAAAAT GGCGAACAAG AAATCAACAC CAATTTTGGT 360
GCAGCTCACT TCGACTTTAT GTTTGATGTG GTTAACTACT TTGCACCATA TCGCGAAAAT 420
CGTTTCTTCC ATTTAATTCC ATGGGTAGGT GTTGGCTACC AACACAAATT CATCGGTAGC 480
GAATGGAGCA AAGACAATGT GGAATCACTG ACGGCGAATG TAGGAGTTAT GATGGCTTTC 540
AGATTAGGAA AGCGAGTAGA CTTTGTGATC GAAGCACAAG CAGCTCACTC CAATCTCAAT 600
CTAAGTCGCG CATACAATGC CAAGAAAACT CCCGTATTCG AAGATCCCGC AGGACGTTAT 660
TACAATGGAT TCCAGGGGAT GGCTACAGCA GGTCTTAATT TCCGCCTGGG AGCCGTAGGC 720
TTCAATGCCA TTGANCCAAT GGACTACGCA CTTATCAATG ATCTGAATGG TCAGATTAAC 780
CGTTTGCGCA GCGAGGTCGA AGAACTCTCA AAACGTCCTG TATCATGCCC CGAATGTCCT 840
GAAGTAACTC CTGTTACTAA GACAGAAAAT ATACTGACGG AAAAAGCTGT ACTGTTCCGT 900
TTCGACAGCC ACGTTGTGGA CAAAGATCAA TTGATCAACC TGTATGACGT AGCTCAGTTT 960
GTAAAAGAAA CTAACGAGCC GATTACCGTT GTTGGTTATG CTGATCCTAC GGGTAATACT 1020
CAATACAACG AGAAATTGTC TGAGCGTCGG GCTAAAGCCG TTGTTGATGT TCTGACAGGT 1080
AAATATGGTG TGCCTTCCGA ATTAATCTCT GTAGAATGGA AGGGCGACTC TACGCAACCG 1140
TTCAGCAAGA AAGCTTGGAA TCGTGTTGTA ATCGTTCGCT CCAAG 1185

(2) INFORMATION FOR SEQ ID NO:49

(i) SEQUENCE CHARACTERISTICS:
(A) LENGTH: 1161 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1161

(xi) SEQUENCE DESCRIPTION: SEQ ID NO:49

TATAAAATGA CATACAGAAT TATGAAAGCT AAATCTTTAT TATTAGCACT TGCGGGTCTC 60
GCATGCACAT TCAGTGCAAC AGCCCAAGAA GCTACTACAC AGAACAAAGC AGGGATGCAC 120
ACCGCATTCC AACGTGATAA GGCCTCCGAT CATTGGTTCA TTGACATTGC AGGTGGAGCA 180
GGTATGGCTC TCTCGGGATG GAATAATGAT GTAGACTTTG TAGATCGTCT AAGTATCGTT 240
CCTACTTTCG GTATCGGTAA ATGGCATGAG CCTTATTTCG GTACTCGTCT CCAATTCACA 300
GGATTCGACA TCTATGGATT CCCGCAAGGG AGCAAGGAGC GTAACCACAA TTACTTTGGA 360
AACGCCCACC TTGACTTCAT GTTCGATCTG ACGAACTATT TCGGTGTATA CCGTCCCAAT 420
CGTGTCTTCC ATATCATCCC ATGGGCAGGT ATAGGATTTG GTTATAAATT CCATAGCGAA 480
AACGCCAATG GTGAAAAAGT AGGAAGTAAA GATGATATGA CCGGAACAGT TAATGTCGGT 540
TTGATGCTGA AATTCCGCCT ATCAAGACTC GTAGACTTCA ATATTGAAGG ACAAGCTTTT 600
GCCGGAAAGA TGAACTTTAT CGGGACAAAG AGAGGAAAAG CAGACTTCCC TGTAATGGCT 660
ACAGCAGGTC TAACGTTCAA CCTTGGCAAG ACAGAGTGGA CAGAAATTGT TCCTATGGAC 720
TATGCTTTGG TCAATGACCT GAACAACCAA ATCAACTCAC TTCGCGGTCA AGTGGAAGAG 780
TTGAGCCGTC GTCCTGTTTC ATGCCCTGAA TGCCCTGAGC CTACACAGCC TACAGTTACT 840
CGTGTAGTCG TTGACAATGT GGTTTACTTC CGTATCAATA GTGCAAAGAT TGATCGTAAT 900
CAAGAAATCA ATGTTTACAA TACAGCTGAA TATGCGAAGA CCAACAACGC ACCGATCAAG 960
GTAGTAGGTT ACGCTGACGA AAAAACCGGT ACTGCGGCCT ATAACATGAA GCTTTCAGAG 1020
CGTCGTGCAA AAGCGGTAGC CAAGATGCTT GAAAAGTATG GTGTTTCTGC GGATCGCATT 1080
ACAATTGAAT CGAAGGGCTC ATCAGAGCAA ATCTATGAAG AGAACGCTTG GAATCGTATT 1140

GTAGTAATGA CTGCAGCGGA A 1161

(2) INFORMATION FOR SEQ ID NO:50

(i) SEQUENCE CHARACTERISTICS:
(A) LENGTH: 585 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...585

(xi) SEQUENCE DESCRIPTION: SEQ ID NO:50

GTAATTGGCA TCATTATGGA ATTTTTCATG TTATTCATAG CGGCGGTTTT CGTTAATAAC 60
GTCGTGCTGT CGCAGTTCCT CGGTATATGC CCATTCTTAG GCGTATCGAA GAAGGTAGAC 120
ACCTCAATCG GTATGGGTGC AGCCGTGACA TTCGTATTGG CACTGGCTAC CTTGGTTACC 180
TTCCTGATTC AGAAGTTCGT TTTGGATCGT TTCGGATTGG GCTTTATGCA GACCATTGCA 240
TTTATTTTGG TCATTGCCGC CTTGGTGCAG ATGGTGGAGA TCATACTCAA GAAAGTATCT 300
CCTCCCCTCT ATCAGGCACT GGGTGTATTC TTGCCCTTGA TTACGACGAA CTGCTGTGTG 360
CTCGGTGTGG CTATTTTGGT TATCCAGAAG GATTATACCC TGCTCCAGAG CTTCGTCTAT 420
GCAATATCCA CGGCTATCGG TTTCACCTTG GCAATGGTTA CTTTCGCAGG TATTCGAGAG 480
CAACTCGATA TGACCAATCT CCCCAAAGCT ATGAAGGGAA TACCTTCGGC ACTCTTGGCT 540
GCCGGTATAT TGGCTATGGC TTTCATGGGC TTCAGCGGTA TCGCC 585

(2) INFORMATION FOR SEQ ID NO:51

(i) SEQUENCE CHARACTERISTICS:
(A) LENGTH: 2628 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2628

(xi) SEQUENCE DESCRIPTION: SEQ ID NO:51

TACCGATCTT ATCGTGGGAT AGGGAGTGGG ACACACTCTC CTAACCTCAA AAACCGACTA 60
AAAAGGATCG GAATAAGGAT ACCGAACAGA CACTATATCC ATATCAAGCC AATCAAACCA 120
AAAAATAAAA TGAAACAACT AAACATTATC AGCTTCATCA TTGCTTTCCT ATTCTTAGGA 180
ACGAGCGCAT CGGCTCAGCA ATCGGGCGGA TCCGTTACAG GTACCGTAGT GGACAAAAGC 240
TCAAAAGAAC CTATCGCATA CGTACAAGTA TTCGTCAAAG GAACCACTCT CGGAACTTCC 300
ACGGATGCAA ACGGAAACTA CTCGATCAAG GGAATCCCTT CGGGTAATCA AACTATCGTA 360
GCCCGACTCA TGGGTTACTC CACTTGCGAA GAAAAAGTAC ATATAGAAAA GGGTGGTTCC 420
CGCCACGTAG ACCTCTATCT GACCGAAGAG ATTCTCTCTC TCGATGGGGT AGTGGTATCT 480
GCCAATAGAA ACGAGACTTT CCGCCGTCAA GCACCCTCGT TGGTAACGGT ACTGTCGCCG 540
GAACTTTTCC TCAAAACCAA CTCTACCAAC CTGAGTCAGG GACTTAAGTT CCAGCCCGGT 600
CTGCGCGTGG AGGACAACTG TCAGAACTGC GGTTTCAACC AAGTTCGTAT CAATGGACTC 660
GAAGGAGCCT ATTCGCAAAT TCTTATCGAC AGCCATCCCA TCTTCAGTTC GCTTGCCGGT 720
GTCTATGGCT TGGAGCAGAT GCCTGCCAAT ATGATCGAAC GTGTAGAAGT AATTCGCGGT 780
GGAGGTTCGG CTCTGTTCGG CTCTAATGCT GTGGGAGGCG TTATCAACGT AATTACGAAA 840

GAACCGCTTC GCAATTCGGC CGAGATCAGC CATTCTACGA TGACCTTCGA CCACGCGAAA 900
GGGTGGGGGA GCTTCCAAAA TACGACCCAG TTCAACGGTT CTATGCTGAC GGAAGACCGC 960
AAAGCCGGTG TCATGGTATT CGGCCAACAC AACTACCGTC CCGGACAGGA TATAGACGGC 1020
GACAACTTTA CCGAACTACC CAATCTGCGC AACCGCTCGC TCGGTTTCCG CTCATACTAT 1080
AAGACCGGTC TCTACAGCAA AGCAACCCTC GAATATCACA GCATGCAGGA GTACCGTCGT 1140
GGTGGCGACA GACTGGACAA TCCTCCTTTC GAAGCCCAGA TAGCGGAATA TCTCCAGCAC 1200
TATATCAATG GCGGAAGTTT CAAATTCGAT CAGGGCTTCA GCGGTGGCAA GGATTTCTTC 1260
AGTCTGTATG CTTCAGCACA AGACGTTCAG CGTCGTAGCT ACTACGGGGG TGGCGACTAT 1320
ACCGAAAATC TGCTGAACGG AGCAGTTCAG AGTGGAAGCA CCGAATCGGA CGAATACAAC 1380
GATGCTTTCA CGGCTCTTAC TTCCTACGGG ACTACCAAGG GATTCGATTT GCAAGGAGGA 1440
GGTATGTACC GTCATACCTT CGGAGAAAAC TGGGACTTTA CCGGCGGACT CGAATATATC 1500
TACGGCCAAC TCGATGACAG AAGCGGCTAC AGACCGAGCA AAATAGATCA GAATACCTCT 1560
ACTTTTAGTC AGTACGACCA GCTCGAATAT AAGACGGAGA AGTTAAGTGC CCTTATCGGA 1620
GCACGTATCG ACTATGTTCT CCTCAATCAG GATGGCAAAC GCTATATCGA TCCGCTCTTC 1680
ATTTTCAGTC CTAGAGCCAA CGTACGATAC AATCCCAATA AGAATCTCAG CTTCCGACTC 1740
TCATACAGCG AAGGATTCCG CGCTCCTCAG TATTTCGATG AAGATCTGCA CGTAGAGTTG 1800
GCCGGTGGTA CTCCTATCAG CCGTGTCCTT TCCCCCAATC TGAAAGAAGA ACGTTCACGA 1860
AGCATCAGTG CTTCTTTCGA TTATTACCAC AGAGCCGACG AATGGCAATT CAATATCATG 1920
GGAGAAGCCT TCTCCACCTT TATCAGCAAT CAGTTCAAAC CATCCGATAA GGTCGAAACC 1980
ACGAGCGATG GCAAAGAATG GATCATTCGT ACCATCTACA ACGACAAGGA TGGAGTATCG 2040
AAGGTATATG GTGTGAATCT GGAGGGAAGA ATCGCCTACA ACAAATCGTT CGACCTCCAG 2100
CTCGGCGGTA CATGGCAGAG AAGCCGCTAC GGAAGCATCT ATACCGCTGT GGAAGCGGAC 2160
AAAACAACGG GACAAGCCGA GATCTCTGTG AAAGACTATG TACGCACTCC GAATCTGTAC 2220
GGCTATTTCG TTGCTACGGT ACGTCCTACC GAGCACTTCG CCATCAATCT CTCCGGTACA 2280
TTCACGGGCA AAATGGATGT AGTACACGAA GCCTATGAAG GCGATATTCC CGCAGAACAC 2340
ATAGCTCCGG ACGGATCGTT CGACTTTGAA ATGAATGGTC AGCAATTCAA AGGTTTGGCC 2400
GAAGGTCATG CCAAGCTCGT CAAGACTCCG GCCTTCGCCG ATATAGACCT CAAGCTGAGC 2460
CACGACTTCC ACCTTGCTTC CACTATGACC TTGGAATTGA ATGCCGGAAT ACAGAACATA 2520
TTCAACAGCT ATCAGAAAGA CACGGACAAG GGACCGGGTA GAGCTTCTAC TTACGTATAC 2580
GGTCCTATGC AGCCCAGAAG GATTTTCGTC GGTACAAAGA TCAATTTC 2628

(2) INFORMATION FOR SEQ ID NO:52

(i) SEQUENCE CHARACTERISTICS:
(A) LENGTH: 2697 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2697

(xi) SEQUENCE DESCRIPTION: SEQ ID NO:52

ATATTGAATC ATTTGAGAAA GACTATGTAC AAAAAGATTA TTGCCGTAGC AGCTCTCTTC 60
TGCGCCAGCA TAGGGATCCT GAAAGGACAG TCCTCGGATC TGACCCCTCA GGATACTATA 120
TATAGCCCTG AAATATCCTA TGCCAAGCCT ATTCATAAGA CCATAGCATC TATTGAGATC 180
GAGGGAATGA GGTCTTTCGA TGACTTTGTC TTGCGCAATC TTTCAGGCTT GGCTGTAGGT 240
GATGAAGTCC TGATTCCTGG AGATGCCATG TCTGCTGCCG TGAATAGAAT TATGCGTCAG 300
GGCTACTTCT CAAATGTGCG AATCATCGCG GATAAATATG TCGGCAATAA AGTCTATCTG 360
AAAATCATTG TCACTGAACG TCCTCGCATC AGTAAGGTTA CTTTTAGCGG GGTAAAGAAG 420
TCTGAGAGAG AAGATCTTGA AATGAAAATC GGTCTTCGCG AGGGGATTCA GATGACCAGA 480
AATAATGAAG ACAAGGTCAG GCAAATCGTA CAGAAGTATT TTAGTGAGAA AGGTTATCGC 540
GATGCCAGCA TACGGATAAC GCAGGAACCG GATCTTTCCA AAGATGGCTT TGTCAATGTG 600
CTTATCTCGA TTGAGAAGAA AAGCAAAACC AAGGTGAATG AAATTTATTT TTCCGGCAAC 660
AAGGCCCTTA GCAATCATAA GCTAAGAATG GCGATGAAGA ACACCAATGC CAAATTCAGT 720
CTTAGAAAGC ATATTCGCTC ATCTTTCTTG AAACTTTTTA GTACTCATAA GTTTGTGGAA 780
GAGAGCTACC GTGAAGATTT GGTCCGATTG ATAGAGAAGT ATCAGGAATA TGGATATCGT 840
GATGCTGAAA TACTGACCGA CAGTGTCGTG AAGGCTCCTG ACGGCAAAAG AGTGGATATT 900
TATCTCAACA TCGAAGAGGG GCAGAAGTAT TATATTAAGG ATGTCAACTT TGTGGGCAAT 960
TCACAATATC CATCGGAGTA TTTGGAACGA GTGCTCGGAA TAAAATCCGG AGATGTGTAC 1020
AATCAGAGAC GATTGGCTAA GCGTCTCAAT GAAGATGAAG ATGCTGTGGG GAACCTGTAC 1080
TATAACAATG GCTATATTTT TGCGTGGGTC GATCCCGTGG AAACAAATGT AGTGGGGGAT 1140
TCTGTTTCGC TTCATATTCG TATAGCGGAG GGGAAGCAGG CCAATATCAA TAAGCTGATC 1200

ATCAAAGGAA ATACTGTCGT GTACGAAGAC GTAGTACGCC GAGAGCTTTA CACAAAGCCC 1260
GGCCAGCTCT TTAGTCGCGA GGATATCATT AACTCTATTC GTCTCATCAA TCAGCTTGGG 1320
CATTTCGATG CCGAAAAATC TATTCCCCGT CCGATTCCCA ATCCCGAAAC AGGAACAGTG 1380
GATATAGAGT ATGATTTGGT GCCGCGTAGC AGTGACCAAT TGGAGCTTTC TGTCGGTTGG 1440
AGTCAGTCCG GACTTCTGTT CCGAGGAGCC ATTAAGTTCA CGAACTTCTC TGTCGGCAAC 1500
TTGCTCCATC CCTCGATGTA TAAGAAAGGG ATCATTCCGC AAGGGGATGG GCAAACACTA 1560
TCACTGAGTG CTCAGACCAA TGGAAAGTAC TATCAGCAGT ATAGTGTCAC ATTTATGGAT 1620
CCATGGTTTG GGGGCAAGCG GCCGGATATG TTCAGCTTCA GTGCATTCTA TTCCAAGACT 1680
ACGGCGATTG ACTCCAAGTT CTACAATAGC AATGCCGGCA ACTACTATAA TGCCTACTAT 1740
AATAGCTACT ACAACAACTA TAATAGTTAT TACAACGGTA TGTCGAACTA TACCGGCGAC 1800
CTCTATACTC AGGCCAGCGA TCCGGATCGT TCGCTTCAGA TGTTAGGTAC TTCGATCGGT 1860
TACGGTAAGC GTTTGACTTG GCCGGACAAT TGGTTCCAGA TTTATACTTC TCTGAACTAC 1920
ACCTACTATA GACTGCGAAA TTGGAGCTAC AATACCTTCC AAAATTTCCA TCATGGCTCG 1980
GCTAATGATC TCAACTTGGA GCTGCGTCTC TCTCGTACTT CCATCGATAA TCCTATTTAT 2040
ACCAGAAGCG GATCGGATTT CATGGTTTCT GTTGCTGCTA CTCTTCCTTA TTCTTTGTGG 2100
GACAATCATG ACTATGCCAG CCAGAACCTC AGCGTAAGCG ATCGTTACAG ATTTATCGAG 2160
TATCACAAGT GGAAGTTTAG AGGACGAGTT TTTACTCCAT TGCTCAATCC TGCTACGCAT 2220
AAATATACAC CGGTGCTCAT GAGTCGAGTG GAAGGAGCAG TTCTTGGTTC GTATAATTCC 2280
AATAAGAAAT CTCCTTTCGG TACTTTCTAT ATGGGAGGTG ATGGTATGTC CAGCTATTAT 2340
GGTGGCTACA TGAATGAGAC TATAGGTTTG CGTGGTTATA AGAACGGATC TATTGCCGGT 2400
AATAACTACG ACTATGCATA TGCTTATATG CGGCTTACGA TGGAACTACG TTTCCCGATT 2460
CTGTTTGAAA ACTCATTCAA TGCGTGGCTC TTAGCTTTTG CCGAAGCAGG CAATGCGTGG 2520
CGCAGTATCG ACAATTATAA TCCCTTTAAC CTGAAGCGAT CGGCCGGTGT AGGATTGCGT 2580
GTAACGTTAC CGATGGTCGG AATGCTCGGT ATCCATTGGG GATATGGCTT TGACCGTCCG 2640
GACAATTCTC TACAGCGAGG AGGAAGCAAT GTCCACTTTG TGCTCGGACA GGAGTTC 2697

(2) INFORMATION FOR SEQ ID NO:53

(i) SEQUENCE CHARACTERISTICS:
(A) LENGTH: 531 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...531

(xi) SEQUENCE DESCRIPTION: SEQ ID NO:53

CAAATAAACA TGAATGGCGA TATGAAACGG TTTTTGATTT TGATCGGCTT TGCACTGGCG 60
GTAGCTTTCT CCGGTTTTTC CCAAAAGTTC GCTTTGGTAG ATATGGAATA TATCCTCAGG 120
AATATTCCTG ACTATGAGAT GATGAACGAA CAGCTGGAAC AGGTGTCCAA GAAATGGCAA 180
AATGAAATCG AAGCTCTCGA AAATGAAGCC CAATCTATGT ATAAGAAGTA TCAGAGCGAT 240
CTCGTATTCT TGTCTGCTGC ACAGAAGAAA ACCCAAGAAG AGGCTATCGT AAAGAAAGAG 300
CAGCAAGCAT CCGAGCTCAA GCGGAAGTAT TTCGGCCCGG AGGGGGAGCT GTATAAGAAA 360
CGCTCCGATC TGATGAAGCC TATTCAGGAT GAGATTTGGA ATGCTATCAA AGAGATTGCC 420
AAGCGTAACA ACTATCAGAT GGTGCTTGAT AGAGGTACGT CCGGAATTAT CTTTGCCAGT 480
CCGTCTATTG ACATTAGCGA CCTTGTACTG AGCAAGATGG GCTTTAGCAA G 531

(2) INFORMATION FOR SEQ ID NO:54

(i) SEQUENCE CHARACTERISTICS:
(A) LENGTH: 510 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...510

(xi) SEQUENCE DESCRIPTION: SEQ ID NO:54

CGAATAAATA AACAACACGA AATGAAGAAA TTTTTTCTCA TGCTTCTGAT GGCTCTTCCT 60
TTGAGCCTCT TGGCACAAAA GGTGGCAGTG GTAAACACTG AGGAGATCAT TTCCAAAATG 120
CCGGAACAAG TAGCTGCTAC CAAACAGCTC AACGAATTGG CCGAAAAGTA TCGCCTTGAT 180
CTCAAGAGTA TGGACGATGA GTTTGCCAAA AAGACAGAAG AATTTGTAAA GGAAAAAGAC 240
TCTCTACTGG AGAACATCCG CAATCGTCGT CAGCAGGAAC TTCAGGATAT TCAAACTCGT 300
TATCAGCAGT CATACCAAAC GATGCAGGAG GATTTGCAAA AGCGCCAACA ACAGCTTTTT 360
GCTCCTATCC AACAAAAGGT GGCTGATGCC ATCAAGAAAG TGGGTGACGA AGAAAACTGT 420
GCCTACATCA TGGAGGCCGG TATGATGCTT TACACCGGAG CTACTGCTAT TGACTTGACC 480
GCAAAGGTAA AAGCGAAACT CGGAATCAAG 510

(2) INFORMATION FOR SEQ ID NO:55

(i) SEQUENCE CHARACTERISTICS:
(A) LENGTH: 2484 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2484

(xi) SEQUENCE DESCRIPTION: SEQ ID NO:55

ATCATGAAGG AAGCTATTCC CCGAAAGAAC AAGTATATAA AGCTCAACGG TATATACAGA 60
TTGTCATTCA TTCTGCTATG CTGCCTGCTA TGCTCTCAGG CAGCTATGGC ACAAGGCGTC 120
AGGGTATCGG GCTATGTGCT CGACCGTGGG GAAAAGCCGA TCCCGTTCGC CGGAGTCAAA 180
GTGCGTGGTA CGGGGACAGG CGCAACGACG AATCTGAAAG GATACTACGA GTTTCGGATG 240
AAGGCCACGA CGGACAGCAT CACGATCGAG TTCAGCTCCA TGGGGTACCA AGGGGTAAGT 300
CGCAGCTTTC CGTCTCTGAC CAAGGACACT CGGCTGAATG TTCGTTTGGC AGAGGCCGAG 360
ATGGAGCTTT CGAGCGTGAC GGTACAGGCC ACAAAACGCA GACTCAACAC GATGGAGCGC 420
GTCAATACCC GAGACCTTCG TGTCAATGCA GGGCCTACGG GAGGGGTGGA ATCGCTCATC 480
AGTACCTACG CAGGAGTAAC GCAGAACAAT GAACTAAGCT CGCAATACTC GGTTCGCGGA 540
GGAAGCTACG ATGAGAATAT GGTCTATGTA AACGGAGTGG AGGTTTATCG CCCGCTGCTG 600
GTTCGCTCTG CACAGCAGGA AGGTCTGAGC TTCGTCAATC CGGATCTGAC ACAATCCGTA 660
CAGTTCTCCG CCGGAGGGTT CACGGCCGAC TATGGCGACA AGATGTCCTC CGTACTGGAT 720
ATTCGCTACA AGCAACCGCA GGAGAAGGAA GGAGCGGTAC TCCTCGGGAT GCTACAATCG 780
AGTGCCTACT ATGGCAGCAG TGCCGGAGCC TTCAGCCAAA TCACGGGTGT ACGCTACAAG 840
AGTGCCAAAT CGCTCTTGGG CACTACGGAC ACGAAAGCCG AATACGATCC GATCTATGCG 900
GACGGACAGA CATTCATGAC GTACCGTTTC AGCCCCAAGC TGTCGGTTAG TTTCCTCGGC 960
AATATTTCGC AAACTCGCTA CAAGTTTGTC CCTCAGACCC GTGAGACGAG CTTCGGTACA 1020
CTGAGCGATG CCAAAAAGTT GAAGATCTTT TTCGACGGTC AGGAACAAGA TCGTTTCCTG 1080
ACCTACTTCG GTGCCTTCAG CATGAACTTC GTGCCGGACG ACAAACAGCG GCATACGGTT 1140
ACGCTTTCGG CCTTCAACAG TAACGAACGG GAGACCTACG ATATTCAGGG AGAATACTTT 1200
CTGAACGATG TGCAGCTGGG GGCGGACGGA ACTGCTTCGA TGGCTTCGGG CTCAGAGAAC 1260
TCCAACGGCT TGGGCATCGG GCGCAATCAC GAGCATGCGC GCAACAGGCT GAGCTACCGC 1320
GTGCTGAACA TGGGTTACAG AGGGGAGATG AAGCTGAACG AGAAGCATCG CCTGCAAGCC 1380
GGCGTATCGG CACAGATGGA GAAAATAGCC GACCATATCA GCGAATGGGA ACGGAGGGAT 1440
TCGGTAGGAT ACAACCTACC TCACTCGGAG ACCGTATTGC TGATGTACAA TAACCTATAT 1500
GCCGATACGC AGATGAGGGG AACGCGCTTG TCGGCATTCG TACAGGATCG ATTCAACTTC 1560
AGCATGGGAG GAGGTACATT TTCTCTCATT CCGGGTATCA GAGCTTCGTG GTGGAGCTTC 1620
AACAAGGAGT TGCTCGTCAG CCCACGTATC AGCGTGGGTT ATTCTCCCGA AAGCAACCCG 1680
GCTTTGGTAC TGCGTGCAGC CGCCGGACTT TATTATCAGG CACCGTTTTA CAAAGAGCTA 1740
AGGCAGACGC ATAAGGATGC CGAAGGCAAT AACGTGGTTG TCCTCAACGA GAAGATCCGC 1800
TCTCAGGGAG CTTTTCACAT TCTCGCAGGA GCAGACTATA CCTTCGAAAT GGGGGGGCGA 1860
AAATACAAGT TTACGGCAGA GGCTTACTAC AAGAGCCTGT TCAACATCAA CCCGTATATA 1920

ATAGAGAACG TGAAGATCCG CTATCTGGGC GAAAACATCG GTTCGGGTTA TGCTGCGGGT 1980
ATCGATCTCA AGCTCTTCGG CGAACTGGTA CCCGGAGTGG ATTCGTGGCT GACGGCTTCC 2040
ATTATAAAAG CCCGTCAGAA ACTGGATGGC TACGGTTCTT TACCACTGAT GAACGCACCC 2100
ACTTACAATT TCTCCTTCTT CCTTCAGGAG TACGTGCCGG GCAATAAACG CATCACAGCC 2160
ACCCTGCGGG CTGCACTAAG CGGAGGATTG CCCCAGCTCA ATCCGAGCAA AGGGCTTAGC 2220
TCGCCGGCCT TTACCGCACC GGCCTATAAG CGTGTCGATC TGGGGGTAAT GTACAAATGG 2280
CTCGACCCGG ATGACTCCTT TGCCGGCCGA AGCAAATGGC TAATGGGAGT AAAAGGGGCC 2340
TACATAGGGG CTGACCTCTT CAATCTGTTC GACATGACCA ACGTCAATTC TTACTACTGG 2400
GTGTCGGATG CCTACCAACA GCAATACGCC GTACCGAACT ACCTGACACG CCGCCAATTC 2460
AACCTGCGTC TCCTCGTCGA ATTC 2484

(2) INFORMATION FOR SEQ ID NO:56

(i) SEQUENCE CHARACTERISTICS:
(A) LENGTH: 2037 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2037

(xi) SEQUENCE DESCRIPTION: SEQ ID NO:56

CCTATCCCTT TTCTTCTTAA TATGTACAGC GGACATCATA AAATCCATTA TCCTTTTCTT 60
ATCCTGTTGG TATGCCTTGC TTTTGCTGCC TGCAAGAGCG TGAAGTTGAA AGATGCGGAG 120
AAGGCACATG ATCGCCAAGA GTATACCAAG GCTGCCGATA TGTACAATAC ATTATACAGG 180
CGTACCCGAC GAAAGCAGGT GGAGATGAAA GCTTATACGG CTTTCCGATC CGGTGAAAAC 240
TATCGTGCCG CCGGCAGACA AGCCAAAGCT TTGCGTGGCT ATCTGAATGC CAGACGCTAC 300
GGGTATCCGG ATTCTGTGGT ACTGCTCCGT TTGGCACAGA CTTATCAGCA AGGAGGTAAC 360
TATAAGGAAG CCGAGGTACT CTTCCGTGGA TATCTGGAAG CTTATCCGAA AAGTTATTTT 420
GCAGCTATCG GTTTGGAGGG GTGTCTCTTT GCCCGCCAGC AAAAGGAATA TCCTACACGT 480
TACCGGATAC GGCGAGCTGC CGAGTGGAAT TCGGCACGGG GCGACTTCGG CCCGGCCTAT 540
GCACCCGATG CTTCGGCTCT CTATTTCACA TCGAGCAGAA GCAAAGACGA CGGTTTGGAT 600
AATAGCAGCA TAACGGGACT GAAACCCAAC GACATTTATA TCATCAAACG AGATGCACAA 660
GGACGATGGG GACGTCCCGA TAGCGTGTCC GGAGGAATCA ACACTCCATG GGATGAAGGC 720
GTGCCAACGA TCACGCCCGA TGGTAGTACC ATATATTATA CGTTGGCGCA GCAAGGAGCC 780
GATTACGACC GTACGGTACA GATCTATTCC GCCGCTCGGA GCGGAGAAGG CGGTTGGAGC 840
AACGGTTCGC TCGTGGACAT TATGCGCGAT TCGCTCCGTA TGGCTGCTCA TCCCTCTATG 900
TCGGCATCCG GCGATTACCT GTATTTCGTC AGCAATATAG GCGGTAGCTA TGGCGGCAAG 960
GATATTTATC GTGTCAAGGT GTCGGATCGT TCTTATGGTT CACCGGAGAA TTTGGGGCCT 1020
GATATCAATA CGCCGGGGGA CGAAATGTTT CCCTTCATAG ATGGGGATAG TACCCTTTTC 1080
TTCGCTTCGG ACGGACACGC CGGTCTGGGA GGACTGGATA TTTTCAAAGC CACGCTGGAC 1140
TCTACCGGCC AATGGCATGT AGTCAATATG GGACAACCGG TCAATTCCTC TGCCGATGAT 1200
TTCGGCTTGG CTGTGGAGCC TAAAGGCAAA AACAAAGAAG AAGCTTTGCC GGACAACGGA 1260
GTCAAAGGTG TATTTTGTTC CAACCGAGGC GATGCACGCG GATGGCCGCA CCTCTTCCAT 1320
TTCGAACTGC CGGCTATCTA CACCGAGATT CAAGGTTATG TGATGGACAG AGAAGAAAAT 1380
CCCATAGCCG GAGCCACTGT CAGGATCGTA GGCGAACGCG GCCCCGTAGG ACAGGGATTC 1440
GTGACTACTC GTGACGATGG CTCCTATAAG ATGAGCGTGC AGGGCGATAC TCGCTATGTA 1500
ATGCTTGCCG GAGCATCGGG TTATTTGAAT CAGTACGTAG AACTCAAGAC CGATACCGCC 1560
AAGCAGAGTG AGACCTACTA TGTGGACTTT TTCCTTGCAT CGCGTGAGAA AGCCGAGGGC 1620
TTGCAAAATA TTTTCTATGA TTTCGATAAA GCTACTCTTC GCCCCGAAAG CATGAAGAGC 1680
TTGGACGAAC TGATTCGTAT CCTCACGGAC AATCCGGATA TTCGGATCGA ATTGGGTTCG 1740
CATGCCGACA GGAAAGGCCC CGATGCTTAC AACCTCGGAC TATCTGACCG CAGAGCCAAA 1800
TCCGTGGTGG ATTACCTCAC GAGTCGTGGC ATAGCGGCCG ACAGGCTTAC GTGGAAAGGC 1860
TACGGTAAGT CTGTCCCCAA GACGGTGACA GCCAAAATTG CCGAACGGCA CGATTTCCTG 1920
AAGGAAGGCG ATGTGCTCAC CGAGGAATTC GTAGCACCTT TGACCGAGGA GCAGCAGTCA 1980
GTCTGCGACC AACTGAACCG TCGTACCGAG TTCCGTGTGA TCGAAGAAGA GTTGCGT 2037

(2) INFORMATION FOR SEQ ID NO:57

(i) SEQUENCE CHARACTERISTICS:
(A) LENGTH: 2316 base pairs

(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2316

(xi) SEQUENCE DESCRIPTION: SEQ ID NO:57

CCTGCACAGC CGAAAACATA TTGCATACGA TACTTTCGGA GAGAGGTATC TCCGAGGAGG 60
CAAACAGAAA GGACATTGTA TGCGGACAAG ATGCGCCGGC ATATCGTCAA GTGCTGTCCC 120
TCTGCCTCCA TACTCTTGCA AGAATACTCC ACAACAGAAT TATTCATTCA TCACTTTAAC 180
ATATCAATAA TTATGAAAAA GTTTTTCTTC GCGCTACTAT CGATTGGTAT TTCAGCGCAG 240
GCTTTTGCCA AGACGGACAA CGTCCCGACA GATTCGCTAC GAGTACACAA TCTTCAGACC 300
GTCACGGTCT ATTCTACACG CACGGCCGTA CCTCTGAAAA AGATACCGGC CAAGATGGAA 360
CTCATCTCAT CGCGCAACAT CAAGCAGTCC GGCTTTAACA ACATGACCGA CATCCTCAAG 420
ACGCAAAGTT CGCTCGATGT CATACAATAC CCGGGCTTTA GTTCGAACAT CGGTATCCGC 480
GGTTTCAAGC CCTCCGGCAA GTATGTAACC GTATTGGTAA ACGGCATCCC TGCGGGAACG 540
GACAATATCT CTACGCTCAA CACGAGCAAC ATCGAACAAA TCGAGATCCT CAAAGGCCCG 600
TTCTCTTCCA TCTACGGCAC CAATGCCATG GGCGGTGTGG TGAACATCAT CACCCACAAA 660
TCCAAGGACA AGATCCATGG CAACGTTTCT CTCTTCGGCG GTAGCTACCA GACCATGGCC 720
GGATCATTCA ACTTGGGTGG CCGCTTCGAG GATATTTTCT CATTCGATCT TAGTCTGGGC 780
TTGGACAAGC AGAACAAGGA CTATAAGACC GGATCAAACA ATTTCCTATC CCTGAGCAAA 840
CTGGAAGAAG CTATAGTAGA TGTAAATGCT ACCAAAAACA AGAAAATGAA GGGGAGCGAC 900
TATACTGTAG CAACGGGACG TCTGCGTTTC GGTATCGACT TCACGCCCGA ATGGTCGCTG 960
AATCTGTATC AAAACGTATT CCTCGGAGAT GCGATCCCCG TAGGAGGATC TATATGGGGC 1020
GTTTACGGAG AATCCAAAAA AAATCTGAAT CGTTCTTCGA CCTCTTTCGA GCTGCTCGGC 1080
AAACATGGCT GCCACACGCT TCAATTCTCC CCCTACTTCA ACATAGAGAA ATCGGAGAAC 1140
TATAACAATG CCGATCCCAC CGGTTTCATC AACTACAAAA GCGACTACTA CACCTATGGT 1200
GCCCTACTCC AGGACAAGAT TTCCTTTGGA GGACAAAATA TCGTACTCGG TGTCGACAGC 1260
CGAAACATGA CGATGGAGTC AGAAAGATTC GAGCAGGCAG GAGTGAATAC AAAGCCATAC 1320
AACCCCGGAT ATGCCACGAA CAATATCGGT TTGTTCGGAC AGGCCAATTT CTACCTGCTG 1380
AACGATGCTC TATCGATATC TGCCGGTGCA CGTGCCGACT TCATGTTCTT TGACCTGAAA 1440
GCGAACGAGT ATCTCAACAA TGAAGCCAAA CAGGAAACTC ATAACGTAAT CAATCCGAAT 1500
GTCGGAATCA AATATGAGTT TGTGAAAGGC CTTACAGCTC ATGGTACATT CGGTAGTGCA 1560
TTCAGTGCTC CCGATGCTTT CCAAAAAGCA GGCCAATACG TAGGCCCGTT CGGCACGACC 1620
ATAGGCAATC CTGACCTGAA ACCCGAAAAG TCCATGACCT GGGACTTCGG TATCGGATAC 1680
AGCAATGCAC GCTGCGGGAT CCAAGCCGAC GTAACCTTAA CCTATTTCCA CACCGACCAC 1740
AAAGATCTGA TCTTGTCCAG CCCTGACTAT GCTAATAATA TCACCACATA CATCAATGCC 1800
GACAAGGCTC GTATGAGCGG TATCGAGGCC CTTTTGTCTT ATGACTTCGG CAGCCTCTTT 1860
GCCAACAAGT TCTCTCTCCG CGCATTTGCG AATGCCACGA TCATGCTCAA TTCCGAGATG 1920
AAGAAAAGCC AGACCGATGC CCCTTGGAGC GAAATGTACT ACGTTCGCAA GCAGAACATC 1980
ACCTTCGGTA TCGAATATCG TGGCAAAGAA GGACTTGAAG TGATGCTCAA CGGTCGCTTC 2040
ATGGGACGCA GGATCGAGCA AAACTGGTAT GCTTACTACC CCGAAGTTCG CCCCGAACTC 2100
CAGCAACTGC TTGCAGCAGA AGAGCCTGAA TTGGCTGCTC AGGGACTGCT CCGTCATCCG 2160
CAAGCAATGG TGTTCAATGC CTCTGCTTAC TACCACATGA ACAAGTATCT CACCTTCGGT 2220
GTGAACTTGA ACAACATCTT GGATGAGCTT TATACGGAGA AAGACGGCTA CCACATGCCC 2280
GGACGTAACA TCATGGGTAA GGTTATGGTC AACTTC 2316

(2) INFORMATION FOR SEQ ID NO:58

(i) SEQUENCE CHARACTERISTICS:
(A) LENGTH: 1452 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:

(A) ORGANISM: PORYPHYROMONAS GINGIVALIS

(ix) FEATURE:
(A) NAME/KEY: misc_feature
(B) LOCATION 1...1452

(xi) SEQUENCE DESCRIPTION: SEQ ID NO:58

GGCCGGGCCT CTTCCCCTTA CAGGCAAATG GATGGCATAC TAAACGATGA ATATAGACAA 60
GCATCTATGA ACAGGTTTTC AAATCATTGG CCCTGCATCC TCGTGGGGTT TGTACTCTGG 120
TTTGTATCGG CGAGTCGGAC TGTGGCACAA AACGCCTCCG AAACGACGGT ATCGTACGAT 180
ACGGATACCG CCGTACTCTC CGAAGCCGAT GTGCTTCGGA TCGCTCTTAG TGAGAATGCC 240
ACAGTGAAAG TGGCCGATAT GGATGTGCGC AAACAGGAAT ATGCACGTAG GGCAGCACGT 300
GCCGATCTCT TCCCGAAAGT AGACCTCAAT GGCGTTTACA GCCATACGCT AAAGAAGCAG 360
GTCTTATATA TAGATATGCC CGGTTTCAGC AGTAGCGAAG GTATCGAAAT GGGGCGTACA 420
CACAATACGC AAGGAGGGGT GAACGTCTCC ATGCCATTGG TGTCGGCACA GCTTTGGAAA 480
AGCATTGCCA TGACCGGAGA ACAGCTCGAT CTGGCTCTGG AGAAAGCTCG CAGCTCCCGA 540
ATCGATTTGG TGGCAGAGGT GAAGAAGGCT TACCTCAGTG TATTGTTGGC CGAGGACTCT 600
TATGGCGTAT TCAAGCGCAG CTATGACAAT GCTCTGGCCA ATTATAAGAA CATATCCGAC 660
AAGTTCGATC GTGGACTTGT GGCCGAGTAT GATAAGATTC GAGCCAATGT ACAGGTACGC 720
AACATCGAGC CTAACCTCTT GCAAGCGCAG AACTCCGTAG CCCTTGCTCT CTGGCAGCTC 780
AAGGTCCTGA TGAGCATGGA AGTGGAAACT CCGATCAGAC TCTCCGGTTC ATTGTCCGAC 840
TATAAAGAAC AAGTCTATAC CGGCTATTTT GCCGCCGATA CGCTTATTTC CAACAACTCC 900
TCCCTGCGTC AGCTCGATAT ACAGCGTCGT CTGGCTGTCA GTGCAGACAA GCTGAACAAG 960
TACAGCTTCC TGCCTACACT CAATCTGGGA GGGCAGTACA CCTATTCGCT CAACAGCAAC 1020
GACATCAAAT TCTGGGGCGA GGGACAACGC TGGACGCCTT TCTCCACCAT ATCGCTCAGC 1080
CTGTACATTC CTATATTCAA TGGAGGCAAA CGTCTGTACA ACGTGAAGCA AAGTGCTTTA 1140
TCGATCCGTC AGATCGATCT GCAACGACGC CACATAGAGC AATCCATCCG AATGGGAATC 1200
AAGAACCAAA ATGACCGTCT GCGTACCTGT ATGCAGAGAT TTGTGGCCTC GGAAGAGGCT 1260
GTCCGAAGTG CAGAAAAGGG CTATCAGATA GCAGAGAAAC GCTATCAGAC AGGCGAAGGC 1320
ACTCTCGTCG AGCTCAACGA TGCCGATGTG GCTCTTTTGC AGGCTCGACT CAATTATAAT 1380
CAGGCCATAT TCGACTTTAT GACCGCAAAG GCCGAATTGG ACAAGATGAA CGGCATGGGG 1440
ATTCCCGAAC AA 1452

(2) INFORMATION FOR SEQ ID NO:59

(i) SEQUENCE CHARACTERISTICS:
(A) LENGTH: 1620 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1620

(xi) SEQUENCE DESCRIPTION: SEQ ID NO:59

TTTCATAACT TTGACTTCCT AAACGCTATA AAATTGTTTT CGATGGCAAA TAATACTCTT 60
TTGGCGAAGA CTCGACGTTA TGTCTGCCTT GTCGGTTTCT GTTGGCTCAT GGCGATGATG 120
CACCTCTCTG GGCAGGAAGT CACTATGTGG GGGGACAGCC ATGGAGTGGC GCCGAACCAA 180
GTGCGCCGAA CGCTGGTGAA GGTAGCCTTA AGTGAATCCC TTCCTCCGGG TGCAAAACAG 240
ATTCGTATCG GATTCTCTCT TCCGAAAGAA ACGGAGGAAA AAGTCACCGC CCTATATCTC 300
CTTGTGAGTG ATTCTTTAGC GGTGCGCGAC TTGCCGGACT ACAAAGGGCG AGTCTCTTAC 360
GATAGCTTCC CGATCTCAAA GGAAGATCGT ACCACAGCCC TTTCTGCGGA TTCGGTAGCC 420
GGACGCCGCT TCTTTTATTT GGCTGCGGAT ATAGGGCCTG TTGCTTCTTT TTCCCGATCC 480
GATACGCTGA CTGCCCGTGT GGAAGAGGTG GCTGTCGATG GCCGCCCTTT GCCGTTGAAA 540
GAGCTGTCGC CTGCCTCCCG TCGTCTGTAT AGGGGGTATG AGGCCCTCTT TGTACCCGGT 600
GATGGCGGAT CGCGGAACTA TCGTATCCCG GCCATTTTGA AAACGGCTAA TGGAACACTC 660
ATAGCGATGG CCGACAGACG AAAATATAAT CAGACGGATC TGCCGGAGGA TATAGATATA 720
GTCATGCGGC GCAGTACGGA CGGAGGGAAA TCGTGGAGCG ATCCCAGGAT TATCGTACAG 780
GGAGAGGGGC GCAATCATGG CTTTGGCGAT GTAGCCCTGG TGCAAACCCA AGCAGGAAAG 840
CTCCTGATGA TCTTTGTCGG TGGAGTAGGC CTGTGGCAGT CTACCCCCGA TCGTCCTCAG 900
CGCACTTATA TATCGGAAAG TCGGGACGAA GGACTGACTT GGTCGCCTCC TCGGGATATA 960
ACCCATTTCA TCTTCGGCAA GGATTGTGCC GATCCGGGAC GCAGTCGCTG GTTGGCCTCC 1020

TTTTGTGCTT CGGGACAAGG GCTTGTGCTG CCATCCGGTC GTATCACGTT TGTGGCTGCC 1080
ATCCGCGAAT CAGGGCAGGA GTACGTCCTG AACAACTATG TCCTCTATAG CGACGATGAG 1140
GGCGATACAT GGCAGCTTTC CGACTGTGCA TACCGCCGTG GCGATGAGGC AAAGCTTTCA 1200
TTGATGCCCG ATGGCAGGGT ACTGATGAGC ATACGCAATC AGGGACGGCA GGAGAGCCGA 1260
CAGCGTTTCT TCGCTCTCTC CTCCGACGAT GGCCTTACTT GGGAGAGAGC CAAGCAGTTC 1320
GAGGGCATCC ATGACCCCGG CTGTAATGGA GCTATGCTTC AAGTGAAAAG GAACGGAAGG 1380
GATCAAGTGC TGCACTCCCT GCCTCTCGGC CCGGATGGGC GTCGCGATGG AGCTGTCTAT 1440
CTCTTCGATC ATGTCTCCGG CCGCTGGTCC GCTCCCGTTG TTGTCAATTC AGGATCGAGT 1500
GCCTACTCGG ATATGACTCT GCTGGCGGAT GGAACGATCG GTTATTTCGT CGAAGAGGGC 1560
GATGAGATCT CATTGGTTTT CATTCGGTTC GTCCTTGACG ATCTCTTCGA TGTCCGGCAA 1620

(2) INFORMATION FOR SEQ ID NO:60

(i) SEQUENCE CHARACTERISTICS:
(A) LENGTH: 879 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...879

(xi) SEQUENCE DESCRIPTION: SEQ ID NO:60

AAGTCTCCGA GCGATTCGGC TTTGCCGTCG GTATGGAGCG CGAATACAAT ATCTGGACTC 60
GTCGGTGGGA AACGCATTAC TTTGCTTATC CTGTATTCTA TGGCGATAAG AAGTAGTAAT 120
ATAGAATCAA TACAGTGCTT TGTGATGAAA AAAGAAAAAC TTTGGATTGC GATCGTCGCC 180
GGTTTGGCTT TCGTATTGGG CCTTTATGCT CTTGGCCGCA GTGTCGCTCA GCTACGCCGC 240
TCTCAGCCTT CGGTGACTGT GACCGGTATG GCCGAGCGTA ATTTCAAATC CGATCTGATC 300
GTTTGGACTG CTTCGTACCA GCTCCAGATG ATGGATCTCG AATCGGCCTA CAAGGCTTTG 360
AAGGAAAAAC AGATATTGGT AGCAGACTAT TTGAAAAACA AGCAGCTGCC CGATTCGTCT 420
TATATCTTCT CAAGCGTAGC CATCTCTAAA GAATACAACT ACTATTACGA TCCTCGGCAG 480
GAACAAAACG TCAGGACCTT TGCCGGGTAT CTGCTCAGCC AGACAGTTAC GGTGACCTCA 540
CAGGACATCG AACATGTGGA GAAAATATCT CGCGATATAA CGGAGCTGAT CAATCAGGGG 600
GTAGAGATTA CCTCCGACCG TCCGGCCTAT TACTACACCA AGCTCAATGA TCTGAAGGTG 660
GAGATGCTGC GCAATGCCTC CGAAGACGCT TTCAATCGTG CTTCGGTCAT TGCGGAGGGG 720
AGCGGTTCCT CCGTGGGTAA GATGCTATCT TCTTCGATGG GCGTGTTCCA GATAGTGGGG 780
CTCAACTCGA ACGAAGATTA TAGCTGGGGA GGTTCGTTCA ATACGTCTTC CAAGATGAAG 840
ACGGCAAGCA TAACGGTTAA GGCTTCTTTC GCTTTGAAG 879

(2) INFORMATION FOR SEQ ID NO:61

(i) SEQUENCE CHARACTERISTICS:
(A) LENGTH: 840 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...840

(xi) SEQUENCE DESCRIPTION: SEQ ID NO:61

GGGAAACTCC AAATGAAAAA AACAATTGCA ATTATCGCCT CAGCCCTCTT GGCTTTAGGA 60
GCCGTCGGCT GTAAGAAAAA TGCTGACACT ACCGCTGTCA GTGAAAAGGA TAGCATAGCC 120
TTGTCCATGG GTATTTTGTA CGGACAGGAT TTTGCCAATC AGTTCGAAAT GTCCCGCTTG 180
CAAGGCCAGC CGATTGATTC GGTAGCTTTC TTGGACGGTT TCAAATATGG TATCGATACG 240
ACGCGCTTCT CGTACAATCT GGGAGCCATC TATGCTTCCA ATATAGCTCG TCAGCTGGCT 300
CATGATTCCA TCGATATCGA CAAGTTCTAT GCAGCCATGC GTGCGGCTCT TCTTAAAGAC 360
ACCGTATCTA TCGCCATGAA GCCTGCAGAT GCACAGGCTT TCATGCAACG AATCCAAGCC 420
AAAAAGCAGC GAGAAAACAA TATGAAGCAG TTTGGCCAGA ACATCGAAAA GGGTAATGAA 480
TACATCGATA CCTTTAAAAA AGAAGATGGT GTAACTGTTA CGACAACTGG TCTGGCATAC 540
AAGACTCTTC AGGAAGGTAC GGGAGCTACT CCCTCTTTGG CCGATACTGT ACGTGTCAAG 600
TATGTGGGTA CTCTGGTCGA TGGTAAAGAG TTCGACAAAA ACGAAGAAGG AATCGAATTT 660
GCCGTTACCG GTGTGATTAA AGGCTGGACG GAGATGCTCC AACTCATCAA GGTCGGTCAG 720
AAAGTTCGCG TGGTAATCCC ACAGGAGCTG GCTTATGGGG AGACCGGCAA CTATACCATC 780
GAACCGTTCT CTACCCTGAC GTTCGAGATG GAACTTATCG GGATCAAGCC CGGGAAAAAG 840

(2) INFORMATION FOR SEQ ID NO:62

(i) SEQUENCE CHARACTERISTICS:
(A) LENGTH: 2409 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2409

(xi) SEQUENCE DESCRIPTION: SEQ ID NO:62

TGTCGAAAGC AGAAGCTCTC AAACCCGAAG AGGAGCCGGT ATCCGTGCAG ACGGATATCA 60
TTCCGACAAA GCGATAAGAA TCCGATGAAA GTATTACGGC AAGTATTCCT CCCCATCCTT 120
TTTGTCCTAC TGACAGGTGC CTGCTCCACC ACAAAGAATC TGCCGGAAGG CGAACAGCTG 180
TATATCGGAA TGGGCAAGAC ACAGATACTC CGGCAGGACA AGAGCCACGC CGGCCAACAG 240
GCTCTGACCG AAGTGGAGAG TACACTGAAA GTTACACCCA ATGGAGCTAT TTTCGGCAGT 300
GCAAGTGCCT CCTTACCCAA GATACCATTC GGGCTATGGC TATACAACAG CTTCGTGGGG 360
GATTCCACTG TCATTTCGAA ATGGATATTC GACAAGTTTG CAGCCAAGCC GGTTTTCATC 420
AGTCAGGTCA AATCCGATAG CCGGGCTAAG GTGGCGACGA ACATCCTCCG CGAACACGGG 480
TACTTCGATG CTAAAGTAAA AAGCAGTGTG ACCACTCTGA AAAAGGACTC GCTCAAAGCC 540
AAAATCTCCT ATACGGTGGA TATGGCCTCT CCTTATCATT ACGACAGCAT CATTCCCTTA 600
CCGATCAGCA CTTTCCCCGA CAGCATTCTG GCTTACAGGC AGACTCCGTC TTTGATCAGG 660
AAAGGAGACC AGTTCAATTT GGCAAAGCTG CACGAAGAGC GTCAGACCAT CAGTGCCCTG 720
CTGAGAGACA ATGGTTACTA CTACTTCCGC CCACAGGATA TTATCTACGA AGCCGATACC 780
CTCCTCGTAA GAGGTGCCGT ATGCCTGCGA GCCAAGCTCT CGGAAGATAC TCCACCCCAA 840
GCCATGCGCC CGTGGAGGAT AGGGAAACGG ACAGCAGTCC TGCTCGGAAT GAACGGAGAA 900
AGCCCGACAG ACTCGCTCGA AGTGGAGGAT ATGAAAGTCC TTTACTATCG TAAAATGCCG 960
GTTCGCCCCA AGATTTTGGC CAAACGCTTT CGTTTCTTCT CCGGCAATCT GTATCGGCAG 1020
AAAGACGATG AGACGACACG CAAATCCTTG GCTCGTTTGG GAGCCTTCTC CGTTATCGAT 1080
CTCAATTTTT TGCAACGCGA TTCCATTTCC GGCCTTTTGG ATGTGCGACT GCTAACCACC 1140
CTCGACAAAC CTTGGGATGC ATCATTAGAG ACCTTGTTCA CGAGCAAAAG CAATGACTTC 1200
ATCGGTCCCG GACTGAATTT TGCTCTTGCT CGGCGCAATG TATTCGGCGG AGGAGAAAAT 1260
CTTTCTTGGA ATATCGGTGG ATCGTATGAG TGGGAGACCG GCAATCGTCC CGAAAATAGC 1320
AGCAATCGGC TGATCGATAT AAATTCGTAC AACATGAATA CGGCCGTGAA CCTCTCGTTT 1380
CCCTCGATTG TATTTCCCGG TCTGCTGGAT AAATACTATT ACTACCCCAC GACTACGACT 1440
TTTCAGGCTT CTGCCACCGC GCTGAACAGG GCACACTACT TTAGCATGTA CTCTTTCGGC 1500
TTTTCGACCA CCTACGAATT TCAGCCCTCC AAGGAACACC GGCATGCTAT TTTCCCGCTC 1560
AAGCTCAACT ACAACCTCCT GGGGCATCAG ACAGAAACTT TCCAGGCCAT TACGGCGAAC 1620
AATCCGCCCC TGCTGCTCAG CCTTCAGAGT CAGTTCCTTG CTCAAATGGG GTATATCTAT 1680
ACGTTCAACA AATCCGTTTC AGAGAAAAGT CCTCATCATC TTTGGATGCA ATTCGGACTA 1740
TCCGAGGCAG GCAATCTCCT GAATCTGATC TATCTGGCAG CCGGCAAGAA GTACAGCGAC 1800
ACCAAGAATT TCGTCGGCGT CCCCTTCTCT CAGTTCATCA AAGCCACGGG AGAACTGCGC 1860
TATTCCTATA CCATAGACCG CAATCAGTCA CTGGCAACCC GTTTCGGGAC AGGCGTGATA 1920
TATAGCTATG GCAATATGCG AGTGGCACCC TATAGCGAGC AGTTCTATGT AGGCGGTGCC 1980
AATAGTATCA GAGCTTTCAC CGTCCGTAGC ATCGGCCCCG GACGGTTCAA TCCGGATTCC 2040
GACAATCAGT ATTCCTATTT GGATCAGGTG GGCGAATTCA AACTCGAAGC CAACGTGGAA 2100
TATAGAGGCA AGCTTTTCGG GGATCTCCAC GCAGCCGTTT TCCTCGATGC GGGCAACGTT 2160

TGGCTCTTGA GGGAGGATTC TTCCCGTCCG GCGGTGCTCC TGTCCGAAGT GGGATCGGTG 2220
AGCAATTTCC TGAATAGCAT CGCTCTCGGC ACCGGTGTCG GCCTTCGCTA CGATCTGGCA 2280
TTTCTCGTGG TTCGTGTCGA TGTCGGCTTC GGTCTCCACC TTCCTTACAA TACGGGTAAG 2340
AAAGGTTACT ACAATATCCC ACGCTTTAAG GATGCCATCG GTTTCCATTT GGCTGTCGGC 2400
TATCCCTTC 2409

(2) INFORMATION FOR SEQ ID NO:63

(i) SEQUENCE CHARACTERISTICS:
(A) LENGTH: 2349 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2349

(xi) SEQUENCE DESCRIPTION: SEQ ID NO:63

TCTCTCTCCC CGTATATCCG TTTTCCTATG TCCTCGCATT CCGTTCGGTA TCTAATCGGC 60
ATTGCCGGCT GCTTGCTCCT CATGCTTGCT TCCTCCTGCT CGGTCACCCG TTATGTGCCG 120
GACGGTAGCA GACTATTAGA CAGGGTAACG ATCGCAAGCG AAACGGGCAG TATCGCTCTG 180
CCGGAAGATA TTCGGGACTA TACCCTCCAG CAACCCAATT ACAGACTGTT CGGGATGACT 240
CGCTGGCTAC TGCGCGTCTA TAGCAGCTCG AATCCGAACA GCAACAGCTG GTGGAACCGT 300
TCGCTCCGGA AAATGGCCGA ACCGCCTGTC CTCATCGATT CTGTCCTCAC CGATCGTACT 360
GCCAACCGTC TGGCAAAGGC GATGGCCGGC GATGGCTTTC TCGATGCTAC TGCTCGTGCC 420
GTGGTAGACA CCGGCTTGTA CAAGAAAGCT CGCATTACTT ATCTGATTCA GCCCGGAAGC 480
CGTTATTATA TACGCAATAT GGCTTTGGAT GTGAAGAATC CACTCCTTCC TCCCGTTGCG 540
CTTGGCAATT CGCTTCCTTC GGCATACAAG GTCGGGATCA GCGAGGGTTC TCCCTTGTCG 600
CCCATCGTAC TCGATGAAGA GAGAAAGGCG ATAGCTCGTC ATATGCGCAA CAACGGCTTC 660
TGGAAGTTCT CCGCCGAGGA TGTTTATTAT GAAGCAGATA CTACCGTTTC AGGAGGATCG 720
GGTACGAAAT CTGCCGATCT GAAATTAGTG GTCAATGGCA TCGGGCGTTA TCCATATCGG 780
ATCGGCAGGG TATTCTTTCA TGCCGATTAT GATCCTCTCG AATCGGACTT CAGAGTTCAG 840
GAGCTGCCAC GTATCGATTC GATTTCGCGT GGCGATTACA CTGTTTACTA TGGGAGTAGC 900
GGACGTTATA TCCGGGCATC GGCTCTCACG CGGTCGGTGT CCGTTACACC GGGAGCTTTT 960
TTCTGCGAGG ATGATGTGGA ACGCTCTTAT ATCAAGCTGA ATGCGCTCCC TATCGTTCGG 1020
AACGTGAATA TCCGATTTGT GGAGCACAAT GGTAAGGATG AGATTGCTCT GGCGGATAGC 1080
TCTCGCCTTG TGGACTGCTA TATTCTTACC GTTCCGGCCA AGAGCAAATC GTTCGAAGCC 1140
GAAGTCCTCG GCACCAATTC CGCTGGAGAC TTCGGGGCGG CTTTGTCTCT CGGTTTCACC 1200
GATCGCAATT TGTTTCGTGG GGCGGAGATG TTCAATATCA AACTCAAGGG TGCTTACGAA 1260
GCCATTCGCA AGGGTTCGCA CAGCTTCATG GAATATGGGG TGGAAAGCTC GCTCCGTTTC 1320
CCTCGTCTCC TCTTCCCATT CATTTCTGAC GAAACGCGCC GGCGGCTACG GGCATCCACG 1380
GAATGGAAGA TCGGGTATAA TTACCAGACA CGTCCGGAGT TTGATCGGGT GATTCTCTCC 1440
GCTCAACTCA ATTATTCATG GCAGACCTAC CTGCACAATC GTCTGCGTCA TACGATCCGC 1500
CTGCTGGATG TCGATTATCT CCATCTCCCG TACATCGATC CCGACTTCGC CCAATCCCTT 1560
CCGCCTACGA CTGCACTGTA TAACTACACG GAGCAGTTTA TCCTCGGCTC GGCATATATA 1620
CTGAACTATA CCACGGCTTC GTCCATGGAG CGTACCGTAT CCAATCCTTT TACGGCACGG 1680
TTCAGTATCC AGACAGCCGG CAACCTGCTG CAAGCCATTT CTTATCTGAC CGATTCTCCG 1740
AAAGACGAAC ACGGGTTGTA TAAAATGTTC GGTCTGCACT ATGCTCAGTT CGTCAAGCTC 1800
GATCTCGATC TGGCTAAAAC CGTTCTTCTC GAAAAGGACA ATACTTTGGC ACTGCATCTG 1860
GGTTTCGGAC TGGCTTTCCC TTATGGCAAT GCTCGCCATA TACCCTTTGA GTTACGTTAC 1920
TTTGCCGGAG GATCGAACAG CGTTCGCGGC TGGAGTGTCC GTACCCTCGG CCCGGGGAGT 1980
ATGAAGATGA CTCCGGACAA GACCTTCTTC GATCAGATGG GTGATATTCG TCTGGATCTG 2040
AATGTCGAAT ACAGGACAAA GCTGTTCTGG AAGTTTCGCG CAGCAGCTTT TGTCGATGCC 2100
GGCAATGTCT GGACGATAAA GGAGTATGAG AATCAGGAGG ACGGTCTCTT TCGTTTCGAT 2160
CGCTTCTACA AGGAAATAGC TTTGGCCTAC GGTCTGGGGC TTCGTCTCGA CTTCGATTAT 2220
TTCCTTGTGC GGCTGGATGC CGGACTGAAA GCCTACGATC CTCAGCAGAC AGGGCGTTAC 2280
AAATGGGCTA TCACACGCCC AAACCTTTCT TCCAATTTCG CTTGGCACAT TGCAGTAGGC 2340
TATCCGTTC 2349

(2) INFORMATION FOR SEQ ID NO:64

(i) SEQUENCE CHARACTERISTICS:

(A) LENGTH: 2625 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2625

(xi) SEQUENCE DESCRIPTION: SEQ ID NO:64

GTCGAATCTA AATTGTTATG TCTTATGAGA AAAAGAATTC TACAACTTTT CCTGACCGCA 60
TTGCTGCTGG CATTAGGCTC CTCTCTCGCC ATAGCGCAAA CAGTGGTGAC CGGTAAGGTG 120
ATCGATTCAG AAACGTCCGA ACCGCTCATC GGTGTATCCG TAAGCACCGG TCAGGGAGCA 180
TCCCTCCGCG GTGTAACCAC CGATATGGAT GGTGGCTTCC GATTCGAAGT ACCGGCCAAA 240
TCTGTCTTGA CTTTCCGTTG CGTAGGTTAT GCTACCGTAA CTCGCTCTAT AGGCAGAGGT 300
TCTCAAGAAG ACCTCGGTAC GATTCTCCTC GATCCCCAGG CCATCGGCTT GGATGAGATT 360
CAGGTAATAG CCTCTGTGGT GCCCAAAGAC CGTATGACGC CGGTACCCGT TTCCAATATC 420
CGTGTGGCTG ATATTCAGGC AGCATCGTTG AATGTCGAAT TTCCCGAACT GGTTAAATCC 480
ACTCCCTCTA CCTATACGAC AAAAGGAAGC GGAGGTTTCG GTGATGGTCG TACCAATGTG 540
CGTGGATTCG ACACTTACAA CTTCGGTGTA CTCATCAACG GAGTTCCTGT CAATGGTATG 600
GAAGACGGGA AAGTATATTG GAGCAATTGG AGTGGTCTGA TGAATCAAGC CAGTACCATT 660
CAGATTCAGC GCGGACTCGG AGCCTCCAAG CTCGGTATCA GCTCGGTAGG TGGTACGATG 720
AACATTATCA CGAAGACTAC GGACGCCAAC ACCGGAGGTT CGGCTTATGT CGGTATGGGT 780
AATGATGGAT TGCACAAAGA ATCGTTCTCC ATTTCTACGG GTATGAACGA CGGTTGGGCT 840
ATCACCATTG CAGGCTCCCA TATGACGGGT CTGGGTTATG TGAAGGGGCT GAAGGGACGT 900
GCATTCTCTT ACTTCTTCAA CGTTTCGAAG AAGTTCAATG AACGTCATAC CCTCTCTCTT 960
ACCGGATTCG GTGCACCACA ATGGCACAAC CAACGTTCTT CCAAATATTC TGTAGCCGAC 1020
TATGACAAAT ACGGCATCCG TCACAATCAA TCCTTCGGCT ATCTGCGAGG CGAACTGACT 1080
CCTACGGCTT ATGCTTACAA TACGTACCAC AAGCCCCAGT TCTCGCTGAA CCACTTCTGG 1140
AAGATGGATG AAAATACCTC TCTTTATACc gCAHCCTACG CATCTTTGGC TACCGGTGGA 1200
GGTCGTCGCG CTTATGGAAA GAACAGTAAG TGGGTATTGA TCAACTACAA CACCGGACAA 1260
CCCTATGAAC AAACAAAGGT GACTCCCGAT GGACTTATCG ACTACGATGC CGTACTGGCT 1320
GCCAATGCTG CGGCGAGCAA TGGCTCGGAA GCAATTTTTG CCCTTGGCTC CAACTCTCAC 1380
AAGTGGTTCG GTCTACTCTC TTCATTCAAG AAGAAACTTA ATAGTTCGCT GACTTTGACA 1440
GCCGGATACG ATGGGCGTTA CTACCGTGGC GACCACTATG ACAAGATCAC CGATCTGCTC 1500
GGCGGTAGCT ACTACATAGA GGATCCCAAG ACAAAGCTCG CATACCATGC GGAAGGTCAG 1560
CAACTGAAAG TGGGTGACAT TGTAAATCGG GACTACACAG GCGAAATCAT GTGGCACGGC 1620
CTCTTCGCAC AGATGGAGCA TTCGTCCGAA TGGATCGATG CATTCGTATC AGGATCTATC 1680
AACTACGAAC TATACCGCAA TCACAACTAT GGCGGTAGCA AGTCCACCGG CTACCTGCCC 1740
GGCGTATCGC CGTGGAAAAG CTTCCTTCCG TGGAGTGGCA AGGCAGGTCT GAGCTACAAG 1800
TTCGCACAGG GACACAATGT ATTCGCCAAT GGCGGTTTCT TCACACGTGC ACCACTCTTT 1860
GGCAATATCT ATGCTGCGGG GGCTATCATT CCCAATGACA AAGCCAATAT GGAAAAGGTG 1920
CTTACAGGAG AGGTCGGCTA TGGATTCACG AATCACAAAA ACTTCGAGTT CAATATCAAC 1980
GGATACTATA CGAAGTGGAT GGATCGCGTG ACCTCGAAGA GAATCGGAAA CGAGTATGTT 2040
TATCTCAATG GCGTTGATGC TGTTCACTGT GGGGTAGAGG CTGAGGTCAG CTATCGTCCT 2100
ATTCGTCAGA TCGACCTTCG CGGTATGTTC TCTCTCGGTG ACTGGACTTG GCAAAACAAT 2160
GTAAGTTACA CTTCTTACGA CGAAGCCGGC AATGAGACAG GGCAGGATAT AACCTATATC 2220
AAGGGTCTTC ACGTCGGAGA TGCAGCACAG ATGACGGCTG CTGTATCGGC AGACATAGAG 2280
CTGTTCAAGG GTTTCCATGT CATAGGTAAG TACAACTTCC TTGGCAAGAA CTATGCAGGA 2340
TTCAACCCCG CAACGCGTAA TGCACAGCAG TACGAAGCGG ATGGCAAAGA AATCGTGGAA 2400
TCATGGAAGT TGCCCGATGT AGGTCTGTTC GATCTGTCTG CATCCTACAA TTTCAAGCTT 2460
GGTTCACTCA GCACCACATT CTATTTCAAC ATGGACAACG TAGCCGACAA GCGATATGTG 2520
AGCGATGCCG ACGACAATAT CATCGGTAAG AAACACGATG AGGCTTCGGC TCTCGTATGG 2580
TACGGTTTCG GCCGCACTTG GTCTACCGGT ATTCGTGTAA ACTTC 2625

(2) INFORMATION FOR SEQ ID NO:65

(i) SEQUENCE CHARACTERISTICS:
(A) LENGTH: 1380 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1380

(xi) SEQUENCE DESCRIPTION: SEQ ID NO:65

AACAGATGTA ATCCCTCGCG TCAATACTTC CACCTATCGC AGAATGACGT TGGAAGATCT 60
CTCCGAGCTT TTCTAATCCA TCTATCTATG AAGTTTTCAA TCCGCCTTTT CCTCTGCATC 120
ATCTTTCTCC TCTCTGCATT TATCCTGCCT GCTCTCGGAC AAAAATCCAA GCAGGTACAG 180
CGACTTGAGA AGCAACGTAA GGAGGCCCTC AAAGCCATCG AAAAAACCGA TCGCGAACTA 240
CGAAATACCA AGAAAGACAA GCAAGACAAA CAAAAGCATC TCAACCTCCT GAACAAGCAG 300
GTTGCTCAAC GCAAGCAGAT GGTACAACTC TTGGACAATG AGGTCAAAGA GTTGCAATCC 360
GACATTGATT CCATGACGGG TGTATGTCAT CAGCTCTCTG TAGAAGAGAA AGCCCGATCC 420
GATGAATATG CCCAAGCTCT ACAGTCTATG CAAAAGCGGA AACGCTCGTT GGATCGCATC 480
CTTTTCATTT CATCGGCCAA GAGCTTTGAC GAAGGCATGC GACGGATGCG TTTCTTGGAA 540
CAATACGCTT CTGCATACAA GCTGGCATCT GTCCGGCTGC GCGATACACG TAGCAAGTTG 600
GAGACTGAAC GTGCGACTGT AGAAGACGCC AAAAAGGAGA AAGGACATCT CTTAGTCATC 660
AGAGAAGAGG AAAAAAAGAA ACTCGAAGGA CAGCAAGCCG AGCAACGTCG GCAGGTGCAG 720
GCTTTGGGAG CCAAACAAAA AGACTTGGAA GCGCAGCTGC GAAAGCAGAA AAAGCAAGCC 780
GAAGCTCTGA ACAGAAAGAT CGAGAAACAG ATTGCCAAGG AAATAGAAGC TGCCGAACGT 840
CGTGCTCGAG AAGAACGTGA ACGGTTGGCA CGCGAAGCCA AAGCCAAGGG TAAGCCGGTT 900
CCTGCCGAAC CGGAACGGAA GGCGGAGACC AAAGGCGGCT ATGCTATGGA TGCCTCTGAG 960
CGTGCTCTCT CGGGCAGCTT TGCACAGAAC AAAGGTCGCC TGCCCGGCCC CGTTCGCGGC 1020
AGATACCGAA TCGTAAGCGA CTTTGGCGTG CATCAGCACA GTGAGCTGAA AAAAGTACAA 1080
GTTAATAATG GAGGTATCGA CATCGCTGTA GCAACAGGAT CCGATGCTAC CAGCGTATTC 1140
GATGGTGTAG TGTCCAGTGT ATTCGTGATA CCCGGTTATA ATTCGGCCGT AATGGTTCGT 1200
CACGGTAACT ATATCACGGT TTATGCGAAT CTGAGCAAAG TGTATGTAAA TTCCGGCACT 1260
CGTGTTAAAA CGGGTCAGGC TCTTGGTCGT GCCTATACGG ATCCTTCCAA CAACCAGACC 1320
ATTATTCACT TCGAAATCTG GAAAGAACGC AGCAAACAAA ACCCAAGACT ATGGTTACGA 1380

(2) INFORMATION FOR SEQ ID NO:66

(i) SEQUENCE CHARACTERISTICS:
(A) LENGTH: 1026 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1026

(xi) SEQUENCE DESCRIPTION: SEQ ID NO:66

AGTTTTTATC AAGAAATAGA CAGACTTATG AAAAAGTATT TGTTATATGC CTCGTTGCTA 60
ACGAGTGTTT TGCTCTTTTC CTGTTCAAAG AACAATCCTA ACGAGCCGCT GGAAGACAGA 120
TCCATCGAAA TTTCTATAAG GGTAGATGAT TTCACCAAAA CGGGTGAGGC AGTACGCTAT 180
GAAAGGAATC AAGGAAGTGC TGCCGAAAGG CTCATTACCA ATCTTTACCT CTTGTTGTTC 240
GATCAGTCAG GGGCGAATCC GGCGAAATAC TATATTACCG GTAACACTTT CACCGGAGGG 300
ACCTGGCTTC CTGACGATAT GAAGGTGAAG TTGGATATGA CACAATCCGA GGCCGGAGAG 360
CGCAAAGTAT ATGTCGTAGC CAATGTTGAT AATGCGGTTA AAACGGCTCT TGATGCTGTC 420
GCTAACGAAA GCGATTTGCA GACTGTAAAG AGGACGACTG CAATGCCGTG GTCGACCGAT 480
ATAGCCTCTC CTTTCCTGAT GTCCGGAAAC AAGACACACG ACTTCTTGGC CAATCGTCTT 540
TTGGACAATG TGCCCCTTGT GCGTGCCATT GCCAAGGTGG AGCTGAATAT CTCGCTGAGT 600
GAGAAATTTC AGATTGTGCC GATAATTGTC AATGGTAGTT TGAGTGAGTT CAAGTTCAGA 660
TACGTAAACT TCGACAAGGA GACCTACGTA GTGAAGCCAA CGACCAAGCC GGACAATCTC 720
ATTAGTTCTG CTAATGGTGT TTGGCCTCAG ATTACAGATT GGACTGTATG GGGTGCTTCC 780

TTAAATACTT CTCCTGCTCC GGATGCGGGC ACAGGTTATA CATTGGATGC AAATGGCAAG 840
GTAACGGCAC TACGGATTGT TACCTATCTG AATGAGCGCG ATAGCAAAGG GGCTACGGTA 900
GAGGTCGCAT TGCCTCGTGT GGATGATGGC ACCCTTCCTC CTCCGGAATT CGGTCCGGAG 960
CTTTATCGTT TGCCTTTGCC GGACAAGATC CTGCGCAATC ATTGGTACAA GTATCAAGTC 1020
GAGATT 1026

(2) INFORMATION FOR SEQ ID NO:67

(i) SEQUENCE CHARACTERISTICS:
(A) LENGTH: 987 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...987

(xi) SEQUENCE DESCRIPTION: SEQ ID NO:67

AACCTTAGGA CACAGCCTTT CTTTTTGGTA GATTTGCAAT CTATGATCAG AACGATACTT 60
TCACGATATG TATCCTCGAA CTTTTGGAGT CGGGGAGCTA CCTTTTTTTT CACGATTTTC 120
CCGGCCTTCA TCCTCGCCGC TACTGCTTTG CCGGCTTGTG GAGGGGGTAC TGCTTCAGGC 180
TCCGATCGTA CGCTGGCTGT GACCATCGAG CCACAGAAAT ACTTCATCGA GTCCATTGCG 240
GATAAGTCGG TGCAGGTGGT GGCATTGGTA CCGGCCGGCA GCAATCCGGA GGAATACGAC 300
CCTTCGCCTA CCGTGATGAA GCGTTTGTCC GAAGCAGATG CCTACTTCTA TATAGGAGGA 360
CTGGGGTTCG AGCAAAGAAA TCTCGCTGCC ATTCGGGACA ATAACCCTAA GCTCCCTCTT 420
TTCGAAATGG GCAAAGCCTT GGCGGATGCC GGAAGTGCAG ATCTCCACGG CTCCTGCACA 480
GATCATTCTC ATACAGACCT GCATGCCCAT GATCCGCACT ATTGGAGCAG TGTGGTAGGG 540
GCAAAGGCAC TCAGTCGTGC TGCATACGAC GCGCTTGTGG AGCTTTATCC GAACGAGAAA 600
GACAAATGGG ACAAAGGCGA CGACCGTCTC AACGGACGTA TCGACAGCGT GAAGAGACTC 660
GTCGATACCA TGTTTGCCAA TGGCAAAGCA GACAAAGCCT TCGTCATATA TCACCCATCG 720
CTCAGCTTTT TCGCCCAAGA GTTCGGCCTG CGGCAGATCG TCATAGAGGA AGATGGGAAA 780
GAGCCTACGG CTGCCCACCT TCGTCGTGTG ATCGATCAGG CACGTGCCGA TGGTGTCAGA 840
ATCGTATTTA TCCAACCCGA ATTTGAAACG CGTCAGGCGG AGGACATCGC ACGCGAGATC 900
GGTGCTCGTC CGGTAAGGAT CAATCCTCTG CGCAGCTCGT GGGAGGAGGA AATTTTACAT 960
ATTGCTCGCG CTTTGGCTCA TGAACGG 987

(2) INFORMATION FOR SEQ ID NO:68

(i) SEQUENCE CHARACTERISTICS:
(A) LENGTH: 2634 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2634

(xi) SEQUENCE DESCRIPTION: SEQ ID NO:68

GCAGATTCTA TTCGATATCC TCTTTACTTT TTTGGGCGGA ATCGGAAGAA ATGCTTTAGG 60
GAACCTATTC CCACCTTATA CAATAAAAAC ATGATCGGAA AAAAAATCTT TTTTATCCTG 120
CTGGCGCTCA TTGCGTTCAG TGGGCTGAAC GCAGCGACAG ACACTGAGTT CAAGTACCCG 180

ACCGATGCCA ATATCATCGG TCACGTCAAA GACAGCAAGA CGGGTGAACA CCTTGTCGGT 240
ATCACTATTG CTATCAAAGG CACTACCTTT GGTACATCTA CAGATGCAAC CGGGCACTAC 300
TATCTTCGTA ACTTGCGTCC GGGTGAGATC ACTTTGATTA TGCGTGGCAT GGGCTATAAG 360
AGCCAGGAGC GCGTAGTCCG CGTAGAAAAG GACAAGACTA TCGAGGTGAA TTTCGAAGCA 420
GAAGAGGATG CCATCAATCT GGACGAAGTC GTGATTTCGG CCAACCGCGA ACTGACGCTT 480
CGCCGTCTTG CTCCTACTCT GGTAAATGTA TTGAACGAAA AAGTCTTCTC GCAAGTCAAT 540
GCTTCTAACC TGGCTCAAGG CTTGTCATTC CAGCCGGGAG TTCGTGTAGA GAACAACTGT 600
CAGAACTGTG GTTTCAATCA AGTTCGTATC AATGGACTGG ATGGTCGTTA TGCACAGATC 660
CTCATCGACA GCCGTCCCAT CATGAGTGCC CTTGCCGGTG TTTACGGTCT GGAGCAGATC 720
CCTGCCAATA TGATCGAACG TGTGGAGGTA GTACGTGGTG GAGGATCGGC CTTGTACGGT 780
TCTTCTGCTA TTGCCGGAGT GGTGAATATC ATCACCAAGG AACCTTCTCA CAATTCTTTC 840
ACATTCAATG AATCTCTGAG CTTTACCGGT TTCAGCAAGC TGGATAACAA CACGAACTTC 900
AATGCCTCCA TCGTCAGCGA TGACAACCGT GCCGGTGCCA TGGTATTCGG GCAGGCTCGT 960
TACCGCAACC ATTGGGATGC TAACAATGAC GGTTATTCCG AATTGGGTAA AATAGATGCC 1020
CGCTCGCTGG GAGCGCATTC TTATTTGCGC TTGAGCGACT ACAGCAAATT GACGGGAGAG 1080
TTTCACACGA TCAGTGAATT CCGCCGTGGT GGCGATCGTA TCGATTTGCC TCCTCACGTA 1140
GTGGGTGTAG CTGAACAAAC TGACCATAGC GTATTTAGCG GAAACTTGAA ATACGATCTC 1200
TTCTCTTCCA ACTATAAACA CCACTTCCAG GCTTATACTT CCGGACAGAT CGTAAATCGC 1260
AAGAGCTATT ACGGAGGTAT CGGAGAGATT GACGTCAATG GCCACCCCGG TGGTACGGAA 1320
GGCTACCCTA TCCCTCAAGA TCAATACGGC AATAATTATG GCGTGACCAA AGGCAAGACA 1380
TATATGGGCG GTATCCAGTA CAGCTACGAC TTGGACAAAT TCCTCCTCAT GCCTTCGCAA 1440
CTTTTGTTCG GAGCCGAATA TACGCGTGAT GAACTCAATG ACGTGATGCC CATCCTTTCA 1500
TGGCAGACCG GCGAGGATGC CAATGGGAAT ACCATTCCCC TCTATCCCGA ATTGGATCAG 1560
AATATCAACA ACTACAGCCT ATTCGGTCAG AACGAATGGA AAAATGACAG ATGGAGCATC 1620
CTTGTTGGCG CTCGCTTGGA CAAGCATAGC GAAGTCAAGG ATATGATTCT GAGTCCTCGT 1680
ACCACACTGC GTTTCAACGT GAATCCGGAC ATCAACCTGC GCGCTACATA TGCAAAAGGG 1740
TTCCGCGCAC CGCAGGTATT CGATGAAGAC TTGCACGTAG GGGTTGTAGG CGGTGAGGCA 1800
CAGAAAGTAT TCAACGATCC GAACCTCAAG CCTGAAATTT CTCATGCATT CAGTTTGAGT 1860
GCCGATATGT ATCATCGTTT CGGTAACGTC CAGACCAACT TCCTTGTGGA AGGCTTCTAT 1920
ACTCGTTTGC TGGATGTATT CACCAACGAG GAGCAGCCTG ATCAGCACGA TGGCATCAAA 1980
CGCTACACGC GTATCAACGG TAGCGGAGCC AAAGTATTCG GTCTCAATCT GGAAGGTAAG 2040
GTCGCATACA AGTCCTTCCA GCTCCAAGCC GGTCTTACCC TGGCCAGCAA CAAATACGAC 2100
GAAGCACAGG AGTGGGGTCT GAATACGGTG AAAGACACCA ACGGAGCTTT TGTTACCGAG 2160
GCCAATGCAA ATGGACAACA GGAATACAAG AACGAATCCA TGACGGATAC GCAGATCACC 2220
CGTACCCCCA GCGTATACGG TTATTTTACT TTGGCCTACA ATCCTGCTCA CTCATGGAAC 2280
ATAGCCCTTA CGGGAGCATA TACCGGTCAG ATGTATGTAC CCCACGCTAT CGAATATGGT 2340
GTGAAGTCTG CCGAACTGGA TATTATGCAG AACAATCCTG AGATTACCGA CGAAACCGGA 2400
AAGGCTCCCC GTATTGATGA GCTGAAGAAG ACACCTGCAT TCTTCGATTT GGGCTTGAAA 2460
GTGGGTTATG ACTTCCACGT ATTCCAGGCT ACTGAGGTTC AACTCTATGT AGGTATGAAC 2520
AATATCTTCA ACTCTTTCCA GAAGGACTTC GATCGTGGAG CTGCACGTGA CAGCGGATAT 2580
ATCTATGGTC CTACGCAGCC GCGTACAGGC TACATGGGCT TGGTAGTGAA GTTC 2634

(2) INFORMATION FOR SEQ ID NO:69

(i) SEQUENCE CHARACTERISTICS:
(A) LENGTH: 618 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...618

(xi) SEQUENCE DESCRIPTION: SEQ ID NO:69

AAACAGATAG TTATGACAGT AAAGCGCGCA GTGCGAATAG CACTTCTCAC GCTGATAGGC 60
ATTCTTTTTT CCTCACCTTC TCTTGTTCGG GCGCAAAGTC TTTTCAGCAC CGAACATGTC 120
TTGCAACTAT ACAACAAGAT ACTCTATGGA GAGTCGGCGG CGGATACCGT CGCACAGAAA 180
ACGGCAGGTG AGTCGGCATT TCCTTTTATA GACAAACTCA TCAATCTCGG CCGCACTTTC 240
CTCGGCAAAC CATATCGCTA TCGCGGTCCT TCCCCATGGC CGATGGACTG CTCGGGCTAT 300
GTGTCTTACC TCTACTCCAA ATTCGACATC AAACTCCCAC GTGGTGCGGC AGCACAGAGC 360
CAATATACGA ATCCTATCGA GCGCGAGGAT GTTCGTCCGG GCGACCTCCT TTTTTTCAAA 420
GGCCGCAATG CACGCAGCAA CCGTATCGGG CATGTAGCTT TGGTCGTATC TGTCGATGAA 480
GATGATATTA CCATGATGCA CAGCCGCAAT TCGCGAGGGA TCGTGATCGA AAAACTCAAT 540

CGCAGTGCAT ACTTCTCCCG TCGCTTGGTG AGCTATGGCA GGGTACCCGG AGCCAAGAGA 600
GTGATCCCAC GAAAAAGT 618

(2) INFORMATION FOR SEQ ID NO:70

(i) SEQUENCE CHARACTERISTICS:
(A) LENGTH: 1401 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1401

(xi) SEQUENCE DESCRIPTION: SEQ ID NO:70

AAAGGTACGT GGAATAGAAA AAACCGAAGA GAAGAAATGA AACGGACAAT CCTCCTGACG 60
GCACTGACCG TCCTATCTTC GCTCTCCTTG CTTCGTGCAC AAAATGAATC CGAAGCATCA 120
ACCAATCCGA TGTCAGGCCT CTCCCTGGAA GACTGTATCC GGATAGCCAA GGAGCGCAAC 180
CTGAATCTGC GCAGACAGGA GATCGAACAA GAAAACCGAA TCATTAGTCT CGATGCAGCA 240
CGACACAGTT TCCTGCCCTC GGTCAATGCA GGCATCGGAC ACAACTATAG CTTCGGACGT 300
TCGAAAGACA AAACGGGAGT AACCGTAGAT CGCTCCTCGA TGAATACCAA TCTCAGCATC 360
GGAGCTTCGG TGGAAGTATT CAGCGGCACA CGTCGTCTGC ACGACCTCAA GCAGCAAAAG 420
TACAACGTGG AGGATGGTAT AGCCCGACTT CAAAAAGCGC GTGAAGACCT CAGCCTGCAA 480
ATCGCGGCTC TCTATATCAA TTTGCTCTTC CGTCAGGAAA TGACTCGTAC GGCAGAAACA 540
CAGTTGGCAC TGATTCGCGA GCAACGCAAT CGCACGGCCG AAATGGTTCG CGTAGGTAAA 600
TGGGCAGAGG GTAAGCTCCT CGACATAAAT GCCCAGATGG CCAAGGACGA ACAACTTCTC 660
GTACAATATC GTTCGGAGGA GGAGCTGGCT CGTCTGGACT TGGGGCAAGC CCTCGAACTG 720
GAGCACCCCG AAAGCATTGC AGTCAAGGCT CCCGACACAG ACGTTCTCGT AGCAGAAAGG 780
TTGGGATCTC TCCTTGCTCC CGAAGAGATC TATCGCACGG CTCTCGGCTT GAAACCGGCA 840
CTGCATTCGA GCGAGCTGCA AATAGCTTCG GCACGCGAAG GTCTGGCCTC GGCTCGTGCG 900
GCATACTTCC CGACGCTCAG CCTCTCTGCC GGATACAGCA ACGGTTACTT CCGCGACCTC 960
GGCAAGGAGT ATGCCGCCAT CAACCCCTCC TTCTCCGAAC AGTGGAAGAA CAACGGCAGC 1020
TACAGTATCG GACTCTCTTT GAATATCCCC ATCTTCTCTG CCATGCAAAC GCAAGATCGC 1080
GTTCGGAGCA GTCGCCTGCA AATACGCTCA AGCGAGCTTC GACTCGTCGA AGAGAAAAAA 1140
GCCCTCTATA AAGAGATCAG GCAAGCATAC AGCAATGCCG TGGCAGCCGA TAAGGCCATC 1200
GCAGCAGCCG AAAACAGCAA GGCCGCTACG CTCAAGGCAT ACGAATACGC TCGCGACAGC 1260
TTCGAGGCAG GGCGCTTGTC TGCCTACGAA TATGCCGAGG CAAAAACAAA ATACGCCCTC 1320
AGCCAAGTGG AAGAACTTCG TGCCAAGTAT GACTTCATAT ACAAAGCCAA AGTTTTGGAT 1380
TTCTATCAGG GCAAAGACTT C 1401

(2) INFORMATION FOR SEQ ID NO:71

(i) SEQUENCE CHARACTERISTICS:
(A) LENGTH: 1353 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1353

(xi) SEQUENCE DESCRIPTION: SEQ ID NO:71

AAAACATCAT ATCGGAATAA TATGCGTTTC CAACATTATC TCATCTGTAC GGCTGCCGTA 60
GCGGCTTTGG CTGCGAATCC CCTTACGGGC CAATCGAATA TGACCCTCGA AGAGTGCATA 120
GACTATGCAC GCCGGCACAG TTCGGCCGTG GCGCTGTCCG CTGCGGAACT GGAGCAGTCC 180
AAGGCCGATT ACCTTCAGGC CGTCGGCAAT TTTCTGCCCC GTGTATCGGC CGGAACCGGT 240
GCTTCGTGGA ATTTCGGACG CGGATTGGAT GCCGAGACGA ATACCTACAC CGACATCAAC 300
AGCTTCAACA ATTCGTACAG CATACATGCC ACGATGACCC TTTTCGACGG TTTGCAGAGT 360
GTCTATCGAC TGCGGATGGC GCATGCACGC CGGGAGGCTT CGCGCCTCTC CGTTCGCGAG 420
CAGCAGGAGC TGGCAGCTCT CGGCACCACG GAGGCCTACT ACGACCTCGT CTATGCGCGC 480
CAAATGCAAG AGCTGGCCAT GCAGAAGTAC GAGGAGAGCA GCCGCCTCCA CCGGCAGACG 540
GCTCGAATGG AAGAGCTGGG GATGAAGAGT CGTCCCGATG TCCTCGAGAT GCAGTCGCGA 600
ATGGCCGGTG ACCGTTTGGC CCTGACTCAA GCGGACAATC AGTGCATCAT CGCTCTGATC 660
CGGCTCAAAG AAAAAATGAA CTTCCCCATC GATGACGAAC TCGTCGTAGA CGATATGCCG 720
GCTGACAGTC TCTCCGCCGA CATGGCCGAA TCGGACAGCT CGGCCGGCGT CTTCGCCCGT 780
GCTGCCCATC ATCATCCCGT CCTCCTCCGT GCCAAACTCG ACGAGCAGGC TGCCACCGAC 840
CGTTTGCGAG CCGCGCGAGG TGCATTCCTG CCGAGTGTGT CGGTATCCGG AGGATGGAAC 900
ACGGGATTCT CACGCTTTTT GAATGGATCG GACTATACGC CCTTCAGCGA GCAGTTTCGG 960
AACCGTCGGG GGGAATACGT CAGTCTGAAT CTGAGTATCC CCATCTTTTC GGGATTCAGC 1020
CTTGTGAGCC ATCTGCGTCA GGCGCGTGCC GAACGCAGGG CGGCAATCGT CCGACGGGGC 1080
GAAGCGGAGC GCAGGCTCTA CAGCGAGATC GCCCAAGCCA TGGCCGACCG GGATGCCGCT 1140
CTGGCTTCCT ACCGCCAGGC GAAGGAGCAT ACCGACGCCA TGCAAACCGC TTACGAAGCC 1200
GTCTTGCAGC GTTATGAGGA GGGGCTGAAT ACGGCCATCG ACCTGACCAC TCAGGCCAAT 1260
CGGCTCCTGG ATGCCCGTGT GCAGCGACTG AGAGCGGCCA TGACCTACCG GCTCAAATGC 1320
AAACTCATAG CCTATTACGG CTGCCTTTCG GAC 1353

(2) INFORMATION FOR SEQ ID NO:72

(i) SEQUENCE CHARACTERISTICS:
(A) LENGTH: 2886 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2886

(xi) SEQUENCE DESCRIPTION: SEQ ID NO:72

GCCATTTTTG TCGTATCATT GCAAATTGAA AAAATAACAG AGAATAAGTA TAATTCAGAC 60
AAGAGCATGA ACAAATTTTA CAAATCACTT TTGCAGTCAG GACTGGCTGC CTTCGTGTCG 120
ATGGCAACTG CACTGACCGC TTCTGCACAG ATTTCGTTCG GAGGGGAACC CTTGAGTTTC 180
TCTTCAAGAT CCGCCGGAAC GCATTCATTC GACGATGCAA TGACTATCCG CCTTACTCCG 240
GATTTCAATC CGGAAGACCT GATCGCACAG AGCCGTTGGC AATCGCAAAG AGATGGCCGG 300
CCCGTCCGGA TAGGACAAGT AATACCGGTG GATGTGGACT TTGCATCCAA GGCTTCGCAC 360
ATCTCTTCCA TCGGAGACGT AGATGTATAT CGCCTGCAAT TCAAGTTGGA AGGAGCCAAA 420
GCCATTACGC TTTATTACGA TGCATTCAAT ATTCCGGAGG GCGGACGCCT CTATATCTAT 480
ACCCCCGACC ATGAAATTGT GTTGGGAGCA TATACGAACG CCACTCATCG CCGCAACGGA 540
GCTTTTGCCA CAGAGCCGGT ACCGGGGAGT GAGCTTATTA TGGATTATGA AGTGTCTCGC 600
GGAGGGACTT TGCCTGACAT CAAGATCTCC GGTGCGGGTT ATATATTCGA CAAAGTCGGC 660
GGACGCCCCG TAACGGATAA CCATTACGGG ATCGGTGAGG ACGATTCCGA TTCGGATTGC 720
GAGATCAACA TCAATTGTCC TGAAGGTGCA GACTGGCAGG CAGAGAAGAA CGGTGTGGTG 780
CAAATGATCA TGGTAAAAGG ACAGTATATC TCAATGTGCT CAGGCAACCT GCTCAATAAT 840
ACGAAAGGAG ACTTTACTCC GCTGATCATT TCTGCCGGAC ACTGTGCTTC CATAACAACC 900
AATTTCGGTG TAACGCAATC CGAGTTGGAT AAGTGGATCT TCACTTTCCA CTATGAAAAA 960
AGAGGATGCA GCAATGGTAC ATTGGCCATC TTCCGTGGCA ACAGTATCAT CGGAGCTTCC 1020
ATGAAGGCTT TCCTCCCGAT CAAAGGTAAA TCCGATGGTC TCTTGCTGCA ACTCAACGAT 1080
GAAGTCCCTC TGCGCTATCG TGTCTATTAC AATGGATGGG ACAGTACGCC CGATATTCCC 1140
TCGAGCGGTG CCGGTATTCA TCATCCGGCC GGAGATGCCA TGAAGATTTC CATCCTAAAG 1200
AAGACTCCGG CTCTGAATAC ATGGATCTCC TCCAGTGGTT CCGGAGGGAC TGACGATCAC 1260
TTCTATTTCA AATACGATCA AGGTGGTACG GAAGGAGGAT CGTCCGGTTC TTCTCTCTTC 1320
AATCAGAATA AGCACGTGGT CGGCACACTG ACCGGAGGTG CCGGCAATTG TGGCGGGACG 1380
GAGTTCTACG GCAGACTGAA CAGTCATTGG AACGAGTATG CATCCGATGG CAATACGAGC 1440
CGCATGGACA TCTATCTGGA TCCCCAAAAC AATGGCCAGA CGACCATCCT CAACGGAACG 1500
TATCGTGACG GTTATAAGCC TTTGCCCTCT GTGCCCCGGC TATTGTTGCA GTCTACAGGC 1560

GATCAGGTCG AATTGAATTG GACGGCTGTT CCTGCCGATC AATATCCATC ATCTTATCAG 1620
GTCGAATACC ACATATTCCG AAATGGAAAG GAAATAGCTA CGACAAAGGA GTTGTCCTAT 1680
TCGGATGCCA TCGACGAAAG TATTATCGGT AGCGGTATCA TTCGATACGA AGTAAGCGCA 1740
CGCTTCATTT ATCCCTCGCC GTTGGATGGA GTGGAATCTT ATAAGGATAC GGACAAGACT 1800
TCTGCCGACC TTGCCATAGG AGACATTCAG ACCAAGCTGA AGCCGGACGT AACACCTCTC 1860
CCCGGAGGAG GAGTATCATT AAGCTGGAAA GTTCCTTTCT TAAGCCAGTT GGTTTCCCGA 1920
TTCGGAGAAA GCCCCAATCC TGTGTTCAAA ACCTTTGAAG TGCCCTATGT TTCTGCCGCA 1980
GCCGCACAAA CCCCCAATCC TCCCGTTGGC GTAGTCATTG CAGACAAGTT TATGGCCGGT 2040
ACATATCCCG AAAAGGCTGC TATCGCTGCC GTTTATGTAA TGCCATCCGC TCCGGACTCT 2100
ACTTTCCACC TCTTCCTCAA GAGCAACACA AACAGAAGAT TGCAGAAGGT GACAACTCCC 2160
TCCGATTGGC AGGCCGGAAC ATGGTTGAGG ATCAATTTGG ATAAGCCGTT CCCGGTGAAT 2220
AATGACCATA TGCTTTTTGC CGGTATCAGA ATGCCTAATA AGTACAAGCT CAATCGTGCT 2280
ATCCGTTATG TAAGAAATCC GGATAACCTT TTCTCCATTA CCGGTAAGAA GATTTCATAT 2340
AACAACGGAG TCTCTTTCGA AGGCTACGGA ATACCCTCGC TCTTGGGCTA TATGGCTATC 2400
AAATATCTGG TGGTAAATAC CGATGCTCCG AAGATCGATA TGTCGCTTGT ACAGGAGCCT 2460
TATGCTAAGG GAACGAATGT GGCTCCATTC CCCGAATTGG TCGGCATATA TGTCTATAAG 2520
AACGGAACAT TTATCGGCAC ACAGGATCCA TCCGTCACAA CTTATTCGGT TTCAGACGGA 2580
ACAGAGAGCG ATGAATACGA AATAAAACTG GTATATAAGG GATCGGGCAT TTCGAATGGC 2640
GTTGCTCAGA TTGAGAATAA CAATGCTGTC GTTGCATATC CGTCTGTTGT AACAGATCGT 2700
TTCAGCATTA AGAACGCTCA TATGGTTCAC GCTGCCGCCC TCTACTCATT GGATGGCAAG 2760
CAGGTTCGTT CTTGGAACAA CCTCCGCAAT GGCGTGACAT TCAGTGTTCA AGGACTTACG 2820
GCCGGTACTT ATATGCTCGT TATGCAGACG GCAAACGGCC CTGTGAGCCA AAAGATCGTG 2880
AAGCAG 2886

(2) INFORMATION FOR SEQ ID NO:73

(i) SEQUENCE CHARACTERISTICS:
(A) LENGTH: 2106 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2106

(xi) SEQUENCE DESCRIPTION: SEQ ID NO:73

TGGAAAAAAA GTAATCCTCA ATACCGGGGC AGGACAGCAA ATTACCGAGA TCATTATAAT 60
ACAGAAAGCT ATTTAAGCCT AAGGAGGAAT CAAACAATGA AATATCTTAT CAGACTCTTC 120
TTATCATTGA TGTTACTCTC TCTCTGGACG GGCTGTACAC ACGAGGAGCT CTCTATTTGC 180
GATGGCGAGA ATACGCTTGT TTTACGCGTA GAGACCGGTA AAGCCCCAAA TGCTCGTGCC 240
ACAGAACCCG GTCAGGGCAT ATACAATGAG AATAAAGTAG GCTCCATTTC TGTGCTCTTC 300
TATTTACAGG GACAACTTCG TTGGCAGGTG AAGTCTACAG ACTATCAAAT CCATGAAGGG 360
GCCTATATCA TTCCGGTCAA AGAGCAAATG CGACCACTAT TCAATGGCAA CAACAACTTC 420
AGCATCTATG TAGTGGCCAA TCTCGATTTC AATGCTCCGG CCACAGAAGC TGCGCTTTCT 480
CAATTTGTGG TAGAGAAATC TATTGAAGTC TCTTCTACGA CAGCCCCTGC CGATTTCGTA 540
ATGCTTGCTC ATGGCAATAA GCAGATCAAT ATGGCTACGA CAGAAGGGAA ACTGTTGGGG 600
GATTATAAAC TCAAACGAGT GGCAGCAAAG ATTCGCATGA TAAAACCCAC CATCAATGTG 660
CAAGGATATG AAGTGGTCGG AAATATACAG GCAAAGTTTC GCAATTCGGT AACGAAGGGG 720
TTCCTTACCA CAGAAGCTCA AGAGATCCCA GCTGCTGCAT CCTATAAGAC ATCGGAATAT 780
CTTGATATTG CAGAGTCGGC ACCTGCCAAT TCTATCCATT TCTATTCTTA CTATAACAAA 840
TGGACACTCT CCACACCGGA GAAGCGACCG GAATTCTTCA TCATGGTCAA ATTCAAAAAG 900
ACAGGACAGC CGGACAACAC AGCCAAACCG TACTACTACA GAGTGCCCCT CGAATCTCAG 960
GACAATCAGG TCAAGAGCAA TGTCCTCTAT AATCTGAATG TGAAAATCGA AATCTTGGGT 1020
TCTTTACAAG AGCCGGAAGC TGTTTCTGTA AACGGCACAC TCGCAATAGA AGAATGGATT 1080
CTCCATCAGG ATGCATTCAA TCTGCCTGCC ACCAATTACT TGATAGTGGA ACAGCACGAA 1140
ATCTTCATGA ATAACGTGAA CACATACTCG GTGAAATATC AAACTTCGCA GAAACCAATC 1200
AGCATTAGCA TACAGTCAGT TACCTTTAGC TACGTCTCTT CTGATGGCAC TCAGCACAAT 1260
GATCTTGTAG CAAGTAGTAG CGACCAGTAT CCTACGATTA CAAGCGATAA TACAAGCATC 1320
ATAATCACTT CCAAGATACC GGTTAATAAC GTACCAAAGA AGATCGTTTT TGAGGTAACT 1380
AATGGGGTAG CCGGTTTGAA AGAGACTGTC ACAGTACTCC AATATCCTGC ACAATTTATT 1440
GTCAATACAC TTGGCACAGC ATCGGCATGG AGACCAGACG GATCTTTGGC TCCGGGGCTT 1500
AACAATAAAG CGATTTACCA TGTCGTAGTA CTGCTTCCAC CCGAGAATTT ATTTGAAGAT 1560
GGGACACAGA CAATCATCGG TTATCCCCCC ACTGAAACAA TTTCTTTTCA TAAGAAAGAG 1620

AACAATACCT ATCCGATAGT ATGGTCTGAC ACAAATACGA CAAAACAGGA CCTTGAGACA 1680
TCAAGAATGA TTTCACCTTC CTTTGAGTTA GCCTCCCAAC TTGGGGCTAC TCTCCCGATG 1740
CCCTATCTCG AGTATTGGCC AGGGACATCA TATCTCCTTG ACTATTCGGG AAACTATAAT 1800
AATAAGAGAT ACGCCTTGTT TAATTGCGCT TTTTACTGGG AGAAAAGAAA AGTTAATAAC 1860
GAAGAAATTA AATTCGATGA CTGGCGTTTG CCGACAGAAG CTGAGATCAA ATTGATAGAT 1920
AAGCTGCAAC ATAATGAGCA GAGTGCTGTC CAAGCTATCA TGACAGGGAA TTATTATTGG 1980
GATAGTTACT CTGCAAATGG GTCTTATAAA ATGCAAGGAG GAGGGGGCCA AGGAAATTCC 2040
TCCAAAGCCT ATGTTCGTTG CGTGCGGGAT GTGAAAAAGC CGATTCGTGA CAAGAAGTCA 2100
GGTAAG 2106

(2) INFORMATION FOR SEQ ID NO:74

(i) SEQUENCE CHARACTERISTICS:
(A) LENGTH: 3936 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...3936

(xi) SEQUENCE DESCRIPTION: SEQ ID NO:74

AAGAGGGTAG ATAGCTATGA ATGCAGACAA AAAGCTTGCA AATGTGCAAT TTGTGTGATA 60
CAAAAATTTA CTAATGTAAA ACTAAATGAT ATGCGAAAAA TTTTGAGCTT TTTGATGATG 120
TGCTCTCTGC ATTTAGGTCT ACAATCTCAG ACTTGGCATG GAGATCCGGA CTCAGTGGCA 180
GCCCTACCTT CTATCGGTAT TCAAGAGTCA AGTTGTACCC GAATCACGTT CGAGGTTGTT 240
TTCCCCGGAT TTTATAGTGT GGAAAAACGA GAAGGCAACC AAGTCTTTCA GCGCATTTCC 300
ATGCCGGGTT GTGGCTCGTT TGGGAATCTG GGCGAAGCTG AATTGCCTGT TTTGAAAAAG 360
ATGATAGCCG TTCCGGAATT TTCAACAGCT AACGTTGCTG TAAAAATCAA AGAGACGGAG 420
ACATTCGACA ATTATAATAT CTATCCTAAT CCTACCTATG TCGTAGAGGA GTTGCCTGAG 480
GGGGGGACTT ATCTGGTAGA GGCTTTCGCG ATAAACAATG ACTATTATAG CCAAAATGTA 540
AGCCTCCCTT CTACTCACTA TGTCTATTCT CAAGACGGGT ATTTTCGCTC ACAAAGATTT 600
ATCGAAGTTA CCCTGTATCC TTTTCGATAC AACCCTGTCC GACAAGAAAT TCTATTTGCA 660
AAAAAAATCG AGGTTACAAT AACTTTCGAT AATCCTCAGC CACCTTTACA AAAAAACACC 720
GGCATATTTA ACAAAGTAGC CTCCTCTGCA TTTATTAATT ATGAAGCTGA TGGCAAATCG 780
GCGATAGAAA ATGATATGGT GTTCAGTCGT GGTACAACAA CGTACATAAG CGGAAATGTT 840
GCCAGCAACC TCCCTCAGAA CTGTGACTAC TTGGTTATTT ACGATGATAT GTTCAACGTA 900
AATCAACAAC CACACGACGA AATCAAACGG CTGTGCGAAC ATAGAGCCTT CTACAACGGC 960
TTTGATGTAG CTGCTGTAAG TATAAAGGAC GTATTGAATA GCTTCCCATC AAATGCCACC 1020
TCATACATCA ACGAAACTAA ACTGAAAAAT TTCATTCGCT CAGTTTACAA CCAAAGCAAT 1080
GCGAAGAGGA CTTTAGATGG CAAACTGGGA TACGTGCTAC TGATCGGAAA ACCATTGAGC 1140
AAATATTTGG CTGACACTGA TAATACAAAA GTCCCAACCT CTTTTATTCA TAATGTCTCC 1200
TTAATTCCAA GTCATCCAAC TTTTGGTTCC ATATGCGCCT CCGACTATTT TTTTAGTTGT 1260
GTTTCGCCCC TTGATACTGT CGGCGATTTG TTTATCGGTC GATTTAGCGT CACCAATGCT 1320
CATGAATTGC ACAATCTGAT TGAAAAGACT ATCAACAAAG AAATCTCATA TAATCCTATT 1380
GCACACAAAA ATATTCTTTA CGCAGAAGGG AAAGGCTGCG ATGCTCCAAT CTTACGTTTA 1440
TTCTTAAAAG AAATCGCCTC TGGTTACACA GTCAACTCTA TCTTAAAATC TAATCAGGTC 1500
TCTGCAATAG ACTCGATATT TGACTGCTTG AATAATGGTT CCCATCATTT TTATTTTAAC 1560
ACTCATGGAA TGCCGACTGT TTGGGGGATA GGGCAGGGAC TCGACGTCAA TACTCTAACA 1620
GCCCGATTGA ACAATACATC TTCGCAGGGA TTATGTACGA GTCTATCATG TAGTTCGGCT 1680
GTAGCAGATT CAACTATTAG ATCGCTTGGA GAAGTCCTGA CCACATACGC ACCTAACAAG 1740
GGATTCTCGG CTTTCTTAGG AGGAAGCAGA GCCACCCAAT ATGCCGTTTA TTTAGAAGGC 1800
CCCTGTCCTC CGTCAGAATT TTATGAATAT TTACCTTATT CTTTATATCA CAATCTCTCG 1860
ACTGTTGTTG GCGAAATGTT GCTATCATCC ATTATCAATA CTAATTCTGT TGATACGTAT 1920
TCGAAATTCA ACTTCAATTT GCTTGGCGAC CCTGCACTAA ACATTATGGC TCATGGCATG 1980
GAGGTTAGTA ATTGTATTAC ACTACCAAAC AACACCATTA TAAGCAGTCC GATAACAATA 2040
AAAAATGGTG GCTGCCTAAA AATACCGGAA AAAGGAGTTT TGCATTTTAC TAATAATGGC 2100
TCCATACAAG TCATGTCCGG AGGAACTCTG GAAATAGGCA ATCAGGCTAA AATATCCGGA 2160
GAGACCGGTG CTAACCCCAC CTTTATTACC GTTTACGGCG ATGGTCTTGC GATTAACAAG 2220
CAGGTAGAGA TAGACAATAT AGACCGACTT AACTTGTTTT CTACGCATTC GGTCATGCCC 2280
AAATTTCATT TTGACAGTGT GAAATTCAAC AGTGCCCCGC TGTATACAAC GAACTGTATT 2340
GTGGAGATAA GCAATTGCGA ATTTACCAAT CGAAGTGACA TTATTTCAAA GAATTGTGAC 2400
CTAAGCGTTG AAAACAGTAT GTTTAGCAGT TCGGGGATAA CGGTATTCAA GCCTATGGCT 2460

ACAAGCTCCA TCACCGGATT ATCTACAAAA GCAAAGATTA CCGACAATAC TTTTTTTGCG 2520
ACAGGAAACT TCGCCTACCA TATCACAAAC ACGCCAGGCT TAACAGCAAC CTCCAATGCT 2580
GCCATCAAGT TAGACAATAT TCCTGAGTAT TACATTTCCG GTAATAAAAT AGTCAATTGC 2640
GATGAGGCTC TTGTACTAAA TAATAGTGGC AACAGAACGA ACAGACTCCA CAATATCACA 2700
CGGAATGTGA TAAAAAACTG TAGGATTGGG AGCACGCTTT ATAATTCCTA TGGTATTTAC 2760
AACCGAAATA AGATCAGTAA CAATCATATA GGAGTACGTC TCCTCAACAA CAGTTGTTTT 2820
TATTTCGATA ATGCTCCTGT AATCAATGAA GAAGATAAGC AGACGTTTAT TTCTAATAGG 2880
ACTTGGCAGC TCTATTCATC AAACGGTACA TTCCCTCTCA ACTTCCATTA CAACAGCTTG 2940
CAGGGGGGAG ATACAGATAC ATGGATTTAC AACGACACGT ATACGAATCG CTATATTGAC 3000
GTTTCAAATA ATCACTGGGG CAACAATGAT TTGTTTGATC CGAATCAGGT TTTCAATACG 3060
CCAGACTTGT TCATTTGGAT ACCTTTTTGG GATGGATTGC CAAATGGGAG ATCGGGCAAT 3120
AGCTCTGCTG AAGCAGTAGA ATTCCAAACA GCATTGGACT GTATTGGCAA TAGCGATTAT 3180
CTTTCGGCAA AAGTGGCTCT CAAGATGATG GTTGAAACCT ACCCGGAATC CGACTTTGCA 3240
ATAGCTGCTT TGAAGGAATT GTTCAGGATA GAGAAAATGT CAGGCAACGA TTACGAAGGC 3300
TTGAAAGATT ATTTCAGATC CAATCCAACC ATCATCTCTT CCCAGAACTT GTTCCCGACA 3360
GCTGATTTCC TGTCTGCGCG ATGCGATATT GTGTGTGAAA ACTATCAGTC TGCCATCGAT 3420
TGGTACGAAA ATCGCTTGAA TAGTGAAATC TCCTATCAGG ACAGTGTTTT TGCAGTCATT 3480
GACCTTGGTG ACATTTATTG GAATATGCAG TTAGACTCAC TCAGAGGGAC TGGTATAGAT 3540
TTGAACATAC TTTCCTGTGA ACAAAGGAAA TCGCTCGAAA GCCATCAAAA TGTAAAAAAT 3600
TATTTGTTGT CAACTCTTCC CGAATCAACA GGTACTCTCC TGCCTCCATT AGAATGCAAC 3660
AAATCAAGCC TTGATAAATC CAAGATAATC TCTATTTCGC CCAATCCGGC GAAAGCTGTT 3720
GTAACAATAA TCTACTATAC CGATAACCCT TCCTGTTCTG TAATAAAAAT ATATGGAATA 3780
AATGGACCCT CGGCTGATAT AACCGGGTTG CCCAAACATC TATCCGAAGG TTATTACAGC 3840
ATACAGTTCA ATACATCCAA CTTTGATCCC GGTTTCTACC TGGTAACGCT AAATGTTGAT 3900
CAGAAAATTA TAGATACGGA AAAATTACGA ATCAAA 3936

(2) INFORMATION FOR SEQ ID NO:75

(i) SEQUENCE CHARACTERISTICS:
(A) LENGTH: 2814 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2814

(xi) SEQUENCE DESCRIPTION: SEQ ID NO:75
   TCAGAAAATT ATAGATACGG AAAAATTACG AATCAAATAA TGGCTATCAT GATGAAAAGT 60
   ATTGTTTTTA GAGCATTTCT AACGATTTTG CTCTCGTGGG CAGCGATCAC GAATCCGACT 120
   GCTCAAGAGA TCTCAGGCAT GAATGCATCC TGTCTGGCTG CTCCGGCTCA ACCGGATACT 180
   ATCTTATATG AAAGTTTTGA GAATGGACCT GTTCCCAATG GCTGGCTTGA GATAGATGCT 240
   GATGCTGATG GTGCCACTTG GGGAAGCCCA TCAGGCTCTT TCTCTGTACC TTACGGACAC 300
   AATGGCCTTT GCACCTACTC CCATATACGT TCCGGTATCT CAACAGCGGG CAACTATCTG 360
   ATTACACCCA ATATAGAAGG AGCCAAACGG GTCAAGTACT GGGTATGCAA TCAGTATAGT 420
   ACCAATCCGG AACATTACGC AGTAATGGTA TCGACAACGG GGACTGCCAT TGAAGACTTT 480
   GTTTTGTTGT TTGATGATTC CATAACAGGG AAACCGACTC CTCTTGTATG GCGTAGACGA 540
   ATCGTGGACT TACCGGAAGG GACCAAATAT ATTGCATGGC GACATTACAA AGTCACCGAC 600
   TCACACACAG AATTCTTGAA ATTGGATGAT GTCACTGTGT ATAGGTCGAT CGAAGGGCCC 660
   GAACCTGCTA CCGACTTCAC AGTAATCAAT ATTGGTCAGA ATGTGGACGA ATTGACTTGG 720
   AACTATCCGG AGGATTATCA ACCGGAAGGA AAGGGGAATG AAGAGTTGCA GCTTAGCGGC 780
   TACAACATCT ATGCGAACGG TACACTACTG GCACAAATAA AAGATGTCTC CATACTGGAG 840
   TATGTGGACA GCACTTACTC TTTGCGAGAC AATCCCTTGC AAGTGGAGTA CTGCGTTACA 900
   GCCGTTTACG ATGAAAGCAT AGAATCTTCG ACCGTATGTG GCACGCTGCA TTACGCCACG 960
   GATGCCATCC TTTATGAAAA TTTTGAGAAT GGACCTGTTC CCAATGGTTG GCTTGTGATA 1020
   GACGCTGATG GAGATGGATT TAGCTGGGGA CACTATTTGA ATGCATACGA CGCTTTTCCC 1080
   GGCCATAATG GAGGCCATTG CTCCTTGTCG GCTTCTTATG TTCCGGGTAT AGGCCCGGTG 1140
   ACTCCCGACA ACTATCTGAT TACCCCCAAG GTTGAAGGAG CCAAACGTGT CAAGTACTGG 1200
   GTAAGCACGC AGGATGCCAA TTGGGCAGCG GAACATTACG CGCTGATGGC TTCGACAACG 1260
   GGGACTGCTG TCGGAGATTT CGTCATATTG TTCGAAGAAA CCATGACAGC GAAGCCGACC 1320
   GGCGCATGGT ATGAAAGAAC CATCAACTTA CCTGAAGGGA CTAAATACAT CGCATGGCGG 1380
   CATTACAACT GTACCGATAT ATATTTCTTG AAGTTGGACG ATATCACTGT ATTCGGGACT 1440
   CCTGCATCAG AGCCCGAACC TGTTACCGAT TTCGTTGTCT CGCTTATTGA AAACAACAAG 1500
   GGACGATTAA AGTGGAATTA TCCTAACGGC TACGAACCCG ATAAGACTGA TGATAAAGAC 1560
   CCATTGCAGC TTGCCGGCTA CAATATCTAT GCAAACGGCT CGCTCCTTGT TCACATACAA 1620
   GACCCGACTG TTTTGGAGTA TATCGATGAG ACTTATTCTT CACGAGACGA TCAGGTGGAA 1680
   GTGGAATATT GTGTCACTGC CGTTTATAAC GACAATATCG AGTCCCAATC GGTTTGCGAT 1740
   AAGCTGATTT ATGATTCTCA ATCGGACATT ATCTTATATG AAGGCTTTGA GGCCGGAAGT 1800
   ATTCCTGAAG GCTGGTTGTT GATTGATGCT GATGGCGACA ATGTTAATTG GGACTATTAT 1860
   CCTTGGACTA TGTATGGACA TGACAGTGAG AAGTGTATTG CATCCCCTTC GTACTTACCG 1920
   ATGATTGGCG TTTTAACTCC GGATAACTAT TTGGTTACAC CCAGACTCGA AGGAGCCAAG 1980
   CTTGTCAAGT ATTGGGTAAG TGCGCAAGAT GCTGTTTATT CGGCTGAGCA TTATGCTGTG 2040
   ATGGTTTCTA CTACGGGAAC TGCTGTTGAA GATTTTGTCC TCTTGTTCGA AGAGACAATG 2100
   ACCGCTAAGG CTAACGGTGC ATGGTATGAG CGAACTATTA CATTGCCTGC AGGAACAAAA 2160
   TATATTGCCT GGCGGCATTA TGATTGCACC GATATGTTTT TCTTGCTCTT GGATGACATT 2220
   ACGGTTTATC GTTCTACTGA GACTGTTCCC GAGCCTGTTA CTGATTTCGT TGTCTCGCTT 2280
   ATTGAGAATA ACAAGGGTCG CCTGAAATGG AATTATCCTA ACGGCTACGA ACCCGATAAG 2340
   ACTGATGATA AAAAACCATT GCAGCTTACC GGCTACAACA TCTATGCAAA TGGCTCGCTC 2400
   CTTGTTCACA TACAAGACCC GACTGTTTTG GAGTATATCG ATGAGACTTA TTCTTCACGA 2460
   GACGGTCAGG TGGAAATGGA ATATTGTGTC ACTGCCGTTT ATAACGACAA TATCGAGTCC 2520
   CAATCGGTTT GCGATAAGCT GAACTATACT ATCACATCCT TGGATAATAT TCAATCTGAT 2580
   ACAAGCTTGA AAATATATCC TAATCCGGCA TCGTATGTGG TAAGGATAGA GGGATTGAGT 2640
   CGGAGCAAGT CGACAATCGA GTTGTATAAT GCGCTGGGAA TTTGCATATT AAGGGAAGAG 2700
   ACTCATTCAG AGAAAACGGA AATCGATGTT TCACGTCTCA ATGACGGAGT CTACTTGATT 2760
   AAAGTAGTCG GTGGAAATAA AACAACAACC GAAAAGGTAG AGATAAAGAG GCCG 2814

(2) INFORMATION FOR SEQ ID NO:76

(i) SEQUENCE CHARACTERISTICS:
(A) LENGTH: 1818 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1818

(xi) SEQUENCE DESCRIPTION: SEQ ID NO:76

ATAATCTTCT GTACGATTCA TCACTCTGAG TTGGAAATAA TGAACAGCAT CATGAAATAT 60
CAATTATATA CGGCCGTCAT AATGGCTCTC TCTGTATCAT CCGTTTGCGG TCAAACCCCA 120
CGAAATACAG AAACCAAACG CCCCGACACG CTGCGCAGGG AGCTTACTAT CGTTAATGAC 180
CAGACTGTGG AGATGGAGCA TGCGGATCCG CTTCCGGCTG CATACAAGGC CATCGAACCT 240
CGATTAAAAC CTTTCCGTCC GGAATATAAC AAGCGTACAT TCGGATTTGT CCCTGAAGTT 300
TCCTCTTCAG GCAGGAACAA TCTTCCGAAT ATCCTGCCGA CGGAAGGTCA TATGAAGCAC 360
CGGGGGTACC TGAATATCGG TATCGGCCAT ACGCTAAACC AGCGAATGGA TGCCGGCTAT 420
CGTCTGATAG ATGCAGAGCA GGAGAGACTG AATCTTTTCC TCTCCTATCG TGGGATGAAA 480
TCGGCTTTCA ATACCGGTGA CTTCGACGGC GACAGAAAGG ATAGACGAAT GATGGCAGGA 540
GTGGACTACG AGCAGCGCAG GCCTTCCTTT GTGCTTGCTA CCGGCTTGTA TTATTCGAAC 600
CATTATTTCA ATAACTACGG ACGGGGAGCT ACCACCAATG TGGGCAGCAT CCCTCAGCTA 660
TCGACACCTG TTACTCCTCA GATGGACAAC GGGACCCACA ACGTCCGTGT ATACTTGGGT 720
GCAAAAAATG ATGTGATCGA TGCCAGGATC GACTATCGTT TCTTCCGTTC TATTCCCTAT 780
CTGGGTACCG ATCCGATGAA GGCTCTCACA GAACATACGC CTGAACTGAA CGTGACGATG 840
AGTAATGAGT TGTCCGATGA TATTAAGCTC GGTGTCGAAG TTCGTACGGG AGGATTGTTT 900
TTTGCCAAAA ACAGCGAAAT GATTCAAACG GGCGTTCTGT CCGAAACCGA CCGCAACCTG 960
TATTATGTGG AGGGCGCGCC CACAATCGGA TTTGTCGGAG ACTCGGACAA TATGCAATGG 1020
AACATACAGG CCGGAGTAGG GATTTCTTCC CATTTCGGAG CCAAAGGGAG GTTGTTTTTC 1080
TGGCCTAAAC TGGATGCTTC GCTTAGTATC TTCCCTTCAT GGCGTGTGTA TGCGAAAGCC 1140
TTCGGCGGTG TGATTCGAAA TGGTCTCGCC GATGTTATGC AAGAGGAGAT GCCCTACCTG 1200
ATGCCCAATA CGATTGTACT CCCTTCGCGC AATGCTTTGA CCGCCCAATT AGGGGTGAAG 1260
GGGAATATAG CCGATGTGGT ACGTATGGAG GTTTATGGCG ACTTCTCCAA GCTGACAGGT 1320
GTGCCTTTCT ATACTCCGAC TCTACCCTTA TATAATCCAT CCGACTTGTA TCAGTATAAT 1380
GTGAGTTTCT TGCCGATATA TGCCGACGGC AGCCGCTGGC GCGCAGGTGG TAAGCTGGAA 1440
TACTCTTATC GCGATATGCT CCGCTTTCTG GTAGACGCAT CCTATGGCAA GTGGAATTTG 1500
GATGGAGGAC TTGTCGCCTC CATGCAGCCC GATCTTATAT TGAAGGCAGA AGTAGGTGTT 1560
CATCCCATTG CCCCATTGGA TGTCAGACTC CGGTATACAC AGCTGAACGG ACGGTATCGG 1620
TATTCTTTCG GCTCGGCTGG CTCGGAAGCC TTGGGTATCG GTAATGTACA TCTTCTTAGT 1680

GCGGATGTTT CATACAAGCT GAAAAAGAAC TTGAGCCTTT ATCTCAAAAT CGATAATATG 1740
CTGGCGGAAA CGACAGAGCT TATCGGTTAT TATCCTATGC AGCCGTTCCA TTGTTTCGCC 1800
GGTTTTAGCT GGACTTTC 1818

(2) INFORMATION FOR SEQ ID NO:77

(i) SEQUENCE CHARACTERISTICS:
(A) LENGTH: 1071 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1071

(xi) SEQUENCE DESCRIPTION: SEQ ID NO:77

AACTCCCATG CAACAGTCTC CGTTATATGT TCAATGATGG AAAAATGTAT CTTTGCTCAC 60
TATCCACATA ACCTTGTGTT CATGATTCGC AAGCATTTCG GTATCATTTT GGGATTTCTT 120
TCTCTTGTGT TTTCGGCAGG TGCTCAACAA GAGAAGCAGG TGTTTCATTT TCTGAACCTT 180
CCGGCTACTG CACAGGCTTT GGCTGCCGGA GGCAAAGCTA TCACCATCGT AGACGACAAT 240
CCCGGACTGG CTTTTGAGAA TCCGGCTCTG CTCGGATATG AATCCGGTGG CCGCGCCTTT 300
CTTTCCTATT TATATTATAT GAGTGGTTCG CATATGGGCA ATGCCTGTTA TGCCTCGTCC 360
GTCGGAGAGC GTGGCATGTG GGGTGTTGGC ATGCGTTTCC TGAACTACGG GTCTATGCAA 420
GGATACGATC AGAATGCGAT TGCCACCGGC TCTTTTAGTG CTTCGGATAT AGCTGTACAA 480
GGATTTTACA GCCATGAACT GAGCAACCAC TTCCGCGGTG GAGTCAGCCT AAAAGCATTG 540
TATTCTTCTA TCGAGACGTA TAGTTCCTTT GGCCTTGGTG TGGATGTCGG TATCAGTTAT 600
TACGACGATG ACAAAGGATA TTCCGCTTCC GCTCTGTTCA AGAACGTAGG GGCGCAACTG 660
AAAGGCTATA ATGAAGAACG GGAACCGCTC GATTGGGATT TCCAGCTCGG CTTTTCCCGC 720
AGTTTTATCA ATGCTCCGTT TCGCTTGCAC ATCACGTTGT TCAATCTGAA TCCGCACTAT 780
TTCAAGCGTC TTGTACCACG CGATCTGTCC AAGATGCAAA AGTTCCTCCG ACACTTCTCG 840
ATAGGAGCAG AATTTACTCC TTCCGAGAGG TTTTGGGTCG GGCTGGGATA TACGCCACAG 900
ATTGCACAGG ATTTCGAGGT GGAAGGCGGC AACAAATGGG GAGGTCTTTC GGCCGGCGTC 960
GGTTTCACTT CAGGTGTAGT ACGTGTAGGC GTATCTGCTG CCACCTATCA TCCTGCAGCT 1020
CTTTCGTTCA TGTGTTCGGT AGGTATCCGT TTGGACGATA AGAGCATCTT C 1071

(2) INFORMATION FOR SEQ ID NO:78

(i) SEQUENCE CHARACTERISTICS:
(A) LENGTH: 1011 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1011

(xi) SEQUENCE DESCRIPTION: SEQ ID NO:78

CCTCAGCCCG TCGGCCTTAA AGAAATAACC ATTAAACCCA TGTGCCTCGA ACCCATAATT 60
GCTCCGATTT CATCCGAGTT GCTCGAGCAG GAGCTGACTG CCGATCGTTT TCTGCGGATG 120
ACAAACAAAG CCGGCAATGA GATCTATGTT TTTACGGCCG AAGAAGCTCC GCATTGCATG 180
AAAGAAGTAG GCCGACTGCG AGAAGAAGCC TTTCGGCATT ATGGCGGAGG TACTGGCAAG 240

GCGATCGATA TAGACGAGTT CGACACCATG CCCGGGAGCT ACAAACAGCT GATCGTATGG 300
GATCCGCAAA ACAAGGCTAT ACTCGGAGGC TACCGCTTTA TCTATGGGCG GGACGTTGCT 360
TTCGATACCG ATGGCAAGCC TTTGCTGGCA ACGGCAGAGA TGTTTCGCTT CAGTGATGCT 420
TTTTTGCACG ATTATCTCCC CTACACAGTC GAATTGGGAC GTTCGTTCGT GTCGCTCCAG 480
TACCAATCGA CACGGATGGG CACAAAGGCC ATTTTTGTGC TGGACAATCT TTGGGACGGT 540
ATCGGAGCAC TCACTGTAGT CAATCCAGAG GCACTCTATT TCTATGGCAA GGTGACCATG 600
TACAAAGACT ATGATCGGCG AGCTCGCAAT CTGATCCTGT ATTTTCTTCG CAAGCACTTC 660
TCCGATCCGG AAGGCTTGGT CAAGCCTATT CATCCCCTAC CGATAGAGAT CAGTGCGGAG 720
GACGAAGCCT TGTTCTCCTC ATCCGACTTT GACACCAATT ACAAGACTCT CAATATAGAA 780
GTGCGCAAGC TGGGTATCAA TATCCCTCCT CTCGTGAGTG CATATATAGC TTTGTCTCCG 840
GAGATGCGTG TTTTCGGCAC TGCAGTGAAT GAGTCTTTCG GAGAGGTGGA GGAAACCGGC 900
ATATTCATTG CTGTGGGTAA GATCCTGGAA GAGAAAAAAC AACGGCACAT AGAGAGCTTC 960
ATCCTCAGCC GGAACGAAAA AAAAGGTCTC GACAGTAGCA ATGGCCGATC A 1011

(2) INFORMATION FOR SEQ ID NO:79

(i) SEQUENCE CHARACTERISTICS:
(A) LENGTH: 1698 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1698

(xi) SEQUENCE DESCRIPTION: SEQ ID NO:79

TACGACGGAG CGAGACTGGT CTATACCCTA TTTCGGAATA GAAACGACAT TCACCCTATG 60
AAAACCATTG TAAGATACAG CCGCCTTCCG GTCGCTCTCT TCTTTTGCCT TTTGGGAGCT 120
GTGCACTTGT CTGTCGAGGC GCAGATGCTC AATACTCCTT TCGAGCTGTC GGATCAGATC 180
GTCCTGTCTC CCACCGAAAG GCAATACAGG GAGATTTGTG TGCAAACGAA AGAAAAAAGG 240
GGGGCCGATC TTTTCCCGTT GAGCGATAAG CTGCGCGATT CGGCCTATGT TCGTTTCGGC 300
TCGGCCTATG GCGATATTGC GGGCGACTAT CTTCCGTACA ACGGCAATAA CTACTCCTCG 360
CTCTCGCTCG AATCGGGTGG TCGCATCAGT GTCCGTAACT ATGGCACATT GCAGGGCAGT 420
GCTTCCTACT CACGTGGCAT GCACAAACGC ATCGGCTGGA ATGCTCTGCG CAACGCCGAA 480
GCCTACTATC CCTATTTGGT GTCCGATTCG ACCGGCGGAG ACTATCATTT CGAAGACTAT 540
CGGCTTGCCG GCTACTATTC TTTTCGCGCC GGCCGCTTGC CCCTCGGTAT AGGCTTCTCA 600
TACAGGGGCG AAGTTGCTTA TCGGCTGACC GATCCGCGTA CGACCAATAC GACCGCTGCA 660
TTGGAGCTTT CTTGTGCTAC CTCTTTGACG CTGCCTCGAG AGAACAGGCT ATCGCTTTCG 720
GCTGCGTATC TCTATCATAG ACAACACCTC ACACAGTACA ACTGGCGTCC CGGGCAGCAG 780
GACAAATTCT TCGTCAGCTA CGGTTTCGGT CAGGTGGATG TCAGCAACAG CCCTATCTGG 840
TTCGGTATCT CCAGAATGAA CTACGTCAAC GGATGGAAGC TTAGCTCCCG TCTGGATACC 900
CGTAGGGGCG ATGCCATCGG TCTCGACTAC AGCGGCTACT TCCTCGATAC CGAaGAGAGG 960
TCGTCCATCA ATCTCTTTGC TTTGCTTTAC AATCGCCTGC GACTCTATGG TAGCTGGCAT 1020
CTGTCGGACT TCGATTTTTC ATTTTCAGCC GACTATGCTC TGCGCCAAGG GATAGAGCGG 1080
ATATACGAAG ACTACAAGCC GGATGATAAT TATCATATCT ACGACCTCCG TATCTTGGCC 1140
ATTCGCCGCT GGTATATGCT CAATGAGTTT TCTGCCCAAG CCCAAGCCTC CTACCGTATT 1200
CGCACGGATA GAGGTTGTGC CCTGAGAGTG AGTGCCGGTA GTGATTTCTA CGGCTATGAT 1260
GAGACGTATC GCAAGCATGG ACATCATACC ATGAGCGGAA TGCTACGTCC TTTTGCCGGT 1320
ATAGCCTATG ACCATGCCGG ATCCAAATTG GATTTTGGAC TTTCGCTTTC CGCTGCTTAT 1380
CGAATGGTGC TGACGCATTC GTATAAGATT CGTACCATCC AGAAAGAGCA GCTCGACTAT 1440
CAGCTGGCCT ATTTGCCCTA TGCCTATCGT AATAGAGAAG GCGTGGAGGT GCGTTCCTCT 1500
CTGTACGTCT CGATTCCGAT GCAGAATACC CACCGCCTGA TGACAGAGCT GCGGTTGTAT 1560
GGCGACCTGA TGAAAAGAAA GGACGGTATA GCCTATGGCA AAACGCCCGG TGTCATCTCA 1620
CATATCCTGT CCGATCCGCA AGCCGAACGA ACGTCCGGCC ATACCATCGG GGCTATCTGC 1680
AATATCTCCT ACCTCTTC 1698

(2) INFORMATION FOR SEQ ID NO:80

(i) SEQUENCE CHARACTERISTICS:
(A) LENGTH: 2457 base pairs
(B) TYPE: nucleic acid
(C) STRANDEDNESS: double

(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2457

(xi) SEQUENCE DESCRIPTION: SEQ ID NO:80

AGGACGAACG TTTTCTTATC TTTGTCCCAT AAAATTGGGA GAAGGGGTGC TTCCTGTAGC 60
AATCGGAATG CGTGGATGGC TGAGAACAAA CCCTCATCAC CTGAACCGGA TAATACCGGC 120
GTAGGAAACT CTCCGTCTGA CTATCTTCAC GGCGAAGCAA TCATTCCCCC TCTCTCTTCT 180
TTGTCCAACT TCAATGATAA GAGATTTATG AAAAAACTTC ACATGATTGC CGCCTTAGCC 240
GTCCTGCCTT TCTGCCTGAC GGCACAAGCA CCCGTCTCCA ACAGCGAGAT AGATAGTCTT 300
AGCAATGTGC AGCTCCAGAC CGTACAGGTC GTAGCTACTC GCGCCACGGC GAAAACCCCT 360
GTCGCTTACA CCAACGTTCG CAAGGCCGAA CTTTCCAAGT CCAATTATGG TCGTGACATC 420
CCCTATCTGC TGATGCTGAC TCCCTCCGTG GTAGCCACCA GCGATGCCGG TACGGGTATC 480
GGATATTCCG GCTTTCGCGT GCGTGGCACC GATGCCAATC GCATCAACAT AACTACCAAT 540
GGAGTACCCC TCAACGACTC CGAATCTCAG TCCGTCTTTT GGGTGAATAT GCCCGACTTC 600
GCCTCTTCCA TCGAAGACCT TCAGGTGCAG CGAGGTGTGG GTACTTCCAC CAATGGTGCC 660
GGAGCTTTTG GGGCAAGTGT CAATATGCGT ACGGATAATT TGGGACTGGC TCCTTATGGC 720
CGTGTCGATT TGAGCGGAGG TTCGTTCGGC ACATTCCGCC GATCGGTCAA ACTCGGTAGC 780
GGACGCATCG GTCGCCATTG GGCAGTGGAT GCCCGCCTGT CCAAAATCGG TTCGGACGGC 840
TACGTGGATA GAGGAAGCGT GGATCTGAAA TCCTATTTCG CACAGGTGGG CTATTTCGGT 900
AGCAACACGG CTCTCAGGTT CATCACTTTC GGAGGAAAAG AAGTTACGGG TATCGCATGG 960
AACGGTCTTT CCAAGGAGGA TGAAGCCAAA TATGGCCGCC GATACAACAG TGCCGGTCTT 1020
ATGTACGTGG ACGCGCAAGG AGTACCGCAC TACTACCACA ATACCGACAA TTACGAGCAG 1080
CGTCACTACC ATGCCATCAT GACGCACAGC TTCTCTCCTT CCGTTATCCT CAACCTCACG 1140
GCACACTACA CGGCCGGATA TGGCTATACG GACGAATATC GTACCGGACG TAAACTAAAG 1200
GAATATGCAC TGCAGCCCTA TGTGGAAAAC AGTGTGACCG TGAAGAAAAC GGATCTCATC 1260
CGTCAGAAGT ATCTGGACAA TGACTTCGGA GGACTCATCG GTTCGCTTAA CTGGCACACC 1320
GGTGCATGGG ATTTGCAGTT CGGGGCCTCG GGCAATATCT ATAAAGGAGA CCACTTCGGC 1380
CGTATCACTT ACATCAAAAA GTACAATCAG CCCTTAGCTC CCGACTTCGA ATATTATCGG 1440
AACAGGGCAG ACAAAAGAGA AGGTGCAGCC TTTGCCAAAG CCAACTGGCA GATCACTCCG 1500
GAACTGAACA TGTATGCCGA CCTCCAGTAT CGTACCATCG GCTACACGAT AAACGGCATC 1560
ACGGACGAAT ATGATGAGGT ACAGGGAAGT ATGCAGCACA TCGATTTGGA CAAGACCTTC 1620
CGCTTCCTCA ATCCGAAGGC CGGTCTTACC TATAGTTTCG ACGATGCTCA TACTGCCTAT 1680
GCTTCTGTTG CGGTAGCACA CCGCGAGCCT AACAGAACCA ATTACACCGA AGCCGGAATA 1740
GGACAGTATC CTACGCCTGA GCGACTGATC GACTATGAGC TGGGCTACCG CTATGCTTCG 1800
CCCCTCTTGT CGGCCGGAGT AGGTCTCTAT TATATGCAAT ACAAGGACCA ACTCGTGCTG 1860
GATGGCCGTT TGAGCGATGT GGGACAGATG CTCACAAGCA ACGTCCCCGA CAGCTACCGT 1920
ATGGGACTGG AGCTGACTCT CGGTTGGCAG ATCCTTCCTC GTTTGCTGCG TTGGGATGCT 1980
TCTTTCACTA TGAGTCGCAA CAAAATCGAC CGCTACGTAC AATATACATC CGTATATGAT 2040
GCGGACTACA ACTGGCTCGA ACTCAAGGAG GAGACCCTCG AAAGCACGGA TATAGCCTAC 2100
TCGCCCAATG TCATTGCCGG CAGCATGCTT ACCCTCTCTC ATGCCGGTTT CGAAATGGCT 2160
TGGACGAGCC GCTTCGTCAG CAAGCAATAT CTGGACAATA CACAGCGCAG CGATCGCATG 2220
CTTTCCTCCT ATTGGGTGAA CGACCTCCGC CTCGGCTATG TGCTGCCGGT TCACTTCGTT 2280
AAGAGAGTGG CACTGGGCGT ACAGCTCAAT AATCTCTTCA ACCTCATGTA TGCGTCCAAT 2340
GCCTACATCT ACGATGCCGG TTACGTACAG GCATCCGGAG AACTAAGTGC ATATGCCGAT 2400
CTGCGTTATT ATCCTCAGGC CGGATTTAAT GCACTGGGTA GTCTGACAAT CGATTTC 2457

(2) INFORMATION FOR SEQ ID NO:81

(i) SEQUENCE CHARACTERISTICS:
(A) LENGTH: 1596 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:

(A) ORGANISM: PORYPHYROMONAS GINGIVALIS

(ix) FEATURE:
(A) NAME/KEY: misc_feature
(B) LOCATION 1...1596

(xi) SEQUENCE DESCRIPTION: SEQ ID NO:81

TACAGGCGAT CGGGAAGGGT TTGTCCTCGT ATCCTCCGGA ACAAACGTTC CTACGCAGAT 60
ACTCGGTTAC AGCCGAGAAG AGCGGTTCGA CTACGAGCCG GCACCAGAAC AAAGATGAAA 120
AGAAGGTTTC TATCGCTGTT ACTGCTGTAC ATACTCTCTT CCATCAGCCT TTCTGCTCAG 180
CGGTTTCCGA TGGTGCAGGG AATCGAGTTG GATACCGATT CGCTTTTCTC TCTGCCCAAG 240
CGTCCTTGGC GCGCCATCGG TAAAACGATA GGCGTCAATC TGGCCGTATG GGGCTTCGAT 300
CATTTCATCA TGAACGAGGA CTTTGCAGAC ATCAGTTGGC AGACTATCAA GAGCAATTTC 360
CAAACAGGCT TTGGCTGGGA CAATGACAAG TTTGTCACCA ACCTCTTCGC ACATCCTTAT 420
CACGGATCGC TCTATTTCAA TGCAGCGAGG TCGAACGGTT TGAGCTTCAG GCACTCTGCT 480
CCGTTTGCCT TCTTTGGCAG TCTCATGTGG GAGCTGCTTA TGGAAAACGA GCCACCGAGT 540
ATCAACGACC TCTGTGCCAC CACCATAGGC GGTATAGCTT TGGGGGAGAT GGGGCACAGG 600
CTGTCGGACC TGCTCATCGA CAATCGTACC ACAGGGTGGG AACGTATGGG GCGCGAGGTG 660
GCTATCGCTC TGATCAATCC GATGCGCTTT CTCAACCGTC TGACAGCAGG AGAGGTGACT 720
TCTGTCGGGA GTCGCAGCGG ACAGATATTT CAGTCTGTCC CCATAAACAT AGTCGTCGAT 780
GCCGGCTTTC GCTTTTTGGC AGACAAGCGG CATGCCCGAA CCGGTGCCAC GGCTCTGACC 840
CTGAATCTGA GATTCGACTA CGGCGATCCA TTCCGAAGCG AGACTTTCTC TCCATACGAT 900
TTCTTCCAAT TCAAAGCCGG ATTGAGTTTC TCCGAATCGC AACCTCTGCT GAGCCAGATC 960
AATCTGATCG GAATCCTAAG CGGATGCCAA CTGCTCGCAC ACGAACGAAC GGTTTTGGTG 1020
GGAGGTCTCT TTCAGCACTT CGACTACTAC AATTCGGAAA AACGAATAAG CAAAAATTCG 1080
GAGGAGGTAC TCGTCACCCC ATACCGTATC TCGCAAGTGG CAGCTCTGGG AGGCGGTCTT 1140
ATCTTCCAGC ACCACGGAAA ATTTCGACGA CGTCCTCTGG AGCTATATGC CGAGACCTAC 1200
CTGAATGTCG TCCCGATGGG AGCCAGTCTG TCGGATCACT ACAACGTGGA CAATCGGGAC 1260
TATAACCTCG GCAGCGGATT GAGCGGCAAG CTATACCTTG GTGCTACGTA CAATGATCTG 1320
TGGAGCTGGC TCTTGGGAGT CGAAAGCTAT CGGCTCTACA CATGGATCGG GTATGAAGAG 1380
CCGCACCAGA AAAATACCGA TGTCAGCTCT TTTATGGTGC AGGGGGACGA AAGCAAGGCG 1440
CGCCTACTGG TGACGAGTTC CGAGTTCGCA TTTCATCCTG GCCCCTGGCA TGTAGCCATC 1500
GTCGCTCGCC GTTTCATCCG CAAAACAGCC TATCAATTCT ACCCTAACGT ATCATTCGAT 1560
ACCGGCGACA TACAGCTGCG TGTCGGATTT CACTTC 1596

(2) INFORMATION FOR SEQ ID NO:82

(i) SEQUENCE CHARACTERISTICS:
(A) LENGTH: 900 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...900

(xi) SEQUENCE DESCRIPTION: SEQ ID NO:82

AAAAGAAGAA AAAAACAGAT GAAACGACTG ATTGTTTTTC TGGCAATGGG TGGCTTGCTG 60
TTCACCCTTG CGAACGCACA AGAAGCAAAC ACTGCATCTG ACACTCCCAA AAAGGACTGG 120
ACTATAAAAG GTGTGACCGG ACTAAATGCC TCTCAGACTT CTCTGACCAA CTGGGCTGCC 180
GGTGGAGAAA ACACGGTGGC AGGTAACCTC TATTTGAACA TAGATGCCAA CTACCTGAAA 240
GATAAATGGA GTTGGGACAA CGGTTTGCGT ACAGACTTCG GTCTGACCTA CACAACAGCC 300
AACAAGTGGA ACAAAAGTGT AGACAAGATC GAACTCTTCA CGAAGGCCGG CTATGAGATC 360
GGCAAACATT GGTACGGAAG TGCGCTTTTC ACTTTCCTCT CACAGTATGC CAAAGGATAT 420
GAGAAGCCCT CGGATCACTT GACAGGAGTC AAGCATATCT CTAATTTCTT CGCTCCTGCA 480
TATCTCACTC TCGGTATTGG TGCGGACTAT AAGCCCAATG AGAAGTTCTC TCTCTACCTC 540
TCTCCTACAA CGGGCAAGCT GACTGTAGTA GCAGACGACT ACCTCTCAAG TTTGGGAGCC 600
TTCGGGGTGA AAGTTGGTGA AAAGACAATG TTCGAACTTG GTGCTTTGGT AGTGGGTTCG 660
GCCAATATAA ATCTGATGGA GAATGTCAAT TTGATAACCA AGGCTTCATT CTTCTCGGCT 720
TATACGCACG ACTTTGGCAA CATTGACATC AATTGGGAGG CTATGCTGGC CATGAAGATC 780
AACAAGTTCC TCACGGCTAC GATAGCCACC AATCTTATCT ACGACGATGA TGTGAAGATC 840
AACGATGGCC CGAAAATCCA GTTCAAAGAA GTTGTGGGCG TGGGTGTTGC GTACACTTTC 900

(2) INFORMATION FOR SEQ ID NO:83

(i) SEQUENCE CHARACTERISTICS:
(A) LENGTH: 663 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...663

(xi) SEQUENCE DESCRIPTION: SEQ ID NO:83

ACGAGAGAGA GTGTGTTACA TTGTAGAACA AAACTCAAAA AAGAACGAAA AATGAAGAAA 60
ATGATTTTGG CAGCTACTAT GCTGCTCGCA ACAATCGGTT TTGCAAATGC TCAGAGTCGT 120
CCTGCTCTTA GACTGGATGC TAACTTTGTC GGTAGTAACT TAATGCAAAA AGTCGCAAAC 180
ACGAGCGTGA ACAATAAGAT GATCGTAGGC TTACGTGTTG GTGCTGCTGC TGAGTTCGCT 240
CTTAGCAATG ATGGATTCTA TCTCGCCCCC GGATTGGCCT ATACGATGAG AGGTGCTAAG 300
ATGGAATCAC TAAGTGAAAC GACAACTCGC TTGCATTATC TGCAAATACC GGTGAATGCC 360
GGTATGAGAT TTAGCTTTGC TGACAACATG GCTATTTCAT TGGAAGCAGG TCCCTATTTC 420
GCATATGGTG TCGCCGGAAC GATTAAGACT AAAGTTGCAG GCGTTACGGC TTCTGTAGAT 480
GCCTTTGGTG ATAACGGATA TAACCGTTTC GACTTGGGCT TGGGCTTGTC TGCTGCCTTG 540
AGCTACGACC GTTATTACGT ACAAATTGGA TATGAGCATG GATTGCTTAA TATGTTGAAG 600
GATGCTCCGG ATAAGACTTC TTTGCGTAAT CATGACTTCT TTGTGGGTCT CGGTGTTCGC 660
TTC 663

(2) INFORMATION FOR SEQ ID NO:84

(i) SEQUENCE CHARACTERISTICS:
(A) LENGTH: 744 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...744

(xi) SEQUENCE DESCRIPTION: SEQ ID NO:84

ATCAAACGAA TAGAAATGAA AAGGATTTTT ACTGTAGCCC TTGTGCTACT TGCTTCGGTC 60
ACTATGGCCA TCGGACAAAG CCGCCCGGCA CTTCGCGTAG ATGCCAACTT CGTAGGCAGC 120
AATCAGAGCA TGAAAAGAGA CGGATATGTG TGGGACACCA AAATGAATGT CGGCCTGCGG 180
GTCGGTGCCG CTGCCGAATT CATGATCGGA TCAAGAGGAT TCTACTTGGC TCCGGGTCTG 240
AACTATACGA TGAAGGGCTC CAAAACCGAA TGGGATATAC CCGAAATGGT TCCTGGTACC 300
TATATTACGA TGGTTTCCAC TCGCTTGCAC TATCTGCAAC TGCCGATCAA TGCCGGCATG 360
CGGTTCGACC TGATGAATGA CATGGCGGTT TCGATCGAAG CGGGTCCTTT CCTTGCATAC 420
GGTATATATG GTACATATCG GCAGAAGTTG GAAGGATGGA AGCCGAACAA CTACAGCACA 480
GAGTTTTTTG GCCCAACGCT TGGTGGCCCA ACAAATATCC GCTGGGACAT CGGGGCAAAC 540
ATAATAGCCG CATTCCACTA TAAGCGTTAT TATATACAGA TAGGCTATGA ACATGGATTT 600
GTGGATATTG TGTCAGGTGG AGGTTCTGAT ATTCCCCGAC TGAACGACAA TAGGCAATCC 660
TCTTCGACGA CCGCTCTAAG AGAAAAGGGA AATAACGAAT ACGCTTATAA TCGTGACTTC 720
TTCGTGGGCA TAGGTTACCG CTTT 744

(2) INFORMATION FOR SEQ ID NO:85

(i) SEQUENCE CHARACTERISTICS:
(A) LENGTH: 633 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...633

(xi) SEQUENCE DESCRIPTION: SEQ ID NO:85

AAAAGAAAGA GTATGAAAAG AATGCTGCTG CTTCTCGTTG TATTATTATA TGGAATTGCA 60
GGCCGATTGG CTGCACAAGA CGTTATCAGA CCATGGTCAT TGCAGGTCGG AGCGGGATAC 120
TCCGATACGG AGAACATCCC GGGAGGATTC ACCTATGGTT TCTATTTGGG AAAGCGTATG 180
GGGAGCTTTC TGGAAGTGGG GCTGTCCATG TACAACTCCA CACGTCAAAC AGCCAACAAT 240
GCAGACTCCT TTGCATCGAA CGAAGGAGAC GGATCTTTTC AGGTAAATAT GTCTTCTCCG 300
AATGAGAAGT GGTCATTCTT CGATGCAGGC AGTGCCAACT GCTATATGAT CGTCGTCGGA 360
GTCAATCCTC TCCATCTGTT TTGGCAGAAT AGCCGGCACA ATTTGTTTCT GGCAGTACAA 420
GCCGGCCTGT CCAATAAGCA CAATATTCAT TTCATCTATG GAGACAAGGG AGCCAAAGTC 480
AGTATCTACA CCAATTCGAA TACCTACATC GGTTACGGAG CACGTGTAGC CTACGAATAT 540
CAAATTCATA AAAACGTGGG GGCGGGTGCC GCTGTAATGT ACGACCACGG CAATAAGATG 600
CTTACGGCCA TGGCCACGCT CTCCACTCAT TTT 633

(2) INFORMATION FOR SEQ ID NO:86

(i) SEQUENCE CHARACTERISTICS:
(A) LENGTH: 2859 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2859

(xi) SEQUENCE DESCRIPTION: SEQ ID NO:86

ATCCGAATGA GAGTATCCGA TCTCTGTTCC AGACTTTCAT GGTTATTACC CGTAATCCTT 60
GTCGGATTGC TCTGTGCTAC TTTGGTCGCT GCGGAACGTC CTATGGCCGG AGCAGTCGGA 120
TTGCACCACC GTCGGCATGC TGCGCTGTCT GATTCTACAG CGAAAGACAC GGTGCCTCTC 180
GCAAAACCTA TTCCTGACAG TGCTTTTCGA GATTCCCTTC CTGCCGATTC CACCGGATCG 240
ATGCGGCAAG ATAGCGTGTA TGACGATGAA TTCGAATTGG AAGATATAGT GGAGTACGAA 300
GCTGCCGATT CCATCGTTTT GCTCGGACAG AATCGTGCCT ATCTTTTCGG CAAGAGCTAT 360
GTGAGCTATC AAAAGAGTCG CTTGGAGGCA AACTTCATGT ATCTCAATAC CGACAGCAGT 420
ACGGTTTATA CTCGCTATGT CCTCGATACG GCCGGTTATC CGATGGCCTT TCCTGTTTTC 480
AAGGATGGAG AGCAGTCGTT CGAAGCCAAG AACTTTACCT ACAACTTCCG CACGGAGAAG 540
GGGATTATCA GCGGAGTGAT CACGCAGCAG GGCGAAGGCT ATCTGACTGC CGGTAAGACC 600
AAGAAGATGC CCGACAATAT CATGTTTATG CAAGGAGGGC GTTATACGAC CTGCGACAAT 660
CACGATCATC CTCACTTCTA TATCAATCTT TCCAAGGCAA AGGTGCATCC GGAGAAAGAC 720
ATCGTCACAG GTCCGGTCAA TCTGGTTATC GCCGATATGC CGCTGCCGAT AGGTCTTCCT 780
TTCGGCTATT TTCCCTTTTC CAACAAATAC TCTTCCGGTA TATTGATGCC CACGTACGGA 840

GAGGACAATC GCTATGGATT TTATTTGAGG AATGGTGGAT ATTATTTTGC CTTCAGCGAC 900
TATATCGATT TGGCATTGCG TGGGGAGATC TTTTCCAAAG GGTCATGGGG CATTTCAGCC 960
CAATCGAAAT ATAAGAAGAG GTATAAGTAC AACGGCTCGT TCGAAGCCAA TTATCTGGTA 1020
TCGAAGTCCG GCGACAAATA CGTGCCCGGA GACTACAGCA AGACCACCAG TCTGAATATC 1080
CGATGGACAC ACAGTCAGGA TCCGAAGGCC AATCCTTTGC AAACGTTGTC GGCCAATGTC 1140
AATTTTGCCA CCGGGAGCTA TTTCCAGAAT TCGCTGAATA CCACCTATGA TGTCAATGCC 1200
CGTACTGCTA CGACACGAAG TTCGGCCGTG AGCTATTCGC GCAAGTTTCC GGGTACTCCT 1260
TTTTCGATTA CGGGTAGCAT GGATATCAGC CAGAACATGC GCGATACGAC GGTGAGCCTT 1320
ACCTTGCCGA ATCTTTCGAT TAATATGTCC ACGCGTTATC CTTTCAAGCG GAAGACCCGT 1380
GTAGGACCGG AGCGATGGTA CGAGAAGTTG AGTGTGGGCT ATTCCGGTCA GCTTCGCAAT 1440
AGTATCTTGA CAAAAGAGAA AGATTTGCTC CAGAGCAATC TCGTGCGCGA TTGGAAGAAT 1500
GGTATGCGTC ATTCCGTACC GATCAGTTTG ACTGTCCCTT TGTTGGATTA TATCAATCTG 1560
ACTATGGGGG TTAACTACAA TGAGTGGTGG TACACGAAAG GCATACGGAA GTCGTGGAAT 1620
GAGGATAAGA AAACATTCCT GCCTTCGGAC ACGACCTATA AATTCCGCAG ACTGTACGAT 1680
TACAGTCTGT CGGCAGGCTT ATCTACCACA TTGTACGGTA TGTTCAAGCC TTGGAAACCT 1740
TTTTCCTTCG GAGGCAATCT CATTATGATC CGTCATCGCT TCACGCCCAC TGTCAGTTTC 1800
TCCTATATGC CGGACTTCAC GAAACGCCGA TATGGCTTTT GGGAGCTTCT TGAGCATACG 1860
GATCAGAACG GCAAGCTGCA TACGCTGCTC TACTCTCCTT ATTTCGAGCA GATATTCGGT 1920
GCTCCCTCCA TGGGCAATGC AGGATCTGTC AATTTCTCTT TTGACAACAA CTTAGAGGCC 1980
AAGATCAAAT CCAAATCGGA TTCGACAGGG ATCAAGAAGA TCAGCCTGAT AGATCAGTTC 2040
ACATGGTCTA CATCCTATAA TATGTTTGCC GATTCGATCC GATGGAGCAA TATCTCGGCT 2100
TCGCTGGCAC TTCGCCTCTC CAAGAGCTTT ACCTTGCGCT TGTCCGGTCT GTTCGATCCC 2160
TATTTGACGA AGTATTATGA GGGAGAAGAT GGGAAGATCA TTCCCTATAA GAGCAACGAC 2220
CTGCGCATTT TTAACGGCAA GGGATTGGCA CGCCTGATCA GTACGGGTAC TTCTTTCAGC 2280
TATACGCTCA ACAAAGAGTC GCTCAGCGGA TTGATAGCTC TTTTCAGTGG CAAAAAGGAG 2340
CGGAGAGATG AAAAGAAAAA CACAGGGGCT ACTCCTCATG AAGGAGACGA TGCTGCCGAT 2400
ATACTTGAGG GAGGAAGACC GCAAAATGAA AGTGGGGGGT CGCTCCTCGA GCGCAACCGT 2460
CAGGGCGGAG CAGTGGATCA GGATGGTTAC TTCGCATATT CGATCCCATG GAGCCTGTCC 2520
TTCGACTATA GTTGGAATAT TGCTACCGAC TACAATAGGT ACAATGTCAA TAAGATGGAG 2580
CACTACTACC GGGTAACGCA GAATCTGAGC TTTCGCGGCA ATATCCAGCC TACACCGAAC 2640
TGGAGCTTCG GATTCAATGC GAACTACAAT TTCGACTTGA AGAAAATAAC ATCGCTTACC 2700
TGCAACGTCA CTCGCGACAT GCACTGCTGG GCTATCTCGG CCAGTTTCAT CCCTATAGGA 2760
GCATACAAGT CCTATAATTT CGTCATATCG GTGAAGAGTT CACTCTTGCA GGATCTGAAG 2820
TATCAGCAGA GCAATCGTCC CATCACGAAT ACTTGGTAT 2859

(2) INFORMATION FOR SEQ ID NO:87

(i) SEQUENCE CHARACTERISTICS:
(A) LENGTH: 3753 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...3753

(xi) SEQUENCE DESCRIPTION: SEQ ID NO:87

AAATGGAAAT TGGCATGCGC TTTTGATTGC GCGTGTTGTT TCGATCCATT TGTAGTAACT 60
AACGAGGTAA TAATAATGAT GAAACGATAT ACAATAATTC TTGCAGTTTT TCTTTTATTC 120
TGCACGGTAT TTACCTTTCA AATAAAAGCT CGCCCTTATG AAAGATTTGC AGATGTAGAG 180
AAGCCTTGGA TTCAGAAACA TTCAATGGAT TCTAAATTGG TGCCTGCAAA TAAGGGTAAC 240
TTAATTCAAG CTGAAATTGT ATACCAATCT GTTTCTGAAC ATAGTGACTT AGTTATTTCA 300
CCTGTGAACG AAATAAGGCC TGCAAATCGT TTCCCTTCGC ATAGGAAGTC TTTTTTTGCA 360
GAAAATCTAC GGGCATCTCC CCCCGTAGTT CCCGTTGCCG TCGACAAGTA TGCGGTACCG 420
GTTGCCAATC CAATGGATCC TGAAAATCCC AATGCCTGGG ATGTGACGCT AAAAATCACT 480
ACTAAAGCGG TAACAGTACC TGTCGATGTG GTGATGGTTA TCGACCAGTC TTCGTCAATG 540
GGAGGGCAAA ACATTGCCAG ATTAAAGTCT GCCATTGCAT CGGGACAGCG TTTTGTGAAA 600
AAAATGTTGC CTAAGGGGAC GGCTACAGAA GGGGTGCGTA TCGCTCTTGT GAGTTATGAC 660
CATGAGCCTC ATCGCTTATC TGATTTTACC AAAGACACTG CTTTTCTCTG TCAAAAAATC 720
CGGGCTTTGA CTCCTATTTG GGGAACACAT ACCCAGGGGG GGCTTAAAAT GGCGAGAAAC 780
ATTATGGCCA CTTCTACTGC TGTGGATAAG CATATCATAT TGATGTCTGA CGGGTTAGCG 840
ACGGAGCAGT ATCCTGTTAA AAATGTAACT ACTGCAGACT TCATTGGCAA AACTGGAAAT 900
GCGAATGATC CCATTGATTT GGTTATACAA GGAGCAATTA ATTTCCCTAC AAATTATGTT 960

TCCAACAATC CATCTACACC TCTTACCCCA AATTATCCAA CTCATTCTTC TAAAGTTGGA 1020
CGGAGAAATC TGCCGGAATC CAAATTCGAT TATAGTAATC TGAGTGCAAG GATTACTTTT 1080
GATGGTGTTG CTGGCGCATT GGTCTATGAA CCGAGGTTTC CTCATCCCTA TTATTATTAT 1140
TTCCCTTGTA ACGCTGCTAT CAATGAGGCT CAGTTTGCGA AAAACTCTGG TTATACAATC 1200
CATACTATTG GCTATGACCT GGGAGATTTT GCCTTGGCCA ACAATTCGTT GAAACTAACC 1260
GCTACAGACG AGAATCACTT CTTTACGGCG ACACCGGCCA ATTTAGCTGC AGCGTTTGAT 1320
AATATTGCCC AAACTATTAA TATAGGTATA CAGAGGGGGG AGGTGACGGA CTTTGTAGCT 1380
CCTGGTTTCA TCGTTAAAAA TCTGACGCAA TCGGGAGATG TTACTCATTT GCTAAATGTT 1440
TCAAATGGAA CGGTGCACTA TGATGTCTCT ACTAAAAAAC TGACATGGAC TACTGGTACT 1500
ATCCTGAGCT CATCAGAAGC TACCATAACT TATCGTATTT ATGCCGATTT GGATTATATA 1560
CAGAACAATG ATATTCCGGT AAATACTACT TCTGCTATCG GCCCGGATCT TGGTGGATTC 1620
GATACCAATA CCGAGGCAAA ATTGACCTAT ACCAATTCCA ATGGCGAACC GAATCAGCAG 1680
TTAATTTTCC CACGTCCGAC GGTTAAGTTA GGTTATGGTG TTATTAAGCG GCACTATGTA 1740
TTGGTAAATA AAGACGGTCA ACCCATACAG GCAAATGGAA CAGTTGTCAG TTCCCTAAGC 1800
GAGGCTCATG TTCTACAGTC ACAAGATTTC TTTTTGCCCT CAGGTGGAGG TCATATTGTT 1860
CCCAAATGGA TAAAGTTGGA CAAAACGACC GAAGCATTAC AGTACTATTC CGTACCGCCG 1920
ACTAACACGG TCATCACTAC TGCCGATGGT AAACGTTATC GTTTTGTCGA AGTCCCAGGC 1980
TCCACGCCGA ATCCGGGCCA AATCGGTATC AGTTGGAAAA AACCGGCAGG AAACGCTTAC 2040
TTCGCTTACA AGCTCCTCAA TTATTGGATG GGAGGAACAA CAGACCAACA GAGTGAATGG 2100
GATGTGACGT CCAATTGGAC AGGAGCCCAA GTACCGCTCA CAGGAGAAGA TGTAGAGTTT 2160
GCAACGACAG AAAATTTCGG TTCTCCGGCG GTAGCCGATT TGCATGTCCC GACAACCAAC 2220
CCCAAAATTA TCGGTAACCT TATCAATAAT TCCGACAAGG ATTTAGTTGT TACCACAAGC 2280
AGTCAATTGA CGATCAACGG CGTGGTTGAG GATAACAATC CGAATGTCGG TACGATCGTC 2340
GTGAAGTCGT CGAAAGACAA TCCTACGGGG ACATTGCTTT TTGCCAATCC GGGCTATAAT 2400
CAAAATGTAG GGGGGACCGT CGAGTTTTAC AATCAGGCAT ATGATTGTGC CGATTGTGGT 2460
ATGTATCGCA GGAGCTGGCA GTATTTCGGT ATCCCTGTCA ATGAATCAGG TTTTCCAATT 2520
AATGATGTGG GCGGAAACGA GACCGTCAAC CAATGGGTTG AGCCTTTCAA TGGCGATAAG 2580
TGGCGGCCAG CACCTTATGC ACCTGATACA GAGCTTCAAA AATTCAAGGG CTACCAGATC 2640
ACGAATGACG TGCAGGCACA GCCTACGGCA GTTTACAGCT TCAAGGGTAT GATTTGTGTG 2700
TGCGATGCCT TCCTGAATCT GACACGCACG TCCGGTGTCA ACTACTCGGG CGCCAACTTG 2760
ATCGGCAACT CATACACTGG AGCCATCGAC ATCAAGCAGG GTATTGTCTT CCCGCCGGAA 2820
GTCGAGCAGA CGGTGTATCT GTTCAACACG GGAACACGCG ACCAGTGGCG TAAGCTTAAT 2880
GGAAGCACGG TTTCAGGCTA TCGAGCCGGT CAGTACCTCT CTGTACCTAA GAATACAGCG 2940
GGTCAGGACA ATCTTCCGGA TCGTATTCCA TCGATGCATT CCTTCTTGGT GAAGATGCAG 3000
AACGGAGCGT CTTGTACGTT GCANATCTTG TACGATAAGC TGCTCAAGAA CACGACTGTA 3060
AACAACGGTA ATGGTACGCA GATCACATGG CGATCCGGCA ACTCCGGATC GGCGAATATG 3120
CCGTCACTTG TGATGGATGT TCTTGGTAAC GAGTCGGCCG ACCGTTTGTG GATCTTTACC 3180
GATGGGGGTC TTTCTTTCGG ATTCGACAAC GGCTGGGATG GTCGCAAGCT GACTGAAAAA 3240
GGTTTGTCAC AACTTTATGC GATGTCTGAC ATCGGTAATG ATAAATTCCA GGTTGCAGGG 3300
GTTCCGGAGT TGAATAACCT GCTGATCGGC TTCGATGCGG ATAAGGATGG TCAATACACG 3360
TTGGAGTTTG CTCTTTCGGA TCATTTTGCG AAAGGGGCTG TTTACCTGCA CGATCTTCAG 3420
TCAGGAGCCA AACACCGTAT TACGAATTCT ACGTCGTATT CATTCGATGC CAAGCGGGGA 3480
GATTCCGGGG CTCGTTTCCG CTTGTCATAT GGATGTGATG AGAACGTAGA TGATTCGCAT 3540
GTCGTGAGTA CAAATGGCCG TGAAATTATA ATTCTGAATC AAGATGCTCT TGACTGCACT 3600
GTAACCTTAT TCACAATAGA AGGTAAGCTT CTTCGCCGCT TGAAAGTATT AGCTGGTCAT 3660
AGAGAAGTCA TGAAAGTGCA GACCGGAGGG GCCTATATTG TGCATCTTCA AAATGCTTTC 3720
ACTAATGATG TGCATAAGGT GCTTGTTGAG TAT 3753

(2) INFORMATION FOR SEQ ID NO:88

(i) SEQUENCE CHARACTERISTICS:
(A) LENGTH: 1278 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1278

(xi) SEQUENCE DESCRIPTION: SEQ ID NO:88

ACTATGGAAG TGAAGAAAAA CACAGTGGTG CTACGCCTTC TGATTTGGTT CGTGGCCATT 60
CTTCTCTTCC ACTCCTCACG GCTGTGGGGA CAGGAAGGGG AGGGGAGTGC CCGATACAGA 120
TTCAAAGGAT TCGTGGATAC CTACCATGCC GTACGCAGCT CTTCTCCTTT TGATTTCATG 180

AGCTCGCGTA CGAGAGTGAG AGGTGAGCTG GAGAGGTCGT TCGGTAATTC GAAAGTAGCC 240
GTATCGGTCA ATGCCACCTA CAATGCTCTA CTGAAAGACG AGACCGGCTT ACGTTTACGT 300
GAAGCCTTCT TCGAGCATCA GGAAGAGCAT TGGGGCTTGC GCCTCGGACG ACAGATTGTC 360
ATTTGGGGGG CTGCCGACGG TGTGCGCATC ACGGATCTGA TCTCCCCGAT GGATATGACC 420
GAGTTTCTGG CACAGGATTA CGATGATATT CGTATGCCGG TCAATGCATT GCGTTTCTCT 480
GTCTTCAACG AATCGATGAA AGTGGAAGTC GTGGTACTGC CTGTATTCGA GGGGTACCGT 540
CTGCCTGTGG ATCCTCGCAA TCCTTGGAAT ATCTTCTCCC TTTCGCCCAT TGCTCAGGGG 600
ATGAATATCG TCTGGAAAGA AGAAGCCGGC AAACCGGCCT TCAAGGTTGC CAATATCGAG 660
TACGGTGCGC GATGGAGCAC TACGCTCTCC GGTATCGACT TCGCTTTGGC TGCATTGCAT 720
ACATGGAACA AGATGCCCGT CATCGAAGTA CAGGGCATTG TGCCGACGGA AATCATCGTT 780
AGCCCTCGCT ATTATCGTAT GGGATTTGTC GGCGGCGACC TCTCCGTACC CGTCGGACAG 840
TTTGTTTTCA GGGGAGAGGC TGCGTTCAAT ATCGACAAAC ACTTCACCTA TAAGAGTCAT 900
GCCGAGCAAG AGGGTTTCCA AACAATCAAT TGGTTGGCCG GAGCCGATTG GTATGCTCCC 960
GGTGAATGGA TGATCTCAGG ACAATTCTCA ATGGAAAGCA TATTCAGGTA TAGGGATTTC 1020
ATCTCCCAAA GACAACATTC TACCCTGATT ACTCTCAATG TTTCCAAGAA ATTCTTCGGC 1080
AGTACACTCC AACTTTCGGA CTTCACCTAC TACGACCTTA CGGGCAAAGG ATGGTTCAGT 1140
CGCTTTGCAG CTGACTATGC CTTGAACGAT CAGATACATC TGATGGCCGG ATATGACTGG 1200
TTCAGTAGTA AGGGCAGCGG TATATTCGAT CGCTACAAAG ACAATTCCGA ACTCTGGTTC 1260
AAAGCCCGCT ACAGCTTC 1278

(2) INFORMATION FOR SEQ ID NO:89

(i) SEQUENCE CHARACTERISTICS:
(A) LENGTH: 1392 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1392

(xi) SEQUENCE DESCRIPTION: SEQ ID NO:89

TACGGCAAAA GAAGAAAGCT CGGAACCTCC GTCCGACCTT CCGTTCTAAC CCAAATCAGA 60
TTTATTTTGG ACTTACACCT TATTACTGAT TTTTTTGAAG GGCTTCGGGT CAATCCTATC 120
GGTGCAGCAG CCATAGTGGC TTTCATTATC GACCTGCTTC TTCTTTGCTG TTCGGCTTTT 180
ATGTCCTCCT GTGAGGTGGC TTATTTTTCA CTAAAGCCGA TCGATCTGCA GAACATCCGC 240
GAACGGAATC ACTCTTCCGA CATCGCGCTT TCCAATTTAT TAGACAATTC GAATCAGCTA 300
TTAGCTACTA TTCTGATCGG GAATAATGTG ATTAATGTAG CCATCGTTAT CCTTTCCAAT 360
TATGCCATCG AGCAGACATT CGTTTTCTCT TCTCCGATCA TTGGATTTCT GATCCAGACG 420
ATACTCCTGA CCACTGTTCT TTTGCTGTTC GGAGAGATTC TGCCGAAAGT GTATGCGCGG 480
AAGAATCCGC TGCAATACTC GCGCTTTTCT GCTGCAGCTA TGTCCGTTAT CTATAAGATA 540
TTGTCACCGT TTTCAAAATT GCTGGTCAAA AGTACCGGCA TCGTTACCAG AGGTATCAGC 600
AAGAAGAAAT ACGATATGTC CGTGGATGAG CTCTCGAAAG CGGTAGCCCT CACCACTACG 660
GAGGGAGAGC CGGAGGAGAA AGAAATGATT AACGAAATCA TCAAATTCTA TAATAAGACA 720
GCCTGCGAAA TCATGGTTCC GCGTATCGAT ATTGTGGATG TGGATCTGAG CTGGCCATTT 780
CGTAAGATGC TTGACTTCGT TGTTTCGTCG GGTTATTCCA GACTTCCCGT TTCAGAGGGG 840
TCAGAAGACA ATATCAAAGG GGTGATTTAC ATCAAAGATC TAATCCCACA CATGGATAAA 900
GGCGATGAAT TCGACTGGCA TCCTCTGATT CGTAAAGCAT ATTTTGTCCC CGAAAACAAG 960
CGCATAGATG ATTTGCTCGA GGAGTTCAGA GCCAATAAGG TGCATGTCTC CATCGTTGTG 1020
GATGAGTTCG GTGGCACTTG CGGACTGATC ACAATGGAGG ACATATTGGA AGAGATCGTC 1080
GGCGAGATTA CGGACGAGTA CGATGAGGAA GAACTCCCCT TTAAGGTTTT GGGGGATGGC 1140
AGTTATCTTT TCGAAGGAAA AACGTCTCTC TCCGATGTTC GACACTATCT TGACCTTCCG 1200
GAAAATGCTT TCGGTGAATT GGGGGACGAG GTAGATACGC TAAGTGGGCT CTTCTTGGAA 1260
ATCAAGCAGG AACTCCCCCA TGTGGGCGAT ACAGCAGTGT ACGAGCCATT CCGCTTTCAA 1320
GTGACCCAAA TGGACAAGCG CCGAATCATC GAAATCAAGA TTTTCCCTTT CGAGCGCACT 1380
TGGGAGGTCG AA 1392

(2) INFORMATION FOR SEQ ID NO:90

(i) SEQUENCE CHARACTERISTICS:
(A) LENGTH: 798 base pairs
(B) TYPE: nucleic acid

(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...798

(xi) SEQUENCE DESCRIPTION: SEQ ID NO:90

ATAATTTATA GATCAACAAT GAAACTATTA CTTTATCTCC TATTGGTCTT GTCGACTCTA 60
TCCCCGATGT ATTCGCAAAT GCTCTTCTCA GAGAATCTCA CAATGAATAT AGACAGCACC 120
AAAACCATAC AAGGAACGAT ATTGCCCGTA CTGGATTTCA AAACCGAAAA GGAAAATGTG 180
TTCACCTTCA AAAATACTGC CAATCTCAAT CTGCTGATAA AGCACGGTCA AGTAATCAAC 240
TTAATTAATA AGCTTGAGTT TTCTACCTAT GGCAATAAAG TAACCGTAAG TGGAGGATAT 300
GTACACACCG AATACCGCTA TTTGTTGCAT CATGTTTTTG AGGTTTATCC TTATGTCGAG 360
TCGCAATGGG CAGAAAGTAG AGGAATGAAA TATAAGGTTT CTACGGGATT ACAGTCGCGT 420
TATCGGCTGG TAAATAGTGA TAACTGTCTC ATGTTTGCAA CATTGGGGGT ATTTTTCGAA 480
TTCGAAAAGT GGGAACAGCC AGCCACTAGC CTCTTTGCAG GAACGTATGC ATACAGCCGA 540
AGTATCAAAA GCCACCTGTC TATCAGTTTC AGACATCGGT TGGGTGAACA TTGGGAATTT 600
ACAACTACGG CTATTCACCA GGGAAAGCCT GACAGTTATT TTAAGAAGGC ACGTTTTGGA 660
GGAGCTATCG ACCTCAAATA CCATATCACA CCTACGATAG GAATACGCGG GGCCTATCGG 720
ATCATCTACG ATACTGCCCC TATTGTACCT GTGCGGAAAG ATTACAACAC CGTTGATGTT 780
GGTATCGATA TTTCGTTT 798

(2) INFORMATION FOR SEQ ID NO:91

(i) SEQUENCE CHARACTERISTICS:
(A) LENGTH: 2721 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2721

(xi) SEQUENCE DESCRIPTION: SEQ ID NO:91

ACTATATGCG TTGCCTTCTT ATCCGCTCCC GTTGCGGCAT TGCGCGAGTC GCCGCCAATG 60
GGGGCGGAGC GGAAGACTCC CTCTCTCCTA CCCCTTCTTT TCGAGCATTC TGATAAAGGA 120
AAGGGGTTCG ACTACAGACT ATTCACAAGC AATAAACTTA AAGTGTTTGC CACAGGTAAC 180
AGCCGATACA TACACAATAA ACCAACAATC ATCCAAGCAA TGAAACGAAT CGTTTTATCA 240
TCTTTCCTGT TCGTTCTGTC CATACTTTCT TTGATGGCAC AGAACAATAC CCTCGATGTA 300
CACATATCCG GTACGATCAA GGATGCCTCC TCCGGCGAAC CAGTGCCCTA TGCCACTGTA 360
AGCATCCGGC TGACAGGAGC AGATACCACA CAGGTGTTCC GACAAGTGAC TGACGGCAAC 420
GGCTACTTCG TCATAGGCCT GCCGGCAGCT CCCTCCTATC ACCTGACAGC TTCGTTCGTA 480
GGTATGAAAA CCCATACCAT GCAGATTAGT CGGGGAAATG GACAGCACGA CATCAAATCC 540
ATCGACATTT CTCTCGAATC CGAGGACAAA CAACTCTCCA CCGTCACCGT ATCGGCAGCA 600
CGACCACTGG TGAAGATGGA GATAGACCGC CTGTCCTATA ATATGAAAGA TGACCCCGCA 660
GCCAAGACGA ACAACCTGCT CGAAATGCTG CGCAACGTTC CTTTGGTAAC GGTGGATGGT 720
CAGGGCAATA TCCAGGTGAA AGGATCTTCC AACTTCAAAA TCCACCTCAA TGGCAGGCCC 780
TCGACCATGG TGAGCAGCAA CCCGAAGGAG GTCTTTCGCT CCATTCCTGC CCATACGATC 840
AAACGGGTGG AGGTCATCAC CGATCCGGGT GTAAAGTACG ATGCGGAAGG CACAAGTGCC 900
ATCCTGGACA TCGTCACGGA AGAAGGTAAG AAGCTGGAAG GATATTCAGG TTCCATCACG 960
GCCAGTGTCA GCAACAATCC CACAGCCAAC GGTAGTATCT TTCTGACGGC AAAGTCCGGC 1020
AAAGTCGGGC TGACTACCAA CTATAACTAC TACGGTGGCA AAAACAAGGG CTCTCGCTAC 1080

TTTACCGAAC GTACTACATC CATGCTCCAA ACGATAGAAG AAGGCAAAGG GCAAGAAACC 1140
TTTGGCGGAC ACTTCGGCAA TGCCCTCCTC TCATTCGAGA TAGATTCGCT CAATCTCTTT 1200
ACGGTGGGCG GCAATGTACG CCTTTGGGAG ATGACCACCG ACCGGAACAG CGTAGAAAAA 1260
AGCTTTGCCG GCAGCAACCT CATGTCCTAC ATAGACAGAA AACTCAAAAC ACAGATGGAT 1320
GCCGGATCAT ACGAGCTCAA TGCCGACTAT CAGCACAGCA CTCGCCTGCC GGGCGAATTG 1380
CTCACCGTTT CCTACCGCTT CACTCACAAT CCTAATAATA GCGAGACCTT CATTGACCAA 1440
TGGAAGCGCG ATCCGCTCAA CACAGCTAAT ACGATCCAGT ACGCCGGCCA GCACTCCAAA 1500
TCCGATGCGG GCATGGACGA ACATACGGCA CAAGTGGACT ATACACGTCC CTTAGGACAA 1560
GCACATTCTT TGGAAGCAGG GCTGAAGTAC ATCTATCGTC ATGCCACGAG CGATCCTCTC 1620
TATGAGATAC GACCATCCGA AGATGCTCCG TGGCAGCCCG GCTCTCTATA TGCACAGAAT 1680
CCGTCGAACG GAAAGTTCCG CCACGATCAA TACATCGGAG CAGCCTATGC CGGCTACAAC 1740
TATCGTAAGG ATCAGTATTC TTTGCAAACC GGCCTCCGAG TGGAAAGCAG CAGGCTGAAA 1800
GCACTCTTTC CCGAAAACGC AGCAGCAGAT TTCTCCCACA ACTCGTTCGA CTGGGTGCCA 1860
CAGCTCACGC TCGGCTATAC CCCCTCGCCC ATGAAGCAGC TTAAGCTGGC CTATAACTTC 1920
CGAATCCAAC GTCCTGCAAT CGGCCAACTG AATCCCTACC GGCTACAGAC CAACGATTAT 1980
CAAGTACAGT ATGGTAATCC CGACCTAAAG TCGGAGAAGC GTCACCACGT CGGTCTCTCC 2040
TATAATCAAT ACGGAGCCAA GGTCATGCTT ACAGCATCGC TCGACTACGA CTTCTGCAAC 2100
AACGCCATCC AGAATTACAC CTTCTCCGAC CCGGCCAATC CCAATCTGTT CCACCAGACC 2160
TATGGCAATA TCGGACGAGA GCATTCTTTC AGCTTGAATA CCTATGCCAT GTACACGCCG 2220
GCCGTATGGG TCAGGATTAT GCTCAACGGA AATATCGATC GCACATTCCA AAAGAGCGAA 2280
GCACTCGGCA TTGATGTCAA TTCATGGTCC GGCATGGTAT ACTCAGGCCT GATGTTCACC 2340
CTGCCGAAGG ATTGGACTGT GAATCTCTTC GGAGGTTATT ATCATGGGGG AAGAAGCTAC 2400
CAGACGAAGT ATGATGGCAA TGTATTCAAC AATATCGGTA TAGCCAAACA GCTTTTCGAC 2460
AAAAAATTGA GAGTCTCGCT GAGCGCAAAC AACATTCATG CGAAGTATTC GACATGGAAG 2520
AGCCGGACCA TCGGCAATGG ATTTACTATT TATTCGGAAA ATGCCGGTAT ACAACGGAGT 2580
GTTTCCCTCA GCCTCACCTA CAGCTTCGGT AAGATGAATA CACAAGTGCG CAAGGTAGAG 2640
CGTACGATCG TCAACGACGA CCTCAAGCAA ACCTCATCCC AAGGACAGCA GGGTGGCGGA 2700
CAAGGAAATC CTACCGGCAA T 2721

(2) INFORMATION FOR SEQ ID NO:92

(i) SEQUENCE CHARACTERISTICS:
(A) LENGTH: 1350 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1350

(xi) SEQUENCE DESCRIPTION: SEQ ID NO:92

TGGTGCCAAT CCGACCCAAT ACCTCATTGC CATGCAGTAT ATCGAAACGC TCAAGGACAT 60
CAACAAGGGC GATCAGACCA AGACCGTTTA CCTCCCGTTC GAGGCTACCG GTATGCTCGG 120
TGCCTTGGGC GGTATGAAGG ATTTGGTGAA AGGATGAGAC TCTCTGCCAT TCTTATCGCT 180
TTGATTGTGA TGCTGCCTGC TGTGCTTAGC GGGCAGCATT ATTATTCCAT GGCGGGAGAG 240
CGACTGGAGA CGGACAGCAT TCGTCCGAAC GAACTCTCGG CATCGATCCG AAGTGCGCTT 300
TTCTTTCGGA ACAATGAATA CAATGCACGT TCGGTCAAAG GTTATACGTT GCCGGGTGCA 360
CGGGTTTCCG CTTTTGCCTC TTACTCGCTG CCGGCAGCAC ATGGTGTGAA GCTTTCGCTC 420
GGAGTATCTA CCCTGAACTA CTGGGGGGCA AGTCGCTATC CGGCCGGTAT CGCTTATTCC 480
GATTTACCTT ATTGGACGGA CTATAACGAC TATGTACGCT TGCGTATCCT GCCTTATGTA 540
CAGGCCATGC TGAAGCCGAC GGCCACGACT GCTCTCATGC TGGGCAATAT AGCCGGTGCT 600
ACGGCTCACG GACTGATCGA ACCGATCTAC AATCCTGAGT TGGATTTGAC GGCTGATCCT 660
GAAGCCGGTG TGCAATTTCG GGGTGATTGG ACACGTTTCC GAATGGATGT TTGGGTCAAT 720
TGGATGAGCA TGATTTTCAA AAATGACAAT CATCAGGAGT CGTTTGTCTT TGGCTTGTCC 780
ACTACTTCGA AATTGTTATC GGGTGAAGGC AAATGGCGAC TCGAACTGCC CTTGCAGGCT 840
ATTGCCACGC ATCGCGGCGG GGAATACAAC TGGGCGCAGC AGGATACCGT GCATACATGG 900
GTCAATGGAG CTGTCGGACT TAAGCTTTCG TATCGCCCTC GTACCGACAA ACCCATGCAG 960
ATTTGGGGAT CTGCTTATGG TGTGGCAGCC TTGTCAAGCG GAGGATACTT CCCTTACGAA 1020
AGAGGGTGGG GCGGTTATCT TTCTCTCGGA ATGGACTTGG AGCACTTCGC TTTTCGTACC 1080
GACTATTGGT ACGGCAGGCA TTACGTTTCT CCCTTTGCTG CACCTTTCGC CAATTCCCTG 1140
ACGTATGACA AACAGCCTCT TACGAACGGT TGGGGCGATT ATATTCGTCT CTATGCCGAC 1200
TATTCGTGGC GGATGGCACG AAGTGTTTCG TTGGCGGCTG TTGCTCGGGT ATGGTTCCAG 1260
CCTTCGGATC GTTTTGCGAT GAGCCACGCC TTGGAACTGA CGATGCGTAT CGATCCCAAA 1320

TTCCCAATAG CTTTTCTGAA AGGCAATCAT 1350

(2) INFORMATION FOR SEQ ID NO:93

(i) SEQUENCE CHARACTERISTICS:
(A) LENGTH: 1341 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1341

(xi) SEQUENCE DESCRIPTION: SEQ ID NO:93

CCGTTTTCAC CGCCATGGGT ATGTGCTGGA GCGGCTTCCT CAGTACGCAC ACGGCCATGC 60
TCGACAGTCT CAAGTACCGC CGCCTCATCA CACAGGCGAT CGGAGCACAT GCGATCGGCG 120
GACTCGTCGC CGGCATATTC GCCCATTGGC TCTTTGTCCT CATCTCACTC ATTTGATTCG 180
ATACCCGACC AACGTATGAA CAAATCGCTA TTATCATTGG CATGCCTCAT CCTGTGCGGT 240
ATGCCGGCCA TCGCCCAACA GACAGGACCG GCCGAACGCA GCGGCGAGCC TTCTCTGGCC 300
GAACGTGTAT TCGGTCTGGA GCAGAAGCAG AAAAAGCTGA AGGTGTACTT AGGCATACAG 360
TCGTTCTACG ACCAGCCGCT TGTCGATGAC GAATCCCATA TCGGACACTT CAAGGTACAG 420
GAGCTGCGGA TGTCTGCTCA TGGCGAACTG AACCGCCACC TCAGCTTCGA CTGGCGACAA 480
CGTCTCAACC GTGCCGCCGA CGGCACTTCG TTTGCCGACA ATCTCTCCAA TGCCATCGAC 540
ATCGCAGGTG TGGACTGGCA CCCGAACGAC AAGGTGTCTT TCTTCTTCGG ACGTCAGTAC 600
GCGCGTTTCG GAGGGATAGA ATACGACATG AACCCCGTAG AGATCTACCA GTACAGCGAC 660
CTTGTGGATT ACATGACCTG CTATACTTCG GGCGTGAACT TCGCATGGAA CTTCCACCCC 720
GAACAGCAGC TGCAGCTACA GGTACTCAAT GCTTACAACA ACCGCTTCGC CGACCGCTAC 780
CACGTGACAC CCGATGTCGC TACCGCCACG AGCTACCCGC TCCTCTACTC GGCACACTGG 840
AACGGTACCC TCCTCGGAGG AGCACTGCAT ATGCGTTACG CCGTGTCGAT GGCTCATCAG 900
GCCCAAGAGC GTAATATGTG GTACTTCACT GCGGGCAACC TGTTCAATCC GGGCAAACGG 960
ATCAACGGAT ACCTCGACCT CACCTACTCG ATCGAGGGAT TGGACGACAA AGGCATTATG 1020
ACTGCTCGCT ACGGCAAGGG CAAGACCCTC ACGGACGTCA AGTACTATGC TCTGGTATCG 1080
AAGTGGAACT TCCGCATTTT CGATCAGGTC AATCTCTTCC TCAAAGGCAT GTACGAGAAC 1140
GGCTATGCGC CTGCCCAATA CGGCGAGAGC AGCCACACGC GCCACTCCTA CGGCTATATG 1200
GGAGGGGTGG AATATTACCC TACGGAGACC AACTTCCGTC TGTTCGTCAC CTACATAGGA 1260
CGGCATTACC GGTACAGTGC GACCGAGACG GAAAGCACCA ATGCTCTTCG CGCCGGTCTG 1320
ATCTATCAGA TACCTTTCTT A 1341

(2) INFORMATION FOR SEQ ID NO:94

(i) SEQUENCE CHARACTERISTICS:
(A) LENGTH: 681 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...681

(xi) SEQUENCE DESCRIPTION: SEQ ID NO:94

TATAAGCAAG CTATAATGAA ACATTTGTTT AAGTCGACAT TAGTACTTCT TTGTGCTCTT 60

TCTTTTTCCG GTACCTATAC CTTTGCACAA GAAAATAATA CAGAAAAGTC ACGATTTGAT 120
TTTTCTGTTA GGCTGGGACA GGGATATATT GCAGGTTCAA CTACCAACCT GATGTATGGG 180
TATACATCTG CTAACGATAG ACTTTTGTCT GGTGCAATTT ATCTGGGCTT GACACCAAGT 240
AAGAAAGAAA ATGCAACCGG CGTAGCATTT CGTTTCTTAT CNCCCTCTCC GGGTTATTAT 300
GTCGATATAT CCGGCAAAGA AAATACCTTG AATTATGCGT TTTACGTTGT CGGAGCATAT 360
AATAGAATAG CCATTCCTAT ACGCCCTATC AAAAATTTTA ATTTCATCTT CTCTACAGAA 420
GTCGGAATGG CTTGGATGAG TCGTCATGAG CAAATTTACA ATTCTACTTC GCAGACTTGG 480
GATAAGCAGC GCAAGTCGAG GTCGGGACTG GATTTTGGTC TCGGGATGCA TCTGCAATNC 540
CACATTAATA AGACCGTTTA CTTTATGGCA GGAACCGATC TTACGTCTTG CATGTTCGGA 600
AAAAGGATCA ATGACTACCA GCAAAAGGAT CGAACCTTCA TTGCACTTAT CGACAACAGT 660
ATTGGCATAG GATTAAACCT C 681

(2) INFORMATION FOR SEQ ID NO:95

(i) SEQUENCE CHARACTERISTICS:
(A) LENGTH: 1218 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1218

(xi) SEQUENCE DESCRIPTION: SEQ ID NO:95

ATATTCATAG ACCCCGATAA GAATACAAAA CAAAACGAAC GAAATATGAT TATCAAGAAA 60
ATGCTGAAAA ATAAATTGGC CCCCTTGGCC ATACTGTTCC TTTTTGCTCC AAAGGCTATG 120
AAGGCTCAGG AGCAACTGAA TGTGGTACAC ACCTCTGTGC CATCGCTGAA TATCAGTCCG 180
GATGCACGTG CGGCCGGTAT GGGGGATATA GGTGTGGCAA CGACGCCGGA TGCGTATTCA 240
CAGTATTGGA ATCCGAGTAA ATATGCTTTC ATGGATACGA AAGCCGGTAT TAGCTTCTCA 300
TATACACCCT GGCTGTCCAA GCTGGTCAAT GATATTGCCC TGATGCAGAT GACCGGTTTC 360
TACAAATTGG GAACAGACGA GAATCAGGCT ATTAGTGCTT CTCTGCGTTA TTTCACATTA 420
GGAAAGTTGG AGACTTTCGA CGAATTGGGC GAATCCATGG GAGAGGCCCA TCCCAATGAA 480
TTTGCTGTCG ATTTGGCGCTA TAGCCGCCAG TTGTCGGAGA ACTTCTCCAT GGCTGTTGCA 540
CTGCGTTACA TCCGCTCAGA CCAAAGCACT CACAACACCG GAGAGAATCA GGCCGGAAAT 600
GCCTTTGCGG CGGATATAGC CGGTTATTTG CAGAAGTATG TGCTACTGGG TAATGCGGAG 660
AGCTTGTGGT CGTTGGGTTT CAACGTAAAG AATATCGGAA CGAAGATCTC CTATGACGGA 720
GGTGTCACGA GTTTTTTCAT CCCTACTTCG TTGAATCTCG GGACGGGGCT GTTGTATCCG 780
ATCGATGACT ATAACAGCAT CAATTTCAAC CTTGAACTTA GCAAGCTGCT TGTACCCACT 840
CCTCCTATCA TGGATCAAAA CGATCAGGCC GGGTATGAGG CTGCACTCAA GAAATATCAG 900
GAAACTTCTT CGATCAGCGG TATATTCTCT TCTTTCGGTG ATGCGCCGGG AGGACTCAAG 960
GAAGAATTCC GTGAGATTAC ATGGGGACTT GGGGCTGAAT ATAGCTATGA CGATAAATTT 1020
TTTGTTCGTG CCGGATATTC ATACCTGCAC CCCACCAAAG GCAATTTGCA GTACTTCACG 1080
GCCGGTGCCG GCTTCAAAAT GAACATATTC CGTATCGATG CTTCCTACCT GTTGTCTACG 1140
ATCCAGAGTA ATCCGTTGGA TCAGACTCTG CGGTTTACGC TTGCTTTCGA TATGGATGGA 1200
TTGCGCAATT TGTTCCAC 1218

(2) INFORMATION FOR SEQ ID NO:96

(i) SEQUENCE CHARACTERISTICS:
(A) LENGTH: 1356 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1356

(xi) SEQUENCE DESCRIPTION: SEQ ID NO:96

CTAATAATCG AAAAGGAAAT GAAAACAACA GTTCAACAAA TTATTCTGTG CCTGGCTTTA 60
ATGATGTCAG GTGTATTGGG CGGAAACGCA CAGAGCTTTT GGGAAGAAAT AGCTCCTCCT 120
TTTATCAGTA ATGAGCCTAA CGTCAAGTAT ATAATTCCCA ATATGGGGAT TGATTCAAAG 180
GGAACAATCT ATGTAACCGT GACAAAAAGG ATTCAGCAGG GAGCAAATTA TACTTCTGAG 240
CAATTGGGTA TGTACTATCG ACCATTAGGT GATAATGAAC AGTGGTGGAA ACATGATCCG 300
TATTTTGATG ACAAGATAGT TGCGGATATT CAGACAGATG CATATGGCAG AGTTTATGTA 360
TGTACGACTT CTTCTCGAGA TCAAGAGTAT CAACTTTATA TAAACGAGCA GAACGAATGG 420
AGGTGTATAT TCAAAACTTC TGTGTCTACA TATGAGCATG GTATGGCTGT TTTTCGCTCT 480
TCGACAGGGG TGACTTATAT AGGTACCAGG CATCACATCT TCGCATCAGG TGTAAATGAT 540
TTCGAGTTCA ACACTATCTA TGAAGACTCT ACACCTATGA GCTGTCGCTT TGCAGAGGCT 600
ACGAATAGTG GCACCATCTA TCTGGCATTG ATGCATGAAA CCACAATGTC TACGACTATC 660
CTTACTTATC AAAACGGTGA GTTCGTCGAT ATCTCGGAAA GTGAATTGAG TAACTCGATT 720
ATTGCATCCA TGTGCTCTAA TAAAGAAGGT GATATAATAG CTCTTGTTAC TTCATATACA 780
GGATTTATGA GTGGAACCCT TGCGATCAGA AAAGCAGATG AAGGCAAATG GCAACTTGTT 840
GGCGGAGATA TACAGAATGC GATCGTTCAA AATATATGCA TGATGGACGA CAACAAGATT 900
GCTTGTGAAG TCTTCGGGAC TCCTAACGGA GTAGATGGTC GGACAAGGGT TTGTGTTTCT 960
GACGCATCTG TCTTTGATTT TGAGTGGTAT GAAGATGAAA TATACGGAGG CCTGATATTT 1020
GACACTTTCT TCTATAGCCC TTGGGACAAA CTTCTTTATG CGAAATTTGG TGGGATTATG 1080
CTCAGGAGTA AAGAGTCTT[?] TATAACCTCT TTCATTTCTC CGACAGTTGT ACAAGGAGTG 1140
GATGTCTATA CTTTGGCCGG GAAGATAAGG ATCGAAAGTG AAACTCCGGT GTCTGAGGTG 1200
TTGCTTTTCG ACCTGGCTGG CAGGATGGTA CTTCGGCAAA CCATTGATAA TAAAATCTAT 1260
TCGGACATAG ATACTAACGG ACTAAAGCGA AGCGGTATTT ACGTAGTCTC GGTGCGGCTC 1320
TCTTCCGGAC AGGTATTCAG TCATAAGGTG CAGGTA 1356

(2) INFORMATION FOR SEQ ID NO:97

(i) SEQUENCE CHARACTERISTICS:
(A) LENGTH: 993 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...993

(xi) SEQUENCE DESCRIPTION: SEQ ID NO:97

GGCCTGTACG GTATGTCTGT TGTACCTATA ATAATTTATC TTTGCGGTAT ATCAAATTAT 60
GCAAGACTCA TGATAATCCG GTGTCTTATC CGTCGTCCGA GAACCGTCCT GTTCGGGTTG 120
ATATTCGTGG TAGGTCTTTT CTCTGCGATG GCGCAAGAGA AAAAGGATAG TCTCTCTACG 180
GTTCAGCCAG TGCCGAATAG CAGCATGGTG GAGCAGACCC CTCTTCTCTC CATTGATCAC 240
CCCGTCCTGC CCGCTTCTTT TCAGAATACC CGTACACTGA AAAGGTTTAG AGACAAACAT 300
CTTTCCGATG CTTTGCTCAA TGGATTGAAG CCTCATCGCT CATCTTTGCA ATTGAATGAG 360
GAACTCAACT TCGCGGCAGA GCGTCGGGAT TTCGTTTCTC CCCTCTTGCA AACTCGCCAC 420
GCTGCCGGTG TCCTTTCATG GCGACCGACC GATAGGATGC ATTTTTATAC ATCGGGCAAT 480
ATCGGTCTTG GCCATGATTT ATTGACCGGT GTGCGCAAGG ACTTCGGATG GAATGCTGGT 540
GCCGACTTCT TGCTGAGTCA AAATCTTACG GCACATGTCC AAGGCGGTTG GCAGCAGAAT 600
TTCGGCTTTA TACCTATGAC GGCTGTCAAT GGCCAACTGC GTTGGCAAGC CACCGAGAGA 660
TTGAGTTTTA CCACCGGTAT CGATTATCGA CAGGTACAGT GGAATGCTTT CGATAATAGA 720
ACGTTCTCGC TTAAAGGAAG TGCTCGATAC GAAGTGATGG ACAATGTCTT TGTCAATGGA 780
TTTGGCAGCT ATCCTCTCTA CAGCAGTACG CGCTCAGGAC TCAATATGGC TGTTCCGATG 840
CATGGATTCG GCCCTCAGTA CGGTGGATCG CTTGAGCTGA AAGTCTCCGA GCGATTCGGC 900
TTTGCCGTCG GTATGGAGCG CGAATACAAT ATCTGGACTC GTCGGTGGGA AACGCATTAC 960
TTTGCTTATC CTGTATTCTA TGGCGATAAG AAG 993

(2) INFORMATION FOR SEQ ID NO:98

(i) SEQUENCE CHARACTERISTICS:
(A) LENGTH: 987 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...987

(xi) SEQUENCE DESCRIPTION: SEQ ID NO:98

GAGACGAACT CTTGGGTATC CAGCGATTGC AATTCGACGA CGATGAAAAC GAATAGACGA 60
TACGCATTTG TTTTGCCGCT TCTGCTACTC ACCGGATTGT TGGCATGGGG GCAGGATTCT 120
TCCCACGGTA GCAATACAGC CTTTGCAACT GATTCTTCGA GTAGAGAGTT GCCCACGGAG 180
CAGTCCGCCT ACCGCATTCA TTCTGCCTAT ATGGTCGGTG GTGGCGGAAG CATAACGCGC 240
GACACCTATT TGTCACCCCT TCGTTATGGA GGATGGACAC TGAATTTGTT GGGAGAGAAG 300
ACGTTCCCTC TCAAAGCCTC CGATTCCCGT TGGATGATCC GTACCGGGCA TGAGCTGGAT 360
TTTGCCCTGA TGGACAATCC GGCCAATAAT GCTCATTTCT ATTCCCTGCT GTATAACGGT 420
TCCGCTGCGG CTCTTTACCG CCTTGGCGCT AAGCATCTGC GAGCCGCGTG GATGGACAAT 480
CTGCGCTTGG CATTCGGCCC GGGCTTGGAA ATCGGGCTTG GAGGAATTTA TAGTACACGC 540
AACGGCAATA ATCCTGCGAC ATTGAAGCTC TACACCAATG CCATCGCCCA AGCCTCGATA 600
GGATACTACG TCCCCTCCGA AACTTTTCCC CTGTATTTTC GGTTGCTCTC CCAGATCAAT 660
CTCTTCGGTA TAGCCTATGG AAATGGTTTT GGTGAGAGCT ATTACGAGAA TTTTTTGCTC 720
AATAACGGCA TTGCAGGCTC CCTGCATTTC ACTTATCCGG GCAAGTTTAC TCGGTTCACG 780
ACACTCATAA CGGCGGATAT TCCCATTCGG AACTTCTGTA CGCTTCGTGT CGGTTATCGC 840
TATTCCCATT TGGGCTCTTC GCTTAACGCA TTGGATACTC GAATCCACAG TCATACGGCT 900
TTTATCGGTT TCGTCACGGA GTTTTACCGA TTCCGTGGGC GCAAAGCCAT GAATACCGGT 960
CGGAGAACCA GTCTTTACTA TCATGAT 987

(2) INFORMATION FOR SEQ ID NO:99

(i) SEQUENCE CHARACTERISTICS:
(A) LENGTH: 957 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...957

(xi) SEQUENCE DESCRIPTION: SEQ ID NO:99

TTTACGTCCG GTACGATATT CGTCCGTATA GCCATATCCG GCCGTGTAGT GTGCCGTGAG 60
GTTGAGGATA ACGGAAGGAG AGAAGCTGTG CGTCATGATG GCATGGTAGT GACGCTGCTC 120
GTAATTGTCG GTATTGTGGT AGTAGTGCGG TACTCCTTGC GCGTCCACGT ACATAAGACC 180
GGCACTGTTG TATCGGCGGC CATATTTGGC TTCATCCTCC TTGGAAAGAC CGTTCCATGC 240
GATACCCGTA ACTTCTTTTC CTCCGAAAGT GATGAACCTG AGAGCCGTGT TGCTACCGAA 300
ATAGCCCACC TGTGCGAAAT AGGATTTCAG ATCCACGCTT CCTCTATCCA CGTAGCCGTC 360
CGAACCGATT TTGGACAGGC GGGCATCCAC TGCCCAATGG CGACCGATGC GTCCGCTACC 420
GAGTTTGACC GATCGGCGGA ATGTGCCGAA CGAACCTCCG CTCAAATCGA CACGGCCATA 480
AGGAGCCAGT CCCAAATTAT CCGTACGCAT ATTGACACTT GCCCCAAAAG CTCCGGCACC 540
ATTGGTGGAA GTACCCACAC CTCGCTGCAC CTGAAGGTCT TCGATGGAAG AGGCGAACTC 600
GGGCATATTC ACCCAAAAGA CGGACTGAGA TTCGGAGTCG TTGAGGGGTA CTCCATTGGT 660

AGTTATGTTG ATGCGATTGG CATCGGTGCC ACGCACGCGA AAGCCGGAAT ATCCGATACC 720
CGTACCGGCA TCGCTGGTGG CTACCACGGA GGGAGTCAGC ATCAGCAGAT AGGGGATGTC 780
ACGACCATAA TTGGACTTGG AAAGTTCGGC CTTGCGAACG TTGGTGTAAG CGACAGGGGT 840
TTTCGCCGTG GCGCGAGTAG CTACGACCTG TACGGTCTGG AGCTGCACAT TGCTAAGACT 900
ATCTATCTCG CTGTTGGAGA CGGGTGCTTG TGCCGTCAGG CAGAAAGGCA GGACGGC 957

(2) INFORMATION FOR SEQ ID NO:100

(i) SEQUENCE CHARACTERISTICS:
(A) LENGTH: 1842 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1842

(xi) SEQUENCE DESCRIPTION: SEQ ID NO:100

CCATATAATG TCCAATCTAT TAGCAACAAG ACGATTAAAA AACAAATGGA AAACTTAAAG 60
AACATTCAGC CCAGAGAGGA TTTCAACTGG GAAGAGTTTG AGGCCGGTGG CGTCCATGCT 120
GCCGTGAGTC GTCAGGAGCA GGAAGCTGCT TATGACAAAA CGCTCAATAC CATCAAGGAA 180
AAGGAAGTGG TAATGGGTAG GGTAACTGCT ATCAACAAGC GTGAAGTGGT TATCAATGTA 240
GGGTACAAAT CGGAAGGTGT GGTACCTGCA ACAGAATTCC GCTACAATCC CGAACTCAAA 300
GTGGGAGACG AAGTGGAAGT TTATATCGAG AATCAGGAAG ATAAGAAGGG CCAGCTCGTC 360
TTGTCTCACC GCAAGGGTCG TGCCGCTCGC TCTTGGGAGC GCGTGAACGA GGCTCTCGAA 420
AAAGACGAAA TCGTAAAGGG CTATGTGAAG TGTCGTACCA AGGGTGGTAT GATCGTCGAT 480
GTATTCGGTA TCGAGGCTTT CCTCCCGGGA TCACAGATCG ACGTGCGCCC CATTCGCGAC 540
TACGATGCAT TCGTTGAGAA GACGATGGAG TTCAAGATTG TGAAAATCAA TCAAGAATAT 600
AAGAATGTAG TTGTTTCCCA CAAGGTGCTC ATCGAAGCAG AGCTCGAACA ACAGAAGAAA 660
GAAATCATCG GCAAGCTCGA AAAAGGGCAG GTACTCGAAG GTATCGTCAA GAATATTACT 720
TCTTACGGAG TATTTATCGA CCTCGGTGGA GTGGATGGTC TTATCCATAT CACTGACCTT 780
TCATGGGGTC GTGTGGCTCA TCCGGAAGAA ATCGTACAGC TGGATCAGAA GATCAATGTC 840
GTTATCCTCG ACTTTGATGA AGATCGCAAG CGTATCGCTC TCGGACTCAA ACAGCTGATG 900
CCTCATCCTT GGGATGCTCT CGACAGCGAG CTTAAGGTAG GCGATAAGGT GAAGGGTAAA 960
GTTGTGGTGA TGGCAGATTA CGGTGCTTTC GTTGAGATTG CACAGGGCGT TGAGGGTCTT 1020
ATCCACGTAA GCGAAATGTC ATGGACACAG CACTTGCGTT CTGCTCAGGA CTTCCTGCAT 1080
GTAGGCGACG AAGTGGAAGC CGTGATCCTG ACGCTCGACC GCGAAGAACG CAAAATGTCG 1140
CTCGGTCTGA AGCAACTCAA GCCGGATCCT TGGGCTGATA TCGAAACTCG TTTCCCTGTA 1200
GGCTCTCGTC ACCATGCTCG TGTTCGCAAC TTCACCAATT TCGGTGTATT CGTTGAGATC 1260
GAAGAGGGCG TAGATGGCCT TATCCATATT TCCGACCTTT CTTGGACGAA GAAGATCAAA 1320
CACCCCAGCG AGTTTACGGA AGTAGGTGCT GATATCGAAG TTCAGGTAAT CGAGATCGAC 1380
AAGGAAAACC GTCGTCTCAG CTTGGGTCAC AAACAGTTGG AAGAGAATCC TTGGGATGTA 1440
TTCGAGACGG TATTCACTGT AGGATCTATC CACGAAGGAA CGGTAATCGA AGTGATGGAC 1500
AAGGGTGCTG TCGTTTCTCT GCCTTACGGT GTGGAAGGTT TTGCCACTCC GAAGCACATG 1560
GTGAAGGAAG ATGGCTCACA GGCTGTACTC GAAGAGAAGT TACCTTTCAA GGTTATTGAG 1620
TTCAATAAGG ATGCCAAGCG AATCATTGTA TCTCATAGCC GTGTATTCGA AGATGAGCAG 1680
AAAATGGCTC AGCGTGAAGC CAATGCAGAG CGTAAGGCTG AAGCCAAAGC GGCTCAGAAA 1740
GAAGCTGCTG CCGAAGCTGC CAATCCTGCA CAGGCTGTAG AGAAAGCCAC TCTCGGAGAC 1800
CTCGGCGAGC TGGCCGCTTT GAAAGAAAAG CTTTCAGAAA AC 1842

(2) INFORMATION FOR SEQ ID NO:101

(i) SEQUENCE CHARACTERISTICS:
(A) LENGTH: 729 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...729

(xi) SEQUENCE DESCRIPTION: SEQ ID NO:101

TCGACAATAA TGAAAAAAGC TATTCTTTCC GGAGCGGCCT TGCTCCTCGG CCTATGTGCC 60
AACGCACAAA ACGTGCAGTT GCACTACGAT TTCGGTCATT CCATCTACGA CGAACTAGAT 120
GGACGTCCCA AACTGACTAC CACAGTGGAA AACTTCACAC CCGACAAATG GGGAAGCACC 180
TTCTTCTTCA TCGACATGGA TTACACGGGC AAGGGTATCC AGTCGGCCTA TTGGGAGATT 240
TCGCGCGAAC TGAAGTTTTG GCAAGCTCCC GTTTCCATTC ATTTGGAGTA CAACGGAGGC 300
CTCTCCACAA GCTTTACTTT CGGACACGAT GCTCTAATCG GTGCCACCTA CACCTACAAC 360
AACCCCTCCT TTACACGTGG ATTTACGATC ACGCCCATGT ACAAGCATCT GGGTGCGCAC 420
GACTTCCACA CCTATCAGAT CACCGGCACT TGGTACATGC ACTTTCTGGA CGGTCTGCTT 480
ACCTTCAACG GCTTCCTCGA TCTTTGGGGT TTCCCCCAAG AGAACCCAAT CGGGGGCCCT 540
GTGCTCAAAG AAGGGGATAA GTTCGTATTC CTGTCCGAAC CGCAGTTCTG GATCAACCTC 600
AATCGCATCA AAGGCATCGA CAAGGATTTC AATCTCAGCA TAGGGACAGA GATGGAAATC 660
AGCAGGAACT TCGCTCGCAT GGACAAATTC TCCTGCATCC CTACTCTTGC GGTCAAATGG 720
ACTTTCAAC 729

(2) INFORMATION FOR SEQ ID NO:102

(i) SEQUENCE CHARACTERISTICS:
(A) LENGTH: 705 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...705

(xi) SEQUENCE DESCRIPTION: SEQ ID NO:102

ATGAAAACAA TTAGTAAGAA CCATGCGGCA CGGATCTGTG CCGCCATTGC TTTGTTTGCA 60
GTGTGTAACG GCCGGATAGC TGCTCAGGAT TTTCTCTATG AAATAGGAGG AGGTTTTGGT 120
GCTGCTCAGT ATTTTGGCGA TGCAAACAGA GGCTTGTTCG GTTCATCCGG AGTAGGTTTG 180
GAGTTGGTCG GACGTTACAA TTATAATTTT CGCTGGGCTT TCAGTACCAT GTTGGATTGG 240
CGTACATTGA GAGGCGATAC CGATAAGTCC GGGAATGTCT TCCCCGATTT TGCTCAAGCG 300
GATTTTAAGG TCGGCTTGAC TCAGCTCCAC GTTAGAAGCG AATTTAACTT TCTCCCTTAT 360
AGCGATGGCT ATAAGTATCT TGGTACAGCT CGGCTGTCTC CTTATGTAGC GGCCGGGTTG 420
TCTTTGGGTT TTGCTTCGGG TGCTAAAGCT TCGGCTTTTG CTCCCGGGAT TACTGCGGGA 480
ATGGGAGTGA AGTATAAGCT TAAACCGCGG ATCAATGTCG GTATCGAGTA TTCTTTCACG 540
GGGTTACTTA CCGATGCTTT GGATGCGCTG ACGGATAAAA GTGTTTGGCT CGAGGATCCA 600
TATAAGATCA ATGACTCCTG GGTCAAAAAC AAGGATGCTA CAGGGGCCTT AGTGCTTAGG 660
ATTACGTATG ACTTCGGCCT GCGTAAGACT TTTTGTAATA AACAA 705

(2) INFORMATION FOR SEQ ID NO:103

(i) SEQUENCE CHARACTERISTICS:
(A) LENGTH: 1300 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1308

(xi) SEQUENCE DESCRIPTION: SEQ ID NO:103

AATATAATGT ATAAAGACTA TAAGGGTTTG TATGCGTCGC TTCGGTGGTA TGCCCTGATC 60
ATTGGGTTGC TATTTGCAGC AGACGGTATA CAGGCTCAGA ACAACAACTT TACCGAGTCG 120
CCTTACACTC GCTTCGGCCT TGGCCGTCTC GGAGAACGGA CGACTATTAG TGGGCATTCC 180
ATGGCAGGAC TCGGCGTCGG TCTGCGTCAG GGCACATACG TCAATGCCGT CAATCCTGCT 240
TCATACTCGG CTGTGGATTC GATGACGTTT ATCTTCGATT TCGGTGCATC TACCGGAATT 300
ACGTGGTATG CCGAGAACGG GAAAAAGGAC AATAGGAAAA TGGGAAACAT TGAGTATTTC 360
GCCATGCTTT TTCCTATTTC CAAATCCATT GCTATGAGTG CGGGAGTGCT TCCTTACTCC 420
GCATCCGGGT ACCAGTTCGG ATCCGTTGAT CAAGTGGAAG GAGGCAGCGT CCAGTACACC 480
CGTAAATACT TGGGGACAGG CAATCTGAAC GATCTCTATG TCGGTATAGG TGCAACCCCG 540
TTCAAAAACT TCTCAATAGG AGCCAATGCT TCATCCCTTT TTGGGCGATT CACACACAGC 600
AGGCAGGTAA TCTTCTCCAC GGAGGCTCCT TACAATCCCG TACATCTCTC GACGCTGTAC 660
TTGAAGGCTG CCAAGTTCGA CTTCGGTATG CAGTATCACC TTCTTCTCAA ATCAGATCGT 720
TCGCTCGTTA TCGGTGCCGT CTATTCTCCG CGGGTGAAGA TGCATAGCGA GCTGACTCAG 780
ATAAAGAATC AGGTTCAGAA CGGTGTAGTA GTGGAGAGCG AAACCCAAGA ATATATCAAG 840
GGAATGGACT ATTATACCCT GCCTCATACA TTGGGGATAG GTTTTTCTTA TGAAAAGAAA 900
GATAAACTTC TCTTAGGAGC AGACGTCCAA TATAGTAAAT GGAAAGGCGA GAAATTTTAT 960
AAATCCGATT GCAAATTCCA GGACAGAATA CGGGTATCTC TCGGCGGAGA GATCATACCG 1020
GATATAAATG CCGTTGGGAT GTGGCCTAAA GTTCGCTATC GCTTCGGTTT ACATGGTGAA 1080
AATTCTTACC TGAAAGTGCC GACTAAAGGC GGTGTATATC AAGGATACCA TATCGTAGGT 1140
GCTGTATTCG GTATAGGAAT CCCGCTCAAT GACAGACGTT CGTTCGTAAA TGTCTCTCTT 1200
GAATATGACC GATTGATCCC GAAGGAGGGT ATGATCAAAG AAAATGCTCT GAAATTGACC 1260
TTCGGCCTCA CGTTCAACGA GTCATGGTTT AAAAAGCTGA AACTGAAC 1308

(2) INFORMATION FOR SEQ ID NO:104

(i) SEQUENCE CHARACTERISTICS:
(A) LENGTH: 2835 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2835

(xi) SEQUENCE DESCRIPTION: SEQ ID NO:104

AATACCATTT CGGAGAATGG GAACGATAGC ACCTATTTTT CTTTTCAAAC TTTTCTTATG 60
CGATCGATTT ATCAATTACT GTTGTCAATA CTCCTTGCTT CTCTTGGTTT CGTCGGGCTG 120
GAAGCCCAAC AAGCCGGAGT AGCAGGTAGA GTATTGGACG AAGAAGGCAA CCCCATGATT 180
CAAGCCAACG TACAGCTTGT ACAGAGTACC GGCCAAGTAG CCGTTGCCGC AGGTGCCACT 240
AATGAAAAAG GGTTGTTCAG CCTGAAAACG TCACAGGAGG GTGACTACAT TCTGCGCGTT 300
TCATATGTAG GTTACACTAC CCACGACGAA AAAATATCTC TTAGAAACGG GCAAACCATT 360
ACGCTCAAAG ATATATCCAT GAACGAAGAT GCCCGTCTTC TACAGAGTGT GACGGTGCAG 420
GCTAAAGCGG CAGAGGTCGT GGTACGCAAC GATACGCTCG AATTCAATGC CGGATCCTAT 480
ACCGTAGCAC AGGGAGCTTC TATCGAGGAA CTGATCAAGA AGCTACCCGG AGCAGAGATC 540
GGATCCGATG GGAAGATCAC CATCAACGGC AAGGACATTA GCAAGATCCT TGTCGATGGC 600
AAAGAGTTTT TCTCCAAAGA TCCACAGGTG GCAATAAAGA ATCTTCCGGC CGATATGGTC 660
AATAAAGTAC AGGTACTGAA CAAACTGAGC GAGCTGTCGC GGATGAGCGG TTTCGATGAT 720
GGAGAAGAGG AGACCGTAAT CAACCTGACG GTGAAGCCCG AAAAAAAGAA AGGCCTCTTC 780
GGAACGCTTC AGGCCGGCTA CGGTACCGAC CAACGCTATA TGGCCGGAGG GAACGTCAAT 840
CGGTTCGATG GAAATAAGCA ATGGACATTG ATCGGTAGTG CGAACAATAC GAACAATATG 900
GGCTTTAGCG AGATGGACAG CGAGATGGGA TCCATGACCT TCTTCTCTCC CCAAGGCGGT 960
GGTCGACGCG GCTTCGGCAA TAGTGGAGCT GTTACGTCTT CGTCGATGCT GGGCGGCAAC 1020

TTCAGTGTCG AATTCTCCTC TGCCCTTAAT ACAGGAGGCG ATGCACGCTA CGGATACAAC 1080
GACAAGGCCA TAGAGACGAC CAAACGCGTG GAAAATATCC TCGCCGAAGG GAATACTTAT 1140
ATGGACGAAA ATATATTGGA ACGCTCTTTC TCTCACAATG GTCAGGCGCG ATTTAGGATG 1200
CAATGGAAAC CGTCCGAACG TACCGAAGTG GTATTCGAGC CGGATCTTTC GATATCCAAG 1260
ATCGATGGGT TCTTTAACGA CACATACGAG ACGAAAGATG CCACCGGAAT CTCTATCAAC 1320
AAAGGTTCTA TCCACCAAAC TACACAAGGA AACAACTTCA GACTGAACGG AGAATTGGAT 1380
ATCAGTCACA AGCTCAACGA CGAAGGCCGT ACGATCAGTG CCTCCGTCAG TGGCGGTCTG 1440
ACCGACGAAG ACGGAGATGG CATATATCAG GCTGTGCTCC AAAGCGTGGA GACGAATCAA 1500
AAGCAATTCA ACGACAACTC CAACCTGCAA TATCGGCTTC GCCTCTCGTA TGTGGAACCG 1560
TTGGGTAAAA ACTACTTCGC ACAAGCGATT CTGAACAGAC GTTTCTCCCG TCGCAATTCG 1620
GATCGTGAGG TGTACCGACT GGGCGATGAC GGGCAATACT CCATATTAGA CAGTCAGTAC 1680
GGACTCTCCT ACAGTAACGA GTTCACCCAG TATCGCATCG GACTCAACCT CAAGAAGATT 1740
GCCAAAACGT GGGACTACAC CGTAGGATTC AATGTGGATC CCAACAGAAC TGTCAGCTAT 1800
CGGAGCGTAG CCGGAGTAGA GCAGGACAAA CTGGCTTTCA ATCGTGTCAA TCTCTCCCCG 1860
ATGCTCCGAA TCAACTACAA ACCGAGCAGG ACTACCAACC TCCGAGTGGA CTACCGAGGA 1920
CGCACGACAC AACCATCCAT CAATCAGATC GCTCCCGTTC AGGACATCAC GAATCCGCTA 1980
TTCGTGACGG AAGGCAATCC CGGTCTGAAG CCGAGCTATT CCAACAATGT GATGGCCATG 2040
TTCTCGGACT TCGATGCCAA AAGTCAGCGA GCTTTCAACA TTGTTTTCTT CGGCAACTAT 2100
ACATTCGACG ACATCGTCCC CAATACGCAC TACGATCCGT CTACAGGGAT CCGTACCACT 2160
CGTTACGAAA ACGCCTCCGG TACGTGGCAA GCGAATCTTC ATGGGACACT ATCGCTTCCA 2220
CTCAAGAACA GGGCATTTTC TTTCAGGATG TCCTTGTTCA ACAGGTTGGC CGAAGGACAA 2280
AGCTTCATCA ATGACGATAA GAACAAAGCT CTCTCTTTCC GAACGAGGGA ACGCCTGACG 2340
CTGACCTATC GCAACAATTG GATCGATACG AGTATCGGTG GCAATATCGG ATTCTATATG 2400
GCGAATAATA GTCTGAGCGG ACAGAAAGAT TCTCGCACAT ACGATTTTGG CGGCAATTAT 2460
CAAGTTGCCC TAACGCTTCC CTATGGATTC CGTATCGACA GCGATGTTGA ATACAATACG 2520
AACTCCGGTT ACAGCGGAGG ATTCAGTCTG GACGAATGGC TTTGGAATGC TTCGCTTTCA 2580
TACAGCTTCC TCCGTGACAA GGCCGGTACA CTGCGTGTCA ATGGCTATGA CATCCTCGGT 2640
CAGCGGTCAA GTATCAGCCG TTCTGCTTCG GCCATCAATA TAGAAGAGAG CATGTCCAAT 2700
ACGATCGGAC GCTACGTGAT GGTGGACTTT ATCTACCGAT TCAACGCCTT CAGTGGTGGT 2760
GGATCTCGCA GCGATCATCA GCGTGGCAAT ATGAATCGTC CGGGCCCACC TTTCGGCGGT 2820
GGCAGACGAC CGTCC 2835

(2) INFORMATION FOR SEQ ID NO:105

(i) SEQUENCE CHARACTERISTICS:
(A) LENGTH: 1236 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1236

(xi) SEQUENCE DESCRIPTION: SEQ ID NO:105

GGAGAGTATC CTGCAAACAG TAACGACAAA AAAGAGATGG TTATGAAGCT GATTAAAAGA 60
AGTTTGCTCC TGCTTGGAGC GGTACTGCTG ATTACGCTTC CTGCGTACTC GCAGAATGAT 120
GACATCTTCG AAGATGACAT CTATACATCG CGAAAAGAAA TACGTAAACA AAACCAAGTT 180
AAAGACTGGC AAAACCAAGA GGACGGATAC GGCGACGATA CGGAATATAC AGTGGCTTCC 240
GATCGGGACA TTGACGCCTA CAATCGTAGA GATGGCCAGT CCTACGATGG GAAAAAGTTG 300
TCCAAAGACA AGAAAAGAGA CTCCACTCCT TCTTCTGTTC CCGGTCGCTA TAGTCGCCGC 360
TTGGCTCGAT TCTATAAGCC GAATACGATC GTCATTTCAG GTGCCGACAA TGTATATGTA 420
ACTGATGATG GTGAGTATTT CGTCTATGGA GACGAATACT ATGATGACGC GTCGTCTGTA 480
AACATTTACA TCAACAGTCC TTGGTGCGAT CCGTTCCCTT ATACGTCATG GTATCCATCT 540
TTCTCCGGCT GGTACAACTA TACGTGGAAC TATCCATGGT TCTACTACGG TAGCCATATC 600
GGATGGGGCG GTTATTACCC CGGATATAAT TGGTATTGGA GCTACTACTA TGATCCTTTC 660
TACAATCCCT ATGGAATCGG TATGGGTTGG GGATATCCTT ATGGCTGGGG CAGCTATTAC 720
GGTTGGGGTG GCTATCCGGG AGTGATACAT CACTACCACC ACTACCCCAA GAAGACCTAT 780
TCCAATGGTC AGCATTCCGG AGCTTACTAT TCTTATGGCC GACCGAATCG TATCAAAGGT 840
GGAACGTCCG GTGCCAAACT TGGGACAGGA CGCTACGATA GAATTCAAAA TTCGTCTTCG 900
CAAAAAAATA AGTTCGGATT GCAGTCGAAC AAACCCAATA ATAATCTGCA AAATGTCAAG 960
TCGGGACGTA CCGGCCGAGC CAATAGAGAC CGAAATATAG AAACGGTAAC TCCAAACAAC 1020
GGGCAAAAGC AGAATCGTCC CGTATTCCAG CAGAATCAGT CCGGCAATGA CCGACCGACC 1080
GGACGGAATA TCCGCAGCGA GAGACAGGGG GAAAATAACG ATAGGACATT TTCGACTCCT 1140

TCTCGTAGCA ATAGTAACGG TGGCTTCTCC ACGCCTTCTC GCTCTTCTTC CGGCTCTATG 1200
AGCGGAGGTG GCGGACGTAG TGGCCGGGGA CGCAAT 1236

(2) INFORMATION FOR SEQ ID NO:106

(i) SEQUENCE CHARACTERISTICS:
(A) LENGTH: 1803 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1803

(xi) SEQUENCE DESCRIPTION: SEQ ID NO:106

AGTAATAGCA GCTCCCACAA GTGGTTAATT TATTATCATA TAGAAAAGAC TAAAAGTATT 60
ATGATCCGCA AGTTGATCTT ACTGCTTGCT CTGATGCCGG TAGCCTCTGT GGCTTTTGCG 120
GTACCAACGG ACAGCACGGA ATCGAAAGAC AATCGTATCC TTACAAGCAT GCAATCCTCC 180
TCTTTGAATA GGGATGATGC TCCGGATAAA TGGCAACCTA TGCATGCCAA TTTCAGTATT 240
CAGAGCGATA TGCTGCTTTC TACTGCCCAA AAGTCCAAGA ACACCTGGTT CGGCAACTCC 300
TATATCATGG GTATAATCAA GAACAATTAT CTGGAGTTTG GTGCCCGTTT CGAGGATCTC 360
TATAAGCCCC TGCCCGGACA TGAACCCGAG ATGGGGCGTG GCGTTCCTCA CATGTATGTG 420
AAGGGAAGCT ATCATTGGGC GGAGCTGACT ATGGGAGACT TCTACGATCA GTTCGGTAGC 480
GGTATGGTAT TCCGCACCTA TGAAGAGCGC AACCTCGGTA TAGACAACGC GGTTCGCGGC 540
GGACGTATAG TACTCACTCC TTTTGATGGA GTGCGTGTCA AGGGTATTGC AGGACAGCAG 600
CGTAACTACT TCGACCGCAC GGGCAAGGTA TTCAATTCCG GCCGAGGCTA CCTACTGGGT 660
TCTGATCTGG AGCTGAATGT AGAGCGTTGG AGCAGTGCCA TGCGCGACAA TGACTATCAT 720
TTGGCTATCG GGGGATCGTT CGTTTCCAAA CACGAAGCAG ACGAAGATAT ATTTGTGGGT 780
GTAGGCGAAG ATCGCAAGCG ACTCAACCTG CCGCTCAATG TCCCGATTAT GGGCCTGCGC 840
ACCAACTTTC AAAAAGGAGG TCTCGCCCTC TACGCAGAGT ATGGATACAA ATACAACGAT 900
CCCTCGGCAG ACAATGACTA TATCTACCAC GACGGACAGG CTGCACTCCT CTCTGCCTCA 960
TACTCCAAAA AAGGGATGAG TATCCTGTTG CAGGCCAAAC GTTGTGAGAA CTTTGCTTTC 1020
CGCAGCAAGC GAAGTGCCCA GCTCACACCG CTTATGATCA ACTATATGCC GGCTTTTACC 1080
CAAGCTCACA CTTATACGCT GGCGGCCATC TACCCCTATG CTACTCAGCC TCAGGGAGAA 1140
TGGGCTTTCC AAGGTGAACT GCGTTACAAC TTTGCTCGCC GGACAGCTCT CGGTGGACGC 1200
TACGGTACCG GCTTGCGTAT CAACGTTTCG CATGTGCGTG GTCTGGACAA AAAGATGCTC 1260
AAAGAGAATC CCGACGAACT GATCGGAACG GATGGCTACA CCGTTTCTTT CTTCGGCATG 1320
GGCGACCTCT ATTATTCGGA TATAGATGTG GAGATTACTA AAAAGGTAAG CCCAGGATTC 1380
AACTTTACGC TCACCTACTT GAATCAGATC TACAATAACA AGGTACTGCA CGGTGCAGCC 1440
GGAGAGAAGC CTGAGAAGAT CTATGCCAAT ATCTTCGTCT ATGATGGTAA GTATAAGCTG 1500
AGTAATAAGG TAGCCCTCCG TACCGAACTG CAATATTTGC ACACGAAGCA GGATCAGGGT 1560
GACTGGATCT ACGGCATGGC CGAGCTCTCT ATCCTGCCTT CTCTGATGCT TTCCCTCTCG 1620
GAGCAGTATA ATATCGGAGA GACCAAGAAA CATTATGTCA TGGGGTCTGT CACCTATACT 1680
CACGGAGCAC ATCGAGTAGC TTTCTCTGCA GGCAAAACCC GTGCAGGGAT GAACTGCTCG 1740
GGAGGTGTAT GTCGTGTGGT CCCTGAGACT CAGGGATTCT ACCTTTCTTA TAGCACCAAT 1800
CTG 1803

(2) INFORMATION FOR SEQ ID NO:107

(i) SEQUENCE CHARACTERISTICS:
(A) LENGTH: 756 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:

(A) ORGANISM: PORYPHYROMONAS GINGIVALIS

(ix) FEATURE:
(A) NAME/KEY: misc_feature
(B) LOCATION 1...756

(xi) SEQUENCE DESCRIPTION: SEQ ID NO:107

AGGGGGTCGA GTTCGGGCAT TTCAGCCCGT GGACGCGACA TGCGTTCTTT ATTTTTGAGC 60
GCGTTGCGCA GCTCCTCTCT CCATGGTTCA GAGCGACGCA GTCGGATAAG TTCTTCTGTA 120
GTCATGTCAA TAAGGCAGAA AATAAGGTTA TTCCATCTCT CGGTATGCGC CCAAACGCAT 180
GATCATCTCA TCGAAATCCA CTTGGTGTGC ATCGAATTCG GGGCCATCGA CACAGACGAA 240
TTTCGTCTGT CCTCCCACGC TTATACGACA AGCCCCACAC ATACCGGTGC CATCCACCAT 300
AATTGTATTG AGAGAAGCTA TGGTCGGTAT CTCGTAACGT TTGGTCAGGA GAGAAACGAA 360
CTTCATCATC ACAGCCGGCC CGATCGTAAC GCAGAGGTCT ACCGTTTCCC GTTTGATAAC 420
GCTTTCCACT CCATCCGTTA CGAGGCCTTT CGTCCCATAA GACCCATCGT CTGTCATGAT 480
GATCACTTCA TCGCTATTGG CTCGCATTTG TTCTTCAAGG ATAACCAGAT CTTTAGTTCT 540
GGCAGCCAAT ACGACAATTA CACGGTTGCC TGCTTTGTGG AAAGCCTCCA CGATCGGGAG 600
CAAAGGAGCC ACACCCACAC CGCCTCCGGC ACAAACCACT GTGCCGACCT TTTCGATATG 660
CGTACTCTGT CCCAGCGGAC CTACCACATC CGTGATATAG TCGCCGACTT CGAGTTCGGC 720
CAATTTCTTG GAAGATTTGC CCACGGCCTG AACCAC 756

(2) INFORMATION FOR SEQ ID NO:108

(i) SEQUENCE CHARACTERISTICS:
(A) LENGTH: 2370 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2370

(xi) SEQUENCE DESCRIPTION: SEQ ID NO:108

CTCTTGTTTT CTTCTCCTTT ACCCCGAATG GATCGTCCTA AGCCTTCATA TATTGTTCGA 60
ATAGCAGCCA TTCTCTGCTT GTTTGTCGGC AGGCCTTTGT TTGCGCAGAG CTATGTGGAC 120
TACGTCGATC CGCTGATCGG GACGCTAAGT TCTTTTGAGC TGAGTGCGGG CAATACCTAT 180
CCGGTGATCG GTTTACCGTG GGGAATGAAT AGCTGGACAC CGATGACCGG TGTACCCGGT 240
GACGGCTGGC AATATACCTA CTCGGCACAC AAGATTCGCG GATTCAAACA GACCCACCAA 300
CCCAGTCCTT GGATCAACGA CTACGGCCAA TTCTCCCTTC TTCCCCTTAC GGCACCGCAG 360
AAGCCATCAT CGAACGACTC CATAGCTCTG ACTAAATGGT GCAAGCAACT CTTTTCGGAC 420
GAACAGACCT CGTGGTTCTC GCACAAAGCG GAGACGGCGA CGCCATACTA TTATAGTGTC 480
TATTTGGCCG ATTACGACAC ACGCGTGGAG ATGGCTCCGA CCGAGCGTGC AGCTATCTTT 540
CGCATACGTT ATTCCGGCAA TACCGAAAGT GGCTCCGGTC GATGGCTTCG TCTTGATGCC 600
TTTACCGGTG GTTCGGAGAT TAGCATCGTG GATCCTCACA CCGTAGTGGG CATATCTCGC 660
AAGAATAGCG GAGGTGTGCC GGCTAACTTC GCCTGTTATT TCATCCTGCA GTCCGATACT 720
CCTATGGCCG ATGTCCTGCT TGAGACAGAT ACCGGCAAGT CAGACGAAGG CACAAGGGCA 780
TGGGCAGCCT GTCGCTTCGA TTCGCAAGAA GTTACCGTCC GGGTGGCATC TTCTTTTATC 840
AGTGTCGAGC AGGCCGAAAG AAATCTTGCG GAAGTCAAAG GGCAGAGTTT CGACCGGATC 900
AGACTTGCCG GTCGCGAAGC TTGGAATAAG GTGCTCGGAC GCATACATGT GGAAGGAGGA 960
ACGAAGGATG AGCGCACTAC ATTCTATTCC GCACTCTATC GCTGTCTGCT TTTTCCCCGT 1020
CGCTTCTATG AGGAGGATGC TTCCGGCAAT TTTGTGCATT ACAGCCCCTA CAATGGAGAG 1080
GTACTTCCCG GTTATCTCTA TACCGATACC GGATTTTGGG ACACTTTTCG AGCCCTTTTC 1140
CCCCTGCTCA ATCTGCTGTA TCCCGATGAA AACATTAAAA TTCAGGAAGG TCTGCTGAAT 1200
GTATATCGCG AGAGTGGCTT TTTCCCCGAA TGGGCCAGTC CGGGCCATCG GGATTGTATG 1260
ATAGGCAACA ACTCTGCTTC TGTTCTGGCG GATGCCTACC TCAAGGGTGT TCGGGTAGAA 1320
GATACCCGTA CACTGATGAA CGGACTCTTG CATGCTACGA AAGCCGTCCA TCCGAAAATC 1380
TCCTCCACGG GTCGCAAAGG TTGGGAGTGG TACAACTCCT TAGGTTATGT TCCGGCTGAT 1440
GCAGGCATCG ACGAAAGTGC TGCCCGTACG CTCGAATATG CTTATAACGA TTGGTGCATC 1500
CTCCGACTGG GGCGCACATT GGGTTGGGAT AGAGCTGCAT TGGACACGTT GGCTCATCGT 1560
TCGATGAACT ATCGTCATCT GTTCGATCCG GAAACCAAAC TCATGCGCGG TAGAAATCAG 1620
GATGGTAGTT TCCGGACACC TTTTTCCCCT TTCAAATGGG GAGATGTATT CACGGAGGGC 1680
AATGCCTGGC ACTACACTTG GTCGGTCTTT CATGATGTGC AGGGGCTTAT CGACCTGATG 1740

GGAGGAGATC GCCCGTTCGT GTCTATGCTC GATTCGGTAT TCAATACTCC TCCTATGTTC 1800
GATGAGAGCT ATTACGGATT TGTCATCCAC GAAATCAGAG AGATGCAAAT AGCGGATATG 1860
GGCAATTATG CTCATGGCAA TCAACCCATA CAGCATATGA TATATCTGTA TAATCATGCC 1920
GGTCATCCAT GGAAAGCTCA GGAGAGACTA CGCGAAGTGA TGGGGCGGCT CTATCGTCCT 1980
ACTCCGGATG GGTATTGCGG CGATGAAGAC AACGGACAGA CTTCGGCTTG GTACGTTTTC 2040
TCTGCTTTAG GCTTCTATCC TGTTACACCC GCTACGGATC AGTATGTGCT CGGTTCGCCG 2100
ATTTTTTCCA AGGTAATACT CTCTTTTCCC GACGGACACA AAACGGTGTT GCATGCTCCG 2160
GCCAACAGTG CCGATACGCC TTACATCCGC TCGATCAGCG TAGAAGGAAA AGAATGGAGC 2220
TGCAATTACC TGACTCACGA ACAGCTTCGC TCTTCTGCAT CCATTCAATG GATGATGGAC 2280
ACGAAACCCA ATTATAATCG TGGTATGAAG GAAAGTGACA GACCTTATTC CTTCTCCACG 2340
GAGCAACAGC GTCGCGCTAA TCACAGTAAT 2370

(2) INFORMATION FOR SEQ ID NO:109

(i) SEQUENCE CHARACTERISTICS:
(A) LENGTH: 858 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...858

(xi) SEQUENCE DESCRIPTION: SEQ ID NO:109

ATTTGTGGCA GTAAAATGAA TCTATCGGGA TTACAATCTT TCACTATGAT GAAATCCATG 60
CGCAGCGTGC TGTTGCTACT CTTTCCATTG TCTTTGATCA CTGCTTTGGG CTGTAGCAAT 120
AACAAAGCTG CCGAATCGAA GTCTGTCTCT TTCGATTCGG CCTATCTCGA ACGCTACATC 180
CCTCTGCGGG CAGACATAGA TACGCCATCG CTGCATGTGA TGATCAGCTA CGTCTATCCT 240
TCGGGAGATG ATATGCTCAC AGAGATTTTC AACGGTTTGC TCTTCGGCGA CAGCCTGATG 300
GATTCCTCTT CCCCGGAGAA TGCCATGGAA GGCTATGCAC AGATGCTGGG AGAAGACTAT 360
CGCTCTAACA ATGCCGAAGC CAATCTGCAA GGGCTTCCTT CTGACCTTTT GGACTATATC 420
TACAAGCAGG AAAATACCAT CGCTTATTGC GATACGGGAT TGATCTCCAC GCGCATCAAT 480
ACATATACTT ACGAAGGCGG TGCACATACG GAGAATACAG TCCGGTTTGC CAACATCCTT 540
CGCACCACCG GCAAGGTGCT CGAAGAGCGA GATATATTCA AGATCGACTA TGCGGAAAGG 600
CTGTCCGCAC TCATCATAGG ACAATTGGTG CACGATTTCG GCAAGACCAC ACCTGCCGAA 660
TTGGATGCAA TAGGTTTCTT CAACGCAGAA GAAATACAGC CCAATGGCAA TTTTATGATC 720
GATGACAAAG GTCTCACATA CTGTTTCAAT GAGTATCAGA TAGCTGCTTA TGCCAGAGGT 780
GCTGTCTATG TCCGTCTCGG ATATGACGTA TTGGCTCCTT TGCTAAGGGA TGATTCCCCA 840
CTAAAGCGTT ACTTGCCG 858

(2) INFORMATION FOR SEQ ID NO:110

(i) SEQUENCE CHARACTERISTICS:
(A) LENGTH: 1134 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1134

(xi) SEQUENCE DESCRIPTION: SEQ ID NO:110

GGGATAATAT CTGTTCTTTC ACATGTGGTT GACCGTCCAC AATGGGGTGC TTCTCCCGAA 60
GCTGCTGGCA CGCATAGTGT GTATTCGATT CTACATCCCT CCGCCGGTAT TATCCGGATC 120
AGGTCTATGG GTATTATCTC AGCCTGCCGT ATCGCAATTC TTGCCGGCAA GCACCCCCGT 180
AGCGGTCTGT CGAGGGCAAA TGTAGGTATT CTTTCGTACA ATCCAGAAAA CACGCCCGAG 240
AAAAAGAGAA AACTGCAAGA AAAAAATGTT TTCCTCCAAA TCCGGCTCCG TCAATCATTT 300
AATAATTTGA TACCTTCGCT CCCATTTAGA ATCGATAACA CAAAAAAAAT CACTGAAATG 360
AAAAAAACTA CTTTGACAGG ATCGATATGT GCTTTACTCC TGTTTTTGGG TCTCTCGGCC 420
AATGCCCAAT CGAAGTTAAA GATCAAGAGC ATTGAGGCAG CTACCACTTT CAGTTCGGCC 480
ACGGCCGGAA ATGGTTTTGG TGGCAATATC TTCGGCATGG ACATGAGCAT ACGGATGAGG 540
GTACACCACA GCATTCTGCC CGAAGGGTTG GATTTTTCGG TAGGAATACA TGAAAGAAGA 600
GCACACTGGG AAGAGGCCGG AAGTCCGAAG CTCATGTATA CGAATGTCCC AAGTATCATT 660
GGTATTGTTG AAAAGGTAAT AGTCTTCGAA GACGCAGAAG ACTTTTTTGA CAAAAAAGCT 720
CTCGGCCGCT TCCTCATCAG TTTGGGGATA TCCTATACCA AGCATCTGGG AGCGTATTGG 780
GGATGGACCA ATGACGCCCA TATTCTTTTC TCACCGATAC CCAAGAGCAA GGTCCACTAT 840
GACACCTACA CAAGAGCTGG CAGTGACCTT GTACTTCAGT CCGAAGATGT TGCCACAGTG 900
AGCAATGGCT TTTCACCGGG GATCGGACTC AAAAGTTCTA TTTGGTGGAA AATGCCCATC 960
AAGAGCAAAT ATGATTTTCG CCTCGGTTTC AGCCTGGGCT ATGAGTATCT GAACCTGCTA 1020
TATCCGTATC GTAATTTCAA GCTGGATGGA AATAAGCCGC TTTCAGCACT ATCTCCTCGC 1080
ATGAACCACA TCGGCCATGT GGGCTTCAAC TTTACCGTGG GTCTTTGGAC TAAT 1134

(2) INFORMATION FOR SEQ ID NO:111

(i) SEQUENCE CHARACTERISTICS:
(A) LENGTH: 3807 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...3807

(xi) SEQUENCE DESCRIPTION: SEQ ID NO:111

GTAAAATGTA TGGGCAAGTA TAAAAGAGCT AAGTACCGCT ATTGGCTTTT TCCTTTCTGT 60
TCGGATTATT ATACCTTTGA GGGAGTTACT TTTTATGCGG CATCTGACGA TATGACAACC 120
AAGAAACCCC AAGCCATTTT AGACTTAGAG AAGGCCTATA ACATTGAAAT TCCTGATCTC 180
TCCTCACAAG AAGGGATAAG CTGGTCGGTA AATAGATATT TCAAGCAAGA TTCCTCCGGT 240
GCAGTCCTTG AGCTTTGCTT GCGAGAATGT CAGATAGAAA GCATGACTTG GCTTATTGAT 300
TTTCCTGCTC TAAAAAAGCT TGATCTATCG TATAACCAAA TCAGTAAGCT AGAGGGTCTA 360
GAACGTCTTA CTTCGTTAAC AAAACTTCGT CTAAGAAGTA ACCAAATCCG TAAACTAGAG 420
GGCCTGGATA GTCTCACCTC GCTAACAAAA CTTTCTCTCT CCGATAACCA AATCAGTAAG 480
CTAGAGGGTC TGGAACGTCT CACCTCGTTA GCGGAGCTTT ATCTTTTGGA TAACCAAATC 540
AGTAAACTAG AGGGTCTGGA ACGTCTCACG TCCTTAGCAA CGCTTGAACT ATCGGGTAAC 600
CAAATCCGTA AGCTGGAGGG TCTGGAACGT CTCACGTCCT TAGCAACGCT TGAACTATCG 660
GGTAACCAAA TCCGTAAGCT AGAGGGTCTG GAACGTCTCA CTTCGTTAAC AAAGCTTCGT 720
CTAAGAAGTA ACCAAATCAG TAAGCTAGAG GGTCTGGAAC GTCTCACGTC CTTAGCAACG 780
CTTGAACTAT CGGGTAACCA AATCCGTAAG CTGGAGGGTC TGGAACGTCT CACGTCCTTA 840
GCAACGCTTG AACTGTCGGG TAACCAAATC AGTAAGCTAG AGGGTCTGGA ACGTCTCTCT 900
TCGTTAACAA AGCTTCGTCT AAGAAGTAAC CAGATCAGTA AACTAGAGGG CCTGGAACGT 960
CTCACCTCGC TAACAAAACT TTCTCTCTCC GATAACCAAA TCAGTAAGCT AGAGGGTCTG 1020
GAACGTCTCA CCTCGTTAGC GGAGCTTTAT CTTTTGGATA ACCAAATCCG TAAGCTGGAG 1080
GGCCTGGAAC GTCTCACCTC GTTAACAAAG CTTCGTCTAA GAAGTAACCA AATCAGTAAA 1140
CTAGAGGGCC TGGATAGTCT CACCTCGCTA ACAAAACTTT CTCTCTCCGA TAACCAAATC 1200
AGTAAACTAG AGGGCCTGGA ACGTCTCACG TCCTTAGCGG AGCTTTATCT TTTGGATAAC 1260
CAAATCCGTA AGCTGGAGGG TCTTGATGGT CTTGCTTCCT TAACAAGGCT TAGTCTAAGG 1320
CGCAACCAAA TCAGTAAGCT GGAAGGACTA GACAGACTAA AGGTTTTGAG AAAACTTGAT 1380
GTTTCGGGCA ATGATATTCA ATCTATTGAT GATATTAAGC TATTGGCTCC GATTCTGGAG 1440
CAAACTTTAG AAAAACTGAG AATCCATGAC AATCCATTTG TTGCATCATC AGGCTTGATA 1500
CTCTCTCCTT ATGATAATCA TTTGCCGGAG ATTAAAGCTC TTCTTGAAAA AGAAAAAGAA 1560
AAACAGAAAA AGACTTCAGT TGAATATCAC CCATTTTGCA AAGTAATGCT ATTGGGAAAT 1620
CATTCTTCGG GTAAAACAAC ATTTCTTAGT CAATACGATA CAAATTATAC GTATCAGAAA 1680
AATACACATG TGTTGTCGAT ACATCGAAGC AATAACCCTA ATGCGATCTT TTACGACTTT 1740
GGGGGACAGG ACTATTATCA TGGGATTTAC CAAGCCTTTT TTACCACCCA ATCGTTATAC 1800

CTTCTCTTTT GGGATGCTAA GAAGGATCGA AACTTTGTGA GCGTAGATGA TAAAGAATAT 1860
CAGACTCTTA ATTTCAATCG CCCCTATTGG TTAGGACAGA TAGCCTATGC CTGCAATCGT 1920
TGTATGTCCG TTGGAGGAAA TCCTGATGGC AAGGACACAC CACAGACCAC AGACGATACA 1980
ATTATCATTC AGACTCATGC CGATGAAACG GGCGCTAAGC AGCAAACCTT AGGCTGTGCA 2040
GCCGAGAATG GAGTATTGGA AGAAATCTAT GTATCCTTAG AGCCCAAGGC GAATAGTGCC 2100
GTACATGCGC TCAACTATCT GAATGAGCGG GTGCGAGAAG TTGTCGCAAG CAGGAGTAAA 2160
TCAATTCAGA TCACAGAAAA AGATAAGGGA TTGTACGAAG CTCTTCCCAC AATCGCCGGT 2220
GATAATAAAC ACATCCCTAT CTCTCTCGAA GCTCTTGCGG CTCAATTGAA TAAGGGAAGA 2280
GCTGAAAATG ATCTTTACAC CATAGAGTAT CTACAGACCG AATTGAACCA GCTTAGTCTG 2340
CGAGGGGAGG TGCTTTACTA TCGTGAGAAT GAGAAGCTGA ACAATTATGT CTGGTTAGAT 2400
CCGGCAGCTT TTGTCCAAAT GATTCATGGA GAAATCCTCC AAAAAGACAA CATCAATAGA 2460
GGAACAGTTC CTAAAGACAT TTTTGAATGC AAACTGCATA ATCTAAGTTC CGGAAGTATA 2520
TTTGAAGAAG ATGGCCAAAA TGGTAATATG ATCTTGCAGC TATTATTGGA AGAGCTGATC 2580
GTATATGAAG ATAAGGACTG CTATGTGATA CCGGGCTATC TCCCTTTGCA TTCCGATGAC 2640
GAAGCCTATA AATGGCTTAC TTTGGGATTC GAGAGGCCCA ATTTTGTCCT CAAATTCGAA 2700
CGTTTTATCC CCTTTGGCCT GATCAACCAG ATTATAGCCT ACTATGGCCG GGAAGAAGGT 2760
GCTCTAAAGC GGTATTGGCG AGATCAGGTC ATCTTCACAG CAGGCCGTGA GATGGATAGG 2820
CAAACGCTTG AGCAAGAAGA AGAGAAAGAG GGTTTGCCCA AGACGAATGC CGAGGATTAT 2880
CAGATCTGGA TCAAGCTCGA CTTTACCGAC TTGGCCATAT CCGTATTCAT CAAAGAGCAG 2940
AGAAAGACAT CAGCTAAGGA TATGCAGCGG AAAGAGGCTA CTATCCTCAG TGATATGTTG 3000
GATATGTATT GGAACAATAT CCCTCCGAGG GAGCAAATAG GAGATAAGGA TACGGAGCAA 3060
ACGAGAAGCA CTATTCGTGA AACAAACAGA AAGAAGAGAC CCATCCAGGA TCTCTACCTC 3120
TCCTGTGCCC AAGCGGATAA AGATTTGACG GAGTCTCATT ATATCCATTT GGGCACGCTG 3180
GACGATGAAA GCAAGACTAC GGCGAGGATT GCAGCCTATC CGTTGAAGAA CGGCGTTATC 3240
GATAAAGAGC GGGTGCGAGA AGTATCGACT CGTCCCTACA AACATCTTTC CGTCAATAAA 3300
AATCTGGCTA CTGCAAAACA GATCTTTATT TCCTATTCCA AAGAGGATCA GACTGAACTG 3360
GAGACCTCTC TGCAATTTTT CAAACCCTTG GAGAAGAATG GTCAGATCGA GATCTACTAT 3420
GATAAGTTGA CTAAGTTTGA AACACCTATT CACCCTGAAA TAAGAAAGCG TATTGTCGAA 3480
GCCGACTGTA TAATCGCTTT GATCAGCCAA CGCTATCTGG CCACGGATTA CATCCTGGAT 3540
CATGAGTTGC CTGTATTTCG GGAGTATAAC AAGACCATAG TGCCGATATT GATCAAGCCT 3600
TGTACATTCG AAGACGATGA GTTCCTTCGG GAGAAATATT TTGCTCAGAA AGCTCAAATA 3660
ATCAATCTTG GAAAAGAGGG AAAAACCATT AAAGCTTATG ATAGTATTAC GGCATCAGCC 3720
CATCGTGATG AAAATTGGGT GGCAGTAGTC AGAGAGTTCA AAGAGAAGAT ATTAAGAATA 3780
ACAAAACAGG AGGTAAATAC AGATGAA 3807

(2) INFORMATION FOR SEQ ID NO:112

(i) SEQUENCE CHARACTERISTICS:
(A) LENGTH: 693 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...693

(xi) SEQUENCE DESCRIPTION: SEQ ID NO:112

AAGTTTATGA TGAAAAAAGC ATTTGTTTTC GTACTACTGG TTTGCCTATT CTCCTCGTTC 60
AGCAGTTCCG CCCAAACAAC GACGAACAGT AGCCGGAGTT ATTTTACAGG ACGAATCGAG 120
AAGGTGAGTT TGAACTTAGG GGTCCCCCCC GTAAGCACAG AGGTTTGGGG AATGACCCAT 180
GATGCGAACG GTCTCCCTTT CGAAATACCT ATCTCTTTCA GTCGTTTCAA CAGCCAGGGA 240
GATATAGCTA CCACTTATTA CATAGCGAAT AGCGAGGCAA CTTTGAATGA ATGGTGCGAC 300
TATGCACACC CGGGCGGCAT CGTGAGGGTA GAAGGTCGTT TTTGGAAAAT GACTTACAAC 360
ATACCAACCT ACAATGCAGT CTGCACCCGG ATTACATTCG AAAATCAAGA AATAGAAGGA 420
ACGATCGTCT TGATACCCAA GCCCAAAGTC TCGCTGCCTC ATGTGTCGGA ATCGGTGCCT 480
TGCATCCGAA CCGAAGCCGG GAGGGAATTT ATCCTTTGCG AAGAAGACGA CACCTTTGTG 540
TCTCACGATG GTAACGAAGT AACGATAGGC GGTAAACCTT TCTTGCTCAA TACCAACGTA 600
AAGATTGTGG GGGACGTATC TCAAAAGTAT GCCGTGGGGG TAGGAGAAAT TCGATTCCTG 660
CAGATTTGTG CCCAAACAGT ATCACAACAA AAA 693

(2) INFORMATION FOR SEQ ID NO:113

(i) SEQUENCE CHARACTERISTICS:
(A) LENGTH: 1689 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1689

(xi) SEQUENCE DESCRIPTION: SEQ ID NO:113

TCGCTAATCA ACAATTCAAG AGACTGGAGG GCTCCTGTCC GATTGAGTCT CAAAAAAAAG 60
ACAAAAACTA TGAAGACAAA AGTTTTACGC AAATTCGTGG TGGCGGCTTT CGCCGTCGCA 120
ACCCTCTGTC CTCTCGCCCA AGCGCAGACG ATGGGAGGAG ATGATGTCAA GGTGGTCCAG 180
TACAATCAGG AAAAACTGGT ACAAACGAGG ATGAGTGTGG CGGACAACGG ATGCATCTAT 240
GTAATGACCC ACAGTGGATA CGACACCGGC AATAGCAATG TGAAGATCTT CCGCTCCAAA 300
GACCAAGGTG CCACATACCA AAAGTTGAGG GATTGGGATC CATCGGATGA TTATCAGTTT 360
CAAGACTTCG ATATCGTGGT AACGGGTAAG AATGAATCCG ACATCAAGAT TTGGTCGGTA 420
GAGCTCATGA ATAAGCCCGG AGGATATAAG AGTAGAGTTG CGGTCTTCAG TCGCGATGCC 480
AACGCGCAGA ATGCGAAACT CGTGTATAAG GAAGACTTCT CCAATGTGCA GTTGTACGAT 540
GTGGATATAG CCTCCAACTA TCGTTCGCCT TCTTCTCTTA ACAATGGTGG CAACCCTTTT 600
GCTTTGGCTT TCGCTTACAC CGGCTTCAAC AATACGCACA AAATAAGTTT TGTGGACTAT 660
GTGTTCTCTC TGAATGGAGG GCAAAATTTC AATAAAAACT TACTCTTCAG TCAAGATGGA 720
GAGAAGAAAA TTGACAAGGT GGATCTCTCA TTGGGTAGCA CCTCTGAATC CATGGGTCAC 780
AATGCCTGGC CGCTAATGGG TGTGGTATTC GAAATGAATA AACAAGGGGG AAAAAGCGAT 840
ATCGGTTTCT TGTCGAACTT TGTCGACAAT GATCCCGAAT TTCAGTGGTC AGGCCCTATA 900
AAAGTGAGTG AAAGCGACAT GTCGTTCAGC CCCAAAATCC AAATGTTGCT GGACGAGGAT 960
AACAATACGA TCAATGGGGA GAGTTGCCAC AACTTCATGA TTACGTACAG CGATTATGAT 1020
TCTGAATATT CGGATTGGGA CATTCGGTAT GTATATCCCA AGAAATCGTT CAAGTATGAA 1080
AAAGGAAAAA CTCCGACTAT GGATGATCTG GTGGAAGCTT TCCTTACAGC TTCGTACCAG 1140
AGTGAGACCA ACTCGGGGCT GGGGTATGAC AAGAACGCCA ATCACTACCT GATTACATAT 1200
GCCAAAAAAG AAGAGAACGG TACGAACACG CTGAAATACC GCTGGGCCAA TTATGACAAG 1260
ATTCATAACA AAGATTTGTG GAGCGACACA TTTACGTATA CATCATCTGC CAATGCTCTC 1320
TACACACCTC AAGTAGACAT CAATCCGACC AAGGGTCTCG TGTGCTGGTC ATGGGTGGAA 1380
TATCTGCCGG GCAAACGGAT CGTTTGGTCT GATACGCAGT GGACCCATGC CAACGGTGTA 1440
GAAGACATCG TAATGCAAGA AGGCAGCATG AAGCTCTACC CGAATCCGGC TCAAGAATAT 1500
GCTGTGATTA GCCTGCCGAC GGCAGCAAAC TGCAAGGCTG TTGTTTACGA TATGCAGGGC 1560
AGAGTAGTCG CTGAGGCTTC TTTCTCCGGC AACGAATACA GGCTGAACGT GCAGCACTTG 1620
GCTAAGGGTA CGTACATACT CAAGGTCGTA TCCGATACGG AGCGTTTCGT AGAGAAGCTC 1680
ATCGTGGAA 1689

(2) INFORMATION FOR SEQ ID NO:114

(i) SEQUENCE CHARACTERISTICS:
(A) LENGTH: 2358 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2358

(xi) SEQUENCE DESCRIPTION: SEQ ID NO:114

ATCGTTTACC TTTGTCACTG TATGAACCAC AGACGATCAA AAACCATGCT GACGATCCGA 60
AACTTCCTCC TCTTTTGTTG TCTGTCGCTG ATAGCGTTTG CTGCCGATGC ACAAAGCTCT 120
GTCTCTTCGG GTAGACGACT GACAGAATAT GTCAATCCCT TTATCGGAAC GGCCAATTAC 180
GGTACCACGA ATCCGGGAGC AGTATTGCCC AATGGGTTGA TGAGCGTTAC CCCTTTCAAT 240
GTCAGCGGAT CGACAGAGAA TCGCTTCGAC AAAGATTCGC GTTGGTGGAG TGCGCCTTAT 300
TCGGCCGACA ATAGTTACTG CATCGGTTTC AGCCATGTGA ATCTGAGTGG AGTAGGCTGT 360
CCCGAACTGA GTGGAATACT GCTGATGGCC ACTTCCGGCA CATTCGATCC TGATTACTGC 420
TGCTATGGCT CTTCGCTCAG TCGAGAATAT GCGCGCCCGG GAGAATACAA GGCTGTATTG 480
GACAAATACG GTATAGATGC AGCCGTGACC GTAACCGAGC GGACTGCTTT GACCGAATTT 540
GCTTTTCCCG AAGGAGAAGG CCATATCCTG CTGAACCTGG GACAGGCCCT AAGCAATGAA 600
TCGGGAGCCT CTGTTCGATT CTTAAACGAC TCCACAGTCG TCGGCAGCAG GCTGATGGGG 660
ACGTTCTGCT ACAATCCGCA AGCAGTTTTT CGTCAGTATT TCGTACTTCA GGTGAGTCGG 720
CGACCGATCT CTGCCGGCTA TTGGAAGAAG CAGCCTCCTA TGACAGTGGA AGCCCAATGG 780
GATTCGACTG CAGGGAAATA TAAGCAGTAC GACGGCTACA AGCGTGAGAT GAGCGGTGAT 840
GACATCGGTG TCCGATTCTC GTTCAACTGC GATCAGGGGG AAAAGATCTA TGTACGATCG 900
GCCGTTTCAT TCGTCAGCGA AGCCAATGCG CTCTATAATC TGGAAGCGGA GCAAGAAGAG 960
GTGTTCAAAA GTGTCGGAGG GAATCCGGCC AAGGCTTTCT CCGCTATACG CTCTCGCGCT 1020
ATAGAGCGTT GGGAGGAAGC CCTCGGTACG GTGCAAGTGG AAGGAGGCAC ACCGGATGAA 1080
AAGACGATAT TCTATACCGC ACTCTATCAC CTGCTGATAC ATCCGAATAT CCTACAAGAT 1140
GCCAATGGAG AATATCCTAT GATGGGCAGT GGCAAAACGG GTAATACGGC TCACGACCGC 1200
TACACCGTGT TCTCTCTTTG GGACACGTAC CGCAATGTAC ACCCGCTGCT CTGCCTCCTC 1260
TATCCGGAGA AGCAGTTGGA TATGGTACGG ACACTGATCG ACATGTACCG AGAGAGCGGG 1320
TGGCTGCCGA GATGGGAGCT GTACGGACAG GAGACCCTGA CGATGGAGGG CGACCCCTCG 1380
CTTATCGTCA TCAATGACAC TTGGCAAAGG GGCCTTCGTG CTTTCGATAC GGCAACGGCC 1440
TATGAAGCCA TGAAAAAAAA TGCTTCTTCG GCAGGAGCGA CCCATCCGAT CCGTCCTGAC 1500
AACGACGACT ATCTCACCCT CGGCTTCGTA CCGCTTCGCG AACAGTACGA CAATTCCGTA 1560
TCGCATGCGC TGGAATACTA TCTGGCCGAC TGGAATCTGT CCCGGTTTGC CCACGCACTT 1620
GGGCATAAAG AAGACGCAGC TCTATTCGGA AAACGCTCGT TGGGCTACAG ACACTATTAT 1680
AATAAGGAGT ATGGTATGCT GTGTCCATTG CTGCCGGATG GATCATTCCT CACTCCTTTC 1740
GATCCCAAAC AGGGTGAAAA CTTCGAGCCT AATCCCGGTT TCCACGAGGG CAGTGCTTAT 1800
AACTATGCCT TTTTCGTTCC CCACGATATA CAAGGGCTTG CCCGGCTGAT GGGAGGAGCA 1860
AAGGTTTTTT CGGAAAGGTT GCAGAAAGTC TTCGATGAAG GATATTATGA TCCGACCAAC 1920
GAGCCGGACA TCGCCTATCC TTACCTCTTC TCCTATTTCC CCAAGGAAGC ATGGCGAACG 1980
CAGAAATTGA CCCGGGAGTT GATAGACAAA CATTTTTGCA ATGCTCCTAA CGGCTTGCCC 2040
GGTAATGACG ATGCCGGTAC GATGAGTGCT TGGCTTGTCT ATTCCATGCT GGGATTCTAC 2100
CCTGACTGTC CGGGCAGCCC CACCTATACA CTGACCTCGC CGGTATTCCC CCGAGTTAGG 2160
ATTCGGCTCA ATCCGCAGTA TTATCCTCAG GGGGAGTTGA TCATTACGAC CAATACAGAG 2220
AATCAACCGA CAGATTCCAT TTACATCCAT ACGGTTTCTC TTGGCAATAA AACACTTCCG 2280
CATGGAACAA GGCATATCAG CCATGCCGAT TTGGTGCGCT GCGGTCACCT CCGTTACGAA 2340
CTAAGCAATC GTCCTCGA 2358

(2) INFORMATION FOR SEQ ID NO:115

(i) SEQUENCE CHARACTERISTICS:
(A) LENGTH: 2442 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2442

(xi) SEQUENCE DESCRIPTION: SEQ ID NO:115

CCCCGTTTTC ATCCCGGAGG GACACACGCT CAGCTTTGCA GAAATGGGAG AAGAAACCAA 60
AAATCAAATC AGTCATCGTG CTTTGGCGGT GGCACAGCTC CGCGATTTTT TATTATGTGC 120
AAAATAAGAT TCAGCCTCTT GCAGGCTCTT GTCGTCTGCT TATTGTTCAC CTCTTTTTCT 180
CTCCAAGCTC AAGAGGAAGG TATTTGGAAT ACCCTCTTGG CTATCCACAA GACGGAAAAA 240
GCCGTAGAAA CGCCCAAGAA AGTCTTTGCC GTAGCCAACG GAGTACTTTA CTCGGTGGGC 300
AAAGAAGCTC CCCATGAGGC AAAGATCTTC GACCGTATCA GCGGACTCAG CGATACATCG 360
GTAAGCAGCA TAGCCTACTC CGAGCAACTA AAATCCTTGG TCATATACTA TGCATCAGGC 420
AATATCGACA TCTTGGACGA AGCAGGCCGT GTGACCAACG TACCTGCATT GAAAGACAAT 480
ATCGATCTGA TAGACAAAAC GCTCAATCGC CTTTTGATCG TAGGCAACAG GGCTTATTTG 540
GCAGGAGGAT TCGGCCTCTC CGTTCTGGAT GTCGCCGAAG CTCGCATACC GGCTACCTAC 600

GCCAAGGGAA CTAAGGTGAC CGATGTGGCT AAGTTGGACA ATGATCGCTT GCTGATGCTC 660
AAAGAAGGGC AGCTCTTCAT CGGAAAAGAG ACCGATAACC TGCAAGATCC GGCCGCATGG 720
ACAGCCTTGT CTTTGAATTT GCCGATGGGC TCGGTCACCG GTCTGGGCAT TGTCGGGGAA 780
GACATCTGTT TCCTGCTCGC CGATGGCCGT GTATATGTCG CTGCAAACCA ATCCTTTGAG 840
CCGGAGCTAT TGCTCTCTTC CTCCGCCGAT TCACGACTGT ATGTGACGGA TCGTGGTCTG 900
TTCATCTGTG CCGAGAATCG AATTTATTTC ATAGAAAAAG GTCGCAAAAC GACACAATTT 960
CCTATAGCCG ACGTCCTTGG TGTCGGTGCC ATGAACGAAA GCAATACGGC ATACATAGCA 1020
TTGGGAGAAG AAGGTTTGGC TTCACTTCTT CTCGCAGAGG GAAGTACGGC CGAAGCCATG 1080
CCTGTAGCAT TCGACGGACC GGGGGACAAT GATTTCTACG AGATGCGGTT TAGTCACGGA 1140
CGTCTGTATG CAGCCAGCGG ACTCTGGGGA ACAAACCTGA TGGGACATGC CGGTATGGTG 1200
AAGCTATACG ACGGCAACCG ATGGACTAAC TTCGACAAGA AGACCGTACA GGAACAGTTG 1260
GGCGGCGGAT TCAGTTTCAA TGATGCTATC GATATAGCTG TTTCCAACGG AGACCCCGAT 1320
CACTTTTTTG TCGGTACATG GGGAAACGGT CTGTTCGAAT TCAAGGATGG CAAAGCGATA 1380
GCTCGCTATT CGGGAAACGA AACTGCTATC GCAGAATGTA ATCCCGGAGA TGCCCGTGTG 1440
AAAGCGATTG CCTTTGACAA TAAGGGCAAC CTCTGGGGGA CGCTCGGTGC CGTAGGCAAG 1500
AACATCTTCA TGTACGATCC GCAGAGTAGC ACATGGCATT CTTTCAGCTA TCCGGATGTA 1560
GCCAATCTGG CCTCCTTCGG CAATATGATT ATCCTACCCA ACGGAGACAA ATGGGTAAAT 1620
ATCCTTCACC GTAGTGGCGG ATCCACGCGC AAAGGTGTCT TGATCTTCAA CGATCGGGGT 1680
ACACCGGAAA CGACTTCGGA CGACAGCCAT CTTTACGTCG AGCAGTTTGT CAATCGCCTC 1740
GGGGCAGCCA TAGGACATAA GACTATCTAT GCAATGGCCG TCGATCATAA CGGCTCTGTC 1800
TGGATGGGAT CGGATATAGG CATTTTCGGC GTCTACAATG CAGCCGGAGT ATTGTCCTCG 1860
ACTTCTACCC CTATCGCTGT TCGGCCGGTC GGAGGAGAAG AACCCAATTT GTACTATGTG 1920
CTGGACAAGG TGACGGTGAC AGACATCGTC GTGGACAAAC TCAATCACAA ATGGGTTGCC 1980
ACCCAAGGGA CAGGACTCTA TCTCCTTTCG GAAGATTGCA GTAAGATCCT CGCGCAATTT 2040
ACCGTAGAAA ACAGCCCTTT GCTTTCTAAC AACATACTAT CCCTGGCCTT AAATGACGAT 2100
AACGGACTGC TGTACATCGG TACGGCGGAC GGACTGATGA CGTTCCAAAC GGGTACGGGG 2160
AGTGGATCAG CTTCCGAACT GGACGGCGTC TATGTATACC CCAATCCGCT AAGGCCGGAA 2220
TATCCCGATG GCGTCACCAT TGCCGGACTG CAAGCCGGCT GTACTGTCAA AATCACCGAT 2280
ACCACCGGCA GACTGCTATA CCAGACTGAG AGCGTAACCA CCGAAGTCAA ATGGAATGCT 2340
CGAGGTGCCG ATGGCAATAG GGTAGCTTCG GGCGTATATG CCGTTGCAGT GTACGATCCG 2400
GTATCGAAAA AGTCCAAACT AATTCGCTTC GCAGTGATTC GC 2442

(2) INFORMATION FOR SEQ ID NO:116

(i) SEQUENCE CHARACTERISTICS:
(A) LENGTH: 3486 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...3486

(xi) SEQUENCE DESCRIPTION: SEQ ID NO:116

GCTATTTCTC AGATGAAACG AATACTTCCA ATAGTCGCAT TCCTTTCTCT CTTCCTTGCC 60
CTTGCTTTGC CTGCGAAAGC GCAACGAGCT ATGGGGAAGA CGGCCGACCG TTCGCTAATG 120
GCTTCGGGAC ATTGGGTCAA GATACGTGTC GATGCAAGTG GAGTGTATCG CCTTACGGAC 180
GAACAGCTCC GTGCCAATGG CTTCTCCGAT CCGTCCAAGG TAGGTGTGTT CGGTTATGGT 240
GGAGGGGTGC TTCCCGAAGA TCTGAGCCGG ATCACGACAG ACGATTTGCC TCCGGTACCG 300
GTACTCCGTC AGGGCAATGC GCTGTATTTC TATGCCGTGG GCCCGGTGAC ATGGTTCTAC 360
AATCCGGCCA AAACCACCAT GGAGCATACG GTGAATACAT ACAGTACGCA TGGCTACTAC 420
TTCCTGTCGG ATGCTGCCGG AGCACCTTTG CAGATGTCCC AATATACGGG TGGAGGTGCG 480
TCGGCCGAGG CTTTGATCGA CTACTACGAT GAGCTGATGC TCCATGAACA GGAATTGTAT 540
TCGCCCAAAG AATCGGGACG AGATCTGTAT GGCGAGTCTT TCAGTGCAGT CAATACGCGT 600
ACGGTCAAGT TCCCTTTGAG GGGCAACACC CGCTCGTCTG GCGAACTCGG TACCGTATTC 660
TCATACATAG CCAAGGCCAG ATCGGCCGGT GGCGGCCGTG AGATGTCGCT CTCGGCGAAT 720
GGCATTCTGA TCTTCAGCGA TCCTTTTTCC ATGACATCGA ATGAAGTCTC CAATTCCTAT 780
TTGGCCGGCA AGAAGCGTCG TCTCTATCAC AGTACGCCGA TGAACAGCTT GGTCAATGAG 840
TTGCGCTTGG ACGCGAACTA TAGCATGACA GGAGATGCGG TCAATCTGGA TTTCATAGAG 900
GTGGCTACAC AGAACGACCT CCGGTACGAT GGCGCACCCA TGCATATCAG GCGGTTTTCC 960
AATTTGCCCG TTTTGGGGGG CGAGTCCTGC CGGTTCGTTA TCAGTGAGGT GCCGGAGTCT 1020
CTGGTGGTTT TGCAGGCCAA TTCTTCCCTG ACAGCATCGC TTGTTCCCGT TAAGACTGTC 1080
GGGGATAAGA CCATTGAGTT CGTGGCTCCG CCGAAGGGTC AGGATCGTAG GACTATCAAT 1140

ACGTTTTATG CCGTGGACTT GTCACAGGCT TCTGCTCCGG AGATCCTCGG AGCGGTACCC 1200
AATCAAAACC TGCATGGAGA GGAAATCCCT GATCTGATCA TTGTCTCTAC TCAGGCGCTC 1260
CTCCTTGAGG CTGATCGACT GGCCACCTAT CGTAGAGAGA AAAACGGGCT GAAGGTTTTG 1320
GTCGTGTTGC AGGAACAGGT GTTCAACGAG TTTTCGGGTG GAACTCCCGA TGCTACAGCA 1380
TACCGCCTCT TTGCCAAAAT GTTCTACGAC AGATGGAAGG CAAATGCACC TGTGGGAGAC 1440
ACCTTCCCGA TGCAAATGCT TCTCTTCGGT GATGGGGCTC ATGACAACAG GAAGGTCTCC 1500
GTAGCTTGGC AGAAACCGTA TCTCCAACAA ACGGAGTTCT TGCTGACATT CCAAGCCGTC 1560
AATTCGACGA ACGTAAACAG TTATGTGACG GATGATTACT TCGGCTTGCT GGATGATCAG 1620
CCGGCCTCGG TCAATATCGG TTGGCGCAAT TATAATATGG CTGTAGGGCG ATTCCCCGTA 1680
CGTACTCCGG CCGAAGCTCG CATCGCAGTG GACAAGACCA TCCGATATGA GGAGGATCGA 1740
GAGAGTGGTG CCTGGCGTAT TCGTGCCTGT TTTGCGGCAG ACAACGGGGA CAAGCACGCA 1800
ACCGAGACTT CCCGTTTGAT CGATACCGTC AAGCGTTATG CTCCTGCCAT CATGCCGGTA 1860
CGCGCCTTTC AGGACGTATA TCCGCATGTC ATCGAGAACG GGTTGCACAG CATTCCGGGT 1920
GCAAAGAAAA AGATGCTGGA AACCCTTCAG TCGGGTATTA TCCTGCTTAA TTATGCTGGT 1980
CATGGCGGTC CTGCCGGATG GTCGGACGAG CATTTGCTGA CGCTCAACGA TATACACAAA 2040
TTCAATTATA AGCATATGCC CATTTGGATT ACTGCCACGT GCGACTTTGC CAACTATGAC 2100
AGTCAGACGA CCTCGGCAGG GGAGGAGGTT TTCCTCCATG AGAAGAGTGG CACTCCGATC 2160
ATGTTCTCGA CTACGCGTGT CGTTTACAAT ACGCAGAATG AGAAGATCAA TGGTTTTATG 2220
CTTCGGCGTA TGTTCGAGAA AGCTAAGGAT GGGCGTTATC GTACGATGGG CGAGATTATC 2280
CGATCGGCCA AACAGGGGAT GCTCAGTACT GTTTTCCCCG ATTCGATCAA CCAGTTGAGT 2340
TTCTTTCTGA TGGGTGATCC GTCCGTGCGT ATGAATCTTC CTACCCACAA AGTGCAATTG 2400
ACCGCAATCA ACGGGCAGGA TCCCGAAGGG CAGTATGGAA CTATTATGCT CAAGTCTTTG 2460
GAACGGGTAG CTCTGAAGGG TAAGGTAACC GATGAAAAGG GGACATTCGA CGAGACATTC 2520
AGTGGCAAGG TTTTCCTGAC CGTCTTCGAT GGCAGAAAGA AAATGACAGC TTTGGAAGAG 2580
GAGGGAAACG ATCTCTCTCT TGTATATTAT GACTATCCTA ACGTGATGTA TGCCGGTATT 2640
GCCGAGGTGA AAGACGGACT CTTCGAAACT TCGTTTATCG TACCCAAGGA TGTGAACTAT 2700
TCCGAGCACG AAGGCCGGAT CAATCTTTAT GCTTATAACG AGAGCACAAA GGCGGAAGCC 2760
ATGGGGGTAG ACTTCTCCAT CAGAGTCCAA CCGGGTATTC CTGATGAGGT AACGGAAGAT 2820
AATACACCGC CTGAAATCAT AAGCTGCTTC CTCAATGACA GTACATTCCG ATCGGGAGAT 2880
GAGGTTAATC CTACTCCTCT GTTTATGGCC GAAGTATTCG ACTTGAATGG AATCAATATC 2940
ACGGGTAGCG GAGTAGGGCA TGATATTACG CTTTGTATCG ATGGCCGTGC CGACCTGACC 3000
TACAACCTCA ATGCATATTT CACAAGTTCG GCTACGGATG CAGGTGTGGG CACTATTCTC 3060
TTCATGATAC CGGCTTTGGC CGAAGGAGAT CATACTGCCC GACTGACGGT TTGGGACATT 3120
TTCAATAATG CCGTCCATCA TGACTTTTCA TTCAGAGTGG TAGATGGCAT TGCTCCGGAT 3180
GTGGCTGATG TGATTCTATT CCCGAATCCG GTACGCGAGA GTGCTACGTT CCGAATCTTC 3240
CACAATCGCC CCGGAAGCGA TTTGAACGTG GCCGTGGAGA TCTATGACTT CACCGGTCGT 3300
CTTGTGAACA GTTTGCCAGT CAAGACCTAT TCGTCTTCCT ACGGAGAACC TATAGAGATC 3360
AAGTGGGATC TGACCTCCAA ATACGGAGTG AAGATCGGAA ACGGATTCTA CCTCTATCGT 3420
TGTGTGGTGA ACTCTCCCGG AGGACAGACG GCCTCCATGG CCAAGAAAAT GATCGTGGTA 3480
GGACAA 3486

(2) INFORMATION FOR SEQ ID NO:117

(i) SEQUENCE CHARACTERISTICS:
(A) LENGTH: 2919 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2919

(xi) SEQUENCE DESCRIPTION: SEQ ID NO:117

TTTTACACCG GAATTGACTT GCATATAGAA AGCCAAATGA AGAAACTTTT CCCATTATTA 60
TTACTCATAT TGTCGATTTT GGTCGGATGT GGAAAAAAAG AAAAACACTC TGTAACTGAA 120
ATCGCCCGAG AGAAAAAGCG TATTACTGCA TTGCTGTACG AAAAAGAACT CCCCACTGAT 180
TCTGTTAAAC AGCTTTACGA GAACAGCGTA CAGAACAAGA ACCTTGTGGG ACAAATGTTA 240
TTCGCGATCG AGGTCGGCAA ACGGATGCGT AATATGTCCC AATACACAGA TGCGATGCTA 300
TATCACCAAA AAGGGTTGAA CGCTGCATTG AATCTAAGGG ACACCATCGT AGCCGCACAA 360
GCATGGAATC ATCTCGGAAC GGATAGCCGT CGTATCGGTG CTTTGGCAGA AGCTTCGGAT 420
TATCATTACA AGGCTCTTTC CTTGATAGAA TCTTTTAGCG GAAACCAGAA TAGGCCTGCG 480
ATCAAAGCCA GATCGGCGGC CCTGAACGGC ATCGGCAACA TCAATCTTGA GTTAGGATAC 540
CATGATGAGG CCGAAAAGAA TTTCCTGAAA GCACTGCAAG GTGAGAAAGA ACTCGACAGT 600

CCTCTCGGGC AAGCTATCAA CTATGCGAAC CTCGGACGTA TCTATCGACA ACGCAAAGAA 660
TACGACAAGG CTCGTACCTA CTTCCTCTTG TCTCTGGAGC AGAACAATAT GGCAGAGAAT 720
CTGATGGGTA TCGGACTCTG TAGCATCAAT CTCGGAGAAG TAGACGAAGA AAAAGGGGAT 780
TATCAAAAGG CTTTGCAAGA GTATGCCACG GCATACAAAC TGATGGAACA GTTGTCCGAT 840
CGATGGCACT GGCTGAATTC CTGTATCCCG ATGGCACGTA TCAATCTCAA ACAAGGTAAC 900
GAAAGGCTCT ACCAGCATTT CATTTCTTTG GCCGAAGGGA CTGCGAAAGA AATTAATTCG 960
ACTTCACATC TGATAGAAAT ATACAATCTT CAATACGAGA ATCTCGAGCG TAAAAAAGAA 1020
TACAAACAAG CCCTCGAAGC ATTCTGTCTG AGCAAGACGT TGAGCGACAG CATGTCCATT 1080
GCGCACAAGG TCAGCAGCAT ACAAGAAACG CGATTCAACT ACGAACGAAA CAAGTCCCAA 1140
AAAGAGCTTG AAGAAATACA GCAAGTAAGC AAGGCAAAAC AAGAGAAATC GAAGTTTATC 1200
CTCTTGAGCA CTCTTTTTGC CCTTTTCATC TCGATTCTTT TGATTTCTGT TCTGACATAT 1260
GCATACCGTC AGGGCAAGAA GCATAACAAG CTGATCAAAG AGACGGATAA ACTTCGCTCC 1320
GGCTTTTTCA CCGGTATTAC ACACGAATTT CGTACGCCTA TCACCGTCAT ACAAGGTTTG 1380
AATGAGAAAA TGAGTTCAAG TCCTGATCTC CAAGCATCGG ACAGAACCGA GCTGCACAAG 1440
ATAATAGACA GACAGAGTAG CCATATGCTG AATTTGGTGA ACCAGCTGTT GGATATTTGC 1500
AAGATCAGAA GCGGAGTATC CACGCCCGAA TGGCGCAATG GCGACATCGT CTCCTTCGTA 1560
CAGATTCTCA TCGATTCGTT TGCACCATAC GCACAGGCTC AAGACATAAC CTTGGAGCTA 1620
CAACCCGAGA GCAAACCTAT TGTCGTGGAC TTCGTCCCCT CCTACTTGCA AAAAATCATA 1680
TCCAATCTTT TGTCCAATGC CATCAAGTAT TCTTTAGCCG GAGGGAGAGT GGTCATATCT 1740
CTGGCAAAAA CCAAGAATGA AAAAAATCTG ATCATACGCG TTGCAGACAA TGGCATAGGA 1800
ATAGATAAAA CTGATCAGGC TCATATCTTC GACATCTTCT ATCGAGGACA CTCCGCTACC 1860
GAAAAGCATG GATCAGGCGT CGGACTCTCG TTTACCAATA TACTGGTCGA AAACCTTCGA 1920
GGTACGATCA AAGTGGAAAG CCAGCCGGGG AAAGGAAGTG CCTTCACCAT CAGTATTCCT 1980
ACACAAAACC AGTCCTCTTC GGCAGAGATT CTTCCTTGGC TACCCTCCTC CGATGACATT 2040
GTCATGCCTG TCCACATCGC GCCCGATGAC TCACCGACAT CTCCGATGGT AGCAGCTCTG 2100
AATCATCGCT TCGAGGACGA ACGTCCGACC ATACTGCTCG TCGAGGACAA TAAGGATATC 2160
AACCTGCTCG TCAAACTACT CCTTTGCGAT CGCTACAATG TGCTATCCGC CGCAAACGGA 2220
AAAGAGGGTA TAGCCCTCGC TACCGAGCAT ATTCCCGACA TTATCATTAC GGATATTATG 2280
ATGCCGATAA TGGATGGGAT AGAAATGACA ATCCGGATGA AGCAATCGCC TCTGCTCTGT 2340
CACATTCCCA TTGTCGCTTT GACGGCCAAG AGTACCGAAC AGGACAGATT GGAAGGAATC 2400
AAAAGCGGTG TAGTCTCTTA TCTATGCAAG CCATTCTCTC CGGAGGAGCT TTTGATGCGG 2460
ATCGAGCAGC TTCTGAAAGA CCGTGAGTTG CTCAAGAAGT TCTATATGCA AAAACTCATG 2520
CTGGATCGGA AGCCGGAGGA GGAGCCTCAA CCGATAGATG ACAGCAGTAT GCAGTTTCTC 2580
CTTGCTGCCA AAGATGCAGT GTCCGGTGGA ATCAAACAAA ATCCGGATTT TTCCGCTCAA 2640
GACTTGGCCG AAAAAATGTG CATGAGTCCA TCCCAACTCA ACAGAAAGCT CACGAGTGTC 2700
GTAGGTTGCT CCACCATCGG CTACATACAG CAGATCAAGA TAAAATTGGC CTGCAAGCTC 2760
CTTGCCGATG AGAGCAAAAA CATCTCCGAC ATTAGCATTG AGGCAGGCTT TTCGGATCCG 2820
GCTTACTTCT CTCGCACCTT CAAACGCTAC ATGAACTGCT CTCCCTCCCA ATATCGGCAA 2880
AAACTCCTTG CCATGCCGGG GAGCGACAAG GAGACAGTT 2919

(2) INFORMATION FOR SEQ ID NO:118

(i) SEQUENCE CHARACTERISTICS:
(A) LENGTH: 1689 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1689

(xi) SEQUENCE DESCRIPTION: SEQ ID NO:118

CATCATAAAA CATATCAAAC AATGAAAAAG CTTTTACAGG CTAAAGCCTT GATTCTGGCA 60
TTGGGACTCT TCCAACTGCC CGCAATCGCC CAAACGCAAA TGCAAGCAGA CCGAACAAAC 120
GGTCAATTTG CAACAGAAGA GATGCAACGA GCATTCCAGG AAACGAATCC CCCTGCAGGT 180
CCTGTGCGTG CTATCGCTGA GTACGAACGC TCTGCAGCCG TTTTGGTACG CTACCCGTTC 240
GGTATCCCGA TGGAATTGAT CAAAGAGCTG GCCAAGAACG ACAAGGTGAT TACCATTGTG 300
GCGAGTGAAA GCCAAAAAAA CACCGTTATA ACCCAGTACA CCCAAAGCGG TGTGAATCTC 360
TCTAATTGCG ATTTCATCAT TGCGAAAACT GACTCTTACT GGACACGCGA CTATACCGGT 420
TGGTTCGCAA TGTACGATAC GAACAAAGTA GGTCTCGTGG ACTTTATTTA TAACCGCCCT 480
CGTCCTAACG ATGATGAATT CCCCAAATAC GAAGCACAAT ATCTGGGCAT CGAGATGTTC 540
GGGATGAAGC TCAAGCAGAC CGGTGGCAAC TACATGACGG ACGGATATGG ATCCGCTGTG 600
CAGTCACATA TCGCATATAC GGAGAACTCC TCTCTGTCTC AAGCTCAAGT AAATCAAAAG 660

ATGAAAGACT ATCTCGGCAT CACACATCAT GATGTGGTAC AAGATCCGAA CGGCGAATAT 720
ATCAACCATG TGGACTGTTG GGGCAAGTAT TTGGCACCGA ACAAAATCCT CATCAGGAAA 780
GTGCCTGACA ATCACCCTCA GCACCAAGCC CTGGAAGATA TGGCAGCCTA CTTCGCAGCA 840
CAGACCTGCG CATGGGGAAC GAAGTACGAG GTATATCGCG CTTTGGCCAC CAATGAACAA 900
CCGTACACGA ACTCTCTGAT TCTGAACAAC AGGGTATTTG TTCCTGTCAA TGGCCCCGCC 960
TCCGTGGACA ACGATGCTCT GAACGTCTAT AAGACGGCAA TGCCCGGTTA CGAAATTATA 1020
GGTGTCAAAG GGGCTTCAGG AACACCTTGG TTAGGAACAG ATGCCCTGCA TTGTCGTACT 1080
CACGAGGTAG CGGATAAGGG CTATCTCTAT ATCAAGCACT ACCCGATACT GGGCGAACAG 1140
GCAGGCCCTG ATTATAAGAT CGAAGCAGAT GTCGTCTCAT GCGCCAATGC TACTATCTCG 1200
CCGGTACAAT GTTACTATCG TATCAATGGT TCCGGTAGCT TTAAGGCTGC TGATATGACG 1260
ATGGAATCAA CAGGTCACTA TACTTATAGC TTTACAGGTC TTAACAAGAA TGATAAGGTA 1320
GAATACTATA TCTCTGCCGC TGACAATAGT GGTCGCAAAG AGACTTATCC CTTTATCGGC 1380
GAACCTGATC CTTTCAAGTT TACGTGTATG AACGAAACCA ATACATGTAC TGTGACCGGA 1440
GCTGCCAAAG CTCTTCGTGC ATGGTTCAAC GCCGGTCGTT CAGAACTGGC TGTTTCGGTA 1500
AGTTTGAATA TTGCCGGCAC ATATCGGATA AAGCTTTATA ACACCGCAGG AGAAGAAGTC 1560
GCTGCAATGA CCAAGGAATT AGTAGCAGGG ACGAGTGTCT TCAGTATGGA TGTGTATTCT 1620
CAGGCTCCGG GCACATATGT TCTGGTTGTT GAAGGAAATG GAATCCGTGA GACAATGAAA 1680
ATTCTCAAA 1689

(2) INFORMATION FOR SEQ ID NO:119

(i) SEQUENCE CHARACTERISTICS:
(A) LENGTH: 1311 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1311

(xi) SEQUENCE DESCRIPTION: SEQ ID NO:119

ACCACAAATA GAAAACCAAA TACTAATATG AAACTTTCAT CTAAGAAAAT CTTAGCAATC 60
ATTGCATTGC TGACGATGGG ACATGCTGTG CAGGCACAGT TTGTTCCGGC TCCCACCACA 120
GGGATTCGCA TGTCTGTCAC TACAACCAAG GCCGTAGGCG AAAAAATCGA ATTGTTGGTT 180
CATTCCATAG AGAAGAAAGG CATCTGGATC GATCTCAATG GGGATGCCAC TTACCAACAA 240
GGAGAGGAAA TAACCGTATT CGATGAGGCA TACCACGAAT ACACGATCGG GACGCAAACC 300
CTCACTATCT ATGGTAATAC GACCCGATTG GGCTGTCGAT CTACCGGTGC AACGGCTGTC 360
GATGTAACGA AAAACCCTAA TCTGACCTAT CTCGCATGCC CGAAAAATAA TCTGAAATCA 420
TTGGACTTGA CGCAAAACCC AAAGCTGCTG CGAGTTTGGT GCGACTCTAA CGAAATAGAA 480
AGTTTGGACC TGAGTGGCAA TCCGGCTTTG ATCATCCTCG GCTGTGACAG GAATAAGCTG 540
ACTGAGCTGA AGACCGATAA CAACCCCAAG TTGGCCTCTC TTTGGTGTTC TGATAATAAC 600
CTGACGGAGT TGGAACTCAG TGCCAATCCT CGTCTCAATG ATCTTTGGTG CTTCGGTAAT 660
CGGATCACGA AACTCGATCT GAGTGCCAAT CCTCTATTGG TAACACTTTG GTGCAGTGAC 720
AATGAGCTTT CGACCTTGGA TCTTTCCAAG AATTCGGACG TTGCTTACCT TTGGTGTTCA 780
TCGAACAAAC TTACATCCTT GAATCTGTCG GGGGTGAAGG GACTGAGTGT TTTGGTTTGT 840
CATTCCAATC AGATCGCAGG TGAAGAAATG ACGAAAGTCG TGAATGCTTT GCCCACACTA 900
TCTCCCGGCG CAGGCGCTCA GAGCAAGTTC GTCGTTGTAG ACCTCAAGGA CACTGATGAG 960
AAGAATATCT GTACCGTAAA GGATGTGGAA AAAGCTAAAA GTAAGAACTG GCGAGTATTT 1020
GACTTCAACG GTGATTCTGA CAATATGCTT CCATACGAAG GAAGTCCGAC ATCGAACTTG 1080
GCAGTAGATG CTCCCACTGT CAGGATATAT CCCAATCCGG TAGGAAGATA TGCGCTCGTC 1140
GAGATCCCCG AGTCTCTTTT AGGGCAGGAA GCTGCTTTAT ACGATATGAA TGGGGTAAAA 1200
GTCTATAGTT TCGCGGTAGA GTCTCTTCGT CAGAACATTG ACCTGACACA TCTTCCCGAC 1260
GGCACTTATT TCTTCCGTCT CGATAACTAT ACCACTAAGC TCATCAAACA G 1311

(2) INFORMATION FOR SEQ ID NO:120

(i) SEQUENCE CHARACTERISTICS:
(A) LENGTH: 954 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...954

(xi) SEQUENCE DESCRIPTION: SEQ ID NO:120

AATCATTTGA AGACAAATAT CAAGATGAGA AAAACAATAA TTTTCTGCTT GTTGCTCGCC 60
CTATTTGGCT GTTCTTGGGC ACAAGAAAGA GTCGATGAAA AAGTATTCTC CGCAGGAACA 120
AGTATTTTTA GGGGCATCCT TGAAAAGGTG AAAGCACCGC TTATGTATGG AGATCGTGAG 180
GTATGGGGTA TGGCTCGTGC GAGCGAGGAT TTCTTTTTTA TACTTCCCGT TACGGATGAC 240
CTCACTCCCG TGCTTTTCTA TAACCGTCTT ACAAACGAAC CCTGCTTTGT GTCAGACCAA 300
GGAATAACTG AGTATTTCAA ATTCGCTCAA GAAGGTGATT ACATTGAAGT CGAAGGAAGC 360
TCTGTATTCA TGGCGAATCT TTTGTACTAT CGTTTTTTCC CGACAAGAAT TACCTCCTAT 420
AATGCTCCCA TTGAAGGTGT TGTGAGCAAG ACGGGAAATC CTGCTTTTAC AATCCCGATG 480
CTCCCGGGGG TTTCTGATTG CATAGAAATC TCAAACAACC GCAAAGTCTT TCTGACCAAT 540
CAATTAGGGG TTGTAAACAT CACTGACGGG ATGGAACCTC CGATTATTGC CGGAGTCTCT 600
GCTTCCTATG GATCTTCCGT CCGGGTGTAT GGTCATGTCT CACAGCGGTG GGACATCATA 660
GGCCATTGCT ATTTGGATAT CTACCCAACC AATTGCTATC CGCTCAGCAC GAAACCCGTT 720
GCAGGAGACG ATGAGGTTTT TGTCAAACAA CAAGGCAGGC AAATAGAGAT CGATAGCAAC 780
AGCCCCATAG TCCAAGTGGT CGTATACGAT CTTGAGGGGA AAAGTGTTTT TCGCAAAAGA 840
ATGACCGAAA ACGCTTATAC CCTATCCTTT AGAGCACCCA TGCTCGGCTT TATGACCATC 900
ATGATCGAAA CACAAAATTC GATTATCAAT AAAAAACTTA ATGTTACACA GCTA 954

(2) INFORMATION FOR SEQ ID NO:121

(i) SEQUENCE CHARACTERISTICS:
(A) LENGTH: 1383 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1383

(xi) SEQUENCE DESCRIPTION: SEQ ID NO:121

AGACGGGCAG TTAAAATCCG ATCACCTCCC CATATCCACT CATTATTTGT ACGTAAATGT 60
CTTTTTTCAG ACTTTAAATA CTTATATTTA TCCCGAAAAA TTACCCAAGA GAGATTGGGT 120
CGATTGTCGA TCCGTCTTAA GTCATACAAT CCAATAATCA TTATTGAAAT GAAAAAAACA 180
ACCATTATTT CTTTGATTGT CTTCGGTGCT TTCTTTGCAG CCGTGGGCCA AACCAAGGAC 240
AATTCTTCTT ACAAACCTTT TTCGAAAGAA GATATTGCCG GAGGAGTTTA CTCTCTCCCG 300
ACTCAAAATC GTGCGCAGAA GGACAATGCC GAGTGGCTTC TTACAGCGAC CGTCTCCACA 360
AACCAGTCTG CAGATACTCA CTTTATCTTC GATGAGAACA ACCGCTATAT CGCTCGTGAC 420
ATAAAAGCCA ATGGGGTAAG AAAATCCACG GACTCCATTT ACTACGATGC CAACGGGCGA 480
ATATCGCATG TGGATCTTTA TATCTCGTTC AGTGGCGGAG AGCCTGCACT CGACACCCGA 540
TTCAAGTACA CCTATGATGA CGAGGGAAAG ATGACCGTGA GGGAAGTATT CATGCTGGTA 600
ATGGATCCGA ATACACCTAT CTCACGCTTG GAATATCATT ATGATGCACA GGGCAGACTG 660
ACCCACTGGA TTTCTTTTGC TTTCGGGGCA GAATCCCAAA AGAATACGTA TCACTATAAT 720
GAAAAAGGTC TGTTGGTCAG CGAAGTGCTG AGCAATGCAA TGGGGACAAC CTATTCAGAC 780
ACCGGCAAAA CGGAATACAG CTATGACGAT GCAGATAATA TGGTGAAGGC CGAGTACTTC 840
GTCGTCCAGC AAGGAAAGGC ATGGCAAGTA CTCAAAAGAG AGGAATACAC CTATGAGGAC 900
AATATCTGCA TACAATATTT GGCTATTAAC GGTACCGACA CAAAGGTGTA CAAGCGAGAC 960
ATCGAGAGCG ATAAGTCCAT CTCCGCAAAT GTCATTGACA TTCCGTCAAT GCCGGAACAG 1020
ACCTGGCCTA ATATGTACGG ATTCAACGCA AAGCGACTGA AAGAGACTTA TTCCTCCTAC 1080
GAAGGAGATG TGGCTACTCC TATATTCGAC TATATCTATA CGTACAAGGC TCTTACCTCA 1140

ATGGCAACAC CTTCGACAGA AGCTCAGGTA GCAGTCTATC TCAATCCGTC AACGGACCGG 1200
TTAGTGATTC TGGCCAACGG CATCACACAT CTGAGCATGT ACGACTTGCA GGGTAAGCTT 1260
ATCCGTGATT GTGCCTTGAG CGGCGATAAG GTGGAAATGG GTGTCGGATC TTTGACCAAA 1320
GGGACATACC TGCTTAAAGT GAATACGGAT CAGGGAGCCT TTGTGAGAAA AGTCGTGATT 1380
CGA 1383

(2) INFORMATION FOR SEQ ID NO:122

(i) SEQUENCE CHARACTERISTICS:
(A) LENGTH: 1353 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1353

(xi) SEQUENCE DESCRIPTION: SEQ ID NO:122

ATGGCAAAAG TTATAAAAAC AAAAAAAGGC CTTGCACTTA ATCTGAAAGG AAAACCGCTG 60
CCCGAGATGC TGGCCGAACC GGCCCAAAGT CCTACTTACG CGGTCGTGCC CGACGATTTT 120
GAAGGTGTTA TCCCCAAGGT GACGGCTCGT CCGGGGGATA AGGTGCGTGC CGGCTCAGCA 180
CTGATGCACC ACAAGGCATA TCCGGAGATG AAGTTTACAA GTCCGGTTAG CGGCGAAGTG 240
ATCGCGGTGA ATCGCGGTGC CAAGCGCAAG GTGTTGAGCA TCGAGGTGAA ACCGGACGGA 300
CTGAACGAAT ACGAGTCATT CCCTGTCGGG GATCCGTCTG CCCTCTCTGC CGAACAGATC 360
AAGGAGCTTT TACTGTCGAG CGGTATGTGG GGTTTTATTA AGCAACGTCC TTACGACATA 420
GTGGCTACAC CGGATATAGC TCCACGCGAC ATTTATATTA CTGCCAACTT TACTGCACCA 480
TTGGCTCCGG ACTTCGATTT CATCGTTCGA GGAGAAGAAC GCGCCCTGCA GACTGCCATC 540
GATGCCTTGG CCAAACTCAC GACAGGAAAG GTGTATGTGG GCCTGAAGCC GGGTTCATCT 600
CTGGGCTTGC ACAATGCAGA AATCGTAGAA GTACACGGAC CTCATCCGGC AGGTAACGTG 660
GGCGTGCTGA TCAATCATAC GAAGCCAATC AATCGGGGCG AAACGGTGTG GACGCTCAAG 720
GCTACCGACC TGATCGTGAT CGGACGTTTC CTGCTTACGG GCAAAGCCGA TTTTACCAGA 780
ATGATTGCCA TGACCGGCTC AGACGCTGCA GCTCACGGAT ACGTCCGTAT TATGCCGGGT 840
TGCAATGTCT TTGCTTCCTT CCCCGGCCGA CTGACAATAA AGGAATCTCA CGAGCGTGTG 900
ATCGATGGCA ATGTGCTGAC CGGTAAGAAG CTCTGCGAGA AGGAGCCTTT CCTGTCAGCC 960
CGGTGTGACC AGATCACGGT GATCCCCGAA GGCGACGATG TGGACGAACT CTTCGGGTGG 1020
GCTGCACCCC GTCTCGATCA GTACAGCATG AGCAGAGCTT ATTTCTCTTG GTTGCAGGGG 1080
AAAAACAAAG AGTACGTACT CGATGCCCGG ATCAAGGGTG GCGAACGTGC TATGATCATG 1140
AGCAACGAGT ATGACCGCGT TTTCCCGATG GACATCTATC CGGAGTATTT GCTCAAGGCT 1200
ATTATAGCAT TCGACATCGA CAAGATGGAG GACTTAGGCA TATATGAAGT GGCTCCGGAG 1260
GACTTTGCCA CTTGCGAATT TGTGGATACA TCCAAGATCG AGCTGCAGCG TATCGTTCGC 1320
GAGGGCTTGG ATATGCTCTA TAAGGAAATG AAT 1353

(2) INFORMATION FOR SEQ ID NO:123

(i) SEQUENCE CHARACTERISTICS:
(A) LENGTH: 585 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...585

(xi) SEQUENCE DESCRIPTION: SEQ ID NO:123

ATGGATAAAG TGAGCTATGC TCTGGGATTG AGCATCGGTA ATAATTTCAA GTCTTCGGGC 60
ATCGACAGCG TCGTTATGGA TGATTTCATG CAAGGTCTGT CTGATGTACT GGAAGAAAAA 120
GCCCCTCAGC TCTCGTATGA CGAGGCCAAG CGCGAAATAG AGGCGTATTT CATGGATTTG 180
CAGCAGAAGG CTGTCAAACT GAACAAAGAG GCCGGAGAAG AATTCCTCAA GATAAATGCA 240
CACAAGGAAG GTGTGACGAC CTTACCGAGC GGCTTGCAAT ACGAAGTCAT TAAGATGGGA 300
GAGGGCCCGA AACCCACCCT TTCGGACACG GTAACCTGTC ATTATCACGG TACGCTCATC 360
AACGGTATCG TTTTCGATAG CTCTATGGAC AGGGGAGAAC CGGCCAGTTT CCCTCTAAGA 420
GGAGTTATAG CCGGCTGGAC GGAGATTCTT CAATTAATGC CTGTAGGATC CAAGTGGAAA 480
GTAACTATAC CGAGCGATCT GGCGTATGGA GATCGTGGTG CCGGCGAACA TATCAAACCG 540
GGTAGTACGC TCATTTTTAT AATCGAATTA TTGAGTATCA ACAAA 585

(2) INFORMATION FOR SEQ ID NO:124

(i) SEQUENCE CHARACTERISTICS:
(A) LENGTH: 819 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...819

(xi) SEQUENCE DESCRIPTION: SEQ ID NO:124

ATGAAAAAAG CATTACTTAT TGGTGCTGCT CTTTTGGGAG CAGTCAGTTT TGCAAGTGCT 60
CAGTCTTTGA GCACAATCAA AGTACAGAAC AATTCAGTAC AGCAACCTCG TGAGGAAGCC 120
ACTATTCAGG TTTGTGGAGA ATTGGCAGAG CAAGTTGACT GCATTGGGAC AGGTAATTCT 180
GCAATCATAG CCGCTGCAGC GAAATTTGAA AGCGATGATC TCGAAAGCTA TGTTGGCTGG 240
GAGATCATGA GTGTTGATTT CTTCCCTGGA TATAAAGCGT GCAAGTACAC ATCTGCAGTC 300
TGGGCTGATG ATATGACCAT TTTGGGCCAA TCAGAAGATA GTGATCCCGA AATGCAGACT 360
ATCAACAATC TTGCTCTCAA GACTAGTGTC AAGATTGAAG CCGGCAAGAA TTACATAGTT 420
GGTTATATTG CTAATACCGC AGGTGGACAT CCTATCGGAT GTCATCAGGG CCCTGCCGTT 480
GATGGTTATG GAGATTTGGT TTCTATATCA GAAGATGGTG GTGCTACTTT CCCTCCGTTC 540
GAATCTCTTC ATCAAGCAGT TCCTACCTTA AATTACAACA TCTATGTCGT TGTTCATTTG 600
AAGAAGGGTG AAGGTGTTGA GGCTGTTCTT ACCAACGACA AGGCTAATGC TTATGTTCAG 660
AATGGCGTTA TCTATGTAGC CGGAGCTAAT GGTCGTCAGG TATCTCTGTT CGACATGAAC 720
GGTAAGGTTG TTTATACCGG CGTTAGCGAA ACGATTGCAG CTCCTCAGAA GGGCATGTAT 780
ATCCTCCGTG TAGGTGCTAA GAGCATCAAG CTGGCTATC 819

(2) INFORMATION FOR SEQ ID NO:125

(i) SEQUENCE CHARACTERISTICS:
(A) LENGTH: 1662 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1662

(xi) SEQUENCE DESCRIPTION: SEQ ID NO:125

ATGCCAAGAA TTATGAAATT AAAAATTGCA CTCAGACTGC TGCTGGCGAC TTTTGCCATA 60
GTTTTATTTA GCCCTCTGGC CAAGGCCCAG ATGGATATTG GTGGAGACGA TGTATTGATC 120
GAGACGATGT CCACCCTATC AGGATATTCA GAGGATTTTT ATTACAAGAT GGCTGTGGCA 180
GACAATGGAT GGATCTATGT GATGTTGGAT TTCTCTCGTA TTTATTTTGA TGATGTCAGG 240
CTGTATCGTT CCAAAGACGG TGGTGCTACT TACCAAAAGT TAGGGTCTTT GGGGTCTTTG 300
GTGCCTTATG ACTTCGATGT CTCGCATTGC GATTTTATTG TAACGGGAAA GGATGAAGAT 360
GATATCAATG TTTGGACAGT CATGACAGCA TTCGAATATG TAGGTGGTAC TATTGGCAAT 420
GGCGTTTTGC TGATGCATCG CCATGATGCA GATATCAATA ATACAGAGTG TGTGTACAAG 480
AAGGATTTCC CTAATAATAG ACTGATGGGT GTAGCCATCG CCTCCAACTA CCGTGCGCCC 540
TCTCCTTACG GTTTGGGGGG CGATCCTTTT GCTCTCGCTG TCGCCGTTAG TGGCTCCGGA 600
AGCGATCACA GCTTCTTGGA CTATATTTTT TCGTTAGATG GTGGAGTACA CTTTGAGCAA 660
AAGCGTATTT ACACAAGACC CCAAAAACTG ACTATCAATA GAGTAGACCT TTCATTAGGC 720
AGTACATCTC CTTCTCTTGG ATTTAATACT TGGCCACTAA TGGGAGTCGT ATTCGAAATG 780
AATAAGAACC TTGATGGCTT CGACATTGGT TTCATTTCCA ACTTTGTGGA CTATGATCCC 840
CGCTATGCGT GGTCTGAACC GATAATAATA GAAGAAGACT GTGGATGGAC TGATTTTAAT 900
CCTTTGGGAG CACTAAGTAT AGAGATCCAA ATGATGTTGG ATGACAATTC GGATAATACC 960
GTGGGTGGAG AACGCTCCCA TAACTTCCTG ATCACTTACC CGGGCCATTA CGTATATCCG 1020
AAGCAATCTT TCAATTATTC TCCCGGACAT ACACCGACAA AGAAAGATCT GGTCTTTAAA 1080
CACTGTATAG GTATTCCGGC TTTGGCATAC GATAAGGAAG GCGATCGTTA TCTGACTACT 1140
TTTCAAGATC ACAATCTAAT GAGATACAGA TGGATCAAAT ACGATGACAT TAACTCTTTT 1200
TATGGTTGGA GTTGGCCATA TGTATATGCA AAAGAAGCTA AAGATAAAAA GAGGCGCCGT 1260
CCGCAAGTAG CACTCAATCC TACCAATGGA AAGGCTTGTT GGGTATGGCA TACTCGCAAG 1320
AGCCCATATG ATGAAACCAA ACCACATCCT ACTCCTGTAA TTATTAAACA TTTCCTATGG 1380
TCCGATACGG AGTGGGTACA TGCTCTGGAC GTGGGGGACG TATTGCAGAA GGAGGGTAGC 1440
ATGAAGCTCT ACCCCAATCC TGCCAAAGAA TATGTTCTGA TCAACCTACC CAAAGAAGGG 1500
GGGCACGAGG CAGTCGTATA CGACATGCAG GGCCGAATCG TGGAGAAAGT TTCATTTTCA 1560
GGGAAAGAAT ATAAGCTGAA TGTGCAGTAT CTGTCCAAAG GTACGTACAT GCTGAAAGTT 1620
GTAGCGGATA CGGAGTATTT CGTGGAAAAA ATCATTGTAG AG 1662

(2) INFORMATION FOR SEQ ID NO:126

(i) SEQUENCE CHARACTERISTICS:
(A) LENGTH: 1650 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1650

(xi) SEQUENCE DESCRIPTION: SEQ ID NO:126

ATGAAATTAA AAATTGCACT CAGACTGCTG CTGGCGACTT TTGCCATAGT TTTATTTAGC 60
CCTCTGGCCA AGGCCCAGAT GGATATTGGT GGAGACGATG TATTGATCGA GACGATGTCC 120
ACCCTATCAG GATATTCAGA GGATTTTTAT TACAAGATGG CTGTGGCAGA CAATGGATGG 180
ATCTATGTGA TGTTGGATTT CTCTCGTATT TATTTTGATG ATGTCAGGCT GTATCGTTCC 240
AAAGACGGTG GTGCTACTTA CCAAAAGTTA GGGTCTTTGG GGTCTTTGGT GCCTTATGAC 300
TTCGATGTCT CGCATTGCGA TTTTATTGTA ACGGGAAAGG ATGAAGATGA TATCAATGTT 360
TGGACAGTCA TGACAGCATT CGAATATGTA GGTGGTACTA TTGGCAATGG CGTTTTGCTG 420
ATGCATCGCC ATGATGCAGA TATCAATAAT ACAGAGTGTG TGTACAAGAA GGATTTCCCT 480
AATAATAGAC TGATGGGTGT AGCCATCGCC TCCAACTACC GTGCGCCCTC TCCTTACGGT 540
TTGGGGGGCG ATCCTTTTGC TCTCGCTGTC GCCGTTAGTG GCTCCGGAAG CGATCACAGC 600
TTCTTGGACT ATATTTTTTC GTTAGATGGT GGAGTACACT TTGAGCAAAA GCGTATTTAC 660
ACAAGACCCC AAAAACTGAC TATCAATAGA GTAGACCTTT CATTAGGCAG TACATCTCCT 720
TCTCTTGGAT TTAATACTTG GCCACTAATG GGAGTCGTAT TCGAAATGAA TAAGAACCTT 780
GATGGCTTCG ACATTGGTTT CATTTCCAAC TTTGTGGACT ATGATCCCCG CTATGCGTGG 840
TCTGAACCGA TAATAATAGA AGAAGACTGT GGATGGACTG ATTTTAATCC TTTGGGAGCA 900
CTAAGTATAG AGATCCAAAT GATGTTGGAT GACAATTCGG ATAATACCGT GGGTGGAGAA 960
CGCTCCCATA ACTTCCTGAT CACTTACCCG GGCCATTACG TATATCCGAA GCAATCTTTC 1020
AATTATTCTC CCGGACATAC ACCGACAAAG AAAGATCTGG TCTTTAAACA CTGTATAGGT 1080
ATTCCGGCTT TGGCATACGA TAAGGAAGGC GATCGTTATC TGACTACTTT TCAAGATCAC 1140
AATCTAATGA GATACAGATG GATCAAATAC GATGACATTA ACTCTTTTTA TGGTTGGAGT 1200

TGGCCATATG TATATGCAAA AGAAGCTAAA GATAAAAAGA GGCGCCGTCC GCAAGTAGCA 1260
CTCAATCCTA CCAATGGAAA GGCTTGTTGG GTATGGCATA CTCGCAAGAG CCCATATGAT 1320
GAAACCAAAC CACATCCTAC TCCTGTAATT ATTAAACATT TCCTATGGTC CGATACGGAG 1380
TGGGTACATG CTCTGGACGT GGGGGACGTA TTGCAGAAGG AGGGTAGCAT GAAGCTCTAC 1440
CCCAATCCTG CCAAAGAATA TGTTCTGATC AACCTACCCA AAGAAGGGGG GCACGAGGCA 1500
GTCGTATACG ACATGCAGGG CCGAATCGTG GAGAAAGTTT CATTTTCAGG GAAAGAATAT 1560
AAGCTGAATG TGCAGTATCT GTCCAAAGGT ACGTACATGC TGAAAGTTGT AGCGGATACG 1620
GAGTATTTCG TGGAAAAAAT CATTGTAGAG 1650

(2) INFORMATION FOR SEQ ID NO:127

(i) SEQUENCE CHARACTERISTICS:
(A) LENGTH: 1170 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1170

(xi) SEQUENCE DESCRIPTION: SEQ ID NO:127

ATGAAACGAT TACTCCCCTT TCTCCTTTTA GCAGGACTCG TAGCCGTAGG AAACGTGTCT 60
GCTCAGTCAC CCCGAATCCC TCAAGTGGAT GTACACACTC GCATCGCAAG AAATGCCCGT 120
TATCGACTGG ACAAGATCAG TGTCCCGGAT TCTCGTCAGA TATTCGATTA CTTCTATAAA 180
GAAGAAACGA TACCCACTAA AATACAAACG ACCACAGGAG GTGCAATTAC AAGCATCGAT 240
TCGCTTTTCT ATGAAGACGA CAGGTTGGTT CAGGTGCGCT ATTTTGACAA TAACCTTGAA 300
TTAAAACAAG CGGAGAAGTA TGTATACGAC GGTTCTAAGC TGGTCCTTCG AGAAATTCGC 360
AAGTCGCCGA CAGACGAAAC GCCAATAAAG AAAGTTAGCT ATCACTATCT CTGTGGCAGC 420
GATATGCCTT TTGAGATTAC GACAGAGATG AGCGATGGCT ATTTTGAAAG CCATACGCTT 480
AACTATCTGA ATGGAAAGAT TGCCCGAATA GATATCATGA CTCAACAGAA CCCATCGGCC 540
GAATTGATCG AAACGGGTAG AATGGTATAT GAGTTTGATG CCAATAATGA TGCTGTACTG 600
CTTCGTGACA GTGTATTTCT TCCTCTTCAA AACAAGTGGG TAGAAATGTT TACTCACCGT 660
TATACATACG ACAATAAGCA TAATTGTATT CGTTGGGAAC AAGACGAATT CGGCACCCTC 720
ACCCTTGCCA ACAACTTCGA ATACGACACC ACTATCCCTC TGTCGTCTGT ATTGTTCCCC 780
ACGCATGAGG AGTTCTTCCG TCCTCTTCTT CCCAATTTTA TGAAGCATAT GCGTACGAAG 840
CAAACGTATT TCAATAACTC CGGAGAAGGC TTGTCAGAGG TATGCGATTA CAACTACTTC 900
TATACCGATA TGCAGGGTAA TGCACTGACC GATGTTGCCG TGAACGAATC GATCAAGATT 960
TATCCTCGTC CTGCCACGGA TTTTCTGCGT ATAGAAGGTT CGCAACTGCT TCGCCTTTCG 1020
CTATTCGACA TGAACGGGAA GCTCATCAGA GCTACCGAAT TGACAGGCGA TTTGGCCATT 1080
ATCGGAGTTG CATCTCTTCC GAGAGGCACT TACATCGCAG AAATAACTGC TGCAAACAGC 1140
AAAACCATAC GTGCAAAAGT ATCGCTCAGA 1170

(2) INFORMATION FOR SEQ ID NO:128

(i) SEQUENCE CHARACTERISTICS:
(A) LENGTH: 1233 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature

(B) LOCATION 1...1233

(xi) SEQUENCE DESCRIPTION: SEQ ID NO:128

ATGAGACAGC ATTTATCTCT ATTTCCTTTT ATCTTGTTTC TGCTTCTTGC CTTCTCTTAT 60
GTCGGTTGCA GAACAGTCCG ACAAACACCT AAGCAGTCGG AACGGTACGT CGTAGTCCTG 120
TCTTTGGACG GCTTCCGACC GGACTATACC GATCGGGCAC GTACACCGGC GTTGGATCGG 180
ATGGCACAGG AGGGATTGAG CGGGTCGCTC CAACCATGCT TCCCCTCGCT TACATTTCCC 240
AATCATTACA GCATGGCTAC GGGGCTTTAC CCCGATCATC ACGGTATCGT AGCCAATGAG 300
TTTGTGGATT CGCTACTGGG CATCTTTCGT ATATCCGACC GAAAAGCCGT GGAGACCCCC 360
GGATTTTGGG GCGGCGAGCC GGTTTGGAAT ACGGCCGCAC GCCAAGGCAT CCGTACCGGT 420
GTCTACTTTT GGGTAGGATC CGAAACGGCT GTGAACGGAA ATCGGCCGTG GCGGTGGAAA 480
AAATTCTCCT CCACCGTTCC GTTTCGTGAC CGTGCCGACT CCGTCATCGC GTGGCTCGGA 540
CTGCCCGAAA AGGAGCGACC GCGCTTGCTC ATGTGGTACA TCGAGGAGCC GGATATGATC 600
GGACACAGCC AAACGCCCGA AAGCCCGCTG ACACTGGCAA TGGTAGAGCG GTTGGACAGT 660
GTGGTCGGCT ATTTCCGCAA GCGGTTGCAC TCTCTGCCCA TAGCCGCACA GACCGACTTC 720
ATCATAGTAT CCGATCACGG TATGGCCACG TACGAAAATG AGAAATGTGT CAATCTGTCG 780
CATTATCTGC CTGCGGACAG TTTCCTCTAC ATGGCCACCG GGGCCTTCAC CCACTTGTAC 840
CCGAAGCCCT CCTATACCGA GCGAGCCTAT GAGATCCTGC GGGCCATTCC ACATATATCG 900
GTTTACCGCA AGGGGGAGGT GCCCAAGCGT TTGCGCTGTG GCACCAATCC TCGTTTGGGC 960
GAACTGGTCG TGATTCCGGA CATAGGCTCC ACCGTCTTTT TCGCAATAAA TGAAGACGTT 1020
CGTCCGGGAG CGGCACATGG CTATGACAAC CAAGCACCGG AAATGCGGGC TTTACTCCGG 1080
GCTGTCGGAC CCGATTTCCG TCCGGGCAGT AGGGTGGAAA ACCTGCCCAA TATCACCATC 1140
TATCCGCTCA TATGCAGGCT GTTGGGTATA GAGCCTGCAC CCAACGATGC GGACGAAACG 1200
TTGCTGAACG GCCTGATCCG AGACAAACGA CCA 1233

(2) INFORMATION FOR SEQ ID NO:129

(i) SEQUENCE CHARACTERISTICS:
(A) LENGTH: 738 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...738

(xi) SEQUENCE DESCRIPTION: SEQ ID NO:129

ATGAAAGTAG GTTTGTTCAT CCCCTGTTAT GTCAATGCAG TGTATCCGGA AGTGGGTATC 60
GCCACGTACA AACTGCTGAA GAGTTTGGAC ATAGATGTCG ACTACCCGAT GGATCAGACA 120
TGTTGCGGCC AGCCTATGGC CAATGCCGGA TTCGAACAGA AAGCTCAAAA GCTGGCTTTC 180
CGATTCGAAG AGCTGTTCGA GTCCTATGAT GTAGTCGTAG GGCCATCGGC CAGTTGCGTT 240
GCTTTCGTGA AAGAAAACTA TGATCATATC CTCAGACCGA CAGGACATGT CTGCAAGTCG 300
GCAGCCAAGG TTCGGGATAT ATGCGAGTTC TTGCACGATG ACCTGAAGAT CACCAGCCTC 360
CCCTCCCGAT TCGCCCATAA GGTGAGCCTG CACAACAGTT GCCACGGTGT GCGCGAACTG 420
CATCTGTCCA CCCCCAGTGA AGTGCACCGA CCGTACCACA ACAAGGTGCG CCGGCTATTG 480
GAGATGGTGC AGGGCATAGA GGTATTCGAG CCGAAGCGAA TAGACGAATG CTGCGGTTTC 540
GGCGGTATGT ACTCGGTGGA GGAGCCGGAG GTATCCACCT GTATGGGGCA TGACAAGGTG 600
CTGGATCACA TATCCACAGG TGCGGAGTAC ATCACAGGGC CGGACAGCTC GTGCCTCATG 660
CATATGCAGG GAGTGATAGA CAGAGAGAAA TTGCCGATCA AGACAATTCA TGCAGTAGAA 720
ATTTTAGCAG CAAACTTA 738

(2) INFORMATION FOR SEQ ID NO:130

(i) SEQUENCE CHARACTERISTICS:
(A) LENGTH: 738 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...738

(xi) SEQUENCE DESCRIPTION: SEQ ID NO:130

ATGGATATTG TAAGTATGGC CGATAAAGCT CTTGTAGTGG AGATGAGAGA TGTGACGCTC 60
TGTCAGGAGG AAAACGTCAT TTTTCAAAAT TTGAATCTGA CCCTTTCCGC CGGAGACTTC 120
GTCTATCTGA TAGGCTCAGT GGGATCGGGG AAGAGCACTT TGCTGAAGGC TTTGTATGCT 180
GAGGTGCCTA TCTCTGCCGG TTATGCCCGC GTGATAGATT ATGATCTGGC AAAGTTGAAA 240
CGGAAGCAGT TGCCCTATCT GCGCAGGAAT TTGGGCATTG TGTTTCAGGA TTTCCAGTTG 300
CTGAACGGAC GTACTGTTGC GGAGAATTTG GATTTCGTTT TGCGAGCTAC GGACTGGAAA 360
AACCGAGCCG ATCGCGAGCA GCGTATCGAG GAGGTTTTGA CCCGTGTGGG AATGTCTCGG 420
AAGGCTTATA AGAGACCGCA CGAACTGTCC GGAGGGGAGC AACAACGTGT GGGTATAGCC 480
AGAGCTTTGC TGGCGAAGCC TGCGTTGATC CTGGCCGACG AACCCACAGG CAACCTCGAT 540
TCGGTGACCG GATTGCAGAT CGCTTCTCTG CTCTACGAAA TCAGTAAGCA GGGCACTGCA 600
GTACTTATGA GCACGCACAA CAGCAGCCTG CTGTCGCATC TGCCGGCACG GACATTGGCC 660
GTTCGTAAGA ATGGCGATGC CTCCTCTTTG GTCGAGCTGA GTGCAGATGC TGTTTCAAGA 720
AAAAATACGG AAATAGAT 738

(2) INFORMATION FOR SEQ ID NO:131

(i) SEQUENCE CHARACTERISTICS:
(A) LENGTH: 723 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...723

(xi) SEQUENCE DESCRIPTION: SEQ ID NO:131

ATGGCCGATA AAGCTCTTGT AGTGGAGATG AGAGATGTGA CGCTCTGTCA GGAGGAAAAC 60
GTCATTTTTC AAAATTTGAA TCTGACCCTT TCCGCCGGAG ACTTCGTCTA TCTGATAGGC 120
TCAGTGGGAT CGGGGAAGAG CACTTTGCTG AAGGCTTTGT ATGCTGAGGT GCCTATCTCT 180
GCCGGTTATG CCCGCGTGAT AGATTATGAT CTGGCAAAGT TGAAACGGAA GCAGTTGCCC 240
TATCTGCGCA GGAATTTGGG CATTGTGTTT CAGGATTTCC AGTTGCTGAA CGGACGTACT 300
GTTGCGGAGA ATTTGGATTT CGTTTTGCGA GCTACGGACT GGAAAAACCG AGCCGATCGC 360
GAGCAGCGTA TCGAGGAGGT TTTGACCCGT GTGGGAATGT CTCGGAAGGC TTATAAGAGA 420
CCGCACGAAC TGTCCGGAGG GGAGCAACAA CGTGTGGGTA TAGCCAGAGC TTTGCTGGCG 480
AAGCCTGCGT TGATCCTGGC CGACGAACCC ACAGGCAACC TCGATTCGGT GACCGGATTG 540
CAGATCGCTT CTCTGCTCTA CGAAATCAGT AAGCAGGGCA CTGCAGTACT TATGAGCACG 600
CACAACAGCA GCCTGCTGTC GCATCTGCCG GCACGGACAT TGGCCGTTCG TAAGAATGGC 660
GATGCCTCCT CTTTGGTCGA GCTGAGTGCA GATGCTGTTT CAAGAAAAAA TACGGAAATA 720
GAT 723

(2) INFORMATION FOR SEQ ID NO:132

(i) SEQUENCE CHARACTERISTICS:
(A) LENGTH: 696 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...696

(xi) SEQUENCE DESCRIPTION: SEQ ID NO:132

ATGAGAGATG TGACGCTCTG TCAGGAGGAA AACGTCATTT TTCAAAATTT GAATCTGACC 60
CTTTCCGCCG GAGACTTCGT CTATCTGATA GGCTCAGTGG GATCGGGGAA GAGCACTTTG 120
CTGAAGGCTT TGTATGCTGA GGTGCCTATC TCTGCCGGTT ATGCCCGCGT GATAGATTAT 180
GATCTGGCAA AGTTGAAACG GAAGCAGTTG CCCTATCTGC GCAGGAATTT GGGCATTGTG 240
TTTCAGGATT TCCAGTTGCT GAACGGACGT ACTGTTGCGG AGAATTTGGA TTTCGTTTTG 300
CGAGCTACGG ACTGGAAAAA CCGAGCCGAT CGCGAGCAGC GTATCGAGGA GGTTTTGACC 360
CGTGTGGGAA TGTCTCGGAA GGCTTATAAG AGACCGCACG AACTGTCCGG AGGGGAGCAA 420
CAACGTGTGG GTATAGCCAG AGCTTTGCTG GCGAAGCCTG CGTTGATCCT GGCCGACGAA 480
CCCACAGGCA ACCTCGATTC GGTGACCGGA TTGCAGATCG CTTCTCTGCT CTACGAAATC 540
AGTAAGCAGG GCACTGCAGT ACTTATGAGC ACGCACAACA GCAGCCTGCT GTCGCATCTG 600
CCGGCACGGA CATTGGCCGT TCGTAAGAAT GGCGATGCCT CCTCTTTGGT CGAGCTGAGT 660
GCAGATGCTG TTTCAAGAAA AAATACGGAA ATAGAT 696

(2) INFORMATION FOR SEQ ID NO:133

(i) SEQUENCE CHARACTERISTICS:
(A) LENGTH: 657 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...657

(xi) SEQUENCE DESCRIPTION: SEQ ID NO:133

ATGATTGAAA TCAGCAACCT CACCAAGGTT TTCAGAACAG AAGAAATAGA GACGGTAGCC 60
CTCGATGGCG TATCGCTCAA AGTGGACAAA GGCGAATTTA TCGCCATAAT GGGGCCTTCG 120
GGATGCGGTA AGTCCACTCT GCTCAATATC CTCGGCCTTC TCGACAATCC CACTTCCGGT 180
ATCTACAAGC TCGATGGGGC AGAAGTGGGC AACCTCCGGG AAAAAGACAG GACTGCCGTC 240
CGTAAGGGCA ATATCGGCTT CGTATTCCAG AGCTTCAACC TCATCGAAGA GATGACGGTA 300
AGCGAGAACG TGGAGTTGCC GCTCGTCTAT CTGGGTGTGA AGGCCTCCGA GCGGAAAGAG 360
CGAGTGGAGG AGGCACTGCG CAAGATGAGC ATCAGCCACC GGGCCGGCCA CTTCCCCAAT 420
CAGCTCTCCG GAGGACAACA GCAGCGCGTG GCTATCGCCC GTGCCGTGGT GGCCAATCCG 480
AAGCTCATCC TCGCCGATGA ACCCACGGGT AACCTCGACT CCAAAAACGG AGCCGATGTC 540
ATGGAACTGC TCAGAGGTCT CAATCGCGAA GGTGCAACCA TCGTCATGGT GACGCACTCC 600
GAGCACGATG CACGTAGTGC CGGCCGCATC ATCAATCTGT TCGACGGTAA GATTCGC 657

(2) INFORMATION FOR SEQ ID NO:134

(i) SEQUENCE CHARACTERISTICS:
(A) LENGTH: 1785 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1785

(xi) SEQUENCE DESCRIPTION: SEQ ID NO:134

ATGAAAGAAT TTTTCAAAAT GTTTTTCGCC TCGATCCTCG GGGTTATAAC GGCAGGAATC 60
ATCTTGTTCT GTATCTTTCT ATTTATCTTT TTCGGCATCG TAGCCGGTAT TGCCTCCAAG 120
GCAACGGGAG GAACCATTCC GAAGATCGAA GCAAACTCCA TCCTACATAT ANACAATTCT 180
TCTTTCCCTG AGATCGTATC GGCCAATCCC TGGAGCATGC TCACAGGCAA AGACGAGTCC 240
GTATCGCTCT CACAGGCAGT CGAAGCCATC GGCCAAGCCA AAAATAATCC CAACATAACC 300
GGTATCTTCC TCGATCTGGA CAACCTTTCC GTCGGTATGG CATCGGCAGA GGAATTGCGT 360
CGCGCGTTGC AGGATTTCAA GATGTCGGGC AAGTTCGTCG TATCCTATGC CGACAGATAC 420
ACCCAAAAGG GTTACTACCT CTCCAGTATT GCAGACAAAC TCTACCTCAA TCCGAAAGGA 480
ATGTTGGGGC TTATCGGGAT TGCGACCCAA ACAATGTTCT ACAAAGATGC CCTCGACAAA 540
TTCGGCGTGA AGATGGAGAT CTTCAAGGTA GGCACCTACA AGGCAGCCGT AGAGCCATTC 600
ATGCTCAACA GGATGAGCGA TGCCAATCGC GAACAAATCA CCACATACAT AAACGGGCTT 660
TGGGACAAGA TCACATCCGA TATTGCAGAG TCGCGCAAGA CGGCAATGGA TTCCGTGAAA 720
ATGTTTGCCG ACAAAGGCGA AATGTTCGGT CTTGCCGAGA AAGCGGTGGA GATGAAGCTC 780
GTGGATGAGC TGGCTTACCG TACCGATGTG GAGAAAGAAC TCAAAAAGAT GTCCCAACGC 840
GGAGAGAAAG ATGAACTTCG GTTCGTATCG CTTTCTCAGG TTCTGGCCAA TGGCCCGATG 900
AACAAAACGA AAGGCAGTCG GATCGCCGTT CTCTTTGCCG AAGGTGAAAT AACGGAAGAA 960
ATAATAAAGA AGCCGTTCGA CACTGACGGT AGCTCCATCA CACAAGAACT CGCCAAAGAA 1020
ATCAAGGCAG CAGCCGATGA CGATGATATC AAAGCCGTAG TACTTCGTGT CAATTCTCCG 1080
GGAGGTAGTG CTTTCACTTC CGAACAGATA TGGAAGCAGG TAGCCGATCT CAAGGCCAAA 1140
AAGCCTATCG TGGTCTCCAT GGGCGACGTA GCAGCCTCGG GCGGATACTA CATAGCCTGC 1200
GCAGCCAACA GTATCGTGGC AGAGCATACG ACTCTGACCG GCTCCATCGG CATATTCGGC 1260
ATGTTCCCGA ACTTCGCGGG CGTAGCCAAG AAGATAGGAG TGAATATGGA CGTCGTACAG 1320
ACATCCAAGT ATGCAGACTT GGGCAACACC TTCGCTCCGA TGACGGTCGA AGATCGTGCC 1380
CTCATCCAAC GCTACATAGA GCAGGGCTAC GACCTCTTCC TCACTCGCGT ATCGGAAGGC 1440
CGCAACCGCA CCAAGGCACA GATCGACAGC ATCGCTCAAG GCCGTGTATG GCTCGGCGAC 1500
AAAGCTCTTG CACTCGGTTT GGTGGATGAG CTTGGAGGTT TGGACACAGC TATCAAACGG 1560
GCCGCGAAGC TGGCTCAGCT CGGTGGCAAC TACAGCATAG AGTATGGCAA GACCAAGCGC 1620
AACTTCTTCG AAGAGTTGCT CTCCTCATCA GCAGCGGATA TGAAGTCTGC CATCCTGAGT 1680
ACCATTCTCT CCGATCCGGA AATAGAAGTT CTGCGCGAAC TCCGCTCCAT GCCGCCCCGT 1740
CCTTCGGGCA TACAGGCACG TCTCCCCTAT TACTTCATGC CGTAC 1785

(2) INFORMATION FOR SEQ ID NO:135

(i) SEQUENCE CHARACTERISTICS:
(A) LENGTH: 1767 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1767

(xi) SEQUENCE DESCRIPTION: SEQ ID NO:135

ATGTTTTTCG CCTCGATCCT CGGGGTTATA ACGGCAGGAA TCATCTTGTT CTGTATCTTT 60
CTATTTATCT TTTTCGGCAT CGTAGCCGGT ATTGCCTCCA AGGCAACGGG AGGAACCATT 120
CCGAAGATCG AAGCAAACTC CATCCTACAT ATANACAATT CTTCTTTCCC TGAGATCGTA 180
TCGGCCAATC CCTGGAGCAT GCTCACAGGC AAAGACGAGT CCGTATCGCT CTCACAGGCA 240
GTCGAAGCCA TCGGCCAAGC CAAAAATAAT CCCAACATAA CCGGTATCTT CCTCGATCTG 300
GACAACCTTT CCGTCGGTAT GGCATCGGCA GAGGAATTGC GTCGCGCGTT GCAGGATTTC 360
AAGATGTCGG GCAAGTTCGT CGTATCCTAT GCCGACAGAT ACACCCAAAA GGGTTACTAC 420
CTCTCCAGTA TTGCAGACAA ACTCTACCTC AATCCGAAAG GAATGTTGGG GCTTATCGGG 480

ATTGCGACCC AAACAATGTT CTACAAAGAT GCCCTCGACA AATTCGGCGT GAAGATGGAG 540
ATCTTCAAGG TAGGCACCTA CAAGGCAGCC GTAGAGCCAT TCATGCTCAA CAGGATGAGC 600
GATGCCAATC GCGAACAAAT CACCACATAC ATAAACGGGC TTTGGGACAA GATCACATCC 660
GATATTGCAG AGTCGCGCAA GACGGCAATG GATTCCGTGA AAATGTTTGC CGACAAAGGC 720
GAAATGTTCG GTCTTGCCGA GAAAGCGGTG GAGATGAAGC TCGTGGATGA GCTGGCTTAC 780
CGTACCGATG TGGAGAAAGA ACTCAAAAAG ATGTCCCAAC GCGGAGAGAA AGATGAACTT 840
CGGTTCGTAT CGCTTTCTCA GGTTCTGGCC AATGGCCCGA TGAACAAAAC GAAAGGCAGT 900
CGGATCGCCG TTCTCTTTGC CGAAGGTGAA ATAACGGAAG AAATAATAAA GAAGCCGTTC 960
GACACTGACG GTAGCTCCAT CACACAAGAA CTCGCCAAAG AAATCAAGGC AGCAGCCGAT 1020
GACGATGATA TCAAAGCCGT AGTACTTCGT GTCAATTCTC CGGGAGGTAG TGCTTTCACT 1080
TCCGAACAGA TATGGAAGCA GGTAGCCGAT CTCAAGGCCA AAAAGCCTAT CGTGGTCTCC 1140
ATGGGCGACG TAGCAGCCTC GGGCGGATAC TACATAGCCT GCGCAGCCAA CAGTATCGTG 1200
GCAGAGCATA CGACTCTGAC CGGCTCCATC GGCATATTCG GCATGTTCCC GAACTTCGCG 1260
GGCGTAGCCA AGAAGATAGG AGTGAATATG GACGTCGTAC AGACATCCAA GTATGCAGAC 1320
TTGGGCAACA CCTTCGCTCC GATGACGGTC GAAGATCGTG CCCTCATCCA ACGCTACATA 1380
GAGCAGGGCT ACGACCTCTT CCTCACTCGC GTATCGGAAG GCCGCAACCG CACCAAGGCA 1440
CAGATCGACA GCATCGCTCA AGGCCGTGTA TGGCTCGGCG ACAAAGCTCT TGCACTCGGT 1500
TTGGTGGATG AGCTTGGAGG TTTGGACACA GCTATCAAAC GGGCCGCGAA GCTGGCTCAG 1560
CTCGGTGGCA ACTACAGCAT AGAGTATGGC AAGACCAAGC GCAACTTCTT CGAAGAGTTG 1620
CTCTCCTCAT CAGCAGCGGA TATGAAGTCT GCCATCCTGA GTACCATTCT CTCCGATCCG 1680
GAAATAGAAG TTCTGCGCGA ACTCCGCTCC ATGCCGCCCC GTCCTTCGGG CATACAGGCA 1740
CGTCTCCCCT ATTACTTCAT GCCGTAC 1767

(2) INFORMATION FOR SEQ ID NO:136

(i) SEQUENCE CHARACTERISTICS:
(A) LENGTH: 939 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...939

(xi) SEQUENCE DESCRIPTION: SEQ ID NO:136

ATGAGAGCAA ACATTTGGCA GATACTTTCC GTTTCGGTTC TCTTTTTCTT CGGGACAGCG 60
ATCGGACACG CTCAGAGTCG AAACCGTACA TACGAGGCTT ATGTGAAACA GTACGCCGAC 120
GAAGCTATCC GACAGATGAG CCGCTACAAT ATACCGGCAA GCATCACCAT AGCACAGGCT 180
TTGGTGGAGA CAGGAGCCGG AGCCAGTACA CTGGCCAGCG TACACAACAA TCACTTCGGG 240
ATCAAATGCC ACAAATCGTG GACGGGCAAG CGCACCTATC GTACCGACGA TGCGCCGAAC 300
GAATGCTTCC GCAGCTATTC GGCCGCTCGC GAATCGTATG AAGATCATTC CCGATTTCTG 360
CTCCAACCAC GCTATCGTCC CCTGTTCAAA CTCGACAGAG AAGACTATCG GGGCTGGGCT 420
ACGGGGTTGC AACGCTGTGG CTATGCCACC AATCGGGGCT ATGCCAATCT GCTGATCAAG 480
ATGGTGGAGC TGTATGAGCT ATATGCTTTG GATCGCGAGA AGTACCCCTC ATGGTTCCAC 540
AAGTCTTACC CCGGGTCCAA CAAAAAATCC CATCAAACGA CCAAGCAGAA GCAGAGCGGA 600
CTCAAGCACG AAGCTTACTT CAGCTACGGA CTGCTCTACA TCATAGCCAA GCAAGGCGAT 660
ACCTTCGATT CTTTGGCCGA AGAGTTCGAC ATGAGAGCCT CCAAACTGGC CAAATACAAC 720
GATGCTCCCG TGGATTTCCC GATCGAAAAG GGCGATGTGA TCTATCTGGA GAAAAAGCAC 780
GCATGCTCCA TCTCCAAACA CACACAGCAC GTAGTGCGTG TGGGCGATTC GATGCACAGT 840
ATCTCCCAAC GCTATGGCAT CCGGATGAAG AACCTCTACA AGCTCAACGA CAAGGATGGC 900
GAATATATAC CCCAAGAGGG CGATATACTG CGCTTGCGC 939

(2) INFORMATION FOR SEQ ID NO:137

(i) SEQUENCE CHARACTERISTICS:
(A) LENGTH: 1569 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1569

(xi) SEQUENCE DESCRIPTION: SEQ ID NO:137

ATGGACGGAC GTCGATATTC GGATGGCCTC CATCAGGCTA TCGAAGCCAA AGAGCATGTG 60
AAAGTAGAGG CTGCGACACA GACATTTGCA ACTATCACTT TGCAGAACTA TTTCCGCATG 120
TATCATAAGC TGGCAGGGAT GACCGGTACT GCTGAAACTG AAGCGGGAGA GCTTTGGGAC 180
ATCTACAAAC TGGACGTTGT AGTTATTCCG ACAAACAAGC CTATCGCCCG TAAGGATATG 240
AATGATCGTA TCTATAAGAC GGCACGTGAA AAATATGCAG CAGTTATCGA AGAGATTGTA 300
CGTCTTGTCG AAGAGGGCAG ACCTGTACTT GTCGGTACTA CTTCGGTGGA AATATCCGAA 360
TTGTTGAGCC GTATGTTACG CTTGCGTGGC ATCCAACACA ATGTACTCAA TGCCAAATTG 420
CATCAGAAGG AGGCCGAGAT TGTAGCTCAG GCCGGTCAGA AAGGAACTGT TACCATCGCA 480
ACGAACATGG CCGGTCGTGG TACCGACATC AAGCTCTCTG CCGAGGTTAA GAAAGCCGGG 540
GGTTTGGCTA TCATTGGTAC GGAAAGGCAC GAATCCAGAC GAGTGGACAG ACAGCTTCGT 600
GGTCGTTCCG GCCGTCAGGG TGATCCCGGT TCGTCCATAT TCTATGTTTC CCTTGAAGAT 660
CATCTGATGC GCCTCTTTGC CACAGAAAAG ATTGCATCAT TGATGGATCG TTTAGGTTTC 720
AACGAAGGAG AAGTGCTCGA AAACAACATG CTGAGTAAGT CCGTGGAGCG TGCTCAAAAG 780
AAGGTGGAAG AGAACAACTT CGGTATCCGT AAACATCTGC TTGAGTACGA TGATGTAATG 840
AATTCGCAGC GTGAAGTCAT TTATACCCGT CGCCGTCATG CTTTGATGGG AGAGCGTATC 900
GGTATGGATG TACTCAATAC CATATACGAC GTATGTAAGG CTCTGATTGA CAATTATGCA 960
GAAGCCAATG ATTTCGAAGG CTTCAAGGAA GATCTGATGC GTGCACTCGC GATAGAATCT 1020
CCTATCACGC AAGAAATATT CAGAGGTAAG AAAGCAGAAG AGCTGACCGA TATGCTTTTC 1080
GATGAAGCTT ACAAGTCTTT CCAACGTAAG ATGGATCTGA TCGCAGAAGT GGCCCACCCT 1140
GTGGTTCATC AGGTATTCGA GACCCAAGCC GCCGTGTACG AGCGCATTCT AATCCCCATT 1200
ACGGATGGTA AACGTGTCTA TAACATAGGA TGCAATTTGC GTGAAGCGGA TGAAACTCAA 1260
GGGAAAAGCA TCATCAAAGA ATTTGAGAAA GCTATCGTAC TGCATACTAT CGATGAGTCT 1320
TGGAAAGAAC ATCTGCGTGA GATGGACGAG CTTCGTAATT CCGTTCAGAA TGCCAGCTAC 1380
GAAAACAAAG ATCCACTACT TATCTATAAA CTCGAATCTT ACGAACTGTT CCGCAAGATG 1440
GTAGAAGCCA TGAACCGTAA GACCGTAGCG ATCCTAATGC GTGCTCGGAT ACCGGTACCG 1500
GAGGCTCCTT CCCAAGAAGA GCTGGAACAC AGGCGGCAAA TAGAAATCCG ACATGCAACC 1560
CAACAACGT 1569

(2) INFORMATION FOR SEQ ID NO:138

(i) SEQUENCE CHARACTERISTICS:
(A) LENGTH: 1125 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1125

(xi) SEQUENCE DESCRIPTION: SEQ ID NO:138

ATGAATTTCT TAAAAAAAGA ACCGTTTAAA ATATTCTCTA TGATTTATCT GCTGTTAGAT 60
ACAATAACAA ACCGTGCCGG TACAGAACGC GCCGTGATCA ACTTGGCTAA CAACCTGCAT 120
GCCAATGGTC ATCGCGTATC ATTAGTCAGC GTTTGTACAA AACAAGGAGA GCCTTCCTTC 180
CAAGTAGAAA AAGGAATAGA AGTACACCAT CTCGGAATTA GGCTTTATGG CAATGCATTA 240
GCCCGCAAAA CAGTATATTT CAAGGCTTAT CGAAGGATAA AAGCCCTATA CAAGAAGCGT 300
GAACCGGTTT TATTGATAGG GACTAATATT TTTATCAATA CAATTTTGTC TCAGATCAGT 360
AACAGAGGCA GAATATTTAC GATCGGATGC GAACATATCT CTTATGATAT TGCCCGCCCT 420
ATTACAAAAC GCATAAGGGG GTTTCTGTAT TCAGGGCTTG ATGCCGTTGT AGCACTGACA 480
AAAAGAGATC AGCAATCGTT CGAGGCAATC TTACGTGGAC GCTCTAAAGC ATATGTCATA 540
CCCAATCAAG TTTCATTTAC TACAGTCCAA AGAGATGCTA CTACTCACAA ACAAATGTTG 600

GCGATTGGCA GGCTTACCTA CCAGAAGGGT TTTGAATTCA TGATAGAAGA TGCATCACGA 660
GTGCTGCGAG AAAGGCCTGA TTGGAAGCTT ATCATAGTCG GAGATGGCGA AAATGAATCG 720
ATGCTACGTA AAGAAATTGC ATCTCGCAAT ATGGAGTCGC AAATAGAAAT ACATCCATCT 780
ACACCGGAAA TTCGCAAATA CTACGAATCA TCTGCTATTT ATCTAATGAC GTCCCGTTTC 840
GAAGGACTAC CAATGGTACT TCTCGAAGCA GAAGCATATG CACTACCTAT AATCTCATAC 900
GATTGTCCGA CCGGCCCGAG GGAACTGATC GAAAACGGTC GCAATGGTTT CCTTGTGCCA 960
ATGGAAGCAC ATGAAGACTT CGCGGATAAG TTACGCTTAT TGATGGATGA TGAAACTCTT 1020
CGTAAGAAAA TGGGACAAGA ATCAGAGTTG ATGGTCAAAT CCTACTCTCC GGCAAATATC 1080
TATGAATGTT GGAAGAAACT ATTCGTCGAA ATCGGCTACA TGAAT 1125

(2) INFORMATION FOR SEQ ID NO:139

(i) SEQUENCE CHARACTERISTICS:
(A) LENGTH: 1086 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1086

(xi) SEQUENCE DESCRIPTION: SEQ ID NO:139

ATGATTTATC TGCTGTTAGA TACAATAACA AACCGTGCCG GTACAGAACG CGCCGTGATC 60
AACTTGGCTA ACAACCTGCA TGCCAATGGT CATCGCGTAT CATTAGTCAG CGTTTGTACA 120
AAAGAAGGAG AGCCTTCCTT CCAAGTAGAA AAAGGAATAG AAGTACACCA TCTCGGAATT 180
AGGCTTTATG GCAATGCATT AGCCCGCAAA ACAGTATATT TCAAGGCTTA TCGAAGGATA 240
AAAGCCCTAT ACAAGAAGCG TGAACCGGTT TTATTGATAG GGACTAATAT TTTTATCAAT 300
ACAATTTTGT CTCAGATCAG TAACAGAGGC AGAATATTTA CGATCGGATG CGAACATATC 360
TCTTATGATA TTGCCCGCCC TATTACAAAA CGCATAAGGG GGTTTCTGTA TTCAGGGCTT 420
GATGCCGTTG TAGCACTGAC AAAAAGAGAT CAGCAATCGT TCGAGGCAAT CTTACGTGGA 480
CGCTCTAAAG CATATGTCAT ACCCAATCAA GTTTCATTTA CTACAGTCCA AAGAGATGCT 540
ACTACTCACA AACAAATGTT GGCGATTGGC AGGCTTACCT ACCAGAAGGG TTTTGAATTC 600
ATGATAGAAG ATGCATCACG AGTGCTGCGA GAAAGGCCTG ATTGGAAGCT TATCATAGTC 660
GGAGATGGCG AAAATGAATC GATGCTACGT AAAGAAATTG CATCTCGCAA TATGGAGTCG 720
CAAATAGAAA TACATCCATC TACACCGGAA ATTCGCAAAT ACTACGAATC ATCTGCTATT 780
TATCTAATGA CGTCCCGTTT CGAAGGACTA CCAATGGTAC TTCTCGAAGC AGAAGCATAT 840
GCACTACCTA TAATCTCATA CGATTGTCCG ACCGGCCCGA GGGAACTGAT CGAAAACGGT 900
CGCAATGGTT TCCTTGTGCC AATGGAAGCA CATGAAGACT TCGCGGATAA GTTACGCTTA 960
TTGATGGATG ATGAAACTCT TCGTAAGAAA ATGGGACAAG AATCAGAGTT GATGGTCAAA 1020
TCCTACTCTC CGGCAAATAT CTATGAATGT TGGAAGAAAC TATTCGTCGA AATCGGCTAC 1080
ATGAAT 1086

(2) INFORMATION FOR SEQ ID NO:140

(i) SEQUENCE CHARACTERISTICS:
(A) LENGTH: 1920 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1920

(xi) SEQUENCE DESCRIPTION: SEQ ID NO:140

ATGGGAAAAA TCATTGGAAT TGACTTAGGC ACAACGAACT CTTGTGTCTC TGTATTGGAA 60
GGTAACGAAC CTATCGTTAT TACAAACAGT GAGGGCAAGC GCACAACGCC CTCGGTAGTG 120
GCTTTTGTGG ATGGTGGCGA GCGTAAGGTG GGCGATCCGG CCAAGCGTCA GGCCATCACC 180
AATCCGACCA AGACGATATA CTCTATCAAA CGCTTCATGG GCGAAACTTA CGATCAGGTT 240
TCCAGAGAAG TGGAGAGAGT GCCATTCAAG GTAGTACGTG GGGACAATAA TACTCCGCGC 300
GTAGATATAG ACGGTCGTCT CTATACGCCG CAGGAAATTT CGGCCATGAT CCTTCAGAAG 360
ATGAAGAAGA CGGCCGAAGA CTACCTCGGT CAGGAAGTAA CGGAGGCCGT GATCACTGTG 420
CCCGCATACT TCAACGACGC TCAACGTCAG GCAACGAAAG AAGCAGGAGA GATCGCCGGC 480
CTGAAAGTTC GCCGTATTGT GAACGAGCCT ACGGCAGCTT CTCTGGCCTA CGGTCTGGAC 540
AAGTCCAATA AGGATATGAA GATCGCTGTC TTCGACTTGG GTGGCGGTAC CTTCGATATC 600
TCTATCTTGG AATTGGGCGA CGGCGTTTTC GAAGTGAAAT CGACCAACGG TGATACGCAC 660
CTCGGAGGAG ACGACTTCGA CCACGTGATC ATTGACTGGC TGGCAGAAGA GTTCAAGTCT 720
CAGGAAGGTG TGGATCTTCG CCAGGATCCT ATGGCTATGC AGCGTCTGAA AGAAGCTGCC 780
GAAAAAGCCA AGATAGAGCT CTCCAGCACT TCATCTACGG AGATCAACCT CCCCTATATC 840
ATGCCGGTGA ACGGCATCCC CAAGCACTTG GTGATGACGC TTACAAGGGC TAAGTTCGAG 900
CAGTTGGCCG ATCGTCTGAT TCAGGCATGT GTGGCACCCT GCGAAACGGC CTTGAAAGAT 960
GCCGGTATGT CACGTGGCGA TATCGATGAA GTGATTCTCG TAGGTGGTTC CACACGTATT 1020
CCTGCTATTC AGGAGATTGT GGAGAAGATC TTCGGTAAGG CTCCGTCCAA GGGTGTGAAT 1080
CCCGACGAAG TGGTAGCTGT GGGTGCCGCT ATTCAAGGCG GTGTTCTGAC CGGTGAGGTA 1140
AAGGATGTCT TGCTGTTGGA CGTTACCCCC TTGTCGCTCG GTATCGAGAC TATGGGAGGC 1200
GTGATGACTC GCTTGATCGA TGCCAATACC ACTATCCCGA CGAAGAAGAG CGAAATCTTT 1260
ACCACAGCAG TGGACAATCA ACCTTCGGTA GAGATTCATG TACTTCAGGG TGAGCGTTCT 1320
TTGGCTAAGG ACAATAAGAG CATCGGCCGT TTCAACTTGG ACGGTATTGC TCCGGCGCCC 1380
CGTCAGACAC CGCAGATCGA AGTAACGTTT GACATCGATG CCAACGGTAT CCTGAATGTA 1440
ACGGCTCATG ACAAAGCTAC CGGCAAGAAG CAGAATATCC GCATCGAAGC CTCCAGCGGT 1500
TTGTCCGATG ATGAGATCAA GCGCATGAAG GAAGAGGCGC AGGCCAATGC CGAAGCAGAT 1560
AAGAAAGAGA AAGAACGTAT CGACAAGATC AATCAGGCCG ACAGCATGAT CTTCCAGACG 1620
GAAAAGCAGT TGAAGGAGTT GGGAGACAAA TTCCCGGCCG ACAAGAAGGC TCCGATCGAT 1680
ACCGCTCTCG ACAAACTGAA AGAAGCACAC AAAGCACAGG ATGTAGCTGC TATCGATACA 1740
GCCATGGCCG AACTGCAAAC CGCTCTTTCC GCAGCGGGCG AAGAGCTTTA CAAGAATGCC 1800
GGAGCAGCCC AAGGTGGCGC ACAACCCGGT CCGGACTTCG GCGGTGCTCA AGGTCCCTCT 1860
GCCGGTGATC AGCCCTCTGA CGACAAGAAC GTCACAGACG TAGACTTCGA GGAAGTGAAG 1920

(2) INFORMATION FOR SEQ ID NO:141

(i) SEQUENCE CHARACTERISTICS:
(A) LENGTH: 1347 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1347

(xi) SEQUENCE DESCRIPTION: SEQ ID NO:141

ATGCGCTACG ACTTAGCTAT CATCGGTGGA GGGCCGGCCG GTTATACGGC TGCCGAACGT 60
GCTGCCAAAG GTGGCCTGAA AACCCTCCTA ATTGAGAAGA ATGCTCTCGG TGGTGTATGC 120
CTCAACGAAG GATGTATACC GACCAAGACG CTACTCTACT CGGCCAAAGT GCTACATCAA 180
ATTGCTACGG CATCTAAATA TGCAGTAAGT GGAACGGCCG ATGGACTTGA CCTCGGCAAG 240
GTGATTGCCA GAAAAGGTAA AATCATTCGC AAGCTGACTG CAGGCATCCG TTCACGCCTG 300
ACAGAGGCCG GAGTAGAGAT GGTGACGGCA GAAGCTACCG TAACGGGATG CGATGCAGAC 360
GGCATCATCG GCATTACTGC GGGCGAAGCA CAGTACAAAG CTGCCAACCT GCTACTATGT 420
ACCGGTTCGG AGACGTTTAT TCCACCCATC CCCGGAGTGG AGCAGACAGA GTATTGGACA 480
AACCGTGAAG CTCTACAGAA CAAAGAGATT CCGACCTCTC TCGTCATCAT CGGTGGTGGA 540
GTGATCGGAA TGGAGTTCGC TTCTTTCTTC AACGGTATCG GTACGCAAGT GCACGTGGTG 600
GAGATGCTGC CGGAAATACT CAACGGTATC GATCCCGAAC ATGCAGCTAT GCTACGCGCT 660
CACTATGAAA AAGAAGGAAT CAAATTCTAC CTCGGGCACA AAGTAACATC GGTTCGCAAC 720
GGAGCTGTTA CGGTAGAATA CGAAGGAGAA AGCAAAGAGA TCGAAGGAGA ACGTATCCTG 780
ATGAGTGTGG GACGTCGCCC CGTGCTGCAA GGATTCGAGT CGCTCGGATT GGTGCTTGCC 840
GGCAAAGGTG TAAAGACTAA TGAGAGGATG CAAACTTCCC TGCCCAATGT CTATGCTGCA 900

GGTGATATTA CAGGCTTCTC GCTTTTGGCA CATACGGCTG TACGGGAAGC AGAGGTAGCA 960
GTAGATCAGA TTTTGGGCAA AACAGACGAA ACGATGAGCT ACCGTGCCGT ACCAGGTGTG 1020
GTGTACACCA ATCCCGAGGT CGCCGGTGTG GGAGAGACGG AAGAATCGCT TCGCAAAGCA 1080
GGACGTGCCT ACACTGTTCG TCGCCTTCCT ATGGCCTTCT CCGGTCGATT TGTAGCAGAA 1140
AACGAACAAG GCAATGGAGA GTGCAAACTA CTACTTGATG AAGAGAACCG CTTGATCGGA 1200
GCACACCTCA TTGGCAATCC GGCCGGCGAA CTCATCGTAA CCGCTGCCAT GGCCATCGAG 1260
ACCGGCATGA CGGATCGACA AATCGAACGA ATCATATTCC CTCATCCGAC TGTAGGCGAA 1320
ATCCTAAAAG AAACTCTCGC CGGAGGT 1347

(2) INFORMATION FOR SEQ ID NO:142

(i) SEQUENCE CHARACTERISTICS:
(A) LENGTH: 2823 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2823

(xi) SEQUENCE DESCRIPTION: SEQ ID NO:142

ATGGAATTGA AAAGATTTTT ATCACTTGGT CTTCTGCTTG TGGGATTCAT TCCGATGAAG 60
CTTTCTGCCC AACAGGCTCA GCCACTCCCT ACAGATCCGG CTGTTCGTGT CGGTAAGTTG 120
GACAACGGAT TGACTTATTT CATCCGTCAC AACGAGAACC CGAAAGATCG TGCGGATTTC 180
TTTATCGCAC AAAAGGTAGG TTCTATTCTT GAAGAAGATA GCCAGTCCGG TTTGGCTCAC 240
TTCTTGGAAC ACATGGCTTT CAACGGTACG AAGAACTTCC CCGGTAAGAA CTTGATCAAC 300
TATCTCGAAA CGATCGGTGT ACGTTTCGGT CAGAACCTGA ACGCTTCTAC CGGATTCGAC 360
AAGACGGAAT ATACGATAAT GGATGTGCCG ACTACACGTC AGGGAATCAT CGACTCCTGC 420
TTGCTTATCC TGCATGATTG GAGTAACAAT ATTACCCTCG ACGGGCATGA GATCGACGAG 480
GAGCGCGGTG TGATCCAGGA AGAGTGGCGT GCTCGTCGCG ATGCCAACCT TCGTATGTTC 540
GAGGCTATAC TTGCCAAGGC TATGCCGGGT AATAAATATG CAGAACGCAT GCCCATCGGT 600
CTGATGGACG TCGTGCTCAA CTTCAAGCAT GATGAGCTGC GCAACTATTA TAAGAAATGG 660
TATCGTCCCG ACCTGCAAGG TCTGGTGATC GTGGGAGATA TCGATGTGGA CTATGTGGAG 720
AACAAGATCA AAGAACTCTT CAAGGACGTT CCTGCTCCCG TGAATCCAGC AGAGCGTATC 780
TATACGCCGG TAGAGGACAA CGATGAGCCT ATCGTAGCCA TTGCTACCGA TGCTGAGGCT 840
ACTACCACGC AGCTCTCCAT CAGCTTCAAG AGCGACCCCA CTCCTCAAGA AGTGCGAGGA 900
TCGATATTCG GACTTGTGGA AGACTATATG AAACAGGTGA TCACTACAGC CGTGAATGAG 960
CGTCTGTCCG AGATTACTCA CAAGCCTAAC GCTCCTTTCC TCAGTGCAGG AGCTTTCTTC 1020
TCTAACTTCA TGTACATCAC CCAGACTAAG GACGCATTCA ATTTTGTTGC CACGGTTCGT 1080
GAGGGTGAAG CGGAGAAAGC GATGAACGCA TTGGTGGCAG AGATAGAAAG CCTCCGTCAG 1140
TTCGGTATCA CCAAAGGCGA ATACGATCGT GCACGCACGA ATGTGCTCAA GCGATACGAG 1200
AATCAATACA ACGAAAGAGA CAAGCGTAAG AACAATGCTT ATGCCAATGA ATACTCCACC 1260
TACTTCACCG ATGGCGGCTA TATCCCGGGT ATTGAGGTGG AATATCAGAC GGTGAATGCT 1320
TTTGCTCCTC AGGTTCCTCT GGAAGCATTC AATCAGGCTA TTGCCCAAAT GATCGATCCG 1380
GTGAAGAATG CTGTCGTTAC CCTCACCGGT CCTTCAAAGG CTGAAGCCAA GATTCCGAGC 1440
GAAGCAGACT TCCTCGCTGC TTTCAAAGCT GCTCGTCAGC AGAAAGTAGA AGCCAAGAAA 1500
GACGAAGTCT CCGACCAAAA ATTGATGGAG AAAGCTCCTA AGGCCGGAAA GATCGTTTCC 1560
GAGAAGAAAG ATCAGAAGTT CGGTACCACG GAACTTACCC TTAGCAATGG CATCAAAGTA 1620
TACCTCAAGA AGACCGATTT CAAATCAAAC GAAATCCTGA TGAGTGCTCT CAGCCCGGCT 1680
GGTATCCTCT CCGGAAAGCA TGCTCCCAAC CAATCTGTGA TGAATTCGTT CATGAACGTG 1740
GGTGGCTTGG GCAACTTCGA TGCTATCCAG CTGGATAAGG TGCTGACAGG TCGCTCTGCT 1800
TCCGTATCTC CCTCTTTGTC TCTGCTCAGT GAAGGTCTTT CGGGCAAAAC GACTGTAGAA 1860
GATATGGAAA CTTTCTTCCA GTTGATCTAT CTCCAAATGA CTGCTAACCG CAAGGATCCC 1920
GAAGCGTTCA AGGCCACACA GGAAAAGTTG TACAATAACT TGAAAAATCA GGAAGCCAAC 1980
CCGATGGCTG CGCTTATGGA CTCTATCCGT CATACCATGT ACGGCGATAA TCCGATGATG 2040
AAACCCATGA AAGCTGCTGA CGTGGAGAAA GTAAATTACG ATCAGGTAAT GGCTTTCTAC 2100
AATGAGCGAT TCGCTGATGC CGGCGACTTT ATGTTCTTCT TTATCGGTAA TCTGGATGAA 2160
GCCAAGATGA AGCCATTGAT CGAAACTTAT CTTGCTTCAT TGCCCAACCT CAAGCGTGGC 2220
GATAAGATGA ATAAGGCTCA GGTACCGGCT GCCCGTTCGG GAAAGATCGA TTGCAAGTTC 2280
GAGAAGGAAA TGGATACTCC TTCGACTACT ATATTCGATG TCGTGTCCGG AAATGTGGAA 2340
TATACGCTCA AGAACAGTCT CCTGCTGGAA GTCTTCTCAG CCGTAATGGA TCAGGTGTAC 2400
ACGGCTACCG TTCGCGAGAA GGAAGGCGGT GCATACAGTG TGGCTGCATT CGGCGGTCTC 2460
GAGCAATATC CTCAGCCCAA GGCTCTGATG CAGATCTATT TCCCCACGGA TCCTGCTCGT 2520

GCCGAGGAAA TGAATGCTAT CGTTTTTGCT GAGTTGGAGA AGCTTGCCAA GGAGGGCCCC 2580
AATGTGGAAT ACTTTAAGAA GACTATCGAA AACCTGAATA AGCAGCACAA AGAAAGTCTG 2640
CGTGAGAATC GTTTCTGGCT CGAAGCCATG AAGGCGTCTT TCTTCGAAGG AAATGACTTC 2700
ATCACAGACT ACGAATCCGT ACTGAACGGT CTTACTCCTG CTGAATTGCA AAAGTTTGCG 2760
GCAGACCTCT TGAAGCAGCA GAATCGGGTT GTTGTCATGA TGGCTCCTGT TGCAAAGGCT 2820
CAA 2823

(2) INFORMATION FOR SEQ ID NO:143

(i) SEQUENCE CHARACTERISTICS:
(A) LENGTH: 2052 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2052

(xi) SEQUENCE DESCRIPTION: SEQ ID NO:143

ATGAGTAAGA AAGGAACAAT CGGGGTAACG AGCGACAATA TATTCCCCGT CATCAAAAAA 60
TTCCTGTACA GCGACCATGA GATATTCCTG CGTGAGATCG TCTCCAATGC CGTGGATGCT 120
ACGCAGAAGC TGAAAACGCT TACATCCGTC GGCGAATTCA AAGGCGAGAC GGGTGACCTC 180
CGCGTAACGG TCAGCGTGGA TGAAGTGGCA CGCACGATCA CGGTCAGCGA CCGCGGCGTA 240
GGGATGACCG AAGAGGAGGT GGAGAAGTAC ATCAATCAGA TTGCTTTCTC CAGTGCGGAA 300
GAGTTTCTTG AAAAGTACAA AGACGACAAG GCCGCCATTA TCGGCCACTT CGGACTCGGA 360
TTTTACTCGG CTTTCATGGT GTCCGAGCGA GTGGACCTGA TCACGCGCTC TTTCCGAGAA 420
GATGCTACGG CGGTGAAATG GAGCTGCGAC GGATCGCCCG AATACACGCT CGAACCTGCG 480
GACAAGGCTG ACCGTGGCAC CGACATCGTG ATGCACATCG ATGAGGAGAA TAGCGAGTTC 540
CTCAAAAAAG AAAAGATAGA GGGGCTCCTC GGCAAATACT GTAAGTTCCT TACCGTGCCG 600
ATCATTTTCG GCAAGAAGCA GGAATGGAAA GACGGCAAGA TGCAAGATAC GGACGAGGAC 660
AATCAGATCA ACGACACACA TCCTGCCTGG ACCAAAAAGC CTGCCGACCT CAAGGACGAA 720
GACTATAAGG AATTTTACCG TTCGCTCTAT CCCATGTCCG AAGAGCCTCT CTTCTGGATC 780
CACCTCAATG TGGACTATCC GTTCAATCTG ACAGGTATCC TCTATTTCCC GAAGATCAAA 840
AACAACTTGG ATCTGCAGCG CAACAAGATT CAGCTCTACT GCAATCAGGT TTACGTCACC 900
GATGAAGTAC AGGGTATCGT GCCGGACTTC CTCACCCTCC TGCACGGGGT CATCGATTCG 960
CCGGATATTC CCCTCAACGT ATCGCGCTCC TATCTGCAGA GCGATGCCAA TGTGAAGAAG 1020
ATCTCGTCTC ATATCACCAA GAAGGTGGCA GACCGTCTGG AAGAAATTTT CAAAAACGAC 1080
CGCCCCACAT TCGAGGAGAA ATGGGATAGT CTGAAGCTCT TCGTCGAATA CGGTATGCTG 1140
ACGGATGAGA AGTTCTATGA GCGTGCAGCC AAATTCTTCC TTTTCACCGA TATGGACGGA 1200
CACAAGTACA CGTTCGACGA ATACCGAACG CTCGTCGAAG GTGTACAGAC GGATAAGGAC 1260
GGACAGGTAG TGTATCTCTA TGCTACGGAC AAGCATGGAC AGTACAGCCA CGTGAAACGT 1320
GCATCCGACA AAGGCTACAG CGTGATGCTG TTGGATGGTC AGTTGGATCC GCATATCGTG 1380
AGCCTGCTGG AGCAAAAGTT GGAGAAGACA CACTTTGTCC GTGTCGATAG CGATACGATC 1440
AACAATCTGA TCCGCAAGGA GGAAAGAGCC GAAGTGAAAC TGTCCGATAC GGAGCGCGCC 1500
ACTCTCGTGA AGCTGTTCGA AGCACGCCTG CCACGGGACG AGAAGAAGCA CTTCAATGTA 1560
GCTTTCGAAT CGCTCGGAGC CGAAGGTGAA GCCATCCTTA TCACACAAGC CGAATTCATG 1620
CGCCGTATGC GCGATATGGC ACAGCTGCAG CCGGGAATGA GCTTCTACGG CGAACTCCCC 1680
GATTCGTACA ATCTGGTACT TAATACCGAT CATCCGCTCA TCGACAGGGT ACTCTCCGGT 1740
GAGAAAGAAT CGGTAGAGCC TTCGCTCACA GAGCTTAGAG CGAAAATCGC CGAGCTGAAA 1800
GCGGAAGAGG CCAAGCTGCT CGATGAGGAA AAAGGGAAGA AACCGGAGGA AATCCCTGTT 1860
GCCACGAAGG AAGCCAAGGA GAACAACGCC GTCGAACAGG CCAAAACCGA AGGCAGTATC 1920
AACGATCAAC TGACCAAATA TGCTCAGGAC AACGAGCTGA TAGGTCAGCT CATCGACTTG 1980
GCTCTGCTCG GAAGCCGATT GCTGACGGGA GAGGCTTTGG CCGAATTCAT TCGTCGCAGC 2040
CAGCGTCTTC TC 2052

(2) INFORMATION FOR SEQ ID NO:144

(i) SEQUENCE CHARACTERISTICS:
(A) LENGTH: 1392 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1392

(xi) SEQUENCE DESCRIPTION: SEQ ID NO:144

ATGGAAAAAC TGATCGATAT TTTGGTCGTA GACGATGATG TGGCAGTCTG TGCCGCACTG 60
CGTCTGGTGC TCAAGCGAGC GGGCTATAAT CCCGTTATAG CCAACAGTCC CGACGAAGCT 120
TTGTCCATAA TGCGGAATCC TGATGGCGGC TGTAAGCCGG CTGTGATTCT GATGGATATG 180
AATTTCTCCC TTTCGACCTC CGGCAGGGAA GGATTGGAAC TACTGGAGAA GATGCAGATA 240
TTCACTTCCT GCCCTGTCAT ACTGATGACG GCTTGGGCTT CGATTCCACT GGCAGTGGAG 300
GGAATGAGGC TTGGAGCTTT CGACTTCATA GGCAAGCCAT GGGACAACGA TCGGCTCCTT 360
CGTACCATAG ATACGGCCTT GCATCTGGCT GCTCCCTCAG CTGTGGCGAA TCCATCGGAA 420
CAGTCTGACA GAGATACAGC CCGTCAGCCG AAAGCTACAG TCCAAGAGAA TGACCCCTGT 480
GCCCATATCA TAGGCCGGAG CGATGCCATC TGTAAGATCA AGGAACGGAT ACGCCGCATA 540
GCTCCCACCC ATGCCTCTGT GCTGATCACG GGCGAGAGCG GTACGGGCAA AGAGTTGATA 600
GCCGAAGCTC TGCACCGTGG GAGCAAACGA GCCTCAGCCC CATTCGTCAA GGTCAATTTG 660
GGTGGGATTC CCGAAAGTTT GTTCGAAAGT GAGCTGTTCG GACATAAGAA AGGAGCTTTT 720
ACCAATGCTT TTTCCGACAG GAAAGGACGG TTCGAGCTGG CTGATGGCGG CACGATCTTT 780
CTGGACGAAA TAGGCGAACT ACCGGTCGGC AACCAAGTAA AACTGCTGCG AGTGCTACAG 840
GAACAGACAT TCGAGCCGTT GGGCGAGAGC GTCTCCCACC GAGTGGACAT CCGTGTGGTA 900
TCGGCTACGA ATGCTTCCTT GGAGCGAATG GTAGCCGAAG GACGTTTCAG AGAGGACCTC 960
TACTATCGAA TCAACCTGAT ACATCTGCAT CTGCCTCCGC TGCGTGAGCG TCAGGAGGAT 1020
ATACAGCTGC TGGTGGAAGC CTTCAGTGAA GCCTTTGCCC AATCGAACGG ATTGCCCCAT 1080
GCCGTTTGGA GTGCGGAAGC TATGCGACGT ATCTGTGCCA TGCCCCTACC GGGCAATGTA 1140
CGCGAACTGA AAAACGTAGT GGAGCGTACG CTATTGCTCT CGGGATCGAG AGAAATCAGT 1200
GCCCGGGATG TGGCTGACTT CGGTTCGCAG GTGACGGCAG CAGACCACTC CGACGAACGG 1260
GCTTTGACCG ACATGGAGGA AGCTGCTATC CGAGAGACGC TGACTAAATA CAACGGCAAC 1320
GTTAGTCGTG CTGCACGAGC CTTGGGATTG AGCCGGGCAG CTCTTTACCG GCGAATGGAG 1380
AAATACGGAC TG 1392

(2) INFORMATION FOR SEQ ID NO:145

(i) SEQUENCE CHARACTERISTICS:
(A) LENGTH: 750 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...750

(xi) SEQUENCE DESCRIPTION: SEQ ID NO:145

ATGCTTAAGA TAAAGAACCT CCACGCCACA GTACAGGGCA AAGAGATATT GAAAGGAATC 60
AATCTGGAGA TCAATGCCGG AGAGATTCAT GCTATCATGG GGCCGAACGG ATCGGGGAAA 120
AGTACGCTCT CTTCCGTTTT GGTGGGACAT CCCTCCTTTG AAGTCACGGA AGGAGAGGTG 180
ACATTCAATG GAATCGACCT GCTCGAACTC GAACCGGAAG AACGTGCACA CCTCGGACTC 240
TTTCTCAGTT TCCAATATCC GGTCGAGATC CCGGGCGTCA GCATGGTGAA TTTCATGAGG 300
GCAGCTGTCA ATGAACATAG GAAAGCGATC GGAGCAGAAC CCGTATCGGC AAGCGACTTC 360
CTCAAGATGA TGCGAGAGAA GCGTGCCATT GTGGAGCTGG ACAACAAATT GGCCAGCCGT 420
TCTGTGAACG AAGGCTTCTC CGGTGGAGAA AAAAAGAGGA ACGAAATCTT CCAAATGGCT 480
ATGCTCGAAC CCAAGCTGGC TATTTTGGAC GAAACCGATA GCGGGCTCGA TATCGACGCT 540
CTCCGCATCG TAGCAGGCGG GGTAAACCGA CTCCGCTCTC CGGAGAATGC TGCTATTGTG 600

ATCACACACT ATCAGCGTTT GCTCGAGTAC ATCAAGCCGG ACTTCGTACA CGTCCTTTAC 660
AAGGGGCGCA TCGTCAAGTC GGGAGGAGCC GAGCTGGCTC TCACGCTCGA AGAAAAAGGC 720
TACGACTGGA TCAAGGAAGA GATAGGAGAA 750

(2) INFORMATION FOR SEQ ID NO:146

(i) SEQUENCE CHARACTERISTICS:
(A) LENGTH: 1383 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1383

(xi) SEQUENCE DESCRIPTION: SEQ ID NO:146

ATGGCTAAGG AGAAAACGAT CTACGTCTGC CGTTCGTGCG GAACCAAATA CGCCAAATGG 60
CAAGGCAACT GCAATGCCTG TGGAGAGTGG AACTGCATTG ATGAGGAGAA GGTGCCGGCA 120
CCGGCATCGG GCAAGCATGC AGCCAAGAGT TTTATGCCTC GGGAGCAGGA CAACCGGCCA 180
AGACTCTTAC AGGATGTGGA GTCCGGCGAT GAAGAGCGTA TTCGCCTCGG CGATGAAGAG 240
TTCGACCGCG TACTGGGTGG AGGAATTGTC AAAGGAGCAT TTGTCCTGCT TGGCGGCGAG 300
CCGGGAATCG GTAAGTCCAC GCTTATCCTC CAGACGGTGC TGCGTCTGCC GCAGTTGCGC 360
ACGCTCTATG TGTCGGGCGA AGAAAGTGCC CGACAACTGA AGATGCGCGC CGAACGACTG 420
GGGCAAGCCA TGAATGGGTG CTACGTATAC TGCGAAACGA ATATAGAGAG GATACTCTCC 480
CGTGCAGAAG AACTCACACC CGATCTCCTC GTGATAGACT CTATACAGAC GGTCTATACC 540
GAGGAAATGG AAAGCTCGGC CGGCAGCGTG GGGCAGATCC GCGAATGTGC CGCCTTACTG 600
CTCAAATACT GCAAGACTAC GGGTATCCCC GTCATCGTCA TCGGACACAT CACCAAAGAA 660
GGTAGCATAG CCGGACCGAA GGTGCTGGAG CATATAGTGG ATACGGTGCT TCTCTTCGAC 720
GGGGATAAGC ATCATCTCTA CCGGATACTC CGAGGACAGA AGAACCGCTA TGGCAGTACT 780
TCCGAGCTGG GGATATACGA GATGCGGCAG GACGGTCTGC GTGGCGTGGA GAATCCGAGC 840
GAACATCTCA TCACACGCAA TAGGGAAGAC CTCAGTGGCA TAGCCATAGC CGTAGCGATG 900
GAGGGCATTC GCCCGATACT CATCGAAGCG CAGGCTTTGG TCAGCTCGGC CATTTATGCC 960
AATCCGCAGC GTTCGGCCAC GGGCTTCGAT ATTCGGCGGA TGAACATGCT CTTAGCCGTA 1020
CTGGAGAAAC GTGCCGGCTT CAAGCTCATA CAGAAGGATG TGTTTCTGAA CATTGCCGGA 1080
GGTATCAAAA TAGCCGATCC GGCTACGGAT CTGGCCGTTA TCTCGGCAGT GCTGGCGTCG 1140
AGTCTGGACA TCGTTATCCC GCCGGCCGTA TGCATGACGG GCGAGGTCGG ACTCTCCGGA 1200
GAGATACGTC CCGTGAGCCG CATCGAGCAG CGCATAACGG AAGCGCGTCG CATAGGGTTC 1260
AAAGAGATAT TGGTACCGGC CGATAATTTC CGGCAGGAGG ATGCCGGCCG CTTCGGTATT 1320
CGGCTCGTGC CGGTCAGAAA GGTGGAGGAA GCCTTCCGCC ATCTGTTCTC GAAAGGAAGA 1380
GAA 1383

(2) INFORMATION FOR SEQ ID NO:147

(i) SEQUENCE CHARACTERISTICS:
(A) LENGTH: 813 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...813

(xi) SEQUENCE DESCRIPTION: SEQ ID NO:147

ATGAATAGCA GACATCTGAC AATCACAATC ATTGCCGGCC TCTCCCTCTT TGTACTGACA 60
TTGGGCGGCT GCTCCGTAGC CCAACAAGAT ACGCAGTGGA CTCTCGGCGG AAAGCTCTTT 120
ACTTCGGCGT GGATACAACG TTCGGCCGAA TATCAAGCGC TTTGCATTCA GGCATACAAC 180
ATCGCTACGG AAAGAGTGGA CGCTCTACCG GCAGAACGTA AACAAGGAGA TAGGCCTTAT 240
GCCATCGTAA CGGACATAGA CGAAACCATT TTGGACAATA CGCCTAACTC CGTGTATCAG 300
GCTCTCAGCG GCAAGGATTA TGATGAAGAG ACTTGGGGGA AATGGTGTGC ACAGGCCGAT 360
GCCGACACAC TGGCAGGAGC TTTGTCTTTC TTCCTCCATG CAGCGAACAA GGGGATCGAG 420
GTCTTTTACG TCACCAACCG CAGAGACAAT CTGCGCGAAG CAACTCTTCA GAACCTTCAG 480
CGTTACGGAT TCCCCTTTGC CGATGAAGAA CATTTGCTTA CGACCCATGG GCCATCCGAC 540
AAAGAACCCC GTCGGCTCAA AATACAAGAA CAGTATGAAA TAGTATTGCT CATAGGAGAC 600
AACTTGGGCG ACTTCCACCA CTTCTTCAAT ACGAAAGAAG AGTCCGGACG CAAACAGGCT 660
CTGGGCCTGA CAGCCGGGGA GTTTGGCCGG CACTTCATCA TGCTGCCCAA TCCCAACTAC 720
GGATCTTGGG AACCGGCATG GTACGGCGGG AAGTATCCGC CACTGCCCGA AAGAGACAAA 780
GCACTTAAAC AACTGCACTC ACAGAACAGC AGA 813

(2) INFORMATION FOR SEQ ID NO:148

(i) SEQUENCE CHARACTERISTICS:
(A) LENGTH: 1251 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1251

(xi) SEQUENCE DESCRIPTION: SEQ ID NO:148

ATGAGCACCA ATATAGATGT ACAACAGATC AAACAGCGTT TCGGCATCAT CGGTAGCAGT 60
CCGCTGATGG AACATGCCAT ACGAGTGGCA GCACAGGTGG CTCCTACCGA CATGTCCGTC 120
CTCGTGACGG GGGAGAGCGG TTCGGGGAAA GAGTTCTTCC CACAGATAAT CCACTACTAC 180
AGCGCCCGGA AACATCATAG CTACATTGCA GTCAATTGCG GAGCCATCCC CGAAGGAACC 240
ATCGATTCCG AGCTGTTCGG ACACCGCAAA GGTTCCTTTA CCGGAGCCGT ATCGGATCGC 300
AAGGGGTACT TCGAAGAAGC ATCCGGCGGC ACGATCTTTC TGGACGAAGT GGGCGAACTG 360
CCTTTGCCCA CGCAGGCGAG GCTGCTGAGG GTGCTGGAGA CGGGCGAGTT CATCCCCGTA 420
GGAGCCAGCC AGTCGCAGAA GACGGATGTC CGTATCGTAG CGGCGACGAA TGTGAACCTC 480
AAGGAGGCGG TAGCGAACGG GAAGTTCCGG GAAGACCTCT TCTTCCGGCT CAATACGGTA 540
CCGATCGAGG TGCCTGCGCT GCGTATGCGA CCGGACGACG TGCCCTTGCT TTTTCGCCGA 600
TTCGCCGCCG ACAGCGCCGA GAAGTATCGG ATGCCTCCGC TGCGCCTATC GGACGAAGCC 660
CGTACCATAT TAATGCGTTA CCGCTGGCCC GGCAATGTGC GAGAGCTGCG CAATATAACC 720
GACAGGCTGA GCATCCTGGA GGAGGAGCGG ACGGTATCGG CAGAGACCAT CACTCGCTAC 780
CTGGACGCTG AGGGGATGCA AGACCTCCAC CCCGTCGTGA TCCGACGGAA CGAAACGACC 840
GAAGCGGACA AACAAATCCC CCATTACGAG CGCGAAATCA TCTACCAGGT GCTATACGAT 900
ATGAAGAAAG AGATAGCCGA TTTGAAGGGG ATGATGAACC GCCTGGCGCA CCACGAACAG 960
CCCTCATGGC CTGTAGGGTC GGACGTCTGG GGCAACGACG ACAAGCGCAC CGCAGATCCG 1020
AAGTGGGGCG TCAGCACGCA CAAGGCCCCC ATCGCGAACG CGGCAGAACC CGTGGAGCCG 1080
ATACAGGAAG CCAGCGAATA CACCGAGGAT CCGGTTTCGC TGGAGGAGGT AGAGAAGAAA 1140
ATGATTTCCC TTGCATTGGA ACGCCACGGC GGAAGGCGCA AGCAGACAGC CGAGGAACTG 1200
AAGATTTCGG AGCGGACACT ATACCGTAAA ATCAAGGAGT ATGGACTGGA A 1251

(2) INFORMATION FOR SEQ ID NO:149

(i) SEQUENCE CHARACTERISTICS:
(A) LENGTH: 1806 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1806

(xi) SEQUENCE DESCRIPTION: SEQ ID NO:149

ATGATCCTAC TCTTCGGCGG TACTACGGAA GGCCGTGCCG CAGCTCGCGT GCTGGATGAA 60
GCGGGAAGTC CGTTTTTCTA CTCCACCAAA GGCAATCTGC AAGAGATCCA GAGTAGCCAC 120
GGCCATCGTC TGACAGGAGC CATGACGGTT GCCGACATGG TTTCGTTTTG TCGGAAAGAA 180
GAGATCCGAC TGATCGTGGA CGCCGCTCAT CCTTTCGCCG AAGAATTGCA CGCTTCAGTG 240
GCAGAAGCCT CTGAACAAAC AGGTATCCCC GTAGTAAGAT ACGAGAGACA ATACCCTCCA 300
CGCGAAGAAG GTATCGTCTG GTGTGCAAAC TACGATACGG CTGCCGAGCG GATGCTTGGC 360
GATGGCGTGC AGCGTCTGCT GATGCTCACA GGAGTGAATA CGATCCCCAA GCTGGCTGCT 420
TTCTGGAAAG AGCGCACCAC CTTTTGCCGC ATATTGAAGC GAGACGAATC GGTTGCTTTG 480
GCAGAGAAGA ACGGCTTTCC TGCGGAGCGC ATCGTTTTCT TCGAACCGCA TGCGGACGAG 540
GAGCTGATGC AAGCCGTTCG CCCCGATGCC ATTATCACAA AAGAAAGCGG AGAGAGCGGT 600
TACTTCCGAG AAAAGATAGA AGCTGCCCGA CGGATGGGCA TCCGTATATA TGCCGTCGTA 660
CGTCCCCCTT TGCCTCCTTC ATTCATTCCC GTAGGCGGGC CTGTCGGTTT GAGACCGGCG 720
GTAGAACGCC TCGTGCCGGG ATTCTTTTCA CTCCGAAGCG GATTCACTAC CGGCACCACA 780
GCTACCGCTG CAGTAGTAGC AGCCATGTAC CGATTGATGG GGCTTGGCTC TCTCGCCGAA 840
GCTCCCGTAG AATTGCCTTC GGGCGAAATA GTCAGTCTGC CCATAGCGGA AATTCGAGAG 900
GAAGAAGATG CTGTCGTATC CGCAGTCCTG AAAGATGCAG GTGATGATCC GGATGTGACC 960
AATGGCATGG CGGTATGCGC TACGATCAGG CTCAATCCCG AACATGAGGA AGTCCGCTTC 1020
CTGCAGGGTG AAGGGGTGGG GGTAGTGACG CTCCCCGGCC TCGGTCTGGA GGTCGGAGGT 1080
CCGGCTATCA ACCTCGTACC TCGACGAATG ATGACAGCAG AGGTACGCCG ACTCTATGCG 1140
CAGGGAGGTC TGGATATTAC GATTAGCGTA CCCGAAGGCC GAGAGGCTGC TACCCAGACA 1200
TTCAATCCCC GACTCGGCAT ACGGGACGGC ATCTCTATTA TCGGAACATC GGGAGTCGTG 1260
AAACCTTTTT CGGCCGAAGC GTTCGTTGGT GCCATCCGTA AGCAAGTGGG TATTGCCACC 1320
GCCTTGGGAG CCAATCATAT CGTCCTCAAT TCGGGAGCCA AGAGTGAGCG TTATGTAAAA 1380
GGAGCCTATC CGGCACTCAT TCCACAGGCC TTTGTGCAGT ATGGCAATTT CGTCGGCGAA 1440
TCACTCAGTT GTGTAGCTTC CTTCCCTTCT GTCCGTTCGG TAACGGTAGG AATCATGCTC 1500
GGCAAAGCAG TGAAACTCGC CGAAGGCTAT CTGGATACGC ACAGTAAAAA GGTAGTGATG 1560
AATCGGGATT TCCTGCACGA ACTGGCTCGT CAGGCAGGTT GTTCGGAAGA CATCCATGCC 1620
ATAATAGACA GCCTGAATTT GGCTCGTGAG CTATGGACTA TGCCGAGTGC GGAGGACAGC 1680
GATCGACTGC TACGAAAGAT TGCCGAACGA TCTTGGGAAA CTTGCCGCCC ATCGGTACCA 1740
TCGGCCGAAT TAGAACTCCT GCTGATCGAT GAGTCCGGAG CGATTCGTTT TCGTATCGGT 1800
GGAGAA 1806

(2) INFORMATION FOR SEQ ID NO:150

(i) SEQUENCE CHARACTERISTICS:
(A) LENGTH: 1329 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1329

(xi) SEQUENCE DESCRIPTION: SEQ ID NO:150

ATGTTGAGGA CTTTCCGAAT CGGTGGTATT CACCCCCCCG AAAACAAGTT GTCGGCAGGC 60
AAGCCCGTAG AGGTGTTGCC TATCCCCTCA CAGGTAGTCA TCCCTCTTGG TCAGCACATC 120
GGTGCACCGG CAACTGCCAC GGTCAAGAAA GGGGATGAAG TTAAGGTCGG GACTATCATT 180
GCTCAGGCCG GAGGATTCGT ATCAGCTAAT ATCCACTCAT CTGTGTCGGG TAAGGTGCTG 240
AAGATCGATA ACGTATACGA CTCAAGCGGC TATCCCAAGC CCGCAGTCTT CATTAGCGTA 300
GAAGGTGACG AATGGGAAGA GGGCATCGAT CGCTCACCAG CCATCGTCAA AGAATGCAAT 360
CTGGATGCAA AAGAAATCGT AGCCAAAATT TCTGCAGCCG GTATTGTGGG TCTTGGCGGT 420

GCTACCTTCC CTACCCATGT GAAGCTGTCC CCTCCTCCGG GCAACAAAGC TGAGATCCTG 480
ATCATCAACG CCGTAGAGTG CGAGCCTTAT CTGACGAGCG ACCATGTCCT TATGCTGGAG 540
CACGGCGAAG AGATCATGAT CGGCGTGAGT ATCCTGATGA AAGCCATTCA GGTAAACAAG 600
GCCGTCATCG GAGTTGAGAA TAATAAGAAA GATGCTATTG CTCACCTCAC CAAACTGGCC 660
ACTGCATATC CGGGCATAGA GGTAATGCCG TTGAAGGTGC AATATCCTCA AGGCGGTGAG 720
AAGCAGCTGA TCGATGCAGT GATCCGCAAG CAGGTAAAAA GCGGTGCCTT GCCTATCAGC 780
ACAGGTGCCG TAGTACAAAA CGTGGGTACG GTATTCGCCG TGTACGAAGC AGTACAGAAG 840
AACAAGCCTC TGGTCGAGCG CATCGTGACG GTTACAGCAA AAAAACTGTC TCGTCCGTCT 900
AACCTCCTCG TTCGTATAGG TACTCCTATT GCGGCTTTGA TCGAAGCAGC AGCTGGCTTG 960
CCGGAGAATA CGGGCAAGAT CATCGGCGGA GGTCCGATGA TGGGACGCGC TCTGCTGTCA 1020
CCGGATGTGC CTGTGACCAA AGGCAGCTCC GGAGTATTGA TTCTCGATAG AGAAGAGGCA 1080
GTTCGCAAGC CTATGCGCGA CTGTATCCGA TGCGCCAAGT GCGTCGGAGT GTGTCCGATG 1140
GGACTCAATC CGGCTTTCCT TATGCGCGAC ACCTTATATA AGAGCTGGGA AACAGCGGAA 1200
AAAGGCAACG TGGTTGACTG TATCGAATGC GGTTCGTGCA GCTTCACCTG TCCGGCCAAC 1260
CGTCCTCTGC TGGATTATAT CCGCCAAGCC AAGAAGACTG TGATGGGTAT CCAAAGAGCA 1320
CGTAAGCAA 1329

(2) INFORMATION FOR SEQ ID NO:151

(i) SEQUENCE CHARACTERISTICS:
(A) LENGTH: 1437 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1437

(xi) SEQUENCE DESCRIPTION: SEQ ID NO:151

ATGAAAAGAA TACAACTAAC TCTTATCGCT CTCTTCGCCG CTGTTGCCGG TTTGGTCGCT 60
CAAAATGCTT ACGAGGGAGT AATTTCATAT AAAATTTCGT TGGACAAAAC CGGAAACAAG 120
GTTGTACTGA ATGGTGCGGC AGATATGAGT AATTTAAAGC TCAAGAGCAC TCAGATGATC 180
ATTGTTACGC CTATTCTTCG TTCAGAAGAT GGTACCAGCC GGGTGGAATT TCCTTCGGTA 240
GTCATTACAG GCCGCAATAG AACAAAAGCT CTCAAGCCTG AAATCGCATT TAGTTCGGCT 300
TTGCCCCAAG CAAAACATGC AGCTCAATAC ATTCGCCGTC ATAATGGGAA GAGCGAGCAG 360
TTTGCTTTTA CAGGAGAACA TGCTTATGCA TCATGGATGA TGGATGCCAA GTTTGTGGTT 420
CGTGAGGAGG TACGAGGTTG TGCTAAATGC CCTGTAGGTC TCTCGAGTAA TATTGTTCCT 480
TTTGATCCAC TCTTCAATCC GGCAGAGGCT CCTTATTTGT TGGCACACAT TACTCCGGCA 540
GAAGAAGTGG AAAAACAGCG AGAGTCCAGC TTCGATGCTT ATATCAACTT CAAAGTCAAT 600
AAGGCAGATG TCCTTCCTGA GTATCGCAAC AATAAGGCGG AGTTAGAGAA AATCAAAGAA 660
TTTGTAAGCA CCGTTAAGGC TAATCCAAAC TATTCGGTCA ATAAAATGAT CATCGAAGGG 720
TTTGCTTCTC CCGAGGCTTC AATAGCCCAC AATAAGGCTT TGTCGGAGCG CCGTGCTAAA 780
AGACTCGCGG AAGAATTGGT GCGTAAGTAT GGCAAAACAT TGCCGAATAT AACCACTGAA 840
TTCGGCGGTG AAGATTGGAA GGGGCTGAAA CTGGCTATCG AAAAGAGTGA TATAGCCGAT 900
CGTGACCGCG TATTGGAGAT AATCAACTCC GATAAATATG CCGATGATGA TGCACGTGAA 960
CAGGCTCTGA AGCAACTTTC GTCTTATCGT TATATCTTGG ATCAGATCTA TCCGAATTTG 1020
CGTCGCAATA CGATAACCAT GGGGTATATC GTTCGTGATT ATACCCTCGA AGAAGCTCGT 1080
GAAATCATTA AGACTGCTCC GAAAGAACTT AGTGAGGCCG AAATGTACCG TGTGGCAATG 1140
TCTTATCCTG AGGGGCACCA AGAGCGTTTG TTTGCTCTGA ATACGACCCT TAAGTATTTC 1200
CCTGAAAGTG TAACGGGCCG AATCAATTTG GCTGTAGCCG CTTTTAATGG TGGAGACGTT 1260
CAACAGGCAA TTGCTCTGTT GAGTCCGATT CAGACAGAAA AGGGTGTAAG CAATATCCTT 1320
GGAGCTGCTT ATGCTCGTAC GGAGATTTTT GCTCGTGCCG AAACCTTCTT CCGTAAGGCC 1380
GTTGCAGAAG GAGATGCAAA TGCGCAGCGC AACCTCGATA TGCTGCTTGG CAAAAAG 1437

(2) INFORMATION FOR SEQ ID NO:152

(i) SEQUENCE CHARACTERISTICS:
(A) LENGTH: 1149 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1149

(xi) SEQUENCE DESCRIPTION: SEQ ID NO:152

ATGGCAGAAA AAAGAGACTA TTACGAAGTC CTCGGTGTAT CGAAGAATGC CACCGACGAT 60
GAACTGAAAA AAGCATATCG CAAGAAGGCT ATCCAATACC ATCCTGATAA GAACCCCGGT 120
GACAAGGAGG CCGAAGAGCA CTTCAAAGAG GTAGCTGAAG CCTACGACGT ATTGAGCGAT 180
CCGCAGAAGC GCAGTCAATA TGACCAGTTC GGCCATGCCG GATTGGGCGG AGCTGCCGGT 240
GGAGGTTTCA GCGGAGGCGG TATGTCCATG GAGGATATTT TCAGTCGCTT CGGTGATCTA 300
TTCGGTGGGT TCGGCGGTTT CGGCGGATTC TCCGATATGG GCGGTGGCAG TCGCAGACGT 360
GTTCGCAGAG GGTCTGACCT GCGAGTACGA GTGAAGCTTT CTTTGGCCGA TATAAGTAAA 420
GGTGTGCAGA AGAAAGTGAA GGTAAAAAAG CAGGTAGTGT GCAGCAAATG TCGTGGCGAT 480
GGCACGGAAG AAGCCAATGG CAAGACTACC TGCCAGACCT GCCATGGAAC CGGCGTGGTT 540
ACACGTGTGA GCAACACTTT CCTTGGGGCC ATGCAGACCC AGAGCACTTG TCCCACTTGC 600
CACGGAGAGG GTGAGATCAT CACGAAGCCA TGCTCCAAGT GTAAGGGCGA AGGTGTGGAG 660
ATCGGCGAAG AGGTGATCTC ATTCCACATC CCTGCCGGTG TAGCCGAAGG AATGCAAATG 720
TCCGTGAACG GCAAGGGAAA TGCCGCGCCC CGAGGAGGCG TGAATGGCGA CTTGATAGTC 780
GTGATCGCCG AGGAACCGGA TCCGAATCTG ATCCGCAATG GCAACGATCT GATATACAAT 840
CTGCTTATAT CCGTTCCGTT GGCTATAAAA GGAGGTAGTG TGGAAGTGCC GACGATAGAC 900
GGACGAGCCA AGATCCGCAT CGAGGCGGGG ACACAACCCG GCAAGATGCT GCGTTTGCGC 960
AATAAGGGGT TGCCCAGCGT AAACGGCTAT GGCATGGGAG ACCAACTGGT GAATGTCAAT 1020
GTCTATATCC CCGAATCGAT CGATGCCAAA GATGAGCAGG CTATCGCAGC GATGGAAAAC 1080
TCGGACAGCT TCAAACCTAC CGATGCTGCT CGTAAGGATA TAGACAAGAA ATACAGAGAG 1140
ATGCTGGAT 1149

(2) INFORMATION FOR SEQ ID NO:153

(i) SEQUENCE CHARACTERISTICS:
(A) LENGTH: 879 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...879

(xi) SEQUENCE DESCRIPTION: SEQ ID NO:153

ATGAAAAAAC TGATTTTAGC GACTTTGGGA CTTATGGCCA TTGCCATGCT CTCATGTTCA 60
AGCAACAACA AGGATTTGGA GAACAAAGGG GAGGCTACTC TTTTGGTAAC GTTTGGTAGC 120
TCCTATAAAG CTCCACGCGA AACCTATGCG AAGATTGAGA AGACTTTTGC CGCAGCTTAT 180
CCCGATCAAA GGATAAGCTG GACATACACG TCTTCTATTA TCCGAAAGAA ACTGGCTCAG 240
CAGGGTATTT ATATCGATGC TCCGGATGAG GCTTTGGAGA AATTGGCTCG TCTGGGTTAT 300
AAGAAGATCA ATGTACAGAG TCTTCATGTG ATTCCCGGCC GAGAATATGA TGAGATGATC 360
GACTTTGTCA ATAAGTTTAA GGCAGCACAT AGTGATATTA CTGTGAAGGT AGGGGCTCCG 420
CTTTTCGATA CCGATGAAGA TATGCGCGAC GTGGCAGAGA TCTTGCACAA GCGTTTTCAG 480
CAAACGATAG AGAAAGGTGA AGCTATTGTA TTCATGGGAC ACGGCACCGA GCATGCTGCC 540
AATGACAGGT ATGCCCGTAT CAATAAGATC ATGAAGAACT ATAGCAAGTT CATGATCGTC 600
GGAACCGTCG AGTCCGATCC CTCTATCAAT GATGTTATTG CCGAACTGAA AGAAACCGGT 660
GCCACGGCCG TAACAATGAT GCCGCTGATG AGTGTGGCAG GCGACCATGC TACGAATGAT 720
ATGGCCGGAG ATGAGGACGA TAGCTGGAAG ACGTTGCTGA CCAATGCCGG CTACACAGTT 780
TCTATAGACA AGCTGGACAA TGGCAATTTC TCAGCTCTTG GAGATATAGA AGAGATCCGG 840
AATATCTGGC TCAAGCATAT GAAAGCCACC TCTGCTCGC 879

(2) INFORMATION FOR SEQ ID NO:154

(i) SEQUENCE CHARACTERISTICS:
(A) LENGTH: 1068 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1068

(xi) SEQUENCE DESCRIPTION: SEQ ID NO:154

ATGACATCCG TCAGCCACTT ACGTACAATT TCTGTCGCAG GTATCCTGGC TGCGCTGGGA 60
GGGGCTGTAC TCATTCTCTT CGGGGTTAAT CTCTTCCTCG GCTCGGTGGC TATTCCGATG 120
AGCGAGATCT TCCGACATCT TTTTTCAGAT CGTCCCGAAG GAGGAGAAGC ACTCGTGCAC 180
TACAATATCC TATGGAAATC CCGCCTGCCC GAAGCCCTCA CGGCTGCTTT TGCCGGCGCA 240
GGTTTATCCG TTAGTGGCTT GCAGATGCAG ACCGTCTTTC GCAATCCTTT GGCCGGTCCG 300
TCCGTTCTCG GCATCAGCTC CGGTGCCAGT TTGGGTGTTG CTTTGGTCGT TCTGCTGAGC 360
GGCTCGCTGG GAGGAGTGGC ATTGAGTAGC CTGGGTTATA TGGGCGAGGT GGCCATGAAT 420
ATAGCCGCTG CCGTAGGCTC GCTGGCAGTA ATGGGGCTGA TCGTTTTTGT CAGCACCAAG 480
GTGCGCAGCC ACGTTACGCT GCTCATTATC GGCGTTATGA TCGGATATGT AGCCACTGCC 540
GTCATCGGGG TATTCAAGTT TTTCAGTATC GAAGAAGATA TTCGGGCATA CGTAATTTGG 600
GGGTTGGGCA GCTTTTCCCG TGCCACGGAT TCGCAACTGA GTTTCTTTGC CATTCTGATG 660
TTGATCTTTA TTCCGGCCGG TATGCTCCTT GTCAAGCAGT TGAATCTCTT ATTGCTGGGA 720
GAAAGCTACG CACGTAATCT GGGACTGAAT ACTCGTCGGG CACGGCTGCT CGTGATCTCT 780
TCCGCCGGTT TGCTCATCGC TACCGTCACG GCCTATTGCG GTCCCATCGG CTTTTTGGGG 840
ATGGCTGTGC CACACTTGGC ACGGGTTATC TTTCACACAT CGGATCATCG GATCCTGATG 900
CCTGCTACCT GTTTGATTGG AAGTGCTCTG GCTCTTTTCT GCAATATCAT TGCTCGTATG 960
CCGGGGTTTG AGGGGGCTTT GCCCGTCAAT TCCGTAACGG CTTTGGTGGG AGCACCTATT 1020
ATCGTCACCG TTTTGTTCCG GCGCAGACGC TTCAAGGAAG AAACCGAC 1068

(2) INFORMATION FOR SEQ ID NO:155

(i) SEQUENCE CHARACTERISTICS:
(A) LENGTH: 2271 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2271

(xi) SEQUENCE DESCRIPTION: SEQ ID NO:155

ATGCGGACAA AAACTATCTT TTTTGCGATT ATCTCTTTTA TTGCTCTATT GTCGTCTTCT 60
CTGTCGGCTC AGAGCAAAGC CGTTTTAACC GGTAGTGTGT CGGATGCCGA AACCGGAGAG 120
CCTCTTGCCG GTGCTCGAAT CGAAGTCAAA CACACCAACA TAGTAGCCGG TGCCGATGCC 180
GGCGGACATT TCGAGATCAA GAACCTGCCG GCAGGGCAGC ATACTATTAT ATGTTCGTTG 240
GGGGGGTATG GACAGAAAGA GGAGGTGGTT GCCATCGAAG CCGGACAGAC CAAAACGATC 300
TCTTTTGCAT TGCGACTGCG AACGAACAAC TTGGAGGAAG TCGTCGTTAC CGGTACCGGT 360
ACACGTTACC GCTTGGTCGA TGCTCCTGTG GCAACGGAAG TCCTTACCGC TAAGGACATA 420

GCCTCTTTCT CGGCTCCTAC TTCCGAGGCC TTATTGCAGG GGCTGAGTCC GTCTTTTGAC 480
TTCGGCCCCA ATCTGATGGG CTCTTTCATG CAGCTGAACG GCCTTAGCAG TAAGTATATC 540
CTCATCCTTA TCGATGGTAA GCGTGTGTAC GGCGATGTAG GCGGTCAGGC CGATTTGAGT 600
CGTATTTCTC CTGATCAGAT CGAACGGATC GAACTGGTGA AAGGTGCTTC GAGTTCGCTC 660
TACGGATCCG ATGCCATCGC CGGGGTAATC AATGTGATCA CAAAAAAGAA TACGAATCGA 720
CTGAGTGCAT ATACGTCACA TCGCATATCG AAGTACAACG ATCGGCAAAC CAATACTTCG 780
CTCGATATAA ACATCGGTAA GTTCAGTAGC AATACCAACT ATTTCTTCTA CCATACGGAT 840
GGCTGGCAGA ATAGTCCGTT CGAAATAAAA AAGAAAAAAG GATCCGGCGA ACCGGTCTTG 900
GAGGAAACGT ATAAGAAAAC TTTTCGTGCA CAGGAAAATC AGGGTGTAAG CCAATCGCTT 960
TCCTATTATG CAACTAACAA TCTTAGCTTC AGCGGAAATG TGCAGTACAA TAAACGTCAG 1020
ATCTTCACTC CGACTTTTTC CGAAAAGAAG GCCTATGACA TGGATTATCG TGCTTTGACG 1080
GCTTCACTCG GTACGAACTA TCTTTTCCCC AATGGTCTGC ATACGCTTTC TTTCGATGCC 1140
GTCTACGATC GCTTCCGTTT CGGATATTTG TATCATGACA AGGACAGCAG TGAGAGCCTG 1200
ATCAACAACC AAGGTCAGAC CGAGCAACCC ACATTCTTTC CGGGTCAGCT ACGCAATAAA 1260
AACGATCAGA TCCGATACAC GGCAGAGGCT CGCGGTGTAT TTACACTGCC TTATGCGCAG 1320
AAACTGACCG GCGGTTTGGA GTATTTCCGT GAGGAATTGA TCTCTCCCTA TAATTTGATT 1380
ACCGACAAGG CAGATGCTTC CACGCTCTCT GCTTATGTAC AAGATGAATG GAAACCGCTC 1440
GATTGGTTCA ATATGACAGC CGGTTTCCGT CTGGTACACC ATCAGGAGTT CGGTACACGA 1500
ATGACGCCTA AGGTATCCAT ACTCGCCAAG TATGGCCGC TGAACTTCCG CGCTACGTAT 1560
GCTAACGGCT ATAAGACTCC CACGCTGAAA GAGCTTTTTG CACGGAACGA ACTCACCACT 1620
ATGGGTTCGC ACAATCTCTA TCTCGGCAAT GCGGATCTTA AGCCACAGAT GTCGGATTAT 1680
TATGCTTTGG GCTTGGAGTA CAATCAAGGC CCTATCTCGT TCAGTGCAAC GGTTTATGAC 1740
AATGAACTTC GCAATCTGAT CTCCTTTATG GATATACCGA CCTCACCCGA GCACGAAGCT 1800
CAGGGAATCA AGAAAACCAA GCAGTATGCC AACATAGGAA AAGCTCGCAG CCGCGGCCTT 1860
GATGTCCTAT GTGATGCCTC TATCGGTTGG GGTATCAAGT TAGGAGCCGG ATACAGCCTC 1920
GTGGAAGCTA AGAATCTCCA GACGGATGAG TGGCTGGAAG GAGCTGCACG TCATCGTGCC 1980
AATGTGCACG CCGATTGGGT TCACTACTGG GGTCAGTATA GACTTGGCGT GAGCCTTTTC 2040
GGCCGTATTC AGAGCGAGCG TTACTACAAA GACGGCAATG CTCCGGACTA TACCTTGTGG 2100
CGACTCGCCA CATCGCATCG TTTCGCTCAT TTCCGCCACA TCATCCTGGA TGGAACGCTC 2160
GGTATAGACA ACCTGTTTGA CTACGTGGAT GATCGTCCTA TGGGTGTCAA TTATGCTACC 2220
GTAACGCCGG GACGTACTTT CTTTGCTCAA ATAGCGATTC GATTCAACAA C 2271

(2) INFORMATION FOR SEQ ID NO:156

(i) SEQUENCE CHARACTERISTICS:
(A) LENGTH: 993 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...993

(xi) SEQUENCE DESCRIPTION: SEQ ID NO:156

ATGACGGACA ACAAACAACG TAATATCGTA TTCCCGGCGT TTCTCCTCTT GCTGGGAGTC 60
ATCGCAGTGG TGACGATCGT TGGTTTTTTC ATGCTCAGAC CGGCCGAGGA GATTATCCAA 120
GGACAGATAG AAGTGACCGA ATACCGAGTG TCCAGCAAAG TGCCCGGGCG CATCAAGGAA 180
CTTAGGGTAT CCGAGGGACA GCAGGTGCAG GCCGGCGATA CCCTCGCTGT CATCGAAGCC 240
CCCGACGTAG CGGCTAAGAT GGAGCAGGCA AAGGCTGCCG AAGCAGCTGC ACAGGCTCAG 300
AACGCCAAGG CTCTCAAAGG AGCACGCAGC GAACAGATAC AGGCAGCCTA TGAGATGTGG 360
CAGAAAGCTC AGGCCGGCGT AGCCATAGCG ACCAAGACAC ACCAGCGCGT GCAGAACCTC 420
TATGACCAGG GAGTGGTACC GGCTCAGAAG TTGGACGAAG CCACTGCCCA GCGCGATGCG 480
GCCATCGCTA CGCAAAAAGC GGCCGAAGCC CAGTACAATA TGGCTCGCAA CGGTGCCCAA 540
CGCGAAGACA AGCTGGCAGC TTCTGCCCTC GTCGATAGAG CGAGAGGAGC CGTCGCCGAG 600
GTGGAGTCGT ACATCAACGA AACCTACCTC ATCGCCCCAC GGGCAGGCGA AGTGTCGGAG 660
ATATTCCCCA AAGCCGGCGA ACTCGTAGGT ACCGGCGCAC CTATCATGAA TATCGCCGAG 720
ATGGCGATA TGTGGGCCAG CTTTGCCGTT CGTGAGGATT TCCTCAGCAG CATGACCATG 780
GGAGCCGTTC TGGAGACTGT GGTGCCGGCT CTGAATGAAG AAAAAGTACG CTTCAAGATC 840
ACATTCATCA AGAACATGGG TACCTATGCT GCCTGGAAAG CGACCAAGAC AACAGGGCAG 900
TACGACCTGA AGACCTTCGA GGTAAAGGCC ACCCTTGCGG ATAAAGACAA GGCACAAAAG 960
CTACGCCCGG GTATGTCCGT GATCATACGC AAG 993

(2) INFORMATION FOR SEQ ID NO:157

(i) SEQUENCE CHARACTERISTICS:
(A) LENGTH: 801 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...801

(xi) SEQUENCE DESCRIPTION: SEQ ID NO:157

ATGCGTATTG TCAGTAATTT TTTGTTCGTC TCTTTTTCGG TTTTGCTTTT TGCATCATGC 60
CGTTCCCAGC GAGAAAAGGT CGTTTACCTG CAAGATATCC AAACTTTTAA TCGGGAGATT 120
ATCGCTAAAC CATATGACGT AAAAATTGAG AAGGACGATG TGCTGAACAT CCTTGTCAGC 180
AGTAGAGACC CGGAGCTTTC AACGCCCTAC AACCAAGTGT TGACCACTCG TGCACTGGCC 240
CGCAACGGCT ATGGAACGAA CTCGAACGAA GGCTTCCTGG TCGATTCGAA AGGGTACATC 300
AATTATCCTA TTTTAGGCCA GATCTATGTA GAGGGCCTTA CTCGTACCGA ACTGGAGAAG 360
GAGATACAGA AGAGGATTAT TTCCAGTGGA TTTATCAAGG ATCCTACGGT AACGGTGCAG 420
CTTCAAAATT TCAAGGTGTC GGTTTTGGGA GAGGTGAATC ATCCGGGTTC GATGTCGGTA 480
AAAGGAGAGC GAATAACTCT TTTGGAAGCG ATCGGAATGG CCGGAGACCT GACAATCTAT 540
GGTCGCCGCG ATCGGGTTTT TGTGATTAGA GAAACCGATG GGCATCGCGA GGTTTTCCAG 600
ACGGATCTCA GAAAGGCCGA CTTGCTCGCA AGCCCCGTGT ACTATCTGCA TCAGAACGAC 660
GTCATCTATG TGGAGCCGAA CGACAAGAAA ACACAGATGA GCGAGATCAA CCAGAATAAT 720
AACGTAAACG TATGGCTGAG TGTTACCTCC ACTTTGGTAT CCATTTCCAC GCTGACGATT 780
ACGATAATAG ATAAGACCAA A 801

(2) INFORMATION FOR SEQ ID NO:158

(i) SEQUENCE CHARACTERISTICS:
(A) LENGTH: 1707 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1707

(xi) SEQUENCE DESCRIPTION: SEQ ID NO:158

ATGAAAAAGA CCAATCTGTT TTTATCTCTG CTGGTGATCT TTATCACCGG TAGTTTTATG 60
ACTGCCTGTG CACAGAAGTC CAAGACGAAC AAACTCACCG AAGAAGATCG GAGCCGCAAT 120
GAGTATGTAC AGTCGATGGA TGTGCTTAGC AATATTATCG GTAACGTCAG GCTGTATTTC 180
GTCGATACCA TAAGTATCAA ACATATGACT CGGCGTGGTA TAGATGCGAT GTTGGGCGGG 240
CTTGACCCCT ATACCGAATA CATTCCTTAC GAGGAAATGG ATGAACTGAA ATTGATGACT 300
ACGGGAGAGT ATGCCGGAGT CGGAGCTATC ATATCGCAGC GCCCGGATAG TGCTGTGATT 360
ATCCAGAGAC CTATGGAAGG TATGCCCGCA GACGAAGCAG GATTGATAGC AGGCGACCGC 420
ATCCTGACTA TCGATGGGAA AGACTTCCGC AAATCCACCA CACCGAAAGT AAGCCAAGCA 480
CTGAAAGGGA TAGCCGGTAC TGTTGCAAAG GTGACAGTAA TGCGCTATGG CGAAACCAAA 540
CCTCGTACTT TTTCCGTGAA ACGTCAAAAA GTGATTATGA ATTCCGTCAC TTACAGCGGA 600
ATGCTCGATG GCTCGATAGG ATATATCCGC TTGAACAACT TTACGGACAA AAGTGCAGAA 660
GAGGTGCGCA CGGCCTTGTT GGATCTTCGT GACAAACAAG GAGCGAAAGG TCTCATTTTG 720
GATTTAAGAG GCAATGGTGG CGGACTGATG CAGGCTGCTA TCGAGATAGT CAATCTGTTC 780

GTCCCTAAGG GCAAAGAGGT GGTAACGACC AAAGGTCGCA TTGCAGAGTC GGCGTCCGTA 840
TTTCGCACAT TGACTGAACC GATCGACACG AAACTCCCGA TAGTAGTCCT GATCGATGGA 900
CAATCGGCAT CTTCCTCGGA GATTGTAGCC GGAGCACTGC AGGATATGGA CAGGGCTGTA 960
CTGATGGGAC AAAAGAGCTA TGGCAAAGGG CTTGTACAAA CGACTCGTCA GCTACCATAC 1020
AACGGCGTGA TCAAATTGAC TACGGCCAAG TACTACATCC CAAGCGGACG TTGTATTCAG 1080
CGTTTGGACT ACAGCCGCAC CAATCGGACA GGTATGGCAA CGGCCATTCC TGACAGTCTG 1140
CACAAAATCT TTTACACTGC TGCCGGAAGA CGTGTAGAAG ATGCAGGAGG AATCCTGCCT 1200
GACATCGAGG TCAAACAAGA TACAGCTGCG ACATTACTTT ATTATATGGC CATCAATAAT 1260
GACGTTTTCG ATTTCGTCAC AGGTTATGTG CTCAAGCATA AAACGATTGC CAAGCCGGAG 1320
GATTTTTCCA TAACGAACGA GGACTATGCA GCTTTCTGCA AGATGATGGA AGAAAAGAAA 1380
TTTGACTATG ATCGCCAGAG TGGCAAGATG CTTGACAAAC TGGAGGAACT GGCTAAGATA 1440
GAAGGCTACC TGCCGGAAGC CAACTCGGAG CTTAAAGCAC TACGCGAAAA GCTAAAACCC 1500
AACCTGTCGC GTGATCTGCT ACGATTCAAA AAGGAGATAA CAAACTATCT CAACAATGAG 1560
ATTGTCACTC GCTATTATTA TGAGCGAGGC AGTATCCGCC AGAGTTTGCC GGAAGATAAG 1620
GTAGTCAAAG AAGCTATTAA GCTGCTGAAG GACCATCCGG AACAAATTCG ACAGATCCTT 1680
GCAGCTCCGA AAGCAGAGAA TAAAGGG 1707

(2) INFORMATION FOR SEQ ID NO:159

(i) SEQUENCE CHARACTERISTICS:
(A) LENGTH: 2943 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2943

(xi) SEQUENCE DESCRIPTION: SEQ ID NO:159

ATGCAAAACA AAGGATTTGT GATTGTTATC ACATCGGCTC TGGCCATCAT CTGTGCGTTT 60
TACCTGTCAT TCTCTTTCGT TACGAACCGT TACGAAAAGA AGGCTAAGGC GATGGGCGAT 120
GTTGCCGGAA TGGCCTATCT TGATTCCATG TCGAATGAGA AGGTCTGGTT CGGCTACACG 180
CTGAAAGAAG CTCAAGCCCA GCAAATTGGT CTTGGCCTTG ACTTAAAGGG GGGTATGAAC 240
GTTATCTTGA AACTTAACGC AAGCGATCTG CTTCGTAACC TCTCTAACAA AAGTTTGGAT 300
CCCAACTTCA ACAAAGCTCT GGAGAATGCT GCCAAGAGCA CGGAGCAATC CGACTTCATC 360
GATATTTTCG TGAAGGAATA TCGCAAGCTC GATCCCAACG GTCGCTTGGC CGTTATCTTC 420
GGTTCGGGTG ACCTTCGCGA CCAGATTACC GCAAAGTCTA CGGATGCAGA CGTAGTGCGT 480
CTGCTCAAAG AAAAATATAA TAGTGCTGTA GAAGCTTCGT TCAATGTGCT CCGTGCTCGT 540
ATCGATGCTT TCGGTGTGGT TGCACCTAAT TTGCAGCGAT TGGAAGGGCA AGGGCGTATC 600
CTTGTCGAAC TCCCCGGAGT GAAAGACCCT GAGCGTGTTC GTACCCTTTT GCAACGCAGT 660
GCCAACCTAC AGTTCTGGCG TACATACAAA TTCGAAGAGG TCAGCGGAGA CTTGATCGCT 720
GCCAATGATC GTCTGAGCGA ATTGGCTATG AACAACACGG ATGCTACCCC GGAAACAGAG 780
CCTGCAACTA CTGACTCTGT AGCTGCAACA GCCGATTCTG CTGCTGTACA AGCTGTAGCT 840
GATTCTGCTA CTGTAGCACA AAAAGAGGCC AAGGATGCTA CTCGTAAAGA CGCACTCTTC 900
TCTCTGCTTA CTCCCGTGAA TCGTGGCGGT GCAGTAGTGG GTGTGGCTCG TCGTGCTAAT 960
ATGGCTCAGA TATCTGAAAT GCTCCAGCAA GCTCACGATC TGAAGGTTAC ACGTGAAGAT 1020
GTGCTTTTCC TCTGGGGTGC TAAAGCAATC GAAGACCCCG AAACCAAAAA GGAGACCGAC 1080
CTCTACGAAC TCTATGCTAT TCGTACCAAT CGTACGGGAG ATCCTGATTT GGGAGGTGAT 1140
GTAGTGACTT CCGCCAAGAG TGATATCCAA AATGACTTCG GTCGTTCCGA ACCGATCGTT 1200
TCGATGACGA TGAATGAAGA AGGTGCTCGT AAATGGGCGC GTATCACAAA GGATAACGTG 1260
GGACGGGCAA TCGCTATCGT TTTGGATGGT GTGGTTTATT CTGCTCCGAA CGTGAATGAT 1320
GAGATCACGG GCGGTCGCTC TCAGATCTCC GGGCACTTCA CCGTGGAGGA GGCCGGTGAC 1380
CTTGCCAACG TACTCAACTC CGGTAAAATG GATGCTACGG TAAGCATCGA ACAGGAAAAC 1440
GTGATTGGTC CTACGCTGGG TGCCGAGTCC ATTAAAGCCG GATTCTTGTC GTTCCTGCTC 1500
GCTTTGGTTA TCCTGATGTG TTACATGTGT CTGGCTTACG GTTTCTTGCC GGGTCTTATC 1560
GCAAACGGCG CATTGATTGT AAACAGCTTC TTCACATTGG GCGTATTGGC TTCTTTCCAT 1620
GCCGTGCTGA CCCTCTCGGG TATCGCAGGT TTGGTGCTGA CGCTGGGTAT GGCTGTGGAT 1680
GCCAACGTAC TTATCTTCGA GCGTATCAAA GAAGAGCTTC GTGCCGGTAA GACTCCGATT 1740
CGTGCCGTTA CGGATGGTTA TGGCAACGCT TTCTCTGCCA TCTTCGACTC GAACGTTACG 1800
ACTATTATTA CCGGTATCAT CCTATTCCTC TACGGGACGG GGCCGATTCG CGGTTTTGCC 1860
ACTACGTTGA TTATCGGTCT TATCGCTTCT TTCATTACGG CTGTCTTCTT GACTCGTATC 1920
GTCTTCGAGA AACTGGCGAA AAAAGGTCGT TTGGATAAGA TTACATTCAC TACGAGCATT 1980
ACTCGCAATC TCCTTGTCAA TCCCTCATAC AACATCTTGG GTAAGCGCAA GACCGGCTTT 2040

ATCATTCCGG TGATTATCAT CGTTTTGGGA CTTATAGCTT CATTTACAAT CGGTCTCAAT 2100
AGGGGTATTG AATTCTCCGG AGGACGTAAC TACGTAGTTA AATTCGACCA GCCTGTATCT 2160
TCCGAAGCCG TTCGTTCGGC CTTGTCTTCT CCCCTGCAGG AAAAGGTATT GGTTACCTCC 2220
ATCGGTACTG AAGGGACAGA GGTGCGTATA TCTACGAACT ATAAGATCCA GGAGGAAAGC 2280
GAAGAAACTG AAGCAGAGAT TACTGACAAA TTGTATCAGA GCCTGAAAGG TTTCTACACC 2340
CAGCAGCCTA CTGCTGATCA GTTCTTGGAC AATATCATTA GCTCTCAGAA AGTAAGTCCC 2400
AGTATGTCGA GTGACATCAC GAGAGGTGCT ATTTGGGCTG TGCTGTTATC GATGATCTTC 2460
ATGGCCATTT ATATTCTGAT TCGCTTCCGT GACATTTCTT TCTCTGCCGG GGTATTCGTA 2520
TCTGTGGCCG CTACTACATT CTGCATTATT GCTCTGTATG CGTTGCTGTG GAAGATTCTG 2580
CCCTTCACCA TGGAGATCGA TCAGAACTTC ATCGCTGCTA TTCTGGCTAT CATCGGTTAC 2640
TCGCTCAATG ACACCGTGGT TGTATTTGAC CGTATCCGAG AGACGATGAA ATTGTACCCC 2700
AACAGAGATC GCTATCAGGT GATCAACGAT GCCCTTAATT CAACATTGGG TCGAACATTA 2760
AATACGTCTT TGACTACGTT TATCGTTATG TTGGTAATCT TCATCTTTGG AGGTGCTACG 2820
ATGCGTAGTT TCACGTTCTC GATCCTGCTC GGTATCGTTA TCGGTACATA CTCTACGCTC 2880
TTTGTTGCTA CACCCCTTGC CTACGAGATC CAAAAGCGCA AGCTCAACAA AGCAGCTAAG 2940
AAA 2943

(2) INFORMATION FOR SEQ ID NO:160

(i) SEQUENCE CHARACTERISTICS:
(A) LENGTH: 3051 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...3051

(xi) SEQUENCE DESCRIPTION: SEQ ID NO:160

ATGAAAAGAA TGACGCTATT CTTCCTTTGC TTGCTGACGA GCATTGGGTG GGCTATGGCC 60
CAGAATAGAA CCGTGAAGGG TACAGTTATC TCCTCCGAGG ATAATGAGCC CCTGATCGGC 120
GCGAATGTCG TGGTTGTCGG AAACACCACT ATCGGTGCTG CAACCGACTT GGATGGCAAC 180
TTCACGCTTA GCGTGCCTGC CAATGCCAAA ATGTTGAGAG TGTCCTATTC CGGTATGACT 240
ACCAAAGAGG TCGCCATCGC TAATGTGATG AAGATCGTAC TGGATCCGGA CTCTAAGGTT 300
CTGGAGCAGG TAGTTGTATT GGGTTACGGT ACGGGACAGA AACTCAGCAC TGTTTCCGGT 360
TCTGTGGCCA AAGTGTCCAG CGAAAAGCTC GCGGAAAAGC CCGTTGCCAA TATCATGGAT 420
GCCCTCCAAG GTCAGGTAGC CGGTATGCAG GTTATGACTA CATCCGGTGA CCCTACTGCC 480
GTCGCTTCTG TGGAGATCCA TGGTACAGGG TCGTTGGGGG CAAGCTCTGC ACCATTGTAT 540
ATCGTGGATG GTATGCAAAC TTCTTTGGAT GTTGTGGCTA CGATGAATCC GAATGATTTT 600
GAATCTATGT CCGTTTTGAA AGATGCTTCT GCAACATCTA TTTATGGAGC TCGTGCTGCA 660
AACGGAGTCG TTTTCATTCA AACGAAGAAA GGTAAAATGA GCGAGAGAGG TCGTATTACC 720
TTTAATGCCA GTTACGGGAT TTCTCAAATC CTGAATACTA AGCCCCTTGA TAATATGATG 780
ACTGGAGATG AATTGCTGGA TTTTCAGGTG AAGGCAGGTT TTTGGGGGAA CAATCAAACC 840
GTTCAGAAGG TTAAAGATAT GATCCTTGCC GGAGCTGAAG ATTTGTATGG CAATTATGAT 900
TCTTTGAAAG ATGAGTATGG TAAGACATTG TTCCCAGTGG ATTTTAATCA TGATGCAGAC 960
TGGCTCAAGG CTTTGTTTAA AACAGCACCC ACCAGTCAAG GTGATATTTC TTTCTCCGGA 1020
GGGTCTCAGG GAACTTCATA TTATGCCTCT ATAGGCTACT TCGATCAGGA AGGTATGGCT 1080
CGTGAACCGG CAAATTTTAA GCGCTATAGT GGCCGGCTCA ACTTCGAAAG TCGTATCAAT 1140
GAATGGCTGA AAGTTGGTGC AAATTTGTCT GGTGCGATAG CGAATAGACG ATCTGCCGAC 1200
TATTTTGGAA AGTATTATAT GGGGTCAGGT ACTTTCGGTG TGTTAACGAT GCCTCGTTAT 1260
TATAACCCTT TTGATGTGAA TGGGGATTTA GCAGATGTCT ATTACATGTA TGGAGCTACC 1320
AGACCTTCTA TGACAGAACC GTACTTCGCA AAAATGAGAC CGTTCAGTTC CGAATCACAT 1380
CAGGCCAATG TAAATGGTTT CGCCCAGATT ACTCCGATCA AAGGCCTTAC TTTAAAGGCA 1440
CAGGCTGGTG TTGATATTAC TAATACTCGC ACTTCTTCTA AGAGAATGCC CAATAATCCG 1500
TATGATTCTA CTCCTCTTGG GGAAAGAAGA GAAAGAGCTT ATCGAGATGT TAGCAAGTCT 1560
TTTACAAATA CGGCTGAATA TAAGTTTTCA ATTGATGAAA AACATGATCT TACAGCATTG 1620
ATGGGGCATG AATATATTGA ATATGAAGGG GATGTTATTG GGGCATCTTC TAAAGGATTT 1680
GAAAGTGATA AGTTGATGTT ACTGAGCCAG GGAAAAACCG GAAATAGTTT GTCTTTGCCT 1740
GAACACAGAG TCGCTGAATA TGCCTATTTG TCTTTCTTTA GTCGTTTTAA TTACGGTTTT 1800
GACAAATGGA TGTATATAGA TTTCTCTGTT CGTAATGACC AATCCTCTCG ATTCGGATCC 1860
AATAATAGAA GCGCGTGGTT CTATTCTGTC GGTGGAATGT TTGACATATA TAATAAATTC 1920
ATTCAAGAAA GTAATTGGCT CAGTGATCTT CGACTGAAAA TGAGTTATGG TACAACGGGT 1980
AACTCGGAGA TTGGTAATTA CAACCACCAA GCACTCGTTA CTGTGAACAA TTATACTGAA 2040

GATGCTATGG GGCTTAGCAT TTCTACAGCA GGCAATCCCG ACCTCTCGTG GGAAAAGCAG 2100
TCTCAGTTCA ACTTCGGTTT GGCTGCAGGG GCTTTCAATA ATCGCTTATC TGCAGAGGTA 2160
GATTTCTATG TCCGCACTAC GAATGATATG TTGATTGATG TCCCGATGCC TTATATCAGT 2220
GGTTTCTTCT CACAGTATCA GAATGTAGGC TCTATGAAAA ATACGGGTGT AGACCTTTCT 2280
CTTAAGGGGA CGATCTACCA AAATAAGGAC TGGAATGTAT ATGCTTCTGC GAATTTCAAC 2340
TACAATAGAC AGGAAATAAC AAAGCTTTTC TTCGGTCTCA ATAAGTACAT GTTGCCTAAT 2400
ACCGGTACTA TATGGGAAAT TGGGTACCCC AATTCGTTCT ATATGGCTGA ATATGCTGGA 2460
ATCGACAAAA AAACCGGTAA GCAGTTGTGG TATGTTCCTG GTCAAGTCGA TGCGGATGGT 2520
AATAAAGTTA CAACAAGCCA GTACTCAGCT GACTTGGAGA CACGAATTGA TAAGTCTGTT 2580
ACTCCTCCTA TTACAGGTGG TTTCTCCTTA GGTGCTTCTT GGAAAGGACT TTCTTTAGAT 2640
GCTGATTTTG CCTACATCGT TGGTAAATGG ATGATCAATA ATGACCGTTA CTTTACAGAG 2700
AATGCAGGTG GATTGATGCA ATTAAATAAA GATAAAATGC TATTGAATGC CTGGACAGAG 2760
GATAATAAAG AAACAGATGT TCCAAAATTG GGACAGTCTC CTCAGTTTGA TACGCATTTG 2820
TTGGAGAATG CTTCTTTCCT GCGTTTGAAG AATCTCAAAC TCACCTATGT ACTCCCCAAT 2880
AGTCTTTTTG CTGGGCAGAA TGTGATTGGT GGAGCTCGTG TCTATTTGAT GGCGCGCAAT 2940
CTGTTAACTG TTACGAAGTA TAAAGGCTTT GACCCTGAAG CAGGGGGGAA TGTGGAAAAA 3000
AATCAATATC CTAATTCTAA GCAGTACGTT GCGGGTATTC AGTTGTCTTT C 3051

(2) INFORMATION FOR SEQ ID NO:161

(i) SEQUENCE CHARACTERISTICS:
(A) LENGTH: 3042 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...3042

(xi) SEQUENCE DESCRIPTION: SEQ ID NO:161

ATGACGCTAT TCTTCCTTTG CTTGCTGACG AGCATTGGGT GGGCTATGGC CCAGAATAGA 60
ACCGTGAAGG GTACAGTTAT CTCCTCCGAG GATAATGAGC CCCTGATCGG CGCGAATGTC 120
GTGGTTGTCG GAAACACCAC TATCGGTGCT GCAACCGACT TGGATGGCAA CTTCACGCTT 180
AGCGTGCCTG CCAATGCCAA AATGTTGAGA GTGTCCTATT CCGGTATGAC TACCAAAGAG 240
GTCGCCATCG CTAATGTGAT GAAGATCGTA CTGGATCCGG ACTCTAAGGT TCTGGAGCAG 300
GTAGTTGTAT TGGGTTACGG TACGGGACAG AAACTCAGCA CTGTTTCCGC TTCTGTGGCC 360
AAAGTGTCCA GCGAAAAGCT CGCGGAAAAG CCCGTTGCCA ATATCATGGA TGCCCTCCAA 420
GGTCAGGTAG CCGGTATGCA GGTTATGACT ACATCCGGTG ACCCTACTGC CGTCGCTTCT 480
GTGGAGATCC ATGGTACAGG GTCGTTGGGG GCAAGCTCTG CACCATTGTA TATCGTGGAT 540
GGTATGCAAA CTTCTTTGGA TGTTGTGGCT ACGATGAATC CGAATGATTT TGAATCTATG 600
TCCGTTTTGA AAGATGCTTC TGCAACATCT ATTTATGGAG CTCGTGCTGC AAACGGAGTC 660
GTTTTCATTC AAACGAAGAA AGGTAAAATG AGCGAGAGAG GTCGTATTAC CTTTAATGCC 720
AGTTACGGGA TTTCTCAAAT CCTGAATACT AAGCCCCTTG ATAATATGAT GACTGGAGAT 780
GAATTGCTGG ATTTTCAGGT GAAGGCAGGT TTTTGGGGGA ACAATCAAAC CGTTCAGAAG 840
GTTAAAGATA TGATCCTTGC CGGAGCTGAA GATTTGTATG GCAATTATGA TTCTTTGAAA 900
GATGAGTATG GTAAGACATT GTTCCCAGTG GATTTTAATC ATGATGCAGA CTGGCTCAAG 960
GCTTTGTTTA AAACAGCACC CACCAGTCAA GGTGATATTT CTTTCTCCGG AGGGTCTCAG 1020
GGAACTTCAT ATTATGCCTC TATAGGCTAC TTCGATCAGG AAGGTATGGC TCGTGAACCG 1080
GCAAATTTTA AGCGCTATAG TGGCCGGCTC AACTTCGAAA GTCGTATCAA TGAATGGCTG 1140
AAAGTTGGTG CAAATTTGTC TGGTGCGATA GCGAATAGAC GATCTGCCGA CTATTTTGGA 1200
AAGTATTATA TGCGGTCAGG TACTTTCGGT GTGTTAACGA TGCCTCGTTA TTATAACCCT 1260
TTTGATGTGA ATGGGGATTT AGCAGATGTC TATTACATGT ATGGAGCTAC CAGACCTTCT 1320
ATGACAGAAC CGTACTTCGC AAAAATGAGA CCGTTCAGTT CCGAATCACA TCAGGCCAAT 1380
GTAAATGGTT TCGCCCAGAT TACTCCGATC AAAGGCCTTA CTTTAAAGGC ACAGGCTGGT 1440
GTTGATATTA CTAATACTCG CACTTCTTCT AAGAGAATGC CCAATAATCC GTATGATTCT 1500
ACTCCTCTTG GGGAAAGAAG AGAAAGAGCT TATCGAGATG TTAGCAAGTC TTTTACAAAT 1560
ACGGCTGAAT ATAAGTTTTC AATTGATGAA AAACATGATC TTACAGCATT GATGGGGCAT 1620
GAATATATTG AATATGAAGG GGATGTTATT GGGGCATCTT CTAAAGGATT TGAAAGTGAT 1680
AAGTTGATGT TACTGAGCCA GGGAAAAACC GGAAATAGTT TGTCTTTGCC TGAACACAGA 1740
GTCGCTGAAT ATGCCTATTT GTCTTTCTTT AGTCGTTTTA ATTACGGTTT TGACAAATGG 1800
ATGTATATAG ATTTCTCTGT TCGTAATGAC CAATCCTCTC GATTCGGATC CAATAATAGA 1860
AGCGCGTGGT TCTATTCTGT CGGTGGAATG TTTGACATAT ATAATAAATT CATTCAAGAA 1920
AGTAATTGGC TCAGTGATCT TCGACTGAAA ATGAGTTATG GTACAACGGG TAACTCGGAG 1980

ATTGGTAATT ACAACCACCA AGCACTCGTT ACTGTGAACA ATTATACTGA AGATGCTATG 2040
GGGCTTAGCA TTTCTACAGC AGGCAATCCC GACCTCTCGT GGGAAAAGCA GTCTCAGTTC 2100
AACTTCGGTT TGGCTGCAGG GGCTTTCAAT AATCGCTTAT CTGCAGAGGT AGATTTCTAT 2160
GTCCGCACTA CGAATGATAT GTTGATTGAT GTCCCGATGC CTTATATCAG TGGTTTCTTC 2220
TCACAGTATC AGAATGTAGG CTCTATGAAA AATACGGGTG TAGACCTTTC TCTTAAGGGG 2280
ACGATCTACC AAAATAAGGA CTGGAATGTA TATGCTTCTG CGAATTTCAA CTACAATAGA 2340
CAGGAAATAA CAAAGCTTTT CTTCGGTCTC AATAAGTACA TGTTGCCTAA TACCGGTACT 2400
ATATGGGAAA TTGGGTACCC CAATTCGTTC TATATGGCTG AATATGCTGG AATCGACAAA 2460
AAAACCGGTA AGCAGTTGTG GTATGTTCCT GGTCAAGTCG ATGCGGATGG TAATAAAGTT 2520
ACAACAAGCC AGTACTCAGC TGACTTGGAG ACACGAATTG ATAAGTCTGT TACTCCTCCT 2580
ATTACAGGTG GTTTCTCCTT AGGTGCTTCT TGGAAAGGAC TTTCTTTAGA TGCTGATTTT 2640
GCCTACATCG TTGGTAAATG GATGATCAAT AATGACCGTT ACTTTACAGA GAATGCAGGT 2700
CGATTGATGC AATTAAATAA AGATAAAATG CTATTGAATG CCTGGACAGA GGATAATAAA 2760
GAAACAGATG TTCCAAAATT GGGACAGTCT CCTCAGTTTG ATACGCATTT GTTGGAGAAT 2820
GCTTCTTTCC TGCGTTTGAA GAATCTCAAA CTCACCTATG TACTCCCCAA TAGTCTTTTT 2880
GCTGGGCAGA ATGTGATTGG TGGAGCTCGT GTCTATTTGA TGGCGCGCAA TCTGTTAACT 2940
GTTACGAAGT ATAAAGGCTT TGACCCTGAA GCAGGGGGGA ATGTGGGAAA AAATCAATAT 3000
CCTAATTCTA AGCAGTACGT TGCGGGTATT CAGTTGTCTT TC 3042

(2) INFORMATION FOR SEQ ID NO:162

(i) SEQUENCE CHARACTERISTICS:
(A) LENGTH: 2463 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2463

(xi) SEQUENCE DESCRIPTION: SEQ ID NO:162

ATGAAGAAAA AGAATTTTTT GCTTCTTGGC ATTTTCGTTG CTTTGCTGAC TTTCATCGGC 60
AGCATGCAGG CACAACAGGC CAAAGATTAT TTCAACTTTG ACGAACGGGG CGAGGCCTAC 120
TTCTCATTCA AAGTGCCTGA TAGGCCCGTT CTACAAGAGC TGGCTCTGAT CATGTCCATC 180
GACGAGTTTG ACCCCGTAAC CAATGAAGCC ATTGCCTATG CCAGCGAAGA GGAGTTCGAG 240
GCATTCCTGC GCTATGGGCT CAAGCCTACA TTCTTGACTC CTCCATCCAT GCAGCGCGCT 300
GTCGAGATGT TCGACTACCG CTCAGGAGAA AAATACGAAT GGAATGCTTA CCCCACCTAT 360
GAAGCCTATA TCAGCATGAT GGAAGAGTTC CAAACAAAGT ATCCATCACT TTGTACTACT 420
TCCGTCATTG GCAAGTCCGT AAAGGATCGT AAACTGATGA TTTGCAAGCT GACGTCCTCT 480
GCCAATACAG GGAAAAAGCC TCGCGTGCTC TATACTTCTA CGATGCACGG AGACGAAACG 540
ACCGGATATG TGGTACTGCT CCGACTCATA GACCATCTGC TGTCGAACTA CGAATCCGAT 600
CCGAGGATTA AGAACATTCT GGATAAAACG GAAGTATGGA TCTGCCCTTT GACCAATCCG 660
GACGGAGCAT ACAGAGCCGG AAACCACACC GTACAAGGAG CTACTCGCTA CAATGCCAAC 720
AATGTCGATT TGAACCGTAA CTTCAAGGAT GATGTAGCCG GTGATCACCC CGATGGAAAA 780
CCTTGGCAGC CGGAGGCAAC TGCATTCATG GATTTGGAAG GAAACACCTC TTTCGTGCTC 840
GGTGCCAATA TACATGGAGG AACAGAGGTG GTGAACTATC CATGGGATAA TAAAAAAGAA 900
AGACATGCAG ACGATGAGTG GTACAAACTG ATCAGTCGCA ACTACGCAGC CGCTTGTCAG 960
AGTATTTCCG CCAGCTACAT GACCTCCGAA ACCAATTCGG GAATCATCAA CGGTTCAGAC 1020
TGGTATGTAA TTCGCGGAAG TCGTCAGGAC AATGCAAATT ATTTCCATCG TCTGCGAGAA 1080
ATTACCCTTG AAATCAGCAA CACGAAGTTG GTGCCGGCCT CTCAACTTCC AAAGTATTGG 1140
AATCTGAACA AAGAATCTCT GCTTGCTCTG ATCGAAGAAT CCTTATACGG CATCCATGGT 1200
ACAGTGACTT CCGCTGCGAA CGGACAGCCT CTCAAATGCC AGATCTTGAT AGAAAACCAT 1260
GACAAGCGCA ACTCCGATGT TTACTCCGAT GCTACCACAG GCTACTACGT ACGTCCTATC 1320
AAAGCCGGCA CTTATACGGT GAAATACAAA GCCGAGGGTT ATCCTGAGGC AACTCGTACC 1380
ATTACGATCA AGGACAAAGA AACCGTCATC ATGGACATTG CATTGGGCAA CTCGGTTCCT 1440
CTGCCTGTAC CCGATTTCAC AGCTTCTCCT ATGACCATCT CAGTAGGCGA AAGCGTCCAA 1500
TTCCAAGATC AAACGACAAA TAACCCCACG AATTGGGAGT GGACGTTCGA AGGCGGACAG 1560
CCTGCCATGA GTACAGAGCA GAATCCGCTC GTATCCTATA GTCATCCCGG TCAGTACGAC 1620
GTTACGCTCA AAGTGTGGAA TGCAAGTGGT TCCAACACGA TTACGAAAGA AAAATTCATC 1680
ACTGTCAATG CCGTTATGCC TGTAGCTGAA TTCGTCGGTA CCCCGACGGA AATAGAAGAG 1740
GGCCAGACGG TATCTTTCCA AAACCAATCC ACCAATGCCA CCAACTACGT ATGGATATTC 1800
GATGGCGGCA CTCCCGCTAC CAGTGAAGAC GAAAACCCGA CTGTGCTTTA CAGCAAAGCC 1860
GGCCAATACG ATGTCACGCT CAAGGCGATC AGTGCTTCCC GTGAAACGGT GAAGACGAAA 1920

GAAAAATACA TCACTGTCAA GAAAGCTCCG GTCCCTGCTC CGGTAGCCGA CTTCGAAGGA 1980
ACACCTCGAA AAGTAAAGAA AGGCGAGACA GTTACTTTCA AAGACTTGTC TACGAACAAT 2040
CCGACTTCAT GGCTTTGGGT GTTCGAAGGC GGCTCTCCTG CCACCAGCAC GGAGCAAAAC 2100
CCGGTGGTCA CCTACAATGA AACAGGCAAG TACGATGTCC AGCTGACTGC CACCAACGAG 2160
GGCGGAAGCA ATGTGAAGAA AGCAGAAGAC TACATTGAGG TTATCCTCGA TGACAGTGTC 2220
GAGGACATAG TGGCACAGAC GGGTATCGTC ATTCGTCCGC AAAACGGAAC GAAGCAGATC 2280
CTCATAGAAG CCAACGCTGC TATCAAAGCG ATCGTTCTCT ATGACATCAA TGGACGGGTC 2340
GTACTCAAAA CTACTCCGAA TCAGCTCCGC TCGACCGTAG ATCTTTCCAT CCTGCCCGAA 2400
GGAATCTACA CCATCAATAT CAAAACGGAA AAATCCGCTC GCACGGAAAA GATCCATATC 2460
GGG 2463

(2) INFORMATION FOR SEQ ID NO:163

(i) SEQUENCE CHARACTERISTICS:
(A) LENGTH: 228 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...228

(xi) SEQUENCE DESCRIPTION: SEQ ID NO:163

ATGCGACTGA TCAAGGCTTT TCTCGTGCAA CTCTTACTGC TCCCCATTTT CTTCTACAAG 60
CGGTTTATAT CGCCGCTTAC ACCGCCTTCA TGCCGGTTTA CCCCCTCATG TTCGTCCTAT 120
GCCATCGAAG CCTTACGTAA ATATGGCCCG GGCAAAGGAC TATTGCTGAG CATCAAGCGT 180
ATTCTCCGCT GTCACCCGTG GGGTGGAAGT GGCTATGACC CCGTTCCG 228

(2) INFORMATION FOR SEQ ID NO:164

(i) SEQUENCE CHARACTERISTICS:
(A) LENGTH: 2577 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2577

(xi) SEQUENCE DESCRIPTION: SEQ ID NO:164

ATGGCATACG ACTTTACACA AACATTCCGC AACAGCCTGG AGTACAGCTA TCAGGAAGCA 60
ACCCGTCTCG GCGTCGTAGC CGTGACGCAA GATATGCTCG TACTCGGTAT CATTCGCGAC 120
GGAGACAATG GCGCGATCGA CATCATGCGG CACTATGGGA TCAACTTGTA CGAACTCAAA 180
CGGTTGATCG AGTTGGAAGC CATCGCCGAG AGTTTGCCTG CTTCGCCTGA GGGATCGCCC 240
ATCTTCACCC CTTCGGCTCG GGAGGCTATC GATGATGCCA CAGACATCTG TGCCGACATG 300
GAGGACGAGG CCGTCAGCCC GGTCCATCTG TTGCTGAGTA TCCTCAACTC GACACAGGAG 360
AGCTTAGTAC AAAAGATATT TATGAAACAA GGTATAAAAT ACGACACCAT CCTGTCGGAT 420
TACTTCGGAC AGCGCAACCC CTCCGAAGGG AAGTCTCCCT CCGAAATGGA GATCCTCGAC 480
GGGTACCAAG ACAACGACTT CGACGACGAA GAGGACGAAT CCTCTCCGCC TTCCGGGAAT 540
AGCGGGACAG GCGGAGGCTC CGGCGACGCC CCCGAACAGA ATACCGGCGG AGGCGATACT 600
ACCACCACGA CACGGAGTGG AGGCGACACG CCTGCACTGG ACACCTTCGG CACCGACATC 660

ACTGCCATGG CGGCAGCAGG CAAGCTCGAC CCGGTAGTGG GTCGGGAGCA GGAGATCGAA 720
AGGGTGATAC AGATACTCAG CCGGCGCAAA AAGAACAATC CGGTGCTCAT CGGCGAACCC 780
GGTGTAGGCA AGAGTGCCAT CGTGGAAGGA CTGGCCGAAC GCATCGTGAA CAGGAAGGTG 840
AGCCGTATTC TTTTCGACAA GCGGATCATC AGCCTCGATT TGGCTCAGAT GGTAGCCGGC 900
ACCAAATATC GCGGACAGTT CGAAGAGCGG TTGAAAGCCG TGCTCGATGA GCTGAAGAAG 960
AATCCGCAGA TCATCCTCTT CATCGACGAG ATACATACCA TCGTGGGAGC AGGCTCTGCA 1020
GCCGGATCGA TGGATACGGC CAATATGCTC AAACCCGCTC TTGCCCGTGG ACAGGTACAG 1080
TGCATCGGAG CCACTACGCT GGATGAGTAT CGTAAGAACA TAGAAAAGGA CGGAGCACTC 1140
GAACGCCGCT TCCAGAAGGT GCCGATAGCC CCCTCGACTG CAGAAGAAAC GCTGACCATC 1200
CTGCAAAACA TCAAAGAGAA ATACGAGGAC TATCACGGTG TACGCTATAC GGACGAAGCG 1260
ATCAAAGCGG CAGTGGAACT GACCGATCGC TATGTATCCG ATCGTTTCTT CCCAGATAAG 1320
GCGATAGATG CCATGGACGA GGCCGGCGCG AGCGTCCATA TCACCAATGT GGTGGCTCCG 1380
AAAGAAATCG AGATACTGGA GGCCGAATTG GCATCGGTGC GAGAGAACAA GCTCTCGGCC 1440
GTAAAGGCTC AGAACTACGA ACTGGCTGCC TCCTTCCGCG ATCAGGAGCG GCGCACTCAG 1500
CAGCAGATAG CGGAAGAGAA GAAAAAATGG GAAGAGCAGA TGTCCAAGCA CCGCGAGACG 1560
GTGGACGAGA ATGTAGTGGC GCATGTAGTG GCGTTGATGA CAGGCGTTCC GGCTGAGCGG 1620
CTGAGCACGG GCGAAGGCGA ACGTCTGCGC ACGATGGCAG ATGATCTCAA GACCAAAGTA 1680
GTAGGTCAGG ACACAGCCAT CGAAAAGATG GTGCATGCCA TCCAGCGCAA TCGTCTGGGA 1740
CTTCGCAATG AAAAGAAACC GATCGGTTCT TTCCTTTTCC TCGGCCCCAC GGGGGTAGGC 1800
AAGACCTATT TGGCCAAGAA GCTCGCCGAA TACCTGTTCG AGGATGAGAA TGCCATGATC 1860
AGGGTGGATA TGAGCGAGTA TATGGAGAAG TTCTCCGTTT CGCGTCTCGT GGGTGCCCCT 1920
CCGGGATATG TGGGCTATGA AGAAGGCGGC CAACTGACGG AGCGCGTAAG ACGCAAACCC 1980
TATTCCGTGG TTCTCTTGGA TGAGATCGAA AAGGCGCATG CCGATGTCTT CAATCTGCTC 2040
TTACAGGTGA TGGACGAAGG TCAGCTGACC GACAGTCTGG GACGGCGCGT GAATTTCAAG 2100
AACACCGTGA TCATCATCAC CTCCAACGTG GGTACACGCC AGCTCAAAGA CTTCGGGCAG 2160
GGTATCGGGT TCCGTTCGGA AAAAGACGAG GAAGCGAACA AGGAGCATAG CCGTTCCGTG 2220
ATCCAAAAAG CTCTGAACAA GACGTTCAGC CCCGAATTTC TCAACCGTTT GGACGATATC 2280
ATCCTCTTCG ACCAACTGGG CAAGACGGAG ATTCGCCGGA TGGTGGACAT AGAGCTTAAA 2340
GCCGTCTTGG CGCGCATCCA TCGTGCCGGA TACGACCTCG TCCTTACCGA TGAAGCCAAG 2400
GATGTGATAG CGACGAAGGG ATACGACCTC CaATACGGAC CACGACCGCT CAAGCGCACA 2460
CTCCAGAACG AAGTGGAGGA TCGCCTCACG GATCTTATCC TCTCCGGACA GATCGAGAAA 2520
GGGCAGACGC TTACGCTCTC TGCTCGCGAT GGCGAGATCA TCGTACAAGA ACAAGCA 2577

(2) INFORMATION FOR SEQ ID NO:165

(i) SEQUENCE CHARACTERISTICS:
(A) LENGTH: 1251 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1251

(xi) SEQUENCE DESCRIPTION: SEQ ID NO:165

ATGAATTACC TGTACATACT GATTACACTT TTACTCTCCG GCTTTTTTTC CGGTGCTGAG 60
ATTGCTTTCC TTTCTTCGGA CAAACTGCGT CTTGAGTTGG ACAGGAATAG AGGCGATCTC 120
ACAGGCAGAG CGTTAAACTT GCTGTATCGA CATCCGGACC AACTGGTGAC TACTCTCCTT 180
GTGGGTAATA ATATCGTTTT GGTAGTCTAT GGTCTGCTGA TGGCGGGATT GCTGGCCGCA 240
CCTTTGGCGC AATGGATTGA TAACGATGCT ATGATCGTCG TTCTCCAATC TGTCTTATCC 300
ACTATCATCA TACTGTTTAC CGGGGAATTT CTACCCAAAG CCATTTTCAA GACCAATGCC 360
AATATGATGA TGAGGGTATT CGCCCTCCCT ATCGTAGCGA TCTATTATCT GCTTTATCCT 420
CTGTCTAAAC TCTTCACCGG TTTATCTCGC TCTTTTATTC GTCTGGTGGA CAAGAATTAT 480
GTGCCTACAA CAGTAGGGTT GGGGCGCGTA GATCTCGATC ATTATTTGGC AGAAAATATG 540
TCCGGAGAAA ACGAACAGAA CGACTTGACT ACCGAAGTGA AAATCATCCA GAATGCGCTG 600
GATTTTTCCG GTATTCAGGT GCGAGACTGC ATGATCCCAC GCAATGAGAT GATAGCATGT 660
GAGTTGCAAA CGGATATTGA AGTACTCAAA ACGACTTTTA TCGATACCGG TTTGTCCAAG 720
ATCATTATCT ACAGACAGAA CATAGATGAC GTAGTAGGAT ATATCCATTC GAGCGAAATG 780
TTTCGTGGGC AAGACTGGCA AAAACGTATC AATACTACTG TATTCGTACC CGAAAGCATG 840
TATGCCAATA AACTGATGCG ACTACTCATG CAGCGCAAGA AAAGCATTGC GATCGTCATC 900
GATGAACTTG GAGGTACGGC CGGAATGGTC ACATTAGAGG ATTTGGTAGA AGAGATTTTC 960
GGTGACATTG AGGACGAACA CGACACTCGC AAGATCATAG CCAAACAGCT CGGCCCTCAT 1020
ACCTATCTGG TCAGTGGTCG TATGGAAATA GATGATGTGA ACGAACGTTT TGGCTTGTCC 1080

TTGCCTGAGT CTGACGACTA CCTTACCGTG GCCGGATTTA TCCTGAATAG CCATCAAAAT 1140
ATCCCACAGG CCAATGAGGT CGTGGAGATT GCTCCTTATA CTTTTACCAT TCTCAGATCT 1200
TCTTCCACCA AGATCGAACT GGTGAAAATG TCCATCGACG ACCAATCGAA C 1251

(2) INFORMATION FOR SEQ ID NO:166

(i) SEQUENCE CHARACTERISTICS:
(A) LENGTH: 879 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...879

(xi) SEQUENCE DESCRIPTION: SEQ ID NO:166

ATGAAACAGA ACTACTTCAA AAGAGTCTGC TCACTGCTTT GGCTGGTTTT ACCCATGCTT 60
ATTATGCCAT TGGAAGTAGC AGCTCAAGAG ATTATTCCGA ACGAAGAGGT GTTGGAATCA 120
TTGACTTTCG TTGCACCGGT TGAGGAGACA GACGCAATAG AGGCAGAGGT AGAAGCTCTG 180
CAGGAGATAG TCGCTACTGA GGAGATTGCG GAGCAGGCTG TTCGTTCTTA TACCTACACG 240
GTCTATCGTG ATGGCGTGAA GATTGCTTCA GGATTGACTG AGCCCACTTT TCTCGATGAA 300
GATGTTCCTG CCGGCGAACA TACCTACTGC GTAGAAGTAC AGTATCAGGG AGGCGTATCC 360
GACAAAGTAT GCGTGGACGT AGAGGTGAAG GACTTCAAAC CGGTTACCAA TCTCACCGGA 420
ACTGCTTCCA ATGACGAAGT TTCTTTGGAC TGGGACGGTG TGGAAGAGAA AGCTGAAGAG 480
CCGGCAAGTG ATAAAGCAGT CAGCTACAAC GTCTACAAGA ATGGAACCTT GATCGGTAAT 540
ACAGCTGAAA CTCATTATGT GGAGACCGGT GTAGCCAATG GTACATACAT CTACGAAGTG 600
GAAGTAAAGT ATCCTGACGG TGTATCTCCG AAGGTGGCTG TAACCGTGAC CGTGACCAAC 660
AGCTCATTGA GCAATGTAGA TGGACAGGCT CCTTACACAT TGCGAGTAGA AGGCAAGAAG 720
ATTATTGCGG AAGCCCATGG TATGATCACG CTCTACGACA TCAACGGACG TACCGTGGCC 780
GTAGCCCCGA ATCGATTGGA ATACATGGCG CAAACCGGTT TCTATGCAGT GCGCTTCGAT 840
GTGGGGAATA AACACCATGT ATCGAAAATA CAAGTAAGA 879

(2) INFORMATION FOR SEQ ID NO:167

(i) SEQUENCE CHARACTERISTICS:
(A) LENGTH: 936 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...936

(xi) SEQUENCE DESCRIPTION: SEQ ID NO:167

ATGATTCCTC TAAGCGAGAG TTTTGAATCA GGTATTCCAG CTATATGGAA GACCATTGAC 60
GCAGATGGCG ATGGCTATAA TTGGATGCAT TTGACCAATT TCACGGGACA GAGTGGTCTC 120
TGTGTCTCTT CGGCTTCATA CATAGGCGGC GTCGGAGCTT TGACTCCGGA CAATTATCTG 180
ATAACACCCG AATTAAAACT ACCCACAGAC GCGTTGGTGG AAATAATCTA TTGGGTATGT 240
ACTCAAGATC TCACTGCTCC ATCGGAGCAC TATGCCGTTT ATTCCTCTTC TACAGGCAAT 300
AATGCTGCTG ACTTTGTTAA TCTCTTATAT GAAGAGACTT TGACTGCCAA ACGGATACAA 360
TCCCCCGAGT TGATCCGCGG AAATCGGACA CAAGGTGTTT GGTATCAAAG AAAGGTGGTA 420

CTCCCTAACG ATACTAAATA TGTTGCTTTC CGCCATTTTA ATTCCACGGA TAATTTCTGG 480
CTCAATTTGG ATGAAGTATC TATCCTGTAT ACCCCTCTTC CCCGAAGAGC TCCGTGTCCG 540
CATCCGGGTG GTTACACTTA TTCTGTATTC CGTGATGGAC AAAAGATAGC GAGTGGATTG 600
TCGGCATTGG CATATATCGA TACGGATGTA CCGTATGGGA CTCAAGACTA TTGTGTCCAA 660
GTCAATTATC TGCAAGGAGA CTCGTATAAA GTCTGCAAAA ATATAGTGGT GGCAAATTCT 720
GCAAACATCT ATGGGGCGGA TAAGCCTTTT GCGTTGACCG TGGTTGGCAA GACCATTGTA 780
GCGAGTGCTT TCAAAGGAGA GATCACTCTT TATGACATTC GTGGCCGGCT GATAGCTTCC 840
GGCTGCGATA CGCTTAGGTA CAAAGCGGAA AATGGTTTTT ACCTCATTAA AATACAGGTA 900
AACGGAACTG TCTATACTGA GAAAATCCAA ATCCAA 936

(2) INFORMATION FOR SEQ ID NO:168

(i) SEQUENCE CHARACTERISTICS:
(A) LENGTH: 2529 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2529

(xi) SEQUENCE DESCRIPTION: SEQ ID NO:168

ATGAAAAAAA GTTTTCTTTT AGCCATAGTA ATGCTCTTTG GCATTGCCAT GCAGGGACAT 60
TCTGCTCCGG TTACGAAAGA GCGAGCTTTG AGTCTGGCTC GGCTGGCTTT GCGACAGGTA 120
TCCTTGCGAA TGGGACAAAC AGCAGTATCT GACAAGATTT CCATCGATTA CGTTTATCGG 180
CAAGGAGATG CTGAGAGGGG TATCACATCA CAAGAGGAAG GCTCTCCTGC ATATTTTTAT 240
GTAGCTAATC GTGGAAATAA TGAGGGCTAT GCTCTTGTAG CAGCAGATGA CAGAATACCG 300
ACAATTTTAG CCTATTCACC CATTGGCCGT TTCGACATGG ACAGTATGCC GGACAATCTT 360
CGCATGTGGC TACAAATTTA CGATCAGGAA ATAGGCCTGA TACTTTCCGG AAAAGCTCAG 420
CTCAATGAAG AGATATTACG TACCGAGGGC GTACCGGCTG AAGTACATGC TCTGATGGAT 480
AACGGTCATT TTGCCAACGA TCCCATGCGA TGGAATCAAG GTTACCCATG GAACAATAAG 540
GAACCACTGC TTCCTAATGG CAATCATGCC TATACCGGCT GTGTTGCTAC TGCTGCAGCA 600
CAAATCATGC GCTACCATAG CTGGCCGCTT CAAGGTGAAG GCTCTTTCGA TTATCATGCA 660
GGTTCATTAG TTGGCAACTG GTCCGGCACA TTTGGTGAAA TGTACGACTG GATCAATATG 720
CCCGGAAATC CCGACCTTGA TAATCTGACT CAATCTCAAG TGGATGCCTA CGCCACACTG 780
ATGCGTGATG TGAGTGCATC TGTTTCGATG AGTTTTTATG AAAATGGAAG TGGTACGTAC 840
AGCGTTTATG TAGTAGGAGC CTTGCGAAAC AACTTTCGCT ACAAGCGTTC ACTGCAGCTA 900
CATGTACGCG CCTTATATAC CTCACAGGAG TGGCACGATA TGATCCGCGG GGAACTTGCC 960
TCCGGAAGCC CGGTCTATTA TGCAGGGAAT AACCAGAGCA TAGGACATGC TTTCGTTTGC 1020
GATGGTTATG CTTCGGATGG TACTTTCCAT TTCAACTGGG GTTGGGGAGG TGTTTCCAAC 1080
GGCTTCTACA AACTAACACT CCTCTCGCCG ACTTCGTTGG GTATCGGAGG TGAGGGAATA 1140
GGTTTTACCA TTTATCAAGA GATCATCACC GGTATCGAAC CGGCTAAGAC TCCCGCTGAA 1200
GCCGGTACAG ATGCCTTGCC GATCTTGGCA CTGAAAGACA TAGAAGCCGA GTATAAAAGT 1260
GAATCCGGAT TGAACGTAGG GTATTCGATA TATAATACAG GTGAAGAGCA ATCAAATCTT 1320
GACCTCGGAT ACAGATTGAA CAAGGCTGAC GGAGAAGTCA TAGAGGTGAA AACTTCATCT 1380
ATCAATATCT CTTGGTACGG ATACGGAGAG CATCCCGAGA GTTTCTCATT GGCACCTAAT 1440
CAGTTGTCAC AAGGAATCAA CACCATCACC CTACTTTATC GTCGCACAGG CACCGAACAG 1500
TGGGAGCCGG TACGGCATGC ACAGGGAGGA TATGTCAATA GCATTAAAGT AAATACGACA 1560
GACCCGAACA ATGTCGTAGT CACGGTAGAT AATAACGAAG GCAAGCTCAG TATCGTCCCC 1620
AACAGCTTTG TCGCAGATCT GAATTCTTAT GAACATAGTA CGATTACAGT ACAGTTCAAT 1680
AGCGACAGCC CTGATGAGAT CCGTACACCC GTAGCCTTTG CTCTATCTAC AGCAGCTACT 1740
GCGGACGATG TAATATCTTT GGGCTGGGTA ATGGCTGAAG TTCCGGGCGG TAGCAGCAAC 1800
TATCCGGTGG TTTGGTCTAA AGACGTTCTC ACTCTCTCGG AAGGCGACTA TACATTGTGG 1860
TATAGATTTT CCATCAACAA CCAAAAGGAT GAATGGAAAA AGATCGGAAG CGTGTCAGTA 1920
AAAACACCGA CAGAGTATAC GCACCCCTTA TTCGAAGTGG GCCATAATCA AACTTCTACC 1980
TATACGCTGG ATATGGCACA CAACAGAGTA TTGCCCGACT TTACACTCAA AAATCTCGGA 2040
TTGCCTTTCA ATGGTGAGTT GGTTGTTGTT TTCCGCCAAA CACAATCCTC ATCGGGGTCT 2100
TTATGGGCAG CTCAAGAAAC AGTACATATC AAGCAAGGAG AAACTTTCGT ATATAAACCT 2160
GTTGTCGAAG GCCCTATACC TGATGGATCC TATCGTGCGA CCCTCCATGC ATTCGTAAAC 2220
GGACAACAAC AGTTGTACCT CAAGGGGAAA AGGAACTACA CGGTGAAGAT CGTCAATGGT 2280
ACAGCGGTAG AAGCAATAGA ATCGTCAGAA GAGATCAGAG TATTCCCTAA TCCGGCACGC 2340
GATTATGTGG AAATATCGGC ACCTTGCATT CCCCAAGAAA CATCTATCAT TCTTTTCGAT 2400
CTGTCAGGCA AGATTGTCAT GAAGAATAGT TTATCAGCGG GGCATGGCAG AATGGATGTC 2460

AGCCGACTTC CTAATGGGGC CTACATCCTT AAGGTGGATG GATATACGAC GAAAATAAAT 2520
ATAGTGCAC 2529

(2) INFORMATION FOR SEQ ID NO:169

(i) SEQUENCE CHARACTERISTICS:
(A) LENGTH: 870 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...870

(xi) SEQUENCE DESCRIPTION: SEQ ID NO:169

ATGAAAAAGC TATTTCTCTC GCTCACGAGT CTTGTAATGG TCTTCGCTGT TGCAAGTTGC 60
GATATAATCG ACAAGGATCA AACCCTCTTG CCGGCTCCGA CCAATGTGAC ACCCGATAAT 120
CCGGATGACA ATCCTTCGGA GATCGACATT ACGCAGACGC ACACAGAAAA ATATGTTTTG 180
GCTGAAGAAT TTACCGGCCA AAAATGTCTC AACTGTCCGA AAGGTCATCG CAAACTGGCG 240
GCTCTCAAGG AGCAATACGG TAAGAGATTG ACTGTTGTCG GTATACATGC CGGCCCTGGA 300
TCTCTCGTGC CACCTCTTTT CCGTACAGAA GCCGGAGACG CATATTATAG CAAGTTCGCC 360
AATAATACCC CTCTCCCTGC GCTGATGGTT TCGCGCAAAA AGTTCGGCTC TTCCTACGTT 420
TATGATAAGA GCTACAAAAC GTGGGACGTG CCTATTGCCG AGCAGATGGA GCAAAAGGCG 480
AAGATCAATA TCTTTGCCGT GGCCGAATAC ACCGATACCC AAAAGATCAA GGTGACTGTA 540
AAGGGTAAAA TACTGGAGGG GAATACACTC CCGAAGTCCA TGGTTCAGGT GTATCTGTTG 600
GAGGATAAGC TGATCGCTCC GCAGGTGGAT GGCAATACGA CAGTCGAGAA TTACGAGCAC 660
AATCACGTGT TGCGTGGAGC CGTTAATGGT ATTTGGGGCG AAGAATTTGT GAATCTCAAA 720
GATTATTTGT ATACTTACGC CGTTGAACCG CTCTCGGGTA TGTCCTTCGT AGCCGAGAAT 780
TATTCGATTG TGGCTTTTGT ATACGATGTG CAGACGTTCG AAGTGTATGA CGTTGTGCAT 840
GTAAAGATCA ATCCGCAATC CGATGGCAAA 870

(2) INFORMATION FOR SEQ ID NO:170

(i) SEQUENCE CHARACTERISTICS:
(A) LENGTH: 669 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...669

(xi) SEQUENCE DESCRIPTION: SEQ ID NO:170

ATGAAGAAAT CAAGTGTAGT AGCCTCAGTT TTGGCCGTGG CTCTCGTGTT CGCCGGTTGC 60
GCACTGAACA ATATGGCAAA AGGCGGCCTT ATCGGCGCCG GAGTAGGAGG TGCCATTGGT 120
GCCGGACTAG GTAACGTAGC CGGAAATACG GCTGTCGGTG CCATCGTCGG TACTGCAGTC 180
GGTGGAGCAG CCGGTGCTCT CATCGGAAAG AAGATGGACA AGCAGAAAAA AGAACTGGAG 240
GCCGCAGTAC CCGATGCTAC GATTCAGACA GTAAATGACG GAGAGGCTAT TCTGGTTACT 300
TTCGATAGCG GTATCCTCTT TGCGACGAAC TCCAGCACTC TGAGTCCCAA CTCACGCACT 360
GCGCTGACGA AGTTTGCTGC AAACATGAAC AAAAACCCCG ACACGGATAT TCGTATCGTA 420
GGCCATACGG ACAATACCGG CTCCGACAAG ATCAACGATC CTCTGTCTGA GAGACGTGCA 480

GCCAGCGTAT ATTCTTTCCT GAATTCTCAG GGTGTGAGTA TGTCGCGCAT GGCAGCCGAA 540
GGGCGTGGGA GCCATGAACC GGTTGCAGAC AATAGCACAG TTGCCGGACG TTCGGCCAAC 600
CGCCGTGTGG AGGTTTATAT CTTGCCGAAT GCCAAGATGA TCGAACAAGC ACAGCAAGGT 660
ACGCTGAAG 669

(2) INFORMATION FOR SEQ ID NO:171

(i) SEQUENCE CHARACTERISTICS:
(A) LENGTH: 1011 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1011

(xi) SEQUENCE DESCRIPTION: SEQ ID NO:171

ATGTCGAAAA AATCGATCCT TCTGCTTTGC TGTTCGCTGT GCTTCATTTC TGCTACGAAG 60
GCTGTGACCC CCGTCAGAAA TGTGCGCAAT AGCCAAGTGA ACAGCAAAGC AAAGACCGAA 120
CGTACAAAGC CCTCGGACTC TGTACGGTAC ATTAGCAACA TGATTGCAGA TCGGCTGGAG 180
TTCCGCAACA AGATTTCTTC CGAAAAAGAG GTAAGAAAAG CCGAATATGA AAATCGGCTG 240
GCGATGGAAG CACTCAATTA CCCTGCCATA GATTTATATG GTGAAGATTC TTGGAGCGAG 300
TATGTAAACC CTTTCGTGGG TGCAGGAACC GATGTCGAAA TTCCGAACTC CTATGACATT 360
GATTGCTCTT CGTTCGTGAT GCCCGTCGAA GATAAGCAGG TCACCTCTCA ATTTGGCTAC 420
CGTCGGCGTT TCGGACGGAT GCACTATGGT ATTGATCTTT CAGTGAATCG TGGCGATACG 480
ATACGAGCAG CCTTTGACGG GAAAGTTCGT GTACGCAGCT ATGAAGCGCG TGGCTATGGC 540
TACTACATAG TCTTGCGCCA TCCGAACGGA CTGGAGACTG TGTACGGACA CATGAGTCGC 600
CAATTGGTAG ACGAGAATCA GATCGTTCGA GCAGGACAAC CGATCGGATT AGGAGGCAGC 660
ACGGGTCGAA GCACCGGTCC TCATCTTCAC TTCGAGACCC GCTTCATGGG TATTCCCATC 720
AATCCGAGTA CCATTATAGA CTTCGATAAC GGAGTGCCGC TCCGAGACAT TTACACATTC 780
AAACGAGGGA GCAATTCTCG CTATGCAAAA GCCTCTAAGA CTTCTTCTCG CTATGCAAAA 840
AAAGGGAAGA AAGGCAGACA AGCTTCTTCT CCTATGACCT ATAGAATCAA AAAAGGCGAT 900
ACTTTGGAAA CAATAGCCAA AAGGCACGGC ACTTCTGTTC AGAAACTCTG TGCTACCAAT 960
GGCATTGGCA AGAGTAAAAT TTTGACTCCG GGCAAAGCCT TGAGGATCAA A 1011

(2) INFORMATION FOR SEQ ID NO:172

(i) SEQUENCE CHARACTERISTICS:
(A) LENGTH: 453 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...453

(xi) SEQUENCE DESCRIPTION: SEQ ID NO:172

ATGGCAAAAA TCAATTTCTA TGCTGAAGGC GTCAGCCTTC CTCGGATCAG AAGACGGATC 60
GTCGGTAAGT GGATAGCCGA AGTATGCAGC CGATATGGGA AAGCGGTGGG AGAAATCTCC 120
TATCTTTTCT GTGATGACGA ATATATCCTG AAAGCCAATC AGGAATTTCT CGATCATGAC 180
TACTACACCG ACATCATCAC CTTCGATTCC TGCGAAGCGG ATACGGTGAA TGGCGACCTG 240

CTTATCAGTC TCGATACCGT ACGCTCGAAT GCCCGTGCTC TTGATCTTCG ATACGAAGAC 300
GAACTGCATC GTGTCATTAT CCACGGCATA CTGCATCTTT GCGGATTGAA AGACAAGAGC 360
AAAAAGGATG AAGCCCAAAT GCGTGCAGCC GAAGAGAAAG CCCTTGTCAT GCTGCGAGAA 420
ACCATCGGAT CGGAGCTTTC CCTATTGCAT ACA 453

(2) INFORMATION FOR SEQ ID NO:173

(i) SEQUENCE CHARACTERISTICS:
(A) LENGTH: 1173 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1173

(xi) SEQUENCE DESCRIPTION: SEQ ID NO:173

ATGAAGGTAA AGTACTTAAT GCTCACATTG GTTGGAGCAA TTGCACTGAA CGCAAGTGCA 60
CAGGAGAATA CTGTACCGGC AACGGGTCAG TTACCCGCTA AGAATGTTGC TTTTGCTCGC 120
AATAAAGCAG GCAGCAATTG GTTTGTAACA CTGCAAGGCG GTGTTGCAGC GCAGTTCCTC 180
AATGACAACA ACAACAAAGA CCTCATGGAC CGCTTAGGAG CCATAGGTTC TCTTTCTGTC 240
GGAAAGTATC ACAGCCCTTT CTTTGCAACT CGTTTGCAAA TTAACGGAGG TCAAGCCCAC 300
ACTTTCCTCG GAAAAAATGG CGAACAAGAA ATCAACACCA ATTTTGGTGC AGCTCACTTC 360
GACTTTATGT TTGATGTGGT TAACTACTTT GCACCATATC GCGAAAATCG TTTCTTCCAT 420
TTAATTCCAT GGGTAGGTGT TGGCTACCAA CACAAATTCA TCGGTAGCGA ATGGAGCAAA 480
GACAATGTGG AATCACTGAC GGCGAATGTA GGAGTTATGA TGGCTTTCAG ATTAGGAAAG 540
CGAGTAGACT TTGTGATCGA AGCACAAGCA GCTCACTCCA ATCTCAATCT AAGTCGCGCA 600
TACAATGCCA AGAAAACTCC CGTATTCGAA GATCCCGCAG GACGTTATTA CAATGGATTC 660
CAGGGGATGG CTACAGCAGG TCTTAATTTC CGCCTGGGAG CCGTAGGCTT CAATGCCATT 720
GATCCAATGG ACTACGCACT TATCAATGAT CTGAATGGTC AGATTAACCG TTTGCGCAGC 780
GAGGTCGAAG AACTCTCAAA ACGTCCTGTA TCATGCCCCG AATGTCCTGA AGTAACTCCT 840
GTTACTAAGA CAGAAAATAT ACTGACGGAA AAAGCTGTAC TGTTCCGTTT CGACAGCCAC 900
GTTGTGGACA AAGATCAATT GATCAACCTG TATGACGTAG CTCAGTTTGT AAAAGAAACT 960
AACGAGCCGA TTACCGTTGT TGGTTATGCT GATCCTACGG GTAATACTCA ATACAACGAG 1020
AAATTGTCTG AGCGTCGGGC TAAAGCCGTT GTTGATGTTC TGACAGGTAA ATATGGTGTG 1080
CCTTCCGAAT TAATCTCTGT AGAATGGAAG GGCGACTCTA CGCAACCGTT CAGCAAGAAA 1140
GCTTGGAATC GTGTTGTAAT CGTTCGCTCC AAG 1173

(2) INFORMATION FOR SEQ ID NO:174

(i) SEQUENCE CHARACTERISTICS:
(A) LENGTH: 1155 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1155

(xi) SEQUENCE DESCRIPTION: SEQ ID NO:174

ATGACATACA GAATTATGAA AGCTAAATCT TTATTATTAG CACTTGCGGG TCTCGCATGC 60

ACATTCAGTG CAACAGCCCA AGAAGCTACT ACACAGAACA AAGCAGGGAT GCACACCGCA 120
TTCCAACGTG ATAAGGCCTC CGATCATTGG TTCATTGACA TTGCAGGTGG AGCAGGTATG 180
GCTCTCTCGG GATGGAATAA TGATGTAGAC TTTGTAGATC GTCTAAGTAT CGTTCCTACT 240
TTCGGTATCG GTAAATGGCA TGAGCCTTAT TTCGGTACTC GTCTCCAATT CACAGGATTC 300
GACATCTATG GATTCCCGCA AGGGAGCAAG GAGCGTAACC ACAATTACTT TGGAAACGCC 360
CACCTTGACT TCATGTTCGA TCTGACGAAC TATTTCGGTG TATACCGTCC CAATCGTGTC 420
TTCCATATCA TCCCATGGGC AGGTATAGGA TTTGGTTATA AATTCCATAG CGAAAACGCC 480
AATGGTGAAA AAGTAGGAAG TAAAGATGAT ATGACCGGAA CAGTTAATGT CGGTTTGATG 540
CTGAAATTCC GCCTATCAAG AGTCGTAGAC TTCAATATTG AAGGACAAGC TTTTGCCGGA 600
AAGATGAACT TTATCGGGAC AAAGAGAGGA AAAGCAGACT TCCCTGTAAT GGCTACAGCA 660
GGTCTAACGT TCAACCTTGG CAAGACAGAG TGGACAGAAA TTGTTCCTAT GGACTATGCT 720
TTGGTCAATG ACCTGAACAA CCAAATCAAC TCACTTCGCG GTCAAGTGGA AGAGTTGAGC 780
CGTCGTCCTG TTTCATGCCC TGAATGCCCT GAGCCTACAC AGCCTACAGT TACTCGTGTA 840
GTCGTTGACA ATGTGGTTTA CTTCCGTATC AATAGTGCAA AGATTGATCG TAATCAAGAA 900
ATCAATGTTT ACAATACAGC TGAATATGCG AAGACCAACA ACGCACCGAT CAAGGTAGTA 960
GGTTACGCTG ACGAAAAAAC CGGTACTGCG GCCTATAACA TGAAGCTTTC AGAGCGTCGT 1020
GCAAAAGCGG TAGCCAAGAT GCTTGAAAAG TATGGTGTTT CTGCGGATCG CATTACAATT 1080
GAATGGAAGG GCTCATCAGA GCAAATCTAT GAAGAGAACG CTTGGAATCG TATTGTAGTA 1140
ATGACTGCAG CGGAA 1155

(2) INFORMATION FOR SEQ ID NO:175

(i) SEQUENCE CHARACTERISTICS:
(A) LENGTH: 570 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...570

(xi) SEQUENCE DESCRIPTION: SEQ ID NO:175

ATGGAATTTT TCATGTTATT CATAGCGGCG GTTTTCGTTA ATAACGTCGT GCTGTCGCAG 60
TTCCTCGGTA TATGCCCATT CTTAGGCGTA TCGAAGAAGG TAGACACCTC AATCGGTATG 120
GGTGCAGCCG TGACATTCGT ATTGGCACTG GCTACCTTGG TTACCTTCCT GATTCAGAAG 180
TTCGTTTTGG ATCGTTTCGG ATTGGGCTTT ATGCAGACCA TTGCATTTAT TTTGGTCATT 240
GCCGCCTTGG TGCAGATGGT GGAGATCATA CTCAAGAAAG TATCTCCTCC CCTCTATCAG 300
GCACTGGGTG TATTCTTGCC CTTGATTACG ACGAACTGCT GTGTGCTCGG TGTGGCTATT 360
TTGGTTATCC AGAAGGATTA TACCCTGCTC CAGAGCTTCG TCTATGCAAT ATCCACGGCT 420
ATCGGTTTCA CCTTGGCAAT GGTTACTTTC GCAGGTATTC GAGAGCAACT CGATATGACC 480
AATCTCCCCA AAGCTATGAA GGGAATACCT TCGGCACTCT TGGCTGCCGG TATATTGGCT 540
ATGGCTTTCA TGGGCTTCAG CGGTATCGCC 570

(2) INFORMATION FOR SEQ ID NO:176

(i) SEQUENCE CHARACTERISTICS:
(A) LENGTH: 558 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature

(B) LOCATION 1...558

(xi) SEQUENCE DESCRIPTION: SEQ ID NO:176

ATGTTATTCA TAGCGGCGGT TTTCGTTAAT AACGTCGTGC TGTCGCAGTT CCTCGGTATA 60
TGCCCATTCT TAGGCGTATC GAAGAAGGTA GACACCTCAA TCGGTATGGG TGCAGCCGTG 120
ACATTCGTAT TGGCACTGGC TACCTTGGTT ACCTTCCTGA TTCAGAAGTT CGTTTTGGAT 180
CGTTTCGGAT TGGGCTTTAT GCAGACCATT GCATTTATTT TGGTCATTGC CGCCTTGGTG 240
CAGATGGTGG AGATCATACT CAAGAAAGTA TCTCCTCCCC TCTATCAGGC ACTGGGTGTA 300
TTCTTGCCCT TGATTACGAC GAACTGCTGT GTGCTCGGTG TGGCTATTTT GGTTATCCAG 360
AAGGATTATA CCCTGCTCCA GAGCTTCGTC TATGCAATAT CCACGGCTAT CGGTTTCACC 420
TTGGCAATGG TTACTTTCGC AGGTATTCGA GAGCAACTCG ATATGACCAA TCTCCCCAAA 480
GCTATGAAGG GAATACCTTC GGCACTCTTG GCTGCCGGTA TATTGGCTAT GGCTTTCATG 540
GGCTTCAGCG GTATCGCC 558

(2) INFORMATION FOR SEQ ID NO:177

(i) SEQUENCE CHARACTERISTICS:
(A) LENGTH: 2499 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2499

(xi) SEQUENCE DESCRIPTION: SEQ ID NO:177
   ATGAAACAAC TAAACATTAT CAGCTTCATC ATTGCTTTCC TATTCTTAGG AACGAGCGCA 60
   TCGGCTCAGC AATCGGGCGG ATCCGTTACA GGTACCGTAG TGGACAAAAG CTCAAAAGAA 120
   CCTATCGCAT ACGTACAAGT ATTCGTCAAA GGAACCACTC TCGGAACTTC CACGGATGCA 180
   AACGGAAACT ACTCGATCAA GGGAATCCCT TCGGGTAATC AAACTATCGT AGCCCGACTC 240
   ATGGGTTACT CCACTTGCGA AGAAAAAGTA CATATAGAAA AGGGTGGTTC CCGCCACGTA 300
   GACCTCTATC TGACCGAAGA GATTCTCTCT CTCGATGGGG TAGTGGTATC TGCCAATAGA 360
   AACGAGACTT TCCGCCGTCA AGCACCCTCG TTGGTAACGG TACTGTCGCC GGAACTTTTC 420
   CTCAAAACCA ACTCTACCAA CCTGAGTCAG GGACTTAAGT TCCAGCCCGG TCTGCGCGTG 480
   GAGGACAACT GTCAGAACTG CGGTTTCAAC CAAGTTCGTA TCAATGGACT CGAAGGAGCC 540
   TATTCGCAAA TTCTTATCGA CAGCCATCCC ATCTTCAGTT CGCTTGCCGG TGTCTATGGC 600
   TTGGAGCAGA TGCCTGCCAA TATGATCGAA CGTGTAGAAG TAATTCGCGG TGGAGGTTCG 660
   GCTCTGTTCG GCTCTAATGC TGTGGGAGGC GTTATCAACG TAATTACGAA AGAACCGCTT 720
   CGCAATTCGG CCGAGATCAG CCATTCTACG ATGACCTTCG ACCACGCGAA AGGGTGGGGG 780
   AGCTTCCAAA ATACGACCCA GTTCAACGGT TCTATGCTGA CGGAAGACCG CAAAGCCGGT 840
   GTCATGGTAT TCGGCCAACA CAACTACCGT CCCGGACAGG ATATAGACGG CGACAACTTT 900
   ACCGAACTAC CCAATCTGCG CAACCGCTCG CTCGGTTTCC GCTCATACTA TAAGACCGGT 960
   CTCTACAGCA AAGCAACCCT CGAATATCAC AGCATGCAGG AGTACCGTCG TGGTGGCGAC 1020
   AGACTGGACA ATCCTCCTTT CGAAGCCCAG ATAGCGGAAT ATCTCCAGCA CTATATCAAT 1080
   GGCGGAAGTT TCAAATTCGA TCAGGGCTTC AGCGGTGGCA AGGATTTCTT CAGTCTGTAT 1140
   GCTTCAGCAC AAGACGTTCA GCGTCGTAGC TACTACGGGG GTGGCGACTA TACCGAAAAT 1200
   CTGCTGAACG GAGCAGTTCA GAGTGGAAGC ACCGAATCGG ACGAATACAA CGATGCTTTC 1260
   ACGGCTCTTA CTTCCTACGG GACTACCAAG GGATTCGATT TGCAAGGAGG AGGTATGTAC 1320
   CGTCATACCT TCGGAGAAAA CTGGGACTTT ACCGGCGGAC TCGAATATAT CTACGGCCAA 1380
   CTCGATGACA GAAGCGGCTA CAGACCGAGC AAAATAGATC AGAATACCTC TACTTTTAGT 1440
   CAGTACGACC AGCTCGAATA TAAGACGGAG AAGTTAAGTG CCCTTATCGG AGCACGTATC 1500
   GACTATGTTC TCCTCAATCA GGATGGCAAA CGCTATATCG ATCCGCTCTT CATTTTCAGT 1560
   CCTAGAGCCA ACGTACGATA CAATCCCAAT AAGAATCTCA GCTTCCGACT CTCATACAGC 1620
   GAAGGATTCC GCGCTCCTCA GTATTTCGAT GAAGATCTGC ACGTAGAGTT GGCCGGTGGT 1680
   ACTCCTATCA GCCGTGTCCT TTCCCCCAAT CTGAAAGAAG AACGTTCACG AAGCATCAGT 1740
   GCTTCTTTCG ATTATTACCA CAGAGCCGAC GAATGGCAAT TCAATATCAT GGGAGAAGCC 1800
   TTCTCCACCT TTATCAGCAA TCAGTTCAAA CCATCCGATA AGGTCGAAAC CACGAGCGAT 1860
   GGCAAAGAAT GGATCATTCG TACCATCTAC AACGACAAGG ATGGAGTATC GAAGGTATAT 1920
   GGTGTGAATC TGGAGGGAAG AATCGCCTAC AACAAATCGT TCGACCTCCA GCTCGGCGGT 1980
   ACATGGCAGA GAAGCCGCTA CGGAAGCATC TATACCGCTG TGGAAGCGGA CAAAACAACG 2040
   GGACAAGCCG AGATCTCTGT GAAAGACTAT GTACGCACTC CGAATCTGTA CGGCTATTTC 2100
   GTTGCTACGG TACGTCCTAC CGAGCACTTC GCCATCAATC TCTCCGGTAC ATTCACGGGC 2160
   AAAATGGATG TAGTACACGA AGCCTATGAA GGCGATATTC CCGCAGAACA CATAGCTCCG 2220
   GACGGATCGT TCGACTTTGA AATGAATGGT CAGCAATTCA AAGGTTTGGC CGAAGGTCAT 2280
   GCCAAGCTCG TCAAGACTCC GGCCTTCGCC GATATAGACC TCAAGCTGAG CCACGACTTC 2340
   CACCTTGCTT CCACTATGAC CTTGGAATTG AATGCCGGAA TACAGAACAT ATTCAACAGC 2400
   TATCAGAAAG ACACGGACAA GGGACCGGGT AGAGCTTCTA CTTACGTATA CGGTCCTATG 2460
   CAGCCCAGAA GCATTTTCGT CGGTACAAAG ATCAATTTC 2499

(2) INFORMATION FOR SEQ ID NO:178

(i) SEQUENCE CHARACTERISTICS:
(A) LENGTH: 2673 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2673

(xi) SEQUENCE DESCRIPTION: SEQ ID NO:178

ATGTACAAAA AGATTATTGC CGTAGCAGCT CTCTTCTGCG CCAGCATAGG GATCCTGAAA 60
GGACAGTCCT CGGATCTGAC CCCTCAGGAT ACTATATATA GCCCTGAAAT ATCCTATGCC 120
AAGCCTATTC ATAAGACCAT AGCATCTATT GAGATCGAGG GAATGAGGTC TTTCGATGAC 180
TTTGTCTTGC GCAATCTTTC AGGCTTGGCT GTAGGTGATG AAGTCCTGAT TCCTGGAGAT 240
GCCATGTCTG CTGCCGTGAA TAGAATTATG CGTCAGGGCT ACTTCTCAAA TGTGCGAATC 300
ATCGCGGATA AATATGTCGG CAATAAAGTC TATCTGAAAA TCATTGTCAC TGAACGTCCT 360
CGCATCAGTA AGGTTACTTT TAGCGGGGTA AAGAAGTCTG AGAGAGAAGA TCTTGAAATG 420
AAAATCGGTC TTCGCGAGGG GATTCAGATG ACCAGAAATA ATGAAGACAA GGTCAGGCAA 480
ATCGTACAGA AGTATTTTAG TGAGAAAGGT TATCGCGATG CCAGCATACG GATAACGCAG 540
GAACCGGATC TTTCCAAAGA TGGCTTTGTC AATGTGCTTA TCTCGATTGA GAAGAAAAGC 600
AAAACCAAGG TGAATGAAAT TTATTTTTCC GGCAACAAGG CCCTTAGCAA TCATAAGCTA 660
AGAATGGCGA TGAAGAACAC CAATGCCAAA TTCAGTCTTA GAAAGCATAT TCGCTCATCT 720
TTCTTGAAAC TTTTTAGTAC TCATAAGTTT GTGGAAGAGA GCTACCGTGA AGATTTGGTC 780
CGATTGATAG AGAAGTATCA GGAATATGGA TATCGTGATG CTGAAATACT GACCGACAGT 840
GTCGTGAAGG CTCCTGACGG CAAAAGAGTG GATATTTATC TCAACATCGA AGAGGGGCAG 900
AAGTATTATA TTAAGGATGT CAACTTTGTG GGCAATTCAC AATATCCATC GGAGTATTTG 960
GAACGAGTGC TCGGAATAAA ATCCGGAGAT GTGTACAATC AGAGACGATT GGCTAAGCGT 1020
CTCAATGAAG ATGAAGATGC TGTGGGGAAC CTGTACTATA ACAATGGCTA TATTTTTGCG 1080
TGGGTCGATC CCGTGGAAAC AAATGTAGTG GGGGATTCTG TTTCGCTTGA TATTCGTATA 1140
GCGGAGGGGA AGCAGGCCAA TATCAATAAG GTGATCATCA AAGGAAATAC TGTCGTGTAC 1200
GAAGACGTAG TACGCCGAGA GCTTTACACA AAGCCCGGCC AGCTCTTTAG TCGCGAGGAT 1260
ATCATTAACT CTATTCGTCT CATCAATCAG CTTGGGCATT TCGATGCCGA AAAATCTATT 1320
CCCCGTCCGA TTCCCAATCC CGAAACAGGA ACAGTGGATA TAGAGTATGA TTTGGTGCCG 1380
CGTAGCAGTG ACCAATTGGA GCTTTCTGTC GGTTGGAGTC AGTCCGGACT TCTGTTCCGA 1440
GGAGCCATTA AGTTCACGAA CTTCTCTGTC GGCAACTTGC TCCATCCCTC GATGTATAAG 1500
AAAGGGATCA TTCCGCAAGG GGATGGGCAA ACACTATCAC TGAGTGCTCA GACCAATGGA 1560
AAGTACTATC AGCAGTATAG TGTCACATTT ATGGATCCAT GGTTTGGGGG CAAGCGGCCG 1620
GATATGTTCA GCTTCAGTGC ATTCTATTCC AAGACTACGG CGATTGACTC CAAGTTCTAC 1680
AATAGCAATG CCGGCAACTA CTATAATGCC TACTATAATA GCTACTACAA CAACTATAAT 1740
AGTTATTACA ACGGTATGTC GAACTATACC GGCGACCTCT ATACTCAGGC CAGCGATCCG 1800
GATCGTTCGC TTCAGATGTT AGGTACTTCG ATCGGTTACG GTAAGCGTTT GACTTGGCCG 1860
GACAATTGGT TCCAGATTTA TACTTCTCTG AACTACACCT ACTATAGACT GCGAAATTGG 1920
AGCTACAATA CCTTCCAAAA TTTCCATCAT GGCTCGGCTA ATGATCTCAA CTTGGAGCTG 1980
CGTCTCTCTC GTACTTCCAT CGATAATCCT ATTTATACCA GAAGCGGATC GGATTTCATG 2040
GTTTCTGTTG CTGCTACTCT TCCTTATTCT TTGTGGGACA ATCATGACTA TGCCAGCCAG 2100
AACCTCAGCG TAAGCGATCG TTACAGATTT ATCGAGTATC ACAAGTGGAA GTTTAGAGGA 2160
CGAGTTTTTA CTCCATTGCT CAATCCTGCT ACGCATAAAT ATACACCGGT GCTCATGAGT 2220
CGAGTGGAAG GAGCAGTTCT TGGTTCGTAT AATTCCAATA AGAAATCTCC TTTCGGTACT 2280
TTCTATATGG GAGGTGATGC TATGTCCAGC TATTATGGTG GCTACATGAA TGAGACTATA 2340
GGTTTGCGTG GTTATAAGAA CGGATCTATT GCCGGTAATA ACTACGACTA TGCATATGCT 2400
TATATGCGGC TTACGATGGA ACTACGTTTC CCGATTCTGT TTGAAAACTC ATTCAATGCG 2460
TGGCTCTTAG CTTTTGCCGA AGCAGGCAAT GCGTGGCGCA GTATCGACAA TTATAATCCC 2520
TTTAACCTGA AGCGATCGGC CGGTGTAGGA TTGCGTGTAA CGTTACCGAT GGTCGGAATG 2580
CTCGGTATCG ATTGGGGATA TGGCTTTGAC CGTCCGGACA ATTCTCTACA GCGAGGAGGA 2640

AGCAATGTCC ACTTTGTGCT CGGACAGGAG TTC 2673

(2) INFORMATION FOR SEQ ID NO:179

(i) SEQUENCE CHARACTERISTICS:
(A) LENGTH: 522 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...522

(xi) SEQUENCE DESCRIPTION: SEQ ID NO:179

ATGAATGGCG ATATGAAACG GTTTTTGATT TTGATCGGCT TTGCACTGGC GGTAGCTTTC 60
TCCGGTTTTT CCCAAAAGTT CGCTTTGGTA GATATGGAAT ATATCCTCAG GAATATTCCT 120
GACTATGAGA TGATGAACGA ACAGCTGGAA CAGGTGTCCA AGAAATGGCA AAATGAAATC 180
GAAGCTCTCG AAAATGAAGC CCAATCTATG TATAAGAAGT ATCAGAGCGA TCTCGTATTC 240
TTGTCTGCTG CACAGAAGAA AACCCAAGAA GAGGCTATCG TAAAGAAAGA GCAGCAAGCA 300
TCCGAGCTCA AGCGGAAGTA TTTCGGCCCG GAGGGGGAGC TGTATAAGAA ACGCTCCGAT 360
CTGATGAAGC CTATTCAGGA TGAGATTTGG AATGCTATCA AAGAGATTGC CAAGCGTAAC 420
AACTATCAGA TGGTGCTTGA TAGAGGTACG TCCGGAATTA TCTTTGCCAG TCCGTCTATT 480
GACATTAGCG ACCTTGTACT GAGCAAGATG GGCTTTAGCA AG 522

(2) INFORMATION FOR SEQ ID NO:180

(i) SEQUENCE CHARACTERISTICS:
(A) LENGTH: 510 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...510

(xi) SEQUENCE DESCRIPTION: SEQ ID NO:180

ATGAAACGGT TTTTGATTTT GATCGGCTTT GCACTGGCGG TAGCTTTCTC CGGTTTTTCC 60
CAAAAGTTCG CTTTGGTAGA TATGGAATAT ATCCTCAGGA ATATTCCTGA CTATGAGATG 120
ATGAACGAAC AGCTGGAACA GGTGTCCAAG AAATGGCAAA ATGAAATCGA AGCTCTCGAA 180
AATGAAGCCC AATCTATGTA TAAGAAGTAT CAGAGCGATC TCGTATTCTT GTCTGCTGCA 240
CAGAAGAAAA CCCAAGAAGA GGCTATCGTA AAGAAAGAGC AGCAAGCATC CGAGCTCAAG 300
CGGAAGTATT TCGGCCCGGA GGGGGAGCTG TATAAGAAAC GCTCCGATCT GATGAAGCCT 360
ATTCAGGATG AGATTTGGAA TGCTATCAAA GAGATTGCCA AGCGTAACAA CTATCAGATG 420
GTGCTTGATA GAGGTACGTC CGGAATTATC TTTGCCAGTC CGTCTATTGA CATTAGCGAC 480
CTTGTACTGA GCAAGATGGG CTTTAGCAAG 510

(2) INFORMATION FOR SEQ ID NO:181

(i) SEQUENCE CHARACTERISTICS:
(A) LENGTH: 489 base pairs

(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...489

(xi) SEQUENCE DESCRIPTION: SEQ ID NO:181

ATGAAGAAAT TTTTTCTCAT GCTTCTGATG GCTCTTCCTT TGAGCCTCTT GGCACAAAAG 60
GTGGCAGTGG TAAACACTGA GGAGATCATT TCCAAAATGC CGGAACAAGT AGCTGCTACC 120
AAACAGCTCA ACGAATTGGC CGAAAAGTAT CGCCTTGATC TCAAGAGTAT GGACGATGAG 180
TTTGCCAAAA AGACAGAAGA ATTTGTAAAG GAAAAAGACT CTCTACTGGA GAACATCCGC 240
AATCGTCGTC AGCAGGAACT TCAGGATATT CAAACTCGTT ATCAGCAGTC ATACCAAACG 300
ATGCAGGAGG ATTTGCAAAA GCGCCAACAA CAGCTTTTTG CTCCTATCCA ACAAAAGGTG 360
GCTGATGCCA TCAAGAAAGT GGGTGACGAA GAAAACTGTG CCTACATCAT GGAGGCCGGT 420
ATGATGCTTT ACACCGGAGC TACTGCTATT GACTTGACCG CAAAGGTAAA AGCGAAACTC 480
GGAATCAAG 489

(2) INFORMATION FOR SEQ ID NO:182

(i) SEQUENCE CHARACTERISTICS:
(A) LENGTH: 2481 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2481

(xi) SEQUENCE DESCRIPTION: SEQ ID NO:182

ATGAAGGAAG CTATTCCCCG AAAGAACAAG TATATAAAGC TCAACGGTAT ATACAGATTG 60
TCATTCATTC TGCTATGCTG CCTGCTATGC TCTCAGGCAG CTATGGCACA AGGCGTCAGG 120
GTATCGGGCT ATGTGCTCGA CCGTGGGGAA AAGCCGATCC CGTTCGCCGG AGTCAAAGTG 180
CGTGGTACGG GGACAGGCGC AACGACGAAT CTGAAAGGAT ACTACGAGTT TCGGATGAAG 240
GCCACGACGG ACAGCATCAC GATCGAGTTC AGCTCCATGG GGTACCAAGG GGTAAGTCGC 300
AGCTTTCCGT CTCTGACCAA GGACACTCGG CTGAATGTTC GTTTGGCAGA GGCCGAGATG 360
GAGCTTTCGA GCGTGACGGT ACAGGCCACA AAACGCAGAC TCAACACGAT GGAGCGCGTC 420
AATACCCGAG ACCTTCGTGT CAATGCAGGG CCTACGGGAG GGGTGGAATC GCTCATCAGT 480
ACCTACGCAG GAGTAACGCA GAACAATGAA CTAAGCTCGC AATACTCGGT TCGCGGAGGA 540
AGCTACGATG AGAATATGGT CTATGTAAAC GGAGTGGAGG TTTATCGCCC GCTGCTGGTT 600
CGCTCTGCAC AGCAGGAAGG TCTGAGCTTC GTCAATCCGG ATCTGACACA ATCCGTACAG 660
TTCTCCGCCG GAGGGTTCAC GGCCGACTAT GGCGACAAGA TGTCCTCCGT ACTGGATATT 720
CGCTACAAGC AACCGCAGGA GAAGGAAGGA GCGGTACTCC TCGGGATGCT ACAATCGAGT 780
GCCTACTATG GCAGCAGTGC CGGAGCCTTC AGCCAAATCA CGGGTCTACG CTACAAGAGT 840
GCCAAATCGC TCTTGGGCAC TACGGACACG AAAGCCGAAT ACGATCCGAT CTATGCGGAC 900
GGACAGACAT TCATGACGTA CCGTTTCAGC CCCAAGCTGT CGGTTAGTTT CCTCGGCAAT 960
ATTTCGCAAA CTCGCTACAA GTTTGTCCCT CAGACCCGTG AGACGAGCTT CGGTACACTG 1020
AGCGATGCCA AAAAGTTGAA GATCTTTTTC GACGGTCAGG AACAAGATCG TTTCCTGACC 1080
TACTTCGGTG CCTTCAGCAT GAACTTCGTG CCGGACGACA AACAGCGGCA TACGGTTACG 1140
CTTTCGGCCT TCAACAGTAA CGAACGGGAG ACCTACGATA TTCAGGGAGA ATACTTTCTG 1200
AACGATGTGC AGCTGGGGGC GGACGGAACT GCTTCGATGG CTTCGGGCTC AGAGAACTCC 1260
AACGGCTTGG GCATCGGGCG CAATCACGAG CATGCGCGCA ACAGGCTGAG CTACCGCGTG 1320

CTGAACATGG GTTACAGAGG GGAGATGAAG CTGAACGAGA AGCATCGCCT GCAAGCCGGC 1380
GTATCGGCAC AGATGGAGAA AATAGCCGAC CATATCAGCG AATGGGAACG GAGGGATTCG 1440
GTAGGATACA ACCTACCTCA CTCGGAGACC GTATTGCTGA TGTACAATAA CCTATATGCC 1500
GATACGCAGA TGAGGGGAAC GCGCTTGTCG GCATTCGTAC AGGATCGATT CAACTTCAGC 1560
ATGGGAGGAG GTACATTTTC TCTCATTCCG GGTATCAGAG CTTCGTGGTG GAGCTTCAAC 1620
AAGGAGTTGC TCGTCAGCCC ACGTATCAGC GTGGGTTATT CTCCCGAAAG CAACCCGGCT 1680
TTGGTACTGC GTGCAGCCGC CGGACTTTAT TATCAGGCAC CGTTTTACAA AGAGCTAAGG 1740
CAGACGCATA AGGATGCCGA AGGCAATAAC GTGGTTGTCC TCAACGAGAA GATCCGCTCT 1800
CAGGGAGCTT TTCACATTCT CGCAGGAGCA GACTATACCT TCGAAATGGG GGGCGAAAAA 1860
TACAAGTTTA CGGCAGAGGC TTACTACAAG AGCCTGTTCA ACATCAACCC GTATATAATA 1920
GAGAACGTGA AGATCCGCTA TCTGGGCGAA AACATCGGTT CGGGTTATGC TGCGGGTATC 1980
GATCTCAAGC TCTTCGGCGA ACTGGTACCC GGAGTGGATT CGTGGCTGAC GGCTTCCATT 2040
ATAAAAGCCC GTCAGAAACT GGATGGCTAC GGTTCTTTAC CACTGATGAA CGCACCCACT 2100
TACAATTTCT CCTTCTTCCT TCAGGAGTAC GTGCCGGGCA ATAAACGCAT CACAGCCACC 2160
CTGCGGGCTG CACTAAGCGG AGGATTGCCC CAGCTCAATC CGAGCAAAGG GCTTAGCTCG 2220
CCGGCCTTTA CCGCACCGGC CTATAAGCGT GTCGATCTGG GGGTAATGTA CAAATGGCTC 2280
GACCCGGATG ACTCCTTTGC CGGCCGAAGC AAATGGCTAA TGGGAGTAAA AGGGGCCTAC 2340
ATAGGGGCTG ACCTCTTCAA TCTGTTCGAC ATGACCAACG TCAATTCTTA CTACTGGGTG 2400
TCGGATGCCT ACCAACAGCA ATACGCCGTA CCGAACTACC TGACACGCCG CCAATTCAAC 2460
CTGCGTCTCC TCGTCGAATT C 2481

(2) INFORMATION FOR SEQ ID NO:183

(i) SEQUENCE CHARACTERISTICS:
(A) LENGTH: 2016 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2016

(xi) SEQUENCE DESCRIPTION: SEQ ID NO:183

ATGTACAGCG GACATCATAA AATCCATTAT CCTTTTCTTA TCCTGTTGGT ATGCCTTGCT 60
TTTGCTGCCT GCAAGAGCGT GAAGTTGAAA GATGCGGAGA AGGCACATGA TCGCCAAGAG 120
TATACCAAGG CTGCCGATAT GTACAATACA TTATACAGGC GTACCCGACG AAAGCAGGTG 180
GAGATGAAAG CTTATACGGC TTTCCGATCC GGTGAAAACT ATCGTGCCGC CGGCAGACAA 240
GCCAAAGCTT TGCGTGGCTA TCTGAATGCC AGACGCTACG GGTATCCGGA TTCTGTGGTA 300
CTGCTCCGTT TGGCACAGAC TTATCAGCAA GGAGGTAACT ATAAGGAAGC CGAGGTACTC 360
TTCCGTGGAT ATCTGGAAGC TTATCCGAAA AGTTATTTTG CAGCTATCGG TTTGGAGGGG 420
TGTCTCTTTG CCCGCCAGCA AAAGGAATAT CCTACACGTT ACCGGATACG GCGAGCTGCC 480
GAGTGGAATT CGGCACGGGG CGACTTCGGC CCGGCCTATG CACCCGATGC TTCGGCTCTC 540
TATTTCACAT CGAGCAGAAG CAAAGACGAC GGTTTGGATA ATAGCAGCAT AACGGGACTG 600
AAACCCAACG ACATTTATAT CATCAAACGA GATGCACAAG GACGATGGGG ACGTCCCGAT 660
AGCGTGTCCG GAGGAATCAA CACTCCATGG GATGAAGGCG TGCCAACGAT CACGCCCGAT 720
GGTAGTACCA TATATTATAC GTTGGCGCAG CAAGGAGCCG ATTACGACCG TACGGTACAG 780
ATCTATTCCG CCGCTCGGAG CGGAGAAGGC GGTTGGAGCA ACGGTTCGCT CGTGGACATT 840
ATGCGCGATT CGCTCCGTAT GGCTGCTCAT CCCTCTATGT CGGCATCCGG CGATTACCTG 900
TATTTCGTCA GCAATATAGG CGGTAGCTAT GGCGGCAAGG ATATTTATCG TGTCAAGGTG 960
TCGGATCGTT CTTATGGTTC ACCGGAGAAT TTGGGGCCTG ATATCAATAC GCCGGGGGAC 1020
GAAATGTTTC CCTTCATAGA TGGGGATAGT ACCCTTTTCT TCGCTTCGGA CGGACACGCC 1080
GGTCTGGGAG GACTGGATAT TTTCAAAGCC ACGCTGGACT CTACCGGCCA ATGGCATGTA 1140
GTCAATATGG GACAACCGGT CAATTCCTCT GCCGATGATT TCGGCTTGGC TGTGGAGCCT 1200
AAAGGCAAAA ACAAAGAAGA AGCTTTGCCG GACAACGGAG TCAAAGGTGT ATTTTGTTCC 1260
AACCGAGGCG ATGCACGCGG ATGGCCGCAC CTCTTCCATT TCGAACTGCC GGCTATCTAC 1320
ACCGAGATTC AAGGTTATGT GATGGACAGA GAAGAAAATC CCATAGCCGG AGCCACTGTC 1380
AGGATCGTAG GCGAACGCGG CCCCGTAGGA CAGGGATTCG TGACTACTCG TGACGATGGC 1440
TCCTATAAGA TGAGCGTGCA GGGCGATACT CGCTATGTAA TGCTTGCCGG AGCATCGGGT 1500
TATTTGAATC AGTACGTAGA ACTCAAGACC GATACCGCCA AGCAGAGTGA GACCTACTAT 1560
GTGGACTTTT TCCTTGCATC GCGTGAGAAA GCCGAGGGCT TGCAAAATAT TTTCTATGAT 1620
TTCGATAAAG CTACTCTTCG CCCCGAAAGC ATGAAGAGCT TGGACGAACT GATTCGTATC 1680
CTCACGGACA ATCCGGATAT TCGGATCGAA TTGGGTTCGC ATGCCGACAG GAAAGGCCCC 1740
GATGCTTACA ACCTCGGACT ATCTGACCGC AGAGCCAAAT CCGTGGTGGA TTACCTCACG 1800

AGTCGTGGCA TAGCGGCCGA CAGGCTTACG TGGAAAGGCT ACGGTAAGTC TGTCCCCAAG 1860
ACGGTGACAG CCAAAATTGC CGAACGGCAC GATTTCCTGA AGGAAGGCGA TGTGCTCACC 1920
GAGGAATTCG TAGCACCTTT GACCGAGGAG CAGCAGTCAG TCTGCGACCA ACTGAACCGT 1980
CGTACCGAGT TCCGTGTGAT CGAAGAAGAG TTGCGT 2016

(2) INFORMATION FOR SEQ ID NO:184

(i) SEQUENCE CHARACTERISTICS:
(A) LENGTH: 2124 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2124

(xi) SEQUENCE DESCRIPTION: SEQ ID NO:184

ATGAAAAAGT TTTTCTTCGC GCTACTATCG ATTGGTATTT CAGCGCAGGC TTTTGCCAAG 60
ACGGACAACG TCCCGACAGA TTCGCTACGA GTACACAATC TTCAGACCGT CACGGTCTAT 120
TCTACACGCA CGGCCGTACC TCTGAAAAAG ATACCGGCCA AGATGGAACT CATCTCATCG 180
CGCAACATCA AGCAGTCCGG CTTTAACAAC ATGACCGACA TCCTCAAGAC GCAAAGTTCG 240
CTCGATGTCA TACAATACCC GGGCTTTAGT TCGAACATCG GTATCCGCGG TTTCAAGCCC 300
TCCGGCAAGT ATGTAACCGT ATTGGTAAAC GGCATCCCTG CGGGAACGGA CAATATCTCT 360
ACGCTCAACA CGAGCAACAT CGAACAAATC GAGATCCTCA AAGGCCCGTT CTCTTCCATC 420
TACGGCACCA ATGCCATGGG CGGTGTGGTG AACATCATCA CCCACAAATC CAAGGACAAG 480
ATCCATGGCA ACGTTTCTCT CTTCGGCGGT AGCTACCAGA CCATGGCCGG ATCATTCAAC 540
TTGGGTGGCC GCTTCGAGGA TATTTTCTCA TTCGATCTTA GTCTGGGCTT GGACAAGCAG 600
AACAAGGACT ATAAGACCGG ATCAAACAAT TTCCTATCCC TGAGCAAACT GGAAGAAGCT 660
ATAGTAGATG TAAATGCTAC CAAAAACAAG AAAATGAAGG GGAGCGACTA TACTGTAGCA 720
ACGGGACGTC TGCGTTTCGG TATCGACTTC ACGCCCGAAT GGTCCCTGAA TCTGTATCAA 780
AACGTATTCC TCGGAGATGC GATCCCCGTA GGAGGATCTA TATGGGGCGT TTACGGAGAA 840
TCCAAAAAAA ATCTGAATCG TTCTTCGACC TCTTTCGAGC TGCTCGGCAA ACATGGCTGC 900
CACACGCTTC AATTCTCCCC CTACTTCAAC ATAGAGAAAT CGGAGAACTA TAACAATGCC 960
GATCCCACCG GTTTCATCAA CTACAAAAGC GACTACTACA CCTATGGTGC CCTACTCCAG 1020
GACAAGATTT CCTTTGGAGG ACAAAATATC GTACTCGGTG TCGACAGCCG AAACATGACG 1080
ATGGAGTCAG AAAGATTCGA GCAGGCAGGA GTGAATACAA AGCCATACAA CCCCGGATAT 1140
GCCACGAACA ATATCGGTTT GTTCGGACAG GCCAATTTCT ACCTGCTGAA CGATGCTCTA 1200
TCGATATCTG CCGGTGCACG TGCCGACTTC ATGTTCTTTG ACCTGAAAGC GAACGAGTAT 1260
CTCAACAATG AAGCCAAACA GGAAACTCAT AACGTAATCA ATCCGAATGT CGGAATCAAA 1320
TATGAGTTTG TGAAAGGCCT TACAGCTCAT GGTACATTCG GTAGTGCATT CAGTGCTCCC 1380
GATGCTTTCC AAAAAGCAGG CCAATACGTA GGCCCGTTCG GCACGACCAT AGGCAATCCT 1440
GACCTGAAAC CCGAAAAGTC CATGACCTGG GACTTCGGTA TCGGATACAG CAATGCACGC 1500
TGCGGGATCC AAGCCGACGT AACCTTAACC TATTTCCACA CCGACCACAA AGATCTGATC 1560
TTGTCCAGCC CTGACTATGC TAATAATATC ACCACATACA TCAATGCCGA CAAGGCTCGT 1620
ATGAGCGGTA TCGAGGCCCT TTTGTCTTAT GACTTCGGCA GCCTCTTTGC CAACAAGTTC 1680
TCTCTCCGCG CATTTGCGAA TGCCACGATC ATGCTCAATT CCGAGATGAA GAAAAGCCAG 1740
ACCGATGCCC CTTGGAGCGA AATGTACTAC GTTCGCAAGC AGAACATCAC CTTCGGTATC 1800
GAATATCGTG GCAAAGAAGG ACTTGAAGTG ATGCTCAACG GTCGCTTCAT GGGACGCAGG 1860
ATCGAGCAAA ACTGGTATGC TTACTACCCC GAAGTTCGCC CCGAACTCCA GCAACTGCTT 1920
GCAGCAGAAG AGCCTGAATT GGCTGCTCAG GGACTGCTCC GTCATCCGCA AGCAATGGTG 1980
TTCAATGCCT CTGCTTACTA CCACATGAAC AAGTATCTCA CCTTCGGTGT GAACTTGAAC 2040
AACATCTTGG ATGAGCTTTA TACGGAGAAA GACGGCTACC ACATGCCCGG ACGTAACATC 2100
ATGGGTAAGG TTATGGTCAA CTTC 2124

(2) INFORMATION FOR SEQ ID NO:185

(i) SEQUENCE CHARACTERISTICS:
(A) LENGTH: 1386 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1386

(xi) SEQUENCE DESCRIPTION: SEQ ID NO:185

ATGAACAGGT TTTCAAATCA TTGGCCCTGC ATCCTCGTGG GGTTTGTACT CTGGTTTGTA 60
TCGGCGAGTC GGACTGTGGC ACAAAACGCC TCCGAAACGA CGGTATCGTA CGATACGGAT 120
ACCGCCGTAC TCTCCGAAGC CGATGTGCTT CGGATCGCTC TTAGTGAGAA TGCCACAGTG 180
AAAGTGGCCG ATATGGATGT GCGCAAACAG GAATATGCAC GTAGGGCAGC ACGTGCCGAT 240
CTCTTCCCGA AAGTAGACCT CAATGGCGTT TACAGCCATA CGCTAAAGAA GCAGGTCTTA 300
TATATAGATA TGCCCGGTTT CAGCAGTAGC GAAGGTATCG AAATGGGGCG TACACACAAT 360
ACGCAAGGAG GGGTGAACGT CTCCATGCCA TTGGTGTCGG CACAGCTTTG GAAAAGCATT 420
GCCATGACCG GAGAACAGCT CGATCTGGCT CTGGAGAAAG CTCGCAGCTC CCGAATCGAT 480
TTGGTGGCAG AGGTGAAGAA GGCTTACCTC AGTGTATTGT TGGCCGAGGA CTCTTATGGC 540
GTATTCAAGC GCAGCTATGA CAATGCTCTG GCCAATTATA AGAACATATC CGACAAGTTC 600
GATCGTGGAC TTGTGGCCGA GTATGATAAG ATTCGAGCCA ATGTACAGGT ACGCAACATC 660
GAGCCTAACC TCTTGCAAGC GCAGAACTCC GTAGCCCTTG CTCTCTGGCA GCTCAAGGTC 720
CTGATGAGCA TGGAAGTGGA AACTCCGATC AGACTCTCCG GTTCATTGTC CGACTATAAA 780
GAACAAGTCT ATACCGGCTA TTTTGCCGCC GATACGCTTA TTTCCAACAA CTCCTCCCTG 840
CGTCAGCTCG ATATACAGCG TCGTCTGGCT GTCAGTGCAG ACAAGCTGAA CAAGTACAGC 900
TTCCTGCCTA CACTCAATCT GGGAGGGCAG TACACCTATT CGCTCAACAG CAACGACATC 960
AAATTCTGGG GCGAGGGACA ACGCTGGACG CCTTTCTCCA CCATATCGCT CAGCCTGTAC 1020
ATTCCTATAT TCAATGGAGG CAAACGTCTG TACAACGTGA AGCAAAGTGC TTTATCGATC 1080
CGTCAGATCG ATCTGCAACG ACGCCACATA GAGCAATCCA TCCGAATGGG AATCAAGAAC 1140
CAAAATGACC GTCTGCGTAC CTGTATGCAG AGATTTGTGG CCTCGGAAGA GGCTGTCCGA 1200
AGTGCAGAAA AGGGCTATCA GATAGCAGAG AAACGCTATC AGACAGGCGA AGGCACTCTC 1260
GTCGAGCTCA ACGATGCCGA TGTGGCTCTT TTGCAGGCTC GACTCAATTA TAATCAGGCC 1320
ATATTCGACT TTATGACCGC AAAGGCCGAA TTGGACAAGA TGAACGGCAT GGGGATTCCC 1380
GAACAA 1386

(2) INFORMATION FOR SEQ ID NO:186

(i) SEQUENCE CHARACTERISTICS:
(A) LENGTH: 1476 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1476

(xi) SEQUENCE DESCRIPTION: SEQ ID NO:186

ATGTGGGGGG ACAGCCATGG AGTGGCGCCG AACCAAGTGC GCCGAACGCT GGTGAAGGTA 60
GCCTTAAGTG AATCCCTTCC TCCGGGTGCA AAACAGATTC GTATCGGATT CTCTCTTCCG 120
AAAGAAACGG AGGAAAAAGT CACCGCCCTA TATCTCCTTG TGAGTGATTC TTTAGCGGTG 180
CGCGACTTGC CGGACTACAA AGGGCGAGTC TCTTACGATA GCTTCCCGAT CTCAAAGGAA 240
GATCGTACCA CAGCCCTTTC TGCGGATTCG GTAGCCGGAC GCCGCTTCTT TTATTTGGCT 300
GCGGATATAG GGCCTGTTGC TTCTTTTTCC CGATCCGATA CGCTGACTGC CCGTGTGGAA 360
GAGGTGGCTG TCGATGGCCG CCCTTTGCCG TTGAAAGAGC TGTCGCCTGC CTCCCGTCGT 420
CTGTATAGGG GGTATGAGGC CCTCTTTGTA CCCGGTGATG GCGGATCGCG GAACTATCGT 480
ATCCCGGCCA TTTTGAAAAC GGCTAATGGA ACACTCATAG CGATGGCCGA CAGACGAAAA 540
TATAATCAGA CGGATCTGCC GGAGGATATA GATATAGTCA TGCGGCGCAG TACGGACGGA 600
GGGAAATCGT GGAGCGATCC CAGGATTATC GTACAGGGAG AGGGGCGCAA TCATGGCTTT 660

GGCGATGTAG CCCTGGTGCA AACCCAAGCA GGAAAGCTCC TGATGATCTT TGTCGGTGGA 720
GTAGGCCTGT GGCAGTCTAC CCCCGATCGT CCTCAGCGCA CTTATATATC GGAAAGTCGG 780
GACGAAGGAC TGACTTGGTC GCCTCCTCGG GATATAACCC ATTTCATCTT CGGCAAGGAT 840
TGTGCCGATC CGGGACGCAG TCGCTGGTTG GCCTCCTTTT GTGCTTCGGG ACAAGGGCTT 900
GTGCTGCCAT CCGGTCGTAT CACGTTTGTG GCTGCCATCC GCGAATCAGG GCAGGAGTAC 960
GTCCTGAACA ACTATGTCCT CTATAGCGAC GATGAGGGCG ATACATGGCA GCTTTCCGAC 1020
TGTGCATACC GCCGTGGCGA TGAGGCAAAG CTTTCATTGA TGCCCGATGG CAGGGTACTG 1080
ATGAGCATAC GCAATCAGGG ACGGCAGGAG AGCCGACAGC GTTTCTTCGC TCTCTCCTCC 1140
GACGATGGCC TTACTTGGGA GAGAGCCAAG CAGTTCGAGG GCATCCATGA CCCCGGCTGT 1200
AATGGAGCTA TGCTTCAAGT GAAAAGGAAC GGAAGGGATC AAGTGCTGCA CTCCCTGCCT 1260
CTCGGCCCGG ATGGGCGTCG CGATGGAGCT GTCTATCTCT TCGATCATGT CTCCGGCCGC 1320
TGGTCCGCTC CCGTTGTTGT CAATTCAGGA TCGAGTGCCT ACTCGGATAT GACTCTGCTG 1380
GCGGATGGAA CGATCGGTTA TTTCGTCGAA GAGGGCGATG AGATCTCATT GGTTTTCATT 1440
CGGTTCGTCC TTGACGATCT CTTCGATGTC CGGCAA 1476

(2) INFORMATION FOR SEQ ID NO:187

(i) SEQUENCE CHARACTERISTICS:
(A) LENGTH: 735 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...735

(xi) SEQUENCE DESCRIPTION: SEQ ID NO:187

ATGAAAAAAG AAAAACTTTG GATTGCGATC GTCGCCGGTT TGGCTTTCGT ATTGGGCCTT 60
TATGCTCTTG GCCGCAGTGT CGCTCAGCTA CGCCGCTCTC AGCCTTCGGT GACTGTGACC 120
GGTATGGCCG AGCGTAATTT CAAATCCGAT CTGATCGTTT GGACTGCTTC GTACCAGCTC 180
CAGATGATGG ATCTCGAATC GGCCTACAAG GCTTTGAAGG AAAAACAGAT ATTGGTAGCA 240
GACTATTTGA AAAACAAGCA GCTGCCCGAT TCGTCTTATA TCTTCTCAAG CGTAGCCATC 300
TCTAAAGAAT ACAACTACTA TTACGATCCT CGGCAGGAAC AAAACGTCAG GACCTTTGCC 360
GGGTATCTGC TCAGCCAGAC AGTTACGGTG ACCTCACAGG ACATCGAACA TGTGGAGAAA 420
ATATCTCGCG ATATAACGGA GCTGATCAAT CAGGGGGTAG AGATTACCTC CGACCGTCCG 480
GCCTATTACT ACACCAAGCT CAATGATCTG AAGGTGGAGA TGCTGCGCAA TGCCTCCGAA 540
GACGCTTTCA ATCGTGCTTC GGTCATTGCG GAGGGGAGCG GTTCCTCCGT GGGTAAGATG 600
CTATCTTCTT CGATGGGCGT GTTCCAGATA GTGGGGCTCA ACTCGAACGA AGATTATAGC 660
TGGGGAGGTT CGTTCAATAC GTCTTCCAAG ATGAAGACGG CAAGCATAAC GGTTAAGGCT 720
TCTTTCGCTT TGAAG 735

(2) INFORMATION FOR SEQ ID NO:188

(i) SEQUENCE CHARACTERISTICS:
(A) LENGTH: 828 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...828

(xi) SEQUENCE DESCRIPTION: SEQ ID NO:188

ATGAAAAAAA CAATTGCAAT TATCGCCTCA GCCCTCTTGG CTTTAGGAGC CGTCGGCTGT 60
AAGAAAAATG CTGACACTAC CGCTGTCAGT GAAAAGGATA GCATAGCCTT GTCCATGGGT 120
ATTTTGTACG GACAGGATTT TGCCAATCAG TTCGAAATGT CCCGCTTGCA AGGCCAGCCG 180
ATTGATTCGG TAGCTTTCTT GGACGGTTTC AAATATGGTA TCGATACGAC GCGCTTCTCG 240
TACAATCTGG GAGCCATCTA TGCTTCCAAT ATAGCTCGTC AGCTGGCTCA TGATTCCATC 300
GATATCGACA AGTTCTATGC AGCCATGCGT GCGGCTCTTC TTAAAGACAC CGTATCTATC 360
GCCATGAAGC CTGCAGATGC ACAGGCTTTC ATGCAACGAA TCCAAGCCAA AAAGCAGCGA 420
GAAAACAATA TGAAGCAGTT TGGCCAGAAC ATCGAAAAGG GTAATGAATA CATCGATACC 480
TTTAAAAAAG AAGATGGTGT AACTGTTACG ACAACTGGTC TGGCATACAA GACTCTTCAG 540
GAAGGTACGG GAGCTACTCC CTCTTTGGCC GATACTGTAC GTGTCAAGTA TGTGGGTACT 600
CTGGTCGATG GTAAAGAGTT CGACAAAAAC GAAGAAGGAA TCGAATTTGC CGTTACCGGT 660
GTGATTAAAG GCTGGACGGA GATGCTCCAA CTCATGAAGG TCGGTCAGAA AGTTCGCGTG 720
GTAATCCCAC AGGAGCTGGC TTATGGGGAG ACCGGCAACT ATACCATCGA ACCGTTCTCT 780
ACCCTGACGT TCGAGATGGA ACTTATCGGG ATCAAGCCCG GGAAAAAG 828

(2) INFORMATION FOR SEQ ID NO:189

(i) SEQUENCE CHARACTERISTICS:
(A) LENGTH: 2325 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2325

(xi) SEQUENCE DESCRIPTION: SEQ ID NO:189

ATGAAAGTAT TACGGCAAGT ATTCCTCCCC ATCCTTTTTG TCCTACTGAC AGGTGCCTGC 60
TCCACCACAA AGAATCTGCC GGAAGGCGAA CAGCTGTATA TCGGAATGGG CAAGACACAG 120
ATACTCCGGC AGGACAAGAG CCACGCCGGC CAACAGGCTC TGACCGAAGT GGAGAGTACA 180
CTGAAAGTTA CACCCAATGG AGCTATTTTC GGCAGTGCAA GTGCCTCCTT ACCCAAGATA 240
CCATTCGGGC TATGGCTATA CAACAGCTTC GTGGGGGATT CCACTGTCAT TTCGAAATGG 300
ATATTCGACA AGTTTGCAGC CAAGCCGGTT TTCATCAGTC AGGTCAAATC CGATAGCCGG 360
GCTAAGGTGG CGACGAACAT CCTCCGCGAA CACGGGTACT TCGATGCTAA AGTAAAAAGC 420
AGTGTGACCA CTCTGAAAAA GGACTCGCTC AAAGCCAAAA TCTCCTATAC GGTGGATATG 480
GCCTCTCCTT ATCATTACGA CAGCATCATT CCCTTACCGA TCAGCACTTT CCCCGACAGC 540
ATTCTGGCTT ACAGGCAGAC TCCGTCTTTG ATCAGGAAAG GAGACCAGTT CAATTTGGCA 600
AAGCTGCACG AAGAGCGTCA GACCATCAGT GCCCTGCTGA GAGACAATGG TTACTACTAC 660
TTCCGCCCAC AGGATATTAT CTACGAAGCC GATACCCTCC TCGTAAGAGG TGCCGTATGC 720
CTGCGAGCCA AGCTCTCGGA AGATACTCCA CCCCAAGCCA TGCGCCCGTG GAGGATAGGG 780
AAACGGACAG CAGTCCTGCT CGGAATGAAC GGAGAAAGCC CGACAGACTC GCTCGAAGTG 840
GAGGATATGA AAGTCCTTTA CTATCGTAAA ATGCCGGTTC GCCCCAAGAT TTTGGCCAAA 900
CGCTTTCGTT TCTTCTCCGG CAATCTGTAT CGGCAGAAAG ACGATGAGAC GACACGCAAA 960
TCCTTGGCTC GTTTGGGAGC CTTCTCCGTT ATCGATCTCA ATTTTTTGCA ACGCGATTCC 1020
ATTTCCGGCC TTTTGGATGT GCGACTGCTA ACCACCCTCG ACAAACCTTG GGATGCATCA 1080
TTAGAGACCT TGTTCACGAG CAAAAGCAAT GACTTCATCG GTCCCGGACT GAATTTTGCT 1140
CTTGCTCGGC GCAATGTATT CGGCGGAGGA GAAAATCTTT CTTGGAATAT CGGTGGATCG 1200
TATGAGTGGG AGACCGGCAA TCGTCCCGAA AATAGCAGCA ATCGGCTGAT CGATATAAAT 1260
TCGTACAACA TGAATACGGC CGTGAACCTC TCGTTTCCCT CGATTGTATT TCCCGGTCTG 1320
CTGGATAAAT ACTATTACTA CCCCACGACT ACGACTTTTC AGGCTTCTGC CACCGCGCTG 1380
AACAGGGCAC ACTACTTTAG CATGTACTCT TTCGGCTTTT CGACCACCTA CGAATTTCAG 1440
CCCTCCAAGG AACACCGGCA TGCTATTTTC CCGCTCAAGC TCAACTACAA CCTCCTGGGG 1500
CATCAGACAG AAACTTTCCA GGCCATTACG GCGAACAATC CGCCCCTGCT GCTCAGCCTT 1560
CAGAGTCAGT TCCTTGCTCA AATGGGGTAT ATCTATACGT TCAACAAATC CGTTTCAGAG 1620
AAAAGTCCTC ATCATCTTTG GATGCAATTC GGACTATCCG AGGCAGGCAA TCTCCTGAAT 1680
CTGATCTATC TGGCAGCCGG CAAGAAGTAC AGCGACACCA AGAATTTCGT CGGCGTCCCC 1740
TTCTCTCAGT TCATCAAAGC CACGGGAGAA CTGCGCTATT CCTATACCAT AGACCGCAAT 1800
CAGTCACTGG CAACCCGTTT CGGGACAGGC GTGATATATA GCTATGGCAA TATGCGAGTG 1860
GCACCCTATA GCGAGCAGTT CTATGTAGGC GGTGCCAATA GTATCAGAGC TTTCACCGTC 1920
CGTAGCATCG GCCCCGGACG GTTCAATCCG GATTCCGACA ATCAGTATTC CTATTTGGAT 1980
CAGGTGGGCG AATTCAAACT CGAAGCCAAC GTGGAATATA GAGGCAAGCT TTTCGGGGAT 2040

CTCCACGCAG CCGTTTTCCT CGATGCGGGC AACGTTTGGC TCTTGAGGGA GGATTCTTCC 2100
CGTCCGGGCG GTGCTCTGTC CGAAGTGGGA TCGGTGAGCA ATTTCCTGAA TAGCATCGCT 2160
CTCGGCACCG GTGTCGGCCT TCGCTACGAT CTGGCATTTC TCGTGGTTCG TGTCGATGTC 2220
GGCTTCGGTC TCCACCTTCC TTACAATACG GGTAAGAAAG GTTACTACAA TATCCCACGC 2280
TTTAAGGATG CCATCGGTTT CCATTTGGCT GTCGGCTATC CCTTC 2325

(2) INFORMATION FOR SEQ ID NO:190

(i) SEQUENCE CHARACTERISTICS:
(A) LENGTH: 2322 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2322

(xi) SEQUENCE DESCRIPTION: SEQ ID NO:190

ATGTCCTCGC ATTCCGTTCG GTATCTAATC GGCATTGCCG GCTGCTTGCT CCTCATGCTT 60
GCTTCCTCCT GCTCGGTCAC CCGTTATGTG CCGGACGGTA GCAGACTATT AGACAGGGTA 120
ACGATCGCAA GCGAAACGGG CAGTATCGCT CTGCCGGAAG ATATTCGGGA CTATACCCTC 180
CAGCAACCCA ATTACAGACT GTTCGGGATG ACTCGCTGGC TACTGCGCGT CTATAGCAGC 240
TCGAATCCGA ACAGCAACAG CTGGTGGAAC CGTTCGCTCC GGAAAATGGG CGAACCGCCT 300
GTCCTCATCG ATTCTGTCCT CACCGATCGT ACTGCCAACC GTCTGGCAAA GGCGATGGCC 360
GGCGATGGCT TTCTCGATGC TACTGCTCGT GCCGTGGTAG ACACCGGCTT GTACAAGAAA 420
GCTCGCATTA CTTATCTGAT TCAGCCCGGA AGCCGTTATT ATATACGCAA TATGGCTTTG 480
GATGTGAAGA ATCCACTCCT TCCTCCCGTT GCGCTTGGCA ATTCGCTTCC TTCGGCATAC 540
AAGGTCGGGA TCAGCGAGGG TTCTCCCTTG TCGCCCATCG TACTCGATGA AGAGAGAAAG 600
GCGATAGCTC GTCATATGCG CAACAACGGC TTCTGGAAGT TCTCCGCCGA GGATGTTTAT 660
TATGAAGCAG ATACTACCGT TTCAGGAGGA TCGGGTACGA AATCTGCCGA TCTGAAATTA 720
GTGGTCAATG GCATCGGGCG TTATCCATAT CGGATCGGCA GGGTATTCTT TCATGCCGAT 780
TATGATCCTC TCGAATCGGA CTTCAGAGTT CAGGAGCTGC CACGTATCGA TTCGATTTCG 840
CGTGGCGATT ACACTGTTTA CTATGGGAGT AGGGGACGTT ATATCCGGGC ATCGGCTCTC 900
ACGCGGTCGG TGTCCGTTAC ACCGGGAGCT TTTTTCTGCG AGGATGATGT GGAACGCTCT 960
TATATCAAGC TGAATGCGCT CCCTATCGTT CGGAACGTGA ATATCCGATT TGTGGAGCAC 1020
AATGGTAAGG ATGAGATTGC TCTGGCGGAT AGCTCTCGCC TTGTGGACTG CTATATTCTT 1080
ACCGTTCCGG CCAAGAGCAA ATCGTTCGAA GCCGAAGTCC TCGGCACCAA TTCCGCTGGA 1140
GACTTCGGGG CGGCTTTGTC TCTCGGTTTC ACCGATCGCA ATTTGTTTCG TGGGGCGGAG 1200
ATGTTCAATA TCAAACTCAA GGGTGCTTAC GAAGCCATTC GCAAGGGTTC GCACAGCTTC 1260
ATGGAATATG GGGTGGAAAG CTCGCTCCGT TTCCCTCGTC TCCTCTTCCC ATTCATTTCT 1320
GACGAAACGC GCCGGCGGCT ACGGGCATCC ACGGAATGGA AGATCGGGTA TAATTACCAG 1380
ACACGTCCGG AGTTTGATCG GGTGATTCTC TCCGCTCAAC TCAATTATTC ATGGCAGACC 1440
TACCTGCACA ATCGTCTGCG TCATACGATC CGCCTGCTGG ATGTCGATTA TCTCCATCTC 1500
CCGTACATCG ATCCCGACTT CGCCCAATCC CTTCCGCCTA CGACTGCACT GTATAACTAC 1560
ACGGAGCAGT TTATCCTCGG CTCGGCATAT ATACTGAACT ATACCACGGC TTCGTCCATG 1620
GAGCGTACCG TATCCAATCC TTTTACGGCA CGGTTCAGTA TCCAGACAGC CGGCAACCTG 1680
CTGCAAGCCA TTTCTTATCT GACCGATTCT CCGAAAGACG AACACGGGTT GTATAAAATG 1740
TTCGGTCTGC ACTATGCTCA GTTCGTCAAG CTCGATCTCG ATCTGGCTAA AACCGTTCTT 1800
CTCGAAAAGG ACAATACTTT GGCACTGCAT CTGGGTTTCG GACTGGCTTT CCCTTATGGC 1860
AATGCTCGCC ATATACCCTT TGAGTTACGT TACTTTGCCG GAGGATCGAA CAGCGTTCGC 1920
GGCTGGAGTG TCCGTACCCT CGGCCCGGGG AGTATGAAGA TGACTCCGGA CAAGACCTTC 1980
TTCGATCAGA TGGGTGATAT TCGTCTGGAT CTGAATGTCG AATACAGGAC AAAGCTGTTC 2040
TGGAAGTTTC GCGCAGCAGC TTTTGTCGAT GCCGGCAATG TCTGGACGAT AAAGGAGTAT 2100
GAGAATCAGG AGGACGGTCT CTTTCGTTTC GATCGCTTCT ACAAGGAAAT AGCTTTGGCC 2160
TACGGTCTGG GGCTTCGTCT CGACTTCGAT TATTTCCTTG TGCGGCTGGA TGCCGGACTG 2220
AAAGCCTACG ATCCTCAGCA GACAGGGCGT TACAAATGGG CTATCACACG CCCAAACCTT 2280
TCTTCCAATT TCGCTTGGCA CATTGCAGTA GGCTATCCGT TC 2322

(2) INFORMATION FOR SEQ ID NO:191

(i) SEQUENCE CHARACTERISTICS:
(A) LENGTH: 2601 base pairs

(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2601

(xi) SEQUENCE DESCRIPTION: SEQ ID NO:191

ATGAGAAAAA GAATTCTACA ACTTTTCCTG ACCGCATTGC TGCTGGCATT AGGCTCCTCT 60
CTCGCCATAG CGCAAACAGT GGTGACCGGT AAGGTGATCG ATTCAGAAAC GTCCGAACCG 120
CTCATCGGTG TATCCGTAAG CACCGGTCAG GGAGCATCCC TCCGCGGTGT AACCACCGAT 180
ATGGATGGTG GCTTCCGATT CGAAGTACCG GCCAAATCTG TCTTGACTTT CCGTTGCGTA 240
GGTTATGCTA CCGTAACTCG CTCTATAGGC AGAGGTTCTC AAGAAGACCT CGGTACGATT 300
CTCCTCGATC CCCAGGCCAT CGGCTTGGAT GAGATTCAGG TAATAGCCTC TGTGGTGCCC 360
AAAGACCGTA TGACGCCGGT ACCCGTTTCC AATATCCGTG TGGCTGATAT TCAGGCAGCA 420
TCGTTGAATG TCGAATTTCC CGAACTGGTT AAATCCACTC CCTCTACCTA TACGACAAAA 480
GGAAGCGGAG GTTTCGGTGA TGGTCGTACC AATGTGCGTG GATTCGACAC TTACAACTTC 540
GGTGTACTCA TCAACGGAGT TCCTGTCAAT GGTATGGAAG ACGGGAAAGT ATATTGGAGC 600
AATTGGAGTG GTCTGATGAA TCAAGCCAGT ACCATTCAGA TTCAGCGCGG ACTCGGAGCC 660
TCCAAGCTCG GTATCAGCTC GGTAGGTGGT ACGATGAACA TTATCACGAA GACTACGGAC 720
GCCAACACCG GAGGTTCGGC TTATGTCGGT ATGGGTAATG ATGGATTGCA CAAAGAATCG 780
TTCTCCATTT CTACGGGTAT GAACGACGGT TGGGCTATCA CCATTGCAGG CTCCCATATG 840
ACGGGTCTGG GTTATGTGAA GGGGCTGAAG GGACGTGCAT TCTCTTACTT CTTCAACGTT 900
TCGAAGAAGT TCAATGAACG TCATACCCTC TCTCTTACCG GATTCGGTGC ACCACAATGG 960
CACAACCAAC GTTCTTCCAA ATATTCTGTA GCCGACTATG ACAAATACGG CATCCGTCAC 1020
AATCAATCCT TCGGCTATCT GCGAGGCGAA CTGACTCCTA CGGCTTATGC TTACAATACG 1080
TACCACAAGC CCCAGTTCTC GCTGAACCAC TTCTGGAAGA TGGATGAAAA TACCTCTCTT 1140
TATACCgCAN CCTACGCATC TTTGGCTACC GGTGGAGGTC GTCGCGCTTA TGGAAAGAAC 1200
AGTAAGTGGG TATTGATCAA CTACAACACC GGACAACCCT ATGAACAAAC AAAGGTGACT 1260
CCCGATGGAC TTATCGACTA CGATGCCGTA CTGGCTGCCA ATGCTGCGGC GAGCAATGGC 1320
TCGGAAGCAA TTTTTGCCCT TGGCTCCAAC TCTCACAAGT GGTTCGGTCT ACTCTCTTCA 1380
TTCAAGAAGA AACTTAATAG TTCGCTGACT TTGACAGCCG GATACGATGG GCGTTACTAC 1440
CGTGGCGACC ACTATGACAA GATCACCGAT CTGCTCGGCG GTAGCTACTA CATAGAGGAT 1500
CCCAAGACAA AGCTCGCATA CCATGCGGAA GGTCAGCAAC TGAAAGTGGG TGACATTGTA 1560
AATCGGGACT ACACAGGCGA AATCATGTGG CACGGCCTCT TCGCACAGAT GGAGCATTCG 1620
TCCGAATGGA TCGATGCATT CGTATCAGGA TCTATCAACT ACGAACTATA CCGCAATCAC 1680
AACTATGGCG GTAGCAAGTC CACCGGCTAC CTGCCCGGCG TATCGCCGTG GAAAAGCTTC 1740
CTTCCGTGGA GTGGCAAGGC AGGTCTGAGC TACAAGTTCG CACAGGGACA CAATGTATTC 1800
GCCAATGGCG GTTTCTTCAC ACGTGCACCA CTCTTTGGCA ATATCTATGC TGCGGGGGCT 1860
ATCATTCCCA ATGACAAAGC CAATATGGAA AAGGTGCTTA CAGGAGAGGT CGGCTATGGA 1920
TTCACGAATC ACAAAAACTT CGAGTTCAAT ATCAACGGAT ACTATACGAA GTGGATGGAT 1980
CGCGTGACCT CGAAGAGAAT CGGAAACGAG TATGTTTATC TCAATGGCGT TGATGCTGTT 2040
CACTGTGGGG TAGAGGCTGA GGTCAGCTAT CGTCCTATTC GTCAGATCGA CCTTCGCGGT 2100
ATGTTCTCTC TCGGTGACTG GACTTGGCAA AACAATGTAA GTTACACTTC TTACGACGAA 2160
GCCGGCAATG AGACAGGGCA GGATATAACC TATATCAAGG GTCTTCACGT CGGAGATGCA 2220
GCACAGATGA CGGCTGCTGT ATCGGCAGAC ATAGAGCTGT TCAAGGGTTT CCATGTCATA 2280
GGTAAGTACA ACTTCCTTGG CAAGAACTAT GCAGGATTCA ACCCCGCAAC GCGTAATGCA 2340
CAGCAGTACG AAGCGGATGG CAAAGAAATC GTGGAATCAT GGAAGTTGCC CGATGTACGT 2400
CTGTTCGATC TGTCTGCATC CTACAATTTC AAGCTTGGTT CACTCAGCAC CACATTCTAT 2460
TTCAACATGG ACAACGTAGC CGACAAGCGA TATGTGAGCG ATGCCGACGA CAATATCATC 2520
GGTAAGAAAC ACGATGAGGC TTCGGCTCTC GTATGGTACG GTTTCGGCCG CACTTGGTCT 2580
ACCGGTATTC GTGTAAACTT C 2601

(2) INFORMATION FOR SEQ ID NO:192

(i) SEQUENCE CHARACTERISTICS:
(A) LENGTH: 1293 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1293

(xi) SEQUENCE DESCRIPTION: SEQ ID NO:192

ATGAAGTTTT CAATCCGCCT TTTCCTCTGC ATCATCTTTC TCCTCTCTGC ATTTATCCTG 60
CCTGCTCTCG GACAAAAATC CAAGCAGGTA CAGCGACTTG AGAAGCAACG TAAGGAGGCC 120
CTCAAAGCCA TCGAAAAAAC CGATCGCGAA CTACGAAATA CCAAGAAAGA CAAGCAAGAC 180
AAACAAAAGC ATCTCAACCT CCTGAACAAG CAGGTTGCTC AACGCAAGCA GATGGTACAA 240
CTCTTGGACA ATGAGGTCAA AGAGTTGCAA TCCGACATTG ATTCCATGAC GGGTGTATGT 300
CATCAGCTCT CTGTAGAAGA GAAAGCCCGA TCCGATGAAT ATGCCCAAGC TCTACAGTCT 360
ATGCAAAAGC GGAAACGCTC GTTGGATCGC ATCCTTTTCA TTTCATCGGC CAAGAGCTTT 420
GACGAAGGCA TGCGACGGAT GCGTTTCTTG GAACAATACG CTTCTGCATA CAAGCTGGCA 480
TCTGTCCGGC TGCGCGATAC ACGTAGCAAG TTGGAGACTG AACGTGCGAC TGTAGAAGAC 540
GCCAAAAAGG AGAAAGGACA TCTCTTAGTC ATCAGAGAAG AGGAAAAAAA GAAACTCGAA 600
GGACAGCAAG CCGAGCAACG TCGGCAGGTG CAGGCTTTGG GAGCCAAACA AAAAGACTTG 660
GAAGCGCAGC TGCGAAAGCA GAAAAAGCAA GCCGAAGCTC TGAACAGAAA GATCGAGAAA 720
CAGATTGCCA AGGAAATAGA AGCTGCCGAA CGTCGTGCTC GAGAAGAACG TGAACGGTTG 780
GCACGCGAAG CCAAAGCCAA GGGTAAGCCG GTTCCTGCCG AACCGGAACG GAAGGCGGAC 840
ACCAAAGGCG GCTATGCTAT GGATGCCTCT GAGCGTGCTC TCTCGGGCAG CTTTGCACAG 900
AACAAAGGTC GCCTGCCCGG CCCCGTTCGC GGCAGATACC GAATCGTAAG CGACTTTGGC 960
GTGCATCAGC ACAGTGAGCT GAAAAAAGTA CAAGTTAATA ATGGAGGTAT CGACATCGCT 1020
GTAGCAACAG GATCCGATGC TACCAGCGTA TTCGATGGTG TAGTGTCCAG TGTATTCGTG 1080
ATACCCGGTT ATAATTCGGC CGTAATGGTT CGTCACGGTA ACTATATCAC GGTTTATGCG 1140
AATCTGAGCA AAGTGTATGT AAATTCCGGC ACTCGTGTTA AAACGGGTCA GGCTCTTGGT 1200
CGTGCCTATA CGGATCCTTC CAACAACCAG ACCATTATTC ACTTCGAAAT CTGGAAAGAA 1260
CGCAGCAAAC AAAACCCAAG ACTATGGTTA CGA 1293

(2) INFORMATION FOR SEQ ID NO:193

(i) SEQUENCE CHARACTERISTICS:
(A) LENGTH: 999 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...999

(xi) SEQUENCE DESCRIPTION: SEQ ID NO:193

ATGAAAAAGT ATTTGTTATA TGCCTCGTTG CTAACGAGTG TTTTGCTCTT TTCCTGTTCA 60
AAGAACAATC CTAACGAGCC GGTGGAAGAC AGATCCATCG AAATTTCTAT AAGGGTAGAT 120
GATTTCACCA AAACGGGTGA GGCAGTACGC TATGAAAGGA ATCAAGGAAG TGCTGCCGAA 180
AGGCTCATTA CCAATCTTTA CCTCTTGTTG TTCGATCAGT CAGGGGCGAA TCCGGCGAAA 240
TACTATATTA CCGGTAACAC TTTCACCGGA GGGACCTGGC TTCCTGACGA TATGAAGGTG 300
AAGTTGGATA TGACACAATC CGAGGCCGGA GAGCGCAAAG TATATGTCGT AGCCAATGTT 360
GATAATGCGC TTAAAACGGC TCTTGATGCT GTCGCTAACG AAAGCGATTT GCAGACTGTA 420
AAGAGGACGA CTGCAATGCC GTGGTCGACC GATATAGCCT CTCCTTTCCT GATGTCCGGA 480
AACAAGACAC ACGACTTCTT GGCCAATCGT CTTTTGGACA ATGTGCCCCT TGTGCGTGCC 540
ATTGCCAAGG TGGAGCTGAA TATCTCGCTG AGTGAGAAAT TTCAGATTGT GCCGATAATT 600
GTCAATGGTA GTTTGAGTGA GTTCAAGTTC AGATACGTAA ACTTCGACAA GGAGACCTAC 660
GTAGTGAAGC CAACGACCAA GCCGGACAAT CTCATTAGTT CTGCTAATGG TGTTTGGCCT 720
CAGATTACAG ATTGGACTGT ATGGGGTGCT TCCTTAAATA CTTCTCCTGC TCCGGATGCG 780
GGCACAGGTT ATACATTGGA TGCAAATGGC AAGGTAACGG CACTACGGAT TGTTACCTAT 840
CTGAATGAGC GCGATAGCAA AGGGGCTACG GTAGAGGTCG CATTGCCTCG TGTGGATGAT 900

GGCACCCTTC CTCCTCCGGA ATTCGGTCCG GAGCTTTATC GTTTGCCTTT GCCGGACAAG 960
ATCCTGCGCA ATCATTGGTA CAAGTATGAA GTCGAGATT 999

(2) INFORMATION FOR SEQ ID NO:194

(i) SEQUENCE CHARACTERISTICS:
(A) LENGTH: 945 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...945

(xi) SEQUENCE DESCRIPTION: SEQ ID NO:194

ATGATCAGAA CGATACTTTC ACGATATGTA TCCTCGAACT TTTGGAGTCG GGGAGCTACC 60
TTTTTTTTCA CGATTTTCCC GGCCTTCATC CTCGCCGCTA CTGCTTTGCC GGCTTGTGGA 120
GGGGGTACTG CTTCAGGCTC CGATCGTACG CTGGCTGTGA CCATCGAGCC ACAGAAATAC 180
TTCATCGAGT CCATTGCGGA TAAGTCGGTG CAGGTGGTGG CATTGGTACC GGCCGGCAGC 240
AATCCGGAGG AATACGACCC TTCGCCTACC GTGATGAAGC GTTTGTCCGA AGCAGATGCC 300
TACTTCTATA TAGGAGGACT GGGGTTCGAG CAAAGAAATC TCGCTGCCAT TCGGGACAAT 360
AACCCTAAGC TCCCTCTTTT CGAAATGGGC AAAGCCTTGG CGGATGCCGG AAGTGCAGAT 420
CTCCACGGCT CCTGCACAGA TCATTCTCAT ACAGACCTGC ATGCCCATGA TCCGCACTAT 480
TGGAGCAGTG TGGTAGGGGC AAAGGCACTC AGTCGTGCTG CATACGACGC GCTTGTGGAG 540
CTTTATCCGA ACGAGAAAGA CAAATGGGAC AAAGGGCACG ACCGTCTCAA CGGACGTATC 600
GACAGCCTGA AGAGACTCGT CGATACCATG TTTGCCAATG GCAAAGCAGA CAAAGCCTTC 660
GTCATATATC ACCCATCGCT CAGCTTTTTC GCCCAAGAGT TCGGCCTGCG GCAGATCGTC 720
ATAGAGGAAG ATGGGAAAGA GCCTACGGCT GCCCACCTTC GTCGTGTGAT CGATCAGGCA 780
CGTGCCGATG GTGTCAGAAT CGTATTTATC CAACCCGAAT TTGAAACGCG TCAGGCGGAG 840
GACATCGCAC GCGAGATCGG TGCTCGTCCG GTAAGGATCA ATCCTCTGCG CAGCTCGTGG 900
GAGGAGGAAA TTTTACATAT TGCTCGCGCT TTGGCTCATG AACGG 945

(2) INFORMATION FOR SEQ ID NO:195

(i) SEQUENCE CHARACTERISTICS:
(A) LENGTH: 2544 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2544

(xi) SEQUENCE DESCRIPTION: SEQ ID NO:195

ATGATCGGAA AAAAAATCTT TTTTATCCTG CTGGCGCTCA TTGCGTTCAG TGGGCTGAAC 60
GCAGCGACAG ACACTGAGTT CAAGTACCCG ACCGATGCCA ATATCATCGG TCACGTCAAA 120
GACAGCAAGA CGGGTGAACA CCTTGTCGGT ATCACTATTG CTATCAAAGG CACTACCTTT 180
GGTACATCTA CAGATGCAAC CGGGCACTAC TATCTTCGTA ACTTGCGTCC GGGTGAGATC 240
ACTTTGATTA TGCGTGGCAT GGGCTATAAG AGCCAGGAGC GCGTAGTCCG CGTAGAAAAG 300
GACAAGACTA TCGAGGTGAA TTTCGAAGCA GAAGAGGATG CCATCAATCT GGACGAAGTC 360
GTGATTTCGG CCAACCGCGA ACTGACGCTT CGCCGTCTTG CTCCTACTCT GGTAAATGTA 420

TTGAACGAAA AAGTCTTCTC GCAAGTCAAT GCTTCTAACC TGGCTCAAGG CTTGTCATTC 480
CAGCCGGGAG TTCGTGTAGA GAACAACTGT CAGAACTGTG GTTTCAATCA AGTTCGTATC 540
AATGGACTGG ATGGTCGTTA TGCACAGATC CTCATCGACA GCCGTCCCAT CATGAGTGCC 600
CTTGCCGGTG TTTACGGTCT GGAGCAGATC CCTGCCAATA TGATCGAACG TGTGGAGGTA 660
GTACGTGGTG GAGGATCGGC CTTGTACGGT TCTTCTGCTA TTGCCGGAGT GGTGAATATC 720
ATCACCAAGG AACCTTCTCA CAATTCTTTC ACATTCAATG AATCTCTGAG CTTTACCGGT 780
TTCAGCAAGC TGGATAACAA CACGAACTTC AATGCCTCCA TCGTCAGCGA TGACAACCGT 840
GCCGGTGCCA TGGTATTCGG GCAGGCTCGT TACCGCAACC ATTGGGATGC TAACAATGAC 900
GGTTATTCCG AATTGGGTAA AATAGATGCC CGCTCGCTGG GAGCGCATTC TTATTTGCGC 960
TTGAGCGACT ACAGCAAATT GACGGGAGAG TTTCACACGA TCAGTGAATT CCGCCGTGGT 1020
GGCGATCGTA TCGATTTGCC TCCTCACGTA GTGGGTGTAG CTGAACAAAC TGACCATAGC 1080
GTATTTAGCG GAAACTTGAA ATACGATCTC TTCTCTTCCA ACTATAAACA CCACTTCCAG 1140
GCTTATACTT CCGGACAGAT CGTAAATCGC AAGAGCTATT ACGGAGGTAT CGGAGAGATT 1200
GACGTCAATG GCCACCCCGG TGGTACGGAA GGCTACCCTA TCCCTCAAGA TCAATACGGC 1260
AATAATTATG GCGTGACCAA AGGCAAGACA TATATGGGCG GTATCCAGTA CAGCTACGAC 1320
TTGGACAAAT TCCTCCTCAT GCCTTCGCAA CTTTTGTTCG GAGCCGAATA TACGCGTGAT 1380
GAACTCAATG ACGTGATGCC CATCCTTTCA TGGCAGACCG GCGAGGATGC CAATGGGAAT 1440
ACCATTCCCC TCTATCCCGA ATTGGATCAG AATATCAACA ACTACAGCCT ATTCGGTCAG 1500
AACGAATGGA AAAATGACAG ATGGAGCATC CTTGTTGGCG CTCGCTTGGA CAAGCATAGC 1560
GAAGTCAAGG ATATGATTCT GAGTCCTCGT ACCACACTGC GTTTCAACCT GAATCCGGAC 1620
ATCAACCTGC GCGCTACATA TGCAAAAGGG TTCCGCGCAC CGCAGGTATT CGATGAAGAC 1680
TTGCACGTAG GGGTTGTAGG CGGTGAGGCA CAGAAAGTAT TCAACGATCC GAACCTCAAG 1740
CCTGAAATTT CTCATGCATT CAGTTTGAGT GCCGATATGT ATCATCGTTT CGGTAACGTC 1800
CAGACCAACT TCCTTGTGGA AGGCTTCTAT ACTCGTTTGC TGGATGTATT CACCAACGAG 1860
GAGCAGCCTG ATCAGCACGA TGGCATCAAA CGCTACACGC GTATCAACGG TAGCGGAGCC 1920
AAAGTATTCG GTCTCAATCT GGAAGGTAAG GTCGCATACA AGTCCTTCCA GCTCCAAGCC 1980
GGTCTTACCC TGGCCAGCAA CAAATACGAC GAAGCACAGG AGTGGGGTCT GAATACGGTG 2040
AAAGACACCA ACGGAGCTTT TGTTACCGAG GCCAATGCAA ATGGACAACA GGAATACAAG 2100
AACGAATCCA TGACGGATAC GCAGATCACC CGTACCCCCA GCGTATACGG TTATTTTACT 2160
TTGGCCTACA ATCCTGCTCA CTCATGGAAC ATAGCCCTTA CGGGAGCATA TACCGGTCAG 2220
ATGTATGTAC CCCACGCTAT CGAATATGGT GTGAAGTCTG CCGAACTGGA TATTATGCAG 2280
AACAATCCTG AGATTACCGA CGAAACCGGA AAGGCTCCCC GTATTGATGA GCTGAAGAAG 2340
ACACCTGCAT TCTTCGATTT GGGCTTGAAA GTGGGTTATG ACTTCCACGT ATTCCAGGCT 2400
ACTGAGGTTC AACTCTATGT AGGTATGAAC AATATCTTCA ACTCTTTCCA GAAGGACTTC 2460
GATCGTGGAG CTGCACGTGA CAGCGGATAT ATCTATGGTC CTACGCAGCC GCGTACAGGC 2520
TACATGGGCT TGGTAGTGAA GTTC 2544

(2) INFORMATION FOR SEQ ID NO:196

(i) SEQUENCE CHARACTERISTICS:
(A) LENGTH: 606 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...606

(xi) SEQUENCE DESCRIPTION: SEQ ID NO:196

ATGACAGTAA AGCGCGCAGT GCGAATAGCA CTTCTCACGC TGATAGGCAT TCTTTTTTCC 60
TCACCTTCTC TTGTTCGGGC GCAAAGTCTT TTCAGCACCG AACATGTCTT GCAACTATAC 120
AACAAGATAC TCTATGGAGA GTCGGCGGCG GATACCGTCG CAGAGAAAAC GGCAGGTGAG 180
TCGGCATTTC CTTTTATAGA CAAACTCATC AATCTCGGCC GCACTTTCCT CGGCAAACCA 240
TATCGCTATC GCGGTCCTTC CCCATGGCCG ATGGACTGCT CGGGCTATGT GTCTTACCTC 300
TACTCCAAAT TCGACATCAA ACTCCCACGT GGTGCGGCAG CACAGAGCCA ATATACGAAT 360
CCTATCGAGC GCGAGGATGT TCGTCCGGGC GACCTCCTTT TTTTCAAAGG CCGCAATGCA 420
CGCAGCAACC GTATCGGGCA TGTAGCTTTG GTCGTATCTG TCGATGAAGA TGATATTACC 480
ATGATGCACA GCCGCAATTC GCGAGGGATC GTGATCGAAA AACTCAATCG CAGTGCATAC 540
TTCTCCCGTC GCTTGGTGAG CTATGGCAGG GTACCCGGAG CCAAGAGAGT GATCCCACGA 600
AAAAGT 606

(2) INFORMATION FOR SEQ ID NO:197

(i) SEQUENCE CHARACTERISTICS:
(A) LENGTH: 1365 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1365

(xi) SEQUENCE DESCRIPTION: SEQ ID NO:197

ATGAAACGGA CAATCCTCCT GACGGCACTG ACCGTCCTAT CTTCGCTCTC CTTGCTTCGT 60
GCACAAAATG AATCCGAAGC ATCAACCAAT CCGATGTCAG GCCTCTCCCT GGAAGACTGT 120
ATCCGGATAG CCAAGGAGCG CAACCTGAAT CTGCGCAGAC AGGAGATCGA ACAAGAAAAC 180
CGAATCATTA GTCTCGATGC AGCACGACAC AGTTTCCTGC CCTCGGTCAA TGCAGGCATC 240
GGACACAACT ATAGCTTCGG ACGTTCGAAA GACAAAACGG GAGTAACCGT AGATCGCTCC 300
TCGATGAATA CCAATCTCAG CATCGGAGCT TCGGTGGAAG TATTCAGCGG CACACGTCGT 360
CTGCACGACC TCAAGCAGCA AAAGTACAAC GTGGAGGATG GTATAGCCCG ACTTCAAAAA 420
GCGCGTGAAG ACCTCAGCCT GCAAATCGCG GCTCTCTATA TCAATTTGCT CTTCCGTCAG 480
GAAATGACTC GTACGGCAGA AACACAGTTG GCACTGATTC GCGAGCAACG CAATCGCACG 540
GCCGAAATGG TTCGCGTAGG TAAATGGGCA GAGGGTAAGC TCCTCGACAT AAATGCCCAG 600
ATGGCCAAGG ACGAACAACT TCTCGTACAA TATCGTTCGG AGGAGGAGCT GGCTCGTCTG 660
GACTTGGGGC AAGCCCTCGA ACTGGAGCAC CCCGAAAGCA TTGCAGTCAA GGCTCCCGAC 720
ACAGACGTTC TCGTAGCAGA AAGGTTGGGA TCTCTCCTTG CTCCCGAAGA GATCTATCGC 780
ACGGCTCTCG GCTTGAAACC GGCACTGCAT TCGAGCGAGC TGCAAATAGC TTCGGCACGC 840
GAAGGTCTGG CCTCGGCTCG TGCGGCATAC TTCCCGACGC TCAGCCTCTC TGCCGGATAC 900
AGCAACGGTT ACTTCCGCGA CCTCGGCAAG GAGTATGCCG CCATCAACCC CTCCTTCTCC 960
GAACAGTGGA AGAACAACGG CAGCTACAGT ATCGGACTCT CTTTGAATAT CCCCATCTTC 1020
TCTGCCATGC AAACGCAAGA TCGCGTTCGG AGCAGTCGCC TGCAAATACG CTCAAGCGAG 1080
CTTCGACTCC TCGAAGAGAA AAAAGCCCTC TATAAAGAGA TCAGGCAAGC ATACAGCAAT 1140
GCCGTGGCAG CCGATAAGGC CATCGCAGCA GCCGAAAACA GCAAGGCCGC TACGCTCAAG 1200
GCATACGAAT ACGCTCGCGA CAGCTTCGAG GCAGGGCGCT TGTCTGCCTA CGAATATGCC 1260
GAGGCAAAAA CAAAATACGC CCTCAGCCAA GTGGAAGAAC TTCGTGCCAA GTATGACTTC 1320
ATATACAAAG CCAAAGTTTT GGATTTCTAT CAGGGCAAAG ACTTC 1365

(2) INFORMATION FOR SEQ ID NO:198

(i) SEQUENCE CHARACTERISTICS:
(A) LENGTH: 1332 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1332

(xi) SEQUENCE DESCRIPTION: SEQ ID NO:198

ATGCGTTTCC AACATTATCT CATCTGTACG GCTGCCGTAG CGGCTTTGGC TGCGAATCCC 60
CTTACGGGCC AATCGAATAT GACCCTCGAA GAGTGCATAG ACTATGCACG CCGGCACAGT 120
TCGGCCGTGG CGCTGTCCGC TGCGGAACTG GAGCAGTCCA AGGCCGATTA CCTTCAGGCC 180
GTCGGCAATT TTCTGCCCCG TGTATCGGCC GGAACCGGTG CTTCGTGGAA TTTCGGACGC 240
GGATTGGATG CCGAGACGAA TACCTACACC GACATCAACA GCTTCAACAA TTCGTACAGC 300

ATACATGCCA CGATGACCCT TTTCGACGGT TTGCAGAGTG TCTATCGACT GCGGATGGCG 360
CATGCACGCC GGGAGGCTTC GCGCCTCTCC GTTCGCGAGC AGCAGGAGCT GGCAGCTCTC 420
GGCACCACGG AGGCCTACTA CGACCTCGTC TATGCGCGCC AAATGCAAGA GCTGGCCATG 480
CAGAAGTACG AGGAGAGCAG CCGCCTCCAC CGGCAGACGG CTCGAATGGA AGAGCTGGGG 540
ATGAAGAGTC GTCCCGATGT CCTCGAGATG CAGTCGCGAA TGGCCGGTGA CCGTTTGGCC 600
CTGACTCAAG CGGACAATCA GTGCATCATC GCTCTGATCC GGCTCAAAGA AAAAATGAAC 660
TTCCCCATCG ATGACGAACT CGTCGTAGAC GATATGCCGG CTGACAGTCT CTCCGCCGAC 720
ATGGCCGAAT CGGACAGCTC GGCCGGCGTC TTCGCCCGTG CTGCCCATCA TCATCCCGTC 780
CTCCTCCGTG CCAAACTCGA CGAGCAGGCT GCCACCGACC GTTTGCGAGC CGCGCGAGGT 840
GCATTCCTGC CGAGTGTGTC GGTATCCGGA GGATGGAACA CGGGATTCTC ACGCTTTTTG 900
AATGGATCGG ACTATACGCC CTTCAGCGAG CAGTTTCGGA ACCGTCGGGG GGAATACGTC 960
ACTCTGAATC TGAGTATCCC CATCTTTTCG GGATTCAGCC TTGTGAGCCA TCTGCGTCAG 1020
GCGCGTGCCG AACGCAGGGC GGCAATCGTC CGACGGGGCG AAGCGGAGCG CAGGCTCTAC 1080
AGCGAGATCG CCCAAGCCAT GGCCGACCGG GATGCCGCTC TGGCTTCCTA CCGCCAGGCG 1140
AAGGAGCATA CCGACGCCAT GCAAACCGCT TACGAAGCCG TCTTGCAGCG TTATGAGGAG 1200
GGCCTGAATA CGGCCATCGA CCTGACCACT CAGGCCAATC GGCTCCTGGA TGCCCGTGTG 1260
CAGCGACTGA GAGCGGCCAT GACCTACCGG CTCAAATGCA AACTCATAGC CTATTACGGC 1320
TGCCTTTCGG AC 1332

(2) INFORMATION FOR SEQ ID NO:199

(i) SEQUENCE CHARACTERISTICS:
(A) LENGTH: 2820 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2820

(xi) SEQUENCE DESCRIPTION: SEQ ID NO:199
   ATGAACAAAT TTTACAAATC ACTTTTGCAG TCAGGACTGG CTGCCTTCGT GTCGATGGCA 60
   ACTGCACTGA CCGCTTCTGC ACAGATTTCG TTCGGAGGGG AACCCTTGAG TTTCTCTTCA 120
   AGATCCGCCG GAACGCATTC ATTCGACGAT GCAATGACTA TCCGCCTTAC TCCGGATTTC 180
   AATCCGGAAG ACCTGATCGC ACAGAGCCGT TGGCAATCGC AAAGAGATGG CCGGCCCGTC 240
   CGGATAGGAC AAGTAATACC GGTGGATGTG GACTTTGCAT CCAAGGCTTC GCACATCTCT 300
   TCCATCGGAG ACGTAGATGT ATATCGCCTG CAATTCAAGT TGGAAGGAGC CAAAGCCATT 360
   ACGCTTTATT ACGATGCATT CAATATTCCG GAGGGCGGAC GCCTCTATAT CTATACCCCC 420
   GACCATGAAA TTGTGTTGGG AGCATATACG AACGCCACTC ATCGCCGCAA CGGAGCTTTT 480
   GCCACAGAGC CGGTACCGGG GAGTGAGCTT ATTATGGATT ATGAAGTGTC TCGCGGAGGG 540
   ACTTTGCCTG ACATCAAGAT CTCCGGTGCG GGTTATATAT TCGACAAAGT CGGCGGACGC 600
   CCCGTAACGG ATAACCATTA CGGGATCGGT GAGGACGATT CCGATTCGGA TTGCGAGATC 660
   AACATCAATT GTCCTGAAGG TGCAGACTGG CAGGCAGAGA AGAACGGTGT GGTGCAAATG 720
   ATCATGGTAA AAGGACAGTA TATCTCAATG TGCTCAGGCA ACCTGCTCAA TAATACGAAA 780
   GGAGACTTTA CTCCGCTGAT CATTTCTGCC GGACACTGTG CTTCCATAAC AACCAATTTC 840
   GGTGTAACGC AATCCGAGTT GGATAAGTGG ATCTTCACTT TCCACTATGA AAAAAGAGGA 900
   TGCAGCAATG GTACATTGGC CATCTTCCGT GGCAACAGTA TCATCGGAGC TTCCATGAAG 960
   GCTTTCCTCC CGATCAAAGG TAAATCCGAT GGTCTCTTGC TGCAACTCAA CGATGAAGTC 1020
   CCTCTGCGCT ATCGTGTCTA TTACAATGGA TGGGACAGTA CGCCCGATAT TCCCTCGAGC 1080
   GGTGCCGGTA TTCATCATCC GGCCGGAGAT GCCATGAAGA TTTCCATCCT AAAGAAGACT 1140
   CCGGCTCTGA ATACATGGAT CTCCTCCAGT GGTTCCGGAG GGACTGACGA TCACTTCTAT 1200
   TTCAAATACG ATCAAGGTGG TACGGAAGGA GGATCGTCCG GTTCTTCTCT CTTCAATCAG 1260
   AATAAGCACG TGGTCGGCAC ACTGACCGGA GGTGCCGGCA ATTGTGGCGG GACGGAGTTC 1320
   TACGGCAGAC TGAACAGTCA TTGGAACGAG TATGCATCCG ATGGCAATAC GAGCCGCATG 1380
   GACATCTATC TGGATCCCCA AAACAATGGC CAGACGACCA TCCTCAACGG AACGTATCGT 1440
   GACGGTTATA AGCCTTTGCC CTCTGTGCCC CGGCTATTGT TGCAGTCTAC AGGCGATCAG 1500
   GTCGAATTGA ATTGGACGGC TGTTCCTGCC GATCAATATC CATCATCTTA TCAGGTCGAA 1560
   TACCACATAT TCCGAAATGG AAAGGAAATA GCTACGACAA AGGAGTTGTC CTATTCGGAT 1620
   GCCATCGACG AAAGTATTAT CGGTAGCGGT ATCATTCGAT ACGAAGTAAG CGCACGCTTC 1680
   ATTTATCCCT CGCCGTTGGA TGGAGTGGAA TCTTATAAGG ATACGGACAA GACTTCTGCC 1740
   GACCTTGCCA TAGGAGACAT TCAGACCAAG CTGAAGCCGG ACGTAACACC TCTCCCCGGA 1800
   GGAGGAGTAT CATTAAGCTG GAAAGTTCCT TTCTTAAGCC AGTTGGTTTC CCGATTCGGA 1860
   GAAAGCCCCA ATCCTGTGTT CAAAACCTTT GAAGTGCCCT ATGTTTCTGC CGCAGCCGCA 1920
   CAAACCCCCA ATCCTCCCGT TGGCGTAGTC ATTGCAGACA AGTTTATGGC CGGTACATAT 1980
   CCCGAAAAGG CTGCTATCGC TGCCGTTTAT GTAATGCCAT CCGCTCCGGA CTCTACTTTC 2040
   CACCTCTTCC TCAAGAGCAA CACAAACAGA AGATTGCAGA AGGTGACAAC TCCCTCCGAT 2100
   TGGCAGGCCG GAACATGGTT GAGGATCAAT TTGGATAAGC CGTTCCCGGT GAATAATGAC 2160
   CATATGCTTT TTGCCGGTAT CAGAATGCCT AATAAGTACA AGCTCAATCG TGCTATCCGT 2220
   TATGTAAGAA ATCCGGATAA CCTTTTCTCC ATTACCGGTA AGAAGATTTC ATATAACAAC 2280
   GGAGTCTCTT TCGAAGGCTA CGGAATACCC TCGCTCTTGG GCTATATGGC TATCAAATAT 2340
   CTGCTGGTAA ATACCGATGC TCCGAAGATC GATATGTCGC TTGTACAGGA GCCTTATCCT 2400
   AAGGGAACGA ATGTGGCTCC ATTCCCCGAA TTGGTCGGCA TATATGTCTA TAAGAACGGA 2460
   ACATTTATCG GCACACAGGA TCCATCCGTC ACAACTTATT CGGTTTCAGA CGGAACAGAG 2520
   AGCGATGAAT ACGAAATAAA ACTGGTATAT AAGGGATCGG GCATTTCGAA TGGCGTTGCT 2580
   CAGATTGAGA ATAACAATGC TGTCGTTGCA TATCCGTCTG TTGTAACAGA TCGTTTCAGC 2640
   ATTAAGAACG CTCATATGGT TCACGCTGCC GCCCTCTACT CATTGGATGG CAAGCAGGTT 2700
   CGTTCTTGGA ACAACCTCCG CAATGGCGTG ACATTCAGTG TTCAAGGACT TACGGCCGGT 2760
   ACTTATATGC TCGTTATGCA GACGGCAAAC GGCCCTGTGA GCCAAAAGAT CGTGAAGCAG 2820

(2) INFORMATION FOR SEQ ID NO:200

(i) SEQUENCE CHARACTERISTICS:
(A) LENGTH: 2010 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2010

(xi) SEQUENCE DESCRIPTION: SEQ ID NO:200

ATGAAATATC TTATCAGACT CTTCTTATCA TTGATGTTAC TCTCTCTCTG GACGGGCTGT 60
ACACACGAGG AGCTCTCTAT TTGCGATGGC GAGAATACGC TTGTTTTACG CGTAGAGACC 120
GGTAAAGCCC CAAATGCTCG TGCCACAGAA CCCGGTCAGG GCATATACAA TGAGAATAAA 180
GTAGGCTCCA TTTCTGTGCT CTTCTATTTA GAGGGACAAC TTCGTTGGCA GGTGAAGTCT 240
ACAGACTATC AAATCCATGA AGGGGCCTAT ATCATTCCGG TCAAAGAGCA AATGCGACCA 300
CTATTCAATG GCAACAACAA CTTCAGCATC TATGTAGTGG CCAATCTCGA TTTCAATGCT 360
CCGGCCACAG AAGCTGCGCT TTCTCAATTT GTGGTAGAGA AATCTATTGA AGTCTCTTCT 420
ACGACAGCCC CTGCCGATTT CGTAATGCTT GCTCATGGCA ATAAGCAGAT CAATATGGCT 480
ACGACAGAAG GGAAACTGTT GGGGGATTAT AAACTCAAAC GAGTGGCAGC AAAGATTCGC 540
ATGATAAAAC CCACCATCAA TGTGCAAGGA TATGAAGTGG TCGGAAATAT ACAGGCAAAG 600
TTTCGCAATT CGGTAACGAA GGGGTTCCTT ACCACAGAAC CTCAAGAGAT CCCAGCTGCT 660
GCATCCTATA AGACATCGGA ATATCTTGAT ATTGCAGAGT CGGCACCTGC CAATTCTATC 720
CATTTCTATT CTTACTATAA CAAATGGACA CTCTCCACAC CGGAGAAGCG ACCGGAATTC 780
TTCATCATGG TCAAATTCAA AAAGACAGGA CAGCCGGACA ACACAGCCAA ACCGTACTAC 840
TACAGAGTGC CCCTCGAATC TCAGGACAAT CAGGTCAAGA GCAATGTCCT CTATAATCTG 900
AATGTGAAAA TCGAAATCTT GGGTTCTTTA CAAGAGCCGG AAGCTGTTTC TGTAAACGGC 960
ACACTCGCAA TAGAAGAATG GATTCTCCAT CAGGATGCAT TCAATCTGCC TGCCACCAAT 1020
TACTTGATAG TGGAACAGCA CGAAATCTTC ATGAATAACG TGAACACATA CTCGGTGAAA 1080
TATCAAACTT CGCAGAAACC AATCAGCATT AGCATACAGT CAGTTACCTT TAGCTACGTC 1140
TCTTCTGATG GCACTCAGCA CAATGATCTT GTAGCAAGTA GTAGCGACCA GTATCCTACG 1200
ATTACAAGCG ATAATACAAG CATCATAATC ACTTCCAAGA TACCGGTTAA TAACGTACCA 1260
AAGAAGATCG TTTTTGAGGT AACTAATGGG GTAGCCGGTT TGAAAGAGAC TGTCACAGTA 1320
CTCCAATATC CTGCACAATT TATTGTCAAT ACACTTGGCA CAGCATCGGC ATGGAGACCA 1380
GACGGATCTT TGGCTCCGGG GCTTAACAAT AAAGCGATTT ACCATGTCGT AGTACTGGTT 1440
CCACCCGAGA ATTTATTTGA AGATGGGACA CAGACAATCA TCGGTTATCC CCCCACTGAA 1500
ACAATTTCTT TTCATAAGAA AGAGAACAAT ACCTATCCGA TAGTATGGTC TGACACAAAT 1560
ACGACAAAAC AGGACCTTGA GACATCAAGA ATGATTTCAC CTTCCTTTGA GTTAGCCTCC 1620
CAACTTGGGG CTACTCTCCC GATGCCCTAT CTCGAGTATT GGCCAGGGAC ATCATATCTC 1680
CTTGACTATT CGGGAAACTA TAATAATAAG AGATACGCCT TGTTTAATTG CGCTTTTTAC 1740
TGGGAGAAAA GAAAAGTTAA TAACGAAGAA ATTAAATTCG ATGACTGGCG TTTCCCGACA 1800
GAAGCTGAGA TCAAATTGAT AGATAAGCTG CAACATAATG AGCAGAGTGC TGTCCAAGCT 1860
ATCATGACAG GGAATTATTA TTGGGATAGT TACTCTGCAA ATGGGTCTTA TAAAATGCAA 1920
GGAGGAGGGG GCCAAGGAAA TTCCTCCAAA GCCTATGTTC GTTGCGTGCG GGATGTGAAA 1980
AAGCCGATTC GTGACAAGAA GTCAGGTAAG 2010

(2) INFORMATION FOR SEQ ID NO:201

(i) SEQUENCE CHARACTERISTICS:
(A) LENGTH: 3846 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...3846

(xi) SEQUENCE DESCRIPTION: SEQ ID NO:201

ATGCGAAAAA TTTTGAGCTT TTTGATGATG TGCTCTCTGC ATTTAGGTCT ACAATCTCAG 60
ACTTGGCATG GAGATCCGGA CTCAGTGGCA GCCCTACCTT CTATCGGTAT TCAAGAGTCA 120
AGTTGTACCC GAATCACGTT CGAGGTTGTT TTCCCCGGAT TTTATAGTGT GGAAAAACGA 180
GAAGGCAACC AAGTCTTTCA GCGCATTTCC ATGCCGGGTT GTGGCTCGTT TGGGAATCTG 240
GGCGAAGCTG AATTGCCTGT TTTGAAAAAG ATGATAGCCG TTCCGGAATT TTCAACAGCT 300
AACGTTGCTG TAAAAATCAA AGAGACGGAG ACATTCGACA ATTATAATAT CTATCCTAAT 360
CCTACCTATG TCGTAGAGGA GTTGCCTGAG GGGGGGACTT ATCTGGTAGA GGCTTTCGCG 420
ATAAACAATG ACTATTATAG CCAAAATGTA AGCCTCCCTT CTACTCACTA TGTCTATTCT 480
CAAGACGGGT ATTTTCGCTC ACAAAGATTT ATCGAAGTTA CCCTGTATCC TTTTCGATAC 540
AACCCTGTCC GACAAGAAAT TCTATTTGCA AAAAAAATCG AGGTTACAAT AACTTTCGAT 600
AATCCTCAGC CACCTTTACA AAAAAACACC GGCATATTTA ACAAAGTAGC CTCCTCTGCA 660
TTTATTAATT ATGAAGCTGA TGGCAAATCG GCGATAGAAA ATGATATGGT GTTCAGTCGT 720
GGTACAACAA CGTACATAAG CGGAAATGTT GCCAGCAACC TCCCTCAGAA CTGTGACTAC 780
TTGGTTATTT ACGATGATAT GTTCAACGTA AATCAACAAC CACACGACGA AATCAAACGG 840
CTGTGCGAAC ATAGAGCCTT CTACAACGGC TTTGATGTAG CTGCTGTAAG TATAAAGGAC 900
GTATTGAATA GCTTCCCATC AAATGCCACC TCATACATCA ACGAAACTAA ACTGAAAAAT 960
TTCATTCGCT CAGTTTACAA CCAAAGCAAT GCGAAGAGGA CTTTAGATGG CAAACTGGGA 1020
TACGTGCTAC TGATCGGAAA ACCATTGAGC AAATATTTGG CTGACACTGA TAATACAAAA 1080
GTCCCAACCT CTTTTATTCA TAATGTCTCC TTAATTCCAA GTCATCCAAC TTTTGGTTCC 1140
ATATGCGCCT CCGACTATTT TTTTAGTTGT GTTTCGCCCC TTGATACTGT CGGCGATTTG 1200
TTTATCGGTC GATTTAGCGT CACCAATGCT CATGAATTGC ACAATCTGAT TGAAAAGACT 1260
ATCAACAAAG AAATCTCATA TAATCCTATT GCACACAAAA ATATTCTTTA CGCAGAAGGG 1320
AAAGGCTGCG ATGCTCCAAT CTTACGTTTA TTCTTAAAAG AAATCGCCTC TGGTTACACA 1380
GTCAACTCTA TCTTAAAATC TAATCAGGTC TCTGCAATAG ACTCGATATT TGACTGCTTG 1440
AATAATGGTT CCCATCATTT TTATTTTAAC ACTCATGGAA TGCCGACTGT TTGGGGGATA 1500
GGGCAGGGAC TCGACGTCAA TACTCTAACA GCCCGATTGA ACAATACATC TTCGCAGGGA 1560
TTATGTACGA GTCTATCATG TAGTTCGGCT GTAGCAGATT CAACTATTAG ATCGCTTGGA 1620
GAAGTCCTGA CCACATACGC ACCTAACAAG GGATTCTCGG CTTTCTTAGG AGGAAGCAGA 1680
GCCACCCAAT ATGCCGTTTA TTTAGAAGGC CCCTGTCCTC CGTCAGAATT TTATGAATAT 1740
TTACCTTATT CTTTATATCA CAATCTCTCG ACTGTTGTTG GCGAAATGTT GCTATCATCC 1800
ATTATCAATA CTAATTCTGT TGATACGTAT TCGAAATTCA ACTTCAATTT GCTTGGCGAC 1860
CCTGCACTAA ACATTATGGC TCATGGCATG GAGGTTAGTA ATTGTATTAC ACTACCAAAC 1920
AACACCATTA TAAGCAGTCC GATAACAATA AAAAATGGTG GCTGCCTAAA AATACCGGAA 1980
AAAGGAGTTT TGCATTTTAC TAATAATGGC TCCATACAAG TCATGTCCGG AGGAACTCTG 2040
GAAATAGGCA ATCAGGCTAA AATATCCGGA GAGACCGGTG CTAACCCCAC CTTTATTACC 2100
GTTTACGGCG ATGGTCTTGC GATTAACAAG CAGGTAGAGA TAGACAATAT AGACCGACTT 2160
AACTTGTTTT CTACGCATTC GGTCATGCCC AAATTTCATT TTGACAGTGT GAAATTCAAC 2220
AGTGCCCCGC TGTATACAAC GAACTGTATT GTGGAGATAA GCAATTGCGA ATTTACCAAT 2280
CGAAGTGACA TTATTTCAAA GAATTGTGAC CTAAGCGTTG AAAACAGTAT GTTTAGCAGT 2340
TCGGGGATAA CGGTATTCAA GCCTATGGCT ACAAGCTCCA TCACCGGATT ATCTACAAAA 2400
GCAAAGATTA CCGACAATAC TTTTTTTGCG ACAGGAAACT TCGCCTACCA TATCACAAAC 2460
ACGCCAGGCT TAACAGCAAC CTCCAATGCT GCCATCAAGT TAGACAATAT TCCTGAGTAT 2520
TACATTTCCG GTAATAAAAT AGTCAATTGC GATGAGGCTC TTGTACTAAA TAATAGTGGC 2580
AACAGAACGA ACAGACTCCA CAATATCACA CGGAATGTGA TAAAAAACTG TAGGATTGGG 2640
AGCACGCTTT ATAATTCCTA TGGTATTTAC AACCGAAATA AGATCAGTAA CAATCATATA 2700
GGAGTACGTC TCCTCAACAA CAGTTGTTTT TATTTCGATA ATGCTCCTGT AATCAATGAA 2760
GAAGATAAGC AGACGTTTAT TTCTAATAGG ACTTGGCAGC TCTATTCATC AAACGGTACA 2820
TTCCCTCTCA ACTTCCATTA CAACAGCTTG CAGGGGGGAG ATACAGATAC ATGGATTTAC 2880
AACGACACGT ATACGAATCG CTATATTGAC GTTTCAAATA ATCACTGGGG CAACAATGAT 2940
TTGTTTGATC CGAATCAGGT TTTCAATACG CCAGACTTGT TCATTTGGAT ACCTTTTTGG 3000
GATGGATTGC CAAATGGGAG ATCGGGCAAT AGCTCTGCTG AAGCAGTAGA ATTCCAAACA 3060

GCATTGGACT GTATTGGCAA TAGCGATTAT CTTTCGGCAA AAGTGGCTCT CAAGATGATG 3120
GTTGAAACCT ACCCGGAATC CGACTTTGCA ATAGCTGCTT TGAAGGAATT GTTCAGGATA 3180
GAGAAAATGT CAGGCAACGA TTACGAAGGC TTGAAAGATT ATTTCAGATC CAATCCAACC 3240
ATCATCTCTT CCCAGAACTT GTTCCCGACA GCTGATTTCC TGTCTGCGCG ATGCGATATT 3300
GTGTGTGAAA ACTATCAGTC TGCCATCGAT TGGTACGAAA ATCGCTTGAA TAGTGAAATC 3360
TCCTATCAGG ACAGTGTTTT TGCAGTCATT GACCTTGGTG ACATTTATTG GAATATGCAG 3420
TTAGACTCAC TCAGAGGGAC TGGTATAGAT TTGAACATAC TTTCCTGTGA ACAAAGGAAA 3480
TCGCTCGAAA GCCATCAAAA TGTAAAAAAT TATTTGTTGT CAACTCTTCC CGAATCAACA 3540
GGTACTCTCC TGCCTCCATT AGAATGCAAC AAATCAAGCC TTGATAAATC CAAGATAATC 3600
TCTATTTCGC CCAATCCGGC GAAAGCTGTT GTAACAATAA TCTACTATAC CGATAACCCT 3660
TCCTGTTCTG TAATAAAAAT ATATGGAATA AATGGAGCCT CGGCTGATAT AACCGGGTTG 3720
CCCAAACATC TATCCGAAGG TTATTACAGC ATACAGTTCA ATACATCCAA CTTTGATCCC 3780
GGTTTCTACC TGGTAACGCT AAATGTTGAT CAGAAAATTA TAGATACGGA AAAATTACGA 3840
ATCAAA 3846

(2) INFORMATION FOR SEQ ID NO:202

(i) SEQUENCE CHARACTERISTICS:
(A) LENGTH: 3822 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...3822

(xi) SEQUENCE DESCRIPTION: SEQ ID NO:202

ATGATGTGCT CTCTGCATTT AGGTCTACAA TCTCAGACTT GGCATGGAGA TCCGGACTCA 60
GTGGCAGCCC TACCTTCTAT CGGTATTCAA GAGTCAAGTT GTACCCGAAT CACGTTCGAG 120
GTTGTTTTCC CCGGATTTTA TAGTGTGGAA AAACGAGAAG GCAACCAAGT CTTTCAGCGC 180
ATTTCCATGC CGGGTTGTGG CTCGTTTGGG AATCTGGGCG AAGCTGAATT GCCTGTTTTG 240
AAAAAGATGA TAGCCGTTCC GGAATTTTCA ACAGCTAACG TTGCTGTAAA AATCAAAGAG 300
ACGGAGACAT TCGACAATTA TAATATCTAT CCTAATCCTA CCTATGTCGT AGAGGAGTTG 360
CCTGAGGGGG GGACTTATCT GGTAGAGGCT TTCGCGATAA ACAATGACTA TTATAGCCAA 420
AATGTAAGCC TCCCTTCTAC TCACTATGTC TATTCTCAAG ACGGGTATTT TCGCTCACAA 480
AGATTTATCG AAGTTACCCT GTATCCTTTT CGATACAACC CTGTCCGACA AGAAATTCTA 540
TTTGCAAAAA AAATCGAGGT TACAATAACT TTCGATAATC CTCAGCCACC TTTACAAAAA 600
AACACCGGCA TATTTAACAA AGTAGCCTCC TCTGCATTTA TTAATTATGA AGCTGATGGC 660
AAATCGGCGA TAGAAAATGA TATGGTGTTC AGTCGTGGTA CAACAACGTA CATAAGCGGA 720
AATGTTGCCA GCAACCTCCC TCAGAACTGT GACTACTTGG TTATTTACGA TGATATGTTC 780
AACGTAAATC AACAACCACA CGACGAAATC AAACGGCTGT GCGAACATAG AGCCTTCTAC 840
AACGGCTTTG ATGTAGCTGC TGTAAGTATA AAGGACCTAT TGAATAGCTT CCCATCAAAT 900
GCCACCTCAT ACATCAACGA AACTAAACTG AAAAATTTCA TTCGCTCAGT TTACAACCAA 960
AGCAATGCGA AGAGGACTTT AGATGGCAAA CTGGGATACG TGCTACTGAT CGGAAAACCA 1020
TTGAGCAAAT ATTTGGCTGA CACTGATAAT ACAAAAGTCC CAACCTCTTT TATTCATAAT 1080
GTCTCCTTAA TTCCAAGTCA TCCAACTTTT GGTTCCATAT GCGCCTCCGA CTATTTTTTT 1140
AGTTGTGTTT CGCCCCTTGA TACTGTCGGC GATTTGTTTA TCGGTCGATT TAGCGTCACC 1200
AATGCTCATG AATTGCACAA TCTGATTGAA AAGACTATCA ACAAAGAAAT CTCATATAAT 1260
CCTATTGCAC ACAAAAATAT TCTTTACGCA GAAGGGAAAG GCTGCGATGC TCCAATCTTA 1320
CGTTTATTCT TAAAAGAAAT CGCCTCTGGT TACACAGTCA ACTCTATCTT AAAATCTAAT 1380
CAGGTCTCTG CAATAGACTC GATATTTGAC TGCTTGAATA ATGCTTCCCA TCATTTTTAT 1440
TTTAACACTC ATGGAATGCC GACTGTTTGG GGGATAGGGC AGGGACTCGA CGTCAATACT 1500
CTAACAGCCC GATTGAACAA TACATCTTCG CAGGGATTAT GTACGAGTCT ATCATGTAGT 1560
TCGGCTGTAG CAGATTCAAC TATTAGATCG CTTGGAGAAG TCCTGACCAC ATACGCACCT 1620
AACAAGGGAT TCTCGGCTTT CTTAGGAGGA AGCAGAGCCA CCCAATATGC CGTTTATTTA 1680
GAAGGCCCCT GTCCTCCGTC AGAATTTTAT GAATATTTAC CTTATTCTTT ATATCACAAT 1740
CTCTCGACTG TTGTTGGCGA AATGTTGCTA TCATCCATTA TCAATACTAA TTCTGTTGAT 1800
ACGTATTCGA AATTCAACTT CAATTTGCTT GGCGACCCTG CACTAAACAT TATGGCTCAT 1860
GGCATGGAGG TTAGTAATTG TATTACACTA CCAAACAACA CCATTATAAG CAGTCCGATA 1920
ACAATAAAAA ATGGTGGCTG CCTAAAAATA CCGGAAAAAG GAGTTTTGCA TTTTACTAAT 1980
AATGGCTCCA TACAAGTCAT GTCCGGAGGA ACTCTGGAAA TAGGCAATCA GGCTAAAATA 2040
TCCGGAGAGA CCGGTGCTAA CCCCACCTTT ATTACCGTTT ACGGCGATGG TCTTGCGATT 2100
AACAAGCAGG TAGAGATAGA CAATATAGAC CGACTTAACT TGTTTTCTAC GCATTCGGTC 2160

ATGCCCAAAT TTCATTTTGA CAGTGTGAAA TTCAACAGTG CCCCGCTGTA TACAACGAAC 2220
TGTATTGTGG AGATAAGCAA TTGCGAATTT ACCAATCGAA GTGACATTAT TTCAAAGAAT 2280
TGTGACCTAA GCGTTGAAAA CAGTATGTTT AGCAGTTCGG GGATAACGGT ATTCAAGCCT 2340
ATGGCTACAA GCTCCATCAC CGGATTATCT ACAAAAGCAA AGATTACCGA CAATACTTTT 2400
TTTGCGACAG GAAACTTCGC CTACCATATC ACAAACACGC CAGGCTTAAC AGCAACCTCC 2460
AATGCTGCCA TCAAGTTAGA CAATATTCCT GAGTATTACA TTTCCGGTAA TAAAATAGTC 2520
AATTGCGATG AGGCTCTTGT ACTAAATAAT AGTGGCAACA GAACGAACAG ACTCCACAAT 2580
ATCACACGGA ATGTGATAAA AAACTGTAGG ATTGGGAGCA CGCTTTATAA TTCCTATGGT 2640
ATTTACAACC GAAATAAGAT CAGTAACAAT CATATAGGAG TACGTCTCCT CAACAACAGT 2700
TGTTTTTATT TCGATAATGC TCCTGTAATC AATGAAGAAG ATAAGCAGAC GTTTATTTCT 2760
AATAGGACTT GGCAGCTCTA TTCATCAAAC GGTACATTCC CTCTCAACTT CCATTACAAC 2820
AGCTTGCAGG GGGGAGATAC AGATACATGG ATTTACAACG ACACGTATAC GAATCGGTAT 2880
ATTGACGTTT CAAATAATCA CTGGGGCAAC AATGATTTGT TTGATCCGAA TCAGGTTTTC 2940
AATACGCCAG ACTTGTTCAT TTGGATACCT TTTTGGGATG GATTGCCAAA TGGGAGATCG 3000
GGCAATAGCT CTGCTGAAGC AGTAGAATTC CAAACAGCAT TGGACTGTAT TGGCAATAGC 3060
GATTATCTTT CGGCAAAAGT GGCTCTCAAG ATGATGGTTG AAACCTACCC GGAATCCGAC 3120
TTTGCAATAG CTGCTTTGAA GGAATTGTTC AGGATAGAGA AAATGTCAGG CAACGATTAC 3180
GAAGGCTTGA AAGATTATTT CAGATCCAAT CCAACCATCA TCTCTTCCCA GAACTTGTTC 3240
CCGACAGCTG ATTTCCTGTC TGCGCGATGC GATATTGTGT GTGAAAACTA TCAGTCTGCC 3300
ATCGATTGGT ACGAAAATCG CTTGAATAGT GAAATCTCCT ATCAGGACAG TGTTTTTGCA 3360
GTCATTGACC TTGGTGACAT TTATTGGAAT ATGCAGTTAG ACTCACTCAG AGGGACTGGT 3420
ATAGATTTGA ACATACTTTC CTGTGAACAA AGGAAATCGC TCGAAAGCCA TCAAAATGTA 3480
AAAAATTATT TGTTGTCAAC TCTTCCCGAA TCAACAGGTA CTCTCCTGCC TCCATTAGAA 3540
TGCAACAAAT CAAGCCTTGA TAAATCCAAG ATAATCTCTA TTTCGCCCAA TCCGGCGAAA 3600
GCTGTTGTAA CAATAATCTA CTATACCGAT AACCCTTCCT GTTCTGTAAT AAAAATATAT 3660
GGAATAAATG GAGCCTCGGC TGATATAACC GGGTTGCCCA AACATCTATC CGAAGGTTAT 3720
TACAGCATAC AGTTCAATAC ATCCAACTTT GATCCCGGTT TCTACCTGGT AACGCTAAAT 3780
GTTGATCAGA AAATTATAGA TACGGAAAAA TTACGAATCA AA 3822

(2) INFORMATION FOR SEQ ID NO:203

(i) SEQUENCE CHARACTERISTICS:
(A) LENGTH: 2775 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2775

(xi) SEQUENCE DESCRIPTION: SEQ ID NO:203

ATGGCTATCA TGATGAAAAG TATTGTTTTT AGAGCATTTC TAACGATTTT GCTCTCGTGG 60
GCAGCGATCA CGAATCCGAC TGCTCAAGAG ATCTCAGGCA TGAATGCATC CTGTCTGGCT 120
GCTCCGGCTC AACCGGATAC TATCTTATAT GAAAGTTTTG AGAATGGACC TGTTCCCAAT 180
GGCTGGCTTG AGATAGATGC TGATGCTGAT GGTGCCACTT GGGGAAGCCC ATCAGGCTCT 240
TTCTCTGTAC CTTACGGACA CAATGGCCTT TGCACCTACT CCCATATACG TTCCGGTATC 300
TCAACAGCGG GCAACTATCT GATTACACCC AATATAGAAG GAGCCAAACG GGTCAAGTAC 360
TGGGTATGCA ATCAGTATAG TACCAATCCG GAACATTACG CAGTAATGGT ATCGACAACC 420
GGGACTGCCA TTGAAGACTT TGTTTTGTTG TTTGATGATT CCATAACAGG GAAACCGACT 480
CCTCTTGTAT GGCGTAGACG AATCGTGGAC TTACCGGAAG GGACCAAATA TATTGCATGG 540
CGACATTACA AAGTCACCGA CTCACACACA GAATTCTTGA AATTGGATGA TGTCACTGTG 600
TATAGGTCGA TCGAAGGGCC CGAACCTGCT ACCGACTTCA CAGTAATCAA TATTGGTCAG 660
AATGTGGGAC GATTGACTTG GAACTATCCG GAGGATTATC AACCGGAAGG AAAGGGGAAT 720
GAAGAGTTGC AGCTTAGCGG CTACAACATC TATGCGAACG GTACACTACT GGCACAAATA 780
AAAGATGTCT CCATACTGGA GTATGTGGAC AGCACTTACT CTTTGCGAGA CAATCCCTTG 840
CAAGTGGAGT ACTGCGTTAC AGCCGTTTAC GATGAAAGCA TAGAATCTTC GACCGTATGT 900
GGCACGCTGC ATTACGCCAC GGATGCCATC CTTTATGAAA ATTTTGAGAA TGGACCTGTT 960
CCCAATGGTT GGCTTGTGAT AGACGCTGAT GGAGATGGAT TTAGCTGGGG ACACTATTTG 1020
AATGCATACG ACGCTTTTCC CGGCCATAAT GGAGGCCATT GCTCCTTGTC GGCTTCTTAT 1080
GTTCCGGGTA TAGGCCCGGT GACTCCCGAC AACTATCTGA TTACCCCCAA GGTTGAAGGA 1140
GCCAAACGTG TCAAGTACTG GGTAAGCACG CAGGATGCCA ATTGGGCAGC GGAACATTAC 1200
GCGGTGATGG CTTCGACAAC GGGGACTGCT GTCGGAGATT TCGTCATATT GTTCGAAGAA 1260
ACCATGACAG CGAAGCCGAC CGGCGCATGG TATGAAAGAA CCATCAACTT ACCTGAAGGG 1320

ACTAAATACA TCGCATGGCG GCATTACAAC TGTACCGATA TATATTTCTT GAAGTTGGAC 1380
GATATCACTG TATTCGGGAC TCCTGCATCA GAGCCCGAAC CTGTTACCGA TTTCGTTGTC 1440
TCGCTTATTG AAAACAACAA GGGACGATTA AAGTGGAATT ATCCTAACGG CTACGAACCC 1500
GATAAGACTG ATGATAAAGA CCCATTGCAG CTTGCCGGCT ACAATATCTA TGCAAACGGC 1560
TCGCTCCTTG TTCACATACA AGACCCGACT GTTTTGGAGT ATATCGATGA GACTTATTCT 1620
TCACGAGACG ATCAGGTGGA AGTGGAATAT TGTGTCACTG CCGTTTATAA CGACAATATC 1680
GAGTCCCAAT CGGTTTGCGA TAAGCTGATT TATGATTCTC AATCGGACAT TATCTTATAT 1740
CAAGGCTTTG AGGCCGGAAG TATTCCTGAA GGCTGGTTGT TGATTGATGC TGATGGCGAC 1800
AATGTTAATT GGGACTATTA TCCTTGGACT ATGTATGGAC ATGACAGTGA GAAGTGTATT 1860
GCATCCCCTT CGTACTTACC GATGATTGGC GTTTTAACTC CGGATAACTA TTTGGTTACA 1920
CCCAGACTCG AAGGAGCCAA GCTTGTCAAG TATTGGGTAA GTGCGCAAGA TGCTGTTTAT 1980
TCGGCTGAGC ATTATGCTGT GATGGTTTCT ACTACGGGAA CTGCTGTTGA AGATTTTGTC 2040
CTCTTGTTCG AAGAGACAAT GACCGCTAAG GCTAACGGTG CATGGTATGA GCGAACTATT 2100
ACATTGCCTG CAGGAACAAA ATATATTGCC TGGCGGCATT ATGATTGCAC CGATATGTTT 2160
TTCTTGCTCT TGGATGACAT TACGGTTTAT CGTTCTACTG AGACTGTTCC CGAGCCTGTT 2220
ACTGATTTCG TTGTCTCGCT TATTGAGAAT AACAAGGGTC GCCTGAAATG GAATTATCCT 2280
AACGGCTACG AACCCGATAA GACTGATGAT AAAAAACCAT TGCAGCTTAC CGGCTACAAC 2340
ATCTATGCAA ATGGCTCGCT CCTTGTTCAC ATACAAGACC CGACTGTTTT GGAGTATATC 2400
GATGAGACTT ATTCTTCACG AGACGGTCAG GTGGAAATGG AATATTGTGT CACTGCCGTT 2460
TATAACGACA ATATCGAGTC CCAATCGGTT TGCGATAAGC TGAACTATAC TATCACATCC 2520
TTGGATAATA TTCAATCTGA TACAAGCTTG AAAATATATC CTAATCCGGC ATCGTATGTG 2580
GTAAGGATAG AGGGATTGAG TCGGAGCAAG TCGACAATCG AGTTGTATAA TGCGCTGGGA 2640
ATTTGCATAT TAAGGGAAGA GACTCATTCA GAGAAAACGG AAATCGATGT TTCACGTCTC 2700
AATGACGGAG TCTACTTGAT TAAAGTAGTC GGTGGAAATA AAACAACAAC CGAAAAGGTA 2760
GAGATAAAGA GGCCG 2775

(2) INFORMATION FOR SEQ ID NO:204

(i) SEQUENCE CHARACTERISTICS:
(A) LENGTH: 2766 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2766

(xi) SEQUENCE DESCRIPTION: SEQ ID NO:204

ATGATGAAAA GTATTGTTTT TAGAGCATTT CTAACGATTT TGCTCTCGTG GGCAGCGATC 60
ACGAATCCGA CTGCTCAAGA GATCTCAGGC ATGAATGCAT CCTGTCTGGC TGCTCCGGCT 120
CAACCGGATA CTATCTTATA TGAAAGTTTT GAGAATGGAC CTGTTCCCAA TGGCTGGCTT 180
GAGATAGATG CTGATGCTGA TGGTGCCACT TGGGGAAGCC CATCAGGCTC TTTCTCTGTA 240
CCTTACGGAC ACAATGGCCT TTGCACCTAC TCCCATATAC GTTCCGGTAT CTCAACAGCG 300
GGCAACTATC TGATTACACC CAATATAGAA GGAGCCAAAC GGGTCAAGTA CTGGGTATGC 360
AATCAGTATA GTACCAATCC GGAACATTAC GCAGTAATGG TATCGACAAC GGGGACTGCC 420
ATTGAAGACT TTGTTTTGTT GTTTGATGAT TCCATAACAG GGAAACCGAC TCCTCTTGTA 480
TGGCGTAGAC GAATCGTGGA CTTACCGGAA GGGACCAAAT ATATTGCATG GCGACATTAC 540
AAAGTCACCG ACTCACACAC AGAATTCTTG AAATTGGATG ATGTCACTGT GTATAGGTCG 600
ATCGAAGGGC CCGAACCTGC TACCGACTTC ACAGTAATCA ATATTGGTCA GAATGTGGGA 660
CGATTGACTT GGAACTATCC GGAGGATTAT CAACCGGAAG GAAAGGGGAA TGAAGAGTTG 720
CAGCTTAGCG GCTACAACAT CTATGCGAAC GGTACACTAC TGGCACAAAT AAAAGATGTC 780
TCCATACTGG AGTATGTGGA CAGCACTTAC TCTTTGCGAG ACAATCCCTT GCAAGTGGAG 840
TACTGCGTTA CAGCCGTTTA CGATGAAAGC ATAGAATCTT CGACCGTATG TGGCACGCTG 900
CATTACGCCA CGGATGCCAT CCTTTATGAA AATTTTGAGA ATGGACCTGT TCCCAATGGT 960
TGGCTTGTGA TAGACGCTGA TGGAGATGGA TTTAGCTGGG GACACTATTT GAATGCATAC 1020
GACGCTTTTC CCGGCCATAA TGGAGGCCAT TGCTCCTTGT CGGCTTCTTA TGTTCCGGGT 1080
ATAGGCCCGG TGACTCCCGA CAACTATCTG ATTACCCCCA AGGTTGAAGG AGCCAAACGT 1140
GTCAAGTACT GGGTAAGCAC GCAGGATGCC AATTGGGCAG CGGAACATTA CGCGGTGATG 1200
GCTTCGACAA CGGGGACTGC TGTCGGAGAT TTCGTCATAT TGTTCGAAGA AACCATGACA 1260
GCGAAGCCGA CCGGCGCATG GTATGAAAGA ACCATCAACT TACCTGAAGG GACTAAATAC 1320
ATCGCATGGC GGCATTACAA CTGTACCGAT ATATATTTCT TGAAGTTGGA CGATATCACT 1380
GTATTCGGGA CTCCTGCATC AGAGCCCGAA CCTGTTACCG ATTTCGTTGT CTCGCTTATT 1440
GAAAACAACA AGGGACGATT AAAGTGGAAT TATCCTAACG GCTACGAACC CGATAAGACT 1500

GATGATAAAG ACCCATTGCA GCTTGCCGGC TACAATATCT ATGCAAACGG CTCGCTCCTT 1560
GTTCACATAC AAGACCCGAC TGTTTTGGAG TATATCGATG AGACTTATTC TTCACGAGAC 1620
GATCAGGTGG AAGTGGAATA TTGTGTCACT GCCGTTTATA ACGACAATAT CGAGTCCCAA 1680
TCGGTTTGCG ATAAGCTGAT TTATGATTCT CAATCGGACA TTATCTTATA TGAAGGCTTT 1740
GAGGCCGGAA GTATTCCTGA AGGCTGGTTG TTGATTGATG CTGATGGCGA CAATGTTAAT 1800
TGGGACTATT ATCCTTGGAC TATGTATGGA CATGACAGTG AGAAGTGTAT TGCATCCCCT 1860
TCGTACTTAC CGATGATTGG CGTTTTAACT CCGGATAACT ATTTGGTTAC ACCCAGACTC 1920
GAAGGAGCCA AGCTTGTCAA GTATTGGGTA AGTGCGCAAG ATGCTGTTTA TTCGGCTGAG 1980
CATTATGCTG TGATGGTTTC TACTACGGGA ACTGCTGTTG AAGATTTTGT CCTCTTGTTC 2040
GAAGAGACAA TGACCGCTAA GGCTAACGGT GCATGGTATG AGCGAACTAT TACATTGCCT 2100
GCAGGAACAA AATATATTGC CTGGCGGCAT TATGATTGCA CCGATATGTT TTTCTTGCTC 2160
TTGGATGACA TTACGGTTTA TCGTTCTACT GAGACTGTTC CCGAGCCTGT TACTGATTTC 2220
GTTGTCTCGC TTATTGAGAA TAACAAGGGT CGCCTGAAAT GGAATTATCC TAACGGCTAC 2280
GAACCCGATA AGACTGATGA TAAAAAACCA TTGCAGCTTA CCGGCTACAA CATCTATGCA 2340
AATGGCTCGC TCCTTGTTCA CATACAAGAC CCGACTGTTT TGGAGTATAT CGATGAGACT 2400
TATTCTTCAC GAGACGGTCA GGTGGAAATG GAATATTGTG TCACTGCCGT TTATAACGAC 2460
AATATCGAGT CCCAATCGGT TTGCGATAAG CTGAACTATA CTATCACATC CTTGGATAAT 2520
ATTCAATCTG ATACAAGCTT GAAAATATAT CCTAATCCGG CATCGTATGT GGTAAGGATA 2580
GAGGGATTGA GTCGGAGCAA GTCGACAATC GAGTTGTATA ATGCGCTGGG AATTTGCATA 2640
TTAAGGGAAG AGACTCATTC AGAGAAAACG GAAATCGATG TTTCACGTCT CAATGACGGA 2700
GTCTACTTGA TTAAAGTAGT CGGTGGAAAT AAAACAACAA CCGAAAAGGT AGAGATAAAG 2760
AGGCCG 2766

(2) INFORMATION FOR SEQ ID NO:205

(i) SEQUENCE CHARACTERISTICS:
(A) LENGTH: 2763 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2763

(xi) SEQUENCE DESCRIPTION: SEQ ID NO:205

ATGAAAAGTA TTGTTTTTAG AGCATTTCTA ACGATTTTGC TCTCGTGGGC AGCGATCACG 60
AATCCGACTG CTCAAGAGAT CTCAGGCATG AATGCATCCT GTCTGGCTGC TCCGGCTCAA 120
CCGGATACTA TCTTATATGA AAGTTTTGAG AATGGACCTG TTCCCAATGG CTGGCTTGAG 180
ATAGATGCTG ATGCTGATGG TGCCACTTGG GGAAGCCCAT CAGGCTCTTT CTCTGTACCT 240
TACGGACACA ATGGCCTTTG CACCTACTCC CATATACGTT CCGGTATCTC AACAGCGGGC 300
AACTATCTGA TTACACCCAA TATAGAAGGA GCCAAACGGG TCAAGTACTG GGTATGCAAT 360
CAGTATAGTA CCAATCCGGA ACATTACGCA GTAATGGTAT CGACAACGGG GACTGCCATT 420
GAAGACTTTG TTTTGTTGTT TGATGATTCC ATAACAGGGA AACCGACTCC TCTTGTATGG 480
CGTAGACGAA TCGTGGACTT ACCGGAAGGG ACCAAATATA TTGCATGGCG ACATTACAAA 540
GTCACCGACT CACACACAGA ATTCTTGAAA TTGGATGATG TCACTGTGTA TAGGTCGATC 600
GAAGGGCCCG AACCTGCTAC CGACTTCACA GTAATCAATA TTGGTCAGAA TGTGGGACGA 660
TTGACTTGGA ACTATCCGGA GGATTATCAA CCGGAAGGAA AGGGGAATGA AGAGTTGCAG 720
CTTAGCGGCT ACAACATCTA TGCGAACGGT ACACTACTGG CACAAATAAA AGATGTCTCC 780
ATACTGGAGT ATGTGGACAG CACTTACTCT TTGCGAGACA ATCCCTTGCA AGTGGAGTAC 840
TGCGTTACAG CCGTTTACGA TGAAAGCATA GAATCTTCGA CCGTATGTGG CACGCTGCAT 900
TACGCCACGG ATGCCATCCT TTATGAAAAT TTTGAGAATG GACCTGTTCC CAATGGTTGG 960
CTTGTGATAG ACGCTGATGG AGATGGATTT AGCTGGGGAC ACTATTTGAA TGCATACGAC 1020
GCTTTTCCCG GCCATAATGG AGGCCATTGC TCCTTGTCGG CTTCTTATGT TCCGGGTATA 1080
GGCCCGGTGA CTCCCGACAA CTATCTGATT ACCCCCAAGG TTGAAGGAGC CAAACGTGTC 1140
AAGTACTGGG TAAGCACGCA GGATGCCAAT TGGGCAGCGG AACATTACGC GGTGATGGCT 1200
TCGACAACGG GGACTGCTGT CGGAGATTTC GTCATATTGT TCGAAGAAAC CATGACAGCG 1260
AAGCCGACCG GCGCATGGTA TGAAAGAACC ATCAACTTAC CTGAAGGGAC TAAATACATC 1320
GCATGGCGGC ATTACAACTG TACCGATATA TATTTCTTGA AGTTGGACGA TATCACTGTA 1380
TTCGGGACTC CTGCATCAGA GCCCGAACCT GTTACCGATT TCGTTGTCTC GCTTATTGAA 1440
AACAACAAGG GACGATTAAA GTGGAATTAT CCTAACGGCT ACGAACCCGA TAAGACTGAT 1500
GATAAAGACC CATTGCAGCT TGCCGGCTAC AATATCTATG CAAACGGCTC GCTCCTTGTT 1560
CACATACAAG ACCCGACTGT TTTGGAGTAT ATCGATGAGA CTTATTCTTC ACGAGACGAT 1620
CAGGTGGAAG TGGAATATTG TGTCACTGCC GTTTATAACG ACAATATCGA GTCCCAATCG 1680

GTTTGCGATA AGCTGATTTA TGATTCTCAA TCGGACATTA TCTTATATGA AGGCTTTGAG 1740
GCCGGAAGTA TTCCTGAAGG CTGGTTGTTG ATTGATGCTG ATGGCGACAA TGTTAATTGG 1800
GACTATTATC CTTGGACTAT GTATGGACAT GACAGTGAGA AGTGTATTGC ATCCCCTTCG 1860
TACTTACCGA TGATTGGCGT TTTAACTCCG GATAACTATT TGGTTACACC CAGACTCGAA 1920
GGAGCCAAGC TTGTCAAGTA TTGGGTAAGT GCGCAAGATG CTGTTTATTC GGCTGAGCAT 1980
TATGCTGTGA TGGTTTCTAC TACGGGAACT GCTGTTGAAG ATTTTGTCCT CTTGTTCGAA 2040
GAGACAATGA CCGCTAAGGC TAACGGTGCA TGGTATGAGC GAACTATTAC ATTGCCTGCA 2100
GGAACAAAAT ATATTGCCTG GCGGCATTAT GATTGCACCG ATATGTTTTT CTTGCTCTTG 2160
GATGACATTA CGGTTTATCG TTCTACTGAG ACTGTTCCCG AGCCTGTTAC TGATTTCGTT 2220
GTCTCGCTTA TTGAGAATAA CAAGGGTCGC CTGAAATGGA ATTATCCTAA CGGCTACGAA 2280
CCCGATAAGA CTGATGATAA AAAACCATTG CAGCTTACCG GCTACAACAT CTATGCAAAT 2340
GGCTCGCTCC TTGTTCACAT ACAAGACCCG ACTGTTTTGG AGTATATCGA TGAGACTTAT 2400
TCTTCACGAG ACGGTCAGGT GGAAATGGAA TATTGTGTCA CTGCCGTTTA TAACGACAAT 2460
ATCGAGTCCC AATCGGTTTG CGATAAGCTG AACTATACTA TCACATCCTT GGATAATATT 2520
CAATCTGATA CAAGCTTGAA AATATATCCT AATCCGGCAT CGTATGTGGT AAGGATAGAG 2580
GGATTGAGTC GGAGCAAGTC GACAATCGAG TTGTATAATG CGCTGGGAAT TTGCATATTA 2640
AGGGAAGAGA CTCATTCAGA GAAAACGGAA ATCGATGTTT CACGTCTCAA TGACGGAGTC 2700
TACTTGATTA AAGTAGTCGG TGGAAATAAA ACAACAACCG AAAAGGTAGA GATAAAGAGG 2760
CCG 2763

(2) INFORMATION FOR SEQ ID NO:206

(i) SEQUENCE CHARACTERISTICS:
(A) LENGTH: 1779 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1779

(xi) SEQUENCE DESCRIPTION: SEQ ID NO:206

ATGAACAGCA TCATGAAATA TCAATTATAT ACGGCCGTCA TAATGGCTCT CTCTGTATCA 60
TCCGTTTGCG GTCAAACCCC ACGAAATACA GAAACCAAAC GCCCCGACAC GCTGCGCAGG 120
CAGCTTACTA TCGTTAATGA CCAGACTGTG GAGATGGAGC ATGCGGATCC GCTTCCGGCT 180
GCATACAAGG CCATCGAACC TCGATTAAAA CCTTTCCGTC CGGAATATAA CAAGCGTACA 240
TTCGGATTTG TCCCTGAAGT TTCCTCTTCA GGCAGGAACA ATCTTCCGAA TATCCTGCCG 300
ACGGAAGGTC ATATGAAGCA CCGGGGGTAC CTGAATATCG GTATCGGCCA TACGCTAAAC 360
CAGCGAATGG ATGCCGGCTA TCGTCTGATA GATGCAGAGC AGGAGAGACT GAATCTTTTC 420
CTCTCCTATC GTGGGATGAA ATCGGCTTTC AATACCGGTG ACTTCGACGG CGACAGAAAG 480
GATAGACGAA TGATGGCAGG AGTGGACTAC GAGCAGCGCA GGCCTTCCTT TGTGCTTGCT 540
ACCGGCTTGT ATTATTCGAA CCATTATTTC AATAACTACG GACGGGGAGC TACCACCAAT 600
GTGGGCAGCA TCCCTCAGCT ATCGACACCT GTTACTCCTC AGATGGACAA CGGGACCCAC 660
AACGTCCGTG TATACTTGGG TGCAAAAAAT GATGTGATCG ATGCCAGGAT CGACTATCGT 720
TTCTTCCGTT CTATTCCCTA TCTGGGTACC GATCCGATGA AGGCTCTCAC AGAACATACG 780
CCTGAACTGA ACGTGACGAT GAGTAATGAG TTGTCCGATG ATATTAAGCT CGGTGTCGAA 840
GTTCGTACGG GAGGATTGTT TTTTGCCAAA AACAGCGAAA TGATTCAAAC GGGCGTTCTG 900
TCCGAAACCG ACCGCAACCT GTATTATGTG GAGGGCGCGC CCACAATCGG ATTTGTCGGA 960
GACTCGGACA ATATGCAATG GAACATACAG GCCGGAGTAG GGATTTCTTC CCATTTCGGA 1020
GCCAAAGGGA GGTTGTTTTT CTGGCCTAAA CTGGATGCTT CGCTTAGTAT CTTCCCTTCA 1080
TGGCGTGTGT ATGCGAAAGC CTTCGGCGGT GTGATTCGAA ATGGTCTCGC CGATGTTATG 1140
CAAGAGGAGA TGCCCTACCT GATGCCCAAT ACGATTGTAC TCCCTTCGCG CAATGCTTTG 1200
ACCGCCCAAT TAGGGGTGAA GGGGAATATA GCCGATGTGG TACGTATGGA GGTTTATGGC 1260
GACTTCTCCA AGCTGACAGG TGTGCCTTTC TATACTCCGA CTCTACCCTT ATATAATCCA 1320
TCCGACTTGT ATCAGTATAA TGTGAGTTTC TTGCCGATAT ATGCCGACGG CAGCCGCTGG 1380
CGCGCAGGTG GTAAGCTGGA ATACTCTTAT CGCGATATGC TCCGCTTTCT GGTAGACGCA 1440
TCCTATGGCA AGTGGAATTT GGATGGAGGA CTTGTCGCCT CCATGCAGCC CGATCTTATA 1500
TTGAAGGCAG AAGTAGGTGT TCATCCCATT GCCCCATTGG ATGTCAGACT CCGGTATACA 1560
CAGCTGAACG GACGGTATCG GTATTCTTTC GGCTCGGCTG GCTCGGAAGC CTTGGGTATC 1620
GGTAATGTAC ATCTTCTTAG TGCGGATGTT TCATACAAGC TGAAAAAGAA CTTGAGCCTT 1680
TATCTCAAAA TCGATAATAT GCTGGCGGAA ACGACAGAGC TTATCGGTTA TTATCCTATG 1740
CAGCCGTTCC ATTGTTTCGC CGGTTTTAGC TGGACTTTC 1779

(2) INFORMATION FOR SEQ ID NO:207

(i) SEQUENCE CHARACTERISTICS:
(A) LENGTH: 1767 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1767

(xi) SEQUENCE DESCRIPTION: SEQ ID NO:207

ATGAAATATC AATTATATAC GGCCGTCATA ATGGCTCTCT CTGTATCATC CGTTTGCGGT 60
CAAACCCCAC GAAATACAGA AACCAAACGC CCCGACACGC TGCGCAGGGA GCTTACTATC 120
GTTAATGACC AGACTGTGGA GATGGAGCAT GCGGATCCGC TTCCGGCTGC ATACAAGGCC 180
ATCGAACCTC GATTAAAACC TTTCCGTCCG GAATATAACA AGCGTACATT CGGATTTGTC 240
CCTGAAGTTT CCTCTTCAGG CAGGAACAAT CTTCCGAATA TCCTGCCGAC GGAAGGTCAT 300
ATGAAGCACC GGGGGTACCT GAATATCGGT ATCGGCCATA CGCTAAACCA GCGAATGGAT 360
GCCGGCTATC GTCTGATAGA TGCAGAGCAG GAGAGACTGA ATCTTTTCCT CTCCTATCGT 420
GGGATGAAAT CGGCTTTCAA TACCGGTGAC TTCGACGGCG ACAGAAAGGA TAGACGAATG 480
ATGGCAGGAG TGGACTACGA GCAGCGCAGG CCTTCCTTTG TGCTTGCTAC CGGCTTGTAT 540
TATTCGAACC ATTATTTCAA TAACTACGGA CGGGGAGCTA CCACCAATGT GGGCAGCATC 600
CCTCAGCTAT CGACACCTGT TACTCCTCAG ATGGACAACG GGACCCACAA CGTCCGTGTA 660
TACTTGGGTG CAAAAAATGA TGTGATCGAT GCCAGGATCG ACTATCGTTT CTTCCGTTCT 720
ATTCCCTATC TGGGTACCGA TCCGATGAAG GCTCTCACAG AACATACGCC TGAACTGAAC 780
GTGACGATGA GTAATGAGTT GTCCGATGAT ATTAAGCTCG GTGTCGAAGT TCGTACGGGA 840
GGATTGTTTT TTGCCAAAAA CAGCGAAATG ATTCAAACGG GCGTTCTGTC CGAAACCGAC 900
CGCAACCTGT ATTATGTGGA GGGCGCGCCC ACAATCGGAT TTGTCGGAGA CTCGGACAAT 960
ATGCAATGGA ACATACAGGC CGGAGTAGGG ATTTCTTCCC ATTTCGGAGC CAAAGGGAGG 1020
TTGTTTTTCT GGCCTAAACT GGATGCTTCG CTTAGTATCT TCCCTTCATG GCGTGTGTAT 1080
GCGAAAGCCT TCGGCGGTGT GATTCGAAAT GGTCTCGCCG ATGTTATGCA AGAGGAGATG 1140
CCCTACCTGA TGCCCAATAC GATTGTACTC CCTTCGCGCA ATGCTTTGAC CGCCCAATTA 1200
GGGGTGAAGG GGAATATAGC CGATGTGGTA CGTATGGAGG TTTATGGCGA CTTCTCCAAG 1260
CTGACAGGTG TGCCTTTCTA TACTCCGACT CTACCCTTAT ATAATCCATC CGACTTGTAT 1320
CAGTATAATG TGAGTTTCTT GCCGATATAT GCCGACGGCA GCCGCTGGCG CGCAGGTGGT 1380
AAGCTGGAAT ACTCTTATCG CGATATGCTC CGCTTTCTGG TAGACGCATC CTATGGCAAG 1440
TGGAATTTGG ATGGAGGACT TGTCGCCTCC ATGCAGCCCG ATCTTATATT GAAGGCAGAA 1500
GTAGGTGTTC ATCCCATTGC CCCATTGGAT GTCAGACTCC GGTATACACA GCTGAACGGA 1560
CGGTATCGGT ATTCTTTCGG CTCGGCTGGC TCGGAAGCCT TGGGTATCGG TAATGTACAT 1620
CTTCTTAGTG CGGATGTTTC ATACAAGCTG AAAAAGAACT TGAGCCTTTA TCTCAAAATC 1680
GATAATATGC TGGCGGAAAC GACAGAGCTT ATCGGTTATT ATCCTATGCA GCCGTTCCAT 1740
TGTTTCGCCG GTTTTAGCTG GACTTTC 1767

(2) INFORMATION FOR SEQ ID NO:208

(i) SEQUENCE CHARACTERISTICS:
(A) LENGTH: 1038 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature

(B) LOCATION 1...1038

(xi) SEQUENCE DESCRIPTION: SEQ ID NO:208

ATGATGGAAA AATGTATCTT TGCTCACTAT CCACATAACC TTGTGTTCAT GATTCGCAAG 60
CATTTCGGTA TCATTTTGGG ATTTCTTTCT CTTGTGTTTT CGGCAGGTGC TCAACAAGAG 120
AAGCAGGTGT TTCATTTTCT GAACCTTCCG GCTACTGCAC AGGCTTTGGC TGCCGGAGGC 180
AAAGCTATCA CCATCGTAGA CGACAATCCC GGACTGGCTT TTGAGAATCC GGCTCTGCTC 240
GGATATGAAT CCGGTGGCCG CGCCTTTCTT TCCTATTTAT ATTATATGAG TGGTTCGCAT 300
ATGGGCAATG CCTGTTATGC CTCGTCCGTC GGAGAGCGTG GCATGTGGGG TGTTGGCATG 360
CGTTTCCTGA ACTACGGGTC TATGCAAGGA TACGATCAGA ATGCGATTGC CACCGGCTCT 420
TTTAGTGCTT CGGATATAGC TGTACAAGGA TTTTACAGCC ATGAACTGAG CAACCACTTC 480
CGCGGTGGAG TCAGCCTAAA AGCATTGTAT TCTTCTATCG AGACGTATAG TTCCTTTGGC 540
CTTGGTGTGG ATGTCGGTAT CAGTTATTAC GACGATGACA AAGGATATTC CGCTTCCGCT 600
CTGTTCAAGA ACGTAGGGGC GCAACTGAAA GGCTATAATG AAGAACGGGA ACCGCTCGAT 660
TGGGATTTCC AGCTCGGCTT TTCCCGCAGT TTTATCAATG CTCCGTTTCG CTTGCACATC 720
ACGTTGTTCA ATCTGAATCC GCACTATTTC AAGCGTCTTG TACCACGCGA TCTGTCCAAG 780
ATGCAAAAGT TCCTCCGACA CTTCTCGATA GGAGCAGAAT TTACTCCTTC CGAGAGGTTT 840
TGGGTCGGGC TGGGATATAC GCCACAGATT GCACAGGATT TCGAGGTGGA AGGCGGCAAC 900
AAATGGGGAG GTCTTTCGGC CGGCGTCGGT TTCACTTCAG GTGTAGTACG TGTAGGCGTA 960
TCTGCTGCCA CCTATCATCC TGCAGCTCTT TCGTTCATGT GTTCGGTAGG TATCCGTTTG 1020
GACGATAAGA GCATCTTC 1038

(2) INFORMATION FOR SEQ ID NO:209

(i) SEQUENCE CHARACTERISTICS:
(A) LENGTH: 1035 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1035

(xi) SEQUENCE DESCRIPTION: SEQ ID NO:209

ATGGAAAAAT GTATCTTTGC TCACTATCCA CATAACCTTG TGTTCATGAT TCGCAAGCAT 60
TTCGGTATCA TTTTGGGATT TCTTTCTCTT GTGTTTTCGG CAGGTGCTCA ACAAGAGAAG 120
CAGGTGTTTC ATTTTCTGAA CCTTCCGGCT ACTGCACAGG CTTTGGCTGC CGGAGGCAAA 180
GCTATCACCA TCGTAGACGA CAATCCCGGA CTGGCTTTTG AGAATCCGGC TCTGCTCGGA 240
TATGAATCCG GTGGCCGCGC CTTTCTTTCC TATTTATATT ATATGAGTGG TTCGCATATG 300
GGCAATGCCT GTTATGCCTC GTCCGTCGGA GAGCGTGGCA TGTGGGGTGT TGGCATGCGT 360
TTCCTGAACT ACGGGTCTAT GCAAGGATAC GATCAGAATG CGATTGCCAC CGGCTCTTTT 420
AGTGCTTCGG ATATAGCTGT ACAAGGATTT TACAGCCATG AACTGAGCAA CCACTTCCGC 480
GGTGGAGTCA GCCTAAAAGC ATTGTATTCT TCTATCGAGA CGTATAGTTC CTTTGGCCTT 540
GGTGTGGATG TCGGTATCAG TTATTACGAC GATGACAAAG GATATTCCGC TTCCGCTCTG 600
TTCAAGAACG TAGGGGCGCA ACTGAAAGGC TATAATGAAG AACGGGAACC GCTCGATTGG 660
GATTTCCAGC TCGGCTTTTC CCGCAGTTTT ATCAATGCTC CGTTTCGCTT GCACATCACG 720
TTGTTCAATC TGAATCCGCA CTATTTCAAG CGTCTTGTAC CACGCGATCT GTCCAAGATG 780
CAAAAGTTCC TCCGACACTT CTCGATAGGA GCAGAATTTA CTCCTTCCGA GAGGTTTTGG 840
GTCGGGCTGG GATATACGCC ACAGATTGCA CAGGATTTCG AGGTGGAAGG CGGCAACAAA 900
TGGGGAGGTC TTTCGGCCGG CGTCGGTTTC ACTTCAGGTG TAGTACGTGT AGGCGTATCT 960
GCTGCCACCT ATCATCCTGC AGCTCTTTCG TTCATGTGTT CGGTAGGTAT CCGTTTGGAC 1020
GATAAGAGCA TCTTC 1035

(2) INFORMATION FOR SEQ ID NO:210

(i) SEQUENCE CHARACTERISTICS:
(A) LENGTH: 990 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...990

(xi) SEQUENCE DESCRIPTION: SEQ ID NO:210

ATGATTCGCA AGCATTTCGG TATCATTTTG GGATTTCTTT CTCTTGTGTT TTCGGCAGGT 60
GCTCAACAAG AGAAGCAGGT GTTTCATTTT CTGAACCTTC CGGCTACTGC ACAGGCTTTG 120
GCTGCCGGAG GCAAAGCTAT CACCATCGTA GACGACAATC CCGGACTGGC TTTTGAGAAT 180
CCGGCTCTGC TCGGATATGA ATCCGGTGGC CGCGCCTTTC TTTCCTATTT ATATTATATG 240
AGTGGTTCGC ATATGGGCAA TGCCTGTTAT GCCTCGTCCG TCGGAGAGCG TGGCATGTGG 300
GGTGTTGGCA TGCGTTTCCT GAACTACGGG TCTATGCAAG GATACGATCA GAATGCGATT 360
GCCACCGGCT CTTTTAGTGC TTCGGATATA GCTGTACAAG GATTTTACAG CCATGAACTG 420
AGCAACCACT TCCGCGGTGG AGTCAGCCTA AAAGCATTGT ATTCTTCTAT CGAGACGTAT 480
AGTTCCTTTG GCCTTGGTGT GGATGTCGGT ATCAGTTATT ACGACGATGA CAAAGGATAT 540
TCCGCTTCCG CTCTGTTCAA GAACGTAGGG GCGCAACTGA AAGGCTATAA TGAAGAACGG 600
GAACCGCTCG ATTGGGATTT CCAGCTCGGC TTTTCCCGCA GTTTTATCAA TGCTCCGTTT 660
CGCTTGCACA TCACGTTCTT CAATCTGAAT CCGCACTATT TCAAGCGTCT TGTACCACGC 720
GATCTGTCCA AGATGCAAAA GTTCCTCCGA CACTTCTCGA TAGGAGCAGA ATTTACTCCT 780
TCCGAGAGGT TTTGGGTCGG GCTGGGATAT ACGCCACAGA TTGCACAGGA TTTCGAGGTG 840
GAAGGCGGCA ACAAATGGGG AGGTCTTTCG GCCGGCGTCG GTTTCACTTC AGGTGTAGTA 900
CGTGTAGGCG TATCTGCTGC CACCTATCAT CCTGCAGCTC TTTCGTTCAT GTGTTCGGTA 960
GGTATCCGTT TGGACGATAA GAGCATCTTC 990

(2) INFORMATION FOR SEQ ID NO:211

(i) SEQUENCE CHARACTERISTICS:
(A) LENGTH: 972 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...972

(xi) SEQUENCE DESCRIPTION: SEQ ID NO:211

ATGTGCCTCG AACCCATAAT TGCTCCGATT TCATCCGAGT TGCTCGAGCA GGAGCTGACT 60
GCCGATCGTT TTCTGCGGAT GACAAACAAA GCCGGCAATG AGATCTATGT TTTTACGGCC 120
GAAGAAGCTC CGCATTGCAT GAAAGAAGTA GGCCGACTGC GAGAAGAAGC CTTTCGGCAT 180
TATGGCGGAG GTACTGGCAA GGCGATCGAT ATAGACGAGT TCGACACCAT GCCCGGGAGC 240
TACAAACAGC TGATCGTATG GGATCCGCAA AACAAGGCTA TACTCGGAGG CTACCGCTTT 300
ATCTATGGGC GGGACGTTGC TTTCGATACC GATGGCAAGC CTTTGCTGGC AACGGCAGAG 360
ATGTTTCGCT TCAGTGATGC TTTTTTGCAC GATTATCTCC CCTACACAGT CGAATTGGGA 420
CGTTCGTTCG TGTCGCTCCA GTACCAATCG ACACGGATGG GCACAAAGGC CATTTTTGTG 480
CTGGACAATC TTTGGGACGG TATCGGAGCA CTCACTGTAG TCAATCCAGA GGCACTCTAT 540
TTCTATGGCA AGGTGACCAT GTACAAAGAC TATGATCGGC GAGCTCGCAA TCTGATCCTG 600
TATTTTCTTC GCAAGCACTT CTCCGATCCG GAAGGCTTGG TCAAGCCTAT TCATCCCCTA 660
CCGATAGAGA TCAGTGCGGA GGACGAAGCC TTGTTCTCCT CATCCGACTT TGACACCAAT 720
TACAAGACTC TCAATATAGA AGTGCGCAAG CTGGGTATCA ATATCCCTCC TCTCGTGAGT 780
GCATATATAG CTTTGTCTCC GGAGATGCGT GTTTTCGGCA CTGCAGTGAA TGAGTCTTTC 840
GGAGAGGTGG AGGAAACCGG CATATTCATT GCTGTGGGTA AGATCCTGGA AGAGAAAAAA 900
CAACGGCACA TAGAGAGCTT CATCCTCAGC CGGAACGAAA AAAAAGGTCT CGACAGTAGC 960
AATGGCCGAT CA 972

(2) INFORMATION FOR SEQ ID NO:212

(i) SEQUENCE CHARACTERISTICS:
(A) LENGTH: 1641 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1641

(xi) SEQUENCE DESCRIPTION: SEQ ID NO:212

ATGAAAACCA TTGTAAGATA CAGCCGCCTT CCGGTCGCTC TCTTCTTTTG CCTTTTGGGA 60
GCTGTGCACT TGTCTGTCGA GGCGCAGATG CTCAATACTC CTTTCGAGCT GTCGGATCAG 120
ATCGTCCTGT CTCCCACCGA AAGGCAATAC AGGGAGATTT GTGTGCAAAC GAAAGAAAAA 180
AGGGGGGCCG ATCTTTTCCC GTTGAGCGAT AAGCTGCGCG ATTCGGCCTA TGTTCGTTTC 240
GGCTCGGCCT ATGGCGATAT TGCGGGCGAC TATCTTCCGT ACAACGGCAA TAACTACTCC 300
TCGCTCTCGC TCGAATCGGG TGGTCGCATC AGTGTCCGTA ACTATGGCAC ATTGCAGGGC 360
AGTGCTTCCT ACTCACGTGG CATGCACAAA CGCATCGGCT GGAATGCTCT GCGCAACGCC 420
GAAGCCTACT ATCCCTATTT GGTGTCCGAT TCGACCGGCG GAGACTATCA TTTCGAAGAC 480
TATCGGCTTG CCGGCTACTA TTCTTTTCGC GCCGGCCGCT TGCCCCTCGG TATAGGCTTC 540
TCATACAGGG GCGAAGTTGC TTATCGGCTG ACCGATCCGC GTACGACCAA TACGACCGGT 600
GCATTGGAGC TTTCTTGTGC TACCTCTTTG ACGCTGCCTC GAGAGAACAG GCTATCGCTT 660
TCGGCTGCGT ATCTCTATCA TAGACAACAC CTCACACAGT ACAACTGGCG TCCCGGGCAG 720
CAGGACAAAT TCTTCGTCAG CTACGGTTTC GGTCAGGTGG ATGTCAGCAA CAGCCCTATC 780
TGGTTCGGTA TCTCCAGAAT GAACTACGTC AACGGATGGA AGCTTAGCTC CCGTCTGGAT 840
ACCCGTAGGG GCGATGCCAT CGGTCTCGAC TACAGCGGCT ACTTCCTCGA TACCGAaGAG 900
AGGTCGTCCA TCAATCTCTT TGCTTTGCTT TACAATCGCC TGCGACTCTA TGGTAGCTGG 960
CATCTGTCGG ACTTCGATTT TTCATTTTCA GCCGACTATG CTCTGCGCCA AGGGATAGAG 1020
CGGATATACG AAGACTACAA GCCGGATGAT AATTATCATA TCTACGACCT CCGTATCTTG 1080
GCCATTCGCC GCTGGTATAT GCTCAATGAG TTTTCTGCCC AAGCCCAAGC CTCCTACCGT 1140
ATTCGCACGG ATAGAGGTTG TGCCCTGAGA GTGAGTGCCG GTAGTGATTT CTACGGCTAT 1200
GATGAGACGT ATCGCAAGCA TGGACATCAT ACCATGAGCG GAATGCTACG TCCTTTTGCC 1260
GGTATAGCCT ATGACCATGC CGGATCCAAA TTGGATTTTG GACTTTCGCT TTCGGCTGCT 1320
TATCGAATGG TGCTGACGCA TTCGTATAAG ATTCGTACCA TCCAGAAAGA GCAGCTCGAC 1380
TATCAGCTGG CCTATTTGCC CTATGCCTAT CGTAATAGAG AAGGCGTGGA GGTGCGTTCC 1440
TCTCTGTACG TCTCGATTCC GATGCAGAAT ACCCACCGCC TGATGACAGA GCTGCGGTTG 1500
TATGGCGACC TGATGAAAAG AAAGGACGGT ATAGCCTATG GCAAAACGCC CGGTGTCATC 1560
TCACATATCC TGTCCGATCC GCAAGCCGAA CGAACGTCCG GCCATACCAT CGGGGCTATC 1620
TGCAATATCT CCTACCTCTT C 1641

(2) INFORMATION FOR SEQ ID NO:213

(i) SEQUENCE CHARACTERISTICS:
(A) LENGTH: 2250 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2250

(xi) SEQUENCE DESCRIPTION: SEQ ID NO:213

ATGAAAAAAC TTCACATGAT TGCCGCCTTA GCCGTCCTGC CTTTCTGCCT GACGGCACAA 60
GCACCCGTCT CCAACAGCGA GATAGATAGT CTTAGCAATG TGCAGCTCCA GACCGTACAG 120
GTCGTAGCTA CTCGCGCCAC GGCGAAAACC CCTGTCGCTT ACACCAACGT TCGCAAGGCC 180
GAACTTTCCA AGTCCAATTA TGGTCGTGAC ATCCCCTATC TGCTGATGCT GACTCCCTCC 240
GTGGTAGCCA CCAGCGATGC CGGTACGGGT ATCGGATATT CCGGCTTTCG CGTGCGTGGC 300
ACCGATGCCA ATCGCATCAA CATAACTACC AATGGAGTAC CCCTCAACGA CTCCGAATCT 360
CAGTCCGTCT TTTGGGTGAA TATGCCCGAC TTCGCCTCTT CCATCGAAGA CCTTCAGGTG 420
CAGCGAGGTG TGGGTACTTC CACCAATGGT GCCGGAGCTT TTGGGGCAAG TGTCAATATG 480
CGTACGGATA ATTTGGGACT GGCTCCTTAT GGCCGTGTCG ATTTGAGCGG AGGTTCGTTC 540
GGCACATTCC GCCGATCGGT CAAACTCGGT AGCGGACCCA TCGGTCGCCA TTGGGCAGTG 600
GATGCCCGCC TGTCCAAAAT CGGTTCGGAC GGCTACGTGG ATAGAGGAAG CGTGGATCTG 660
AAATCCTATT TCGCACAGGT GGGCTATTTC GGTAGCAACA CGGCTCTCAG GTTCATCACT 720
TTCGGAGGAA AAGAAGTTAC GGGTATCGCA TGGAACGGTC TTTCCAAGGA GGATGAAGCC 780
AAATATGGCC GCCGATACAA CAGTGCCGGT CTTATGTACG TGGACGCGCA AGGAGTACCG 840
CACTACTACC ACAATACCGA CAATTACGAG CAGCGTCACT ACCATGCCAT CATGACGCAC 900
AGCTTCTCTC CTTCCGTTAT CCTCAACCTC ACGGCACACT ACACGGCCGG ATATGGCTAT 960
ACGGACGAAT ATCGTACCGG ACGTAAACTA AAGGAATATG CACTGCAGCC CTATGTGGAA 1020
AACAGTGTGA CCGTGAAGAA AACGGATCTC ATCCGTCAGA AGTATCTGGA CAATGACTTC 1080
GGAGGACTCA TCGGTTCGCT TAACTGGCAC ACCGGTGCAT GGCATTTGCA GTTCGGGGCC 1140
TCGGGCAATA TCTATAAAGG AGACCACTTC GGCCGTATCA CTTACATCAA AAAGTACAAT 1200
CAGCCCTTAG CTCCCGACTT CGAATATTAT CGGAACAGGG CAGACAAAAG AGAAGGTGCA 1260
GCCTTTGCCA AAGCCAACTG GCAGATCACT CCGGAACTGA ACATGTATGC CGACCTCCAG 1320
TATCGTACCA TCGGCTACAC GATAAACGGC ATCACGGACG AATATGATGA GGTACAGGGA 1380
AG[?]ATGCAGC ACATCGATTT GGACAAGACC TTCCGCTTCC TCAATCCGAA GGCCGGTCTT 1440
ACCTATAGTT TCGACGATGC TCATACTGCC TATGCTTCTG TTGCGGTAGC ACACCGCGAG 1500
CCTAACAGAA CCAATTACAC CGAAGCCGGA ATAGGACAGT ATCCTACGCC TGAGCGACTG 1560
ATCGACTATG AGCTGGGCTA CCGCTATGCT TCGCCCCTCT TGTCGGCCGG AGTAGGTCTC 1620
TATTATATGC AATACAAGGA CCAACTCGTG CTGGATGGCC GTTTGAGCGA TGTGGGACAG 1680
ATGCTCACAA GCAACGTCCC CGACAGCTAC CGTATGGGAC TGGAGCTGAC TCTCGGTTGG 1740
CAGATCCTTC CTCGTTTGCT GCGTTGGGAT GCTTCTTTCA CTATGAGTCG CAACAAAATC 1800
GACCGCTACG TACAATATAC ATCCGTATAT GATGCGGACT ACAACTGGCT CGAACTCAAG 1860
GAGGAGACCC TCGAAAGCAC GGATATAGCC TACTCGCCCA ATGTCATTGC CGGCAGCATG 1920
CTTACCCTCT CTCATGCCGG TTTCGAAATG GCTTGGACGA GCCGCTTCGT CAGCAAGCAA 1980
TATCTGGACA ATACACAGCG CAGCGATCGC ATGCTTTCCT CCTATTGGGT GAACGACCTC 2040
CGCCTCGGCT ATGTGCTGCC GGTTCACTTC GTTAAGAGAG TGGCACTGGG CGTACAGCTC 2100
AATAATCTCT TCAACCTCAT GTATGCGTCC AATGCCTACA TCTACGATGC CGGTTACGTA 2160
CAGGCATCCG GAGAACTAAG TGCATATGCC GATCTGCGTT ATTATCCTCA GGCCGGATTT 2220
AATGCACTGG GTAGTCTGAC AATCGATTTC 2250

(2) INFORMATION FOR SEQ ID NO:214

(i) SEQUENCE CHARACTERISTICS:
(A) LENGTH: 1482 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1482

(xi) SEQUENCE DESCRIPTION: SEQ ID NO:214
   ATGAAAAGAA GGTTTCTATC GCTGTTACTG CTGTACATAC TCTCTTCCAT CAGCCTTTCT 60
   GCTCAGCGGT TTCCGATGGT GCAGGGAATC GAGTTGGATA CCGATTCGCT TTTCTCTCTG 120
   CCCAAGCGTC CTTGGCGCGC CATCGGTAAA ACGATAGGCG TCAATCTGGC CGTATGGGGC 180
   TTCGATCATT TCATCATGAA CGAGGACTTT GCAGACATCA GTTGGCAGAC TATCAAGAGC 240
   AATTTCCAAA CAGGCTTTGG CTGGGACAAT GACAAGTTTG TCACCAACCT CTTCGCACAT 300
   CCTTATCACG GATCGCTCTA TTTCAATGCA GCGAGGTCGA ACCGTTTGAG CTTCAGGCAC 360
   TCTGCTCCGT TTGCCTTCTT TGGCAGTCTC ATGTGGGAGC TGCTTATGGA AAACGAGCCA 420
   CCGAGTATCA ACGACCTCTG TGCCACCACC ATAGGCGGTA TAGCTTTGGG GGAGATGGGG 480
   CACAGGCTGT CGGACCTGCT CATCGACAAT CGTACCACAG GGTGGGAACG TATGGGGCGC 540
   GAGGTGGCTA TCGCTCTGAT CAATCCGATG CGCTTTCTCA ACCGTCTGAC AGCAGGAGAG 600
   GTGACTTCTG TCGGGAGTCG CAGCGGACAG ATATTTCAGT CTGTCCCCAT AAACATAGTC 660
   GTCGATGCCG GCTTTCGCTT TTTGGCAGAC AAGCGGCATG CCCGAACCGG TGCCACGGCT 720
   CTGACCCTGA ATCTGAGATT CGACTACGGC GATCCATTCC GAAGCGAGAC TTTCTCTCCA 780
   TACGATTTCT TCCAATTCAA AGCCGGATTG AGTTTCTCCG AATCGCAACC TCTGCTGAGC 840
   CAGATCAATC TGATCGGAAT CCTAAGCGGA TGCCAACTGC TCGCACACGA ACGAACGGTT 900
   TTGGTGGGAG GTCTCTTTCA GCACTTCGAC TACTACAATT CGGAAAAACG AATAAGCAAA 960
   AATTCGGAGG AGGTACTCGT CACCCCATAC CGTATCTCGC AAGTGGCAGC TCTGGGAGGC 1020
   GGTCTTATCT TCCAGCACCA CGGAAAATTT CGACGACGTC CTCTGGAGCT ATATGCCGAG 1080
   ACCTACCTGA ATGTCGTCCC GATGGGAGCC AGTCTGTCGG ATCACTACAA CGTGGACAAT 1140
   CGGGACTATA ACCTCGGCAG CGGATTGAGC GGCAAGCTAT ACCTTGGTGC TACGTACAAT 1200
   GATCTGTGGA GCTGGCTCTT GGGAGTCGAA AGCTATCGGC TCTACACATG GATCGGGTAT 1260
   GAAGAGCCGC ACCAGAAAAA TACCGATGTC AGCTCTTTTA TGGTGCAGGG GGACGAAAGC 1320
   AAGGCGCGCC TACTGGTGAC GAGTTCCGAG TTCGCATTTC ATCCTGGCCC CTGGCATGTA 1380
   GCCATCGTCG CTCGCCGTTT CATCCGCAAA ACAGCCTATC AATTCTACCC TAACGTATCA 1440
   TTCGATACCG GCGACATACA GCTGCGTGTC GGATTTCACT TC 1482

(2) INFORMATION FOR SEQ ID NO:215

(i) SEQUENCE CHARACTERISTICS:
(A) LENGTH: 882 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...882

(xi) SEQUENCE DESCRIPTION: SEQ ID NO:215

ATGAAACGAC TGATTGTTTT TCTGGCAATG GGTGGCTTGC TGTTCACCCT TGCGAACGCA 60
CAAGAAGCAA ACACTGCATC TGACACTCCC AAAAAGGACT GGACTATAAA AGGTGTGACC 120
GGACTAAATG CCTCTCAGAC TTCTCTGACC AACTGGGCTG CCGGTGGAGA AAACACGGTG 180
GCAGGTAACC TCTATTTGAA CATAGATGCC AACTACCTGA AAGATAAATG GAGTTGGGAC 240
AACGGTTTGC GTACAGACTT CGGTCTGACC TACACAACAG CCAACAAGTG GAACAAAAGT 300
GTAGACAAGA TCGAACTCTT CACGAAGGCC GGCTATGAGA TCGGCAAACA TTGGTACGGA 360
AGTGCGCTTT TCACTTTCCT CTCACAGTAT GCCAAAGGAT ATGAGAAGCC CTCGGATCAC 420
TTGACAGGAG TCAAGCATAT CTCTAATTTC TTCGCTCCTG CATATCTCAC TCTCGGTATT 480
GGTGCGGACT ATAAGCCCAA TGAGAAGTTC TCTCTCTACC TCTCTCCTAC AACGGGCAAG 540
CTGACTGTAG TAGCAGACGA CTACCTCTCA AGTTTGGGAG CCTTCGGGGT GAAAGTTGGT 600
GAAAAGACAA TGTTCGAACT TGGTGCTTTG GTAGTGGGTT CGGCCAATAT AAATCTGATG 660
GAGAATGTCA ATTTGATAAC CAAGGCTTCA TTCTTCTCGG CTTATACGCA CGACTTTGGC 720
AACATTGACA TCAATTGGGA GGCTATGCTG GCCATGAAGA TCAACAAGTT CCTCACGGCT 780
ACGATAGCCA CCAATCTTAT CTACGACGAT GATGTGAAGA TCAACGATGG CCCGAAAATC 840
CAGTTCAAAG AAGTTGTGGG CGTGGGTGTT GCGTACACTT TC 882

(2) INFORMATION FOR SEQ ID NO:216

(i) SEQUENCE CHARACTERISTICS:
(A) LENGTH: 612 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:

(A) NAME/KEY: misc_feature
(B) LOCATION 1...612

(xi) SEQUENCE DESCRIPTION: SEQ ID NO:216

ATGAAGAAAA TGATTTTGGC AGCTACTATG CTGCTCGCAA CAATCGGTTT TGCAAATGCT 60
CAGAGTCGTC CTGCTCTTAG ACTGGATGCT AACTTTGTCG GTAGTAACTT AATGCAAAAA 120
GTCGCAAACA CGAGCGTGAA CAATAAGATG ATCGTAGGCT TACGTGTTGG TGCTGCTGCT 180
GAGTTCGCTC TTAGCAATGA TGGATTCTAT CTCGCCCCCG GATTGGCCTA TACGATGAGA 240
GGTGCTAAGA TGGAATCACT AAGTGAAACG ACAACTCGCT TGCATTATCT GCAAATACCG 300
GTGAATGCCG GTATGAGATT TAGCTTTGCT GACAACATGG CTATTTCATT GGAAGCAGGT 360
CCCTATTTCG CATATGGTGT CGCCGGAACG ATTAAGACTA AAGTTGCAGG CGTTACGGCT 420
TCTGTAGATG CCTTTGGTGA TAACGGATAT AACCGTTTCG ACTTGGGCTT GGGCTTGTCT 480
GCTGCCTTGA GCTACGACCG TTATTACGTA CAAATTGGAT ATGAGCATGG ATTGCTTAAT 540
ATGTTGAAGG ATGCTCCGGA TAAGACTTCT TTGCGTAATC ATGACTTCTT TGTGGGTCTC 600
GGTGTTCGCT TC 612

(2) INFORMATION FOR SEQ ID NO:217

(i) SEQUENCE CHARACTERISTICS:
(A) LENGTH: 729 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...729

(xi) SEQUENCE DESCRIPTION: SEQ ID NO:217

ATGAAAAGGA TTTTTACTGT AGCCCTTGTG CTACTTGCTT CGGTCACTAT GGCCATCGGA 60
CAAAGCCGCC CGGCACTTCG CGTAGATGCC AACTTCGTAG GCAGCAATCA GAGCATGAAA 120
AGAGACGGAT ATGTGTGGGA CACCAAAATG AATGTCGGCC TGCGGGTCGG TGCCGCTGCC 180
GAATTCATGA TCGGATCAAG AGGATTCTAC TTGGCTCCGG GTCTGAACTA TACGATGAAG 240
GGCTCCAAAA CCGAATGGGA TATACCCGAA ATGGTTCCTG GTACCTATAT TACGATGGTT 300
TCCACTCGCT TGCACTATCT GCAACTGCCG ATCAATGCCG GCATGCGGTT CGACCTGATG 360
AATGACATGG CGGTTTCGAT CGAAGCGGGT CCTTTCCTTG CATACGGTAT ATATGGTACA 420
TATCGGCAGA AGTTGGAAGG ATGGAAGCCG AACAACTACA GCACAGAGTT TTTTGGCCCA 480
ACGCTTGGTG GCCCAACAAA TATCCGCTGG GACATCGGGG CAAACATAAT AGCCGCATTC 540
CACTATAAGC GTTATTATAT ACAGATAGGC TATGAACATG GATTTGTGGA TATTGTGTCA 600
GGTGGAGGTT CTGATATTCC CCGACTGAAC GACAATAGGC AATCCTCTTC GACGACCGCT 660
CTAAGAGAAA AGGGAAATAA CGAATACGCT TATAATCGTG ACTTCTTCGT GGGCATAGGT 720
TACCGCTTT 729

(2) INFORMATION FOR SEQ ID NO:218

(i) SEQUENCE CHARACTERISTICS:
(A) LENGTH: 621 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature

(B) LOCATION 1...621

(xi) SEQUENCE DESCRIPTION: SEQ ID NO:218

ATGAAAAGAA TGCTGCTGCT TCTCGTTGTA TTATTATATG GAATTGCAGG CCGATTGGCT 60
GCACAAGACG TTATCAGACC ATGGTCATTG CAGGTCGGAG CGGGATACTC CGATACGGAG 120
AACATCCCGG GAGGATTCAC CTATGGTTTC TATTTGGGAA AGCGTATGGG GAGCTTTCTG 180
GAAGTGGGGC TGTCCATGTA CAACTCCACA CGTCAAACAG CCAACAATGC AGACTCCTTT 240
GCATCGAACG AAGGAGACGG ATCTTTTCAG GTAAATATGT CTTCTCCGAA TGAGAAGTGG 300
TCATTCTTCG ATGCAGGCAG TGCCAACTGC TATATGATCG TCGTCGGAGT CAATCCTCTC 360
CATCTGTTTT GGCAGAATAG CCGGCACAAT TTGTTTCTGG CAGTACAAGC CGGCCTGTCC 420
AATAAGCACA ATATTCATTT CATCTATGGA GACAAGGGAG CCAAAGTCAG TATCTACACC 480
AATTCGAATA CCTACATCGG TTACGGAGCA CGTGTAGCCT ACGAATATCA AATTCATAAA 540
AACGTGGGGG CGGGTGCCGC TGTAATGTAC GACCACGGCA ATAAGATGCT TACGCCCATG 600
GCCACGCTCT CCACTCATTT T 621

(2) INFORMATION FOR SEQ ID NO:219

(i) SEQUENCE CHARACTERISTICS:
(A) LENGTH: 2853 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2853

(xi) SEQUENCE DESCRIPTION: SEQ ID NO:219

ATGAGAGTAT CCGATCTCTG TTCCAGACTT TCATGGTTAT TACCCGTAAT CCTTGTCGGA 60
TTGCTCTGTG CTACTTTGGT CGCTGCGGAA CGTCCTATGG CCGGAGCAGT CGGATTGCAC 120
CACCGTCGGC ATGCTGCGCT GTCTGATTCT ACAGCGAAAG ACACGGTGCC TCTCGCAAAA 180
CCTATTCCTG ACAGTGCTTT TCGAGATTCC CTTCCTGCCG ATTCCACCGG ATCGATGCGG 240
CAAGATAGCG TGTATGACGA TGAATTCGAA TTGGAAGATA TAGTGGAGTA CGAAGCTGCC 300
GATTCCATCG TTTTGCTCGG ACAGAATCGT GCCTATCTTT TCGGCAAGAG CTATGTGAGC 360
TATCAAAAGA GTCGCTTGGA GGCAAACTTC ATGTATCTCA ATACCGACAG CAGTACGGTT 420
TATACTCGCT ATGTCCTCGA TACGGCCGGT TATCCGATGG CCTTTCCTGT TTTCAAGGAT 480
GGAGAGCACT CGTTCGAAGC CAAGAACTTT ACCTACAACT TCCGCACGGA GAAGGGGATT 540
ATCAGCGGAG TGATCACGCA GCAGGGCGAA GGCTATCTGA CTGCCGGTAA GACCAAGAAG 600
ATGCCCGACA ATATCATGTT TATGCAAGGA GGGCGTTATA CGACCTGCGA CAATCACGAT 660
CATCCTCACT TCTATATCAA TCTTTCCAAG GCAAAGGTGC ATCCGGAGAA AGACATCGTC 720
ACAGGTCCGG TCAATCTGGT TATCGCCGAT ATGCCGCTGC CGATAGGTCT TCCTTTCGGC 780
TATTTTCCCT TTTCCAACAA ATACTCTTCC GGTATATTGA TGCCCACGTA CGGAGAGGAC 840
AATCGCTATG GATTTTATTT GAGGAATGGT GGATATTATT TTGCCTTCAG CGACTATATC 900
GATTTGGCAT TGCGTGGGGA GATCTTTTCC AAAGGGTCAT GGGGCATTTC AGCCCAATCG 960
AAATATAAGA AGAGGTATAA GTACAACGGC TCGTTCGAAG CCAATTATCT GGTATCGAAG 1020
TCCGGCGACA AATACGTGCC CGGAGACTAC AGCAAGACCA CCAGTCTGAA TATCCGATGG 1080
ACACACAGTC AGGATCCGAA GGCCAATCCT TTGCAAACGT TGTCGGCCAA TGTCAATTTT 1140
GCCACCGGGA GCTATTTCCA GAATTCGCTG AATACCACCT ATGATGTCAA TGCCCGTACT 1200
GCTACGACAC GAAGTTCGGC CGTGAGCTAT TCGCGCAAGT TTCCGGGTAC TCCTTTTTCG 1260
ATTACGGGTA GCATGGATAT CAGCCAGAAC ATGCGCGATA CGACGGTGAG CCTTACCTTG 1320
CCGAATCTTT CGATTAATAT GTCCACGCGT TATCCTTTCA AGCGGAAGAC CCGTGTAGGA 1380
CCGGAGCGAT GGTACGAGAA GTTGAGTGTG GGCTATTCCG GTCAGCTTCG CAATAGTATC 1440
TTGACAAAAG AGAAAGATTT GCTCCAGAGC AATCTCGTGC GCGATTGGAA GAATGGTATG 1500
CGTCATTCCG TACCGATCAG TTTGACTGTC CCTTTGTTGG ATTATATCAA TCTGACTATG 1560
GGGGTTAACT ACAATGAGTG GTGGTACACG AAAGGCATAC GGAAGTCGTG GAATGAGGAT 1620
AAGAAAACAT TCCTGCCTTC GGACACGACC TATAAATTCC GCAGACTGTA CGATTACAGT 1680
CTGTCGGCAG GCTTATCTAC CACATTGTAC GGTATGTTCA AGCCTTGGAA ACCTTTTTCC 1740
TTCGGAGGCA ATCTCATTAT GATCCGTCAT CGCTTCACGC CCACTGTCAG TTTCTCCTAT 1800
ATGCCGGACT TCACGAAACG CCGATATGGC TTTTGGGAGC TTCTTGAGCA TACGGATCAG 1860
AACGGCAAGC TGCATACGCT GCTCTACTCT CCTTATTTCG AGCAGATATT CGGTGCTCCC 1920
TCCATGGGCA ATGCAGGATC TGTCAATTTC TCTTTTGACA ACAACTTAGA GCCCAAGATC 1980
AAATCCAAAT CGGATTCGAC AGGGATCAAG AAGATCAGCC TGATAGATCA GTTCACATGG 2040
TCTACATCCT ATAATATGTT TGCCGATTCG ATCCGATGGA GCAATATCTC GGCTTCGCTG 2100

GCACTTCGCC TCTCCAAGAG CTTTACCTTG CGCTTGTCCG GTCTGTTCGA TCCCTATTTG 2160
ACGAAGTATT ATGAGGGAGA AGATGGGAAG ATCATTCCCT ATAAGAGCAA CGACCTGCGC 2220
ATTTTTAACG GCAAGGGATT GGCACGCCTG ATCAGTACGG GTACTTCTTT CAGCTATACG 2280
CTCAACAAAG AGTCGCTCAG CGGATTGATA GCTCTTTTCA GTGGCAAAAA GGAGCGGAGA 2340
GATGAAAAGA AAAACACAGG GGCTACTCCT CATGAAGGAG ACGATGCTGC CGATATACTT 2400
GAGGGAGGAA GACCGCAAAA TGAAAGTGGG GGGTCGCTCC TCGAGCGCAA CCGTCAGGGC 2460
GGAGCAGTGG ATCAGGATGG TTACTTCGCA TATTCGATCC CATGGAGCCT GTCCTTCGAC 2520
TATAGTTGGA ATATTGCTAC CGACTACAAT AGGTACAATG TCAATAAGAT GGAGCACTAC 2580
TACCGGGTAA CGCAGAATCT GAGCTTTCGC GGCAATATCC AGCCTACACC GAACTGGAGC 2640
TTCGGATTCA ATGCGAACTA CAATTTCGAC TTGAAGAAAA TAACATCGCT TACCTGCAAC 2700
GTCACTCGCG ACATGCACTG CTGGGCTATC TCGGCCAGTT TCATCCCTAT AGGAGCATAC 2760
AAGTCCTATA ATTTCGTCAT ATCGGTGAAG AGTTCACTCT TGCAGGATCT GAAGTATCAG 2820
CAGAGCAATC GTCCCATCAC GAATACTTGG TAT 2853

(2) INFORMATION FOR SEQ ID NO:220

(i) SEQUENCE CHARACTERISTICS:
(A) LENGTH: 3678 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...3678

(xi) SEQUENCE DESCRIPTION: SEQ ID NO:220

ATGATGAAAC GATATACAAT AATTCTTGCA GTTTTTCTTT TATTCTGCAC GGTATTTACC 60
TTTCAAATAA AAGCTCGCCC TTATGAAAGA TTTGCAGATG TAGAGAAGCC TTGGATTCAG 120
AAACATTCAA TGGATTCTAA ATTGGTGCCT GCAAATAAGG GTAACTTAAT TCAAGCTGAA 180
ATTGTATACC AATCTGTTTC TGAACATAGT GACTTAGTTA TTTCACCTGT GAACGAAATA 240
AGGCCTGCAA ATCGTTTCCC TTCGCATAGG AAGTCTTTTT TTGCAGAAAA TCTACGGGCA 300
TCTCCCCCCG TAGTTCCCGT TGCCGTCGAC AAGTATGCGG TACCGGTTGC CAATCCAATG 360
GATCCTGAAA ATCCCAATGC CTGGGATGTG ACGCTAAAAA TCACTACTAA AGCGGTAACA 420
GTACCTGTCG ATGTGGTGAT GGTTATCGAC CAGTCTTCGT CAATGGGAGG GCAAAACATT 480
GCCAGATTAA AGTCTGCCAT TGCATCGGGA CAGCGTTTTG TGAAAAAAAT GTTGCCTAAG 540
GGGACGGCTA CAGAAGGGGT GCGTATCGCT CTTGTGAGTT ATGACCATGA GCCTCATCGC 600
TTATCTGATT TTACCAAAGA CACTGCTTTT CTCTGTCAAA AAATCCGGGC TTTGACTCCT 660
ATTTGGGGAA CACATACCCA GGGGGGGCTT AAAATGGCGA GAAACATTAT GGCCACTTCT 720
ACTGCTGTGG ATAAGCATAT CATATTGATG TCTGACGGGT TAGCGACGGA GCAGTATCCT 780
GTTAAAAATG TAACTACTGC AGACTTCATT GGCAAAACTG GAAATGCGAA TGATCCCATT 840
GATTTGGTTA TACAAGGAGC AATTAATTTC CCTACAAATT ATGTTTCCAA CAATCCATCT 900
ACACCTCTTA CCCCAAATTA TCCAACTCAT TCTTCTAAAG TTGGACGGAG AAATCTGCCG 960
GAATCCAAAT TCGATTATAG TAATCTGAGT GCAAGGATTA CTTTTGATGG TGTTGCTGGC 1020
GCATTGGTCT ATGAACCGAG GTTTCCTCAT CCCTATTATT ATTATTTCCC TTGTAACGCT 1080
GCTATCAATG AGGCTCAGTT TGCGAAAAAC TCTGGTTATA CAATCCATAC TATTGGCTAT 1140
GACCTGGGAG ATTTTGCCTT GGCCAACAAT TCGTTGAAAC TAACCGCTAC AGACGAGAAT 1200
CACTTCTTTA CGGCGACACC GGCCAATTTA GCTGCAGCGT TTGATAATAT TGCCCAAACT 1260
ATTAATATAG GTATACAGAG GGGGGAGGTG ACGGACTTTG TAGCTCCTGG TTTCATCGTT 1320
AAAAATCTGA CGCAATCGGG AGATGTTACT CATTTGCTAA ATGTTTCAAA TGGAACGGTG 1380
CACTATGATG TCTCTACTAA AAAACTGACA TGGACTACTG GTACTATCCT GAGCTCATCA 1440
GAAGCTACCA TAACTTATCG TATTTATGCC GATTTGGATT ATATACAGAA CAATGATATT 1500
CCGGTAAATA CTACTTCTGC TATCGGCCCG GATCTTGGTG GATTCGATAC CAATACCGAG 1560
GCAAAATTGA CCTATACCAA TTCCAATGGC GAACCGAATC AGCAGTTAAT TTTCCCACGT 1620
CCGACGGTTA AGTTAGGTTA TGGTGTTATT AAGCGGCACT ATGTATTGGT AAATAAAGAC 1680
GGTCAACCCA TACAGGCAAA TGGAACAGTT GTCAGTTCCC TAAGCGAGGC TCATGTTCTA 1740
CAGTCACAAG ATTTCTTTTT GCCCTCAGGT GGAGGTCATA TTGTTCCCAA ATGGATAAAG 1800
TTGGACAAAA CGACCGAAGC ATTACAGTAC TATTCCGTAC CGCCGACTAA CACGGTCATC 1860
ACTACTGCCG ATGGTAAACG TTATCGTTTT GTCGAAGTCC CAGGCTCCAC GCCGAATCCG 1920
GGCCAAATCG GTATCAGTTG GAAAAAACCG GCAGGAAACG CTTACTTCGC TTACAAGCTC 1980
CTCAATTATT CGATGGGAGG AACAACAGAC CAACAGAGTG AATGGGATGT GACGTCCAAT 2040
TGGACAGGAG CCCAAGTACC GCTCACAGGA GAAGATGTAG AGTTTGCAAC GACAGAAAAT 2100
TTCGGTTCTC CGGCGGTAGC CGATTTGCAT GTCCCGACAA CCAACCCCAA AATTATCGGT 2160
AACCTTATCA ATAATTCCGA CAAGGATTTA GTTGTTACCA CAAGCAGTCA ATTGACGATC 2220

AACGGCGTGG TTGAGGATAA CAATCCGAAT GTCGGTACGA TCGTCGTGAA GTCGTCGAAA 2280
GACAATCCTA CGGGGACATT GCTTTTTGCC AATCCGGGCT ATAATCAAAA TGTAGGGGGG 2340
ACCGTCGAGT TTTACAATCA GGGATATGAT TGTGCCGATT GTGGTATGTA TCGCAGGAGC 2400
TGGCAGTATT TCGGTATCCC TGTCAATGAA TCAGGTTTTC CAATTAATGA TGTGGGCGGA 2460
AACGAGACCG TCAACCAATG GGTTGAGCCT TTCAATGGCG ATAAGTGGCG GCCAGCACCT 2520
TATGCACCTG ATACAGAGCT TCAAAAATTC AAGGGCTACC AGATCACGAA TGACGTGCAG 2580
GCACAGCCTA CGGGAGTTTA CAGCTTCAAG GGTATGATTT GTGTGTGCGA TGCCTTCCTG 2640
AATCTGACAC GCACGTCCGG TGTCAACTAC TCGGGCGCCA ACTTGATCGG CAACTCATAC 2700
ACTGGAGCCA TCGACATCAA GCAGGGTATT GTCTTCCCGC CGGAAGTCGA GCAGACGGTG 2760
TATCTGTTCA ACACGGGAAC ACGCGACCAG TGGCGTAAGC TTAATGGAAG CACGGTTTCA 2820
GGCTATCGAG CCGGTCAGTA CCTCTCTGTA CCTAAGAATA CAGCGGGTCA GGACAATCTT 2880
CCGGATCGTA TTCCATCGAT GCATTCCTTC TTGGTGAAGA TGCAGAACGG AGCGTCTTGT 2940
ACGTTGCAHA TCTTGTACGA TAAGCTGCTC AAGAACACGA CTGTAAACAA CGGTAATGGT 3000
ACGCAGATCA CATGGCGATC CGGCAACTCC GGATCGGCGA ATATGCCGTC ACTTGTGATG 3060
GATGTTCTTG GTAACGAGTC GGCCGACCGT TTGTGGATCT TTACCGATGG GGGTCTTTCT 3120
TTCGGATTCG ACAACGGCTG GGATGGTCGC AAGCTGACTG AAAAAGGTTT GTCACAACTT 3180
TATGCGATGT CTGACATCGG TAATGATAAA TTCCAGGTTG CAGGGGTTCC GGAGTTGAAT 3240
AACCTGCTGA TCGGCTTCGA TGCGGATAAG GATGGTCAAT ACACGTTGGA GTTTGCTCTT 3300
TCGGATCATT TTGCGAAAGG GGCTGTTTAC CTGCACGATC TTCAGTCAGG AGCCAAACAC 3360
CGTATTACGA ATTCTACGTC GTATTCATTC GATGCCAAGC GGGGAGATTC CGGGGCTCGT 3420
TTCCGCTTGT CATATGGATG TGATGAGAAC GTAGATGATT CGCATGTCGT GAGTACAAAT 3480
GGCCGTGAAA TTATAATTCT GAATCAAGAT GCTCTTGACT GCACTGTAAC CTTATTCACA 3540
ATAGAAGGTA AGCTTCTTCG CCGCTTGAAA GTATTAGCTG GTCATAGAGA AGTCATGAAA 3600
GTGCAGACCG GAGGGGCCTA TATTGTGCAT CTTCAAAATG CTTTCACTAA TGATGTGCAT 3660
AAGGTGCTTG TTGAGTAT 3678

(2) INFORMATION FOR SEQ ID NO:221

(i) SEQUENCE CHARACTERISTICS:
(A) LENGTH: 3675 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...3675

(xi) SEQUENCE DESCRIPTION: SEQ ID NO:221

ATGAAACGAT ATACAATAAT TCTTGCAGTT TTTCTTTTAT TCTGCACGGT ATTTACCTTT 60
CAAATAAAAG CTCGCCCTTA TGAAAGATTT GCAGATGTAG AGAAGCCTTG GATTCAGAAA 120
CATTCAATGG ATTCTAAATT GGTGCCTGCA AATAAGGGTA ACTTAATTCA AGCTGAAATT 180
GTATACCAAT CTGTTTCTGA ACATAGTGAC TTAGTTATTT CACCTGTGAA CGAAATAAGG 240
CCTGCAAATC GTTTCCCTTC GCATAGCAAG TCTTTTTTTG CAGAAAATCT ACGGGCATCT 300
CCCCCCGTAG TTCCCGTTGC CGTCGACAAG TATGCGGTAC CGGTTGCCAA TCCAATGGAT 360
CCTGAAAATC CCAATGCCTG GGATGTGACG CTAAAAATCA CTACTAAAGC GGTAACAGTA 420
CCTGTCGATG TGGTGATGGT TATCGACCAG TCTTCGTCAA TGGGAGGGCA AAACATTGCC 480
AGATTAAAGT CTGCCATTGC ATCGGGACAG CGTTTTGTGA AAAAAATGTT GCCTAAGGGG 540
ACGGCTACAG AAGGGGTGCG TATCGCTCTT GTGAGTTATG ACCATGAGCC TCATCGCTTA 600
TCTGATTTTA CCAAAGACAC TGCTTTTCTC TGTCAAAAAA TCCGGGCTTT GACTCCTATT 660
TGGGGAACAC ATACCCAGGG GGGGCTTAAA ATGGCGAGAA ACATTATGGC CACTTCTACT 720
GCTGTGGATA AGCATATCAT ATTGATGTCT GACGGGTTAG CGACGGAGCA GTATCCTGTT 780
AAAAATGTAA CTACTGCAGA CTTCATTGGC AAAACTGGAA ATGCGAATGA TCCCATTGAT 840
TTGGTTATAC AAGGAGCAAT TAATTTCCCT ACAAATTATG TTTCCAACAA TCCATCTACA 900
CCTCTTACCC CAAATTATCC AACTCATTCT TCTAAAGTTG GACGGAGAAA TCTGCCGGAA 960
TCCAAATTCG ATTATAGTAA TCTGAGTGCA AGGATTACTT TTGATGGTGT TGCTGGCGCA 1020
TTGGTCTATG AACCGAGGTT TCCTCATCCC TATTATTATT ATTTCCCTTG TAACGCTGCT 1080
ATCAATGAGG CTCAGTTTGC GAAAAACTCT GGTTATACAA TCCATACTAT TGGCTATGAC 1140
CTGGGAGATT TTGCCTTGGC CAACAATTCG TTGAAACTAA CCGCTACAGA CGAGAATCAC 1200
TTCTTTACGG CGACACCGGC CAATTTAGCT GCAGCGTTTG ATAATATTGC CCAAACTATT 1260
AATATAGGTA TACAGAGGGG GGAGGTGACG GACTTTGTAG CTCCTGGTTT CATCGTTAAA 1320
AATCTGACGC AATCGGGAGA TGTTACTCAT TTGCTAAATG TTTCAAATGG AACGGTGCAC 1380
TATGATGTCT CTACTAAAAA ACTGACATGG ACTACTGGTA CTATCCTGAG CTCATCAGAA 1440
GCTACCATAA CTTATCGTAT TTATGCCGAT TTGGATTATA TACAGAACAA TGATATTCCG 1500

GTAAATACTA CTTCTGCTAT CGGCCCGGAT CTTGGTGGAT TCGATACCAA TACCGAGGCA 1560
AAATTGACCT ATACCAATTC CAATGGCGAA CCGAATCAGC AGTTAATTTT CCCACGTCCG 1620
ACGGTTAAGT TAGGTTATGG TGTTATTAAG CGGCACTATG TATTGGTAAA TAAAGACGGT 1680
CAACCCATAC AGGCAAATGG AACAGTTGTC AGTTCCCTAA GCGAGGCTCA TGTTCTACAG 1740
TCACAAGATT TCTTTTTGCC CTCAGGTGGA GGTCATATTG TTCCCAAATG GATAAAGTTG 1800
GACAAAACGA CCGAAGCATT ACAGTACTAT TCCGTACCGC CGACTAACAC GGTCATCACT 1860
ACTGCCGATG GTAAACGTTA TCGTTTTGTC GAAGTCCCAG GCTCCACGCC GAATCCGGGC 1920
CAAATCGGTA TCAGTTGGAA AAAACCGGCA GGAAACGCTT ACTTCGCTTA CAAGCTCCTC 1980
AATTATTGGA TGGGAGGAAC AACAGACCAA CAGAGTGAAT GGGATGTGAC GTCCAATTGG 2040
ACAGGAGCCC AAGTACCGCT CACAGGAGAA GATGTAGAGT TTGCAACGAC AGAAAATTTC 2100
GGTTCTCCGG CGGTAGCCGA TTTGCATGTC CCGACAACCA ACCCCAAAAT TATCGGTAAC 2160
CTTATCAATA ATTCCGACAA GGATTTAGTT GTTACCACAA GCAGTCAATT GACGATCAAC 2220
GGCGTGGTTG AGGATAACAA TCCGAATGTC GGTACGATCG TCGTGAAGTC GTCGAAAGAC 2280
AATCCTACGG GGACATTGCT TTTTGCCAAT CCGGGCTATA ATCAAAATGT AGGGGGGACC 2340
GTCGAGTTTT ACAATCAGGG ATATGATTGT GCCGATTGTG GTATGTATCG CAGGAGCTGG 2400
CAGTATTTCG GTATCCCTGT CAATGAATCA GGTTTTCCAA TTAATGATGT GGGCGGAAAC 2460
GAGACCGTCA ACCAATGGGT TGAGCCTTTC AATGGCGATA AGTGGCGGCC AGCACCTTAT 2520
GCACCTGATA CAGAGCTTCA AAAATTCAAG GGCTACCAGA TCACGAATGA CGTGCAGGCA 2580
CAGCCTACGG GAGTTTACAG CTTCAAGGGT ATGATTTGTG TGTGCGATGC CTTCCTGAAT 2640
CTGACACGCA CGTCCGGTGT CAACTACTCG GGCGCCAACT TGATCGGCAA CTCATACACT 2700
GGAGCCATCG ACATCAAGCA GGGTATTGTC TTCCCGCCGG AAGTCGAGCA GACGGTGTAT 2760
CTGTTCAACA CGGGAACACG CGACCAGTGG CGTAAGCTTA ATGGAAGCAC GGTTTCAGGC 2820
TATCGAGCCG GTCAGTACCT CTCTGTACCT AAGAATACAG CGGGTCAGGA CAATCTTCCG 2880
GATCGTATTC CATCGATGCA TTCCTTCTTG GTGAAGATGC AGAACGGAGC GTCTTGTACG 2940
TTGCAAATCT TGTACGATAA GCTGCTCAAG AACACGACTG TAAACAACGG TAATGGTACG 3000
CAGATCACAT GGCGATCCGG CAACTCCGGA TCGGCGAATA TGCCGTCACT TGTGATGGAT 3060
GTTCTTGGTA ACGAGTCGGC CGACCGTTTG TGGATCTTTA CCGATGGGGG TCTTTCTTTC 3120
GGATTCGACA ACGGCTGGGA TGGTCGCAAG CTGACTGAAA AAGGTTTGTC ACAACTTTAT 3180
GCGATGTCTG ACATCGGTAA TGATAAATTC CAGGTTGCAG GGGTTCCGGA GTTGAATAAC 3240
CTGCTGATCG GCTTCGATGC GGATAAGGAT GGTCAATACA CGTTGGAGTT TGCTCTTTCG 3300
GATCATTTTG CGAAAGGGGC TGTTTACCTG CACGATCTTC AGTCAGGAGC CAAACACCGT 3360
ATTACGAATT CTACGTCGTA TTCATTCGAT GCCAAGCGGG GAGATTCCGG GGCTCGTTTC 3420
CGCTTGTCAT ATGGATGTGA TGAGAACGTA GATGATTCGC ATGTCGTGAG TACAAATGGC 3480
CGTGAAATTA TAATTCTGAA TCAAGATGCT CTTGACTGCA CTGTAACCTT ATTCACAATA 3540
GAAGGTAAGC TTCTTCGCCG CTTGAAAGTA TTAGCTGGTC ATAGAGAAGT CATGAAAGTG 3600
CAGACCGGAG GGGCCTATAT TGTGCATCTT CAAAATGCTT TCACTAATGA TGTGCATAAG 3660
GTGCTTGTTG AGTAT 3675

(2) INFORMATION FOR SEQ ID NO:222

(i) SEQUENCE CHARACTERISTICS:
(A) LENGTH: 1275 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1275

(xi) SEQUENCE DESCRIPTION: SEQ ID NO:222
   ATGGAAGTGA AGAAAAACAC AGTGGTGCTA CGCCTTCTGA TTTGGTTCGT GGCCATTCTT 60
   CTCTTCCACT CCTCACGGCT GTGGGGACAG GAAGGGGAGG GGAGTGCCCG ATACAGATTC 120
   AAAGGATTCG TGGATACCTA CCATGCCGTA CGCAGCTCTT CTCCTTTTGA TTTCATGAGC 180
   TCGCGTACGA GAGTGAGAGG TGAGCTGGAG AGGTCGTTCG GTAATTCGAA AGTAGCCGTA 240
   TCGGTCAATG CCACCTACAA TGCTCTACTG AAAGACGAGA CCGGCTTACG TTTACGTGAA 300
   GCCTTCTTCG AGCATCAGGA AGAGCATTGG GGGTTGCGCC TCGGACGACA GATTGTCATT 360
   TGGGGGGCTG CCGACGGTGT GCGCATCACG GATCTGATCT CCCCGATGGA TATGACCGAG 420
   TTTCTGGCAC AGGATTACGA TGATATTCGT ATGCCGGTCA ATGCATTGCG TTTCTCTGTC 480
   TTCAACGAAT CGATGAAAGT GGAAGTCGTG GTACTGCCTG TATTCGAGGG GTACCGTCTG 540
   CCTGTGGATC CTCGCAATCC TTGGAATATC TTCTCCCTTT CGCCCATTGC TCAGGGGATG 600
   AATATCGTCT GGAAAGAAGA AGCCGGCAAA CCGGCCTTCA AGGTTGCCAA TATCGAGTAC 660
   GGTGCGCGAT GGAGCACTAC GCTCTCCGGT ATCGACTTCG CTTTGGCTGC ATTGCATACA 720
   TGGAACAAGA TGCCCGTCAT CGAAGTACAG GGCATTGTGC CGACGGAAAT CATCGTTAGC 780
   CCTCGCTATT ATCGTATGGG ATTTGTCGGC GGCGACCTCT CCGTACCCGT CGGACAGTTT 840
   GTTTTCAGGG GAGAGGCTGC GTTCAATATC GACAAACACT TCACCTATAA GAGTCATGCC 900
   GAGCAAGAGG GTTTCCAAAC AATCAATTGG TTGGCCGGAG CCGATTGGTA TGCTCCCGGT 960
   GAATGGATGA TCTCAGGACA ATTCTCAATG GAAAGCATAT TCAGGTATAG GGATTTCATC 1020
   TCCCAAAGAC AACATTCTAC CCTGATTACT CTCAATGTTT CCAAGAAATT CTTCGGCAGT 1080
   ACACTCCAAC TTTCGGACTT CACCTACTAC GACCTTACGG GCAAAGGATG GTTCAGTCGC 1140
   TTTGCAGCTG ACTATGCCTT GAACGATCAG ATACATCTGA TGGCCGGATA TGACTGGTTC 1200
   AGTAGTAAGG GCAGCGGTAT ATTCGATCGC TACAAAGACA ATTCCGAACT CTGGTTCAAA 1260
   GCCCGCTACA GCTTC 1275

(2) INFORMATION FOR SEQ ID NO:223

(i) SEQUENCE CHARACTERISTICS:
(A) LENGTH: 1212 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1212

(xi) SEQUENCE DESCRIPTION: SEQ ID NO:223

ATGTCCTCCT GTGAGGTGGC TTATTTTTCA CTAAAGCCGA TCGATCTGCA GAACATCCGC 60
GAACGGAATC ACTCTTCCGA CATCGCGCTT TCCAATTTAT TAGACAATTC GAATCAGCTA 120
TTAGCTACTA TTCTGATCGG GAATAATGTG ATTAATGTAG CCATCGTTAT CCTTTCCAAT 180
TATGCCATCG AGCAGACATT CGTTTTCTCT TCTCCGATCA TTGGATTTCT GATCCAGACG 240
ATACTCCTGA CCACTGTTCT TTTGCTGTTC GGAGAGATTC TGCCGAAAGT GTATGCGCGG 300
AAGAATCCGC TGCAATACTC GCGCTTTTCT GCTGCAGCTA TGTCCGTTAT CTATAAGATA 360
TTGTCACCGT TTTCAAAATT GCTGGTCAAA AGTACCGGCA TCGTTACCAG AGGTATCAGC 420
AAGAAGAAAT ACGATATGTC CGTGGATGAG CTCTCGAAAG CGGTAGCCCT CACCACTACG 480
GAGGCAGAGC CGGAGGAGAA AGAAATGATT AACGAAATCA TCAAATTCTA TAATAAGACA 540
GCCTGCGAAA TCATGGTTCC GCGTATCGAT ATTGTGGATG TGGATCTGAG CTGGCCATTT 600
CGTAAGATGC TTGACTTCGT TGTTTCGTCG GGTTATTCCA GACTTCCCGT TTCAGAGGGG 660
TCAGAAGACA ATATCAAAGG GGTGATTTAC ATCAAAGATC TAATCCCACA CATGGATAAA 720
GGCGATGAAT TCGACTGGCA TCCTCTGATT CGTAAAGCAT ATTTTGTCCC CGAAAACAAG 780
CGCATAGATG ATTTGCTCGA GGAGTTCAGA GCCAATAAGG TGCATGTCTC CATCGTTGTG 840
GATGAGTTCG GTGGCACTTG CGGACTGATC ACAATGGAGG ACATATTGGA AGAGATCGTC 900
GGCGAGATTA CGGACGAGTA CGATGAGGAA GAACTCCCCT TTAAGGTTTT GGGGGATGGC 960
AGTTATCTTT TCGAAGGAAA AACGTCTCTC TCCGATGTTC GACACTATCT TGACCTTCCG 1020
GAAAATGCTT TCGGTGAATT GGGGGACGAG GTAGATACGC TAAGTGGGCT CTTCTTGGAA 1080
ATCAAGCAGG AACTCCCCCA TGTGGGCGAT ACAGCAGTGT ACGAGCCATT CCGCTTTCAA 1140
GTGACCCAAA TGGACAAGCG CCGAATCATC GAAATCAAGA TTTTCCCTTT CGAGCGCACT 1200
TGGGAGGTCG AA 1212

(2) INFORMATION FOR SEQ ID NO:224

(i) SEQUENCE CHARACTERISTICS:
(A) LENGTH: 780 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:

(A) NAME/KEY: misc_feature
(B) LOCATION 1...780

(xi) SEQUENCE DESCRIPTION: SEQ ID NO:224

ATGAAACTAT TACTTTATCT CCTATTGGTC TTGTCGACTC TATCCCCGAT GTATTCGCAA 60
ATGCTCTTCT CAGAGAATCT CACAATGAAT ATAGACAGCA CCAAAACCAT ACAAGGAACG 120
ATATTGCCCG TACTGGATTT CAAAACCGAA AAGGAAAATG TGTTCACCTT CAAAAATACT 180
GCCAATCTCA ATCTGCTGAT AAAGCACGGT CAAGTAATCA ACTTAATTAA TAAGCTTGAG 240
TTTTCTACCT ATGGCAATAA AGTAACCGTA AGTGGAGGAT ATGTACACAC CGAATACCGC 300
TATTTGTTGC ATCATGTTTT TGAGGTTTAT CCTTATGTCG AGTCGCAATG GGCAGAAAGT 360
ACAGGAATGA AATATAAGGT TTCTACGGGA TTACAGTCGC GTTATCGGCT GGTAAATAGT 420
GATAACTGTC TCATGTTTGC AACATTGGGG GTATTTTTCG AATTCGAAAA GTGGGAACAG 480
CCAGCCACTA GCCTCTTTGC AGGAACGTAT GCATACAGCC GAAGTATCAA AAGCCACCTG 540
TCTATCAGTT TCAGACATCG GTTGGGTGAA CATTGGGAAT TTACAACTAC GGCTATTCAC 600
CAGGAAAAGC CTGACAGTTA TTTTAAGAAG GCACGTTTTG GAGGAGCTAT CGACCTCAAA 660
TACCATATCA CACCTACGAT AGGAATACGC GGGGCCTATC GGATCATCTA CGATACTGCC 720
CCTATTGTAC CTGTGCGGAA AGATTACAAC ACCGTTGATG TTGGTATCGA TATTTCGTTT 780

(2) INFORMATION FOR SEQ ID NO:225

(i) SEQUENCE CHARACTERISTICS:
(A) LENGTH: 2502 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2502

(xi) SEQUENCE DESCRIPTION: SEQ ID NO:225

ATGAAACGAA TCGTTTTATC ATCTTTCCTG TTCGTTCTGT CCATACTTTC TTTGATGGCA 60
CAGAACAATA CCCTCGATGT ACACATATCC GGTACGATCA AGGATGCCTC CTCCGGCGAA 120
CCAGTGCCCT ATGCCACTGT AAGCATCCGG CTGACAGGAG CAGATACCAC ACAGGTGTTC 180
CGACAAGTGA CTGACGGCAA CGGCTACTTC GTCATAGGCC TGCCGGCAGC TCCCTCCTAT 240
CACCTGACAG CTTCGTTCGT AGGTATGAAA ACCCATACCA TGCAGATTAG TCGGGGAAAT 300
GGACAGCACG ACATCAAATC CATCGACATT TCTCTCGAAT CCGAGGACAA ACAACTCTCC 360
ACCGTCACCG TATCGGCAGC ACGACCACTG GTGAAGATGG AGATAGACCG CCTGTCCTAT 420
AATATGAAAG ATGACCCCGC AGCCAAGACG AACAACCTGC TCGAAATGCT GCGCAACGTT 480
CCTTTGGTAA CGGTGGATGG TCAGGGCAAT ATCCAGGTGA AAGGATCTTC CAACTTCAAA 540
ATCCACCTCA ATGGCAGGCC CTCGACCATG GTGAGCAGCA ACCCGAAGGA GGTCTTTCGC 600
TCCATTCCTG CCCATACGAT CAAACGGGTG GAGGTCATCA CCGATCCGGG TGTAAAGTAC 660
GATGCGGAAG GCACAAGTGC CATCCTGGAC ATCGTCACGG AAGAAGGTAA GAAGCTGGAA 720
GGATATTCAG GTTCCATCAC GGCCAGTGTC ACCAACAATC CCACAGCCAA CGGTAGTATC 780
TTTCTGACGG CAAAGTCCGG CAAAGTCGGG CTGACTACCA ACTATAACTA CTACGGTGGC 840
AAAAACAAGG GCTCTCGCTA CTTTACCGAA CGTACTACAT CCATGCTCCA AACGATAGAA 900
GAAGGCAAAG GGCAAGAAAC CTTTGGCGGA CACTTCGGCA ATGCCCTCCT CTCATTCGAG 960
ATAGATTCGC TCAATCTCTT TACGGTGGCC GGCAATGTAC GCCTTTGGGA GATGACCACC 1020
GACCGGAACA GCGTAGAAAA AAGCTTTGCC GGCAGCAACC TCATGTCCTA CATAGACAGA 1080
AAACTCAAAA CACAGATGGA TGCCGGATCA TACGAGCTCA ATGCCGACTA TCAGCACAGC 1140
ACTCGCCTGC CGGGCGAATT GCTCACCGTT TCCTACCGCT TCACTCACAA TCCTAATAAT 1200
AGCGAGACCT TCATTGACCA ATGGAAGCGC GATCCGCTCA ACACAGCTAA TACGATCCAG 1260
TACGCCGGCC AGCACTCCAA ATCCGATGCG GGCATGGACG AACATACGGC ACAAGTGGAC 1320
TATACACGTC CCTTAGGACA AGCACATTCT TTGGAAGCAG GGCTGAAGTA CATCTATCGT 1380
CATGCCACGA GCGATCCTCT CTATGAGATA CGACCATCCG AAGATGCTCC GTGGCAGCCC 1440
GGCTCTCTAT ATGCACAGAA TCCGTCGAAC GGAAAGTTCC GCCACGATCA ATACATCGGA 1500
GCAGCCTATG CCGGCTACAA CTATCGTAAG GATCAGTATT CTTTGCAAAC CGGCCTCCGA 1560
GTGGAAAGCA GCAGGCTGAA AGCACTCTTT CCCGAAAACG CAGCAGCAGA TTTCTCCCAC 1620
AACTCGTTCG ACTGGGTGCC ACAGCTCACG CTCGGCTATA CCCCCTCGCC CATGAAGCAG 1680
CTTAAGCTGG CCTATAACTT CCGAATCCAA CGTCCTGCAA TCGGCCAACT GAATCCCTAC 1740
CGGCTACAGA CCAACGATTA TCAAGTACAG TATGGTAATC CCGACCTAAA GTCGGAGAAG 1800
CGTCACCACG TCGGTCTCTC CTATAATCAA TACGGAGCCA AGGTCATGCT TACAGCATCG 1860
CTCGACTACG ACTTCTGCAA CAACGCCATC CAGAATTACA CCTTCTCCGA CCCGGCCAAT 1920

CCCAATCTCT TCCACCAGAC CTATGGCAAT ATCGGACGAG AGCATTCTTT CAGCTTGAAT 1980
ACCTATGCCA TGTACACGCC GGCCGTATGG GTCAGGATTA TGCTCAACGG AAATATCGAT 2040
CGCACATTCC AAAAGAGCGA AGCACTCGGC ATTGATGTCA ATTCATGGTC CGGCATGGTA 2100
TACTCAGGCC TGATGTTCAC CCTGCCGAAG GATTGGACTG TGAATCTCTT CGGAGGTTAT 2160
TATCATGGGG GAAGAAGCTA CCAGACGAAG TATGATGGCA ATGTATTCAA CAATATCGGT 2220
ATAGCCAAAC AGCTTTTCGA CAAAAAATTG AGAGTCTCGC TGAGCGCAAA CAACATTCAT 2280
GCGAAGTATT CGACATGGAA GAGCCGGACC ATCGGCAATG GATTTACTAT TTATTCGGAA 2340
AATGCCGGTA TACAACGGAG TGTTTCCCTC AGCCTCACCT ACAGCTTCGG TAAGATGAAT 2400
ACACAAGTGC GCAAGGTAGA GCGTACGATC GTCAACGACG ACCTCAAGCA AACCTCATCC 2460
CAAGGACAGC AGGGTGGCGG ACAAGGAAAT CCTACCGGCA AT 2502

(2) INFORMATION FOR SEQ ID NO:226

(i) SEQUENCE CHARACTERISTICS:
(A) LENGTH: 1197 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1197

(xi) SEQUENCE DESCRIPTION: SEQ ID NO:226

ATGAGACTCT CTGCCATTCT TATCGCTTTG ATTGTGATGC TGCCTGCTGT GCTTAGCGGG 60
CAGCATTATT ATTCCATGGC GGGAGAGCGA CTGGAGACGG ACAGCATTCG TCCGAACGAA 120
CTCTCGGCAT CGATCCGAAG TGCGCTTTTC TTTCGGAACA ATGAATACAA TGCACGTTCG 180
GTCAAAGGTT ATACGTTGCC GGGTGCACGG GTTTCCGCTT TTGCCTCTTA CTCGCTGCCG 240
GCAGCACATG GTGTGAAGCT TTCGCTCGGA GTATCTACCC TGAACTACTG GGGGGCAAGT 300
CGCTATCCGG CCGGTATCGC TTATTCCGAT TTACCTTATT GGACGGACTA TAACGACTAT 360
GTACGCTTGC GTATCCTGCC TTATGTACAG GCCATGCTGA AGCCGACGGC CACGACTGCT 420
CTCATGCTGG GCAATATAGC CGGTGGTACG GCTCACGGAC TGATCGAACC GATCTACAAT 480
CCTGAGTTGG ATTTGACGGC TGATCCTGAA GCCGGTGTGC AATTTCGGGG TGATTGGACA 540
CGTTTCCGAA TGGATGTTTG GGTCAATTGG ATGAGCATGA TTTTCAAAAA TGACAATCAT 600
CAGGAGTCGT TTGTCTTTGG CTTGTCCACT ACTTCGAAAT TGTTATCGGC TGAAGGCAAA 660
TGGCGACTCG AACTGCCCTT GCAGGCTATT GCCACGCATC GCGGCGGGGA ATACAACTGG 720
GCGCAGCAGG ATACCGTGCA TACATGGGTC AATGGAGCTG TCGGACTTAA GCTTTCGTAT 780
CGCCCTCGTA CCGACAAACC CATGCAGATT TGGGGATCTG CTTATGGTGT GGCAGCCTTG 840
TCAAGCGGAG GATACTTCCC TTACGAAAGA GGGTGGGGCG GTTATCTTTC TCTCGGAATG 900
GACTTGGAGC ACTTCGCTTT TCGTACCGAC TATTGGTACG GCAGGCATTA CGTTTCTCCC 960
TTTGCTGCAC CTTTCGCCAA TTCCCTGACG TATGACAAAC AGCCTCTTAC GAACGGTTGG 1020
GGCGATTATA TTCGTCTCTA TGCCGACTAT TCGTGGCGGA TGGCACGAAG TGTTTCGTTG 1080
GCGGCTGTTG CTCGGGTATG GTTCCAGCCT TCGGATCGTT TTGCGATGAG CCACGCCTTG 1140
GAACTGACGA TGCGTATCGA TCCCAAATTC CCAATAGCTT TTCTGAAAGG CAATCAT 1197

(2) INFORMATION FOR SEQ ID NO:227

(i) SEQUENCE CHARACTERISTICS:
(A) LENGTH: 1146 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:

(A) NAME/KEY: misc_feature
(B) LOCATION 1...1146

(xi) SEQUENCE DESCRIPTION: SEQ ID NO:227

ATGAACAAAT CGCTATTATC ATTGGCATGC CTCATCCTGT GCGGTATGCC GGCCATCGCC 60
CAACAGACAG GACCGGCCGA ACGCAGCGGC GAGCCTTCTC TGGCCGAACG TGTATTCGGT 120
CTGGAGCAGA AGCAGAAAAA GCTGAAGGTG TACTTAGGCA TACAGTCGTT CTACGACCAG 180
CCGCTTGTCG ATGACGAATC CCATATCGGA CACTTCAAGG TACAGGAGCT GCGGATGTCT 240
GCTCATGGCG AACTGAACCG CCACCTCAGC TTCGACTGGC GACAACGTCT CAACCGTGCC 300
GCCGACGGCA CTTCGTTTGC CGACAATCTC TCCAATGCCA TCGACATCGC AGGTGTGGAC 360
TGGCACCCGA ACGACAAGGT GTCTTTCTTC TTCGGACGTC AGTACGCGCG TTTCGGAGGG 420
ATAGAATACG ACATGAACCC CGTAGAGATC TACCAGTACA GCGACCTTGT GGATTACATG 480
ACCTGCTATA CTTCGGGCGT GAACTTCGCA TGGAACTTCC ACCCCGAACA GCAGCTGCAG 540
CTACAGGTAC TCAATGCTTA CAACAACCGC TTCGCCGACC GCTACCACGT GACACCCGAT 600
GTCGCTACCG CCACGAGCTA CCCGCTCCTC TACTCGGCAC AGTGGAACGG TACCCTCCTC 660
GGAGGAGCAC TGCATATGCG TTACGCCGTG TCGATGGCTC ATCAGGCCCA AGAGCGTAAT 720
ATGTGGTACT TCACTGCGGG CAACCTGTTC AATCCGGGCA AACGGATCAA CGGATACCTC 780
GACCTCACCT ACTCGATCGA GGGATTGGAC GACAAAGGCA TTATGACTGC TCGCTACGGC 840
AAGGGCAAGA CCCTCACGGA CGTCAAGTAC TATGCTCTGG TATCGAAGTG GAACTTCCGC 900
ATTTTCGATC AGGTCAATCT CTTCCTCAAA GGCATGTACG AGAACGGCTA TGCGCCTGCC 960
CAATACGGCG AGAGCAGCCA CACGCGCCAC TCCTACGGCT ATATGGGAGG GGTGGAATAT 1020
TACCCTACGG AGACCAACTT CCGTCTGTTC GTCACCTACA TAGGACGGCA TTACCGGTAC 1080
AGTGCGACCG AGACGGAAAG CACCAATGCT CTTCGCGCCG GTCTGATCTA TCAGATACCT 1140
TTCTTA 1146

(2) INFORMATION FOR SEQ ID NO:228

(i) SEQUENCE CHARACTERISTICS:
(A) LENGTH: 666 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...666

(xi) SEQUENCE DESCRIPTION: SEQ ID NO:228

ATGAAACATT TGTTTAAGTC GACATTAGTA CTTCTTTGTG CTCTTTCTTT TTCCGGTACC 60
TATACCTTTG CACAAGAAAA TAATACAGAA AAGTCACGAT TTGATTTTTC TGTTAGGCTG 120
GGACAGGGAT ATATTGCAGG TTCAACTACC AACCTGATGT ATGGGTATAC ATCTGCTAAC 180
GATAGACTTT TGTCTGGTGC AATTTATCTG GGCTTGACAC CAAGTAAGAA AGAAAATGCA 240
ACCGGCGTAG CATTTCGTTT CTTATCNCCC TCTCCGGGTT ATTATGTCGA TATATCCGGC 300
AAAGAAAATA CCTTGAATTA TGCGTTTTAC GTTGTCGGAG CATATAATAG AATAGCCATT 360
CCTATACGCC CTATCAAAAA TTTTAATTTC ATCTTCTCTA CAGAAGTCGG AATGGCTTGG 420
ATCAGTCGTC ATGAGCAAAT TTACAATTCT ACTTCGCAGA CTTGGGATAA GCAGCGCAAG 480
TCGAGGTCGG GACTGGATTT TGGTCTCGGG ATGCATCTGC AATNCCACAT TAATAAGACC 540
GTTTACTTTA TGGCAGGAAC CGATCTTACG TCTTGCATGT TCGGAAAAAG GATCAATGAC 600
TACCAGCAAA AGGATCGAAC CTTCATTGCA CTTATCGACA ACAGTATTGG CATAGGATTA 660
AACCTC 666

(2) INFORMATION FOR SEQ ID NO:229

(i) SEQUENCE CHARACTERISTICS:
(A) LENGTH: 1173 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1173

(xi) SEQUENCE DESCRIPTION: SEQ ID NO:229

ATGATTATCA AGAAAATGCT GAAAAATAAA TTGGCCCCCT TGGCCATACT GTTCCTTTTT 60
GCTCCAAAGG CTATGAAGGC TCAGGAGCAA CTGAATGTGG TACACACCTC TGTGCCATCG 120
CTGAATATCA GTCCGGATGC ACGTGCGGCC GGTATGGGGG ATATAGGTGT GGCAACGACG 180
CCGGATGCGT ATTCACAGTA TTGGAATCCG AGTAAATATG CTTTCATGGA TACGAAAGCC 240
GGTATTAGCT TCTCATATAC ACCCTGGCTG TCCAAGCTGG TCAATGATAT TGCCCTGATG 300
CAGATGACCG GTTTCTACAA ATTGGGAACA GACGAGAATC AGGCTATTAG TGCTTCTCTG 360
CGTTATTTCA CATTAGGAAA GTTGGAGACT TTCGACGAAT TGGGCGAATC CATGGGAGAG 420
GCCCATCCCA ATGAATTTGC TGTCGATTTG GGCTATAGCC GCCAGTTGTC GGAGAACTTC 480
TCCATGGCTG TTGCACTGCG TTACATCCGC TCAGACCAAA GCACTCACAA CACCGGAGAG 540
AATCAGGCCG GAAATGCCTT TGCGGCGGAT ATAGCCGGTT ATTTGCAGAA GTATGTGCTA 600
CTGGGTAATG CGGAGAGCTT GTGGTCGTTG GGTTTCAACG TAAAGAATAT CGGAACGAAG 660
ATCTCCTATG ACGGAGGTGT CACGAGTTTT TTCATCCCTA CTTCGTTGAA TCTCGGGACG 720
GGGCTGTTGT ATCCGATCGA TGACTATAAC AGCATCAATT TCAACCTTGA ACTTAGCAAG 780
CTGCTTGTAC CCACTCCTCC TATCATGGAT CAAAACGATC AGGCCGGGTA TGAGGCTGCA 840
CTCAAGAAAT ATCAGGAAAC TTCTTCGATC AGCGGTATAT TCTCTTCTTT CGGTGATGCG 900
CCGGGAGGAC TCAAGGAAGA ATTCCGTGAG ATTACATGGG GACTTGGGGC TGAATATAGC 960
TATGACGATA AATTTTTTGT TCGTGCCGGA TATTCATACC TGCACCCCAC CAAAGGCAAT 1020
TTGCAGTACT TCACGGCCGG TGCCGGCTTC AAAATGAACA TATTCCGTAT CGATGCTTCC 1080
TACCTGTTGT CTACGATCCA GAGTAATCCG TTGGATCAGA CTCTGCGGTT TACGCTTGCT 1140
TTCGATATGG ATGGATTGCG CAATTTGTTC CAC 1173

(2) INFORMATION FOR SEQ ID NO:230

(i) SEQUENCE CHARACTERISTICS:
(A) LENGTH: 1338 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1338

(xi) SEQUENCE DESCRIPTION: SEQ ID NO:230

ATGAAAACAA CAGTTCAACA AATTATTCTG TGCCTGGCTT TAATGATGTC AGGTGTATTG 60
GGCGGAAACG CACAGAGCTT TTGGGAAGAA ATAGCTCCTC CTTTTATCAG TAATGAGCCT 120
AACGTCAAGT ATATAATTCC CAATATGGGG ATTGATTCAA AGGGAACAAT CTATGTAACC 180
GTGACAAAAA GGATTCAGCA GGGAGCAAAT TATACTTCTG AGCAATTGGG TATGTACTAT 240
CGACCATTAG GTGATAATGA ACAGTGGTGG AAACATGATC CGTATTTTGA TGACAAGATA 300
GTTGCGGATA TTCAGACAGA TGCATATGGC AGAGTTTATG TATGTACGAC TTCTTCTCGA 360
GATCAAGAGT ATCAACTTTA TATAAACGAG CAGAACGAAT GGAGGTGTAT ATTCAAAACT 420
TCTGTGTCTA CATATGAGCA TGGTATGGCT GTTTTTCGCT CTTCGACAGG GGTGACTTAT 480
ATAGGTACCA GGCATCACAT CTTCGCATCA GGTGTAAATG ATTTCGAGTT CAACACTATC 540
TATGAAGACT CTACACCTAT GAGCTGTCGC TTTGCAGACG CTACGAATAG TGGCACCATC 600
TATCTGGCAT TGATGCATGA AACCACAATG TCTACGACTA TCCTTACTTA TCAAAACGGT 660
GAGTTCGTCG ATATCTCGGA AAGTGAATTG AGTAACTCGA TTATTGCATC CATGTGCTCT 720
AATAAAGAAG GTGATATAAT AGCTCTTGTT ACTTCATATA CAGGATTTAT GAGTGGAACC 780
CTTGCGATCA GAAAAGCAGA TGAAGGCAAA TGGCAACTTG TTGGCGGAGA TATACAGAAT 840
GCGATCGTTC AAAATATATG CATGATGGAC GACAACAAGA TTGCTTGTGA AGTCTTCGGG 900
ACTCCTAACG GAGTAGATGG TCGGACAAGG GTTTGTGTTT CTGACGCATC TGTCTTTGAT 960
TTTGAGTGGT ATGAAGATGA AATATACGGA GGCCTGATAT TTGACACTTT CTTCTATAGC 1020
CCTTGGGACA AACTTCTTTA TGCGAAATTT GGTGGGATTA TGCTCAGGAG TAAAGAGTCT 1080

TTTATAACCT CTTTCATTTC TCCGACAGTT GTACAAGGAG TGGATGTCTA TACTTTGGCC 1140
GGGAAGATAA GGATCGAAAG TGAAACTCCG GTGTCTGAGG TGTTGCTTTT CGACCTGGCT 1200
GGCAGGATGG TACTTCGGCA AACCATTGAT AATAAAATCT ATTCGGACAT AGATACTAAC 1260
GGACTAAAGC GAAGCGGTAT TTACGTAGTC TCGGTGCGGC TCTCTTCCGG ACAGGTATTC 1320
AGTCATAAGG TGCAGGTA 1338

(2) INFORMATION FOR SEQ ID NO:231

(i) SEQUENCE CHARACTERISTICS:
(A) LENGTH: 924 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...924

(xi) SEQUENCE DESCRIPTION: SEQ ID NO:231

ATGATAATCC GGTGTCTTAT CCGTCGTCCG AGAACCGTCC TGTTCGGGTT GATATTCGTG 60
GTAGGTCTTT TCTCTGCGAT GGCGCAAGAG AAAAAGGATA GTCTCTCTAC GGTTCAGCCA 120
GTGCCGAATA GCAGCATGGT GGAGCAGACC CCTCTTCTCT CCATTGATCA CCCCGTCCTG 180
CCCGCTTCTT TTCAGAATAC CCGTACACTG AAAAGGTTTA GAGACAAACA TCTTTCCGAT 240
GCTTTGCTCA ATGGATTGAA GCCTCATCGC TCATCTTTGC AATTGAATGA GGAACTCAAC 300
TTCGCGGCAG AGCGTCGGGA TTTCGTTTCT CCCCTCTTGC AAACTCGCCA CGCTGCCGGT 360
GTCCTTTCAT GGCGACCGAC CGATAGGATG CATTTTTATA CATCGGGCAA TATCGGTCTT 420
GGCCATGATT TATTGACCGG TGTGCGCAAG GACTTCGGAT GGAATGCTGG TGCCGACTTC 480
TTGCTGAGTC AAAATCTTAC GGCACATGTC CAAGGCGGTT GGCAGCAGAA TTTCGGCTTT 540
ATACCTATGA CGGCTGTCAA TGGCCAACTG CGTTGGCAAG CCACCGAGAG ATTGAGTTTT 600
ACCACCGGTA TCGATTATCG ACAGGTACAG TGGAATGCTT TCGATAATAG AACGTTCTCG 660
CTTAAAGGAA GTGCTCGATA CGAAGTGATG GACAATGTCT TTGTCAATGG ATTTGGCAGC 720
TATCCTCTCT ACAGCAGTAC GCGCTCAGGA CTCAATATGG CTGTTCCGAT GCATGGATTC 780
GGCCCTCAGT ACGGTGGATC GCTTGAGCTG AAAGTCTCCG AGCGATTCGG CTTTGCCGTC 840
GGTATGGAGC GCGAATACAA TATCTGGACT CGTCGGTGGG AAACGCATTA CTTTGCTTAT 900
CCTGTATTCT ATGGCGATAA GAAG 924

(2) INFORMATION FOR SEQ ID NO:232

(i) SEQUENCE CHARACTERISTICS:
(A) LENGTH: 945 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...945

(xi) SEQUENCE DESCRIPTION: SEQ ID NO:232

ATGAAAACGA ATAGACGATA CGCATTTGTT TTGCCGCTTC TGCTACTCAC CGGATTGTTG 60
GCATGGGGGC AGGATTCTTC CCACGGTAGC AATACAGCGT TTGCAACTGA TTCTTCGAGT 120
AGAGAGTTGC CCACGGAGCA GTCCGCCTAC CGCATTCATT CTGCCTATAT GGTCGGTGGT 180
GGCGGAAGCA TAACGCGCGA CACCTATTTG TCACCCCTTC GTTATGGAGC ATGGACACTG 240

AATTTGTTGG GAGAGAAGAC GTTCCCTCTC AAAGCCTCCG ATTCCCGTTG GATGATCCGT 300
ACCGGGCATG AGCTGGATTT TGCCCTGATG GACAATCCGG CCAATAATGC TCATTTCTAT 360
TCCCTGCTGT ATAACGGTTC CGCTGCGGCT CTTTACCGCC TTGGCGCTAA GCATCTGCGA 420
GCCGCGTGGA TGGACAATCT GCGCTTGGCA TTCGGCCCGG GCTTGGAAAT CGGGCTTGGA 480
GGAATTTATA GTACACGCAA CGGCAATAAT CCTGCGACAT TGAAGCTCTA CACCAATGCC 540
ATCGCCCAAG CCTCGATAGG ATACTACGTC CCCTCCGAAA CTTTTCCCCT GTATTTTCGG 600
TTGCTCTCCC AGATCAATCT CTTCGGTATA GCCTATGGAA ATGGTTTTGG TGAGAGCTAT 660
TACGAGAATT TTTTGCTCAA TAACGGCATT GCAGGCTCCC TGCATTTCAC TTATCCGGGC 720
AAGTTTACTC GGTTCACGAC ACTCATAACG GCGGATATTC CCATTCGGAA CTTCTGTACG 780
CTTCGTGTCG GTTATCGCTA TTCCCATTTG GGCTCTTCGC TTAACGCATT GGATACTCGA 840
ATCCACAGTC ATACGGCTTT TATCGGTTTC GTCACGGAGT TTTACCGATT CCGTGGGCGC 900
AAAGCCATGA ATACCGGTCG GAGAACCAGT CTTTACTATC ATGAT 945

(2) INFORMATION FOR SEQ ID NO:233

(i) SEQUENCE CHARACTERISTICS:
(A) LENGTH: 855 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...855

(xi) SEQUENCE DESCRIPTION: SEQ ID NO:233

ATGGTAGTGA CGCTGCTCGT AATTGTCGGT ATTGTGGTAG TAGTGCGGTA CTCCTTGCGC 60
GTCCACGTAC ATAAGACCGG CACTGTTGTA TCGGCGGCCA TATTTGGCTT CATCCTCCTT 120
GGAAAGACCG TTCCATGCGA TACCCGTAAC TTCTTTTCCT CCGAAAGTGA TGAACCTGAG 180
AGCCGTGTTG CTACCGAAAT AGCCCACCTG TGCGAAATAG GATTTCAGAT CCACGCTTCC 240
TCTATCCACG TAGCCGTCCG AACCGATTTT GGACAGGCGG GCATCCACTG CCCAATGGCG 300
ACCGATGCGT CCGCTACCGA GTTTGACCGA TCGGCGGAAT GTGCCGAACG AACCTCCGCT 360
CAAATCGACA CGGCCATAAG GAGCCAGTCC CAAATTATCC GTACGCATAT TGACACTTGC 420
CCCAAAAGCT CCGGCACCAT TGGTGGAAGT ACCCACACCT CGCTGCACCT GAAGGTCTTC 480
GATGGAAGAG GCGAAGTCGG GCATATTCAC CCAAAAGACG GACTGAGATT CGGAGTCGTT 540
GAGGGGTACT CCATTGGTAG TTATGTTGAT GCGATTGGCA TCGGTGCCAC GCACGCGAAA 600
GCCGGAATAT CCGATACCCG TACCGGCATC GCTGGTGGCT ACCACGGAGG GAGTCAGCAT 660
CAGCAGATAG GGGATGTCAC GACCATAATT GGACTTGGAA AGTTCGGCCT TGCGAACGTT 720
GGTGTAAGCG ACAGGGGTTT TCGCCGTGGC GCGAGTAGCT ACGACCTGTA CGGTCTGGAG 780
CTGCACATTG CTAAGACTAT CTATCTCGCT GTTGGAGACG GGTGCTTGTC CCGTCAGGCA 840
GAAAGGCAGG ACGGC 855

(2) INFORMATION FOR SEQ ID NO:234

(i) SEQUENCE CHARACTERISTICS:
(A) LENGTH: 1797 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1797

(xi) SEQUENCE DESCRIPTION: SEQ ID NO:234

ATGGAAAACT TAAAGAACAT TCAGCCCAGA GAGGATTTCA ACTGGGAAGA GTTTGAGGCC 60
GGTGGCGTCC ATGCTGCCGT GAGTCGTCAG GAGCAGGAAG CTGCTTATGA CAAAACGCTC 120
AATACCATCA AGGAAAAGGA AGTGGTAATG GGTAGGGTAA CTGCTATCAA CAAGCGTGAA 180
GTGGTTATCA ATGTAGGGTA CAAATCGGAA GGTGTGGTAC CTGCAACAGA ATTCCGCTAC 240
AATCCCGAAC TCAAAGTGGG AGACGAAGTG GAAGTTTATA TCGAGAATCA GGAAGATAAG 300
AAGGGCCAGC TCGTCTTGTC TCACCGCAAG GGTCGTGCCG CTCGCTCTTG GGAGCGCGTG 360
AACGAGGCTC TCGAAAAAGA CGAAATCGTA AAGGGCTATG TGAAGTGTCG TACCAAGGGT 420
GGTATGATCG TCGATGTATT CGGTATCGAG GCTTTCCTCC CGGGATCACA GATCGACGTG 480
CGCCCCATTC GCGACTACGA TGCATTCGTT GAGAAGACGA TGGAGTTCAA GATTGTGAAA 540
ATCAATCAAG AATATAAGAA TGTAGTTGTT TCCCACAAGG TGCTCATCGA AGCAGAGCTC 600
GAACAACAGA AGAAAGAAAT CATCGGCAAG CTCGAAAAAG GGCAGGTACT CGAAGGTATC 660
GTCAAGAATA TTACTTCTTA CGGAGTATTT ATCGACCTCG GTGGAGTGGA TGGTCTTATC 720
CATATCACTG ACCTTTCATG GGGTCGTGTG GCTCATCCGG AAGAAATCGT ACAGCTGGAT 780
CAGAAGATCA ATGTCGTTAT CCTCGACTTT GATGAAGATC GCAAGCGTAT CGCTCTCGGA 840
CTCAAACAGC TGATGCCTCA TCCTTGGGAT GCTCTCGACA GCGAGCTTAA GGTAGGCGAT 900
AAGGTGAAGG GTAAAGTTGT GGTGATGGCA GATTACGGTG CTTTCGTTGA GATTGCACAG 960
GGCGTTGAGG GTCTTATCCA CGTAAGCGAA ATGTCATGGA CACAGCACTT GCGTTCTGCT 1020
CAGGACTTCC TGCATGTAGG CGACGAAGTG GAAGCCGTGA TCCTGACGCT CGACCGCGAA 1080
GAACGCAAAA TGTCGCTCGG TCTGAAGCAA CTCAAGCCGG ATCCTTGGGC TGATATCGAA 1140
ACTCGTTTCC CTGTAGGCTC TCGTCACCAT GCTCGTGTTC GCAACTTCAC CAATTTCGGT 1200
GTATTCGTTG AGATCGAAGA GGGCGTAGAT GGCCTTATCC ATATTTCCGA CCTTTCTTGG 1260
ACGAAGAACA TCAAACACCC CAGCGAGTTT ACGGAAGTAG GTGCTGATAT CGAAGTTCAG 1320
GTAATCGAGA TCGACAAGGA AAACCGTCGT CTCAGCTTGG GTCACAAACA GTTGGAAGAG 1380
AATCCTTGGG ATGTATTCGA GACGGTATTC ACTGTAGGAT CTATCCACGA AGGAACGGTA 1440
ATCGAAGTGA TGGACAAGGG TGCTGTCGTT TCTCTGCCTT ACGGTGTGGA AGGTTTTGCC 1500
ACTCCGAAGC ACATGGTGAA GGAAGATGGC TCACAGGCTG TACTCGAAGA GAAGTTACCT 1560
TTCAAGGTTA TTGAGTTCAA TAAGGATGCC AAGCGAATCA TTGTATCTCA TAGCCGTGTA 1620
TTCGAAGATG AGCAGAAAAT GGCTCAGCGT GAAGCCAATG CAGAGCGTAA GGCTGAAGCC 1680
AAAGCGGCTC AGAAAGAAGC TGCTGCCGAA GCTGCCAATC CTGCACAGGC TGTAGAGAAA 1740
GCCACTCTCG GAGACCTCGG CGAGCTGGCC GCTTTGAAAG AAAAGCTTTC AGAAAAC 1797

(2) INFORMATION FOR SEQ ID NO:235

(i) SEQUENCE CHARACTERISTICS:
(A) LENGTH: 1650 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1650

(xi) SEQUENCE DESCRIPTION: SEQ ID NO:235

ATGGGTAGGG TAACTGCTAT CAACAAGCGT GAAGTGGTTA TCAATGTAGG GTACAAATCG 60
GAAGGTGTGG TACCTGCAAC AGAATTCCGC TACAATCCCG AACTCAAAGT GGGAGACGAA 120
GTGGAAGTTT ATATCGAGAA TCAGGAAGAT AAGAAGGGCC AGCTCGTCTT GTCTCACCGC 180
AAGGGTCGTG CCGCTCGCTC TTGGGAGCGC GTGAACGAGG CTCTCGAAAA AGACGAAATC 240
GTAAAGGGCT ATGTGAAGTG TCGTACCAAG GGTGGTATGA TCGTCGATGT ATTCGGTATC 300
GAGGCTTTCC TCCCGGGATC ACAGATCGAC GTGCGCCCCA TTCGCGACTA CGATGCATTC 360
GTTGAGAAGA CGATGGAGTT CAAGATTGTG AAAATCAATC AAGAATATAA GAATGTAGTT 420
GTTTCCCACA AGGTGCTCAT CGAAGCAGAG CTCGAACAAC AGAAGAAAGA AATCATCGGC 480
AAGCTCGAAA AAGGGCAGGT ACTCGAAGGT ATCGTCAAGA ATATTACTTC TTACGGAGTA 540
TTTATCGACC TCGGTGGAGT GGATGGTCTT ATCCATATCA CTGACCTTTC ATGGGGTCGT 600
GTGGCTCATC CGGAAGAAAT CGTACAGCTG GATCAGAAGA TCAATGTCGT TATCCTCGAC 660
TTTGATGAAG ATCGCAAGCG TATCGCTCTC GGACTCAAAC AGCTGATGCC TCATCCTTGG 720
GATGCTCTCG ACAGCGAGCT TAAGGTAGGC GATAAGGTGA AGGGTAAAGT TGTGGTGATG 780
GCAGATTACG GTGCTTTCGT TGAGATTGCA CAGGGCGTTG AGGGTCTTAT CCACGTAAGC 840
GAAATGTCAT GGACACAGCA CTTGCGTTCT GCTCAGGACT TCCTGCATGT AGGCGACGAA 900
GTGGAAGCCG TGATCCTGAC GCTCGACCGC GAAGAACGCA AAATGTCGCT CGGTCTGAAG 960
CAACTCAAGC CGGATCCTTG GGCTGATATC GAAACTCGTT TCCCTGTAGG CTCTCGTCAC 1020
CATGCTCGTG TTCGCAACTT CACCAATTTC GGTGTATTCG TTGAGATCGA AGAGGGCGTA 1080

GATGGCCTTA TCCATATTTC CGACCTTTCT TGGACGAAGA AGATCAAACA CCCCAGCGAG 1140
TTTACGGAAG TAGGTGCTGA TATCGAAGTT CAGGTAATCG AGATCGACAA GGAAAACCGT 1200
CGTCTCAGCT TGGGTCACAA ACAGTTGGAA GAGAATCCTT GGGATGTATT CGAGACGGTA 1260
TTCACTGTAG GATCTATCCA CGAAGGAACG GTAATCGAAG TGATGGACAA GGGTGCTGTC 1320
GTTTCTCTGC CTTACGGTGT GGAAGGTTTT GCCACTCCGA AGCACATGGT GAAGGAAGAT 1380
GGCTCACAGG CTGTACTCGA AGAGAAGTTA CCTTTCAAGG TTATTGAGTT CAATAAGGAT 1440
GCCAAGCGAA TCATTGTATC TCATAGCCGT GTATTCGAAG ATGAGCAGAA AATGGCTCAG 1500
CGTGAAGCCA ATGCAGAGCG TAAGGCTGAA GCCAAAGCGG CTCAGAAAGA AGCTGCTGCC 1560
GAAGCTGCCA ATCCTGCACA GGCTGTAGAG AAAGCCACTC TCGGAGACCT CGGCGAGCTG 1620
GCCGCTTTGA AAGAAAAGCT TTCAGAAAAC 1650

(2) INFORMATION FOR SEQ ID NO:236

(i) SEQUENCE CHARACTERISTICS:
(A) LENGTH: 1374 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1374

(xi) SEQUENCE DESCRIPTION: SEQ ID NO:236

ATGATCGTCG ATGTATTCGG TATCGAGGCT TTCCTCCCGG GATCACAGAT CGACGTGCGC 60
CCCATTCGCG ACTACGATGC ATTCGTTGAG AAGACGATGG AGTTCAAGAT TGTGAAAATC 120
AATCAAGAAT ATAAGAATGT AGTTGTTTCC CACAAGGTGC TCATCGAAGC AGAGCTCGAA 180
CAACAGAAGA AAGAAATCAT CGGCAAGCTC GAAAAAGGGC AGGTACTCGA AGGTATCGTC 240
AAGAATATTA CTTCTTACGG AGTATTTATC GACCTCGGTG GAGTGGATGG TCTTATCCAT 300
ATCACTGACC TTTCATGGGG TCGTGTGGCT CATCCGGAAG AAATCGTACA GCTGGATCAG 360
AAGATCAATG TCGTTATCCT CGACTTTGAT GAAGATCGCA AGCGTATCGC TCTCGGACTC 420
AAACAGCTGA TGCCTCATCC TTGGGATGCT CTCGACAGCG AGCTTAAGGT AGGCGATAAG 480
GTGAAGGGTA AAGTTGTGGT GATGGCAGAT TACGGTGCTT TCGTTGAGAT TGCACAGGGC 540
GTTGAGGGTC TTATCCACGT AAGCGAAATG TCATGGACAC AGCACTTGCG TTCTGCTCAG 600
GACTTCCTGC ATGTAGGCGA CGAAGTGGAA GCCGTGATCC TGACGCTCGA CCGCGAAGAA 660
CGCAAAATGT CGCTCGGTCT GAAGCAACTC AAGCCGGATC CTTGGGCTGA TATCGAAACT 720
CGTTTCCCTG TAGGCTCTCG TCACCATGCT CGTGTTCGCA ACTTCACCAA TTTCGGTGTA 780
TTCGTTGAGA TCGAAGAGGG CGTAGATGGC CTTATCCATA TTTCCGACCT TTCTTGGACG 840
AAGAAGATCA AACACCCCAG CGAGTTTACG GAAGTAGGTG CTGATATCGA AGTTCAGGTA 900
ATCGAGATCG ACAAGGAAAA CCGTCGTCTC AGCTTGGGTC ACAAACAGTT GGAAGAGAAT 960
CCTTGGGATG TATTCGAGAC GGTATTCACT GTAGGATCTA TCCACGAAGG AACGGTAATC 1020
GAAGTGATGG ACAAGGGTGC TGTCGTTTCT CTGCCTTACG GTGTGGAAGG TTTTGCCACT 1080
CCGAAGCACA TGGTGAAGGA AGATGGCTCA CAGGCTGTAC TCGAAGAGAA GTTACCTTTC 1140
AAGGTTATTG AGTTCAATAA GGATGCCAAG CGAATCATTG TATCTCATAG CCGTGTATTC 1200
GAAGATGAGC AGAAAATGGC TCAGCGTGAA GCCAATGCAG AGCGTAAGGC TGAAGCCAAA 1260
GCGGCTCAGA AAGAAGCTGC TGCCGAAGCT GCCAATCCTG CACAGGCTGT AGAGAAAGCC 1320
ACTCTCGGAG ACCTCGGCGA GCTGGCCGCT TTGAAAGAAA AGCTTTCAGA AAAC 1374

(2) INFORMATION FOR SEQ ID NO:237

(i) SEQUENCE CHARACTERISTICS:
(A) LENGTH: 1278 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:

(A) ORGANISM: PORYPHYROMONAS GINGIVALIS

(ix) FEATURE:
(A) NAME/KEY: misc_feature
(B) LOCATION 1...1278

(xi) SEQUENCE DESCRIPTION: SEQ ID NO:237

ATGGAGTTCA AGATTGTGAA AATCAATCAA GAATATAAGA ATGTAGTTGT TTCCCACAAG 60
GTGCTCATCG AAGCAGAGCT CGAACAACAG AAGAAAGAAA TCATCGGCAA GCTCGAAAAA 120
GGGCAGGTAC TCGAAGGTAT CGTCAAGAAT ATTACTTCTT ACGGAGTATT TATCGACCTC 180
GGTGGAGTGG ATGGTCTTAT CCATATCACT GACCTTTCAT GGGGTCGTGT GGCTCATCCG 240
GAAGAAATCG TACAGCTGGA TCAGAAGATC AATGTCGTTA TCCTCGACTT TGATGAAGAT 300
CGCAAGCGTA TCGCTCTCGG ACTCAAACAG CTGATGCCTC ATCCTTGGGA TGCTCTCGAC 360
AGCGAGCTTA AGGTAGGCGA TAAGGTGAAG GGTAAAGTTG TGGTGATGGC AGATTACGGT 420
GCTTTCGTTG AGATTGCACA GGGCGTTGAG GGTCTTATCC ACGTAAGCGA AATGTCATGG 480
ACACAGCACT TGCGTTCTGC TCAGGACTTC CTGCATGTAG GCGACGAAGT GGAAGCCGTG 540
ATCCTGACGC TCGACCGCGA AGAACGCAAA ATGTCGCTCG GTCTGAAGCA ACTCAAGCCG 600
GATCCTTGGG CTGATATCGA AACTCGTTTC CCTGTAGGCT CTCGTCACCA TGCTCGTGTT 660
CGCAACTTCA CCAATTTCGG TGTATTCGTT GAGATCGAAG AGGGCGTAGA TGGCCTTATC 720
CATATTTCCG ACCTTTCTTG GACGAAGAAG ATCAAACACC CCAGCGAGTT TACGGAAGTA 780
GGTGCTGATA TCGAAGTTCA GGTAATCGAG ATCGACAAGG AAAACCGTCG TCTCAGCTTG 840
GGTCACAAAC AGTTGGAAGA GAATCCTTGG GATGTATTCG AGACGGTATT CACTGTAGGA 900
TCTATCCACG AAGGAACGGT AATCGAAGTG ATGGACAAGG GTGCTGTCGT TTCTCTGCCT 960
TACGGTGTGG AAGGTTTTGC CACTCCGAAG CACATGGTGA AGGAAGATGG CTCACAGGCT 1020
GTACTCGAAG AGAAGTTACC TTTCAAGGTT ATTGAGTTCA ATAAGGATGC CAAGCGAATC 1080
ATTGTATCTC ATAGCCGTGT ATTCGAAGAT GAGCAGAAAA TGGCTCAGCG TGAAGCCAAT 1140
GCAGAGCGTA AGGCTGAAGC CAAAGCGGCT CAGAAAGAAG CTGCTGCCGA AGCTGCCAAT 1200
CCTGCACAGG CTGTAGAGAA AGCCACTCTC GGAGACCTCG GCGAGCTGGC CGCTTTGAAA 1260
GAAAAGCTTT CAGAAAAC 1278

(2) INFORMATION FOR SEQ ID NO:238

(i) SEQUENCE CHARACTERISTICS:
(A) LENGTH: 720 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...720

(xi) SEQUENCE DESCRIPTION: SEQ ID NO:238

ATGAAAAAAG CTATTCTTTC CGGAGCGGCC TTGCTCCTCG GCCTATGTGC CAACGCACAA 60
AACGTGCAGT TGCACTACGA TTTCGGTCAT TCCATCTACG ACGAACTAGA TGGACGTCCC 120
AAACTGACTA CCACAGTGGA AAACTTCACA CCCGACAAAT GGGGAAGCAC CTTCTTCTTC 180
ATCGACATGG ATTACACGGG CAAGGGTATC CAGTCGGCCT ATTGGGAGAT TTCGCGCGAA 240
CTGAAGTTTT GGCAAGCTCC CGTTTCCATT CATTTGGAGT ACAACGGAGG CCTCTCCACA 300
AGCTTTACTT TCGGACACGA TGCTCTAATC GGTGCCACCT ACACCTACAA CAACCCCTCC 360
TTTACACGTG GATTTACGAT CACGCCCATG TACAAGCATC TGGGTGCGCA CGACTTCCAC 420
ACCTATCAGA TCACCGGCAC TTGGTACATG CACTTTCTGG ACGGTCTGCT TACCTTCAAC 480
GGCTTCCTCG ATCTTTGGGG TTTCCCCCAA GAGAACCCAA TCGGGGGCCC TGTGCTCAAA 540
GAAGGGGATA AGTTCGTATT CCTGTCCGAA CCGCAGTTCT GGATCAACCT CAATCGCATC 600
AAAGGCATCG ACAAGGATTT CAATCTCAGC ATAGGGACAG AGATGGAAAT CAGCAGGAAC 660
TTCGCTCGCA TGGACAAATT CTCCTGCATC CCTACTCTTG CGGTCAAATG GACTTTCAAC 720

(2) INFORMATION FOR SEQ ID NO:239

(i) SEQUENCE CHARACTERISTICS:
(A) LENGTH: 1302 base pairs
(B) TYPE: nucleic acid
(C) STRANDEDNESS: double

(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1302

(xi) SEQUENCE DESCRIPTION: SEQ ID NO:239

ATGTATAAAG ACTATAAGGG TTTGTATGCG TCGCTTCGGT GGTATGCCCT GATCATTGGG 60
TTGCTATTTG CAGCAGACGG TATACAGGCT CAGAACAACA ACTTTACCGA GTCGCCTTAC 120
ACTCGCTTCG GCCTTGGCCG TCTCGGAGAA CGGACGACTA TTAGTGGGCA TTCCATGGGA 180
GGACTCGGCG TCGGTCTGCG TCAGGGCACA TACGTCAATG CCGTCAATCC TGCTTCATAC 240
TCGGCTGTGG ATTCGATGAC GTTTATCTTC GATTTCGGTG CATCTACCGG AATTACGTGG 300
TATGCCGAGA ACGGAAAAAA GGACAATAGG AAAATGGCAA ACATTGAGTA TTTCGCCATG 360
CTTTTTCCTA TTTCCAAATC CATTGCTATG AGTGCGGGAG TGCTTCCTTA CTCCGCATCC 420
GGGTACCAGT TCGGATCCGT TGATCAAGTG GAAGGAGGCA GCGTCCAGTA CACCCGTAAA 480
TACTTGGGGA CAGGCAATCT GAACGATCTC TATGTCGGTA TAGGTGCAAC CCCGTTCAAA 540
AACTTCTCAA TAGGAGCCAA TGCTTCATCC CTTTTTGGGC GATTCACACA CAGCAGGCAG 600
GTAATCTTCT CCACGGAGGC TCCTTACAAT CCCGTACATC TCTCGACGCT GTACTTGAAG 660
GCTGCCAAGT TCGACTTCGG TATGCAGTAT CACCTTCTTC TCAAATCAGA TCGTTCGCTC 720
GTTATCGGTG CCGTCTATTC TCCGCGGGTG AAGATGCATA GCGAGCTGAC TCAGATAAAG 780
AATCAGGTTC AGAACGGTGT AGTAGTGGAG AGCGAAACCC AAGAATATAT CAAGGGAATG 840
GACTATTATA CCCTGCCTCA TACATTGGGG ATAGGTTTTT CTTATGAAAA GAAAGATAAA 900
CTTCTCTTAG GAGCAGACGT CCAATATAGT AAATGGAAAG GCGAGAAATT TTATAAATCC 960
GATTGCAAAT TCCAGGACAG AATACGGGTA TCTCTCGGCG GAGAGATCAT ACCGGATATA 1020
AATGCCGTTG GGATGTGGCC TAAAGTTCGC TATCGCTTCG GTTTACATGG TGAAAATTCT 1080
TACCTGAAAG TGCCGACTAA AGGCGGTGTA TATCAAGGAT ACCATATCGT AGGTGCTGTA 1140
TTCGGTATAG GAATCCCGCT CAATGACAGA CGTTCGTTCG TAAATGTCTC TCTTGAATAT 1200
GACCGATTGA TCCCGAAGGA GGGTATGATC AAAGAAAATG CTCTGAAATT GACCTTCGGC 1260
CTCACGTTCA ACGAGTCATG GTTTAAAAAG CTGAAACTGA AC 1302

(2) INFORMATION FOR SEQ ID NO:240

(i) SEQUENCE CHARACTERISTICS:
(A) LENGTH: 2778 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2778

(xi) SEQUENCE DESCRIPTION: SEQ ID NO:240

ATGCGATCGA TTTATCAATT ACTGTTGTCA ATACTCCTTG CTTCTCTTGG TTTCGTCGGG 60
CTGGAAGCCC AACAAGCCGG AGTAGCAGGT AGAGTATTGG ACGAAGAAGG CAACCCCATG 120
ATTCAAGCCA ACGTACAGCT TGTACAGAGT ACCGGCCAAG TAGCCGTTGC CGCAGGTGCC 180
ACTAATGAAA AAGGGTTGTT CAGCCTGAAA ACGTCACAGG AGGGTGACTA CATTCTGCGC 240
GTTTCATATG TAGGTTACAC TACCCACGAC GAAAAAATAT CTCTTAGAAA CGGGCAAACC 300
ATTACGCTCA AAGATATATC CATGAACGAA GATGCCCGTC TTCTACAGAG TGTGACGGTG 360
CAGGCTAAAG CGGCAGAGGT CGTGGTACGC AACGATACGC TCGAATTCAA TGCCGGATCC 420
TATACCGTAG CACAGGGAGC TTCTATCGAG GAACTGATCA AGAAGCTACC CGGAGCAGAG 480
ATCGGATCCG ATGGGAAGAT CACCATCAAC GGCAAGGACA TTAGCAAGAT CCTTGTCGAT 540
GGCAAAGAGT TTTTCTCCAA AGATCCACAG GTGGCAATAA AGAATCTTCC GGCCGATATG 600
GTCAATAAAG TACAGGTACT GAACAAACTG AGCGAGCTGT CGCGGATGAG CGGTTTCGAT 660

GATGGAGAAG AGGAGACCGT AATCAACCTG ACGGTGAAGC CCGAAAAAAA GAAAGGCCTC 720
TTCGGAACGC TTCAGGCCGG CTACGGTACC GACCAACGCT ATATGGCCGG AGGGAACGTC 780
AATCGGTTCG ATGGAAATAA GCAATGGACA TTGATCGGTA GTGCGAACAA TACGAACAAT 840
ATGGGCTTTA GCGAGATGGA CAGCGAGATG GGATCCATGA CCTTCTTCTC TCCCCAAGGC 900
GGTGGTCGAC GCGGCTTCGG CAATAGTGGA GGTGTTACGT CTTCGTCGAT GCTGGGCGGC 960
AACTTCAGTG TCGAATTCTC CTCTGCCCTT AATACAGGAG GCGATGCACG CTACGGATAC 1020
AACGACAAGG CCATAGAGAC GACCAAACGC GTGGAAAATA TCCTCGCCGA AGGGAATACT 1080
TATATGGACG AAAATATATT GGAACGCTCT TTCTCTCACA ATGGTCAGGC GCGATTTAGG 1140
ATGCAATGGA AACCGTCCGA ACGTACCGAA GTGGTATTCG AGCCGGATCT TTCGATATCC 1200
AAGATCGATG GGTTCTTTAA CGACACATAC GAGACGAAAG ATGCCACCGG AATCTCTATC 1260
AACAAAGGTT CTATCCACCA AACTACACAA GGAAACAACT TCAGACTGAA CGGAGAATTG 1320
GATATCAGTC ACAAGCTCAA CGACGAAGGC CGTACGATCA GTGCCTCCGT CAGTGGCGGT 1380
CTGACCGACG AAGACGGAGA TGGCATATAT CAGGCTGTGC TCCAAAGCGT GGAGACGAAT 1440
CAAAAGCAAT TCAACGACAA CTCCAACCTG CAATATCGGC TTCGCCTCTC GTATGTGGAA 1500
CCGTTGGGTA AAAACTACTT CGCACAAGCG ATTCTGAACA GACGTTTCTC CCGTCGCAAT 1560
TCGGATCGTG AGGTGTACCG ACTGGGCGAT GACGGGCAAT ACTCCATATT AGACAGTCAG 1620
TACGGACTCT CCTACAGTAA CGAGTTCACC CAGTATCGCA TCGGACTCAA CCTCAAGAAG 1680
ATTGCCAAAA CGTGGGACTA CACCGTAGGA TTCAATGTGG ATCCCAACAG AACTGTCAGC 1740
TATCGGAGCG TAGCCGGAGT AGAGCAGGAC AAACTGGCTT TCAATCGTGT CAATCTCTCC 1800
CCGATGCTCC GAATCAACTA CAAACCGAGC AGGACTACCA ACCTCCGAGT GGACTACCGA 1860
GGACGCACGA CACAACCATC CATCAATCAG ATCGCTCCCG TTCAGGACAT CACGAATCCG 1920
CTATTCGTGA CGGAAGGCAA TCCCGGTCTG AAGCCGAGCT ATTCCAACAA TGTGATGGCC 1980
ATGTTCTCGG ACTTCGATGC CAAAAGTCAG CGAGCTTTCA ACATTGTTTT CTTCGGCAAC 2040
TATACATTCG ACGACATCGT CCCCAATACG CACTACGATC CGTCTACAGG GATCCGTACC 2100
ACTCGTTACG AAAACGCCTC CGGTACGTGG CAAGCGAATC TTCATGGGAC ACTATCGCTT 2160
CCACTCAAGA ACAGGGCATT TTCTTTCAGC ATGTCCTTGT TCAACAGGTT GGCCGAAGGA 2220
CAAAGCTTCA TCAATGACGA TAAGAACAAA GCTCTCTCTT TCCGAACGAG GGAACGCCTG 2280
ACGCTGACCT ATCGCAACAA TTGGATCGAT ACGAGTATCG GTGGCAATAT CGGATTCTAT 2340
ATGGCGAATA ATAGTCTGAG CGGACAGAAA GATTCTCGCA CATACGATTT TGGCGGCAAT 2400
TATCAAGTTG CCCTAACGCT TCCCTATGGA TTCCGTATCG ACAGCGATGT TGAATACAAT 2460
ACGAACTCCG GTTACAGCGG AGGATTCAGT CTGGACGAAT GGCTTTGGAA TGCTTCGCTT 2520
TCATACAGCT TCCTCCGTGA CAAGGCCGGT ACACTGCGTG.TCAATGGCTA TGACATCCTC 2580
GGTCAGCGGT CAAGTATCAG CCGTTCTGCT TCGGCCATCA ATATAGAAGA GAGCATGTCC 2640
AATACGATCG GACGCTACGT GATGGTGGAC TTTATCTACC GATTCAACGC CTTCAGTGGT 2700
GGTGGATCTC GCAGCGATCA TCAGCGTGGC AATATGAATC GTCCGGGCCC ACCTTTCGGC 2760
GGTGGCAGAC GACCGTCC 2778

(2) INFORMATION FOR SEQ ID NO:241

(i) SEQUENCE CHARACTERISTICS:
(A) LENGTH: 1200 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1200

(xi) SEQUENCE DESCRIPTION: SEQ ID NO:241

ATGGTTATGA AGCTGATTAA AAGAAGTTTG CTCCTGCTTG GAGCGGTACT GCTGATTACG 60
CTTCCTGCGT ACTCGCAGAA TGATGACATC TTCGAAGATG ACATCTATAC ATCGCGAAAA 120
GAAATACGTA AACAAAACCA AGTTAAAGAC TGGCAAAACC AAGAGGACGG ATACGGCGAC 180
GATACGGAAT ATACAGTGGC TTCCGATCGG GACATTGACG CCTACAATCG TAGAGATGGC 240
CAGTCCTACG ATGGGAAAAA GTTGTCCAAA GACAAGAAAA GAGACTCCAC TCGTTCTTCT 300
GTTCCCGGTC GCTATAGTCG CCGCTTGGCT CGATTCTATA AGCCGAATAC GATCGTCATT 360
TCAGGTGCCG ACAATGTATA TGTAACTGAT GATGGTGAGT ATTTCGTCTA TGGAGACGAA 420
TACTATGATG ACGCGTCGTC TGTAAACATT TACATCAACA GTCCTTGGTG CGATCCGTTC 480
CCTTATACGT CATGGTATCC ATCTTTCTCC GGCTGGTACA ACTATACGTG GAACTATCCA 540
TGGTTCTACT ACGGTAGCCA TATCCGATGG GGCGGTTATT ACCCCGGATA TAATTGGTAT 600
TGGAGCTACT ACTATGATCC TTTCTACAAT CCCTATGGAA TCGGTATGGG TTGGGGATAT 660
CCTTATGGCT GGGGCAGCTA TTACGGTTGG GGTGGCTATC CGGGAGTGAT ACATCACTAC 720
CACCACTACC CCAAGAAGAC CTATTCCAAT GGTCAGCATT CCGGAGCTTA CTATTCTTAT 780
GGCCGACCGA ATCGTATCAA AGGTGGAACG TCCGGTGCCA AACTTGGGAC AGGACGCTAC 840

GATAGAATTC AAAATTCGTC TTCGCAAAAA AATAAGTTCG GATTGCAGTC GAACAAACCC 900
AATAATAATC TGCAAAATGT CAAGTCGGGA CGTACCGGCC GAGCCAATAG AGACCGAAAT 960
ATAGAAACGG TAACTCCAAA CAACGGGCAA AAGCAGAATC GTCCCGTATT CCAGCAGAAT 1020
CAGTCCGGCA ATGACCGACC GACCGGACGG AATATCCGCA GCGAGAGACA GGGGGAAAAT 1080
AACGATAGGA CATTTTCGAC TCCTTCTCGT AGCAATAGTA ACGGTGGCTT CTCCACGCCT 1140
TCTCGCTCTT CTTCCGGCTC TATGAGCGGA GGTGGCGGAC GTAGTGGCCG GGGACGCAAT 1200

(2) INFORMATION FOR SEQ ID NO:242

(i) SEQUENCE CHARACTERISTICS:
(A) LENGTH: 1194 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1194

(xi) SEQUENCE DESCRIPTION: SEQ ID NO:242

ATGAAGCTGA TTAAAAGAAG TTTGCTCCTG CTTGGAGCGG TACTGCTGAT TACGCTTCCT 60
GCGTACTCGC AGAATGATGA CATCTTCGAA GATGACATCT ATACATCGCG AAAAGAAATA 120
CGTAAACAAA ACCAAGTTAA AGACTGGCAA AACCAAGAGG ACGGATACGG CGACGATACG 180
GAATATACAG TGGCTTCCGA TCGGGACATT GACGCCTACA ATCGTAGAGA TGGCCAGTCC 240
TACGATGGGA AAAAGTTGTC CAAAGACAAG AAAAGAGACT CCACTCGTTC TTCTGTTCCC 300
GGTCGCTATA GTCGCCGCTT GGCTCGATTC TATAAGCCGA ATACGATCGT CATTTCAGGT 360
GCCGACAATG TATATGTAAC TGATGATGGT GAGTATTTCG TCTATGGAGA CGAATACTAT 420
GATGACGCGT CGTCTGTAAA CATTTACATC AACAGTCCTT GGTGCGATCC GTTCCCTTAT 480
ACGTCATGGT ATCCATCTTT CTCCGGCTGG TACAACTATA CGTGGAACTA TCCATGGTTC 540
TACTACGGTA GCCATATCGG ATGGGGCGGT TATTACCCCG GATATAATTG GTATTGGAGC 600
TACTACTATG ATCCTTTCTA CAATCCCTAT GGAATCGGTA TGGGTTGGGG ATATCCTTAT 660
GGCTGGGGCA GCTATTACGG TTGGGGTGGC TATCCGGGAG TGATACATCA CTACCACCAC 720
TACCCCAAGA AGACCTATTC CAATGGTCAG CATTCCGGAG CTTACTATTC TTATGGCCGA 780
CCGAATCGTA TCAAAGGTGG AACGTCCGGT GCCAAACTTG GGACAGGACG CTACGATAGA 840
ATTCAAAATT CGTCTTCGCA AAAAAATAAG TTCGGATTGC AGTCGAACAA ACCCAATAAT 900
AATCTGCAAA ATGTCAAGTC GGGACGTACC GGCCGAGCCA ATAGAGACCG AAATATAGAA 960
ACGGTAACTC CAAACAACGG GCAAAAGCAG AATCGTCCCG TATTCCAGCA GAATCAGTCC 1020
GGCAATGACC GACCGACCGG ACGGAATATC CGCAGCGAGA GACAGGGGGA AAATAACGAT 1080
AGGACATTTT CGACTCCTTC TCGTAGCAAT AGTAACGGTG GCTTCTCCAC GCCTTCTCGC 1140
TCTTCTTCCG GCTCTATGAG CGGAGGTGGC GGACGTAGTG GCCGGGGACG CAAT 1194

(2) INFORMATION FOR SEQ ID NO:243

(i) SEQUENCE CHARACTERISTICS:
(A) LENGTH: 1743 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1743

(xi) SEQUENCE DESCRIPTION: SEQ ID NO:243

ATGATCCGCA AGTTGATCTT ACTGCTTGCT CTGATGCCGG TAGCCTCTGT GGCTTTTGCG 60
GTACCAACGG ACAGCACGGA ATCGAAAGAC AATCGTATCC TTACAAGCAT GCAATCCTCC 120
TCTTTGAATA GGGATGATGC TCCGGATAAA TGGCAACCTA TGCATGCCAA TTTCAGTATT 180
CAGAGCGATA TGCTGCTTTC TACTGCCCAA AAGTCCAAGA ACACCTGGTT CGGCAACTCC 240
TATATCATGG GTATAATCAA GAACAATTAT CTGGAGTTTG GTGCCCGTTT CGAGGATCTC 300
TATAAGCCCC TGCCCGGACA TGAACCCGAG ATGGGGCGTG GCGTTCCTCA CATGTATGTG 360
AAGGGAAGCT ATCATTGGGC GGAGCTGACT ATGGGAGACT TCTACGATCA GTTCGGTAGC 420
GGTATGGTAT TCCGCACCTA TGAAGAGCGC AACCTCGGTA TAGACAACGC GGTTCGCGGC 480
GGACGTATAG TACTCACTCC TTTTGATGGA GTGCGTGTCA AGGGTATTGC AGGACAGCAG 540
CGTAACTACT TCGACCGCAC GGGCAAGGTA TTCAATTCCG GCCGAGGCTA CCTACTGGGT 600
TCTGATCTGG AGCTGAATGT AGAGCGTTGG AGCAGTGCCA TGCGCGACAA TGACTATCAT 660
TTGGCTATCG GGGGATCGTT CGTTTCCAAA CACGAAGCAG ACGAAGATAT ATTTGTGGGT 720
GTAGGCGAAG ATCGCAAGCG ACTCAACCTG CCGCTCAATG TCCCGATTAT GGGCCTGCGC 780
ACCAACTTTC AAAAAGGAGG TCTCGCCCTC TACGCAGAGT ATGGATACAA ATACAACGAT 840
CCCTCGGCAG ACAATGACTA TATCTACCAC GACGGACAGG CTGCACTCCT CTCTGCCTCA 900
TACTCCAAAA AAGGGATGAG TATCCTGTTG CAGGCCAAAC GTTGTGAGAA CTTTGCTTTC 960
CGCAGCAAGC GAAGTGCCCA GCTCACACCG CTTATGATCA ACTATATGCC GGCTTTTACC 1020
CAAGCTCACA CTTATACGCT GGCGGCCATC TACCCCTATG CTACTCAGCC TCAGGGAGAA 1080
TGGGCTTTCC AAGGTGAACT GCGTTACAAC TTTGCTCGCC GGACAGCTCT CGGTGGACGC 1140
TACGGTACCG GCTTGCGTAT CAACGTTTCG CATGTGCGTG GTCTGGACAA AAAGATGCTC 1200
AAAGAGAATC CCGACGAACT GATCGGAACG GATGGCTACA CCGTTTCTTT CTTCGGCATG 1260
GGCGACCTCT ATTATTCGGA TATAGATGTG GAGATTACTA AAAAGGTAAG CCCAGGATTC 1320
AACTTTACGC TCACCTACTT GAATCAGATC TACAATAACA AGGTACTGCA CGGTGCAGCC 1380
GGAGAGAAGC CTGAGAAGAT CTATGCCAAT ATCTTCGTCT ATGATGGTAA GTATAAGCTG 1440
AGTAATAAGG TAGCCCTCCG TACCGAACTG CAATATTTGC ACACGAAGCA GGATCAGGGT 1500
GACTGGATCT ACGGCATGGC CGAGCTCTCT ATCCTGCCTT CTCTGATGCT TTCCCTCTCG 1560
GAGCAGTATA ATATCGGAGA GACCAAGAAA CATTATGTCA TGGGGTCTGT CACCTATACT 1620
CACGGAGCAC ATCGAGTAGC TTTCTCTGCA GGCAAAACCC GTGCAGGGAT GAACTGCTCG 1680
GGAGGTGTAT GTCGTGTGGT CCCTGAGACT CAGGGATTCT ACCTTTCTTA TAGCACCAAT 1740
CTG 1743

(2) INFORMATION FOR SEQ ID NO:244

(i) SEQUENCE CHARACTERISTICS:
(A) LENGTH: 717 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...717

(xi) SEQUENCE DESCRIPTION: SEQ ID NO:244

ATGCGTTCTT TATTTTTGAG CGCGTTGCGC AGCTCCTCTC TCCATGGTTC AGAGCGACGC 60
AGTCGGATAA GTTCTTCTGT AGTCATGTCA ATAAGGCAGA AAATAAGGTT ATTCCATCTC 120
TCGGTATGCG CCCAAACGCA TGATCATCTC ATCGAAATCC ACTTGGTGTG CATCGAATTC 180
GGGGCCATCG ACACAGACGA ATTTCGTCTG TCCTCCCACG CTTATACGAC AAGCCCCACA 240
CATACCGGTG CCATCCACCA TAATTGTATT GAGAGAAGCT ATGGTCGGTA TCTCGTAACG 300
TTTGGTCAGG AGAGAAACGA ACTTCATCAT CACAGCCGGC CCGATCGTAA CGCAGAGGTC 360
TACCGTTTCC CGTTTGATAA CGCTTTCCAC TCCATCCGTT ACGAGGCCTT TCGTCCCATA 420
AGACCCATCG TCTGTCATGA TGATCACTTC ATCGCTATTG GCTCGCATTT GTTCTTCAAG 480
GATAACCAGA TCTTTAGTTC TGGCAGCCAA TACGACAATT ACACGGTTGC CTGCTTTGTG 540
GAAAGCCTCC ACGATCGGGA GCAAAGGAGC CACACCCACA CCGCCTCCGG CACAAACCAC 600
TGTGCCGACC TTTTCGATAT GCGTACTCTG TCCCAGCGGA CCTACCACAT CCGTGATATA 660
GTCGCCGACT TCGAGTTCGG CCAATTTCTT GGAAGATTTG CCCACGGCCT GAACCAC 717

(2) INFORMATION FOR SEQ ID NO:245

(i) SEQUENCE CHARACTERISTICS:
(A) LENGTH: 633 base pairs
(B) TYPE: nucleic acid

(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...633

(xi) SEQUENCE DESCRIPTION: SEQ ID NO:245

ATGTCAATAA GGCAGAAAAT AAGGTTATTC CATCTCTCGG TATGCGCCCA AACGCATGAT 60
CATCTCATCG AAATCCACTT GGTGTGCATC GAATTCGGGG CCATCGACAC AGACGAATTT 120
CGTCTGTCCT CCCACGCTTA TACGACAAGC CCCACACATA CCGGTGCCAT CCACCATAAT 180
TGTATTGAGA GAAGCTATGG TCGGTATCTC GTAACGTTTG GTCAGGAGAG AAACGAACTT 240
CATCATCACA GCCGGCCCGA TCGTAACGCA GAGGTCTACC GTTTCCCGTT TGATAACGCT 300
TTCCACTCCA TCCGTTACGA GGCCTTTCGT CCCATAAGAC CCATCGTCTG TCATGATGAT 360
CACTTCATCG CTATTGGCTC GCATTTGTTC TTCAAGGATA ACCAGATCTT TAGTTCTGGC 420
AGCCAATACG ACAATTACAC GGTTGCCTGC TTTGTGGAAA GCCTCCACGA TCGGGAGCAA 480
AGGAGCCACA CCCACACCGC CTCCGGCACA AACCACTGTG CCGACCTTTT CGATATGCGT 540
ACTCTGTCCC AGCGGACCTA CCACATCCGT GATATAGTCG CCGACTTCGA GTTCGGCCAA 600
TTTCTTGGAA GATTTGCCCA CGGCCTGAAC CAC 633

(2) INFORMATION FOR SEQ ID NO:246

(i) SEQUENCE CHARACTERISTICS:
(A) LENGTH: 2343 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2343

(xi) SEQUENCE DESCRIPTION: SEQ ID NO:246

ATGGATCGTC CTAAGCCTTC ATATATTGTT CGAATAGCAG CCATTCTCTG CTTGTTTGTC 60
GGCAGGCCTT TGTTTGCGCA GAGCTATGTG GACTACGTCG ATCCGCTGAT CGGGACGCTA 120
AGTTCTTTTG AGCTGAGTGC GGGCAATACC TATCCGGTGA TCGGTTTACC GTGGGGAATG 180
AATAGCTGGA CACCGATGAC CGGTGTACCC GGTGACGGCT GGCAATATAC CTACTCGGCA 240
CACAAGATTC GCGGATTCAA ACAGACCCAC CAACCCAGTC CTTGGATCAA CGACTACGGC 300
CAATTCTCCC TTCTTCCCCT TACGGCACCG CAGAAGCCAT CATCGAACGA CTCCATAGCT 360
CTGACTAAAT GGTGCAAGCA ACTCTTTTCG GACGAACAGA CCTCGTGGTT CTCGCACAAA 420
GCGGAGACGG CGACGCCATA CTATTATAGT GTCTATTTGG CCGATTACGA CACACGCGTG 480
GAGATGGCTC CGACCGAGCG TGCAGCTATC TTTCGCATAC GTTATTCCGG CAATACCGAA 540
AGTGGCTCCG GTCGATGGCT TCGTCTTGAT GCCTTTACCG GTGGTTCGGA GATTAGCATC 600
GTGGATCCTC ACACCGTAGT GGGCATATCT CGCAAGAATA GCGGAGGTGT GCCGGCTAAC 660
TTCGCCTGTT ATTTCATCCT GCAGTCCGAT ACTCCTATGG CCGATGTCCT GCTTGAGACA 720
GATACCGGCA AGTCAGACGA AGGCACAAGG GCATGGGCAG CCTGTCGCTT CGATTCGCAA 780
GAAGTTACCG TCCGGGTGGC ATCTTCTTTT ATCAGTGTCG AGCAGGCCGA AAGAAATCTT 840
GCGGAAGTCA AAGGGCAGAG TTTCGACCGG ATCAGACTTG CCGGTCGCGA AGCTTGGAAT 900
AAGGTGCTCG GACGCATACA TGTGGAAGGA GGAACGAAGG ATGAGCGCAC TACATTCTAT 960
TCCGCACTCT ATCGCTGTCT GCTTTTTCCC CGTCGCTTCT ATGAGGAGGA TGCTTCCGGC 1020
AATTTTGTGC ATTACAGCCC CTACAATGGA GAGGTACTTC CCGGTTATCT CTATACCGAT 1080
ACCGGATTTT GGGACACTTT TCGAGCCCTT TTCCCCCTGC TCAATCTGCT GTATCCCGAT 1140
GAAAACATTA AAATTCAGGA AGGTCTGCTG AATGTATATC GCGAGAGTGG CTTTTTCCCC 1200
GAATGGGCCA GTCCGGGCCA TCGGGATTGT ATGATAGGCA ACAACTCTGC TTCTGTTCTG 1260

GCGGATGCCT ACCTCAAGGG TGTTCGGGTA GAAGATACCC GTACACTGAT GAACGGACTC 1320
TTGCATGCTA CGAAAGCCGT CCATCCGAAA ATCTCCTCCA CGGGTCGCAA AGGTTGGGAG 1380
TGGTACAACT CCTTAGGTTA TGTTCCGGCT GATGCAGGCA TCGACGAAAG TGCTGCCCGT 1440
ACGCTCGAAT ATGCTTATAA CGATTGGTGC ATCCTCCGAC TGGGGCGCAC ATTGGGTTGG 1500
GATAGAGCTG CATTGGACAC GTTGGCTCAT CGTTCGATGA ACTATCGTCA TCTGTTCGAT 1560
CCGGAAACCA AACTCATGCG CGGTAGAAAT CAGGATGGTA GTTTCCGGAC ACCTTTTTCC 1620
CCTTTCAAAT GGGGAGATGT ATTCACGGAG GGCAATGCCT GGCACTACAC TTGGTCGGTC 1680
TTTCATGATG TGCAGGGGCT TATCGACCTG ATGGGAGGAG ATCGCCCGTT CGTGTCTATG 1740
CTCGATTCGG TATTCAATAC TCCTCCTATG TTCGATGAGA GCTATTACGG ATTTGTCATC 1800
CACGAAATCA GAGAGATGCA AATAGCGGAT ATGGGCAATT ATGCTCATGG CAATCAACCC 1860
ATACAGCATA TGATATATCT GTATAATCAT GCCGGTCATC CATGGAAAGC TCAGGAGAGA 1920
CTACGCGAAG TGATGGGGCG GCTCTATCGT CCTACTCCGG ATGGGTATTG CGGCGATGAA 1980
GACAACGGAC AGACTTCGGC TTGGTACGTT TTCTCTGCTT TAGGCTTCTA TCCTGTTACA 2040
CCCGCTACGG ATCAGTATGT GCTCGGTTCG CCGATTTTTT CCAAGGTAAT ACTCTCTTTT 2100
CCCGACGGAC ACAAAACGGT GTTGCATGCT CCGGCCAACA GTGCCGATAC GCCTTACATC 2160
CGCTCGATCA GCGTAGAAGG AAAAGAATGG AGCTGCAATT ACCTGACTCA CGAACAGCTT 2220
CGCTCTTCTG CATCCATTCA ATGGATGATG GACACGAAAC CCAATTATAA TCGTGGTATG 2280
AAGGAAAGTG ACAGACCTTA TTCCTTCTCC ACGGAGCAAC AGCGTCGCGC TAATCACAGT 2340
AAT 2343

(2) INFORMATION FOR SEQ ID NO:247

(i) SEQUENCE CHARACTERISTICS:
(A) LENGTH: 813 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...813

(xi) SEQUENCE DESCRIPTION: SEQ ID NO:247

ATGATGAAAT CCATGCGCAG CGTGCTGTTG CTACTCTTTC CATTGTCTTT GATCACTGCT 60
TTGGGCTGTA GCAATAACAA AGCTGCCGAA TCGAAGTCTG TCTCTTTCGA TTCGGCCTAT 120
CTCGAACGCT ACATCCCTCT GCGGGCAGAC ATAGATACGC CATCGCTGCA TGTGATGATC 180
AGCTACGTCT ATCCTTCGGG AGATGATATG CTCACAGAGA TTTTCAACGG TTTGCTCTTC 240
GGCGACAGCC TGATGGATTC CTCTTCGCCG GAGAATGCCA TGGAAGGCTA TGCACAGATG 300
CTGGGACAAG ACTATCGCTC TAACAATGCC GAAGCCAATC TGCAAGGGCT TCCTTCTGAC 360
CTTTTGGACT ATATCTACAA GCAGGAAAAT ACCATCGCTT ATTGCGATAC GGGATTGATC 420
TCCACGCGCA TCAATACATA TACTTACGAA GGCGGTGCAC ATACGGAGAA TACAGTCCGG 480
TTTGCCAACA TCCTTCGCAC CACCGGCAAG GTGCTCGAAG AGCGAGATAT ATTCAAGATC 540
GACTATGCGG AAAGGCTGTC CGCACTCATC ATAGGACAAT TGGTGCACGA TTTCGGCAAG 600
ACCACACCTG CCGAATTGGA TGCAATAGGT TTCTTCAACG CAGAAGAAAT ACAGCCCAAT 660
GGCAATTTTA TGATCGATGA CAAAGGTCTC ACATACTGTT TCAATGAGTA TCAGATAGCT 720
GCTTATGCCA GAGGTGCTGT CTATGTCCGT CTCGGATATG ACGTATTGGC TCCTTTGCTA 780
AGGGATGATT CCCCACTAAA GCGTTACTTG CCG 813

(2) INFORMATION FOR SEQ ID NO:248

(i) SEQUENCE CHARACTERISTICS:
(A) LENGTH: 810 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:

(A) ORGANISM: PORYPHYROMONAS GINGIVALIS

(ix) FEATURE:
(A) NAME/KEY: misc_feature
(B) LOCATION 1...810

(xi) SEQUENCE DESCRIPTION: SEQ ID NO:248

ATGAAATCCA TGCGCAGCGT GCTGTTGCTA CTCTTTCCAT TGTCTTTGAT CACTGCTTTG 60
GGCTGTAGCA ATAACAAAGC TGCCGAATCG AAGTCTGTCT CTTTCGATTC GGCCTATCTC 120
GAACGCTACA TCCCTCTGCG GGCAGACATA GATACGCCAT CGCTGCATGT GATGATCAGC 180
TACGTCTATC CTTCGGGAGA TGATATGCTC ACAGAGATTT TCAACGGTTT GCTCTTCGGC 240
GACAGCCTGA TGGATTCCTC TTCGCCGGAG AATGCCATGG AAGGCTATGC ACAGATGCTG 300
GGAGAAGACT ATCGCTCTAA CAATGCCGAA GCCAATCTGC AAGGGCTTCC TTCTGACCTT 360
TTGGACTATA TCTACAAGCA GGAAAATACC ATCGCTTATT GCGATACGGG ATTGATCTCC 420
ACGCGCATCA ATACATATAC TTACGAAGGC GGTGCACATA CGGAGAATAC AGTCCGGTTT 480
GCCAACATCC TTCGCACCAC CGGCAAGGTG CTCGAAGAGC GAGATATATT CAAGATCGAC 540
TATGCGGAAA GGCTGTCCGC ACTCATCATA GGACAATTGG TGCACGATTT CGGCAAGACC 600
ACACCTGCCG AATTGGATGC AATAGGTTTC TTCAACGCAG AAGAAATACA GCCCAATGGC 660
AATTTTATGA TCGATGACAA AGGTCTCACA TACTGTTTCA ATGAGTATCA GATAGCTGCT 720
TATGCCAGAG GTGCTGTCTA TGTCCGTCTC GGATATGACG TATTGGCTCC TTTGCTAAGG 780
GATGATTCCC CACTAAAGCG TTACTTGCCG 810

(2) INFORMATION FOR SEQ ID NO:249

(i) SEQUENCE CHARACTERISTICS:
(A) LENGTH: 801 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...801

(xi) SEQUENCE DESCRIPTION: SEQ ID NO:249

ATGCGCAGCG TGCTGTTGCT ACTCTTTCCA TTGTCTTTGA TCACTGCTTT GGGCTGTAGC 60
AATAACAAAG CTGCCGAATC GAAGTCTGTC TCTTTCGATT CGGCCTATCT CGAACGCTAC 120
ATCCCTCTGC GGGCAGACAT AGATACGCCA TCGCTGCATG TGATGATCAG CTACGTCTAT 180
CCTTCGGGAG ATGATATGCT CACAGAGATT TTCAACGGTT TGCTCTTCGG CGACAGCCTG 240
ATGGATTCCT CTTCGCCGGA GAATGCCATG GAAGGCTATG CACAGATGCT GGGAGAAGAC 300
TATCGCTCTA ACAATGCCGA AGCCAATCTG CAAGGGCTTC CTTCTGACCT TTTGGACTAT 360
ATCTACAAGC AGGAAAATAC CATCGCTTAT TGCGATACGG GATTGATCTC CACGCGCATC 420
AATACATATA CTTACGAAGG CGGTGCACAT ACGGAGAATA CAGTCCGGTT TGCCAACATC 480
CTTCGCACCA CCGGCAAGGT GCTCGAAGAG CGAGATATAT TCAAGATCGA CTATGCGGAA 540
AGGCTGTCCG CACTCATCAT AGGACAATTG GTGCACGATT TCGGCAAGAC CACACCTGCC 600
GAATTGGATG CAATAGGTTT CTTCAACGCA GAAGAAATAC AGCCCAATGG CAATTTTATG 660
ATCGATGACA AAGGTCTCAC ATACTGTTTC AATGAGTATC AGATAGCTGC TTATGCCAGA 720
GGTGCTGTCT ATGTCCGTCT CGGATATGAC GTATTGGCTC CTTTGCTAAG GGATGATTCC 780
CCACTAAAGC GTTACTTGCC G 801

(2) INFORMATION FOR SEQ ID NO:250

(i) SEQUENCE CHARACTERISTICS:
(A) LENGTH: 777 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...777

(xi) SEQUENCE DESCRIPTION: SEQ ID NO:250

ATGAAAAAAA CTACTTTGAC AGGATCGATA TGTGCTTTAC TCCTGTTTTT GGGTCTCTCG 60
GCCAATGCCC AATCGAAGTT AAAGATCAAG AGCATTGAGG CAGCTACCAC TTTCAGTTCG 120
GCCACGGCCG GAAATGGTTT TGGTGGCAAT ATCTTCGGCA TGGACATGAG CATACGGATG 180
AGGGTACACC ACAGCATTCT GCCCGAAGGG TTGGATTTTT CGGTAGGAAT ACATGAAAGA 240
AGAGCACACT GGGAAGAGGC CGGAAGTCCG AAGCTCATGT ATACGAATGT CCCAAGTATC 300
ATTGGTATTG TTGAAAAGGT AATAGTCTTC GAAGACGCAG AAGACTTTTT TGACAAAAAA 360
GCTCTCGGCC GCTTCCTCAT CAGTTTGGGG ATATCCTATA CCAAGCATCT GGGAGCGTAT 420
TGGGGATGGA CCAATGACGC CCATATTCTT TTCTCACCGA TACCCAAGAG CAAGGTCCAC 480
TATGACACCT ACACAAGAGC TGGCAGTGAC CTTGTACTTC AGTCCGAAGA TGTTGCCACA 540
GTGAGCAATG GCTTTTCACC GGGGATCGGA CTCAAAAGTT CTATTTGGTG GAAAATGCCC 600
ATCAAGAGCA AATATGATTT TCGCCTCGGT TTCAGCCTGG GCTATGAGTA TCTGAACCTG 660
CTATATCCGT ATCGTAATTT CAAGCTGGAT GGAAATAAGC CGCTTTCAGC ACTATCTCCT 720
CGCATGAACC ACATCGGCCA TGTGGGCTTC AACTTTACCG TGGGTCTTTG GACTAAT 777

(2) INFORMATION FOR SEQ ID NO:251

(i) SEQUENCE CHARACTERISTICS:
(A) LENGTH: 3798 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...3798

(xi) SEQUENCE DESCRIPTION: SEQ ID NO:251

ATGGGCAAGT ATAAAAGAGC TAAGTACCGC TATTGGCTTT TTCCTTTCTG TTCGGATTAT 60
TATACCTTTG AGGGAGTTAC TTTTTTATGC GCATCTGACG ATATGACAAC CAAGAAACCC 120
CAAGCCATTT TAGACTTAGA GAAGGCCTAT AACATTGAAA TTCCTGATCT CTCCTCACAA 180
GAAGGGATAA GCTGGTCGGT AAATAGATAT TTCAAGCAAG ATTCCTCCGG TGCAGTCGTT 240
GAGCTTTGCT TGCGAGAATG TCAGATAGAA AGCATGACTT GGCTTATTGA TTTTCCTGCT 300
CTAAAAAAGC TTGATCTATC GTATAACCAA ATCAGTAAGC TAGAGGGTCT AGAACGTCTT 360
ACTTCGTTAA CAAAACTTCG TCTAAGAAGT AACCAAATCC GTAAACTAGA GGGCCTGGAT 420
AGTCTCACCT CGCTAACAAA ACTTTCTCTC TCCGATAACC AAATCAGTAA GCTAGAGGGT 480
CTGGAACGTC TCACCTCGTT AGCGGAGCTT TATCTTTTGG ATAACCAAAT CAGTAAACTA 540
GAGGCTCTGG AACGTCTCAC GTCCTTAGCA ACGCTTGAAC TATCGGGTAA CCAAATCCGT 600
AAGCTGGAGG GTCTGGAACG TCTCACGTCC TTAGCAACGC TTGAACTATC GGGTAACCAA 660
ATCCGTAAGC TAGAGGGTCT GGAACGTCTC ACTTCGTTAA CAAAGCTTCG TCTAAGAAGT 720
AACCAAATCA GTAAGCTAGA GGGTCTGGAA CGTCTCACGT CCTTAGCAAC GCTTGAACTA 780
TCGGGTAACC AAATCCGTAA GCTGGAGGGT CTGGAACGTC TCACGTCCTT AGCAACGCTT 840
GAACTGTCGG GTAACCAAAT CAGTAAGCTA GAGGGTCTGG AACGTCTCTC TTCGTTAACA 900
AAGCTTCGTC TAAGAAGTAA CCAGATCAGT AAACTAGAGG GCCTGGAACG TCTCACCTCG 960
CTAACAAAAC TTTCTCTCTC CGATAACCAA ATCAGTAAGC TAGAGGGTCT GGAACGTCTC 1020
ACCTCGTTAG CGGAGCTTTA TCTTTTGGAT AACCAAATCC GTAAGCTGGA GGGCCTGGAA 1080
CGTCTCACCT CGTTAACAAA GCTTCGTCTA AGAAGTAACC AAATCAGTAA ACTAGAGGGC 1140
CTGGATAGTC TCACCTCGCT AACAAAACTT TCTCTCTCCG ATAACCAAAT CAGTAAACTA 1200
GAGGGCCTGG AACGTCTCAC GTCCTTAGCG GAGCTTTATC TTTTGGATAA CCAAATCCGT 1260
AAGCTGGAGG GTCTTGATGG TCTTGCTTCC TTAACAAGGC TTAGTCTAAG GCGCAACCAA 1320
ATCAGTAAGC TGGAAGGACT AGACAGACTA AAGGTTTTGA GAAAACTTGA TGTTTCGGGC 1380
AATGATATTC AATCTATTGA TGATATTAAG CTATTGGCTC CGATTCTGGA GCAAACTTTA 1440
GAAAAACTGA GAATCCATGA CAATCCATTT GTTGCATCAT CAGGCTTGAT ACTCTCTCCT 1500
TATGATAATC ATTTGCCGGA GATTAAAGCT CTTCTTGAAA AAGAAAAAGA AAAACAGAAA 1560

AAGACTTCAG TTGAATATCA CCCATTTTGC AAAGTAATGC TATTGGGAAA TCATTCTTCG 1620
GGTAAAACAA CATTTCTTAG TCAATACGAT ACAAATTATA CGTATCAGAA AAATACACAT 1680
GTGTTGTCGA TACATCGAAG CAATAACCCT AATGCGATCT TTTACGACTT TGGGGGACAG 1740
GACTATTATC ATGGGATTTA CCAAGCCTTT TTTACCACCC AATCGTTATA CCTTCTCTTT 1800
TGGGATGCTA AGAAGGATCG AAACTTTGTG AGCGTAGATG ATAAAGAATA TCAGACTCTT 1860
AATTTCAATC GCCCCTATTG GTTAGGACAG ATAGCCTATG CCTGCAATCG TTGTATGTCC 1920
GTTGGAGGAA ATCCTGATGG CAAGGACACA CCACAGACCA CAGACGATAC AATTATCATT 1980
CAGACTCATG CCGATGAAAC GGGCGCTAAG CAGCAAACCT TAGGCTGTGC AGCCGAGAAT 2040
GGAGTATTGG AAGAAATCTA TGTATCCTTA GAGCCCAAGG CGAATAGTGC CGTACATGCG 2100
CTCAACTATC TGAATGAGCG GGTGCGAGAA GTTGTCGCAA GCAGGAGTAA ATCAATTCAG 2160
ATCACAGAAA AAGATAAGGG ATTGTACGAA GCTCTTCCCA CAATCGCCGG TGATAATAAA 2220
CACATCCCTA TCTCTCTCGA AGCTCTTGCG GCTCAATTGA ATAAGGGAAG AGCTGAAAAT 2280
GATCTTTACA CCATAGAGTA TCTACAGACC GAATTGAACC AGCTTAGTCT GCGAGGGGAG 2340
GTGCTTTACT ATCGTGAGAA TGAGAAGCTG AACAATTATG TCTGGTTAGA TCCGGCAGCT 2400
TTTGTCCAAA TGATTCATGG AGAAATCCTC CAAAAAGACA ACATCAATAG AGGAACAGTT 2460
CCTAAAGACA TTTTTGAATG CAAACTGCAT AATCTAAGTT CCGGAAGTAT ATTTGAAGAA 2520
GATGGCCAAA ATGGTAATAT GATCTTGCAG CTATTATTGG AAGAGCTGAT CGTATATGAA 2580
GATAAGGACT GCTATGTGAT ACCGGGCTAT CTCCCTTTGC ATTCCGATGA CGAAGCCTAT 2640
AAATGGCTTA CTTTGGGATT CGAGAGGCCC AATTTTGTCC TCAAATTCGA ACGTTTTATC 2700
CCCTTTGGCC TGATCAACCA GATTATAGCC TACTATGGCC GGGAAGAAGG TGCTCTAAAG 2760
CGGTATTGGC GAGATCAGGT CATCTTCACA GCAGGCCGTG AGATGGATAG GCAAACGCTT 2820
GAGCAAGAAG AAGAGAAAGA GGGTTTGCCC AAGACGAATG CCGAGGATTA TCAGATCTGG 2880
ATCAAGCTCG ACTTTACCGA CTTGGCCATA TCCGTATTCA TCAAAGAGCA GAGAAAGACA 2940
TCAGCTAAGG ATATGCAGCG GAAAGAGGCT ACTATCCTCA GTGATATGTT GGATATGTAT 3000
TGGAACAATA TCCCTCCGAG GGAGCAAATA GGAGATAAGG ATACGGAGCA AACGAGAAGC 3060
ACTATTCGTG AAACAAACAG AAAGAAGAGA CCCATCCAGG ATCTCTACCT CTCCTGTGCC 3120
CAAGCGGATA AAGATTTGAC GGAGTCTCAT TATATCCATT TGGGCACGCT GGACGATGAA 3180
AGCAAGACTA CGGCGAGGAT TGCAGCCTAT CCGTTGAAGA ACGGCGTTAT CGATAAAGAG 3240
CGGGTGCGAG AAGTATCGAC TCGTCCCTAC AAACATCTTT CCGTCAATAA AAATCTGGCT 3300
ACTGCAAAAC AGATCTTTAT TTCCTATTCC AAAGAGGATC AGACTGAACT GGAGACCTGT 3360
CTGCAATTTT TCAAACCCTT GGAGAAGAAT GGTCAGATCG AGATCTACTA TGATAAGTTG 3420
ACTAAGTTTG AAACACCTAT TCACCCTGAA ATAAGAAAGC GTATTGTCGA AGCCGACTGT 3480
ATAATCGCTT TGATCAGCCA ACGCTATCTG GCCACGGATT ACATCCTGGA TCATGAGTTG 3540
CCTGTATTTC GGGAGTATAA CAAGACCATA GTGCCGATAT TGATCAAGCC TTGTACATTC 3600
GAAGACGATG AGTTCCTTCG GGAGAAATAT TTTGCTCAGA AAGCTCAAAT AATCAATCTT 3660
GGAAAAGAGG GAAAAACCAT TAAAGCTTAT GATAGTATTA CGGCATCAGC CCATCGTGAT 3720
GAAAATTGGG TGGCAGTAGT CAGAGAGTTC AAAGAGAAGA TATTAAGAAT AACAAAACAG 3780
GAGGTAAATA CAGATGAA 3798

(2) INFORMATION FOR SEQ ID NO:252

(i) SEQUENCE CHARACTERISTICS:
(A) LENGTH: 3696 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...3696

(xi) SEQUENCE DESCRIPTION: SEQ ID NO:252

ATGACAACCA AGAAACCCCA AGCCATTTTA GACTTAGAGA AGGCCTATAA CATTGAAATT 60
CCTGATCTCT CCTCACAAGA AGGGATAAGC TGGTCGGTAA ATAGATATTT CAAGCAAGAT 120
TCCTCCGGTG CAGTCGTTGA GCTTTGCTTG CGAGAATGTC AGATAGAAAG CATGACTTGG 180
CTTATTGATT TTCCTGCTCT AAAAAAGCTT GATCTATCGT ATAACCAAAT CAGTAAGCTA 240
GAGGGTCTAG AACGTCTTAC TTCGTTAACA AAACTTCGTC TAAGAAGTAA CCAAATCCGT 300
AAACTAGAGG GCCTGGATAG TCTCACCTCG CTAACAAAAC TTTCTCTCTC CGATAACCAA 360
ATCAGTAAGC TAGAGGGTCT GGAACGTCTC ACCTCGTTAG CGGAGCTTTA TCTTTTGGAT 420
AACCAAATCA GTAAACTAGA GGGTCTGGAA CGTCTCACGT CCTTAGCAAC GCTTGAACTA 480
TCGGGTAACC AAATCCGTAA GCTGGAGGGT CTGGAACGTC TCACGTCCTT AGCAACGCTT 540
GAACTATCGG GTAACCAAAT CCGTAAGCTA GAGGGTCTGG AACGTCTCAC TTCGTTAACA 600
AAGCTTCGTC TAAGAAGTAA CCAAATCAGT AAGCTAGAGG GTCTGGAACG TCTCACGTCC 660
TTAGCAACGC TTGAACTATC GGGTAACCAA ATCCGTAAGC TGGAGGGTCT GGAACGTCTC 720

ACGTCCTTAG CAACGCTTGA ACTGTCGGGT AACCAAATCA GTAAGCTAGA GGGTCTGGAA 780
CGTCTCTCTT CGTTAACAAA GCTTCGTCTA AGAAGTAACC AGATCAGTAA ACTAGAGGGC 840
CTGGAACGTC TCACCTCGCT AACAAAACTT TCTCTCTCCG ATAACCAAAT CAGTAAGCTA 900
GAGGGTCTGG AACGTCTCAC CTCGTTAGCG GAGCTTTATC TTTTGGATAA CCAAATCCGT 960
AAGCTGGAGG GCCTGGAACG TCTCACCTCG TTAACAAAGC TTCGTCTAAG AAGTAACCAA 1020
ATCAGTAAAC TAGAGGGCCT GGATAGTCTC ACCTCGCTAA CAAAACTTTC TCTCTCCGAT 1080
AACCAAATCA GTAAACTAGA GGGCCTGGAA CGTCTCACGT CCTTAGCGGA GCTTTATCTT 1140
TTGGATAACC AAATCCGTAA GCTGGAGGGT CTTGATGGTC TTGCTTCCTT AACAAGGCTT 1200
AGTCTAAGGC GCAACCAAAT CAGTAAGCTG GAAGGACTAG ACAGACTAAA GGTTTTGAGA 1260
AAACTTGATG TTTCGGGCAA TGATATTCAA TCTATTGATG ATATTAAGCT ATTGGCTCCG 1320
ATTCTGGAGC AAACTTTAGA AAAACTGAGA ATCCATGACA ATCCATTTGT TGCATCATCA 1380
GGCTTGATAC TCTCTCCTTA TGATAATCAT TTGCCGGAGA TTAAAGCTCT TCTTGAAAAA 1440
GAAAAAGAAA AACAGAAAAA GACTTCAGTT GAATATCACC CATTTTGCAA AGTAATGCTA 1500
TTGGGAAATC ATTCTTCGGG TAAAACAACA TTTCTTAGTC AATACGATAC AAATTATACG 1560
TATCAGAAAA ATACACATGT GTTGTCGATA CATCGAAGCA ATAACCCTAA TGCGATCTTT 1620
TACGACTTTG GGGGACAGGA CTATTATCAT GGGATTTACC AAGCCTTTTT TACCACCCAA 1680
TCGTTATACC TTCTCTTTTG GGATGCTAAG AAGGATCGAA ACTTTGTGAG CGTAGATGAT 1740
AAAGAATATC AGACTCTTAA TTTCAATCGC CCCTATTGGT TAGGACAGAT AGCCTATGCC 1800
TGCAATCGTT GTATGTCCGT TGGAGGAAAT CCTGATGGCA AGGACACACC ACAGACCACA 1860
GACGATACAA TTATCATTCA GACTCATGCC GATGAAACGG GCGCTAAGCA GCAAACCTTA 1920
GGCTGTGCAG CCGAGAATGG AGTATTGGAA GAAATCTATG TATCCTTAGA GCCCAAGGCG 1980
AATAGTGCCG TACATGCGCT CAACTATCTG AATGAGCGGG TGCGAGAAGT TGTCGCAAGC 2040
AGGAGTAAAT CAATTCAGAT CACAGAAAAA GATAAGGGAT TGTACGAAGC TCTTCCCACA 2100
ATCGCCGGTG ATAATAAACA CATCCCTATC TCTCTCGAAG CTCTTGCGGC TCAATTGAAT 2160
AAGGGAAGAG CTGAAAATGA TCTTTACACC ATAGAGTATC TACAGACCGA ATTGAACCAG 2220
CTTAGTCTGC GAGGGGAGGT GCTTTACTAT CGTGAGAATG AGAAGCTGAA CAATTATGTC 2280
TGGTTAGATC CGGCAGCTTT TGTCCAAATG ATTCATGGAG AAATCCTCCA AAAAGACAAC 2340
ATCAATAGAG GAACAGTTCC TAAAGACATT TTTGAATGCA AACTGCATAA TCTAAGTTCC 2400
GGAAGTATAT TTGAAGAAGA TGGCCAAAAT GGTAATATGA TCTTGCAGCT ATTATTGGAA 2460
GAGCTGATCG TATATGAAGA TAAGGACTGC TATGTGATAC CGGGCTATCT CCCTTTGCAT 2520
TCCGATGACG AAGCCTATAA ATGGCTTACT TTGGGATTCG AGAGGCCCAA TTTTGTCCTC 2580
AAATTCGAAC GTTTTATCCC CTTTGGCCTG ATCAACCAGA TTATAGCCTA CTATGGCCGG 2640
GAAGAAGGTG CTCTAAAGCG GTATTGGCGA GATCAGGTCA TCTTCACAGC AGGCCGTGAG 2700
ATGGATAGGC AAACGCTTGA GCAAGAAGAA GAGAAAGAGG GTTTGCCCAA GACGAATGCC 2760
GAGGATTATC AGATCTGGAT CAAGCTCGAC TTTACCGACT TGGCCATATC CGTATTCATC 2820
AAAGAGCAGA GAAAGACATC AGCTAAGGAT ATGCAGCGGA AAGAGGCTAC TATCCTCAGT 2880
GATATGTTGG ATATGTATTG GAACAATATC CCTCCGAGGG AGCAAATAGG AGATAAGGAT 2940
ACGGAGCAAA CGAGAAGCAC TATTCGTGAA ACAAACAGAA AGAAGAGACC CATCCAGGAT 3000
CTCTACCTCT CCTGTGCCCA AGCGGATAAA GATTTGACGG AGTCTCATTA TATCCATTTG 3060
GGCACGCTGG ACGATGAAAG CAAGACTACG GCGAGGATTG CAGCCTATCC GTTGAAGAAC 3120
GGCGTTATCG ATAAAGAGCG GGTGCCGAGA GTATCGACTC GTCCCTACAA ACATCTTTCC 3180
GTCAATAAAA ATCTGGCTAC TGCAAAACAG ATCTTTATTT CCTATTCCAA AGAGGATCAG 3240
ACTGAACTGG AGACCTGTCT GCAATTTTTC AAACCCTTGG AGAAGAATGG TCAGATCGAG 3300
ATCTACTATG ATAAGTTGAC TAAGTTTGAA ACACCTATTC ACCCTGAAAT AAGAAAGCGT 3360
ATTGTCGAAG CCGACTGTAT AATCGCTTTG ATCAGCCAAC GCTATCTGGC CACGGATTAC 3420
ATCCTGGATC ATGAGTTGCC TGTATTTCGG GAGTATAACA AGACCATAGT GCCGATATTG 3480
ATCAAGCCTT GTACATTCGA AGACGATGAG TTCCTTCGGG AGAAATATTT TGCTCAGAAA 3540
GCTCAAATAA TCAATCTTGG AAAAGAGGGA AAAACCATTA AAGCTTATGA TAGTATTACG 3600
GCATCAGCCC ATCGTGATGA AAATTGGGTG GCAGTAGTCA GAGAGTTCAA AGAGAAGATA 3660
TTAAGAATAA CAAAACAGGA GGTAAATACA GATGAA 3696

(2) INFORMATION FOR SEQ ID NO:253

(i) SEQUENCE CHARACTERISTICS:
(A) LENGTH: 3525 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...3525

(xi) SEQUENCE DESCRIPTION: SEQ ID NO:253

ATGACTTGGC TTATTGATTT TCCTGCTCTA AAAAAGCTTG ATCTATCGTA TAACCAAATC 60
AGTAAGCTAG AGGGTCTAGA ACGTCTTACT TCGTTAACAA AACTTCGTCT AAGAAGTAAC 120
CAAATCCGTA AACTAGAGGG CCTGGATAGT CTCACCTCGC TAACAAAACT TTCTCTCTCC 180
GATAACCAAA TCAGTAAGCT AGAGGGTCTG GAACGTCTCA CCTCGTTAGC GGAGCTTTAT 240
CTTTTGGATA ACCAAATCAG TAAACTAGAG GGTCTGGAAC GTCTCACGTC CTTAGCAACG 300
CTTGAACTAT CGGGTAACCA AATCCGTAAG CTGGAGGGTC TGGAACGTCT CACGTCCTTA 360
GCAACGCTTG AACTATCGGG TAACCAAATC CGTAAGCTAG AGGGTCTGGA ACGTCTCACT 420
TCGTTAACAA AGCTTCGTCT AAGAAGTAAC CAAATCAGTA AGCTAGAGGG TCTGGAACGT 480
CTCACGTCCT TAGCAACGCT TGAACTATCG GGTAACCAAA TCCGTAAGCT GGAGGGTCTG 540
GAACGTCTCA CGTCCTTAGC AACGCTTGAA CTGTCGGGTA ACCAAATCAG TAAGCTAGAG 600
GGTCTGGAAC GTCTCTCTTC GTTAACAAAG CTTCGTCTAA GAAGTAACCA GATCAGTAAA 660
CTAGAGGGCC TGGAACGTCT CACCTCGCTA ACAAAACTTT CTCTCTCCGA TAACCAAATC 720
AGTAAGCTAG AGGGTCTGGA ACGTCTCACC TCGTTAGCGG AGCTTTATCT TTTGGATAAC 780
CAAATCCGTA AGCTGGAGGG CCTGGAACGT CTCACCTCGT TAACAAAGCT TCGTCTAAGA 840
AGTAACCAAA TCAGTAAACT AGAGGGCCTG GATAGTCTCA CCTCGCTAAC AAAACTTTCT 900
CTCTCCGATA ACCAAATCAG TAAACTAGAG GGCCTGGAAC GTCTCACGTC CTTAGCGGAG 960
CTTTATCTTT TGGATAACCA AATCCGTAAG CTGGAGGGTC TTGATGGTCT TGCTTCCTTA 1020
ACAAGGCTTA GTCTAAGGCG CAACCAAATC AGTAAGCTGG AAGGACTAGA CAGACTAAAG 1080
GTTTTGAGAA AACTTGATGT TTCGGCCAAT GATATTCAAT CTATTGATGA TATTAAGCTA 1140
TTGGCTCCGA TTCTGGAGCA AACTTTAGAA AAACTGAGAA TCCATGACAA TCCATTTGTT 1200
GCATCATCAG GCTTGATACT CTCTCCTTAT GATAATCATT TGCCGGAGAT TAAAGCTCTT 1260
CTTGAAAAAG AAAAAGAAAA ACAGAAAAAG ACTTCAGTTG AATATCACCC ATTTTGCAAA 1320
GTAATGCTAT TGGGAAATCA TTCTTCGGGT AAAACAACAT TTCTTAGTCA ATACGATACA 1380
AATTATACGT ATCAGAAAAA TACACATGTG TTGTCGATAC ATCGAAGCAA TAACCCTAAT 1440
GCGATCTTTT ACGACTTTGG GGGACAGGAC TATTATCATG GGATTTACCA AGCCTTTTTT 1500
ACCACCCAAT CGTTATACCT TCTCTTTTGG GATGCTAAGA AGGATCGAAA CTTTGTGAGC 1560
GTAGATGATA AAGAATATCA GACTCTTAAT TTCAATCGCC CCTATTGGTT AGGACAGATA 1620
GCCTATGCCT GCAATCGTTG TATGTCCGTT GGAGGAAATC CTGATGGCAA GGACACACCA 1680
CAGACCACAG ACGATACAAT TATCATTCAG ACTCATGCCG ATGAAACGGG CGCTAAGCAG 1740
CAAACCTTAG GCTGTGCAGC CGAGAATGGA GTATTGGAAG AAATCTATGT ATCCTTAGAG 1800
CCCAAGGCGA ATAGTGCCGT ACATGCGCTC AACTATCTGA ATGAGCGGGT GCGAGAAGTT 1860
GTCGCAAGCA GGAGTAAATC AATTCAGATC ACAGAAAAAG ATAAGGGATT GTACGAAGCT 1920
CTTCCCACAA TCGCCGGTGA TAATAAACAC ATCCCTATCT CTCTCGAAGC TCTTGCGGCT 1980
CAATTGAATA AGGGAAGAGC TGAAAATGAT CTTTACACCA TAGAGTATCT ACAGACCGAA 2040
TTGAACCAGC TTAGTCTGCG AGGGGAGGTG CTTTACTATC GTGAGAATGA GAAGCTGAAC 2100
AATTATGTCT GGTTAGATCC GGCAGCTTTT GTCCAAATGA TTCATGGAGA AATCCTCCAA 2160
AAAGACAACA TCAATAGAGG AACAGTTCCT AAAGACATTT TTGAATGCAA ACTGCATAAT 2220
CTAAGTTCCG GAAGTATATT TGAAGAAGAT GGCCAAAATG GTAATATGAT CTTGCAGCTA 2280
TTATTGGAAG AGCTGATCGT ATATGAAGAT AAGGACTGCT ATGTGATACC GGGCTATCTC 2340
CCTTTGCATT CCGATGACGA AGCCTATAAA TGGCTTACTT TGGGATTCGA GAGGCCCAAT 2400
TTTGTCCTCA AATTCGAACG TTTTATCCCC TTTGGCCTGA TCAACCAGAT TATAGCCTAC 2460
TATGGCCGGG AAGAAGGTGC TCTAAAGCGG TATTGGCGAG ATCAGGTCAT CTTCACAGCA 2520
GGCCGTGAGA TGGATAGGCA AACGCTTGAG CAAGAAGAAG AGAAAGAGGG TTTGCCCAAG 2580
ACGAATGCCG AGGATTATCA GATCTGGATC AAGCTCGACT TTACCGACTT GGCCATATCC 2640
GTATTCATCA AAGAGCAGAG AAAGACATCA GCTAAGGATA TGCAGCGGAA AGAGGCTACT 2700
ATCCTCAGTG ATATGTTGGA TATGTATTGG AACAATATCC CTCCGAGGGA GCAAATAGGA 2760
GATAAGGATA CGGAGCAAAC GAGAAGCACT ATTCGTGAAA CAAACAGAAA GAAGAGACCC 2820
ATCCAGGATC TCTACCTCTC CTGTGCCCAA GCGGATAAAG ATTTGACGGA GTCTCATTAT 2880
ATCCATTTGG GCACGCTGGA CGATGAAAGC AAGACTACGG CGAGGATTGC AGCCTATCCG 2940
TTGAAGAACG GCGTTATCGA TAAAGAGCGG GTGCGAGAAG TATCGACTCG TCCCTACAAA 3000
CATCTTTCCG TCAATAAAAA TCTGGCTACT GCAAAACAGA TCTTTATTTC CTATTCCAAA 3060
GAGGATCAGA CTGAACTGGA GACCTGTCTG CAATTTTTCA AACCCTTGGA GAAGAATGGT 3120
CAGATCGAGA TCTACTATGA TAAGTTGACT AAGTTTGAAA CACCTATTCA CCCTGAAATA 3180
AGAAAGCGTA TTGTCGAAGC CGACTGTATA ATCGCTTTGA TCAGCCAACG CTATCTGGCC 3240
ACGGATTACA TCCTGGATCA TGAGTTGCCT GTATTTCGGG AGTATAACAA GACCATAGTG 3300
CCGATATTGA TCAAGCCTTG TACATTCGAA GACGATGAGT TCCTTCGGGA GAAATATTTT 3360
GCTCAGAAAG CTCAAATAAT CAATCTTGGA AAAGAGGGAA AAACCATTAA AGCTTATGAT 3420
AGTATTACGG CATCAGCCCA TCGTGATGAA AATTGGGTGG CAGTAGTCAG AGAGTTCAAA 3480
GAGAAGATAT TAAGAATAAC AAAACAGGAG GTAAATACAG ATGAA 3525

(2) INFORMATION FOR SEQ ID NO:254

(i) SEQUENCE CHARACTERISTICS:
(A) LENGTH: 687 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...687

(xi) SEQUENCE DESCRIPTION: SEQ ID NO:254

ATGATGAAAA AAGCATTTGT TTTCGTACTA CTGGTTTGCC TATTCTCCTC GTTCAGCAGT 60
TCCGCCCAAA CAACGACGAA CAGTAGCCGG AGTTATTTTA CAGGACGAAT CGAGAAGGTG 120
AGTTTGAACT TAGGGGTCCC CCCCGTAAGC ACAGAGGTTT GGGGAATGAC CCATGATGCG 180
AACGGTCTCC CTTTCGAAAT ACCTATCTCT TTCAGTCGTT TCAACAGCCA GGGAGATATA 240
GCTACCACTT ATTACATAGC GAATAGCGAG GCAACTTTGA ATGAATGGTG CGACTATGCA 300
CACCCGGGCG GCATCGTGAG GGTAGAAGGT CGTTTTTGGA AAATGACTTA CAACATACCA 360
ACCTACAATG CAGTCTGCAC CCGGATTACA TTCGAAAATC AAGAAATAGA AGGAACGATC 420
GTCTTGATAC CCAAGCCCAA AGTCTCGCTG CCTCATGTGT CGGAATCGGT GCCTTGCATC 480
CGAACCGAAG CCGGGAGGGA ATTTATCCTT TGCGAAGAAG ACGACACCTT TGTGTCTCAC 540
GATGGTAACG AAGTAACGAT AGGCGGTAAA CCTTTCTTGC TCAATACCAA CGTAAAGATT 600
GTGGGGGACG TATCTCAAAA GTATGCCGTG GGGGTAGGAG AAATTCGATT CCTGCAGATT 660
TGTGCCCAAA CAGTATCACA ACAAAAA 687

(2) INFORMATION FOR SEQ ID NO:255

(i) SEQUENCE CHARACTERISTICS:
(A) LENGTH: 684 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...684

(xi) SEQUENCE DESCRIPTION: SEQ ID NO:255

ATGAAAAAAG CATTTGTTTT CGTACTACTG GTTTGCCTAT TCTCCTCGTT CAGCAGTTCC 60
GCCCAAACAA CGACGAACAG TAGCCGGAGT TATTTTACAG GACGAATCGA GAAGGTGAGT 120
TTGAACTTAG GGGTCCCCCC CGTAAGCACA GAGGTTTGGG GAATGACCCA TGATGCGAAC 180
GGTCTCCCTT TCGAAATACC TATCTCTTTC AGTCGTTTCA ACAGCCAGGG AGATATAGCT 240
ACCACTTATT ACATAGCGAA TAGCGAGGCA ACTTTGAATG AATGGTGCGA CTATGCACAC 300
CCGGGCGGCA TCGTGAGGGT AGAAGGTCGT TTTTGGAAAA TGACTTACAA CATACCAACC 360
TACAATGCAG TCTGCACCCG GATTACATTC GAAAATCAAG AAATAGAAGG AACGATCGTC 420
TTGATACCCA AGCCCAAAGT CTCGCTGCCT CATGTGTCGG AATCGGTGCC TTGCATCCGA 480
ACCGAAGCCG GGAGGGAATT TATCCTTTGC GAAGAAGACG ACACCTTTGT GTCTCACGAT 540
GGTAACGAAG TAACGATAGG CGGTAAACCT TTCTTGCTCA ATACCAACGT AAAGATTGTG 600
GGGGACGTAT CTCAAAAGTA TGCCGTGGGG GTAGGAGAAA TTCGATTCCT GCAGATTTGT 660
GCCCAAACAG TATCACAACA AAAA 684

(2) INFORMATION FOR SEQ ID NO:256

(i) SEQUENCE CHARACTERISTICS:
(A) LENGTH: 1620 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:

(A) ORGANISM: PORYPHYROMONAS GINGIVALIS

(ix) FEATURE:
(A) NAME/KEY: misc_feature
(B) LOCATION 1...1620

(xi) SEQUENCE DESCRIPTION: SEQ ID NO:256

ATGAAGACAA AAGTTTTACG CAAATTCGTG GTGGCGGCTT TCGCCGTCGC AACCCTCTGT 60
CCTCTCGCCC AAGCGCAGAC GATGGGAGGA GATGATGTCA AGGTGGTCCA GTACAATCAG 120
GAAAAACTGG TACAAACGAG GATGAGTGTG GCGGACAACG GATGGATCTA TGTAATGACC 180
CACAGTGGAT ACGACACCGG CAATAGCAAT GTGAAGATCT TCCGCTCCAA AGACCAAGGT 240
GCCACATACC AAAAGTTGAG GGATTGGGAT CCATCGGATG ATTATCAGTT TCAAGACTTC 300
GATATCGTGG TAACGGGTAA GAATGAATCC GACATCAAGA TTTGGTCGGT AGAGCTCATG 360
AATAAGCCCG GAGGATATAA GAGTAGAGTT GCGGTCTTCA GTCGCGATGC CAACGCGCAG 420
AATGCGAAAC TCGTGTATAA GGAAGACTTC TCCAATGTGC AGTTGTACGA TGTGGATATA 480
GCCTCCAACT ATCGTTCGCC TTCTTCTCTT AACAATGGTG GCAACCCTTT TGCTTTGGCT 540
TTCGCTTACA CCGGCTTCAA CAATACGCAC AAAATAAGTT TTGTGGACTA TGTGTTCTCT 600
CTGAATGGAG GGCAAAATTT CAATAAAAAC TTACTCTTCA GTCAAGATGG AGAGAAGAAA 660
ATTGACAAGG TGGATCTCTC ATTGGGTAGC ACCTCTGAAT CCATGGGTCA CAATGCCTGG 720
CCGCTAATGG GTGTGGTATT CGAAATGAAT AAACAAGGGG GAAAAAGCGA TATCGGTTTC 780
TTGTCGAACT TTGTCGACAA TGATCCCGAA TTTCAGTGGT CAGGCCCTAT AAAAGTGAGT 840
GAAAGCGACA TGTCGTTCAG CCCCAAAATC CAAATGTTGC TGGACGAGGA TAACAATACG 900
ATCAATGGGG AGAGTTGCCA CAACTTCATG ATTACGTACA GCGATTATGA TTCTGAATAT 960
TCGGATTGGG ACATTCGGTA TGTATATCCC AAGAAATCGT TCAAGTATGA AAAAGGAAAA 1020
ACTCCGACTA TGGATGATCT GGTGGAAGCT TTCCTTACAG CTTCGTACCA GAGTGAGACC 1080
AACTCGGGGC TGGGGTATGA CAAGAACGCC AATCACTACC TGATTACATA TGCCAAAAAA 1140
GAAGAGAACG GTACGAACAC GCTGAAATAC CGCTGGGCCA ATTATGACAA GATTCATAAC 1200
AAAGATTTGT GGAGCGACAC ATTTACGTAT ACATCATCTG CCAATGCTCT CTACACACCT 1260
CAAGTAGACA TCAATCCGAC CAAGGGTCTC GTGTGCTGGT CATGGGTGGA ATATCTGCCG 1320
GGCAAACGGA TCGTTTGGTC TGATACGCAG TGGACCCATG CCAACGGTGT AGAAGACATC 1380
GTAATGCAAG AAGGCAGCAT GAAGCTCTAC CCGAATCCGG CTCAAGAATA TGCTGTGATT 1440
AGCCTGCCGA CGGCAGCAAA CTGCAAGGCT GTTGTTTACG ATATGCAGGG CAGAGTAGTC 1500
GCTGAGGCTT CTTTCTCCGG CAACGAATAC AGGCTGAACG TGCAGCACTT GGCTAAGGGT 1560
ACGTACATAC TCAAGGTCGT ATCCGATACG GAGCGTTTCG TAGAGAAGCT CATCGTGGAA 1620

(2) INFORMATION FOR SEQ ID NO:257

(i) SEQUENCE CHARACTERISTICS:
(A) LENGTH: 2313 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2313

(xi) SEQUENCE DESCRIPTION: SEQ ID NO:257

ATGCTGACGA TCCGAAACTT CCTCCTCTTT TGTTGTCTGT CGCTGATAGC GTTTGCTGCC 60
GATGCACAAA GCTCTGTCTC TTCGGGTAGA CGACTGACAG AATATGTCAA TCCCTTTATC 120
GGAACGGCCA ATTACGGTAC CACGAATCCG GGAGCAGTAT TGCCCAATGG GTTGATGAGC 180
GTTACCCCTT TCAATGTCAG CGGATCGACA GAGAATCGCT TCGACAAAGA TTCGCGTTGG 240
TGGAGTGCGC CTTATTCGGC CGACAATAGT TACTGCATCG GTTTCAGCCA TGTGAATCTG 300
AGTGGAGTAG GCTGTCCCGA ACTGAGTGGA ATACTGCTGA TGGCCACTTC CGGCACATTC 360
GATCCTGATT ACTGCTGCTA TGGCTCTTCG CTCAGTCGAG AATATGCGCG CCCGGGAGAA 420
TACAAGGCTG TATTGGACAA ATACGGTATA GATGCAGCCG TGACCGTAAC CGAGCGGACT 480
GCTTTGACCG AATTTGCTTT TCCCGAAGGA GAAGGCCATA TCCTGCTGAA CCTGGGACAG 540
GCCCTAAGCA ATGAATCGGG AGCCTCTGTT CGATTCTTAA ACGACTCCAC AGTCGTCGGC 600
AGCAGGCTGA TGGGGACGTT CTGCTACAAT CCGCAAGCAG TTTTTCGTCA GTATTTCGTA 660
CTTCAGGTGA GTCGGCGACC GATCTCTGCC GGCTATTGGA AGAAGCAGCC TCCTATGACA 720
GTGGAAGCCC AATGGGATTC GACTGCAGGG AAATATAAGC AGTACGACGG CTACAAGCGT 780
GAGATGAGCG GTGATGACAT CGGTGTCCGA TTCTCGTTCA ACTGCGATCA GGGGGAAAAG 840
ATCTATGTAC GATCGGCCGT TTCATTCGTC AGCGAAGCCA ATGCGCTCTA TAATCTGGAA 900

GCGGAGCAAG AAGAGGTGTT CAAAAGTGTC GGAGGGAATC CGGCCAAGGC TTTCTCCGCT 960
ATACGCTCTC GCGCTATAGA GCGTTGGGAG GAAGCCCTCG GTACGGTGGA AGTGGAAGGA 1020
GGCACACCGG ATGAAAAGAC GATATTCTAT ACCGCACTCT ATCACCTGCT GATACATCCG 1080
AATATCCTAC AAGATGCCAA TGGAGAATAT CCTATGATGG GCAGTGGCAA AACGGGTAAT 1140
ACGGCTCACG ACCGCTACAC CGTGTTCTCT CTTTGGGACA CGTACCGCAA TGTACACCCG 1200
CTGCTCTGCC TCCTCTATCC GGAGAAGCAG TTGGATATGG TACGGACACT GATCGACATG 1260
TACCGAGAGA GCGGGTGGCT GCCGAGATGG GAGCTGTACG GACAGGAGAC CCTGACGATG 1320
GAGGGCGACC CCTCGCTTAT CGTCATCAAT GACACTTGGC AAAGGGGCCT TCGTGCTTTC 1380
GATACGGCAA CGGCCTATGA AGCCATGAAA AAAAATGCTT CTTCGGCAGG AGCGACCCAT 1440
CCGATCCGTC CTGACAACGA CGACTATCTC ACCCTCGGCT TCGTACCGCT TCGCGAACAG 1500
TACGACAATT CCGTATCGCA TGCGCTGGAA TACTATCTGG CCGACTGGAA TCTGTCCCGG 1560
TTTGCCCACG CACTTGGGCA TAAAGAAGAC GCAGCTCTAT TCGGAAAACG CTCGTTGGGC 1620
TACAGACACT ATTATAATAA GGAGTATGGT ATGCTGTGTC CATTGCTGCC GGATGGATCA 1680
TTCCTCACTC CTTTCGATCC CAAACAGGGT GAAAACTTCG AGCCTAATCC CGGTTTCCAC 1740
GAGGGCAGTG CTTATAACTA TGCCTTTTTC GTTCCCCACG ATATACAAGG GCTTGCCCGG 1800
CTGATGGGAG GAGCAAAGGT TTTTTCGGAA AGGTTGCAGA AAGTCTTCGA TGAAGGATAT 1860
TATGATCCGA CCAACGAGCC GGACATCGCC TATCCTTACC TCTTCTCCTA TTTCCCCAAG 1920
GAAGCATGGC GAACGCAGAA ATTGACCCGG GAGTTGATAG ACAAACATTT TTGCAATGCT 1980
CCTAACGGCT TGCCCGGTAA TGACGATGCC GGTACGATGA GTGCTTGGCT TGTCTATTCC 2040
ATGCTGGGAT TCTACCCTGA CTGTCCGGGC AGCCCCACCT ATACACTGAC CTCGCCGGTA 2100
TTCCCCCGAG TTAGGATTCG GCTCAATCCG CAGTATTATC CTCAGGGGGA GTTGATCATT 2160
ACGACCAATA CAGAGAATCA ACCGACAGAT TCCATTTACA TCCATACGGT TTCTCTTGGC 2220
AATAAAACAC TTCCGCATGG AACAAGGCAT ATCAGCCATG CCGATTTGGT GCGCTGCGGT 2280
CACCTCCGTT ACGAACTAAG CAATCGTCCT CGA 2313

(2) INFORMATION FOR SEQ ID NO:258

(i) SEQUENCE CHARACTERISTICS:
(A) LENGTH: 2328 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2328

(xi) SEQUENCE DESCRIPTION: SEQ ID NO:258

ATGTGCAAAA TAAGATTCAG CCTCTTGCAG GCTCTTGTCG TCTGCTTATT GTTCACCTCT 60
TTTTCTCTCC AAGCTCAAGA GGAAGGTATT TGGAATACCC TCTTGGCTAT CCACAAGACG 120
GAAAAAGCCG TAGAAACGCC CAAGAAAGTC TTTGCCGTAG CCAACGGAGT ACTTTACTCG 180
GTGGGCAAAG AAGCTCCCCA TGAGGCAAAG ATCTTCGACC GTATCAGCGG ACTCAGCGAT 240
ACATCGGTAA GCAGCATAGC CTACTCCGAG CAACTAAAAT CCTTGGTCAT ATACTATGCA 300
TCAGGCAATA TCGACATCTT GGACGAAGCA GGCCGTGTGA CCAACGTACC TGCATTGAAA 360
GACAATATCG ATCTGATAGA CAAAACGCTC AATCGCCTTT TGATCGTAGG CAACAGGGCT 420
TATTTGGCAG GAGGATTCGG CCTCTCCGTT CTGGATGTCG CCGAAGCTCG CATACCGGCT 480
ACCTACGCCA AGGGAACTAA GGTGACCGAT GTGGCTAAGT TGGACAATGA TCGCTTGCTG 540
ATGCTGAAAG AAGGGCAGCT CTTCATCGGA AAAGAGACCG ATAACCTGCA AGATCCGGCC 600
GCATGGACAG CCTTGTCTTT GAATTTGCCG ATGGGCTCGG TCACCGGTCT GGGCATTGTC 660
GGGGAAGACA TCTGTTTCCT GCTCGCCGAT GGCCGTGTAT ATGTCGCTGC AAACCAATCG 720
TTTGAGCCGG AGCTATTGCT CTCTTCCTCC GCCGATTCAC GACTGTATGT GACGGATCGT 780
GGTCTGTTCA TCTGTGCCGA GAATCGAATT TATTTCATAG AAAAAGGTCG CAAAACGACA 840
CAATTTCCTA TAGCCGACGT CCTTGGTGTC GGTGCCATGA ACGAAAGCAA TACGGCATAC 900
ATAGCATTGG GAGAAGAAGG TTTGGCTTCA CTTCTTCTCG CAGAGGGAAG TACGGCCGAA 960
GCCATGCCTG TAGCATTCGA CGGACCGGGG GACAATGATT TCTACGAGAT GCGGTTTAGT 1020
CACGGACGTC TGTATGCAGC CAGCGGACTC TGGGGAACAA ACCTGATGGG ACATGCCGGT 1080
ATGGTGAAGC TATACGACGG CAACCGATGG ACTAACTTCG ACAAGAAGAC CGTACAGGAA 1140
CAGTTGGGCG GCGGATTCAG TTTCAATGAT GCTATCGATA TAGCTGTTTC CAACGGAGAC 1200
CCCGATCACT TTTTTGTCGG TACATGGGGA AACGGTCTGT TCGAATTCAA GGATGGCAAA 1260
GCGATAGCTC GCTATTCGGG AAACGAAACT GCTATCGCAG AATGTAATCC CGGAGATGCC 1320
CGTGTGAAAG CGATTGCCTT TGACAATAAG GGCAACCTCT GGGGGACGCT CGGTGCCGTA 1380
GGCAAGAACA TCTTCATGTA CGATCCGCAG AGTAGCACAT GGCATTCTTT CAGCTATCCG 1440
GATGTAGCCA ATCTGGCCTC CTTCGGCAAT ATGATTATCC TACCCAACGG AGACAAATGG 1500
GTAAATATCC TTCACCGTAG TGGCGGATCC ACGCGCAAAG GTGTCTTGAT CTTCAACGAT 1560

CGGGGTACAC CGGAAACGAC TTCGGACGAC AGCCATCTTT ACGTCGAGCA GTTTGTCAAT 1620
CGCCTCGGGG CAGCCATAGG ACATAAGACT ATCTATGCAA TGGCCGTCGA TCATAACGGC 1680
TCTGTCTGGA TGGGATCGGA TATAGGCATT TTCGGCGTCT ACAATGCAGC CGGAGTATTG 1740
TCCTCGACTT CTACCCCTAT CGCTGTTCGG CCGGTCGGAG GAGAAGAACC CAATTTGTAC 1800
TATGTGCTGG ACAAGGTGAC GGTGACAGAC ATCGTCGTGG ACAAACTCAA TCACAAATGG 1860
GTTGCCACCC AAGGGACAGG ACTCTATCTC CTTTCGGAAG ATTGCAGTAA GATCCTCGCG 1920
CAATTTACCG TAGAAAACAG CCCTTTGCTT TCTAACAACA TACTATCCCT GGCCTTAAAT 1980
GACGATAACG GACTGCTGTA CATCGGTACG GCGGACGGAC TGATGACGTT CCAAACGGGT 2040
ACGGGGAGTG GATCAGCTTC CGAACTGGAC GGCGTCTATG TATACCCCAA TCCGCTAAGG 2100
CCGGAATATC CCGATGGCGT CACCATTGCC GGACTGCAAG CCGGCTGTAG TGTCAAAATC 2160
ACCGATACCA CCGGCAGACT GCTATACCAG ACTGAGAGCG TAACCACCGA AGTCAAATGG 2220
AATGCTCGAG GTGCCGATGG CAATAGGGTA GCTTCGGGCG TATATGCCGT TGCAGTGTAC 2280
GATCCGGTAT CGAAAAAGTC CAAACTAATT CGCTTCGCAG TGATTCGC 2328

(2) INFORMATION FOR SEQ ID NO:259

(i) SEQUENCE CHARACTERISTICS:
(A) LENGTH: 3474 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...3474

(xi) SEQUENCE DESCRIPTION: SEQ ID NO:259

ATGAAACGAA TACTTCCAAT AGTCGCATTC CTTTCTCTCT TCCTTGCCCT TGCTTTGCCT 60
GCGAAAGCGC AACGAGCTAT GGGGAAGACG GCCGACCGTT CGCTAATGGC TTCGGGACAT 120
TGGGTCAAGA TACGTGTCGA TGCAAGTGGA GTGTATCGCC TTACGGACGA ACAGCTCCGT 180
GCCAATGGCT TCTCCGATCC GTCCAAGGTA GGTGTGTTCG GTTATGGTGG AGGGGTGCTT 240
CCCGAAGATC TGAGCCGGAT CACGACAGAC GATTTGCCTC CGGTACCGGT ACTCCGTCAG 300
GGCAATGCGC TGTATTTCTA TGCCGTGGGC CCGGTGACAT GGTTCTACAA TCCGGCCAAA 360
ACCACCATGG AGCATACGGT GAATACATAC AGTACGCATG GCTACTACTT CCTGTCGGAT 420
GCTGCCGGAG CACCTTTGCA GATGTCCCAA TATACGGGTG GAGGTGCGTC GGCCGAGGCT 480
TTGATCGACT ACTACGATGA GCTGATGCTC CATGAACAGG AATTGTATTC GCCCAAAGAA 540
TCGGGACGAG ATCTGTATGG CGAGTCTTTC AGTGCAGTCA ATACGCGTAC GGTCAAGTTC 600
CCTTTGAGGG GCAACACCCG CTCGTCTGGC GAACTCGGTA CCGTATTCTC ATACATAGCC 660
AAGGCCAGAT CGGCCGGTGG CGGCCGTGAG ATGTCGCTCT CGGCGAATGG CATTCTGATC 720
TTCAGCGATC CTTTTTCCAT GACATCGAAT GAAGTCTCCA ATTCCTATTT GGCCGGCAAG 780
AAGCGTCGTC TCTATCACAG TACGCCGATG AACAGCTTGG TCAATGAGTT GCGCTTGGAC 840
GCGAACTATA GCATGACAGG AGATGCGGTC AATCTGGATT TCATAGAGGT GGCTACACAG 900
AACGACCTCC GGTACGATGG CGCACCCATG CATATCAGGC GGTTTTCCAA TTTGCCCGTT 960
TTGGGGGGCG AGTCCTGCCG GTTCGTTATC AGTGAGGTGC CGGAGTCTCT GGTGGTTTTG 1020
CAGGCCAATT CTTCCCTGAC AGCATCGCTT GTTCCCGTTA AGACTGTCGG GGATAAGACC 1080
ATTGAGTTCG TGGCTCCGCC GAAGGGTCAG GATCGTAGGA CTATCAATAC GTTTTATGCC 1140
GTGGACTTGT CACAGGCTTC TGCTCCGGAG ATCCTCGGAG CGGTACCCAA TCAAAACCTG 1200
CATGGAGAGG AAATCCCTGA TCTGATCATT GTCTCTACTC AGGCGCTCCT CCTTGAGGCT 1260
GATCGACTGG CCACCTATCG TAGAGAGAAA AACGGGCTGA AGGTTTTGGT CGTGTTGCAG 1320
GAACAGGTGT TCAACGAGTT TTCGGGTGGA ACTCCCGATG CTACAGCATA CCGCCTCTTT 1380
GCCAAAATGT TCTACGACAG ATGGAAGGCA AATGCACCTG TGGGAGAGAC CTTCCCGATG 1440
CAAATGCTTC TCTTCGGTGA TGGGGCTCAT GACAACAGGA AGGTCTCCGT AGCTTGGCAG 1500
AAACCGTATC TCCAACAAAC GGAGTTCTTG CTGACATTCC AAGCCGTCAA TTCGACGAAC 1560
GTAAACAGTT ATGTGACGGA TGATTACTTC GGCTTGCTGG ATGATCAGCC GGCCTCGGTC 1620
AATATCGGTT GGCGCAATTA TAATATGGCT GTAGGGCGAT TCCCCGTACG TACTCCGGCC 1680
GAAGCTCGCA TCGCAGTGGA CAAGACCATC CGATATGAGG AGGATCGAGA GAGTGGTGCC 1740
TGGCGTATTC GTGCCTGTTT TGCGGCAGAC AACGGGGACA AGCACGCAAC CGAGACTTCC 1800
CGTTTGATCG ATACCGTCAA GCGTTATGCT CCTGCCATCA TGCCGGTACG CGCCTTTCAG 1860
GACGTATATC CGCATGTCAT CGAGAACGGG TTGCACAGCA TTCCGGGTGC AAAGAAAAAG 1920
ATGCTGGAAA CCCTTCAGTC GGGTATTATC CTGCTTAATT ATGCTGGTCA TGGCGGTCCT 1980
GCCGGATGGT CGGACGAGCA TTTGCTGACG CTCAACGATA TACACAAATT CAATTATAAG 2040
CATATGCCCA TTTGGATTAC TGCCACGTGC GACTTTGCCA ACTATGACAG TCAGACGACC 2100
TCGGCAGGGG AGGAGGTTTT CCTCCATGAG AAGAGTGGCA CTCCGATCAT GTTCTCGACT 2160
ACGCGTGTCG TTTACAATAC GCAGAATGAG AAGATCAATG GTTTTATGCT TCGGCGTATG 2220

TTCGAGAAAG CTAAGGATGG GCGTTATCGT ACGATGGGCG AGATTATCCG ATCGGCCAAA 2280
CAGGGGATGC TCAGTACTGT TTTCCCCGAT TCGATCAACC AGTTGAGTTT CTTTCTGATG 2340
GGTGATCCGT CCGTGCGTAT GAATCTTCCT ACCCACAAAG TGCAATTGAC CGCAATCAAC 2400
GGGCAGGATC CCGAAGGGCA GTATGGAACT ATTATGCTCA AGTCTTTGGA ACGGGTAGCT 2460
CTGAAGGGTA AGGTAACCGA TGAAAAGGGG ACATTCGACG AGACATTCAG TGGCAAGGTT 2520
TTCCTGACCG TCTTCGATGG CAGAAAGAAA ATGACAGCTT TGGAAGAGGA GGGAAACGAT 2580
CTCTCTCTTG TATATTATGA CTATCCTAAC GTGATGTATG CCGGTATTGC CGAGGTGAAA 2640
GACGGACTCT TCGAAACTTC GTTTATCGTA CCCAAGGATG TGAACTATTC CGAGCACGAA 2700
GGCCGGATCA ATCTTTATGC TTATAACGAG AGCACAAAGG CGGAAGCCAT GGGGGTAGAC 2760
TTCTCCATCA GAGTCCAACC GGGTATTCCT GATGAGGTAA CGGAAGATAA TACACCGCCT 2820
GAAATCATAA GCTGCTTCCT CAATGACAGT ACATTCCGAT CGGGAGATGA GGTTAATCCT 2880
ACTCCTCTGT TTATGGCCGA AGTATTCGAC TTGAATGGAA TCAATATCAC GGGTAGCGGA 2940
GTAGGGCATG ATATTACGCT TTGTATCGAT GGCCGTGCCG ACCTGACCTA CAACCTCAAT 3000
GCATATTTCA CAAGTTCGGC TACGGATGCA GGTGTGGGCA CTATTCTCTT CATGATACCG 3060
GCTTTGGCCG AAGGAGATCA TACTGCCCGA CTGACGGTTT GGGACATTTT CAATAATGCC 3120
GTCCATCATG ACTTTTCATT CAGAGTGGTA GATGGCATTG CTCCGGATGT GGCTGATGTG 3180
ATTCTATTCC CGAATCCGGT ACGCGAGAGT GCTACGTTCC GAATCTTCCA CAATCCCCCC 3240
GGAAGCGATT TGAACGTGGC CCTGGAGATC TATGACTTCA CCGGTCGTCT TGTGAACAGT 3300
TTGCCAGTCA AGACCTATTC GTCTTCCTAC GGAGAACCTA TAGAGATCAA GTGGGATCTG 3360
ACCTCCAAAT ACGGAGTGAA GATCGGAAAC GGATTCTACC TCTATCGTTG TGTGGTGAAC 3420
TCTCCCGGAG GACAGACGGC CTCCATGGCC AAGAAAATGA TCGTGGTAGG ACAA 3474

(2) INFORMATION FOR SEQ ID NO:260

(i) SEQUENCE CHARACTERISTICS:
(A) LENGTH: 2883 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...2883

(xi) SEQUENCE DESCRIPTION: SEQ ID NO:260

ATGAAGAAAC TTTTCCCATT ATTATTACTC ATATTGTCGA TTTTGGTCGG ATGTGGAAAA 60
AAAGAAAAAC ACTCTGTAAC TGAAATCGCC CGAGAGAAAA AGCGTATTAC TGCATTGCTG 120
TACGAAAAAG AACTCCCCAC TGATTCTGTT AAACAGCTTT ACGAGAACAG CGTACAGAAC 180
AAGAACCTTG TGGGACAAAT GTTATTCGCG ATCGAGGTCG GCAAACGGAT CCGTAATATG 240
TCCCAATACA CAGATGCGAT GCTATATCAC CAAAAAGGGT TGAACGCTGC ATTGAATCTA 300
AGGGACACCA TCGTAGCCGC ACAAGCATGG AATCATCTCG GAACGGATAG CCGTCGTATC 360
GGTGCTTTGG CAGAAGCTTC GGATTATCAT TACAAGGCTC TTTCCTTGAT AGAATCTTTT 420
AGCGGAAACC AGAATAGGCC TGCGATCAAA GCCAGATCGG CGGCCCTGAA CGGCATCGGC 480
AACATCAATC TTGAGTTAGG ATACCATGAT GAGGCCGAAA AGAATTTCCT GAAAGCACTG 540
CAAGGTGAGA AAGAACTCGA CAGTCCTCTC GGGCAAGCTA TCAACTATGC GAACCTCGGA 600
CGTATCTATC GACAACGCAA AGAATACGAC AAGGCTCGTA CCTACTTCCT CTTGTCTCTG 660
GAGCAGAACA ATATGGCAGA GAATCTGATG GGTATCGGAC TCTGTAGCAT CAATCTCGGA 720
GAAGTAGACG AAGAAAAAGG GGATTATCAA AAGGCTTTGC AAGAGTATGC CACGGCATAC 780
AAACTGATGG AACAGTTGTC CGATCGATGG CACTGGCTGA ATTCCTGTAT CCCGATGGCA 840
CGTATCAATC TCAAACAAGG TAACGAAAGG CTCTACCAGC ATTTCATTTC TTTGGCCGAA 900
GGGACTGCGA AAGAAATTAA TTCGACTTCA CATCTGATAG AAATATACAA TCTTCAATAC 960
GAGAATCTCG AGCGTAAAAA AGAATACAAA CAAGCCCTCG AAGCATTCTG TCTGAGCAAG 1020
ACGTTGAGCG ACAGCATGTC CATTGCGCAC AAGGTCAGCA GCATACAAGA AACGCGATTC 1080
AACTACGAAC GAAACAAGTC CCAAAAAGAG CTTGAAGAAA TACAGCAAGT AAGCAAGGCA 1140
AAACAAGAGA AATCGAAGTT TATCCTCTTG AGCACTCTTT TTGCCCTTTT CATCTCGATT 1200
CTTTTGATTT CTGTTCTGAC ATATGCATAC CGTCAGGGCA AGAAGCATAA CAAGCTGATC 1260
AAAGAGACGG ATAAACTTCG CTCCGGCTTT TTCACCGGTA TTACACACGA ATTTCGTACG 1320
CCTATCACCG TCATACAAGG TTTGAATGAG AAAATGAGTT CAAGTCCTGA TCTCCAAGCA 1380
TCGGACAGAA CCGAGCTGCA CAAGATAATA GACAGACAGA GTAGCCATAT GCTGAATTTG 1440
GTGAACCAGC TGTTCGATAT TTGCAAGATC AGAAGCGGAG TATCCACGCC CGAATGGCGC 1500
AATGGCGACA TCGTCTCCTT CGTACAGATT CTCATCGATT CGTTTGCACC ATACGCACAG 1560
GCTCAAGACA TAACCTTGGA GCTACAACCC GAGAGCAAAC CTATTGTCGT GGACTTCGTC 1620
CCCTCCTACT TGCAAAAAAT CATATCCAAT CTTTTGTCCA ATGCCATCAA GTATTCTTTA 1680
GCCGGAGGGA GAGTGGTCAT ATCTCTGGCA AAAACCAAGA ATGAAAAAAA TCTGATCATA 1740

CGCGTTGCAG ACAATGGCAT AGGAATAGAT AAAACTGATC AGGCTCATAT CTTCGACATC 1800
TTCTATCGAG GACAGTCCGC TACCGAAAAG CATGGATCAG GCGTCGGACT CTCGTTTACC 1860
AATATACTGG TCGAAAACCT TCGAGGTACG ATCAAAGTGG AAAGCCAGCC GGGGAAAGGA 1920
AGTGCCTTCA CCATCAGTAT TCCTACACAA AACCAGTCCT CTTCGGCAGA GATTCTTCCT 1980
TGGCTACCCT CCTCCGATGA CATTGTCATG CCTGTCCACA TCGCGCCCGA TGACTCACCG 2040
ACATCTCCGA TGGTAGCAGC TCTGAATCAT CGCTTCGAGG ACGAACGTCC GACCATACTG 2100
CTCGTCGAGG ACAATAAGGA TATCAACCTG CTCGTCAAAC TACTCCTTTG CGATCGCTAC 2160
AATGTGCTAT CCGCCGCAAA CGGAAAAGAG GGTATAGCCC TCGCTACCGA GCATATTCCC 2220
GACATTATCA TTACGGATAT TATGATGCCG ATAATGGATG GGATAGAAAT GACAATCCGG 2280
ATGAAGCAAT CGCCTCTGCT CTGTCACATT CCCATTGTCG CTTTGACGGC CAAGAGTACC 2340
GAACAGGACA GATTGGAAGG AATCAAAAGC GGTGTAGTCT CTTATCTATG CAAGCCATTC 2400
TCTCCGGAGG AGCTTTTGAT GCGGATCGAG CAGCTTCTGA AAGACCGTGA GTTGCTCAAG 2460
AAGTTCTATA TGCAAAAACT CATGCTGGAT CGGAAGCCGG AGGAGGAGCC TCAACCGATA 2520
GATGACAGCA GTATGCAGTT TCTCCTTGCT GCCAAAGATG CAGTGTCCGG TGGAATCAAA 2580
CAAAATCCGG ATTTTTCCGC TCAAGACTTG GCCGAAAAAA TGTGCATGAG TCCATCCCAA 2640
CTCAACAGAA AGCTCACGAG TGTCGTAGGT TGCTCCACCA TCGGCTACAT ACAGCAGATC 2700
AAGATAAAAT TGGCCTGCAA GCTCCTTGCC GATGAGAGCA AAAACATCTC CGACATTAGC 2760
ATTGAGGCAG GCTTTTCGGA TCCGGCTTAC TTCTCTCGCA CCTTCAAACG CTACATGAAC 2820
TGCTCTCCCT CCCAATATCG GCAAAAACTC CTTGCCATGC CGGGGAGCGA CAAGGAGACA 2880
GTT 2883

(2) INFORMATION FOR SEQ ID NO:261

(i) SEQUENCE CHARACTERISTICS:
(A) LENGTH: 1668 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1668

(xi) SEQUENCE DESCRIPTION: SEQ ID NO:261

ATGAAAAAGC TTTTACAGGC TAAAGCCTTG ATTCTGGCAT TGGGACTCTT CCAACTGCCC 60
GCAATCGCCC AAACGCAAAT GCAAGCAGAC CGAACAAACG GTCAATTTGC AACAGAAGAG 120
ATGCAACGAG CATTCCAGGA AACGAATCCC CCTGCAGGTC CTGTGCGTGC TATCGCTGAG 180
TACGAACGCT CTGCAGCCGT TTTGGTACGC TACCCGTTCG GTATCCCGAT GGAATTGATC 240
AAAGAGCTCG CCAAGAACGA CAAGGTGATT ACCATTGTGG CGAGTGAAAG CCAAAAAAAC 300
ACCGTTATAA CCCAGTACAC CCAAAGCGGT GTGAATCTCT CTAATTGCGA TTTCATCATT 360
GCGAAAACTG ACTCTTACTG GACACGCGAC TATACCGGTT GGTTCGCAAT GTACGATACG 420
AACAAAGTAG GTCTCGTGGA CTTTATTTAT AACCGCCCTC GTCCTAACGA TGATGAATTC 480
CCCAAATACG AAGCACAATA TCTGGGCATC GAGATGTTCG GGATGAAGCT CAAGCAGACC 540
GGTGGCAACT ACATGACGGA CGGATATGGA TCCGCTGTGC AGTCACATAT CGCATATACG 600
GAGAACTCCT CTCTGTCTCA AGCTCAAGTA AATCAAAAGA TGAAAGACTA TCTCGGCATC 660
ACACATCATG ATGTGGTACA AGATCCGAAC GGCGAATATA TCAACCATGT GGACTGTTGG 720
GGCAAGTATT TGGCACCGAA CAAAATCCTC ATCAGGAAAG TGCCTGACAA TCACCCTCAG 780
CACCAAGCCC TGGAAGATAT GGCAGCCTAC TTCGCAGCAC AGACCTGCGC ATGGGGAACG 840
AAGTACGAGG TATATCGCGC TTTGGCCACC AATGAACAAC CGTACACGAA CTCTCTGATT 900
CTGAACAACA GGGTATTTGT TCCTGTCAAT GGCCCCGCCT CCGTGGACAA CGATGCTCTG 960
AACGTCTATA AGACGGCAAT GCCCGGTTAC GAAATTATAG GTGTCAAAGG GGCTTCAGGA 1020
ACACCTTCGT TAGGAACAGA TGCCCTGCAT TGTCGTACTC ACGAGGTAGC GGATAAGGGC 1080
TATCTCTATA TCAAGCACTA CCCGATACTG GGCGAACAGG CAGGCCCTGA TTATAAGATC 1140
GAAGCAGATG TCGTCTCATG CGCCAATGCT ACTATCTCGC CGGTACAATG TTACTATCGT 1200
ATCAATGGTT CCGGTAGCTT TAAGGCTGCT GATATGACGA TGGAATCAAC AGGTCACTAT 1260
ACTTATAGCT TTACAGGTCT TAACAAGAAT GATAAGGTAG AATACTATAT CTCTGCCGCT 1320
GACAATAGTG GTCGCAAAGA GACTTATCCC TTTATCGGCG AACCTGATCC TTTCAAGTTT 1380
ACGTGTATGA ACGAAACCAA TACATGTACT GTGACCGGAG CTGCCAAAGC TCTTCGTGCA 1440
TGGTTCAACG CCGGTCGTTC AGAACTGGCT GTTTCGGTAA GTTTGAATAT TGCCGGCACA 1500
TATCGGATAA AGCTTTATAA CACCGCAGGA GAAGAAGTCG CTGCAATGAC CAAGGAATTA 1560
GTAGCAGGGA CGAGTGTCTT CAGTATGGAT GTGTATTCTC AGGCTCCGGG CACATATGTT 1620
CTGGTTGTTG AAGGAAATGG AATCCGTGAG ACAATGAAAA TTCTCAAA 1668

(2) INFORMATION FOR SEQ ID NO:262

(i) SEQUENCE CHARACTERISTICS:
(A) LENGTH: 1284 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1284

(xi) SEQUENCE DESCRIPTION: SEQ ID NO:262

ATGAAACTTT CATCTAAGAA AATCTTAGCA ATCATTGCAT TGCTGACGAT GGGACATGCT 60
GTGCAGGCAC AGTTTGTTCC GGCTCCCACC ACAGGGATTC GCATGTCTGT CACTACAACC 120
AAGGCCGTAG GCGAAAAAAT CGAATTGTTG GTTCATTCCA TAGAGAAGAA AGGCATCTGG 180
ATCGATCTCA ATGGGGATGC CACTTACCAA CAAGGAGAGG AAATAACCGT ATTCGATGAG 240
GCATACCACG AATACACGAT CGGGACGCAA ACCCTCACTA TCTATGGTAA TACGACCCGA 300
TTGGGCTGTC GATCTACCGG TGCAACGGCT GTCGATGTAA CGAAAAACCC TAATCTGACC 360
TATCTCGCAT GCCCGAAAAA TAATCTGAAA TCATTGGACT TGACGCAAAA CCCAAAGCTG 420
CTGCGAGTTT GGTGCGACTC TAACGAAATA GAAAGTTTGG ACCTGAGTGG CAATCCGGCT 480
TTGATCATCC TCGGCTGTGA CAGGAATAAG CTGACTGAGC TGAAGACCGA TAACAACCCC 540
AAGTTGGCCT CTCTTTGGTG TTCTGATAAT AACCTGACGG AGTTGGAACT CAGTGCCAAT 600
CCTCGTCTCA ATGATCTTTG GTGCTTCGGT AATCGGATCA CGAAACTCGA TCTGAGTGCC 660
AATCCTCTAT TGGTAACACT TTGGTGCAGT GACAATGAGC TTTCGACCTT GGATCTTTCC 720
AAGAATTCGG ACGTTGCTTA CCTTTGGTGT TCATCGAACA AACTTACATC CTTGAATCTG 780
TCGGGGGTGA AGGGACTGAG TGTTTTGGTT TGTCATTCCA ATCAGATCGC AGGTGAAGAA 840
ATGACGAAAG TGGTGAATGC TTTGCCCACA CTATCTCCCG GCGCAGGCGC TCAGAGCAAG 900
TTCGTCGTTG TAGACCTCAA GGACACTGAT GAGAAGAATA TCTGTACCGT AAAGGATGTG 960
GAAAAAGCTA AAAGTAAGAA CTGGCGAGTA TTTGACTTCA ACGGTGATTC TGACAATATG 1020
CTTCCATACG AAGGAAGTCC GACATCGAAC TTGGCAGTAG ATGCTCCCAC TGTCAGGATA 1080
TATCCCAATC CGGTAGGAAG ATATGCGCTC GTCGAGATCC CCGAGTCTCT TTTAGGGCAG 1140
GAAGCTGCTT TATACGATAT GAATGGGGTA AAAGTCTATA GTTTCGCGGT AGAGTCTCTT 1200
CGTCAGAACA TTGACCTGAC ACATCTTCCC GACGGCACTT ATTTCTTCCG TCTCGATAAC 1260
TATACCACTA AGCTCATCAA ACAG 1284

(2) INFORMATION FOR SEQ ID NO:263

(i) SEQUENCE CHARACTERISTICS:
(A) LENGTH: 930 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...930

(xi) SEQUENCE DESCRIPTION: SEQ ID NO:263

ATGAGAAAAA CAATAATTTT CTGCTTGTTG CTCGCCCTAT TTGGCTGTTC TTGGGCACAA 60
GAAAGAGTCG ATGAAAAAGT ATTCTCCGCA GGAACAAGTA TTTTTAGGGG CATCCTTGAA 120
AAGGTGAAAG CACCGCTTAT GTATGGAGAT CGTGAGGTAT GGGGTATGGC TCGTGCGAGC 180
GAGGATTTCT TTTTTATACT TCCCGTTACG GATGACCTCA CTCCCGTGCT TTTCTATAAC 240
CGTCTTACAA ACGAACCCTG CTTTGTGTCA GACCAAGGAA TAACTGAGTA TTTCAAATTC 300

GCTCAAGAAG GTGATTACAT TGAAGTCGAA GGAAGCTCTG TATTCATGGC GAATCTTTTG 360
TACTATCGTT TTTTCCCGAC AAGAATTACC TCCTATAATG CTCCCATTGA AGGTGTTGTG 420
AGCAAGACGG GAAATCCTGC TTTTACAATC CCGATGCTCC CGGGGGTTTC TGATTGCATA 480
GAAATCTCAA ACAACCGCAA AGTCTTTCTG ACCAATCAAT TAGGGGTTGT AAACATCACT 540
GACGGGATGG AACCTCCGAT TATTGCCGGA GTCTCTGCTT CCTATGGATC TTCCGTCCGG 600
GTGTATGGTC ATGTCTCACA GCGGTGGGAC ATCATAGGCC ATTGCTATTT GGATATCTAC 660
CCAACCAATT GCTATCCGCT CAGCACGAAA CCCGTTGCAG GAGACGATGA GGTTTTTGTC 720
AAACAACAAG GCAGGCAAAT AGAGATCGAT AGCAACAGCC CCATAGTCCA AGTGGTCGTA 780
TACGATCTTG AGGGGAAAAG TGTTTTTCGC AAAAGAATGA CCGAAAACGC TTATACCCTA 840
TCCTTTAGAG CACCCATGCT CGGCTTTATG ACCATCATGA TCGAAACACA AAATTCGATT 900
ATCAATAAAA AACTTAATGT TACACAGCTA 930

(2) INFORMATION FOR SEQ ID NO:264

(i) SEQUENCE CHARACTERISTICS:
(A) LENGTH: 1215 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: PORYPHYROMONAS GINGIVALIS

(ix) FEATURE:
(A) NAME/KEY: misc_feature
(B) LOCATION 1...1215

(xi) SEQUENCE DESCRIPTION: SEQ ID NO:264
   ATGAAAAAAA CAACCATTAT TTCTTTGATT GTCTTCGGTG CTTTCTTTGC AGCCGTGGGC 60
   CAAACCAAGG ACAATTCTTC TTACAAACCT TTTTCGAAAG AAGATATTGC CGGAGGAGTT 120
   TACTCTCTCC CGACTCAAAA TCGTGCGCAG AAGGACAATG CCGAGTGGCT TCTTACAGCG 180
   ACCGTCTCCA CAAACCAGTC TGCAGATACT CACTTTATCT TCGATGAGAA CAACCGCTAT 240
   ATCGCTCGTG ACATAAAAGC CAATGGGGTA AGAAAATCCA CGGACTCCAT TTACTACGAT 300
   GCCAACGGGC GAATATCGCA TGTGGATCTT TATATCTCGT TCAGTGGCGG AGAGCCTGCA 360
   CTCGACACCC GATTCAAGTA CACCTATGAT GACGAGGGAA AGATGACCGT GAGGGAAGTA 420
   TTCATGCTGG TAATGGATCC GAATACACCT ATCTCACGCT TGGAATATCA TTATGATGCA 480
   CAGGGCAGAC TGACCCACTG GATTTCTTTT GCTTTCGGGG CAGAATCCCA AAAGAATACG 540
   TATCACTATA ATGAAAAAGG TCTGTTGGTC AGCGAAGTGC TGAGCAATGC AATGGGGACA 600
   ACCTATTCAG ACACCGGCAA AACGGAATAC AGCTATGACG ATGCAGATAA TATGGTGAAG 660
   GCCGAGTACT TCGTCGTCCA GCAAGGAAAG GCATGGCAAG TACTCAAAAG AGAGGAATAC 720
   ACCTATGAGG ACAATATCTG CATACAATAT TTGGCTATTA ACGGTACCGA CACAAAGGTG 780
   TACAAGCGAG ACATCGAGAG CGATAAGTCC ATCTCCGCAA ATGTCATTGA CATTCCGTCA 840
   ATGCCGGAAC AGACCTGGCC TAATATGTAC GGATTCAACG CAAAGCGACT GAAAGAGACT 900
   TATTCCTCCT ACGAAGGAGA TGTGGCTACT CCTATATTCG ACTATATCTA TACGTACAAG 960
   GCTCTTACCT CAATGGCAAC ACCTTCGACA GAAGCTCAGG TAGCAGTCTA TCTCAATCCG 1020
   TCAACGGACC GGTTAGTGAT TCTGGCCAAC GGCATCACAC ATCTGAGCAT GTACGACTTG 1080
   CAGGGTAAGC TTATCCGTGA TTGTGCCTTG AGCGGCGATA AGGTGGAAAT GGGTGTCGGA 1140
   TCTTTGACCA AAGGGACATA CCTGCTTAAA GTGAATACGG ATCAGGGAGC CTTTGTGAGA 1200
   AAAGTCGTGA TTCGA 1215

(2) INFORMATION FOR SEQ ID NO:265

(i) SEQUENCE CHARACTERISTICS:
(A) LENGTH: 454 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature

(B) LOCATION 1...454

(xi) SEQUENCE DESCRIPTION: SEQ ID NO:265

Phe Cys Val Met Ala Lys Val Ile Lys Thr Lys Lys Gly Leu Ala Leu
1               5                   10                  15
Asn Leu Lys Gly Lys Pro Leu Pro Glu Met Leu Ala Glu Pro Ala Gln
            20                  25                  30
Ser Pro Thr Tyr Ala Val Val Pro Asp Asp Phe Glu Gly Val Ile Pro
        35                  40                  45
Lys Val Thr Ala Arg Pro Gly Asp Lys Val Arg Ala Gly Ser Ala Leu
    50                  55                  60
Met His His Lys Ala Tyr Pro Glu Met Lys Phe Thr Ser Pro Val Ser
65                  70                  75                  80
Gly Glu Val Ile Ala Val Asn Arg Gly Ala Lys Arg Lys Val Leu Ser
                85                  90                  95
Ile Glu Val Lys Pro Asp Gly Leu Asn Glu Tyr Glu Ser Phe Pro Val
            100                 105                 110
Gly Asp Pro Ser Ala Leu Ser Ala Glu Gln Ile Lys Glu Leu Leu Leu
        115                 120                 125
Ser Ser Gly Met Trp Gly Phe Ile Lys Gln Arg Pro Tyr Asp Ile Val
    130                 135                 140
Ala Thr Pro Asp Ile Ala Pro Arg Asp Ile Tyr Ile Thr Ala Asn Phe
145                 150                 155                 160
Thr Ala Pro Leu Ala Pro Asp Phe Asp Phe Ile Val Arg Gly Glu Glu
                165                 170                 175
Arg Ala Leu Gln Thr Ala Ile Asp Ala Leu Ala Lys Leu Thr Thr Gly
            180                 185                 190
Lys Val Tyr Val Gly Leu Lys Pro Gly Ser Ser Leu Gly Leu His Asn
        195                 200                 205
Ala Glu Ile Val Glu Val His Gly Pro His Pro Ala Gly Asn Val Gly
    210                 215                 220
Val Leu Ile Asn His Thr Lys Pro Ile Asn Arg Gly Glu Thr Val Trp
225                 230                 235                 240
Thr Leu Lys Ala Thr Asp Leu Ile Val Ile Gly Arg Phe Leu Leu Thr
                245                 250                 255
Gly Lys Ala Asp Phe Thr Arg Met Ile Ala Met Thr Gly Ser Asp Ala
            260                 265                 270
Ala Ala His Gly Tyr Val Arg Ile Met Pro Gly Cys Asn Val Phe Ala
        275                 280                 285
Ser Phe Pro Gly Arg Leu Thr Ile Lys Glu Ser His Glu Arg Val Ile
    290                 295                 300
Asp Gly Asn Val Leu Thr Gly Lys Lys Leu Cys Glu Lys Glu Pro Phe
305                 310                 315                 320
Leu Ser Ala Arg Cys Asp Gln Ile Thr Val Ile Pro Glu Gly Asp Asp
                325                 330                 335
Val Asp Glu Leu Phe Gly Trp Ala Ala Pro Arg Leu Asp Gln Tyr Ser
            340                 345                 350
Met Ser Arg Ala Tyr Phe Ser Trp Leu Gln Gly Lys Asn Lys Glu Tyr
        355                 360                 365
Val Leu Asp Ala Arg Ile Lys Gly Gly Glu Arg Ala Met Ile Met Ser
    370                 375                 380
Asn Glu Tyr Asp Arg Val Phe Pro Met Asp Ile Tyr Pro Glu Tyr Leu
385                 390                 395                 400
Leu Lys Ala Ile Ile Ala Phe Asp Ile Asp Lys Met Glu Asp Leu Gly
                405                 410                 415
Ile Tyr Glu Val Ala Pro Glu Asp Phe Ala Thr Cys Glu Phe Val Asp
            420                 425                 430
Thr Ser Lys Ile Glu Leu Gln Arg Ile Val Arg Glu Gly Leu Asp Met
        435                 440                 445
Leu Tyr Lys Glu Met Asn
    450

(2) INFORMATION FOR SEQ ID NO:266

(i) SEQUENCE CHARACTERISTICS:
(A) LENGTH: 201 amino acids
(B) TYPE: amino acid
(D) TOPOLOGY: linear

(ii) MOLECULE TYPE: protein

(iii) HYPOTHETICAL: YES

(vi) ORIGINAL SOURCE:

(A) ORGANISM: Porphyromonas gingivalis

(ix) FEATURE:
(A) NAME/KEY: misc_feature
(B) LOCATION 1...201

(xi) SEQUENCE DESCRIPTION: SEQ ID NO:266

Glu Leu Ser Lys Cys Tyr Met Asp Lys Val Ser Tyr Ala Leu Gly Leu
1               5                   10                  15
Ser Ile Gly Asn Asn Phe Lys Ser Ser Gly Ile Asp Ser Val Val Met
            20                  25                  30
Asp Asp Phe Met Gln Gly Leu Ser Asp Val Leu Glu Glu Lys Ala Pro
        35                  40                  45
Gln Leu Ser Tyr Asp Glu Ala Lys Arg Glu Ile Glu Ala Tyr Phe Met
    50                  55                  60
Asp Leu Gln Gln Lys Ala Val Lys Leu Asn Lys Glu Ala Gly Glu Glu
65                  70                  75                  80
Phe Leu Lys Ile Asn Ala His Lys Glu Gly Val Thr Thr Leu Pro Ser
                85                  90                  95
Gly Leu Gln Tyr Glu Val Ile Lys Met Gly Glu Gly Pro Lys Pro Thr
            100                 105                 110
Leu Ser Asp Thr Val Thr Cys His Tyr His Gly Thr Leu Ile Asn Gly
        115                 120                 125
Ile Val Phe Asp Ser Ser Met Asp Arg Gly Glu Pro Ala Ser Phe Pro
    130                 135                 140
Leu Arg Gly Val Ile Ala Gly Trp Thr Glu Ile Leu Gln Leu Met Pro
145                 150                 155                 160
Val Gly Ser Lys Trp Lys Val Thr Ile Pro Ser Asp Leu Ala Tyr Gly
                165                 170                 175
Asp Arg Gly Ala Gly Glu His Ile Lys Pro Gly Ser Thr Leu Ile Phe
            180                 185                 190
Ile Ile Glu Leu Leu Ser Ile Asn Lys
        195                 200

(2) INFORMATION FOR SEQ ID NO:267

(i) SEQUENCE CHARACTERISTICS:
(A) LENGTH: 279 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...279

(xi) SEQUENCE DESCRIPTION: SEQ ID NO:267

Gln Lys Asn Lys Arg Lys Met Lys Lys Ala Leu Leu Ile Gly Ala Ala
1               5                   10                  15
Leu Leu Gly Ala Val Ser Phe Ala Ser Ala Gln Ser Leu Ser Thr Ile
            20                  25                  30
Lys Val Gln Asn Asn Ser Val Gln Gln Pro Arg Glu Glu Ala Thr Ile
        35                  40                  45
Gln Val Cys Gly Glu Leu Ala Glu Gln Val Asp Cys Ile Gly Thr Gly
    50                  55                  60
Asn Ser Ala Ile Ile Ala Ala Ala Ala Lys Phe Glu Ser Asp Asp Leu
65                  70                  75                  80
Glu Ser Tyr Val Gly Trp Glu Ile Met Ser Val Asp Phe Phe Pro Gly
                85                  90                  95
Tyr Lys Ala Cys Lys Tyr Thr Ser Ala Val Trp Ala Asp Asp Met Thr
            100                 105                 110
Ile Leu Gly Gln Ser Glu Asp Ser Asp Pro Glu Met Gln Thr Ile Asn
        115                 120                 125
Asn Leu Ala Leu Lys Thr Ser Val Lys Ile Glu Ala Gly Lys Asn Tyr
    130                 135                 140
Ile Val Gly Tyr Ile Ala Asn Thr Ala Gly Gly His Pro Ile Gly Cys
145                 150                 155                 160

Asp Gln Gly Pro Ala Val Asp Gly Tyr Gly Asp Leu Val Ser Ile Ser
                165                 170                 175
Glu Asp Gly Gly Ala Thr Phe Pro Pro Phe Glu Ser Leu His Gln Ala
            180                 185                 190
Val Pro Thr Leu Asn Tyr Asn Ile Tyr Val Val Val His Leu Lys Lys
        195                 200                 205
Gly Glu Gly Val Glu Ala Val Leu Thr Asn Asp Lys Ala Asn Ala Tyr
    210                 215                 220
Val Gln Asn Gly Val Ile Tyr Val Ala Gly Ala Asn Gly Arg Gln Val
225                 230                 235                 240
Ser Leu Phe Asp Met Asn Gly Lys Val Val Tyr Thr Gly Val Ser Glu
                245                 250                 255
Thr Ile Ala Ala Pro Gln Lys Gly Met Tyr Ile Leu Arg Val Gly Ala
            260                 265                 270
Lys Ser Ile Lys Leu Ala Ile
        275

(2) INFORMATION FOR SEQ ID NO:268

     (i) SEQUENCE CHARACTERISTICS:
          (A) LENGTH: 157 amino acids
          (B) TYPE: amino acid
          (D) TOPOLOGY: linear

    (ii) MOLECULE TYPE: protein

   (iii) HYPOTHETICAL: YES

    (vi) ORIGINAL SOURCE:
          (A) ORGANISM: Porphyromonas gingivalis

    (ix) FEATURE:
          (A) NAME/KEY: misc_feature
          (B) LOCATION 1...157

    (xi) SEQUENCE DESCRIPTION: SEQ ID NO:268

Arg Unk Phe Leu Pro Glu Lys Ala Leu Tyr Ile Gly Cys Arg Val Glu
1               5                   10                  15
Thr Gln Glu Gly His Ala Val Gly Phe Gly Leu Asp Asp Gly Pro Ala
            20                  25                  30
Met Lys Gly Lys Gly Asp Leu Val Gly Ser Tyr Leu Pro Gly Ala Ala
        35                  40                  45
Pro Met Pro Phe Val Pro Leu Ser Asp Ile Pro Ala Arg Ser Met Asp
    50                  55                  60
Ala Asn Phe Tyr Ile Tyr Ser Arg Ile Ser Leu Gly Ser Gly Thr Gln
65                  70                  75                  80
Asp Val Leu Gln His Arg Met Lys Val Tyr Pro Asn Pro Ala Thr Thr
                85                  90                  95
Glu Leu His Val Glu Ala Leu Ser Ala Trp Val Gly Glu Gln Ala Ala
            100                 105                 110
Val Tyr Asp Met Arg Gly Arg Arg Val Ser Ala Arg Thr Val Asp Ser
        115                 120                 125
Glu Lys Leu Cys Ile Asp Ile Ala Ser Leu Pro Val Gly Val Tyr Met
    130                 135                 140
Leu Arg Ile Gly Ser Tyr Ser Ala Lys Phe Glu Lys Arg
145                 150                 155

(2) INFORMATION FOR SEQ ID NO:269

     (i) SEQUENCE CHARACTERISTICS:
          (A) LENGTH: 562 amino acids
          (B) TYPE: amino acid
          (D) TOPOLOGY: linear

    (ii) MOLECULE TYPE: protein

   (iii) HYPOTHETICAL: YES

    (vi) ORIGINAL SOURCE:
          (A) ORGANISM: Porphyromonas gingivalis

    (ix) FEATURE:
          (A) NAME/KEY: misc_feature
          (B) LOCATION 1...562

(xi) SEQUENCE DESCRIPTION: SEQ ID NO:269

Thr Arg Asn Val Ser Leu Ile Lys Met Pro Arg Ile Met Lys Leu Lys
1               5                   10                  15
Ile Ala Leu Arg Leu Leu Leu Ala Thr Phe Ala Ile Val Leu Phe Ser
            20                  25                  30
Pro Leu Ala Lys Ala Gln Met Asp Ile Gly Gly Asp Asp Val Leu Ile
        35                  40                  45
Glu Thr Met Ser Thr Leu Ser Gly Tyr Ser Glu Asp Phe Tyr Tyr Lys
    50                  55                  60
Met Ala Val Ala Asp Asn Gly Trp Ile Tyr Val Met Leu Asp Phe Ser
65                  70                  75                  80
Arg Ile Tyr Phe Asp Asp Val Arg Leu Tyr Arg Ser Lys Asp Gly Gly
                85                  90                  95
Ala Thr Tyr Gln Lys Leu Gly Ser Leu Gly Ser Leu Val Pro Tyr Asp
            100                 105                 110
Phe Asp Val Ser His Cys Asp Phe Ile Val Thr Gly Lys Asp Glu Asp
        115                 120                 125
Asp Ile Asn Val Trp Thr Val Met Thr Ala Phe Glu Tyr Val Gly Gly
    130                 135                 140
Thr Ile Gly Asn Gly Val Leu Leu Met His Arg His Asp Ala Asp Ile
145                 150                 155                 160
Asn Asn Thr Glu Cys Val Tyr Lys Lys Asp Phe Pro Asn Asn Arg Leu
                165                 170                 175
Met Gly Val Ala Ile Ala Ser Asn Tyr Arg Ala Pro Ser Pro Tyr Gly
            180                 185                 190
Leu Gly Gly Asp Pro Phe Ala Leu Ala Val Ala Val Ser Gly Ser Gly
        195                 200                 205
Ser Asp His Ser Phe Leu Asp Tyr Ile Phe Ser Leu Asp Gly Gly Val
    210                 215                 220
His Phe Glu Gln Lys Arg Ile Tyr Thr Arg Pro Gln Lys Leu Thr Ile
225                 230                 235                 240
Asn Arg Val Asp Leu Ser Leu Gly Ser Thr Ser Pro Ser Leu Gly Phe
                245                 250                 255
Asn Thr Trp Pro Leu Met Gly Val Val Phe Glu Met Asn Lys Asn Leu
            260                 265                 270
Asp Gly Phe Asp Ile Gly Phe Ile Ser Asn Phe Val Asp Tyr Asp Pro
        275                 280                 285
Arg Tyr Ala Trp Ser Glu Pro Ile Ile Ile Glu Glu Asp Cys Gly Trp
    290                 295                 300
Thr Asp Phe Asn Pro Leu Gly Ala Leu Ser Ile Glu Ile Gln Met Met
305                 310                 315                 320
Leu Asp Asp Asn Ser Asp Asn Thr Val Gly Gly Glu Arg Ser His Asn
                325                 330                 335
Phe Leu Ile Thr Tyr Pro Gly His Tyr Val Tyr Pro Lys Gln Ser Phe
            340                 345                 350
Asn Tyr Ser Pro Gly His Thr Pro Thr Lys Lys Asp Leu Val Phe Lys
        355                 360                 365
His Cys Ile Gly Ile Pro Ala Leu Ala Tyr Asp Lys Glu Gly Asp Arg
    370                 375                 380
Tyr Leu Thr Thr Phe Gln Asp His Asn Leu Met Arg Tyr Arg Trp Ile
385                 390                 395                 400
Lys Tyr Asp Asp Ile Asn Ser Phe Tyr Gly Trp Ser Trp Pro Tyr Val
                405                 410                 415
Tyr Ala Lys Glu Ala Lys Asp Lys Lys Arg Arg Arg Pro Gln Val Ala
            420                 425                 430
Leu Asn Pro Thr Asn Gly Lys Ala Cys Trp Val Trp His Thr Arg Lys
        435                 440                 445
Ser Pro Tyr Asp Glu Thr Lys Pro His Pro Thr Pro Val Ile Ile Lys
    450                 455                 460
His Phe Leu Trp Ser Asp Thr Glu Trp Val His Ala Leu Asp Val Gly
465                 470                 475                 480
Asp Val Leu Gln Lys Glu Gly Ser Met Lys Leu Tyr Pro Asn Pro Ala
                485                 490                 495
Lys Glu Tyr Val Leu Ile Asn Leu Pro Lys Glu Gly Gly His Glu Ala
            500                 505                 510
Val Val Tyr Asp Met Gln Gly Arg Ile Val Glu Lys Val Ser Phe Ser
        515                 520                 525
Gly Lys Glu Tyr Lys Leu Asn Val Gln Tyr Leu Ser Lys Gly Thr Tyr
    530                 535                 540
Met Leu Lys Val Val Ala Asp Thr Glu Tyr Phe Val Glu Lys Ile Ile
545                 550                 555                 560
Val Glu

(2) INFORMATION FOR SEQ ID NO:270

(i) SEQUENCE CHARACTERISTICS:
(A) LENGTH: 391 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...391

(xi) SEQUENCE DESCRIPTION: SEQ ID NO:270

Gln Met Lys Arg Leu Leu Pro Phe Leu Leu Leu Ala Gly Leu Val Ala
1               5                   10                  15
Val Gly Asn Val Ser Ala Gln Ser Pro Arg Ile Pro Gln Val Asp Val
            20                  25                  30
His Thr Arg Ile Ala Arg Asn Ala Arg Tyr Arg Leu Asp Lys Ile Ser
        35                  40                  45
Val Pro Asp Ser Arg Gln Ile Phe Asp Tyr Phe Tyr Lys Glu Glu Thr
    50                  55                  60
Ile Pro Thr Lys Ile Gln Thr Thr Thr Gly Gly Ala Ile Thr Ser Ile
65                  70                  75                  80
Asp Ser Leu Phe Tyr Glu Asp Asp Arg Leu Val Gln Val Arg Tyr Phe
                85                  90                  95
Asp Asn Asn Leu Glu Leu Lys Gln Ala Glu Lys Tyr Val Tyr Asp Gly
            100                 105                 110
Ser Lys Leu Val Leu Arg Glu Ile Arg Lys Ser Pro Thr Asp Glu Thr
        115                 120                 125
Pro Ile Lys Lys Val Ser Tyr His Tyr Leu Cys Gly Ser Asp Met Pro
    130                 135                 140
Phe Glu Ile Thr Thr Glu Met Ser Asp Gly Tyr Phe Glu Ser His Thr
145                 150                 155                 160
Leu Asn Tyr Leu Asn Gly Lys Ile Ala Arg Ile Asp Ile Met Thr Gln
                165                 170                 175
Gln Asn Pro Ser Ala Glu Leu Ile Glu Thr Gly Arg Met Val Tyr Glu
            180                 185                 190
Phe Asp Ala Asn Asn Asp Ala Val Leu Leu Arg Asp Ser Val Phe Leu
        195                 200                 205
Pro Leu Gln Asn Lys Trp Val Glu Met Phe Thr His Arg Tyr Thr Tyr
    210                 215                 220
Asp Asn Lys His Asn Cys Ile Arg Trp Glu Gln Asp Glu Phe Gly Thr
225                 230                 235                 240
Leu Thr Leu Ala Asn Asn Phe Glu Tyr Asp Thr Thr Ile Pro Leu Ser
                245                 250                 255
Ser Val Leu Phe Pro Thr His Glu Glu Phe Phe Arg Pro Leu Leu Pro
            260                 265                 270
Asn Phe Met Lys His Met Arg Thr Lys Gln Thr Tyr Phe Asn Asn Ser
        275                 280                 285
Gly Glu Gly Leu Ser Glu Val Cys Asp Tyr Asn Tyr Phe Tyr Thr Asp
    290                 295                 300
Met Gln Gly Asn Ala Leu Thr Asp Val Ala Val Asn Glu Ser Ile Lys
305                 310                 315                 320
Ile Tyr Pro Arg Pro Ala Thr Asp Phe Leu Arg Ile Glu Gly Ser Gln
                325                 330                 335
Leu Leu Arg Leu Ser Leu Phe Asp Met Asn Gly Lys Leu Ile Arg Ala
            340                 345                 350
Thr Glu Leu Thr Gly Asp Leu Ala Ile Ile Gly Val Ala Ser Leu Pro
        355                 360                 365
Arg Gly Thr Tyr Ile Ala Glu Ile Thr Ala Ala Asn Ser Lys Thr Ile
    370                 375                 380
Arg Ala Lys Val Ser Leu Arg
385                 390

(2) INFORMATION FOR SEQ ID NO:271

(i) SEQUENCE CHARACTERISTICS:

(A) LENGTH: 428 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...428

(xi) SEQUENCE DESCRIPTION: SEQ ID NO:271

Lys Arg Asn Pro Leu Pro Leu Thr Ala Ser Asn Arg Lys Ile Phe Ile
1               5                   10                  15
Lys Met Arg Gln His Leu Ser Leu Phe Pro Phe Ile Leu Phe Leu Leu
            20                  25                  30
Leu Ala Phe Ser Tyr Val Gly Cys Arg Thr Val Arg Gln Thr Pro Lys
        35                  40                  45
Gln Ser Glu Arg Tyr Val Val Val Leu Ser Leu Asp Gly Phe Arg Pro
    50                  55                  60
Asp Tyr Thr Asp Arg Ala Arg Thr Pro Ala Leu Asp Arg Met Ala Gln
65                  70                  75                  80
Glu Gly Leu Ser Gly Ser Leu Gln Pro Cys Phe Pro Ser Leu Thr Phe
                85                  90                  95
Pro Asn His Tyr Ser Met Ala Thr Gly Leu Tyr Pro Asp His His Gly
            100                 105                 110
Ile Val Ala Asn Glu Phe Val Asp Ser Leu Leu Gly Ile Phe Arg Ile
        115                 120                 125
Ser Asp Arg Lys Ala Val Glu Thr Pro Gly Phe Trp Gly Gly Glu Pro
    130                 135                 140
Val Trp Asn Thr Ala Ala Arg Gln Gly Ile Arg Thr Gly Val Tyr Phe
145                 150                 155                 160
Trp Val Gly Ser Glu Thr Ala Val Asn Gly Asn Arg Pro Trp Arg Trp
                165                 170                 175
Lys Lys Phe Ser Ser Thr Val Pro Phe Arg Asp Arg Ala Asp Ser Val
            180                 185                 190
Ile Ala Trp Leu Gly Leu Pro Glu Lys Glu Arg Pro Arg Leu Leu Met
        195                 200                 205
Trp Tyr Ile Glu Glu Pro Asp Met Ile Gly His Ser Gln Thr Pro Glu
    210                 215                 220
Ser Pro Leu Thr Leu Ala Met Val Glu Arg Leu Asp Ser Val Val Gly
225                 230                 235                 240
Tyr Phe Arg Lys Arg Leu Asp Ser Leu Pro Ile Ala Ala Gln Thr Asp
                245                 250                 255
Phe Ile Ile Val Ser Asp His Gly Met Ala Thr Tyr Glu Asn Glu Lys
            260                 265                 270
Cys Val Asn Leu Ser His Tyr Leu Pro Ala Asp Ser Phe Leu Tyr Met
        275                 280                 285
Ala Thr Gly Ala Phe Thr His Leu Tyr Pro Lys Pro Ser Tyr Thr Glu
    290                 295                 300
Arg Ala Tyr Glu Ile Leu Arg Ala Ile Pro His Ile Ser Val Tyr Arg
305                 310                 315                 320
Lys Gly Glu Val Pro Lys Arg Leu Arg Cys Gly Thr Asn Pro Arg Leu
                325                 330                 335
Gly Glu Leu Val Val Ile Pro Asp Ile Gly Ser Thr Val Phe Phe Ala
            340                 345                 350
Ile Asn Glu Asp Val Arg Pro Gly Ala Ala His Gly Tyr Asp Asn Gln
        355                 360                 365
Ala Pro Glu Met Arg Ala Leu Leu Arg Ala Val Gly Pro Asp Phe Arg
    370                 375                 380
Pro Gly Ser Arg Val Glu Asn Leu Pro Asn Ile Thr Ile Tyr Pro Leu
385                 390                 395                 400
Ile Cys Arg Leu Leu Gly Ile Glu Pro Ala Pro Asn Asp Ala Asp Glu
                405                 410                 415
Thr Leu Leu Asn Gly Leu Ile Arg Asp Lys Arg Pro
            420                 425

(2) INFORMATION FOR SEQ ID NO:272

(i) SEQUENCE CHARACTERISTICS:

(A) LENGTH: 282 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...282

(xi) SEQUENCE DESCRIPTION: SEQ ID NO:272

Leu Ser Arg Gly Val Phe Pro Leu Met Tyr Gly Arg Arg Gly Ser Ile
1               5                   10                  15
Arg Ala Ser Ser Gly His Arg Asp Lys Ile Phe Lys Asn Thr Ile Ile
            20                  25                  30
Arg Phe Ile Thr Met Lys Val Gly Leu Phe Ile Pro Cys Tyr Val Asn
        35                  40                  45
Ala Val Tyr Pro Glu Val Gly Ile Ala Thr Tyr Lys Leu Leu Lys Ser
    50                  55                  60
Leu Asp Ile Asp Val Asp Tyr Pro Met Asp Gln Thr Cys Cys Gly Gln
65                  70                  75                  80
Pro Met Ala Asn Ala Gly Phe Glu Gln Lys Ala Gln Lys Leu Ala Leu
                85                  90                  95
Arg Phe Glu Glu Leu Phe Glu Ser Tyr Asp Val Val Val Gly Pro Ser
            100                 105                 110
Ala Ser Cys Val Ala Phe Val Lys Glu Asn Tyr Asp His Ile Leu Arg
        115                 120                 125
Pro Thr Gly His Val Cys Lys Ser Ala Ala Lys Val Arg Asp Ile Cys
    130                 135                 140
Glu Phe Leu His Asp Asp Leu Lys Ile Thr Ser Leu Pro Ser Arg Phe
145                 150                 155                 160
Ala His Lys Val Ser Leu His Asn Ser Cys His Gly Val Arg Glu Leu
                165                 170                 175
His Leu Ser Thr Pro Ser Glu Val His Arg Pro Tyr His Asn Lys Val
            180                 185                 190
Arg Arg Leu Leu Glu Met Val Gln Gly Ile Glu Val Phe Glu Pro Lys
        195                 200                 205
Arg Ile Asp Glu Cys Cys Gly Phe Gly Gly Met Tyr Ser Val Glu Glu
    210                 215                 220
Pro Glu Val Ser Thr Cys Met Gly His Asp Lys Val Leu Asp His Ile
225                 230                 235                 240
Ser Thr Gly Ala Glu Tyr Ile Thr Gly Pro Asp Ser Ser Cys Leu Met
                245                 250                 255
His Met Gln Gly Val Ile Asp Arg Glu Lys Leu Pro Ile Lys Thr Ile
            260                 265                 270
His Ala Val Glu Ile Leu Ala Ala Asn Leu
        275                 280

(2) INFORMATION FOR SEQ ID NO:273

(i) SEQUENCE CHARACTERISTICS:
(A) LENGTH: 251 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...251

(xi) SEQUENCE DESCRIPTION: SEQ ID NO:273

Pro Leu Lys Lys Arg Met Asp Ile Val Ser Met Ala Asp Lys Ala Leu

1                   5                   10                  15
Val Val Glu Met Arg Asp Val Thr Leu Cys Gln Glu Glu Asn Val Ile
                20                  25                  30
Phe Gln Asn Leu Asn Leu Thr Leu Ser Ala Gly Asp Phe Val Tyr Leu
        35                  40                  45
Ile Gly Ser Val Gly Ser Gly Lys Ser Thr Leu Leu Lys Ala Leu Tyr
    50                  55                  60
Ala Glu Val Pro Ile Ser Ala Gly Tyr Ala Arg Val Ile Asp Tyr Asp
65                  70                  75                  80
Leu Ala Lys Leu Lys Arg Lys Gln Leu Pro Tyr Leu Arg Arg Asn Leu
                85                  90                  95
Gly Ile Val Phe Gln Asp Phe Gln Leu Leu Asn Gly Arg Thr Val Ala
            100                 105                 110
Glu Asn Leu Asp Phe Val Leu Arg Ala Thr Asp Trp Lys Asn Arg Ala
        115                 120                 125
Asp Arg Glu Gln Arg Ile Glu Glu Val Leu Thr Arg Val Gly Met Ser
    130                 135                 140
Arg Lys Ala Tyr Lys Arg Pro His Glu Leu Ser Gly Gly Glu Gln Gln
145                 150                 155                 160
Arg Val Gly Ile Ala Arg Ala Leu Leu Ala Lys Pro Ala Leu Ile Leu
                165                 170                 175
Ala Asp Glu Pro Thr Gly Asn Leu Asp Ser Val Thr Gly Leu Gln Ile
            180                 185                 190
Ala Ser Leu Leu Tyr Glu Ile Ser Lys Gln Gly Thr Ala Val Leu Met
        195                 200                 205
Ser Thr His Asn Ser Ser Leu Leu Ser His Leu Pro Ala Arg Thr Leu
    210                 215                 220
Ala Val Arg Lys Asn Gly Asp Ala Ser Ser Leu Val Glu Leu Ser Ala
225                 230                 235                 240
Asp Ala Val Ser Arg Lys Asn Thr Glu Ile Asp
                245

(2) INFORMATION FOR SEQ ID NO:274

    (i) SEQUENCE CHARACTERISTICS:
        (A) LENGTH: 238 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...238

    (xi) SEQUENCE DESCRIPTION: SEQ ID NO:274

Thr Arg His Cys Pro Ala Cys Arg Ser Ser Phe His Leu Ile Lys Thr
1                   5                   10                  15
Ser Lys Thr Met Ile Glu Ile Ser Asn Leu Thr Lys Val Phe Arg Thr
                20                  25                  30
Glu Glu Ile Glu Thr Val Ala Leu Asp Gly Val Ser Leu Lys Val Asp
        35                  40                  45
Lys Gly Glu Phe Ile Ala Ile Met Gly Pro Ser Gly Cys Gly Lys Ser
    50                  55                  60
Thr Leu Leu Asn Ile Leu Gly Leu Leu Asp Asn Pro Thr Ser Gly Ile
65                  70                  75                  80
Tyr Lys Leu Asp Gly Ala Glu Val Gly Asn Leu Arg Glu Lys Asp Arg
                85                  90                  95
Thr Ala Val Arg Lys Gly Asn Ile Gly Phe Val Phe Gln Ser Phe Asn
            100                 105                 110
Leu Ile Glu Glu Met Thr Val Ser Glu Asn Val Glu Leu Pro Leu Val
        115                 120                 125
Tyr Leu Gly Val Lys Ala Ser Glu Arg Lys Glu Arg Val Glu Glu Ala
    130                 135                 140
Leu Arg Lys Met Ser Ile Ser His Arg Ala Gly His Phe Pro Asn Gln
145                 150                 155                 160
Leu Ser Gly Gly Gln Gln Gln Arg Val Ala Ile Ala Arg Ala Val Val
                165                 170                 175
Ala Asn Pro Lys Leu Ile Leu Ala Asp Glu Pro Thr Gly Asn Leu Asp

180                 185                 190
Ser Lys Asn Gly Ala Asp Val Met Glu Leu Leu Arg Gly Leu Asn Arg
        195                 200                 205
Glu Gly Ala Thr Ile Val Met Val Thr His Ser Glu His Asp Ala Arg
    210                 215                 220
Ser Ala Gly Arg Ile Ile Asn Leu Phe Asp Gly Lys Ile Arg
225                 230                 235

(2) INFORMATION FOR SEQ ID NO:275

    (i) SEQUENCE CHARACTERISTICS:
        (A) LENGTH: 604 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...604

    (xi) SEQUENCE DESCRIPTION: SEQ ID NO:275

Ser Thr Glu Thr Asn Ser Lys Ser Glu Met Lys Glu Phe Phe Lys Met
1               5                   10                  15
Phe Phe Ala Ser Ile Leu Gly Val Ile Thr Ala Gly Ile Ile Leu Phe
            20                  25                  30
Cys Ile Phe Leu Phe Ile Phe Phe Gly Ile Val Ala Gly Ile Ala Ser
        35                  40                  45
Lys Ala Thr Gly Gly Thr Ile Pro Lys Ile Glu Ala Asn Ser Ile Leu
    50                  55                  60
His Ile Unk Asn Ser Ser Phe Pro Glu Ile Val Ser Ala Asn Pro Trp
65                  70                  75                  80
Ser Met Leu Thr Gly Lys Asp Glu Ser Val Ser Leu Ser Gln Ala Val
                85                  90                  95
Glu Ala Ile Gly Gln Ala Lys Asn Asn Pro Asn Ile Thr Gly Ile Phe
            100                 105                 110
Leu Asp Leu Asp Asn Leu Ser Val Gly Met Ala Ser Ala Glu Glu Leu
        115                 120                 125
Arg Arg Ala Leu Gln Asp Phe Lys Met Ser Gly Lys Phe Val Val Ser
    130                 135                 140
Tyr Ala Asp Arg Tyr Thr Gln Lys Gly Tyr Tyr Leu Ser Ser Ile Ala
145                 150                 155                 160
Asp Lys Leu Tyr Leu Asn Pro Lys Gly Met Leu Gly Leu Ile Gly Ile
                165                 170                 175
Ala Thr Gln Thr Met Phe Tyr Lys Asp Ala Leu Asp Lys Phe Gly Val
            180                 185                 190
Lys Met Glu Ile Phe Lys Val Gly Thr Tyr Lys Ala Ala Val Glu Pro
        195                 200                 205
Phe Met Leu Asn Arg Met Ser Asp Ala Asn Arg Glu Gln Ile Thr Thr
    210                 215                 220
Tyr Ile Asn Gly Leu Trp Asp Lys Ile Thr Ser Asp Ile Ala Glu Ser
225                 230                 235                 240
Arg Lys Thr Ala Met Asp Ser Val Lys Met Phe Ala Asp Lys Gly Glu
                245                 250                 255
Met Phe Gly Leu Ala Glu Lys Ala Val Glu Met Lys Leu Val Asp Glu
            260                 265                 270
Leu Ala Tyr Arg Thr Asp Val Glu Lys Glu Leu Lys Lys Met Ser Gln
        275                 280                 285
Arg Gly Glu Lys Asp Glu Leu Arg Phe Val Ser Leu Ser Gln Val Leu
    290                 295                 300
Ala Asn Gly Pro Met Asn Lys Thr Lys Gly Ser Arg Ile Ala Val Leu
305                 310                 315                 320
Phe Ala Glu Gly Glu Ile Thr Glu Glu Ile Ile Lys Lys Pro Phe Asp
                325                 330                 335
Thr Asp Gly Ser Ser Ile Thr Gln Glu Leu Ala Lys Glu Ile Lys Ala
            340                 345                 350
Ala Ala Asp Asp Asp Asp Ile Lys Ala Val Val Leu Arg Val Asn Ser
        355                 360                 365
Pro Gly Gly Ser Ala Phe Thr Ser Glu Gln Ile Trp Lys Gln Val Ala

370                 375                 380
Asp Leu Lys Ala Lys Lys Pro Ile Val Val Ser Met Gly Asp Val Ala
385                 390                 395                 400
Ala Ser Gly Gly Tyr Tyr Ile Ala Cys Ala Ala Asn Ser Ile Val Ala
                405                 410                 415
Glu His Thr Thr Leu Thr Gly Ser Ile Gly Ile Phe Gly Met Phe Pro
            420                 425                 430
Asn Phe Ala Gly Val Ala Lys Lys Ile Gly Val Asn Met Asp Val Val
        435                 440                 445
Gln Thr Ser Lys Tyr Ala Asp Leu Gly Asn Thr Phe Ala Pro Met Thr
    450                 455                 460
Val Glu Asp Arg Ala Leu Ile Gln Arg Tyr Ile Glu Gln Gly Tyr Asp
465                 470                 475                 480
Leu Phe Leu Thr Arg Val Ser Glu Gly Arg Asn Arg Thr Lys Ala Gln
                485                 490                 495
Ile Asp Ser Ile Ala Gln Gly Arg Val Trp Leu Gly Asp Lys Ala Leu
            500                 505                 510
Ala Leu Gly Leu Val Asp Glu Leu Gly Gly Leu Asp Thr Ala Ile Lys
        515                 520                 525
Arg Ala Ala Lys Leu Ala Gln Leu Gly Gly Asn Tyr Ser Ile Glu Tyr
    530                 535                 540
Gly Lys Thr Lys Arg Asn Phe Phe Glu Glu Leu Leu Ser Ser Ser Ala
545                 550                 555                 560
Ala Asp Met Lys Ser Ala Ile Leu Ser Thr Ile Leu Ser Asp Pro Glu
                565                 570                 575
Ile Glu Val Leu Arg Glu Leu Arg Ser Met Pro Pro Arg Pro Ser Gly
            580                 585                 590
Ile Gln Ala Arg Leu Pro Tyr Tyr Phe Met Pro Tyr
        595                 600

(2) INFORMATION FOR SEQ ID NO:276

    (i) SEQUENCE CHARACTERISTICS:
        (A) LENGTH: 324 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...324

    (xi) SEQUENCE DESCRIPTION: SEQ ID NO:276

Leu Thr Leu Phe Cys Cys Asn Tyr Phe Lys Gln Met Arg Ala Asn Ile
1               5                   10                  15
Trp Gln Ile Leu Ser Val Ser Val Leu Phe Phe Phe Gly Thr Ala Ile
            20                  25                  30
Gly Gln Ala Gln Ser Arg Asn Arg Thr Tyr Glu Ala Tyr Val Lys Gln
        35                  40                  45
Tyr Ala Asp Glu Ala Ile Arg Gln Met Ser Arg Tyr Asn Ile Pro Ala
    50                  55                  60
Ser Ile Thr Ile Ala Gln Ala Leu Val Glu Thr Gly Ala Gly Ala Ser
65                  70                  75                  80
Thr Leu Ala Ser Val His Asn Asn His Phe Gly Ile Lys Cys His Lys
                85                  90                  95
Ser Trp Thr Gly Lys Arg Thr Tyr Arg Thr Asp Asp Ala Pro Asn Glu
            100                 105                 110
Cys Phe Arg Ser Tyr Ser Ala Ala Arg Glu Ser Tyr Glu Asp His Ser
        115                 120                 125
Arg Phe Leu Leu Gln Pro Arg Tyr Arg Pro Leu Phe Lys Leu Asp Arg
    130                 135                 140
Glu Asp Tyr Arg Gly Trp Ala Thr Gly Leu Gln Arg Cys Gly Tyr Ala
145                 150                 155                 160
Thr Asn Arg Gly Tyr Ala Asn Leu Leu Ile Lys Met Val Glu Leu Tyr
                165                 170                 175
Glu Leu Tyr Ala Leu Asp Arg Glu Lys Tyr Pro Ser Trp Phe His Lys
            180                 185                 190
Ser Tyr Pro Gly Ser Asn Lys Lys Ser His Gln Thr Thr Lys Gln Lys

195                 200                 205
Gln Ser Gly Leu Lys His Glu Ala Tyr Phe Ser Tyr Gly Leu Leu Tyr
    210                 215                 220
Ile Ile Ala Lys Gln Gly Asp Thr Phe Asp Ser Leu Ala Glu Glu Phe
225                 230                 235                 240
Asp Met Arg Ala Ser Lys Leu Ala Lys Tyr Asn Asp Ala Pro Val Asp
                245                 250                 255
Phe Pro Ile Glu Lys Gly Asp Val Ile Tyr Leu Glu Lys Lys His Ala
            260                 265                 270
Cys Ser Ile Ser Lys His Thr Gln His Val Val Arg Val Gly Asp Ser
        275                 280                 285
Met His Ser Ile Ser Gln Arg Tyr Gly Ile Arg Met Lys Asn Leu Tyr
    290                 295                 300
Lys Leu Asn Asp Lys Asp Gly Glu Tyr Ile Pro Gln Glu Gly Asp Ile
305                 310                 315                 320
Leu Arg Leu Arg

(2) INFORMATION FOR SEQ ID NO:277

(i) SEQUENCE CHARACTERISTICS:
(A) LENGTH: 533 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...533

(xi) SEQUENCE DESCRIPTION: SEQ ID NO:277

Arg Ile Pro Asp Glu Gln Thr Gly Arg Ile Met Asp Gly Arg Arg Tyr
1               5                   10                  15
Ser Asp Gly Leu His Gln Ala Ile Glu Ala Lys Glu His Val Lys Val
            20                  25                  30
Glu Ala Ala Thr Gln Thr Phe Ala Thr Ile Thr Leu Gln Asn Tyr Phe
        35                  40                  45
Arg Met Tyr His Lys Leu Ala Gly Met Thr Gly Thr Ala Glu Thr Glu
    50                  55                  60
Ala Gly Glu Leu Trp Asp Ile Tyr Lys Leu Asp Val Val Val Ile Pro
65                  70                  75                  80
Thr Asn Lys Pro Ile Ala Arg Lys Asp Met Asn Asp Arg Ile Tyr Lys
                85                  90                  95
Thr Ala Arg Glu Lys Tyr Ala Ala Val Ile Glu Glu Ile Val Arg Leu
            100                 105                 110
Val Glu Glu Gly Arg Pro Val Leu Val Gly Thr Thr Ser Val Glu Ile
        115                 120                 125
Ser Glu Leu Leu Ser Arg Met Leu Arg Leu Arg Gly Ile Gln His Asn
    130                 135                 140
Val Leu Asn Ala Lys Leu His Gln Lys Glu Ala Glu Ile Val Ala Gln
145                 150                 155                 160
Ala Gly Gln Lys Gly Thr Val Thr Ile Ala Thr Asn Met Ala Gly Arg
                165                 170                 175
Gly Thr Asp Ile Lys Leu Ser Ala Glu Val Lys Lys Ala Gly Gly Leu
            180                 185                 190
Ala Ile Ile Gly Thr Glu Arg His Glu Ser Arg Arg Val Asp Arg Gln
        195                 200                 205
Leu Arg Gly Arg Ser Gly Arg Gln Gly Asp Pro Gly Ser Ser Ile Phe
    210                 215                 220
Tyr Val Ser Leu Glu Asp His Leu Met Arg Leu Phe Ala Thr Glu Lys
225                 230                 235                 240
Ile Ala Ser Leu Met Asp Arg Leu Gly Phe Lys Glu Gly Glu Val Leu
                245                 250                 255
Glu Asn Asn Met Leu Ser Lys Ser Val Glu Arg Ala Gln Lys Lys Val
            260                 265                 270
Glu Glu Asn Asn Phe Gly Ile Arg Lys His Leu Leu Glu Tyr Asp Asp
        275                 280                 285
Val Met Asn Ser Gln Arg Glu Val Ile Tyr Thr Arg Arg Arg His Ala

290                     295                     300
Leu Met Gly Glu Arg Ile Gly Met Asp Val Leu Asn Thr Ile Tyr Asp
305                 310                 315                 320
Val Cys Lys Ala Leu Ile Asp Asn Tyr Ala Glu Ala Asn Asp Phe Glu
                325                 330                 335
Gly Phe Lys Glu Asp Leu Met Arg Ala Leu Ala Ile Glu Ser Pro Ile
            340                 345                 350
Thr Gln Glu Ile Phe Arg Gly Lys Lys Ala Glu Glu Leu Thr Asp Met
        355                 360                 365
Leu Phe Asp Glu Ala Tyr Lys Ser Phe Gln Arg Lys Met Asp Leu Ile
    370                 375                 380
Ala Glu Val Ala His Pro Val Val His Gln Val Phe Glu Thr Gln Ala
385                 390                 395                 400
Ala Val Tyr Glu Arg Ile Leu Ile Pro Ile Thr Asp Gly Lys Arg Val
                405                 410                 415
Tyr Asn Ile Gly Cys Asn Leu Arg Glu Ala Asp Glu Thr Gln Gly Lys
            420                 425                 430
Ser Ile Ile Lys Glu Phe Glu Lys Ala Ile Val Leu His Thr Ile Asp
        435                 440                 445
Glu Ser Trp Lys Glu His Leu Arg Glu Met Asp Glu Leu Arg Asn Ser
    450                 455                 460
Val Gln Asn Ala Ser Tyr Glu Asn Lys Asp Pro Leu Leu Ile Tyr Lys
465                 470                 475                 480
Leu Glu Ser Tyr Glu Leu Phe Arg Lys Met Val Glu Ala Met Asn Arg
                485                 490                 495
Lys Thr Val Ala Ile Leu Met Arg Ala Arg Ile Pro Val Pro Glu Ala
            500                 505                 510
Pro Ser Gln Glu Glu Leu Glu His Arg Arg Gln Ile Glu Ile Arg His
        515                 520                 525
Ala Thr Gln Gln Arg
    530

(2) INFORMATION FOR SEQ ID NO:278

     (i) SEQUENCE CHARACTERISTICS:
          (A) LENGTH: 720 amino acids
          (B) TYPE: amino acid
          (D) TOPOLOGY: linear

    (ii) MOLECULE TYPE: protein

   (iii) HYPOTHETICAL: YES

    (vi) ORIGINAL SOURCE:
          (A) ORGANISM: Porphyromonas gingivalis

    (ix) FEATURE:
          (A) NAME/KEY: misc_feature
          (B) LOCATION 1...720

    (xi) SEQUENCE DESCRIPTION: SEQ ID NO:278

Lys Ser Cys Arg Val Ile Gly Gln Thr Arg Arg Tyr Gly Cys Cys Pro
1               5                   10                  15
Val Gly Leu Arg Pro Cys His Gln Ser Leu Ser Gly Val Cys Arg Cys
            20                  25                  30
Leu Phe Gln Ser Arg Pro Ala Val Ala Phe Ala Arg Lys Gly Gln Arg
        35                  40                  45
Arg His Arg Arg Ser Glu Ser Gly Arg Arg Ile Arg Ala Leu Gln Gly
    50                  55                  60
Val Gln His His Gln Thr Asn Glu His Glu Val Met Ile Ser Val Asn
65                  70                  75                  80
Asn Leu Thr Val Asp Phe Gly Thr Arg Leu Leu Phe Asp Gln Val Ser
                85                  90                  95
Phe Val Ile Asn Arg Arg Asp Arg Ile Ala Leu Val Gly Lys Asn Gly
            100                 105                 110
Ala Gly Lys Ser Thr Leu Leu Lys Leu Ile Ala Gly Met Glu Glu Pro
        115                 120                 125
Thr Ser Gly His Ile Ala Arg Pro Lys Gly Ile Arg Ile Gly Tyr Leu
    130                 135                 140
Pro Gln Val Met Arg Leu Gln Asp Gly His Thr Val Tyr Glu Glu Val
145                 150                 155                 160
Glu Gln Ala Phe Asn Asp Ile Arg Gln Ile Glu Glu Glu Ile Arg Arg
                165                 170                 175
Leu Ser Asp Glu Met Ala Gly Arg Thr Asp Tyr Glu Ser Asp Asp Tyr

            180                 185                 190
Ile Arg Leu Ile Glu His Tyr Thr Asn Met Ser Glu Thr Leu Ser Leu
        195                 200                 205
Met Gln Gln Gly Asn Tyr His Ala Ala Ile Glu Gln Thr Leu Ile Gly
    210                 215                 220
Leu Gly Phe Gly Arg Glu Asp Phe His Arg Pro Thr Ala Asp Phe Ser
225                 230                 235                 240
Gly Gly Trp Arg Met Arg Ile Glu Leu Ala Lys Leu Leu Leu Gln Arg
                245                 250                 255
Pro Glu Val Leu Leu Leu Asp Glu Pro Thr Asn His Leu Asp Ile Glu
            260                 265                 270
Ser Ile Gly Trp Leu Glu Gln Phe Ile Ala Thr Asn Ala Gly Ala Val
        275                 280                 285
Ile Leu Val Ser His Asp Arg Ala Phe Ile Asp Asn Thr Thr Thr Arg
    290                 295                 300
Thr Ile Glu Ile Glu Leu Gly His Ile Tyr Asp Tyr Lys Thr Asn Tyr
305                 310                 315                 320
Ser His Tyr Val Glu Leu Arg Glu Glu Arg Leu Arg Gln Gln Met Arg
                325                 330                 335
Ala Tyr Glu Asn Gln Gln Lys Met Ile Arg Asp Thr Glu Asp Phe Ile
            340                 345                 350
Glu Arg Phe Arg Tyr Lys Ala Thr Lys Ser Val Gln Val Gln Ser Arg
        355                 360                 365
Ile Lys Gln Leu Glu Lys Val Glu Arg Val Glu Ile Asp Glu Arg Asp
    370                 375                 380
Arg Ser Ala Phe His Phe Arg Phe Ile Pro Ala Gln Pro Ser Gly Ser
385                 390                 395                 400
Tyr Pro Leu Ile Val Asp Asp Leu Ala Lys Ala Tyr Gly Asp His Gln
                405                 410                 415
Val Phe Ser Gly Ala Thr Tyr Thr Ile Glu Arg Gly Glu Lys Val Ala
            420                 425                 430
Phe Val Gly Lys Asn Gly Ala Gly Lys Ser Thr Met Val Lys Cys Ile
        435                 440                 445
Met Gly Glu Leu Thr Asp Tyr Thr Gly Lys Leu Glu Leu Gly His Asn
    450                 455                 460
Val Gln Leu Gly Tyr Phe Ala Gln Asn Glu Ala Gln Glu Leu Arg Gly
465                 470                 475                 480
Asp Leu Thr Val Phe Asp Thr Ile Asp Arg Glu Ala Val Gly Asp Ile
                485                 490                 495
Arg Leu Arg Leu Asn Asp Leu Leu Gly Ala Phe Leu Phe Gly Gly Glu
            500                 505                 510
Ala Ser Glu Lys Lys Val Ser Val Leu Ser Gly Gly Glu Arg Ala Arg
        515                 520                 525
Leu Ala Ile Ile Arg Leu Leu Leu Gln Pro Ala Asn Phe Leu Ile Leu
    530                 535                 540
Asp Glu Pro Thr Asn His Leu Asp Met Arg Ser Lys Asp Val Leu Lys
545                 550                 555                 560
Glu Ala Ile Lys Asn Phe Asp Gly Thr Val Ile Val Val Ser His Asp
                565                 570                 575
Arg Glu Phe Leu Asp Gly Leu Val Ser Lys Val Tyr Glu Phe Ala Asp
            580                 585                 590
Gly Gln Val Asn Glu His Leu Gly Gly Ile Tyr Asp Tyr Leu Arg Thr
        595                 600                 605
Arg Arg Met Gln Thr Leu Thr Glu Leu Glu Arg Thr Thr Thr Ile Glu
    610                 615                 620
Thr Lys Thr Thr Arg Glu Ala Ile Pro Glu Thr Glu Ala Lys Ala Asp
625                 630                 635                 640
Tyr Arg Arg Gln Lys Glu Val Ala Lys Gln Leu Arg Thr Leu Glu Arg
                645                 650                 655
Thr Val Ala Thr Cys Glu Glu Arg Ile Gly Lys Leu Glu Ser Glu Leu
            660                 665                 670
Gln Ala Ile Glu Met Leu Leu Gln Asp Pro Lys His Ala Thr Asp Ala
        675                 680                 685
Asn Leu Phe Glu Arg Tyr Ala Gly Met Lys Gln Glu Leu Glu Lys Ala
    690                 695                 700
Met Glu Asp Trp Glu Gln Ala Ser Glu Ala Leu Ser Glu Ala Gln Gly
705                 710                 715                 720

(2) INFORMATION FOR SEQ ID NO:279

(i) SEQUENCE CHARACTERISTICS:
(A) LENGTH: 386 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...386

(xi) SEQUENCE DESCRIPTION: SEQ ID NO:279

Tyr Leu Lys Leu Leu Ile Leu Gln Ile Ala Leu Met Asn Phe Leu Lys
1               5                   10                  15
Lys Glu Pro Phe Lys Ile Phe Ser Met Ile Tyr Leu Leu Leu Asp Thr
            20                  25                  30
Ile Thr Asn Arg Ala Gly Thr Glu Arg Ala Val Ile Asn Leu Ala Asn
        35                  40                  45
Asn Leu His Ala Asn Gly His Arg Val Ser Leu Val Ser Val Cys Thr
    50                  55                  60
Lys Glu Gly Glu Pro Ser Phe Gln Val Glu Lys Gly Ile Glu Val His
65                  70                  75                  80
His Leu Gly Ile Arg Leu Tyr Gly Asn Ala Leu Ala Arg Lys Thr Val
                85                  90                  95
Tyr Phe Lys Ala Tyr Arg Arg Ile Lys Ala Leu Tyr Lys Lys Arg Glu
            100                 105                 110
Pro Val Leu Leu Ile Gly Thr Asn Ile Phe Ile Asn Thr Ile Leu Ser
        115                 120                 125
Gln Ile Ser Asn Arg Gly Arg Ile Phe Thr Ile Gly Cys Glu His Ile
    130                 135                 140
Ser Tyr Asp Ile Ala Arg Pro Ile Thr Lys Arg Ile Arg Gly Phe Leu
145                 150                 155                 160
Tyr Ser Gly Leu Asp Ala Val Val Ala Leu Thr Lys Arg Asp Gln Gln
                165                 170                 175
Ser Phe Glu Ala Ile Leu Arg Gly Arg Ser Lys Ala Tyr Val Ile Pro
            180                 185                 190
Asn Gln Val Ser Phe Thr Thr Val Gln Arg Asp Ala Thr Thr His Lys
        195                 200                 205
Gln Met Leu Ala Ile Gly Arg Leu Thr Tyr Gln Lys Gly Phe Glu Phe
    210                 215                 220
Met Ile Glu Asp Ala Ser Arg Val Leu Arg Glu Arg Pro Asp Trp Lys
225                 230                 235                 240
Leu Ile Ile Val Gly Asp Gly Glu Asn Glu Ser Met Leu Arg Lys Glu
                245                 250                 255
Ile Ala Ser Arg Asn Met Glu Ser Gln Ile Glu Ile His Pro Ser Thr
            260                 265                 270
Pro Glu Ile Arg Lys Tyr Tyr Glu Ser Ser Ala Ile Tyr Leu Met Thr
        275                 280                 285
Ser Arg Phe Glu Gly Leu Pro Met Val Leu Leu Glu Ala Glu Ala Tyr
    290                 295                 300
Ala Leu Pro Ile Ile Ser Tyr Asp Cys Pro Thr Gly Pro Arg Glu Leu
305                 310                 315                 320
Ile Glu Asn Gly Arg Asn Gly Phe Leu Val Pro Met Glu Ala His Glu
                325                 330                 335
Asp Phe Ala Asp Lys Leu Arg Leu Leu Met Asp Asp Glu Thr Leu Arg
            340                 345                 350
Lys Lys Met Gly Gln Glu Ser Glu Leu Met Val Lys Ser Tyr Ser Pro
        355                 360                 365
Ala Asn Ile Tyr Glu Cys Trp Lys Lys Leu Phe Val Glu Ile Gly Tyr
    370                 375                 380
Met Asn
385

(2) INFORMATION FOR SEQ ID NO:280

(i) SEQUENCE CHARACTERISTICS:
(A) LENGTH: 655 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...655

(xi) SEQUENCE DESCRIPTION: SEQ ID NO:280

Thr Thr Lys Glu Asn Glu Thr Thr Thr Lys Asn Glu Tyr Arg Ile Met
1               5                   10                  15
Gly Lys Ile Ile Gly Ile Asp Leu Gly Thr Thr Asn Ser Cys Val Ser
            20                  25                  30
Val Leu Glu Gly Asn Glu Pro Ile Val Ile Thr Asn Ser Glu Gly Lys
        35                  40                  45
Arg Thr Thr Pro Ser Val Val Ala Phe Val Asp Gly Gly Glu Arg Lys
    50                  55                  60
Val Gly Asp Pro Ala Lys Arg Gln Ala Ile Thr Asn Pro Thr Lys Thr
65                  70                  75                  80
Ile Tyr Ser Ile Lys Arg Phe Met Gly Glu Thr Tyr Asp Gln Val Ser
                85                  90                  95
Arg Glu Val Glu Arg Val Pro Phe Lys Val Val Arg Gly Asp Asn Asn
            100                 105                 110
Thr Pro Arg Val Asp Ile Asp Gly Arg Leu Tyr Thr Pro Gln Glu Ile
        115                 120                 125
Ser Ala Met Ile Leu Gln Lys Met Lys Lys Thr Ala Glu Asp Tyr Leu
    130                 135                 140
Gly Gln Glu Val Thr Glu Ala Val Ile Thr Val Pro Ala Tyr Phe Asn
145                 150                 155                 160
Asp Ala Gln Arg Gln Ala Thr Lys Glu Ala Gly Glu Ile Ala Gly Leu
                165                 170                 175
Lys Val Arg Arg Ile Val Asn Glu Pro Thr Ala Ala Ser Leu Ala Tyr
            180                 185                 190
Gly Leu Asp Lys Ser Asn Lys Asp Met Lys Ile Ala Val Phe Asp Leu
        195                 200                 205
Gly Gly Gly Thr Phe Asp Ile Ser Ile Leu Glu Leu Gly Asp Gly Val
    210                 215                 220
Phe Glu Val Lys Ser Thr Asn Gly Asp Thr His Leu Gly Gly Asp Asp
225                 230                 235                 240
Phe Asp His Val Ile Ile Asp Trp Leu Ala Glu Glu Phe Lys Ser Gln
                245                 250                 255
Glu Gly Val Asp Leu Arg Gln Asp Pro Met Ala Met Gln Arg Leu Lys
            260                 265                 270
Glu Ala Ala Glu Lys Ala Lys Ile Glu Leu Ser Ser Thr Ser Ser Thr
        275                 280                 285
Glu Ile Asn Leu Pro Tyr Ile Met Pro Val Asn Gly Ile Pro Lys His
    290                 295                 300
Leu Val Met Thr Leu Thr Arg Ala Lys Phe Glu Gln Leu Ala Asp Arg
305                 310                 315                 320
Leu Ile Gln Ala Cys Val Ala Pro Cys Glu Thr Ala Leu Lys Asp Ala
                325                 330                 335
Gly Met Ser Arg Gly Asp Ile Asp Glu Val Ile Leu Val Gly Gly Ser
            340                 345                 350
Thr Arg Ile Pro Ala Ile Gln Glu Ile Val Glu Lys Ile Phe Gly Lys
        355                 360                 365
Ala Pro Ser Lys Gly Val Asn Pro Asp Glu Val Val Ala Val Gly Ala
    370                 375                 380
Ala Ile Gln Gly Gly Val Leu Thr Gly Glu Val Lys Asp Val Leu Leu
385                 390                 395                 400
Leu Asp Val Thr Pro Leu Ser Leu Gly Ile Glu Thr Met Gly Gly Val
                405                 410                 415
Met Thr Arg Leu Ile Asp Ala Asn Thr Thr Ile Pro Thr Lys Lys Ser
            420                 425                 430
Glu Ile Phe Thr Thr Ala Val Asp Asn Gln Pro Ser Val Glu Ile His
        435                 440                 445
Val Leu Gln Gly Glu Arg Ser Leu Ala Lys Asp Asn Lys Ser Ile Gly
    450                 455                 460
Arg Phe Asn Leu Asp Gly Ile Ala Pro Ala Pro Arg Gln Thr Pro Gln
465                 470                 475                 480
Ile Glu Val Thr Phe Asp Ile Asp Ala Asn Gly Ile Leu Asn Val Thr
                485                 490                 495
Ala His Asp Lys Ala Thr Gly Lys Lys Gln Asn Ile Arg Ile Glu Ala
            500                 505                 510
Ser Ser Gly Leu Ser Asp Asp Glu Ile Lys Arg Met Lys Glu Glu Ala
        515                 520                 525

Gln Ala Asn Ala Glu Ala Asp Lys Lys Glu Lys Glu Arg Ile Asp Lys
    530                 535                 540
Ile Asn Gln Ala Asp Ser Met Ile Phe Gln Thr Glu Lys Gln Leu Lys
545                 550                 555                 560
Glu Leu Gly Asp Lys Phe Pro Ala Asp Lys Lys Ala Pro Ile Asp Thr
                565                 570                 575
Ala Leu Asp Lys Leu Lys Glu Ala His Lys Ala Gln Asp Val Ala Ala
            580                 585                 590
Ile Asp Thr Ala Met Ala Glu Leu Gln Thr Ala Leu Ser Ala Ala Gly
        595                 600                 605
Glu Glu Leu Tyr Lys Asn Ala Gly Ala Ala Gln Gly Gly Ala Gln Pro
    610                 615                 620
Gly Pro Asp Phe Gly Gly Ala Gln Gly Pro Ser Ala Gly Asp Gln Pro
625                 630                 635                 640
Ser Asp Asp Lys Asn Val Thr Asp Val Asp Phe Glu Glu Val Lys
                645                 650                 655

(2) INFORMATION FOR SEQ ID NO:281

    (i) SEQUENCE CHARACTERISTICS:
        (A) LENGTH: 467 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...467

    (xi) SEQUENCE DESCRIPTION: SEQ ID NO:281

Lys Trp Ala Arg Thr Thr Thr Leu Arg Ile Ser Asn Ala Thr Ile Tyr
1               5                   10                  15
Lys Thr Met Arg Tyr Asp Leu Ala Ile Ile Gly Gly Gly Pro Ala Gly
            20                  25                  30
Tyr Thr Ala Ala Glu Arg Ala Ala Lys Gly Gly Leu Lys Thr Leu Leu
        35                  40                  45
Ile Glu Lys Asn Ala Leu Gly Gly Val Cys Leu Asn Glu Gly Cys Ile
    50                  55                  60
Pro Thr Lys Thr Leu Leu Tyr Ser Ala Lys Val Leu His Gln Ile Ala
65                  70                  75                  80
Thr Ala Ser Lys Tyr Ala Val Ser Gly Thr Ala Asp Gly Leu Asp Leu
                85                  90                  95
Gly Lys Val Ile Ala Arg Lys Gly Lys Ile Ile Arg Lys Leu Thr Ala
            100                 105                 110
Gly Ile Arg Ser Arg Leu Thr Glu Ala Gly Val Glu Met Val Thr Ala
        115                 120                 125
Glu Ala Thr Val Thr Gly Cys Asp Ala Asp Gly Ile Ile Gly Ile Thr
    130                 135                 140
Ala Gly Glu Ala Gln Tyr Lys Ala Ala Asn Leu Leu Leu Cys Thr Gly
145                 150                 155                 160
Ser Glu Thr Phe Ile Pro Pro Ile Pro Gly Val Glu Gln Thr Glu Tyr
                165                 170                 175
Trp Thr Asn Arg Glu Ala Leu Gln Asn Lys Glu Ile Pro Thr Ser Leu
            180                 185                 190
Val Ile Ile Gly Gly Gly Val Ile Gly Met Glu Phe Ala Ser Phe Phe
        195                 200                 205
Asn Gly Ile Gly Thr Gln Val His Val Val Glu Met Leu Pro Glu Ile
    210                 215                 220
Leu Asn Gly Ile Asp Pro Glu His Ala Ala Met Leu Arg Ala His Tyr
225                 230                 235                 240
Glu Lys Glu Gly Ile Lys Phe Tyr Leu Gly His Lys Val Thr Ser Val
                245                 250                 255
Arg Asn Gly Ala Val Thr Val Glu Tyr Glu Gly Glu Ser Lys Glu Ile
            260                 265                 270
Glu Gly Glu Arg Ile Leu Met Ser Val Gly Arg Arg Pro Val Leu Gln
        275                 280                 285
Gly Phe Glu Ser Leu Gly Leu Val Leu Ala Gly Lys Gly Val Lys Thr
    290                 295                 300

Asn Glu Arg Met Gln Thr Ser Leu Pro Asn Val Tyr Ala Ala Gly Asp
305                 310                 315                 320
Ile Thr Gly Phe Ser Leu Leu Ala His Thr Ala Val Arg Glu Ala Glu
                325                 330                 335
Val Ala Val Asp Gln Ile Leu Gly Lys Thr Asp Glu Thr Met Ser Tyr
            340                 345                 350
Arg Ala Val Pro Gly Val Val Tyr Thr Asn Pro Glu Val Ala Gly Val
        355                 360                 365
Gly Glu Thr Glu Glu Ser Leu Arg Lys Ala Gly Arg Ala Tyr Thr Val
    370                 375                 380
Arg Arg Leu Pro Met Ala Phe Ser Gly Arg Phe Val Ala Glu Asn Glu
385                 390                 395                 400
Gln Gly Asn Gly Glu Cys Lys Leu Leu Leu Asp Glu Glu Asn Arg Leu
                405                 410                 415
Ile Gly Ala His Leu Ile Gly Asn Pro Ala Gly Glu Leu Ile Val Thr
            420                 425                 430
Ala Ala Met Ala Ile Glu Thr Gly Met Thr Asp Arg Gln Ile Glu Arg
        435                 440                 445
Ile Ile Phe Pro His Pro Thr Val Gly Glu Ile Leu Lys Glu Thr Leu
    450                 455                 460
Ala Gly Gly
465

(2) INFORMATION FOR SEQ ID NO:282

(i) SEQUENCE CHARACTERISTICS:
(A) LENGTH: 945 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...945

(xi) SEQUENCE DESCRIPTION: SEQ ID NO:282

Pro Lys Ile Leu Met Glu Leu Lys Arg Phe Leu Ser Leu Gly Leu Leu
1               5                   10                  15
Leu Val Gly Phe Ile Pro Met Lys Leu Ser Ala Gln Gln Ala Gln Pro
            20                  25                  30
Leu Pro Thr Asp Pro Ala Val Arg Val Gly Lys Leu Asp Asn Gly Leu
        35                  40                  45
Thr Tyr Phe Ile Arg His Asn Glu Asn Pro Lys Asp Arg Ala Asp Phe
    50                  55                  60
Phe Ile Ala Gln Lys Val Gly Ser Ile Leu Glu Glu Asp Ser Gln Ser
65                  70                  75                  80
Gly Leu Ala His Phe Leu Glu His Met Ala Phe Asn Gly Thr Lys Asn
                85                  90                  95
Phe Pro Gly Lys Asn Leu Ile Asn Tyr Leu Glu Thr Ile Gly Val Arg
            100                 105                 110
Phe Gly Gln Asn Leu Asn Ala Ser Thr Gly Phe Asp Lys Thr Glu Tyr
        115                 120                 125
Thr Ile Met Asp Val Pro Thr Thr Arg Gln Gly Ile Ile Asp Ser Cys
    130                 135                 140
Leu Leu Ile Leu His Asp Trp Ser Asn Asn Ile Thr Leu Asp Gly His
145                 150                 155                 160
Glu Ile Asp Glu Glu Arg Gly Val Ile Gln Glu Glu Trp Arg Ala Arg
                165                 170                 175
Arg Asp Ala Asn Leu Arg Met Phe Glu Ala Ile Leu Ala Lys Ala Met
            180                 185                 190
Pro Gly Asn Lys Tyr Ala Glu Arg Met Pro Ile Gly Leu Met Asp Val
        195                 200                 205
Val Leu Asn Phe Lys His Asp Glu Leu Arg Asn Tyr Tyr Lys Lys Trp
    210                 215                 220
Tyr Arg Pro Asp Leu Gln Gly Leu Val Ile Val Gly Asp Ile Asp Val
225                 230                 235                 240
Asp Tyr Val Glu Asn Lys Ile Lys Glu Leu Phe Lys Asp Val Pro Ala
                245                 250                 255

Pro Val Asn Pro Ala Glu Arg Ile Tyr Thr Pro Val Glu Asp Asn Asp
            260                 265                 270
Glu Pro Ile Val Ala Ile Ala Thr Asp Ala Glu Ala Thr Thr Thr Gln
        275                 280                 285
Leu Ser Ile Ser Phe Lys Ser Asp Pro Thr Pro Gln Glu Val Arg Gly
    290                 295                 300
Ser Ile Phe Gly Leu Val Glu Asp Tyr Met Lys Gln Val Ile Thr Thr
305                 310                 315                 320
Ala Val Asn Glu Arg Leu Ser Glu Ile Thr His Lys Pro Asn Ala Pro
                325                 330                 335
Phe Leu Ser Ala Gly Ala Phe Phe Ser Asn Phe Met Tyr Ile Thr Gln
            340                 345                 350
Thr Lys Asp Ala Phe Asn Phe Val Ala Thr Val Arg Glu Gly Glu Ala
        355                 360                 365
Glu Lys Ala Met Asn Ala Leu Val Ala Glu Ile Glu Ser Leu Arg Gln
    370                 375                 380
Phe Gly Ile Thr Lys Gly Glu Tyr Asp Arg Ala Arg Thr Asn Val Leu
385                 390                 395                 400
Lys Arg Tyr Glu Asn Gln Tyr Asn Glu Arg Asp Lys Arg Lys Asn Asn
                405                 410                 415
Ala Tyr Ala Asn Glu Tyr Ser Thr Tyr Phe Thr Asp Gly Gly Tyr Ile
            420                 425                 430
Pro Gly Ile Glu Val Glu Tyr Gln Thr Val Asn Ala Phe Ala Pro Gln
        435                 440                 445
Val Pro Leu Glu Ala Phe Asn Gln Ala Ile Ala Gln Met Ile Asp Pro
    450                 455                 460
Val Lys Asn Ala Val Val Thr Leu Thr Gly Pro Ser Lys Ala Glu Ala
465                 470                 475                 480
Lys Ile Pro Ser Glu Ala Asp Phe Leu Ala Ala Phe Lys Ala Ala Arg
                485                 490                 495
Gln Gln Lys Val Glu Ala Lys Lys Asp Glu Val Ser Asp Gln Lys Leu
            500                 505                 510
Met Glu Lys Ala Pro Lys Ala Gly Lys Ile Val Ser Glu Lys Lys Asp
        515                 520                 525
Gln Lys Phe Gly Thr Thr Glu Leu Thr Leu Ser Asn Gly Ile Lys Val
    530                 535                 540
Tyr Leu Lys Lys Thr Asp Phe Lys Ser Asn Glu Ile Leu Met Ser Ala
545                 550                 555                 560
Leu Ser Pro Gly Gly Ile Leu Ser Gly Lys His Ala Pro Asn Gln Ser
                565                 570                 575
Val Met Asn Ser Phe Met Asn Val Gly Gly Leu Gly Asn Phe Asp Ala
            580                 585                 590
Ile Gln Leu Asp Lys Val Leu Thr Gly Arg Ser Ala Ser Val Ser Pro
        595                 600                 605
Ser Leu Ser Leu Leu Ser Glu Gly Leu Ser Gly Lys Thr Thr Val Glu
    610                 615                 620
Asp Met Glu Thr Phe Phe Gln Leu Ile Tyr Leu Gln Met Thr Ala Asn
625                 630                 635                 640
Arg Lys Asp Pro Glu Ala Phe Lys Ala Thr Gln Glu Lys Leu Tyr Asn
                645                 650                 655
Asn Leu Lys Asn Gln Glu Ala Asn Pro Met Ala Ala Leu Met Asp Ser
            660                 665                 670
Ile Arg His Thr Met Tyr Gly Asp Asn Pro Met Met Lys Pro Met Lys
        675                 680                 685
Ala Ala Asp Val Glu Lys Val Asn Tyr Asp Gln Val Met Ala Phe Tyr
    690                 695                 700
Asn Glu Arg Phe Ala Asp Ala Gly Asp Phe Met Phe Phe Phe Ile Gly
705                 710                 715                 720
Asn Leu Asp Glu Ala Lys Met Lys Pro Leu Ile Glu Thr Tyr Leu Ala
                725                 730                 735
Ser Leu Pro Asn Leu Lys Arg Gly Asp Lys Met Asn Lys Ala Gln Val
            740                 745                 750
Pro Ala Ala Arg Ser Gly Lys Ile Asp Cys Lys Phe Glu Lys Glu Met
        755                 760                 765
Asp Thr Pro Ser Thr Thr Ile Phe Asp Val Val Ser Gly Asn Val Glu
    770                 775                 780
Tyr Thr Leu Lys Asn Ser Leu Leu Leu Glu Val Phe Ser Ala Val Met
785                 790                 795                 800
Asp Gln Val Tyr Thr Ala Thr Val Arg Glu Lys Glu Gly Gly Ala Tyr
                805                 810                 815
Ser Val Ala Ala Phe Gly Gly Leu Glu Gln Tyr Pro Gln Pro Lys Ala
            820                 825                 830
Leu Met Gln Ile Tyr Phe Pro Thr Asp Pro Ala Arg Ala Glu Glu Met
        835                 840                 845
Asn Ala Ile Val Phe Ala Glu Leu Glu Lys Leu Ala Lys Glu Gly Pro

850                 855                 860
Asn Val Glu Tyr Phe Lys Lys Thr Ile Glu Asn Leu Asn Lys Gln His
865                 870                 875                 880
Lys Glu Ser Leu Arg Glu Asn Arg Phe Trp Leu Glu Ala Met Lys Ala
                885                 890                 895
Ser Phe Phe Glu Gly Asn Asp Phe Ile Thr Asp Tyr Glu Ser Val Leu
            900                 905                 910
Asn Gly Leu Thr Pro Ala Glu Leu Gln Lys Phe Ala Ala Asp Leu Leu
        915                 920                 925
Lys Gln Gln Asn Arg Val Val Val Met Met Ala Pro Val Ala Lys Ala
    930                 935                 940
Gln
945

(2) INFORMATION FOR SEQ ID NO:283

     (i) SEQUENCE CHARACTERISTICS:
          (A) LENGTH: 686 amino acids
          (B) TYPE: amino acid
          (D) TOPOLOGY: linear

    (ii) MOLECULE TYPE: protein

   (iii) HYPOTHETICAL: YES

    (vi) ORIGINAL SOURCE:
          (A) ORGANISM: Porphyromonas gingivalis

    (ix) FEATURE:
          (A) NAME/KEY: misc_feature
          (B) LOCATION 1...686

    (xi) SEQUENCE DESCRIPTION: SEQ ID NO:283

Tyr Thr Met Ser Lys Lys Gly Thr Ile Gly Val Thr Ser Asp Asn Ile
1               5                   10                  15
Phe Pro Val Ile Lys Lys Phe Leu Tyr Ser Asp His Glu Ile Phe Leu
            20                  25                  30
Arg Glu Ile Val Ser Asn Ala Val Asp Ala Thr Gln Lys Leu Lys Thr
        35                  40                  45
Leu Thr Ser Val Gly Glu Phe Lys Gly Glu Thr Gly Asp Leu Arg Val
    50                  55                  60
Thr Val Ser Val Asp Glu Val Ala Arg Thr Ile Thr Val Ser Asp Arg
65                  70                  75                  80
Gly Val Gly Met Thr Glu Glu Glu Val Glu Lys Tyr Ile Asn Gln Ile
                85                  90                  95
Ala Phe Ser Ser Ala Glu Glu Phe Leu Glu Lys Tyr Lys Asp Asp Lys
            100                 105                 110
Ala Ala Ile Ile Gly His Phe Gly Leu Gly Phe Tyr Ser Ala Phe Met
        115                 120                 125
Val Ser Glu Arg Val Asp Val Ile Thr Arg Ser Phe Arg Glu Asp Ala
    130                 135                 140
Thr Ala Val Lys Trp Ser Cys Asp Gly Ser Pro Glu Tyr Thr Leu Glu
145                 150                 155                 160
Pro Ala Asp Lys Ala Asp Arg Gly Thr Asp Ile Val Met His Ile Asp
                165                 170                 175
Glu Glu Asn Ser Glu Phe Leu Lys Lys Glu Lys Ile Glu Gly Leu Leu
            180                 185                 190
Gly Lys Tyr Cys Lys Phe Leu Thr Val Pro Ile Ile Phe Gly Lys Lys
        195                 200                 205
Gln Glu Trp Lys Asp Gly Lys Met Gln Asp Thr Asp Glu Asp Asn Gln
    210                 215                 220
Ile Asn Asp Thr His Pro Ala Trp Thr Lys Lys Pro Ala Asp Leu Lys
225                 230                 235                 240
Asp Glu Asp Tyr Lys Glu Phe Tyr Arg Ser Leu Tyr Pro Met Ser Glu
                245                 250                 255
Glu Pro Leu Phe Trp Ile His Leu Asn Val Asp Tyr Pro Phe Asn Leu
            260                 265                 270
Thr Gly Ile Leu Tyr Phe Pro Lys Ile Lys Asn Asn Leu Asp Leu Gln
        275                 280                 285
Arg Asn Lys Ile Gln Leu Tyr Cys Asn Gln Val Tyr Val Thr Asp Glu
    290                 295                 300
Val Gln Gly Ile Val Pro Asp Phe Leu Thr Leu Leu His Gly Val Ile
305                 310                 315                 320
Asp Ser Pro Asp Ile Pro Leu Asn Val Ser Arg Ser Tyr Leu Gln Ser

                325                 330                 335
Asp Ala Asn Val Lys Lys Ile Ser Ser His Ile Thr Lys Lys Val Ala
            340                 345                 350
Asp Arg Leu Glu Glu Ile Phe Lys Asn Asp Arg Pro Thr Phe Glu Glu
        355                 360                 365
Lys Trp Asp Ser Leu Lys Leu Phe Val Glu Tyr Gly Met Leu Thr Asp
    370                 375                 380
Glu Lys Phe Tyr Glu Arg Ala Ala Lys Phe Phe Leu Phe Thr Asp Met
385                 390                 395                 400
Asp Gly His Lys Tyr Thr Phe Asp Glu Tyr Arg Thr Leu Val Glu Gly
                405                 410                 415
Val Gln Thr Asp Lys Asp Gly Gln Val Val Tyr Leu Tyr Ala Thr Asp
            420                 425                 430
Lys His Gly Gln Tyr Ser His Val Lys Arg Ala Ser Asp Lys Gly Tyr
        435                 440                 445
Ser Val Met Leu Leu Asp Gly Gln Leu Asp Pro His Ile Val Ser Leu
    450                 455                 460
Leu Glu Gln Lys Leu Glu Lys Thr His Phe Val Arg Val Asp Ser Asp
465                 470                 475                 480
Thr Ile Asn Asn Leu Ile Arg Lys Glu Glu Arg Ala Glu Val Lys Leu
                485                 490                 495
Ser Asp Thr Glu Arg Ala Thr Leu Val Lys Leu Phe Glu Ala Arg Leu
            500                 505                 510
Pro Arg Asp Glu Lys Lys His Phe Asn Val Ala Phe Glu Ser Leu Gly
        515                 520                 525
Ala Glu Gly Glu Ala Ile Leu Ile Thr Gln Ala Glu Phe Met Arg Arg
    530                 535                 540
Met Arg Asp Met Ala Gln Leu Gln Pro Gly Met Ser Phe Tyr Gly Glu
545                 550                 555                 560
Leu Pro Asp Ser Tyr Asn Leu Val Leu Asn Thr Asp His Pro Leu Ile
                565                 570                 575
Asp Arg Val Leu Ser Gly Glu Lys Glu Ser Val Glu Pro Ser Leu Thr
            580                 585                 590
Glu Leu Arg Ala Lys Ile Ala Glu Leu Lys Ala Glu Glu Ala Lys Leu
        595                 600                 605
Leu Asp Glu Glu Lys Gly Lys Lys Pro Glu Glu Ile Pro Val Ala Thr
    610                 615                 620
Lys Glu Ala Lys Glu Asn Asn Ala Val Glu Gln Ala Lys Thr Glu Gly
625                 630                 635                 640
Ser Ile Asn Asp Gln Leu Thr Lys Tyr Ala Gln Asp Asn Glu Leu Ile
                645                 650                 655
Gly Gln Leu Ile Asp Leu Ala Leu Leu Gly Ser Gly Leu Leu Thr Gly
            660                 665                 670
Glu Ala Leu Ala Glu Phe Ile Arg Arg Ser Gln Arg Leu Leu
        675                 680                 685

(2) INFORMATION FOR SEQ ID NO:284

(i) SEQUENCE CHARACTERISTICS:
(A) LENGTH: 482 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...482

(xi) SEQUENCE DESCRIPTION: SEQ ID NO:284

Asp Ile Arg Gln Lys Arg Pro Cys Phe Asn Ala Asn Leu Tyr Phe Tyr
1               5                   10                  15
Arg Ala Met Glu Lys Leu Ile Asp Ile Leu Val Val Asp Asp Asp Val
            20                  25                  30
Ala Val Cys Ala Ala Leu Arg Leu Val Leu Lys Arg Ala Gly Tyr Asn
        35                  40                  45
Pro Val Ile Ala Asn Ser Pro Asp Glu Ala Leu Ser Ile Met Arg Asn
    50                  55                  60
Pro Asp Gly Gly Cys Lys Pro Ala Val Ile Leu Met Asp Met Asn Phe

65                  70                  75                  80
Ser Leu Ser Thr Ser Gly Arg Glu Gly Leu Glu Leu Leu Glu Lys Met
                85                  90                  95
Gln Ile Phe Thr Ser Cys Pro Val Ile Leu Met Thr Ala Trp Ala Ser
            100                 105                 110
Ile Pro Leu Ala Val Glu Gly Met Arg Leu Gly Ala Phe Asp Phe Ile
        115                 120                 125
Gly Lys Pro Trp Asp Asn Asp Arg Leu Leu Arg Thr Ile Asp Thr Ala
    130                 135                 140
Leu His Leu Ala Ala Pro Ser Ala Val Ala Asn Pro Ser Glu Gln Ser
145                 150                 155                 160
Asp Arg Asp Thr Ala Arg Gln Pro Lys Ala Thr Val Gln Glu Asn Asp
                165                 170                 175
Pro Cys Ala His Ile Ile Gly Arg Ser Asp Ala Ile Cys Lys Ile Lys
            180                 185                 190
Glu Arg Ile Arg Arg Ile Ala Pro Thr His Ala Ser Val Leu Ile Thr
        195                 200                 205
Gly Glu Ser Gly Thr Gly Lys Glu Leu Ile Ala Glu Ala Leu His Arg
    210                 215                 220
Gly Ser Lys Arg Ala Ser Ala Pro Phe Val Lys Val Asn Leu Gly Gly
225                 230                 235                 240
Ile Pro Glu Ser Leu Phe Glu Ser Glu Leu Phe Gly His Lys Lys Gly
                245                 250                 255
Ala Phe Thr Asn Ala Phe Ser Asp Arg Lys Gly Arg Phe Glu Leu Ala
            260                 265                 270
Asp Gly Gly Thr Ile Phe Leu Asp Glu Ile Gly Glu Leu Pro Val Gly
        275                 280                 285
Asn Gln Val Lys Leu Leu Arg Val Leu Gln Glu Gln Thr Phe Glu Pro
    290                 295                 300
Leu Gly Glu Ser Val Ser His Arg Val Asp Ile Arg Val Val Ser Ala
305                 310                 315                 320
Thr Asn Ala Ser Leu Glu Arg Met Val Ala Glu Gly Arg Phe Arg Glu
                325                 330                 335
Asp Leu Tyr Tyr Arg Ile Asn Leu Ile His Leu His Leu Pro Pro Leu
            340                 345                 350
Arg Glu Arg Gln Glu Asp Ile Gln Leu Leu Val Glu Ala Phe Ser Glu
        355                 360                 365
Ala Phe Ala Gln Ser Asn Gly Leu Pro His Ala Val Trp Ser Ala Glu
    370                 375                 380
Ala Met Arg Arg Ile Cys Ala Met Pro Leu Pro Gly Asn Val Arg Glu
385                 390                 395                 400
Leu Lys Asn Val Val Glu Arg Thr Leu Leu Leu Ser Gly Ser Arg Glu
                405                 410                 415
Ile Ser Ala Arg Asp Val Ala Asp Phe Gly Ser Gln Val Thr Ala Ala
            420                 425                 430
Asp His Ser Asp Glu Arg Ala Leu Thr Asp Met Glu Glu Ala Ala Ile
        435                 440                 445
Arg Glu Thr Leu Thr Lys Tyr Asn Gly Asn Val Ser Arg Ala Ala Arg
    450                 455                 460
Ala Leu Gly Leu Ser Arg Ala Ala Leu Tyr Arg Arg Met Glu Lys Tyr
465                 470                 475                 480
Gly Leu

(2) INFORMATION FOR SEQ ID NO:285

(i) SEQUENCE CHARACTERISTICS:
(A) LENGTH: 263 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...263

(xi) SEQUENCE DESCRIPTION: SEQ ID NO:285

Arg Ser Leu Gln Ser Phe Gln Asn Lys Lys His Ser Ser Met Leu Lys

1               5                   10                  15
Ile Lys Asn Leu His Ala Thr Val Gln Gly Lys Glu Ile Leu Lys Gly
            20                  25                  30
Ile Asn Leu Glu Ile Asn Ala Gly Glu Ile His Ala Ile Met Gly Pro
        35                  40                  45
Asn Gly Ser Gly Lys Ser Thr Leu Ser Ser Val Leu Val Gly His Pro
    50                  55                  60
Ser Phe Glu Val Thr Glu Gly Glu Val Thr Phe Asn Gly Ile Asp Leu
65                  70                  75                  80
Leu Glu Leu Glu Pro Glu Glu Arg Ala His Leu Gly Leu Phe Leu Ser
                85                  90                  95
Phe Gln Tyr Pro Val Glu Ile Pro Gly Val Ser Met Val Asn Phe Met
            100                 105                 110
Arg Ala Ala Val Asn Glu His Arg Lys Ala Ile Gly Ala Glu Pro Val
        115                 120                 125
Ser Ala Ser Asp Phe Leu Lys Met Met Arg Glu Lys Arg Ala Ile Val
    130                 135                 140
Glu Leu Asp Asn Lys Leu Ala Ser Arg Ser Val Asn Glu Gly Phe Ser
145                 150                 155                 160
Gly Gly Glu Lys Lys Arg Asn Glu Ile Phe Gln Met Ala Met Leu Glu
                165                 170                 175
Pro Lys Leu Ala Ile Leu Asp Glu Thr Asp Ser Gly Leu Asp Ile Asp
            180                 185                 190
Ala Leu Arg Ile Val Ala Gly Gly Val Asn Arg Leu Arg Ser Pro Glu
        195                 200                 205
Asn Ala Ala Ile Val Ile Thr His Tyr Gln Arg Leu Leu Glu Tyr Ile
    210                 215                 220
Lys Pro Asp Phe Val His Val Leu Tyr Lys Gly Arg Ile Val Lys Ser
225                 230                 235                 240
Gly Gly Ala Glu Leu Ala Leu Thr Leu Glu Glu Lys Gly Tyr Asp Trp
                245                 250                 255
Ile Lys Glu Glu Ile Gly Glu
            260

(2) INFORMATION FOR SEQ ID NO:286

     (i) SEQUENCE CHARACTERISTICS:
          (A) LENGTH: 462 amino acids
          (B) TYPE: amino acid
          (D) TOPOLOGY: linear

    (ii) MOLECULE TYPE: protein

   (iii) HYPOTHETICAL: YES

    (vi) ORIGINAL SOURCE:
          (A) ORGANISM: Porphyromonas gingivalis

    (ix) FEATURE:
          (A) NAME/KEY: misc_feature
          (B) LOCATION 1...462

    (xi) SEQUENCE DESCRIPTION: SEQ ID NO:286

Ser Met Ala Lys Glu Lys Thr Ile Tyr Val Cys Arg Ser Cys Gly Thr
1               5                   10                  15
Lys Tyr Ala Lys Trp Gln Gly Asn Cys Asn Ala Cys Gly Glu Trp Asn
            20                  25                  30
Cys Ile Asp Glu Glu Lys Val Pro Ala Pro Ala Ser Gly Lys His Ala
        35                  40                  45
Ala Lys Ser Phe Met Pro Arg Glu Gln Asp Asn Arg Pro Arg Leu Leu
    50                  55                  60
Gln Asp Val Glu Ser Gly Asp Glu Glu Arg Ile Arg Leu Gly Asp Glu
65                  70                  75                  80
Glu Phe Asp Arg Val Leu Gly Gly Gly Ile Val Lys Gly Ala Phe Val
                85                  90                  95
Leu Leu Gly Gly Glu Pro Gly Ile Gly Lys Ser Thr Leu Ile Leu Gln
            100                 105                 110
Thr Val Leu Arg Leu Pro Gln Leu Arg Thr Leu Tyr Val Ser Gly Glu
        115                 120                 125
Glu Ser Ala Arg Gln Leu Lys Met Arg Ala Glu Arg Leu Gly Gln Ala
    130                 135                 140
Met Asn Gly Cys Tyr Val Tyr Cys Glu Thr Asn Ile Glu Arg Ile Leu
145                 150                 155                 160
Ser Arg Ala Glu Glu Leu Thr Pro Asp Leu Leu Val Ile Asp Ser Ile

                    165                 170                 175
Gln Thr Val Tyr Thr Glu Glu Met Glu Ser Ser Ala Gly Ser Val Gly
            180                 185                 190
Gln Ile Arg Glu Cys Ala Ala Leu Leu Leu Lys Tyr Cys Lys Thr Thr
        195                 200                 205
Gly Ile Pro Val Ile Val Ile Gly His Ile Thr Lys Glu Gly Ser Ile
    210                 215                 220
Ala Gly Pro Lys Val Leu Glu His Ile Val Asp Thr Val Leu Leu Phe
225                 230                 235                 240
Asp Gly Asp Lys His His Leu Tyr Arg Ile Leu Arg Gly Gln Lys Asn
                245                 250                 255
Arg Tyr Gly Ser Thr Ser Glu Leu Gly Ile Tyr Glu Met Arg Gln Asp
            260                 265                 270
Gly Leu Arg Gly Val Glu Asn Pro Ser Glu His Leu Ile Thr Arg Asn
        275                 280                 285
Arg Glu Asp Leu Ser Gly Ile Ala Ile Ala Val Ala Met Glu Gly Ile
    290                 295                 300
Arg Pro Ile Leu Ile Glu Ala Gln Ala Leu Val Ser Ser Ala Ile Tyr
305                 310                 315                 320
Ala Asn Pro Gln Arg Ser Ala Thr Gly Phe Asp Ile Arg Arg Met Asn
                325                 330                 335
Met Leu Leu Ala Val Leu Glu Lys Arg Ala Gly Phe Lys Leu Ile Gln
            340                 345                 350
Lys Asp Val Phe Leu Asn Ile Ala Gly Gly Ile Lys Ile Ala Asp Pro
        355                 360                 365
Ala Thr Asp Leu Ala Val Ile Ser Ala Val Leu Ala Ser Ser Leu Asp
    370                 375                 380
Ile Val Ile Pro Pro Ala Val Cys Met Thr Gly Glu Val Gly Leu Ser
385                 390                 395                 400
Gly Glu Ile Arg Pro Val Ser Arg Ile Glu Gln Arg Ile Thr Glu Ala
                405                 410                 415
Arg Arg Ile Gly Phe Lys Glu Ile Leu Val Pro Ala Asp Asn Phe Arg
            420                 425                 430
Gln Glu Asp Ala Gly Arg Phe Gly Ile Arg Leu Val Pro Val Arg Lys
        435                 440                 445
Val Glu Glu Ala Phe Arg His Leu Phe Ser Lys Gly Arg Glu
    450                 455                 460

(2) INFORMATION FOR SEQ ID NO:287

     (i) SEQUENCE CHARACTERISTICS:
          (A) LENGTH: 373 amino acids
          (B) TYPE: amino acid
          (D) TOPOLOGY: linear

    (ii) MOLECULE TYPE: protein

   (iii) HYPOTHETICAL: YES

    (vi) ORIGINAL SOURCE:
          (A) ORGANISM: Porphyromonas gingivalis

    (ix) FEATURE:
          (A) NAME/KEY: misc_feature
          (B) LOCATION 1...373

    (xi) SEQUENCE DESCRIPTION: SEQ ID NO:287

Gly Ser Cys Gly Ser Ser Pro Ala Ile Gly Arg Ser Gly Cys Ser Arg
1               5                   10                  15
Cys Asp Arg Arg Ile Gly His Gly Leu Ser Thr Ser Ala Phe Arg Thr
            20                  25                  30
Tyr Ala Gly Leu Val Cys His Ser Arg Leu Arg Asp Gln Ser Ser Cys
        35                  40                  45
Arg Val Cys Gly Met Arg Pro Ile Gly Gln Leu Phe Phe Pro Ser Cys
    50                  55                  60
Pro Arg Arg Ser Ala Ser Asp Ala Gly Arg Thr Glu Arg Ile Gln Ser
65                  70                  75                  80
Pro Leu Gln Asn Leu Leu Gln Glu Met Gln Phe Ser Pro Thr Asn Arg
                85                  90                  95
Arg Asn Pro Phe Asp Asn Met Asn Ser Arg His Leu Thr Ile Thr Ile
            100                 105                 110
Ile Ala Gly Leu Ser Leu Phe Val Leu Thr Leu Gly Gly Cys Ser Val
        115                 120                 125
Ala Gln Gln Asp Thr Gln Trp Thr Leu Gly Gly Lys Leu Phe Thr Ser

130                 135                 140
Ala Trp Ile Gln Arg Ser Ala Glu Tyr Gln Ala Leu Cys Ile Gln Ala
145                 150                 155                 160
Tyr Asn Ile Ala Thr Glu Arg Val Asp Ala Leu Pro Ala Glu Arg Lys
                165                 170                 175
Gln Gly Asp Arg Pro Tyr Ala Ile Val Thr Asp Ile Asp Glu Thr Ile
            180                 185                 190
Leu Asp Asn Thr Pro Asn Ser Val Tyr Gln Ala Leu Arg Gly Lys Asp
        195                 200                 205
Tyr Asp Glu Glu Thr Trp Gly Lys Trp Cys Ala Gln Ala Asp Ala Asp
    210                 215                 220
Thr Leu Ala Gly Ala Leu Ser Phe Phe Leu His Ala Ala Asn Lys Gly
225                 230                 235                 240
Ile Glu Val Phe Tyr Val Thr Asn Arg Arg Asp Asn Leu Arg Glu Ala
                245                 250                 255
Thr Leu Gln Asn Leu Gln Arg Tyr Gly Phe Pro Phe Ala Asp Glu Glu
            260                 265                 270
His Leu Leu Thr Thr His Gly Pro Ser Asp Lys Glu Pro Arg Arg Leu
        275                 280                 285
Lys Ile Gln Glu Gln Tyr Glu Ile Val Leu Leu Ile Gly Asp Asn Leu
    290                 295                 300
Gly Asp Phe His His Phe Phe Asn Thr Lys Glu Glu Ser Gly Arg Lys
305                 310                 315                 320
Gln Ala Leu Gly Leu Thr Ala Gly Glu Phe Gly Arg His Phe Ile Met
                325                 330                 335
Leu Pro Asn Pro Asn Tyr Gly Ser Trp Glu Pro Ala Trp Tyr Gly Gly
            340                 345                 350
Lys Tyr Pro Pro Leu Pro Glu Arg Asp Lys Ala Leu Lys Gln Leu His
        355                 360                 365
Ser Gln Asn Ser Arg
    370

(2) INFORMATION FOR SEQ ID NO:288

    (i) SEQUENCE CHARACTERISTICS:
        (A) LENGTH: 426 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...426

    (xi) SEQUENCE DESCRIPTION: SEQ ID NO:288

Arg Trp Gly Phe Ser Asn Phe Val Ala Met Ser Thr Asn Ile Asp Val
1               5                   10                  15
Gln Gln Ile Lys Gln Arg Phe Gly Ile Ile Gly Ser Ser Pro Leu Met
            20                  25                  30
Glu His Ala Ile Arg Val Ala Ala Gln Val Ala Pro Thr Asp Met Ser
        35                  40                  45
Val Leu Val Thr Gly Glu Ser Gly Ser Gly Lys Glu Phe Phe Pro Gln
    50                  55                  60
Ile Ile His Tyr Tyr Ser Ala Arg Lys His His Ser Tyr Ile Ala Val
65                  70                  75                  80
Asn Cys Gly Ala Ile Pro Glu Gly Thr Ile Asp Ser Glu Leu Phe Gly
                85                  90                  95
His Arg Lys Gly Ser Phe Thr Gly Ala Val Ser Asp Arg Lys Gly Tyr
            100                 105                 110
Phe Glu Glu Ala Ser Gly Gly Thr Ile Phe Leu Asp Glu Val Gly Glu
        115                 120                 125
Leu Pro Leu Pro Thr Gln Ala Arg Leu Leu Arg Val Leu Glu Thr Gly
    130                 135                 140
Glu Phe Ile Pro Val Gly Ala Ser Gln Ser Gln Lys Thr Asp Val Arg
145                 150                 155                 160
Ile Val Ala Ala Thr Asn Val Asn Leu Lys Glu Ala Val Ala Asn Gly
                165                 170                 175
Lys Phe Arg Glu Asp Leu Phe Phe Arg Leu Asn Thr Val Pro Ile Glu

180                 185                 190
Val Pro Ala Leu Arg Met Arg Pro Asp Asp Val Pro Leu Leu Phe Arg
        195                 200                 205
Arg Phe Ala Ala Asp Ser Ala Glu Lys Tyr Arg Met Pro Pro Leu Arg
    210                 215                 220
Leu Ser Asp Glu Ala Arg Thr Ile Leu Met Arg Tyr Arg Trp Pro Gly
225                 230                 235                 240
Asn Val Arg Glu Leu Arg Asn Ile Thr Asp Arg Leu Ser Ile Leu Glu
                245                 250                 255
Glu Glu Arg Thr Val Ser Ala Glu Thr Ile Thr Arg Tyr Leu Asp Ala
            260                 265                 270
Glu Gly Met Gln Asp Leu His Pro Val Val Ile Arg Arg Asn Glu Thr
        275                 280                 285
Thr Glu Ala Asp Lys Gln Ile Pro His Tyr Glu Arg Glu Ile Ile Tyr
    290                 295                 300
Gln Val Leu Tyr Asp Met Lys Lys Glu Ile Ala Asp Leu Lys Gly Met
305                 310                 315                 320
Met Asn Arg Leu Ala His His Glu Gln Pro Ser Trp Pro Val Gly Ser
                325                 330                 335
Asp Val Trp Gly Asn Asp Asp Lys Arg Thr Ala Asp Pro Lys Trp Gly
            340                 345                 350
Val Ser Thr His Lys Ala Pro Ile Ala Asn Ala Ala Glu Pro Val Glu
        355                 360                 365
Pro Ile Gln Glu Ala Ser Glu Tyr Thr Glu Asp Pro Val Ser Leu Glu
    370                 375                 380
Glu Val Glu Lys Lys Met Ile Ser Leu Ala Leu Glu Arg His Gly Gly
385                 390                 395                 400
Arg Arg Lys Gln Thr Ala Glu Glu Leu Lys Ile Ser Glu Arg Thr Leu
                405                 410                 415
Tyr Arg Lys Ile Lys Glu Tyr Gly Leu Glu
            420                 425

(2) INFORMATION FOR SEQ ID NO:289

     (i) SEQUENCE CHARACTERISTICS:
          (A) LENGTH: 653 amino acids
          (B) TYPE: amino acid
          (D) TOPOLOGY: linear

    (ii) MOLECULE TYPE: protein

   (iii) HYPOTHETICAL: YES

    (vi) ORIGINAL SOURCE:
          (A) ORGANISM: Porphyromonas gingivalis

    (ix) FEATURE:
          (A) NAME/KEY: misc_feature
          (B) LOCATION 1...653

    (xi) SEQUENCE DESCRIPTION: SEQ ID NO:289

Lys Asn Leu Pro Arg Tyr Ala Pro Ala Phe Gly Arg Asn Arg Lys Arg
1               5                   10                  15
Glu Gln Pro Asp Pro Asp Asn Asp Asp Ser Arg Gly Arg Ser His Arg
            20                  25                  30
Gln Ser Gly Arg Ala Val Thr Val Val Cys Arg Arg Ile Gln Thr Pro
        35                  40                  45
Leu Pro His Met Ile Leu Leu Phe Gly Gly Thr Thr Glu Gly Arg Ala
    50                  55                  60
Ala Ala Arg Val Leu Asp Glu Ala Gly Ser Pro Phe Phe Tyr Ser Thr
65                  70                  75                  80
Lys Gly Asn Leu Gln Glu Ile Gln Ser Ser His Gly His Arg Leu Thr
                85                  90                  95
Gly Ala Met Thr Val Ala Asp Met Val Ser Phe Cys Arg Lys Glu Glu
            100                 105                 110
Ile Arg Leu Ile Val Asp Ala Ala His Pro Phe Ala Glu Glu Leu His
        115                 120                 125
Ala Ser Val Ala Glu Ala Ser Glu Gln Thr Gly Ile Pro Val Val Arg
    130                 135                 140
Tyr Glu Arg Gln Tyr Pro Pro Arg Glu Glu Gly Ile Val Trp Cys Ala
145                 150                 155                 160
Asn Tyr Asp Thr Ala Ala Glu Arg Met Leu Gly Asp Gly Val Gln Arg
                165                 170                 175
Leu Leu Met Leu Thr Gly Val Asn Thr Ile Pro Lys Leu Ala Ala Phe

                180                 185                 190
Trp Lys Glu Arg Thr Thr Phe Cys Arg Ile Leu Lys Arg Asp Glu Ser
        195                 200                 205
Val Ala Leu Ala Glu Lys Asn Gly Phe Pro Ala Glu Arg Ile Val Phe
    210                 215                 220
Phe Glu Pro His Ala Asp Glu Glu Leu Met Gln Ala Val Arg Pro Asp
225                 230                 235                 240
Ala Ile Ile Thr Lys Glu Ser Gly Glu Ser Gly Tyr Phe Arg Glu Lys
                245                 250                 255
Ile Glu Ala Ala Arg Arg Met Gly Ile Arg Ile Tyr Ala Val Val Arg
            260                 265                 270
Pro Pro Leu Pro Pro Ser Phe Ile Pro Val Gly Gly Pro Val Gly Leu
        275                 280                 285
Arg Arg Ala Val Glu Arg Leu Val Pro Gly Phe Phe Ser Leu Arg Ser
    290                 295                 300
Gly Phe Thr Thr Gly Thr Thr Ala Thr Ala Ala Val Val Ala Ala Met
305                 310                 315                 320
Tyr Arg Leu Met Gly Leu Gly Ser Leu Ala Glu Ala Pro Val Glu Leu
                325                 330                 335
Pro Ser Gly Glu Ile Val Ser Leu Pro Ile Ala Glu Ile Arg Glu Glu
            340                 345                 350
Glu Asp Ala Val Val Ser Ala Val Leu Lys Asp Ala Gly Asp Asp Pro
        355                 360                 365
Asp Val Thr Asn Gly Met Ala Val Cys Ala Thr Ile Arg Leu Asn Pro
    370                 375                 380
Glu His Glu Glu Val Arg Phe Leu Gln Gly Glu Gly Val Gly Val Val
385                 390                 395                 400
Thr Leu Pro Gly Leu Gly Leu Glu Val Gly Gly Pro Ala Ile Asn Leu
                405                 410                 415
Val Pro Arg Arg Met Met Thr Ala Glu Val Arg Arg Leu Tyr Ala Gln
            420                 425                 430
Gly Gly Val Asp Ile Thr Ile Ser Val Pro Glu Gly Arg Glu Ala Ala
        435                 440                 445
Thr Gln Thr Phe Asn Pro Arg Leu Gly Ile Arg Asp Gly Ile Ser Ile
    450                 455                 460
Ile Gly Thr Ser Gly Val Val Lys Pro Phe Ser Ala Glu Ala Phe Val
465                 470                 475                 480
Gly Ala Ile Arg Lys Gln Val Gly Ile Ala Thr Ala Leu Gly Ala Asn
                485                 490                 495
His Ile Val Leu Asn Ser Gly Ala Lys Ser Glu Arg Tyr Val Lys Gly
            500                 505                 510
Ala Tyr Pro Ala Leu Ile Pro Gln Ala Phe Val Gln Tyr Gly Asn Phe
        515                 520                 525
Val Gly Glu Ser Leu Ser Cys Val Ala Ser Phe Pro Ser Val Arg Ser
    530                 535                 540
Val Thr Val Gly Ile Met Leu Gly Lys Ala Val Lys Leu Ala Glu Gly
545                 550                 555                 560
Tyr Leu Asp Thr His Ser Lys Lys Val Val Met Asn Arg Asp Phe Leu
                565                 570                 575
His Glu Leu Ala Arg Gln Ala Gly Cys Ser Glu Asp Ile His Ala Ile
            580                 585                 590
Ile Asp Ser Leu Asn Leu Ala Arg Glu Leu Trp Thr Met Pro Ser Ala
        595                 600                 605
Glu Asp Ser Asp Arg Leu Leu Arg Lys Ile Ala Glu Arg Ser Trp Glu
    610                 615                 620
Thr Cys Arg Pro Ser Val Pro Ser Ala Glu Leu Glu Leu Leu Leu Ile
625                 630                 635                 640
Asp Glu Ser Gly Ala Ile Arg Phe Arg Ile Gly Gly Glu
                645                 650

(2) INFORMATION FOR SEQ ID NO:290

     (i) SEQUENCE CHARACTERISTICS:
          (A) LENGTH: 451 amino acids
          (B) TYPE: amino acid
          (D) TOPOLOGY: linear

    (ii) MOLECULE TYPE: protein

   (iii) HYPOTHETICAL: YES

    (vi) ORIGINAL SOURCE:
          (A) ORGANISM: Porphyromonas gingivalis

    (ix) FEATURE:

(A) NAME/KEY: misc_feature
(B) LOCATION 1...451

(xi) SEQUENCE DESCRIPTION: SEQ ID NO:290

Gln Gln Arg Pro His Arg Ile Ile Met Leu Arg Thr Phe Arg Ile Gly
1               5                   10                  15
Gly Ile His Pro Pro Glu Asn Lys Leu Ser Ala Gly Lys Pro Val Glu
            20                  25                  30
Val Leu Pro Ile Pro Ser Gln Val Val Ile Pro Leu Gly Gln His Ile
        35                  40                  45
Gly Ala Pro Ala Thr Ala Thr Val Lys Lys Gly Asp Glu Val Lys Val
    50                  55                  60
Gly Thr Ile Ile Ala Gln Ala Gly Gly Phe Val Ser Ala Asn Ile His
65                  70                  75                  80
Ser Ser Val Ser Gly Lys Val Leu Lys Ile Asp Asn Val Tyr Asp Ser
                85                  90                  95
Ser Gly Tyr Pro Lys Pro Ala Val Phe Ile Ser Val Glu Gly Asp Glu
            100                 105                 110
Trp Glu Glu Gly Ile Asp Arg Ser Pro Ala Ile Val Lys Glu Cys Asn
        115                 120                 125
Leu Asp Ala Lys Glu Ile Val Ala Lys Ile Ser Ala Ala Gly Ile Val
    130                 135                 140
Gly Leu Gly Gly Ala Thr Phe Pro Thr His Val Lys Leu Ser Pro Pro
145                 150                 155                 160
Pro Gly Asn Lys Ala Glu Ile Leu Ile Ile Asn Ala Val Glu Cys Glu
                165                 170                 175
Pro Tyr Leu Thr Ser Asp His Val Leu Met Leu Glu His Gly Glu Glu
            180                 185                 190
Ile Met Ile Gly Val Ser Ile Leu Met Lys Ala Ile Gln Val Asn Lys
        195                 200                 205
Ala Val Ile Gly Val Glu Asn Asn Lys Lys Asp Ala Ile Ala His Leu
    210                 215                 220
Thr Lys Leu Ala Thr Ala Tyr Pro Gly Ile Glu Val Met Pro Leu Lys
225                 230                 235                 240
Val Gln Tyr Pro Gln Gly Gly Glu Lys Gln Leu Ile Asp Ala Val Ile
                245                 250                 255
Arg Lys Gln Val Lys Ser Gly Ala Leu Pro Ile Ser Thr Gly Ala Val
            260                 265                 270
Val Gln Asn Val Gly Thr Val Phe Ala Val Tyr Glu Ala Val Gln Lys
        275                 280                 285
Asn Lys Pro Leu Val Glu Arg Ile Val Thr Val Thr Gly Lys Lys Leu
    290                 295                 300
Ser Arg Pro Ser Asn Leu Leu Val Arg Ile Gly Thr Pro Ile Ala Ala
305                 310                 315                 320
Leu Ile Glu Ala Ala Gly Gly Leu Pro Glu Asn Thr Gly Lys Ile Ile
                325                 330                 335
Gly Gly Gly Pro Met Met Gly Arg Ala Leu Leu Ser Pro Asp Val Pro
            340                 345                 350
Val Thr Lys Gly Ser Ser Gly Val Leu Ile Leu Asp Arg Glu Glu Ala
        355                 360                 365
Val Arg Lys Pro Met Arg Asp Cys Ile Arg Cys Ala Lys Cys Val Gly
    370                 375                 380
Val Cys Pro Met Gly Leu Asn Pro Ala Phe Leu Met Arg Asp Thr Leu
385                 390                 395                 400
Tyr Lys Ser Trp Glu Thr Ala Glu Lys Gly Asn Val Val Asp Cys Ile
                405                 410                 415
Glu Cys Gly Ser Cys Ser Phe Thr Cys Pro Ala Asn Arg Pro Leu Leu
            420                 425                 430
Asp Tyr Ile Arg Gln Ala Lys Lys Thr Val Met Gly Ile Gln Arg Ala
        435                 440                 445
Arg Lys Gln
    450

(2) INFORMATION FOR SEQ ID NO:291

(i) SEQUENCE CHARACTERISTICS:
(A) LENGTH: 489 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...489

(xi) SEQUENCE DESCRIPTION: SEQ ID NO:291

Met Asn Ser Gln Lys Lys Glu Ala Phe Asn Met Lys Arg Ile Gln Leu
1               5                   10                  15
Thr Leu Ile Ala Leu Phe Ala Ala Val Ala Gly Leu Val Ala Gln Asn
            20                  25                  30
Ala Tyr Glu Gly Val Ile Ser Tyr Lys Ile Ser Leu Asp Lys Thr Gly
        35                  40                  45
Asn Lys Val Val Leu Asn Gly Ala Ala Asp Met Ser Asn Leu Lys Leu
    50                  55                  60
Lys Ser Thr Gln Met Ile Ile Val Thr Pro Ile Leu Arg Ser Glu Asp
65                  70                  75                  80
Gly Thr Ser Arg Val Glu Phe Pro Ser Val Val Ile Thr Gly Arg Asn
                85                  90                  95
Arg Thr Lys Ala Leu Lys Arg Glu Ile Ala Phe Ser Ser Ala Leu Pro
            100                 105                 110
Gln Ala Lys His Ala Ala Gln Tyr Ile Arg Arg His Asn Gly Lys Ser
        115                 120                 125
Glu Gln Phe Ala Phe Thr Gly Glu His Ala Tyr Ala Ser Trp Met Met
    130                 135                 140
Asp Ala Lys Phe Val Val Arg Glu Glu Val Arg Gly Cys Ala Lys Cys
145                 150                 155                 160
Pro Val Gly Leu Ser Ser Asn Ile Val Pro Phe Asp Pro Leu Phe Asn
                165                 170                 175
Pro Ala Glu Ala Pro Tyr Leu Leu Ala His Ile Thr Pro Ala Glu Glu
            180                 185                 190
Val Glu Lys Gln Arg Glu Ser Ser Phe Asp Ala Tyr Ile Asn Phe Lys
        195                 200                 205
Val Asn Lys Ala Asp Val Leu Pro Glu Tyr Arg Asn Asn Lys Ala Glu
    210                 215                 220
Leu Glu Lys Ile Lys Glu Phe Val Ser Thr Val Lys Ala Asn Pro Asn
225                 230                 235                 240
Tyr Ser Val Asn Lys Met Ile Ile Glu Gly Phe Ala Ser Pro Glu Ala
                245                 250                 255
Ser Ile Ala His Asn Lys Ala Leu Ser Glu Arg Arg Ala Lys Arg Leu
            260                 265                 270
Ala Glu Glu Leu Val Arg Lys Tyr Gly Lys Thr Leu Pro Asn Ile Thr
        275                 280                 285
Thr Glu Phe Gly Gly Glu Asp Trp Lys Gly Leu Lys Leu Ala Ile Glu
    290                 295                 300
Lys Ser Asp Ile Ala Asp Arg Asp Arg Val Leu Glu Ile Ile Asn Ser
305                 310                 315                 320
Asp Lys Tyr Ala Asp Asp Asp Ala Arg Glu Gln Ala Leu Lys Gln Leu
                325                 330                 335
Ser Ser Tyr Arg Tyr Ile Leu Asp Gln Ile Tyr Pro Asn Leu Arg Arg
            340                 345                 350
Asn Thr Ile Thr Met Gly Tyr Ile Val Arg Asp Tyr Thr Leu Glu Glu
        355                 360                 365
Ala Arg Glu Ile Ile Lys Thr Ala Pro Lys Glu Leu Ser Glu Ala Glu
    370                 375                 380
Met Tyr Arg Val Ala Met Ser Tyr Pro Glu Gly His Gln Glu Arg Leu
385                 390                 395                 400
Phe Ala Leu Asn Thr Thr Leu Lys Tyr Phe Pro Glu Ser Val Thr Gly
                405                 410                 415
Arg Ile Asn Leu Ala Val Ala Ala Phe Asn Gly Gly Asp Val Gln Gln
            420                 425                 430
Ala Ile Ala Leu Leu Ser Pro Ile Gln Thr Glu Lys Gly Val Ser Asn
        435                 440                 445
Ile Leu Gly Ala Ala Tyr Ala Arg Thr Gly Asp Phe Ala Arg Ala Glu
    450                 455                 460
Thr Phe Phe Arg Lys Ala Val Ala Glu Gly Asp Ala Asn Ala Gln Arg
465                 470                 475                 480
Asn Leu Asp Met Leu Leu Gly Lys Lys
                485

(2) INFORMATION FOR SEQ ID NO:292

(i) SEQUENCE CHARACTERISTICS:

(A) LEHGTH: 384 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc feature
(B) LOCATION 1...384

(xi) SEQUENCE DESCRIPTION: SEQ ID NO:292

Asp Met Ala Glu Lys Arg Asp Tyr Tyr Glu Val Leu Gly Val Ser Lys
1               5                   10                  15
Asn Ala Thr Asp Asp Glu Leu Lys Lys Ala Tyr Arg Lys Lys Ala Ile
            20                  25                  30
Gln Tyr His Pro Asp Lys Asn Pro Gly Asp Lys Glu Ala Glu Glu His
        35                  40                  45
Phe Lys Glu Val Ala Glu Ala Tyr Asp Val Leu Ser Asp Pro Gln Lys
    50                  55                  60
Arg Ser Gln Tyr Asp Gln Phe Gly His Ala Gly Leu Gly Gly Ala Ala
65                  70                  75                  80
Gly Gly Gly Phe Ser Gly Gly Gly Met Ser Met Glu Asp Ile Phe Ser
                85                  90                  95
Arg Phe Gly Asp Leu Phe Gly Gly Phe Gly Gly Phe Gly Gly Phe Ser
            100                 105                 110
Asp Met Gly Gly Gly Ser Arg Arg Arg Val Arg Arg Gly Ser Asp Leu
        115                 120                 125
Arg Val Arg Val Lys Leu Ser Leu Ala Asp Ile Ser Lys Gly Val Glu
    130                 135                 140
Lys Lys Val Lys Val Lys Lys Gln Val Val Cys Ser Lys Cys Arg Gly
145                 150                 155                 160
Asp Gly Thr Glu Glu Ala Asn Gly Lys Thr Thr Cys Gln Thr Cys His
                165                 170                 175
Gly Thr Gly Val Val Thr Arg Val Ser Asn Thr Phe Leu Gly Ala Met
            180                 185                 190
Gln Thr Gln Ser Thr Cys Pro Thr Cys His Gly Glu Gly Glu Ile Ile
        195                 200                 205
Thr Lys Pro Cys Ser Lys Cys Lys Gly Glu Gly Val Glu Ile Gly Glu
    210                 215                 220
Glu Val Ile Ser Phe His Ile Pro Ala Gly Val Ala Glu Gly Met Gln
225                 230                 235                 240
Met Ser Val Asn Gly Lys Gly Asn Ala Ala Pro Arg Gly Gly Val Asn
                245                 250                 255
Gly Asp Leu Ile Val Val Ile Ala Glu Glu Pro Asp Pro Asn Leu Ile
            260                 265                 270
Arg Asn Gly Asn Asp Leu Ile Tyr Asn Leu Leu Ile Ser Val Pro Leu
        275                 280                 285
Ala Ile Lys Gly Gly Ser Val Glu Val Pro Thr Ile Asp Gly Arg Ala
    290                 295                 300
Lys Ile Arg Ile Glu Ala Gly Thr Gln Pro Gly Lys Met Leu Arg Leu
305                 310                 315                 320
Arg Asn Lys Gly Leu Pro Ser Val Asn Gly Tyr Gly Met Gly Asp Gln
                325                 330                 335
Leu Val Asn Val Asn Val Tyr Ile Pro Glu Ser Ile Asp Ala Lys Asp
            340                 345                 350
Glu Gln Ala Ile Ala Ala Met Glu Asn Ser Asp Ser Phe Lys Pro Thr
        355                 360                 365
Asp Ala Ala Arg Lys Asp Ile Asp Lys Lys Tyr Arg Glu Met Leu Asp
    370                 375                 380

(2) INFORMATION FOR SEQ ID NO:293

(i) SEQUENCE CHARACTERISTICS:
(A) LENGTH: 309 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...309

(xi) SEQUENCE DESCRIPTION: SEQ ID NO:293

Arg Phe Asp Ser Thr Thr Asn Val Ser Gln Ile Asn Leu Arg Thr Glu
1               5                   10                  15
Met Lys Lys Leu Ile Leu Ala Thr Leu Gly Leu Met Ala Ile Ala Met
            20                  25                  30
Leu Ser Cys Ser Ser Asn Asn Lys Asp Leu Glu Asn Lys Gly Glu Ala
        35                  40                  45
Thr Leu Leu Val Thr Phe Gly Ser Ser Tyr Lys Ala Pro Arg Glu Thr
    50                  55                  60
Tyr Ala Lys Ile Glu Lys Thr Phe Ala Ala Ala Tyr Pro Asp Gln Arg
65                  70                  75                  80
Ile Ser Trp Thr Tyr Thr Ser Ser Ile Ile Arg Lys Lys Leu Ala Gln
                85                  90                  95
Gln Gly Ile Tyr Ile Asp Ala Pro Asp Glu Ala Leu Glu Lys Leu Ala
            100                 105                 110
Arg Leu Gly Tyr Lys Lys Ile Asn Val Gln Ser Leu His Val Ile Pro
        115                 120                 125
Gly Arg Glu Tyr Asp Glu Met Ile Asp Phe Val Asn Lys Phe Lys Ala
    130                 135                 140
Ala His Ser Asp Ile Thr Val Lys Val Gly Ala Pro Leu Phe Asp Thr
145                 150                 155                 160
Asp Glu Asp Met Arg Glu Val Ala Glu Ile Leu His Lys Arg Phe Gln
                165                 170                 175
Gln Thr Ile Glu Lys Gly Glu Ala Ile Val Phe Met Gly His Gly Thr
            180                 185                 190
Glu His Ala Ala Asn Asp Arg Tyr Ala Arg Ile Asn Lys Ile Met Lys
        195                 200                 205
Asn Tyr Ser Lys Phe Met Ile Val Gly Thr Val Glu Ser Asp Pro Ser
    210                 215                 220
Ile Asn Asp Val Ile Ala Glu Leu Lys Glu Thr Gly Ala Thr Ala Val
225                 230                 235                 240
Thr Met Met Pro Leu Met Ser Val Ala Gly Asp His Ala Thr Asn Asp
                245                 250                 255
Met Ala Gly Asp Glu Asp Asp Ser Trp Lys Thr Leu Leu Thr Asn Ala
            260                 265                 270
Gly Tyr Thr Val Ser Ile Asp Lys Leu Asp Asn Gly Asn Phe Ser Ala
        275                 280                 285
Leu Gly Asp Ile Glu Glu Ile Arg Asn Ile Trp Leu Lys His Met Lys
    290                 295                 300
Ala Thr Ser Ala Arg
305

(2) INFORMATION FOR SEQ ID NO:294

(i) SEQUENCE CHARACTERISTICS:
(A) LENGTH: 491 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...491

(xi) SEQUENCE DESCRIPTION: SEQ ID NO:294

Arg Arg Asp Ala Arg Arg Gln Leu Val Arg Arg Arg Arg Lys Glu Leu
1               5                   10                  15
Ser Gly Thr Ala Asn Thr Arg Cys Arg Arg Arg Val Phe Pro Glu Arg

                20                  25                  30
Arg Ser Ala Ile Arg Trp Cys Phe Pro Arg Leu Arg Asp Gly Leu Gln
            35                  40                  45
Pro Ile Arg Phe Gly Ser Leu Leu Ala Asn Pro Gln Gln Leu Ser Arg
        50                  55                  60
Gly Val Arg Leu Arg Ser Phe Glu Ser Arg Arg Gln Pro Ile Cys Arg
65                  70                  75                  80
Phe Gln Gly Ile Gln Gly Lys Arg Arg Asp Ile Leu Gln Pro Glu Gly
                85                  90                  95
Asn Ser Leu Leu Arg Thr His Ala His Ala Ser Arg Leu Gly Ala Gly
            100                 105                 110
Arg Ser Tyr Arg Tyr Leu Ala Pro Arg Thr Thr Ser Arg Pro Pro Thr
        115                 120                 125
Ala Phe Leu Leu Phe Ala Pro Met Thr Ser Val Ser His Leu Arg Thr
    130                 135                 140
Ile Ser Val Ala Gly Ile Leu Ala Ala Leu Gly Gly Ala Val Leu Ile
145                 150                 155                 160
Leu Phe Gly Val Asn Leu Phe Leu Gly Ser Val Ala Ile Pro Met Ser
                165                 170                 175
Glu Ile Phe Arg His Leu Phe Ser Asp Arg Pro Glu Gly Gly Glu Ala
            180                 185                 190
Leu Val His Tyr Asn Ile Leu Trp Lys Ser Arg Leu Pro Glu Ala Leu
        195                 200                 205
Thr Ala Ala Phe Ala Gly Ala Gly Leu Ser Val Ser Gly Leu Gln Met
    210                 215                 220
Gln Thr Val Phe Arg Asn Pro Leu Ala Gly Pro Ser Val Leu Gly Ile
225                 230                 235                 240
Ser Ser Gly Ala Ser Leu Gly Val Ala Leu Val Val Leu Leu Ser Gly
                245                 250                 255
Ser Leu Gly Gly Val Ala Leu Ser Ser Leu Gly Tyr Met Gly Glu Val
            260                 265                 270
Ala Met Asn Ile Ala Ala Ala Val Gly Ser Leu Ala Val Met Gly Leu
        275                 280                 285
Ile Val Phe Val Ser Thr Lys Val Arg Ser His Val Thr Leu Leu Ile
    290                 295                 300
Ile Gly Val Met Ile Gly Tyr Val Ala Thr Ala Val Ile Gly Val Phe
305                 310                 315                 320
Lys Phe Phe Ser Ile Glu Glu Asp Ile Arg Ala Tyr Val Ile Trp Gly
                325                 330                 335
Leu Gly Ser Phe Ser Arg Ala Thr Asp Ser Gln Leu Ser Phe Phe Ala
            340                 345                 350
Ile Leu Met Leu Ile Phe Ile Pro Ala Gly Met Leu Leu Val Lys Gln
        355                 360                 365
Leu Asn Leu Leu Leu Leu Gly Glu Ser Tyr Ala Arg Asn Leu Gly Leu
    370                 375                 380
Asn Thr Arg Arg Ala Arg Leu Leu Val Ile Ser Ser Ala Gly Leu Leu
385                 390                 395                 400
Ile Ala Thr Val Thr Ala Tyr Cys Gly Pro Ile Gly Phe Leu Gly Met
                405                 410                 415
Ala Val Pro His Leu Ala Arg Val Ile Phe His Thr Ser Asp His Arg
            420                 425                 430
Ile Leu Met Pro Ala Thr Cys Leu Ile Gly Ser Ala Leu Ala Leu Phe
        435                 440                 445
Cys Asn Ile Ile Ala Arg Met Pro Gly Phe Glu Gly Ala Leu Pro Val
    450                 455                 460
Asn Ser Val Thr Ala Leu Val Gly Ala Pro Ile Ile Val Thr Val Leu
465                 470                 475                 480
Phe Arg Arg Arg Arg Phe Lys Glu Glu Thr Asp
                485                 490

(2) INFORMATION FOR SEQ ID NO:295

    (i) SEQUENCE CHARACTERISTICS:
        (A) LENGTH: 763 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:

(A) NAME/KEY: misc_feature
(B) LOCATION 1...763

(xi) SEQUENCE DESCRIPTION: SEQ ID NO:295

His Phe Leu Val Thr Ile Met Arg Thr Lys Thr Ile Phe Phe Ala Ile
1               5                   10                  15
Ile Ser Phe Ile Ala Leu Leu Ser Ser Ser Leu Ser Ala Gln Ser Lys
            20                  25                  30
Ala Val Leu Thr Gly Ser Val Ser Asp Ala Glu Thr Gly Glu Pro Leu
        35                  40                  45
Ala Gly Ala Arg Ile Glu Val Lys His Thr Asn Ile Val Ala Gly Ala
    50                  55                  60
Asp Ala Gly Gly His Phe Glu Ile Lys Asn Leu Pro Ala Gly Gln His
65                  70                  75                  80
Thr Ile Ile Cys Ser Leu Gly Gly Tyr Gly Gln Lys Glu Glu Val Val
                85                  90                  95
Ala Ile Glu Ala Gly Gln Thr Lys Thr Ile Ser Phe Ala Leu Arg Leu
            100                 105                 110
Arg Thr Asn Asn Leu Glu Glu Val Val Val Thr Gly Thr Gly Thr Arg
        115                 120                 125
Tyr Arg Leu Val Asp Ala Pro Val Ala Thr Glu Val Leu Thr Ala Lys
    130                 135                 140
Asp Ile Ala Ser Phe Ser Ala Pro Thr Ser Glu Ala Leu Leu Gln Gly
145                 150                 155                 160
Leu Ser Pro Ser Phe Asp Phe Gly Pro Asn Leu Met Gly Ser Phe Met
                165                 170                 175
Gln Leu Asn Gly Leu Ser Ser Lys Tyr Ile Leu Ile Leu Ile Asp Gly
            180                 185                 190
Lys Arg Val Tyr Gly Asp Val Gly Gly Gln Ala Asp Leu Ser Arg Ile
        195                 200                 205
Ser Pro Asp Gln Ile Glu Arg Ile Glu Leu Val Lys Gly Ala Ser Ser
    210                 215                 220
Ser Leu Tyr Gly Ser Asp Ala Ile Ala Gly Val Ile Asn Val Ile Thr
225                 230                 235                 240
Lys Lys Asn Thr Asn Arg Leu Ser Ala Tyr Thr Ser His Arg Ile Ser
                245                 250                 255
Lys Tyr Asn Asp Arg Gln Thr Asn Thr Ser Leu Asp Ile Asn Ile Gly
            260                 265                 270
Lys Phe Ser Ser Asn Thr Asn Tyr Phe Phe Tyr His Thr Asp Gly Trp
        275                 280                 285
Gln Asn Ser Pro Phe Glu Ile Lys Lys Lys Lys Gly Ser Gly Glu Pro
    290                 295                 300
Val Leu Glu Glu Thr Tyr Lys Lys Thr Phe Arg Ala Gln Glu Asn Gln
305                 310                 315                 320
Gly Val Ser Gln Ser Leu Ser Tyr Tyr Ala Thr Asn Asn Leu Ser Phe
                325                 330                 335
Ser Gly Asn Val Gln Tyr Asn Lys Arg Gln Ile Phe Thr Pro Thr Phe
            340                 345                 350
Ser Glu Lys Lys Ala Tyr Asp Met Asp Tyr Arg Ala Leu Thr Ala Ser
        355                 360                 365
Leu Gly Thr Asn Tyr Leu Phe Pro Asn Gly Leu His Thr Leu Ser Phe
    370                 375                 380
Asp Ala Val Tyr Asp Arg Phe Arg Phe Gly Tyr Leu Tyr His Asp Lys
385                 390                 395                 400
Asp Ser Ser Glu Ser Leu Ile Asn Asn Gln Gly Gln Thr Glu Gln Pro
                405                 410                 415
Thr Phe Phe Pro Gly Gln Leu Arg Asn Lys Asn Asp Gln Ile Arg Tyr
            420                 425                 430
Thr Ala Glu Ala Arg Gly Val Phe Thr Leu Pro Tyr Ala Gln Lys Leu
        435                 440                 445
Thr Gly Gly Leu Glu Tyr Phe Arg Glu Glu Leu Ile Ser Pro Tyr Asn
    450                 455                 460
Leu Ile Thr Asp Lys Ala Asp Ala Ser Thr Leu Ser Ala Tyr Val Gln
465                 470                 475                 480
Asp Glu Trp Lys Pro Leu Asp Trp Phe Asn Met Thr Ala Gly Phe Arg
                485                 490                 495
Leu Val His His Gln Glu Phe Gly Thr Arg Met Thr Pro Lys Val Ser
            500                 505                 510
Ile Leu Ala Lys Tyr Gly Pro Leu Asn Phe Arg Ala Thr Tyr Ala Asn
        515                 520                 525
Gly Tyr Lys Thr Pro Thr Leu Lys Glu Leu Phe Ala Arg Asn Glu Leu
    530                 535                 540
Thr Thr Met Gly Ser His Asn Leu Tyr Leu Gly Asn Ala Asp Leu Lys
545                 550                 555                 560

Pro Gln Met Ser Asp Tyr Tyr Ala Leu Gly Leu Glu Tyr Asn Gln Gly
                565                 570                 575
Pro Ile Ser Phe Ser Ala Thr Val Tyr Asp Asn Glu Leu Arg Asn Leu
            580                 585                 590
Ile Ser Phe Met Asp Ile Pro Thr Ser Pro Glu His Glu Ala Gln Gly
        595                 600                 605
Ile Lys Lys Thr Lys Gln Tyr Ala Asn Ile Gly Lys Ala Arg Ser Arg
    610                 615                 620
Gly Leu Asp Val Leu Cys Asp Ala Ser Ile Gly Trp Gly Ile Lys Leu
625                 630                 635                 640
Gly Ala Gly Tyr Ser Leu Val Glu Ala Lys Asn Leu Gln Thr Asp Glu
                645                 650                 655
Trp Leu Glu Gly Ala Ala Arg His Arg Ala Asn Val His Ala Asp Trp
            660                 665                 670
Val His Tyr Trp Gly Gln Tyr Arg Leu Gly Val Ser Leu Phe Gly Arg
        675                 680                 685
Ile Gln Ser Glu Arg Tyr Tyr Lys Asp Gly Asn Ala Pro Asp Tyr Thr
    690                 695                 700
Leu Trp Arg Leu Ala Thr Ser His Arg Phe Ala His Phe Arg His Ile
705                 710                 715                 720
Ile Leu Asp Gly Thr Leu Gly Ile Asp Asn Leu Phe Asp Tyr Val Asp
                725                 730                 735
Asp Arg Pro Met Gly Val Asn Tyr Ala Thr Val Thr Pro Gly Arg Thr
            740                 745                 750
Phe Phe Ala Gln Ile Ala Ile Arg Phe Asn Asn
        755                 760

(2) INFORMATION FOR SEQ ID NO:296

(i) SEQUENCE CHARACTERISTICS:
(A) LENGTH: 365 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...365

(xi) SEQUENCE DESCRIPTION: SEQ ID NO:296

Cys Cys Ala Arg Leu Arg Asp Thr Ser Pro Arg Lys Lys Ser Phe Arg
1               5                   10                  15
Asn Asp Thr Val Phe Pro Tyr Phe Val Ile His Leu Ile Lys His Ile
            20                  25                  30
Ile Ile Met Thr Asp Asn Lys Gln Arg Asn Ile Val Phe Pro Ala Phe
        35                  40                  45
Leu Leu Leu Leu Gly Val Ile Ala Val Val Thr Ile Val Gly Phe Phe
    50                  55                  60
Met Leu Arg Pro Ala Glu Glu Ile Ile Gln Gly Gln Ile Glu Val Thr
65                  70                  75                  80
Glu Tyr Arg Val Ser Ser Lys Val Pro Gly Arg Ile Lys Glu Leu Arg
                85                  90                  95
Val Ser Glu Gly Gln Gln Val Gln Ala Gly Asp Thr Leu Ala Val Ile
            100                 105                 110
Glu Ala Pro Asp Val Ala Ala Lys Met Glu Gln Ala Lys Ala Ala Glu
        115                 120                 125
Ala Ala Ala Gln Ala Gln Asn Ala Lys Ala Leu Lys Gly Ala Arg Ser
    130                 135                 140
Glu Gln Ile Gln Ala Ala Tyr Glu Met Trp Gln Lys Ala Gln Ala Gly
145                 150                 155                 160
Val Ala Ile Ala Thr Lys Thr His Gln Arg Val Gln Asn Leu Tyr Asp
                165                 170                 175
Gln Gly Val Val Pro Ala Gln Lys Leu Asp Glu Ala Thr Ala Gln Arg
            180                 185                 190
Asp Ala Ala Ile Ala Thr Gln Lys Ala Ala Glu Ala Gln Tyr Asn Met
        195                 200                 205
Ala Arg Asn Gly Ala Glu Arg Glu Asp Lys Leu Ala Ala Ser Ala Leu
    210                 215                 220

Val Asp Arg Ala Arg Gly Ala Val Ala Glu Val Glu Ser Tyr Ile Asn
225                 230                 235                 240
Glu Thr Tyr Leu Ile Ala Pro Arg Ala Gly Glu Val Ser Glu Ile Phe
                245                 250                 255
Pro Lys Ala Gly Glu Leu Val Gly Thr Gly Ala Pro Ile Met Asn Ile
            260                 265                 270
Ala Glu Met Gly Asp Met Trp Ala Ser Phe Ala Val Arg Glu Asp Phe
        275                 280                 285
Leu Ser Ser Met Thr Met Gly Ala Val Leu Glu Thr Val Val Pro Ala
    290                 295                 300
Leu Asn Glu Glu Lys Val Arg Phe Lys Ile Thr Phe Ile Lys Asn Met
305                 310                 315                 320
Gly Thr Tyr Ala Ala Trp Lys Ala Thr Lys Thr Thr Gly Gln Tyr Asp
                325                 330                 335
Leu Lys Thr Phe Glu Val Lys Ala Thr Leu Ala Asp Lys Asp Lys Ala
            340                 345                 350
Gln Lys Leu Arg Pro Gly Met Ser Val Ile Ile Arg Lys
        355                 360                 365

(2) INFORMATION FOR SEQ ID NO:297

(i) SEQUENCE CHARACTERISTICS:
(A) LENGTH: 320 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...320

(xi) SEQUENCE DESCRIPTION: SEQ ID NO:297

Pro Gln Ser Ser Pro Asp Arg Arg Ser Phe Gln Asn Val Met Asn Lys
1               5                   10                  15
Tyr His Ser Gln Ser Val Leu Glu Val Gly Lys Ile Gly Ile Val Ile
            20                  25                  30
Ile Phe Ala Pro Ile Val Arg Asn Val His Gln Gln Pro Pro Phe Leu
        35                  40                  45
Ser His Lys Ser Ile Met Arg Ile Val Ser Asn Phe Leu Phe Val Ser
    50                  55                  60
Phe Ser Val Leu Leu Phe Ala Ser Cys Arg Ser Gln Arg Glu Lys Val
65                  70                  75                  80
Val Tyr Leu Gln Asp Ile Gln Thr Phe Asn Arg Glu Ile Ile Ala Lys
                85                  90                  95
Pro Tyr Asp Val Lys Ile Glu Lys Asp Asp Val Leu Asn Ile Leu Val
            100                 105                 110
Ser Ser Arg Asp Pro Glu Leu Ser Thr Pro Tyr Asn Gln Val Leu Thr
        115                 120                 125
Thr Arg Ala Leu Ala Arg Asn Gly Tyr Gly Thr Asn Ser Asn Glu Gly
    130                 135                 140
Phe Leu Val Asp Ser Lys Gly Tyr Ile Asn Tyr Pro Ile Leu Gly Gln
145                 150                 155                 160
Ile Tyr Val Glu Gly Leu Thr Arg Thr Glu Leu Glu Lys Glu Ile Gln
                165                 170                 175
Lys Arg Ile Ile Ser Ser Gly Phe Ile Lys Asp Pro Thr Val Thr Val
            180                 185                 190
Gln Leu Gln Asn Phe Lys Val Ser Val Leu Gly Glu Val Asn His Pro
        195                 200                 205
Gly Ser Met Ser Val Lys Gly Glu Arg Ile Thr Leu Leu Glu Ala Ile
    210                 215                 220
Gly Met Ala Gly Asp Leu Thr Ile Tyr Gly Arg Arg Asp Arg Val Phe
225                 230                 235                 240
Val Ile Arg Glu Thr Asp Gly His Arg Glu Val Phe Gln Thr Asp Leu
                245                 250                 255
Arg Lys Ala Asp Leu Leu Ala Ser Pro Val Tyr Tyr Leu His Gln Asn
            260                 265                 270
Asp Val Ile Tyr Val Glu Pro Asn Asp Lys Lys Thr Gln Met Ser Glu
        275                 280                 285

Ile Asn Gln Asn Asn Asn Val Asn Val Trp Leu Ser Val Thr Ser Thr
    290                 295                 300
Leu Val Ser Ile Ser Thr Leu Thr Ile Thr Ile Ile Asp Lys Thr Lys
305                 310                 315                 320

(2) INFORMATION FOR SEQ ID NO:298

     (i) SEQUENCE CHARACTERISTICS:
          (A) LENGTH: 582 amino acids
          (B) TYPE: amino acid
          (D) TOPOLOGY: linear

    (ii) MOLECULE TYPE: protein

   (iii) HYPOTHETICAL: YES

    (vi) ORIGINAL SOURCE:
          (A) ORGANISM: Porphyromonas gingivalis

    (ix) FEATURE:
          (A) NAME/KEY: misc_feature
          (B) LOCATION 1...582

    (xi) SEQUENCE DESCRIPTION: SEQ ID NO:298

Ser Lys Ile Val Leu Arg Lys Phe Cys Thr Leu Ala Arg Met Lys Lys
1               5                   10                  15
Thr Asn Leu Phe Leu Ser Leu Leu Val Ile Phe Ile Thr Gly Ser Phe
            20                  25                  30
Met Thr Ala Cys Ala Gln Lys Ser Lys Thr Asn Lys Leu Thr Glu Glu
        35                  40                  45
Asp Arg Ser Arg Asn Glu Tyr Val Gln Ser Met Asp Val Leu Ser Asn
    50                  55                  60
Ile Ile Gly Asn Val Arg Leu Tyr Phe Val Asp Thr Ile Ser Ile Lys
65                  70                  75                  80
His Met Thr Arg Arg Gly Ile Asp Ala Met Leu Gly Gly Leu Asp Pro
                85                  90                  95
Tyr Thr Glu Tyr Ile Pro Tyr Glu Glu Met Asp Glu Leu Lys Leu Met
            100                 105                 110
Thr Thr Gly Glu Tyr Ala Gly Val Gly Ala Ile Ile Ser Gln Arg Pro
        115                 120                 125
Asp Ser Ala Val Ile Ile Gln Arg Pro Met Glu Gly Met Pro Ala Asp
    130                 135                 140
Glu Ala Gly Leu Ile Ala Gly Asp Arg Ile Leu Thr Ile Asp Gly Lys
145                 150                 155                 160
Asp Phe Arg Lys Ser Thr Thr Pro Lys Val Ser Gln Ala Leu Lys Gly
                165                 170                 175
Ile Ala Gly Thr Val Ala Lys Val Thr Val Met Arg Tyr Gly Glu Thr
            180                 185                 190
Lys Pro Arg Thr Phe Ser Val Lys Arg Gln Lys Val Ile Met Asn Ser
        195                 200                 205
Val Thr Tyr Ser Gly Met Leu Asp Gly Ser Ile Gly Tyr Ile Arg Leu
    210                 215                 220
Asn Asn Phe Thr Asp Lys Ser Ala Glu Glu Val Arg Thr Ala Leu Leu
225                 230                 235                 240
Asp Leu Arg Asp Lys Gln Gly Ala Lys Gly Leu Ile Leu Asp Leu Arg
                245                 250                 255
Gly Asn Gly Gly Gly Leu Met Gln Ala Ala Ile Glu Ile Val Asn Leu
            260                 265                 270
Phe Val Pro Lys Gly Lys Glu Val Val Thr Thr Lys Gly Arg Ile Ala
        275                 280                 285
Glu Ser Ala Ser Val Phe Arg Thr Leu Thr Glu Pro Ile Asp Thr Lys
    290                 295                 300
Leu Pro Ile Val Val Leu Ile Asp Gly Gln Ser Ala Ser Ser Ser Glu
305                 310                 315                 320
Ile Val Ala Gly Ala Leu Gln Asp Met Asp Arg Ala Val Leu Met Gly
                325                 330                 335
Gln Lys Ser Tyr Gly Lys Gly Leu Val Gln Thr Thr Arg Gln Leu Pro
            340                 345                 350
Tyr Asn Gly Val Ile Lys Leu Thr Thr Ala Lys Tyr Tyr Ile Pro Ser
        355                 360                 365
Gly Arg Cys Ile Gln Arg Leu Asp Tyr Ser Arg Thr Asn Arg Thr Gly
    370                 375                 380
Met Ala Thr Ala Ile Pro Asp Ser Leu His Lys Ile Phe Tyr Thr Ala
385                 390                 395                 400

Ala Gly Arg Arg Val Glu Asp Ala Gly Gly Ile Leu Pro Asp Ile Glu
                405                 410                 415
Val Lys Gln Asp Thr Ala Ala Thr Leu Leu Tyr Tyr Met Ala Ile Asn
            420                 425                 430
Asn Asp Val Phe Asp Phe Val Thr Gly Tyr Val Leu Lys His Lys Thr
        435                 440                 445
Ile Ala Lys Pro Glu Asp Phe Ser Ile Thr Asn Glu Asp Tyr Ala Ala
    450                 455                 460
Phe Cys Lys Met Met Glu Glu Lys Lys Phe Asp Tyr Asp Arg Gln Ser
465                 470                 475                 480
Gly Lys Met Leu Asp Lys Leu Glu Glu Leu Ala Lys Ile Glu Gly Tyr
                485                 490                 495
Leu Pro Glu Ala Asn Ser Glu Leu Lys Ala Leu Arg Glu Lys Leu Lys
            500                 505                 510
Pro Asn Leu Ser Arg Asp Leu Leu Arg Phe Lys Lys Glu Ile Thr Asn
        515                 520                 525
Tyr Leu Asn Asn Glu Ile Val Thr Arg Tyr Tyr Tyr Glu Arg Gly Ser
    530                 535                 540
Ile Arg Gln Ser Leu Pro Glu Asp Lys Val Val Lys Glu Ala Ile Lys
545                 550                 555                 560
Leu Leu Lys Asp His Pro Glu Gln Ile Arg Gln Ile Leu Ala Ala Pro
                565                 570                 575
Lys Ala Glu Asn Lys Gly
                580

(2) INFORMATION FOR SEQ ID NO:299

     (i) SEQUENCE CHARACTERISTICS:
          (A) LENGTH: 985 amino acids
          (B) TYPE: amino acid
          (D) TOPOLOGY: linear

    (ii) MOLECULE TYPE: protein

   (iii) HYPOTHETICAL: YES

    (vi) ORIGINAL SOURCE:
          (A) ORGANISM: Porphyromonas gingivalis

    (ix) FEATURE:
          (A) NAME/KEY: misc_feature
          (B) LOCATION 1...985

    (xi) SEQUENCE DESCRIPTION: SEQ ID NO:299

Val Thr Asp Lys Met Gln Asn Lys Gly Phe Val Ile Val Ile Thr Ser
1               5                   10                  15
Ala Leu Ala Ile Ile Cys Ala Phe Tyr Leu Ser Phe Ser Phe Val Thr
            20                  25                  30
Asn Arg Tyr Glu Lys Lys Ala Lys Ala Met Gly Asp Val Ala Gly Met
        35                  40                  45
Ala Tyr Leu Asp Ser Met Ser Asn Glu Lys Val Trp Phe Gly Tyr Thr
    50                  55                  60
Leu Lys Glu Ala Gln Ala Gln Gln Ile Gly Leu Gly Leu Asp Leu Lys
65                  70                  75                  80
Gly Gly Met Asn Val Ile Leu Lys Leu Asn Ala Ser Asp Leu Leu Arg
                85                  90                  95
Asn Leu Ser Asn Lys Ser Leu Asp Pro Asn Phe Asn Lys Ala Leu Glu
            100                 105                 110
Asn Ala Ala Lys Ser Thr Glu Gln Ser Asp Phe Ile Asp Ile Phe Val
        115                 120                 125
Lys Glu Tyr Arg Lys Leu Asp Pro Asn Gly Arg Leu Ala Val Ile Phe
    130                 135                 140
Gly Ser Gly Asp Leu Arg Asp Gln Ile Thr Ala Lys Ser Thr Asp Ala
145                 150                 155                 160
Asp Val Val Arg Leu Leu Lys Glu Lys Tyr Asn Ser Ala Val Glu Ala
                165                 170                 175
Ser Phe Asn Val Leu Arg Ala Arg Ile Asp Ala Phe Gly Val Val Ala
            180                 185                 190
Pro Asn Leu Gln Arg Leu Glu Gly Gln Gly Arg Ile Leu Val Glu Leu
        195                 200                 205
Pro Gly Val Lys Asp Pro Glu Arg Val Arg Thr Leu Leu Gln Arg Ser
    210                 215                 220
Ala Asn Leu Gln Phe Trp Arg Thr Tyr Lys Phe Glu Glu Val Ser Gly
225                 230                 235                 240

Asp Leu Ile Ala Ala Asn Asp Arg Leu Ser Glu Leu Ala Met Asn Asn
                245                 250                 255
Thr Asp Ala Thr Pro Glu Thr Glu Pro Ala Thr Thr Asp Ser Val Ala
            260                 265                 270
Ala Thr Ala Asp Ser Ala Ala Val Gln Ala Val Ala Asp Ser Ala Thr
        275                 280                 285
Val Ala Gln Lys Glu Ala Lys Asp Ala Thr Arg Lys Asp Ala Leu Phe
    290                 295                 300
Ser Leu Leu Thr Pro Val Asn Arg Gly Gly Ala Val Val Gly Val Ala
305                 310                 315                 320
Arg Arg Ala Asn Met Ala Gln Ile Ser Glu Met Leu Gln Gln Ala His
                325                 330                 335
Asp Leu Lys Val Thr Arg Glu Asp Val Leu Phe Leu Trp Gly Ala Lys
            340                 345                 350
Ala Ile Glu Asp Pro Glu Thr Lys Lys Glu Thr Asp Leu Tyr Glu Leu
        355                 360                 365
Tyr Ala Ile Arg Thr Asn Arg Thr Gly Asp Pro Asp Leu Gly Gly Asp
    370                 375                 380
Val Val Thr Ser Ala Lys Ser Asp Ile Gln Asn Asp Phe Gly Arg Ser
385                 390                 395                 400
Glu Pro Ile Val Ser Met Thr Met Asn Glu Glu Gly Ala Arg Lys Trp
                405                 410                 415
Ala Arg Ile Thr Lys Asp Asn Val Gly Arg Ala Ile Ala Ile Val Leu
            420                 425                 430
Asp Gly Val Val Tyr Ser Ala Pro Asn Val Asn Asp Glu Ile Thr Gly
        435                 440                 445
Gly Arg Ser Gln Ile Ser Gly His Phe Thr Val Glu Glu Ala Gly Asp
    450                 455                 460
Leu Ala Asn Val Leu Asn Ser Gly Lys Met Asp Ala Thr Val Ser Ile
465                 470                 475                 480
Glu Gln Glu Asn Val Ile Gly Pro Thr Leu Gly Ala Glu Ser Ile Lys
                485                 490                 495
Ala Gly Phe Leu Ser Phe Leu Leu Ala Leu Val Ile Leu Met Cys Tyr
            500                 505                 510
Met Cys Leu Ala Tyr Gly Phe Leu Pro Gly Leu Ile Ala Asn Gly Ala
        515                 520                 525
Leu Ile Val Asn Ser Phe Phe Thr Leu Gly Val Leu Ala Ser Phe His
    530                 535                 540
Ala Val Leu Thr Leu Ser Gly Ile Ala Gly Leu Val Leu Thr Leu Gly
545                 550                 555                 560
Met Ala Val Asp Ala Asn Val Leu Ile Phe Glu Arg Ile Lys Glu Glu
                565                 570                 575
Leu Arg Ala Gly Lys Thr Pro Ile Arg Ala Val Thr Asp Gly Tyr Gly
            580                 585                 590
Asn Ala Phe Ser Ala Ile Phe Asp Ser Asn Val Thr Thr Ile Ile Thr
        595                 600                 605
Gly Ile Ile Leu Phe Leu Tyr Gly Thr Gly Pro Ile Arg Gly Phe Ala
    610                 615                 620
Thr Thr Leu Ile Ile Gly Leu Ile Ala Ser Phe Ile Thr Ala Val Phe
625                 630                 635                 640
Leu Thr Arg Ile Val Phe Glu Lys Leu Ala Lys Lys Gly Arg Leu Asp
                645                 650                 655
Lys Ile Thr Phe Thr Thr Ser Ile Thr Arg Asn Leu Leu Val Asn Pro
            660                 665                 670
Ser Tyr Asn Ile Leu Gly Lys Arg Lys Thr Gly Phe Ile Ile Pro Val
        675                 680                 685
Ile Ile Ile Val Leu Gly Leu Ile Ala Ser Phe Thr Ile Gly Leu Asn
    690                 695                 700
Arg Gly Ile Glu Phe Ser Gly Gly Arg Asn Tyr Val Val Lys Phe Asp
705                 710                 715                 720
Gln Pro Val Ser Ser Glu Ala Val Arg Ser Ala Leu Ser Ser Pro Leu
                725                 730                 735
Gln Glu Lys Val Leu Val Thr Ser Ile Gly Thr Glu Gly Thr Glu Val
            740                 745                 750
Arg Ile Ser Thr Asn Tyr Lys Ile Gln Glu Glu Ser Glu Glu Thr Glu
        755                 760                 765
Ala Glu Ile Thr Asp Lys Leu Tyr Gln Ser Leu Lys Gly Phe Tyr Thr
    770                 775                 780
Gln Gln Pro Thr Ala Asp Gln Phe Leu Asp Asn Ile Ile Ser Ser Gln
785                 790                 795                 800
Lys Val Ser Pro Ser Met Ser Ser Asp Ile Thr Arg Gly Ala Ile Trp
                005                 810                 815
Ala Val Leu Leu Ser Met Ile Phe Met Ala Ile Tyr Ile Leu Ile Arg
            820                 825                 830
Phe Arg Asp Ile Ser Phe Ser Ala Gly Val Phe Val Ser Val Ala Ala

835                     840                     845
Thr Thr Phe Cys Ile Ile Ala Leu Tyr Ala Leu Leu Trp Lys Ile Leu
    850                     855                     860
Pro Phe Thr Met Glu Ile Asp Gln Asn Phe Ile Ala Ala Ile Leu Ala
865                     870                     875                     880
Ile Ile Gly Tyr Ser Leu Asn Asp Thr Val Val Val Phe Asp Arg Ile
                885                     890                     895
Arg Glu Thr Met Lys Leu Tyr Pro Asn Arg Asp Arg Tyr Gln Val Ile
            900                     905                     910
Asn Asp Ala Leu Asn Ser Thr Leu Gly Arg Thr Leu Asn Thr Ser Leu
        915                     920                     925
Thr Thr Phe Ile Val Met Leu Val Ile Phe Ile Phe Gly Gly Ala Thr
    930                     935                     940
Met Arg Ser Phe Thr Phe Ser Ile Leu Leu Gly Ile Val Ile Gly Thr
945                     950                     955                     960
Tyr Ser Thr Leu Phe Val Ala Thr Pro Leu Ala Tyr Glu Ile Gln Lys
                965                     970                     975
Arg Lys Leu Asn Lys Ala Ala Lys Lys
            980                     985

(2) INFORMATION FOR SEQ ID NO:300

    (i) SEQUENCE CHARACTERISTICS:
        (A) LENGTH: 1046 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...1046

    (xi) SEQUENCE DESCRIPTION: SEQ ID NO:300

Asp Phe Pro Trp Val Arg Val Lys Pro Glu Lys Lys Arg Lys Gln His
1               5                   10                  15
Asn Ser Asn Asn Phe Lys Phe Asn Ala Lys Glu Lys Ser Met Lys Arg
            20                  25                  30
Met Thr Leu Phe Phe Leu Cys Leu Leu Thr Ser Ile Gly Trp Ala Met
        35                  40                  45
Ala Gln Asn Arg Thr Val Lys Gly Thr Val Ile Ser Ser Glu Asp Asn
    50                  55                  60
Glu Pro Leu Ile Gly Ala Asn Val Val Val Val Gly Asn Thr Thr Ile
65                  70                  75                  80
Gly Ala Ala Thr Asp Leu Asp Gly Asn Phe Thr Leu Ser Val Pro Ala
                85                  90                  95
Asn Ala Lys Met Leu Arg Val Ser Tyr Ser Gly Met Thr Thr Lys Glu
            100                 105                 110
Val Ala Ile Ala Asn Val Met Lys Ile Val Leu Asp Pro Asp Ser Lys
        115                 120                 125
Val Leu Glu Gln Val Val Val Leu Gly Tyr Gly Thr Gly Gln Lys Leu
    130                 135                 140
Ser Thr Val Ser Gly Ser Val Ala Lys Val Ser Ser Glu Lys Leu Ala
145                 150                 155                 160
Glu Lys Pro Val Ala Asn Ile Met Asp Ala Leu Gln Gly Gln Val Ala
                165                 170                 175
Gly Met Gln Val Met Thr Thr Ser Gly Asp Pro Thr Ala Val Ala Ser
            180                 185                 190
Val Glu Ile His Gly Thr Gly Ser Leu Gly Ala Ser Ser Ala Pro Leu
        195                 200                 205
Tyr Ile Val Asp Gly Met Gln Thr Ser Leu Asp Val Val Ala Thr Met
    210                 215                 220
Asn Pro Asn Asp Phe Glu Ser Met Ser Val Leu Lys Asp Ala Ser Ala
225                 230                 235                 240
Thr Ser Ile Tyr Gly Ala Arg Ala Ala Asn Gly Val Val Phe Ile Gln
                245                 250                 255
Thr Lys Lys Gly Lys Met Ser Glu Arg Gly Arg Ile Thr Phe Asn Ala
            260                 265                 270
Ser Tyr Gly Ile Ser Gln Ile Leu Asn Thr Lys Pro Leu Asp Asn Met

        275                 280                 285
Met Thr Gly Asp Glu Leu Leu Asp Phe Gln Val Lys Ala Gly Phe Trp
    290                 295                 300
Gly Asn Asn Gln Thr Val Gln Lys Val Lys Asp Met Ile Leu Ala Gly
305                 310                 315                 320
Ala Glu Asp Leu Tyr Gly Asn Tyr Asp Ser Leu Lys Asp Glu Tyr Gly
                325                 330                 335
Lys Thr Leu Phe Pro Val Asp Phe Asn His Asp Ala Asp Trp Leu Lys
            340                 345                 350
Ala Leu Phe Lys Thr Ala Pro Thr Ser Gln Gly Asp Ile Ser Phe Ser
        355                 360                 365
Gly Gly Ser Gln Gly Thr Ser Tyr Tyr Ala Ser Ile Gly Tyr Phe Asp
    370                 375                 380
Gln Glu Gly Met Ala Arg Glu Pro Ala Asn Phe Lys Arg Tyr Ser Gly
385                 390                 395                 400
Arg Leu Asn Phe Glu Ser Arg Ile Asn Glu Trp Leu Lys Val Gly Ala
                405                 410                 415
Asn Leu Ser Gly Ala Ile Ala Asn Arg Arg Ser Ala Asp Tyr Phe Gly
            420                 425                 430
Lys Tyr Tyr Met Gly Ser Gly Thr Phe Gly Val Leu Thr Met Pro Arg
        435                 440                 445
Tyr Tyr Asn Pro Phe Asp Val Asn Gly Asp Leu Ala Asp Val Tyr Tyr
    450                 455                 460
Met Tyr Gly Ala Thr Arg Pro Ser Met Thr Glu Pro Tyr Phe Ala Lys
465                 470                 475                 480
Met Arg Pro Phe Ser Ser Glu Ser His Gln Ala Asn Val Asn Gly Phe
                485                 490                 495
Ala Gln Ile Thr Pro Ile Lys Gly Leu Thr Leu Lys Ala Gln Ala Gly
            500                 505                 510
Val Asp Ile Thr Asn Thr Arg Thr Ser Ser Lys Arg Met Pro Asn Asn
        515                 520                 525
Pro Tyr Asp Ser Thr Pro Leu Gly Glu Arg Arg Glu Arg Ala Tyr Arg
    530                 535                 540
Asp Val Ser Lys Ser Phe Thr Asn Thr Ala Glu Tyr Lys Phe Ser Ile
545                 550                 555                 560
Asp Glu Lys His Asp Leu Thr Ala Leu Met Gly His Glu Tyr Ile Glu
                565                 570                 575
Tyr Glu Gly Asp Val Ile Gly Ala Ser Ser Lys Gly Phe Glu Ser Asp
            580                 585                 590
Lys Leu Met Leu Leu Ser Gln Gly Lys Thr Gly Asn Ser Leu Ser Leu
        595                 600                 605
Pro Glu His Arg Val Ala Glu Tyr Ala Tyr Leu Ser Phe Phe Ser Arg
    610                 615                 620
Phe Asn Tyr Gly Phe Asp Lys Trp Met Tyr Ile Asp Phe Ser Val Arg
625                 630                 635                 640
Asn Asp Gln Ser Ser Arg Phe Gly Ser Asn Asn Arg Ser Ala Trp Phe
                645                 650                 655
Tyr Ser Val Gly Gly Met Phe Asp Ile Tyr Asn Lys Phe Ile Gln Glu
            660                 665                 670
Ser Asn Trp Leu Ser Asp Leu Arg Leu Lys Met Ser Tyr Gly Thr Thr
        675                 680                 685
Gly Asn Ser Glu Ile Gly Asn Tyr Asn His Gln Ala Leu Val Thr Val
    690                 695                 700
Asn Asn Tyr Thr Glu Asp Ala Met Gly Leu Ser Ile Ser Thr Ala Gly
705                 710                 715                 720
Asn Pro Asp Leu Ser Trp Glu Lys Gln Ser Gln Phe Asn Phe Gly Leu
                725                 730                 735
Ala Ala Gly Ala Phe Asn Asn Arg Leu Ser Ala Glu Val Asp Phe Tyr
            740                 745                 750
Val Arg Thr Thr Asn Asp Met Leu Ile Asp Val Pro Met Pro Tyr Ile
        755                 760                 765
Ser Gly Phe Phe Ser Gln Tyr Gln Asn Val Gly Ser Met Lys Asn Thr
    770                 775                 780
Gly Val Asp Leu Ser Leu Lys Gly Thr Ile Tyr Gln Asn Lys Asp Trp
785                 790                 795                 800
Asn Val Tyr Ala Ser Ala Asn Phe Asn Tyr Asn Arg Gln Glu Ile Thr
                805                 810                 815
Lys Leu Phe Phe Gly Leu Asn Lys Tyr Met Leu Pro Asn Thr Gly Thr
            820                 825                 830
Ile Trp Glu Ile Gly Tyr Pro Asn Ser Phe Tyr Met Ala Glu Tyr Ala
        835                 840                 845
Gly Ile Asp Lys Lys Thr Gly Lys Gln Leu Trp Tyr Val Pro Gly Gln
    850                 855                 860
Val Asp Ala Asp Gly Asn Lys Val Thr Thr Ser Gln Tyr Ser Ala Asp
865                 870                 875                 880

Leu Glu Thr Arg Ile Asp Lys Ser Val Thr Pro Pro Ile Thr Gly Gly
                885                 890                 895
Phe Ser Leu Gly Ala Ser Trp Lys Gly Leu Ser Leu Asp Ala Asp Phe
            900                 905                 910
Ala Tyr Ile Val Gly Lys Trp Met Ile Asn Asn Asp Arg Tyr Phe Thr
        915                 920                 925
Glu Asn Ala Gly Gly Leu Met Gln Leu Asn Lys Asp Lys Met Leu Leu
    930                 935                 940
Asn Ala Trp Thr Glu Asp Asn Lys Glu Thr Asp Val Pro Lys Leu Gly
945                 950                 955                 960
Gln Ser Pro Gln Phe Asp Thr His Leu Leu Glu Asn Ala Ser Phe Leu
                965                 970                 975
Arg Leu Lys Asn Leu Lys Leu Thr Tyr Val Leu Pro Asn Ser Leu Phe
            980                 985                 990
Ala Gly Gln Asn Val Ile Gly Gly Ala Arg Val Tyr Leu Met Ala Arg
        995                 1000                1005
Asn Leu Leu Thr Val Thr Lys Tyr Lys Gly Phe Asp Pro Glu Ala Gly
    1010                1015                1020
Gly Asn Val Gly Lys Asn Gln Tyr Pro Asn Ser Lys Gln Tyr Val Ala
1025                1030                1035                1040
Gly Ile Gln Leu Ser Phe
                1045

(2) INFORMATION FOR SEQ ID NO:301

     (i) SEQUENCE CHARACTERISTICS:
          (A) LENGTH: 869 amino acids
          (B) TYPE: amino acid
          (D) TOPOLOGY: linear

    (ii) MOLECULE TYPE: protein

   (iii) HYPOTHETICAL: YES

    (vi) ORIGINAL SOURCE:
          (A) ORGANISM: Porphyromonas gingivalis

    (ix) FEATURE:
          (A) NAME/KEY: misc_feature
          (B) LOCATION 1...869

    (xi) SEQUENCE DESCRIPTION: SEQ ID NO:301
   Trp His Arg Asn Ile Phe Ile Phe Ala Ser Thr Phe Ser Pro Lys Asn
   1 5 10 15
   Met Leu Pro Leu Pro Tyr Arg Tyr Ala Lys Thr Glu His Leu Phe Leu
   20 25 30
   Ala Lys Gly Tyr Cys Lys Asn Pro Ile Thr Asn Ile Ile Ile Leu Phe
   35 40 45
   Met Lys Lys Lys Asn Phe Leu Leu Leu Gly Ile Phe Val Ala Leu Leu
   50 55 60
   Thr Phe Ile Gly Ser Met Gln Ala Gln Gln Ala Lys Asp Tyr Phe Asn
   65 70 75 80
   Phe Asp Glu Arg Gly Glu Ala Tyr Phe Ser Phe Lys Val Pro Asp Arg
   85 90 95
   Ala Val Leu Gln Glu Leu Ala Leu Ile Met Ser Ile Asp Glu Phe Asp
   100 105 110
   Pro Val Thr Asn Glu Ala Ile Ala Tyr Ala Ser Glu Glu Glu Phe Glu
   115 120 125
   Ala Phe Leu Arg Tyr Gly Leu Lys Pro Thr Phe Leu Thr Pro Pro Ser
   130 135 140
   Met Gln Arg Ala Val Glu Met Phe Asp Tyr Arg Ser Gly Glu Lys Tyr
   145 150 155 160
   Glu Trp Asn Ala Tyr Pro Thr Tyr Glu Ala Tyr Ile Ser Met Met Glu
   165 170 175
   Glu Phe Gln Thr Lys Tyr Pro Ser Leu Cys Thr Thr Ser Val Ile Gly
   180 185 190
   Lys Ser Val Lys Asp Arg Lys Leu Met Ile Cys Lys Leu Thr Ser Ser
   195 200 205
   Ala Asn Thr Gly Lys Lys Pro Arg Val Leu Tyr Thr Ser Thr Met His
   210 215 220
   Gly Asp Glu Thr Thr Gly Tyr Val Val Leu Leu Arg Leu Ile Asp His
   225 230 235 240
   Leu Leu Ser Asn Tyr Glu Ser Asp Pro Arg Ile Lys Asn Ile Leu Asp
   245 250 255
   Lys Thr Glu Val Trp Ile Cys Pro Leu Thr Asn Pro Asp Gly Ala Tyr
               260                 265                 270
   Arg Ala Gly Asn His Thr Val Gln Gly Ala Thr Arg Tyr Asn Ala Asn
           275                 280                 285
   Asn Val Asp Leu Asn Arg Asn Phe Lys Asp Asp Val Ala Gly Asp His
       290                 295                 300
   Pro Asp Gly Lys Pro Trp Gln Pro Glu Ala Thr Ala Phe Met Asp Leu
   305                 310                 315                 320
   Glu Gly Asn Thr Ser Phe Val Leu Gly Ala Asn Ile His Gly Gly Thr
                   325                 330                 335
   Glu Val Val Asn Tyr Pro Trp Asp Asn Lys Lys Glu Arg His Ala Asp
               340                 345                 350
   Asp Glu Trp Tyr Lys Leu Ile Ser Arg Asn Tyr Ala Ala Ala Cys Gln
           355                 360                 365
   Ser Ile Ser Ala Ser Tyr Met Thr Ser Glu Thr Asn Ser Gly Ile Ile
       370                 375                 380
   Asn Gly Ser Asp Trp Tyr Val Ile Arg Gly Ser Arg Gln Asp Asn Ala
   385                 390                 395                 400
   Asn Tyr Phe His Arg Leu Arg Glu Ile Thr Leu Glu Ile Ser Asn Thr
                   405                 410                 415
   Lys Leu Val Pro Ala Ser Gln Leu Pro Lys Tyr Trp Asn Leu Asn Lys
               420                 425                 430
   Glu Ser Leu Leu Ala Leu Ile Glu Glu Ser Leu Tyr Gly Ile His Gly
           435                 440                 445
   Thr Val Thr Ser Ala Ala Asn Gly Gln Pro Leu Lys Cys Gln Ile Leu
       450                 455                 460
   Ile Glu Asn His Asp Lys Arg Asn Ser Asp Val Tyr Ser Asp Ala Thr
   465                 470                 475                 480
   Thr Gly Tyr Tyr Val Arg Pro Ile Lys Ala Gly Thr Tyr Thr Val Lys
                   485                 490                 495
   Tyr Lys Ala Glu Gly Tyr Pro Glu Ala Thr Arg Thr Ile Thr Ile Lys
               500                 505                 510
   Asp Lys Glu Thr Val Ile Met Asp Ile Ala Leu Gly Asn Ser Val Pro
           515                 520                 525
   Leu Pro Val Pro Asp Phe Thr Ala Ser Pro Met Thr Ile Ser Val Gly
       530                 535                 540
   Glu Ser Val Gln Phe Gln Asp Gln Thr Thr Asn Asn Pro Thr Asn Trp
   545                 550                 555                 560
   Glu Trp Thr Phe Glu Gly Gly Gln Pro Ala Met Ser Thr Glu Gln Asn
                   565                 570                 575
   Pro Leu Val Ser Tyr Ser His Pro Gly Gln Tyr Asp Val Thr Leu Lys
               580                 585                 590
   Val Trp Asn Ala Ser Gly Ser Asn Thr Ile Thr Lys Glu Lys Phe Ile
           595                 600                 605
   Thr Val Asn Ala Val Met Pro Val Ala Glu Phe Val Gly Thr Pro Thr
       610                 615                 620
   Glu Ile Glu Glu Gly Gln Thr Val Ser Phe Gln Asn Gln Ser Thr Asn
   625                 630                 635                 640
   Ala Thr Asn Tyr Val Trp Ile Phe Asp Gly Gly Thr Pro Ala Thr Ser
                   645                 650                 655
   Glu Asp Glu Asn Pro Thr Val Leu Tyr Ser Lys Ala Gly Gln Tyr Asp
               660                 665                 670
   Val Thr Leu Lys Ala Ile Ser Ala Ser Gly Glu Thr Val Lys Thr Lys
           675                 680                 685
   Glu Lys Tyr Ile Thr Val Lys Lys Ala Pro Val Pro Ala Pro Val Ala
       690                 695                 700
   Asp Phe Glu Gly Thr Pro Arg Lys Val Lys Lys Gly Glu Thr Val Thr
   705                 710                 715                 720
   Phe Lys Asp Leu Ser Thr Asn Asn Pro Thr Ser Trp Leu Trp Val Phe
                   725                 730                 735
   Glu Gly Gly Ser Pro Ala Thr Ser Thr Glu Gln Asn Pro Val Val Thr
               740                 745                 750
   Tyr Asn Glu Thr Gly Lys Tyr Asp Val Gln Leu Thr Ala Thr Asn Glu
           755                 760                 765
   Gly Gly Ser Asn Val Lys Lys Ala Glu Asp Tyr Ile Glu Val Ile Leu
       770                 775                 780
   Asp Asp Ser Val Glu Asp Ile Val Ala Gln Thr Gly Ile Val Ile Arg
   785                 790                 795                 800
   Pro Gln Asn Gly Thr Lys Gln Ile Leu Ile Glu Ala Asn Ala Ala Ile
                   805                 810                 815
   Lys Ala Ile Val Leu Tyr Asp Ile Asn Gly Arg Val Val Leu Lys Thr
               820                 825                 830
   Thr Pro Asn Gln Leu Arg Ser Thr Val Asp Leu Ser Ile Leu Pro Glu
           835                 840                 845
   Gly Ile Tyr Thr Ile Asn Ile Lys Thr Glu Lys Ser Ala Arg Thr Glu
   850                     855                     860
   Lys Ile His Ile Gly
   865

(2) INFORMATION FOR SEQ ID NO:302

(i) SEQUENCE CHARACTERISTICS:
(A) LENGTH: 106 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...106

(xi) SEQUENCE DESCRIPTION: SEQ ID NO:302

Leu Ser Tyr Ser Gly Glu Ser Asp Ala Lys Glu Ser Asp Gln Asn Cys
1               5                   10                  15
Arg Lys Cys Thr Phe Ile Gly Phe Glu Lys Arg Val Asn Thr Met Arg
            20                  25                  30
Leu Ile Lys Ala Phe Leu Val Gln Leu Leu Leu Leu Pro Ile Phe Phe
        35                  40                  45
Tyr Lys Arg Phe Ile Ser Pro Leu Thr Pro Pro Ser Cys Arg Phe Thr
    50                  55                  60
Pro Ser Cys Ser Ser Tyr Ala Ile Glu Ala Leu Arg Lys Tyr Gly Pro
65                  70                  75                  80
Gly Lys Gly Leu Leu Leu Ser Ile Lys Arg Ile Leu Arg Cys His Pro
                85                  90                  95
Trp Gly Gly Ser Gly Tyr Asp Pro Val Pro
            100                 105

(2) INFORMATION FOR SEQ ID NO:303

(i) SEQUENCE CHARACTERISTICS:
(A) LENGTH: 861 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...861

(xi) SEQUENCE DESCRIPTION: SEQ ID NO:303

Leu Glu Met Ala Tyr Asp Phe Thr Gln Thr Phe Arg Asn Ser Leu Glu
1               5                   10                  15
Tyr Ser Tyr Gln Glu Ala Thr Arg Leu Gly Val Val Ala Val Thr Gln
            20                  25                  30
Asp Met Leu Val Leu Gly Ile Ile Arg Asp Gly Asp Asn Gly Ala Ile
        35                  40                  45
Asp Ile Met Arg His Tyr Gly Ile Asn Leu Tyr Glu Leu Lys Arg Leu
    50                  55                  60
Ile Glu Leu Glu Ala Ile Ala Glu Ser Leu Pro Ala Ser Pro Glu Gly
65                  70                  75                  80
Ser Pro Ile Phe Thr Pro Ser Ala Arg Glu Ala Ile Asp Asp Ala Thr
                85                  90                  95
Asp Ile Cys Ala Asp Met Glu Asp Glu Ala Val Ser Pro Val His Leu
            100                 105                 110
Leu Leu Ser Ile Leu Asn Ser Thr Gln Glu Ser Leu Val Gln Lys Ile
        115                 120                 125

Phe Met Lys Gln Gly Ile Lys Tyr Asp Thr Ile Leu Ser Asp Tyr Phe
    130                 135                 140
Gly Gln Arg Asn Pro Ser Glu Gly Lys Ser Pro Ser Glu Met Glu Ile
145                 150                 155                 160
Leu Asp Gly Tyr Gln Asp Asn Asp Phe Asp Asp Glu Glu Asp Glu Ser
                165                 170                 175
Ser Pro Pro Ser Gly Asn Ser Gly Thr Gly Gly Gly Ser Gly Asp Ala
            180                 185                 190
Pro Glu Gln Asn Thr Gly Gly Gly Asp Thr Thr Thr Thr Thr Arg Ser
        195                 200                 205
Gly Gly Asp Thr Pro Ala Leu Asp Thr Phe Gly Thr Asp Ile Thr Ala
    210                 215                 220
Met Ala Ala Ala Gly Lys Leu Asp Pro Val Val Gly Arg Glu Gln Glu
225                 230                 235                 240
Ile Glu Arg Val Ile Gln Ile Leu Ser Arg Arg Lys Lys Asn Asn Pro
                245                 250                 255
Val Leu Ile Gly Glu Pro Gly Val Gly Lys Ser Ala Ile Val Glu Gly
            260                 265                 270
Leu Ala Glu Arg Ile Val Asn Arg Lys Val Ser Arg Ile Leu Phe Asp
        275                 280                 285
Lys Arg Ile Ile Ser Leu Asp Leu Ala Gln Met Val Ala Gly Thr Lys
    290                 295                 300
Tyr Arg Gly Gln Phe Glu Glu Arg Leu Lys Ala Val Leu Asp Glu Leu
305                 310                 315                 320
Lys Lys Asn Pro Gln Ile Ile Leu Phe Ile Asp Glu Ile His Thr Ile
                325                 330                 335
Val Gly Ala Gly Ser Ala Ala Gly Ser Met Asp Thr Ala Asn Met Leu
            340                 345                 350
Lys Pro Ala Leu Ala Arg Gly Gln Val Gln Cys Ile Gly Ala Thr Thr
        355                 360                 365
Leu Asp Glu Tyr Arg Lys Asn Ile Glu Lys Asp Gly Ala Leu Glu Arg
    370                 375                 380
Arg Phe Gln Lys Val Pro Ile Ala Pro Ser Thr Ala Glu Glu Thr Leu
385                 390                 395                 400
Thr Ile Leu Gln Asn Ile Lys Glu Lys Tyr Glu Asp Tyr His Gly Val
                405                 410                 415
Arg Tyr Thr Asp Glu Ala Ile Lys Ala Ala Val Glu Leu Thr Asp Arg
            420                 425                 430
Tyr Val Ser Asp Arg Phe Phe Pro Asp Lys Ala Ile Asp Ala Met Asp
        435                 440                 445
Glu Ala Gly Ala Ser Val His Ile Thr Asn Val Val Ala Pro Lys Glu
    450                 455                 460
Ile Glu Ile Leu Glu Ala Glu Leu Ala Ser Val Arg Glu Asn Lys Leu
465                 470                 475                 480
Ser Ala Val Lys Ala Gln Asn Tyr Glu Leu Ala Ala Ser Phe Arg Asp
                485                 490                 495
Gln Glu Arg Arg Thr Gln Gln Gln Ile Ala Glu Glu Lys Lys Lys Trp
            500                 505                 510
Glu Glu Gln Met Ser Lys His Arg Glu Thr Val Asp Glu Asn Val Val
        515                 520                 525
Ala His Val Val Ala Leu Met Thr Gly Val Pro Ala Glu Arg Leu Ser
    530                 535                 540
Thr Gly Glu Gly Glu Arg Leu Arg Thr Met Ala Asp Asp Leu Lys Thr
545                 550                 555                 560
Lys Val Val Gly Gln Asp Thr Ala Ile Glu Lys Met Val His Ala Ile
                565                 570                 575
Gln Arg Asn Arg Leu Gly Leu Arg Asn Glu Lys Lys Pro Ile Gly Ser
            580                 585                 590
Phe Leu Phe Leu Gly Pro Thr Gly Val Gly Lys Thr Tyr Leu Ala Lys
        595                 600                 605
Lys Leu Ala Glu Tyr Leu Phe Glu Asp Glu Asn Ala Met Ile Arg Val
    610                 615                 620
Asp Met Ser Glu Tyr Met Glu Lys Phe Ser Val Ser Arg Leu Val Gly
625                 630                 635                 640
Ala Pro Pro Gly Tyr Val Gly Tyr Glu Glu Gly Gly Gln Leu Thr Glu
                645                 650                 655
Arg Val Arg Arg Lys Pro Tyr Ser Val Val Leu Leu Asp Glu Ile Glu
            660                 665                 670
Lys Ala His Ala Asp Val Phe Asn Leu Leu Leu Gln Val Met Asp Glu
        675                 680                 685
Gly Gln Leu Thr Asp Ser Leu Gly Arg Arg Val Asn Phe Lys Asn Thr
    690                 695                 700
Val Ile Ile Ile Thr Ser Asn Val Gly Thr Arg Gln Leu Lys Asp Phe
705                 710                 715                 720
Gly Gln Gly Ile Gly Phe Arg Ser Glu Lys Asp Glu Glu Ala Asn Lys

725                 730                 735
Glu His Ser Arg Ser Val Ile Gln Lys Ala Leu Asn Lys Thr Phe Ser
            740                 745                 750
Pro Glu Phe Leu Asn Arg Leu Asp Asp Ile Ile Leu Phe Asp Gln Leu
        755                 760                 765
Gly Lys Thr Glu Ile Arg Arg Met Val Asp Ile Glu Leu Lys Ala Val
    770                 775                 780
Leu Ala Arg Ile His Arg Ala Gly Tyr Asp Leu Val Leu Thr Asp Glu
785                 790                 795                 800
Ala Lys Asp Val Ile Ala Thr Lys Gly Tyr Asp Leu Gln Tyr Gly Ala
                805                 810                 815
Arg Pro Leu Lys Arg Thr Leu Gln Asn Glu Val Glu Asp Arg Leu Thr
            820                 825                 830
Asp Leu Ile Leu Ser Gly Gln Ile Glu Lys Gly Gln Thr Leu Thr Leu
        835                 840                 845
Ser Ala Arg Asp Gly Glu Ile Ile Val Gln Glu Gln Ala
    850                 855                 860

(2) INFORMATION FOR SEQ ID NO:304

     (i) SEQUENCE CHARACTERISTICS:
          (A) LENGTH: 456 amino acids
          (B) TYPE: amino acid
          (D) TOPOLOGY: linear

    (ii) MOLECULE TYPE: protein

   (iii) HYPOTHETICAL: YES

    (vi) ORIGINAL SOURCE:
          (A) ORGANISM: Porphyromonas gingivalis

    (ix) FEATURE:
          (A) NAME/KEY: misc_feature
          (B) LOCATION 1...456

    (xi) SEQUENCE DESCRIPTION: SEQ ID NO:304

Arg Gly Gly Gln Ile Arg Arg His His Thr Asp Ser Ser Arg Gly Ser
1               5                   10                  15
Asp Ser Lys Ala Cys Arg Phe Ser Phe His Ala Glu Thr Ile Gly Phe
            20                  25                  30
Ser Asn His Gln Arg Ala Lys Met Asn Tyr Leu Tyr Ile Leu Ile Thr
        35                  40                  45
Leu Leu Leu Ser Gly Phe Phe Ser Gly Ala Glu Ile Ala Phe Leu Ser
    50                  55                  60
Ser Asp Lys Leu Arg Leu Glu Leu Asp Arg Asn Arg Gly Asp Leu Thr
65                  70                  75                  80
Gly Arg Ala Leu Asn Leu Leu Tyr Arg His Pro Asp Gln Leu Val Thr
                85                  90                  95
Thr Leu Leu Val Gly Asn Asn Ile Val Leu Val Val Tyr Gly Leu Leu
            100                 105                 110
Met Ala Gly Leu Leu Ala Ala Pro Leu Ala Gln Trp Ile Asp Asn Asp
        115                 120                 125
Ala Met Ile Val Val Leu Gln Ser Val Leu Ser Thr Ile Ile Ile Leu
    130                 135                 140
Phe Thr Gly Glu Phe Leu Pro Lys Ala Ile Phe Lys Thr Asn Ala Asn
145                 150                 155                 160
Met Met Met Arg Val Phe Ala Leu Pro Ile Val Ala Ile Tyr Tyr Leu
                165                 170                 175
Leu Tyr Pro Leu Ser Lys Leu Phe Thr Gly Leu Ser Arg Ser Phe Ile
            180                 185                 190
Arg Leu Val Asp Lys Asn Tyr Val Pro Thr Thr Val Gly Leu Gly Arg
        195                 200                 205
Val Asp Leu Asp His Tyr Leu Ala Glu Asn Met Ser Gly Glu Asn Glu
    210                 215                 220
Gln Asn Asp Leu Thr Thr Glu Val Lys Ile Ile Gln Asn Ala Leu Asp
225                 230                 235                 240
Phe Ser Gly Ile Gln Val Arg Asp Cys Met Ile Pro Arg Asn Glu Met
                245                 250                 255
Ile Ala Cys Glu Leu Gln Thr Asp Ile Glu Val Leu Lys Thr Thr Phe
            260                 265                 270
Ile Asp Thr Gly Leu Ser Lys Ile Ile Ile Tyr Arg Gln Asn Ile Asp
        275                 280                 285
Asp Val Val Gly Tyr Ile His Ser Ser Glu Met Phe Arg Gly Gln Asp

290                 295                 300
Trp Gln Lys Arg Ile Asn Thr Thr Val Phe Val Pro Glu Ser Met Tyr
305                 310                 315                 320
Ala Asn Lys Leu Met Arg Leu Leu Met Gln Arg Lys Lys Ser Ile Ala
                325                 330                 335
Ile Val Ile Asp Glu Leu Gly Gly Thr Ala Gly Met Val Thr Leu Glu
            340                 345                 350
Asp Leu Val Glu Glu Ile Phe Gly Asp Ile Glu Asp Glu His Asp Thr
        355                 360                 365
Arg Lys Ile Ile Ala Lys Gln Leu Gly Pro His Thr Tyr Leu Val Ser
    370                 375                 380
Gly Arg Met Glu Ile Asp Asp Val Asn Glu Arg Phe Gly Leu Ser Leu
385                 390                 395                 400
Pro Glu Ser Asp Asp Tyr Leu Thr Val Ala Gly Phe Ile Leu Asn Ser
                405                 410                 415
His Gln Asn Ile Pro Gln Ala Asn Glu Val Val Glu Ile Ala Pro Tyr
            420                 425                 430
Thr Phe Thr Ile Leu Arg Ser Ser Ser Thr Lys Ile Glu Leu Val Lys
        435                 440                 445
Met Ser Ile Asp Asp Gln Ser Asn
    450                 455

(2) INFORMATION FOR SEQ ID NO:305

(i) SEQUENCE CHARACTERISTICS:
(A) LENGTH: 299 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...299

(xi) SEQUENCE DESCRIPTION: SEQ ID NO:305

Leu Lys Lys Glu Ile Thr Met Lys Gln Asn Tyr Phe Lys Arg Val Cys
1               5                   10                  15
Ser Leu Leu Trp Leu Val Leu Pro Met Leu Ile Met Pro Leu Glu Val
            20                  25                  30
Ala Ala Gln Glu Ile Ile Pro Asn Glu Glu Val Leu Glu Ser Leu Thr
        35                  40                  45
Phe Val Ala Pro Val Glu Glu Thr Asp Ala Ile Glu Ala Glu Val Glu
    50                  55                  60
Ala Leu Gln Glu Ile Val Ala Thr Glu Glu Ile Ala Glu Gln Ala Val
65                  70                  75                  80
Arg Ser Tyr Thr Tyr Thr Val Tyr Arg Asp Gly Val Lys Ile Ala Ser
                85                  90                  95
Gly Leu Thr Glu Pro Thr Phe Leu Asp Glu Asp Val Pro Ala Gly Glu
            100                 105                 110
His Thr Tyr Cys Val Glu Val Gln Tyr Gln Gly Gly Val Ser Asp Lys
        115                 120                 125
Val Cys Val Asp Val Glu Val Lys Asp Phe Lys Pro Val Thr Asn Leu
    130                 135                 140
Thr Gly Thr Ala Ser Asn Asp Glu Val Ser Leu Asp Trp Asp Gly Val
145                 150                 155                 160
Glu Glu Lys Ala Glu Glu Pro Ala Ser Asp Lys Ala Val Ser Tyr Asn
                165                 170                 175
Val Tyr Lys Asn Gly Thr Leu Ile Gly Asn Thr Ala Glu Thr His Tyr
            180                 185                 190
Val Glu Thr Gly Val Ala Asn Gly Thr Tyr Ile Tyr Glu Val Glu Val
        195                 200                 205
Lys Tyr Pro Asp Gly Val Ser Pro Lys Val Ala Val Thr Val Thr Val
    210                 215                 220
Thr Asn Ser Ser Leu Ser Asn Val Asp Gly Gln Ala Pro Tyr Thr Leu
225                 230                 235                 240
Arg Val Glu Gly Lys Lys Ile Ile Ala Glu Ala His Gly Met Ile Thr
                245                 250                 255
Leu Tyr Asp Ile Asn Gly Arg Thr Val Ala Val Ala Pro Asn Arg Leu

            260                 265                 270
Glu Tyr Met Ala Gln Thr Gly Phe Tyr Ala Val Arg Phe Asp Val Gly
        275                 280                 285
Asn Lys His His Val Ser Lys Ile Gln Val Arg
    290                 295

(2) INFORMATION FOR SEQ ID NO:306

(i) SEQUENCE CHARACTERISTICS:
(A) LENGTH: 377 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...377

(xi) SEQUENCE DESCRIPTION: SEQ ID NO:306

Leu Phe Asn His Lys Lys Ser Trp Tyr Glu Thr Phe Gln Phe Tyr Leu
1               5                   10                  15
Val Val Phe Arg Ser Gly Phe Ile Leu Leu Cys Glu Asn Thr Leu Ala
            20                  25                  30
Gln Gln Lys Thr Glu Glu Phe Ala Pro Val Ser Asp Leu Arg Ala Glu
        35                  40                  45
Ala Tyr Gly Ser Thr Val Phe Leu His Trp Thr Pro Pro Tyr Asp Asn
    50                  55                  60
Pro Met Ile Pro Leu Ser Glu Ser Phe Glu Ser Gly Ile Pro Ala Ile
65                  70                  75                  80
Trp Lys Thr Ile Asp Ala Asp Gly Asp Gly Tyr Asn Trp Met His Leu
                85                  90                  95
Thr Asn Phe Thr Gly Gln Ser Gly Leu Cys Val Ser Ser Ala Ser Tyr
            100                 105                 110
Ile Gly Gly Val Gly Ala Leu Thr Pro Asp Asn Tyr Leu Ile Thr Pro
        115                 120                 125
Glu Leu Lys Leu Pro Thr Asp Ala Leu Val Glu Ile Ile Tyr Trp Val
    130                 135                 140
Cys Thr Gln Asp Leu Thr Ala Pro Ser Glu His Tyr Ala Val Tyr Ser
145                 150                 155                 160
Ser Ser Thr Gly Asn Asn Ala Ala Asp Phe Val Asn Leu Leu Tyr Glu
                165                 170                 175
Glu Thr Leu Thr Ala Lys Arg Ile Gln Ser Pro Glu Leu Ile Arg Gly
            180                 185                 190
Asn Arg Thr Gln Gly Val Trp Tyr Gln Arg Lys Val Val Leu Pro Asn
        195                 200                 205
Asp Thr Lys Tyr Val Ala Phe Arg His Phe Asn Ser Thr Asp Asn Phe
    210                 215                 220
Trp Leu Asn Leu Asp Glu Val Ser Ile Leu Tyr Thr Pro Leu Pro Arg
225                 230                 235                 240
Arg Ala Pro Cys Pro His Pro Gly Gly Tyr Thr Tyr Ser Val Phe Arg
                245                 250                 255
Asp Gly Gln Lys Ile Ala Ser Gly Leu Ser Ala Leu Ala Tyr Ile Asp
            260                 265                 270
Thr Asp Val Pro Tyr Gly Thr Gln Asp Tyr Cys Val Gln Val Asn Tyr
        275                 280                 285
Leu Gln Gly Asp Ser Tyr Lys Val Cys Lys Asn Ile Val Val Ala Asn
    290                 295                 300
Ser Ala Asn Ile Tyr Gly Ala Asp Lys Pro Phe Ala Leu Thr Val Val
305                 310                 315                 320
Gly Lys Thr Ile Val Ala Ser Ala Phe Lys Gly Glu Ile Thr Leu Tyr
                325                 330                 335
Asp Ile Arg Gly Arg Leu Ile Ala Ser Gly Cys Asp Thr Leu Arg Tyr
            340                 345                 350
Lys Ala Glu Asn Gly Phe Tyr Leu Ile Lys Ile Gln Val Asn Gly Thr
        355                 360                 365
Val Tyr Thr Glu Lys Ile Gln Ile Gln
    370                 375

(2) INFORMATION FOR SEQ ID NO:307

(i) SEQUENCE CHARACTERISTICS:
(A) LENGTH: 849 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...849

(xi) SEQUENCE DESCRIPTION: SEQ ID NO:307

Phe Gly Ile Ser Pro Ser Met Lys Lys Ser Phe Leu Leu Ala Ile Val
1               5                   10                  15
Met Leu Phe Gly Ile Ala Met Gln Gly His Ser Ala Pro Val Thr Lys
            20                  25                  30
Glu Arg Ala Leu Ser Leu Ala Arg Leu Ala Leu Arg Gln Val Ser Leu
        35                  40                  45
Arg Met Gly Gln Thr Ala Val Ser Asp Lys Ile Ser Ile Asp Tyr Val
    50                  55                  60
Tyr Arg Gln Gly Asp Ala Glu Arg Gly Ile Thr Ser Gln Glu Glu Gly
65                  70                  75                  80
Ser Pro Ala Tyr Phe Tyr Val Ala Asn Arg Gly Asn Asn Glu Gly Tyr
                85                  90                  95
Ala Leu Val Ala Ala Asp Asp Arg Ile Pro Thr Ile Leu Ala Tyr Ser
            100                 105                 110
Pro Ile Gly Arg Phe Asp Met Asp Ser Met Pro Asp Asn Leu Arg Met
        115                 120                 125
Trp Leu Gln Ile Tyr Asp Gln Glu Ile Gly Leu Ile Leu Ser Gly Lys
    130                 135                 140
Ala Gln Leu Asn Glu Glu Ile Leu Arg Thr Glu Gly Val Pro Ala Glu
145                 150                 155                 160
Val His Ala Leu Met Asp Asn Gly His Phe Ala Asn Asp Pro Met Arg
                165                 170                 175
Trp Asn Gln Gly Tyr Pro Trp Asn Asn Lys Glu Pro Leu Leu Pro Asn
            180                 185                 190
Gly Asn His Ala Tyr Thr Gly Cys Val Ala Thr Ala Ala Ala Gln Ile
        195                 200                 205
Met Arg Tyr His Ser Trp Pro Leu Gln Gly Glu Gly Ser Phe Asp Tyr
    210                 215                 220
His Ala Gly Ser Leu Val Gly Asn Trp Ser Gly Thr Phe Gly Glu Met
225                 230                 235                 240
Tyr Asp Trp Ile Asn Met Pro Gly Asn Pro Asp Leu Asp Asn Leu Thr
                245                 250                 255
Gln Ser Gln Val Asp Ala Tyr Ala Thr Leu Met Arg Asp Val Ser Ala
            260                 265                 270
Ser Val Ser Met Ser Phe Tyr Glu Asn Gly Ser Gly Thr Tyr Ser Val
        275                 280                 285
Tyr Val Val Gly Ala Leu Arg Asn Asn Phe Arg Tyr Lys Arg Ser Leu
    290                 295                 300
Gln Leu His Val Arg Ala Leu Tyr Thr Ser Gln Glu Trp His Asp Met
305                 310                 315                 320
Ile Arg Gly Glu Leu Ala Ser Gly Arg Pro Val Tyr Tyr Ala Gly Asn
                325                 330                 335
Asn Gln Ser Ile Gly His Ala Phe Val Cys Asp Gly Tyr Ala Ser Asp
            340                 345                 350
Gly Thr Phe His Phe Asn Trp Gly Trp Gly Gly Val Ser Asn Gly Phe
        355                 360                 365
Tyr Lys Leu Thr Leu Leu Ser Pro Thr Ser Leu Gly Ile Gly Gly Glu
    370                 375                 380
Gly Ile Gly Phe Thr Ile Tyr Gln Glu Ile Ile Thr Gly Ile Glu Pro
385                 390                 395                 400
Ala Lys Thr Pro Ala Glu Ala Gly Thr Asp Ala Leu Pro Ile Leu Ala
                405                 410                 415
Leu Lys Asp Ile Glu Ala Glu Tyr Lys Ser Glu Ser Gly Leu Asn Val
            420                 425                 430
Gly Tyr Ser Ile Tyr Asn Thr Gly Glu Glu Gln Ser Asn Leu Asp Leu

        435                 440                 445
Gly Tyr Arg Leu Asn Lys Ala Asp Gly Glu Val Ile Glu Val Lys Thr
    450                 455                 460
Ser Ser Ile Asn Ile Ser Trp Tyr Gly Tyr Gly Glu His Pro Glu Ser
465                 470                 475                 480
Phe Ser Leu Ala Pro Asn Gln Leu Ser Gln Gly Ile Asn Thr Ile Thr
                485                 490                 495
Leu Leu Tyr Arg Arg Thr Gly Thr Glu Gln Trp Glu Pro Val Arg His
            500                 505                 510
Ala Gln Gly Gly Tyr Val Asn Ser Ile Lys Val Asn Thr Thr Asp Pro
        515                 520                 525
Asn Asn Val Val Val Thr Val Asp Asn Asn Glu Gly Lys Leu Ser Ile
    530                 535                 540
Val Pro Asn Ser Phe Val Ala Asp Leu Asn Ser Tyr Glu His Ser Thr
545                 550                 555                 560
Ile Thr Val Gln Phe Asn Ser Asp Ser Pro Asp Glu Ile Arg Thr Pro
                565                 570                 575
Val Ala Phe Ala Leu Ser Thr Gly Ala Thr Ala Asp Asp Val Ile Ser
            580                 585                 590
Leu Gly Trp Val Met Ala Glu Val Pro Gly Gly Ser Ser Asn Tyr Pro
        595                 600                 605
Val Val Trp Ser Lys Asp Val Leu Thr Leu Ser Glu Gly Asp Tyr Thr
    610                 615                 620
Leu Trp Tyr Arg Phe Ser Ile Asn Asn Gln Lys Asp Glu Trp Lys Lys
625                 630                 635                 640
Ile Gly Ser Val Ser Val Lys Thr Pro Thr Glu Tyr Thr His Pro Leu
                645                 650                 655
Phe Glu Val Gly His Asn Gln Thr Ser Thr Tyr Thr Leu Asp Met Ala
            660                 665                 670
His Asn Arg Val Leu Pro Asp Phe Thr Leu Lys Asn Leu Gly Leu Pro
        675                 680                 685
Phe Asn Gly Glu Leu Val Val Val Phe Arg Gln Thr Gln Ser Ser Ser
    690                 695                 700
Gly Ser Leu Trp Ala Ala Gln Glu Thr Val His Ile Lys Gln Gly Glu
705                 710                 715                 720
Thr Phe Val Tyr Lys Pro Val Val Glu Gly Pro Ile Pro Asp Gly Ser
                725                 730                 735
Tyr Arg Ala Thr Leu His Ala Phe Val Asn Gly Gln Gln Gln Leu Tyr
            740                 745                 750
Leu Lys Gly Lys Arg Asn Tyr Thr Val Lys Ile Val Asn Gly Thr Ala
        755                 760                 765
Val Glu Ala Ile Glu Ser Ser Glu Glu Ile Arg Val Phe Pro Asn Pro
    770                 775                 780
Ala Arg Asp Tyr Val Glu Ile Ser Ala Pro Cys Ile Pro Gln Glu Thr
785                 790                 795                 800
Ser Ile Ile Leu Phe Asp Leu Ser Gly Lys Ile Val Met Lys Asn Ser
                805                 810                 815
Leu Ser Ala Gly His Gly Arg Met Asp Val Ser Arg Leu Pro Asn Gly
            820                 825                 830
Ala Tyr Ile Leu Lys Val Asp Gly Tyr Thr Thr Lys Ile Asn Ile Val
        835                 840                 845
His

(2) INFORMATION FOR SEQ ID NO:308

     (i) SEQUENCE CHARACTERISTICS:
          (A) LENGTH: 295 amino acids
          (B) TYPE: amino acid
          (D) TOPOLOGY: linear

    (ii) MOLECULE TYPE: protein

   (iii) HYPOTHETICAL: YES

    (vi) ORIGINAL SOURCE:
          (A) ORGANISM: Porphyromonas gingivalis

    (ix) FEATURE:
          (A) NAME/KEY: misc_feature
          (B) LOCATION 1...295

    (xi) SEQUENCE DESCRIPTION: SEQ ID NO:308

Thr Lys Lys Thr Leu Met Lys Lys Leu Phe Leu Ser Leu Thr Ser Leu

1                   5                   10                  15
Val Met Val Phe Ala Val Ala Ser Cys Asp Ile Ile Asp Lys Asp Gln
            20                  25                  30
Thr Leu Leu Pro Ala Pro Thr Asn Val Thr Pro Asp Asn Pro Asp Asp
        35                  40                  45
Asn Pro Ser Glu Ile Asp Ile Thr Gln Thr His Thr Glu Lys Tyr Val
    50                  55                  60
Leu Ala Glu Glu Phe Thr Gly Gln Lys Cys Leu Asn Cys Pro Lys Gly
65                  70                  75                  80
His Arg Lys Leu Ala Ala Leu Lys Glu Gln Tyr Gly Lys Arg Leu Thr
                85                  90                  95
Val Val Gly Ile His Ala Gly Pro Gly Ser Leu Val Pro Pro Leu Phe
            100                 105                 110
Arg Thr Glu Ala Gly Asp Ala Tyr Tyr Ser Lys Phe Ala Asn Asn Thr
        115                 120                 125
Pro Leu Pro Ala Leu Met Val Ser Arg Lys Lys Phe Gly Ser Ser Tyr
    130                 135                 140
Val Tyr Asp Lys Ser Tyr Lys Thr Trp Asp Val Pro Ile Ala Glu Gln
145                 150                 155                 160
Met Glu Gln Lys Ala Lys Ile Asn Ile Phe Ala Val Ala Glu Tyr Thr
                165                 170                 175
Asp Thr Gln Lys Ile Lys Val Thr Val Lys Gly Lys Ile Leu Glu Gly
            180                 185                 190
Asn Thr Leu Pro Lys Ser Met Val Gln Val Tyr Leu Leu Glu Asp Lys
        195                 200                 205
Leu Ile Ala Pro Gln Val Asp Gly Asn Thr Thr Val Glu Asn Tyr Glu
    210                 215                 220
His Asn His Val Leu Arg Gly Ala Val Asn Gly Ile Trp Gly Glu Glu
225                 230                 235                 240
Phe Val Asn Leu Lys Asp Tyr Leu Tyr Thr Tyr Ala Val Glu Pro Leu
                245                 250                 255
Ser Gly Met Ser Phe Val Ala Glu Asn Tyr Ser Ile Val Ala Phe Val
            260                 265                 270
Tyr Asp Val Gln Thr Phe Glu Val Tyr Asp Val Val His Val Lys Ile
        275                 280                 285
Asn Pro Gln Ser Asp Gly Lys
    290                 295

(2) INFORMATION FOR SEQ ID NO:309

(i) SEQUENCE CHARACTERISTICS:
(A) LENGTH: 230 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...230

(xi) SEQUENCE DESCRIPTION: SEQ ID NO:309

Thr Asn Lys Lys Glu Glu Thr Met Lys Lys Ser Ser Val Val Ala Ser
1                   5                   10                  15
Val Leu Ala Val Ala Leu Val Phe Ala Gly Cys Gly Leu Asn Asn Met
            20                  25                  30
Ala Lys Gly Gly Leu Ile Gly Ala Gly Val Gly Gly Ala Ile Gly Ala
        35                  40                  45
Gly Val Gly Asn Val Ala Gly Asn Thr Ala Val Gly Ala Ile Val Gly
    50                  55                  60
Thr Ala Val Gly Gly Ala Ala Gly Ala Leu Ile Gly Lys Lys Met Asp
65                  70                  75                  80
Lys Gln Lys Lys Glu Leu Glu Ala Ala Val Pro Asp Ala Thr Ile Gln
                85                  90                  95
Thr Val Asn Asp Gly Glu Ala Ile Leu Val Thr Phe Asp Ser Gly Ile
            100                 105                 110
Leu Phe Ala Thr Asn Ser Ser Thr Leu Ser Pro Asn Ser Arg Thr Ala
        115                 120                 125
Leu Thr Lys Phe Ala Ala Asn Met Asn Lys Asn Pro Asp Thr Asp Ile

130                 135                 140
Arg Ile Val Gly His Thr Asp Asn Thr Gly Ser Asp Lys Ile Asn Asp
145                 150                 155                 160
Pro Leu Ser Glu Arg Arg Ala Ala Ser Val Tyr Ser Phe Leu Asn Ser
                165                 170                 175
Gln Gly Val Ser Met Ser Arg Met Ala Ala Glu Gly Arg Gly Ser His
            180                 185                 190
Glu Pro Val Ala Asp Asn Ser Thr Val Ala Gly Arg Ser Ala Asn Arg
        195                 200                 205
Arg Val Glu Val Tyr Ile Leu Pro Asn Ala Lys Met Ile Glu Gln Ala
    210                 215                 220
Gln Gln Gly Thr Leu Lys
225                 230

(2) INFORMATION FOR SEQ ID NO:310

    (i) SEQUENCE CHARACTERISTICS:
        (A) LENGTH: 342 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...342

    (xi) SEQUENCE DESCRIPTION: SEQ ID NO:310

Asn Arg Asn Arg Asn Met Ser Lys Lys Ser Ile Leu Leu Leu Cys Cys
1               5                   10                  15
Ser Leu Cys Phe Ile Ser Ala Thr Lys Ala Val Thr Pro Val Arg Asn
            20                  25                  30
Val Arg Asn Ser Gln Val Asn Ser Lys Ala Lys Thr Glu Arg Thr Lys
        35                  40                  45
Pro Ser Asp Ser Val Arg Tyr Ile Ser Asn Met Ile Ala Asp Arg Leu
    50                  55                  60
Glu Phe Arg Asn Lys Ile Ser Ser Glu Lys Glu Val Arg Lys Ala Glu
65                  70                  75                  80
Tyr Glu Asn Arg Leu Ala Met Glu Ala Leu Asn Tyr Pro Ala Ile Asp
                85                  90                  95
Leu Tyr Gly Glu Asp Ser Trp Ser Glu Tyr Val Asn Pro Phe Val Gly
            100                 105                 110
Ala Gly Thr Asp Val Glu Ile Pro Asn Ser Tyr Asp Ile Asp Cys Ser
        115                 120                 125
Ser Phe Val Met Pro Val Glu Asp Lys Gln Val Thr Ser Gln Phe Gly
    130                 135                 140
Tyr Arg Arg Arg Phe Gly Arg Met His Tyr Gly Ile Asp Leu Ser Val
145                 150                 155                 160
Asn Arg Gly Asp Thr Ile Arg Ala Ala Phe Asp Gly Lys Val Arg Val
                165                 170                 175
Arg Ser Tyr Glu Ala Arg Gly Tyr Gly Tyr Tyr Ile Val Leu Arg His
            180                 185                 190
Pro Asn Gly Leu Glu Thr Val Tyr Gly His Met Ser Arg Gln Leu Val
        195                 200                 205
Asp Glu Asn Gln Ile Val Arg Ala Gly Gln Pro Ile Gly Leu Gly Gly
    210                 215                 220
Ser Thr Gly Arg Ser Thr Gly Pro His Leu His Phe Glu Thr Arg Phe
225                 230                 235                 240
Met Gly Ile Pro Ile Asn Pro Ser Thr Ile Ile Asp Phe Asp Asn Gly
                245                 250                 255
Val Pro Leu Arg Asp Ile Tyr Thr Phe Lys Arg Gly Ser Asn Ser Arg
            260                 265                 270
Tyr Ala Lys Ala Ser Lys Thr Ser Ser Arg Tyr Ala Lys Lys Gly Lys
        275                 280                 285
Lys Gly Arg Gln Ala Ser Ser Pro Met Thr Tyr Arg Ile Lys Lys Gly
    290                 295                 300
Asp Thr Leu Glu Thr Ile Ala Lys Arg His Gly Thr Ser Val Gln Lys
305                 310                 315                 320
Leu Cys Ala Thr Asn Gly Ile Gly Lys Ser Lys Ile Leu Thr Pro Gly

325                    330                    335
Lys Ala Leu Arg Ile Lys
            340

(2) INFORMATION FOR SEQ ID NO:311

     (i) SEQUENCE CHARACTERISTICS:
          (A) LENGTH: 159 amino acids
          (B) TYPE: amino acid
          (D) TOPOLOGY: linear

    (ii) MOLECULE TYPE: protein

   (iii) HYPOTHETICAL: YES

    (vi) ORIGINAL SOURCE:
          (A) ORGANISM: Porphyromonas gingivalis

    (ix) FEATURE:
          (A) NAME/KEY: misc_feature
          (B) LOCATION 1...159

    (xi) SEQUENCE DESCRIPTION: SEQ ID NO:311

Pro Ser Lys Thr Ile Ile Lys Thr Met Ala Lys Ile Asn Phe Tyr Ala
1               5                   10                  15
Glu Gly Val Ser Leu Pro Arg Ile Arg Arg Arg Ile Val Gly Lys Trp
            20                  25                  30
Ile Ala Glu Val Cys Ser Arg Tyr Gly Lys Ala Val Gly Glu Ile Ser
        35                  40                  45
Tyr Leu Phe Cys Asp Asp Glu Tyr Ile Leu Lys Ala Asn Gln Glu Phe
    50                  55                  60
Leu Asp His Asp Tyr Tyr Thr Asp Ile Ile Thr Phe Asp Ser Cys Glu
65                  70                  75                  80
Ala Asp Thr Val Asn Gly Asp Leu Leu Ile Ser Leu Asp Thr Val Arg
                85                  90                  95
Ser Asn Ala Arg Ala Leu Asp Leu Arg Tyr Glu Asp Glu Leu His Arg
            100                 105                 110
Val Ile Ile His Gly Ile Leu His Leu Cys Gly Leu Lys Asp Lys Ser
        115                 120                 125
Lys Lys Asp Glu Ala Gln Met Arg Ala Ala Glu Glu Lys Ala Leu Val
    130                 135                 140
Met Leu Arg Glu Thr Ile Gly Ser Glu Leu Ser Leu Leu His Thr
145                 150                 155

(2) INFORMATION FOR SEQ ID NO:312

     (i) SEQUENCE CHARACTERISTICS:
          (A) LENGTH: 395 amino acids
          (B) TYPE: amino acid
          (D) TOPOLOGY: linear

    (ii) MOLECULE TYPE: protein

   (iii) HYPOTHETICAL: YES

    (vi) ORIGINAL SOURCE:
          (A) ORGANISM: Porphyromonas gingivalis

    (ix) FEATURE:
          (A) NAME/KEY: misc_feature
          (B) LOCATION 1...395

    (xi) SEQUENCE DESCRIPTION: SEQ ID NO:312

Lys Ser Lys Thr Met Lys Val Lys Tyr Leu Met Leu Thr Leu Val Gly
1               5                   10                  15
Ala Ile Ala Leu Asn Ala Ser Ala Gln Glu Asn Thr Val Pro Ala Thr
            20                  25                  30
Gly Gln Leu Pro Ala Lys Asn Val Ala Phe Ala Arg Asn Lys Ala Gly
        35                  40                  45
Ser Asn Trp Phe Val Thr Leu Gln Gly Gly Val Ala Ala Gln Phe Leu
    50                  55                  60
Asn Asp Asn Asn Asn Lys Asp Leu Met Asp Arg Leu Gly Ala Ile Gly
65                  70                  75                  80

Ser Leu Ser Val Gly Lys Tyr His Ser Pro Phe Phe Ala Thr Arg Leu
                85                  90                  95
Gln Ile Asn Gly Gly Gln Ala His Thr Phe Leu Gly Lys Asn Gly Glu
            100                 105                 110
Gln Glu Ile Asn Thr Asn Phe Gly Ala Ala His Phe Asp Phe Met Phe
        115                 120                 125
Asp Val Val Asn Tyr Phe Ala Pro Tyr Arg Glu Asn Arg Phe Phe His
    130                 135                 140
Leu Ile Pro Trp Val Gly Val Gly Tyr Gln His Lys Phe Ile Gly Ser
145                 150                 155                 160
Glu Trp Ser Lys Asp Asn Val Glu Ser Leu Thr Ala Asn Val Gly Val
                165                 170                 175
Met Met Ala Phe Arg Leu Gly Lys Arg Val Asp Phe Val Ile Glu Ala
            180                 185                 190
Gln Ala Ala His Ser Asn Leu Asn Leu Ser Arg Ala Tyr Asn Ala Lys
        195                 200                 205
Lys Thr Pro Val Phe Glu Asp Pro Ala Gly Arg Tyr Tyr Asn Gly Phe
    210                 215                 220
Gln Gly Met Ala Thr Ala Gly Leu Asn Phe Arg Leu Gly Ala Val Gly
225                 230                 235                 240
Phe Asn Ala Ile Unk Pro Met Asp Tyr Ala Leu Ile Asn Asp Leu Asn
                245                 250                 255
Gly Gln Ile Asn Arg Leu Arg Ser Glu Val Glu Glu Leu Ser Lys Arg
            260                 265                 270
Pro Val Ser Cys Pro Glu Cys Pro Glu Val Thr Pro Val Thr Lys Thr
        275                 280                 285
Glu Asn Ile Leu Thr Glu Lys Ala Val Leu Phe Arg Phe Asp Ser His
    290                 295                 300
Val Val Asp Lys Asp Gln Leu Ile Asn Leu Tyr Asp Val Ala Gln Phe
305                 310                 315                 320
Val Lys Glu Thr Asn Glu Pro Ile Thr Val Val Gly Tyr Ala Asp Pro
                325                 330                 335
Thr Gly Asn Thr Gln Tyr Asn Glu Lys Leu Ser Glu Arg Arg Ala Lys
            340                 345                 350
Ala Val Val Asp Val Leu Thr Gly Lys Tyr Gly Val Pro Ser Glu Leu
        355                 360                 365
Ile Ser Val Glu Trp Lys Gly Asp Ser Thr Gln Pro Phe Ser Lys Lys
    370                 375                 380
Ala Trp Asn Arg Val Val Ile Val Arg Ser Lys
385                 390                 395

(2) INFORMATION FOR SEQ ID NO:313

    (i) SEQUENCE CHARACTERISTICS:
        (A) LENGTH: 387 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...387

    (xi) SEQUENCE DESCRIPTION: SEQ ID NO:313

Tyr Lys Met Thr Tyr Arg Ile Met Lys Ala Lys Ser Leu Leu Leu Ala
1               5                   10                  15
Leu Ala Gly Leu Ala Cys Thr Phe Ser Ala Thr Ala Gln Glu Ala Thr
            20                  25                  30
Thr Gln Asn Lys Ala Gly Met His Thr Ala Phe Gln Arg Asp Lys Ala
        35                  40                  45
Ser Asp His Trp Phe Ile Asp Ile Ala Gly Gly Ala Gly Met Ala Leu
    50                  55                  60
Ser Gly Trp Asn Asn Asp Val Asp Phe Val Asp Arg Leu Ser Ile Val
65                  70                  75                  80
Pro Thr Phe Gly Ile Gly Lys Trp His Glu Pro Tyr Phe Gly Thr Arg
                85                  90                  95
Leu Gln Phe Thr Gly Phe Asp Ile Tyr Gly Phe Pro Gln Gly Ser Lys
            100                 105                 110

Glu Arg Asn His Asn Tyr Phe Gly Asn Ala His Leu Asp Phe Met Phe
        115                 120                 125
Asp Leu Thr Asn Tyr Phe Gly Val Tyr Arg Pro Asn Arg Val Phe His
    130                 135                 140
Ile Ile Pro Trp Ala Gly Ile Gly Phe Gly Tyr Lys Phe His Ser Glu
145                 150                 155                 160
Asn Ala Asn Gly Glu Lys Val Gly Ser Lys Asp Asp Met Thr Gly Thr
                165                 170                 175
Val Asn Val Gly Leu Met Leu Lys Phe Arg Leu Ser Arg Val Val Asp
            180                 185                 190
Phe Asn Ile Glu Gly Gln Ala Phe Ala Gly Lys Met Asn Phe Ile Gly
        195                 200                 205
Thr Lys Arg Gly Lys Ala Asp Phe Pro Val Met Ala Thr Ala Gly Leu
    210                 215                 220
Thr Phe Asn Leu Gly Lys Thr Glu Trp Thr Glu Ile Val Pro Met Asp
225                 230                 235                 240
Tyr Ala Leu Val Asn Asp Leu Asn Asn Gln Ile Asn Ser Leu Arg Gly
                245                 250                 255
Gln Val Glu Glu Leu Ser Arg Arg Pro Val Ser Cys Pro Glu Cys Pro
            260                 265                 270
Glu Pro Thr Gln Pro Thr Val Thr Arg Val Val Val Asp Asn Val Val
        275                 280                 285
Tyr Phe Arg Ile Asn Ser Ala Lys Ile Asp Arg Asn Gln Glu Ile Asn
    290                 295                 300
Val Tyr Asn Thr Ala Glu Tyr Ala Lys Thr Asn Asn Ala Pro Ile Lys
305                 310                 315                 320
Val Val Gly Tyr Ala Asp Glu Lys Thr Gly Thr Ala Ala Tyr Asn Met
                325                 330                 335
Lys Leu Ser Glu Arg Arg Ala Lys Ala Val Ala Lys Met Leu Glu Lys
            340                 345                 350
Tyr Gly Val Ser Ala Asp Arg Ile Thr Ile Glu Trp Lys Gly Ser Ser
        355                 360                 365
Glu Gln Ile Tyr Glu Glu Asn Ala Trp Asn Arg Ile Val Val Met Thr
    370                 375                 380
Ala Ala Glu
385

(2) INFORMATION FOR SEQ ID NO:314

    (i) SEQUENCE CHARACTERISTICS:
        (A) LENGTH: 195 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...195

    (xi) SEQUENCE DESCRIPTION: SEQ ID NO:314

Val Ile Gly Ile Ile Met Glu Phe Phe Met Leu Phe Ile Ala Ala Val
1               5                   10                  15
Phe Val Asn Asn Val Val Leu Ser Gln Phe Leu Gly Ile Cys Pro Phe
            20                  25                  30
Leu Gly Val Ser Lys Lys Val Asp Thr Ser Ile Gly Met Gly Ala Ala
        35                  40                  45
Val Thr Phe Val Leu Ala Leu Ala Thr Leu Val Thr Phe Leu Ile Gln
    50                  55                  60
Lys Phe Val Leu Asp Arg Phe Gly Leu Gly Phe Met Gln Thr Ile Ala
65                  70                  75                  80
Phe Ile Leu Val Ile Ala Ala Leu Val Gln Met Val Glu Ile Ile Leu
                85                  90                  95
Lys Lys Val Ser Pro Pro Leu Tyr Gln Ala Leu Gly Val Phe Leu Pro
            100                 105                 110
Leu Ile Thr Thr Asn Cys Cys Val Leu Gly Val Ala Ile Leu Val Ile
        115                 120                 125
Gln Lys Asp Tyr Thr Leu Leu Gln Ser Phe Val Tyr Ala Ile Ser Thr
    130                 135                 140

Ala Ile Gly Phe Thr Leu Ala Met Val Thr Phe Ala Gly Ile Arg Glu
145                 150                 155                 160
Gln Leu Asp Met Thr Asn Leu Pro Lys Ala Met Lys Gly Ile Pro Ser
                165                 170                 175
Ala Leu Leu Ala Ala Gly Ile Leu Ala Met Ala Phe Met Gly Phe Ser
            180                 185                 190
Gly Ile Ala
        195

(2) INFORMATION FOR SEQ ID NO:315

     (i) SEQUENCE CHARACTERISTICS:
          (A) LENGTH: 876 amino acids
          (B) TYPE: amino acid
          (D) TOPOLOGY: linear

    (ii) MOLECULE TYPE: protein

   (iii) HYPOTHETICAL: YES

    (vi) ORIGINAL SOURCE:
          (A) ORGANISM: Porphyromonas gingivalis

    (ix) FEATURE:
          (A) NAME/KEY: misc_feature
          (B) LOCATION 1...876

    (xi) SEQUENCE DESCRIPTION: SEQ ID NO:315

Tyr Arg Ser Tyr Arg Gly Ile Gly Ser Gly Thr His Ser Pro Asn Leu
1               5                   10                  15
Lys Asn Arg Leu Lys Arg Ile Gly Ile Arg Ile Pro Asn Arg His Tyr
            20                  25                  30
Ile His Ile Lys Pro Ile Lys Pro Lys Asn Lys Met Lys Gln Leu Asn
        35                  40                  45
Ile Ile Ser Phe Ile Ile Ala Phe Leu Phe Leu Gly Thr Ser Ala Ser
    50                  55                  60
Ala Gln Gln Ser Gly Gly Ser Val Thr Gly Thr Val Val Asp Lys Ser
65                  70                  75                  80
Ser Lys Glu Pro Ile Ala Tyr Val Gln Val Phe Val Lys Gly Thr Thr
                85                  90                  95
Leu Gly Thr Ser Thr Asp Ala Asn Gly Asn Tyr Ser Ile Lys Gly Ile
            100                 105                 110
Pro Ser Gly Asn Gln Thr Ile Val Ala Arg Leu Met Gly Tyr Ser Thr
        115                 120                 125
Cys Glu Glu Lys Val His Ile Glu Lys Gly Gly Ser Arg His Val Asp
    130                 135                 140
Leu Tyr Leu Thr Glu Glu Ile Leu Ser Leu Asp Gly Val Val Val Ser
145                 150                 155                 160
Ala Asn Arg Asn Glu Thr Phe Arg Arg Gln Ala Pro Ser Leu Val Thr
                165                 170                 175
Val Leu Ser Pro Glu Leu Phe Leu Lys Thr Asn Ser Thr Asn Leu Ser
            180                 185                 190
Gln Gly Leu Lys Phe Gln Pro Gly Leu Arg Val Glu Asp Asn Cys Gln
        195                 200                 205
Asn Cys Gly Phe Asn Gln Val Arg Ile Asn Gly Leu Glu Gly Ala Tyr
    210                 215                 220
Ser Gln Ile Leu Ile Asp Ser His Pro Ile Phe Ser Ser Leu Ala Gly
225                 230                 235                 240
Val Tyr Gly Leu Glu Gln Met Pro Ala Asn Met Ile Glu Arg Val Glu
                245                 250                 255
Val Ile Arg Gly Gly Gly Ser Ala Leu Phe Gly Ser Asn Ala Val Gly
            260                 265                 270
Gly Val Ile Asn Val Ile Thr Lys Glu Pro Leu Arg Asn Ser Ala Glu
        275                 280                 285
Ile Ser His Ser Thr Met Thr Phe Asp His Ala Lys Gly Trp Gly Ser
    290                 295                 300
Phe Gln Asn Thr Thr Gln Phe Asn Gly Ser Met Leu Thr Glu Asp Arg
305                 310                 315                 320
Lys Ala Gly Val Met Val Phe Gly Gln His Asn Tyr Arg Pro Gly Gln
                325                 330                 335
Asp Ile Asp Gly Asp Asn Phe Thr Glu Leu Pro Asn Leu Arg Asn Arg
            340                 345                 350
Ser Leu Gly Phe Arg Ser Tyr Tyr Lys Thr Gly Leu Tyr Ser Lys Ala
        355                 360                 365

Thr Leu Glu Tyr His Ser Met Gln Glu Tyr Arg Arg Gly Gly Asp Arg
    370                 375                 380
Leu Asp Asn Pro Pro Phe Glu Ala Gln Ile Ala Glu Tyr Leu Gln His
385                 390                 395                 400
Tyr Ile Asn Gly Gly Ser Phe Lys Phe Asp Gln Gly Phe Ser Gly Gly
                405                 410                 415
Lys Asp Phe Phe Ser Leu Tyr Ala Ser Ala Gln Asp Val Gln Arg Arg
            420                 425                 430
Ser Tyr Tyr Gly Gly Gly Asp Tyr Thr Glu Asn Leu Leu Asn Gly Ala
        435                 440                 445
Val Gln Ser Gly Ser Thr Glu Ser Asp Glu Tyr Asn Asp Ala Phe Thr
    450                 455                 460
Ala Leu Thr Ser Tyr Gly Thr Thr Lys Gly Phe Asp Leu Gln Gly Gly
465                 470                 475                 480
Gly Met Tyr Arg His Thr Phe Gly Glu Asn Trp Asp Phe Thr Gly Gly
                485                 490                 495
Leu Glu Tyr Ile Tyr Gly Gln Leu Asp Asp Arg Ser Gly Tyr Arg Pro
            500                 505                 510
Ser Lys Ile Asp Gln Asn Thr Ser Thr Phe Ser Gln Tyr Asp Gln Leu
        515                 520                 525
Glu Tyr Lys Thr Glu Lys Leu Ser Ala Leu Ile Gly Ala Arg Ile Asp
    530                 535                 540
Tyr Val Leu Leu Asn Gln Asp Gly Lys Arg Tyr Ile Asp Pro Leu Phe
545                 550                 555                 560
Ile Phe Ser Pro Arg Ala Asn Val Arg Tyr Asn Pro Asn Lys Asn Leu
                565                 570                 575
Ser Phe Arg Leu Ser Tyr Ser Glu Gly Phe Arg Ala Pro Gln Tyr Phe
            580                 585                 590
Asp Glu Asp Leu His Val Glu Leu Ala Gly Gly Thr Pro Ile Ser Arg
        595                 600                 605
Val Leu Ser Pro Asn Leu Lys Glu Glu Arg Ser Arg Ser Ile Ser Ala
    610                 615                 620
Ser Phe Asp Tyr Tyr His Arg Ala Asp Glu Trp Gln Phe Asn Ile Met
625                 630                 635                 640
Gly Glu Ala Phe Ser Thr Phe Ile Ser Asn Gln Phe Lys Pro Ser Asp
                645                 650                 655
Lys Val Glu Thr Thr Ser Asp Gly Lys Glu Trp Ile Ile Arg Thr Ile
            660                 665                 670
Tyr Asn Asp Lys Asp Gly Val Ser Lys Val Tyr Gly Val Asn Leu Glu
        675                 680                 685
Gly Arg Ile Ala Tyr Asn Lys Ser Phe Asp Leu Gln Leu Gly Gly Thr
    690                 695                 700
Trp Gln Arg Ser Arg Tyr Gly Ser Ile Tyr Thr Ala Val Glu Ala Asp
705                 710                 715                 720
Lys Thr Thr Gly Gln Ala Glu Ile Ser Val Lys Asp Tyr Val Arg Thr
                725                 730                 735
Pro Asn Leu Tyr Gly Tyr Phe Val Ala Thr Val Arg Pro Thr Glu His
            740                 745                 750
Phe Ala Ile Asn Leu Ser Gly Thr Phe Thr Gly Lys Met Asp Val Val
        755                 760                 765
His Glu Ala Tyr Glu Gly Asp Ile Pro Ala Glu His Ile Ala Pro Asp
    770                 775                 780
Gly Ser Phe Asp Phe Glu Met Asn Gly Gln Gln Phe Lys Gly Leu Ala
785                 790                 795                 800
Glu Gly His Ala Lys Leu Val Lys Thr Pro Ala Phe Ala Asp Ile Asp
                805                 810                 815
Leu Lys Leu Ser His Asp Phe His Leu Ala Ser Thr Met Thr Leu Glu
            820                 825                 830
Leu Asn Ala Gly Ile Gln Asn Ile Phe Asn Ser Tyr Gln Lys Asp Thr
        835                 840                 845
Asp Lys Gly Pro Gly Arg Ala Ser Thr Tyr Val Tyr Gly Pro Met Gln
    850                 855                 860
Pro Arg Arg Ile Phe Val Gly Thr Lys Ile Asn Phe
865                 870                 875

(2) INFORMATION FOR SEQ ID NO:316

    (i) SEQUENCE CHARACTERISTICS:
        (A) LENGTH: 899 amino acids
        (B) TYPE: amino acid
        (D) TOPOLOGY: linear

    (ii) MOLECULE TYPE: protein

    (iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...899

(xi) SEQUENCE DESCRIPTION: SEQ ID NO:316

Ile Leu Asn His Leu Arg Lys Thr Met Tyr Lys Lys Ile Ile Ala Val
1               5                   10                  15
Ala Ala Leu Phe Cys Ala Ser Ile Gly Ile Leu Lys Gly Gln Ser Ser
            20                  25                  30
Asp Leu Thr Pro Gln Asp Thr Ile Tyr Ser Pro Glu Ile Ser Tyr Ala
        35                  40                  45
Lys Pro Ile His Lys Thr Ile Ala Ser Ile Glu Ile Glu Gly Met Arg
    50                  55                  60
Ser Phe Asp Asp Phe Val Leu Arg Asn Leu Ser Gly Leu Ala Val Gly
65                  70                  75                  80
Asp Glu Val Leu Ile Pro Gly Asp Ala Met Ser Ala Ala Val Asn Arg
                85                  90                  95
Ile Met Arg Gln Gly Tyr Phe Ser Asn Val Arg Ile Ile Ala Asp Lys
            100                 105                 110
Tyr Val Gly Asn Lys Val Tyr Leu Lys Ile Ile Val Thr Glu Arg Pro
        115                 120                 125
Arg Ile Ser Lys Val Thr Phe Ser Gly Val Lys Lys Ser Glu Arg Glu
    130                 135                 140
Asp Leu Glu Met Lys Ile Gly Leu Arg Glu Gly Ile Gln Met Thr Arg
145                 150                 155                 160
Asn Asn Glu Asp Lys Val Arg Gln Ile Val Gln Lys Tyr Phe Ser Glu
                165                 170                 175
Lys Gly Tyr Arg Asp Ala Ser Ile Arg Ile Thr Gln Glu Pro Asp Leu
            180                 185                 190
Ser Lys Asp Gly Phe Val Asn Val Leu Ile Ser Ile Glu Lys Lys Ser
        195                 200                 205
Lys Thr Lys Val Asn Glu Ile Tyr Phe Ser Gly Asn Lys Ala Leu Ser
    210                 215                 220
Asn His Lys Leu Arg Met Ala Met Lys Asn Thr Asn Ala Lys Phe Ser
225                 230                 235                 240
Leu Arg Lys His Ile Arg Ser Ser Phe Leu Lys Leu Phe Ser Thr His
                245                 250                 255
Lys Phe Val Glu Glu Ser Tyr Arg Glu Asp Leu Val Arg Leu Ile Glu
            260                 265                 270
Lys Tyr Gln Glu Tyr Gly Tyr Arg Asp Ala Glu Ile Leu Thr Asp Ser
        275                 280                 285
Val Val Lys Ala Pro Asp Gly Lys Arg Val Asp Ile Tyr Leu Asn Ile
    290                 295                 300
Glu Glu Gly Gln Lys Tyr Tyr Ile Lys Asp Val Asn Phe Val Gly Asn
305                 310                 315                 320
Ser Gln Tyr Pro Ser Glu Tyr Leu Glu Arg Val Leu Gly Ile Lys Ser
                325                 330                 335
Gly Asp Val Tyr Asn Gln Arg Arg Leu Ala Lys Arg Leu Asn Glu Asp
            340                 345                 350
Glu Asp Ala Val Gly Asn Leu Tyr Tyr Asn Asn Gly Tyr Ile Phe Ala
        355                 360                 365
Trp Val Asp Pro Val Glu Thr Asn Val Val Gly Asp Ser Val Ser Leu
    370                 375                 380
Asp Ile Arg Ile Ala Glu Gly Lys Gln Ala Asn Ile Asn Lys Val Ile
385                 390                 395                 400
Ile Lys Gly Asn Thr Val Val Tyr Glu Asp Val Val Arg Arg Glu Leu
                405                 410                 415
Tyr Thr Lys Pro Gly Gln Leu Phe Ser Arg Glu Asp Ile Ile Asn Ser
            420                 425                 430
Ile Arg Leu Ile Asn Gln Leu Gly His Phe Asp Ala Glu Lys Ser Ile
        435                 440                 445
Pro Arg Pro Ile Pro Asn Pro Glu Thr Gly Thr Val Asp Ile Glu Tyr
    450                 455                 460
Asp Leu Val Pro Arg Ser Ser Asp Gln Leu Glu Leu Ser Val Gly Trp
465                 470                 475                 480
Ser Gln Ser Gly Leu Leu Phe Arg Gly Ala Ile Lys Phe Thr Asn Phe
                485                 490                 495
Ser Val Gly Asn Leu Leu His Pro Ser Met Tyr Lys Lys Gly Ile Ile
            500                 505                 510
Pro Gln Gly Asp Gly Gln Thr Leu Ser Leu Ser Ala Gln Thr Asn Gly

515                 520                 525
Lys Tyr Tyr Gln Gln Tyr Ser Val Thr Phe Met Asp Pro Trp Phe Gly
    530                 535                 540
Gly Lys Arg Pro Asp Met Phe Ser Phe Ser Ala Phe Tyr Ser Lys Thr
545                 550                 555                 560
Thr Ala Ile Asp Ser Lys Phe Tyr Asn Ser Asn Ala Gly Asn Tyr Tyr
                565                 570                 575
Asn Ala Tyr Tyr Asn Ser Tyr Tyr Asn Asn Tyr Asn Ser Tyr Tyr Asn
            580                 585                 590
Gly Met Ser Asn Tyr Thr Gly Asp Leu Tyr Thr Gln Ala Ser Asp Pro
        595                 600                 605
Asp Arg Ser Leu Gln Met Leu Gly Thr Ser Ile Gly Tyr Gly Lys Arg
    610                 615                 620
Leu Thr Trp Pro Asp Asn Trp Phe Gln Ile Tyr Thr Ser Leu Asn Tyr
625                 630                 635                 640
Thr Tyr Tyr Arg Leu Arg Asn Trp Ser Tyr Asn Thr Phe Gln Asn Phe
                645                 650                 655
His His Gly Ser Ala Asn Asp Leu Asn Leu Glu Leu Arg Leu Ser Arg
            660                 665                 670
Thr Ser Ile Asp Asn Pro Ile Tyr Thr Arg Ser Gly Ser Asp Phe Met
        675                 680                 685
Val Ser Val Ala Ala Thr Leu Pro Tyr Ser Leu Trp Asp Asn His Asp
    690                 695                 700
Tyr Ala Ser Gln Asn Leu Ser Val Ser Asp Arg Tyr Arg Phe Ile Glu
705                 710                 715                 720
Tyr His Lys Trp Lys Phe Arg Gly Arg Val Phe Thr Pro Leu Leu Asn
                725                 730                 735
Pro Ala Thr His Lys Tyr Thr Pro Val Leu Met Ser Arg Val Glu Gly
            740                 745                 750
Ala Val Leu Gly Ser Tyr Asn Ser Asn Lys Lys Ser Pro Phe Gly Thr
        755                 760                 765
Phe Tyr Met Gly Gly Asp Gly Met Ser Ser Tyr Tyr Gly Gly Tyr Met
    770                 775                 780
Asn Glu Thr Ile Gly Leu Arg Gly Tyr Lys Asn Gly Ser Ile Ala Gly
785                 790                 795                 800
Asn Asn Tyr Asp Tyr Ala Tyr Ala Tyr Met Arg Leu Thr Met Glu Leu
                805                 810                 815
Arg Phe Pro Ile Leu Phe Glu Asn Ser Phe Asn Ala Trp Leu Leu Ala
            820                 825                 830
Phe Ala Glu Ala Gly Asn Ala Trp Arg Ser Ile Asp Asn Tyr Asn Pro
        835                 840                 845
Phe Asn Leu Lys Arg Ser Ala Gly Val Gly Leu Arg Val Thr Leu Pro
    850                 855                 860
Met Val Gly Met Leu Gly Ile Asp Trp Gly Tyr Gly Phe Asp Arg Pro
865                 870                 875                 880
Asp Asn Ser Leu Gln Arg Gly Gly Ser Asn Val His Phe Val Leu Gly
                885                 890                 895
Gln Glu Phe

(2) INFORMATION FOR SEQ ID NO:317

     (i) SEQUENCE CHARACTERISTICS:
          (A) LENGTH: 177 amino acids
          (B) TYPE: amino acid
          (D) TOPOLOGY: linear

     (ii) MOLECULE TYPE: protein

     (iii) HYPOTHETICAL: YES

     (vi) ORIGINAL SOURCE:
          (A) ORGANISM: Porphyromonas gingivalis

     (ix) FEATURE:
          (A) NAME/KEY: misc_feature
          (B) LOCATION 1...177

     (xi) SEQUENCE DESCRIPTION: SEQ ID NO:317

Gln Ile Asn Met Asn Gly Asp Met Lys Arg Phe Leu Ile Leu Ile Gly
1               5                   10                  15
Phe Ala Leu Ala Val Ala Phe Ser Gly Phe Ser Gln Lys Phe Ala Leu
            20                  25                  30
Val Asp Met Glu Tyr Ile Leu Arg Asn Ile Pro Asp Tyr Glu Met Met

35                  40                  45
Asn Glu Gln Leu Glu Gln Val Ser Lys Lys Trp Gln Asn Glu Ile Glu
    50                  55                  60
Ala Leu Glu Asn Glu Ala Gln Ser Met Tyr Lys Lys Tyr Gln Ser Asp
65                  70                  75                  80
Leu Val Phe Leu Ser Ala Ala Gln Lys Lys Thr Gln Glu Glu Ala Ile
                85                  90                  95
Val Lys Lys Glu Gln Gln Ala Ser Glu Leu Lys Arg Lys Tyr Phe Gly
            100                 105                 110
Pro Glu Gly Glu Leu Tyr Lys Lys Arg Ser Asp Leu Met Lys Pro Ile
        115                 120                 125
Gln Asp Glu Ile Trp Asn Ala Ile Lys Glu Ile Ala Lys Arg Asn Asn
    130                 135                 140
Tyr Gln Met Val Leu Asp Arg Gly Thr Ser Gly Ile Ile Phe Ala Ser
145                 150                 155                 160
Pro Ser Ile Asp Ile Ser Asp Leu Val Leu Ser Lys Met Gly Phe Ser
                165                 170                 175
Lys

(2) INFORMATION FOR SEQ ID NO:318

(i) SEQUENCE CHARACTERISTICS:
(A) LENGTH: 170 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...170

(xi) SEQUENCE DESCRIPTION: SEQ ID NO:318

Arg Ile Asn Lys Gln His Glu Met Lys Lys Phe Phe Leu Met Leu Leu
1               5                   10                  15
Met Ala Leu Pro Leu Ser Leu Leu Ala Gln Lys Val Ala Val Val Asn
            20                  25                  30
Thr Glu Glu Ile Ile Ser Lys Met Pro Glu Gln Val Ala Ala Thr Lys
        35                  40                  45
Gln Leu Asn Glu Leu Ala Glu Lys Tyr Arg Leu Asp Leu Lys Ser Met
    50                  55                  60
Asp Asp Glu Phe Ala Lys Lys Thr Glu Glu Phe Val Lys Glu Lys Asp
65                  70                  75                  80
Ser Leu Leu Glu Asn Ile Arg Asn Arg Arg Gln Gln Glu Leu Gln Asp
                85                  90                  95
Ile Gln Thr Arg Tyr Gln Gln Ser Tyr Gln Thr Met Gln Glu Asp Leu
            100                 105                 110
Gln Lys Arg Gln Gln Gln Leu Phe Ala Pro Ile Gln Gln Lys Val Ala
        115                 120                 125
Asp Ala Ile Lys Lys Val Gly Asp Glu Glu Asn Cys Ala Tyr Ile Met
    130                 135                 140
Glu Ala Gly Met Met Leu Tyr Thr Gly Ala Thr Ala Ile Asp Leu Thr
145                 150                 155                 160
Ala Lys Val Lys Ala Lys Leu Gly Ile Lys
                165                 170

(2) INFORMATION FOR SEQ ID NO:319

(i) SEQUENCE CHARACTERISTICS:
(A) LENGTH: 828 amino acids
(B) TYPE: amino acid
(D) TOPOLOGY: linear

(ii) MOLECULE TYPE: protein

(iii) HYPOTHETICAL: YES

(vi) ORIGINAL SOURCE:

(A) ORGANISM: Porphyromonas gingivalis

(ix) FEATURE:
(A) NAME/KEY: misc_feature
(B) LOCATION 1...828

(xi) SEQUENCE DESCRIPTION: SEQ ID NO:319

Ile Met Lys Glu Ala Ile Pro Arg Lys Asn Lys Tyr Ile Lys Leu Asn
1               5                   10                  15
Gly Ile Tyr Arg Leu Ser Phe Ile Leu Leu Cys Cys Leu Leu Cys Ser
            20                  25                  30
Gln Ala Ala Met Ala Gln Gly Val Arg Val Ser Gly Tyr Val Leu Asp
        35                  40                  45
Arg Gly Glu Lys Pro Ile Pro Phe Ala Gly Val Lys Val Arg Gly Thr
    50                  55                  60
Gly Thr Gly Ala Thr Thr Asn Leu Lys Gly Tyr Tyr Glu Phe Arg Met
65                  70                  75                  80
Lys Ala Thr Thr Asp Ser Ile Thr Ile Glu Phe Ser Ser Met Gly Tyr
                85                  90                  95
Gln Gly Val Ser Arg Ser Phe Pro Ser Leu Thr Lys Asp Thr Arg Leu
            100                 105                 110
Asn Val Arg Leu Ala Glu Ala Glu Met Glu Leu Ser Ser Val Thr Val
        115                 120                 125
Gln Ala Thr Lys Arg Arg Leu Asn Thr Met Glu Arg Val Asn Thr Arg
    130                 135                 140
Asp Leu Arg Val Asn Ala Gly Pro Thr Gly Gly Val Glu Ser Leu Ile
145                 150                 155                 160
Ser Thr Tyr Ala Gly Val Thr Gln Asn Asn Glu Leu Ser Ser Gln Tyr
                165                 170                 175
Ser Val Arg Gly Gly Ser Tyr Asp Glu Asn Met Val Tyr Val Asn Gly
            180                 185                 190
Val Glu Val Tyr Arg Pro Leu Leu Val Arg Ser Ala Gln Gln Glu Gly
        195                 200                 205
Leu Ser Phe Val Asn Pro Asp Leu Thr Gln Ser Val Gln Phe Ser Ala
    210                 215                 220
Gly Gly Phe Thr Ala Asp Tyr Gly Asp Lys Met Ser Ser Val Leu Asp
225                 230                 235                 240
Ile Arg Tyr Lys Gln Pro Gln Glu Lys Glu Gly Ala Val Leu Leu Gly
                245                 250                 255
Met Leu Gln Ser Ser Ala Tyr Tyr Gly Ser Ser Ala Gly Ala Phe Ser
            260                 265                 270
Gln Ile Thr Gly Val Arg Tyr Lys Ser Ala Lys Ser Leu Leu Gly Thr
        275                 280                 285
Thr Asp Thr Lys Ala Glu Tyr Asp Pro Ile Tyr Ala Asp Gly Gln Thr
    290                 295                 300
Phe Met Thr Tyr Arg Phe Ser Pro Lys Leu Ser Val Ser Phe Leu Gly
305                 310                 315                 320
Asn Ile Ser Gln Thr Arg Tyr Lys Phe Val Pro Gln Thr Arg Glu Thr
                325                 330                 335
Ser Phe Gly Thr Leu Ser Asp Ala Lys Lys Leu Lys Ile Phe Phe Asp
            340                 345                 350
Gly Gln Glu Gln Asp Arg Phe Leu Thr Tyr Phe Gly Ala Phe Ser Met
        355                 360                 365
Asn Phe Val Pro Asp Asp Lys Gln Arg His Thr Val Thr Leu Ser Ala
    370                 375                 380
Phe Asn Ser Asn Glu Arg Glu Thr Tyr Asp Ile Gln Gly Glu Tyr Phe
385                 390                 395                 400
Leu Asn Asp Val Gln Leu Gly Ala Asp Gly Thr Ala Ser Met Ala Ser
                405                 410                 415
Gly Ser Glu Asn Ser Asn Gly Leu Gly Ile Gly Arg Asn His Glu His
            420                 425                 430
Ala Arg Asn Arg Leu Ser Tyr Arg Val Leu Asn Met Gly Tyr Arg Gly
        435                 440                 445
Glu Met Lys Leu Asn Glu Lys His Arg Leu Gln Ala Gly Val Ser Ala
    450                 455                 460
Gln Met Glu Lys Ile Ala Asp His Ile Ser Glu Trp Glu Arg Arg Asp
465                 470                 475                 480
Ser Val Gly Tyr Asn Leu Pro His Ser Glu Thr Val Leu Leu Met Tyr
                485                 490                 495
Asn Asn Leu Tyr Ala Asp Thr Gln Met Arg Gly Thr Arg Leu Ser Ala
            500                 505                 510
Phe Val Gln Asp Arg Phe Asn Phe Ser Met Gly Gly Gly Thr Phe Ser
        515                 520                 525
Leu Ile Pro Gly Ile Arg Ala Ser Trp Trp Ser Phe Asn Lys Glu Leu

530                 535                 540
Leu Val Ser Pro Arg Ile Ser Val Gly Tyr Ser Pro Glu Ser Asn Pro
545                 550                 555                 560
Ala Leu Val Leu Arg Ala Ala Ala Gly Leu Tyr Tyr Gln Ala Pro Phe
                565                 570                 575
Tyr Lys Glu Leu Arg Gln Thr His Lys Asp Ala Glu Gly Asn Asn Val
            580                 585                 590
Val Val Leu Asn Glu Lys Ile Arg Ser Gln Gly Ala Phe His Ile Leu
        595                 600                 605
Ala Gly Ala Asp Tyr Thr Phe Glu Met Gly Gly Arg Lys Tyr Lys Phe
    610                 615                 620
Thr Ala Glu Ala Tyr Tyr Lys Ser Leu Phe Asn Ile Asn Pro Tyr Ile
625                 630                 635                 640
Ile Glu Asn Val Lys Ile Arg Tyr Leu Gly Glu Asn Ile Gly Ser Gly
                645                 650                 655
Tyr Ala Ala Gly Ile Asp Leu Lys Leu Phe Gly Glu Leu Val Pro Gly
            660                 665                 670
Val Asp Ser Trp Leu Thr Ala Ser Ile Ile Lys Ala Arg Gln Lys Leu
        675                 680                 685
Asp Gly Tyr Gly Ser Leu Pro Leu Met Asn Ala Pro Thr Tyr Asn Phe
    690                 695                 700
Ser Phe Phe Leu Gln Glu Tyr Val Pro Gly Asn Lys Arg Ile Thr Ala
705                 710                 715                 720
Thr Leu Arg Ala Ala Leu Ser Gly Gly Leu Pro Gln Leu Asn Pro Ser
                725                 730                 735
Lys Gly Leu Ser Ser Pro Ala Phe Thr Ala Pro Ala Tyr Lys Arg Val
            740                 745                 750
Asp Leu Gly Val Met Tyr Lys Trp Leu Asp Pro Asp Asp Ser Phe Ala
        755                 760                 765
Gly Arg Ser Lys Trp Leu Met Gly Val Lys Gly Ala Tyr Ile Gly Ala
    770                 775                 780
Asp Leu Phe Asn Leu Phe Asp Met Thr Asn Val Asn Ser Tyr Tyr Trp
785                 790                 795                 800
Val Ser Asp Ala Tyr Gln Gln Gln Tyr Ala Val Pro Asn Tyr Leu Thr
                805                 810                 815
Arg Arg Gln Phe Asn Leu Arg Leu Leu Val Glu Phe
            820                 825

(2) INFORMATION FOR SEQ ID NO:320

(i) SEQUENCE CHARACTERISTICS:
(A) LENGTH: 679 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...679

(xi) SEQUENCE DESCRIPTION: SEQ ID NO:320

Pro Ile Pro Phe Leu Leu Asn Met Tyr Ser Gly His His Lys Ile His
1               5                   10                  15
Tyr Pro Phe Leu Ile Leu Leu Val Cys Leu Ala Phe Ala Ala Cys Lys
            20                  25                  30
Ser Val Lys Leu Lys Asp Ala Glu Lys Ala His Asp Arg Gln Glu Tyr
        35                  40                  45
Thr Lys Ala Ala Asp Met Tyr Asn Thr Leu Tyr Arg Arg Thr Arg Arg
    50                  55                  60
Lys Gln Val Glu Met Lys Ala Tyr Thr Ala Phe Arg Ser Gly Glu Asn
65                  70                  75                  80
Tyr Arg Ala Ala Gly Arg Gln Ala Lys Ala Leu Arg Gly Tyr Leu Asn
                85                  90                  95
Ala Arg Arg Tyr Gly Tyr Pro Asp Ser Val Val Leu Leu Arg Leu Ala
            100                 105                 110
Gln Thr Tyr Gln Gln Gly Gly Asn Tyr Lys Glu Ala Glu Val Leu Phe
        115                 120                 125
Arg Gly Tyr Leu Glu Ala Tyr Pro Lys Ser Tyr Phe Ala Ala Ile Gly

130                 135                 140
Leu Glu Gly Cys Leu Phe Ala Arg Gln Gln Lys Glu Tyr Pro Thr Arg
145                 150                 155                 160
Tyr Arg Ile Arg Arg Ala Ala Glu Trp Asn Ser Ala Arg Gly Asp Phe
                165                 170                 175
Gly Pro Ala Tyr Ala Pro Asp Ala Ser Ala Leu Tyr Phe Thr Ser Ser
            180                 185                 190
Arg Ser Lys Asp Asp Gly Leu Asp Asn Ser Ser Ile Thr Gly Leu Lys
        195                 200                 205
Pro Asn Asp Ile Tyr Ile Ile Lys Arg Asp Ala Gln Gly Arg Trp Gly
    210                 215                 220
Arg Pro Asp Ser Val Ser Gly Gly Ile Asn Thr Pro Trp Asp Glu Gly
225                 230                 235                 240
Val Pro Thr Ile Thr Pro Asp Gly Ser Thr Ile Tyr Tyr Thr Leu Ala
                245                 250                 255
Gln Gln Gly Ala Asp Tyr Asp Arg Thr Val Gln Ile Tyr Ser Ala Ala
            260                 265                 270
Arg Ser Gly Glu Gly Gly Trp Ser Asn Gly Ser Leu Val Asp Ile Met
        275                 280                 285
Arg Asp Ser Leu Arg Met Ala Ala His Pro Ser Met Ser Ala Ser Gly
    290                 295                 300
Asp Tyr Leu Tyr Phe Val Ser Asn Ile Gly Gly Ser Tyr Gly Gly Lys
305                 310                 315                 320
Asp Ile Tyr Arg Val Lys Val Ser Asp Arg Ser Tyr Gly Ser Pro Glu
                325                 330                 335
Asn Leu Gly Pro Asp Ile Asn Thr Pro Gly Asp Glu Met Phe Pro Phe
            340                 345                 350
Ile Asp Gly Asp Ser Thr Leu Phe Phe Ala Ser Asp Gly His Ala Gly
        355                 360                 365
Leu Gly Gly Leu Asp Ile Phe Lys Ala Thr Leu Asp Ser Thr Gly Gln
    370                 375                 380
Trp His Val Val Asn Met Gly Gln Pro Val Asn Ser Ser Ala Asp Asp
385                 390                 395                 400
Phe Gly Leu Ala Val Glu Pro Lys Gly Lys Asn Lys Glu Glu Ala Leu
                405                 410                 415
Pro Asp Asn Gly Val Lys Gly Val Phe Cys Ser Asn Arg Gly Asp Ala
            420                 425                 430
Arg Gly Trp Pro His Leu Phe His Phe Glu Leu Pro Ala Ile Tyr Thr
        435                 440                 445
Glu Ile Gln Gly Tyr Val Met Asp Arg Glu Glu Asn Pro Ile Ala Gly
    450                 455                 460
Ala Thr Val Arg Ile Val Gly Glu Arg Gly Pro Val Gly Gln Gly Phe
465                 470                 475                 480
Val Thr Thr Arg Asp Asp Gly Ser Tyr Lys Met Ser Val Gln Gly Asp
                485                 490                 495
Thr Arg Tyr Val Met Leu Ala Gly Ala Ser Gly Tyr Leu Asn Gln Tyr
            500                 505                 510
Val Glu Leu Lys Thr Asp Thr Ala Lys Gln Ser Glu Thr Tyr Tyr Val
        515                 520                 525
Asp Phe Phe Leu Ala Ser Arg Glu Lys Ala Glu Gly Leu Gln Asn Ile
    530                 535                 540
Phe Tyr Asp Phe Asp Lys Ala Thr Leu Arg Pro Glu Ser Met Lys Ser
545                 550                 555                 560
Leu Asp Glu Leu Ile Arg Ile Leu Thr Asp Asn Pro Asp Ile Arg Ile
                565                 570                 575
Glu Leu Gly Ser His Ala Asp Arg Lys Gly Pro Asp Ala Tyr Asn Leu
            580                 585                 590
Gly Leu Ser Asp Arg Arg Ala Lys Ser Val Val Asp Tyr Leu Thr Ser
        595                 600                 605
Arg Gly Ile Ala Ala Asp Arg Leu Thr Trp Lys Gly Tyr Gly Lys Ser
    610                 615                 620
Val Pro Lys Thr Val Thr Ala Lys Ile Ala Glu Arg His Asp Phe Leu
625                 630                 635                 640
Lys Glu Gly Asp Val Leu Thr Glu Glu Phe Val Ala Pro Leu Thr Glu
                645                 650                 655
Glu Gln Gln Ser Val Cys Asp Gln Leu Asn Arg Arg Thr Glu Phe Arg
            660                 665                 670
Val Ile Glu Glu Glu Leu Arg
        675

(2) INFORMATION FOR SEQ ID NO:321

    (i) SEQUENCE CHARACTERISTICS:
        (A) LENGTH: 772 amino acids
        (B) TYPE: amino acid

(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...772

(xi) SEQUENCE DESCRIPTION: SEQ ID NO:321

Pro Ala Gln Pro Lys Thr Tyr Cys Ile Arg Tyr Phe Arg Arg Glu Val
1               5                   10                  15
Ser Pro Arg Arg Gln Thr Glu Arg Thr Leu Tyr Ala Asp Lys Met Arg
            20                  25                  30
Arg His Ile Val Lys Cys Cys Pro Ser Ala Ser Ile Leu Leu Gln Glu
        35                  40                  45
Tyr Ser Thr Thr Glu Leu Phe Ile His His Phe Asn Ile Ser Ile Ile
    50                  55                  60
Met Lys Lys Phe Phe Phe Ala Leu Leu Ser Ile Gly Ile Ser Ala Gln
65                  70                  75                  80
Ala Phe Ala Lys Thr Asp Asn Val Pro Thr Asp Ser Leu Arg Val His
                85                  90                  95
Asn Leu Gln Thr Val Thr Val Tyr Ser Thr Arg Thr Ala Val Pro Leu
            100                 105                 110
Lys Lys Ile Pro Ala Lys Met Glu Leu Ile Ser Ser Arg Asn Ile Lys
        115                 120                 125
Gln Ser Gly Phe Asn Asn Met Thr Asp Ile Leu Lys Thr Gln Ser Ser
    130                 135                 140
Leu Asp Val Ile Gln Tyr Pro Gly Phe Ser Ser Asn Ile Gly Ile Arg
145                 150                 155                 160
Gly Phe Lys Pro Ser Gly Lys Tyr Val Thr Val Leu Val Asn Gly Ile
                165                 170                 175
Pro Ala Gly Thr Asp Asn Ile Ser Thr Leu Asn Thr Ser Asn Ile Glu
            180                 185                 190
Gln Ile Glu Ile Leu Lys Gly Pro Phe Ser Ser Ile Tyr Gly Thr Asn
        195                 200                 205
Ala Met Gly Gly Val Val Asn Ile Ile Thr His Lys Ser Lys Asp Lys
    210                 215                 220
Ile His Gly Asn Val Ser Leu Phe Gly Gly Ser Tyr Gln Thr Met Ala
225                 230                 235                 240
Gly Ser Phe Asn Leu Gly Gly Arg Phe Glu Asp Ile Phe Ser Phe Asp
                245                 250                 255
Leu Ser Leu Gly Leu Asp Lys Gln Asn Lys Asp Tyr Lys Thr Gly Ser
            260                 265                 270
Asn Asn Phe Leu Ser Leu Ser Lys Leu Glu Glu Ala Ile Val Asp Val
        275                 280                 285
Asn Ala Thr Lys Asn Lys Lys Met Lys Gly Ser Asp Tyr Thr Val Ala
    290                 295                 300
Thr Gly Arg Leu Arg Phe Gly Ile Asp Phe Thr Pro Glu Trp Ser Leu
305                 310                 315                 320
Asn Leu Tyr Gln Asn Val Phe Leu Gly Asp Ala Ile Pro Val Gly Gly
                325                 330                 335
Ser Ile Trp Gly Val Tyr Gly Glu Ser Lys Lys Asn Leu Asn Arg Ser
            340                 345                 350
Ser Thr Ser Phe Glu Leu Leu Gly Lys His Gly Cys His Thr Leu Gln
        355                 360                 365
Phe Ser Pro Tyr Phe Asn Ile Glu Lys Ser Glu Asn Tyr Asn Asn Ala
    370                 375                 380
Asp Pro Thr Gly Phe Ile Asn Tyr Lys Ser Asp Tyr Tyr Thr Tyr Gly
385                 390                 395                 400
Ala Leu Leu Gln Asp Lys Ile Ser Phe Gly Gly Gln Asn Ile Val Leu
                405                 410                 415
Gly Val Asp Ser Arg Asn Met Thr Met Glu Ser Glu Arg Phe Glu Gln
            420                 425                 430
Ala Gly Val Asn Thr Lys Pro Tyr Asn Pro Gly Tyr Ala Thr Asn Asn
        435                 440                 445
Ile Gly Leu Phe Gly Gln Ala Asn Phe Tyr Leu Leu Asn Asp Ala Leu
    450                 455                 460
Ser Ile Ser Ala Gly Ala Arg Ala Asp Phe Met Phe Phe Asp Leu Lys
465                 470                 475                 480

Ala Asn Glu Tyr Leu Asn Asn Glu Ala Lys Gln Glu Thr His Asn Val
                485                 490                 495
Ile Asn Pro Asn Val Gly Ile Lys Tyr Glu Phe Val Lys Gly Leu Thr
            500                 505                 510
Ala His Gly Thr Phe Gly Ser Ala Phe Ser Ala Pro Asp Ala Phe Gln
        515                 520                 525
Lys Ala Gly Gln Tyr Val Gly Pro Phe Gly Thr Thr Ile Gly Asn Pro
    530                 535                 540
Asp Leu Lys Pro Glu Lys Ser Met Thr Trp Asp Phe Gly Ile Gly Tyr
545                 550                 555                 560
Ser Asn Ala Arg Cys Gly Ile Gln Ala Asp Val Thr Leu Thr Tyr Phe
                565                 570                 575
His Thr Asp His Lys Asp Leu Ile Leu Ser Ser Pro Asp Tyr Ala Asn
            580                 585                 590
Asn Ile Thr Thr Tyr Ile Asn Ala Asp Lys Ala Arg Met Ser Gly Ile
        595                 600                 605
Glu Ala Leu Leu Ser Tyr Asp Phe Gly Ser Leu Phe Ala Asn Lys Phe
    610                 615                 620
Ser Leu Arg Ala Phe Ala Asn Ala Thr Ile Met Leu Asn Ser Glu Met
625                 630                 635                 640
Lys Lys Ser Gln Thr Asp Ala Pro Trp Ser Glu Met Tyr Tyr Val Arg
                645                 650                 655
Lys Gln Asn Ile Thr Phe Gly Ile Glu Tyr Arg Gly Lys Glu Gly Leu
            660                 665                 670
Glu Val Met Leu Asn Gly Arg Phe Met Gly Arg Arg Ile Glu Gln Asn
        675                 680                 685
Trp Tyr Ala Tyr Tyr Pro Glu Val Arg Pro Glu Leu Gln Gln Leu Leu
    690                 695                 700
Ala Ala Glu Glu Pro Glu Leu Ala Ala Gln Gly Leu Leu Arg His Pro
705                 710                 715                 720
Gln Ala Met Val Phe Asn Ala Ser Ala Tyr Tyr His Met Asn Lys Tyr
                725                 730                 735
Leu Thr Phe Gly Val Asn Leu Asn Asn Ile Leu Asp Glu Leu Tyr Thr
            740                 745                 750
Glu Lys Asp Gly Tyr His Met Pro Gly Arg Asn Ile Met Gly Lys Val
        755                 760                 765
Met Val Asn Phe
    770

(2) INFORMATION FOR SEQ ID NO:322

    (i) SEQUENCE CHARACTERISTICS:
        (A) LENGTH: 484 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...484

    (xi) SEQUENCE DESCRIPTION: SEQ ID NO:322

Gly Arg Ala Ser Ser Pro Tyr Arg Gln Met Asp Gly Ile Leu Asn Asp
1               5                   10                  15
Glu Tyr Arg Gln Ala Ser Met Asn Arg Phe Ser Asn His Trp Pro Cys
            20                  25                  30
Ile Leu Val Gly Phe Val Leu Trp Phe Val Ser Ala Ser Arg Thr Val
        35                  40                  45
Ala Gln Asn Ala Ser Glu Thr Thr Val Ser Tyr Asp Thr Asp Thr Ala
    50                  55                  60
Val Leu Ser Glu Ala Asp Val Leu Arg Ile Ala Leu Ser Glu Asn Ala
65                  70                  75                  80
Thr Val Lys Val Ala Asp Met Asp Val Arg Lys Gln Glu Tyr Ala Arg
                85                  90                  95
Arg Ala Ala Arg Ala Asp Leu Phe Pro Lys Val Asp Leu Asn Gly Val
            100                 105                 110
Tyr Ser His Thr Leu Lys Lys Gln Val Leu Tyr Ile Asp Met Pro Gly
        115                 120                 125

Phe Ser Ser Ser Glu Gly Ile Glu Met Gly Arg Thr His Asn Thr Gln
    130                 135                 140
Gly Gly Val Asn Val Ser Met Pro Leu Val Ser Ala Gln Leu Trp Lys
145                 150                 155                 160
Ser Ile Ala Met Thr Gly Glu Gln Leu Asp Leu Ala Leu Glu Lys Ala
                165                 170                 175
Arg Ser Ser Arg Ile Asp Leu Val Ala Glu Val Lys Lys Ala Tyr Leu
            180                 185                 190
Ser Val Leu Leu Ala Glu Asp Ser Tyr Gly Val Phe Lys Arg Ser Tyr
        195                 200                 205
Asp Asn Ala Leu Ala Asn Tyr Lys Asn Ile Ser Asp Lys Phe Asp Arg
    210                 215                 220
Gly Leu Val Ala Glu Tyr Asp Lys Ile Arg Ala Asn Val Gln Val Arg
225                 230                 235                 240
Asn Ile Glu Pro Asn Leu Leu Gln Ala Gln Asn Ser Val Ala Leu Ala
                245                 250                 255
Leu Trp Gln Leu Lys Val Leu Met Ser Met Glu Val Glu Thr Pro Ile
            260                 265                 270
Arg Leu Ser Gly Ser Leu Ser Asp Tyr Lys Glu Gln Val Tyr Thr Gly
        275                 280                 285
Tyr Phe Ala Ala Asp Thr Leu Ile Ser Asn Asn Ser Ser Leu Arg Gln
    290                 295                 300
Leu Asp Ile Gln Arg Arg Leu Ala Val Ser Ala Asp Lys Leu Asn Lys
305                 310                 315                 320
Tyr Ser Phe Leu Pro Thr Leu Asn Leu Gly Gly Gln Tyr Thr Tyr Ser
                325                 330                 335
Leu Asn Ser Asn Asp Ile Lys Phe Trp Gly Glu Gly Gln Arg Trp Thr
            340                 345                 350
Pro Phe Ser Thr Ile Ser Leu Ser Leu Tyr Ile Pro Ile Phe Asn Gly
        355                 360                 365
Gly Lys Arg Leu Tyr Asn Val Lys Gln Ser Ala Leu Ser Ile Arg Gln
    370                 375                 380
Ile Asp Leu Gln Arg Arg His Ile Glu Gln Ser Ile Arg Met Gly Ile
385                 390                 395                 400
Lys Asn Gln Asn Asp Arg Leu Arg Thr Cys Met Gln Arg Phe Val Ala
                405                 410                 415
Ser Glu Glu Ala Val Arg Ser Ala Glu Lys Gly Tyr Gln Ile Ala Glu
            420                 425                 430
Lys Arg Tyr Gln Thr Gly Glu Gly Thr Leu Val Glu Leu Asn Asp Ala
        435                 440                 445
Asp Val Ala Leu Leu Gln Ala Arg Leu Asn Tyr Asn Gln Ala Ile Phe
    450                 455                 460
Asp Phe Met Thr Ala Lys Ala Glu Leu Asp Lys Met Asn Gly Met Gly
465                 470                 475                 480
Ile Pro Glu Gln

(2) INFORMATION FOR SEQ ID NO:323

(i) SEQUENCE CHARACTERISTICS:
(A) LENGTH: 540 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...540

(xi) SEQUENCE DESCRIPTION: SEQ ID NO:323

Phe His Asn Phe Asp Phe Leu Asn Gly Ile Lys Leu Phe Ser Met Ala
1               5                   10                  15
Asn Asn Thr Leu Leu Ala Lys Thr Arg Arg Tyr Val Cys Leu Val Gly
            20                  25                  30
Phe Cys Trp Leu Met Ala Met Met His Leu Ser Gly Gln Glu Val Thr
        35                  40                  45
Met Trp Gly Asp Ser His Gly Val Ala Pro Asn Gln Val Arg Arg Thr
    50                  55                  60

Leu Val Lys Val Ala Leu Ser Glu Ser Leu Pro Pro Gly Ala Lys Gln
65                  70                  75                  80
Ile Arg Ile Gly Phe Ser Leu Pro Lys Glu Thr Glu Glu Lys Val Thr
                85                  90                  95
Ala Leu Tyr Leu Leu Val Ser Asp Ser Leu Ala Val Arg Asp Leu Pro
            100                 105                 110
Asp Tyr Lys Gly Arg Val Ser Tyr Asp Ser Phe Pro Ile Ser Lys Glu
        115                 120                 125
Asp Arg Thr Thr Ala Leu Ser Ala Asp Ser Val Ala Gly Arg Arg Phe
    130                 135                 140
Phe Tyr Leu Ala Ala Asp Ile Gly Pro Val Ala Ser Phe Ser Arg Ser
145                 150                 155                 160
Asp Thr Leu Thr Ala Arg Val Glu Glu Val Ala Val Asp Gly Arg Pro
                165                 170                 175
Leu Pro Leu Lys Glu Leu Ser Pro Ala Ser Arg Arg Leu Tyr Arg Gly
            180                 185                 190
Tyr Glu Ala Leu Phe Val Pro Gly Asp Gly Gly Ser Arg Asn Tyr Arg
        195                 200                 205
Ile Pro Ala Ile Leu Lys Thr Ala Asn Gly Thr Leu Ile Ala Met Ala
    210                 215                 220
Asp Arg Arg Lys Tyr Asn Gln Thr Asp Leu Pro Glu Asp Ile Asp Ile
225                 230                 235                 240
Val Met Arg Arg Ser Thr Asp Gly Gly Lys Ser Trp Ser Asp Pro Arg
                245                 250                 255
Ile Ile Val Gln Gly Glu Gly Arg Asn His Gly Phe Gly Asp Val Ala
            260                 265                 270
Leu Val Gln Thr Gln Ala Gly Lys Leu Leu Met Ile Phe Val Gly Gly
        275                 280                 285
Val Gly Leu Trp Gln Ser Thr Pro Asp Arg Pro Gln Arg Thr Tyr Ile
    290                 295                 300
Ser Glu Ser Arg Asp Glu Gly Leu Thr Trp Ser Pro Pro Arg Asp Ile
305                 310                 315                 320
Thr His Phe Ile Phe Gly Lys Asp Cys Ala Asp Pro Gly Arg Ser Arg
                325                 330                 335
Trp Leu Ala Ser Phe Cys Ala Ser Gly Gln Gly Leu Val Leu Pro Ser
            340                 345                 350
Gly Arg Ile Thr Phe Val Ala Ala Ile Arg Glu Ser Gly Gln Glu Tyr
        355                 360                 365
Val Leu Asn Asn Tyr Val Leu Tyr Ser Asp Asp Glu Gly Asp Thr Trp
    370                 375                 380
Gln Leu Ser Asp Cys Ala Tyr Arg Arg Gly Asp Glu Ala Lys Leu Ser
385                 390                 395                 400
Leu Met Pro Asp Gly Arg Val Leu Met Ser Ile Arg Asn Gln Gly Arg
                405                 410                 415
Gln Glu Ser Arg Gln Arg Phe Phe Ala Leu Ser Ser Asp Asp Gly Leu
            420                 425                 430
Thr Trp Glu Arg Ala Lys Gln Phe Glu Gly Ile His Asp Pro Gly Cys
        435                 440                 445
Asn Gly Ala Met Leu Gln Val Lys Arg Asn Gly Arg Asp Gln Val Leu
    450                 455                 460
His Ser Leu Pro Leu Gly Pro Asp Gly Arg Arg Asp Gly Ala Val Tyr
465                 470                 475                 480
Leu Phe Asp His Val Ser Gly Arg Trp Ser Ala Pro Val Val Val Asn
                485                 490                 495
Ser Gly Ser Ser Ala Tyr Ser Asp Met Thr Leu Leu Ala Asp Gly Thr
            500                 505                 510
Ile Gly Tyr Phe Val Glu Glu Gly Asp Glu Ile Ser Leu Val Phe Ile
        515                 520                 525
Arg Phe Val Leu Asp Asp Leu Phe Asp Val Arg Gln
    530                 535                 540

(2) INFORMATION FOR SEQ ID NO:324

    (i) SEQUENCE CHARACTERISTICS:
        (A) LENGTH: 293 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

(ix) FEATURE:
(A) NAME/KEY: misc_feature
(B) LOCATION 1...293

(xi) SEQUENCE DESCRIPTION: SEQ ID NO:324

Lys Ser Pro Ser Asp Ser Ala Leu Pro Ser Val Trp Ser Ala Asn Thr
1               5                   10                  15
Ile Ser Gly Leu Val Gly Gly Lys Arg Ile Thr Leu Leu Ile Leu Tyr
            20                  25                  30
Ser Met Ala Ile Arg Ser Ser Asn Ile Glu Ser Ile Gln Cys Phe Val
        35                  40                  45
Met Lys Lys Glu Lys Leu Trp Ile Ala Ile Val Ala Gly Leu Ala Phe
    50                  55                  60
Val Leu Gly Leu Tyr Ala Leu Gly Arg Ser Val Ala Gln Leu Arg Arg
65                  70                  75                  80
Ser Gln Pro Ser Val Thr Val Thr Gly Met Ala Glu Arg Asn Phe Lys
                85                  90                  95
Ser Asp Leu Ile Val Trp Thr Ala Ser Tyr Gln Leu Gln Met Met Asp
            100                 105                 110
Leu Glu Ser Ala Tyr Lys Ala Leu Lys Glu Lys Gln Ile Leu Val Ala
        115                 120                 125
Asp Tyr Leu Lys Asn Lys Gln Leu Pro Asp Ser Ser Tyr Ile Phe Ser
    130                 135                 140
Ser Val Ala Ile Ser Lys Glu Tyr Asn Tyr Tyr Tyr Asp Pro Arg Gln
145                 150                 155                 160
Glu Gln Asn Val Arg Thr Phe Ala Gly Tyr Leu Leu Ser Gln Thr Val
                165                 170                 175
Thr Val Thr Ser Gln Asp Ile Glu His Val Glu Lys Ile Ser Arg Asp
            180                 185                 190
Ile Thr Glu Leu Ile Asn Gln Gly Val Glu Ile Thr Ser Asp Arg Pro
        195                 200                 205
Ala Tyr Tyr Tyr Thr Lys Leu Asn Asp Leu Lys Val Glu Met Leu Arg
    210                 215                 220
Asn Ala Ser Glu Asp Ala Phe Asn Arg Ala Ser Val Ile Ala Glu Gly
225                 230                 235                 240
Ser Gly Ser Ser Val Gly Lys Met Leu Ser Ser Ser Met Gly Val Phe
                245                 250                 255
Gln Ile Val Gly Leu Asn Ser Asn Glu Asp Tyr Ser Trp Gly Gly Ser
            260                 265                 270
Phe Asn Thr Ser Ser Lys Met Lys Thr Ala Ser Ile Thr Val Lys Ala
        275                 280                 285
Ser Phe Ala Leu Lys
    290

(2) INFORMATION FOR SEQ ID NO:325

(i) SEQUENCE CHARACTERISTICS:
(A) LENGTH: 280 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...280

(xi) SEQUENCE DESCRIPTION: SEQ ID NO:325

Gly Lys Leu Gln Met Lys Lys Thr Ile Ala Ile Ile Ala Ser Ala Leu
1               5                   10                  15
Leu Ala Leu Gly Ala Val Gly Cys Lys Lys Asn Ala Asp Thr Thr Ala
            20                  25                  30
Val Ser Glu Lys Asp Ser Ile Ala Leu Ser Met Gly Ile Leu Tyr Gly
        35                  40                  45
Gln Asp Phe Ala Asn Gln Phe Glu Met Ser Arg Leu Gln Gly Gln Pro
    50                  55                  60
Ile Asp Ser Val Ala Phe Leu Asp Gly Phe Lys Tyr Gly Ile Asp Thr
65                  70                  75                  80

Thr Arg Phe Ser Tyr Asn Leu Gly Ala Ile Tyr Ala Ser Asn Ile Ala
                85                  90                  95
Arg Gln Leu Ala His Asp Ser Ile Asp Ile Asp Lys Phe Tyr Ala Ala
            100                 105                 110
Met Arg Ala Ala Leu Leu Lys Asp Thr Val Ser Ile Ala Met Lys Pro
        115                 120                 125
Ala Asp Ala Gln Ala Phe Met Gln Arg Ile Gln Ala Lys Lys Gln Arg
    130                 135                 140
Glu Asn Asn Met Lys Gln Phe Gly Gln Asn Ile Glu Lys Gly Asn Glu
145                 150                 155                 160
Tyr Ile Asp Thr Phe Lys Lys Glu Asp Gly Val Thr Val Thr Thr Thr
                165                 170                 175
Gly Leu Ala Tyr Lys Thr Leu Gln Glu Gly Thr Gly Ala Thr Pro Ser
            180                 185                 190
Leu Ala Asp Thr Val Arg Val Lys Tyr Val Gly Thr Leu Val Asp Gly
        195                 200                 205
Lys Glu Phe Asp Lys Asn Glu Glu Gly Ile Glu Phe Ala Val Thr Gly
    210                 215                 220
Val Ile Lys Gly Trp Thr Glu Met Leu Gln Leu Met Lys Val Gly Gln
225                 230                 235                 240
Lys Val Arg Val Val Ile Pro Gln Glu Leu Ala Tyr Gly Glu Thr Gly
                245                 250                 255
Asn Tyr Thr Ile Glu Pro Phe Ser Thr Leu Thr Phe Glu Met Glu Leu
            260                 265                 270
Ile Gly Ile Lys Pro Gly Lys Lys
        275                 280

(2) INFORMATION FOR SEQ ID NO:326

    (i) SEQUENCE CHARACTERISTICS:
        (A) LENGTH: 803 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...803

    (xi) SEQUENCE DESCRIPTION: SEQ ID NO:326

Cys Arg Lys Gln Lys Leu Ser Asn Pro Lys Arg Ser Arg Tyr Pro Cys
1               5                   10                  15
Arg Arg Ile Ser Phe Arg Gln Ser Asp Lys Asn Pro Met Lys Val Leu
            20                  25                  30
Arg Gln Val Phe Leu Pro Ile Leu Phe Val Leu Leu Thr Gly Ala Cys
        35                  40                  45
Ser Thr Thr Lys Asn Leu Pro Glu Gly Glu Gln Leu Tyr Ile Gly Met
    50                  55                  60
Gly Lys Thr Gln Ile Leu Arg Gln Asp Lys Ser His Ala Gly Gln Gln
65                  70                  75                  80
Ala Leu Thr Glu Val Glu Ser Thr Leu Lys Val Thr Pro Asn Gly Ala
                85                  90                  95
Ile Phe Gly Ser Ala Ser Ala Ser Leu Pro Lys Ile Pro Phe Gly Leu
            100                 105                 110
Trp Leu Tyr Asn Ser Phe Val Gly Asp Ser Thr Val Ile Ser Lys Trp
        115                 120                 125
Ile Phe Asp Lys Phe Ala Ala Lys Pro Val Phe Ile Ser Gln Val Lys
    130                 135                 140
Ser Asp Ser Arg Ala Lys Val Ala Thr Asn Ile Leu Arg Glu His Gly
145                 150                 155                 160
Tyr Phe Asp Ala Lys Val Lys Ser Ser Val Thr Thr Leu Lys Lys Asp
                165                 170                 175
Ser Leu Lys Ala Lys Ile Ser Tyr Thr Val Asp Met Ala Ser Pro Tyr
            180                 185                 190
His Tyr Asp Ser Ile Ile Pro Leu Pro Ile Ser Thr Phe Pro Asp Ser
        195                 200                 205
Ile Leu Ala Tyr Arg Gln Thr Pro Ser Leu Ile Arg Lys Gly Asp Gln
    210                 215                 220

Phe Asn Leu Ala Lys Leu His Glu Glu Arg Gln Thr Ile Ser Ala Leu
225                 230                 235                 240
Leu Arg Asp Asn Gly Tyr Tyr Tyr Phe Arg Pro Gln Asp Ile Ile Tyr
                245                 250                 255
Glu Ala Asp Thr Leu Leu Val Arg Gly Ala Val Cys Leu Arg Ala Lys
            260                 265                 270
Leu Ser Glu Asp Thr Pro Pro Gln Ala Met Arg Pro Trp Arg Ile Gly
        275                 280                 285
Lys Arg Thr Ala Val Leu Leu Gly Met Asn Gly Glu Ser Pro Thr Asp
    290                 295                 300
Ser Leu Glu Val Glu Asp Met Lys Val Leu Tyr Tyr Arg Lys Met Pro
305                 310                 315                 320
Val Arg Pro Lys Ile Leu Ala Lys Arg Phe Arg Phe Phe Ser Gly Asn
                325                 330                 335
Leu Tyr Arg Gln Lys Asp Asp Glu Thr Thr Arg Lys Ser Leu Ala Arg
            340                 345                 350
Leu Gly Ala Phe Ser Val Ile Asp Leu Asn Phe Leu Gln Arg Asp Ser
        355                 360                 365
Ile Ser Gly Leu Leu Asp Val Arg Leu Leu Thr Thr Leu Asp Lys Pro
    370                 375                 380
Trp Asp Ala Ser Leu Glu Thr Leu Phe Thr Ser Lys Ser Asn Asp Phe
385                 390                 395                 400
Ile Gly Pro Gly Leu Asn Phe Ala Leu Ala Arg Arg Asn Val Phe Gly
                405                 410                 415
Gly Gly Glu Asn Leu Ser Trp Asn Ile Gly Gly Ser Tyr Glu Trp Glu
            420                 425                 430
Thr Gly Asn Arg Pro Glu Asn Ser Ser Asn Arg Leu Ile Asp Ile Asn
        435                 440                 445
Ser Tyr Asn Met Asn Thr Ala Val Asn Leu Ser Phe Pro Ser Ile Val
    450                 455                 460
Phe Pro Gly Leu Leu Asp Lys Tyr Tyr Tyr Tyr Pro Thr Thr Thr Thr
465                 470                 475                 480
Phe Gln Ala Ser Ala Thr Ala Leu Asn Arg Ala His Tyr Phe Ser Met
                485                 490                 495
Tyr Ser Phe Gly Phe Ser Thr Thr Tyr Glu Phe Gln Pro Ser Lys Glu
            500                 505                 510
His Arg His Ala Ile Phe Pro Leu Lys Leu Asn Tyr Asn Leu Leu Gly
        515                 520                 525
His Gln Thr Glu Thr Phe Gln Ala Ile Thr Ala Asn Asn Pro Pro Leu
    530                 535                 540
Leu Leu Ser Leu Gln Ser Gln Phe Leu Ala Gln Met Gly Tyr Ile Tyr
545                 550                 555                 560
Thr Phe Asn Lys Ser Val Ser Glu Lys Ser Pro His His Leu Trp Met
                565                 570                 575
Gln Phe Gly Leu Ser Glu Ala Gly Asn Leu Leu Asn Leu Ile Tyr Leu
            580                 585                 590
Ala Ala Gly Lys Lys Tyr Ser Asp Thr Lys Asn Phe Val Gly Val Pro
        595                 600                 605
Phe Ser Gln Phe Ile Lys Ala Thr Gly Glu Leu Arg Tyr Ser Tyr Thr
    610                 615                 620
Ile Asp Arg Asn Gln Ser Leu Ala Thr Arg Phe Gly Thr Gly Val Ile
625                 630                 635                 640
Tyr Ser Tyr Gly Asn Met Arg Val Ala Pro Tyr Ser Glu Gln Phe Tyr
                645                 650                 655
Val Gly Gly Ala Asn Ser Ile Arg Ala Phe Thr Val Arg Ser Ile Gly
            660                 665                 670
Pro Gly Arg Phe Asn Pro Asp Ser Asp Asn Gln Tyr Ser Tyr Leu Asp
        675                 680                 685
Gln Val Gly Glu Phe Lys Leu Glu Ala Asn Val Glu Tyr Arg Gly Lys
    690                 695                 700
Leu Phe Gly Asp Leu His Ala Ala Val Phe Leu Asp Ala Gly Asn Val
705                 710                 715                 720
Trp Leu Leu Arg Glu Asp Ser Ser Arg Pro Gly Gly Ala Leu Ser Glu
                725                 730                 735
Val Gly Ser Val Ser Asn Phe Leu Asn Ser Ile Ala Leu Gly Thr Gly
            740                 745                 750
Val Gly Leu Arg Tyr Asp Leu Ala Phe Leu Val Val Arg Val Asp Val
        755                 760                 765
Gly Phe Gly Leu His Leu Pro Tyr Asn Thr Gly Lys Lys Gly Tyr Tyr
    770                 775                 780
Asn Ile Pro Arg Phe Lys Asp Ala Ile Gly Phe His Leu Ala Val Gly
785                 790                 795                 800
Tyr Pro Phe

(2) INFORMATION FOR SEQ ID NO:327

(i) SEQUENCE CHARACTERISTICS:
(A) LENGTH: 783 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...783

(xi) SEQUENCE DESCRIPTION: SEQ ID NO:327

Ser Leu Ser Pro Tyr Ile Arg Phe Pro Met Ser Ser His Ser Val Arg
1               5                   10                  15
Tyr Leu Ile Gly Ile Ala Gly Cys Leu Leu Leu Met Leu Ala Ser Ser
            20                  25                  30
Cys Ser Val Thr Arg Tyr Val Pro Asp Gly Ser Arg Leu Leu Asp Arg
        35                  40                  45
Val Thr Ile Ala Ser Glu Thr Gly Ser Ile Ala Leu Pro Glu Asp Ile
    50                  55                  60
Arg Asp Tyr Thr Leu Gln Gln Pro Asn Tyr Arg Leu Phe Gly Met Thr
65                  70                  75                  80
Arg Trp Leu Leu Arg Val Tyr Ser Ser Ser Asn Pro Asn Ser Asn Ser
                85                  90                  95
Trp Trp Asn Arg Ser Leu Arg Lys Met Gly Glu Pro Pro Val Leu Ile
            100                 105                 110
Asp Ser Val Leu Thr Asp Arg Thr Ala Asn Arg Leu Ala Lys Ala Met
        115                 120                 125
Ala Gly Asp Gly Phe Leu Asp Ala Thr Ala Arg Ala Val Val Asp Thr
    130                 135                 140
Gly Leu Tyr Lys Lys Ala Arg Ile Thr Tyr Leu Ile Gln Pro Gly Ser
145                 150                 155                 160
Arg Tyr Tyr Ile Arg Asn Met Ala Leu Asp Val Lys Asn Pro Leu Leu
                165                 170                 175
Pro Pro Val Ala Leu Gly Asn Ser Leu Pro Ser Ala Tyr Lys Val Gly
            180                 185                 190
Ile Ser Glu Gly Ser Pro Leu Ser Pro Ile Val Leu Asp Glu Glu Arg
        195                 200                 205
Lys Ala Ile Ala Arg His Met Arg Asn Asn Gly Phe Trp Lys Phe Ser
    210                 215                 220
Ala Glu Asp Val Tyr Tyr Glu Ala Asp Thr Thr Val Ser Gly Gly Ser
225                 230                 235                 240
Gly Thr Lys Ser Ala Asp Leu Lys Leu Val Val Asn Gly Ile Gly Arg
                245                 250                 255
Tyr Pro Tyr Arg Ile Gly Arg Val Phe Phe His Ala Asp Tyr Asp Pro
            260                 265                 270
Leu Glu Ser Asp Phe Arg Val Gln Glu Leu Pro Arg Ile Asp Ser Ile
        275                 280                 285
Ser Arg Gly Asp Tyr Thr Val Tyr Tyr Gly Ser Arg Gly Arg Tyr Ile
    290                 295                 300
Arg Ala Ser Ala Leu Thr Arg Ser Val Ser Val Thr Pro Gly Ala Phe
305                 310                 315                 320
Phe Cys Glu Asp Asp Val Glu Arg Ser Tyr Ile Lys Leu Asn Ala Leu
                325                 330                 335
Pro Ile Val Arg Asn Val Asn Ile Arg Phe Val Glu His Asn Gly Lys
            340                 345                 350
Asp Glu Ile Ala Leu Ala Asp Ser Ser Arg Leu Val Asp Cys Tyr Ile
        355                 360                 365
Leu Thr Val Pro Ala Lys Ser Lys Ser Phe Glu Ala Glu Val Leu Gly
    370                 375                 380
Thr Asn Ser Ala Gly Asp Phe Gly Ala Ala Leu Ser Leu Gly Phe Thr
385                 390                 395                 400
Asp Arg Asn Leu Phe Arg Gly Ala Glu Met Phe Asn Ile Lys Leu Lys
                405                 410                 415
Gly Ala Tyr Glu Ala Ile Arg Lys Gly Ser His Ser Phe Met Glu Tyr
            420                 425                 430
Gly Val Glu Ser Ser Leu Arg Phe Pro Arg Leu Leu Phe Pro Phe Ile

435                    440                    445
Ser Asp Glu Thr Arg Arg Arg Leu Arg Ala Ser Thr Glu Trp Lys Ile
    450                    455                    460
Gly Tyr Asn Tyr Gln Thr Arg Pro Glu Phe Asp Arg Val Ile Leu Ser
465                    470                    475                    480
Ala Gln Leu Asn Tyr Ser Trp Gln Thr Tyr Leu His Asn Arg Leu Arg
                485                    490                    495
His Thr Ile Arg Leu Leu Asp Val Asp Tyr Leu His Leu Pro Tyr Ile
            500                    505                    510
Asp Pro Asp Phe Ala Gln Ser Leu Pro Pro Thr Thr Ala Leu Tyr Asn
        515                    520                    525
Tyr Thr Glu Gln Phe Ile Leu Gly Ser Ala Tyr Ile Leu Asn Tyr Thr
    530                    535                    540
Thr Ala Ser Ser Met Glu Arg Thr Val Ser Asn Pro Phe Thr Ala Arg
545                    550                    555                    560
Phe Ser Ile Gln Thr Ala Gly Asn Leu Leu Gln Ala Ile Ser Tyr Leu
                565                    570                    575
Thr Asp Ser Pro Lys Asp Glu His Gly Leu Tyr Lys Met Phe Gly Leu
            580                    585                    590
His Tyr Ala Gln Phe Val Lys Leu Asp Leu Asp Leu Ala Lys Thr Val
        595                    600                    605
Leu Leu Glu Lys Asp Asn Thr Leu Ala Leu His Leu Gly Phe Gly Leu
    610                    615                    620
Ala Phe Pro Tyr Gly Asn Ala Arg His Ile Pro Phe Glu Leu Arg Tyr
625                    630                    635                    640
Phe Ala Gly Gly Ser Asn Ser Val Arg Gly Trp Ser Val Arg Thr Leu
                645                    650                    655
Gly Pro Gly Ser Met Lys Met Thr Pro Asp Lys Thr Phe Phe Asp Gln
            660                    665                    670
Met Gly Asp Ile Arg Leu Asp Leu Asn Val Glu Tyr Arg Thr Lys Leu
        675                    680                    685
Phe Trp Lys Phe Arg Ala Ala Ala Phe Val Asp Ala Gly Asn Val Trp
    690                    695                    700
Thr Ile Lys Glu Tyr Glu Asn Gln Glu Asp Gly Leu Phe Arg Phe Asp
705                    710                    715                    720
Arg Phe Tyr Lys Glu Ile Ala Leu Ala Tyr Gly Leu Gly Leu Arg Leu
                725                    730                    735
Asp Phe Asp Tyr Phe Leu Val Arg Leu Asp Ala Gly Leu Lys Ala Tyr
            740                    745                    750
Asp Pro Gln Gln Thr Gly Arg Tyr Lys Trp Ala Ile Thr Arg Pro Asn
        755                    760                    765
Leu Ser Ser Asn Phe Ala Trp His Ile Ala Val Gly Tyr Pro Phe
    770                    775                    780

(2) INFORMATION FOR SEQ ID NO:328

    (i) SEQUENCE CHARACTERISTICS:
        (A) LENGTH: 875 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...875

    (xi) SEQUENCE DESCRIPTION: SEQ ID NO:328

Val Glu Ser Lys Leu Leu Cys Leu Met Arg Lys Arg Ile Leu Gln Leu
1               5                   10                  15
Phe Leu Thr Ala Leu Leu Leu Ala Leu Gly Ser Ser Leu Ala Ile Ala
            20                  25                  30
Gln Thr Val Val Thr Gly Lys Val Ile Asp Ser Glu Thr Ser Glu Pro
        35                  40                  45
Leu Ile Gly Val Ser Val Ser Thr Gly Gln Gly Ala Ser Leu Arg Gly
    50                  55                  60
Val Thr Thr Asp Met Asp Gly Gly Phe Arg Phe Glu Val Pro Ala Lys
65                  70                  75                  80
Ser Val Leu Thr Phe Arg Cys Val Gly Tyr Ala Thr Val Thr Arg Ser

Ile Gly Arg Gly Ser Gln Glu Asp Leu Gly Thr Ile Leu Leu Asp Pro
85 90 95
Gln Ala Ile Gly Leu Asp Glu Ile Gln Val Ile Ala Ser Val Val Pro
100 105 110
Lys Asp Arg Met Thr Pro Val Pro Val Ser Asn Ile Arg Val Ala Asp
115 120 125
Ile Gln Ala Ala Ser Leu Asn Val Glu Phe Pro Glu Leu Val Lys Ser
130 135 140
Thr Pro Ser Thr Tyr Thr Thr Lys Gly Ser Gly Gly Phe Gly Asp Gly
145 150 155 160
Arg Thr Asn Val Arg Gly Phe Asp Thr Tyr Asn Phe Gly Val Leu Ile
165 170 175
Asn Gly Val Pro Val Asn Gly Met Glu Asp Gly Lys Val Tyr Trp Ser
180 185 190
Asn Trp Ser Gly Leu Met Asn Gln Ala Ser Thr Ile Gln Ile Gln Arg
195 200 205
Gly Leu Gly Ala Ser Lys Leu Gly Ile Ser Ser Val Gly Gly Thr Met
210 215 220
Asn Ile Ile Thr Lys Thr Thr Asp Ala Asn Thr Gly Gly Ser Ala Tyr
225 230 235 240
Val Gly Met Gly Asn Asp Gly Leu His Lys Glu Ser Phe Ser Ile Ser
245 250 255
Thr Gly Met Asn Asp Gly Trp Ala Ile Thr Ile Ala Gly Ser His Met
260 265 270
Thr Gly Leu Gly Tyr Val Lys Gly Leu Lys Gly Arg Ala Phe Ser Tyr
275 280 285
Phe Phe Asn Val Ser Lys Lys Phe Asn Glu Arg His Thr Leu Ser Leu
290 295 300
Thr Gly Phe Gly Ala Pro Gln Trp His Asn Gln Arg Ser Ser Lys Tyr
305 310 315 320
Ser Val Ala Asp Tyr Asp Lys Tyr Gly Ile Arg His Asn Gln Ser Phe
325 330 335
Gly Tyr Leu Arg Gly Glu Leu Thr Pro Thr Ala Tyr Ala Tyr Asn Thr
340 345 350
Tyr His Lys Pro Gln Phe Ser Leu Asn His Phe Trp Lys Met Asp Glu
355 360 365
Asn Thr Ser Leu Tyr Thr Ala Unk Tyr Ala Ser Leu Ala Thr Gly Gly
370 375 380
Gly Arg Arg Ala Tyr Gly Lys Asn Ser Lys Trp Val Leu Ile Asn Tyr
385 390 395 400
Asn Thr Gly Gln Pro Tyr Glu Gln Thr Lys Val Thr Pro Asp Gly Leu
405 410 415
Ile Asp Tyr Asp Ala Val Leu Ala Ala Asn Ala Ala Ala Ser Asn Gly
420 425 430
Ser Glu Ala Ile Phe Ala Leu Gly Ser Asn Ser His Lys Trp Phe Gly
435 440 445
Leu Leu Ser Ser Phe Lys Lys Lys Leu Asn Ser Ser Leu Thr Leu Thr
450 455 460
Ala Gly Tyr Asp Gly Arg Tyr Tyr Arg Gly Asp His Tyr Asp Lys Ile
465 470 475 480
Thr Asp Leu Leu Gly Gly Ser Tyr Tyr Ile Glu Asp Pro Lys Thr Lys
485 490 495
Leu Ala Tyr His Ala Glu Gly Gln Gln Leu Lys Val Gly Asp Ile Val
500 505 510
Asn Arg Asp Tyr Thr Gly Glu Ile Met Trp His Gly Leu Phe Ala Gln
515 520 525
Met Glu His Ser Ser Glu Trp Ile Asp Ala Phe Val Ser Gly Ser Ile
530 535 540
Asn Tyr Glu Leu Tyr Arg Asn His Asn Tyr Gly Gly Ser Lys Ser Thr
545 550 555 560
Gly Tyr Leu Pro Gly Val Ser Pro Trp Lys Ser Phe Leu Pro Trp Ser
565 570 575
Gly Lys Ala Gly Leu Ser Tyr Lys Phe Ala Gln Gly His Asn Val Phe
580 585 590
Ala Asn Gly Gly Phe Phe Thr Arg Ala Pro Leu Phe Gly Asn Ile Tyr
595 600 605
Ala Ala Gly Ala Ile Ile Pro Asn Asp Lys Ala Asn Met Glu Lys Val
610 615 620
Leu Thr Gly Glu Val Gly Tyr Gly Phe Thr Asn His Lys Asn Phe Glu
625 630 635 640
Phe Asn Ile Asn Gly Tyr Tyr Thr Lys Trp Met Asp Arg Val Thr Ser
645 650 655
Lys Arg Ile Gly Asn Glu Tyr Val Tyr Leu Asn Gly Val Asp Ala Val
660 665 670
675 680 685

His Cys Gly Val Glu Ala Glu Val Ser Tyr Arg Pro Ile Arg Gln Ile
    690                 695                 700
Asp Leu Arg Gly Met Phe Ser Leu Gly Asp Trp Thr Trp Gln Asn Asn
705                 710                 715                 720
Val Ser Tyr Thr Ser Tyr Asp Glu Ala Gly Asn Glu Thr Gly Gln Asp
                725                 730                 735
Ile Thr Tyr Ile Lys Gly Leu His Val Gly Asp Ala Ala Gln Met Thr
            740                 745                 750
Ala Ala Val Ser Ala Asp Ile Glu Leu Phe Lys Gly Phe His Val Ile
        755                 760                 765
Gly Lys Tyr Asn Phe Leu Gly Lys Asn Tyr Ala Gly Phe Asn Pro Ala
    770                 775                 780
Thr Arg Asn Ala Gln Gln Tyr Glu Ala Asp Gly Lys Glu Ile Val Glu
785                 790                 795                 800
Ser Trp Lys Leu Pro Asp Val Gly Leu Phe Asp Leu Ser Ala Ser Tyr
                805                 810                 815
Asn Phe Lys Leu Gly Ser Leu Ser Thr Thr Phe Tyr Phe Asn Met Asp
            820                 825                 830
Asn Val Ala Asp Lys Arg Tyr Val Ser Asp Ala Asp Asp Asn Ile Ile
        835                 840                 845
Gly Lys Lys His Asp Glu Ala Ser Ala Leu Val Trp Tyr Gly Phe Gly
    850                 855                 860
Arg Thr Trp Ser Thr Gly Ile Arg Val Asn Phe
865                 870                 875

(2) INFORMATION FOR SEQ ID NO:329

    (i) SEQUENCE CHARACTERISTICS:
        (A) LENGTH: 460 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...460

    (xi) SEQUENCE DESCRIPTION: SEQ ID NO:329

Asn Arg Cys Asn Pro Ser Arg Gln Tyr Phe His Leu Ser Gln Asn Asp
1               5                   10                  15
Val Gly Arg Ser Leu Arg Ala Phe Leu Ile His Leu Ser Met Lys Phe
            20                  25                  30
Ser Ile Arg Leu Phe Leu Cys Ile Ile Phe Leu Leu Ser Ala Phe Ile
        35                  40                  45
Leu Pro Ala Leu Gly Gln Lys Ser Lys Gln Val Gln Arg Leu Glu Lys
    50                  55                  60
Gln Arg Lys Glu Ala Leu Lys Ala Ile Glu Lys Thr Asp Arg Glu Leu
65                  70                  75                  80
Arg Asn Thr Lys Lys Asp Lys Gln Asp Lys Gln Lys His Leu Asn Leu
                85                  90                  95
Leu Asn Lys Gln Val Ala Gln Arg Lys Gln Met Val Gln Leu Leu Asp
            100                 105                 110
Asn Glu Val Lys Glu Leu Gln Ser Asp Ile Asp Ser Met Thr Gly Val
        115                 120                 125
Cys His Gln Leu Ser Val Glu Glu Lys Ala Arg Ser Asp Glu Tyr Ala
    130                 135                 140
Gln Ala Leu Gln Ser Met Gln Lys Arg Lys Arg Ser Leu Asp Arg Ile
145                 150                 155                 160
Leu Phe Ile Ser Ser Ala Lys Ser Phe Asp Glu Gly Met Arg Arg Met
                165                 170                 175
Arg Phe Leu Glu Gln Tyr Ala Ser Ala Tyr Lys Leu Ala Ser Val Arg
            180                 185                 190
Leu Arg Asp Thr Arg Ser Lys Leu Glu Thr Glu Arg Ala Thr Val Glu
        195                 200                 205
Asp Ala Lys Lys Glu Lys Gly His Leu Leu Val Ile Arg Glu Glu Glu
    210                 215                 220
Lys Lys Lys Leu Glu Gly Gln Gln Ala Glu Gln Arg Arg Gln Val Gln
225                 230                 235                 240

Ala Leu Gly Ala Lys Gln Lys Asp Leu Glu Ala Gln Leu Arg Lys Gln
                245                 250                 255
Lys Lys Gln Ala Glu Ala Leu Asn Arg Lys Ile Glu Lys Gln Ile Ala
            260                 265                 270
Lys Glu Ile Glu Ala Ala Glu Arg Arg Ala Arg Glu Glu Arg Glu Arg
        275                 280                 285
Leu Ala Arg Glu Ala Lys Ala Lys Gly Lys Pro Val Pro Ala Glu Pro
    290                 295                 300
Glu Arg Lys Ala Glu Thr Lys Gly Gly Tyr Ala Met Asp Ala Ser Glu
305                 310                 315                 320
Arg Ala Leu Ser Gly Ser Phe Ala Gln Asn Lys Gly Arg Leu Pro Gly
                325                 330                 335
Pro Val Arg Gly Arg Tyr Arg Ile Val Ser Asp Phe Gly Val His Gln
            340                 345                 350
His Ser Glu Leu Lys Lys Val Gln Val Asn Asn Gly Gly Ile Asp Ile
        355                 360                 365
Ala Val Ala Thr Gly Ser Asp Ala Thr Ser Val Phe Asp Gly Val Val
    370                 375                 380
Ser Ser Val Phe Val Ile Pro Gly Tyr Asn Ser Ala Val Met Val Arg
385                 390                 395                 400
His Gly Asn Tyr Ile Thr Val Tyr Ala Asn Leu Ser Lys Val Tyr Val
                405                 410                 415
Asn Ser Gly Thr Arg Val Lys Thr Gly Gln Ala Leu Gly Arg Ala Tyr
            420                 425                 430
Thr Asp Pro Ser Asn Asn Gln Thr Ile Ile His Phe Glu Ile Trp Lys
        435                 440                 445
Glu Arg Ser Lys Gln Asn Pro Arg Leu Trp Leu Arg
    450                 455                 460

(2) INFORMATION FOR SEQ ID NO:330

(i) SEQUENCE CHARACTERISTICS:
(A) LENGTH: 342 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...342

(xi) SEQUENCE DESCRIPTION: SEQ ID NO:330

Ser Phe Tyr Gln Glu Ile Asp Arg Leu Met Lys Lys Tyr Leu Leu Tyr
1               5                   10                  15
Ala Ser Leu Leu Thr Ser Val Leu Leu Phe Ser Cys Ser Lys Asn Asn
            20                  25                  30
Pro Asn Glu Pro Val Glu Asp Arg Ser Ile Glu Ile Ser Ile Arg Val
        35                  40                  45
Asp Asp Phe Thr Lys Thr Gly Glu Ala Val Arg Tyr Glu Arg Asn Gln
    50                  55                  60
Gly Ser Ala Ala Glu Arg Leu Ile Thr Asn Leu Tyr Leu Leu Leu Phe
65                  70                  75                  80
Asp Gln Ser Gly Ala Asn Pro Ala Lys Tyr Tyr Ile Thr Gly Asn Thr
                85                  90                  95
Phe Thr Gly Gly Thr Trp Leu Pro Asp Asp Met Lys Val Lys Leu Asp
            100                 105                 110
Met Thr Gln Ser Glu Ala Gly Glu Arg Lys Val Tyr Val Val Ala Asn
        115                 120                 125
Val Asp Asn Ala Val Lys Thr Ala Leu Asp Ala Val Ala Asn Glu Ser
    130                 135                 140
Asp Leu Gln Thr Val Lys Arg Thr Thr Ala Met Pro Trp Ser Thr Asp
145                 150                 155                 160
Ile Ala Ser Pro Phe Leu Met Ser Gly Asn Lys Thr His Asp Phe Leu
                165                 170                 175
Ala Asn Arg Leu Leu Asp Asn Val Pro Leu Val Arg Ala Ile Ala Lys
            180                 185                 190
Val Glu Leu Asn Ile Ser Leu Ser Glu Lys Phe Gln Ile Val Pro Ile
        195                 200                 205

Ile Val Asn Gly Ser Leu Ser Glu Phe Lys Phe Arg Tyr Val Asn Phe
    210                 215                 220
Asp Lys Glu Thr Tyr Val Val Lys Pro Thr Thr Lys Pro Asp Asn Leu
225                 230                 235                 240
Ile Ser Ser Ala Asn Gly Val Trp Pro Gln Ile Thr Asp Trp Thr Val
                245                 250                 255
Trp Gly Ala Ser Leu Asn Thr Ser Pro Ala Pro Asp Ala Gly Thr Gly
            260                 265                 270
Tyr Thr Leu Asp Ala Asn Gly Lys Val Thr Ala Leu Arg Ile Val Thr
        275                 280                 285
Tyr Leu Asn Glu Arg Asp Ser Lys Gly Ala Thr Val Glu Val Ala Leu
    290                 295                 300
Pro Arg Val Asp Asp Gly Thr Leu Pro Pro Pro Glu Phe Gly Pro Glu
305                 310                 315                 320
Leu Tyr Arg Leu Pro Leu Pro Asp Lys Ile Leu Arg Asn His Trp Tyr
                325                 330                 335
Lys Tyr Glu Val Glu Ile
            340

(2) INFORMATION FOR SEQ ID NO:331

     (i) SEQUENCE CHARACTERISTICS:
          (A) LENGTH: 329 amino acids
          (B) TYPE: amino acid
          (D) TOPOLOGY: linear

     (ii) MOLECULE TYPE: protein

    (iii) HYPOTHETICAL: YES

     (vi) ORIGINAL SOURCE:
          (A) ORGANISM: Porphyromonas gingivalis

     (ix) FEATURE:
          (A) NAME/KEY: misc_feature
          (B) LOCATION 1...329

     (xi) SEQUENCE DESCRIPTION: SEQ ID NO:331

Asn Leu Arg Thr Gln Pro Phe Phe Leu Val Asp Leu Gln Ser Met Ile
1               5                   10                  15
Arg Thr Ile Leu Ser Arg Tyr Val Ser Ser Asn Phe Trp Ser Arg Gly
            20                  25                  30
Ala Thr Phe Phe Phe Thr Ile Phe Pro Ala Phe Ile Leu Ala Ala Thr
        35                  40                  45
Ala Leu Pro Ala Cys Gly Gly Gly Thr Ala Ser Gly Ser Asp Arg Thr
    50                  55                  60
Leu Ala Val Thr Ile Glu Pro Gln Lys Tyr Phe Ile Glu Ser Ile Ala
65                  70                  75                  80
Asp Lys Ser Val Gln Val Val Ala Leu Val Pro Ala Gly Ser Asn Pro
                85                  90                  95
Glu Glu Tyr Asp Pro Ser Pro Thr Val Met Lys Arg Leu Ser Glu Ala
            100                 105                 110
Asp Ala Tyr Phe Tyr Ile Gly Gly Leu Gly Phe Glu Gln Arg Asn Leu
        115                 120                 125
Ala Ala Ile Arg Asp Asn Asn Pro Lys Leu Pro Leu Phe Glu Met Gly
    130                 135                 140
Lys Ala Leu Ala Asp Ala Gly Ser Ala Asp Leu His Gly Ser Cys Thr
145                 150                 155                 160
Asp His Ser His Thr Asp Leu His Ala His Asp Pro His Tyr Trp Ser
                165                 170                 175
Ser Val Val Gly Ala Lys Ala Leu Ser Arg Ala Ala Tyr Asp Ala Leu
            180                 185                 190
Val Glu Leu Tyr Pro Asn Glu Lys Asp Lys Trp Asp Lys Gly His Asp
        195                 200                 205
Arg Leu Asn Gly Arg Ile Asp Ser Val Lys Arg Leu Val Asp Thr Met
    210                 215                 220
Phe Ala Asn Gly Lys Ala Asp Lys Ala Phe Val Ile Tyr His Pro Ser
225                 230                 235                 240
Leu Ser Phe Phe Ala Gln Glu Phe Gly Leu Arg Gln Ile Val Ile Glu
                245                 250                 255
Glu Asp Gly Lys Glu Pro Thr Ala Ala His Leu Arg Arg Val Ile Asp
            260                 265                 270
Gln Ala Arg Ala Asp Gly Val Arg Ile Val Phe Ile Gln Pro Glu Phe
        275                 280                 285

Glu Thr Arg Gln Ala Glu Asp Ile Ala Arg Glu Ile Gly Ala Arg Pro
    290                 295                 300
Val Arg Ile Asn Pro Leu Arg Ser Ser Trp Glu Glu Glu Ile Leu His
305                 310                 315                 320
Ile Ala Arg Ala Leu Ala His Glu Arg
                325

(2) INFORMATION FOR SEQ ID NO:332

    (i) SEQUENCE CHARACTERISTICS:
        (A) LENGTH: 878 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...878

    (xi) SEQUENCE DESCRIPTION: SEQ ID NO:332

Ala Asp Ser Ile Arg Tyr Pro Leu Tyr Phe Phe Gly Arg Asn Arg Lys
1               5                   10                  15
Lys Cys Phe Arg Glu Pro Ile Pro Thr Leu Tyr Asn Lys Asn Met Ile
            20                  25                  30
Gly Lys Lys Ile Phe Phe Ile Leu Leu Ala Leu Ile Ala Phe Ser Gly
        35                  40                  45
Leu Asn Ala Ala Thr Asp Thr Glu Phe Lys Tyr Pro Thr Asp Ala Asn
    50                  55                  60
Ile Ile Gly His Val Lys Asp Ser Lys Thr Gly Glu His Leu Val Gly
65                  70                  75                  80
Ile Thr Ile Ala Ile Lys Gly Thr Thr Phe Gly Thr Ser Thr Asp Ala
                85                  90                  95
Thr Gly His Tyr Tyr Leu Arg Asn Leu Arg Pro Gly Glu Ile Thr Leu
            100                 105                 110
Ile Met Arg Gly Met Gly Tyr Lys Ser Gln Glu Arg Val Val Arg Val
        115                 120                 125
Glu Lys Asp Lys Thr Ile Glu Val Asn Phe Glu Ala Glu Glu Asp Ala
    130                 135                 140
Ile Asn Leu Asp Glu Val Val Ile Ser Ala Asn Arg Glu Leu Thr Leu
145                 150                 155                 160
Arg Arg Leu Ala Pro Thr Leu Val Asn Val Leu Asn Glu Lys Val Phe
                165                 170                 175
Ser Gln Val Asn Ala Ser Asn Leu Ala Gln Gly Leu Ser Phe Gln Pro
            180                 185                 190
Gly Val Arg Val Glu Asn Asn Cys Gln Asn Cys Gly Phe Asn Gln Val
        195                 200                 205
Arg Ile Asn Gly Leu Asp Gly Arg Tyr Ala Gln Ile Leu Ile Asp Ser
    210                 215                 220
Arg Pro Ile Met Ser Ala Leu Ala Gly Val Tyr Gly Leu Glu Gln Ile
225                 230                 235                 240
Pro Ala Asn Met Ile Glu Arg Val Glu Val Val Arg Gly Gly Gly Ser
                245                 250                 255
Ala Leu Tyr Gly Ser Ser Ala Ile Ala Gly Val Val Asn Ile Ile Thr
            260                 265                 270
Lys Glu Pro Ser His Asn Ser Phe Thr Phe Asn Glu Ser Leu Ser Phe
        275                 280                 285
Thr Gly Phe Ser Lys Leu Asp Asn Asn Thr Asn Phe Asn Ala Ser Ile
    290                 295                 300
Val Ser Asp Asp Asn Arg Ala Gly Ala Met Val Phe Gly Gln Ala Arg
305                 310                 315                 320
Tyr Arg Asn His Trp Asp Ala Asn Asn Asp Gly Tyr Ser Glu Leu Gly
                325                 330                 335
Lys Ile Asp Ala Arg Ser Leu Gly Ala His Ser Tyr Leu Arg Leu Ser
            340                 345                 350
Asp Tyr Ser Lys Leu Thr Gly Glu Phe His Thr Ile Ser Glu Phe Arg
        355                 360                 365
Arg Gly Gly Asp Arg Ile Asp Leu Pro Pro His Val Val Gly Val Ala
    370                 375                 380

Glu Gln Thr Asp His Ser Val Phe Ser Gly Asn Leu Lys Tyr Asp Leu
385                 390                 395                 400
Phe Ser Ser Asn Tyr Lys His His Phe Gln Ala Tyr Thr Ser Gly Gln
                405                 410                 415
Ile Val Asn Arg Lys Ser Tyr Tyr Gly Gly Ile Gly Glu Ile Asp Val
            420                 425                 430
Asn Gly His Pro Gly Gly Thr Glu Gly Tyr Pro Ile Pro Gln Asp Gln
        435                 440                 445
Tyr Gly Asn Asn Tyr Gly Val Thr Lys Gly Lys Thr Tyr Met Gly Gly
    450                 455                 460
Ile Gln Tyr Ser Tyr Asp Leu Asp Lys Phe Leu Leu Met Pro Ser Gln
465                 470                 475                 480
Leu Leu Phe Gly Ala Glu Tyr Thr Arg Asp Glu Leu Asn Asp Val Met
                485                 490                 495
Pro Ile Leu Ser Trp Gln Thr Gly Glu Asp Ala Asn Gly Asn Thr Ile
            500                 505                 510
Pro Leu Tyr Pro Glu Leu Asp Gln Asn Ile Asn Asn Tyr Ser Leu Phe
        515                 520                 525
Gly Gln Asn Glu Trp Lys Asn Asp Arg Trp Ser Ile Leu Val Gly Ala
    530                 535                 540
Arg Leu Asp Lys His Ser Glu Val Lys Asp Met Ile Leu Ser Pro Arg
545                 550                 555                 560
Thr Thr Leu Arg Phe Asn Val Asn Pro Asp Ile Asn Leu Arg Ala Thr
                565                 570                 575
Tyr Ala Lys Gly Phe Arg Ala Pro Gln Val Phe Asp Glu Asp Leu His
            580                 585                 590
Val Gly Val Val Gly Gly Glu Ala Gln Lys Val Phe Asn Asp Pro Asn
        595                 600                 605
Leu Lys Pro Glu Ile Ser His Ala Phe Ser Leu Ser Ala Asp Met Tyr
    610                 615                 620
His Arg Phe Gly Asn Val Gln Thr Asn Phe Leu Val Glu Gly Phe Tyr
625                 630                 635                 640
Thr Arg Leu Leu Asp Val Phe Thr Asn Glu Glu Gln Pro Asp Gln His
                645                 650                 655
Asp Gly Ile Lys Arg Tyr Thr Arg Ile Asn Gly Ser Gly Ala Lys Val
            660                 665                 670
Phe Gly Leu Asn Leu Glu Gly Lys Val Ala Tyr Lys Ser Phe Gln Leu
        675                 680                 685
Gln Ala Gly Leu Thr Leu Ala Ser Asn Lys Tyr Asp Glu Ala Gln Glu
    690                 695                 700
Trp Gly Leu Asn Thr Val Lys Asp Thr Asn Gly Ala Phe Val Thr Glu
705                 710                 715                 720
Ala Asn Ala Asn Gly Gln Gln Glu Tyr Lys Asn Glu Ser Met Thr Asp
                725                 730                 735
Thr Gln Ile Thr Arg Thr Pro Ser Val Tyr Gly Tyr Phe Thr Leu Ala
            740                 745                 750
Tyr Asn Pro Ala His Ser Trp Asn Ile Ala Leu Thr Gly Ala Tyr Thr
        755                 760                 765
Gly Gln Met Tyr Val Pro His Ala Ile Glu Tyr Gly Val Lys Ser Ala
    770                 775                 780
Glu Leu Asp Ile Met Gln Asn Asn Pro Glu Ile Thr Asp Glu Thr Gly
785                 790                 795                 800
Lys Ala Pro Arg Ile Asp Glu Leu Lys Lys Thr Pro Ala Phe Phe Asp
                805                 810                 815
Leu Gly Leu Lys Val Gly Tyr Asp Phe His Val Phe Gln Ala Thr Glu
            820                 825                 830
Val Gln Leu Tyr Val Gly Met Asn Asn Ile Phe Asn Ser Phe Gln Lys
        835                 840                 845
Asp Phe Asp Arg Gly Ala Ala Arg Asp Ser Gly Tyr Ile Tyr Gly Pro
    850                 855                 860
Thr Gln Pro Arg Thr Gly Tyr Met Gly Leu Val Val Lys Phe
865                 870                 875

(2) INFORMATION FOR SEQ ID NO:333

    (i) SEQUENCE CHARACTERISTICS:
        (A) LENGTH: 206 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:

(A) ORGANISM: Porphyromonas gingivalis

(ix) FEATURE:
(A) NAME/KEY: misc_feature
(B) LOCATION 1...206

(xi) SEQUENCE DESCRIPTION: SEQ ID NO:333

Lys Gln Ile Val Met Thr Val Lys Arg Ala Val Arg Ile Ala Leu Leu
1               5                   10                  15
Thr Leu Ile Gly Ile Leu Phe Ser Ser Pro Ser Leu Val Arg Ala Gln
            20                  25                  30
Ser Leu Phe Ser Thr Glu His Val Leu Gln Leu Tyr Asn Lys Ile Leu
        35                  40                  45
Tyr Gly Glu Ser Ala Ala Asp Thr Val Ala Glu Lys Thr Ala Gly Glu
    50                  55                  60
Ser Ala Phe Pro Phe Ile Asp Lys Leu Ile Asn Leu Gly Arg Thr Phe
65                  70                  75                  80
Leu Gly Lys Pro Tyr Arg Tyr Arg Gly Pro Ser Pro Trp Pro Met Asp
                85                  90                  95
Cys Ser Gly Tyr Val Ser Tyr Leu Tyr Ser Lys Phe Asp Ile Lys Leu
            100                 105                 110
Pro Arg Gly Ala Ala Ala Gln Ser Gln Tyr Thr Asn Pro Ile Glu Arg
        115                 120                 125
Glu Asp Val Arg Pro Gly Asp Leu Leu Phe Phe Lys Gly Arg Asn Ala
    130                 135                 140
Arg Ser Asn Arg Ile Gly His Val Ala Leu Val Val Ser Val Asp Glu
145                 150                 155                 160
Asp Asp Ile Thr Met Met His Ser Arg Asn Ser Arg Gly Ile Val Ile
                165                 170                 175
Glu Lys Leu Asn Arg Ser Ala Tyr Phe Ser Arg Arg Leu Val Ser Tyr
            180                 185                 190
Gly Arg Val Pro Gly Ala Lys Arg Val Ile Pro Arg Lys Ser
        195                 200                 205

(2) INFORMATION FOR SEQ ID NO:334

(i) SEQUENCE CHARACTERISTICS:
(A) LENGTH: 467 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...467

(xi) SEQUENCE DESCRIPTION: SEQ ID NO:334

Lys Gly Thr Trp Asn Arg Lys Asn Arg Arg Glu Glu Met Lys Arg Thr
1               5                   10                  15
Ile Leu Leu Thr Ala Leu Thr Val Leu Ser Ser Leu Ser Leu Leu Arg
            20                  25                  30
Ala Gln Asn Glu Ser Glu Ala Ser Thr Asn Pro Met Ser Gly Leu Ser
        35                  40                  45
Leu Glu Asp Cys Ile Arg Ile Ala Lys Glu Arg Asn Leu Asn Leu Arg
    50                  55                  60
Arg Gln Glu Ile Glu Gln Glu Asn Arg Ile Ile Ser Leu Asp Ala Ala
65                  70                  75                  80
Arg His Ser Phe Leu Pro Ser Val Asn Ala Gly Ile Gly His Asn Tyr
                85                  90                  95
Ser Phe Gly Arg Ser Lys Asp Lys Thr Gly Val Thr Val Asp Arg Ser
            100                 105                 110
Ser Met Asn Thr Asn Leu Ser Ile Gly Ala Ser Val Glu Val Phe Ser
        115                 120                 125
Gly Thr Arg Arg Leu His Asp Leu Lys Gln Gln Lys Tyr Asn Val Glu
    130                 135                 140
Asp Gly Ile Ala Arg Leu Gln Lys Ala Arg Glu Asp Leu Ser Leu Gln
145                 150                 155                 160

Ile Ala Ala Leu Tyr Ile Asn Leu Leu Phe Arg Gln Glu Met Thr Arg
                165                 170                 175
Thr Ala Glu Thr Gln Leu Ala Leu Ile Arg Glu Gln Arg Asn Arg Thr
            180                 185                 190
Ala Glu Met Val Arg Val Gly Lys Trp Ala Glu Gly Lys Leu Leu Asp
        195                 200                 205
Ile Asn Ala Gln Met Ala Lys Asp Glu Gln Leu Leu Val Gln Tyr Arg
    210                 215                 220
Ser Glu Glu Glu Leu Ala Arg Leu Asp Leu Gly Gln Ala Leu Glu Leu
225                 230                 235                 240
Glu His Pro Glu Ser Ile Ala Val Lys Ala Pro Asp Thr Asp Val Leu
                245                 250                 255
Val Ala Glu Arg Leu Gly Ser Leu Leu Ala Pro Glu Glu Ile Tyr Arg
            260                 265                 270
Thr Ala Leu Gly Leu Lys Pro Ala Leu His Ser Ser Glu Leu Gln Ile
        275                 280                 285
Ala Ser Ala Arg Glu Gly Leu Ala Ser Ala Arg Ala Ala Tyr Phe Pro
    290                 295                 300
Thr Leu Ser Leu Ser Ala Gly Tyr Ser Asn Gly Tyr Phe Arg Asp Leu
305                 310                 315                 320
Gly Lys Glu Tyr Ala Ala Ile Asn Pro Ser Phe Ser Glu Gln Trp Lys
                325                 330                 335
Asn Asn Gly Ser Tyr Ser Ile Gly Leu Ser Leu Asn Ile Pro Ile Phe
            340                 345                 350
Ser Ala Met Gln Thr Gln Asp Arg Val Arg Ser Ser Arg Leu Gln Ile
        355                 360                 365
Arg Ser Ser Glu Leu Arg Leu Val Glu Glu Lys Lys Ala Leu Tyr Lys
    370                 375                 380
Glu Ile Arg Gln Ala Tyr Ser Asn Ala Val Ala Ala Asp Lys Ala Ile
385                 390                 395                 400
Ala Ala Ala Glu Asn Ser Lys Ala Ala Thr Leu Lys Ala Tyr Glu Tyr
                405                 410                 415
Ala Arg Asp Ser Phe Glu Ala Gly Arg Leu Ser Ala Tyr Glu Tyr Ala
            420                 425                 430
Glu Ala Lys Thr Lys Tyr Ala Leu Ser Gln Val Glu Glu Leu Arg Ala
        435                 440                 445
Lys Tyr Asp Phe Ile Tyr Lys Ala Lys Val Leu Asp Phe Tyr Gln Gly
    450                 455                 460
Lys Asp Phe
465

(2) INFORMATION FOR SEQ ID NO:335

(i) SEQUENCE CHARACTERISTICS:
(A) LENGTH: 451 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...451

(xi) SEQUENCE DESCRIPTION: SEQ ID NO:335

Lys Thr Ser Tyr Arg Asn Asn Met Arg Phe Gln His Tyr Leu Ile Cys
1               5                   10                  15
Thr Ala Ala Val Ala Ala Leu Ala Ala Asn Pro Leu Thr Gly Gln Ser
            20                  25                  30
Asn Met Thr Leu Glu Glu Cys Ile Asp Tyr Ala Arg Arg His Ser Ser
        35                  40                  45
Ala Val Ala Leu Ser Ala Ala Glu Leu Glu Gln Ser Lys Ala Asp Tyr
    50                  55                  60
Leu Gln Ala Val Gly Asn Phe Leu Pro Arg Val Ser Ala Gly Thr Gly
65                  70                  75                  80
Ala Ser Trp Asn Phe Gly Arg Gly Leu Asp Ala Glu Thr Asn Thr Tyr
                85                  90                  95
Thr Asp Ile Asn Ser Phe Asn Asn Ser Tyr Ser Ile His Ala Thr Met
            100                 105                 110

Thr Leu Phe Asp Gly Leu Gln Ser Val Tyr Arg Leu Arg Met Ala His
        115                 120                 125
Ala Arg Arg Glu Ala Ser Arg Leu Ser Val Arg Glu Gln Gln Glu Leu
    130                 135                 140
Ala Ala Leu Gly Thr Thr Glu Ala Tyr Tyr Asp Leu Val Tyr Ala Arg
145                 150                 155                 160
Gln Met Gln Glu Leu Ala Met Gln Lys Tyr Glu Glu Ser Ser Arg Leu
                165                 170                 175
His Arg Gln Thr Ala Arg Met Glu Glu Leu Gly Met Lys Ser Arg Pro
            180                 185                 190
Asp Val Leu Glu Met Gln Ser Arg Met Ala Gly Asp Arg Leu Ala Leu
        195                 200                 205
Thr Gln Ala Asp Asn Gln Cys Ile Ile Ala Leu Ile Arg Leu Lys Glu
    210                 215                 220
Lys Met Asn Phe Pro Ile Asp Asp Glu Leu Val Val Asp Asp Met Pro
225                 230                 235                 240
Ala Asp Ser Leu Ser Ala Asp Met Ala Glu Ser Asp Ser Ser Ala Gly
                245                 250                 255
Val Phe Ala Arg Ala Ala His His His Pro Val Leu Leu Arg Ala Lys
            260                 265                 270
Leu Asp Glu Gln Ala Ala Thr Asp Arg Leu Arg Ala Ala Arg Gly Ala
        275                 280                 285
Phe Leu Pro Ser Val Ser Val Ser Gly Gly Trp Asn Thr Gly Phe Ser
    290                 295                 300
Arg Phe Leu Asn Gly Ser Asp Tyr Thr Pro Phe Ser Glu Gln Phe Arg
305                 310                 315                 320
Asn Arg Arg Gly Glu Tyr Val Ser Leu Asn Leu Ser Ile Pro Ile Phe
                325                 330                 335
Ser Gly Phe Ser Leu Val Ser His Leu Arg Gln Ala Arg Ala Glu Arg
            340                 345                 350
Arg Ala Ala Ile Val Arg Arg Gly Glu Ala Glu Arg Arg Leu Tyr Ser
        355                 360                 365
Glu Ile Ala Gln Ala Met Ala Asp Arg Asp Ala Ala Leu Ala Ser Tyr
    370                 375                 380
Arg Gln Ala Lys Glu His Thr Asp Ala Met Gln Thr Ala Tyr Glu Ala
385                 390                 395                 400
Val Leu Gln Arg Tyr Glu Glu Gly Leu Asn Thr Ala Ile Asp Leu Thr
                405                 410                 415
Thr Gln Ala Asn Arg Leu Leu Asp Ala Arg Val Gln Arg Leu Arg Ala
            420                 425                 430
Ala Met Thr Tyr Arg Leu Lys Cys Lys Leu Ile Ala Tyr Tyr Gly Cys
        435                 440                 445
Leu Ser Asp
    450

(2) INFORMATION FOR SEQ ID NO:336

(i) SEQUENCE CHARACTERISTICS:
(A) LENGTH: 962 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...962

(xi) SEQUENCE DESCRIPTION: SEQ ID NO:336

Ala Ile Phe Val Val Ser Leu Gln Ile Glu Lys Ile Thr Glu Asn Lys
1               5                   10                  15
Tyr Asn Ser Asp Lys Ser Met Asn Lys Phe Tyr Lys Ser Leu Leu Gln
            20                  25                  30
Ser Gly Leu Ala Ala Phe Val Ser Met Ala Thr Ala Leu Thr Ala Ser
        35                  40                  45
Ala Gln Ile Ser Phe Gly Gly Glu Pro Leu Ser Phe Ser Ser Arg Ser
    50                  55                  60
Ala Gly Thr His Ser Phe Asp Asp Ala Met Thr Ile Arg Leu Thr Pro
65                  70                  75                  80

Asp Phe Asn Pro Glu Asp Leu Ile Ala Gln Ser Arg Trp Gln Ser Gln
                85                  90                  95
Arg Asp Gly Arg Pro Val Arg Ile Gly Gln Val Ile Pro Val Asp Val
            100                 105                 110
Asp Phe Ala Ser Lys Ala Ser His Ile Ser Ser Ile Gly Asp Val Asp
        115                 120                 125
Val Tyr Arg Leu Gln Phe Lys Leu Glu Gly Ala Lys Ala Ile Thr Leu
    130                 135                 140
Tyr Tyr Asp Ala Phe Asn Ile Pro Glu Gly Gly Arg Leu Tyr Ile Tyr
145                 150                 155                 160
Thr Pro Asp His Glu Ile Val Leu Gly Ala Tyr Thr Asn Ala Thr His
                165                 170                 175
Arg Arg Asn Gly Ala Phe Ala Thr Glu Pro Val Pro Gly Ser Glu Leu
            180                 185                 190
Ile Met Asp Tyr Glu Val Ser Arg Gly Gly Thr Leu Pro Asp Ile Lys
        195                 200                 205
Ile Ser Gly Ala Gly Tyr Ile Phe Asp Lys Val Gly Gly Arg Pro Val
    210                 215                 220
Thr Asp Asn His Tyr Gly Ile Gly Glu Asp Asp Ser Asp Ser Asp Cys
225                 230                 235                 240
Glu Ile Asn Ile Asn Cys Pro Glu Gly Ala Asp Trp Gln Ala Glu Lys
                245                 250                 255
Asn Gly Val Val Gln Met Ile Met Val Lys Gly Gln Tyr Ile Ser Met
            260                 265                 270
Cys Ser Gly Asn Leu Leu Asn Asn Thr Lys Gly Asp Phe Thr Pro Leu
        275                 280                 285
Ile Ile Ser Ala Gly His Cys Ala Ser Ile Thr Thr Asn Phe Gly Val
    290                 295                 300
Thr Gln Ser Glu Leu Asp Lys Trp Ile Phe Thr Phe His Tyr Glu Lys
305                 310                 315                 320
Arg Gly Cys Ser Asn Gly Thr Leu Ala Ile Phe Arg Gly Asn Ser Ile
                325                 330                 335
Ile Gly Ala Ser Met Lys Ala Phe Leu Pro Ile Lys Gly Lys Ser Asp
            340                 345                 350
Gly Leu Leu Leu Gln Leu Asn Asp Glu Val Pro Leu Arg Tyr Arg Val
        355                 360                 365
Tyr Tyr Asn Gly Trp Asp Ser Thr Pro Asp Ile Pro Ser Ser Gly Ala
    370                 375                 380
Gly Ile His His Pro Ala Gly Asp Ala Met Lys Ile Ser Ile Leu Lys
385                 390                 395                 400
Lys Thr Pro Ala Leu Asn Thr Trp Ile Ser Ser Ser Gly Ser Gly Gly
                405                 410                 415
Thr Asp Asp His Phe Tyr Phe Lys Tyr Asp Gln Gly Gly Thr Glu Gly
            420                 425                 430
Gly Ser Ser Gly Ser Ser Leu Phe Asn Gln Asn Lys His Val Val Gly
        435                 440                 445
Thr Leu Thr Gly Gly Ala Gly Asn Cys Gly Gly Thr Glu Phe Tyr Gly
    450                 455                 460
Arg Leu Asn Ser His Trp Asn Glu Tyr Ala Ser Asp Gly Asn Thr Ser
465                 470                 475                 480
Arg Met Asp Ile Tyr Leu Asp Pro Gln Asn Asn Gly Gln Thr Thr Ile
                485                 490                 495
Leu Asn Gly Thr Tyr Arg Asp Gly Tyr Lys Pro Leu Pro Ser Val Pro
            500                 505                 510
Arg Leu Leu Leu Gln Ser Thr Gly Asp Gln Val Glu Leu Asn Trp Thr
        515                 520                 525
Ala Val Pro Ala Asp Gln Tyr Pro Ser Ser Tyr Gln Val Glu Tyr His
    530                 535                 540
Ile Phe Arg Asn Gly Lys Glu Ile Ala Thr Thr Lys Glu Leu Ser Tyr
545                 550                 555                 560
Ser Asp Ala Ile Asp Glu Ser Ile Ile Gly Ser Gly Ile Ile Arg Tyr
                565                 570                 575
Glu Val Ser Ala Arg Phe Ile Tyr Pro Ser Pro Leu Asp Gly Val Glu
            580                 585                 590
Ser Tyr Lys Asp Thr Asp Lys Thr Ser Ala Asp Leu Ala Ile Gly Asp
        595                 600                 605
Ile Gln Thr Lys Leu Lys Pro Asp Val Thr Pro Leu Pro Gly Gly Gly
    610                 615                 620
Val Ser Leu Ser Trp Lys Val Pro Phe Leu Ser Gln Leu Val Ser Arg
625                 630                 635                 640
Phe Gly Glu Ser Pro Asn Pro Val Phe Lys Thr Phe Glu Val Pro Tyr
                645                 650                 655
Val Ser Ala Ala Ala Ala Gln Thr Pro Asn Pro Pro Val Gly Val Val
            660                 665                 670
Ile Ala Asp Lys Phe Met Ala Gly Thr Tyr Pro Glu Lys Ala Ala Ile

675                 680                 685
Ala Ala Val Tyr Val Met Pro Ser Ala Pro Asp Ser Thr Phe His Leu
    690                 695                 700
Phe Leu Lys Ser Asn Thr Asn Arg Arg Leu Gln Lys Val Thr Thr Pro
705                 710                 715                 720
Ser Asp Trp Gln Ala Gly Thr Trp Leu Arg Ile Asn Leu Asp Lys Pro
                725                 730                 735
Phe Pro Val Asn Asn Asp His Met Leu Phe Ala Gly Ile Arg Met Pro
            740                 745                 750
Asn Lys Tyr Lys Leu Asn Arg Ala Ile Arg Tyr Val Arg Asn Pro Asp
        755                 760                 765
Asn Leu Phe Ser Ile Thr Gly Lys Lys Ile Ser Tyr Asn Asn Gly Val
    770                 775                 780
Ser Phe Glu Gly Tyr Gly Ile Pro Ser Leu Leu Gly Tyr Met Ala Ile
785                 790                 795                 800
Lys Tyr Leu Val Val Asn Thr Asp Ala Pro Lys Ile Asp Met Ser Leu
                805                 810                 815
Val Gln Glu Pro Tyr Ala Lys Gly Thr Asn Val Ala Pro Phe Pro Glu
            820                 825                 830
Leu Val Gly Ile Tyr Val Tyr Lys Asn Gly Thr Phe Ile Gly Thr Gln
        835                 840                 845
Asp Pro Ser Val Thr Thr Tyr Ser Val Ser Asp Gly Thr Glu Ser Asp
    850                 855                 860
Glu Tyr Glu Ile Lys Leu Val Tyr Lys Gly Ser Gly Ile Ser Asn Gly
865                 870                 875                 880
Val Ala Gln Ile Glu Asn Asn Asn Ala Val Val Ala Tyr Pro Ser Val
                885                 890                 895
Val Thr Asp Arg Phe Ser Ile Lys Asn Ala His Met Val His Ala Ala
            900                 905                 910
Ala Leu Tyr Ser Leu Asp Gly Lys Gln Val Arg Ser Trp Asn Asn Leu
        915                 920                 925
Arg Asn Gly Val Thr Phe Ser Val Gln Gly Leu Thr Ala Gly Thr Tyr
    930                 935                 940
Met Leu Val Met Gln Thr Ala Asn Gly Pro Val Ser Gln Lys Ile Val
945                 950                 955                 960
Lys Gln

(2) INFORMATION FOR SEQ ID NO:337

    (i) SEQUENCE CHARACTERISTICS:
        (A) LENGTH: 702 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...702

    (xi) SEQUENCE DESCRIPTION: SEQ ID NO:337

Trp Lys Lys Ser Asn Pro Gln Tyr Arg Gly Arg Thr Ala Asn Tyr Arg
1               5                   10                  15
Asp His Tyr Asn Thr Glu Ser Tyr Leu Ser Leu Arg Arg Asn Gln Thr
            20                  25                  30
Met Lys Tyr Leu Ile Arg Leu Phe Leu Ser Leu Met Leu Leu Ser Leu
        35                  40                  45
Trp Thr Gly Cys Thr His Glu Glu Leu Ser Ile Cys Asp Gly Glu Asn
    50                  55                  60
Thr Leu Val Leu Arg Val Glu Thr Gly Lys Ala Pro Asn Ala Arg Ala
65                  70                  75                  80
Thr Glu Pro Gly Gln Gly Ile Tyr Asn Glu Asn Lys Val Gly Ser Ile
                85                  90                  95
Ser Val Leu Phe Tyr Leu Glu Gly Gln Leu Arg Trp Gln Val Lys Ser
            100                 105                 110
Thr Asp Tyr Gln Ile His Glu Gly Ala Tyr Ile Ile Pro Val Lys Glu
        115                 120                 125
Gln Met Arg Pro Leu Phe Asn Gly Asn Asn Asn Phe Ser Ile Tyr Val

    130                 135                 140
Val Ala Asn Leu Asp Phe Asn Ala Pro Ala Thr Glu Ala Ala Leu Ser
145                 150                 155                 160
Gln Phe Val Val Glu Lys Ser Ile Glu Val Ser Ser Thr Thr Ala Pro
                165                 170                 175
Ala Asp Phe Val Met Leu Ala His Gly Asn Lys Gln Ile Asn Met Ala
            180                 185                 190
Thr Thr Glu Gly Lys Leu Leu Gly Asp Tyr Lys Leu Lys Arg Val Ala
        195                 200                 205
Ala Lys Ile Arg Met Ile Lys Pro Thr Ile Asn Val Gln Gly Tyr Glu
    210                 215                 220
Val Val Gly Asn Ile Gln Ala Lys Phe Arg Asn Ser Val Thr Lys Gly
225                 230                 235                 240
Phe Leu Thr Thr Glu Ala Gln Glu Ile Pro Ala Ala Ala Ser Tyr Lys
                245                 250                 255
Thr Ser Glu Tyr Leu Asp Ile Ala Glu Ser Ala Pro Ala Asn Ser Ile
            260                 265                 270
His Phe Tyr Ser Tyr Tyr Asn Lys Trp Thr Leu Ser Thr Pro Glu Lys
        275                 280                 285
Arg Pro Glu Phe Phe Ile Met Val Lys Phe Lys Lys Thr Gly Gln Pro
    290                 295                 300
Asp Asn Thr Ala Lys Pro Tyr Tyr Tyr Arg Val Pro Leu Glu Ser Gln
305                 310                 315                 320
Asp Asn Gln Val Lys Ser Asn Val Leu Tyr Asn Leu Asn Val Lys Ile
                325                 330                 335
Glu Ile Leu Gly Ser Leu Gln Glu Pro Glu Ala Val Ser Val Asn Gly
            340                 345                 350
Thr Leu Ala Ile Glu Glu Trp Ile Leu His Gln Asp Ala Phe Asn Leu
        355                 360                 365
Pro Ala Thr Asn Tyr Leu Ile Val Glu Gln His Glu Ile Phe Met Asn
    370                 375                 380
Asn Val Asn Thr Tyr Ser Val Lys Tyr Gln Thr Ser Gln Lys Pro Ile
385                 390                 395                 400
Ser Ile Ser Ile Gln Ser Val Thr Phe Ser Tyr Val Ser Ser Asp Gly
                405                 410                 415
Thr Gln His Asn Asp Leu Val Ala Ser Ser Ser Asp Gln Tyr Pro Thr
            420                 425                 430
Ile Thr Ser Asp Asn Thr Ser Ile Ile Ile Thr Ser Lys Ile Pro Val
        435                 440                 445
Asn Asn Val Pro Lys Lys Ile Val Phe Glu Val Thr Asn Gly Val Ala
    450                 455                 460
Gly Leu Lys Glu Thr Val Thr Val Leu Gln Tyr Pro Ala Gln Phe Ile
465                 470                 475                 480
Val Asn Thr Leu Gly Thr Ala Ser Ala Trp Arg Pro Asp Gly Ser Leu
                485                 490                 495
Ala Pro Gly Leu Asn Asn Lys Ala Ile Tyr His Val Val Val Leu Val
            500                 505                 510
Pro Pro Glu Asn Leu Phe Glu Asp Gly Thr Gln Thr Ile Ile Gly Tyr
        515                 520                 525
Pro Pro Thr Glu Thr Ile Ser Phe His Lys Lys Glu Asn Asn Thr Tyr
    530                 535                 540
Pro Ile Val Trp Ser Asp Thr Asn Thr Thr Lys Gln Asp Leu Glu Thr
545                 550                 555                 560
Ser Arg Met Ile Ser Pro Ser Phe Glu Leu Ala Ser Gln Leu Gly Ala
                565                 570                 575
Thr Leu Pro Met Pro Tyr Leu Glu Tyr Trp Pro Gly Thr Ser Tyr Leu
            580                 585                 590
Leu Asp Tyr Ser Gly Asn Tyr Asn Asn Lys Arg Tyr Ala Leu Phe Asn
        595                 600                 605
Cys Ala Phe Tyr Trp Glu Lys Arg Lys Val Asn Asn Glu Glu Ile Lys
    610                 615                 620
Phe Asp Asp Trp Arg Leu Pro Thr Glu Ala Glu Ile Lys Leu Ile Asp
625                 630                 635                 640
Lys Leu Gln His Asn Glu Gln Ser Ala Val Gln Ala Ile Met Thr Gly
                645                 650                 655
Asn Tyr Tyr Trp Asp Ser Tyr Ser Ala Asn Gly Ser Tyr Lys Met Gln
            660                 665                 670
Gly Gly Gly Gly Gln Gly Asn Ser Ser Lys Ala Tyr Val Arg Cys Val
        675                 680                 685
Arg Asp Val Lys Lys Pro Ile Arg Asp Lys Lys Ser Gly Lys
    690                 695                 700

(2) INFORMATION FOR SEQ ID NO:338

(i) SEQUENCE CHARACTERISTICS:

(A) LENGTH: 1312 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...1312

(xi) SEQUENCE DESCRIPTION: SEQ ID NO:338

Lys Arg Val Asp Ser Tyr Glu Cys Arg Gln Lys Ala Cys Lys Cys Ala
1               5                   10                  15
Ile Cys Val Ile Gln Lys Phe Thr Asn Val Lys Leu Asn Asp Met Arg
            20                  25                  30
Lys Ile Leu Ser Phe Leu Met Met Cys Ser Leu His Leu Gly Leu Gln
        35                  40                  45
Ser Gln Thr Trp His Gly Asp Pro Asp Ser Val Ala Ala Leu Pro Ser
    50                  55                  60
Ile Gly Ile Gln Glu Ser Ser Cys Thr Arg Ile Thr Phe Glu Val Val
65                  70                  75                  80
Phe Pro Gly Phe Tyr Ser Val Glu Lys Arg Glu Gly Asn Gln Val Phe
                85                  90                  95
Gln Arg Ile Ser Met Pro Gly Cys Gly Ser Phe Gly Asn Leu Gly Glu
            100                 105                 110
Ala Glu Leu Pro Val Leu Lys Lys Met Ile Ala Val Pro Glu Phe Ser
        115                 120                 125
Thr Ala Asn Val Ala Val Lys Ile Lys Glu Thr Glu Thr Phe Asp Asn
    130                 135                 140
Tyr Asn Ile Tyr Pro Asn Pro Thr Tyr Val Val Glu Glu Leu Pro Glu
145                 150                 155                 160
Gly Gly Thr Tyr Leu Val Glu Ala Phe Ala Ile Asn Asn Asp Tyr Tyr
                165                 170                 175
Ser Gln Asn Val Ser Leu Pro Ser Thr His Tyr Val Tyr Ser Gln Asp
            180                 185                 190
Gly Tyr Phe Arg Ser Gln Arg Phe Ile Glu Val Thr Leu Tyr Pro Phe
        195                 200                 205
Arg Tyr Asn Pro Val Arg Gln Glu Ile Leu Phe Ala Lys Lys Ile Glu
    210                 215                 220
Val Thr Ile Thr Phe Asp Asn Pro Gln Pro Pro Leu Gln Lys Asn Thr
225                 230                 235                 240
Gly Ile Phe Asn Lys Val Ala Ser Ser Ala Phe Ile Asn Tyr Glu Ala
                245                 250                 255
Asp Gly Lys Ser Ala Ile Glu Asn Asp Met Val Phe Ser Arg Gly Thr
            260                 265                 270
Thr Thr Tyr Ile Ser Gly Asn Val Ala Ser Asn Leu Pro Gln Asn Cys
        275                 280                 285
Asp Tyr Leu Val Ile Tyr Asp Asp Met Phe Asn Val Asn Gln Gln Pro
    290                 295                 300
His Asp Glu Ile Lys Arg Leu Cys Glu His Arg Ala Phe Tyr Asn Gly
305                 310                 315                 320
Phe Asp Val Ala Ala Val Ser Ile Lys Asp Val Leu Asn Ser Phe Pro
                325                 330                 335
Ser Asn Ala Thr Ser Tyr Ile Asn Glu Thr Lys Leu Lys Asn Phe Ile
            340                 345                 350
Arg Ser Val Tyr Asn Gln Ser Asn Ala Lys Arg Thr Leu Asp Gly Lys
        355                 360                 365
Leu Gly Tyr Val Leu Leu Ile Gly Lys Pro Leu Ser Lys Tyr Leu Ala
    370                 375                 380
Asp Thr Asp Asn Thr Lys Val Pro Thr Ser Phe Ile His Asn Val Ser
385                 390                 395                 400
Leu Ile Pro Ser His Pro Thr Phe Gly Ser Ile Cys Ala Ser Asp Tyr
                405                 410                 415
Phe Phe Ser Cys Val Ser Pro Leu Asp Thr Val Gly Asp Leu Phe Ile
            420                 425                 430
Gly Arg Phe Ser Val Thr Asn Ala His Glu Leu His Asn Leu Ile Glu
        435                 440                 445
Lys Thr Ile Asn Lys Glu Ile Ser Tyr Asn Pro Ile Ala His Lys Asn
    450                 455                 460

Ile Leu Tyr Ala Glu Gly Lys Gly Cys Asp Ala Pro Ile Leu Arg Leu
465                 470                 475                 480
Phe Leu Lys Glu Ile Ala Ser Gly Tyr Thr Val Asn Ser Ile Leu Lys
                485                 490                 495
Ser Asn Gln Val Ser Ala Ile Asp Ser Ile Phe Asp Cys Leu Asn Asn
            500                 505                 510
Gly Ser His His Phe Tyr Phe Asn Thr His Gly Met Pro Thr Val Trp
        515                 520                 525
Gly Ile Gly Gln Gly Leu Asp Val Asn Thr Leu Thr Ala Arg Leu Asn
    530                 535                 540
Asn Thr Ser Ser Gln Gly Leu Cys Thr Ser Leu Ser Cys Ser Ser Ala
545                 550                 555                 560
Val Ala Asp Ser Thr Ile Arg Ser Leu Gly Glu Val Leu Thr Thr Tyr
                565                 570                 575
Ala Pro Asn Lys Gly Phe Ser Ala Phe Leu Gly Gly Ser Arg Ala Thr
            580                 585                 590
Gln Tyr Ala Val Tyr Leu Glu Gly Pro Cys Pro Pro Ser Glu Phe Tyr
        595                 600                 605
Glu Tyr Leu Pro Tyr Ser Leu Tyr His Asn Leu Ser Thr Val Val Gly
    610                 615                 620
Glu Met Leu Leu Ser Ser Ile Ile Asn Thr Asn Ser Val Asp Thr Tyr
625                 630                 635                 640
Ser Lys Phe Asn Phe Asn Leu Leu Gly Asp Pro Ala Leu Asn Ile Met
                645                 650                 655
Ala His Gly Met Glu Val Ser Asn Cys Ile Thr Leu Pro Asn Asn Thr
            660                 665                 670
Ile Ile Ser Ser Pro Ile Thr Ile Lys Asn Gly Gly Cys Leu Lys Ile
        675                 680                 685
Pro Glu Lys Gly Val Leu His Phe Thr Asn Asn Gly Ser Ile Gln Val
    690                 695                 700
Met Ser Gly Gly Thr Leu Glu Ile Gly Asn Gln Ala Lys Ile Ser Gly
705                 710                 715                 720
Glu Thr Gly Ala Asn Pro Thr Phe Ile Thr Val Tyr Gly Asp Gly Leu
                725                 730                 735
Ala Ile Asn Lys Gln Val Glu Ile Asp Asn Ile Asp Arg Leu Asn Leu
            740                 745                 750
Phe Ser Thr His Ser Val Met Pro Lys Phe His Phe Asp Ser Val Lys
        755                 760                 765
Phe Asn Ser Ala Pro Leu Tyr Thr Thr Asn Cys Ile Val Glu Ile Ser
    770                 775                 780
Asn Cys Glu Phe Thr Asn Arg Ser Asp Ile Ile Ser Lys Asn Cys Asp
785                 790                 795                 800
Leu Ser Val Glu Asn Ser Met Phe Ser Ser Ser Gly Ile Thr Val Phe
                805                 810                 815
Lys Pro Met Ala Thr Ser Ser Ile Thr Gly Leu Ser Thr Lys Ala Lys
            820                 825                 830
Ile Thr Asp Asn Thr Phe Phe Ala Thr Gly Asn Phe Ala Tyr His Ile
        835                 840                 845
Thr Asn Thr Pro Gly Leu Thr Ala Thr Ser Asn Ala Ala Ile Lys Leu
    850                 855                 860
Asp Asn Ile Pro Glu Tyr Tyr Ile Ser Gly Asn Lys Ile Val Asn Cys
865                 870                 875                 880
Asp Glu Ala Leu Val Leu Asn Asn Ser Gly Asn Arg Thr Asn Arg Leu
                885                 890                 895
His Asn Ile Thr Arg Asn Val Ile Lys Asn Cys Arg Ile Gly Ser Thr
            900                 905                 910
Leu Tyr Asn Ser Tyr Gly Ile Tyr Asn Arg Asn Lys Ile Ser Asn Asn
        915                 920                 925
His Ile Gly Val Arg Leu Leu Asn Asn Ser Cys Phe Tyr Phe Asp Asn
    930                 935                 940
Ala Pro Val Ile Asn Glu Glu Asp Lys Gln Thr Phe Ile Ser Asn Arg
945                 950                 955                 960
Thr Trp Gln Leu Tyr Ser Ser Asn Gly Thr Phe Pro Leu Asn Phe His
                965                 970                 975
Tyr Asn Ser Leu Gln Gly Gly Asp Thr Asp Thr Trp Ile Tyr Asn Asp
            980                 985                 990
Thr Tyr Thr Asn Arg Tyr Ile Asp Val Ser Asn Asn His Trp Gly Asn
        995                 1000                1005
Asn Asp Leu Phe Asp Pro Asn Gln Val Phe Asn Thr Pro Asp Leu Phe
    1010                1015                1020
Ile Trp Ile Pro Phe Trp Asp Gly Leu Pro Asn Gly Arg Ser Gly Asn
1025                1030                1035                1040
Ser Ser Ala Glu Ala Val Glu Phe Gln Thr Ala Leu Asp Cys Ile Gly
                1045                1050                1055
Asn Ser Asp Tyr Leu Ser Ala Lys Val Ala Leu Lys Met Met Val Glu

1060                    1065                    1070
Thr Tyr Pro Glu Ser Asp Phe Ala Ile Ala Ala Leu Lys Glu Leu Phe
        1075                    1080                    1085
Arg Ile Glu Lys Met Ser Gly Asn Asp Tyr Glu Gly Leu Lys Asp Tyr
    1090                    1095                    1100
Phe Arg Ser Asn Pro Thr Ile Ile Ser Ser Gln Asn Leu Phe Pro Thr
1105                    1110                    1115                    1120
Ala Asp Phe Leu Ser Ala Arg Cys Asp Ile Val Cys Glu Asn Tyr Gln
                1125                    1130                    1135
Ser Ala Ile Asp Trp Tyr Glu Asn Arg Leu Asn Ser Glu Ile Ser Tyr
            1140                    1145                    1150
Gln Asp Ser Val Phe Ala Val Ile Asp Leu Gly Asp Ile Tyr Trp Asn
        1155                    1160                    1165
Met Gln Leu Asp Ser Leu Arg Gly Thr Gly Ile Asp Leu Asn Ile Leu
    1170                    1175                    1180
Ser Cys Glu Gln Arg Lys Ser Leu Glu Ser His Gln Asn Val Lys Asn
1185                    1190                    1195                    1200
Tyr Leu Leu Ser Thr Leu Pro Glu Ser Thr Gly Thr Leu Leu Pro Pro
                1205                    1210                    1215
Leu Glu Cys Asn Lys Ser Ser Leu Asp Lys Ser Lys Ile Ile Ser Ile
            1220                    1225                    1230
Ser Pro Asn Pro Ala Lys Ala Val Val Thr Ile Ile Tyr Tyr Thr Asp
        1235                    1240                    1245
Asn Pro Ser Cys Ser Val Ile Lys Ile Tyr Gly Ile Asn Gly Ala Ser
    1250                    1255                    1260
Ala Asp Ile Thr Gly Leu Pro Lys His Leu Ser Glu Gly Tyr Tyr Ser
1265                    1270                    1275                    1280
Ile Gln Phe Asn Thr Ser Asn Phe Asp Pro Gly Phe Tyr Leu Val Thr
                1285                    1290                    1295
Leu Asn Val Asp Gln Lys Ile Ile Asp Thr Glu Lys Leu Arg Ile Lys
            1300                    1305                    1310

(2) INFORMATION FOR SEQ ID NO:339

(i) SEQUENCE CHARACTERISTICS:
(A) LENGTH: 938 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...938

(xi) SEQUENCE DESCRIPTION: SEQ ID NO:339

Ser Glu Asn Tyr Arg Tyr Gly Lys Ile Thr Asn Gln Ile Met Ala Ile
1               5                   10                  15
Met Met Lys Ser Ile Val Phe Arg Ala Phe Leu Thr Ile Leu Leu Ser
            20                  25                  30
Trp Ala Ala Ile Thr Asn Pro Thr Ala Gln Glu Ile Ser Gly Met Asn
        35                  40                  45
Ala Ser Cys Leu Ala Ala Pro Ala Gln Pro Asp Thr Ile Leu Tyr Glu
    50                  55                  60
Ser Phe Glu Asn Gly Pro Val Pro Asn Gly Trp Leu Glu Ile Asp Ala
65                  70                  75                  80
Asp Ala Asp Gly Ala Thr Trp Gly Ser Pro Ser Gly Ser Phe Ser Val
                85                  90                  95
Pro Tyr Gly His Asn Gly Leu Cys Thr Tyr Ser His Ile Arg Ser Gly
            100                 105                 110
Ile Ser Thr Ala Gly Asn Tyr Leu Ile Thr Pro Asn Ile Glu Gly Ala
        115                 120                 125
Lys Arg Val Lys Tyr Trp Val Cys Asn Gln Tyr Ser Thr Asn Pro Glu
    130                 135                 140
His Tyr Ala Val Met Val Ser Thr Thr Gly Thr Ala Ile Glu Asp Phe
145                 150                 155                 160
Val Leu Leu Phe Asp Asp Ser Ile Thr Gly Lys Pro Thr Pro Leu Val
                165                 170                 175
Trp Arg Arg Arg Ile Val Asp Leu Pro Glu Gly Thr Lys Tyr Ile Ala

                180                 185                 190
Trp Arg His Tyr Lys Val Thr Asp Ser His Thr Glu Phe Leu Lys Leu
        195                 200                 205
Asp Asp Val Thr Val Tyr Arg Ser Ile Glu Gly Pro Glu Pro Ala Thr
    210                 215                 220
Asp Phe Thr Val Ile Asn Ile Gly Gln Asn Val Gly Arg Leu Thr Trp
225                 230                 235                 240
Asn Tyr Pro Glu Asp Tyr Gln Pro Glu Gly Lys Gly Asn Glu Glu Leu
                245                 250                 255
Gln Leu Ser Gly Tyr Asn Ile Tyr Ala Asn Gly Thr Leu Leu Ala Gln
            260                 265                 270
Ile Lys Asp Val Ser Ile Leu Glu Tyr Val Asp Ser Thr Tyr Ser Leu
        275                 280                 285
Arg Asp Asn Pro Leu Gln Val Glu Tyr Cys Val Thr Ala Val Tyr Asp
    290                 295                 300
Glu Ser Ile Glu Ser Ser Thr Val Cys Gly Thr Leu His Tyr Ala Thr
305                 310                 315                 320
Asp Ala Ile Leu Tyr Glu Asn Phe Glu Asn Gly Pro Val Pro Asn Gly
                325                 330                 335
Trp Leu Val Ile Asp Ala Asp Gly Asp Gly Phe Ser Trp Gly His Tyr
            340                 345                 350
Leu Asn Ala Tyr Asp Ala Phe Pro Gly His Asn Gly Gly His Cys Ser
        355                 360                 365
Leu Ser Ala Ser Tyr Val Pro Gly Ile Gly Pro Val Thr Pro Asp Asn
    370                 375                 380
Tyr Leu Ile Thr Pro Lys Val Glu Gly Ala Lys Arg Val Lys Tyr Trp
385                 390                 395                 400
Val Ser Thr Gln Asp Ala Asn Trp Ala Ala Glu His Tyr Ala Val Met
                405                 410                 415
Ala Ser Thr Thr Gly Thr Ala Val Gly Asp Phe Val Ile Leu Phe Glu
            420                 425                 430
Glu Thr Met Thr Ala Lys Pro Thr Gly Ala Trp Tyr Glu Arg Thr Ile
        435                 440                 445
Asn Leu Pro Glu Gly Thr Lys Tyr Ile Ala Trp Arg His Tyr Asn Cys
    450                 455                 460
Thr Asp Ile Tyr Phe Leu Lys Leu Asp Asp Ile Thr Val Phe Gly Thr
465                 470                 475                 480
Pro Ala Ser Glu Pro Glu Pro Val Thr Asp Phe Val Val Ser Leu Ile
                485                 490                 495
Glu Asn Asn Lys Gly Arg Leu Lys Trp Asn Tyr Pro Asn Gly Tyr Glu
            500                 505                 510
Pro Asp Lys Thr Asp Asp Lys Asp Pro Leu Gln Leu Ala Gly Tyr Asn
        515                 520                 525
Ile Tyr Ala Asn Gly Ser Leu Leu Val His Ile Gln Asp Pro Thr Val
    530                 535                 540
Leu Glu Tyr Ile Asp Glu Thr Tyr Ser Ser Arg Asp Asp Gln Val Glu
545                 550                 555                 560
Val Glu Tyr Cys Val Thr Ala Val Tyr Asn Asp Asn Ile Glu Ser Gln
                565                 570                 575
Ser Val Cys Asp Lys Leu Ile Tyr Asp Ser Gln Ser Asp Ile Ile Leu
            580                 585                 590
Tyr Glu Gly Phe Glu Ala Gly Ser Ile Pro Glu Gly Trp Leu Leu Ile
        595                 600                 605
Asp Ala Asp Gly Asp Asn Val Asn Trp Asp Tyr Tyr Pro Trp Thr Met
    610                 615                 620
Tyr Gly His Asp Ser Glu Lys Cys Ile Ala Ser Pro Ser Tyr Leu Pro
625                 630                 635                 640
Met Ile Gly Val Leu Thr Pro Asp Asn Tyr Leu Val Thr Pro Arg Leu
                645                 650                 655
Glu Gly Ala Lys Leu Val Lys Tyr Trp Val Ser Ala Gln Asp Ala Val
            660                 665                 670
Tyr Ser Ala Glu His Tyr Ala Val Met Val Ser Thr Thr Gly Thr Ala
        675                 680                 685
Val Glu Asp Phe Val Leu Leu Phe Glu Glu Thr Met Thr Ala Lys Ala
    690                 695                 700
Asn Gly Ala Trp Tyr Glu Arg Thr Ile Thr Leu Pro Ala Gly Thr Lys
705                 710                 715                 720
Tyr Ile Ala Trp Arg His Tyr Asp Cys Thr Asp Met Phe Phe Leu Leu
                725                 730                 735
Leu Asp Asp Ile Thr Val Tyr Arg Ser Thr Glu Thr Val Pro Glu Pro
            740                 745                 750
Val Thr Asp Phe Val Val Ser Leu Ile Glu Asn Asn Lys Gly Arg Leu
        755                 760                 765
Lys Trp Asn Tyr Pro Asn Gly Tyr Glu Pro Asp Lys Thr Asp Asp Lys
    770                 775                 780

Lys Pro Leu Gln Leu Thr Gly Tyr Asn Ile Tyr Ala Asn Gly Ser Leu
785                 790                 795                 800
Leu Val His Ile Gln Asp Pro Thr Val Leu Glu Tyr Ile Asp Glu Thr
                805                 810                 815
Tyr Ser Ser Arg Asp Gly Gln Val Glu Met Glu Tyr Cys Val Thr Ala
            820                 825                 830
Val Tyr Asn Asp Asn Ile Glu Ser Gln Ser Val Cys Asp Lys Leu Asn
        835                 840                 845
Tyr Thr Ile Thr Ser Leu Asp Asn Ile Gln Ser Asp Thr Ser Leu Lys
    850                 855                 860
Ile Tyr Pro Asn Pro Ala Ser Tyr Val Val Arg Ile Glu Gly Leu Ser
865                 870                 875                 880
Arg Ser Lys Ser Thr Ile Glu Leu Tyr Asn Ala Leu Gly Ile Cys Ile
                885                 890                 895
Leu Arg Glu Glu Thr His Ser Glu Lys Thr Glu Ile Asp Val Ser Arg
            900                 905                 910
Leu Asn Asp Gly Val Tyr Leu Ile Lys Val Val Gly Gly Asn Lys Thr
        915                 920                 925
Thr Thr Glu Lys Val Glu Ile Lys Arg Pro
    930                 935

(2) INFORMATION FOR SEQ ID NO:340

(i) SEQUENCE CHARACTERISTICS:
(A) LENGTH: 606 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...606

(xi) SEQUENCE DESCRIPTION: SEQ ID NO:340

Ile Ile Phe Cys Thr Ile His His Ser Glu Leu Glu Ile Met Asn Ser
1               5                   10                  15
Ile Met Lys Tyr Gln Leu Tyr Thr Ala Val Ile Met Ala Leu Ser Val
            20                  25                  30
Ser Ser Val Cys Gly Gln Thr Pro Arg Asn Thr Glu Thr Lys Arg Pro
        35                  40                  45
Asp Thr Leu Arg Arg Glu Leu Thr Ile Val Asn Asp Gln Thr Val Glu
    50                  55                  60
Met Glu His Ala Asp Pro Leu Pro Ala Ala Tyr Lys Ala Ile Glu Pro
65                  70                  75                  80
Arg Leu Lys Pro Phe Arg Pro Glu Tyr Asn Lys Arg Thr Phe Gly Phe
                85                  90                  95
Val Pro Glu Val Ser Ser Ser Gly Arg Asn Asn Leu Pro Asn Ile Leu
            100                 105                 110
Pro Thr Glu Gly His Met Lys His Arg Gly Tyr Leu Asn Ile Gly Ile
        115                 120                 125
Gly His Thr Leu Asn Gln Arg Met Asp Ala Gly Tyr Arg Leu Ile Asp
    130                 135                 140
Ala Glu Gln Glu Arg Leu Asn Leu Phe Leu Ser Tyr Arg Gly Met Lys
145                 150                 155                 160
Ser Ala Phe Asn Thr Gly Asp Phe Asp Gly Asp Arg Lys Asp Arg Arg
                165                 170                 175
Met Met Ala Gly Val Asp Tyr Glu Gln Arg Arg Pro Ser Phe Val Leu
            180                 185                 190
Ala Thr Gly Leu Tyr Tyr Ser Asn His Tyr Phe Asn Asn Tyr Gly Arg
        195                 200                 205
Gly Ala Thr Thr Asn Val Gly Ser Ile Pro Gln Leu Ser Thr Pro Val
    210                 215                 220
Thr Pro Gln Met Asp Asn Gly Thr His Asn Val Arg Val Tyr Leu Gly
225                 230                 235                 240
Ala Lys Asn Asp Val Ile Asp Ala Arg Ile Asp Tyr Arg Phe Phe Arg
                245                 250                 255
Ser Ile Pro Tyr Leu Gly Thr Asp Pro Met Lys Ala Leu Thr Glu His
            260                 265                 270

Thr Pro Glu Leu Asn Val Thr Met Ser Asn Glu Leu Ser Asp Asp Ile
        275                 280                 285
Lys Leu Gly Val Glu Val Arg Thr Gly Gly Leu Phe Phe Ala Lys Asn
    290                 295                 300
Ser Glu Met Ile Gln Thr Gly Val Leu Ser Glu Thr Asp Arg Asn Leu
305                 310                 315                 320
Tyr Tyr Val Glu Gly Ala Pro Thr Ile Gly Phe Val Gly Asp Ser Asp
                325                 330                 335
Asn Met Gln Trp Asn Ile Gln Ala Gly Val Gly Ile Ser Ser His Phe
            340                 345                 350
Gly Ala Lys Gly Arg Leu Phe Phe Trp Pro Lys Leu Asp Ala Ser Leu
        355                 360                 365
Ser Ile Phe Pro Ser Trp Arg Val Tyr Ala Lys Ala Phe Gly Gly Val
    370                 375                 380
Ile Arg Asn Gly Leu Ala Asp Val Met Gln Glu Glu Met Pro Tyr Leu
385                 390                 395                 400
Met Pro Asn Thr Ile Val Leu Pro Ser Arg Asn Ala Leu Thr Ala Gln
                405                 410                 415
Leu Gly Val Lys Gly Asn Ile Ala Asp Val Val Arg Met Glu Val Tyr
            420                 425                 430
Gly Asp Phe Ser Lys Leu Thr Gly Val Pro Phe Tyr Thr Pro Thr Leu
        435                 440                 445
Pro Leu Tyr Asn Pro Ser Asp Leu Tyr Gln Tyr Asn Val Ser Phe Leu
    450                 455                 460
Pro Ile Tyr Ala Asp Gly Ser Arg Trp Arg Ala Gly Gly Lys Leu Glu
465                 470                 475                 480
Tyr Ser Tyr Arg Asp Met Leu Arg Phe Leu Val Asp Ala Ser Tyr Gly
                485                 490                 495
Lys Trp Asn Leu Asp Gly Gly Leu Val Ala Ser Met Gln Pro Asp Leu
            500                 505                 510
Ile Leu Lys Ala Glu Val Gly Val His Pro Ile Ala Pro Leu Asp Val
        515                 520                 525
Arg Leu Arg Tyr Thr Gln Leu Asn Gly Arg Tyr Arg Tyr Ser Phe Gly
    530                 535                 540
Ser Ala Gly Ser Glu Ala Leu Gly Ile Gly Asn Val His Leu Leu Ser
545                 550                 555                 560
Ala Asp Val Ser Tyr Lys Leu Lys Lys Asn Leu Ser Leu Tyr Leu Lys
                565                 570                 575
Ile Asp Asn Met Leu Ala Glu Thr Thr Glu Leu Ile Gly Tyr Tyr Pro
            580                 585                 590
Met Gln Pro Phe His Cys Phe Ala Gly Phe Ser Trp Thr Phe
        595                 600                 605

(2) INFORMATION FOR SEQ ID NO:341

    (i) SEQUENCE CHARACTERISTICS:
        (A) LENGTH: 357 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...357

    (xi) SEQUENCE DESCRIPTION: SEQ ID NO:341

Asn Ser His Ala Thr Val Ser Val Ile Cys Ser Met Met Glu Lys Cys
1               5                   10                  15
Ile Phe Ala His Tyr Pro His Asn Leu Val Phe Met Ile Arg Lys His
            20                  25                  30
Phe Gly Ile Ile Leu Gly Phe Leu Ser Leu Val Phe Ser Ala Gly Ala
        35                  40                  45
Gln Gln Glu Lys Gln Val Phe His Phe Leu Asn Leu Pro Ala Thr Ala
    50                  55                  60
Gln Ala Leu Ala Ala Gly Gly Lys Ala Ile Thr Ile Val Asp Asp Asn
65                  70                  75                  80
Pro Gly Leu Ala Phe Glu Asn Pro Ala Leu Leu Gly Tyr Glu Ser Gly
                85                  90                  95

Gly Arg Ala Phe Leu Ser Tyr Leu Tyr Tyr Met Ser Gly Ser His Met
            100                 105                 110
Gly Asn Ala Cys Tyr Ala Ser Ser Val Gly Glu Arg Gly Met Trp Gly
        115                 120                 125
Val Gly Met Arg Phe Leu Asn Tyr Gly Ser Met Gln Gly Tyr Asp Gln
    130                 135                 140
Asn Ala Ile Ala Thr Gly Ser Phe Ser Ala Ser Asp Ile Ala Val Gln
145                 150                 155                 160
Gly Phe Tyr Ser His Glu Leu Ser Asn His Phe Arg Gly Gly Val Ser
                165                 170                 175
Leu Lys Ala Leu Tyr Ser Ser Ile Glu Thr Tyr Ser Ser Phe Gly Leu
            180                 185                 190
Gly Val Asp Val Gly Ile Ser Tyr Tyr Asp Asp Asp Lys Gly Tyr Ser
        195                 200                 205
Ala Ser Ala Leu Phe Lys Asn Val Gly Ala Gln Leu Lys Gly Tyr Asn
    210                 215                 220
Glu Glu Arg Glu Pro Leu Asp Trp Asp Phe Gln Leu Gly Phe Ser Arg
225                 230                 235                 240
Ser Phe Ile Asn Ala Pro Phe Arg Leu His Ile Thr Leu Phe Asn Leu
                245                 250                 255
Asn Pro His Tyr Phe Lys Arg Leu Val Pro Arg Asp Leu Ser Lys Met
            260                 265                 270
Gln Lys Phe Leu Arg His Phe Ser Ile Gly Ala Glu Phe Thr Pro Ser
        275                 280                 285
Glu Arg Phe Trp Val Gly Leu Gly Tyr Thr Pro Gln Ile Ala Gln Asp
    290                 295                 300
Phe Glu Val Glu Gly Gly Asn Lys Trp Gly Gly Leu Ser Ala Gly Val
305                 310                 315                 320
Gly Phe Thr Ser Gly Val Val Arg Val Gly Val Ser Ala Ala Thr Tyr
                325                 330                 335
His Pro Ala Ala Leu Ser Phe Met Cys Ser Val Gly Ile Arg Leu Asp
            340                 345                 350
Asp Lys Ser Ile Phe
        355

(2) INFORMATION FOR SEQ ID NO:342

     (i) SEQUENCE CHARACTERISTICS:
          (A) LENGTH: 337 amino acids
          (B) TYPE: amino acid
          (D) TOPOLOGY: linear

     (ii) MOLECULE TYPE: protein

    (iii) HYPOTHETICAL: YES

     (vi) ORIGINAL SOURCE:
          (A) ORGANISM: Porphyromonas gingivalis

     (ix) FEATURE:
          (A) NAME/KEY: misc_feature
          (B) LOCATION 1...337

     (xi) SEQUENCE DESCRIPTION: SEQ ID NO:342

Pro Gln Pro Val Gly Leu Lys Glu Ile Thr Ile Lys Pro Met Cys Leu
1               5                   10                  15
Glu Pro Ile Ile Ala Pro Ile Ser Ser Glu Leu Leu Glu Gln Glu Leu
            20                  25                  30
Thr Ala Asp Arg Phe Leu Arg Met Thr Asn Lys Ala Gly Asn Glu Ile
        35                  40                  45
Tyr Val Phe Thr Ala Glu Glu Ala Pro His Cys Met Lys Glu Val Gly
    50                  55                  60
Arg Leu Arg Glu Glu Ala Phe Arg His Tyr Gly Gly Gly Thr Gly Lys
65                  70                  75                  80
Ala Ile Asp Ile Asp Glu Phe Asp Thr Met Pro Gly Ser Tyr Lys Gln
                85                  90                  95
Leu Ile Val Trp Asp Pro Gln Asn Lys Ala Ile Leu Gly Gly Tyr Arg
            100                 105                 110
Phe Ile Tyr Gly Arg Asp Val Ala Phe Asp Thr Asp Gly Lys Pro Leu
        115                 120                 125
Leu Ala Thr Ala Glu Met Phe Arg Phe Ser Asp Ala Phe Leu His Asp
    130                 135                 140
Tyr Leu Pro Tyr Thr Val Glu Leu Gly Arg Ser Phe Val Ser Leu Gln
145                 150                 155                 160

Tyr Gln Ser Thr Arg Met Gly Thr Lys Ala Ile Phe Val Leu Asp Asn
                165                 170                 175
Leu Trp Asp Gly Ile Gly Ala Leu Thr Val Val Asn Pro Glu Ala Leu
            180                 185                 190
Tyr Phe Tyr Gly Lys Val Thr Met Tyr Lys Asp Tyr Asp Arg Arg Ala
        195                 200                 205
Arg Asn Leu Ile Leu Tyr Phe Leu Arg Lys His Phe Ser Asp Pro Glu
    210                 215                 220
Gly Leu Val Lys Pro Ile His Pro Leu Pro Ile Glu Ile Ser Ala Glu
225                 230                 235                 240
Asp Glu Ala Leu Phe Ser Ser Ser Asp Phe Asp Thr Asn Tyr Lys Thr
                245                 250                 255
Leu Asn Ile Glu Val Arg Lys Leu Gly Ile Asn Ile Pro Pro Leu Val
            260                 265                 270
Ser Ala Tyr Ile Ala Leu Ser Pro Glu Met Arg Val Phe Gly Thr Ala
        275                 280                 285
Val Asn Glu Ser Phe Gly Glu Val Glu Glu Thr Gly Ile Phe Ile Ala
    290                 295                 300
Val Gly Lys Ile Leu Glu Glu Lys Lys Gln Arg His Ile Glu Ser Phe
305                 310                 315                 320
Ile Leu Ser Arg Asn Glu Lys Lys Gly Leu Asp Ser Ser Asn Gly Arg
                325                 330                 335
Ser

(2) INFORMATION FOR SEQ ID NO:343

     (i) SEQUENCE CHARACTERISTICS:
          (A) LENGTH: 566 amino acids
          (B) TYPE: amino acid
          (D) TOPOLOGY: linear

     (ii) MOLECULE TYPE: protein

    (iii) HYPOTHETICAL: YES

     (vi) ORIGINAL SOURCE:
          (A) ORGANISM: Porphyromonas gingivalis

     (ix) FEATURE:
          (A) NAME/KEY: misc_feature
          (B) LOCATION 1...566

     (xi) SEQUENCE DESCRIPTION: SEQ ID NO:343

Tyr Asp Gly Ala Arg Leu Val Tyr Thr Leu Phe Arg Asn Arg Asn Asp
1               5                   10                  15
Ile His Pro Met Lys Thr Ile Val Arg Tyr Ser Arg Leu Pro Val Ala
            20                  25                  30
Leu Phe Phe Cys Leu Leu Gly Ala Val His Leu Ser Val Glu Ala Gln
        35                  40                  45
Met Leu Asn Thr Pro Phe Glu Leu Ser Asp Gln Ile Val Leu Ser Pro
    50                  55                  60
Thr Glu Arg Gln Tyr Arg Glu Ile Cys Val Gln Thr Lys Glu Lys Arg
65                  70                  75                  80
Gly Ala Asp Leu Phe Pro Leu Ser Asp Lys Leu Arg Asp Ser Ala Tyr
                85                  90                  95
Val Arg Phe Gly Ser Ala Tyr Gly Asp Ile Ala Gly Asp Tyr Leu Pro
            100                 105                 110
Tyr Asn Gly Asn Asn Tyr Ser Ser Leu Ser Leu Glu Ser Gly Gly Arg
        115                 120                 125
Ile Ser Val Arg Asn Tyr Gly Thr Leu Gln Gly Ser Ala Ser Tyr Ser
    130                 135                 140
Arg Gly Met His Lys Arg Ile Gly Trp Asn Ala Leu Arg Asn Ala Glu
145                 150                 155                 160
Ala Tyr Tyr Pro Tyr Leu Val Ser Asp Ser Thr Gly Gly Asp Tyr His
                165                 170                 175
Phe Glu Asp Tyr Arg Leu Ala Gly Tyr Tyr Ser Phe Arg Ala Gly Arg
            180                 185                 190
Leu Pro Leu Gly Ile Gly Phe Ser Tyr Arg Gly Glu Val Ala Tyr Arg
        195                 200                 205
Leu Thr Asp Pro Arg Thr Thr Asn Thr Thr Gly Ala Leu Glu Leu Ser
    210                 215                 220
Cys Ala Thr Ser Leu Thr Leu Pro Arg Glu Asn Arg Leu Ser Leu Ser
225                 230                 235                 240

Ala Ala Tyr Leu Tyr His Arg Gln His Leu Thr Gln Tyr Asn Trp Arg
                245                 250                 255
Pro Gly Gln Gln Asp Lys Phe Phe Val Ser Tyr Gly Phe Gly Gln Val
            260                 265                 270
Asp Val Ser Asn Ser Pro Ile Trp Phe Gly Ile Ser Arg Met Asn Tyr
        275                 280                 285
Val Asn Gly Trp Lys Leu Ser Ser Arg Leu Asp Thr Arg Arg Gly Asp
    290                 295                 300
Ala Ile Gly Leu Asp Tyr Ser Gly Tyr Phe Leu Asp Thr Glu Glu Arg
305                 310                 315                 320
Ser Ser Ile Asn Leu Phe Ala Leu Leu Tyr Asn Arg Leu Arg Leu Tyr
                325                 330                 335
Gly Ser Trp His Leu Ser Asp Phe Asp Phe Ser Phe Ser Ala Asp Tyr
            340                 345                 350
Ala Leu Arg Gln Gly Ile Glu Arg Ile Tyr Glu Asp Tyr Lys Pro Asp
        355                 360                 365
Asp Asn Tyr His Ile Tyr Asp Leu Arg Ile Leu Ala Ile Arg Arg Trp
    370                 375                 380
Tyr Met Leu Asn Glu Phe Ser Ala Gln Ala Gln Ala Ser Tyr Arg Ile
385                 390                 395                 400
Arg Thr Asp Arg Gly Cys Ala Leu Arg Val Ser Ala Gly Ser Asp Phe
                405                 410                 415
Tyr Gly Tyr Asp Glu Thr Tyr Arg Lys His Gly His His Thr Met Ser
            420                 425                 430
Gly Met Leu Arg Pro Phe Ala Gly Ile Ala Tyr Asp His Ala Gly Ser
        435                 440                 445
Lys Leu Asp Phe Gly Leu Ser Leu Ser Ala Ala Tyr Arg Met Val Leu
    450                 455                 460
Thr His Ser Tyr Lys Ile Arg Thr Ile Gln Lys Glu Gln Leu Asp Tyr
465                 470                 475                 480
Gln Leu Ala Tyr Leu Pro Tyr Ala Tyr Arg Asn Arg Glu Gly Val Glu
                485                 490                 495
Val Arg Ser Ser Leu Tyr Val Ser Ile Pro Met Gln Asn Thr His Arg
            500                 505                 510
Leu Met Thr Glu Leu Arg Leu Tyr Gly Asp Leu Met Lys Arg Lys Asp
        515                 520                 525
Gly Ile Ala Tyr Gly Lys Thr Pro Gly Val Ile Ser His Ile Leu Ser
    530                 535                 540
Asp Pro Gln Ala Glu Arg Thr Ser Gly His Thr Ile Gly Ala Ile Cys
545                 550                 555                 560
Asn Ile Ser Tyr Leu Phe
                565

(2) INFORMATION FOR SEQ ID NO:344

    (i) SEQUENCE CHARACTERISTICS:
        (A) LENGTH: 819 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...819

    (xi) SEQUENCE DESCRIPTION: SEQ ID NO:344

Arg Thr Asn Val Phe Leu Ser Leu Ser His Lys Ile Gly Arg Arg Gly
1               5                   10                  15
Ala Ser Cys Ser Asn Arg Asn Ala Trp Met Ala Glu Asn Lys Pro Ser
            20                  25                  30
Ser Pro Glu Pro Asp Asn Thr Gly Val Gly Asn Ser Pro Ser Asp Tyr
        35                  40                  45
Leu His Gly Glu Ala Ile Ile Pro Pro Leu Ser Ser Leu Ser Asn Phe
    50                  55                  60
Asn Asp Lys Arg Phe Met Lys Lys Leu His Met Ile Ala Ala Leu Ala
65                  70                  75                  80
Val Leu Pro Phe Cys Leu Thr Ala Gln Ala Pro Val Ser Asn Ser Glu
                85                  90                  95

Ile Asp Ser Leu Ser Asn Val Gln Leu Gln Thr Val Gln Val Val Ala
            100                 105                 110
Thr Arg Ala Thr Ala Lys Thr Pro Val Ala Tyr Thr Asn Val Arg Lys
        115                 120                 125
Ala Glu Leu Ser Lys Ser Asn Tyr Gly Arg Asp Ile Pro Tyr Leu Leu
    130                 135                 140
Met Leu Thr Pro Ser Val Val Ala Thr Ser Asp Ala Gly Thr Gly Ile
145                 150                 155                 160
Gly Tyr Ser Gly Phe Arg Val Arg Gly Thr Asp Ala Asn Arg Ile Asn
                165                 170                 175
Ile Thr Thr Asn Gly Val Pro Leu Asn Asp Ser Glu Ser Gln Ser Val
            180                 185                 190
Phe Trp Val Asn Met Pro Asp Phe Ala Ser Ser Ile Glu Asp Leu Gln
        195                 200                 205
Val Gln Arg Gly Val Gly Thr Ser Thr Asn Gly Ala Gly Ala Phe Gly
    210                 215                 220
Ala Ser Val Asn Met Arg Thr Asp Asn Leu Gly Leu Ala Pro Tyr Gly
225                 230                 235                 240
Arg Val Asp Leu Ser Gly Gly Ser Phe Gly Thr Phe Arg Arg Ser Val
                245                 250                 255
Lys Leu Gly Ser Gly Arg Ile Gly Arg His Trp Ala Val Asp Ala Arg
            260                 265                 270
Leu Ser Lys Ile Gly Ser Asp Gly Tyr Val Asp Arg Gly Ser Val Asp
        275                 280                 285
Leu Lys Ser Tyr Phe Ala Gln Val Gly Tyr Phe Gly Ser Asn Thr Ala
    290                 295                 300
Leu Arg Phe Ile Thr Phe Gly Gly Lys Glu Val Thr Gly Ile Ala Trp
305                 310                 315                 320
Asn Gly Leu Ser Lys Glu Asp Glu Ala Lys Tyr Gly Arg Arg Tyr Asn
                325                 330                 335
Ser Ala Gly Leu Met Tyr Val Asp Ala Gln Gly Val Pro His Tyr Tyr
            340                 345                 350
His Asn Thr Asp Asn Tyr Glu Gln Arg His Tyr His Ala Ile Met Thr
        355                 360                 365
His Ser Phe Ser Pro Ser Val Ile Leu Asn Leu Thr Ala His Tyr Thr
    370                 375                 380
Ala Gly Tyr Gly Tyr Thr Asp Glu Tyr Arg Thr Gly Arg Lys Leu Lys
385                 390                 395                 400
Glu Tyr Ala Leu Gln Pro Tyr Val Glu Asn Ser Val Thr Val Lys Lys
                405                 410                 415
Thr Asp Leu Ile Arg Gln Lys Tyr Leu Asp Asn Asp Phe Gly Gly Leu
            420                 425                 430
Ile Gly Ser Leu Asn Trp His Thr Gly Ala Trp Asp Leu Gln Phe Gly
        435                 440                 445
Ala Ser Gly Asn Ile Tyr Lys Gly Asp His Phe Gly Arg Ile Thr Tyr
    450                 455                 460
Ile Lys Lys Tyr Asn Gln Pro Leu Ala Pro Asp Phe Glu Tyr Tyr Arg
465                 470                 475                 480
Asn Arg Ala Asp Lys Arg Glu Gly Ala Ala Phe Ala Lys Ala Asn Trp
                485                 490                 495
Gln Ile Thr Pro Glu Leu Asn Met Tyr Ala Asp Leu Gln Tyr Arg Thr
            500                 505                 510
Ile Gly Tyr Thr Ile Asn Gly Ile Thr Asp Glu Tyr Asp Glu Val Gln
        515                 520                 525
Gly Ser Met Gln His Ile Asp Leu Asp Lys Thr Phe Arg Phe Leu Asn
    530                 535                 540
Pro Lys Ala Gly Leu Thr Tyr Ser Phe Asp Asp Ala His Thr Ala Tyr
545                 550                 555                 560
Ala Ser Val Ala Val Ala His Arg Glu Pro Asn Arg Thr Asn Tyr Thr
                565                 570                 575
Glu Ala Gly Ile Gly Gln Tyr Pro Thr Pro Glu Arg Leu Ile Asp Tyr
            580                 585                 590
Glu Leu Gly Tyr Arg Tyr Ala Ser Pro Leu Leu Ser Ala Gly Val Gly
        595                 600                 605
Leu Tyr Tyr Met Gln Tyr Lys Asp Gln Leu Val Leu Asp Gly Arg Leu
    610                 615                 620
Ser Asp Val Gly Gln Met Leu Thr Ser Asn Val Pro Asp Ser Tyr Arg
625                 630                 635                 640
Met Gly Leu Glu Leu Thr Leu Gly Trp Gln Ile Leu Pro Arg Leu Leu
                645                 650                 655
Arg Trp Asp Ala Ser Phe Thr Met Ser Arg Asn Lys Ile Asp Arg Tyr
            660                 665                 670
Val Gln Tyr Thr Ser Val Tyr Asp Ala Asp Tyr Asn Trp Leu Glu Leu
        675                 680                 685
Lys Glu Glu Thr Leu Glu Ser Thr Asp Ile Ala Tyr Ser Pro Asn Val

690                 695                 700
Ile Ala Gly Ser Met Leu Thr Leu Ser His Ala Gly Phe Glu Met Ala
705                 710                 715                 720
Trp Thr Ser Arg Phe Val Ser Lys Gln Tyr Leu Asp Asn Thr Gln Arg
                725                 730                 735
Ser Asp Arg Met Leu Ser Ser Tyr Trp Val Asn Asp Leu Arg Leu Gly
            740                 745                 750
Tyr Val Leu Pro Val His Phe Val Lys Arg Val Ala Leu Gly Val Gln
        755                 760                 765
Leu Asn Asn Leu Phe Asn Leu Met Tyr Ala Ser Asn Ala Tyr Ile Tyr
    770                 775                 780
Asp Ala Gly Tyr Val Gln Ala Ser Gly Glu Leu Ser Ala Tyr Ala Asp
785                 790                 795                 800
Leu Arg Tyr Tyr Pro Gln Ala Gly Phe Asn Ala Leu Gly Ser Leu Thr
                805                 810                 815
Ile Asp Phe

(2) INFORMATION FOR SEQ ID NO:345

    (i) SEQUENCE CHARACTERISTICS:
        (A) LENGTH: 532 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...532

    (xi) SEQUENCE DESCRIPTION: SEQ ID NO:345

Tyr Arg Arg Ser Gly Arg Val Cys Pro Arg Ile Leu Arg Asn Lys Arg
1               5                   10                  15
Ser Tyr Ala Asp Thr Arg Leu Gln Pro Arg Arg Ala Val Arg Leu Arg
            20                  25                  30
Ala Gly Thr Arg Thr Lys Met Lys Arg Arg Phe Leu Ser Leu Leu Leu
        35                  40                  45
Leu Tyr Ile Leu Ser Ser Ile Ser Leu Ser Ala Gln Arg Phe Pro Met
    50                  55                  60
Val Gln Gly Ile Glu Leu Asp Thr Asp Ser Leu Phe Ser Leu Pro Lys
65                  70                  75                  80
Arg Pro Trp Arg Ala Ile Gly Lys Thr Ile Gly Val Asn Leu Ala Val
                85                  90                  95
Trp Gly Phe Asp His Phe Ile Met Asn Glu Asp Phe Ala Asp Ile Ser
            100                 105                 110
Trp Gln Thr Ile Lys Ser Asn Phe Gln Thr Gly Phe Gly Trp Asp Asn
        115                 120                 125
Asp Lys Phe Val Thr Asn Leu Phe Ala His Pro Tyr His Gly Ser Leu
    130                 135                 140
Tyr Phe Asn Ala Ala Arg Ser Asn Gly Leu Ser Phe Arg His Ser Ala
145                 150                 155                 160
Pro Phe Ala Phe Phe Gly Ser Leu Met Trp Glu Leu Leu Met Glu Asn
                165                 170                 175
Glu Pro Pro Ser Ile Asn Asp Leu Cys Ala Thr Thr Ile Gly Gly Ile
            180                 185                 190
Ala Leu Gly Glu Met Gly His Arg Leu Ser Asp Leu Leu Ile Asp Asn
        195                 200                 205
Arg Thr Thr Gly Trp Glu Arg Met Gly Arg Glu Val Ala Ile Ala Leu
    210                 215                 220
Ile Asn Pro Met Arg Phe Leu Asn Arg Leu Thr Ala Gly Glu Val Thr
225                 230                 235                 240
Ser Val Gly Ser Arg Ser Gly Gln Ile Phe Gln Ser Val Pro Ile Asn
                245                 250                 255
Ile Val Val Asp Ala Gly Phe Arg Phe Leu Ala Asp Lys Arg His Ala
            260                 265                 270
Arg Thr Gly Ala Thr Ala Leu Thr Leu Asn Leu Arg Phe Asp Tyr Gly
        275                 280                 285
Asp Pro Phe Arg Ser Glu Thr Phe Ser Pro Tyr Asp Phe Phe Gln Phe

290                 295                 300
Lys Ala Gly Leu Ser Phe Ser Glu Ser Gln Pro Leu Leu Ser Gln Ile
305                 310                 315                 320
Asn Leu Ile Gly Ile Leu Ser Gly Cys Gln Leu Leu Ala His Glu Arg
                325                 330                 335
Thr Val Leu Val Gly Gly Leu Phe Gln His Phe Asp Tyr Tyr Asn Ser
            340                 345                 350
Glu Lys Arg Ile Ser Lys Asn Ser Glu Glu Val Leu Val Thr Pro Tyr
        355                 360                 365
Arg Ile Ser Gln Val Ala Ala Leu Gly Gly Gly Leu Ile Phe Gln His
    370                 375                 380
His Gly Lys Phe Arg Arg Arg Pro Leu Glu Leu Tyr Ala Glu Thr Tyr
385                 390                 395                 400
Leu Asn Val Val Pro Met Gly Ala Ser Leu Ser Asp His Tyr Asn Val
                405                 410                 415
Asp Asn Arg Asp Tyr Asn Leu Gly Ser Gly Leu Ser Gly Lys Leu Tyr
            420                 425                 430
Leu Gly Ala Thr Tyr Asn Asp Leu Trp Ser Trp Leu Leu Gly Val Glu
        435                 440                 445
Ser Tyr Arg Leu Tyr Thr Trp Ile Gly Tyr Glu Glu Pro His Gln Lys
    450                 455                 460
Asn Thr Asp Val Ser Ser Phe Met Val Gln Gly Asp Glu Ser Lys Ala
465                 470                 475                 480
Arg Leu Leu Val Thr Ser Ser Glu Phe Ala Phe His Pro Gly Pro Trp
                485                 490                 495
His Val Ala Ile Val Ala Arg Arg Phe Ile Arg Lys Thr Ala Tyr Gln
            500                 505                 510
Phe Tyr Pro Asn Val Ser Phe Asp Thr Gly Asp Ile Gln Leu Arg Val
        515                 520                 525
Gly Phe His Phe
    530

(2) INFORMATION FOR SEQ ID NO:346

    (i) SEQUENCE CHARACTERISTICS:
        (A) LENGTH: 300 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...300

    (xi) SEQUENCE DESCRIPTION: SEQ ID NO:346

Lys Arg Arg Lys Lys Gln Met Lys Arg Leu Ile Val Phe Leu Ala Met
1               5                   10                  15
Gly Gly Leu Leu Phe Thr Leu Ala Asn Ala Gln Glu Ala Asn Thr Ala
            20                  25                  30
Ser Asp Thr Pro Lys Lys Asp Trp Thr Ile Lys Gly Val Thr Gly Leu
        35                  40                  45
Asn Ala Ser Gln Thr Ser Leu Thr Asn Trp Ala Ala Gly Gly Glu Asn
    50                  55                  60
Thr Val Ala Gly Asn Leu Tyr Leu Asn Ile Asp Ala Asn Tyr Leu Lys
65                  70                  75                  80
Asp Lys Trp Ser Trp Asp Asn Gly Leu Arg Thr Asp Phe Gly Leu Thr
                85                  90                  95
Tyr Thr Thr Ala Asn Lys Trp Asn Lys Ser Val Asp Lys Ile Glu Leu
            100                 105                 110
Phe Thr Lys Ala Gly Tyr Glu Ile Gly Lys His Trp Tyr Gly Ser Ala
        115                 120                 125
Leu Phe Thr Phe Leu Ser Gln Tyr Ala Lys Gly Tyr Glu Lys Pro Ser
    130                 135                 140
Asp His Leu Thr Gly Val Lys His Ile Ser Asn Phe Phe Ala Pro Ala
145                 150                 155                 160
Tyr Leu Thr Leu Gly Ile Gly Ala Asp Tyr Lys Pro Asn Glu Lys Phe
                165                 170                 175
Ser Leu Tyr Leu Ser Pro Thr Thr Gly Lys Leu Thr Val Val Ala Asp

180                 185                 190
Asp Tyr Leu Ser Ser Leu Gly Ala Phe Gly Val Lys Val Gly Glu Lys
        195                 200                 205
Thr Met Phe Glu Leu Gly Ala Leu Val Val Gly Ser Ala Asn Ile Asn
    210                 215                 220
Leu Met Glu Asn Val Asn Leu Ile Thr Lys Ala Ser Phe Phe Ser Ala
225                 230                 235                 240
Tyr Thr His Asp Phe Gly Asn Ile Asp Ile Asn Trp Glu Ala Met Leu
                245                 250                 255
Ala Met Lys Ile Asn Lys Phe Leu Thr Ala Thr Ile Ala Thr Asn Leu
            260                 265                 270
Ile Tyr Asp Asp Asp Val Lys Ile Asn Asp Gly Pro Lys Ile Gln Phe
        275                 280                 285
Lys Glu Val Val Gly Val Gly Val Ala Tyr Thr Phe
    290                 295                 300

(2) INFORMATION FOR SEQ ID NO:347

(i) SEQUENCE CHARACTERISTICS:
(A) LENGTH: 221 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...221

(xi) SEQUENCE DESCRIPTION: SEQ ID NO:347

Thr Arg Glu Ser Val Leu His Cys Arg Thr Lys Leu Lys Lys Glu Arg
1               5                   10                  15
Lys Met Lys Lys Met Ile Leu Ala Ala Thr Met Leu Leu Ala Thr Ile
            20                  25                  30
Gly Phe Ala Asn Ala Gln Ser Arg Pro Ala Leu Arg Leu Asp Ala Asn
        35                  40                  45
Phe Val Gly Ser Asn Leu Met Gln Lys Val Ala Asn Thr Ser Val Asn
    50                  55                  60
Asn Lys Met Ile Val Gly Leu Arg Val Gly Ala Ala Ala Glu Phe Ala
65                  70                  75                  80
Leu Ser Asn Asp Gly Phe Tyr Leu Ala Pro Gly Leu Ala Tyr Thr Met
                85                  90                  95
Arg Gly Ala Lys Met Glu Ser Leu Ser Glu Thr Thr Thr Arg Leu His
            100                 105                 110
Tyr Leu Gln Ile Pro Val Asn Ala Gly Met Arg Phe Ser Phe Ala Asp
        115                 120                 125
Asn Met Ala Ile Ser Leu Glu Ala Gly Pro Tyr Phe Ala Tyr Gly Val
    130                 135                 140
Ala Gly Thr Ile Lys Thr Lys Val Ala Gly Val Thr Ala Ser Val Asp
145                 150                 155                 160
Ala Phe Gly Asp Asn Gly Tyr Asn Arg Phe Asp Leu Gly Leu Gly Leu
                165                 170                 175
Ser Ala Ala Leu Ser Tyr Asp Arg Tyr Tyr Val Gln Ile Gly Tyr Glu
            180                 185                 190
His Gly Leu Leu Asn Met Leu Lys Asp Ala Pro Asp Lys Thr Ser Leu
        195                 200                 205
Arg Asn His Asp Phe Phe Val Gly Leu Gly Val Arg Phe
    210                 215                 220

(2) INFORMATION FOR SEQ ID NO:348

(i) SEQUENCE CHARACTERISTICS:
(A) LENGTH: 248 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...248

(xi) SEQUENCE DESCRIPTION: SEQ ID NO:348

Ile Lys Arg Ile Glu Met Lys Arg Ile Phe Thr Val Ala Leu Val Leu
1               5                   10                  15
Leu Ala Ser Val Thr Met Ala Ile Gly Gln Ser Arg Pro Ala Leu Arg
            20                  25                  30
Val Asp Ala Asn Phe Val Gly Ser Asn Gln Ser Met Lys Arg Asp Gly
        35                  40                  45
Tyr Val Trp Asp Thr Lys Met Asn Val Gly Leu Arg Val Gly Ala Ala
    50                  55                  60
Ala Glu Phe Met Ile Gly Ser Arg Gly Phe Tyr Leu Ala Pro Gly Leu
65                  70                  75                  80
Asn Tyr Thr Met Lys Gly Ser Lys Thr Glu Trp Asp Ile Pro Glu Met
                85                  90                  95
Val Pro Gly Thr Tyr Ile Thr Met Val Ser Thr Arg Leu His Tyr Leu
            100                 105                 110
Gln Leu Pro Ile Asn Ala Gly Met Arg Phe Asp Leu Met Asn Asp Met
        115                 120                 125
Ala Val Ser Ile Glu Ala Gly Pro Phe Leu Ala Tyr Gly Ile Tyr Gly
    130                 135                 140
Thr Tyr Arg Gln Lys Leu Glu Gly Trp Lys Pro Asn Asn Tyr Ser Thr
145                 150                 155                 160
Glu Phe Phe Gly Pro Thr Leu Gly Gly Pro Thr Asn Ile Arg Trp Asp
                165                 170                 175
Ile Gly Ala Asn Ile Ile Ala Ala Phe His Tyr Lys Arg Tyr Tyr Ile
            180                 185                 190
Gln Ile Gly Tyr Glu His Gly Phe Val Asp Ile Val Ser Gly Gly Gly
        195                 200                 205
Ser Asp Ile Pro Arg Leu Asn Asp Asn Arg Gln Ser Ser Ser Thr Thr
    210                 215                 220
Ala Leu Arg Glu Lys Gly Asn Asn Glu Tyr Ala Tyr Asn Arg Asp Phe
225                 230                 235                 240
Phe Val Gly Ile Gly Tyr Arg Phe
                245

(2) INFORMATION FOR SEQ ID NO:349

(i) SEQUENCE CHARACTERISTICS:
(A) LENGTH: 211 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...211

(xi) SEQUENCE DESCRIPTION: SEQ ID NO:349

Lys Arg Lys Ser Met Lys Arg Met Leu Leu Leu Leu Val Val Leu Leu
1               5                   10                  15
Tyr Gly Ile Ala Gly Arg Leu Ala Ala Gln Asp Val Ile Arg Pro Trp
            20                  25                  30
Ser Leu Gln Val Gly Ala Gly Tyr Ser Asp Thr Glu Asn Ile Pro Gly
        35                  40                  45
Gly Phe Thr Tyr Gly Phe Tyr Leu Gly Lys Arg Met Gly Ser Phe Leu
    50                  55                  60
Glu Val Gly Leu Ser Met Tyr Asn Ser Thr Arg Gln Thr Ala Asn Asn
65                  70                  75                  80
Ala Asp Ser Phe Ala Ser Asn Glu Gly Asp Gly Ser Phe Gln Val Asn
                85                  90                  95

Met Ser Ser Pro Asn Glu Lys Trp Ser Phe Phe Asp Ala Gly Ser Ala
            100                 105                 110
Asn Cys Tyr Met Ile Val Val Gly Val Asn Pro Leu His Leu Phe Trp
        115                 120                 125
Gln Asn Ser Arg His Asn Leu Phe Leu Ala Val Gln Ala Gly Leu Ser
    130                 135                 140
Asn Lys His Asn Ile His Phe Ile Tyr Gly Asp Lys Gly Ala Lys Val
145                 150                 155                 160
Ser Ile Tyr Thr Asn Ser Asn Thr Tyr Ile Gly Tyr Gly Ala Arg Val
                165                 170                 175
Ala Tyr Glu Tyr Gln Ile His Lys Asn Val Gly Ala Gly Ala Ala Val
            180                 185                 190
Met Tyr Asp His Gly Asn Lys Met Leu Thr Ala Met Ala Thr Leu Ser
        195                 200                 205
Thr His Phe
    210

(2) INFORMATION FOR SEQ ID NO:350

     (i) SEQUENCE CHARACTERISTICS:
          (A) LENGTH: 953 amino acids
          (B) TYPE: amino acid
          (D) TOPOLOGY: linear

    (ii) MOLECULE TYPE: protein

   (iii) HYPOTHETICAL: YES

    (vi) ORIGINAL SOURCE:
          (A) ORGANISM: Porphyromonas gingivalis

    (ix) FEATURE:
          (A) NAME/KEY: misc_feature
          (B) LOCATION 1...953

    (xi) SEQUENCE DESCRIPTION: SEQ ID NO:350

Ile Arg Met Arg Val Ser Asp Leu Cys Ser Arg Leu Ser Trp Leu Leu
1               5                   10                  15
Pro Val Ile Leu Val Gly Leu Leu Cys Ala Thr Leu Val Ala Ala Glu
            20                  25                  30
Arg Pro Met Ala Gly Ala Val Gly Leu His His Arg Arg His Ala Ala
        35                  40                  45
Leu Ser Asp Ser Thr Ala Lys Asp Thr Val Pro Leu Ala Lys Pro Ile
    50                  55                  60
Pro Asp Ser Ala Phe Arg Asp Ser Leu Pro Ala Asp Ser Thr Gly Ser
65                  70                  75                  80
Met Arg Gln Asp Ser Val Tyr Asp Asp Glu Phe Glu Leu Glu Asp Ile
                85                  90                  95
Val Glu Tyr Glu Ala Ala Asp Ser Ile Val Leu Leu Gly Gln Asn Arg
            100                 105                 110
Ala Tyr Leu Phe Gly Lys Ser Tyr Val Ser Tyr Gln Lys Ser Arg Leu
        115                 120                 125
Glu Ala Asn Phe Met Tyr Leu Asn Thr Asp Ser Ser Thr Val Tyr Thr
    130                 135                 140
Arg Tyr Val Leu Asp Thr Ala Gly Tyr Pro Met Ala Phe Pro Val Phe
145                 150                 155                 160
Lys Asp Gly Glu Gln Ser Phe Glu Ala Lys Asn Phe Thr Tyr Asn Phe
                165                 170                 175
Arg Thr Glu Lys Gly Ile Ile Ser Gly Val Ile Thr Gln Gln Gly Glu
            180                 185                 190
Gly Tyr Leu Thr Ala Gly Lys Thr Lys Lys Met Pro Asp Asn Ile Met
        195                 200                 205
Phe Met Gln Gly Gly Arg Tyr Thr Thr Cys Asp Asn His Asp His Pro
    210                 215                 220
His Phe Tyr Ile Asn Leu Ser Lys Ala Lys Val His Pro Glu Lys Asp
225                 230                 235                 240
Ile Val Thr Gly Pro Val Asn Leu Val Ile Ala Asp Met Pro Leu Pro
                245                 250                 255
Ile Gly Leu Pro Phe Gly Tyr Phe Pro Phe Ser Asn Lys Tyr Ser Ser
            260                 265                 270
Gly Ile Leu Met Pro Thr Tyr Gly Glu Asp Asn Arg Tyr Gly Phe Tyr
        275                 280                 285
Leu Arg Asn Gly Gly Tyr Tyr Phe Ala Phe Ser Asp Tyr Ile Asp Leu
    290                 295                 300

Ala Leu Arg Gly Glu Ile Phe Ser Lys Gly Ser Trp Gly Ile Ser Ala
305                 310                 315                 320
Gln Ser Lys Tyr Lys Lys Arg Tyr Lys Tyr Asn Gly Ser Phe Glu Ala
                325                 330                 335
Asn Tyr Leu Val Ser Lys Ser Gly Asp Lys Tyr Val Pro Gly Asp Tyr
            340                 345                 350
Ser Lys Thr Thr Ser Leu Asn Ile Arg Trp Thr His Ser Gln Asp Pro
        355                 360                 365
Lys Ala Asn Pro Leu Gln Thr Leu Ser Ala Asn Val Asn Phe Ala Thr
    370                 375                 380
Gly Ser Tyr Phe Gln Asn Ser Leu Asn Thr Thr Tyr Asp Val Asn Ala
385                 390                 395                 400
Arg Thr Ala Thr Thr Arg Ser Ser Ala Val Ser Tyr Ser Arg Lys Phe
                405                 410                 415
Pro Gly Thr Pro Phe Ser Ile Thr Gly Ser Met Asp Ile Ser Gln Asn
            420                 425                 430
Met Arg Asp Thr Thr Val Ser Leu Thr Leu Pro Asn Leu Ser Ile Asn
        435                 440                 445
Met Ser Thr Arg Tyr Pro Phe Lys Arg Lys Thr Arg Val Gly Pro Glu
    450                 455                 460
Arg Trp Tyr Glu Lys Leu Ser Val Gly Tyr Ser Gly Gln Leu Arg Asn
465                 470                 475                 480
Ser Ile Leu Thr Lys Glu Lys Asp Leu Leu Gln Ser Asn Leu Val Arg
                405                 490                 495
Asp Trp Lys Asn Gly Met Arg His Ser Val Pro Ile Ser Leu Thr Val
            500                 505                 510
Pro Leu Leu Asp Tyr Ile Asn Leu Thr Met Gly Val Asn Tyr Asn Glu
        515                 520                 525
Trp Trp Tyr Thr Lys Gly Ile Arg Lys Ser Trp Asn Glu Asp Lys Lys
    530                 535                 540
Thr Phe Leu Pro Ser Asp Thr Thr Tyr Lys Phe Arg Arg Leu Tyr Asp
545                 550                 555                 560
Tyr Ser Leu Ser Ala Gly Leu Ser Thr Thr Leu Tyr Gly Met Phe Lys
                565                 570                 575
Pro Trp Lys Pro Phe Ser Phe Gly Gly Asn Leu Ile Met Ile Arg His
            580                 585                 590
Arg Phe Thr Pro Thr Val Ser Phe Ser Tyr Met Pro Asp Phe Thr Lys
        595                 600                 605
Arg Arg Tyr Gly Phe Trp Glu Leu Leu Glu His Thr Asp Gln Asn Gly
    610                 615                 620
Lys Leu His Thr Leu Leu Tyr Ser Pro Tyr Phe Glu Gln Ile Phe Gly
625                 630                 635                 640
Ala Pro Ser Met Gly Asn Ala Gly Ser Val Asn Phe Ser Phe Asp Asn
                645                 650                 655
Asn Leu Glu Ala Lys Ile Lys Ser Lys Ser Asp Ser Thr Gly Ile Lys
            660                 665                 670
Lys Ile Ser Leu Ile Asp Gln Phe Thr Trp Ser Thr Ser Tyr Asn Met
        675                 680                 685
Phe Ala Asp Ser Ile Arg Trp Ser Asn Ile Ser Ala Ser Leu Ala Leu
    690                 695                 700
Arg Leu Ser Lys Ser Phe Thr Leu Arg Leu Ser Gly Leu Phe Asp Pro
705                 710                 715                 720
Tyr Leu Thr Lys Tyr Tyr Glu Gly Glu Asp Gly Lys Ile Ile Pro Tyr
                725                 730                 735
Lys Ser Asn Asp Leu Arg Ile Phe Asn Gly Lys Gly Leu Ala Arg Leu
            740                 745                 750
Ile Ser Thr Gly Thr Ser Phe Ser Tyr Thr Leu Asn Lys Glu Ser Leu
        755                 760                 765
Ser Gly Leu Ile Ala Leu Phe Ser Gly Lys Lys Glu Arg Arg Asp Glu
    770                 775                 780
Lys Lys Asn Thr Gly Ala Thr Pro His Glu Gly Asp Asp Ala Ala Asp
785                 790                 795                 800
Ile Leu Glu Gly Gly Arg Pro Gln Asn Glu Ser Gly Gly Ser Leu Leu
                805                 810                 815
Glu Arg Asn Arg Gln Gly Gly Ala Val Asp Gln Asp Gly Tyr Phe Ala
            820                 825                 830
Tyr Ser Ile Pro Trp Ser Leu Ser Phe Asp Tyr Ser Trp Asn Ile Ala
        835                 840                 845
Thr Asp Tyr Asn Arg Tyr Asn Val Asn Lys Met Glu His Tyr Tyr Arg
    850                 855                 860
Val Thr Gln Asn Leu Ser Phe Arg Gly Asn Ile Gln Pro Thr Pro Asn
865                 870                 875                 880
Trp Ser Phe Gly Phe Asn Ala Asn Tyr Asn Phe Asp Leu Lys Lys Ile
                885                 890                 895
Thr Ser Leu Thr Cys Asn Val Thr Arg Asp Met His Cys Trp Ala Ile

900                 905                 910
Ser Ala Ser Phe Ile Pro Ile Gly Ala Tyr Lys Ser Tyr Asn Phe Val
        915                 920                 925
Ile Ser Val Lys Ser Ser Leu Leu Gln Asp Leu Lys Tyr Gln Gln Ser
    930                 935                 940
Asn Arg Pro Ile Thr Asn Thr Trp Tyr
945                 950

(2) INFORMATION FOR SEQ ID NO:351

     (i) SEQUENCE CHARACTERISTICS:
          (A) LENGTH: 1251 amino acids
          (B) TYPE: amino acid
          (D) TOPOLOGY: linear

    (ii) MOLECULE TYPE: protein

   (iii) HYPOTHETICAL: YES

    (vi) ORIGINAL SOURCE:
          (A) ORGANISM: Porphyromonas gingivalis

    (ix) FEATURE:
          (A) NAME/KEY: misc_feature
          (B) LOCATION 1...1251

    (xi) SEQUENCE DESCRIPTION: SEQ ID NO:351

Lys Trp Lys Leu Ala Cys Ala Phe Asp Cys Ala Cys Cys Phe Asp Pro
1               5                   10                  15
Phe Val Val Thr Asn Glu Val Ile Ile Met Met Lys Arg Tyr Thr Ile
            20                  25                  30
Ile Leu Ala Val Phe Leu Leu Phe Cys Thr Val Phe Thr Phe Gln Ile
        35                  40                  45
Lys Ala Arg Pro Tyr Glu Arg Phe Ala Asp Val Glu Lys Pro Trp Ile
    50                  55                  60
Gln Lys His Ser Met Asp Ser Lys Leu Val Pro Ala Asn Lys Gly Asn
65                  70                  75                  80
Leu Ile Gln Ala Glu Ile Val Tyr Gln Ser Val Ser Glu His Ser Asp
                85                  90                  95
Leu Val Ile Ser Pro Val Asn Glu Ile Arg Pro Ala Asn Arg Phe Pro
            100                 105                 110
Ser His Arg Lys Ser Phe Phe Ala Glu Asn Leu Arg Ala Ser Pro Pro
        115                 120                 125
Val Val Pro Val Ala Val Asp Lys Tyr Ala Val Pro Val Ala Asn Pro
    130                 135                 140
Met Asp Pro Glu Asn Pro Asn Ala Trp Asp Val Thr Leu Lys Ile Thr
145                 150                 155                 160
Thr Lys Ala Val Thr Val Pro Val Asp Val Val Met Val Ile Asp Gln
                165                 170                 175
Ser Ser Ser Met Gly Gly Gln Asn Ile Ala Arg Leu Lys Ser Ala Ile
            180                 185                 190
Ala Ser Gly Gln Arg Phe Val Lys Lys Met Leu Pro Lys Gly Thr Ala
        195                 200                 205
Thr Glu Gly Val Arg Ile Ala Leu Val Ser Tyr Asp His Glu Pro His
    210                 215                 220
Arg Leu Ser Asp Phe Thr Lys Asp Thr Ala Phe Leu Cys Gln Lys Ile
225                 230                 235                 240
Arg Ala Leu Thr Pro Ile Trp Gly Thr His Thr Gln Gly Gly Leu Lys
                245                 250                 255
Met Ala Arg Asn Ile Met Ala Thr Ser Thr Ala Val Asp Lys His Ile
            260                 265                 270
Ile Leu Met Ser Asp Gly Leu Ala Thr Glu Gln Tyr Pro Val Lys Asn
        275                 280                 285
Val Thr Thr Ala Asp Phe Ile Gly Lys Thr Gly Asn Ala Asn Asp Pro
    290                 295                 300
Ile Asp Leu Val Ile Gln Gly Ala Ile Asn Phe Pro Thr Asn Tyr Val
305                 310                 315                 320
Ser Asn Asn Pro Ser Thr Pro Leu Thr Pro Asn Tyr Pro Thr His Ser
                325                 330                 335
Ser Lys Val Gly Arg Arg Asn Leu Pro Glu Ser Lys Phe Asp Tyr Ser
            340                 345                 350
Asn Leu Ser Ala Arg Ile Thr Phe Asp Gly Val Ala Gly Ala Leu Val
        355                 360                 365
Tyr Glu Pro Arg Phe Pro His Pro Tyr Tyr Tyr Tyr Phe Pro Cys Asn

370                 375                 380
Ala Ala Ile Asn Glu Ala Gln Phe Ala Lys Asn Ser Gly Tyr Thr Ile
385                 390                 395                 400
His Thr Ile Gly Tyr Asp Leu Gly Asp Phe Ala Leu Ala Asn Asn Ser
                405                 410                 415
Leu Lys Leu Thr Ala Thr Asp Glu Asn His Phe Phe Thr Ala Thr Pro
            420                 425                 430
Ala Asn Leu Ala Ala Ala Phe Asp Asn Ile Ala Gln Thr Ile Asn Ile
        435                 440                 445
Gly Ile Gln Arg Gly Glu Val Thr Asp Phe Val Ala Pro Gly Phe Ile
    450                 455                 460
Val Lys Asn Leu Thr Gln Ser Gly Asp Val Thr His Leu Leu Asn Val
465                 470                 475                 480
Ser Asn Gly Thr Val His Tyr Asp Val Ser Thr Lys Lys Leu Thr Trp
                485                 490                 495
Thr Thr Gly Thr Ile Leu Ser Ser Ser Glu Ala Thr Ile Thr Tyr Arg
            500                 505                 510
Ile Tyr Ala Asp Leu Asp Tyr Ile Gln Asn Asn Asp Ile Pro Val Asn
        515                 520                 525
Thr Thr Ser Ala Ile Gly Pro Asp Leu Gly Gly Phe Asp Thr Asn Thr
    530                 535                 540
Glu Ala Lys Leu Thr Tyr Thr Asn Ser Asn Gly Glu Pro Asn Gln Gln
545                 550                 555                 560
Leu Ile Phe Pro Arg Pro Thr Val Lys Leu Gly Tyr Gly Val Ile Lys
                565                 570                 575
Arg His Tyr Val Leu Val Asn Lys Asp Gly Gln Pro Ile Gln Ala Asn
            580                 585                 590
Gly Thr Val Val Ser Ser Leu Ser Glu Ala His Val Leu Gln Ser Gln
        595                 600                 605
Asp Phe Phe Leu Pro Ser Gly Gly Gly His Ile Val Pro Lys Trp Ile
    610                 615                 620
Lys Leu Asp Lys Thr Thr Glu Ala Leu Gln Tyr Tyr Ser Val Pro Pro
625                 630                 635                 640
Thr Asn Thr Val Ile Thr Thr Ala Asp Gly Lys Arg Tyr Arg Phe Val
                645                 650                 655
Glu Val Pro Gly Ser Thr Pro Asn Pro Gly Gln Ile Gly Ile Ser Trp
            660                 665                 670
Lys Lys Pro Ala Gly Asn Ala Tyr Phe Ala Tyr Lys Leu Leu Asn Tyr
        675                 680                 685
Trp Met Gly Gly Thr Thr Asp Gln Gln Ser Glu Trp Asp Val Thr Ser
    690                 695                 700
Asn Trp Thr Gly Ala Gln Val Pro Leu Thr Gly Glu Asp Val Glu Phe
705                 710                 715                 720
Ala Thr Thr Glu Asn Phe Gly Ser Pro Ala Val Ala Asp Leu His Val
                725                 730                 735
Pro Thr Thr Asn Pro Lys Ile Ile Gly Asn Leu Ile Asn Asn Ser Asp
            740                 745                 750
Lys Asp Leu Val Val Thr Thr Ser Ser Gln Leu Thr Ile Asn Gly Val
        755                 760                 765
Val Glu Asp Asn Asn Pro Asn Val Gly Thr Ile Val Val Lys Ser Ser
    770                 775                 780
Lys Asp Asn Pro Thr Gly Thr Leu Leu Phe Ala Asn Pro Gly Tyr Asn
785                 790                 795                 800
Gln Asn Val Gly Gly Thr Val Glu Phe Tyr Asn Gln Gly Tyr Asp Cys
                805                 810                 815
Ala Asp Cys Gly Met Tyr Arg Arg Ser Trp Gln Tyr Phe Gly Ile Pro
            820                 825                 830
Val Asn Glu Ser Gly Phe Pro Ile Asn Asp Val Gly Gly Asn Glu Thr
        835                 840                 845
Val Asn Gln Trp Val Glu Pro Phe Asn Gly Asp Lys Trp Arg Pro Ala
    850                 855                 860
Pro Tyr Ala Pro Asp Thr Glu Leu Gln Lys Phe Lys Gly Tyr Gln Ile
865                 870                 875                 880
Thr Asn Asp Val Gln Ala Gln Pro Thr Gly Val Tyr Ser Phe Lys Gly
                885                 890                 895
Met Ile Cys Val Cys Asp Ala Phe Leu Asn Leu Thr Arg Thr Ser Gly
            900                 905                 910
Val Asn Tyr Ser Gly Ala Asn Leu Ile Gly Asn Ser Tyr Thr Gly Ala
        915                 920                 925
Ile Asp Ile Lys Gln Gly Ile Val Phe Pro Pro Glu Val Glu Gln Thr
    930                 935                 940
Val Tyr Leu Phe Asn Thr Gly Thr Arg Asp Gln Trp Arg Lys Leu Asn
945                 950                 955                 960
Gly Ser Thr Val Ser Gly Tyr Arg Ala Gly Gln Tyr Leu Ser Val Pro
                965                 970                 975

Lys Asn Thr Ala Gly Gln Asp Asn Leu Pro Asp Arg Ile Pro Ser Met
            980                 985                 990
His Ser Phe Leu Val Lys Met Gln Asn Gly Ala Ser Cys Thr Leu Unk
        995                 1000                1005
Ile Leu Tyr Asp Lys Leu Leu Lys Asn Thr Thr Val Asn Asn Gly Asn
    1010                1015                1020
Gly Thr Gln Ile Thr Trp Arg Ser Gly Asn Ser Gly Ser Ala Asn Met
1025                1030                1035                1040
Pro Ser Leu Val Met Asp Val Leu Gly Asn Glu Ser Ala Asp Arg Leu
                1045                1050                1055
Trp Ile Phe Thr Asp Gly Gly Leu Ser Phe Gly Phe Asp Asn Gly Trp
            1060                1065                1070
Asp Gly Arg Lys Leu Thr Glu Lys Gly Leu Ser Gln Leu Tyr Ala Met
        1075                1080                1085
Ser Asp Ile Gly Asn Asp Lys Phe Gln Val Ala Gly Val Pro Glu Leu
    1090                1095                1100
Asn Asn Leu Leu Ile Gly Phe Asp Ala Asp Lys Asp Gly Gln Tyr Thr
1105                1110                1115                1120
Leu Glu Phe Ala Leu Ser Asp His Phe Ala Lys Gly Ala Val Tyr Leu
                1125                1130                1135
His Asp Leu Gln Ser Gly Ala Lys His Arg Ile Thr Asn Ser Thr Ser
            1140                1145                1150
Tyr Ser Phe Asp Ala Lys Arg Gly Asp Ser Gly Ala Arg Phe Arg Leu
        1155                1160                1165
Ser Tyr Gly Cys Asp Glu Asn Val Asp Asp Ser His Val Val Ser Thr
    1170                1175                1180
Asn Gly Arg Glu Ile Ile Ile Leu Asn Gln Asp Ala Leu Asp Cys Thr
1185                1190                1195                1200
Val Thr Leu Phe Thr Ile Glu Gly Lys Leu Leu Arg Arg Leu Lys Val
                1205                1210                1215
Leu Ala Gly His Arg Glu Val Met Lys Val Gln Thr Gly Gly Ala Tyr
            1220                1225                1230
Ile Val His Leu Gln Asn Ala Phe Thr Asn Asp Val His Lys Val Leu
        1235                1240                1245
Val Glu Tyr
    1250

(2) INFORMATION FOR SEQ ID NO:352

    (i) SEQUENCE CHARACTERISTICS:
        (A) LENGTH: 426 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...426

    (xi) SEQUENCE DESCRIPTION: SEQ ID NO:352

Thr Met Glu Val Lys Lys Asn Thr Val Val Leu Arg Leu Leu Ile Trp
1               5                   10                  15
Phe Val Ala Ile Leu Leu Phe His Ser Ser Arg Leu Trp Gly Gln Glu
            20                  25                  30
Gly Glu Gly Ser Ala Arg Tyr Arg Phe Lys Gly Phe Val Asp Thr Tyr
        35                  40                  45
His Ala Val Arg Ser Ser Ser Pro Phe Asp Phe Met Ser Ser Arg Thr
    50                  55                  60
Arg Val Arg Gly Glu Leu Glu Arg Ser Phe Gly Asn Ser Lys Val Ala
65                  70                  75                  80
Val Ser Val Asn Ala Thr Tyr Asn Ala Leu Leu Lys Asp Glu Thr Gly
                85                  90                  95
Leu Arg Leu Arg Glu Ala Phe Phe Glu His Gln Glu Glu His Trp Gly
            100                 105                 110
Leu Arg Leu Gly Arg Gln Ile Val Ile Trp Gly Ala Ala Asp Gly Val
        115                 120                 125
Arg Ile Thr Asp Leu Ile Ser Pro Met Asp Met Thr Glu Phe Leu Ala
    130                 135                 140

Gln Asp Tyr Asp Asp Ile Arg Met Pro Val Asn Ala Leu Arg Phe Ser
145                 150                 155                 160
Val Phe Asn Glu Ser Met Lys Val Glu Val Val Val Leu Pro Val Phe
                165                 170                 175
Glu Gly Tyr Arg Leu Pro Val Asp Pro Arg Asn Pro Trp Asn Ile Phe
            180                 185                 190
Ser Leu Ser Pro Ile Ala Gln Gly Met Asn Ile Val Trp Lys Glu Glu
        195                 200                 205
Ala Gly Lys Pro Ala Phe Lys Val Ala Asn Ile Glu Tyr Gly Ala Arg
    210                 215                 220
Trp Ser Thr Thr Leu Ser Gly Ile Asp Phe Ala Leu Ala Ala Leu His
225                 230                 235                 240
Thr Trp Asn Lys Met Pro Val Ile Glu Val Gln Gly Ile Val Pro Thr
                245                 250                 255
Glu Ile Ile Val Ser Pro Arg Tyr Tyr Arg Met Gly Phe Val Gly Gly
            260                 265                 270
Asp Leu Ser Val Pro Val Gly Gln Phe Val Phe Arg Gly Glu Ala Ala
        275                 280                 285
Phe Asn Ile Asp Lys His Phe Thr Tyr Lys Ser His Ala Glu Gln Glu
    290                 295                 300
Gly Phe Gln Thr Ile Asn Trp Leu Ala Gly Ala Asp Trp Tyr Ala Pro
305                 310                 315                 320
Gly Glu Trp Met Ile Ser Gly Gln Phe Ser Met Glu Ser Ile Phe Arg
                325                 330                 335
Tyr Arg Asp Phe Ile Ser Gln Arg Gln His Ser Thr Leu Ile Thr Leu
            340                 345                 350
Asn Val Ser Lys Lys Phe Phe Gly Ser Thr Leu Gln Leu Ser Asp Phe
        355                 360                 365
Thr Tyr Tyr Asp Leu Thr Gly Lys Gly Trp Phe Ser Arg Phe Ala Ala
    370                 375                 380
Asp Tyr Ala Leu Asn Asp Gln Ile His Leu Met Ala Gly Tyr Asp Trp
385                 390                 395                 400
Phe Ser Ser Lys Gly Ser Gly Ile Phe Asp Arg Tyr Lys Asp Asn Ser
                405                 410                 415
Glu Leu Trp Phe Lys Ala Arg Tyr Ser Phe
            420                 425

(2) INFORMATION FOR SEQ ID NO:353

(i) SEQUENCE CHARACTERISTICS:
(A) LENGTH: 464 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...464

(xi) SEQUENCE DESCRIPTION: SEQ ID NO:353

Tyr Gly Lys Arg Arg Lys Leu Gly Thr Ser Val Arg Pro Ser Val Leu
1               5                   10                  15
Thr Gln Ile Arg Phe Ile Leu Asp Leu His Leu Ile Thr Asp Phe Phe
            20                  25                  30
Glu Gly Leu Arg Val Asn Pro Ile Gly Ala Ala Ala Ile Val Ala Phe
        35                  40                  45
Ile Ile Asp Leu Leu Leu Leu Cys Cys Ser Ala Phe Met Ser Ser Cys
    50                  55                  60
Glu Val Ala Tyr Phe Ser Leu Lys Pro Ile Asp Leu Gln Asn Ile Arg
65                  70                  75                  80
Glu Arg Asn His Ser Ser Asp Ile Ala Leu Ser Asn Leu Leu Asp Asn
                85                  90                  95
Ser Asn Gln Leu Leu Ala Thr Ile Leu Ile Gly Asn Asn Val Ile Asn
            100                 105                 110
Val Ala Ile Val Ile Leu Ser Asn Tyr Ala Ile Glu Gln Thr Phe Val
        115                 120                 125
Phe Ser Ser Pro Ile Ile Gly Phe Leu Ile Gln Thr Ile Leu Leu Thr
    130                 135                 140

Thr Val Leu Leu Leu Phe Gly Glu Ile Leu Pro Lys Val Tyr Ala Arg
145                 150                 155                 160
Lys Asn Pro Leu Gln Tyr Ser Arg Phe Ser Ala Ala Ala Met Ser Val
                165                 170                 175
Ile Tyr Lys Ile Leu Ser Pro Phe Ser Lys Leu Leu Val Lys Ser Thr
            180                 185                 190
Gly Ile Val Thr Arg Gly Ile Ser Lys Lys Lys Tyr Asp Met Ser Val
        195                 200                 205
Asp Glu Leu Ser Lys Ala Val Ala Leu Thr Thr Thr Glu Gly Glu Pro
    210                 215                 220
Glu Glu Lys Glu Met Ile Asn Glu Ile Ile Lys Phe Tyr Asn Lys Thr
225                 230                 235                 240
Ala Cys Glu Ile Met Val Pro Arg Ile Asp Ile Val Asp Val Asp Leu
                245                 250                 255
Ser Trp Pro Phe Arg Lys Met Leu Asp Phe Val Val Ser Ser Gly Tyr
            260                 265                 270
Ser Arg Leu Pro Val Ser Glu Gly Ser Glu Asp Asn Ile Lys Gly Val
        275                 280                 285
Ile Tyr Ile Lys Asp Leu Ile Pro His Met Asp Lys Gly Asp Glu Phe
    290                 295                 300
Asp Trp His Pro Leu Ile Arg Lys Ala Tyr Phe Val Pro Glu Asn Lys
305                 310                 315                 320
Arg Ile Asp Asp Leu Leu Glu Glu Phe Arg Ala Asn Lys Val His Val
                325                 330                 335
Ser Ile Val Val Asp Glu Phe Gly Gly Thr Cys Gly Leu Ile Thr Met
            340                 345                 350
Glu Asp Ile Leu Glu Glu Ile Val Gly Glu Ile Thr Asp Glu Tyr Asp
        355                 360                 365
Glu Glu Glu Leu Pro Phe Lys Val Leu Gly Asp Gly Ser Tyr Leu Phe
    370                 375                 380
Glu Gly Lys Thr Ser Leu Ser Asp Val Arg His Tyr Leu Asp Leu Pro
385                 390                 395                 400
Glu Asn Ala Phe Gly Glu Leu Gly Asp Glu Val Asp Thr Leu Ser Gly
                405                 410                 415
Leu Phe Leu Glu Ile Lys Gln Glu Leu Pro His Val Gly Asp Thr Ala
            420                 425                 430
Val Tyr Glu Pro Phe Arg Phe Gln Val Thr Gln Met Asp Lys Arg Arg
        435                 440                 445
Ile Ile Glu Ile Lys Ile Phe Pro Phe Glu Arg Thr Trp Glu Val Glu
    450                 455                 460

(2) INFORMATION FOR SEQ ID NO:354

    (i) SEQUENCE CHARACTERISTICS:
        (A) LENGTH: 266 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...266

    (xi) SEQUENCE DESCRIPTION: SEQ ID NO:354

Ile Ile Tyr Arg Ser Thr Met Lys Leu Leu Leu Tyr Leu Leu Leu Val
1               5                   10                  15
Leu Ser Thr Leu Ser Pro Met Tyr Ser Gln Met Leu Phe Ser Glu Asn
            20                  25                  30
Leu Thr Met Asn Ile Asp Ser Thr Lys Thr Ile Gln Gly Thr Ile Leu
        35                  40                  45
Pro Val Leu Asp Phe Lys Thr Glu Lys Glu Asn Val Phe Thr Phe Lys
    50                  55                  60
Asn Thr Ala Asn Leu Asn Leu Leu Ile Lys His Gly Gln Val Ile Asn
65                  70                  75                  80
Leu Ile Asn Lys Leu Glu Phe Ser Thr Tyr Gly Asn Lys Val Thr Val
                85                  90                  95
Ser Gly Gly Tyr Val His Thr Glu Tyr Arg Tyr Leu Leu His His Val
            100                 105                 110

Phe Glu Val Tyr Pro Tyr Val Glu Ser Gln Trp Ala Glu Ser Arg Gly
        115                 120                 125
Met Lys Tyr Lys Val Ser Thr Gly Leu Gln Ser Arg Tyr Arg Leu Val
    130                 135                 140
Asn Ser Asp Asn Cys Leu Met Phe Ala Thr Leu Gly Val Phe Phe Glu
145                 150                 155                 160
Phe Glu Lys Trp Glu Gln Pro Ala Thr Ser Leu Phe Ala Gly Thr Tyr
                165                 170                 175
Ala Tyr Ser Arg Ser Ile Lys Ser His Leu Ser Ile Ser Phe Arg His
            180                 185                 190
Arg Leu Gly Glu His Trp Glu Phe Thr Thr Thr Ala Ile His Gln Gly
        195                 200                 205
Lys Pro Asp Ser Tyr Phe Lys Lys Ala Arg Phe Gly Gly Ala Ile Asp
    210                 215                 220
Leu Lys Tyr His Ile Thr Pro Thr Ile Gly Ile Arg Gly Ala Tyr Arg
225                 230                 235                 240
Ile Ile Tyr Asp Thr Ala Pro Ile Val Pro Val Arg Lys Asp Tyr Asn
                245                 250                 255
Thr Val Asp Val Gly Ile Asp Ile Ser Phe
            260                 265

(2) INFORMATION FOR SEQ ID NO:355

    (i) SEQUENCE CHARACTERISTICS:
        (A) LENGTH: 907 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...907

    (xi) SEQUENCE DESCRIPTION: SEQ ID NO:355

Thr Ile Cys Val Ala Phe Leu Ser Ala Pro Val Ala Ala Leu Arg Glu
1               5                   10                  15
Ser Pro Pro Met Gly Ala Glu Arg Lys Thr Pro Ser Leu Leu Pro Leu
            20                  25                  30
Leu Phe Glu His Ser Asp Lys Gly Lys Gly Phe Asp Tyr Arg Leu Phe
        35                  40                  45
Thr Ser Asn Lys Leu Lys Val Phe Ala Thr Gly Asn Ser Arg Tyr Ile
    50                  55                  60
His Asn Lys Pro Thr Ile Ile Gln Ala Met Lys Arg Ile Val Leu Ser
65                  70                  75                  80
Ser Phe Leu Phe Val Leu Ser Ile Leu Ser Leu Met Ala Gln Asn Asn
                85                  90                  95
Thr Leu Asp Val His Ile Ser Gly Thr Ile Lys Asp Ala Ser Ser Gly
            100                 105                 110
Glu Pro Val Pro Tyr Ala Thr Val Ser Ile Arg Leu Thr Gly Ala Asp
        115                 120                 125
Thr Thr Gln Val Phe Arg Gln Val Thr Asp Gly Asn Gly Tyr Phe Val
    130                 135                 140
Ile Gly Leu Pro Ala Ala Pro Ser Tyr His Leu Thr Ala Ser Phe Val
145                 150                 155                 160
Gly Met Lys Thr His Thr Met Gln Ile Ser Arg Gly Asn Gly Gln His
                165                 170                 175
Asp Ile Lys Ser Ile Asp Ile Ser Leu Glu Ser Glu Asp Lys Gln Leu
            180                 185                 190
Ser Thr Val Thr Val Ser Ala Ala Arg Pro Leu Val Lys Met Glu Ile
        195                 200                 205
Asp Arg Leu Ser Tyr Asn Met Lys Asp Asp Pro Ala Ala Lys Thr Asn
    210                 215                 220
Asn Leu Leu Glu Met Leu Arg Asn Val Pro Leu Val Thr Val Asp Gly
225                 230                 235                 240
Gln Gly Asn Ile Gln Val Lys Gly Ser Ser Asn Phe Lys Ile His Leu
                245                 250                 255
Asn Gly Arg Pro Ser Thr Met Val Ser Ser Asn Pro Lys Glu Val Phe
            260                 265                 270

Arg Ser Ile Pro Ala His Thr Ile Lys Arg Val Glu Val Ile Thr Asp
        275                 280                 285
Pro Gly Val Lys Tyr Asp Ala Glu Gly Thr Ser Ala Ile Leu Asp Ile
    290                 295                 300
Val Thr Glu Glu Gly Lys Lys Leu Glu Gly Tyr Ser Gly Ser Ile Thr
305                 310                 315                 320
Ala Ser Val Ser Asn Asn Pro Thr Ala Asn Gly Ser Ile Phe Leu Thr
                325                 330                 335
Ala Lys Ser Gly Lys Val Gly Leu Thr Thr Asn Tyr Asn Tyr Tyr Gly
            340                 345                 350
Gly Lys Asn Lys Gly Ser Arg Tyr Phe Thr Glu Arg Thr Thr Ser Met
        355                 360                 365
Leu Gln Thr Ile Glu Glu Gly Lys Gly Gln Glu Thr Phe Gly Gly His
    370                 375                 380
Phe Gly Asn Ala Leu Leu Ser Phe Glu Ile Asp Ser Leu Asn Leu Phe
385                 390                 395                 400
Thr Val Gly Gly Asn Val Arg Leu Trp Glu Met Thr Thr Asp Arg Asn
                405                 410                 415
Ser Val Glu Lys Ser Phe Ala Gly Ser Asn Leu Met Ser Tyr Ile Asp
            420                 425                 430
Arg Lys Leu Lys Thr Gln Met Asp Ala Gly Ser Tyr Glu Leu Asn Ala
        435                 440                 445
Asp Tyr Gln His Ser Thr Arg Leu Pro Gly Glu Leu Leu Thr Val Ser
    450                 455                 460
Tyr Arg Phe Thr His Asn Pro Asn Asn Ser Glu Thr Phe Ile Asp Gln
465                 470                 475                 480
Trp Lys Arg Asp Pro Leu Asn Thr Ala Asn Thr Ile Gln Tyr Ala Gly
                485                 490                 495
Gln His Ser Lys Ser Asp Ala Gly Met Asp Glu His Thr Ala Gln Val
            500                 505                 510
Asp Tyr Thr Arg Pro Leu Gly Gln Ala His Ser Leu Glu Ala Gly Leu
        515                 520                 525
Lys Tyr Ile Tyr Arg His Ala Thr Ser Asp Pro Leu Tyr Glu Ile Arg
    530                 535                 540
Pro Ser Glu Asp Ala Pro Trp Gln Pro Gly Ser Leu Tyr Ala Gln Asn
545                 550                 555                 560
Pro Ser Asn Gly Lys Phe Arg His Asp Gln Tyr Ile Gly Ala Ala Tyr
                565                 570                 575
Ala Gly Tyr Asn Tyr Arg Lys Asp Gln Tyr Ser Leu Gln Thr Gly Leu
            580                 585                 590
Arg Val Glu Ser Ser Arg Leu Lys Ala Leu Phe Pro Glu Asn Ala Ala
        595                 600                 605
Ala Asp Phe Ser His Asn Ser Phe Asp Trp Val Pro Gln Leu Thr Leu
    610                 615                 620
Gly Tyr Thr Pro Ser Pro Met Lys Gln Leu Lys Leu Ala Tyr Asn Phe
625                 630                 635                 640
Arg Ile Gln Arg Pro Ala Ile Gly Gln Leu Asn Pro Tyr Arg Leu Gln
                645                 650                 655
Thr Asn Asp Tyr Gln Val Gln Tyr Gly Asn Pro Asp Leu Lys Ser Glu
            660                 665                 670
Lys Arg His His Val Gly Leu Ser Tyr Asn Gln Tyr Gly Ala Lys Val
        675                 680                 685
Met Leu Thr Ala Ser Leu Asp Tyr Asp Phe Cys Asn Asn Ala Ile Gln
    690                 695                 700
Asn Tyr Thr Phe Ser Asp Pro Ala Asn Pro Asn Leu Phe His Gln Thr
705                 710                 715                 720
Tyr Gly Asn Ile Gly Arg Glu His Ser Phe Ser Leu Asn Thr Tyr Ala
                725                 730                 735
Met Tyr Thr Pro Ala Val Trp Val Arg Ile Met Leu Asn Gly Asn Ile
            740                 745                 750
Asp Arg Thr Phe Gln Lys Ser Glu Ala Leu Gly Ile Asp Val Asn Ser
        755                 760                 765
Trp Ser Gly Met Val Tyr Ser Gly Leu Met Phe Thr Leu Pro Lys Asp
    770                 775                 780
Trp Thr Val Asn Leu Phe Gly Gly Tyr Tyr His Gly Gly Arg Ser Tyr
785                 790                 795                 800
Gln Thr Lys Tyr Asp Gly Asn Val Phe Asn Asn Ile Gly Ile Ala Lys
                805                 810                 815
Gln Leu Phe Asp Lys Lys Leu Arg Val Ser Leu Ser Ala Asn Asn Ile
            820                 825                 830
His Ala Lys Tyr Ser Thr Trp Lys Ser Arg Thr Ile Gly Asn Gly Phe
        835                 840                 845
Thr Ile Tyr Ser Glu Asn Ala Gly Ile Gln Arg Ser Val Ser Leu Ser
    850                 855                 860
Leu Thr Tyr Ser Phe Gly Lys Met Asn Thr Gln Val Arg Lys Val Glu

865                 870                 875                 880
Arg Thr Ile Val Asn Asp Asp Leu Lys Gln Thr Ser Ser Gln Gly Gln
                885                 890                 895
Gln Gly Gly Gly Gln Gly Asn Pro Thr Gly Asn
            900                 905

(2) INFORMATION FOR SEQ ID NO:356

     (i) SEQUENCE CHARACTERISTICS:
          (A) LENGTH: 450 amino acids
          (B) TYPE: amino acid
          (D) TOPOLOGY: linear

    (ii) MOLECULE TYPE: protein

   (iii) HYPOTHETICAL: YES

    (vi) ORIGINAL SOURCE:
          (A) ORGANISM: Porphyromonas gingivalis

    (ix) FEATURE:
          (A) NAME/KEY: misc_feature
          (B) LOCATION 1...450

    (xi) SEQUENCE DESCRIPTION: SEQ ID NO:356

Trp Cys Gln Ser Asp Pro Ile Pro His Cys His Ala Val Tyr Arg Asn
1               5                   10                  15
Ala Gln Gly His Gln Gln Gly Arg Ser Asp Gln Asp Arg Leu Pro Pro
            20                  25                  30
Val Arg Gly Tyr Arg Tyr Ala Arg Cys Leu Gly Arg Tyr Glu Gly Phe
        35                  40                  45
Gly Glu Arg Met Arg Leu Ser Ala Ile Leu Ile Ala Leu Ile Val Met
    50                  55                  60
Leu Pro Ala Val Leu Ser Gly Gln His Tyr Tyr Ser Met Ala Gly Glu
65                  70                  75                  80
Arg Leu Glu Thr Asp Ser Ile Arg Pro Asn Glu Leu Ser Ala Ser Ile
                85                  90                  95
Arg Ser Ala Leu Phe Phe Arg Asn Asn Glu Tyr Asn Ala Arg Ser Val
            100                 105                 110
Lys Gly Tyr Thr Leu Pro Gly Ala Arg Val Ser Ala Phe Ala Ser Tyr
        115                 120                 125
Ser Leu Pro Ala Ala His Gly Val Lys Leu Ser Leu Gly Val Ser Thr
    130                 135                 140
Leu Asn Tyr Trp Gly Ala Ser Arg Tyr Pro Ala Gly Ile Ala Tyr Ser
145                 150                 155                 160
Asp Leu Pro Tyr Trp Thr Asp Tyr Asn Asp Tyr Val Arg Leu Arg Ile
                165                 170                 175
Leu Pro Tyr Val Gln Ala Met Leu Lys Pro Thr Ala Thr Thr Ala Leu
            180                 185                 190
Met Leu Gly Asn Ile Ala Gly Gly Thr Ala His Gly Leu Ile Glu Pro
        195                 200                 205
Ile Tyr Asn Pro Glu Leu Asp Leu Thr Ala Asp Pro Glu Ala Gly Val
    210                 215                 220
Gln Phe Arg Gly Asp Trp Thr Arg Phe Arg Met Asp Val Trp Val Asn
225                 230                 235                 240
Trp Met Ser Met Ile Phe Lys Asn Asp Asn His Gln Glu Ser Phe Val
                245                 250                 255
Phe Gly Leu Ser Thr Thr Ser Lys Leu Leu Ser Gly Glu Gly Lys Trp
            260                 265                 270
Arg Leu Glu Leu Pro Leu Gln Ala Ile Ala Thr His Arg Gly Gly Glu
        275                 280                 285
Tyr Asn Trp Ala Gln Gln Asp Thr Val His Thr Trp Val Asn Gly Ala
    290                 295                 300
Val Gly Leu Lys Leu Ser Tyr Arg Pro Arg Thr Asp Lys Pro Met Gln
305                 310                 315                 320
Ile Trp Gly Ser Ala Tyr Gly Val Ala Ala Leu Ser Ser Gly Gly Tyr
                325                 330                 335
Phe Pro Tyr Glu Arg Gly Trp Gly Gly Tyr Leu Ser Leu Gly Met Asp
            340                 345                 350
Leu Glu His Phe Ala Phe Arg Thr Asp Tyr Trp Tyr Gly Arg His Tyr
        355                 360                 365
Val Ser Pro Phe Ala Ala Pro Phe Ala Asn Ser Leu Thr Tyr Asp Lys
    370                 375                 380
Gln Pro Leu Thr Asn Gly Trp Gly Asp Tyr Ile Arg Leu Tyr Ala Asp

385                 390                 395                 400
Tyr Ser Trp Arg Met Ala Arg Ser Val Ser Leu Ala Ala Val Ala Arg
                405                 410                 415
Val Trp Phe Gln Pro Ser Asp Arg Phe Ala Met Ser His Ala Leu Glu
            420                 425                 430
Leu Thr Met Arg Ile Asp Pro Lys Phe Pro Ile Ala Phe Leu Lys Gly
        435                 440                 445
Asn His
    450

(2) INFORMATION FOR SEQ ID NO:357

     (i) SEQUENCE CHARACTERISTICS:
          (A) LENGTH: 447 amino acids
          (B) TYPE: amino acid
          (D) TOPOLOGY: linear

    (ii) MOLECULE TYPE: protein

   (iii) HYPOTHETICAL: YES

    (vi) ORIGINAL SOURCE:
          (A) ORGANISM: Porphyromonas gingivalis

    (ix) FEATURE:
          (A) NAME/KEY: misc_feature
          (B) LOCATION 1...447

    (xi) SEQUENCE DESCRIPTION: SEQ ID NO:357

Pro Phe Ser Pro Pro Trp Val Cys Ala Gly Ala Ala Ser Ser Val Arg
1               5                   10                  15
Thr Arg Pro Cys Ser Thr Val Ser Ser Thr Ala Ala Ser Ser His Arg
            20                  25                  30
Arg Ser Glu His Met Arg Ser Ala Asp Ser Ser Pro Ala Tyr Ser Pro
        35                  40                  45
Ile Gly Ser Leu Ser Ser Ser His Ser Phe Asp Ser Ile Pro Asp Gln
    50                  55                  60
Arg Met Asn Lys Ser Leu Leu Ser Leu Ala Cys Leu Ile Leu Cys Gly
65                  70                  75                  80
Met Pro Ala Ile Ala Gln Gln Thr Gly Pro Ala Glu Arg Ser Gly Glu
                85                  90                  95
Pro Ser Leu Ala Glu Arg Val Phe Gly Leu Glu Gln Lys Gln Lys Lys
            100                 105                 110
Leu Lys Val Tyr Leu Gly Ile Gln Ser Phe Tyr Asp Gln Pro Leu Val
        115                 120                 125
Asp Asp Glu Ser His Ile Gly His Phe Lys Val Gln Glu Leu Arg Met
    130                 135                 140
Ser Ala His Gly Glu Leu Asn Arg His Leu Ser Phe Asp Trp Arg Gln
145                 150                 155                 160
Arg Leu Asn Arg Ala Ala Asp Gly Thr Ser Phe Ala Asp Asn Leu Ser
                165                 170                 175
Asn Ala Ile Asp Ile Ala Gly Val Asp Trp His Pro Asn Asp Lys Val
            180                 185                 190
Ser Phe Phe Phe Gly Arg Gln Tyr Ala Arg Phe Gly Gly Ile Glu Tyr
        195                 200                 205
Asp Met Asn Pro Val Glu Ile Tyr Gln Tyr Ser Asp Leu Val Asp Tyr
    210                 215                 220
Met Thr Cys Tyr Thr Ser Gly Val Asn Phe Ala Trp Asn Phe His Pro
225                 230                 235                 240
Glu Gln Gln Leu Gln Leu Gln Val Leu Asn Ala Tyr Asn Asn Arg Phe
                245                 250                 255
Ala Asp Arg Tyr His Val Thr Pro Asp Val Ala Thr Ala Thr Ser Tyr
            260                 265                 270
Pro Leu Leu Tyr Ser Ala Gln Trp Asn Gly Thr Leu Leu Gly Gly Ala
        275                 280                 285
Leu His Met Arg Tyr Ala Val Ser Met Ala His Gln Ala Gln Glu Arg
    290                 295                 300
Asn Met Trp Tyr Phe Thr Ala Gly Asn Leu Phe Asn Pro Gly Lys Arg
305                 310                 315                 320
Ile Asn Gly Tyr Leu Asp Leu Thr Tyr Ser Ile Glu Gly Leu Asp Asp
                325                 330                 335
Lys Gly Ile Met Thr Ala Arg Tyr Gly Lys Gly Lys Thr Leu Thr Asp
            340                 345                 350
Val Lys Tyr Tyr Ala Leu Val Ser Lys Trp Asn Phe Arg Ile Phe Asp

Gln Val Asn Leu Phe Leu Lys Gly Met Tyr Glu Asn Gly Tyr Ala Pro
        355                 360                 365
Ala Gln Tyr Gly Glu Ser Ser His Thr Arg His Ser Tyr Gly Tyr Met
    370                 375                 380
Gly Gly Val Glu Tyr Tyr Pro Thr Glu Thr Asn Phe Arg Leu Phe Val
385                 390                 395                 400
Thr Tyr Ile Gly Arg His Tyr Arg Tyr Ser Ala Thr Glu Thr Glu Ser
                405                 410                 415
Thr Asn Ala Leu Arg Ala Gly Leu Ile Tyr Gln Ile Pro Phe Leu
            420                 425                 430
        435                 440                 445

(2) INFORMATION FOR SEQ ID NO:358

    (i) SEQUENCE CHARACTERISTICS:
        (A) LENGTH: 227 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...227

    (xi) SEQUENCE DESCRIPTION: SEQ ID NO:358

Tyr Lys Gln Ala Ile Met Lys His Leu Phe Lys Ser Thr Leu Val Leu
1               5                   10                  15
Leu Cys Ala Leu Ser Phe Ser Gly Thr Tyr Thr Phe Ala Gln Glu Asn
            20                  25                  30
Asn Thr Glu Lys Ser Arg Phe Asp Phe Ser Val Arg Leu Gly Gln Gly
        35                  40                  45
Tyr Ile Ala Gly Ser Thr Thr Asn Leu Met Tyr Gly Tyr Thr Ser Ala
    50                  55                  60
Asn Asp Arg Leu Leu Ser Gly Ala Ile Tyr Leu Gly Leu Thr Pro Ser
65                  70                  75                  80
Lys Lys Glu Asn Ala Thr Gly Val Ala Phe Arg Phe Leu Ser Pro Ser
                85                  90                  95
Pro Gly Tyr Tyr Val Asp Ile Ser Gly Lys Glu Asn Thr Leu Asn Tyr
            100                 105                 110
Ala Phe Tyr Val Val Gly Ala Tyr Asn Arg Ile Ala Ile Pro Ile Arg
        115                 120                 125
Pro Ile Lys Asn Phe Asn Phe Ile Phe Ser Thr Glu Val Gly Met Ala
    130                 135                 140
Trp Met Ser Arg His Glu Gln Ile Tyr Asn Ser Thr Ser Gln Thr Trp
145                 150                 155                 160
Asp Lys Gln Arg Lys Ser Arg Ser Gly Leu Asp Phe Gly Leu Gly Met
                165                 170                 175
His Leu Gln Unk His Ile Asn Lys Thr Val Tyr Phe Met Ala Gly Thr
            180                 185                 190
Asp Leu Thr Ser Cys Met Phe Gly Lys Arg Ile Asn Asp Tyr Gln Gln
        195                 200                 205
Lys Asp Arg Thr Phe Ile Ala Leu Ile Asp Asn Ser Ile Gly Ile Gly
    210                 215                 220
Leu Asn Leu
225

(2) INFORMATION FOR SEQ ID NO:359

    (i) SEQUENCE CHARACTERISTICS:
        (A) LENGTH: 406 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:

(A) ORGANISM: Porphyromonas gingivalis

(ix) FEATURE:
(A) NAME/KEY: misc_feature
(B) LOCATION 1...406

(xi) SEQUENCE DESCRIPTION: SEQ ID NO:359

Ile Phe Ile Asp Pro Asp Lys Asn Thr Lys Gln Asn Glu Arg Asn Met
1               5                   10                  15
Ile Ile Lys Lys Met Leu Lys Asn Lys Leu Ala Pro Leu Ala Ile Leu
            20                  25                  30
Phe Leu Phe Ala Pro Lys Ala Met Lys Ala Gln Glu Gln Leu Asn Val
        35                  40                  45
Val His Thr Ser Val Pro Ser Leu Asn Ile Ser Pro Asp Ala Arg Ala
    50                  55                  60
Ala Gly Met Gly Asp Ile Gly Val Ala Thr Thr Pro Asp Ala Tyr Ser
65                  70                  75                  80
Gln Tyr Trp Asn Pro Ser Lys Tyr Ala Phe Met Asp Thr Lys Ala Gly
                85                  90                  95
Ile Ser Phe Ser Tyr Thr Pro Trp Leu Ser Lys Leu Val Asn Asp Ile
            100                 105                 110
Ala Leu Met Gln Met Thr Gly Phe Tyr Lys Leu Gly Thr Asp Glu Asn
        115                 120                 125
Gln Ala Ile Ser Ala Ser Leu Arg Tyr Phe Thr Leu Gly Lys Leu Glu
    130                 135                 140
Thr Phe Asp Glu Leu Gly Glu Ser Met Gly Glu Ala His Pro Asn Glu
145                 150                 155                 160
Phe Ala Val Asp Leu Gly Tyr Ser Arg Gln Leu Ser Glu Asn Phe Ser
                165                 170                 175
Met Ala Val Ala Leu Arg Tyr Ile Arg Ser Asp Gln Ser Thr His Asn
            180                 185                 190
Thr Gly Glu Asn Gln Ala Gly Asn Ala Phe Ala Ala Asp Ile Ala Gly
        195                 200                 205
Tyr Leu Gln Lys Tyr Val Leu Leu Gly Asn Ala Glu Ser Leu Trp Ser
    210                 215                 220
Leu Gly Phe Asn Val Lys Asn Ile Gly Thr Lys Ile Ser Tyr Asp Gly
225                 230                 235                 240
Gly Val Thr Ser Phe Phe Ile Pro Thr Ser Leu Asn Leu Gly Thr Gly
                245                 250                 255
Leu Leu Tyr Pro Ile Asp Asp Tyr Asn Ser Ile Asn Phe Asn Leu Glu
            260                 265                 270
Leu Ser Lys Leu Leu Val Pro Thr Pro Pro Ile Met Asp Gln Asn Asp
        275                 280                 285
Gln Ala Gly Tyr Glu Ala Ala Leu Lys Lys Tyr Gln Glu Thr Ser Ser
    290                 295                 300
Ile Ser Gly Ile Phe Ser Ser Phe Gly Asp Ala Pro Gly Gly Leu Lys
305                 310                 315                 320
Glu Glu Phe Arg Glu Ile Thr Trp Gly Leu Gly Ala Glu Tyr Ser Tyr
                325                 330                 335
Asp Asp Lys Phe Phe Val Arg Ala Gly Tyr Ser Tyr Leu His Pro Thr
            340                 345                 350
Lys Gly Asn Leu Gln Tyr Phe Thr Ala Gly Ala Gly Phe Lys Met Asn
        355                 360                 365
Ile Phe Arg Ile Asp Ala Ser Tyr Leu Leu Ser Thr Ile Gln Ser Asn
    370                 375                 380
Pro Leu Asp Gln Thr Leu Arg Phe Thr Leu Ala Phe Asp Met Asp Gly
385                 390                 395                 400
Leu Arg Asn Leu Phe His
                405

(2) INFORMATION FOR SEQ ID NO:360

(i) SEQUENCE CHARACTERISTICS:
(A) LENGTH: 452 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...452

(xi) SEQUENCE DESCRIPTION: SEQ ID NO:360

Leu Ile Ile Glu Lys Glu Met Lys Thr Thr Val Gln Gln Ile Ile Leu
1               5                   10                  15
Cys Leu Ala Leu Met Met Ser Gly Val Leu Gly Gly Asn Ala Gln Ser
            20                  25                  30
Phe Trp Glu Glu Ile Ala Pro Pro Phe Ile Ser Asn Glu Pro Asn Val
        35                  40                  45
Lys Tyr Ile Ile Pro Asn Met Gly Ile Asp Ser Lys Gly Thr Ile Tyr
    50                  55                  60
Val Thr Val Thr Lys Arg Ile Gln Gln Gly Ala Asn Tyr Thr Ser Glu
65                  70                  75                  80
Gln Leu Gly Met Tyr Tyr Arg Pro Leu Gly Asp Asn Glu Gln Trp Trp
                85                  90                  95
Lys His Asp Pro Tyr Phe Asp Asp Lys Ile Val Ala Asp Ile Gln Thr
            100                 105                 110
Asp Ala Tyr Gly Arg Val Tyr Val Cys Thr Thr Ser Ser Arg Asp Gln
        115                 120                 125
Glu Tyr Gln Leu Tyr Ile Asn Glu Gln Asn Glu Trp Arg Cys Ile Phe
    130                 135                 140
Lys Thr Ser Val Ser Thr Tyr Glu His Gly Met Ala Val Phe Arg Ser
145                 150                 155                 160
Ser Thr Gly Val Thr Tyr Ile Gly Thr Arg His His Ile Phe Ala Ser
                165                 170                 175
Gly Val Asn Asp Phe Glu Phe Asn Thr Ile Tyr Glu Asp Ser Thr Pro
            180                 185                 190
Met Ser Cys Arg Phe Ala Glu Ala Thr Asn Ser Gly Thr Ile Tyr Leu
        195                 200                 205
Ala Leu Met His Glu Thr Thr Met Ser Thr Thr Ile Leu Thr Tyr Gln
    210                 215                 220
Asn Gly Glu Phe Val Asp Ile Ser Glu Ser Glu Leu Ser Asn Ser Ile
225                 230                 235                 240
Ile Ala Ser Met Cys Ser Asn Lys Glu Gly Asp Ile Ile Ala Leu Val
                245                 250                 255
Thr Ser Tyr Thr Gly Phe Met Ser Gly Thr Leu Ala Ile Arg Lys Ala
            260                 265                 270
Asp Glu Gly Lys Trp Gln Leu Val Gly Gly Asp Ile Gln Asn Ala Ile
        275                 280                 285
Val Gln Asn Ile Cys Met Met Asp Asp Asn Lys Ile Ala Cys Glu Val
    290                 295                 300
Phe Gly Thr Pro Asn Gly Val Asp Gly Arg Thr Arg Val Cys Val Ser
305                 310                 315                 320
Asp Ala Ser Val Phe Asp Phe Glu Trp Tyr Glu Asp Glu Ile Tyr Gly
                325                 330                 335
Gly Leu Ile Phe Asp Thr Phe Phe Tyr Ser Pro Trp Asp Lys Leu Leu
            340                 345                 350
Tyr Ala Lys Phe Gly Gly Ile Met Leu Arg Ser Lys Glu Ser Phe Ile
        355                 360                 365
Thr Ser Phe Ile Ser Pro Thr Val Val Gln Gly Val Asp Val Tyr Thr
    370                 375                 380
Leu Ala Gly Lys Ile Arg Ile Glu Ser Glu Thr Pro Val Ser Glu Val
385                 390                 395                 400
Leu Leu Phe Asp Leu Ala Gly Arg Met Val Leu Arg Gln Thr Ile Asp
                405                 410                 415
Asn Lys Ile Tyr Ser Asp Ile Asp Thr Asn Gly Leu Lys Arg Ser Gly
            420                 425                 430
Ile Tyr Val Val Ser Val Arg Leu Ser Ser Gly Gln Val Phe Ser His
        435                 440                 445
Lys Val Gln Val
    450

(2) INFORMATION FOR SEQ ID NO:361

(i) SEQUENCE CHARACTERISTICS:
(A) LENGTH: 331 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...331

(xi) SEQUENCE DESCRIPTION: SEQ ID NO:361

Gly Leu Tyr Gly Met Ser Val Val Pro Ile Ile Ile Tyr Leu Cys Gly
1               5                   10                  15
Ile Ser Asn Tyr Ala Arg Leu Met Ile Ile Arg Cys Leu Ile Arg Arg
            20                  25                  30
Pro Arg Thr Val Leu Phe Gly Leu Ile Phe Val Val Gly Leu Phe Ser
        35                  40                  45
Ala Met Ala Gln Glu Lys Lys Asp Ser Leu Ser Thr Val Gln Pro Val
    50                  55                  60
Pro Asn Ser Ser Met Val Glu Gln Thr Pro Leu Leu Ser Ile Asp His
65                  70                  75                  80
Pro Val Leu Pro Ala Ser Phe Gln Asn Thr Arg Thr Leu Lys Arg Phe
                85                  90                  95
Arg Asp Lys His Leu Ser Asp Ala Leu Leu Asn Gly Leu Lys Pro His
            100                 105                 110
Arg Ser Ser Leu Gln Leu Asn Glu Glu Leu Asn Phe Ala Ala Glu Arg
        115                 120                 125
Arg Asp Phe Val Ser Pro Leu Leu Gln Thr Arg His Ala Ala Gly Val
    130                 135                 140
Leu Ser Trp Arg Pro Thr Asp Arg Met His Phe Tyr Thr Ser Gly Asn
145                 150                 155                 160
Ile Gly Leu Gly His Asp Leu Leu Thr Gly Val Arg Lys Asp Phe Gly
                165                 170                 175
Trp Asn Ala Gly Ala Asp Phe Leu Leu Ser Gln Asn Leu Thr Ala His
            180                 185                 190
Val Gln Gly Gly Trp Gln Gln Asn Phe Gly Phe Ile Pro Met Thr Ala
        195                 200                 205
Val Asn Gly Gln Leu Arg Trp Gln Ala Thr Glu Arg Leu Ser Phe Thr
    210                 215                 220
Thr Gly Ile Asp Tyr Arg Gln Val Gln Trp Asn Ala Phe Asp Asn Arg
225                 230                 235                 240
Thr Phe Ser Leu Lys Gly Ser Ala Arg Tyr Glu Val Met Asp Asn Val
                245                 250                 255
Phe Val Asn Gly Phe Gly Ser Tyr Pro Leu Tyr Ser Ser Thr Arg Ser
            260                 265                 270
Gly Leu Asn Met Ala Val Pro Met His Gly Phe Gly Pro Gln Tyr Gly
        275                 280                 285
Gly Ser Leu Glu Leu Lys Val Ser Glu Arg Phe Gly Phe Ala Val Gly
    290                 295                 300
Met Glu Arg Glu Tyr Asn Ile Trp Thr Arg Arg Trp Glu Thr His Tyr
305                 310                 315                 320
Phe Ala Tyr Pro Val Phe Tyr Gly Asp Lys Lys
                325                 330

(2) INFORMATION FOR SEQ ID NO:362

(i) SEQUENCE CHARACTERISTICS:
(A) LENGTH: 329 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...329

(xi) SEQUENCE DESCRIPTION: SEQ ID NO:362

Glu Thr Asn Ser Trp Val Ser Ser Asp Cys Asn Ser Thr Thr Met Lys
1               5                   10                  15

Thr Asn Arg Arg Tyr Ala Phe Val Leu Pro Leu Leu Leu Leu Thr Gly
            20                  25                  30
Leu Leu Ala Trp Gly Gln Asp Ser Ser His Gly Ser Asn Thr Ala Phe
        35                  40                  45
Ala Thr Asp Ser Ser Ser Arg Glu Leu Pro Thr Glu Gln Ser Ala Tyr
    50                  55                  60
Arg Ile His Ser Ala Tyr Met Val Gly Gly Gly Gly Ser Ile Thr Arg
65                  70                  75                  80
Asp Thr Tyr Leu Ser Pro Leu Arg Tyr Gly Gly Trp Thr Leu Asn Leu
                85                  90                  95
Leu Gly Glu Lys Thr Phe Pro Leu Lys Ala Ser Asp Ser Arg Trp Met
            100                 105                 110
Ile Arg Thr Gly His Glu Leu Asp Phe Ala Leu Met Asp Asn Pro Ala
        115                 120                 125
Asn Asn Ala His Phe Tyr Ser Leu Leu Tyr Asn Gly Ser Ala Ala Ala
    130                 135                 140
Leu Tyr Arg Leu Gly Ala Lys His Leu Arg Ala Ala Trp Met Asp Asn
145                 150                 155                 160
Leu Arg Leu Ala Phe Gly Pro Gly Leu Glu Ile Gly Leu Gly Gly Ile
                165                 170                 175
Tyr Ser Thr Arg Asn Gly Asn Asn Pro Ala Thr Leu Lys Leu Tyr Thr
            180                 185                 190
Asn Ala Ile Ala Gln Ala Ser Ile Gly Tyr Tyr Val Pro Ser Glu Thr
        195                 200                 205
Phe Pro Leu Tyr Phe Arg Leu Leu Ser Gln Ile Asn Leu Phe Gly Ile
    210                 215                 220
Ala Tyr Gly Asn Gly Phe Gly Glu Ser Tyr Tyr Glu Asn Phe Leu Leu
225                 230                 235                 240
Asn Asn Gly Ile Ala Gly Ser Leu His Phe Thr Tyr Pro Gly Lys Phe
                245                 250                 255
Thr Arg Phe Thr Thr Leu Ile Thr Ala Asp Ile Pro Ile Arg Asn Phe
            260                 265                 270
Cys Thr Leu Arg Val Gly Tyr Arg Tyr Ser His Leu Gly Ser Ser Leu
        275                 280                 285
Asn Ala Leu Asp Thr Arg Ile His Ser His Thr Ala Phe Ile Gly Phe
    290                 295                 300
Val Thr Glu Phe Tyr Arg Phe Arg Gly Arg Lys Ala Met Asn Thr Gly
305                 310                 315                 320
Arg Arg Thr Ser Leu Tyr Tyr His Asp
                325

(2) INFORMATION FOR SEQ ID NO:363

    (i) SEQUENCE CHARACTERISTICS:
        (A) LENGTH: 319 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...319

    (xi) SEQUENCE DESCRIPTION: SEQ ID NO:363

Phe Thr Ser Gly Thr Ile Phe Val Arg Ile Ala Ile Ser Gly Arg Val
1               5                   10                  15
Val Cys Arg Glu Val Glu Asp Asn Gly Arg Arg Glu Ala Val Arg His
            20                  25                  30
Asp Gly Met Val Val Thr Leu Leu Val Ile Val Gly Ile Val Val Val
        35                  40                  45
Val Arg Tyr Ser Leu Arg Val His Val His Lys Thr Gly Thr Val Val
    50                  55                  60
Ser Ala Ala Ile Phe Gly Phe Ile Leu Leu Gly Lys Thr Val Pro Cys
65                  70                  75                  80
Asp Thr Arg Asn Phe Phe Ser Ser Glu Ser Asp Glu Pro Glu Ser Arg
                85                  90                  95
Val Ala Thr Glu Ile Ala His Leu Cys Glu Ile Gly Phe Gln Ile His
            100                 105                 110

Ala Ser Ser Ile His Val Ala Val Arg Thr Asp Phe Gly Gln Ala Gly
        115                 120                 125
Ile His Cys Pro Met Ala Thr Asp Ala Ser Ala Thr Glu Phe Asp Arg
    130                 135                 140
Ser Ala Glu Cys Ala Glu Arg Thr Ser Ala Gln Ile Asp Thr Ala Ile
145                 150                 155                 160
Arg Ser Gln Ser Gln Ile Ile Arg Thr His Ile Asp Thr Cys Pro Lys
                165                 170                 175
Ser Ser Gly Thr Ile Gly Gly Ser Thr His Thr Ser Leu His Leu Lys
            180                 185                 190
Val Phe Asp Gly Arg Gly Glu Val Gly His Ile His Pro Lys Asp Gly
        195                 200                 205
Leu Arg Phe Gly Val Val Glu Gly Tyr Ser Ile Gly Ser Tyr Val Asp
    210                 215                 220
Ala Ile Gly Ile Gly Ala Thr His Ala Lys Ala Gly Ile Ser Asp Thr
225                 230                 235                 240
Arg Thr Gly Ile Ala Gly Gly Tyr His Gly Gly Ser Gln His Gln Gln
                245                 250                 255
Ile Gly Asp Val Thr Thr Ile Ile Gly Leu Gly Lys Phe Gly Leu Ala
            260                 265                 270
Asn Val Gly Val Ser Asp Arg Gly Phe Arg Arg Gly Ala Ser Ser Tyr
        275                 280                 285
Asp Leu Tyr Gly Leu Glu Leu His Ile Ala Lys Thr Ile Tyr Leu Ala
    290                 295                 300
Val Gly Asp Gly Cys Leu Cys Arg Gln Ala Glu Arg Gln Asp Gly
305                 310                 315

(2) INFORMATION FOR SEQ ID NO:364

    (i) SEQUENCE CHARACTERISTICS:
        (A) LENGTH: 614 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...614

    (xi) SEQUENCE DESCRIPTION: SEQ ID NO:364

Pro Tyr Asn Val Gln Ser Ile Ser Asn Lys Thr Ile Lys Lys Gln Met
1               5                   10                  15
Glu Asn Leu Lys Asn Ile Gln Pro Arg Glu Asp Phe Asn Trp Glu Glu
            20                  25                  30
Phe Glu Ala Gly Gly Val His Ala Ala Val Ser Arg Gln Glu Gln Glu
        35                  40                  45
Ala Ala Tyr Asp Lys Thr Leu Asn Thr Ile Lys Glu Lys Glu Val Val
    50                  55                  60
Met Gly Arg Val Thr Ala Ile Asn Lys Arg Glu Val Val Ile Asn Val
65                  70                  75                  80
Gly Tyr Lys Ser Glu Gly Val Val Pro Ala Thr Glu Phe Arg Tyr Asn
                85                  90                  95
Pro Glu Leu Lys Val Gly Asp Glu Val Glu Val Tyr Ile Glu Asn Gln
            100                 105                 110
Glu Asp Lys Lys Gly Gln Leu Val Leu Ser His Arg Lys Gly Arg Ala
        115                 120                 125
Ala Arg Ser Trp Glu Arg Val Asn Glu Ala Leu Glu Lys Asp Glu Ile
    130                 135                 140
Val Lys Gly Tyr Val Lys Cys Arg Thr Lys Gly Gly Met Ile Val Asp
145                 150                 155                 160
Val Phe Gly Ile Glu Ala Phe Leu Pro Gly Ser Gln Ile Asp Val Arg
                165                 170                 175
Pro Ile Arg Asp Tyr Asp Ala Phe Val Glu Lys Thr Met Glu Phe Lys
            180                 185                 190
Ile Val Lys Ile Asn Gln Glu Tyr Lys Asn Val Val Val Ser His Lys
        195                 200                 205
Val Leu Ile Glu Ala Glu Leu Glu Gln Gln Lys Lys Glu Ile Ile Gly
    210                 215                 220

Lys Leu Glu Lys Gly Gln Val Leu Glu Gly Ile Val Lys Asn Ile Thr
225                 230                 235                 240
Ser Tyr Gly Val Phe Ile Asp Leu Gly Gly Val Asp Gly Leu Ile His
                245                 250                 255
Ile Thr Asp Leu Ser Trp Gly Arg Val Ala His Pro Glu Glu Ile Val
            260                 265                 270
Gln Leu Asp Gln Lys Ile Asn Val Val Ile Leu Asp Phe Asp Glu Asp
        275                 280                 285
Arg Lys Arg Ile Ala Leu Gly Leu Lys Gln Leu Met Pro His Pro Trp
    290                 295                 300
Asp Ala Leu Asp Ser Glu Leu Lys Val Gly Asp Lys Val Lys Gly Lys
305                 310                 315                 320
Val Val Val Met Ala Asp Tyr Gly Ala Phe Val Glu Ile Ala Gln Gly
                325                 330                 335
Val Glu Gly Leu Ile His Val Ser Glu Met Ser Trp Thr Gln His Leu
            340                 345                 350
Arg Ser Ala Gln Asp Phe Leu His Val Gly Asp Glu Val Glu Ala Val
        355                 360                 365
Ile Leu Thr Leu Asp Arg Glu Glu Arg Lys Met Ser Leu Gly Leu Lys
    370                 375                 380
Gln Leu Lys Pro Asp Pro Trp Ala Asp Ile Glu Thr Arg Phe Pro Val
385                 390                 395                 400
Gly Ser Arg His His Ala Arg Val Arg Asn Phe Thr Asn Phe Gly Val
                405                 410                 415
Phe Val Glu Ile Glu Glu Gly Val Asp Gly Leu Ile His Ile Ser Asp
            420                 425                 430
Leu Ser Trp Thr Lys Lys Ile Lys His Pro Ser Glu Phe Thr Glu Val
        435                 440                 445
Gly Ala Asp Ile Glu Val Gln Val Ile Glu Ile Asp Lys Glu Asn Arg
    450                 455                 460
Arg Leu Ser Leu Gly His Lys Gln Leu Glu Glu Asn Pro Trp Asp Val
465                 470                 475                 480
Phe Glu Thr Val Phe Thr Val Gly Ser Ile His Glu Gly Thr Val Ile
                485                 490                 495
Glu Val Met Asp Lys Gly Ala Val Val Ser Leu Pro Tyr Gly Val Glu
            500                 505                 510
Gly Phe Ala Thr Pro Lys His Met Val Lys Glu Asp Gly Ser Gln Ala
        515                 520                 525
Val Leu Glu Glu Lys Leu Pro Phe Lys Val Ile Glu Phe Asn Lys Asp
    530                 535                 540
Ala Lys Arg Ile Ile Val Ser His Ser Arg Val Phe Glu Asp Glu Gln
545                 550                 555                 560
Lys Met Ala Gln Arg Glu Ala Asn Ala Glu Arg Lys Ala Glu Ala Lys
                565                 570                 575
Ala Ala Gln Lys Glu Ala Ala Ala Glu Ala Ala Asn Pro Ala Gln Ala
            580                 585                 590
Val Glu Lys Ala Thr Leu Gly Asp Leu Gly Glu Leu Ala Ala Leu Lys
        595                 600                 605
Glu Lys Leu Ser Glu Asn
    610

(2) INFORMATION FOR SEQ ID NO:365

    (i) SEQUENCE CHARACTERISTICS:
        (A) LENGTH: 243 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...243

    (xi) SEQUENCE DESCRIPTION: SEQ ID NO:365

Ser Thr Ile Met Lys Lys Ala Ile Leu Ser Gly Ala Ala Leu Leu Leu
1               5                   10                  15
Gly Leu Cys Ala Asn Ala Gln Asn Val Gln Leu His Tyr Asp Phe Gly
            20                  25                  30

His Ser Ile Tyr Asp Glu Leu Asp Gly Arg Pro Lys Leu Thr Thr Thr
        35                  40                  45
Val Glu Asn Phe Thr Pro Asp Lys Trp Gly Ser Thr Phe Phe Phe Ile
    50                  55                  60
Asp Met Asp Tyr Thr Gly Lys Gly Ile Gln Ser Ala Tyr Trp Glu Ile
65                  70                  75                  80
Ser Arg Glu Leu Lys Phe Trp Gln Ala Pro Val Ser Ile His Leu Glu
                85                  90                  95
Tyr Asn Gly Gly Leu Ser Thr Ser Phe Thr Phe Gly His Asp Ala Leu
            100                 105                 110
Ile Gly Ala Thr Tyr Thr Tyr Asn Asn Pro Ser Phe Thr Arg Gly Phe
        115                 120                 125
Thr Ile Thr Pro Met Tyr Lys His Leu Gly Ala His Asp Phe His Thr
    130                 135                 140
Tyr Gln Ile Thr Gly Thr Trp Tyr Met His Phe Leu Asp Gly Leu Leu
145                 150                 155                 160
Thr Phe Asn Gly Phe Leu Asp Leu Trp Gly Phe Pro Gln Glu Asn Pro
                165                 170                 175
Ile Gly Gly Pro Val Leu Lys Glu Gly Asp Lys Phe Val Phe Leu Ser
            180                 185                 190
Glu Pro Gln Phe Trp Ile Asn Leu Asn Arg Ile Lys Gly Ile Asp Lys
        195                 200                 205
Asp Phe Asn Leu Ser Ile Gly Thr Glu Met Glu Ile Ser Arg Asn Phe
    210                 215                 220
Ala Arg Met Asp Lys Phe Ser Cys Ile Pro Thr Leu Ala Val Lys Trp
225                 230                 235                 240
Thr Phe Asn

(2) INFORMATION FOR SEQ ID NO:366

    (i) SEQUENCE CHARACTERISTICS:
        (A) LENGTH: 235 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...235

    (xi) SEQUENCE DESCRIPTION: SEQ ID NO:366

Met Lys Thr Ile Ser Lys Asn His Ala Ala Arg Ile Cys Ala Ala Ile
1               5                   10                  15
Ala Leu Phe Ala Val Cys Asn Gly Arg Ile Ala Ala Gln Asp Phe Leu
            20                  25                  30
Tyr Glu Ile Gly Gly Gly Phe Gly Ala Ala Gln Tyr Phe Gly Asp Ala
        35                  40                  45
Asn Arg Gly Leu Phe Gly Ser Ser Gly Val Gly Leu Glu Leu Val Gly
    50                  55                  60
Arg Tyr Asn Tyr Asn Phe Arg Trp Ala Phe Ser Thr Met Leu Asp Trp
65                  70                  75                  80
Arg Thr Leu Arg Gly Asp Thr Asp Lys Ser Gly Asn Val Phe Pro Asp
                85                  90                  95
Phe Ala Gln Ala Asp Phe Lys Val Gly Leu Thr Gln Leu His Val Arg
            100                 105                 110
Ser Glu Phe Asn Phe Leu Pro Tyr Ser Asp Gly Tyr Lys Tyr Leu Gly
        115                 120                 125
Thr Ala Arg Leu Ser Pro Tyr Val Ala Ala Gly Leu Ser Leu Gly Phe
    130                 135                 140
Ala Ser Gly Ala Lys Gly Ser Ala Phe Ala Pro Gly Ile Thr Ala Gly
145                 150                 155                 160
Met Gly Val Lys Tyr Lys Leu Lys Pro Arg Ile Asn Val Gly Ile Glu
                165                 170                 175
Tyr Ser Phe Thr Gly Leu Leu Thr Asp Ala Leu Asp Ala Leu Thr Asp
            180                 185                 190
Lys Ser Val Trp Leu Glu Asp Pro Tyr Lys Ile Asn Asp Ser Trp Val
        195                 200                 205

Lys Asn Lys Asp Ala Thr Gly Ala Leu Val Leu Arg Ile Thr Tyr Asp
    210                 215                 220
Phe Gly Leu Arg Lys Thr Phe Cys Asn Lys Gln
225                 230                 235

(2) INFORMATION FOR SEQ ID NO:367

    (i) SEQUENCE CHARACTERISTICS:
        (A) LENGTH: 436 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...436

    (xi) SEQUENCE DESCRIPTION: SEQ ID NO:367

Asn Ile Met Tyr Lys Asp Tyr Lys Gly Leu Tyr Ala Ser Leu Arg Trp
1               5                   10                  15
Tyr Ala Leu Ile Ile Gly Leu Leu Phe Ala Ala Asp Gly Ile Gln Ala
            20                  25                  30
Gln Asn Asn Asn Phe Thr Glu Ser Pro Tyr Thr Arg Phe Gly Leu Gly
        35                  40                  45
Arg Leu Gly Glu Arg Thr Thr Ile Ser Gly His Ser Met Gly Gly Leu
    50                  55                  60
Gly Val Gly Leu Arg Gln Gly Thr Tyr Val Asn Ala Val Asn Pro Ala
65                  70                  75                  80
Ser Tyr Ser Ala Val Asp Ser Met Thr Phe Ile Phe Asp Phe Gly Ala
                85                  90                  95
Ser Thr Gly Ile Thr Trp Tyr Ala Glu Asn Gly Lys Lys Asp Asn Arg
            100                 105                 110
Lys Met Gly Asn Ile Glu Tyr Phe Ala Met Leu Phe Pro Ile Ser Lys
        115                 120                 125
Ser Ile Ala Met Ser Ala Gly Val Leu Pro Tyr Ser Ala Ser Gly Tyr
    130                 135                 140
Gln Phe Gly Ser Val Asp Gln Val Glu Gly Gly Ser Val Gln Tyr Thr
145                 150                 155                 160
Arg Lys Tyr Leu Gly Thr Gly Asn Leu Asn Asp Leu Tyr Val Gly Ile
                165                 170                 175
Gly Ala Thr Pro Phe Lys Asn Phe Ser Ile Gly Ala Asn Ala Ser Ser
            180                 185                 190
Leu Phe Gly Arg Phe Thr His Ser Arg Gln Val Ile Phe Ser Thr Glu
        195                 200                 205
Ala Pro Tyr Asn Pro Val His Leu Ser Thr Leu Tyr Leu Lys Ala Ala
    210                 215                 220
Lys Phe Asp Phe Gly Met Gln Tyr His Leu Leu Leu Lys Ser Asp Arg
225                 230                 235                 240
Ser Leu Val Ile Gly Ala Val Tyr Ser Pro Arg Val Lys Met His Ser
                245                 250                 255
Glu Leu Thr Gln Ile Lys Asn Gln Val Gln Asn Gly Val Val Val Glu
            260                 265                 270
Ser Glu Thr Gln Glu Tyr Ile Lys Gly Met Asp Tyr Tyr Thr Leu Pro
        275                 280                 285
His Thr Leu Gly Ile Gly Phe Ser Tyr Glu Lys Lys Asp Lys Leu Leu
    290                 295                 300
Leu Gly Ala Asp Val Gln Tyr Ser Lys Trp Lys Gly Glu Lys Phe Tyr
305                 310                 315                 320
Lys Ser Asp Cys Lys Phe Gln Asp Arg Ile Arg Val Ser Leu Gly Gly
                325                 330                 335
Glu Ile Ile Pro Asp Ile Asn Ala Val Gly Met Trp Pro Lys Val Arg
            340                 345                 350
Tyr Arg Phe Gly Leu His Gly Glu Asn Ser Tyr Leu Lys Val Pro Thr
        355                 360                 365
Lys Gly Gly Val Tyr Gln Gly Tyr His Ile Val Gly Ala Val Phe Gly
    370                 375                 380
Ile Gly Ile Pro Leu Asn Asp Arg Arg Ser Phe Val Asn Val Ser Leu
385                 390                 395                 400

Glu Tyr Asp Arg Leu Ile Pro Lys Glu Gly Met Ile Lys Glu Asn Ala
                405                 410                 415
Leu Lys Leu Thr Phe Gly Leu Thr Phe Asn Glu Ser Trp Phe Lys Lys
            420                 425                 430
Leu Lys Leu Asn
        435

(2) INFORMATION FOR SEQ ID NO:368

     (i) SEQUENCE CHARACTERISTICS:
          (A) LENGTH: 945 amino acids
          (B) TYPE: amino acid
          (D) TOPOLOGY: linear

    (ii) MOLECULE TYPE: protein

   (iii) HYPOTHETICAL: YES

    (vi) ORIGINAL SOURCE:
          (A) ORGANISM: Porphyromonas gingivalis

    (ix) FEATURE:
          (A) NAME/KEY: misc_feature
          (B) LOCATION 1...945

    (xi) SEQUENCE DESCRIPTION: SEQ ID NO:368

Asn Thr Ile Ser Glu Asn Gly Asn Asp Ser Thr Tyr Phe Ser Phe Gln
1               5                   10                  15
Thr Phe Leu Met Arg Ser Ile Tyr Gln Leu Leu Leu Ser Ile Leu Leu
            20                  25                  30
Ala Ser Leu Gly Phe Val Gly Leu Glu Ala Gln Gln Ala Gly Val Ala
        35                  40                  45
Gly Arg Val Leu Asp Glu Glu Gly Asn Pro Met Ile Gln Ala Asn Val
    50                  55                  60
Gln Leu Val Gln Ser Thr Gly Gln Val Ala Val Ala Ala Gly Ala Thr
65                  70                  75                  80
Asn Glu Lys Gly Leu Phe Ser Leu Lys Thr Ser Gln Glu Gly Asp Tyr
                85                  90                  95
Ile Leu Arg Val Ser Tyr Val Gly Tyr Thr Thr His Asp Glu Lys Ile
            100                 105                 110
Ser Leu Arg Asn Gly Gln Thr Ile Thr Leu Lys Asp Ile Ser Met Asn
        115                 120                 125
Glu Asp Ala Arg Leu Leu Gln Ser Val Thr Val Gln Ala Lys Ala Ala
    130                 135                 140
Glu Val Val Val Arg Asn Asp Thr Leu Glu Phe Asn Ala Gly Ser Tyr
145                 150                 155                 160
Thr Val Ala Gln Gly Ala Ser Ile Glu Glu Leu Ile Lys Lys Leu Pro
                165                 170                 175
Gly Ala Glu Ile Gly Ser Asp Gly Lys Ile Thr Ile Asn Gly Lys Asp
            180                 185                 190
Ile Ser Lys Ile Leu Val Asp Gly Lys Glu Phe Phe Ser Lys Asp Pro
        195                 200                 205
Gln Val Ala Ile Lys Asn Leu Pro Ala Asp Met Val Asn Lys Val Gln
    210                 215                 220
Val Leu Asn Lys Leu Ser Glu Leu Ser Arg Met Ser Gly Phe Asp Asp
225                 230                 235                 240
Gly Glu Glu Glu Thr Val Ile Asn Leu Thr Val Lys Pro Glu Lys Lys
                245                 250                 255
Lys Gly Leu Phe Gly Thr Leu Gln Ala Gly Tyr Gly Thr Asp Gln Arg
            260                 265                 270
Tyr Met Ala Gly Gly Asn Val Asn Arg Phe Asp Gly Asn Lys Gln Trp
        275                 280                 285
Thr Leu Ile Gly Ser Ala Asn Asn Thr Asn Asn Met Gly Phe Ser Glu
    290                 295                 300
Met Asp Ser Glu Met Gly Ser Met Thr Phe Phe Ser Pro Gln Gly Gly
305                 310                 315                 320
Gly Arg Arg Gly Phe Gly Asn Ser Gly Gly Val Thr Ser Ser Ser Met
                325                 330                 335
Leu Gly Gly Asn Phe Ser Val Glu Phe Ser Ser Ala Leu Asn Thr Gly
            340                 345                 350
Gly Asp Ala Arg Tyr Gly Tyr Asn Asp Lys Ala Ile Glu Thr Thr Lys
        355                 360                 365
Arg Val Glu Asn Ile Leu Ala Glu Gly Asn Thr Tyr Met Asp Glu Asn
    370                 375                 380

Ile Leu Glu Arg Ser Phe Ser His Asn Gly Gln Ala Arg Phe Arg Met
385                 390                 395                 400
Gln Trp Lys Pro Ser Glu Arg Thr Glu Val Val Phe Glu Pro Asp Leu
                405                 410                 415
Ser Ile Ser Lys Ile Asp Gly Phe Phe Asn Asp Thr Tyr Glu Thr Lys
                420                 425                 430
Asp Ala Thr Gly Ile Ser Ile Asn Lys Gly Ser Ile His Gln Thr Thr
        435                 440                 445
Gln Gly Asn Asn Phe Arg Leu Asn Gly Glu Leu Asp Ile Ser His Lys
    450                 455                 460
Leu Asn Asp Glu Gly Arg Thr Ile Ser Ala Ser Val Ser Gly Gly Leu
465                 470                 475                 480
Thr Asp Glu Asp Gly Asp Gly Ile Tyr Gln Ala Val Leu Gln Ser Val
                485                 490                 495
Glu Thr Asn Gln Lys Gln Phe Asn Asp Asn Ser Asn Leu Gln Tyr Arg
            500                 505                 510
Leu Arg Leu Ser Tyr Val Glu Pro Leu Gly Lys Asn Tyr Phe Ala Gln
        515                 520                 525
Ala Ile Leu Asn Arg Arg Phe Ser Arg Arg Asn Ser Asp Arg Glu Val
    530                 535                 540
Tyr Arg Leu Gly Asp Asp Gly Gln Tyr Ser Ile Leu Asp Ser Gln Tyr
545                 550                 555                 560
Gly Leu Ser Tyr Ser Asn Glu Phe Thr Gln Tyr Arg Ile Gly Leu Asn
                565                 570                 575
Leu Lys Lys Ile Ala Lys Thr Trp Asp Tyr Thr Val Gly Phe Asn Val
            580                 585                 590
Asp Pro Asn Arg Thr Val Ser Tyr Arg Ser Val Ala Gly Val Glu Gln
        595                 600                 605
Asp Lys Leu Ala Phe Asn Arg Val Asn Leu Ser Pro Met Leu Arg Ile
    610                 615                 620
Asn Tyr Lys Pro Ser Arg Thr Thr Asn Leu Arg Val Asp Tyr Arg Gly
625                 630                 635                 640
Arg Thr Thr Gln Pro Ser Ile Asn Gln Ile Ala Pro Val Gln Asp Ile
                645                 650                 655
Thr Asn Pro Leu Phe Val Thr Glu Gly Asn Pro Gly Leu Lys Pro Ser
            660                 665                 670
Tyr Ser Asn Asn Val Met Ala Met Phe Ser Asp Phe Asp Ala Lys Ser
        675                 680                 685
Gln Arg Ala Phe Asn Ile Val Phe Phe Gly Asn Tyr Thr Phe Asp Asp
    690                 695                 700
Ile Val Pro Asn Thr His Tyr Asp Pro Ser Thr Gly Ile Arg Thr Thr
705                 710                 715                 720
Arg Tyr Glu Asn Ala Ser Gly Thr Trp Gln Ala Asn Leu His Gly Thr
                725                 730                 735
Leu Ser Leu Pro Leu Lys Asn Arg Ala Phe Ser Phe Arg Met Ser Leu
            740                 745                 750
Phe Asn Arg Leu Ala Glu Gly Gln Ser Phe Ile Asn Asp Asp Lys Asn
        755                 760                 765
Lys Ala Leu Ser Phe Arg Thr Arg Glu Arg Leu Thr Leu Thr Tyr Arg
    770                 775                 780
Asn Asn Trp Ile Asp Thr Ser Ile Gly Gly Asn Ile Gly Phe Tyr Met
785                 790                 795                 800
Ala Asn Asn Ser Leu Ser Gly Gln Lys Asp Ser Arg Thr Tyr Asp Phe
                805                 810                 815
Gly Gly Asn Tyr Gln Val Ala Leu Thr Leu Pro Tyr Gly Phe Arg Ile
            820                 825                 830
Asp Ser Asp Val Glu Tyr Asn Thr Asn Ser Gly Tyr Ser Gly Gly Phe
        835                 840                 845
Ser Leu Asp Glu Trp Leu Trp Asn Ala Ser Leu Ser Tyr Ser Phe Leu
    850                 855                 860
Arg Asp Lys Ala Gly Thr Leu Arg Val Asn Gly Tyr Asp Ile Leu Gly
865                 870                 875                 880
Gln Arg Ser Ser Ile Ser Arg Ser Ala Ser Ala Ile Asn Ile Glu Glu
                885                 890                 895
Ser Met Ser Asn Thr Ile Gly Arg Tyr Val Met Val Asp Phe Ile Tyr
            900                 905                 910
Arg Phe Asn Ala Phe Ser Gly Gly Gly Ser Arg Ser Asp His Gln Arg
        915                 920                 925
Gly Asn Met Asn Arg Pro Gly Pro Pro Phe Gly Gly Gly Arg Arg Pro
    930                 935                 940
Ser
945

(2) INFORMATION FOR SEQ ID NO:369

(i) SEQUENCE CHARACTERISTICS:
(A) LENGTH: 412 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...412

(xi) SEQUENCE DESCRIPTION: SEQ ID NO:369

Gly Glu Tyr Pro Ala Asn Ser Asn Asp Lys Lys Glu Met Val Met Lys
1               5                   10                  15
Leu Ile Lys Arg Ser Leu Leu Leu Leu Gly Ala Val Leu Leu Ile Thr
            20                  25                  30
Leu Pro Ala Tyr Ser Gln Asn Asp Asp Ile Phe Glu Asp Asp Ile Tyr
        35                  40                  45
Thr Ser Arg Lys Glu Ile Arg Lys Gln Asn Gln Val Lys Asp Trp Gln
    50                  55                  60
Asn Gln Glu Asp Gly Tyr Gly Asp Asp Thr Glu Tyr Thr Val Ala Ser
65                  70                  75                  80
Asp Arg Asp Ile Asp Ala Tyr Asn Arg Arg Asp Gly Gln Ser Tyr Asp
                85                  90                  95
Gly Lys Lys Leu Ser Lys Asp Lys Lys Arg Asp Ser Thr Arg Ser Ser
            100                 105                 110
Val Pro Gly Arg Tyr Ser Arg Arg Leu Ala Arg Phe Tyr Lys Pro Asn
        115                 120                 125
Thr Ile Val Ile Ser Gly Ala Asp Asn Val Tyr Val Thr Asp Asp Gly
    130                 135                 140
Glu Tyr Phe Val Tyr Gly Asp Glu Tyr Tyr Asp Asp Ala Ser Ser Val
145                 150                 155                 160
Asn Ile Tyr Ile Asn Ser Pro Trp Cys Asp Pro Phe Pro Tyr Thr Ser
                165                 170                 175
Trp Tyr Pro Ser Phe Ser Gly Trp Tyr Asn Tyr Thr Trp Asn Tyr Pro
            180                 185                 190
Trp Phe Tyr Tyr Gly Ser His Ile Gly Trp Gly Gly Tyr Tyr Pro Gly
        195                 200                 205
Tyr Asn Trp Tyr Trp Ser Tyr Tyr Tyr Asp Pro Phe Tyr Asn Pro Tyr
    210                 215                 220
Gly Ile Gly Met Gly Trp Gly Tyr Pro Tyr Gly Trp Gly Ser Tyr Tyr
225                 230                 235                 240
Gly Trp Gly Gly Tyr Pro Gly Val Ile His His Tyr His His Tyr Pro
                245                 250                 255
Lys Lys Thr Tyr Ser Asn Gly Gln His Ser Gly Ala Tyr Tyr Ser Tyr
            260                 265                 270
Gly Arg Pro Asn Arg Ile Lys Gly Gly Thr Ser Gly Ala Lys Leu Gly
        275                 280                 285
Thr Gly Arg Tyr Asp Arg Ile Gln Asn Ser Ser Ser Gln Lys Asn Lys
    290                 295                 300
Phe Gly Leu Gln Ser Asn Lys Pro Asn Asn Asn Leu Gln Asn Val Lys
305                 310                 315                 320
Ser Gly Arg Thr Gly Arg Ala Asn Arg Asp Arg Asn Ile Glu Thr Val
                325                 330                 335
Thr Pro Asn Asn Gly Gln Lys Gln Asn Arg Pro Val Phe Gln Gln Asn
            340                 345                 350
Gln Ser Gly Asn Asp Arg Pro Thr Gly Arg Asn Ile Arg Ser Glu Arg
        355                 360                 365
Gln Gly Glu Asn Asn Asp Arg Thr Phe Ser Thr Pro Ser Arg Ser Asn
    370                 375                 380
Ser Asn Gly Gly Phe Ser Thr Pro Ser Arg Ser Ser Ser Gly Ser Met
385                 390                 395                 400
Ser Gly Gly Gly Gly Arg Ser Gly Arg Gly Arg Asn
                405                 410

(2) INFORMATION FOR SEQ ID NO:370

(i) SEQUENCE CHARACTERISTICS:
(A) LENGTH: 601 amino acids

(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...601

(xi) SEQUENCE DESCRIPTION: SEQ ID NO:370

Ser Asn Ser Ser Ser His Lys Trp Leu Ile Tyr Tyr His Ile Glu Lys
1               5                   10                  15
Thr Lys Ser Ile Met Ile Arg Lys Leu Ile Leu Leu Leu Ala Leu Met
            20                  25                  30
Pro Val Ala Ser Val Ala Phe Ala Val Pro Thr Asp Ser Thr Glu Ser
        35                  40                  45
Lys Asp Asn Arg Ile Leu Thr Ser Met Gln Ser Ser Ser Leu Asn Arg
    50                  55                  60
Asp Asp Ala Pro Asp Lys Trp Gln Pro Met His Ala Asn Phe Ser Ile
65                  70                  75                  80
Gln Ser Asp Met Leu Leu Ser Thr Ala Gln Lys Ser Lys Asn Thr Trp
                85                  90                  95
Phe Gly Asn Ser Tyr Ile Met Gly Ile Ile Lys Asn Asn Tyr Leu Glu
            100                 105                 110
Phe Gly Ala Arg Phe Glu Asp Leu Tyr Lys Pro Leu Pro Gly His Glu
        115                 120                 125
Pro Glu Met Gly Arg Gly Val Pro His Met Tyr Val Lys Gly Ser Tyr
    130                 135                 140
His Trp Ala Glu Leu Thr Met Gly Asp Phe Tyr Asp Gln Phe Gly Ser
145                 150                 155                 160
Gly Met Val Phe Arg Thr Tyr Glu Glu Arg Asn Leu Gly Ile Asp Asn
                165                 170                 175
Ala Val Arg Gly Gly Arg Ile Val Leu Thr Pro Phe Asp Gly Val Arg
            180                 185                 190
Val Lys Gly Ile Ala Gly Gln Gln Arg Asn Tyr Phe Asp Arg Thr Gly
        195                 200                 205
Lys Val Phe Asn Ser Gly Arg Gly Tyr Leu Leu Gly Ser Asp Leu Glu
    210                 215                 220
Leu Asn Val Glu Arg Trp Ser Ser Ala Met Arg Asp Asn Asp Tyr His
225                 230                 235                 240
Leu Ala Ile Gly Gly Ser Phe Val Ser Lys His Glu Ala Asp Glu Asp
                245                 250                 255
Ile Phe Val Gly Val Gly Glu Asp Arg Lys Arg Leu Asn Leu Pro Leu
            260                 265                 270
Asn Val Pro Ile Met Gly Leu Arg Thr Asn Phe Gln Lys Gly Gly Leu
        275                 280                 285
Ala Leu Tyr Ala Glu Tyr Gly Tyr Lys Tyr Asn Asp Pro Ser Ala Asp
    290                 295                 300
Asn Asp Tyr Ile Tyr His Asp Gly Gln Ala Ala Leu Leu Ser Ala Ser
305                 310                 315                 320
Tyr Ser Lys Lys Gly Met Ser Ile Leu Leu Gln Ala Lys Arg Cys Glu
                325                 330                 335
Asn Phe Ala Phe Arg Ser Lys Arg Ser Ala Gln Leu Thr Pro Leu Met
            340                 345                 350
Ile Asn Tyr Met Pro Ala Phe Thr Gln Ala His Thr Tyr Thr Leu Ala
        355                 360                 365
Ala Ile Tyr Pro Tyr Ala Thr Gln Pro Gln Gly Glu Trp Ala Phe Gln
    370                 375                 380
Gly Glu Leu Arg Tyr Asn Phe Ala Arg Arg Thr Ala Leu Gly Gly Arg
385                 390                 395                 400
Tyr Gly Thr Gly Leu Arg Ile Asn Val Ser His Val Arg Gly Leu Asp
                405                 410                 415
Lys Lys Met Leu Lys Glu Asn Pro Asp Glu Leu Ile Gly Thr Asp Gly
            420                 425                 430
Tyr Thr Val Ser Phe Phe Gly Met Gly Asp Leu Tyr Tyr Ser Asp Ile
        435                 440                 445
Asp Val Glu Ile Thr Lys Lys Val Ser Pro Gly Phe Asn Phe Thr Leu
    450                 455                 460
Thr Tyr Leu Asn Gln Ile Tyr Asn Asn Lys Val Leu His Gly Ala Ala

465                 470                 475                 480
Gly Glu Lys Pro Glu Lys Ile Tyr Ala Asn Ile Phe Val Tyr Asp Gly
                485                 490                 495
Lys Tyr Lys Leu Ser Asn Lys Val Ala Leu Arg Thr Glu Leu Gln Tyr
            500                 505                 510
Leu His Thr Lys Gln Asp Gln Gly Asp Trp Ile Tyr Gly Met Ala Glu
        515                 520                 525
Leu Ser Ile Leu Pro Ser Leu Met Leu Ser Leu Ser Glu Gln Tyr Asn
    530                 535                 540
Ile Gly Glu Thr Lys Lys His Tyr Val Met Gly Ser Val Thr Tyr Thr
545                 550                 555                 560
His Gly Ala His Arg Val Ala Phe Ser Ala Gly Lys Thr Arg Ala Gly
                565                 570                 575
Met Asn Cys Ser Gly Gly Val Cys Arg Val Val Pro Glu Thr Gln Gly
            580                 585                 590
Phe Tyr Leu Ser Tyr Ser Thr Asn Leu
        595                 600

(2) INFORMATION FOR SEQ ID NO:371

     (i) SEQUENCE CHARACTERISTICS:
          (A) LENGTH: 252 amino acids
          (B) TYPE: amino acid
          (D) TOPOLOGY: linear

     (ii) MOLECULE TYPE: protein

     (iii) HYPOTHETICAL: YES

     (vi) ORIGINAL SOURCE:
          (A) ORGANISM: Porphyromonas gingivalis

     (ix) FEATURE:
          (A) NAME/KEY: misc_feature
          (B) LOCATION 1...252

     (xi) SEQUENCE DESCRIPTION: SEQ ID NO:371

Arg Gly Ser Ser Ser Gly Ile Ser Ala Arg Gly Arg Asp Met Arg Ser
1               5                   10                  15
Leu Phe Leu Ser Ala Leu Arg Ser Ser Ser Leu His Gly Ser Glu Arg
            20                  25                  30
Arg Ser Arg Ile Ser Ser Ser Val Val Met Ser Ile Arg Gln Lys Ile
        35                  40                  45
Arg Leu Phe His Leu Ser Val Cys Ala Gln Thr His Asp His Leu Ile
    50                  55                  60
Glu Ile His Leu Val Cys Ile Glu Phe Gly Ala Ile Asp Thr Asp Glu
65                  70                  75                  80
Phe Arg Leu Ser Ser His Ala Tyr Thr Thr Ser Pro Thr His Thr Gly
                85                  90                  95
Ala Ile His His Asn Cys Ile Glu Arg Ser Tyr Gly Arg Tyr Leu Val
            100                 105                 110
Thr Phe Gly Gln Glu Arg Asn Glu Leu His His His Ser Arg Pro Asp
        115                 120                 125
Arg Asn Ala Glu Val Tyr Arg Phe Pro Phe Asp Asn Ala Phe His Ser
    130                 135                 140
Ile Arg Tyr Glu Ala Phe Arg Pro Ile Arg Pro Ile Val Cys His Asp
145                 150                 155                 160
Asp His Phe Ile Ala Ile Gly Ser His Leu Phe Phe Lys Asp Asn Gln
                165                 170                 175
Ile Phe Ser Ser Gly Ser Gln Tyr Asp Asn Tyr Thr Val Ala Cys Phe
            180                 185                 190
Val Glu Ser Leu His Asp Arg Glu Gln Arg Ser His Thr His Thr Ala
        195                 200                 205
Ser Gly Thr Asn His Cys Ala Asp Leu Phe Asp Met Arg Thr Leu Ser
    210                 215                 220
Gln Arg Thr Tyr His Ile Arg Asp Ile Val Ala Asp Phe Glu Phe Gly
225                 230                 235                 240
Gln Phe Leu Gly Arg Phe Ala His Gly Leu Asn His
                245                 250

(2) INFORMATION FOR SEQ ID NO:372

     (i) SEQUENCE CHARACTERISTICS:
          (A) LENGTH: 790 amino acids

(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...790

(xi) SEQUENCE DESCRIPTION: SEQ ID NO:372

Leu Leu Phe Ser Ser Pro Leu Pro Arg Met Asp Arg Pro Lys Pro Ser
1               5                   10                  15
Tyr Ile Val Arg Ile Ala Ala Ile Leu Cys Leu Phe Val Gly Arg Pro
            20                  25                  30
Leu Phe Ala Gln Ser Tyr Val Asp Tyr Val Asp Pro Leu Ile Gly Thr
        35                  40                  45
Leu Ser Ser Phe Glu Leu Ser Ala Gly Asn Thr Tyr Pro Val Ile Gly
    50                  55                  60
Leu Pro Trp Gly Met Asn Ser Trp Thr Pro Met Thr Gly Val Pro Gly
65                  70                  75                  80
Asp Gly Trp Gln Tyr Thr Tyr Ser Ala His Lys Ile Arg Gly Phe Lys
                85                  90                  95
Gln Thr His Gln Pro Ser Pro Trp Ile Asn Asp Tyr Gly Gln Phe Ser
            100                 105                 110
Leu Leu Pro Leu Thr Ala Pro Gln Lys Pro Ser Ser Asn Asp Ser Ile
        115                 120                 125
Ala Leu Thr Lys Trp Cys Lys Gln Leu Phe Ser Asp Glu Gln Thr Ser
    130                 135                 140
Trp Phe Ser His Lys Ala Glu Thr Ala Thr Pro Tyr Tyr Tyr Ser Val
145                 150                 155                 160
Tyr Leu Ala Asp Tyr Asp Thr Arg Val Glu Met Ala Pro Thr Glu Arg
                165                 170                 175
Ala Ala Ile Phe Arg Ile Arg Tyr Ser Gly Asn Thr Glu Ser Gly Ser
            180                 185                 190
Gly Arg Trp Leu Arg Leu Asp Ala Phe Thr Gly Gly Ser Glu Ile Ser
        195                 200                 205
Ile Val Asp Pro His Thr Val Val Gly Ile Ser Arg Lys Asn Ser Gly
    210                 215                 220
Gly Val Pro Ala Asn Phe Ala Cys Tyr Phe Ile Leu Gln Ser Asp Thr
225                 230                 235                 240
Pro Met Ala Asp Val Leu Leu Glu Thr Asp Thr Gly Lys Ser Asp Glu
                245                 250                 255
Gly Thr Arg Ala Trp Ala Ala Cys Arg Phe Asp Ser Gln Glu Val Thr
            260                 265                 270
Val Arg Val Ala Ser Ser Phe Ile Ser Val Glu Gln Ala Glu Arg Asn
        275                 280                 285
Leu Ala Glu Val Lys Gly Gln Ser Phe Asp Arg Ile Arg Leu Ala Gly
    290                 295                 300
Arg Glu Ala Trp Asn Lys Val Leu Gly Arg Ile His Val Glu Gly Gly
305                 310                 315                 320
Thr Lys Asp Glu Arg Thr Thr Phe Tyr Ser Ala Leu Tyr Arg Cys Leu
                325                 330                 335
Leu Phe Pro Arg Arg Phe Tyr Glu Glu Asp Ala Ser Gly Asn Phe Val
            340                 345                 350
His Tyr Ser Pro Tyr Asn Gly Glu Val Leu Pro Gly Tyr Leu Tyr Thr
        355                 360                 365
Asp Thr Gly Phe Trp Asp Thr Phe Arg Ala Leu Phe Pro Leu Leu Asn
    370                 375                 380
Leu Leu Tyr Pro Asp Glu Asn Ile Lys Ile Gln Glu Gly Leu Leu Asn
385                 390                 395                 400
Val Tyr Arg Glu Ser Gly Phe Phe Pro Glu Trp Ala Ser Pro Gly His
                405                 410                 415
Arg Asp Cys Met Ile Gly Asn Asn Ser Ala Ser Val Leu Ala Asp Ala
            420                 425                 430
Tyr Leu Lys Gly Val Arg Val Glu Asp Thr Arg Thr Leu Met Asn Gly
        435                 440                 445
Leu Leu His Ala Thr Lys Ala Val His Pro Lys Ile Ser Ser Thr Gly
    450                 455                 460
Arg Lys Gly Trp Glu Trp Tyr Asn Ser Leu Gly Tyr Val Pro Ala Asp

465                 470                 475                 480
Ala Gly Ile Asp Glu Ser Ala Ala Arg Thr Leu Glu Tyr Ala Tyr Asn
                485                 490                 495
Asp Trp Cys Ile Leu Arg Leu Gly Arg Thr Leu Gly Trp Asp Arg Ala
            500                 505                 510
Ala Leu Asp Thr Leu Ala His Arg Ser Met Asn Tyr Arg His Leu Phe
        515                 520                 525
Asp Pro Glu Thr Lys Leu Met Arg Gly Arg Asn Gln Asp Gly Ser Phe
    530                 535                 540
Arg Thr Pro Phe Ser Pro Phe Lys Trp Gly Asp Val Phe Thr Glu Gly
545                 550                 555                 560
Asn Ala Trp His Tyr Thr Trp Ser Val Phe His Asp Val Gln Gly Leu
                565                 570                 575
Ile Asp Leu Met Gly Gly Asp Arg Pro Phe Val Ser Met Leu Asp Ser
            580                 585                 590
Val Phe Asn Thr Pro Pro Met Phe Asp Glu Ser Tyr Tyr Gly Phe Val
        595                 600                 605
Ile His Glu Ile Arg Glu Met Gln Ile Ala Asp Met Gly Asn Tyr Ala
    610                 615                 620
His Gly Asn Gln Pro Ile Gln His Met Ile Tyr Leu Tyr Asn His Ala
625                 630                 635                 640
Gly His Pro Trp Lys Ala Gln Glu Arg Leu Arg Glu Val Met Gly Arg
                645                 650                 655
Leu Tyr Arg Pro Thr Pro Asp Gly Tyr Cys Gly Asp Glu Asp Asn Gly
            660                 665                 670
Gln Thr Ser Ala Trp Tyr Val Phe Ser Ala Leu Gly Phe Tyr Pro Val
        675                 680                 685
Thr Pro Ala Thr Asp Gln Tyr Val Leu Gly Ser Pro Ile Phe Ser Lys
    690                 695                 700
Val Ile Leu Ser Phe Pro Asp Gly His Lys Thr Val Leu His Ala Pro
705                 710                 715                 720
Ala Asn Ser Ala Asp Thr Pro Tyr Ile Arg Ser Ile Ser Val Glu Gly
                725                 730                 735
Lys Glu Trp Ser Cys Asn Tyr Leu Thr His Glu Gln Leu Arg Ser Ser
            740                 745                 750
Ala Ser Ile Gln Trp Met Met Asp Thr Lys Pro Asn Tyr Asn Arg Gly
        755                 760                 765
Met Lys Glu Ser Asp Arg Pro Tyr Ser Phe Ser Thr Glu Gln Gln Arg
    770                 775                 780
Arg Ala Asn His Ser Asn
785                 790

(2) INFORMATION FOR SEQ ID NO:373

    (i) SEQUENCE CHARACTERISTICS:
        (A) LENGTH: 286 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...286

    (xi) SEQUENCE DESCRIPTION: SEQ ID NO:373

Ile Cys Gly Ser Lys Met Asn Leu Ser Gly Leu Gln Ser Phe Thr Met
1               5                   10                  15
Met Lys Ser Met Arg Ser Val Leu Leu Leu Leu Phe Pro Leu Ser Leu
            20                  25                  30
Ile Thr Ala Leu Gly Cys Ser Asn Asn Lys Ala Ala Glu Ser Lys Ser
        35                  40                  45
Val Ser Phe Asp Ser Ala Tyr Leu Glu Arg Tyr Ile Pro Leu Arg Ala
    50                  55                  60
Asp Ile Asp Thr Pro Ser Leu His Val Met Ile Ser Tyr Val Tyr Pro
65                  70                  75                  80
Ser Gly Asp Asp Met Leu Thr Glu Ile Phe Asn Gly Leu Leu Phe Gly
                85                  90                  95
Asp Ser Leu Met Asp Ser Ser Ser Pro Glu Asn Ala Met Glu Gly Tyr

                100                 105                 110
Ala Gln Met Leu Gly Glu Asp Tyr Arg Ser Asn Asn Ala Glu Ala Asn
        115                 120                 125
Leu Gln Gly Leu Pro Ser Asp Leu Leu Asp Tyr Ile Tyr Lys Gln Glu
    130                 135                 140
Asn Thr Ile Ala Tyr Cys Asp Thr Gly Leu Ile Ser Thr Arg Ile Asn
145                 150                 155                 160
Thr Tyr Thr Tyr Glu Gly Gly Ala His Thr Glu Asn Thr Val Arg Phe
                165                 170                 175
Ala Asn Ile Leu Arg Thr Thr Gly Lys Val Leu Glu Glu Arg Asp Ile
            180                 185                 190
Phe Lys Ile Asp Tyr Ala Glu Arg Leu Ser Ala Leu Ile Ile Gly Gln
        195                 200                 205
Leu Val His Asp Phe Gly Lys Thr Thr Pro Ala Glu Leu Asp Ala Ile
    210                 215                 220
Gly Phe Phe Asn Ala Glu Glu Ile Gln Pro Asn Gly Asn Phe Met Ile
225                 230                 235                 240
Asp Asp Lys Gly Leu Thr Tyr Cys Phe Asn Glu Tyr Gln Ile Ala Ala
                245                 250                 255
Tyr Ala Arg Gly Ala Val Tyr Val Arg Leu Gly Tyr Asp Val Leu Ala
            260                 265                 270
Pro Leu Leu Arg Asp Asp Ser Pro Leu Lys Arg Tyr Leu Pro
        275                 280                 285

(2) INFORMATION FOR SEQ ID NO:374

    (i) SEQUENCE CHARACTERISTICS:
        (A) LENGTH: 378 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...378

    (xi) SEQUENCE DESCRIPTION: SEQ ID NO:374

Gly Ile Ile Ser Val Leu Ser His Val Val Asp Arg Pro Gln Trp Gly
1               5                   10                  15
Ala Ser Pro Glu Ala Ala Gly Thr His Ser Val Tyr Ser Ile Leu His
            20                  25                  30
Pro Ser Ala Gly Ile Ile Arg Ile Arg Ser Met Gly Ile Ile Ser Ala
        35                  40                  45
Cys Arg Ile Ala Ile Leu Ala Gly Lys His Pro Arg Ser Gly Leu Ser
    50                  55                  60
Arg Ala Asn Val Gly Ile Leu Ser Tyr Asn Pro Glu Asn Thr Pro Glu
65                  70                  75                  80
Lys Lys Arg Lys Leu Gln Glu Lys Asn Val Phe Leu Gln Ile Arg Leu
                85                  90                  95
Arg Gln Ser Phe Asn Asn Leu Ile Pro Ser Leu Pro Phe Arg Ile Asp
            100                 105                 110
Asn Thr Lys Lys Ile Thr Glu Met Lys Lys Thr Thr Leu Thr Gly Ser
        115                 120                 125
Ile Cys Ala Leu Leu Leu Phe Leu Gly Leu Ser Ala Asn Ala Gln Ser
    130                 135                 140
Lys Leu Lys Ile Lys Ser Ile Glu Ala Ala Thr Thr Phe Ser Ser Ala
145                 150                 155                 160
Thr Ala Gly Asn Gly Phe Gly Gly Asn Ile Phe Gly Met Asp Met Ser
                165                 170                 175
Ile Arg Met Arg Val His His Ser Ile Leu Pro Glu Gly Leu Asp Phe
            180                 185                 190
Ser Val Gly Ile His Glu Arg Arg Ala His Trp Glu Glu Ala Gly Ser
        195                 200                 205
Pro Lys Leu Met Tyr Thr Asn Val Pro Ser Ile Ile Gly Ile Val Glu
    210                 215                 220
Lys Val Ile Val Phe Glu Asp Ala Glu Asp Phe Phe Asp Lys Lys Ala
225                 230                 235                 240
Leu Gly Arg Phe Leu Ile Ser Leu Gly Ile Ser Tyr Thr Lys His Leu

                    245                     250                     255
Gly Ala Tyr Trp Gly Trp Thr Asn Asp Ala His Ile Leu Phe Ser Pro
            260                     265                     270
Ile Pro Lys Ser Lys Val His Tyr Asp Thr Tyr Thr Arg Ala Gly Ser
        275                     280                     285
Asp Leu Val Leu Gln Ser Glu Asp Val Ala Thr Val Ser Asn Gly Phe
    290                     295                     300
Ser Pro Gly Ile Gly Leu Lys Ser Ser Ile Trp Trp Lys Met Pro Ile
305                     310                     315                     320
Lys Ser Lys Tyr Asp Phe Arg Leu Gly Phe Ser Leu Gly Tyr Glu Tyr
                    325                     330                     335
Leu Asn Leu Leu Tyr Pro Tyr Arg Asn Phe Lys Leu Asp Gly Asn Lys
            340                     345                     350
Pro Leu Ser Ala Leu Ser Pro Arg Met Asn His Ile Gly His Val Gly
        355                     360                     365
Phe Asn Phe Thr Val Gly Leu Trp Thr Asn
    370                     375

(2) INFORMATION FOR SEQ ID NO:375

      (i) SEQUENCE CHARACTERISTICS:
           (A) LENGTH: 1269 amino acids
           (B) TYPE: amino acid
           (D) TOPOLOGY: linear

     (ii) MOLECULE TYPE: protein

    (iii) HYPOTHETICAL: YES

     (vi) ORIGINAL SOURCE:
           (A) ORGANISM: Porphyromonas gingivalis

     (ix) FEATURE:
           (A) NAME/KEY: misc_feature
           (B) LOCATION 1...1269

     (xi) SEQUENCE DESCRIPTION: SEQ ID NO:375

Val Lys Cys Met Gly Lys Tyr Lys Arg Ala Lys Tyr Arg Tyr Trp Leu
1               5                   10                  15
Phe Pro Phe Cys Ser Asp Tyr Tyr Thr Phe Glu Gly Val Thr Phe Leu
            20                  25                  30
Cys Ala Ser Asp Asp Met Thr Thr Lys Lys Pro Gln Ala Ile Leu Asp
        35                  40                  45
Leu Glu Lys Ala Tyr Asn Ile Glu Ile Pro Asp Leu Ser Ser Gln Glu
    50                  55                  60
Gly Ile Ser Trp Ser Val Asn Arg Tyr Phe Lys Gln Asp Ser Ser Gly
65                  70                  75                  80
Ala Val Val Glu Leu Cys Leu Arg Glu Cys Gln Ile Glu Ser Met Thr
                85                  90                  95
Trp Leu Ile Asp Phe Pro Ala Leu Lys Lys Leu Asp Leu Ser Tyr Asn
            100                 105                 110
Gln Ile Ser Lys Leu Glu Gly Leu Glu Arg Leu Thr Ser Leu Thr Lys
        115                 120                 125
Leu Arg Leu Arg Ser Asn Gln Ile Arg Lys Leu Glu Gly Leu Asp Ser
    130                 135                 140
Leu Thr Ser Leu Thr Lys Leu Ser Leu Ser Asp Asn Gln Ile Ser Lys
145                 150                 155                 160
Leu Glu Gly Leu Glu Arg Leu Thr Ser Leu Ala Glu Leu Tyr Leu Leu
                165                 170                 175
Asp Asn Gln Ile Ser Lys Leu Glu Gly Leu Glu Arg Leu Thr Ser Leu
            180                 185                 190
Ala Thr Leu Glu Leu Ser Gly Asn Gln Ile Arg Lys Leu Glu Gly Leu
        195                 200                 205
Glu Arg Leu Thr Ser Leu Ala Thr Leu Glu Leu Ser Gly Asn Gln Ile
    210                 215                 220
Arg Lys Leu Glu Gly Leu Glu Arg Leu Thr Ser Leu Thr Lys Leu Arg
225                 230                 235                 240
Leu Arg Ser Asn Gln Ile Ser Lys Leu Glu Gly Leu Glu Arg Leu Thr
                245                 250                 255
Ser Leu Ala Thr Leu Glu Leu Ser Gly Asn Gln Ile Arg Lys Leu Glu
            260                 265                 270
Gly Leu Glu Arg Leu Thr Ser Leu Ala Thr Leu Glu Leu Ser Gly Asn
        275                 280                 285
Gln Ile Ser Lys Leu Glu Gly Leu Glu Arg Leu Ser Ser Leu Thr Lys

290                 295                 300
Leu Arg Leu Arg Ser Asn Gln Ile Ser Lys Leu Glu Gly Leu Glu Arg
305                 310                 315                 320
Leu Thr Ser Leu Thr Lys Leu Ser Leu Ser Asp Asn Gln Ile Ser Lys
                325                 330                 335
Leu Glu Gly Leu Glu Arg Leu Thr Ser Leu Ala Glu Leu Tyr Leu Leu
            340                 345                 350
Asp Asn Gln Ile Arg Lys Leu Glu Gly Leu Glu Arg Leu Thr Ser Leu
        355                 360                 365
Thr Lys Leu Arg Leu Arg Ser Asn Gln Ile Ser Lys Leu Glu Gly Leu
    370                 375                 380
Asp Ser Leu Thr Ser Leu Thr Lys Leu Ser Leu Ser Asp Asn Gln Ile
385                 390                 395                 400
Ser Lys Leu Glu Gly Leu Glu Arg Leu Thr Ser Leu Ala Glu Leu Tyr
                405                 410                 415
Leu Leu Asp Asn Gln Ile Arg Lys Leu Glu Gly Leu Asp Gly Leu Ala
            420                 425                 430
Ser Leu Thr Arg Leu Ser Leu Arg Arg Asn Gln Ile Ser Lys Leu Glu
        435                 440                 445
Gly Leu Asp Arg Leu Lys Val Leu Arg Lys Leu Asp Val Ser Gly Asn
    450                 455                 460
Asp Ile Gln Ser Ile Asp Asp Ile Lys Leu Leu Ala Pro Ile Leu Glu
465                 470                 475                 480
Gln Thr Leu Glu Lys Leu Arg Ile His Asp Asn Pro Phe Val Ala Ser
                485                 490                 495
Ser Gly Leu Ile Leu Ser Pro Tyr Asp Asn His Leu Pro Glu Ile Lys
            500                 505                 510
Ala Leu Leu Glu Lys Glu Lys Glu Lys Gln Lys Lys Thr Ser Val Glu
        515                 520                 525
Tyr His Pro Phe Cys Lys Val Met Leu Leu Gly Asn His Ser Ser Gly
    530                 535                 540
Lys Thr Thr Phe Leu Ser Gln Tyr Asp Thr Asn Tyr Thr Tyr Gln Lys
545                 550                 555                 560
Asn Thr His Val Leu Ser Ile His Arg Ser Asn Asn Pro Asn Ala Ile
                565                 570                 575
Phe Tyr Asp Phe Gly Gly Gln Asp Tyr Tyr His Gly Ile Tyr Gln Ala
            580                 585                 590
Phe Phe Thr Thr Gln Ser Leu Tyr Leu Leu Phe Trp Asp Ala Lys Lys
        595                 600                 605
Asp Arg Asn Phe Val Ser Val Asp Asp Lys Glu Tyr Gln Thr Leu Asn
    610                 615                 620
Phe Asn Arg Pro Tyr Trp Leu Gly Gln Ile Ala Tyr Ala Cys Asn Arg
625                 630                 635                 640
Cys Met Ser Val Gly Gly Asn Pro Asp Gly Lys Asp Thr Pro Gln Thr
                645                 650                 655
Thr Asp Asp Thr Ile Ile Ile Gln Thr His Ala Asp Glu Thr Gly Ala
            660                 665                 670
Lys Gln Gln Thr Leu Gly Cys Ala Ala Glu Asn Gly Val Leu Glu Glu
        675                 680                 685
Ile Tyr Val Ser Leu Glu Pro Lys Ala Asn Ser Ala Val His Ala Leu
    690                 695                 700
Asn Tyr Leu Asn Glu Arg Val Arg Glu Val Val Ala Ser Arg Ser Lys
705                 710                 715                 720
Ser Ile Gln Ile Thr Glu Lys Asp Lys Gly Leu Tyr Glu Ala Leu Pro
                725                 730                 735
Thr Ile Ala Gly Asp Asn Lys His Ile Pro Ile Ser Leu Glu Ala Leu
            740                 745                 750
Ala Ala Gln Leu Asn Lys Gly Arg Ala Glu Asn Asp Leu Tyr Thr Ile
        755                 760                 765
Glu Tyr Leu Gln Thr Glu Leu Asn Gln Leu Ser Leu Arg Gly Glu Val
    770                 775                 780
Leu Tyr Tyr Arg Glu Asn Glu Lys Leu Asn Asn Tyr Val Trp Leu Asp
785                 790                 795                 800
Pro Ala Ala Phe Val Gln Met Ile His Gly Glu Ile Leu Gln Lys Asp
                805                 810                 815
Asn Ile Asn Arg Gly Thr Val Pro Lys Asp Ile Phe Glu Cys Lys Leu
            820                 825                 830
His Asn Leu Ser Ser Gly Ser Ile Phe Glu Glu Asp Gly Gln Asn Gly
        835                 840                 845
Asn Met Ile Leu Gln Leu Leu Leu Glu Glu Leu Ile Val Tyr Glu Asp
    850                 855                 860
Lys Asp Cys Tyr Val Ile Pro Gly Tyr Leu Pro Leu His Ser Asp Asp
865                 870                 875                 880
Glu Ala Tyr Lys Trp Leu Thr Leu Gly Phe Glu Arg Pro Asn Phe Val
                885                 890                 895

Leu Lys Phe Glu Arg Phe Ile Pro Phe Gly Leu Ile Asn Gln Ile Ile
            900                 905                 910
Ala Tyr Tyr Gly Arg Glu Glu Gly Ala Leu Lys Arg Tyr Trp Arg Asp
        915                 920                 925
Gln Val Ile Phe Thr Ala Gly Arg Glu Met Asp Arg Gln Thr Leu Glu
    930                 935                 940
Gln Glu Glu Glu Lys Glu Gly Leu Pro Lys Thr Asn Ala Glu Asp Tyr
945                 950                 955                 960
Gln Ile Trp Ile Lys Leu Asp Phe Thr Asp Leu Ala Ile Ser Val Phe
                965                 970                 975
Ile Lys Glu Gln Arg Lys Thr Ser Ala Lys Asp Met Gln Arg Lys Glu
            980                 985                 990
Ala Thr Ile Leu Ser Asp Met Leu Asp Met Tyr Trp Asn Asn Ile Pro
        995                 1000                1005
Pro Arg Glu Gln Ile Gly Asp Lys Asp Thr Glu Gln Thr Arg Ser Thr
    1010                1015                1020
Ile Arg Glu Thr Asn Arg Lys Lys Arg Pro Ile Gln Asp Leu Tyr Leu
1025                1030                1035                1040
Ser Cys Ala Gln Ala Asp Lys Asp Leu Thr Glu Ser His Tyr Ile His
                1045                1050                1055
Leu Gly Thr Leu Asp Asp Glu Ser Lys Thr Thr Ala Arg Ile Ala Ala
            1060                1065                1070
Tyr Pro Leu Lys Asn Gly Val Ile Asp Lys Glu Arg Val Arg Glu Val
        1075                1080                1085
Ser Thr Arg Pro Tyr Lys His Leu Ser Val Asn Lys Asn Leu Ala Thr
    1090                1095                1100
Ala Lys Gln Ile Phe Ile Ser Tyr Ser Lys Glu Asp Gln Thr Glu Leu
1105                1110                1115                1120
Glu Thr Cys Leu Gln Phe Phe Lys Pro Leu Glu Lys Asn Gly Gln Ile
                1125                1130                1135
Glu Ile Tyr Tyr Asp Lys Leu Thr Lys Phe Glu Thr Pro Ile His Pro
            1140                1145                1150
Glu Ile Arg Lys Arg Ile Val Glu Ala Asp Cys Ile Ile Ala Leu Ile
        1155                1160                1165
Ser Gln Arg Tyr Leu Ala Thr Asp Tyr Ile Leu Asp His Glu Leu Pro
    1170                1175                1180
Val Phe Arg Glu Tyr Asn Lys Thr Ile Val Pro Ile Leu Ile Lys Pro
1185                1190                1195                1200
Cys Thr Phe Glu Asp Asp Glu Phe Leu Arg Glu Lys Tyr Phe Ala Gln
                1205                1210                1215
Lys Ala Gln Ile Ile Asn Leu Gly Lys Glu Gly Lys Thr Ile Lys Ala
            1220                1225                1230
Tyr Asp Ser Ile Thr Ala Ser Ala His Arg Asp Glu Asn Trp Val Ala
        1235                1240                1245
Val Val Arg Glu Phe Lys Glu Lys Ile Leu Arg Ile Thr Lys Gln Glu
    1250                1255                1260
Val Asn Thr Asp Glu
1265

(2) INFORMATION FOR SEQ ID NO:376

    (i) SEQUENCE CHARACTERISTICS:
        (A) LENGTH: 231 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...231

    (xi) SEQUENCE DESCRIPTION: SEQ ID NO:376

Lys Phe Met Met Lys Lys Ala Phe Val Phe Val Leu Leu Val Cys Leu
1               5                   10                  15
Phe Ser Ser Phe Ser Ser Ser Ala Gln Thr Thr Thr Asn Ser Ser Arg
            20                  25                  30
Ser Tyr Phe Thr Gly Arg Ile Glu Lys Val Ser Leu Asn Leu Gly Val
        35                  40                  45

Pro Pro Val Ser Thr Glu Val Trp Gly Met Thr His Asp Ala Asn Gly
    50                  55                  60
Leu Pro Phe Glu Ile Pro Ile Ser Phe Ser Arg Phe Asn Ser Gln Gly
65                  70                  75                  80
Asp Ile Ala Thr Thr Tyr Tyr Ile Ala Asn Ser Glu Ala Thr Leu Asn
                85                  90                  95
Glu Trp Cys Asp Tyr Ala His Pro Gly Gly Ile Val Arg Val Glu Gly
            100                 105                 110
Arg Phe Trp Lys Met Thr Tyr Asn Ile Pro Thr Tyr Asn Ala Val Cys
        115                 120                 125
Thr Arg Ile Thr Phe Glu Asn Gln Glu Ile Glu Gly Thr Ile Val Leu
    130                 135                 140
Ile Pro Lys Pro Lys Val Ser Leu Pro His Val Ser Glu Ser Val Pro
145                 150                 155                 160
Cys Ile Arg Thr Glu Ala Gly Arg Glu Phe Ile Leu Cys Glu Glu Asp
                165                 170                 175
Asp Thr Phe Val Ser His Asp Gly Asn Glu Val Thr Ile Gly Gly Lys
            180                 185                 190
Pro Phe Leu Leu Asn Thr Asn Val Lys Ile Val Gly Asp Val Ser Gln
        195                 200                 205
Lys Tyr Ala Val Gly Val Gly Glu Ile Arg Phe Leu Gln Ile Cys Ala
    210                 215                 220
Gln Thr Val Ser Gln Gln Lys
225                 230

(2) INFORMATION FOR SEQ ID NO:377

    (i) SEQUENCE CHARACTERISTICS:
        (A) LENGTH: 563 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...563

    (xi) SEQUENCE DESCRIPTION: SEQ ID NO:377

Ser Leu Ile Asn Asn Ser Arg Asp Trp Arg Ala Pro Val Arg Leu Ser
1               5                   10                  15
Leu Lys Lys Lys Thr Lys Thr Met Lys Thr Lys Val Leu Arg Lys Phe
            20                  25                  30
Val Val Ala Ala Phe Ala Val Ala Thr Leu Cys Pro Leu Ala Gln Ala
        35                  40                  45
Gln Thr Met Gly Gly Asp Asp Val Lys Val Val Gln Tyr Asn Gln Glu
    50                  55                  60
Lys Leu Val Gln Thr Arg Met Ser Val Ala Asp Asn Gly Trp Ile Tyr
65                  70                  75                  80
Val Met Thr His Ser Gly Tyr Asp Thr Gly Asn Ser Asn Val Lys Ile
                85                  90                  95
Phe Arg Ser Lys Asp Gln Gly Ala Thr Tyr Gln Lys Leu Arg Asp Trp
            100                 105                 110
Asp Pro Ser Asp Asp Tyr Gln Phe Gln Asp Phe Asp Ile Val Val Thr
        115                 120                 125
Gly Lys Asn Glu Ser Asp Ile Lys Ile Trp Ser Val Glu Leu Met Asn
    130                 135                 140
Lys Pro Gly Gly Tyr Lys Ser Arg Val Ala Val Phe Ser Arg Asp Ala
145                 150                 155                 160
Asn Ala Gln Asn Ala Lys Leu Val Tyr Lys Glu Asp Phe Ser Asn Val
                165                 170                 175
Gln Leu Tyr Asp Val Asp Ile Ala Ser Asn Tyr Arg Ser Pro Ser Ser
            180                 185                 190
Leu Asn Asn Gly Gly Asn Pro Phe Ala Leu Ala Phe Ala Tyr Thr Gly
        195                 200                 205
Phe Asn Asn Thr His Lys Ile Ser Phe Val Asp Tyr Val Phe Ser Leu
    210                 215                 220
Asn Gly Gly Gln Asn Phe Asn Lys Asn Leu Leu Phe Ser Gln Asp Gly
225                 230                 235                 240

Glu Lys Lys Ile Asp Lys Val Asp Leu Ser Leu Gly Ser Thr Ser Glu
                245                 250                 255
Ser Met Gly His Asn Ala Trp Pro Leu Met Gly Val Val Phe Glu Met
            260                 265                 270
Asn Lys Gln Gly Gly Lys Ser Asp Ile Gly Phe Leu Ser Asn Phe Val
        275                 280                 285
Asp Asn Asp Pro Glu Phe Gln Trp Ser Gly Pro Ile Lys Val Ser Glu
    290                 295                 300
Ser Asp Met Ser Phe Ser Pro Lys Ile Gln Met Leu Leu Asp Glu Asp
305                 310                 315                 320
Asn Asn Thr Ile Asn Gly Glu Ser Cys His Asn Phe Met Ile Thr Tyr
                325                 330                 335
Ser Asp Tyr Asp Ser Glu Tyr Ser Asp Trp Asp Ile Arg Tyr Val Tyr
            340                 345                 350
Pro Lys Lys Ser Phe Lys Tyr Glu Lys Gly Lys Thr Pro Thr Met Asp
        355                 360                 365
Asp Leu Val Glu Ala Phe Leu Thr Ala Ser Tyr Gln Ser Glu Thr Asn
    370                 375                 380
Ser Gly Leu Gly Tyr Asp Lys Asn Ala Asn His Tyr Leu Ile Thr Tyr
385                 390                 395                 400
Ala Lys Lys Glu Glu Asn Gly Thr Asn Thr Leu Lys Tyr Arg Trp Ala
                405                 410                 415
Asn Tyr Asp Lys Ile His Asn Lys Asp Leu Trp Ser Asp Thr Phe Thr
            420                 425                 430
Tyr Thr Ser Ser Ala Asn Ala Leu Tyr Thr Pro Gln Val Asp Ile Asn
        435                 440                 445
Pro Thr Lys Gly Leu Val Cys Trp Ser Trp Val Glu Tyr Leu Pro Gly
    450                 455                 460
Lys Arg Ile Val Trp Ser Asp Thr Gln Trp Thr His Ala Asn Gly Val
465                 470                 475                 480
Glu Asp Ile Val Met Gln Glu Gly Ser Met Lys Leu Tyr Pro Asn Pro
                485                 490                 495
Ala Gln Glu Tyr Ala Val Ile Ser Leu Pro Thr Ala Ala Asn Cys Lys
            500                 505                 510
Ala Val Val Tyr Asp Met Gln Gly Arg Val Val Ala Glu Ala Ser Phe
        515                 520                 525
Ser Gly Asn Glu Tyr Arg Leu Asn Val Gln His Leu Ala Lys Gly Thr
    530                 535                 540
Tyr Ile Leu Lys Val Val Ser Asp Thr Glu Arg Phe Val Glu Lys Leu
545                 550                 555                 560
Ile Val Glu

(2) INFORMATION FOR SEQ ID NO:378

    (i) SEQUENCE CHARACTERISTICS:
        (A) LENGTH: 786 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...786

    (xi) SEQUENCE DESCRIPTION: SEQ ID NO:378

Ile Val Tyr Leu Cys His Cys Met Asn His Arg Arg Ser Lys Thr Met
1               5                   10                  15
Leu Thr Ile Arg Asn Phe Leu Leu Phe Cys Cys Leu Ser Leu Ile Ala
            20                  25                  30
Phe Ala Ala Asp Ala Gln Ser Ser Val Ser Ser Gly Arg Arg Leu Thr
        35                  40                  45
Glu Tyr Val Asn Pro Phe Ile Gly Thr Ala Asn Tyr Gly Thr Thr Asn
    50                  55                  60
Pro Gly Ala Val Leu Pro Asn Gly Leu Met Ser Val Thr Pro Phe Asn
65                  70                  75                  80
Val Ser Gly Ser Thr Glu Asn Arg Phe Asp Lys Asp Ser Arg Trp Trp
                85                  90                  95

Ser Ala Pro Tyr Ser Ala Asp Asn Ser Tyr Cys Ile Gly Phe Ser His
            100                 105                 110
Val Asn Leu Ser Gly Val Gly Cys Pro Glu Leu Ser Gly Ile Leu Leu
        115                 120                 125
Met Ala Thr Ser Gly Thr Phe Asp Pro Asp Tyr Cys Cys Tyr Gly Ser
    130                 135                 140
Ser Leu Ser Arg Glu Tyr Ala Arg Pro Gly Glu Tyr Lys Ala Val Leu
145                 150                 155                 160
Asp Lys Tyr Gly Ile Asp Ala Ala Val Thr Val Thr Glu Arg Thr Ala
                165                 170                 175
Leu Thr Glu Phe Ala Phe Pro Glu Gly Glu Gly His Ile Leu Leu Asn
            180                 185                 190
Leu Gly Gln Ala Leu Ser Asn Glu Ser Gly Ala Ser Val Arg Phe Leu
        195                 200                 205
Asn Asp Ser Thr Val Val Gly Ser Arg Leu Met Gly Thr Phe Cys Tyr
    210                 215                 220
Asn Pro Gln Ala Val Phe Arg Gln Tyr Phe Val Leu Gln Val Ser Arg
225                 230                 235                 240
Arg Pro Ile Ser Ala Gly Tyr Trp Lys Lys Gln Pro Pro Met Thr Val
                245                 250                 255
Glu Ala Gln Trp Asp Ser Thr Ala Gly Lys Tyr Lys Gln Tyr Asp Gly
            260                 265                 270
Tyr Lys Arg Glu Met Ser Gly Asp Asp Ile Gly Val Arg Phe Ser Phe
        275                 280                 285
Asn Cys Asp Gln Gly Glu Lys Ile Tyr Val Arg Ser Ala Val Ser Phe
    290                 295                 300
Val Ser Glu Ala Asn Ala Leu Tyr Asn Leu Glu Ala Glu Gln Glu Glu
305                 310                 315                 320
Val Phe Lys Ser Val Gly Gly Asn Pro Ala Lys Ala Phe Ser Ala Ile
                325                 330                 335
Arg Ser Arg Ala Ile Glu Arg Trp Glu Glu Ala Leu Gly Thr Val Glu
            340                 345                 350
Val Glu Gly Gly Thr Pro Asp Glu Lys Thr Ile Phe Tyr Thr Ala Leu
        355                 360                 365
Tyr His Leu Leu Ile His Pro Asn Ile Leu Gln Asp Ala Asn Gly Glu
    370                 375                 380
Tyr Pro Met Met Gly Ser Gly Lys Thr Gly Asn Thr Ala His Asp Arg
385                 390                 395                 400
Tyr Thr Val Phe Ser Leu Trp Asp Thr Tyr Arg Asn Val His Pro Leu
                405                 410                 415
Leu Cys Leu Leu Tyr Pro Glu Lys Gln Leu Asp Met Val Arg Thr Leu
            420                 425                 430
Ile Asp Met Tyr Arg Glu Ser Gly Trp Leu Pro Arg Trp Glu Leu Tyr
        435                 440                 445
Gly Gln Glu Thr Leu Thr Met Glu Gly Asp Pro Ser Leu Ile Val Ile
    450                 455                 460
Asn Asp Thr Trp Gln Arg Gly Leu Arg Ala Phe Asp Thr Ala Thr Ala
465                 470                 475                 480
Tyr Glu Ala Met Lys Lys Asn Ala Ser Ser Ala Gly Ala Thr His Pro
                485                 490                 495
Ile Arg Pro Asp Asn Asp Asp Tyr Leu Thr Leu Gly Phe Val Pro Leu
            500                 505                 510
Arg Glu Gln Tyr Asp Asn Ser Val Ser His Ala Leu Glu Tyr Tyr Leu
        515                 520                 525
Ala Asp Trp Asn Leu Ser Arg Phe Ala His Ala Leu Gly His Lys Glu
    530                 535                 540
Asp Ala Ala Leu Phe Gly Lys Arg Ser Leu Gly Tyr Arg His Tyr Tyr
545                 550                 555                 560
Asn Lys Glu Tyr Gly Met Leu Cys Pro Leu Leu Pro Asp Gly Ser Phe
                565                 570                 575
Leu Thr Pro Phe Asp Pro Lys Gln Gly Glu Asn Phe Glu Pro Asn Pro
            580                 585                 590
Gly Phe His Glu Gly Ser Ala Tyr Asn Tyr Ala Phe Phe Val Pro His
        595                 600                 605
Asp Ile Gln Gly Leu Ala Arg Leu Met Gly Gly Ala Lys Val Phe Ser
    610                 615                 620
Glu Arg Leu Gln Lys Val Phe Asp Glu Gly Tyr Tyr Asp Pro Thr Asn
625                 630                 635                 640
Glu Pro Asp Ile Ala Tyr Pro Tyr Leu Phe Ser Tyr Phe Pro Lys Glu
                645                 650                 655
Ala Trp Arg Thr Gln Lys Leu Thr Arg Glu Leu Ile Asp Lys His Phe
            660                 665                 670
Cys Asn Ala Pro Asn Gly Leu Pro Gly Asn Asp Asp Ala Gly Thr Met
        675                 680                 685
Ser Ala Trp Leu Val Tyr Ser Met Leu Gly Phe Tyr Pro Asp Cys Pro

690                 695                 700
Gly Ser Pro Thr Tyr Thr Leu Thr Ser Pro Val Phe Pro Arg Val Arg
705                 710                 715                 720
Ile Arg Leu Asn Pro Gln Tyr Tyr Pro Gln Gly Glu Leu Ile Ile Thr
                725                 730                 735
Thr Asn Thr Glu Asn Gln Pro Thr Asp Ser Ile Tyr Ile His Thr Val
            740                 745                 750
Ser Leu Gly Asn Lys Thr Leu Pro His Gly Thr Arg His Ile Ser His
        755                 760                 765
Ala Asp Leu Val Arg Cys Gly His Leu Arg Tyr Glu Leu Ser Asn Arg
    770                 775                 780
Pro Arg
785

(2) INFORMATION FOR SEQ ID NO:379

    (i) SEQUENCE CHARACTERISTICS:
        (A) LENGTH: 814 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...814

    (xi) SEQUENCE DESCRIPTION: SEQ ID NO:379

Pro Arg Phe His Pro Gly Gly Thr His Ala Gln Leu Cys Arg Asn Gly
1               5                   10                  15
Arg Arg Asn Gln Lys Ser Asn Gln Ser Ser Cys Phe Gly Gly Gly Thr
            20                  25                  30
Ala Pro Arg Phe Phe Ile Met Cys Lys Ile Arg Phe Ser Leu Leu Gln
        35                  40                  45
Ala Leu Val Val Cys Leu Leu Phe Thr Ser Phe Ser Leu Gln Ala Gln
    50                  55                  60
Glu Glu Gly Ile Trp Asn Thr Leu Leu Ala Ile His Lys Thr Glu Lys
65                  70                  75                  80
Ala Val Glu Thr Pro Lys Lys Val Phe Ala Val Ala Asn Gly Val Leu
                85                  90                  95
Tyr Ser Val Gly Lys Glu Ala Pro His Glu Ala Lys Ile Phe Asp Arg
            100                 105                 110
Ile Ser Gly Leu Ser Asp Thr Ser Val Ser Ser Ile Ala Tyr Ser Glu
        115                 120                 125
Gln Leu Lys Ser Leu Val Ile Tyr Tyr Ala Ser Gly Asn Ile Asp Ile
    130                 135                 140
Leu Asp Glu Ala Gly Arg Val Thr Asn Val Pro Ala Leu Lys Asp Asn
145                 150                 155                 160
Ile Asp Leu Ile Asp Lys Thr Leu Asn Arg Leu Leu Ile Val Gly Asn
                165                 170                 175
Arg Ala Tyr Leu Ala Gly Gly Phe Gly Leu Ser Val Leu Asp Val Ala
            180                 185                 190
Glu Ala Arg Ile Pro Ala Thr Tyr Ala Lys Gly Thr Lys Val Thr Asp
        195                 200                 205
Val Ala Lys Leu Asp Asn Asp Arg Leu Leu Met Leu Lys Glu Gly Gln
    210                 215                 220
Leu Phe Ile Gly Lys Glu Thr Asp Asn Leu Gln Asp Pro Ala Ala Trp
225                 230                 235                 240
Thr Ala Leu Ser Leu Asn Leu Pro Met Gly Ser Val Thr Gly Leu Gly
                245                 250                 255
Ile Val Gly Glu Asp Ile Cys Phe Leu Leu Ala Asp Gly Arg Val Tyr
            260                 265                 270
Val Ala Ala Asn Gln Ser Phe Glu Pro Glu Leu Leu Leu Ser Ser Ser
        275                 280                 285
Ala Asp Ser Arg Leu Tyr Val Thr Asp Arg Gly Leu Phe Ile Cys Ala
    290                 295                 300
Glu Asn Arg Ile Tyr Phe Ile Glu Lys Gly Arg Lys Thr Thr Gln Phe
305                 310                 315                 320
Pro Ile Ala Asp Val Leu Gly Val Gly Ala Met Asn Glu Ser Asn Thr

                        325                 330                 335
Ala Tyr Ile Ala Leu Gly Glu Glu Gly Leu Ala Ser Leu Leu Leu Ala
                340                 345                 350
Glu Gly Ser Thr Ala Glu Ala Met Pro Val Ala Phe Asp Gly Pro Gly
            355                 360                 365
Asp Asn Asp Phe Tyr Glu Met Arg Phe Ser His Gly Arg Leu Tyr Ala
    370                 375                 380
Ala Ser Gly Leu Trp Gly Thr Asn Leu Met Gly His Ala Gly Met Val
385                 390                 395                 400
Lys Leu Tyr Asp Gly Asn Arg Trp Thr Asn Phe Asp Lys Lys Thr Val
                405                 410                 415
Gln Glu Gln Leu Gly Gly Gly Phe Ser Phe Asn Asp Ala Ile Asp Ile
                420                 425                 430
Ala Val Ser Asn Gly Asp Pro Asp His Phe Phe Val Gly Thr Trp Gly
            435                 440                 445
Asn Gly Leu Phe Glu Phe Lys Asp Gly Lys Ala Ile Ala Arg Tyr Ser
    450                 455                 460
Gly Asn Glu Thr Ala Ile Ala Glu Cys Asn Pro Gly Asp Ala Arg Val
465                 470                 475                 480
Lys Ala Ile Ala Phe Asp Asn Lys Gly Asn Leu Trp Gly Thr Leu Gly
                485                 490                 495
Ala Val Gly Lys Asn Ile Phe Met Tyr Asp Pro Gln Ser Ser Thr Trp
                500                 505                 510
His Ser Phe Ser Tyr Pro Asp Val Ala Asn Leu Ala Ser Phe Gly Asn
            515                 520                 525
Met Ile Ile Leu Pro Asn Gly Asp Lys Trp Val Asn Ile Leu His Arg
    530                 535                 540
Ser Gly Gly Ser Thr Arg Lys Gly Val Leu Ile Phe Asn Asp Arg Gly
545                 550                 555                 560
Thr Pro Glu Thr Thr Ser Asp Asp Ser His Leu Tyr Val Glu Gln Phe
                565                 570                 575
Val Asn Arg Leu Gly Ala Ala Ile Gly His Lys Thr Ile Tyr Ala Met
                580                 585                 590
Ala Val Asp His Asn Gly Ser Val Trp Met Gly Ser Asp Ile Gly Ile
            595                 600                 605
Phe Gly Val Tyr Asn Ala Ala Gly Val Leu Ser Ser Thr Ser Thr Pro
    610                 615                 620
Ile Ala Val Arg Pro Val Gly Gly Glu Glu Pro Asn Leu Tyr Tyr Val
625                 630                 635                 640
Leu Asp Lys Val Thr Val Thr Asp Ile Val Val Asp Lys Leu Asn His
                645                 650                 655
Lys Trp Val Ala Thr Gln Gly Thr Gly Leu Tyr Leu Leu Ser Glu Asp
                660                 665                 670
Cys Ser Lys Ile Leu Ala Gln Phe Thr Val Glu Asn Ser Pro Leu Leu
            675                 680                 685
Ser Asn Asn Ile Leu Ser Leu Ala Leu Asn Asp Asp Asn Gly Leu Leu
    690                 695                 700
Tyr Ile Gly Thr Ala Asp Gly Leu Met Thr Phe Gln Thr Gly Thr Gly
705                 710                 715                 720
Ser Gly Ser Ala Ser Glu Leu Asp Gly Val Tyr Val Tyr Pro Asn Pro
                725                 730                 735
Leu Arg Pro Glu Tyr Pro Asp Gly Val Thr Ile Ala Gly Leu Gln Ala
                740                 745                 750
Gly Cys Ser Val Lys Ile Thr Asp Thr Thr Gly Arg Leu Leu Tyr Gln
            755                 760                 765
Thr Glu Ser Val Thr Thr Glu Val Lys Trp Asn Ala Arg Gly Ala Asp
    770                 775                 780
Gly Asn Arg Val Ala Ser Gly Val Tyr Ala Val Ala Val Tyr Asp Pro
785                 790                 795                 800
Val Ser Lys Lys Ser Lys Leu Ile Arg Phe Ala Val Ile Arg
                805                 810

(2) INFORMATION FOR SEQ ID NO:380

    (i) SEQUENCE CHARACTERISTICS:
        (A) LENGTH: 1162 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

(ix) FEATURE:
(A) NAME/KEY: misc_feature
(B) LOCATION 1...1162

(xi) SEQUENCE DESCRIPTION: SEQ ID NO:380
   Ala Ile Ser Gln Met Lys Arg Ile Leu Pro Ile Val Ala Phe Leu Ser
   1               5                   10                  15
   Leu Phe Leu Ala Leu Ala Leu Pro Ala Lys Ala Gln Arg Ala Met Gly
               20                  25                  30
   Lys Thr Ala Asp Arg Ser Leu Met Ala Ser Gly His Trp Val Lys Ile
           35                  40                  45
   Arg Val Asp Ala Ser Gly Val Tyr Arg Leu Thr Asp Glu Gln Leu Arg
       50                  55                  60
   Ala Asn Gly Phe Ser Asp Pro Ser Lys Val Gly Val Phe Gly Tyr Gly
   65                  70                  75                  80
   Gly Gly Val Leu Pro Glu Asp Leu Ser Arg Ile Thr Thr Asp Asp Leu
                   85                  90                  95
   Pro Pro Val Pro Val Leu Arg Gln Gly Asn Ala Leu Tyr Phe Tyr Ala
               100                 105                 110
   Val Gly Pro Val Thr Trp Phe Tyr Asn Pro Ala Lys Thr Thr Met Glu
           115                 120                 125
   His Thr Val Asn Thr Tyr Ser Thr His Gly Tyr Tyr Phe Leu Ser Asp
       130                 135                 140
   Ala Ala Gly Ala Pro Leu Gln Met Ser Gln Tyr Thr Gly Gly Gly Ala
   145                 150                 155                 160
   Ser Ala Glu Ala Leu Ile Asp Tyr Tyr Asp Glu Leu Met Leu His Glu
                   165                 170                 175
   Gln Glu Leu Tyr Ser Pro Lys Glu Ser Gly Arg Asp Leu Tyr Gly Glu
               180                 185                 190
   Ser Phe Ser Ala Val Asn Thr Arg Thr Val Lys Phe Pro Leu Arg Gly
           195                 200                 205
   Asn Thr Arg Ser Ser Gly Glu Leu Gly Thr Val Phe Ser Tyr Ile Ala
       210                 215                 220
   Lys Ala Arg Ser Ala Gly Gly Gly Arg Glu Met Ser Leu Ser Ala Asn
   225                 230                 235                 240
   Gly Ile Leu Ile Phe Ser Asp Pro Phe Ser Met Thr Ser Asn Glu Val
                   245                 250                 255
   Ser Asn Ser Tyr Leu Ala Gly Lys Lys Arg Arg Leu Tyr His Ser Thr
               260                 265                 270
   Pro Met Asn Ser Leu Val Asn Glu Leu Arg Leu Asp Ala Asn Tyr Ser
           275                 280                 285
   Met Thr Gly Asp Ala Val Asn Leu Asp Phe Ile Glu Val Ala Thr Gln
       290                 295                 300
   Asn Asp Leu Arg Tyr Asp Gly Ala Pro Met His Ile Arg Arg Phe Ser
   305                 310                 315                 320
   Asn Leu Pro Val Leu Gly Gly Glu Ser Cys Arg Phe Val Ile Ser Glu
                   325                 330                 335
   Val Pro Glu Ser Leu Val Val Leu Gln Ala Asn Ser Ser Leu Thr Ala
               340                 345                 350
   Ser Leu Val Pro Val Lys Thr Val Gly Asp Lys Thr Ile Glu Phe Val
           355                 360                 365
   Ala Pro Pro Lys Gly Gln Asp Arg Arg Thr Ile Asn Thr Phe Tyr Ala
       370                 375                 380
   Val Asp Leu Ser Gln Ala Ser Ala Pro Glu Ile Leu Gly Ala Val Pro
   385                 390                 395                 400
   Asn Gln Asn Leu His Gly Glu Glu Ile Pro Asp Leu Ile Ile Val Ser
                   405                 410                 415
   Thr Gln Ala Leu Leu Leu Glu Ala Asp Arg Leu Ala Thr Tyr Arg Arg
               420                 425                 430
   Glu Lys Asn Gly Leu Lys Val Leu Val Val Leu Gln Glu Gln Val Phe
           435                 440                 445
   Asn Glu Phe Ser Gly Gly Thr Pro Asp Ala Thr Ala Tyr Arg Leu Phe
       450                 455                 460
   Ala Lys Met Phe Tyr Asp Arg Trp Lys Ala Asn Ala Pro Val Gly Glu
   465                 470                 475                 480
   Thr Phe Pro Met Gln Met Leu Leu Phe Gly Asp Gly Ala His Asp Asn
                   485                 490                 495
   Arg Lys Val Ser Val Ala Trp Gln Lys Pro Tyr Leu Gln Gln Thr Glu
               500                 505                 510
   Phe Leu Leu Thr Phe Gln Ala Val Asn Ser Thr Asn Val Asn Ser Tyr
           515                 520                 525
   Val Thr Asp Asp Tyr Phe Gly Leu Leu Asp Asp Gln Pro Ala Ser Val
       530                 535                 540
   Asn Ile Gly Trp Arg Asn Tyr Asn Met Ala Val Gly Arg Phe Pro Val
   545                 550                 555                 560
   Arg Thr Pro Ala Glu Ala Arg Ile Ala Val Asp Lys Thr Ile Arg Tyr
                   565                 570                 575
   Glu Glu Asp Arg Glu Ser Gly Ala Trp Arg Ile Arg Ala Cys Phe Ala
               580                 585                 590
   Ala Asp Asn Gly Asp Lys His Ala Thr Glu Thr Ser Arg Leu Ile Asp
           595                 600                 605
   Thr Val Lys Arg Tyr Ala Pro Ala Ile Met Pro Val Arg Ala Phe Gln
       610                 615                 620
   Asp Val Tyr Pro His Val Ile Glu Asn Gly Leu His Ser Ile Pro Gly
   625                 630                 635                 640
   Ala Lys Lys Lys Met Leu Glu Thr Leu Gln Ser Gly Ile Ile Leu Leu
                   645                 650                 655
   Asn Tyr Ala Gly His Gly Gly Pro Ala Gly Trp Ser Asp Glu His Leu
               660                 665                 670
   Leu Thr Leu Asn Asp Ile His Lys Phe Asn Tyr Lys His Met Pro Ile
           675                 680                 685
   Trp Ile Thr Ala Thr Cys Asp Phe Ala Asn Tyr Asp Ser Gln Thr Thr
       690                 695                 700
   Ser Ala Gly Glu Glu Val Phe Leu His Glu Lys Ser Gly Thr Pro Ile
   705                 710                 715                 720
   Met Phe Ser Thr Thr Arg Val Val Tyr Asn Thr Gln Asn Glu Lys Ile
                   725                 730                 735
   Asn Gly Phe Met Leu Arg Arg Met Phe Glu Lys Ala Lys Asp Gly Arg
               740                 745                 750
   Tyr Arg Thr Met Gly Glu Ile Ile Arg Ser Ala Lys Gln Gly Met Leu
           755                 760                 765
   Ser Thr Val Phe Pro Asp Ser Ile Asn Gln Leu Ser Phe Phe Leu Met
       770                 775                 780
   Gly Asp Pro Ser Val Arg Met Asn Leu Pro Thr His Lys Val Gln Leu
   785                 790                 795                 800
   Thr Ala Ile Asn Gly Gln Asp Pro Glu Gly Gln Tyr Gly Thr Ile Met
                   805                 810                 815
   Leu Lys Ser Leu Glu Arg Val Ala Leu Lys Gly Lys Val Thr Asp Glu
               820                 825                 830
   Lys Gly Thr Phe Asp Glu Thr Phe Ser Gly Lys Val Phe Leu Thr Val
           835                 840                 845
   Phe Asp Gly Arg Lys Lys Met Thr Ala Leu Glu Glu Glu Gly Asn Asp
       850                 855                 860
   Leu Ser Leu Val Tyr Tyr Asp Tyr Pro Asn Val Met Tyr Ala Gly Ile
   865                 870                 875                 880
   Ala Glu Val Lys Asp Gly Leu Phe Glu Thr Ser Phe Ile Val Pro Lys
                   885                 890                 895
   Asp Val Asn Tyr Ser Glu His Glu Gly Arg Ile Asn Leu Tyr Ala Tyr
               900                 905                 910
   Asn Glu Ser Thr Lys Ala Glu Ala Met Gly Val Asp Phe Ser Ile Arg
           915                 920                 925
   Val Gln Pro Gly Ile Pro Asp Glu Val Thr Glu Asp Asn Thr Pro Pro
       930                 935                 940
   Glu Ile Ile Ser Cys Phe Leu Asn Asp Ser Thr Phe Arg Ser Gly Asp
   945                 950                 955                 960
   Glu Val Asn Pro Thr Pro Leu Phe Met Ala Glu Val Phe Asp Leu Asn
                   965                 970                 975
   Gly Ile Asn Ile Thr Gly Ser Gly Val Gly His Asp Ile Thr Leu Cys
               980                 985                 990
   Ile Asp Gly Arg Ala Asp Leu Thr Tyr Asn Leu Asn Ala Tyr Phe Thr
           995                 1000                1005
   Ser Ser Ala Thr Asp Ala Gly Val Gly Thr Ile Leu Phe Met Ile Pro
       1010                1015                1020
   Ala Leu Ala Glu Gly Asp His Thr Ala Arg Leu Thr Val Trp Asp Ile
   1025                1030                1035                1040
   Phe Asn Asn Ala Val His His Asp Phe Ser Phe Arg Val Val Asp Gly
                   1045                1050                1055
   Ile Ala Pro Asp Val Ala Asp Val Ile Leu Phe Pro Asn Pro Val Arg
               1060                1065                1070
   Glu Ser Ala Thr Phe Arg Ile Phe His Asn Arg Pro Gly Ser Asp Leu
           1075                1080                1085
   Asn Val Ala Val Glu Ile Tyr Asp Phe Thr Gly Arg Leu Val Asn Ser
       1090                1095                1100
   Leu Pro Val Lys Thr Tyr Ser Ser Ser Tyr Gly Glu Pro Ile Glu Ile
   1105                1110                1115                1120
   Lys Trp Asp Leu Thr Ser Lys Tyr Gly Val Lys Ile Gly Asn Gly Phe
                   1125                1130                1135
   Tyr Leu Tyr Arg Cys Val Val Asn Ser Pro Gly Gly Gln Thr Ala Ser
   1140                    1145                    1150
   Met Ala Lys Lys Met Ile Val Val Gly Gln
           1155                    1160

(2) INFORMATION FOR SEQ ID NO:381

    (i) SEQUENCE CHARACTERISTICS:
        (A) LENGTH: 973 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...973

    (xi) SEQUENCE DESCRIPTION: SEQ ID NO:381

Phe Tyr Thr Gly Ile Asp Leu His Ile Glu Ser Gln Met Lys Lys Leu
1               5                   10                  15
Phe Pro Leu Leu Leu Leu Ile Leu Ser Ile Leu Val Gly Cys Gly Lys
            20                  25                  30
Lys Glu Lys His Ser Val Thr Glu Ile Ala Arg Glu Lys Lys Arg Ile
        35                  40                  45
Thr Ala Leu Leu Tyr Glu Lys Glu Leu Pro Thr Asp Ser Val Lys Gln
    50                  55                  60
Leu Tyr Glu Asn Ser Val Gln Asn Lys Asn Leu Val Gly Gln Met Leu
65                  70                  75                  80
Phe Ala Ile Glu Val Gly Lys Arg Met Arg Asn Met Ser Gln Tyr Thr
                85                  90                  95
Asp Ala Met Leu Tyr His Gln Lys Gly Leu Asn Ala Ala Leu Asn Leu
            100                 105                 110
Arg Asp Thr Ile Val Ala Ala Gln Ala Trp Asn His Leu Gly Thr Asp
        115                 120                 125
Ser Arg Arg Ile Gly Ala Leu Ala Glu Ala Ser Asp Tyr His Tyr Lys
    130                 135                 140
Ala Leu Ser Leu Ile Glu Ser Phe Ser Gly Asn Gln Asn Arg Pro Ala
145                 150                 155                 160
Ile Lys Ala Arg Ser Ala Ala Leu Asn Gly Ile Gly Asn Ile Asn Leu
                165                 170                 175
Glu Leu Gly Tyr His Asp Glu Ala Glu Lys Asn Phe Leu Lys Ala Leu
            180                 185                 190
Gln Gly Glu Lys Glu Leu Asp Ser Pro Leu Gly Gln Ala Ile Asn Tyr
        195                 200                 205
Ala Asn Leu Gly Arg Ile Tyr Arg Gln Arg Lys Glu Tyr Asp Lys Ala
    210                 215                 220
Arg Thr Tyr Phe Leu Leu Ser Leu Glu Gln Asn Asn Met Ala Glu Asn
225                 230                 235                 240
Leu Met Gly Ile Gly Leu Cys Ser Ile Asn Leu Gly Glu Val Asp Glu
                245                 250                 255
Glu Lys Gly Asp Tyr Gln Lys Ala Leu Gln Glu Tyr Ala Thr Ala Tyr
            260                 265                 270
Lys Leu Met Glu Gln Leu Ser Asp Arg Trp His Trp Leu Asn Ser Cys
        275                 280                 285
Ile Pro Met Ala Arg Ile Asn Leu Lys Gln Gly Asn Glu Arg Leu Tyr
    290                 295                 300
Gln His Phe Ile Ser Leu Ala Glu Gly Thr Ala Lys Glu Ile Asn Ser
305                 310                 315                 320
Thr Ser His Leu Ile Glu Ile Tyr Asn Leu Gln Tyr Glu Asn Leu Glu
                325                 330                 335
Arg Lys Lys Glu Tyr Lys Gln Ala Leu Glu Ala Phe Cys Leu Ser Lys
            340                 345                 350
Thr Leu Ser Asp Ser Met Ser Ile Ala His Lys Val Ser Ser Ile Gln
        355                 360                 365
Glu Thr Arg Phe Asn Tyr Glu Arg Asn Lys Ser Gln Lys Glu Leu Glu
    370                 375                 380
Glu Ile Gln Gln Val Ser Lys Ala Lys Gln Glu Lys Ser Lys Phe Ile
385                 390                 395                 400
Leu Leu Ser Thr Leu Phe Ala Leu Phe Ile Ser Ile Leu Leu Ile Ser

                              405                                     410                                     415
Val Leu Thr Tyr Ala Tyr Arg Gln Gly Lys Lys His Asn Lys Leu Ile
                    420                     425                     430
Lys Glu Thr Asp Lys Leu Arg Ser Gly Phe Phe Thr Gly Ile Thr His
            435                     440                     445
Glu Phe Arg Thr Pro Ile Thr Val Ile Gln Gly Leu Asn Glu Lys Met
    450                     455                     460
Ser Ser Ser Pro Asp Leu Gln Ala Ser Asp Arg Thr Glu Leu His Lys
465                     470                     475                     480
Ile Ile Asp Arg Gln Ser Ser His Met Leu Asn Leu Val Asn Gln Leu
                    485                     490                     495
Leu Asp Ile Cys Lys Ile Arg Ser Gly Val Ser Thr Pro Glu Trp Arg
                    500                     505                     510
Asn Gly Asp Ile Val Ser Phe Val Gln Ile Leu Ile Asp Ser Phe Ala
            515                     520                     525
Pro Tyr Ala Gln Ala Gln Asp Ile Thr Leu Glu Leu Gln Pro Glu Ser
    530                     535                     540
Lys Pro Ile Val Val Asp Phe Val Pro Ser Tyr Leu Gln Lys Ile Ile
545                     550                     555                     560
Ser Asn Leu Leu Ser Asn Ala Ile Lys Tyr Ser Leu Ala Gly Gly Arg
                    565                     570                     575
Val Val Ile Ser Leu Ala Lys Thr Lys Asn Glu Lys Asn Leu Ile Ile
                    580                     585                     590
Arg Val Ala Asp Asn Gly Ile Gly Ile Asp Lys Thr Asp Gln Ala His
            595                     600                     605
Ile Phe Asp Ile Phe Tyr Arg Gly Gln Ser Ala Thr Glu Lys His Gly
    610                     615                     620
Ser Gly Val Gly Leu Ser Phe Thr Asn Ile Leu Val Glu Asn Leu Arg
625                     630                     635                     640
Gly Thr Ile Lys Val Glu Ser Gln Pro Gly Lys Gly Ser Ala Phe Thr
                    645                     650                     655
Ile Ser Ile Pro Thr Gln Asn Gln Ser Ser Ser Ala Glu Ile Leu Pro
                    660                     665                     670
Trp Leu Pro Ser Ser Asp Asp Ile Val Met Pro Val His Ile Ala Pro
            675                     680                     685
Asp Asp Ser Pro Thr Ser Pro Met Val Ala Ala Leu Asn His Arg Phe
    690                     695                     700
Glu Asp Glu Arg Pro Thr Ile Leu Leu Val Glu Asp Asn Lys Asp Ile
705                     710                     715                     720
Asn Leu Leu Val Lys Leu Leu Leu Cys Asp Arg Tyr Asn Val Leu Ser
                    725                     730                     735
Ala Ala Asn Gly Lys Glu Gly Ile Ala Leu Ala Thr Glu His Ile Pro
                    740                     745                     750
Asp Ile Ile Ile Thr Asp Ile Met Met Pro Ile Met Asp Gly Ile Glu
            755                     760                     765
Met Thr Ile Arg Met Lys Gln Ser Pro Leu Leu Cys His Ile Pro Ile
    770                     775                     780
Val Ala Leu Thr Ala Lys Ser Thr Glu Gln Asp Arg Leu Glu Gly Ile
785                     790                     795                     800
Lys Ser Gly Val Val Ser Tyr Leu Cys Lys Pro Phe Ser Pro Glu Glu
                    805                     810                     815
Leu Leu Met Arg Ile Glu Gln Leu Leu Lys Asp Arg Glu Leu Leu Lys
                    820                     825                     830
Lys Phe Tyr Met Gln Lys Leu Met Leu Asp Arg Lys Pro Glu Glu Glu
            835                     840                     845
Pro Gln Pro Ile Asp Asp Ser Ser Met Gln Phe Leu Leu Ala Ala Lys
    850                     855                     860
Asp Ala Val Ser Gly Gly Ile Lys Gln Asn Pro Asp Phe Ser Ala Gln
865                     870                     875                     880
Asp Leu Ala Glu Lys Met Cys Met Ser Pro Ser Gln Leu Asn Arg Lys
                    885                     890                     895
Leu Thr Ser Val Val Gly Cys Ser Thr Ile Gly Tyr Ile Gln Gln Ile
                    900                     905                     910
Lys Ile Lys Leu Ala Cys Lys Leu Leu Ala Asp Glu Ser Lys Asn Ile
            915                     920                     925
Ser Asp Ile Ser Ile Glu Ala Gly Phe Ser Asp Pro Ala Tyr Phe Ser
    930                     935                     940
Arg Thr Phe Lys Arg Tyr Met Asn Cys Ser Pro Ser Gln Tyr Arg Gln
945                     950                     955                     960
Lys Leu Leu Ala Met Pro Gly Ser Asp Lys Glu Thr Val
                    965                     970

(2) INFORMATION FOR SEQ ID NO:382

    (i) SEQUENCE CHARACTERISTICS:

(A) LENGTH: 563 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...563

(xi) SEQUENCE DESCRIPTION: SEQ ID NO:382

His His Lys Thr Tyr Gln Thr Met Lys Lys Leu Leu Gln Ala Lys Ala
1               5                   10                  15
Leu Ile Leu Ala Leu Gly Leu Phe Gln Leu Pro Ala Ile Ala Gln Thr
            20                  25                  30
Gln Met Gln Ala Asp Arg Thr Asn Gly Gln Phe Ala Thr Glu Glu Met
        35                  40                  45
Gln Arg Ala Phe Gln Glu Thr Asn Pro Pro Ala Gly Pro Val Arg Ala
    50                  55                  60
Ile Ala Glu Tyr Glu Arg Ser Ala Ala Val Leu Val Arg Tyr Pro Phe
65                  70                  75                  80
Gly Ile Pro Met Glu Leu Ile Lys Glu Leu Ala Lys Asn Asp Lys Val
                85                  90                  95
Ile Thr Ile Val Ala Ser Glu Ser Gln Lys Asn Thr Val Ile Thr Gln
            100                 105                 110
Tyr Thr Gln Ser Gly Val Asn Leu Ser Asn Cys Asp Phe Ile Ile Ala
        115                 120                 125
Lys Thr Asp Ser Tyr Trp Thr Arg Asp Tyr Thr Gly Trp Phe Ala Met
    130                 135                 140
Tyr Asp Thr Asn Lys Val Gly Leu Val Asp Phe Ile Tyr Asn Arg Pro
145                 150                 155                 160
Arg Pro Asn Asp Asp Glu Phe Pro Lys Tyr Glu Ala Gln Tyr Leu Gly
                165                 170                 175
Ile Glu Met Phe Gly Met Lys Leu Lys Gln Thr Gly Gly Asn Tyr Met
            180                 185                 190
Thr Asp Gly Tyr Gly Ser Ala Val Gln Ser His Ile Ala Tyr Thr Glu
        195                 200                 205
Asn Ser Ser Leu Ser Gln Ala Gln Val Asn Gln Lys Met Lys Asp Tyr
    210                 215                 220
Leu Gly Ile Thr His His Asp Val Val Gln Asp Pro Asn Gly Glu Tyr
225                 230                 235                 240
Ile Asn His Val Asp Cys Trp Gly Lys Tyr Leu Ala Pro Asn Lys Ile
                245                 250                 255
Leu Ile Arg Lys Val Pro Asp Asn His Pro Gln His Gln Ala Leu Glu
            260                 265                 270
Asp Met Ala Ala Tyr Phe Ala Ala Gln Thr Cys Ala Trp Gly Thr Lys
        275                 280                 285
Tyr Glu Val Tyr Arg Ala Leu Ala Thr Asn Glu Gln Pro Tyr Thr Asn
    290                 295                 300
Ser Leu Ile Leu Asn Asn Arg Val Phe Val Pro Val Asn Gly Pro Ala
305                 310                 315                 320
Ser Val Asp Asn Asp Ala Leu Asn Val Tyr Lys Thr Ala Met Pro Gly
                325                 330                 335
Tyr Glu Ile Ile Gly Val Lys Gly Ala Ser Gly Thr Pro Trp Leu Gly
            340                 345                 350
Thr Asp Ala Leu His Cys Arg Thr His Glu Val Ala Asp Lys Gly Tyr
        355                 360                 365
Leu Tyr Ile Lys His Tyr Pro Ile Leu Gly Glu Gln Ala Gly Pro Asp
    370                 375                 380
Tyr Lys Ile Glu Ala Asp Val Val Ser Cys Ala Asn Ala Thr Ile Ser
385                 390                 395                 400
Pro Val Gln Cys Tyr Tyr Arg Ile Asn Gly Ser Gly Ser Phe Lys Ala
                405                 410                 415
Ala Asp Met Thr Met Glu Ser Thr Gly His Tyr Thr Tyr Ser Phe Thr
            420                 425                 430
Gly Leu Asn Lys Asn Asp Lys Val Glu Tyr Tyr Ile Ser Ala Ala Asp
        435                 440                 445
Asn Ser Gly Arg Lys Glu Thr Tyr Pro Phe Ile Gly Glu Pro Asp Pro
    450                 455                 460

Phe Lys Phe Thr Cys Met Asn Glu Thr Asn Thr Cys Thr Val Thr Gly
465                 470                 475                 480
Ala Ala Lys Ala Leu Arg Ala Trp Phe Asn Ala Gly Arg Ser Glu Leu
                485                 490                 495
Ala Val Ser Val Ser Leu Asn Ile Ala Gly Thr Tyr Arg Ile Lys Leu
            500                 505                 510
Tyr Asn Thr Ala Gly Glu Glu Val Ala Ala Met Thr Lys Glu Leu Val
        515                 520                 525
Ala Gly Thr Ser Val Phe Ser Met Asp Val Tyr Ser Gln Ala Pro Gly
    530                 535                 540
Thr Tyr Val Leu Val Val Glu Gly Asn Gly Ile Arg Glu Thr Met Lys
545                 550                 555                 560
Ile Leu Lys

(2) INFORMATION FOR SEQ ID NO:383

    (i) SEQUENCE CHARACTERISTICS:
        (A) LENGTH: 437 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...437

    (xi) SEQUENCE DESCRIPTION: SEQ ID NO:383

Thr Thr Asn Arg Lys Pro Asn Thr Asn Met Lys Leu Ser Ser Lys Lys
1               5                   10                  15
Ile Leu Ala Ile Ile Ala Leu Leu Thr Met Gly His Ala Val Gln Ala
            20                  25                  30
Gln Phe Val Pro Ala Pro Thr Thr Gly Ile Arg Met Ser Val Thr Thr
        35                  40                  45
Thr Lys Ala Val Gly Glu Lys Ile Glu Leu Leu Val His Ser Ile Glu
    50                  55                  60
Lys Lys Gly Ile Trp Ile Asp Leu Asn Gly Asp Ala Thr Tyr Gln Gln
65                  70                  75                  80
Gly Glu Glu Ile Thr Val Phe Asp Glu Ala Tyr His Glu Tyr Thr Ile
                85                  90                  95
Gly Thr Gln Thr Leu Thr Ile Tyr Gly Asn Thr Thr Arg Leu Gly Cys
            100                 105                 110
Arg Ser Thr Gly Ala Thr Ala Val Asp Val Thr Lys Asn Pro Asn Leu
        115                 120                 125
Thr Tyr Leu Ala Cys Pro Lys Asn Asn Leu Lys Ser Leu Asp Leu Thr
    130                 135                 140
Gln Asn Pro Lys Leu Leu Arg Val Trp Cys Asp Ser Asn Glu Ile Glu
145                 150                 155                 160
Ser Leu Asp Leu Ser Gly Asn Pro Ala Leu Ile Ile Leu Gly Cys Asp
                165                 170                 175
Arg Asn Lys Leu Thr Glu Leu Lys Thr Asp Asn Asn Pro Lys Leu Ala
            180                 185                 190
Ser Leu Trp Cys Ser Asp Asn Asn Leu Thr Glu Leu Glu Leu Ser Ala
        195                 200                 205
Asn Pro Arg Leu Asn Asp Leu Trp Cys Phe Gly Asn Arg Ile Thr Lys
    210                 215                 220
Leu Asp Leu Ser Ala Asn Pro Leu Leu Val Thr Leu Trp Cys Ser Asp
225                 230                 235                 240
Asn Glu Leu Ser Thr Leu Asp Leu Ser Lys Asn Ser Asp Val Ala Tyr
                245                 250                 255
Leu Trp Cys Ser Ser Asn Lys Leu Thr Ser Leu Asn Leu Ser Gly Val
            260                 265                 270
Lys Gly Leu Ser Val Leu Val Cys His Ser Asn Gln Ile Ala Gly Glu
        275                 280                 285
Glu Met Thr Lys Val Val Asn Ala Leu Pro Thr Leu Ser Pro Gly Ala
    290                 295                 300
Gly Ala Gln Ser Lys Phe Val Val Val Asp Leu Lys Asp Thr Asp Glu
305                 310                 315                 320

Lys Asn Ile Cys Thr Val Lys Asp Val Glu Lys Ala Lys Ser Lys Asn
                325                 330                 335
Trp Arg Val Phe Asp Phe Asn Gly Asp Ser Asp Asn Met Leu Pro Tyr
            340                 345                 350
Glu Gly Ser Pro Thr Ser Asn Leu Ala Val Asp Ala Pro Thr Val Arg
        355                 360                 365
Ile Tyr Pro Asn Pro Val Gly Arg Tyr Ala Leu Val Glu Ile Pro Glu
    370                 375                 380
Ser Leu Leu Gly Gln Glu Ala Ala Leu Tyr Asp Met Asn Gly Val Lys
385                 390                 395                 400
Val Tyr Ser Phe Ala Val Glu Ser Leu Arg Gln Asn Ile Asp Leu Thr
                405                 410                 415
His Leu Pro Asp Gly Thr Tyr Phe Phe Arg Leu Asp Asn Tyr Thr Thr
            420                 425                 430
Lys Leu Ile Lys Gln
        435

(2) INFORMATION FOR SEQ ID NO:384

    (i) SEQUENCE CHARACTERISTICS:
        (A) LENGTH: 318 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...318

    (xi) SEQUENCE DESCRIPTION: SEQ ID NO:384

Asn His Leu Lys Thr Asn Ile Lys Met Arg Lys Thr Ile Ile Phe Cys
1               5                   10                  15
Leu Leu Leu Ala Leu Phe Gly Cys Ser Trp Ala Gln Glu Arg Val Asp
            20                  25                  30
Glu Lys Val Phe Ser Ala Gly Thr Ser Ile Phe Arg Gly Ile Leu Glu
        35                  40                  45
Lys Val Lys Ala Pro Leu Met Tyr Gly Asp Arg Glu Val Trp Gly Met
    50                  55                  60
Ala Arg Ala Ser Glu Asp Phe Phe Phe Ile Leu Pro Val Thr Asp Asp
65                  70                  75                  80
Leu Thr Pro Val Leu Phe Tyr Asn Arg Leu Thr Asn Glu Pro Cys Phe
                85                  90                  95
Val Ser Asp Gln Gly Ile Thr Glu Tyr Phe Lys Phe Ala Gln Glu Gly
            100                 105                 110
Asp Tyr Ile Glu Val Glu Gly Ser Ser Val Phe Met Ala Asn Leu Leu
        115                 120                 125
Tyr Tyr Arg Phe Phe Pro Thr Arg Ile Thr Ser Tyr Asn Ala Pro Ile
    130                 135                 140
Glu Gly Val Val Ser Lys Thr Gly Asn Pro Ala Phe Thr Ile Pro Met
145                 150                 155                 160
Leu Pro Gly Val Ser Asp Cys Ile Glu Ile Ser Asn Asn Arg Lys Val
                165                 170                 175
Phe Leu Thr Asn Gln Leu Gly Val Val Asn Ile Thr Asp Gly Met Glu
            180                 185                 190
Pro Pro Ile Ile Ala Gly Val Ser Ala Ser Tyr Gly Ser Ser Val Arg
        195                 200                 205
Val Tyr Gly His Val Ser Gln Arg Trp Asp Ile Ile Gly His Cys Tyr
    210                 215                 220
Leu Asp Ile Tyr Pro Thr Asn Cys Tyr Pro Leu Ser Thr Lys Pro Val
225                 230                 235                 240
Ala Gly Asp Asp Glu Val Phe Val Lys Gln Gln Gly Arg Gln Ile Glu
                245                 250                 255
Ile Asp Ser Asn Ser Pro Ile Val Gln Val Val Val Tyr Asp Leu Glu
            260                 265                 270
Gly Lys Ser Val Phe Arg Lys Arg Met Thr Glu Asn Ala Tyr Thr Leu
        275                 280                 285
Ser Phe Arg Ala Pro Met Leu Gly Phe Met Thr Ile Met Ile Glu Thr
    290                 295                 300

Gln Asn Ser Ile Ile Asn Lys Lys Leu Asn Val Thr Gln Leu
305                 310                 315

(2) INFORMATION FOR SEQ ID NO:385

     (i) SEQUENCE CHARACTERISTICS:
          (A) LENGTH: 461 amino acids
          (B) TYPE: amino acid
          (D) TOPOLOGY: linear

     (ii) MOLECULE TYPE: protein

     (iii) HYPOTHETICAL: YES

     (vi) ORIGINAL SOURCE:
          (A) ORGANISM: Porphyromonas gingivalis

     (ix) FEATURE:
          (A) NAME/KEY: misc_feature
          (B) LOCATION 1...461

     (xi) SEQUENCE DESCRIPTION: SEQ ID NO:385

Arg Arg Ala Val Lys Ile Arg Ser Pro Pro His Ile His Ser Leu Phe
1               5                   10                  15
Val Arg Lys Cys Leu Phe Ser Asp Phe Lys Tyr Leu Tyr Leu Ser Arg
            20                  25                  30
Lys Ile Thr Gln Glu Arg Leu Gly Arg Leu Ser Ile Arg Leu Lys Ser
        35                  40                  45
Tyr Asn Pro Ile Ile Ile Ile Glu Met Lys Lys Thr Thr Ile Ile Ser
    50                  55                  60
Leu Ile Val Phe Gly Ala Phe Phe Ala Ala Val Gly Gln Thr Lys Asp
65                  70                  75                  80
Asn Ser Ser Tyr Lys Pro Phe Ser Lys Glu Asp Ile Ala Gly Gly Val
                85                  90                  95
Tyr Ser Leu Pro Thr Gln Asn Arg Ala Gln Lys Asp Asn Ala Glu Trp
            100                 105                 110
Leu Leu Thr Ala Thr Val Ser Thr Asn Gln Ser Ala Asp Thr His Phe
        115                 120                 125
Ile Phe Asp Glu Asn Asn Arg Tyr Ile Ala Arg Asp Ile Lys Ala Asn
    130                 135                 140
Gly Val Arg Lys Ser Thr Asp Ser Ile Tyr Tyr Asp Ala Asn Gly Arg
145                 150                 155                 160
Ile Ser His Val Asp Leu Tyr Ile Ser Phe Ser Gly Gly Glu Pro Ala
                165                 170                 175
Leu Asp Thr Arg Phe Lys Tyr Thr Tyr Asp Asp Glu Gly Lys Met Thr
            180                 185                 190
Val Arg Glu Val Phe Met Leu Val Met Asp Pro Asn Thr Pro Ile Ser
        195                 200                 205
Arg Leu Glu Tyr His Tyr Asp Ala Gln Gly Arg Leu Thr His Trp Ile
    210                 215                 220
Ser Phe Ala Phe Gly Ala Glu Ser Gln Lys Asn Thr Tyr His Tyr Asn
225                 230                 235                 240
Glu Lys Gly Leu Leu Val Ser Glu Val Leu Ser Asn Ala Met Gly Thr
                245                 250                 255
Thr Tyr Ser Asp Thr Gly Lys Thr Glu Tyr Ser Tyr Asp Asp Ala Asp
            260                 265                 270
Asn Met Val Lys Ala Glu Tyr Phe Val Val Gln Gln Gly Lys Ala Trp
        275                 280                 285
Gln Val Leu Lys Arg Glu Glu Tyr Thr Tyr Glu Asp Asn Ile Cys Ile
    290                 295                 300
Gln Tyr Leu Ala Ile Asn Gly Thr Asp Thr Lys Val Tyr Lys Arg Asp
305                 310                 315                 320
Ile Glu Ser Asp Lys Ser Ile Ser Ala Asn Val Ile Asp Ile Pro Ser
                325                 330                 335
Met Pro Glu Gln Thr Trp Pro Asn Met Tyr Gly Phe Asn Ala Lys Arg
            340                 345                 350
Leu Lys Glu Thr Tyr Ser Ser Tyr Glu Gly Asp Val Ala Thr Pro Ile
        355                 360                 365
Phe Asp Tyr Ile Tyr Thr Tyr Lys Ala Leu Thr Ser Met Ala Thr Pro
    370                 375                 380
Ser Thr Glu Ala Gln Val Ala Val Tyr Leu Asn Pro Ser Thr Asp Arg
385                 390                 395                 400
Leu Val Ile Leu Ala Asn Gly Ile Thr His Leu Ser Met Tyr Asp Leu
                405                 410                 415

Gln Gly Lys Leu Ile Arg Asp Cys Ala Leu Ser Gly Asp Lys Val Glu
            420                 425                 430
Met Gly Val Gly Ser Leu Thr Lys Gly Thr Tyr Leu Leu Lys Val Asn
        435                 440                 445
Thr Asp Gln Gly Ala Phe Val Arg Lys Val Val Ile Arg
    450                 455                 460

(2) INFORMATION FOR SEQ ID NO:386

(i) SEQUENCE CHARACTERISTICS:
(A) LENGTH: 451 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...451

(xi) SEQUENCE DESCRIPTION: SEQ ID NO:386
   Met Ala Lys Val Ile Lys Thr Lys Lys Gly Leu Ala Leu Asn Leu Lys
   1               5                   10                  15
   Gly Lys Pro Leu Pro Glu Met Leu Ala Glu Pro Ala Gln Ser Pro Thr
               20                  25                  30
   Tyr Ala Val Val Pro Asp Asp Phe Glu Gly Val Ile Pro Lys Val Thr
           35                  40                  45
   Ala Arg Pro Gly Asp Lys Val Arg Ala Gly Ser Ala Leu Met His His
       50                  55                  60
   Lys Ala Tyr Pro Glu Met Lys Phe Thr Ser Pro Val Ser Gly Glu Val
   65                  70                  75                  80
   Ile Ala Val Asn Arg Gly Ala Lys Arg Lys Val Leu Ser Ile Glu Val
                   85                  90                  95
   Lys Pro Asp Gly Leu Asn Glu Tyr Glu Ser Phe Pro Val Gly Asp Pro
               100                 105                 110
   Ser Ala Leu Ser Ala Glu Gln Ile Lys Glu Leu Leu Leu Ser Ser Gly
           115                 120                 125
   Met Trp Gly Phe Ile Lys Gln Arg Pro Tyr Asp Ile Val Ala Thr Pro
       130                 135                 140
   Asp Ile Ala Pro Arg Asp Ile Tyr Ile Thr Ala Asn Phe Thr Ala Pro
   145                 150                 155                 160
   Leu Ala Pro Asp Phe Asp Phe Ile Val Arg Gly Glu Glu Arg Ala Leu
                   165                 170                 175
   Gln Thr Ala Ile Asp Ala Leu Ala Lys Leu Thr Thr Gly Lys Val Tyr
               180                 185                 190
   Val Gly Leu Lys Pro Gly Ser Ser Leu Gly Leu His Asn Ala Glu Ile
           195                 200                 205
   Val Glu Val His Gly Pro His Pro Ala Gly Asn Val Gly Val Leu Ile
       210                 215                 220
   Asn His Thr Lys Pro Ile Asn Arg Gly Glu Thr Val Trp Thr Leu Lys
   225                 230                 235                 240
   Ala Thr Asp Leu Ile Val Ile Gly Arg Phe Leu Leu Thr Gly Lys Ala
                   245                 250                 255
   Asp Phe Thr Arg Met Ile Ala Met Thr Gly Ser Asp Ala Ala Ala His
               260                 265                 270
   Gly Tyr Val Arg Ile Met Pro Gly Cys Asn Val Phe Ala Ser Phe Pro
           275                 280                 285
   Gly Arg Leu Thr Ile Lys Glu Ser His Glu Arg Val Ile Asp Gly Asn
       290                 295                 300
   Val Leu Thr Gly Lys Lys Leu Cys Glu Lys Glu Pro Phe Leu Ser Ala
   305                 310                 315                 320
   Arg Cys Asp Gln Ile Thr Val Ile Pro Glu Gly Asp Asp Val Asp Glu
                   325                 330                 335
   Leu Phe Gly Trp Ala Ala Pro Arg Leu Asp Gln Tyr Ser Met Ser Arg
               340                 345                 350
   Ala Tyr Phe Ser Trp Leu Gln Gly Lys Asn Lys Glu Tyr Val Leu Asp
           355                 360                 365
   Ala Arg Ile Lys Gly Gly Glu Arg Ala Met Ile Met Ser Asn Glu Tyr
       370                 375                 380
   Asp Arg Val Phe Pro Met Asp Ile Tyr Pro Glu Tyr Leu Leu Lys Ala
   385                 390                 395                 400
   Ile Ile Ala Phe Asp Ile Asp Lys Met Glu Asp Leu Gly Ile Tyr Glu
                   405                 410                 415
   Val Ala Pro Glu Asp Phe Ala Thr Cys Glu Phe Val Asp Thr Ser Lys
               420                 425                 430
   Ile Glu Leu Gln Arg Ile Val Arg Glu Gly Leu Asp Met Leu Tyr Lys
           435                 440                 445
   Glu Met Asn
       450

(2) INFORMATION FOR SEQ ID NO:387

(i) SEQUENCE CHARACTERISTICS:
(A) LENGTH: 195 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...195

(xi) SEQUENCE DESCRIPTION: SEQ ID NO:387

Met Asp Lys Val Ser Tyr Ala Leu Gly Leu Ser Ile Gly Asn Asn Phe
1               5                   10                  15
Lys Ser Ser Gly Ile Asp Ser Val Val Met Asp Asp Phe Met Gln Gly
            20                  25                  30
Leu Ser Asp Val Leu Glu Glu Lys Ala Pro Gln Leu Ser Tyr Asp Glu
        35                  40                  45
Ala Lys Arg Glu Ile Glu Ala Tyr Phe Met Asp Leu Gln Gln Lys Ala
    50                  55                  60
Val Lys Leu Asn Lys Glu Ala Gly Glu Glu Phe Leu Lys Ile Asn Ala
65                  70                  75                  80
His Lys Glu Gly Val Thr Thr Leu Pro Ser Gly Leu Gln Tyr Glu Val
                85                  90                  95
Ile Lys Met Gly Glu Gly Pro Lys Pro Thr Leu Ser Asp Thr Val Thr
            100                 105                 110
Cys His Tyr His Gly Thr Leu Ile Asn Gly Ile Val Phe Asp Ser Ser
        115                 120                 125
Met Asp Arg Gly Glu Pro Ala Ser Phe Pro Leu Arg Gly Val Ile Ala
    130                 135                 140
Gly Trp Thr Glu Ile Leu Gln Leu Met Pro Val Gly Ser Lys Trp Lys
145                 150                 155                 160
Val Thr Ile Pro Ser Asp Leu Ala Tyr Gly Asp Arg Gly Ala Gly Glu
                165                 170                 175
His Ile Lys Pro Gly Ser Thr Leu Ile Phe Ile Ile Glu Leu Leu Ser
            180                 185                 190
Ile Asn Lys
        195

(2) INFORMATION FOR SEQ ID NO:388

(i) SEQUENCE CHARACTERISTICS:
(A) LENGTH: 273 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...273

(xi) SEQUENCE DESCRIPTION: SEQ ID NO:388

Met Lys Lys Ala Leu Leu Ile Gly Ala Ala Leu Leu Gly Ala Val Ser
1               5                   10                  15
Phe Ala Ser Ala Gln Ser Leu Ser Thr Ile Lys Val Gln Asn Asn Ser
            20                  25                  30
Val Gln Gln Pro Arg Glu Glu Ala Thr Ile Gln Val Cys Gly Glu Leu
        35                  40                  45
Ala Glu Gln Val Asp Cys Ile Gly Thr Gly Asn Ser Ala Ile Ile Ala
    50                  55                  60
Ala Ala Ala Lys Phe Glu Ser Asp Asp Leu Glu Ser Tyr Val Gly Trp
65                  70                  75                  80
Glu Ile Met Ser Val Asp Phe Phe Pro Gly Tyr Lys Ala Cys Lys Tyr
                85                  90                  95
Thr Ser Ala Val Trp Ala Asp Asp Met Thr Ile Leu Gly Gln Ser Glu
            100                 105                 110
Asp Ser Asp Pro Glu Met Gln Thr Ile Asn Asn Leu Ala Leu Lys Thr
        115                 120                 125
Ser Val Lys Ile Glu Ala Gly Lys Asn Tyr Ile Val Gly Tyr Ile Ala
    130                 135                 140
Asn Thr Ala Gly Gly His Pro Ile Gly Cys Asp Gln Gly Pro Ala Val
145                 150                 155                 160
Asp Gly Tyr Gly Asp Leu Val Ser Ile Ser Glu Asp Gly Gly Ala Thr
                165                 170                 175
Phe Pro Pro Phe Glu Ser Leu His Gln Ala Val Pro Thr Leu Asn Tyr
            180                 185                 190
Asn Ile Tyr Val Val Val His Leu Lys Lys Gly Glu Gly Val Glu Ala
        195                 200                 205
Val Leu Thr Asn Asp Lys Ala Asn Ala Tyr Val Gln Asn Gly Val Ile
    210                 215                 220
Tyr Val Ala Gly Ala Asn Gly Arg Gln Val Ser Leu Phe Asp Met Asn
225                 230                 235                 240
Gly Lys Val Val Tyr Thr Gly Val Ser Glu Thr Ile Ala Ala Pro Gln
                245                 250                 255
Lys Gly Met Tyr Ile Leu Arg Val Gly Ala Lys Ser Ile Lys Leu Ala
            260                 265                 270
Ile


(2) INFORMATION FOR SEQ ID NO:389

    (i) SEQUENCE CHARACTERISTICS:
        (A) LENGTH: 554 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...554

    (xi) SEQUENCE DESCRIPTION: SEQ ID NO:389

Met Pro Arg Ile Met Lys Leu Lys Ile Ala Leu Arg Leu Leu Leu Ala
1               5                   10                  15
Thr Phe Ala Ile Val Leu Phe Ser Pro Leu Ala Lys Ala Gln Met Asp
            20                  25                  30
Ile Gly Gly Asp Asp Val Leu Ile Glu Thr Met Ser Thr Leu Ser Gly
        35                  40                  45
Tyr Ser Glu Asp Phe Tyr Tyr Lys Met Ala Val Ala Asp Asn Gly Trp
    50                  55                  60
Ile Tyr Val Met Leu Asp Phe Ser Arg Ile Tyr Phe Asp Asp Val Arg
65                  70                  75                  80
Leu Tyr Arg Ser Lys Asp Gly Gly Ala Thr Tyr Gln Lys Leu Gly Ser
                85                  90                  95
Leu Gly Ser Leu Val Pro Tyr Asp Phe Asp Val Ser His Cys Asp Phe
            100                 105                 110
Ile Val Thr Gly Lys Asp Glu Asp Asp Ile Asn Val Trp Thr Val Met

115                 120                 125
Thr Ala Phe Glu Tyr Val Gly Gly Thr Ile Gly Asn Gly Val Leu Leu
    130                 135                 140
Met His Arg His Asp Ala Asp Ile Asn Asn Thr Glu Cys Val Tyr Lys
145                 150                 155                 160
Lys Asp Phe Pro Asn Asn Arg Leu Met Gly Val Ala Ile Ala Ser Asn
                165                 170                 175
Tyr Arg Ala Pro Ser Pro Tyr Gly Leu Gly Gly Asp Pro Phe Ala Leu
            180                 185                 190
Ala Val Ala Val Ser Gly Ser Gly Ser Asp His Ser Phe Leu Asp Tyr
        195                 200                 205
Ile Phe Ser Leu Asp Gly Gly Val His Phe Glu Gln Lys Arg Ile Tyr
    210                 215                 220
Thr Arg Pro Gln Lys Leu Thr Ile Asn Arg Val Asp Leu Ser Leu Gly
225                 230                 235                 240
Ser Thr Ser Pro Ser Leu Gly Phe Asn Thr Trp Pro Leu Met Gly Val
                245                 250                 255
Val Phe Glu Met Asn Lys Asn Leu Asp Gly Phe Asp Ile Gly Phe Ile
            260                 265                 270
Ser Asn Phe Val Asp Tyr Asp Pro Arg Tyr Ala Trp Ser Glu Pro Ile
        275                 280                 285
Ile Ile Glu Glu Asp Cys Gly Trp Thr Asp Phe Asn Pro Leu Gly Ala
    290                 295                 300
Leu Ser Ile Glu Ile Gln Met Met Leu Asp Asp Asn Ser Asp Asn Thr
305                 310                 315                 320
Val Gly Gly Glu Arg Ser His Asn Phe Leu Ile Thr Tyr Pro Gly His
                325                 330                 335
Tyr Val Tyr Pro Lys Gln Ser Phe Asn Tyr Ser Pro Gly His Thr Pro
            340                 345                 350
Thr Lys Lys Asp Leu Val Phe Lys His Cys Ile Gly Ile Pro Ala Leu
        355                 360                 365
Ala Tyr Asp Lys Glu Gly Asp Arg Tyr Leu Thr Thr Phe Gln Asp His
    370                 375                 380
Asn Leu Met Arg Tyr Arg Trp Ile Lys Tyr Asp Asp Ile Asn Ser Phe
385                 390                 395                 400
Tyr Gly Trp Ser Trp Pro Tyr Val Tyr Ala Lys Glu Ala Lys Asp Lys
                405                 410                 415
Lys Arg Arg Arg Pro Gln Val Ala Leu Asn Pro Thr Asn Gly Lys Ala
            420                 425                 430
Cys Trp Val Trp His Thr Arg Lys Ser Pro Tyr Asp Glu Thr Lys Pro
        435                 440                 445
His Pro Thr Pro Val Ile Ile Lys His Phe Leu Trp Ser Asp Thr Glu
    450                 455                 460
Trp Val His Ala Leu Asp Val Gly Asp Val Leu Gln Lys Glu Gly Ser
465                 470                 475                 480
Met Lys Leu Tyr Pro Asn Pro Ala Lys Glu Tyr Val Leu Ile Asn Leu
                485                 490                 495
Pro Lys Glu Gly Gly His Glu Ala Val Val Tyr Asp Met Gln Gly Arg
            500                 505                 510
Ile Val Glu Lys Val Ser Phe Ser Gly Lys Glu Tyr Lys Leu Asn Val
        515                 520                 525
Gln Tyr Leu Ser Lys Gly Thr Tyr Met Leu Lys Val Val Ala Asp Thr
    530                 535                 540
Glu Tyr Phe Val Glu Lys Ile Ile Val Glu
545                 550

(2) INFORMATION FOR SEQ ID NO:390

    (i) SEQUENCE CHARACTERISTICS:
        (A) LENGTH: 550 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...550

    (xi) SEQUENCE DESCRIPTION: SEQ ID NO:390

Met Lys Leu Lys Ile Ala Leu Arg Leu Leu Leu Ala Thr Phe Ala Ile
1               5                   10                  15
Val Leu Phe Ser Pro Leu Ala Lys Ala Gln Met Asp Ile Gly Gly Asp
            20                  25                  30
Asp Val Leu Ile Glu Thr Met Ser Thr Leu Ser Gly Tyr Ser Glu Asp
        35                  40                  45
Phe Tyr Tyr Lys Met Ala Val Ala Asp Asn Gly Trp Ile Tyr Val Met
    50                  55                  60
Leu Asp Phe Ser Arg Ile Tyr Phe Asp Asp Val Arg Leu Tyr Arg Ser
65                  70                  75                  80
Lys Asp Gly Gly Ala Thr Tyr Gln Lys Leu Gly Ser Leu Gly Ser Leu
                85                  90                  95
Val Pro Tyr Asp Phe Asp Val Ser His Cys Asp Phe Ile Val Thr Gly
            100                 105                 110
Lys Asp Glu Asp Asp Ile Asn Val Trp Thr Val Met Thr Ala Phe Glu
        115                 120                 125
Tyr Val Gly Gly Thr Ile Gly Asn Gly Val Leu Leu Met His Arg His
    130                 135                 140
Asp Ala Asp Ile Asn Asn Thr Glu Cys Val Tyr Lys Lys Asp Phe Pro
145                 150                 155                 160
Asn Asn Arg Leu Met Gly Val Ala Ile Ala Ser Asn Tyr Arg Ala Pro
                165                 170                 175
Ser Pro Tyr Gly Leu Gly Gly Asp Pro Phe Ala Leu Ala Val Ala Val
            180                 185                 190
Ser Gly Ser Gly Ser Asp His Ser Phe Leu Asp Tyr Ile Phe Ser Leu
        195                 200                 205
Asp Gly Gly Val His Phe Glu Gln Lys Arg Ile Tyr Thr Arg Pro Gln
    210                 215                 220
Lys Leu Thr Ile Asn Arg Val Asp Leu Ser Leu Gly Ser Thr Ser Pro
225                 230                 235                 240
Ser Leu Gly Phe Asn Thr Trp Pro Leu Met Gly Val Val Phe Glu Met
                245                 250                 255
Asn Lys Asn Leu Asp Gly Phe Asp Ile Gly Phe Ile Ser Asn Phe Val
            260                 265                 270
Asp Tyr Asp Pro Arg Tyr Ala Trp Ser Glu Pro Ile Ile Ile Glu Glu
        275                 280                 285
Asp Cys Gly Trp Thr Asp Phe Asn Pro Leu Gly Ala Leu Ser Ile Glu
    290                 295                 300
Ile Gln Met Met Leu Asp Asp Asn Ser Asp Asn Thr Val Gly Gly Glu
305                 310                 315                 320
Arg Ser His Asn Phe Leu Ile Thr Tyr Pro Gly His Tyr Val Tyr Pro
                325                 330                 335
Lys Gln Ser Phe Asn Tyr Ser Pro Gly His Thr Pro Thr Lys Lys Asp
            340                 345                 350
Leu Val Phe Lys His Cys Ile Gly Ile Pro Ala Leu Ala Tyr Asp Lys
        355                 360                 365
Glu Gly Asp Arg Tyr Leu Thr Thr Phe Gln Asp His Asn Leu Met Arg
    370                 375                 380
Tyr Arg Trp Ile Lys Tyr Asp Asp Ile Asn Ser Phe Tyr Gly Trp Ser
385                 390                 395                 400
Trp Pro Tyr Val Tyr Ala Lys Glu Ala Lys Asp Lys Lys Arg Arg Arg
                405                 410                 415
Pro Gln Val Ala Leu Asn Pro Thr Asn Gly Lys Ala Cys Trp Val Trp
            420                 425                 430
His Thr Arg Lys Ser Pro Tyr Asp Glu Thr Lys Pro His Pro Thr Pro
        435                 440                 445
Val Ile Ile Lys His Phe Leu Trp Ser Asp Thr Glu Trp Val His Ala
    450                 455                 460
Leu Asp Val Gly Asp Val Leu Gln Lys Glu Gly Ser Met Lys Leu Tyr
465                 470                 475                 480
Pro Asn Pro Ala Lys Glu Tyr Val Leu Ile Asn Leu Pro Lys Glu Gly
                485                 490                 495
Gly His Glu Ala Val Val Tyr Asp Met Gln Gly Arg Ile Val Glu Lys
            500                 505                 510
Val Ser Phe Ser Gly Lys Glu Tyr Lys Leu Asn Val Gln Tyr Leu Ser
        515                 520                 525
Lys Gly Thr Tyr Met Leu Lys Val Val Ala Asp Thr Glu Tyr Phe Val
    530                 535                 540
Glu Lys Ile Ile Val Glu
545                 550

(2) INFORMATION FOR SEQ ID NO:391

(i) SEQUENCE CHARACTERISTICS:
(A) LENGTH: 390 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...390

(xi) SEQUENCE DESCRIPTION: SEQ ID NO:391

Met Lys Arg Leu Leu Pro Phe Leu Leu Leu Ala Gly Leu Val Ala Val
1               5                   10                  15
Gly Asn Val Ser Ala Gln Ser Pro Arg Ile Pro Gln Val Asp Val His
            20                  25                  30
Thr Arg Ile Ala Arg Asn Ala Arg Tyr Arg Leu Asp Lys Ile Ser Val
        35                  40                  45
Pro Asp Ser Arg Gln Ile Phe Asp Tyr Phe Tyr Lys Glu Glu Thr Ile
    50                  55                  60
Pro Thr Lys Ile Gln Thr Thr Thr Gly Gly Ala Ile Thr Ser Ile Asp
65                  70                  75                  80
Ser Leu Phe Tyr Glu Asp Asp Arg Leu Val Gln Val Arg Tyr Phe Asp
                85                  90                  95
Asn Asn Leu Glu Leu Lys Gln Ala Glu Lys Tyr Val Tyr Asp Gly Ser
            100                 105                 110
Lys Leu Val Leu Arg Glu Ile Arg Lys Ser Pro Thr Asp Glu Thr Pro
        115                 120                 125
Ile Lys Lys Val Ser Tyr His Tyr Leu Cys Gly Ser Asp Met Pro Phe
    130                 135                 140
Glu Ile Thr Thr Glu Met Ser Asp Gly Tyr Phe Glu Ser His Thr Leu
145                 150                 155                 160
Asn Tyr Leu Asn Gly Lys Ile Ala Arg Ile Asp Ile Met Thr Gln Gln
                165                 170                 175
Asn Pro Ser Ala Glu Leu Ile Glu Thr Gly Arg Met Val Tyr Glu Phe
            180                 185                 190
Asp Ala Asn Asn Asp Ala Val Leu Leu Arg Asp Ser Val Phe Leu Pro
        195                 200                 205
Leu Gln Asn Lys Trp Val Glu Met Phe Thr His Arg Tyr Thr Tyr Asp
    210                 215                 220
Asn Lys His Asn Cys Ile Arg Trp Glu Gln Asp Glu Phe Gly Thr Leu
225                 230                 235                 240
Thr Leu Ala Asn Asn Phe Glu Tyr Asp Thr Thr Ile Pro Leu Ser Ser
                245                 250                 255
Val Leu Phe Pro Thr His Glu Glu Phe Phe Arg Pro Leu Leu Pro Asn
            260                 265                 270
Phe Met Lys His Met Arg Thr Lys Gln Thr Tyr Phe Asn Asn Ser Gly
        275                 280                 285
Glu Gly Leu Ser Glu Val Cys Asp Tyr Asn Tyr Phe Tyr Thr Asp Met
    290                 295                 300
Gln Gly Asn Ala Leu Thr Asp Val Ala Val Asn Glu Ser Ile Lys Ile
305                 310                 315                 320
Tyr Pro Arg Pro Ala Thr Asp Phe Leu Arg Ile Glu Gly Ser Gln Leu
                325                 330                 335
Leu Arg Leu Ser Leu Phe Asp Met Asn Gly Lys Leu Ile Arg Ala Thr
            340                 345                 350
Glu Leu Thr Gly Asp Leu Ala Ile Ile Gly Val Ala Ser Leu Pro Arg
        355                 360                 365
Gly Thr Tyr Ile Ala Glu Ile Thr Ala Ala Asn Ser Lys Thr Ile Arg
    370                 375                 380
Ala Lys Val Ser Leu Arg
385                 390

(2) INFORMATION FOR SEQ ID NO:392

(i) SEQUENCE CHARACTERISTICS:
(A) LENGTH: 411 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...411

(xi) SEQUENCE DESCRIPTION: SEQ ID NO:392

Met Arg Gln His Leu Ser Leu Phe Pro Phe Ile Leu Phe Leu Leu Leu
1               5                   10                  15
Ala Phe Ser Tyr Val Gly Cys Arg Thr Val Arg Gln Thr Pro Lys Gln
            20                  25                  30
Ser Glu Arg Tyr Val Val Val Leu Ser Leu Asp Gly Phe Arg Pro Asp
        35                  40                  45
Tyr Thr Asp Arg Ala Arg Thr Pro Ala Leu Asp Arg Met Ala Gln Glu
    50                  55                  60
Gly Leu Ser Gly Ser Leu Gln Pro Cys Phe Pro Ser Leu Thr Phe Pro
65                  70                  75                  80
Asn His Tyr Ser Met Ala Thr Gly Leu Tyr Pro Asp His His Gly Ile
                85                  90                  95
Val Ala Asn Glu Phe Val Asp Ser Leu Leu Gly Ile Phe Arg Ile Ser
            100                 105                 110
Asp Arg Lys Ala Val Glu Thr Pro Gly Phe Trp Gly Gly Glu Pro Val
        115                 120                 125
Trp Asn Thr Ala Ala Arg Gln Gly Ile Arg Thr Gly Val Tyr Phe Trp
    130                 135                 140
Val Gly Ser Glu Thr Ala Val Asn Gly Asn Arg Pro Trp Arg Trp Lys
145                 150                 155                 160
Lys Phe Ser Ser Thr Val Pro Phe Arg Asp Arg Ala Asp Ser Val Ile
                165                 170                 175
Ala Trp Leu Gly Leu Pro Glu Lys Glu Arg Pro Arg Leu Leu Met Trp
            180                 185                 190
Tyr Ile Glu Glu Pro Asp Met Ile Gly His Ser Gln Thr Pro Glu Ser
        195                 200                 205
Pro Leu Thr Leu Ala Met Val Glu Arg Leu Asp Ser Val Val Gly Tyr
    210                 215                 220
Phe Arg Lys Arg Leu Asp Ser Leu Pro Ile Ala Ala Gln Thr Asp Phe
225                 230                 235                 240
Ile Ile Val Ser Asp His Gly Met Ala Thr Tyr Glu Asn Glu Lys Cys
                245                 250                 255
Val Asn Leu Ser His Tyr Leu Pro Ala Asp Ser Phe Leu Tyr Met Ala
            260                 265                 270
Thr Gly Ala Phe Thr His Leu Tyr Pro Lys Pro Ser Tyr Thr Glu Arg
        275                 280                 285
Ala Tyr Glu Ile Leu Arg Ala Ile Pro His Ile Ser Val Tyr Arg Lys
    290                 295                 300
Gly Glu Val Pro Lys Arg Leu Arg Cys Gly Thr Asn Pro Arg Leu Gly
305                 310                 315                 320
Glu Leu Val Val Ile Pro Asp Ile Gly Ser Thr Val Phe Phe Ala Ile
                325                 330                 335
Asn Glu Asp Val Arg Pro Gly Ala Ala His Gly Tyr Asp Asn Gln Ala
            340                 345                 350
Pro Glu Met Arg Ala Leu Leu Arg Ala Val Gly Pro Asp Phe Arg Pro
        355                 360                 365
Gly Ser Arg Val Glu Asn Leu Pro Asn Ile Thr Ile Tyr Pro Leu Ile
    370                 375                 380
Cys Arg Leu Leu Gly Ile Glu Pro Ala Pro Asn Asp Ala Asp Glu Thr
385                 390                 395                 400
Leu Leu Asn Gly Leu Ile Arg Asp Lys Arg Pro
                405                 410

(2) INFORMATION FOR SEQ ID NO:393

(i) SEQUENCE CHARACTERISTICS:
(A) LENGTH: 246 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...246

(xi) SEQUENCE DESCRIPTION: SEQ ID NO:393

Met Lys Val Gly Leu Phe Ile Pro Cys Tyr Val Asn Ala Val Tyr Pro
1               5                   10                  15
Glu Val Gly Ile Ala Thr Tyr Lys Leu Leu Lys Ser Leu Asp Ile Asp
            20                  25                  30
Val Asp Tyr Pro Met Asp Gln Thr Cys Cys Gly Gln Pro Met Ala Asn
        35                  40                  45
Ala Gly Phe Glu Gln Lys Ala Gln Lys Leu Ala Leu Arg Phe Glu Glu
    50                  55                  60
Leu Phe Glu Ser Tyr Asp Val Val Val Gly Pro Ser Ala Ser Cys Val
65                  70                  75                  80
Ala Phe Val Lys Glu Asn Tyr Asp His Ile Leu Arg Pro Thr Gly His
                85                  90                  95
Val Cys Lys Ser Ala Ala Lys Val Arg Asp Ile Cys Glu Phe Leu His
            100                 105                 110
Asp Asp Leu Lys Ile Thr Ser Leu Pro Ser Arg Phe Ala His Lys Val
        115                 120                 125
Ser Leu His Asn Ser Cys His Gly Val Arg Glu Leu His Leu Ser Thr
    130                 135                 140
Pro Ser Glu Val His Arg Pro Tyr His Asn Lys Val Arg Arg Leu Leu
145                 150                 155                 160
Glu Met Val Gln Gly Ile Glu Val Phe Glu Pro Lys Arg Ile Asp Glu
                165                 170                 175
Cys Cys Gly Phe Gly Gly Met Tyr Ser Val Glu Glu Pro Glu Val Ser
            180                 185                 190
Thr Cys Met Gly His Asp Lys Val Leu Asp His Ile Ser Thr Gly Ala
        195                 200                 205
Glu Tyr Ile Thr Gly Pro Asp Ser Ser Cys Leu Met His Met Gln Gly
    210                 215                 220
Val Ile Asp Arg Glu Lys Leu Pro Ile Lys Thr Ile His Ala Val Glu
225                 230                 235                 240
Ile Leu Ala Ala Asn Leu
                245

(2) INFORMATION FOR SEQ ID NO:394

(i) SEQUENCE CHARACTERISTICS:
(A) LENGTH: 246 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...246

(xi) SEQUENCE DESCRIPTION: SEQ ID NO:394

Met Asp Ile Val Ser Met Ala Asp Lys Ala Leu Val Val Glu Met Arg
1               5                   10                  15
Asp Val Thr Leu Cys Gln Glu Glu Asn Val Ile Phe Gln Asn Leu Asn
            20                  25                  30
Leu Thr Leu Ser Ala Gly Asp Phe Val Tyr Leu Ile Gly Ser Val Gly
        35                  40                  45
Ser Gly Lys Ser Thr Leu Leu Lys Ala Leu Tyr Ala Glu Val Pro Ile
    50                  55                  60
Ser Ala Gly Tyr Ala Arg Val Ile Asp Tyr Asp Leu Ala Lys Leu Lys
65                  70                  75                  80

Arg Lys Gln Leu Pro Tyr Leu Arg Arg Asn Leu Gly Ile Val Phe Gln
                85                  90                  95
Asp Phe Gln Leu Leu Asn Gly Arg Thr Val Ala Glu Asn Leu Asp Phe
            100                 105                 110
Val Leu Arg Ala Thr Asp Trp Lys Asn Arg Ala Asp Arg Glu Gln Arg
        115                 120                 125
Ile Glu Glu Val Leu Thr Arg Val Gly Met Ser Arg Lys Ala Tyr Lys
    130                 135                 140
Arg Pro His Glu Leu Ser Gly Gly Glu Gln Gln Arg Val Gly Ile Ala
145                 150                 155                 160
Arg Ala Leu Leu Ala Lys Pro Ala Leu Ile Leu Ala Asp Glu Pro Thr
                165                 170                 175
Gly Asn Leu Asp Ser Val Thr Gly Leu Gln Ile Ala Ser Leu Leu Tyr
            180                 185                 190
Glu Ile Ser Lys Gln Gly Thr Ala Val Leu Met Ser Thr His Asn Ser
        195                 200                 205
Ser Leu Leu Ser His Leu Pro Ala Arg Thr Leu Ala Val Arg Lys Asn
    210                 215                 220
Gly Asp Ala Ser Ser Leu Val Glu Leu Ser Ala Asp Ala Val Ser Arg
225                 230                 235                 240
Lys Asn Thr Glu Ile Asp

(2) INFORMATION FOR SEQ ID NO:395

     (i) SEQUENCE CHARACTERISTICS:
          (A) LENGTH: 241 amino acids
          (B) TYPE: amino acid
          (D) TOPOLOGY: linear

    (ii) MOLECULE TYPE: protein

   (iii) HYPOTHETICAL: YES

    (vi) ORIGINAL SOURCE:
          (A) ORGANISM: Porphyromonas gingivalis

    (ix) FEATURE:
          (A) NAME/KEY: misc_feature
          (B) LOCATION 1...241

    (xi) SEQUENCE DESCRIPTION: SEQ ID NO:395

Met Ala Asp Lys Ala Leu Val Val Glu Met Arg Asp Val Thr Leu Cys
1               5                   10                  15
Gln Glu Glu Asn Val Ile Phe Gln Asn Leu Asn Leu Thr Leu Ser Ala
            20                  25                  30
Gly Asp Phe Val Tyr Leu Ile Gly Ser Val Gly Ser Gly Lys Ser Thr
        35                  40                  45
Leu Leu Lys Ala Leu Tyr Ala Glu Val Pro Ile Ser Ala Gly Tyr Ala
    50                  55                  60
Arg Val Ile Asp Tyr Asp Leu Ala Lys Leu Lys Arg Lys Gln Leu Pro
65                  70                  75                  80
Tyr Leu Arg Arg Asn Leu Gly Ile Val Phe Gln Asp Phe Gln Leu Leu
                85                  90                  95
Asn Gly Arg Thr Val Ala Glu Asn Leu Asp Phe Val Leu Arg Ala Thr
            100                 105                 110
Asp Trp Lys Asn Arg Ala Asp Arg Glu Gln Arg Ile Glu Glu Val Leu
        115                 120                 125
Thr Arg Val Gly Met Ser Arg Lys Ala Tyr Lys Arg Pro His Glu Leu
    130                 135                 140
Ser Gly Gly Glu Gln Gln Arg Val Gly Ile Ala Arg Ala Leu Leu Ala
145                 150                 155                 160
Lys Pro Ala Leu Ile Leu Ala Asp Glu Pro Thr Gly Asn Leu Asp Ser
                165                 170                 175
Val Thr Gly Leu Gln Ile Ala Ser Leu Leu Tyr Glu Ile Ser Lys Gln
            180                 185                 190
Gly Thr Ala Val Leu Met Ser Thr His Asn Ser Ser Leu Leu Ser His
        195                 200                 205
Leu Pro Ala Arg Thr Leu Ala Val Arg Lys Asn Gly Asp Ala Ser Ser
    210                 215                 220
Leu Val Glu Leu Ser Ala Asp Ala Val Ser Arg Lys Asn Thr Glu Ile
225                 230                 235
Asp

(2) INFORMATION FOR SEQ ID NO:396

(i) SEQUENCE CHARACTERISTICS:
(A) LENGTH: 232 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...232

(xi) SEQUENCE DESCRIPTION: SEQ ID NO:396

Met Arg Asp Val Thr Leu Cys Gln Glu Glu Asn Val Ile Phe Gln Asn
1               5                   10                  15
Leu Asn Leu Thr Leu Ser Ala Gly Asp Phe Val Tyr Leu Ile Gly Ser
            20                  25                  30
Val Gly Ser Gly Lys Ser Thr Leu Leu Lys Ala Leu Tyr Ala Glu Val
        35                  40                  45
Pro Ile Ser Ala Gly Tyr Ala Arg Val Ile Asp Tyr Asp Leu Ala Lys
    50                  55                  60
Leu Lys Arg Lys Gln Leu Pro Tyr Leu Arg Arg Asn Leu Gly Ile Val
65                  70                  75                  80
Phe Gln Asp Phe Gln Leu Leu Asn Gly Arg Thr Val Ala Glu Asn Leu
                85                  90                  95
Asp Phe Val Leu Arg Ala Thr Asp Trp Lys Asn Arg Ala Asp Arg Glu
            100                 105                 110
Gln Arg Ile Glu Glu Val Leu Thr Arg Val Gly Met Ser Arg Lys Ala
        115                 120                 125
Tyr Lys Arg Pro His Glu Leu Ser Gly Gly Glu Gln Gln Arg Val Gly
    130                 135                 140
Ile Ala Arg Ala Leu Leu Ala Lys Pro Ala Leu Ile Leu Ala Asp Glu
145                 150                 155                 160
Pro Thr Gly Asn Leu Asp Ser Val Thr Gly Leu Gln Ile Ala Ser Leu
                165                 170                 175
Leu Tyr Glu Ile Ser Lys Gln Gly Thr Ala Val Leu Met Ser Thr His
            180                 185                 190
Asn Ser Ser Leu Leu Ser His Leu Pro Ala Arg Thr Leu Ala Val Arg
        195                 200                 205
Lys Asn Gly Asp Ala Ser Ser Leu Val Glu Leu Ser Ala Asp Ala Val
    210                 215                 220
Ser Arg Lys Asn Thr Glu Ile Asp
225                 230

(2) INFORMATION FOR SEQ ID NO:397

(i) SEQUENCE CHARACTERISTICS:
(A) LENGTH: 219 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...219

(xi) SEQUENCE DESCRIPTION: SEQ ID NO:397

Met Ile Glu Ile Ser Asn Leu Thr Lys Val Phe Arg Thr Glu Glu Ile
1               5                   10                  15
Glu Thr Val Ala Leu Asp Gly Val Ser Leu Lys Val Asp Lys Gly Glu

                    20                  25                  30
Phe Ile Ala Ile Met Gly Pro Ser Gly Cys Gly Lys Ser Thr Leu Leu
        35                  40                  45
Asn Ile Leu Gly Leu Leu Asp Asn Pro Thr Ser Gly Ile Tyr Lys Leu
    50                  55                  60
Asp Gly Ala Glu Val Gly Asn Leu Arg Glu Lys Asp Arg Thr Ala Val
65                  70                  75                  80
Arg Lys Gly Asn Ile Gly Phe Val Phe Gln Ser Phe Asn Leu Ile Glu
                85                  90                  95
Glu Met Thr Val Ser Glu Asn Val Glu Leu Pro Leu Val Tyr Leu Gly
            100                 105                 110
Val Lys Ala Ser Glu Arg Lys Glu Arg Val Glu Glu Ala Leu Arg Lys
        115                 120                 125
Met Ser Ile Ser His Arg Ala Gly His Phe Pro Asn Gln Leu Ser Gly
    130                 135                 140
Gly Gln Gln Gln Arg Val Ala Ile Ala Arg Ala Val Val Ala Asn Pro
145                 150                 155                 160
Lys Leu Ile Leu Ala Asp Glu Pro Thr Gly Asn Leu Asp Ser Lys Asn
                165                 170                 175
Gly Ala Asp Val Met Glu Leu Leu Arg Gly Leu Asn Arg Glu Gly Ala
            180                 185                 190
Thr Ile Val Met Val Thr His Ser Glu His Asp Ala Arg Ser Ala Gly
        195                 200                 205
Arg Ile Ile Asn Leu Phe Asp Gly Lys Ile Arg
    210                 215

(2) INFORMATION FOR SEQ ID NO:398

    (i) SEQUENCE CHARACTERISTICS:
        (A) LENGTH: 595 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...595

    (xi) SEQUENCE DESCRIPTION: SEQ ID NO:398

Met Lys Glu Phe Phe Lys Met Phe Phe Ala Ser Ile Leu Gly Val Ile
1               5                   10                  15
Thr Ala Gly Ile Ile Leu Phe Cys Ile Phe Leu Phe Ile Phe Phe Gly
            20                  25                  30
Ile Val Ala Gly Ile Ala Ser Lys Ala Thr Gly Gly Thr Ile Pro Lys
        35                  40                  45
Ile Glu Ala Asn Ser Ile Leu His Ile Unk Asn Ser Ser Phe Pro Glu
    50                  55                  60
Ile Val Ser Ala Asn Pro Trp Ser Met Leu Thr Gly Lys Asp Glu Ser
65                  70                  75                  80
Val Ser Leu Ser Gln Ala Val Glu Ala Ile Gly Gln Ala Lys Asn Asn
                85                  90                  95
Pro Asn Ile Thr Gly Ile Phe Leu Asp Leu Asp Asn Leu Ser Val Gly
            100                 105                 110
Met Ala Ser Ala Glu Glu Leu Arg Arg Ala Leu Gln Asp Phe Lys Met
        115                 120                 125
Ser Gly Lys Phe Val Val Ser Tyr Ala Asp Arg Tyr Thr Gln Lys Gly
    130                 135                 140
Tyr Tyr Leu Ser Ser Ile Ala Asp Lys Leu Tyr Leu Asn Pro Lys Gly
145                 150                 155                 160
Met Leu Gly Leu Ile Gly Ile Ala Thr Gln Thr Met Phe Tyr Lys Asp
                165                 170                 175
Ala Leu Asp Lys Phe Gly Val Lys Met Glu Ile Phe Lys Val Gly Thr
            180                 185                 190
Tyr Lys Ala Ala Val Glu Pro Phe Met Leu Asn Arg Met Ser Asp Ala
        195                 200                 205
Asn Arg Glu Gln Ile Thr Thr Tyr Ile Asn Gly Leu Trp Asp Lys Ile
    210                 215                 220
Thr Ser Asp Ile Ala Glu Ser Arg Lys Thr Ala Met Asp Ser Val Lys

225                 230                 235                 240
Met Phe Ala Asp Lys Gly Glu Met Phe Gly Leu Ala Glu Lys Ala Val
                245                 250                 255
Glu Met Lys Leu Val Asp Glu Leu Ala Tyr Arg Thr Asp Val Glu Lys
            260                 265                 270
Glu Leu Lys Lys Met Ser Gln Arg Gly Glu Lys Asp Glu Leu Arg Phe
        275                 280                 285
Val Ser Leu Ser Gln Val Leu Ala Asn Gly Pro Met Asn Lys Thr Lys
    290                 295                 300
Gly Ser Arg Ile Ala Val Leu Phe Ala Glu Gly Glu Ile Thr Glu Glu
305                 310                 315                 320
Ile Ile Lys Lys Pro Phe Asp Thr Asp Gly Ser Ser Ile Thr Gln Glu
                325                 330                 335
Leu Ala Lys Glu Ile Lys Ala Ala Ala Asp Asp Asp Asp Ile Lys Ala
            340                 345                 350
Val Val Leu Arg Val Asn Ser Pro Gly Gly Ser Ala Phe Thr Ser Glu
        355                 360                 365
Gln Ile Trp Lys Gln Val Ala Asp Leu Lys Ala Lys Lys Pro Ile Val
    370                 375                 380
Val Ser Met Gly Asp Val Ala Ala Ser Gly Gly Tyr Tyr Ile Ala Cys
385                 390                 395                 400
Ala Ala Asn Ser Ile Val Ala Glu His Thr Thr Leu Thr Gly Ser Ile
                405                 410                 415
Gly Ile Phe Gly Met Phe Pro Asn Phe Ala Gly Val Ala Lys Lys Ile
            420                 425                 430
Gly Val Asn Met Asp Val Val Gln Thr Ser Lys Tyr Ala Asp Leu Gly
        435                 440                 445
Asn Thr Phe Ala Pro Met Thr Val Glu Asp Arg Ala Leu Ile Gln Arg
    450                 455                 460
Tyr Ile Glu Gln Gly Tyr Asp Leu Phe Leu Thr Arg Val Ser Glu Gly
465                 470                 475                 480
Arg Asn Arg Thr Lys Ala Gln Ile Asp Ser Ile Ala Gln Gly Arg Val
                485                 490                 495
Trp Leu Gly Asp Lys Ala Leu Ala Leu Gly Leu Val Asp Glu Leu Gly
            500                 505                 510
Gly Leu Asp Thr Ala Ile Lys Arg Ala Ala Lys Leu Ala Gln Leu Gly
        515                 520                 525
Gly Asn Tyr Ser Ile Glu Tyr Gly Lys Thr Lys Arg Asn Phe Phe Glu
    530                 535                 540
Glu Leu Leu Ser Ser Ser Ala Ala Asp Met Lys Ser Ala Ile Leu Ser
545                 550                 555                 560
Thr Ile Leu Ser Asp Pro Glu Ile Glu Val Leu Arg Glu Leu Arg Ser
                565                 570                 575
Met Pro Pro Arg Pro Ser Gly Ile Gln Ala Arg Leu Pro Tyr Tyr Phe
            580                 585                 590
Met Pro Tyr
        595

(2) INFORMATION FOR SEQ ID NO:399

(i) SEQUENCE CHARACTERISTICS:
(A) LENGTH: 589 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...589

(xi) SEQUENCE DESCRIPTION: SEQ ID NO:399

Met Phe Phe Ala Ser Ile Leu Gly Val Ile Thr Ala Gly Ile Ile Leu
1               5                   10                  15
Phe Cys Ile Phe Leu Phe Ile Phe Phe Gly Ile Val Ala Gly Ile Ala
            20                  25                  30
Ser Lys Ala Thr Gly Gly Thr Ile Pro Lys Ile Glu Ala Asn Ser Ile
        35                  40                  45
Leu His Ile Unk Asn Ser Ser Phe Pro Glu Ile Val Ser Ala Asn Pro

50                  55                  60
Trp Ser Met Leu Thr Gly Lys Asp Glu Ser Val Ser Leu Ser Gln Ala
65                  70                  75                  80
Val Glu Ala Ile Gly Gln Ala Lys Asn Asn Pro Asn Ile Thr Gly Ile
                85                  90                  95
Phe Leu Asp Leu Asp Asn Leu Ser Val Gly Met Ala Ser Ala Glu Glu
            100                 105                 110
Leu Arg Arg Ala Leu Gln Asp Phe Lys Met Ser Gly Lys Phe Val Val
        115                 120                 125
Ser Tyr Ala Asp Arg Tyr Thr Gln Lys Gly Tyr Tyr Leu Ser Ser Ile
    130                 135                 140
Ala Asp Lys Leu Tyr Leu Asn Pro Lys Gly Met Leu Gly Leu Ile Gly
145                 150                 155                 160
Ile Ala Thr Gln Thr Met Phe Tyr Lys Asp Ala Leu Asp Lys Phe Gly
                165                 170                 175
Val Lys Met Glu Ile Phe Lys Val Gly Thr Tyr Lys Ala Ala Val Glu
            180                 185                 190
Pro Phe Met Leu Asn Arg Met Ser Asp Ala Asn Arg Glu Gln Ile Thr
        195                 200                 205
Thr Tyr Ile Asn Gly Leu Trp Asp Lys Ile Thr Ser Asp Ile Ala Glu
    210                 215                 220
Ser Arg Lys Thr Ala Met Asp Ser Val Lys Met Phe Ala Asp Lys Gly
225                 230                 235                 240
Glu Met Phe Gly Leu Ala Glu Lys Ala Val Glu Met Lys Leu Val Asp
                245                 250                 255
Glu Leu Ala Tyr Arg Thr Asp Val Glu Lys Glu Leu Lys Lys Met Ser
            260                 265                 270
Gln Arg Gly Glu Lys Asp Glu Leu Arg Phe Val Ser Leu Ser Gln Val
        275                 280                 285
Leu Ala Asn Gly Pro Met Asn Lys Thr Lys Gly Ser Arg Ile Ala Val
    290                 295                 300
Leu Phe Ala Glu Gly Glu Ile Thr Glu Glu Ile Ile Lys Lys Pro Phe
305                 310                 315                 320
Asp Thr Asp Gly Ser Ser Ile Thr Gln Glu Leu Ala Lys Glu Ile Lys
                325                 330                 335
Ala Ala Ala Asp Asp Asp Ile Lys Ala Val Val Leu Arg Val Asn
            340                 345                 350
Ser Pro Gly Gly Ser Ala Phe Thr Ser Glu Gln Ile Trp Lys Gln Val
        355                 360                 365
Ala Asp Leu Lys Ala Lys Lys Pro Ile Val Val Ser Met Gly Asp Val
    370                 375                 380
Ala Ala Ser Gly Gly Tyr Tyr Ile Ala Cys Ala Ala Asn Ser Ile Val
385                 390                 395                 400
Ala Glu His Thr Thr Leu Thr Gly Ser Ile Gly Ile Phe Gly Met Phe
                405                 410                 415
Pro Asn Phe Ala Gly Val Ala Lys Lys Ile Gly Val Asn Met Asp Val
            420                 425                 430
Val Gln Thr Ser Lys Tyr Ala Asp Leu Gly Asn Thr Phe Ala Pro Met
        435                 440                 445
Thr Val Glu Asp Arg Ala Leu Ile Gln Arg Tyr Ile Glu Gln Gly Tyr
    450                 455                 460
Asp Leu Phe Leu Thr Arg Val Ser Glu Gly Arg Asn Arg Thr Lys Ala
465                 470                 475                 480
Gln Ile Asp Ser Ile Ala Gln Gly Arg Val Trp Leu Gly Asp Lys Ala
                485                 490                 495
Leu Ala Leu Gly Leu Val Asp Glu Leu Gly Gly Leu Asp Thr Ala Ile
            500                 505                 510
Lys Arg Ala Ala Lys Leu Ala Gln Leu Gly Gly Asn Tyr Ser Ile Glu
        515                 520                 525
Tyr Gly Lys Thr Lys Arg Asn Phe Phe Glu Glu Leu Leu Ser Ser Ser
    530                 535                 540
Ala Ala Asp Met Lys Ser Ala Ile Leu Ser Thr Ile Leu Ser Asp Pro
545                 550                 555                 560
Glu Ile Glu Val Leu Arg Glu Leu Arg Ser Met Pro Pro Arg Pro Ser
                565                 570                 575
Gly Ile Gln Ala Arg Leu Pro Tyr Tyr Phe Met Pro Tyr
            580                 585

(2) INFORMATION FOR SEQ ID NO:400

     (i) SEQUENCE CHARACTERISTICS:
          (A) LENGTH: 313 amino acids
          (B) TYPE: amino acid
          (D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...313

(xi) SEQUENCE DESCRIPTION: SEQ ID NO:400

Met Arg Ala Asn Ile Trp Gln Ile Leu Ser Val Ser Val Leu Phe Phe
1               5                   10                  15
Phe Gly Thr Ala Ile Gly Gln Ala Gln Ser Arg Asn Arg Thr Tyr Glu
            20                  25                  30
Ala Tyr Val Lys Gln Tyr Ala Asp Glu Ala Ile Arg Gln Met Ser Arg
        35                  40                  45
Tyr Asn Ile Pro Ala Ser Ile Thr Ile Ala Gln Ala Leu Val Glu Thr
    50                  55                  60
Gly Ala Gly Ala Ser Thr Leu Ala Ser Val His Asn Asn His Phe Gly
65                  70                  75                  80
Ile Lys Cys His Lys Ser Trp Thr Gly Lys Arg Thr Tyr Arg Thr Asp
                85                  90                  95
Asp Ala Pro Asn Glu Cys Phe Arg Ser Tyr Ser Ala Ala Arg Glu Ser
            100                 105                 110
Tyr Glu Asp His Ser Arg Phe Leu Leu Gln Pro Arg Tyr Arg Pro Leu
        115                 120                 125
Phe Lys Leu Asp Arg Glu Asp Tyr Arg Gly Trp Ala Thr Gly Leu Gln
    130                 135                 140
Arg Cys Gly Tyr Ala Thr Asn Arg Gly Tyr Ala Asn Leu Leu Ile Lys
145                 150                 155                 160
Met Val Glu Leu Tyr Glu Leu Tyr Ala Leu Asp Arg Glu Lys Tyr Pro
                165                 170                 175
Ser Trp Phe His Lys Ser Tyr Pro Gly Ser Asn Lys Lys Ser His Gln
            180                 185                 190
Thr Thr Lys Gln Lys Gln Ser Gly Leu Lys His Glu Ala Tyr Phe Ser
        195                 200                 205
Tyr Gly Leu Leu Tyr Ile Ile Ala Lys Gln Gly Asp Thr Phe Asp Ser
    210                 215                 220
Leu Ala Glu Glu Phe Asp Met Arg Ala Ser Lys Leu Ala Lys Tyr Asn
225                 230                 235                 240
Asp Ala Pro Val Asp Phe Pro Ile Glu Lys Gly Asp Val Ile Tyr Leu
                245                 250                 255
Glu Lys Lys His Ala Cys Ser Ile Ser Lys His Thr Gln His Val Val
            260                 265                 270
Arg Val Gly Asp Ser Met His Ser Ile Ser Gln Arg Tyr Gly Ile Arg
        275                 280                 285
Met Lys Asn Leu Tyr Lys Leu Asn Asp Lys Asp Gly Glu Tyr Ile Pro
    290                 295                 300
Gln Glu Gly Asp Ile Leu Arg Leu Arg
305                 310

(2) INFORMATION FOR SEQ ID NO:401

(i) SEQUENCE CHARACTERISTICS:
(A) LENGTH: 523 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...523

(xi) SEQUENCE DESCRIPTION: SEQ ID NO:401

Met Asp Gly Arg Arg Tyr Ser Asp Gly Leu His Gln Ala Ile Glu Ala

1                   5                   10                  15
Lys Glu His Val Lys Val Glu Ala Ala Thr Gln Thr Phe Ala Thr Ile
                20                  25                  30
Thr Leu Gln Asn Tyr Phe Arg Met Tyr His Lys Leu Ala Gly Met Thr
            35                  40                  45
Gly Thr Ala Glu Thr Glu Ala Gly Glu Leu Trp Asp Ile Tyr Lys Leu
    50                  55                  60
Asp Val Val Val Ile Pro Thr Asn Lys Pro Ile Ala Arg Lys Asp Met
65                  70                  75                  80
Asn Asp Arg Ile Tyr Lys Thr Ala Arg Glu Lys Tyr Ala Ala Val Ile
                85                  90                  95
Glu Glu Ile Val Arg Leu Val Glu Glu Gly Arg Pro Val Leu Val Gly
            100                 105                 110
Thr Thr Ser Val Glu Ile Ser Glu Leu Leu Ser Arg Met Leu Arg Leu
        115                 120                 125
Arg Gly Ile Gln His Asn Val Leu Asn Ala Lys Leu His Gln Lys Glu
    130                 135                 140
Ala Glu Ile Val Ala Gln Ala Gly Gln Lys Gly Thr Val Thr Ile Ala
145                 150                 155                 160
Thr Asn Met Ala Gly Arg Gly Thr Asp Ile Lys Leu Ser Ala Glu Val
                165                 170                 175
Lys Lys Ala Gly Gly Leu Ala Ile Ile Gly Thr Glu Arg His Glu Ser
            180                 185                 190
Arg Arg Val Asp Arg Gln Leu Arg Gly Arg Ser Gly Arg Gln Gly Asp
        195                 200                 205
Pro Gly Ser Ser Ile Phe Tyr Val Ser Leu Glu Asp His Leu Met Arg
    210                 215                 220
Leu Phe Ala Thr Glu Lys Ile Ala Ser Leu Met Asp Arg Leu Gly Phe
225                 230                 235                 240
Lys Glu Gly Glu Val Leu Glu Asn Asn Met Leu Ser Lys Ser Val Glu
                245                 250                 255
Arg Ala Gln Lys Lys Val Glu Glu Asn Asn Phe Gly Ile Arg Lys His
            260                 265                 270
Leu Leu Glu Tyr Asp Asp Val Met Asn Ser Gln Arg Glu Val Ile Tyr
        275                 280                 285
Thr Arg Arg Arg His Ala Leu Met Gly Glu Arg Ile Gly Met Asp Val
    290                 295                 300
Leu Asn Thr Ile Tyr Asp Val Cys Lys Ala Leu Ile Asp Asn Tyr Ala
305                 310                 315                 320
Glu Ala Asn Asp Phe Glu Gly Phe Lys Glu Asp Leu Met Arg Ala Leu
                325                 330                 335
Ala Ile Glu Ser Pro Ile Thr Gln Glu Ile Phe Arg Gly Lys Lys Ala
            340                 345                 350
Glu Glu Leu Thr Asp Met Leu Phe Asp Glu Ala Tyr Lys Ser Phe Gln
        355                 360                 365
Arg Lys Met Asp Leu Ile Ala Glu Val Ala His Pro Val Val His Gln
    370                 375                 380
Val Phe Glu Thr Gln Ala Ala Val Tyr Glu Arg Ile Leu Ile Pro Ile
385                 390                 395                 400
Thr Asp Gly Lys Arg Val Tyr Asn Ile Gly Cys Asn Leu Arg Glu Ala
                405                 410                 415
Asp Glu Thr Gln Gly Lys Ser Ile Ile Lys Glu Phe Glu Lys Ala Ile
            420                 425                 430
Val Leu His Thr Ile Asp Glu Ser Trp Lys Glu His Leu Arg Glu Met
        435                 440                 445
Asp Glu Leu Arg Asn Ser Val Gln Asn Ala Ser Tyr Glu Asn Lys Asp
    450                 455                 460
Pro Leu Leu Ile Tyr Lys Leu Glu Ser Tyr Glu Leu Phe Arg Lys Met
465                 470                 475                 480
Val Glu Ala Met Asn Arg Lys Thr Val Ala Ile Leu Met Arg Ala Arg
                485                 490                 495
Ile Pro Val Pro Glu Ala Pro Ser Gln Glu Glu Leu Glu His Arg Arg
            500                 505                 510
Gln Ile Glu Ile Arg His Ala Thr Gln Gln Arg
        515                 520

(2) INFORMATION FOR SEQ ID NO:402

    (i) SEQUENCE CHARACTERISTICS:
        (A) LENGTH: 375 amino acids
        (B) TYPE: amino acid
        (D) TOPOLOGY: linear

    (ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...375

(xi) SEQUENCE DESCRIPTION: SEQ ID NO:402

Met Asn Phe Leu Lys Lys Glu Pro Phe Lys Ile Phe Ser Met Ile Tyr
1               5                   10                  15
Leu Leu Leu Asp Thr Ile Thr Asn Arg Ala Gly Thr Glu Arg Ala Val
            20                  25                  30
Ile Asn Leu Ala Asn Asn Leu His Ala Asn Gly His Arg Val Ser Leu
        35                  40                  45
Val Ser Val Cys Thr Lys Glu Gly Glu Pro Ser Phe Gln Val Glu Lys
    50                  55                  60
Gly Ile Glu Val His His Leu Gly Ile Arg Leu Tyr Gly Asn Ala Leu
65                  70                  75                  80
Ala Arg Lys Thr Val Tyr Phe Lys Ala Tyr Arg Arg Ile Lys Ala Leu
                85                  90                  95
Tyr Lys Lys Arg Glu Pro Val Leu Leu Ile Gly Thr Asn Ile Phe Ile
            100                 105                 110
Asn Thr Ile Leu Ser Gln Ile Ser Asn Arg Gly Arg Ile Phe Thr Ile
        115                 120                 125
Gly Cys Glu His Ile Ser Tyr Asp Ile Ala Arg Pro Ile Thr Lys Arg
    130                 135                 140
Ile Arg Gly Phe Leu Tyr Ser Gly Leu Asp Ala Val Val Ala Leu Thr
145                 150                 155                 160
Lys Arg Asp Gln Gln Ser Phe Glu Ala Ile Leu Arg Gly Arg Ser Lys
                165                 170                 175
Ala Tyr Val Ile Pro Asn Gln Val Ser Phe Thr Thr Val Gln Arg Asp
            180                 185                 190
Ala Thr Thr His Lys Gln Met Leu Ala Ile Gly Arg Leu Thr Tyr Gln
        195                 200                 205
Lys Gly Phe Glu Phe Met Ile Glu Asp Ala Ser Arg Val Leu Arg Glu
    210                 215                 220
Arg Pro Asp Trp Lys Leu Ile Ile Val Gly Asp Gly Glu Asn Glu Ser
225                 230                 235                 240
Met Leu Arg Lys Glu Ile Ala Ser Arg Asn Met Glu Ser Gln Ile Glu
                245                 250                 255
Ile His Pro Ser Thr Pro Glu Ile Arg Lys Tyr Tyr Glu Ser Ser Ala
            260                 265                 270
Ile Tyr Leu Met Thr Ser Arg Phe Glu Gly Leu Pro Met Val Leu Leu
        275                 280                 285
Glu Ala Glu Ala Tyr Ala Leu Pro Ile Ile Ser Tyr Asp Cys Pro Thr
    290                 295                 300
Gly Pro Arg Glu Leu Ile Glu Asn Gly Arg Asn Gly Phe Leu Val Pro
305                 310                 315                 320
Met Glu Ala His Glu Asp Phe Ala Asp Lys Leu Arg Leu Leu Met Asp
                325                 330                 335
Asp Glu Thr Leu Arg Lys Lys Met Gly Gln Glu Ser Glu Leu Met Val
            340                 345                 350
Lys Ser Tyr Ser Pro Ala Asn Ile Tyr Glu Cys Trp Lys Lys Leu Phe
        355                 360                 365
Val Glu Ile Gly Tyr Met Asn
    370                 375

(2) INFORMATION FOR SEQ ID NO:403

(i) SEQUENCE CHARACTERISTICS:
(A) LENGTH: 362 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:

(A) NAME/KEY: misc_feature
(B) LOCATION 1...362

(xi) SEQUENCE DESCRIPTION: SEQ ID NO:403

Met Ile Tyr Leu Leu Leu Asp Thr Ile Thr Asn Arg Ala Gly Thr Glu
1               5                   10                  15
Arg Ala Val Ile Asn Leu Ala Asn Asn Leu His Ala Asn Gly His Arg
            20                  25                  30
Val Ser Leu Val Ser Val Cys Thr Lys Glu Gly Glu Pro Ser Phe Gln
        35                  40                  45
Val Glu Lys Gly Ile Glu Val His His Leu Gly Ile Arg Leu Tyr Gly
    50                  55                  60
Asn Ala Leu Ala Arg Lys Thr Val Tyr Phe Lys Ala Tyr Arg Arg Ile
65                  70                  75                  80
Lys Ala Leu Tyr Lys Lys Arg Glu Pro Val Leu Leu Ile Gly Thr Asn
                85                  90                  95
Ile Phe Ile Asn Thr Ile Leu Ser Gln Ile Ser Asn Arg Gly Arg Ile
            100                 105                 110
Phe Thr Ile Gly Cys Glu His Ile Ser Tyr Asp Ile Ala Arg Pro Ile
        115                 120                 125
Thr Lys Arg Ile Arg Gly Phe Leu Tyr Ser Gly Leu Asp Ala Val Val
    130                 135                 140
Ala Leu Thr Lys Arg Asp Gln Gln Ser Phe Glu Ala Ile Leu Arg Gly
145                 150                 155                 160
Arg Ser Lys Ala Tyr Val Ile Pro Asn Gln Val Ser Phe Thr Thr Val
                165                 170                 175
Gln Arg Asp Ala Thr Thr His Lys Gln Met Leu Ala Ile Gly Arg Leu
            180                 185                 190
Thr Tyr Gln Lys Gly Phe Glu Phe Met Ile Glu Asp Ala Ser Arg Val
        195                 200                 205
Leu Arg Glu Arg Pro Asp Trp Lys Leu Ile Ile Val Gly Asp Gly Glu
    210                 215                 220
Asn Glu Ser Met Leu Arg Lys Glu Ile Ala Ser Arg Asn Met Glu Ser
225                 230                 235                 240
Gln Ile Glu Ile His Pro Ser Thr Pro Glu Ile Arg Lys Tyr Tyr Glu
                245                 250                 255
Ser Ser Ala Ile Tyr Leu Met Thr Ser Arg Phe Glu Gly Leu Pro Met
            260                 265                 270
Val Leu Leu Glu Ala Glu Ala Tyr Ala Leu Pro Ile Ile Ser Tyr Asp
        275                 280                 285
Cys Pro Thr Gly Pro Arg Glu Leu Ile Glu Asn Gly Arg Asn Gly Phe
    290                 295                 300
Leu Val Pro Met Glu Ala His Glu Asp Phe Ala Asp Lys Leu Arg Leu
305                 310                 315                 320
Leu Met Asp Asp Glu Thr Leu Arg Lys Lys Met Gly Gln Glu Ser Glu
                325                 330                 335
Leu Met Val Lys Ser Tyr Ser Pro Ala Asn Ile Tyr Glu Cys Trp Lys
            340                 345                 350
Lys Leu Phe Val Glu Ile Gly Tyr Met Asn
        355                 360

(2) INFORMATION FOR SEQ ID NO:404

(i) SEQUENCE CHARACTERISTICS:
(A) LENGTH: 640 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...640

(xi) SEQUENCE DESCRIPTION: SEQ ID NO:404

Met Gly Lys Ile Ile Gly Ile Asp Leu Gly Thr Thr Asn Ser Cys Val
1               5                   10                  15
Ser Val Leu Glu Gly Asn Glu Pro Ile Val Ile Thr Asn Ser Glu Gly

                    20                  25                  30
Lys Arg Thr Thr Pro Ser Val Val Ala Phe Val Asp Gly Gly Glu Arg
        35                  40                  45
Lys Val Gly Asp Pro Ala Lys Arg Gln Ala Ile Thr Asn Pro Thr Lys
    50                  55                  60
Thr Ile Tyr Ser Ile Lys Arg Phe Met Gly Glu Thr Tyr Asp Gln Val
65                  70                  75                  80
Ser Arg Glu Val Glu Arg Val Pro Phe Lys Val Val Arg Gly Asp Asn
                85                  90                  95
Asn Thr Pro Arg Val Asp Ile Asp Gly Arg Leu Tyr Thr Pro Gln Glu
            100                 105                 110
Ile Ser Ala Met Ile Leu Gln Lys Met Lys Lys Thr Ala Glu Asp Tyr
        115                 120                 125
Leu Gly Gln Glu Val Thr Glu Ala Val Ile Thr Val Pro Ala Tyr Phe
    130                 135                 140
Asn Asp Ala Gln Arg Gln Ala Thr Lys Glu Ala Gly Glu Ile Ala Gly
145                 150                 155                 160
Leu Lys Val Arg Arg Ile Val Asn Glu Pro Thr Ala Ala Ser Leu Ala
                165                 170                 175
Tyr Gly Leu Asp Lys Ser Asn Lys Asp Met Lys Ile Ala Val Phe Asp
            180                 185                 190
Leu Gly Gly Gly Thr Phe Asp Ile Ser Ile Leu Glu Leu Gly Asp Gly
        195                 200                 205
Val Phe Glu Val Lys Ser Thr Asn Gly Asp Thr His Leu Gly Gly Asp
    210                 215                 220
Asp Phe Asp His Val Ile Ile Asp Trp Leu Ala Glu Glu Phe Lys Ser
225                 230                 235                 240
Gln Glu Gly Val Asp Leu Arg Gln Asp Pro Met Ala Met Gln Arg Leu
                245                 250                 255
Lys Glu Ala Ala Glu Lys Ala Lys Ile Glu Leu Ser Ser Thr Ser Ser
            260                 265                 270
Thr Glu Ile Asn Leu Pro Tyr Ile Met Pro Val Asn Gly Ile Pro Lys
        275                 280                 285
His Leu Val Met Thr Leu Thr Arg Ala Lys Phe Glu Gln Leu Ala Asp
    290                 295                 300
Arg Leu Ile Gln Ala Cys Val Ala Pro Cys Glu Thr Ala Leu Lys Asp
305                 310                 315                 320
Ala Gly Met Ser Arg Gly Asp Ile Asp Glu Val Ile Leu Val Gly Gly
                325                 330                 335
Ser Thr Arg Ile Pro Ala Ile Gln Glu Ile Val Glu Lys Ile Phe Gly
            340                 345                 350
Lys Ala Pro Ser Lys Gly Val Asn Pro Asp Glu Val Val Ala Val Gly
        355                 360                 365
Ala Ala Ile Gln Gly Gly Val Leu Thr Gly Glu Val Lys Asp Val Leu
    370                 375                 380
Leu Leu Asp Val Thr Pro Leu Ser Leu Gly Ile Glu Thr Met Gly Gly
385                 390                 395                 400
Val Met Thr Arg Leu Ile Asp Ala Asn Thr Thr Ile Pro Thr Lys Lys
                405                 410                 415
Ser Glu Ile Phe Thr Thr Ala Val Asp Asn Gln Pro Ser Val Glu Ile
            420                 425                 430
His Val Leu Gln Gly Glu Arg Ser Leu Ala Lys Asp Asn Lys Ser Ile
        435                 440                 445
Gly Arg Phe Asn Leu Asp Gly Ile Ala Pro Ala Pro Arg Gln Thr Pro
    450                 455                 460
Gln Ile Glu Val Thr Phe Asp Ile Asp Ala Asn Gly Ile Leu Asn Val
465                 470                 475                 480
Thr Ala His Asp Lys Ala Thr Gly Lys Lys Gln Asn Ile Arg Ile Glu
                485                 490                 495
Ala Ser Ser Gly Leu Ser Asp Asp Glu Ile Lys Arg Met Lys Glu Glu
            500                 505                 510
Ala Gln Ala Asn Ala Glu Ala Asp Lys Lys Glu Lys Glu Arg Ile Asp
        515                 520                 525
Lys Ile Asn Gln Ala Asp Ser Met Ile Phe Gln Thr Glu Lys Gln Leu
    530                 535                 540
Lys Glu Leu Gly Asp Lys Phe Pro Ala Asp Lys Lys Ala Pro Ile Asp
545                 550                 555                 560
Thr Ala Leu Asp Lys Leu Lys Glu Ala His Lys Ala Gln Asp Val Ala
                565                 570                 575
Ala Ile Asp Thr Ala Met Ala Glu Leu Gln Thr Ala Leu Ser Ala Ala
            580                 585                 590
Gly Glu Glu Leu Tyr Lys Asn Ala Gly Ala Ala Gln Gly Gly Ala Gln
        595                 600                 605
Pro Gly Pro Asp Phe Gly Gly Ala Gln Gly Pro Ser Ala Gly Asp Gln
    610                 615                 620

Pro Ser Asp Asp Lys Asn Val Thr Asp Val Asp Phe Glu Glu Val Lys
625                 630                 635                 640

(2) INFORMATION FOR SEQ ID NO:405

     (i) SEQUENCE CHARACTERISTICS:
          (A) LENGTH: 449 amino acids
          (B) TYPE: amino acid
          (D) TOPOLOGY: linear

    (ii) MOLECULE TYPE: protein

   (iii) HYPOTHETICAL: YES

    (vi) ORIGINAL SOURCE:
          (A) ORGANISM: Porphyromonas gingivalis

    (ix) FEATURE:
          (A) NAME/KEY: misc_feature
          (B) LOCATION 1...449

    (xi) SEQUENCE DESCRIPTION: SEQ ID NO:405

Met Arg Tyr Asp Leu Ala Ile Ile Gly Gly Gly Pro Ala Gly Tyr Thr
1               5                   10                  15
Ala Ala Glu Arg Ala Ala Lys Gly Gly Leu Lys Thr Leu Leu Ile Glu
            20                  25                  30
Lys Asn Ala Leu Gly Gly Val Cys Leu Asn Glu Gly Cys Ile Pro Thr
        35                  40                  45
Lys Thr Leu Leu Tyr Ser Ala Lys Val Leu His Gln Ile Ala Thr Ala
    50                  55                  60
Ser Lys Tyr Ala Val Ser Gly Thr Ala Asp Gly Leu Asp Leu Gly Lys
65                  70                  75                  80
Val Ile Ala Arg Lys Gly Lys Ile Ile Arg Lys Leu Thr Ala Gly Ile
                85                  90                  95
Arg Ser Arg Leu Thr Glu Ala Gly Val Glu Met Val Thr Ala Glu Ala
            100                 105                 110
Thr Val Thr Gly Cys Asp Ala Asp Gly Ile Ile Gly Ile Thr Ala Gly
        115                 120                 125
Glu Ala Gln Tyr Lys Ala Ala Asn Leu Leu Leu Cys Thr Gly Ser Glu
    130                 135                 140
Thr Phe Ile Pro Pro Ile Pro Gly Val Glu Gln Thr Glu Tyr Trp Thr
145                 150                 155                 160
Asn Arg Glu Ala Leu Gln Asn Lys Glu Ile Pro Thr Ser Leu Val Ile
                165                 170                 175
Ile Gly Gly Gly Val Ile Gly Met Glu Phe Ala Ser Phe Phe Asn Gly
            180                 185                 190
Ile Gly Thr Gln Val His Val Val Glu Met Leu Pro Glu Ile Leu Asn
        195                 200                 205
Gly Ile Asp Pro Glu His Ala Ala Met Leu Arg Ala His Tyr Glu Lys
    210                 215                 220
Glu Gly Ile Lys Phe Tyr Leu Gly His Lys Val Thr Ser Val Arg Asn
225                 230                 235                 240
Gly Ala Val Thr Val Glu Tyr Glu Gly Glu Ser Lys Glu Ile Glu Gly
                245                 250                 255
Glu Arg Ile Leu Met Ser Val Gly Arg Arg Pro Val Leu Gln Gly Phe
            260                 265                 270
Glu Ser Leu Gly Leu Val Leu Ala Gly Lys Gly Val Lys Thr Asn Glu
        275                 280                 285
Arg Met Gln Thr Ser Leu Pro Asn Val Tyr Ala Ala Gly Asp Ile Thr
    290                 295                 300
Gly Phe Ser Leu Leu Ala His Thr Ala Val Arg Glu Ala Glu Val Ala
305                 310                 315                 320
Val Asp Gln Ile Leu Gly Lys Thr Asp Glu Thr Met Ser Tyr Arg Ala
                325                 330                 335
Val Pro Gly Val Val Tyr Thr Asn Pro Glu Val Ala Gly Val Gly Glu
            340                 345                 350
Thr Glu Glu Ser Leu Arg Lys Ala Gly Arg Ala Tyr Thr Val Arg Arg
        355                 360                 365
Leu Pro Met Ala Phe Ser Gly Arg Phe Val Ala Glu Asn Glu Gln Gly
    370                 375                 380
Asn Gly Glu Cys Lys Leu Leu Leu Asp Glu Glu Asn Arg Leu Ile Gly
385                 390                 395                 400
Ala His Leu Ile Gly Asn Pro Ala Gly Glu Leu Ile Val Thr Ala Ala
                405                 410                 415

Met Ala Ile Glu Thr Gly Met Thr Asp Arg Gln Ile Glu Arg Ile Ile
            420                 425                 430
Phe Pro His Pro Thr Val Gly Glu Ile Leu Lys Glu Thr Leu Ala Gly
        435                 440                 445
Gly

(2) INFORMATION FOR SEQ ID NO:406

     (i) SEQUENCE CHARACTERISTICS:
          (A) LENGTH: 941 amino acids
          (B) TYPE: amino acid
          (D) TOPOLOGY: linear

     (ii) MOLECULE TYPE: protein

     (iii) HYPOTHETICAL: YES

     (vi) ORIGINAL SOURCE:
          (A) ORGANISM: Porphyromonas gingivalis

     (ix) FEATURE:
          (A) NAME/KEY: misc_feature
          (B) LOCATION 1...941

     (xi) SEQUENCE DESCRIPTION: SEQ ID NO:406

Met Glu Leu Lys Arg Phe Leu Ser Leu Gly Leu Leu Leu Val Gly Phe
1               5                   10                  15
Ile Pro Met Lys Leu Ser Ala Gln Gln Ala Gln Pro Leu Pro Thr Asp
            20                  25                  30
Pro Ala Val Arg Val Gly Lys Leu Asp Asn Gly Leu Thr Tyr Phe Ile
        35                  40                  45
Arg His Asn Glu Asn Pro Lys Asp Arg Ala Asp Phe Phe Ile Ala Gln
    50                  55                  60
Lys Val Gly Ser Ile Leu Glu Glu Asp Ser Gln Ser Gly Leu Ala His
65                  70                  75                  80
Phe Leu Glu His Met Ala Phe Asn Gly Thr Lys Asn Phe Pro Gly Lys
                85                  90                  95
Asn Leu Ile Asn Tyr Leu Glu Thr Ile Gly Val Arg Phe Gly Gln Asn
            100                 105                 110
Leu Asn Ala Ser Thr Gly Phe Asp Lys Thr Glu Tyr Thr Ile Met Asp
        115                 120                 125
Val Pro Thr Thr Arg Gln Gly Ile Ile Asp Ser Cys Leu Leu Ile Leu
    130                 135                 140
His Asp Trp Ser Asn Asn Ile Thr Leu Asp Gly His Glu Ile Asp Glu
145                 150                 155                 160
Glu Arg Gly Val Ile Gln Glu Glu Trp Arg Ala Arg Arg Asp Ala Asn
                165                 170                 175
Leu Arg Met Phe Glu Ala Ile Leu Ala Lys Ala Met Pro Gly Asn Lys
            180                 185                 190
Tyr Ala Glu Arg Met Pro Ile Gly Leu Met Asp Val Val Leu Asn Phe
        195                 200                 205
Lys His Asp Glu Leu Arg Asn Tyr Tyr Lys Lys Trp Tyr Arg Pro Asp
    210                 215                 220
Leu Gln Gly Leu Val Ile Val Gly Asp Ile Asp Val Asp Tyr Val Glu
225                 230                 235                 240
Asn Lys Ile Lys Glu Leu Phe Lys Asp Val Pro Ala Pro Val Asn Pro
                245                 250                 255
Ala Glu Arg Ile Tyr Thr Pro Val Glu Asp Asn Asp Glu Pro Ile Val
            260                 265                 270
Ala Ile Ala Thr Asp Ala Glu Ala Thr Thr Thr Gln Leu Ser Ile Ser
        275                 280                 285
Phe Lys Ser Asp Pro Thr Pro Gln Glu Val Arg Gly Ser Ile Phe Gly
    290                 295                 300
Leu Val Glu Asp Tyr Met Lys Gln Val Ile Thr Thr Ala Val Asn Glu
305                 310                 315                 320
Arg Leu Ser Glu Ile Thr His Lys Pro Asn Ala Pro Phe Leu Ser Ala
                325                 330                 335
Gly Ala Phe Phe Ser Asn Phe Met Tyr Ile Thr Gln Thr Lys Asp Ala
            340                 345                 350
Phe Asn Phe Val Ala Thr Val Arg Glu Gly Glu Ala Glu Lys Ala Met
        355                 360                 365
Asn Ala Leu Val Ala Glu Ile Glu Ser Leu Arg Gln Phe Gly Ile Thr
    370                 375                 380

Lys Gly Glu Tyr Asp Arg Ala Arg Thr Asn Val Leu Lys Arg Tyr Glu
385                 390                 395                 400
Asn Gln Tyr Asn Glu Arg Asp Lys Arg Lys Asn Asn Ala Tyr Ala Asn
                405                 410                 415
Glu Tyr Ser Thr Tyr Phe Thr Asp Gly Gly Tyr Ile Pro Gly Ile Glu
            420                 425                 430
Val Glu Tyr Gln Thr Val Asn Ala Phe Ala Pro Gln Val Pro Leu Glu
        435                 440                 445
Ala Phe Asn Gln Ala Ile Ala Gln Met Ile Asp Pro Val Lys Asn Ala
    450                 455                 460
Val Val Thr Leu Thr Gly Pro Ser Lys Ala Glu Ala Lys Ile Pro Ser
465                 470                 475                 480
Glu Ala Asp Phe Leu Ala Ala Phe Lys Ala Ala Arg Gln Gln Lys Val
                485                 490                 495
Glu Ala Lys Lys Asp Glu Val Ser Asp Gln Lys Leu Met Glu Lys Ala
            500                 505                 510
Pro Lys Ala Gly Lys Ile Val Ser Glu Lys Lys Asp Gln Lys Phe Gly
        515                 520                 525
Thr Thr Glu Leu Thr Leu Ser Asn Gly Ile Lys Val Tyr Leu Lys Lys
    530                 535                 540
Thr Asp Phe Lys Ser Asn Glu Ile Leu Met Ser Ala Leu Ser Pro Gly
545                 550                 555                 560
Gly Ile Leu Ser Gly Lys His Ala Pro Asn Gln Ser Val Met Asn Ser
                565                 570                 575
Phe Met Asn Val Gly Gly Leu Gly Asn Phe Asp Ala Ile Gln Leu Asp
            580                 585                 590
Lys Val Leu Thr Gly Arg Ser Ala Ser Val Ser Pro Ser Leu Ser Leu
        595                 600                 605
Leu Ser Glu Gly Leu Ser Gly Lys Thr Thr Val Glu Asp Met Glu Thr
    610                 615                 620
Phe Phe Gln Leu Ile Tyr Leu Gln Met Thr Ala Asn Arg Lys Asp Pro
625                 630                 635                 640
Glu Ala Phe Lys Ala Thr Gln Glu Lys Leu Tyr Asn Asn Leu Lys Asn
                645                 650                 655
Gln Glu Ala Asn Pro Met Ala Ala Leu Met Asp Ser Ile Arg His Thr
            660                 665                 670
Met Tyr Gly Asp Asn Pro Met Met Lys Pro Met Lys Ala Ala Asp Val
        675                 680                 685
Glu Lys Val Asn Tyr Asp Gln Val Met Ala Phe Tyr Asn Glu Arg Phe
    690                 695                 700
Ala Asp Ala Gly Asp Phe Met Phe Phe Phe Ile Gly Asn Leu Asp Glu
705                 710                 715                 720
Ala Lys Met Lys Pro Leu Ile Glu Thr Tyr Leu Ala Ser Leu Pro Asn
                725                 730                 735
Leu Lys Arg Gly Asp Lys Met Asn Lys Ala Gln Val Pro Ala Ala Arg
            740                 745                 750
Ser Gly Lys Ile Asp Cys Lys Phe Glu Lys Glu Met Asp Thr Pro Ser
        755                 760                 765
Thr Thr Ile Phe Asp Val Val Ser Gly Asn Val Glu Tyr Thr Leu Lys
    770                 775                 780
Asn Ser Leu Leu Leu Glu Val Phe Ser Ala Val Met Asp Gln Val Tyr
785                 790                 795                 800
Thr Ala Thr Val Arg Glu Lys Glu Gly Gly Ala Tyr Ser Val Ala Ala
                805                 810                 815
Phe Gly Gly Leu Glu Gln Tyr Pro Gln Pro Lys Ala Leu Met Gln Ile
            820                 825                 830
Tyr Phe Pro Thr Asp Pro Ala Arg Ala Glu Glu Met Asn Ala Ile Val
        835                 840                 845
Phe Ala Glu Leu Glu Lys Leu Ala Lys Glu Gly Pro Asn Val Glu Tyr
    850                 855                 860
Phe Lys Lys Thr Ile Glu Asn Leu Asn Lys Gln His Lys Glu Ser Leu
865                 870                 875                 880
Arg Glu Asn Arg Phe Trp Leu Glu Ala Met Lys Ala Ser Phe Phe Glu
                885                 890                 895
Gly Asn Asp Phe Ile Thr Asp Tyr Glu Ser Val Leu Asn Gly Leu Thr
            900                 905                 910
Pro Ala Glu Leu Gln Lys Phe Ala Ala Asp Leu Leu Lys Gln Gln Asn
        915                 920                 925
Arg Val Val Val Met Met Ala Pro Val Ala Lys Ala Gln
    930                 935                 940

(2) INFORMATION FOR SEQ ID NO:407

     (i) SEQUENCE CHARACTERISTICS:
          (A) LENGTH: 684 amino acids

(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...684

(xi) SEQUENCE DESCRIPTION: SEQ ID NO:407

Met Ser Lys Lys Gly Thr Ile Gly Val Thr Ser Asp Asn Ile Phe Pro
1               5                   10                  15
Val Ile Lys Lys Phe Leu Tyr Ser Asp His Glu Ile Phe Leu Arg Glu
            20                  25                  30
Ile Val Ser Asn Ala Val Asp Ala Thr Gln Lys Leu Lys Thr Leu Thr
        35                  40                  45
Ser Val Gly Glu Phe Lys Gly Glu Thr Gly Asp Leu Arg Val Thr Val
    50                  55                  60
Ser Val Asp Glu Val Ala Arg Thr Ile Thr Val Ser Asp Arg Gly Val
65                  70                  75                  80
Gly Met Thr Glu Glu Glu Val Glu Lys Tyr Ile Asn Gln Ile Ala Phe
                85                  90                  95
Ser Ser Ala Glu Glu Phe Leu Glu Lys Tyr Lys Asp Asp Lys Ala Ala
            100                 105                 110
Ile Ile Gly His Phe Gly Leu Gly Phe Tyr Ser Ala Phe Met Val Ser
        115                 120                 125
Glu Arg Val Asp Val Ile Thr Arg Ser Phe Arg Glu Asp Ala Thr Ala
    130                 135                 140
Val Lys Trp Ser Cys Asp Gly Ser Pro Glu Tyr Thr Leu Glu Pro Ala
145                 150                 155                 160
Asp Lys Ala Asp Arg Gly Thr Asp Ile Val Met His Ile Asp Glu Glu
                165                 170                 175
Asn Ser Glu Phe Leu Lys Lys Glu Lys Ile Glu Gly Leu Leu Gly Lys
            180                 185                 190
Tyr Cys Lys Phe Leu Thr Val Pro Ile Ile Phe Gly Lys Lys Gln Glu
        195                 200                 205
Trp Lys Asp Gly Lys Met Gln Asp Thr Asp Glu Asp Asn Gln Ile Asn
    210                 215                 220
Asp Thr His Pro Ala Trp Thr Lys Lys Pro Ala Asp Leu Lys Asp Glu
225                 230                 235                 240
Asp Tyr Lys Glu Phe Tyr Arg Ser Leu Tyr Pro Met Ser Glu Glu Pro
                245                 250                 255
Leu Phe Trp Ile His Leu Asn Val Asp Tyr Pro Phe Asn Leu Thr Gly
            260                 265                 270
Ile Leu Tyr Phe Pro Lys Ile Lys Asn Asn Leu Asp Leu Gln Arg Asn
        275                 280                 285
Lys Ile Gln Leu Tyr Cys Asn Gln Val Tyr Val Thr Asp Glu Val Gln
    290                 295                 300
Gly Ile Val Pro Asp Phe Leu Thr Leu Leu His Gly Val Ile Asp Ser
305                 310                 315                 320
Pro Asp Ile Pro Leu Asn Val Ser Arg Ser Tyr Leu Gln Ser Asp Ala
                325                 330                 335
Asn Val Lys Lys Ile Ser Ser His Ile Thr Lys Lys Val Ala Asp Arg
            340                 345                 350
Leu Glu Glu Ile Phe Lys Asn Asp Arg Pro Thr Phe Glu Glu Lys Trp
        355                 360                 365
Asp Ser Leu Lys Leu Phe Val Glu Tyr Gly Met Leu Thr Asp Glu Lys
    370                 375                 380
Phe Tyr Glu Arg Ala Ala Lys Phe Phe Leu Phe Thr Asp Met Asp Gly
385                 390                 395                 400
His Lys Tyr Thr Phe Asp Glu Tyr Arg Thr Leu Val Glu Gly Val Gln
                405                 410                 415
Thr Asp Lys Asp Gly Gln Val Val Tyr Leu Tyr Ala Thr Asp Lys His
            420                 425                 430
Gly Gln Tyr Ser His Val Lys Arg Ala Ser Asp Lys Gly Tyr Ser Val
        435                 440                 445
Met Leu Leu Asp Gly Gln Leu Asp Pro His Ile Val Ser Leu Leu Glu
    450                 455                 460
Gln Lys Leu Glu Lys Thr His Phe Val Arg Val Asp Ser Asp Thr Ile

465                 470                 475                 480
Asn Asn Leu Ile Arg Lys Glu Glu Arg Ala Glu Val Lys Leu Ser Asp
                485                 490                 495
Thr Glu Arg Ala Thr Leu Val Lys Leu Phe Glu Ala Arg Leu Pro Arg
            500                 505                 510
Asp Glu Lys Lys His Phe Asn Val Ala Phe Glu Ser Leu Gly Ala Glu
        515                 520                 525
Gly Glu Ala Ile Leu Ile Thr Gln Ala Glu Phe Met Arg Arg Met Arg
    530                 535                 540
Asp Met Ala Gln Leu Gln Pro Gly Met Ser Phe Tyr Gly Glu Leu Pro
545                 550                 555                 560
Asp Ser Tyr Asn Leu Val Leu Asn Thr Asp His Pro Leu Ile Asp Arg
                565                 570                 575
Val Leu Ser Gly Glu Lys Glu Ser Val Glu Pro Ser Leu Thr Glu Leu
            580                 585                 590
Arg Ala Lys Ile Ala Glu Leu Lys Ala Glu Glu Ala Lys Leu Leu Asp
        595                 600                 605
Glu Glu Lys Gly Lys Lys Pro Glu Glu Ile Pro Val Ala Thr Lys Glu
    610                 615                 620
Ala Lys Glu Asn Asn Ala Val Glu Gln Ala Lys Thr Glu Gly Ser Ile
625                 630                 635                 640
Asn Asp Gln Leu Thr Lys Tyr Ala Gln Asp Asn Glu Leu Ile Gly Gln
                645                 650                 655
Leu Ile Asp Leu Ala Leu Leu Gly Ser Gly Leu Leu Thr Gly Glu Ala
            660                 665                 670
Leu Ala Glu Phe Ile Arg Arg Ser Gln Arg Leu Leu
        675                 680

(2) INFORMATION FOR SEQ ID NO:408

    (i) SEQUENCE CHARACTERISTICS:
        (A) LENGTH: 464 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...464

    (xi) SEQUENCE DESCRIPTION: SEQ ID NO:408

Met Glu Lys Leu Ile Asp Ile Leu Val Val Asp Asp Asp Val Ala Val
1               5                   10                  15
Cys Ala Ala Leu Arg Leu Val Leu Lys Arg Ala Gly Tyr Asn Pro Val
            20                  25                  30
Ile Ala Asn Ser Pro Asp Glu Ala Leu Ser Ile Met Arg Asn Pro Asp
        35                  40                  45
Gly Gly Cys Lys Pro Ala Val Ile Leu Met Asp Met Asn Phe Ser Leu
    50                  55                  60
Ser Thr Ser Gly Arg Glu Gly Leu Glu Leu Leu Glu Lys Met Gln Ile
65                  70                  75                  80
Phe Thr Ser Cys Pro Val Ile Leu Met Thr Ala Trp Ala Ser Ile Pro
                85                  90                  95
Leu Ala Val Glu Gly Met Arg Leu Gly Ala Phe Asp Phe Ile Gly Lys
            100                 105                 110
Pro Trp Asp Asn Asp Arg Leu Leu Arg Thr Ile Asp Thr Ala Leu His
        115                 120                 125
Leu Ala Ala Pro Ser Ala Val Ala Asn Pro Ser Glu Gln Ser Asp Arg
    130                 135                 140
Asp Thr Ala Arg Gln Pro Lys Ala Thr Val Gln Glu Asn Asp Pro Cys
145                 150                 155                 160
Ala His Ile Ile Gly Arg Ser Asp Ala Ile Cys Lys Ile Lys Glu Arg
                165                 170                 175
Ile Arg Arg Ile Ala Pro Thr His Ala Ser Val Leu Ile Thr Gly Glu
            180                 185                 190
Ser Gly Thr Gly Lys Glu Leu Ile Ala Glu Ala Leu His Arg Gly Ser
        195                 200                 205
Lys Arg Ala Ser Ala Pro Phe Val Lys Val Asn Leu Gly Gly Ile Pro

210                 215                 220
Glu Ser Leu Phe Glu Ser Glu Leu Phe Gly His Lys Lys Gly Ala Phe
225                 230                 235                 240
Thr Asn Ala Phe Ser Asp Arg Lys Gly Arg Phe Glu Leu Ala Asp Gly
                245                 250                 255
Gly Thr Ile Phe Leu Asp Glu Ile Gly Glu Leu Pro Val Gly Asn Gln
            260                 265                 270
Val Lys Leu Leu Arg Val Leu Gln Glu Gln Thr Phe Glu Pro Leu Gly
        275                 200                 285
Glu Ser Val Ser His Arg Val Asp Ile Arg Val Val Ser Ala Thr Asn
    290                 295                 300
Ala Ser Leu Glu Arg Met Val Ala Glu Gly Arg Phe Arg Glu Asp Leu
305                 310                 315                 320
Tyr Tyr Arg Ile Asn Leu Ile His Leu His Leu Pro Pro Leu Arg Glu
                325                 330                 335
Arg Gln Glu Asp Ile Gln Leu Leu Val Glu Ala Phe Ser Glu Ala Phe
            340                 345                 350
Ala Gln Ser Asn Gly Leu Pro His Ala Val Trp Ser Ala Glu Ala Met
        355                 360                 365
Arg Arg Ile Cys Ala Met Pro Leu Pro Gly Asn Val Arg Glu Leu Lys
    370                 375                 380
Asn Val Val Glu Arg Thr Leu Leu Leu Ser Gly Ser Arg Glu Ile Ser
385                 390                 395                 400
Ala Arg Asp Val Ala Asp Phe Gly Ser Gln Val Thr Ala Ala Asp His
                405                 410                 415
Ser Asp Glu Arg Ala Leu Thr Asp Met Glu Glu Ala Ala Ile Arg Glu
            420                 425                 430
Thr Leu Thr Lys Tyr Asn Gly Asn Val Ser Arg Ala Ala Arg Ala Leu
        435                 440                 445
Gly Leu Ser Arg Ala Ala Leu Tyr Arg Arg Met Glu Lys Tyr Gly Leu
    450                 455                 460

(2) INFORMATION FOR SEQ ID NO:409

    (i) SEQUENCE CHARACTERISTICS:
        (A) LENGTH: 250 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...250

    (xi) SEQUENCE DESCRIPTION: SEQ ID NO:409

Met Leu Lys Ile Lys Asn Leu His Ala Thr Val Gln Gly Lys Glu Ile
1               5                   10                  15
Leu Lys Gly Ile Asn Leu Glu Ile Asn Ala Gly Glu Ile His Ala Ile
            20                  25                  30
Met Gly Pro Asn Gly Ser Gly Lys Ser Thr Leu Ser Ser Val Leu Val
        35                  40                  45
Gly His Pro Ser Phe Glu Val Thr Glu Gly Glu Val Thr Phe Asn Gly
    50                  55                  60
Ile Asp Leu Leu Glu Leu Glu Pro Glu Glu Arg Ala His Leu Gly Leu
65                  70                  75                  80
Phe Leu Ser Phe Gln Tyr Pro Val Glu Ile Pro Gly Val Ser Met Val
                85                  90                  95
Asn Phe Met Arg Ala Ala Val Asn Glu His Arg Lys Ala Ile Gly Ala
            100                 105                 110
Glu Pro Val Ser Ala Ser Asp Phe Leu Lys Met Met Arg Glu Lys Arg
        115                 120                 125
Ala Ile Val Glu Leu Asp Asn Lys Leu Ala Ser Arg Ser Val Asn Glu
    130                 135                 140
Gly Phe Ser Gly Gly Glu Lys Lys Arg Asn Glu Ile Phe Gln Met Ala
145                 150                 155                 160
Met Leu Glu Pro Lys Leu Ala Ile Leu Asp Glu Thr Asp Ser Gly Leu
                165                 170                 175
Asp Ile Asp Ala Leu Arg Ile Val Ala Gly Gly Val Asn Arg Leu Arg

                180                 185                 190
Ser Pro Glu Asn Ala Ala Ile Val Ile Thr His Tyr Gln Arg Leu Leu
            195                 200                 205
Glu Tyr Ile Lys Pro Asp Phe Val His Val Leu Tyr Lys Gly Arg Ile
    210                 215                 220
Val Lys Ser Gly Gly Ala Glu Leu Ala Leu Thr Leu Glu Glu Lys Gly
225                 230                 235                 240
Tyr Asp Trp Ile Lys Glu Glu Ile Gly Glu
                245                 250

(2) INFORMATION FOR SEQ ID NO:410

     (i) SEQUENCE CHARACTERISTICS:
          (A) LENGTH: 461 amino acids
          (B) TYPE: amino acid
          (D) TOPOLOGY: linear

    (ii) MOLECULE TYPE: protein

   (iii) HYPOTHETICAL: YES

    (vi) ORIGINAL SOURCE:
          (A) ORGANISM: Porphyromonas gingivalis

    (ix) FEATURE:
          (A) NAME/KEY: misc_feature
          (B) LOCATION 1...461

    (xi) SEQUENCE DESCRIPTION: SEQ ID NO:410

Met Ala Lys Glu Lys Thr Ile Tyr Val Cys Arg Ser Cys Gly Thr Lys
1               5                   10                  15
Tyr Ala Lys Trp Gln Gly Asn Cys Asn Ala Cys Gly Glu Trp Asn Cys
            20                  25                  30
Ile Asp Glu Glu Lys Val Pro Ala Pro Ala Ser Gly Lys His Ala Ala
        35                  40                  45
Lys Ser Phe Met Pro Arg Glu Gln Asp Asn Arg Pro Arg Leu Leu Gln
    50                  55                  60
Asp Val Glu Ser Gly Asp Glu Glu Arg Ile Arg Leu Gly Asp Glu Glu
65                  70                  75                  80
Phe Asp Arg Val Leu Gly Gly Gly Ile Val Lys Gly Ala Phe Val Leu
                85                  90                  95
Leu Gly Gly Glu Pro Gly Ile Gly Lys Ser Thr Leu Ile Leu Gln Thr
            100                 105                 110
Val Leu Arg Leu Pro Gln Leu Arg Thr Leu Tyr Val Ser Gly Glu Glu
        115                 120                 125
Ser Ala Arg Gln Leu Lys Met Arg Ala Glu Arg Leu Gly Gln Ala Met
    130                 135                 140
Asn Gly Cys Tyr Val Tyr Cys Glu Thr Asn Ile Glu Arg Ile Leu Ser
145                 150                 155                 160
Arg Ala Glu Glu Leu Thr Pro Asp Leu Leu Val Ile Asp Ser Ile Gln
                165                 170                 175
Thr Val Tyr Thr Glu Glu Met Glu Ser Ser Ala Gly Ser Val Gly Gln
            180                 185                 190
Ile Arg Glu Cys Ala Ala Leu Leu Leu Lys Tyr Cys Lys Thr Thr Gly
        195                 200                 205
Ile Pro Val Ile Val Ile Gly His Ile Thr Lys Glu Gly Ser Ile Ala
    210                 215                 220
Gly Pro Lys Val Leu Glu His Ile Val Asp Thr Val Leu Leu Phe Asp
225                 230                 235                 240
Gly Asp Lys His His Leu Tyr Arg Ile Leu Arg Gly Gln Lys Asn Arg
                245                 250                 255
Tyr Gly Ser Thr Ser Glu Leu Gly Ile Tyr Glu Met Arg Gln Asp Gly
            260                 265                 270
Leu Arg Gly Val Glu Asn Pro Ser Glu His Leu Ile Thr Arg Asn Arg
        275                 280                 285
Glu Asp Leu Ser Gly Ile Ala Ile Ala Val Ala Met Glu Gly Ile Arg
    290                 295                 300
Pro Ile Leu Ile Glu Ala Gln Ala Leu Val Ser Ser Ala Ile Tyr Ala
305                 310                 315                 320
Asn Pro Gln Arg Ser Ala Thr Gly Phe Asp Ile Arg Arg Met Asn Met
                325                 330                 335
Leu Leu Ala Val Leu Glu Lys Arg Ala Gly Phe Lys Leu Ile Gln Lys
            340                 345                 350
Asp Val Phe Leu Asn Ile Ala Gly Gly Ile Lys Ile Ala Asp Pro Ala

355                 360                 365
Thr Asp Leu Ala Val Ile Ser Ala Val Leu Ala Ser Ser Leu Asp Ile
    370                 375                 380
Val Ile Pro Pro Ala Val Cys Met Thr Gly Glu Val Gly Leu Ser Gly
385                 390                 395                 400
Glu Ile Arg Pro Val Ser Arg Ile Glu Gln Arg Ile Thr Glu Ala Arg
                405                 410                 415
Arg Ile Gly Phe Lys Glu Ile Leu Val Pro Ala Asp Asn Phe Arg Gln
            420                 425                 430
Glu Asp Ala Gly Arg Phe Gly Ile Arg Leu Val Pro Val Arg Lys Val
        435                 440                 445
Glu Glu Ala Phe Arg His Leu Phe Ser Lys Gly Arg Glu
    450                 455                 460

(2) INFORMATION FOR SEQ ID NO:411

     (i) SEQUENCE CHARACTERISTICS:
          (A) LENGTH: 271 amino acids
          (B) TYPE: amino acid
          (D) TOPOLOGY: linear

     (ii) MOLECULE TYPE: protein

     (iii) HYPOTHETICAL: YES

     (vi) ORIGINAL SOURCE:
          (A) ORGANISM: Porphyromonas gingivalis

     (ix) FEATURE:
          (A) NAME/KEY: misc_feature
          (B) LOCATION 1...271

     (xi) SEQUENCE DESCRIPTION: SEQ ID NO:411

Met Asn Ser Arg His Leu Thr Ile Thr Ile Ile Ala Gly Leu Ser Leu
1               5                   10                  15
Phe Val Leu Thr Leu Gly Gly Cys Ser Val Ala Gln Gln Asp Thr Gln
            20                  25                  30
Trp Thr Leu Gly Gly Lys Leu Phe Thr Ser Ala Trp Ile Gln Arg Ser
        35                  40                  45
Ala Glu Tyr Gln Ala Leu Cys Ile Gln Ala Tyr Asn Ile Ala Thr Glu
    50                  55                  60
Arg Val Asp Ala Leu Pro Ala Glu Arg Lys Gln Gly Asp Arg Pro Tyr
65                  70                  75                  80
Ala Ile Val Thr Asp Ile Asp Glu Thr Ile Leu Asp Asn Thr Pro Asn
                85                  90                  95
Ser Val Tyr Gln Ala Leu Arg Gly Lys Asp Tyr Asp Glu Glu Thr Trp
            100                 105                 110
Gly Lys Trp Cys Ala Gln Ala Asp Ala Asp Thr Leu Ala Gly Ala Leu
        115                 120                 125
Ser Phe Phe Leu His Ala Ala Asn Lys Gly Ile Glu Val Phe Tyr Val
    130                 135                 140
Thr Asn Arg Arg Asp Asn Leu Arg Glu Ala Thr Leu Gln Asn Leu Gln
145                 150                 155                 160
Arg Tyr Gly Phe Pro Phe Ala Asp Glu Glu His Leu Leu Thr Thr His
                165                 170                 175
Gly Pro Ser Asp Lys Glu Pro Arg Arg Leu Lys Ile Gln Glu Gln Tyr
            180                 185                 190
Glu Ile Val Leu Leu Ile Gly Asp Asn Leu Gly Asp Phe His His Phe
        195                 200                 205
Phe Asn Thr Lys Glu Glu Ser Gly Arg Lys Gln Ala Leu Gly Leu Thr
    210                 215                 220
Ala Gly Glu Phe Gly Arg His Phe Ile Met Leu Pro Asn Pro Asn Tyr
225                 230                 235                 240
Gly Ser Trp Glu Pro Ala Trp Tyr Gly Gly Lys Tyr Pro Pro Leu Pro
                245                 250                 255
Glu Arg Asp Lys Ala Leu Lys Gln Leu His Ser Gln Asn Ser Arg
            260                 265                 270

(2) INFORMATION FOR SEQ ID NO:412

     (i) SEQUENCE CHARACTERISTICS:
          (A) LENGTH: 417 amino acids
          (B) TYPE: amino acid
          (D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...417

(xi) SEQUENCE DESCRIPTION: SEQ ID NO:412

Met Ser Thr Asn Ile Asp Val Gln Gln Ile Lys Gln Arg Phe Gly Ile
1               5                   10                  15
Ile Gly Ser Ser Pro Leu Met Glu His Ala Ile Arg Val Ala Ala Gln
            20                  25                  30
Val Ala Pro Thr Asp Met Ser Val Leu Val Thr Gly Glu Ser Gly Ser
        35                  40                  45
Gly Lys Glu Phe Phe Pro Gln Ile Ile His Tyr Tyr Ser Ala Arg Lys
    50                  55                  60
His His Ser Tyr Ile Ala Val Asn Cys Gly Ala Ile Pro Glu Gly Thr
65                  70                  75                  80
Ile Asp Ser Glu Leu Phe Gly His Arg Lys Gly Ser Phe Thr Gly Ala
                85                  90                  95
Val Ser Asp Arg Lys Gly Tyr Phe Glu Glu Ala Ser Gly Gly Thr Ile
            100                 105                 110
Phe Leu Asp Glu Val Gly Glu Leu Pro Leu Pro Thr Gln Ala Arg Leu
        115                 120                 125
Leu Arg Val Leu Glu Thr Gly Glu Phe Ile Pro Val Gly Ala Ser Gln
    130                 135                 140
Ser Gln Lys Thr Asp Val Arg Ile Val Ala Ala Thr Asn Val Asn Leu
145                 150                 155                 160
Lys Glu Ala Val Ala Asn Gly Lys Phe Arg Glu Asp Leu Phe Phe Arg
                165                 170                 175
Leu Asn Thr Val Pro Ile Glu Val Pro Ala Leu Arg Met Arg Pro Asp
            180                 185                 190
Asp Val Pro Leu Leu Phe Arg Arg Phe Ala Ala Asp Ser Ala Glu Lys
        195                 200                 205
Tyr Arg Met Pro Pro Leu Arg Leu Ser Asp Glu Ala Arg Thr Ile Leu
    210                 215                 220
Met Arg Tyr Arg Trp Pro Gly Asn Val Arg Glu Leu Arg Asn Ile Thr
225                 230                 235                 240
Asp Arg Leu Ser Ile Leu Glu Glu Glu Arg Thr Val Ser Ala Glu Thr
                245                 250                 255
Ile Thr Arg Tyr Leu Asp Ala Glu Gly Met Gln Asp Leu His Pro Val
            260                 265                 270
Val Ile Arg Arg Asn Glu Thr Thr Glu Ala Asp Lys Gln Ile Pro His
        275                 280                 285
Tyr Glu Arg Glu Ile Ile Tyr Gln Val Leu Tyr Asp Met Lys Lys Glu
    290                 295                 300
Ile Ala Asp Leu Lys Gly Met Met Asn Arg Leu Ala His His Glu Gln
305                 310                 315                 320
Pro Ser Trp Pro Val Gly Ser Asp Val Trp Gly Asn Asp Asp Lys Arg
                325                 330                 335
Thr Ala Asp Pro Lys Trp Gly Val Ser Thr His Lys Ala Pro Ile Ala
            340                 345                 350
Asn Ala Ala Glu Pro Val Glu Pro Ile Gln Glu Ala Ser Glu Tyr Thr
        355                 360                 365
Glu Asp Pro Val Ser Leu Glu Glu Val Glu Lys Lys Met Ile Ser Leu
    370                 375                 380
Ala Leu Glu Arg His Gly Gly Arg Arg Lys Gln Thr Ala Glu Glu Leu
385                 390                 395                 400
Lys Ile Ser Glu Arg Thr Leu Tyr Arg Lys Ile Lys Glu Tyr Gly Leu
                405                 410                 415
Glu

(2) INFORMATION FOR SEQ ID NO:413

(i) SEQUENCE CHARACTERISTICS:
(A) LENGTH: 602 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...602

(xi) SEQUENCE DESCRIPTION: SEQ ID NO:413

Met Ile Leu Leu Phe Gly Gly Thr Thr Glu Gly Arg Ala Ala Ala Arg
1               5                   10                  15
Val Leu Asp Glu Ala Gly Ser Pro Phe Phe Tyr Ser Thr Lys Gly Asn
            20                  25                  30
Leu Gln Glu Ile Gln Ser Ser His Gly His Arg Leu Thr Gly Ala Met
        35                  40                  45
Thr Val Ala Asp Met Val Ser Phe Cys Arg Lys Glu Glu Ile Arg Leu
    50                  55                  60
Ile Val Asp Ala Ala His Pro Phe Ala Glu Glu Leu His Ala Ser Val
65                  70                  75                  80
Ala Glu Ala Ser Glu Gln Thr Gly Ile Pro Val Val Arg Tyr Glu Arg
                85                  90                  95
Gln Tyr Pro Pro Arg Glu Glu Gly Ile Val Trp Cys Ala Asn Tyr Asp
            100                 105                 110
Thr Ala Ala Glu Arg Met Leu Gly Asp Gly Val Gln Arg Leu Leu Met
        115                 120                 125
Leu Thr Gly Val Asn Thr Ile Pro Lys Leu Ala Ala Phe Trp Lys Glu
    130                 135                 140
Arg Thr Thr Phe Cys Arg Ile Leu Lys Arg Asp Glu Ser Val Ala Leu
145                 150                 155                 160
Ala Glu Lys Asn Gly Phe Pro Ala Glu Arg Ile Val Phe Phe Glu Pro
                165                 170                 175
His Ala Asp Glu Glu Leu Met Gln Ala Val Arg Pro Asp Ala Ile Ile
            180                 185                 190
Thr Lys Glu Ser Gly Glu Ser Gly Tyr Phe Arg Glu Lys Ile Glu Ala
        195                 200                 205
Ala Arg Arg Met Gly Ile Arg Ile Tyr Ala Val Val Arg Pro Pro Leu
    210                 215                 220
Pro Pro Ser Phe Ile Pro Val Gly Gly Pro Val Gly Leu Arg Arg Ala
225                 230                 235                 240
Val Glu Arg Leu Val Pro Gly Phe Phe Ser Leu Arg Ser Gly Phe Thr
                245                 250                 255
Thr Gly Thr Thr Ala Thr Ala Ala Val Val Ala Ala Met Tyr Arg Leu
            260                 265                 270
Met Gly Leu Gly Ser Leu Ala Glu Ala Pro Val Glu Leu Pro Ser Gly
        275                 280                 285
Glu Ile Val Ser Leu Pro Ile Ala Glu Ile Arg Glu Glu Glu Asp Ala
    290                 295                 300
Val Val Ser Ala Val Leu Lys Asp Ala Gly Asp Asp Pro Asp Val Thr
305                 310                 315                 320
Asn Gly Met Ala Val Cys Ala Thr Ile Arg Leu Asn Pro Glu His Glu
                325                 330                 335
Glu Val Arg Phe Leu Gln Gly Glu Gly Val Gly Val Val Thr Leu Pro
            340                 345                 350
Gly Leu Gly Leu Glu Val Gly Gly Pro Ala Ile Asn Leu Val Pro Arg
        355                 360                 365
Arg Met Met Thr Ala Glu Val Arg Arg Leu Tyr Ala Gln Gly Gly Val
    370                 375                 380
Asp Ile Thr Ile Ser Val Pro Glu Gly Arg Glu Ala Ala Thr Gln Thr
385                 390                 395                 400
Phe Asn Pro Arg Leu Gly Ile Arg Asp Gly Ile Ser Ile Ile Gly Thr
                405                 410                 415
Ser Gly Val Val Lys Pro Phe Ser Ala Glu Ala Phe Val Gly Ala Ile
            420                 425                 430
Arg Lys Gln Val Gly Ile Ala Thr Ala Leu Gly Ala Asn His Ile Val
        435                 440                 445
Leu Asn Ser Gly Ala Lys Ser Glu Arg Tyr Val Lys Gly Ala Tyr Pro
    450                 455                 460
Ala Leu Ile Pro Gln Ala Phe Val Gln Tyr Gly Asn Phe Val Gly Glu
465                 470                 475                 480
Ser Leu Ser Cys Val Ala Ser Phe Pro Ser Val Arg Ser Val Thr Val

                    485                     490                     495
Gly Ile Met Leu Gly Lys Ala Val Lys Leu Ala Glu Gly Tyr Leu Asp
            500                     505                     510
Thr His Ser Lys Lys Val Val Met Asn Arg Asp Phe Leu His Glu Leu
        515                     520                     525
Ala Arg Gln Ala Gly Cys Ser Glu Asp Ile His Ala Ile Ile Asp Ser
    530                     535                     540
Leu Asn Leu Ala Arg Glu Leu Trp Thr Met Pro Ser Ala Glu Asp Ser
545                     550                     555                     560
Asp Arg Leu Leu Arg Lys Ile Ala Glu Arg Ser Trp Glu Thr Cys Arg
                565                     570                     575
Pro Ser Val Pro Ser Ala Glu Leu Glu Leu Leu Leu Ile Asp Glu Ser
            580                     585                     590
Gly Ala Ile Arg Phe Arg Ile Gly Gly Glu
        595                     600

(2) INFORMATION FOR SEQ ID NO:414

     (i) SEQUENCE CHARACTERISTICS:
          (A) LENGTH: 443 amino acids
          (B) TYPE: amino acid
          (D) TOPOLOGY: linear

     (ii) MOLECULE TYPE: protein

     (iii) HYPOTHETICAL: YES

     (vi) ORIGINAL SOURCE:
          (A) ORGANISM: Porphyromonas gingivalis

     (ix) FEATURE:
          (A) NAME/KEY: misc_feature
          (B) LOCATION 1...443

     (xi) SEQUENCE DESCRIPTION: SEQ ID NO:414

Met Leu Arg Thr Phe Arg Ile Gly Gly Ile His Pro Pro Glu Asn Lys
1               5                   10                  15
Leu Ser Ala Gly Lys Pro Val Glu Val Leu Pro Ile Pro Ser Gln Val
            20                  25                  30
Val Ile Pro Leu Gly Gln His Ile Gly Ala Pro Ala Thr Ala Thr Val
        35                  40                  45
Lys Lys Gly Asp Glu Val Lys Val Gly Thr Ile Ile Ala Gln Ala Gly
    50                  55                  60
Gly Phe Val Ser Ala Asn Ile His Ser Ser Val Ser Gly Lys Val Leu
65                  70                  75                  80
Lys Ile Asp Asn Val Tyr Asp Ser Ser Gly Tyr Pro Lys Pro Ala Val
                85                  90                  95
Phe Ile Ser Val Glu Gly Asp Glu Trp Glu Glu Gly Ile Asp Arg Ser
            100                 105                 110
Pro Ala Ile Val Lys Glu Cys Asn Leu Asp Ala Lys Glu Ile Val Ala
        115                 120                 125
Lys Ile Ser Ala Ala Gly Ile Val Gly Leu Gly Gly Ala Thr Phe Pro
    130                 135                 140
Thr His Val Lys Leu Ser Pro Pro Pro Gly Asn Lys Ala Glu Ile Leu
145                 150                 155                 160
Ile Ile Asn Ala Val Glu Cys Glu Pro Tyr Leu Thr Ser Asp His Val
                165                 170                 175
Leu Met Leu Glu His Gly Glu Glu Ile Met Ile Gly Val Ser Ile Leu
            180                 185                 190
Met Lys Ala Ile Gln Val Asn Lys Ala Val Ile Gly Val Glu Asn Asn
        195                 200                 205
Lys Lys Asp Ala Ile Ala His Leu Thr Lys Leu Ala Thr Ala Tyr Pro
    210                 215                 220
Gly Ile Glu Val Met Pro Leu Lys Val Gln Tyr Pro Gln Gly Gly Glu
225                 230                 235                 240
Lys Gln Leu Ile Asp Ala Val Ile Arg Lys Gln Val Lys Ser Gly Ala
                245                 250                 255
Leu Pro Ile Ser Thr Gly Ala Val Val Gln Asn Val Gly Thr Val Phe
            260                 265                 270
Ala Val Tyr Glu Ala Val Gln Lys Asn Lys Pro Leu Val Glu Arg Ile
        275                 280                 285
Val Thr Val Thr Gly Lys Lys Leu Ser Arg Pro Ser Asn Leu Leu Val
    290                 295                 300
Arg Ile Gly Thr Pro Ile Ala Ala Leu Ile Glu Ala Ala Gly Gly Leu

305                 310                 315                 320
Pro Glu Asn Thr Gly Lys Ile Ile Gly Gly Gly Pro Met Met Gly Arg
                325                 330                 335
Ala Leu Leu Ser Pro Asp Val Pro Val Thr Lys Gly Ser Ser Gly Val
            340                 345                 350
Leu Ile Leu Asp Arg Glu Glu Ala Val Arg Lys Pro Met Arg Asp Cys
        355                 360                 365
Ile Arg Cys Ala Lys Cys Val Gly Val Cys Pro Met Gly Leu Asn Pro
    370                 375                 380
Ala Phe Leu Met Arg Asp Thr Leu Tyr Lys Ser Trp Glu Thr Ala Glu
385                 390                 395                 400
Lys Gly Asn Val Val Asp Cys Ile Glu Cys Gly Ser Cys Ser Phe Thr
                405                 410                 415
Cys Pro Ala Asn Arg Pro Leu Leu Asp Tyr Ile Arg Gln Ala Lys Lys
            420                 425                 430
Thr Val Met Gly Ile Gln Arg Ala Arg Lys Gln
        435                 440

(2) INFORMATION FOR SEQ ID NO:415

     (i) SEQUENCE CHARACTERISTICS:
          (A) LENGTH: 479 amino acids
          (B) TYPE: amino acid
          (D) TOPOLOGY: linear

     (ii) MOLECULE TYPE: protein

     (iii) HYPOTHETICAL: YES

     (vi) ORIGINAL SOURCE:
          (A) ORGANISM: Porphyromonas gingivalis

     (ix) FEATURE:
          (A) NAME/KEY: misc_feature
          (B) LOCATION 1...479

     (xi) SEQUENCE DESCRIPTION: SEQ ID NO:415

Met Lys Arg Ile Gln Leu Thr Leu Ile Ala Leu Phe Ala Ala Val Ala
1               5                   10                  15
Gly Leu Val Ala Gln Asn Ala Tyr Glu Gly Val Ile Ser Tyr Lys Ile
            20                  25                  30
Ser Leu Asp Lys Thr Gly Asn Lys Val Val Leu Asn Gly Ala Ala Asp
        35                  40                  45
Met Ser Asn Leu Lys Leu Lys Ser Thr Gln Met Ile Ile Val Thr Pro
    50                  55                  60
Ile Leu Arg Ser Glu Asp Gly Thr Ser Arg Val Glu Phe Pro Ser Val
65                  70                  75                  80
Val Ile Thr Gly Arg Asn Arg Thr Lys Ala Leu Lys Arg Glu Ile Ala
                85                  90                  95
Phe Ser Ser Ala Leu Pro Gln Ala Lys His Ala Ala Gln Tyr Ile Arg
            100                 105                 110
Arg His Asn Gly Lys Ser Glu Gln Phe Ala Phe Thr Gly Glu His Ala
        115                 120                 125
Tyr Ala Ser Trp Met Met Asp Ala Lys Phe Val Val Arg Glu Glu Val
    130                 135                 140
Arg Gly Cys Ala Lys Cys Pro Val Gly Leu Ser Ser Asn Ile Val Pro
145                 150                 155                 160
Phe Asp Pro Leu Phe Asn Pro Ala Glu Ala Pro Tyr Leu Leu Ala His
                165                 170                 175
Ile Thr Pro Ala Glu Glu Val Glu Lys Gln Arg Glu Ser Ser Phe Asp
            180                 185                 190
Ala Tyr Ile Asn Phe Lys Val Asn Lys Ala Asp Val Leu Pro Glu Tyr
        195                 200                 205
Arg Asn Asn Lys Ala Glu Leu Glu Lys Ile Lys Glu Phe Val Ser Thr
    210                 215                 220
Val Lys Ala Asn Pro Asn Tyr Ser Val Asn Lys Met Ile Ile Glu Gly
225                 230                 235                 240
Phe Ala Ser Pro Glu Ala Ser Ile Ala His Asn Lys Ala Leu Ser Glu
                245                 250                 255
Arg Arg Ala Lys Arg Leu Ala Glu Glu Leu Val Arg Lys Tyr Gly Lys
            260                 265                 270
Thr Leu Pro Asn Ile Thr Thr Glu Phe Gly Gly Glu Asp Trp Lys Gly
        275                 280                 285
Leu Lys Leu Ala Ile Glu Lys Ser Asp Ile Ala Asp Arg Asp Arg Val

290                 295                 300
Leu Glu Ile Ile Asn Ser Asp Lys Tyr Ala Asp Asp Asp Ala Arg Glu
305                 310                 315                 320
Gln Ala Leu Lys Gln Leu Ser Ser Tyr Arg Tyr Ile Leu Asp Gln Ile
                325                 330                 335
Tyr Pro Asn Leu Arg Arg Asn Thr Ile Thr Met Gly Tyr Ile Val Arg
            340                 345                 350
Asp Tyr Thr Leu Glu Glu Ala Arg Glu Ile Ile Lys Thr Ala Pro Lys
        355                 360                 365
Glu Leu Ser Glu Ala Glu Met Tyr Arg Val Ala Met Ser Tyr Pro Glu
    370                 375                 380
Gly His Gln Glu Arg Leu Phe Ala Leu Asn Thr Thr Leu Lys Tyr Phe
385                 390                 395                 400
Pro Glu Ser Val Thr Gly Arg Ile Asn Leu Ala Val Ala Ala Phe Asn
                405                 410                 415
Gly Gly Asp Val Gln Gln Ala Ile Ala Leu Leu Ser Pro Ile Gln Thr
            420                 425                 430
Glu Lys Gly Val Ser Asn Ile Leu Gly Ala Ala Tyr Ala Arg Thr Gly
        435                 440                 445
Asp Phe Ala Arg Ala Glu Thr Phe Phe Arg Lys Ala Val Ala Glu Gly
    450                 455                 460
Asp Ala Asn Ala Gln Arg Asn Leu Asp Met Leu Leu Gly Lys Lys
465                 470                 475

(2) INFORMATION FOR SEQ ID NO:416

     (i) SEQUENCE CHARACTERISTICS:
          (A) LENGTH: 383 amino acids
          (B) TYPE: amino acid
          (D) TOPOLOGY: linear

     (ii) MOLECULE TYPE: protein

    (iii) HYPOTHETICAL: YES

     (vi) ORIGINAL SOURCE:
          (A) ORGANISM: Porphyromonas gingivalis

     (ix) FEATURE:
          (A) NAME/KEY: misc_feature
          (B) LOCATION 1...383

     (xi) SEQUENCE DESCRIPTION: SEQ ID NO:416

Met Ala Glu Lys Arg Asp Tyr Tyr Glu Val Leu Gly Val Ser Lys Asn
1               5                   10                  15
Ala Thr Asp Asp Glu Leu Lys Lys Ala Tyr Arg Lys Lys Ala Ile Gln
            20                  25                  30
Tyr His Pro Asp Lys Asn Pro Gly Asp Lys Glu Ala Glu Glu His Phe
        35                  40                  45
Lys Glu Val Ala Glu Ala Tyr Asp Val Leu Ser Asp Pro Gln Lys Arg
    50                  55                  60
Ser Gln Tyr Asp Gln Phe Gly His Ala Gly Leu Gly Gly Ala Ala Gly
65                  70                  75                  80
Gly Gly Phe Ser Gly Gly Gly Met Ser Met Glu Asp Ile Phe Ser Arg
                85                  90                  95
Phe Gly Asp Leu Phe Gly Gly Phe Gly Gly Phe Gly Gly Phe Ser Asp
            100                 105                 110
Met Gly Gly Gly Ser Arg Arg Arg Val Arg Arg Gly Ser Asp Leu Arg
        115                 120                 125
Val Arg Val Lys Leu Ser Leu Ala Asp Ile Ser Lys Gly Val Glu Lys
    130                 135                 140
Lys Val Lys Val Lys Lys Gln Val Val Cys Ser Lys Cys Arg Gly Asp
145                 150                 155                 160
Gly Thr Glu Glu Ala Asn Gly Lys Thr Thr Cys Gln Thr Cys His Gly
                165                 170                 175
Thr Gly Val Val Thr Arg Val Ser Asn Thr Phe Leu Gly Ala Met Gln
            180                 185                 190
Thr Gln Ser Thr Cys Pro Thr Cys His Gly Glu Gly Glu Ile Ile Thr
        195                 200                 205
Lys Pro Cys Ser Lys Cys Lys Gly Glu Gly Val Glu Ile Gly Glu Glu
    210                 215                 220
Val Ile Ser Phe His Ile Pro Ala Gly Val Ala Glu Gly Met Gln Met
225                 230                 235                 240
Ser Val Asn Gly Lys Gly Asn Ala Ala Pro Arg Gly Gly Val Asn Gly

245                 250                 255
Asp Leu Ile Val Val Ile Ala Glu Glu Pro Asp Pro Asn Leu Ile Arg
            260                 265                 270
Asn Gly Asn Asp Leu Ile Tyr Asn Leu Leu Ile Ser Val Pro Leu Ala
        275                 280                 285
Ile Lys Gly Gly Ser Val Glu Val Pro Thr Ile Asp Gly Arg Ala Lys
    290                 295                 300
Ile Arg Ile Glu Ala Gly Thr Gln Pro Gly Lys Met Leu Arg Leu Arg
305                 310                 315                 320
Asn Lys Gly Leu Pro Ser Val Asn Gly Tyr Gly Met Gly Asp Gln Leu
                325                 330                 335
Val Asn Val Asn Val Tyr Ile Pro Glu Ser Ile Asp Ala Lys Asp Glu
            340                 345                 350
Gln Ala Ile Ala Ala Met Glu Asn Ser Asp Ser Phe Lys Pro Thr Asp
        355                 360                 365
Ala Ala Arg Lys Asp Ile Asp Lys Lys Tyr Arg Glu Met Leu Asp
    370                 375                 380

(2) INFORMATION FOR SEQ ID NO:417

     (i) SEQUENCE CHARACTERISTICS:
          (A) LENGTH: 293 amino acids
          (B) TYPE: amino acid
          (D) TOPOLOGY: linear

    (ii) MOLECULE TYPE: protein

   (iii) HYPOTHETICAL: YES

    (vi) ORIGINAL SOURCE:
          (A) ORGANISM: Porphyromonas gingivalis

    (ix) FEATURE:
          (A) NAME/KEY: misc_feature
          (B) LOCATION 1...293

    (xi) SEQUENCE DESCRIPTION: SEQ ID NO:417

Met Lys Lys Leu Ile Leu Ala Thr Leu Gly Leu Met Ala Ile Ala Met
1               5                   10                  15
Leu Ser Cys Ser Ser Asn Asn Lys Asp Leu Glu Asn Lys Gly Glu Ala
            20                  25                  30
Thr Leu Leu Val Thr Phe Gly Ser Ser Tyr Lys Ala Pro Arg Glu Thr
        35                  40                  45
Tyr Ala Lys Ile Glu Lys Thr Phe Ala Ala Ala Tyr Pro Asp Gln Arg
    50                  55                  60
Ile Ser Trp Thr Tyr Thr Ser Ser Ile Ile Arg Lys Lys Leu Ala Gln
65                  70                  75                  80
Gln Gly Ile Tyr Ile Asp Ala Pro Asp Glu Ala Leu Glu Lys Leu Ala
                85                  90                  95
Arg Leu Gly Tyr Lys Lys Ile Asn Val Gln Ser Leu His Val Ile Pro
            100                 105                 110
Gly Arg Glu Tyr Asp Glu Met Ile Asp Phe Val Asn Lys Phe Lys Ala
        115                 120                 125
Ala His Ser Asp Ile Thr Val Lys Val Gly Ala Pro Leu Phe Asp Thr
    130                 135                 140
Asp Glu Asp Met Arg Glu Val Ala Glu Ile Leu His Lys Arg Phe Gln
145                 150                 155                 160
Gln Thr Ile Glu Lys Gly Glu Ala Ile Val Phe Met Gly His Gly Thr
                165                 170                 175
Glu His Ala Ala Asn Asp Arg Tyr Ala Arg Ile Asn Lys Ile Met Lys
            180                 185                 190
Asn Tyr Ser Lys Phe Met Ile Val Gly Thr Val Glu Ser Asp Pro Ser
        195                 200                 205
Ile Asn Asp Val Ile Ala Glu Leu Lys Glu Thr Gly Ala Thr Ala Val
    210                 215                 220
Thr Met Met Pro Leu Met Ser Val Ala Gly Asp His Ala Thr Asn Asp
225                 230                 235                 240
Met Ala Gly Asp Glu Asp Asp Ser Trp Lys Thr Leu Leu Thr Asn Ala
                245                 250                 255
Gly Tyr Thr Val Ser Ile Asp Lys Leu Asp Asn Gly Asn Phe Ser Ala
            260                 265                 270
Leu Gly Asp Ile Glu Glu Ile Arg Asn Ile Trp Leu Lys His Met Lys
        275                 280                 285
Ala Thr Ser Ala Arg

    290

(2) INFORMATION FOR SEQ ID NO:418

(i) SEQUENCE CHARACTERISTICS:
(A) LENGTH: 356 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...356

(xi) SEQUENCE DESCRIPTION: SEQ ID NO:418

Met Thr Ser Val Ser His Leu Arg Thr Ile Ser Val Ala Gly Ile Leu
1               5                   10                  15
Ala Ala Leu Gly Gly Ala Val Leu Ile Leu Phe Gly Val Asn Leu Phe
            20                  25                  30
Leu Gly Ser Val Ala Ile Pro Met Ser Glu Ile Phe Arg His Leu Phe
        35                  40                  45
Ser Asp Arg Pro Glu Gly Gly Glu Ala Leu Val His Tyr Asn Ile Leu
    50                  55                  60
Trp Lys Ser Arg Leu Pro Glu Ala Leu Thr Ala Ala Phe Ala Gly Ala
65                  70                  75                  80
Gly Leu Ser Val Ser Gly Leu Gln Met Gln Thr Val Phe Arg Asn Pro
                85                  90                  95
Leu Ala Gly Pro Ser Val Leu Gly Ile Ser Ser Gly Ala Ser Leu Gly
            100                 105                 110
Val Ala Leu Val Val Leu Leu Ser Gly Ser Leu Gly Gly Val Ala Leu
        115                 120                 125
Ser Ser Leu Gly Tyr Met Gly Glu Val Ala Met Asn Ile Ala Ala Ala
    130                 135                 140
Val Gly Ser Leu Ala Val Met Gly Leu Ile Val Phe Val Ser Thr Lys
145                 150                 155                 160
Val Arg Ser His Val Thr Leu Leu Ile Ile Gly Val Met Ile Gly Tyr
                165                 170                 175
Val Ala Thr Ala Val Ile Gly Val Phe Lys Phe Phe Ser Ile Glu Glu
            180                 185                 190
Asp Ile Arg Ala Tyr Val Ile Trp Gly Leu Gly Ser Phe Ser Arg Ala
        195                 200                 205
Thr Asp Ser Gln Leu Ser Phe Phe Ala Ile Leu Met Leu Ile Phe Ile
    210                 215                 220
Pro Ala Gly Met Leu Leu Val Lys Gln Leu Asn Leu Leu Leu Leu Gly
225                 230                 235                 240
Glu Ser Tyr Ala Arg Asn Leu Gly Leu Asn Thr Arg Arg Ala Arg Leu
                245                 250                 255
Leu Val Ile Ser Ser Ala Gly Leu Leu Ile Ala Thr Val Thr Ala Tyr
            260                 265                 270
Cys Gly Pro Ile Gly Phe Leu Gly Met Ala Val Pro His Leu Ala Arg
        275                 280                 285
Val Ile Phe His Thr Ser Asp His Arg Ile Leu Met Pro Ala Thr Cys
    290                 295                 300
Leu Ile Gly Ser Ala Leu Ala Leu Phe Cys Asn Ile Ile Ala Arg Met
305                 310                 315                 320
Pro Gly Phe Glu Gly Ala Leu Pro Val Asn Ser Val Thr Ala Leu Val
                325                 330                 335
Gly Ala Pro Ile Ile Val Thr Val Leu Phe Arg Arg Arg Arg Phe Lys
            340                 345                 350
Glu Glu Thr Asp
        355

(2) INFORMATION FOR SEQ ID NO:419

(i) SEQUENCE CHARACTERISTICS:
(A) LENGTH: 757 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...757

(xi) SEQUENCE DESCRIPTION: SEQ ID NO:419

Met Arg Thr Lys Thr Ile Phe Phe Ala Ile Ile Ser Phe Ile Ala Leu
1               5                   10                  15
Leu Ser Ser Ser Leu Ser Ala Gln Ser Lys Ala Val Leu Thr Gly Ser
            20                  25                  30
Val Ser Asp Ala Glu Thr Gly Glu Pro Leu Ala Gly Ala Arg Ile Glu
        35                  40                  45
Val Lys His Thr Asn Ile Val Ala Gly Ala Asp Ala Gly Gly His Phe
    50                  55                  60
Glu Ile Lys Asn Leu Pro Ala Gly Gln His Thr Ile Ile Cys Ser Leu
65                  70                  75                  80
Gly Gly Tyr Gly Gln Lys Glu Glu Val Val Ala Ile Glu Ala Gly Gln
                85                  90                  95
Thr Lys Thr Ile Ser Phe Ala Leu Arg Leu Arg Thr Asn Asn Leu Glu
            100                 105                 110
Glu Val Val Val Thr Gly Thr Gly Thr Arg Tyr Arg Leu Val Asp Ala
        115                 120                 125
Pro Val Ala Thr Glu Val Leu Thr Ala Lys Asp Ile Ala Ser Phe Ser
    130                 135                 140
Ala Pro Thr Ser Glu Ala Leu Leu Gln Gly Leu Ser Pro Ser Phe Asp
145                 150                 155                 160
Phe Gly Pro Asn Leu Met Gly Ser Phe Met Gln Leu Asn Gly Leu Ser
                165                 170                 175
Ser Lys Tyr Ile Leu Ile Leu Ile Asp Gly Lys Arg Val Tyr Gly Asp
            180                 185                 190
Val Gly Gly Gln Ala Asp Leu Ser Arg Ile Ser Pro Asp Gln Ile Glu
        195                 200                 205
Arg Ile Glu Leu Val Lys Gly Ala Ser Ser Ser Leu Tyr Gly Ser Asp
    210                 215                 220
Ala Ile Ala Gly Val Ile Asn Val Ile Thr Lys Lys Asn Thr Asn Arg
225                 230                 235                 240
Leu Ser Ala Tyr Thr Ser His Arg Ile Ser Lys Tyr Asn Asp Arg Gln
                245                 250                 255
Thr Asn Thr Ser Leu Asp Ile Asn Ile Gly Lys Phe Ser Ser Asn Thr
            260                 265                 270
Asn Tyr Phe Phe Tyr His Thr Asp Gly Trp Gln Asn Ser Pro Phe Glu
        275                 280                 285
Ile Lys Lys Lys Lys Gly Ser Gly Glu Pro Val Leu Glu Glu Thr Tyr
    290                 295                 300
Lys Lys Thr Phe Arg Ala Gln Glu Asn Gln Gly Val Ser Gln Ser Leu
305                 310                 315                 320
Ser Tyr Tyr Ala Thr Asn Asn Leu Ser Phe Ser Gly Asn Val Gln Tyr
                325                 330                 335
Asn Lys Arg Gln Ile Phe Thr Pro Thr Phe Ser Glu Lys Lys Ala Tyr
            340                 345                 350
Asp Met Asp Tyr Arg Ala Leu Thr Ala Ser Leu Gly Thr Asn Tyr Leu
        355                 360                 365
Phe Pro Asn Gly Leu His Thr Leu Ser Phe Asp Ala Val Tyr Asp Arg
    370                 375                 380
Phe Arg Phe Gly Tyr Leu Tyr His Asp Lys Asp Ser Ser Glu Ser Leu
385                 390                 395                 400
Ile Asn Asn Gln Gly Gln Thr Glu Gln Pro Thr Phe Phe Pro Gly Gln
                405                 410                 415
Leu Arg Asn Lys Asn Asp Gln Ile Arg Tyr Thr Ala Glu Ala Arg Gly
            420                 425                 430
Val Phe Thr Leu Pro Tyr Ala Gln Lys Leu Thr Gly Gly Leu Glu Tyr
        435                 440                 445
Phe Arg Glu Glu Leu Ile Ser Pro Tyr Asn Leu Ile Thr Asp Lys Ala
    450                 455                 460
Asp Ala Ser Thr Leu Ser Ala Tyr Val Gln Asp Glu Trp Lys Pro Leu
465                 470                 475                 480
Asp Trp Phe Asn Met Thr Ala Gly Phe Arg Leu Val His His Gln Glu

485                 490                 495
Phe Gly Thr Arg Met Thr Pro Lys Val Ser Ile Leu Ala Lys Tyr Gly
            500                 505                 510
Pro Leu Asn Phe Arg Ala Thr Tyr Ala Asn Gly Tyr Lys Thr Pro Thr
        515                 520                 525
Leu Lys Glu Leu Phe Ala Arg Asn Glu Leu Thr Thr Met Gly Ser His
    530                 535                 540
Asn Leu Tyr Leu Gly Asn Ala Asp Leu Lys Pro Gln Met Ser Asp Tyr
545                 550                 555                 560
Tyr Ala Leu Gly Leu Glu Tyr Asn Gln Gly Pro Ile Ser Phe Ser Ala
                565                 570                 575
Thr Val Tyr Asp Asn Glu Leu Arg Asn Leu Ile Ser Phe Met Asp Ile
            580                 585                 590
Pro Thr Ser Pro Glu His Glu Ala Gln Gly Ile Lys Lys Thr Lys Gln
        595                 600                 605
Tyr Ala Asn Ile Gly Lys Ala Arg Ser Arg Gly Leu Asp Val Leu Cys
    610                 615                 620
Asp Ala Ser Ile Gly Trp Gly Ile Lys Leu Gly Ala Gly Tyr Ser Leu
625                 630                 635                 640
Val Glu Ala Lys Asn Leu Gln Thr Asp Glu Trp Leu Glu Gly Ala Ala
                645                 650                 655
Arg His Arg Ala Asn Val His Ala Asp Trp Val His Tyr Trp Gly Gln
            660                 665                 670
Tyr Arg Leu Gly Val Ser Leu Phe Gly Arg Ile Gln Ser Glu Arg Tyr
        675                 680                 685
Tyr Lys Asp Gly Asn Ala Pro Asp Tyr Thr Leu Trp Arg Leu Ala Thr
    690                 695                 700
Ser His Arg Phe Ala His Phe Arg His Ile Ile Leu Asp Gly Thr Leu
705                 710                 715                 720
Gly Ile Asp Asn Leu Phe Asp Tyr Val Asp Asp Arg Pro Met Gly Val
                725                 730                 735
Asn Tyr Ala Thr Val Thr Pro Gly Arg Thr Phe Phe Ala Gln Ile Ala
            740                 745                 750
Ile Arg Phe Asn Asn
        755

(2) INFORMATION FOR SEQ ID NO:420

(i) SEQUENCE CHARACTERISTICS:
(A) LENGTH: 331 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...331

(xi) SEQUENCE DESCRIPTION: SEQ ID NO:420

Met Thr Asp Asn Lys Gln Arg Asn Ile Val Phe Pro Ala Phe Leu Leu
1               5                   10                  15
Leu Leu Gly Val Ile Ala Val Val Thr Ile Val Gly Phe Phe Met Leu
            20                  25                  30
Arg Pro Ala Glu Glu Ile Ile Gln Gly Gln Ile Glu Val Thr Glu Tyr
        35                  40                  45
Arg Val Ser Ser Lys Val Pro Gly Arg Ile Lys Glu Leu Arg Val Ser
    50                  55                  60
Glu Gly Gln Gln Val Gln Ala Gly Asp Thr Leu Ala Val Ile Glu Ala
65                  70                  75                  80
Pro Asp Val Ala Ala Lys Met Glu Gln Ala Lys Ala Ala Glu Ala Ala
                85                  90                  95
Ala Gln Ala Gln Asn Ala Lys Ala Leu Lys Gly Ala Arg Ser Glu Gln
            100                 105                 110
Ile Gln Ala Ala Tyr Glu Met Trp Gln Lys Ala Gln Ala Gly Val Ala
        115                 120                 125
Ile Ala Thr Lys Thr His Gln Arg Val Gln Asn Leu Tyr Asp Gln Gly
    130                 135                 140
Val Val Pro Ala Gln Lys Leu Asp Glu Ala Thr Ala Gln Arg Asp Ala

145                 150                 155                 160
Ala Ile Ala Thr Gln Lys Ala Ala Glu Ala Gln Tyr Asn Met Ala Arg
                165                 170                 175
Asn Gly Ala Glu Arg Glu Asp Lys Leu Ala Ala Ser Ala Leu Val Asp
            180                 185                 190
Arg Ala Arg Gly Ala Val Ala Glu Val Glu Ser Tyr Ile Asn Glu Thr
        195                 200                 205
Tyr Leu Ile Ala Pro Arg Ala Gly Glu Val Ser Glu Ile Phe Pro Lys
    210                 215                 220
Ala Gly Glu Leu Val Gly Thr Gly Ala Pro Ile Met Asn Ile Ala Glu
225                 230                 235                 240
Met Gly Asp Met Trp Ala Ser Phe Ala Val Arg Glu Asp Phe Leu Ser
                245                 250                 255
Ser Met Thr Met Gly Ala Val Leu Glu Thr Val Val Pro Ala Leu Asn
            260                 265                 270
Glu Glu Lys Val Arg Phe Lys Ile Thr Phe Ile Lys Asn Met Gly Thr
        275                 280                 285
Tyr Ala Ala Trp Lys Ala Thr Lys Thr Thr Gly Gln Tyr Asp Leu Lys
    290                 295                 300
Thr Phe Glu Val Lys Ala Thr Leu Ala Asp Lys Asp Lys Ala Gln Lys
305                 310                 315                 320
Leu Arg Pro Gly Met Ser Val Ile Ile Arg Lys
                325                 330

(2) INFORMATION FOR SEQ ID NO:421

     (i) SEQUENCE CHARACTERISTICS:
          (A) LENGTH: 267 amino acids
          (B) TYPE: amino acid
          (D) TOPOLOGY: linear

     (ii) MOLECULE TYPE: protein

     (iii) HYPOTHETICAL: YES

     (vi) ORIGINAL SOURCE:
          (A) ORGANISM: Porphyromonas gingivalis

     (ix) FEATURE:
          (A) NAME/KEY: misc_feature
          (B) LOCATION 1...267

     (xi) SEQUENCE DESCRIPTION: SEQ ID NO:421

Met Arg Ile Val Ser Asn Phe Leu Phe Val Ser Phe Ser Val Leu Leu
1               5                   10                  15
Phe Ala Ser Cys Arg Ser Gln Arg Glu Lys Val Val Tyr Leu Gln Asp
            20                  25                  30
Ile Gln Thr Phe Asn Arg Glu Ile Ile Ala Lys Pro Tyr Asp Val Lys
        35                  40                  45
Ile Glu Lys Asp Asp Val Leu Asn Ile Leu Val Ser Ser Arg Asp Pro
    50                  55                  60
Glu Leu Ser Thr Pro Tyr Asn Gln Val Leu Thr Thr Arg Ala Leu Ala
65                  70                  75                  80
Arg Asn Gly Tyr Gly Thr Asn Ser Asn Glu Gly Phe Leu Val Asp Ser
                85                  90                  95
Lys Gly Tyr Ile Asn Tyr Pro Ile Leu Gly Gln Ile Tyr Val Glu Gly
            100                 105                 110
Leu Thr Arg Thr Glu Leu Glu Lys Glu Ile Gln Lys Arg Ile Ile Ser
        115                 120                 125
Ser Gly Phe Ile Lys Asp Pro Thr Val Thr Val Gln Leu Gln Asn Phe
    130                 135                 140
Lys Val Ser Val Leu Gly Glu Val Asn His Pro Gly Ser Met Ser Val
145                 150                 155                 160
Lys Gly Glu Arg Ile Thr Leu Leu Glu Ala Ile Gly Met Ala Gly Asp
                165                 170                 175
Leu Thr Ile Tyr Gly Arg Arg Asp Arg Val Phe Val Ile Arg Glu Thr
            180                 185                 190
Asp Gly His Arg Glu Val Phe Gln Thr Asp Leu Arg Lys Ala Asp Leu
        195                 200                 205
Leu Ala Ser Pro Val Tyr Tyr Leu His Gln Asn Asp Val Ile Tyr Val
    210                 215                 220
Glu Pro Asn Asp Lys Lys Thr Gln Met Ser Glu Ile Asn Gln Asn Asn
225                 230                 235                 240
Asn Val Asn Val Trp Leu Ser Val Thr Ser Thr Leu Val Ser Ile Ser

                245                 250                 255
Thr Leu Thr Ile Thr Ile Ile Asp Lys Thr Lys
            260                 265

(2) INFORMATION FOR SEQ ID NO:422

(i) SEQUENCE CHARACTERISTICS:
(A) LENGTH: 569 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...569

(xi) SEQUENCE DESCRIPTION: SEQ ID NO:422

Met Lys Lys Thr Asn Leu Phe Leu Ser Leu Leu Val Ile Phe Ile Thr
1               5                   10                  15
Gly Ser Phe Met Thr Ala Cys Ala Gln Lys Ser Lys Thr Asn Lys Leu
            20                  25                  30
Thr Glu Glu Asp Arg Ser Arg Asn Glu Tyr Val Gln Ser Met Asp Val
        35                  40                  45
Leu Ser Asn Ile Ile Gly Asn Val Arg Leu Tyr Phe Val Asp Thr Ile
    50                  55                  60
Ser Ile Lys His Met Thr Arg Arg Gly Ile Asp Ala Met Leu Gly Gly
65                  70                  75                  80
Leu Asp Pro Tyr Thr Glu Tyr Ile Pro Tyr Glu Glu Met Asp Glu Leu
                85                  90                  95
Lys Leu Met Thr Thr Gly Glu Tyr Ala Gly Val Gly Ala Ile Ile Ser
            100                 105                 110
Gln Arg Pro Asp Ser Ala Val Ile Ile Gln Arg Pro Met Glu Gly Met
        115                 120                 125
Pro Ala Asp Glu Ala Gly Leu Ile Ala Gly Asp Arg Ile Leu Thr Ile
    130                 135                 140
Asp Gly Lys Asp Phe Arg Lys Ser Thr Thr Pro Lys Val Ser Gln Ala
145                 150                 155                 160
Leu Lys Gly Ile Ala Gly Thr Val Ala Lys Val Thr Val Met Arg Tyr
                165                 170                 175
Gly Glu Thr Lys Pro Arg Thr Phe Ser Val Lys Arg Gln Lys Val Ile
            180                 185                 190
Met Asn Ser Val Thr Tyr Ser Gly Met Leu Asp Gly Ser Ile Gly Tyr
        195                 200                 205
Ile Arg Leu Asn Asn Phe Thr Asp Lys Ser Ala Glu Glu Val Arg Thr
    210                 215                 220
Ala Leu Leu Asp Leu Arg Asp Lys Gln Gly Ala Lys Gly Leu Ile Leu
225                 230                 235                 240
Asp Leu Arg Gly Asn Gly Gly Gly Leu Met Gln Ala Ala Ile Glu Ile
                245                 250                 255
Val Asn Leu Phe Val Pro Lys Gly Lys Glu Val Val Thr Thr Lys Gly
            260                 265                 270
Arg Ile Ala Glu Ser Ala Ser Val Phe Arg Thr Leu Thr Glu Pro Ile
        275                 280                 285
Asp Thr Lys Leu Pro Ile Val Val Leu Ile Asp Gly Gln Ser Ala Ser
    290                 295                 300
Ser Ser Glu Ile Val Ala Gly Ala Leu Gln Asp Met Asp Arg Ala Val
305                 310                 315                 320
Leu Met Gly Gln Lys Ser Tyr Gly Lys Gly Leu Val Gln Thr Thr Arg
                325                 330                 335
Gln Leu Pro Tyr Asn Gly Val Ile Lys Leu Thr Thr Ala Lys Tyr Tyr
            340                 345                 350
Ile Pro Ser Gly Arg Cys Ile Gln Arg Leu Asp Tyr Ser Arg Thr Asn
        355                 360                 365
Arg Thr Gly Met Ala Thr Ala Ile Pro Asp Ser Leu His Lys Ile Phe
    370                 375                 380
Tyr Thr Ala Ala Gly Arg Arg Val Glu Asp Ala Gly Gly Ile Leu Pro
385                 390                 395                 400
Asp Ile Glu Val Lys Gln Asp Thr Ala Ala Thr Leu Leu Tyr Tyr Met

405                 410                 415
Ala Ile Asn Asn Asp Val Phe Asp Phe Val Thr Gly Tyr Val Leu Lys
            420                 425                 430
His Lys Thr Ile Ala Lys Pro Glu Asp Phe Ser Ile Thr Asn Glu Asp
        435                 440                 445
Tyr Ala Ala Phe Cys Lys Met Met Glu Glu Lys Lys Phe Asp Tyr Asp
    450                 455                 460
Arg Gln Ser Gly Lys Met Leu Asp Lys Leu Glu Glu Leu Ala Lys Ile
465                 470                 475                 480
Glu Gly Tyr Leu Pro Glu Ala Asn Ser Glu Leu Lys Ala Leu Arg Glu
                485                 490                 495
Lys Leu Lys Pro Asn Leu Ser Arg Asp Leu Leu Arg Phe Lys Lys Glu
            500                 505                 510
Ile Thr Asn Tyr Leu Asn Asn Glu Ile Val Thr Arg Tyr Tyr Tyr Glu
        515                 520                 525
Arg Gly Ser Ile Arg Gln Ser Leu Pro Glu Asp Lys Val Val Lys Glu
    530                 535                 540
Ala Ile Lys Leu Leu Lys Asp His Pro Glu Gln Ile Arg Gln Ile Leu
545                 550                 555                 560
Ala Ala Pro Lys Ala Glu Asn Lys Gly
                565

(2) INFORMATION FOR SEQ ID NO:423

(i) SEQUENCE CHARACTERISTICS:
(A) LENGTH: 981 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...981

(xi) SEQUENCE DESCRIPTION: SEQ ID NO:423

Met Gln Asn Lys Gly Phe Val Ile Val Ile Thr Ser Ala Leu Ala Ile
1               5                   10                  15
Ile Cys Ala Phe Tyr Leu Ser Phe Ser Phe Val Thr Asn Arg Tyr Glu
            20                  25                  30
Lys Lys Ala Lys Ala Met Gly Asp Val Ala Gly Met Ala Tyr Leu Asp
        35                  40                  45
Ser Met Ser Asn Glu Lys Val Trp Phe Gly Tyr Thr Leu Lys Glu Ala
    50                  55                  60
Gln Ala Gln Gln Ile Gly Leu Gly Leu Asp Leu Lys Gly Gly Met Asn
65                  70                  75                  80
Val Ile Leu Lys Leu Asn Ala Ser Asp Leu Leu Arg Asn Leu Ser Asn
                85                  90                  95
Lys Ser Leu Asp Pro Asn Phe Asn Lys Ala Leu Glu Asn Ala Ala Lys
            100                 105                 110
Ser Thr Glu Gln Ser Asp Phe Ile Asp Ile Phe Val Lys Glu Tyr Arg
        115                 120                 125
Lys Leu Asp Pro Asn Gly Arg Leu Ala Val Ile Phe Gly Ser Gly Asp
    130                 135                 140
Leu Arg Asp Gln Ile Thr Ala Lys Ser Thr Asp Ala Asp Val Val Arg
145                 150                 155                 160
Leu Leu Lys Glu Lys Tyr Asn Ser Ala Val Glu Ala Ser Phe Asn Val
                165                 170                 175
Leu Arg Ala Arg Ile Asp Ala Phe Gly Val Val Ala Pro Asn Leu Gln
            180                 185                 190
Arg Leu Glu Gly Gln Gly Arg Ile Leu Val Glu Leu Pro Gly Val Lys
        195                 200                 205
Asp Pro Glu Arg Val Arg Thr Leu Leu Gln Arg Ser Ala Asn Leu Gln
    210                 215                 220
Phe Trp Arg Thr Tyr Lys Phe Glu Glu Val Ser Gly Asp Leu Ile Ala
225                 230                 235                 240
Ala Asn Asp Arg Leu Ser Glu Leu Ala Met Asn Asn Thr Asp Ala Thr
                245                 250                 255
Pro Glu Thr Glu Pro Ala Thr Thr Asp Ser Val Ala Ala Thr Ala Asp

            260                 265                 270
Ser Ala Ala Val Gln Ala Val Ala Asp Ser Ala Thr Val Ala Gln Lys
        275                 280                 285
Glu Ala Lys Asp Ala Thr Arg Lys Asp Ala Leu Phe Ser Leu Leu Thr
    290                 295                 300
Pro Val Asn Arg Gly Gly Ala Val Val Gly Val Ala Arg Arg Ala Asn
305                 310                 315                 320
Met Ala Gln Ile Ser Glu Met Leu Gln Gln Ala His Asp Leu Lys Val
                325                 330                 335
Thr Arg Glu Asp Val Leu Phe Leu Trp Gly Ala Lys Ala Ile Glu Asp
            340                 345                 350
Pro Glu Thr Lys Lys Glu Thr Asp Leu Tyr Glu Leu Tyr Ala Ile Arg
        355                 360                 365
Thr Asn Arg Thr Gly Asp Pro Asp Leu Gly Gly Asp Val Val Thr Ser
    370                 375                 380
Ala Lys Ser Asp Ile Gln Asn Asp Phe Gly Arg Ser Glu Pro Ile Val
385                 390                 395                 400
Ser Met Thr Met Asn Glu Glu Gly Ala Arg Lys Trp Ala Arg Ile Thr
                405                 410                 415
Lys Asp Asn Val Gly Arg Ala Ile Ala Ile Val Leu Asp Gly Val Val
            420                 425                 430
Tyr Ser Ala Pro Asn Val Asn Asp Glu Ile Thr Gly Gly Arg Ser Gln
        435                 440                 445
Ile Ser Gly His Phe Thr Val Glu Glu Ala Gly Asp Leu Ala Asn Val
    450                 455                 460
Leu Asn Ser Gly Lys Met Asp Ala Thr Val Ser Ile Glu Gln Glu Asn
465                 470                 475                 480
Val Ile Gly Pro Thr Leu Gly Ala Glu Ser Ile Lys Ala Gly Phe Leu
                485                 490                 495
Ser Phe Leu Leu Ala Leu Val Ile Leu Met Cys Tyr Met Cys Leu Ala
            500                 505                 510
Tyr Gly Phe Leu Pro Gly Leu Ile Ala Asn Gly Ala Leu Ile Val Asn
        515                 520                 525
Ser Phe Phe Thr Leu Gly Val Leu Ala Ser Phe His Ala Val Leu Thr
    530                 535                 540
Leu Ser Gly Ile Ala Gly Leu Val Leu Thr Leu Gly Met Ala Val Asp
545                 550                 555                 560
Ala Asn Val Leu Ile Phe Glu Arg Ile Lys Glu Glu Leu Arg Ala Gly
                565                 570                 575
Lys Thr Pro Ile Arg Ala Val Thr Asp Gly Tyr Gly Asn Ala Phe Ser
            580                 585                 590
Ala Ile Phe Asp Ser Asn Val Thr Thr Ile Ile Thr Gly Ile Ile Leu
        595                 600                 605
Phe Leu Tyr Gly Thr Gly Pro Ile Arg Gly Phe Ala Thr Thr Leu Ile
    610                 615                 620
Ile Gly Leu Ile Ala Ser Phe Ile Thr Ala Val Phe Leu Thr Arg Ile
625                 630                 635                 640
Val Phe Glu Lys Leu Ala Lys Lys Gly Arg Leu Asp Lys Ile Thr Phe
                645                 650                 655
Thr Thr Ser Ile Thr Arg Asn Leu Leu Val Asn Pro Ser Tyr Asn Ile
            660                 665                 670
Leu Gly Lys Arg Lys Thr Gly Phe Ile Ile Pro Val Ile Ile Ile Val
        675                 680                 685
Leu Gly Leu Ile Ala Ser Phe Thr Ile Gly Leu Asn Arg Gly Ile Glu
    690                 695                 700
Phe Ser Gly Gly Arg Asn Tyr Val Val Lys Phe Asp Gln Pro Val Ser
705                 710                 715                 720
Ser Glu Ala Val Arg Ser Ala Leu Ser Ser Pro Leu Gln Glu Lys Val
                725                 730                 735
Leu Val Thr Ser Ile Gly Thr Glu Gly Thr Glu Val Arg Ile Ser Thr
            740                 745                 750
Asn Tyr Lys Ile Gln Glu Glu Ser Glu Glu Thr Glu Ala Glu Ile Thr
        755                 760                 765
Asp Lys Leu Tyr Gln Ser Leu Lys Gly Phe Tyr Thr Gln Gln Pro Thr
    770                 775                 780
Ala Asp Gln Phe Leu Asp Asn Ile Ile Ser Ser Gln Lys Val Ser Pro
785                 790                 795                 800
Ser Met Ser Ser Asp Ile Thr Arg Gly Ala Ile Trp Ala Val Leu Leu
                805                 810                 815
Ser Met Ile Phe Met Ala Ile Tyr Ile Leu Ile Arg Phe Arg Asp Ile
            820                 825                 830
Ser Phe Ser Ala Gly Val Phe Val Ser Val Ala Ala Thr Thr Phe Cys
        835                 840                 845
Ile Ile Ala Leu Tyr Ala Leu Leu Trp Lys Ile Leu Pro Phe Thr Met
    850                 855                 860

Glu Ile Asp Gln Asn Phe Ile Ala Ala Ile Leu Ala Ile Ile Gly Tyr
865                 870                 875                 880
Ser Leu Asn Asp Thr Val Val Val Phe Asp Arg Ile Arg Glu Thr Met
                885                 890                 895
Lys Leu Tyr Pro Asn Arg Asp Arg Tyr Gln Val Ile Asn Asp Ala Leu
            900                 905                 910
Asn Ser Thr Leu Gly Arg Thr Leu Asn Thr Ser Leu Thr Thr Phe Ile
        915                 920                 925
Val Met Leu Val Ile Phe Ile Phe Gly Gly Ala Thr Met Arg Ser Phe
    930                 935                 940
Thr Phe Ser Ile Leu Leu Gly Ile Val Ile Gly Thr Tyr Ser Thr Leu
945                 950                 955                 960
Phe Val Ala Thr Pro Leu Ala Tyr Glu Ile Gln Lys Arg Lys Leu Asn
                965                 970                 975
Lys Ala Ala Lys Lys
            980

(2) INFORMATION FOR SEQ ID NO:424

     (i) SEQUENCE CHARACTERISTICS:
          (A) LENGTH: 1017 amino acids
          (B) TYPE: amino acid
          (D) TOPOLOGY: linear

     (ii) MOLECULE TYPE: protein

     (iii) HYPOTHETICAL: YES

     (vi) ORIGINAL SOURCE:
          (A) ORGANISM: Porphyromonas gingivalis

     (ix) FEATURE:
          (A) NAME/KEY: misc_feature
          (B) LOCATION 1...1017

     (xi) SEQUENCE DESCRIPTION: SEQ ID NO:424
   Met Lys Arg Met Thr Leu Phe Phe Leu Cys Leu Leu Thr Ser Ile Gly
   1               5                   10                  15
   Trp Ala Met Ala Gln Asn Arg Thr Val Lys Gly Thr Val Ile Ser Ser
               20                  25                  30
   Glu Asp Asn Glu Pro Leu Ile Gly Ala Asn Val Val Val Val Gly Asn
           35                  40                  45
   Thr Thr Ile Gly Ala Ala Thr Asp Leu Asp Gly Asn Phe Thr Leu Ser
       50                  55                  60
   Val Pro Ala Asn Ala Lys Met Leu Arg Val Ser Tyr Ser Gly Met Thr
   65                  70                  75                  80
   Thr Lys Glu Val Ala Ile Ala Asn Val Met Lys Ile Val Leu Asp Pro
                   85                  90                  95
   Asp Ser Lys Val Leu Glu Gln Val Val Val Leu Gly Tyr Gly Thr Gly
               100                 105                 110
   Gln Lys Leu Ser Thr Val Ser Gly Ser Val Ala Lys Val Ser Ser Glu
           115                 120                 125
   Lys Leu Ala Glu Lys Pro Val Ala Asn Ile Met Asp Ala Leu Gln Gly
       130                 135                 140
   Gln Val Ala Gly Met Gln Val Met Thr Thr Ser Gly Asp Pro Thr Ala
   145                 150                 155                 160
   Val Ala Ser Val Glu Ile His Gly Thr Gly Ser Leu Gly Ala Ser Ser
                   165                 170                 175
   Ala Pro Leu Tyr Ile Val Asp Gly Met Gln Thr Ser Leu Asp Val Val
               180                 185                 190
   Ala Thr Met Asn Pro Asn Asp Phe Glu Ser Met Ser Val Leu Lys Asp
           195                 200                 205
   Ala Ser Ala Thr Ser Ile Tyr Gly Ala Arg Ala Ala Asn Gly Val Val
       210                 215                 220
   Phe Ile Gln Thr Lys Lys Gly Lys Met Ser Glu Arg Gly Arg Ile Thr
   225                 230                 235                 240
   Phe Asn Ala Ser Tyr Gly Ile Ser Gln Ile Leu Asn Thr Lys Pro Leu
                   245                 250                 255
   Asp Asn Met Met Thr Gly Asp Glu Leu Leu Asp Phe Gln Val Lys Ala
               260                 265                 270
   Gly Phe Trp Gly Asn Asn Gln Thr Val Gln Lys Val Lys Asp Met Ile
           275                 280                 285
   Leu Ala Gly Ala Glu Asp Leu Tyr Gly Asn Tyr Asp Ser Leu Lys Asp
       290                 295                 300
   Glu Tyr Gly Lys Thr Leu Phe Pro Val Asp Phe Asn His Asp Ala Asp
   305                 310                 315                 320
   Trp Leu Lys Ala Leu Phe Lys Thr Ala Pro Thr Ser Gln Gly Asp Ile
                   325                 330                 335
   Ser Phe Ser Gly Gly Ser Gln Gly Thr Ser Tyr Tyr Ala Ser Ile Gly
               340                 345                 350
   Tyr Phe Asp Gln Glu Gly Met Ala Arg Glu Pro Ala Asn Phe Lys Arg
           355                 360                 365
   Tyr Ser Gly Arg Leu Asn Phe Glu Ser Arg Ile Asn Glu Trp Leu Lys
       370                 375                 380
   Val Gly Ala Asn Leu Ser Gly Ala Ile Ala Asn Arg Arg Ser Ala Asp
   385                 390                 395                 400
   Tyr Phe Gly Lys Tyr Tyr Met Gly Ser Gly Thr Phe Gly Val Leu Thr
                   405                 410                 415
   Met Pro Arg Tyr Tyr Asn Pro Phe Asp Val Asn Gly Asp Leu Ala Asp
               420                 425                 430
   Val Tyr Tyr Met Tyr Gly Ala Thr Arg Pro Ser Met Thr Glu Pro Tyr
           435                 440                 445
   Phe Ala Lys Met Arg Pro Phe Ser Ser Glu Ser His Gln Ala Asn Val
       450                 455                 460
   Asn Gly Phe Ala Gln Ile Thr Pro Ile Lys Gly Leu Thr Leu Lys Ala
   465                 470                 475                 480
   Gln Ala Gly Val Asp Ile Thr Asn Thr Arg Thr Ser Ser Lys Arg Met
                   485                 490                 495
   Pro Asn Asn Pro Tyr Asp Ser Thr Pro Leu Gly Glu Arg Arg Glu Arg
               500                 505                 510
   Ala Tyr Arg Asp Val Ser Lys Ser Phe Thr Asn Thr Ala Glu Tyr Lys
           515                 520                 525
   Phe Ser Ile Asp Glu Lys His Asp Leu Thr Ala Leu Met Gly His Glu
       530                 535                 540
   Tyr Ile Glu Tyr Glu Gly Asp Val Ile Gly Ala Ser Ser Lys Gly Phe
   545                 550                 555                 560
   Glu Ser Asp Lys Leu Met Leu Leu Ser Gln Gly Lys Thr Gly Asn Ser
                   565                 570                 575
   Leu Ser Leu Pro Glu His Arg Val Ala Glu Tyr Ala Tyr Leu Ser Phe
               580                 585                 590
   Phe Ser Arg Phe Asn Tyr Gly Phe Asp Lys Trp Met Tyr Ile Asp Phe
           595                 600                 605
   Ser Val Arg Asn Asp Gln Ser Ser Arg Phe Gly Ser Asn Asn Arg Ser
       610                 615                 620
   Ala Trp Phe Tyr Ser Val Gly Gly Met Phe Asp Ile Tyr Asn Lys Phe
   625                 630                 635                 640
   Ile Gln Glu Ser Asn Trp Leu Ser Asp Leu Arg Leu Lys Met Ser Tyr
                   645                 650                 655
   Gly Thr Thr Gly Asn Ser Glu Ile Gly Asn Tyr Asn His Gln Ala Leu
               660                 665                 670
   Val Thr Val Asn Asn Tyr Thr Glu Asp Ala Met Gly Leu Ser Ile Ser
           675                 680                 685
   Thr Ala Gly Asn Pro Asp Leu Ser Trp Glu Lys Gln Ser Gln Phe Asn
       690                 695                 700
   Phe Gly Leu Ala Ala Gly Ala Phe Asn Asn Arg Leu Ser Ala Glu Val
   705                 710                 715                 720
   Asp Phe Tyr Val Arg Thr Thr Asn Asp Met Leu Ile Asp Val Pro Met
                   725                 730                 735
   Pro Tyr Ile Ser Gly Phe Phe Ser Gln Tyr Gln Asn Val Gly Ser Met
               740                 745                 750
   Lys Asn Thr Gly Val Asp Leu Ser Leu Lys Gly Thr Ile Tyr Gln Asn
           755                 760                 765
   Lys Asp Trp Asn Val Tyr Ala Ser Ala Asn Phe Asn Tyr Asn Arg Gln
       770                 775                 780
   Glu Ile Thr Lys Leu Phe Phe Gly Leu Asn Lys Tyr Met Leu Pro Asn
   785                 790                 795                 800
   Thr Gly Thr Ile Trp Glu Ile Gly Tyr Pro Asn Ser Phe Tyr Met Ala
                   805                 810                 815
   Glu Tyr Ala Gly Ile Asp Lys Lys Thr Gly Lys Gln Leu Trp Tyr Val
               820                 825                 830
   Pro Gly Gln Val Asp Ala Asp Gly Asn Lys Val Thr Thr Ser Gln Tyr
           835                 840                 845
   Ser Ala Asp Leu Glu Thr Arg Ile Asp Lys Ser Val Thr Pro Pro Ile
       850                 855                 860
   Thr Gly Gly Phe Ser Leu Gly Ala Ser Trp Lys Gly Leu Ser Leu Asp
   865                 870                 875                 880
   Ala Asp Phe Ala Tyr Ile Val Gly Lys Trp Met Ile Asn Asn Asp Arg
                   885                 890                 895
   Tyr Phe Thr Glu Asn Ala Gly Gly Leu Met Gln Leu Asn Lys Asp Lys
                   900                          905                          910
   Met Leu Leu Asn Ala Trp Thr Glu Asp Asn Lys Glu Thr Asp Val Pro
             915                          920                          925
   Lys Leu Gly Gln Ser Pro Gln Phe Asp Thr His Leu Leu Glu Asn Ala
        930                          935                          940
   Ser Phe Leu Arg Leu Lys Asn Leu Lys Leu Thr Tyr Val Leu Pro Asn
   945                          950                          955                          960
   Ser Leu Phe Ala Gly Gln Asn Val Ile Gly Gly Ala Arg Val Tyr Leu
                       965                          970                          975
   Met Ala Arg Asn Leu Leu Thr Val Thr Lys Tyr Lys Gly Phe Asp Pro
                  980                          985                          990
   Glu Ala Gly Gly Asn Val Gly Lys Asn Gln Tyr Pro Asn Ser Lys Gln
             995                          1000                         1005
   Tyr Val Ala Gly Ile Gln Leu Ser Phe
        1010                         1015

(2) INFORMATION FOR SEQ ID NO:425

      (i) SEQUENCE CHARACTERISTICS:
            (A) LENGTH: 1014 amino acids
            (B) TYPE: amino acid
            (D) TOPOLOGY: linear

      (ii) MOLECULE TYPE: protein

      (iii) HYPOTHETICAL: YES

      (vi) ORIGINAL SOURCE:
            (A) ORGANISM: Porphyromonas gingivalis

      (ix) FEATURE:
            (A) NAME/KEY: misc_feature
            (B) LOCATION 1...1014

      (xi) SEQUENCE DESCRIPTION: SEQ ID NO:425

Met Thr Leu Phe Phe Leu Cys Leu Leu Thr Ser Ile Gly Trp Ala Met
1                   5                             10                            15
Ala Gln Asn Arg Thr Val Lys Gly Thr Val Ile Ser Ser Glu Asp Asn
            20                            25                            30
Glu Pro Leu Ile Gly Ala Asn Val Val Val Val Gly Asn Thr Thr Ile
        35                            40                            45
Gly Ala Ala Thr Asp Leu Asp Gly Asn Phe Thr Leu Ser Val Pro Ala
    50                            55                            60
Asn Ala Lys Met Leu Arg Val Ser Tyr Ser Gly Met Thr Thr Lys Glu
65                            70                            75                            80
Val Ala Ile Ala Asn Val Met Lys Ile Val Leu Asp Pro Asp Ser Lys
                    85                            90                            95
Val Leu Glu Gln Val Val Val Leu Gly Tyr Gly Thr Gly Gln Lys Leu
            100                           105                           110
Ser Thr Val Ser Gly Ser Val Ala Lys Val Ser Ser Glu Lys Leu Ala
        115                           120                           125
Glu Lys Pro Val Ala Asn Ile Met Asp Ala Leu Gln Gly Gln Val Ala
    130                           135                           140
Gly Met Gln Val Met Thr Thr Ser Gly Asp Pro Thr Ala Val Ala Ser
145                           150                           155                           160
Val Glu Ile His Gly Thr Gly Ser Leu Gly Ala Ser Ser Ala Pro Leu
                    165                           170                           175
Tyr Ile Val Asp Gly Met Gln Thr Ser Leu Asp Val Val Ala Thr Met
            180                           185                           190
Asn Pro Asn Asp Phe Glu Ser Met Ser Val Leu Lys Asp Ala Ser Ala
        195                           200                           205
Thr Ser Ile Tyr Gly Ala Arg Ala Ala Asn Gly Val Val Phe Ile Gln
    210                           215                           220
Thr Lys Lys Gly Lys Met Ser Glu Arg Gly Arg Ile Thr Phe Asn Ala
225                           230                           235                           240
Ser Tyr Gly Ile Ser Gln Ile Leu Asn Thr Lys Pro Leu Asp Asn Met
                    245                           250                           255
Met Thr Gly Asp Glu Leu Leu Asp Phe Gln Val Lys Ala Gly Phe Trp
            260                           265                           270
Gly Asn Asn Gln Thr Val Gln Lys Val Lys Asp Met Ile Leu Ala Gly
        275                           280                           285
Ala Glu Asp Leu Tyr Gly Asn Tyr Asp Ser Leu Lys Asp Glu Tyr Gly
    290                           295                           300
Lys Thr Leu Phe Pro Val Asp Phe Asn His Asp Ala Asp Trp Leu Lys

305                 310                 315                 320
Ala Leu Phe Lys Thr Ala Pro Thr Ser Gln Gly Asp Ile Ser Phe Ser
                325                 330                 335
Gly Gly Ser Gln Gly Thr Ser Tyr Tyr Ala Ser Ile Gly Tyr Phe Asp
            340                 345                 350
Gln Glu Gly Met Ala Arg Glu Pro Ala Asn Phe Lys Arg Tyr Ser Gly
        355                 360                 365
Arg Leu Asn Phe Glu Ser Arg Ile Asn Glu Trp Leu Lys Val Gly Ala
    370                 375                 380
Asn Leu Ser Gly Ala Ile Ala Asn Arg Arg Ser Ala Asp Tyr Phe Gly
385                 390                 395                 400
Lys Tyr Tyr Met Gly Ser Gly Thr Phe Gly Val Leu Thr Met Pro Arg
                405                 410                 415
Tyr Tyr Asn Pro Phe Asp Val Asn Gly Asp Leu Ala Asp Val Tyr Tyr
            420                 425                 430
Met Tyr Gly Ala Thr Arg Pro Ser Met Thr Glu Pro Tyr Phe Ala Lys
        435                 440                 445
Met Arg Pro Phe Ser Ser Glu Ser His Gln Ala Asn Val Asn Gly Phe
    450                 455                 460
Ala Gln Ile Thr Pro Ile Lys Gly Leu Thr Leu Lys Ala Gln Ala Gly
465                 470                 475                 480
Val Asp Ile Thr Asn Thr Arg Thr Ser Ser Lys Arg Met Pro Asn Asn
                485                 490                 495
Pro Tyr Asp Ser Thr Pro Leu Gly Glu Arg Arg Glu Arg Ala Tyr Arg
            500                 505                 510
Asp Val Ser Lys Ser Phe Thr Asn Thr Ala Glu Tyr Lys Phe Ser Ile
        515                 520                 525
Asp Glu Lys His Asp Leu Thr Ala Leu Met Gly His Glu Tyr Ile Glu
    530                 535                 540
Tyr Glu Gly Asp Val Ile Gly Ala Ser Ser Lys Gly Phe Glu Ser Asp
545                 550                 555                 560
Lys Leu Met Leu Leu Ser Gln Gly Lys Thr Gly Asn Ser Leu Ser Leu
                565                 570                 575
Pro Glu His Arg Val Ala Glu Tyr Ala Tyr Leu Ser Phe Phe Ser Arg
            580                 585                 590
Phe Asn Tyr Gly Phe Asp Lys Trp Met Tyr Ile Asp Phe Ser Val Arg
        595                 600                 605
Asn Asp Gln Ser Ser Arg Phe Gly Ser Asn Asn Arg Ser Ala Trp Phe
    610                 615                 620
Tyr Ser Val Gly Gly Met Phe Asp Ile Tyr Asn Lys Phe Ile Gln Glu
625                 630                 635                 640
Ser Asn Trp Leu Ser Asp Leu Arg Leu Lys Met Ser Tyr Gly Thr Thr
                645                 650                 655
Gly Asn Ser Glu Ile Gly Asn Tyr Asn His Gln Ala Leu Val Thr Val
            660                 665                 670
Asn Asn Tyr Thr Glu Asp Ala Met Gly Leu Ser Ile Ser Thr Ala Gly
        675                 680                 685
Asn Pro Asp Leu Ser Trp Glu Lys Gln Ser Gln Phe Asn Phe Gly Leu
    690                 695                 700
Ala Ala Gly Ala Phe Asn Asn Arg Leu Ser Ala Glu Val Asp Phe Tyr
705                 710                 715                 720
Val Arg Thr Thr Asn Asp Met Leu Ile Asp Val Pro Met Pro Tyr Ile
                725                 730                 735
Ser Gly Phe Phe Ser Gln Tyr Gln Asn Val Gly Ser Met Lys Asn Thr
            740                 745                 750
Gly Val Asp Leu Ser Leu Lys Gly Thr Ile Tyr Gln Asn Lys Asp Trp
        755                 760                 765
Asn Val Tyr Ala Ser Ala Asn Phe Asn Tyr Asn Arg Gln Glu Ile Thr
    770                 775                 780
Lys Leu Phe Phe Gly Leu Asn Lys Tyr Met Leu Pro Asn Thr Gly Thr
785                 790                 795                 800
Ile Trp Glu Ile Gly Tyr Pro Asn Ser Phe Tyr Met Ala Glu Tyr Ala
                805                 810                 815
Gly Ile Asp Lys Lys Thr Gly Lys Gln Leu Trp Tyr Val Pro Gly Gln
            820                 825                 830
Val Asp Ala Asp Gly Asn Lys Val Thr Thr Ser Gln Tyr Ser Ala Asp
        835                 840                 845
Leu Glu Thr Arg Ile Asp Lys Ser Val Thr Pro Pro Ile Thr Gly Gly
    850                 855                 860
Phe Ser Leu Gly Ala Ser Trp Lys Gly Leu Ser Leu Asp Ala Asp Phe
865                 870                 875                 880
Ala Tyr Ile Val Gly Lys Trp Met Ile Asn Asn Asp Arg Tyr Phe Thr
                885                 890                 895
Glu Asn Ala Gly Gly Leu Met Gln Leu Asn Lys Asp Lys Met Leu Leu
            900                 905                 910

Asn Ala Trp Thr Glu Asp Asn Lys Glu Thr Asp Val Pro Lys Leu Gly
        915                 920                 925
Gln Ser Pro Gln Phe Asp Thr His Leu Leu Glu Asn Ala Ser Phe Leu
    930                 935                 940
Arg Leu Lys Asn Leu Lys Leu Thr Tyr Val Leu Pro Asn Ser Leu Phe
945                 950                 955                 960
Ala Gly Gln Asn Val Ile Gly Gly Ala Arg Val Tyr Leu Met Ala Arg
                965                 970                 975
Asn Leu Leu Thr Val Thr Lys Tyr Lys Gly Phe Asp Pro Glu Ala Gly
            980                 985                 990
Gly Asn Val Gly Lys Asn Gln Tyr Pro Asn Ser Lys Gln Tyr Val Ala
        995                 1000                1005
Gly Ile Gln Leu Ser Phe
    1010

(2) INFORMATION FOR SEQ ID NO:426

(i) SEQUENCE CHARACTERISTICS:
(A) LENGTH: 821 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...821

(xi) SEQUENCE DESCRIPTION: SEQ ID NO:426

Met Lys Lys Lys Asn Phe Leu Leu Leu Gly Ile Phe Val Ala Leu Leu
1               5                   10                  15
Thr Phe Ile Gly Ser Met Gln Ala Gln Gln Ala Lys Asp Tyr Phe Asn
            20                  25                  30
Phe Asp Glu Arg Gly Glu Ala Tyr Phe Ser Phe Lys Val Pro Asp Arg
        35                  40                  45
Ala Val Leu Gln Glu Leu Ala Leu Ile Met Ser Ile Asp Glu Phe Asp
    50                  55                  60
Pro Val Thr Asn Glu Ala Ile Ala Tyr Ala Ser Glu Glu Glu Phe Glu
65                  70                  75                  80
Ala Phe Leu Arg Tyr Gly Leu Lys Pro Thr Phe Leu Thr Pro Pro Ser
                85                  90                  95
Met Gln Arg Ala Val Glu Met Phe Asp Tyr Arg Ser Gly Glu Lys Tyr
            100                 105                 110
Glu Trp Asn Ala Tyr Pro Thr Tyr Glu Ala Tyr Ile Ser Met Met Glu
        115                 120                 125
Glu Phe Gln Thr Lys Tyr Pro Ser Leu Cys Thr Thr Ser Val Ile Gly
    130                 135                 140
Lys Ser Val Lys Asp Arg Lys Leu Met Ile Cys Lys Leu Thr Ser Ser
145                 150                 155                 160
Ala Asn Thr Gly Lys Lys Pro Arg Val Leu Tyr Thr Ser Thr Met His
                165                 170                 175
Gly Asp Glu Thr Thr Gly Tyr Val Val Leu Leu Arg Leu Ile Asp His
            180                 185                 190
Leu Leu Ser Asn Tyr Glu Ser Asp Pro Arg Ile Lys Asn Ile Leu Asp
        195                 200                 205
Lys Thr Glu Val Trp Ile Cys Pro Leu Thr Asn Pro Asp Gly Ala Tyr
    210                 215                 220
Arg Ala Gly Asn His Thr Val Gln Gly Ala Thr Arg Tyr Asn Ala Asn
225                 230                 235                 240
Asn Val Asp Leu Asn Arg Asn Phe Lys Asp Asp Val Ala Gly Asp His
                245                 250                 255
Pro Asp Gly Lys Pro Trp Gln Pro Glu Ala Thr Ala Phe Met Asp Leu
            260                 265                 270
Glu Gly Asn Thr Ser Phe Val Leu Gly Ala Asn Ile His Gly Gly Thr
        275                 280                 285
Glu Val Val Asn Tyr Pro Trp Asp Asn Lys Lys Glu Arg His Ala Asp
    290                 295                 300
Asp Glu Trp Tyr Lys Leu Ile Ser Arg Asn Tyr Ala Ala Ala Cys Gln
305                 310                 315                 320

Ser Ile Ser Ala Ser Tyr Met Thr Ser Glu Thr Asn Ser Gly Ile Ile
                325                 330                 335
Asn Gly Ser Asp Trp Tyr Val Ile Arg Gly Ser Arg Gln Asp Asn Ala
            340                 345                 350
Asn Tyr Phe His Arg Leu Arg Glu Ile Thr Leu Glu Ile Ser Asn Thr
        355                 360                 365
Lys Leu Val Pro Ala Ser Gln Leu Pro Lys Tyr Trp Asn Leu Asn Lys
    370                 375                 380
Glu Ser Leu Leu Ala Leu Ile Glu Glu Ser Leu Tyr Gly Ile His Gly
385                 390                 395                 400
Thr Val Thr Ser Ala Ala Asn Gly Gln Pro Leu Lys Cys Gln Ile Leu
                405                 410                 415
Ile Glu Asn His Asp Lys Arg Asn Ser Asp Val Tyr Ser Asp Ala Thr
            420                 425                 430
Thr Gly Tyr Tyr Val Arg Pro Ile Lys Ala Gly Thr Tyr Thr Val Lys
        435                 440                 445
Tyr Lys Ala Glu Gly Tyr Pro Glu Ala Thr Arg Thr Ile Thr Ile Lys
    450                 455                 460
Asp Lys Glu Thr Val Ile Met Asp Ile Ala Leu Gly Asn Ser Val Pro
465                 470                 475                 480
Leu Pro Val Pro Asp Phe Thr Ala Ser Pro Met Thr Ile Ser Val Gly
                485                 490                 495
Glu Ser Val Gln Phe Gln Asp Gln Thr Thr Asn Asn Pro Thr Asn Trp
            500                 505                 510
Glu Trp Thr Phe Glu Gly Gly Gln Pro Ala Met Ser Thr Glu Gln Asn
        515                 520                 525
Pro Leu Val Ser Tyr Ser His Pro Gly Gln Tyr Asp Val Thr Leu Lys
    530                 535                 540
Val Trp Asn Ala Ser Gly Ser Asn Thr Ile Thr Lys Glu Lys Phe Ile
545                 550                 555                 560
Thr Val Asn Ala Val Met Pro Val Ala Glu Phe Val Gly Thr Pro Thr
                565                 570                 575
Glu Ile Glu Glu Gly Gln Thr Val Ser Phe Gln Asn Gln Ser Thr Asn
            580                 585                 590
Ala Thr Asn Tyr Val Trp Ile Phe Asp Gly Gly Thr Pro Ala Thr Ser
        595                 600                 605
Glu Asp Glu Asn Pro Thr Val Leu Tyr Ser Lys Ala Gly Gln Tyr Asp
    610                 615                 620
Val Thr Leu Lys Ala Ile Ser Ala Ser Gly Glu Thr Val Lys Thr Lys
625                 630                 635                 640
Glu Lys Tyr Ile Thr Val Lys Lys Ala Pro Val Pro Ala Pro Val Ala
                645                 650                 655
Asp Phe Glu Gly Thr Pro Arg Lys Val Lys Lys Gly Glu Thr Val Thr
            660                 665                 670
Phe Lys Asp Leu Ser Thr Asn Asn Pro Thr Ser Trp Leu Trp Val Phe
        675                 680                 685
Glu Gly Gly Ser Pro Ala Thr Ser Thr Glu Gln Asn Pro Val Val Thr
    690                 695                 700
Tyr Asn Glu Thr Gly Lys Tyr Asp Val Gln Leu Thr Ala Thr Asn Glu
705                 710                 715                 720
Gly Gly Ser Asn Val Lys Lys Ala Glu Asp Tyr Ile Glu Val Ile Leu
                725                 730                 735
Asp Asp Ser Val Glu Asp Ile Val Ala Gln Thr Gly Ile Val Ile Arg
            740                 745                 750
Pro Gln Asn Gly Thr Lys Gln Ile Leu Ile Glu Ala Asn Ala Ala Ile
        755                 760                 765
Lys Ala Ile Val Leu Tyr Asp Ile Asn Gly Arg Val Val Leu Lys Thr
    770                 775                 780
Thr Pro Asn Gln Leu Arg Ser Thr Val Asp Leu Ser Ile Leu Pro Glu
785                 790                 795                 800
Gly Ile Tyr Thr Ile Asn Ile Lys Thr Glu Lys Ser Ala Arg Thr Glu
                805                 810                 815
Lys Ile His Ile Gly
                820

(2) INFORMATION FOR SEQ ID NO:427

    (i) SEQUENCE CHARACTERISTICS:
        (A) LENGTH: 76 amino acids
        (B) TYPE: amino acid
        (D) TOPOLOGY: linear

    (ii) MOLECULE TYPE: protein

    (iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...76

(xi) SEQUENCE DESCRIPTION: SEQ ID NO:427

Met Arg Leu Ile Lys Ala Phe Leu Val Gln Leu Leu Leu Leu Pro Ile
1               5                   10                  15
Phe Phe Tyr Lys Arg Phe Ile Ser Pro Leu Thr Pro Pro Ser Cys Arg
            20                  25                  30
Phe Thr Pro Ser Cys Ser Ser Tyr Ala Ile Glu Ala Leu Arg Lys Tyr
        35                  40                  45
Gly Pro Gly Lys Gly Leu Leu Leu Ser Ile Lys Arg Ile Leu Arg Cys
    50                  55                  60
His Pro Trp Gly Gly Ser Gly Tyr Asp Pro Val Pro
65                  70                  75

(2) INFORMATION FOR SEQ ID NO:428

(i) SEQUENCE CHARACTERISTICS:
(A) LENGTH: 859 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...859

(xi) SEQUENCE DESCRIPTION: SEQ ID NO:428

Met Ala Tyr Asp Phe Thr Gln Thr Phe Arg Asn Ser Leu Glu Tyr Ser
1               5                   10                  15
Tyr Gln Glu Ala Thr Arg Leu Gly Val Val Ala Val Thr Gln Asp Met
            20                  25                  30
Leu Val Leu Gly Ile Ile Arg Asp Gly Asp Asn Gly Ala Ile Asp Ile
        35                  40                  45
Met Arg His Tyr Gly Ile Asn Leu Tyr Glu Leu Lys Arg Leu Ile Glu
    50                  55                  60
Leu Glu Ala Ile Ala Glu Ser Leu Pro Ala Ser Pro Glu Gly Ser Pro
65                  70                  75                  80
Ile Phe Thr Pro Ser Ala Arg Glu Ala Ile Asp Asp Ala Thr Asp Ile
                85                  90                  95
Cys Ala Asp Met Glu Asp Glu Ala Val Ser Pro Val His Leu Leu Leu
            100                 105                 110
Ser Ile Leu Asn Ser Thr Gln Glu Ser Leu Val Gln Lys Ile Phe Met
        115                 120                 125
Lys Gln Gly Ile Lys Tyr Asp Thr Ile Leu Ser Asp Tyr Phe Gly Gln
    130                 135                 140
Arg Asn Pro Ser Glu Gly Lys Ser Pro Ser Glu Met Glu Ile Leu Asp
145                 150                 155                 160
Gly Tyr Gln Asp Asn Asp Phe Asp Asp Glu Glu Asp Glu Ser Ser Pro
                165                 170                 175
Pro Ser Gly Asn Ser Gly Thr Gly Gly Gly Ser Gly Asp Ala Pro Glu
            180                 185                 190
Gln Asn Thr Gly Gly Gly Asp Thr Thr Thr Thr Thr Arg Ser Gly Gly
        195                 200                 205
Asp Thr Pro Ala Leu Asp Thr Phe Gly Thr Asp Ile Thr Ala Met Ala
    210                 215                 220
Ala Ala Gly Lys Leu Asp Pro Val Val Gly Arg Glu Gln Glu Ile Glu
225                 230                 235                 240
Arg Val Ile Gln Ile Leu Ser Arg Arg Lys Lys Asn Asn Pro Val Leu
                245                 250                 255
Ile Gly Glu Pro Gly Val Gly Lys Ser Ala Ile Val Glu Gly Leu Ala
            260                 265                 270

Glu Arg Ile Val Asn Arg Lys Val Ser Arg Ile Leu Phe Asp Lys Arg
        275                 280                 285
Ile Ile Ser Leu Asp Leu Ala Gln Met Val Ala Gly Thr Lys Tyr Arg
    290                 295                 300
Gly Gln Phe Glu Glu Arg Leu Lys Ala Val Leu Asp Glu Leu Lys Lys
305                 310                 315                 320
Asn Pro Gln Ile Ile Leu Phe Ile Asp Glu Ile His Thr Ile Val Gly
                325                 330                 335
Ala Gly Ser Ala Ala Gly Ser Met Asp Thr Ala Asn Met Leu Lys Pro
            340                 345                 350
Ala Leu Ala Arg Gly Gln Val Gln Cys Ile Gly Ala Thr Thr Leu Asp
        355                 360                 365
Glu Tyr Arg Lys Asn Ile Glu Lys Asp Gly Ala Leu Glu Arg Arg Phe
    370                 375                 380
Gln Lys Val Pro Ile Ala Pro Ser Thr Ala Glu Glu Thr Leu Thr Ile
385                 390                 395                 400
Leu Gln Asn Ile Lys Glu Lys Tyr Glu Asp Tyr His Gly Val Arg Tyr
                405                 410                 415
Thr Asp Glu Ala Ile Lys Ala Ala Val Glu Leu Thr Asp Arg Tyr Val
            420                 425                 430
Ser Asp Arg Phe Phe Pro Asp Lys Ala Ile Asp Ala Met Asp Glu Ala
        435                 440                 445
Gly Ala Ser Val His Ile Thr Asn Val Val Ala Pro Lys Glu Ile Glu
    450                 455                 460
Ile Leu Glu Ala Glu Leu Ala Ser Val Arg Glu Asn Lys Leu Ser Ala
465                 470                 475                 480
Val Lys Ala Gln Asn Tyr Glu Leu Ala Ala Ser Phe Arg Asp Gln Glu
                485                 490                 495
Arg Arg Thr Gln Gln Gln Ile Ala Glu Glu Lys Lys Lys Trp Glu Glu
            500                 505                 510
Gln Met Ser Lys His Arg Glu Thr Val Asp Glu Asn Val Val Ala His
        515                 520                 525
Val Val Ala Leu Met Thr Gly Val Pro Ala Glu Arg Leu Ser Thr Gly
    530                 535                 540
Glu Gly Glu Arg Leu Arg Thr Met Ala Asp Asp Leu Lys Thr Lys Val
545                 550                 555                 560
Val Gly Gln Asp Thr Ala Ile Glu Lys Met Val His Ala Ile Gln Arg
                565                 570                 575
Asn Arg Leu Gly Leu Arg Asn Glu Lys Lys Pro Ile Gly Ser Phe Leu
            580                 585                 590
Phe Leu Gly Pro Thr Gly Val Gly Lys Thr Tyr Leu Ala Lys Lys Leu
        595                 600                 605
Ala Glu Tyr Leu Phe Glu Asp Glu Asn Ala Met Ile Arg Val Asp Met
    610                 615                 620
Ser Glu Tyr Met Glu Lys Phe Ser Val Ser Arg Leu Val Gly Ala Pro
625                 630                 635                 640
Pro Gly Tyr Val Gly Tyr Glu Glu Gly Gly Gln Leu Thr Glu Arg Val
                645                 650                 655
Arg Arg Lys Pro Tyr Ser Val Val Leu Leu Asp Glu Ile Glu Lys Ala
            660                 665                 670
His Ala Asp Val Phe Asn Leu Leu Leu Gln Val Met Asp Glu Gly Gln
        675                 680                 685
Leu Thr Asp Ser Leu Gly Arg Arg Val Asn Phe Lys Asn Thr Val Ile
    690                 695                 700
Ile Ile Thr Ser Asn Val Gly Thr Arg Gln Leu Lys Asp Phe Gly Gln
705                 710                 715                 720
Gly Ile Gly Phe Arg Ser Glu Lys Asp Glu Glu Ala Asn Lys Glu His
                725                 730                 735
Ser Arg Ser Val Ile Gln Lys Ala Leu Asn Lys Thr Phe Ser Pro Glu
            740                 745                 750
Phe Leu Asn Arg Leu Asp Asp Ile Ile Leu Phe Asp Gln Leu Gly Lys
        755                 760                 765
Thr Glu Ile Arg Arg Met Val Asp Ile Glu Leu Lys Ala Val Leu Ala
    770                 775                 780
Arg Ile His Arg Ala Gly Tyr Asp Leu Val Leu Thr Asp Glu Ala Lys
785                 790                 795                 800
Asp Val Ile Ala Thr Lys Gly Tyr Asp Leu Gln Tyr Gly Ala Arg Pro
                805                 810                 815
Leu Lys Arg Thr Leu Gln Asn Glu Val Glu Asp Arg Leu Thr Asp Leu
            820                 825                 830
Ile Leu Ser Gly Gln Ile Glu Lys Gly Gln Thr Leu Thr Leu Ser Ala
        835                 840                 845
Arg Asp Gly Glu Ile Ile Val Gln Glu Gln Ala
    850                 855

(2) INFORMATION FOR SEQ ID NO:429

(i) SEQUENCE CHARACTERISTICS:
(A) LENGTH: 417 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...417

(xi) SEQUENCE DESCRIPTION: SEQ ID NO:429

Met Asn Tyr Leu Tyr Ile Leu Ile Thr Leu Leu Leu Ser Gly Phe Phe
1               5                   10                  15
Ser Gly Ala Glu Ile Ala Phe Leu Ser Ser Asp Lys Leu Arg Leu Glu
            20                  25                  30
Leu Asp Arg Asn Arg Gly Asp Leu Thr Gly Arg Ala Leu Asn Leu Leu
        35                  40                  45
Tyr Arg His Pro Asp Gln Leu Val Thr Thr Leu Leu Val Gly Asn Asn
    50                  55                  60
Ile Val Leu Val Val Tyr Gly Leu Leu Met Ala Gly Leu Leu Ala Ala
65                  70                  75                  80
Pro Leu Ala Gln Trp Ile Asp Asn Asp Ala Met Ile Val Val Leu Gln
                85                  90                  95
Ser Val Leu Ser Thr Ile Ile Ile Leu Phe Thr Gly Glu Phe Leu Pro
            100                 105                 110
Lys Ala Ile Phe Lys Thr Asn Ala Asn Met Met Met Arg Val Phe Ala
        115                 120                 125
Leu Pro Ile Val Ala Ile Tyr Tyr Leu Leu Tyr Pro Leu Ser Lys Leu
    130                 135                 140
Phe Thr Gly Leu Ser Arg Ser Phe Ile Arg Leu Val Asp Lys Asn Tyr
145                 150                 155                 160
Val Pro Thr Thr Val Gly Leu Gly Arg Val Asp Leu Asp His Tyr Leu
                165                 170                 175
Ala Glu Asn Met Ser Gly Glu Asn Glu Gln Asn Asp Leu Thr Thr Glu
            180                 185                 190
Val Lys Ile Ile Gln Asn Ala Leu Asp Phe Ser Gly Ile Gln Val Arg
        195                 200                 205
Asp Cys Met Ile Pro Arg Asn Glu Met Ile Ala Cys Glu Leu Gln Thr
    210                 215                 220
Asp Ile Glu Val Leu Lys Thr Thr Phe Ile Asp Thr Gly Leu Ser Lys
225                 230                 235                 240
Ile Ile Ile Tyr Arg Gln Asn Ile Asp Asp Val Val Gly Tyr Ile His
                245                 250                 255
Ser Ser Glu Met Phe Arg Gly Gln Asp Trp Gln Lys Arg Ile Asn Thr
            260                 265                 270
Thr Val Phe Val Pro Glu Ser Met Tyr Ala Asn Lys Leu Met Arg Leu
        275                 280                 285
Leu Met Gln Arg Lys Lys Ser Ile Ala Ile Val Ile Asp Glu Leu Gly
    290                 295                 300
Gly Thr Ala Gly Met Val Thr Leu Glu Asp Leu Val Glu Glu Ile Phe
305                 310                 315                 320
Gly Asp Ile Glu Asp Glu His Asp Thr Arg Lys Ile Ile Ala Lys Gln
                325                 330                 335
Leu Gly Pro His Thr Tyr Leu Val Ser Gly Arg Met Glu Ile Asp Asp
            340                 345                 350
Val Asn Glu Arg Phe Gly Leu Ser Leu Pro Glu Ser Asp Asp Tyr Leu
        355                 360                 365
Thr Val Ala Gly Phe Ile Leu Asn Ser His Gln Asn Ile Pro Gln Ala
    370                 375                 380
Asn Glu Val Val Glu Ile Ala Pro Tyr Thr Phe Thr Ile Leu Arg Ser
385                 390                 395                 400
Ser Ser Thr Lys Ile Glu Leu Val Lys Met Ser Ile Asp Asp Gln Ser
                405                 410                 415
Asn

(2) INFORMATION FOR SEQ ID NO:430

(i) SEQUENCE CHARACTERISTICS:
(A) LENGTH: 293 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...293

(xi) SEQUENCE DESCRIPTION: SEQ ID NO:430

Met Lys Gln Asn Tyr Phe Lys Arg Val Cys Ser Leu Leu Trp Leu Val
1               5                   10                  15
Leu Pro Met Leu Ile Met Pro Leu Glu Val Ala Ala Gln Glu Ile Ile
            20                  25                  30
Pro Asn Glu Glu Val Leu Glu Ser Leu Thr Phe Val Ala Pro Val Glu
        35                  40                  45
Glu Thr Asp Ala Ile Glu Ala Glu Val Glu Ala Leu Gln Glu Ile Val
    50                  55                  60
Ala Thr Glu Glu Ile Ala Glu Gln Ala Val Arg Ser Tyr Thr Tyr Thr
65                  70                  75                  80
Val Tyr Arg Asp Gly Val Lys Ile Ala Ser Gly Leu Thr Glu Pro Thr
                85                  90                  95
Phe Leu Asp Glu Asp Val Pro Ala Gly Glu His Thr Tyr Cys Val Glu
            100                 105                 110
Val Gln Tyr Gln Gly Gly Val Ser Asp Lys Val Cys Val Asp Val Glu
        115                 120                 125
Val Lys Asp Phe Lys Pro Val Thr Asn Leu Thr Gly Thr Ala Ser Asn
    130                 135                 140
Asp Glu Val Ser Leu Asp Trp Asp Gly Val Glu Glu Lys Ala Glu Glu
145                 150                 155                 160
Pro Ala Ser Asp Lys Ala Val Ser Tyr Asn Val Tyr Lys Asn Gly Thr
                165                 170                 175
Leu Ile Gly Asn Thr Ala Glu Thr His Tyr Val Glu Thr Gly Val Ala
            180                 185                 190
Asn Gly Thr Tyr Ile Tyr Glu Val Glu Val Lys Tyr Pro Asp Gly Val
        195                 200                 205
Ser Pro Lys Val Ala Val Thr Val Thr Val Thr Asn Ser Ser Leu Ser
    210                 215                 220
Asn Val Asp Gly Gln Ala Pro Tyr Thr Leu Arg Val Glu Gly Lys Lys
225                 230                 235                 240
Ile Ile Ala Glu Ala His Gly Met Ile Thr Leu Tyr Asp Ile Asn Gly
                245                 250                 255
Arg Thr Val Ala Val Ala Pro Asn Arg Leu Glu Tyr Met Ala Gln Thr
            260                 265                 270
Gly Phe Tyr Ala Val Arg Phe Asp Val Gly Asn Lys His His Val Ser
        275                 280                 285
Lys Ile Gln Val Arg
    290

(2) INFORMATION FOR SEQ ID NO:431

(i) SEQUENCE CHARACTERISTICS:
(A) LENGTH: 312 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature

(B) LOCATION 1...312

(xi) SEQUENCE DESCRIPTION: SEQ ID NO:431

Met Ile Pro Leu Ser Glu Ser Phe Glu Ser Gly Ile Pro Ala Ile Trp
1               5                   10                  15
Lys Thr Ile Asp Ala Asp Gly Asp Gly Tyr Asn Trp Met His Leu Thr
            20                  25                  30
Asn Phe Thr Gly Gln Ser Gly Leu Cys Val Ser Ser Ala Ser Tyr Ile
        35                  40                  45
Gly Gly Val Gly Ala Leu Thr Pro Asp Asn Tyr Leu Ile Thr Pro Glu
    50                  55                  60
Leu Lys Leu Pro Thr Asp Ala Leu Val Glu Ile Ile Tyr Trp Val Cys
65                  70                  75                  80
Thr Gln Asp Leu Thr Ala Pro Ser Glu His Tyr Ala Val Tyr Ser Ser
                85                  90                  95
Ser Thr Gly Asn Asn Ala Ala Asp Phe Val Asn Leu Leu Tyr Glu Glu
            100                 105                 110
Thr Leu Thr Ala Lys Arg Ile Gln Ser Pro Glu Leu Ile Arg Gly Asn
        115                 120                 125
Arg Thr Gln Gly Val Trp Tyr Gln Arg Lys Val Val Leu Pro Asn Asp
    130                 135                 140
Thr Lys Tyr Val Ala Phe Arg His Phe Asn Ser Thr Asp Asn Phe Trp
145                 150                 155                 160
Leu Asn Leu Asp Glu Val Ser Ile Leu Tyr Thr Pro Leu Pro Arg Arg
                165                 170                 175
Ala Pro Cys Pro His Pro Gly Gly Tyr Thr Tyr Ser Val Phe Arg Asp
            180                 185                 190
Gly Gln Lys Ile Ala Ser Gly Leu Ser Ala Leu Ala Tyr Ile Asp Thr
        195                 200                 205
Asp Val Pro Tyr Gly Thr Gln Asp Tyr Cys Val Gln Val Asn Tyr Leu
    210                 215                 220
Gln Gly Asp Ser Tyr Lys Val Cys Lys Asn Ile Val Val Ala Asn Ser
225                 230                 235                 240
Ala Asn Ile Tyr Gly Ala Asp Lys Pro Phe Ala Leu Thr Val Val Gly
                245                 250                 255
Lys Thr Ile Val Ala Ser Ala Phe Lys Gly Glu Ile Thr Leu Tyr Asp
            260                 265                 270
Ile Arg Gly Arg Leu Ile Ala Ser Gly Cys Asp Thr Leu Arg Tyr Lys
        275                 280                 285
Ala Glu Asn Gly Phe Tyr Leu Ile Lys Ile Gln Val Asn Gly Thr Val
    290                 295                 300
Tyr Thr Glu Lys Ile Gln Ile Gln
305                 310

(2) INFORMATION FOR SEQ ID NO:432

(i) SEQUENCE CHARACTERISTICS:
(A) LENGTH: 843 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...843

(xi) SEQUENCE DESCRIPTION: SEQ ID NO:432

Met Lys Lys Ser Phe Leu Leu Ala Ile Val Met Leu Phe Gly Ile Ala
1               5                   10                  15
Met Gln Gly His Ser Ala Pro Val Thr Lys Glu Arg Ala Leu Ser Leu
            20                  25                  30
Ala Arg Leu Ala Leu Arg Gln Val Ser Leu Arg Met Gly Gln Thr Ala
        35                  40                  45
Val Ser Asp Lys Ile Ser Ile Asp Tyr Val Tyr Arg Gln Gly Asp Ala
    50                  55                  60
Glu Arg Gly Ile Thr Ser Gln Glu Glu Gly Ser Pro Ala Tyr Phe Tyr
65                  70                  75                  80

Val Ala Asn Arg Gly Asn Asn Glu Gly Tyr Ala Leu Val Ala Ala Asp
                85                  90                  95
Asp Arg Ile Pro Thr Ile Leu Ala Tyr Ser Pro Ile Gly Arg Phe Asp
            100                 105                 110
Met Asp Ser Met Pro Asp Asn Leu Arg Met Trp Leu Gln Ile Tyr Asp
        115                 120                 125
Gln Glu Ile Gly Leu Ile Leu Ser Gly Lys Ala Gln Leu Asn Glu Glu
    130                 135                 140
Ile Leu Arg Thr Glu Gly Val Pro Ala Glu Val His Ala Leu Met Asp
145                 150                 155                 160
Asn Gly His Phe Ala Asn Asp Pro Met Arg Trp Asn Gln Gly Tyr Pro
                165                 170                 175
Trp Asn Asn Lys Glu Pro Leu Leu Pro Asn Gly Asn His Ala Tyr Thr
            180                 185                 190
Gly Cys Val Ala Thr Ala Ala Ala Gln Ile Met Arg Tyr His Ser Trp
        195                 200                 205
Pro Leu Gln Gly Glu Gly Ser Phe Asp Tyr His Ala Gly Ser Leu Val
    210                 215                 220
Gly Asn Trp Ser Gly Thr Phe Gly Glu Met Tyr Asp Trp Ile Asn Met
225                 230                 235                 240
Pro Gly Asn Pro Asp Leu Asp Asn Leu Thr Gln Ser Gln Val Asp Ala
                245                 250                 255
Tyr Ala Thr Leu Met Arg Asp Val Ser Ala Ser Val Ser Met Ser Phe
            260                 265                 270
Tyr Glu Asn Gly Ser Gly Thr Tyr Ser Val Tyr Val Val Gly Ala Leu
        275                 280                 285
Arg Asn Asn Phe Arg Tyr Lys Arg Ser Leu Gln Leu His Val Arg Ala
    290                 295                 300
Leu Tyr Thr Ser Gln Glu Trp His Asp Met Ile Arg Gly Glu Leu Ala
305                 310                 315                 320
Ser Gly Arg Pro Val Tyr Tyr Ala Gly Asn Asn Gln Ser Ile Gly His
                325                 330                 335
Ala Phe Val Cys Asp Gly Tyr Ala Ser Asp Gly Thr Phe His Phe Asn
            340                 345                 350
Trp Gly Trp Gly Gly Val Ser Asn Gly Phe Tyr Lys Leu Thr Leu Leu
        355                 360                 365
Ser Pro Thr Ser Leu Gly Ile Gly Gly Glu Gly Ile Gly Phe Thr Ile
    370                 375                 380
Tyr Gln Glu Ile Ile Thr Gly Ile Glu Pro Ala Lys Thr Pro Ala Glu
385                 390                 395                 400
Ala Gly Thr Asp Ala Leu Pro Ile Leu Ala Leu Lys Asp Ile Glu Ala
                405                 410                 415
Glu Tyr Lys Ser Glu Ser Gly Leu Asn Val Gly Tyr Ser Ile Tyr Asn
            420                 425                 430
Thr Gly Glu Glu Gln Ser Asn Leu Asp Leu Gly Tyr Arg Leu Asn Lys
        435                 440                 445
Ala Asp Gly Glu Val Ile Glu Val Lys Thr Ser Ser Ile Asn Ile Ser
    450                 455                 460
Trp Tyr Gly Tyr Gly Glu His Pro Glu Ser Phe Ser Leu Ala Pro Asn
465                 470                 475                 480
Gln Leu Ser Gln Gly Ile Asn Thr Ile Thr Leu Leu Tyr Arg Arg Thr
                485                 490                 495
Gly Thr Glu Gln Trp Glu Pro Val Arg His Ala Gln Gly Gly Tyr Val
            500                 505                 510
Asn Ser Ile Lys Val Asn Thr Thr Asp Pro Asn Asn Val Val Val Thr
        515                 520                 525
Val Asp Asn Asn Glu Gly Lys Leu Ser Ile Val Pro Asn Ser Phe Val
    530                 535                 540
Ala Asp Leu Asn Ser Tyr Glu His Ser Thr Ile Thr Val Gln Phe Asn
545                 550                 555                 560
Ser Asp Ser Pro Asp Glu Ile Arg Thr Pro Val Ala Phe Ala Leu Ser
                565                 570                 575
Thr Gly Ala Thr Ala Asp Asp Val Ile Ser Leu Gly Trp Val Met Ala
            580                 585                 590
Glu Val Pro Gly Gly Ser Ser Asn Tyr Pro Val Val Trp Ser Lys Asp
        595                 600                 605
Val Leu Thr Leu Ser Glu Gly Asp Tyr Thr Leu Trp Tyr Arg Phe Ser
    610                 615                 620
Ile Asn Asn Gln Lys Asp Glu Trp Lys Lys Ile Gly Ser Val Ser Val
625                 630                 635                 640
Lys Thr Pro Thr Glu Tyr Thr His Pro Leu Phe Glu Val Gly His Asn
                645                 650                 655
Gln Thr Ser Thr Tyr Thr Leu Asp Met Ala His Asn Arg Val Leu Pro
            660                 665                 670
Asp Phe Thr Leu Lys Asn Leu Gly Leu Pro Phe Asn Gly Glu Leu Val

675                 680                 685
Val Val Phe Arg Gln Thr Gln Ser Ser Ser Gly Ser Leu Trp Ala Ala
    690                 695                 700
Gln Glu Thr Val His Ile Lys Gln Gly Glu Thr Phe Val Tyr Lys Pro
705                 710                 715                 720
Val Val Glu Gly Pro Ile Pro Asp Gly Ser Tyr Arg Ala Thr Leu His
                725                 730                 735
Ala Phe Val Asn Gly Gln Gln Gln Leu Tyr Leu Lys Gly Lys Arg Asn
            740                 745                 750
Tyr Thr Val Lys Ile Val Asn Gly Thr Ala Val Glu Ala Ile Glu Ser
        755                 760                 765
Ser Glu Glu Ile Arg Val Phe Pro Asn Pro Ala Arg Asp Tyr Val Glu
    770                 775                 780
Ile Ser Ala Pro Cys Ile Pro Gln Glu Thr Ser Ile Ile Leu Phe Asp
785                 790                 795                 800
Leu Ser Gly Lys Ile Val Met Lys Asn Ser Leu Ser Ala Gly His Gly
                805                 810                 815
Arg Met Asp Val Ser Arg Leu Pro Asn Gly Ala Tyr Ile Leu Lys Val
            820                 825                 830
Asp Gly Tyr Thr Thr Lys Ile Asn Ile Val His
        835                 840

(2) INFORMATION FOR SEQ ID NO:433

(i) SEQUENCE CHARACTERISTICS:
(A) LENGTH: 290 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...290

(xi) SEQUENCE DESCRIPTION: SEQ ID NO:433

Met Lys Lys Leu Phe Leu Ser Leu Thr Ser Leu Val Met Val Phe Ala
1               5                   10                  15
Val Ala Ser Cys Asp Ile Ile Asp Lys Asp Gln Thr Leu Leu Pro Ala
            20                  25                  30
Pro Thr Asn Val Thr Pro Asp Asn Pro Asp Asp Asn Pro Ser Glu Ile
        35                  40                  45
Asp Ile Thr Gln Thr His Thr Glu Lys Tyr Val Leu Ala Glu Glu Phe
    50                  55                  60
Thr Gly Gln Lys Cys Leu Asn Cys Pro Lys Gly His Arg Lys Leu Ala
65                  70                  75                  80
Ala Leu Lys Glu Gln Tyr Gly Lys Arg Leu Thr Val Val Gly Ile His
                85                  90                  95
Ala Gly Pro Gly Ser Leu Val Pro Pro Leu Phe Arg Thr Glu Ala Gly
            100                 105                 110
Asp Ala Tyr Tyr Ser Lys Phe Ala Asn Asn Thr Pro Leu Pro Ala Leu
        115                 120                 125
Met Val Ser Arg Lys Lys Phe Gly Ser Ser Tyr Val Tyr Asp Lys Ser
    130                 135                 140
Tyr Lys Thr Trp Asp Val Pro Ile Ala Glu Gln Met Glu Gln Lys Ala
145                 150                 155                 160
Lys Ile Asn Ile Phe Ala Val Ala Glu Tyr Thr Asp Thr Gln Lys Ile
                165                 170                 175
Lys Val Thr Val Lys Gly Lys Ile Leu Glu Gly Asn Thr Leu Pro Lys
            180                 185                 190
Ser Met Val Gln Val Tyr Leu Leu Glu Asp Lys Leu Ile Ala Pro Gln
        195                 200                 205
Val Asp Gly Asn Thr Thr Val Glu Asn Tyr Glu His Asn His Val Leu
    210                 215                 220
Arg Gly Ala Val Asn Gly Ile Trp Gly Glu Glu Phe Val Asn Leu Lys
225                 230                 235                 240
Asp Tyr Leu Tyr Thr Tyr Ala Val Glu Pro Leu Ser Gly Met Ser Phe
                245                 250                 255
Val Ala Glu Asn Tyr Ser Ile Val Ala Phe Val Tyr Asp Val Gln Thr

                260                 265                 270
Phe Glu Val Tyr Asp Val Val His Val Lys Ile Asn Pro Gln Ser Asp
        275                 280                 285
Gly Lys
    290

(2) INFORMATION FOR SEQ ID NO:434

      (i) SEQUENCE CHARACTERISTICS:
            (A) LENGTH: 223 amino acids
            (B) TYPE: amino acid
            (D) TOPOLOGY: linear

     (ii) MOLECULE TYPE: protein

    (iii) HYPOTHETICAL: YES

     (vi) ORIGINAL SOURCE:
            (A) ORGANISM: Porphyromonas gingivalis

     (ix) FEATURE:
            (A) NAME/KEY: misc_feature
            (B) LOCATION 1...223

     (xi) SEQUENCE DESCRIPTION: SEQ ID NO:434

Met Lys Lys Ser Ser Val Val Ala Ser Val Leu Ala Val Ala Leu Val
1               5                   10                  15
Phe Ala Gly Cys Gly Leu Asn Asn Met Ala Lys Gly Gly Leu Ile Gly
            20                  25                  30
Ala Gly Val Gly Gly Ala Ile Gly Ala Gly Val Gly Asn Val Ala Gly
        35                  40                  45
Asn Thr Ala Val Gly Ala Ile Val Gly Thr Ala Val Gly Gly Ala Ala
    50                  55                  60
Gly Ala Leu Ile Gly Lys Lys Met Asp Lys Gln Lys Lys Glu Leu Glu
65                  70                  75                  80
Ala Ala Val Pro Asp Ala Thr Ile Gln Thr Val Asn Asp Gly Glu Ala
                85                  90                  95
Ile Leu Val Thr Phe Asp Ser Gly Ile Leu Phe Ala Thr Asn Ser Ser
            100                 105                 110
Thr Leu Ser Pro Asn Ser Arg Thr Ala Leu Thr Lys Phe Ala Ala Asn
        115                 120                 125
Met Asn Lys Asn Pro Asp Thr Asp Ile Arg Ile Val Gly His Thr Asp
    130                 135                 140
Asn Thr Gly Ser Asp Lys Ile Asn Asp Pro Leu Ser Glu Arg Arg Ala
145                 150                 155                 160
Ala Ser Val Tyr Ser Phe Leu Asn Ser Gln Gly Val Ser Met Ser Arg
                165                 170                 175
Met Ala Ala Glu Gly Arg Gly Ser His Glu Pro Val Ala Asp Asn Ser
            180                 185                 190
Thr Val Ala Gly Arg Ser Ala Asn Arg Arg Val Glu Val Tyr Ile Leu
        195                 200                 205
Pro Asn Ala Lys Met Ile Glu Gln Ala Gln Gln Gly Thr Leu Lys
    210                 215                 220

(2) INFORMATION FOR SEQ ID NO:435

      (i) SEQUENCE CHARACTERISTICS:
            (A) LENGTH: 337 amino acids
            (B) TYPE: amino acid
            (D) TOPOLOGY: linear

     (ii) MOLECULE TYPE: protein

    (iii) HYPOTHETICAL: YES

     (vi) ORIGINAL SOURCE:
            (A) ORGANISM: Porphyromonas gingivalis

     (ix) FEATURE:
            (A) NAME/KEY: misc_feature
            (B) LOCATION 1...337

     (xi) SEQUENCE DESCRIPTION: SEQ ID NO:435

Met Ser Lys Lys Ser Ile Leu Leu Leu Cys Cys Ser Leu Cys Phe Ile
1               5                   10                  15
Ser Ala Thr Lys Ala Val Thr Pro Val Arg Asn Val Arg Asn Ser Gln
            20                  25                  30
Val Asn Ser Lys Ala Lys Thr Glu Arg Thr Lys Pro Ser Asp Ser Val
        35                  40                  45
Arg Tyr Ile Ser Asn Met Ile Ala Asp Arg Leu Glu Phe Arg Asn Lys
    50                  55                  60
Ile Ser Ser Glu Lys Glu Val Arg Lys Ala Glu Tyr Glu Asn Arg Leu
65                  70                  75                  80
Ala Met Glu Ala Leu Asn Tyr Pro Ala Ile Asp Leu Tyr Gly Glu Asp
                85                  90                  95
Ser Trp Ser Glu Tyr Val Asn Pro Phe Val Gly Ala Gly Thr Asp Val
            100                 105                 110
Glu Ile Pro Asn Ser Tyr Asp Ile Asp Cys Ser Ser Phe Val Met Pro
        115                 120                 125
Val Glu Asp Lys Gln Val Thr Ser Gln Phe Gly Tyr Arg Arg Arg Phe
    130                 135                 140
Gly Arg Met His Tyr Gly Ile Asp Leu Ser Val Asn Arg Gly Asp Thr
145                 150                 155                 160
Ile Arg Ala Ala Phe Asp Gly Lys Val Arg Val Arg Ser Tyr Glu Ala
                165                 170                 175
Arg Gly Tyr Gly Tyr Tyr Ile Val Leu Arg His Pro Asn Gly Leu Glu
            180                 185                 190
Thr Val Tyr Gly His Met Ser Arg Gln Leu Val Asp Glu Asn Gln Ile
        195                 200                 205
Val Arg Ala Gly Gln Pro Ile Gly Leu Gly Gly Ser Thr Gly Arg Ser
    210                 215                 220
Thr Gly Pro His Leu His Phe Glu Thr Arg Phe Met Gly Ile Pro Ile
225                 230                 235                 240
Asn Pro Ser Thr Ile Ile Asp Phe Asp Asn Gly Val Pro Leu Arg Asp
                245                 250                 255
Ile Tyr Thr Phe Lys Arg Gly Ser Asn Ser Arg Tyr Ala Lys Ala Ser
            260                 265                 270
Lys Thr Ser Ser Arg Tyr Ala Lys Lys Gly Lys Lys Gly Arg Gln Ala
        275                 280                 285
Ser Ser Pro Met Thr Tyr Arg Ile Lys Lys Gly Asp Thr Leu Glu Thr
    290                 295                 300
Ile Ala Lys Arg His Gly Thr Ser Val Gln Lys Leu Cys Ala Thr Asn
305                 310                 315                 320
Gly Ile Gly Lys Ser Lys Ile Leu Thr Pro Gly Lys Ala Leu Arg Ile
                325                 330                 335
Lys

(2) INFORMATION FOR SEQ ID NO:436

(i) SEQUENCE CHARACTERISTICS:
(A) LENGTH: 151 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...151

(xi) SEQUENCE DESCRIPTION: SEQ ID NO:436

Met Ala Lys Ile Asn Phe Tyr Ala Glu Gly Val Ser Leu Pro Arg Ile
1               5                   10                  15
Arg Arg Arg Ile Val Gly Lys Trp Ile Ala Glu Val Cys Ser Arg Tyr
            20                  25                  30
Gly Lys Ala Val Gly Glu Ile Ser Tyr Leu Phe Cys Asp Asp Glu Tyr
        35                  40                  45
Ile Leu Lys Ala Asn Gln Glu Phe Leu Asp His Asp Tyr Tyr Thr Asp
    50                  55                  60
Ile Ile Thr Phe Asp Ser Cys Glu Ala Asp Thr Val Asn Gly Asp Leu
65                  70                  75                  80

Leu Ile Ser Leu Asp Thr Val Arg Ser Asn Ala Arg Ala Leu Asp Leu
                85                  90                  95
Arg Tyr Glu Asp Glu Leu His Arg Val Ile Ile His Gly Ile Leu His
            100                 105                 110
Leu Cys Gly Leu Lys Asp Lys Ser Lys Lys Asp Glu Ala Gln Met Arg
        115                 120                 125
Ala Ala Glu Glu Lys Ala Leu Val Met Leu Arg Glu Thr Ile Gly Ser
    130                 135                 140
Glu Leu Ser Leu Leu His Thr
145                 150

(2) INFORMATION FOR SEQ ID NO:437

      (i) SEQUENCE CHARACTERISTICS:
           (A) LENGTH: 391 amino acids
           (B) TYPE: amino acid
           (D) TOPOLOGY: linear

     (ii) MOLECULE TYPE: protein

    (iii) HYPOTHETICAL: YES

     (vi) ORIGINAL SOURCE:
           (A) ORGANISM: Porphyromonas gingivalis

     (ix) FEATURE:
           (A) NAME/KEY: misc_feature
           (B) LOCATION 1...391

     (xi) SEQUENCE DESCRIPTION: SEQ ID NO:437

Met Lys Val Lys Tyr Leu Met Leu Thr Leu Val Gly Ala Ile Ala Leu
1               5                   10                  15
Asn Ala Ser Ala Gln Glu Asn Thr Val Pro Ala Thr Gly Gln Leu Pro
            20                  25                  30
Ala Lys Asn Val Ala Phe Ala Arg Asn Lys Ala Gly Ser Asn Trp Phe
        35                  40                  45
Val Thr Leu Gln Gly Gly Val Ala Ala Gln Phe Leu Asn Asp Asn Asn
    50                  55                  60
Asn Lys Asp Leu Met Asp Arg Leu Gly Ala Ile Gly Ser Leu Ser Val
65                  70                  75                  80
Gly Lys Tyr His Ser Pro Phe Phe Ala Thr Arg Leu Gln Ile Asn Gly
                85                  90                  95
Gly Gln Ala His Thr Phe Leu Gly Lys Asn Gly Glu Gln Glu Ile Asn
            100                 105                 110
Thr Asn Phe Gly Ala Ala His Phe Asp Phe Met Phe Asp Val Val Asn
        115                 120                 125
Tyr Phe Ala Pro Tyr Arg Glu Asn Arg Phe Phe His Leu Ile Pro Trp
    130                 135                 140
Val Gly Val Gly Tyr Gln His Lys Phe Ile Gly Ser Glu Trp Ser Lys
145                 150                 155                 160
Asp Asn Val Glu Ser Leu Thr Ala Asn Val Gly Val Met Met Ala Phe
                165                 170                 175
Arg Leu Gly Lys Arg Val Asp Phe Val Ile Glu Ala Gln Ala Ala His
            180                 185                 190
Ser Asn Leu Asn Leu Ser Arg Ala Tyr Asn Ala Lys Lys Thr Pro Val
        195                 200                 205
Phe Glu Asp Pro Ala Gly Arg Tyr Tyr Asn Gly Phe Gln Gly Met Ala
    210                 215                 220
Thr Ala Gly Leu Asn Phe Arg Leu Gly Ala Val Gly Phe Asn Ala Ile
225                 230                 235                 240
Unk Pro Met Asp Tyr Ala Leu Ile Asn Asp Leu Asn Gly Gln Ile Asn
                245                 250                 255
Arg Leu Arg Ser Glu Val Glu Glu Leu Ser Lys Arg Pro Val Ser Cys
            260                 265                 270
Pro Glu Cys Pro Glu Val Thr Pro Val Thr Lys Thr Glu Asn Ile Leu
        275                 280                 285
Thr Glu Lys Ala Val Leu Phe Arg Phe Asp Ser His Val Val Asp Lys
    290                 295                 300
Asp Gln Leu Ile Asn Leu Tyr Asp Val Ala Gln Phe Val Lys Glu Thr
305                 310                 315                 320
Asn Glu Pro Ile Thr Val Val Gly Tyr Ala Asp Pro Thr Gly Asn Thr
                325                 330                 335
Gln Tyr Asn Glu Lys Leu Ser Glu Arg Arg Ala Lys Ala Val Val Asp
            340                 345                 350

Val Leu Thr Gly Lys Tyr Gly Val Pro Ser Glu Leu Ile Ser Val Glu
        355                 360                 365
Trp Lys Gly Asp Ser Thr Gln Pro Phe Ser Lys Lys Ala Trp Asn Arg
    370                 375                 380
Val Val Ile Val Arg Ser Lys
385                 390

(2) INFORMATION FOR SEQ ID NO:438

    (i) SEQUENCE CHARACTERISTICS:
        (A) LENGTH: 385 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...385

    (xi) SEQUENCE DESCRIPTION: SEQ ID NO:438

Met Thr Tyr Arg Ile Met Lys Ala Lys Ser Leu Leu Leu Ala Leu Ala
1               5                   10                  15
Gly Leu Ala Cys Thr Phe Ser Ala Thr Ala Gln Glu Ala Thr Thr Gln
            20                  25                  30
Asn Lys Ala Gly Met His Thr Ala Phe Gln Arg Asp Lys Ala Ser Asp
        35                  40                  45
His Trp Phe Ile Asp Ile Ala Gly Gly Ala Gly Met Ala Leu Ser Gly
    50                  55                  60
Trp Asn Asn Asp Val Asp Phe Val Asp Arg Leu Ser Ile Val Pro Thr
65                  70                  75                  80
Phe Gly Ile Gly Lys Trp His Glu Pro Tyr Phe Gly Thr Arg Leu Gln
                85                  90                  95
Phe Thr Gly Phe Asp Ile Tyr Gly Phe Pro Gln Gly Ser Lys Glu Arg
            100                 105                 110
Asn His Asn Tyr Phe Gly Asn Ala His Leu Asp Phe Met Phe Asp Leu
        115                 120                 125
Thr Asn Tyr Phe Gly Val Tyr Arg Pro Asn Arg Val Phe His Ile Ile
    130                 135                 140
Pro Trp Ala Gly Ile Gly Phe Gly Tyr Lys Phe His Ser Glu Asn Ala
145                 150                 155                 160
Asn Gly Glu Lys Val Gly Ser Lys Asp Asp Met Thr Gly Thr Val Asn
                165                 170                 175
Val Gly Leu Met Leu Lys Phe Arg Leu Ser Arg Val Val Asp Phe Asn
            180                 185                 190
Ile Glu Gly Gln Ala Phe Ala Gly Lys Met Asn Phe Ile Gly Thr Lys
        195                 200                 205
Arg Gly Lys Ala Asp Phe Pro Val Met Ala Thr Ala Gly Leu Thr Phe
    210                 215                 220
Asn Leu Gly Lys Thr Glu Trp Thr Glu Ile Val Pro Met Asp Tyr Ala
225                 230                 235                 240
Leu Val Asn Asp Leu Asn Asn Gln Ile Asn Ser Leu Arg Gly Gln Val
                245                 250                 255
Glu Glu Leu Ser Arg Arg Pro Val Ser Cys Pro Glu Cys Pro Glu Pro
            260                 265                 270
Thr Gln Pro Thr Val Thr Arg Val Val Val Asp Asn Val Val Tyr Phe
        275                 280                 285
Arg Ile Asn Ser Ala Lys Ile Asp Arg Asn Gln Glu Ile Asn Val Tyr
    290                 295                 300
Asn Thr Ala Glu Tyr Ala Lys Thr Asn Asn Ala Pro Ile Lys Val Val
305                 310                 315                 320
Gly Tyr Ala Asp Glu Lys Thr Gly Thr Ala Ala Tyr Asn Met Lys Leu
                325                 330                 335
Ser Glu Arg Arg Ala Lys Ala Val Ala Lys Met Leu Glu Lys Tyr Gly
            340                 345                 350
Val Ser Ala Asp Arg Ile Thr Ile Glu Trp Lys Gly Ser Ser Glu Gln
        355                 360                 365
Ile Tyr Glu Glu Asn Ala Trp Asn Arg Ile Val Val Met Thr Ala Ala
    370                 375                 380

Glu
385

(2) INFORMATION FOR SEQ ID NO:439

(i) SEQUENCE CHARACTERISTICS:
(A) LENGTH: 190 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc feature
(B) LOCATION 1...190

(xi) SEQUENCE DESCRIPTION: SEQ ID NO:439

Met Glu Phe Phe Met Leu Phe Ile Ala Ala Val Phe Val Asn Asn Val
1               5                   10                  15
Val Leu Ser Gln Phe Leu Gly Ile Cys Pro Phe Leu Gly Val Ser Lys
            20                  25                  30
Lys Val Asp Thr Ser Ile Gly Met Gly Ala Ala Val Thr Phe Val Leu
        35                  40                  45
Ala Leu Ala Thr Leu Val Thr Phe Leu Ile Gln Lys Phe Val Leu Asp
    50                  55                  60
Arg Phe Gly Leu Gly Phe Met Gln Thr Ile Ala Phe Ile Leu Val Ile
65                  70                  75                  80
Ala Ala Leu Val Gln Met Val Glu Ile Ile Leu Lys Lys Val Ser Pro
                85                  90                  95
Pro Leu Tyr Gln Ala Leu Gly Val Phe Leu Pro Leu Ile Thr Thr Asn
            100                 105                 110
Cys Cys Val Leu Gly Val Ala Ile Leu Val Ile Gln Lys Asp Tyr Thr
        115                 120                 125
Leu Leu Gln Ser Phe Val Tyr Ala Ile Ser Thr Ala Ile Gly Phe Thr
    130                 135                 140
Leu Ala Met Val Thr Phe Ala Gly Ile Arg Glu Gln Leu Asp Met Thr
145                 150                 155                 160
Asn Leu Pro Lys Ala Met Lys Gly Ile Pro Ser Ala Leu Leu Ala Ala
                165                 170                 175
Gly Ile Leu Ala Met Ala Phe Met Gly Phe Ser Gly Ile Ala
            180                 185                 190

(2) INFORMATION FOR SEQ ID NO:440

(i) SEQUENCE CHARACTERISTICS:
(A) LENGTH: 186 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...186

(xi) SEQUENCE DESCRIPTION: SEQ ID NO:440

Met Leu Phe Ile Ala Ala Val Phe Val Asn Asn Val Val Leu Ser Gln
1               5                   10                  15
Phe Leu Gly Ile Cys Pro Phe Leu Gly Val Ser Lys Lys Val Asp Thr
            20                  25                  30
Ser Ile Gly Met Gly Ala Ala Val Thr Phe Val Leu Ala Leu Ala Thr
        35                  40                  45
Leu Val Thr Phe Leu Ile Gln Lys Phe Val Leu Asp Arg Phe Gly Leu

50                  55                  60
Gly Phe Met Gln Thr Ile Ala Phe Ile Leu Val Ile Ala Ala Leu Val
65                  70                  75                  80
Gln Met Val Glu Ile Ile Leu Lys Lys Val Ser Pro Pro Leu Tyr Gln
                85                  90                  95
Ala Leu Gly Val Phe Leu Pro Leu Ile Thr Thr Asn Cys Cys Val Leu
            100                 105                 110
Gly Val Ala Ile Leu Val Ile Gln Lys Asp Tyr Thr Leu Leu Gln Ser
        115                 120                 125
Phe Val Tyr Ala Ile Ser Thr Ala Ile Gly Phe Thr Leu Ala Met Val
    130                 135                 140
Thr Phe Ala Gly Ile Arg Glu Gln Leu Asp Met Thr Asn Leu Pro Lys
145                 150                 155                 160
Ala Met Lys Gly Ile Pro Ser Ala Leu Leu Ala Ala Gly Ile Leu Ala
                165                 170                 175
Met Ala Phe Met Gly Phe Ser Gly Ile Ala
            180                 185

(2) INFORMATION FOR SEQ ID NO:441

     (i) SEQUENCE CHARACTERISTICS:
          (A) LENGTH: 833 amino acids
          (B) TYPE: amino acid
          (D) TOPOLOGY: linear

     (ii) MOLECULE TYPE: protein

    (iii) HYPOTHETICAL: YES

     (vi) ORIGINAL SOURCE:
          (A) ORGANISM: Porphyromonas gingivalis

     (ix) FEATURE:
          (A) NAME/KEY: misc_feature
          (B) LOCATION 1...833

     (xi) SEQUENCE DESCRIPTION: SEQ ID NO:441

Met Lys Gln Leu Asn Ile Ile Ser Phe Ile Ile Ala Phe Leu Phe Leu
1               5                   10                  15
Gly Thr Ser Ala Ser Ala Gln Gln Ser Gly Gly Ser Val Thr Gly Thr
            20                  25                  30
Val Val Asp Lys Ser Ser Lys Glu Pro Ile Ala Tyr Val Gln Val Phe
        35                  40                  45
Val Lys Gly Thr Thr Leu Gly Thr Ser Thr Asp Ala Asn Gly Asn Tyr
    50                  55                  60
Ser Ile Lys Gly Ile Pro Ser Gly Asn Gln Thr Ile Val Ala Arg Leu
65                  70                  75                  80
Met Gly Tyr Ser Thr Cys Glu Glu Lys Val His Ile Glu Lys Gly Gly
                85                  90                  95
Ser Arg His Val Asp Leu Tyr Leu Thr Glu Glu Ile Leu Ser Leu Asp
            100                 105                 110
Gly Val Val Val Ser Ala Asn Arg Asn Glu Thr Phe Arg Arg Gln Ala
        115                 120                 125
Pro Ser Leu Val Thr Val Leu Ser Pro Glu Leu Phe Leu Lys Thr Asn
    130                 135                 140
Ser Thr Asn Leu Ser Gln Gly Leu Lys Phe Gln Pro Gly Leu Arg Val
145                 150                 155                 160
Glu Asp Asn Cys Gln Asn Cys Gly Phe Asn Gln Val Arg Ile Asn Gly
                165                 170                 175
Leu Glu Gly Ala Tyr Ser Gln Ile Leu Ile Asp Ser His Pro Ile Phe
            180                 185                 190
Ser Ser Leu Ala Gly Val Tyr Gly Leu Glu Gln Met Pro Ala Asn Met
        195                 200                 205
Ile Glu Arg Val Glu Val Ile Arg Gly Gly Gly Ser Ala Leu Phe Gly
    210                 215                 220
Ser Asn Ala Val Gly Gly Val Ile Asn Val Ile Thr Lys Glu Pro Leu
225                 230                 235                 240
Arg Asn Ser Ala Glu Ile Ser His Ser Thr Met Thr Phe Asp His Ala
                245                 250                 255
Lys Gly Trp Gly Ser Phe Gln Asn Thr Thr Gln Phe Asn Gly Ser Met
            260                 265                 270
Leu Thr Glu Asp Arg Lys Ala Gly Val Met Val Phe Gly Gln His Asn
        275                 280                 285
Tyr Arg Pro Gly Gln Asp Ile Asp Gly Asp Asn Phe Thr Glu Leu Pro

290                 295                 300
Asn Leu Arg Asn Arg Ser Leu Gly Phe Arg Ser Tyr Tyr Lys Thr Gly
305                 310                 315                 320
Leu Tyr Ser Lys Ala Thr Leu Glu Tyr His Ser Met Gln Glu Tyr Arg
                325                 330                 335
Arg Gly Gly Asp Arg Leu Asp Asn Pro Pro Phe Glu Ala Gln Ile Ala
            340                 345                 350
Glu Tyr Leu Gln His Tyr Ile Asn Gly Gly Ser Phe Lys Phe Asp Gln
        355                 360                 365
Gly Phe Ser Gly Gly Lys Asp Phe Phe Ser Leu Tyr Ala Ser Ala Gln
    370                 375                 380
Asp Val Gln Arg Arg Ser Tyr Tyr Gly Gly Gly Asp Tyr Thr Glu Asn
385                 390                 395                 400
Leu Leu Asn Gly Ala Val Gln Ser Gly Ser Thr Glu Ser Asp Glu Tyr
                405                 410                 415
Asn Asp Ala Phe Thr Ala Leu Thr Ser Tyr Gly Thr Thr Lys Gly Phe
            420                 425                 430
Asp Leu Gln Gly Gly Gly Met Tyr Arg His Thr Phe Gly Glu Asn Trp
        435                 440                 445
Asp Phe Thr Gly Gly Leu Glu Tyr Ile Tyr Gly Gln Leu Asp Asp Arg
    450                 455                 460
Ser Gly Tyr Arg Pro Ser Lys Ile Asp Gln Asn Thr Ser Thr Phe Ser
465                 470                 475                 480
Gln Tyr Asp Gln Leu Glu Tyr Lys Thr Glu Lys Leu Ser Ala Leu Ile
                485                 490                 495
Gly Ala Arg Ile Asp Tyr Val Leu Leu Asn Gln Asp Gly Lys Arg Tyr
            500                 505                 510
Ile Asp Pro Leu Phe Ile Phe Ser Pro Arg Ala Asn Val Arg Tyr Asn
        515                 520                 525
Pro Asn Lys Asn Leu Ser Phe Arg Leu Ser Tyr Ser Glu Gly Phe Arg
    530                 535                 540
Ala Pro Gln Tyr Phe Asp Glu Asp Leu His Val Glu Leu Ala Gly Gly
545                 550                 555                 560
Thr Pro Ile Ser Arg Val Leu Ser Pro Asn Leu Lys Glu Glu Arg Ser
                565                 570                 575
Arg Ser Ile Ser Ala Ser Phe Asp Tyr Tyr His Arg Ala Asp Glu Trp
            580                 585                 590
Gln Phe Asn Ile Met Gly Glu Ala Phe Ser Thr Phe Ile Ser Asn Gln
        595                 600                 605
Phe Lys Pro Ser Asp Lys Val Glu Thr Thr Ser Asp Gly Lys Glu Trp
    610                 615                 620
Ile Ile Arg Thr Ile Tyr Asn Asp Lys Asp Gly Val Ser Lys Val Tyr
625                 630                 635                 640
Gly Val Asn Leu Glu Gly Arg Ile Ala Tyr Asn Lys Ser Phe Asp Leu
                645                 650                 655
Gln Leu Gly Gly Thr Trp Gln Arg Ser Arg Tyr Gly Ser Ile Tyr Thr
            660                 665                 670
Ala Val Glu Ala Asp Lys Thr Thr Gly Gln Ala Glu Ile Ser Val Lys
        675                 680                 685
Asp Tyr Val Arg Thr Pro Asn Leu Tyr Gly Tyr Phe Val Ala Thr Val
    690                 695                 700
Arg Pro Thr Glu His Phe Ala Ile Asn Leu Ser Gly Thr Phe Thr Gly
705                 710                 715                 720
Lys Met Asp Val Val His Glu Ala Tyr Glu Gly Asp Ile Pro Ala Glu
                725                 730                 735
His Ile Ala Pro Asp Gly Ser Phe Asp Phe Glu Met Asn Gly Gln Gln
            740                 745                 750
Phe Lys Gly Leu Ala Glu Gly His Ala Lys Leu Val Lys Thr Pro Ala
        755                 760                 765
Phe Ala Asp Ile Asp Leu Lys Leu Ser His Asp Phe His Leu Ala Ser
    770                 775                 780
Thr Met Thr Leu Glu Leu Asn Ala Gly Ile Gln Asn Ile Phe Asn Ser
785                 790                 795                 800
Tyr Gln Lys Asp Thr Asp Lys Gly Pro Gly Arg Ala Ser Thr Tyr Val
                805                 810                 815
Tyr Gly Pro Met Gln Pro Arg Arg Ile Phe Val Gly Thr Lys Ile Asn
            820                 825                 830
Phe

(2) INFORMATION FOR SEQ ID NO:442

    (i) SEQUENCE CHARACTERISTICS:
        (A) LENGTH: 091 amino acids
        (B) TYPE: amino acid

(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...891

(xi) SEQUENCE DESCRIPTION: SEQ ID NO:442

Met Tyr Lys Lys Ile Ile Ala Val Ala Ala Leu Phe Cys Ala Ser Ile
1               5                   10                  15
Gly Ile Leu Lys Gly Gln Ser Ser Asp Leu Thr Pro Gln Asp Thr Ile
            20                  25                  30
Tyr Ser Pro Glu Ile Ser Tyr Ala Lys Pro Ile His Lys Thr Ile Ala
        35                  40                  45
Ser Ile Glu Ile Glu Gly Met Arg Ser Phe Asp Asp Phe Val Leu Arg
    50                  55                  60
Asn Leu Ser Gly Leu Ala Val Gly Asp Glu Val Leu Ile Pro Gly Asp
65                  70                  75                  80
Ala Met Ser Ala Ala Val Asn Arg Ile Met Arg Gln Gly Tyr Phe Ser
                85                  90                  95
Asn Val Arg Ile Ile Ala Asp Lys Tyr Val Gly Asn Lys Val Tyr Leu
            100                 105                 110
Lys Ile Ile Val Thr Glu Arg Pro Arg Ile Ser Lys Val Thr Phe Ser
        115                 120                 125
Gly Val Lys Lys Ser Glu Arg Glu Asp Leu Glu Met Lys Ile Gly Leu
    130                 135                 140
Arg Glu Gly Ile Gln Met Thr Arg Asn Asn Glu Asp Lys Val Arg Gln
145                 150                 155                 160
Ile Val Gln Lys Tyr Phe Ser Glu Lys Gly Tyr Arg Asp Ala Ser Ile
                165                 170                 175
Arg Ile Thr Gln Glu Pro Asp Leu Ser Lys Asp Gly Phe Val Asn Val
            180                 185                 190
Leu Ile Ser Ile Glu Lys Lys Ser Lys Thr Lys Val Asn Glu Ile Tyr
        195                 200                 205
Phe Ser Gly Asn Lys Ala Leu Ser Asn His Lys Leu Arg Met Ala Met
    210                 215                 220
Lys Asn Thr Asn Ala Lys Phe Ser Leu Arg Lys His Ile Arg Ser Ser
225                 230                 235                 240
Phe Leu Lys Leu Phe Ser Thr His Lys Phe Val Glu Glu Ser Tyr Arg
                245                 250                 255
Glu Asp Leu Val Arg Leu Ile Glu Lys Tyr Gln Glu Tyr Gly Tyr Arg
            260                 265                 270
Asp Ala Glu Ile Leu Thr Asp Ser Val Val Lys Ala Pro Asp Gly Lys
        275                 280                 285
Arg Val Asp Ile Tyr Leu Asn Ile Glu Glu Gly Gln Lys Tyr Tyr Ile
    290                 295                 300
Lys Asp Val Asn Phe Val Gly Asn Ser Gln Tyr Pro Ser Glu Tyr Leu
305                 310                 315                 320
Glu Arg Val Leu Gly Ile Lys Ser Gly Asp Val Tyr Asn Gln Arg Arg
                325                 330                 335
Leu Ala Lys Arg Leu Asn Glu Asp Glu Asp Ala Val Gly Asn Leu Tyr
            340                 345                 350
Tyr Asn Asn Gly Tyr Ile Phe Ala Trp Val Asp Pro Val Glu Thr Asn
        355                 360                 365
Val Val Gly Asp Ser Val Ser Leu Asp Ile Arg Ile Ala Glu Gly Lys
    370                 375                 380
Gln Ala Asn Ile Asn Lys Val Ile Ile Lys Gly Asn Thr Val Val Tyr
385                 390                 395                 400
Glu Asp Val Val Arg Arg Glu Leu Tyr Thr Lys Pro Gly Gln Leu Phe
                405                 410                 415
Ser Arg Glu Asp Ile Ile Asn Ser Ile Arg Leu Ile Asn Gln Leu Gly
            420                 425                 430
His Phe Asp Ala Glu Lys Ser Ile Pro Arg Pro Ile Pro Asn Pro Glu
        435                 440                 445
Thr Gly Thr Val Asp Ile Glu Tyr Asp Leu Val Pro Arg Ser Ser Asp
    450                 455                 460
Gln Leu Glu Leu Ser Val Gly Trp Ser Gln Ser Gly Leu Leu Phe Arg
465                 470                 475                 480

Gly Ala Ile Lys Phe Thr Asn Phe Ser Val Gly Asn Leu Leu His Pro
                485                 490                 495
Ser Met Tyr Lys Lys Gly Ile Ile Pro Gln Gly Asp Gly Gln Thr Leu
            500                 505                 510
Ser Leu Ser Ala Gln Thr Asn Gly Lys Tyr Tyr Gln Gln Tyr Ser Val
        515                 520                 525
Thr Phe Met Asp Pro Trp Phe Gly Gly Lys Arg Pro Asp Met Phe Ser
    530                 535                 540
Phe Ser Ala Phe Tyr Ser Lys Thr Thr Ala Ile Asp Ser Lys Phe Tyr
545                 550                 555                 560
Asn Ser Asn Ala Gly Asn Tyr Tyr Asn Ala Tyr Tyr Asn Ser Tyr Tyr
                565                 570                 575
Asn Asn Tyr Asn Ser Tyr Tyr Asn Gly Met Ser Asn Tyr Thr Gly Asp
            580                 585                 590
Leu Tyr Thr Gln Ala Ser Asp Pro Asp Arg Ser Leu Gln Met Leu Gly
        595                 600                 605
Thr Ser Ile Gly Tyr Gly Lys Arg Leu Thr Trp Pro Asp Asn Trp Phe
    610                 615                 620
Gln Ile Tyr Thr Ser Leu Asn Tyr Thr Tyr Tyr Arg Leu Arg Asn Trp
625                 630                 635                 640
Ser Tyr Asn Thr Phe Gln Asn Phe His His Gly Ser Ala Asn Asp Leu
                645                 650                 655
Asn Leu Glu Leu Arg Leu Ser Arg Thr Ser Ile Asp Asn Pro Ile Tyr
            660                 665                 670
Thr Arg Ser Gly Ser Asp Phe Met Val Ser Val Ala Ala Thr Leu Pro
        675                 680                 685
Tyr Ser Leu Trp Asp Asn His Asp Tyr Ala Ser Gln Asn Leu Ser Val
    690                 695                 700
Ser Asp Arg Tyr Arg Phe Ile Glu Tyr His Lys Trp Lys Phe Arg Gly
705                 710                 715                 720
Arg Val Phe Thr Pro Leu Leu Asn Pro Ala Thr His Lys Tyr Thr Pro
                725                 730                 735
Val Leu Met Ser Arg Val Glu Gly Ala Val Leu Gly Ser Tyr Asn Ser
            740                 745                 750
Asn Lys Lys Ser Pro Phe Gly Thr Phe Tyr Met Gly Gly Asp Gly Met
        755                 760                 765
Ser Ser Tyr Tyr Gly Gly Tyr Met Asn Glu Thr Ile Gly Leu Arg Gly
    770                 775                 780
Tyr Lys Asn Gly Ser Ile Ala Gly Asn Asn Tyr Asp Tyr Ala Tyr Ala
785                 790                 795                 800
Tyr Met Arg Leu Thr Met Glu Leu Arg Phe Pro Ile Leu Phe Glu Asn
                805                 810                 815
Ser Phe Asn Ala Trp Leu Leu Ala Phe Ala Glu Ala Gly Asn Ala Trp
            820                 825                 830
Arg Ser Ile Asp Asn Tyr Asn Pro Phe Asn Leu Lys Arg Ser Ala Gly
        835                 840                 845
Val Gly Leu Arg Val Thr Leu Pro Met Val Gly Met Leu Gly Ile Asp
    850                 855                 860
Trp Gly Tyr Gly Phe Asp Arg Pro Asp Asn Ser Leu Gln Arg Gly Gly
865                 870                 875                 880
Ser Asn Val His Phe Val Leu Gly Gln Glu Phe
                885                 890

(2) INFORMATION FOR SEQ ID NO:443

     (i) SEQUENCE CHARACTERISTICS:
          (A) LENGTH: 174 amino acids
          (B) TYPE: amino acid
          (D) TOPOLOGY: linear

    (ii) MOLECULE TYPE: protein

   (iii) HYPOTHETICAL: YES

    (vi) ORIGINAL SOURCE:
          (A) ORGANISM: Porphyromonas gingivalis

    (ix) FEATURE:
          (A) NAME/KEY: misc_feature
          (B) LOCATION 1...174

    (xi) SEQUENCE DESCRIPTION: SEQ ID NO:443

Met Asn Gly Asp Met Lys Arg Phe Leu Ile Leu Ile Gly Phe Ala Leu
1               5                   10                  15

Ala Val Ala Phe Ser Gly Phe Ser Gln Lys Phe Ala Leu Val Asp Met
            20                  25                  30
Glu Tyr Ile Leu Arg Asn Ile Pro Asp Tyr Glu Met Met Asn Glu Gln
        35                  40                  45
Leu Glu Gln Val Ser Lys Lys Trp Gln Asn Glu Ile Glu Ala Leu Glu
    50                  55                  60
Asn Glu Ala Gln Ser Met Tyr Lys Lys Tyr Gln Ser Asp Leu Val Phe
65                  70                  75                  80
Leu Ser Ala Ala Gln Lys Lys Thr Gln Glu Glu Ala Ile Val Lys Lys
                85                  90                  95
Glu Gln Gln Ala Ser Glu Leu Lys Arg Lys Tyr Phe Gly Pro Glu Gly
            100                 105                 110
Glu Leu Tyr Lys Lys Arg Ser Asp Leu Met Lys Pro Ile Gln Asp Glu
        115                 120                 125
Ile Trp Asn Ala Ile Lys Glu Ile Ala Lys Arg Asn Asn Tyr Gln Met
    130                 135                 140
Val Leu Asp Arg Gly Thr Ser Gly Ile Ile Phe Ala Ser Pro Ser Ile
145                 150                 155                 160
Asp Ile Ser Asp Leu Val Leu Ser Lys Met Gly Phe Ser Lys
                165                 170

(2) INFORMATION FOR SEQ ID NO:444

     (i) SEQUENCE CHARACTERISTICS:
          (A) LENGTH: 170 amino acids
          (B) TYPE: amino acid
          (D) TOPOLOGY: linear

    (ii) MOLECULE TYPE: protein

   (iii) HYPOTHETICAL: YES

    (vi) ORIGINAL SOURCE:
          (A) ORGANISM: Porphyromonas gingivalis

    (ix) FEATURE:
          (A) NAME/KEY: misc_feature
          (B) LOCATION 1...170

    (xi) SEQUENCE DESCRIPTION: SEQ ID NO:444

Met Lys Arg Phe Leu Ile Leu Ile Gly Phe Ala Leu Ala Val Ala Phe
1               5                   10                  15
Ser Gly Phe Ser Gln Lys Phe Ala Leu Val Asp Met Glu Tyr Ile Leu
            20                  25                  30
Arg Asn Ile Pro Asp Tyr Glu Met Met Asn Glu Gln Leu Glu Gln Val
        35                  40                  45
Ser Lys Lys Trp Gln Asn Glu Ile Glu Ala Leu Glu Asn Glu Ala Gln
    50                  55                  60
Ser Met Tyr Lys Lys Tyr Gln Ser Asp Leu Val Phe Leu Ser Ala Ala
65                  70                  75                  80
Gln Lys Lys Thr Gln Glu Glu Ala Ile Val Lys Lys Glu Gln Gln Ala
                85                  90                  95
Ser Glu Leu Lys Arg Lys Tyr Phe Gly Pro Glu Gly Glu Leu Tyr Lys
            100                 105                 110
Lys Arg Ser Asp Leu Met Lys Pro Ile Gln Asp Glu Ile Trp Asn Ala
        115                 120                 125
Ile Lys Glu Ile Ala Lys Arg Asn Asn Tyr Gln Met Val Leu Asp Arg
    130                 135                 140
Gly Thr Ser Gly Ile Ile Phe Ala Ser Pro Ser Ile Asp Ile Ser Asp
145                 150                 155                 160
Leu Val Leu Ser Lys Met Gly Phe Ser Lys
                165                 170

(2) INFORMATION FOR SEQ ID NO:445

     (i) SEQUENCE CHARACTERISTICS:
          (A) LENGTH: 163 amino acids
          (B) TYPE: amino acid
          (D) TOPOLOGY: linear

    (ii) MOLECULE TYPE: protein

   (iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...163

(xi) SEQUENCE DESCRIPTION: SEQ ID NO:445

Met Lys Lys Phe Phe Leu Met Leu Leu Met Ala Leu Pro Leu Ser Leu
1               5                   10                  15
Leu Ala Gln Lys Val Ala Val Val Asn Thr Glu Glu Ile Ile Ser Lys
            20                  25                  30
Met Pro Glu Gln Val Ala Ala Thr Lys Gln Leu Asn Glu Leu Ala Glu
        35                  40                  45
Lys Tyr Arg Leu Asp Leu Lys Ser Met Asp Asp Glu Phe Ala Lys Lys
    50                  55                  60
Thr Glu Glu Phe Val Lys Glu Lys Asp Ser Leu Leu Glu Asn Ile Arg
65                  70                  75                  80
Asn Arg Arg Gln Gln Glu Leu Gln Asp Ile Gln Thr Arg Tyr Gln Gln
                85                  90                  95
Ser Tyr Gln Thr Met Gln Glu Asp Leu Gln Lys Arg Gln Gln Gln Leu
            100                 105                 110
Phe Ala Pro Ile Gln Gln Lys Val Ala Asp Ala Ile Lys Lys Val Gly
        115                 120                 125
Asp Glu Glu Asn Cys Ala Tyr Ile Met Glu Ala Gly Met Met Leu Tyr
    130                 135                 140
Thr Gly Ala Thr Ala Ile Asp Leu Thr Ala Lys Val Lys Ala Lys Leu
145                 150                 155                 160
Gly Ile Lys

(2) INFORMATION FOR SEQ ID NO:446

(i) SEQUENCE CHARACTERISTICS:
(A) LENGTH: 827 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...827

(xi) SEQUENCE DESCRIPTION: SEQ ID NO:446
   Met Lys Glu Ala Ile Pro Arg Lys Asn Lys Tyr Ile Lys Leu Asn Gly
   1               5                   10                  15
   Ile Tyr Arg Leu Ser Phe Ile Leu Leu Cys Cys Leu Leu Cys Ser Gln
               20                  25                  30
   Ala Ala Met Ala Gln Gly Val Arg Val Ser Gly Tyr Val Leu Asp Arg
           35                  40                  45
   Gly Glu Lys Pro Ile Pro Phe Ala Gly Val Lys Val Arg Gly Thr Gly
       50                  55                  60
   Thr Gly Ala Thr Thr Asn Leu Lys Gly Tyr Tyr Glu Phe Arg Met Lys
   65                  70                  75                  80
   Ala Thr Thr Asp Ser Ile Thr Ile Glu Phe Ser Ser Met Gly Tyr Gln
                   85                  90                  95
   Gly Val Ser Arg Ser Phe Pro Ser Leu Thr Lys Asp Thr Arg Leu Asn
               100                 105                 110
   Val Arg Leu Ala Glu Ala Glu Met Glu Leu Ser Ser Val Thr Val Gln
           115                 120                 125
   Ala Thr Lys Arg Arg Leu Asn Thr Met Glu Arg Val Asn Thr Arg Asp
       130                 135                 140
   Leu Arg Val Asn Ala Gly Pro Thr Gly Gly Val Glu Ser Leu Ile Ser
   145                 150                 155                 160
   Thr Tyr Ala Gly Val Thr Gln Asn Asn Glu Leu Ser Ser Gln Tyr Ser
                   165                 170                 175
   Val Arg Gly Gly Ser Tyr Asp Glu Asn Met Val Tyr Val Asn Gly Val
   180                          185                          190
   Glu Val Tyr Arg Pro Leu Leu Val Arg Ser Ala Gln Gln Glu Gly Leu
           195                          200                          205
   Ser Phe Val Asn Pro Asp Leu Thr Gln Ser Val Gln Phe Ser Ala Gly
       210                          215                          220
   Gly Phe Thr Ala Asp Tyr Gly Asp Lys Met Ser Ser Val Leu Asp Ile
   225                          230                          235                          240
   Arg Tyr Lys Gln Pro Gln Glu Lys Glu Gly Ala Val Leu Leu Gly Met
                        245                          250                          255
   Leu Gln Ser Ser Ala Tyr Tyr Gly Ser Ser Ala Gly Ala Phe Ser Gln
               260                          265                          270
   Ile Thr Gly Val Arg Tyr Lys Ser Ala Lys Ser Leu Leu Gly Thr Thr
           275                          280                          285
   Asp Thr Lys Ala Glu Tyr Asp Pro Ile Tyr Ala Asp Gly Gln Thr Phe
       290                          295                          300
   Met Thr Tyr Arg Phe Ser Pro Lys Leu Ser Val Ser Phe Leu Gly Asn
   305                          310                          315                          320
   Ile Ser Gln Thr Arg Tyr Lys Phe Val Pro Gln Thr Arg Glu Thr Ser
                        325                          330                          335
   Phe Gly Thr Leu Ser Asp Ala Lys Lys Leu Lys Ile Phe Phe Asp Gly
               340                          345                          350
   Gln Glu Gln Asp Arg Phe Leu Thr Tyr Phe Gly Ala Phe Ser Met Asn
           355                          360                          365
   Phe Val Pro Asp Asp Lys Gln Arg His Thr Val Thr Leu Ser Ala Phe
       370                          375                          380
   Asn Ser Asn Glu Arg Glu Thr Tyr Asp Ile Gln Gly Glu Tyr Phe Leu
   385                          390                          395                          400
   Asn Asp Val Gln Leu Gly Ala Asp Gly Thr Ala Ser Met Ala Ser Gly
                        405                          410                          415
   Ser Glu Asn Ser Asn Gly Leu Gly Ile Gly Arg Asn His Glu His Ala
               420                          425                          430
   Arg Asn Arg Leu Ser Tyr Arg Val Leu Asn Met Gly Tyr Arg Gly Glu
           435                          440                          445
   Met Lys Leu Asn Glu Lys His Arg Leu Gln Ala Gly Val Ser Ala Gln
       450                          455                          460
   Met Glu Lys Ile Ala Asp His Ile Ser Glu Trp Glu Arg Arg Asp Ser
   465                          470                          475                          480
   Val Gly Tyr Asn Leu Pro His Ser Glu Thr Val Leu Leu Met Tyr Asn
                        485                          490                          495
   Asn Leu Tyr Ala Asp Thr Gln Met Arg Gly Thr Arg Leu Ser Ala Phe
               500                          505                          510
   Val Gln Asp Arg Phe Asn Phe Ser Met Gly Gly Gly Thr Phe Ser Leu
           515                          520                          525
   Ile Pro Gly Ile Arg Ala Ser Trp Trp Ser Phe Asn Lys Glu Leu Leu
       530                          535                          540
   Val Ser Pro Arg Ile Ser Val Gly Tyr Ser Pro Glu Ser Asn Pro Ala
   545                          550                          555                          560
   Leu Val Leu Arg Ala Ala Ala Gly Leu Tyr Tyr Gln Ala Pro Phe Tyr
                        565                          570                          575
   Lys Glu Leu Arg Gln Thr His Lys Asp Ala Glu Gly Asn Asn Val Val
               580                          585                          590
   Val Leu Asn Glu Lys Ile Arg Ser Gln Gly Ala Phe His Ile Leu Ala
           595                          600                          605
   Gly Ala Asp Tyr Thr Phe Glu Met Gly Gly Arg Lys Tyr Lys Phe Thr
       610                          615                          620
   Ala Glu Ala Tyr Tyr Lys Ser Leu Phe Asn Ile Asn Pro Tyr Ile Ile
   625                          630                          635                          640
   Glu Asn Val Lys Ile Arg Tyr Leu Gly Glu Asn Ile Gly Ser Gly Tyr
                        645                          650                          655
   Ala Ala Gly Ile Asp Leu Lys Leu Phe Gly Glu Leu Val Pro Gly Val
               660                          665                          670
   Asp Ser Trp Leu Thr Ala Ser Ile Ile Lys Ala Arg Gln Lys Leu Asp
           675                          680                          685
   Gly Tyr Gly Ser Leu Pro Leu Met Asn Ala Pro Thr Tyr Asn Phe Ser
       690                          695                          700
   Phe Phe Leu Gln Glu Tyr Val Pro Gly Asn Lys Arg Ile Thr Ala Thr
   705                          710                          715                          720
   Leu Arg Ala Ala Leu Ser Gly Gly Leu Pro Gln Leu Asn Pro Ser Lys
                        725                          730                          735
   Gly Leu Ser Ser Pro Ala Phe Thr Ala Pro Ala Tyr Lys Arg Val Asp
               740                          745                          750
   Leu Gly Val Met Tyr Lys Trp Leu Asp Pro Asp Asp Ser Phe Ala Gly
           755                          760                          765
   Arg Ser Lys Trp Leu Met Gly Val Lys Gly Ala Tyr Ile Gly Ala Asp
       770                          775                          780
   Leu Phe Asn Leu Phe Asp Met Thr Asn Val Asn Ser Tyr Tyr Trp Val
   785                 790                 795                 800
   Ser Asp Ala Tyr Gln Gln Gln Tyr Ala Val Pro Asn Tyr Leu Thr Arg
                   805                 810                 815
   Arg Gln Phe Asn Leu Arg Leu Leu Val Glu Phe
               820                 825

(2) INFORMATION FOR SEQ ID NO:447

     (i) SEQUENCE CHARACTERISTICS:
          (A) LENGTH: 672 amino acids
          (B) TYPE: amino acid
          (D) TOPOLOGY: linear

     (ii) MOLECULE TYPE: protein

     (iii) HYPOTHETICAL: YES

     (vi) ORIGINAL SOURCE:
          (A) ORGANISM: Porphyromonas gingivalis

     (ix) FEATURE:
          (A) NAME/KEY: misc_feature
          (B) LOCATION 1...672

     (xi) SEQUENCE DESCRIPTION: SEQ ID NO:447

Met Tyr Ser Gly His His Lys Ile His Tyr Pro Phe Leu Ile Leu Leu
1               5                   10                  15
Val Cys Leu Ala Phe Ala Ala Cys Lys Ser Val Lys Leu Lys Asp Ala
            20                  25                  30
Glu Lys Ala His Asp Arg Gln Glu Tyr Thr Lys Ala Ala Asp Met Tyr
        35                  40                  45
Asn Thr Leu Tyr Arg Arg Thr Arg Arg Lys Gln Val Glu Met Lys Ala
    50                  55                  60
Tyr Thr Ala Phe Arg Ser Gly Glu Asn Tyr Arg Ala Ala Gly Arg Gln
65                  70                  75                  80
Ala Lys Ala Leu Arg Gly Tyr Leu Asn Ala Arg Arg Tyr Gly Tyr Pro
                85                  90                  95
Asp Ser Val Val Leu Leu Arg Leu Ala Gln Thr Tyr Gln Gln Gly Gly
            100                 105                 110
Asn Tyr Lys Glu Ala Glu Val Leu Phe Arg Gly Tyr Leu Glu Ala Tyr
        115                 120                 125
Pro Lys Ser Tyr Phe Ala Ala Ile Gly Leu Glu Gly Cys Leu Phe Ala
    130                 135                 140
Arg Gln Gln Lys Glu Tyr Pro Thr Arg Tyr Arg Ile Arg Arg Ala Ala
145                 150                 155                 160
Glu Trp Asn Ser Ala Arg Gly Asp Phe Gly Pro Ala Tyr Ala Pro Asp
                165                 170                 175
Ala Ser Ala Leu Tyr Phe Thr Ser Ser Arg Ser Lys Asp Asp Gly Leu
            180                 185                 190
Asp Asn Ser Ser Ile Thr Gly Leu Lys Pro Asn Asp Ile Tyr Ile Ile
        195                 200                 205
Lys Arg Asp Ala Gln Gly Arg Trp Gly Arg Pro Asp Ser Val Ser Gly
    210                 215                 220
Gly Ile Asn Thr Pro Trp Asp Glu Gly Val Pro Thr Ile Thr Pro Asp
225                 230                 235                 240
Gly Ser Thr Ile Tyr Tyr Thr Leu Ala Gln Gln Gly Ala Asp Tyr Asp
                245                 250                 255
Arg Thr Val Gln Ile Tyr Ser Ala Ala Arg Ser Gly Glu Gly Gly Trp
            260                 265                 270
Ser Asn Gly Ser Leu Val Asp Ile Met Arg Asp Ser Leu Arg Met Ala
        275                 280                 285
Ala His Pro Ser Met Ser Ala Ser Gly Asp Tyr Leu Tyr Phe Val Ser
    290                 295                 300
Asn Ile Gly Gly Ser Tyr Gly Gly Lys Asp Ile Tyr Arg Val Lys Val
305                 310                 315                 320
Ser Asp Arg Ser Tyr Gly Ser Pro Glu Asn Leu Gly Pro Asp Ile Asn
                325                 330                 335
Thr Pro Gly Asp Glu Met Phe Pro Phe Ile Asp Gly Asp Ser Thr Leu
            340                 345                 350
Phe Phe Ala Ser Asp Gly His Ala Gly Leu Gly Gly Leu Asp Ile Phe
        355                 360                 365
Lys Ala Thr Leu Asp Ser Thr Gly Gln Trp His Val Val Asn Met Gly
    370                 375                 380

Gln Pro Val Asn Ser Ser Ala Asp Asp Phe Gly Leu Ala Val Glu Pro
385                 390                 395                 400
Lys Gly Lys Asn Lys Glu Glu Ala Leu Pro Asp Asn Gly Val Lys Gly
                405                 410                 415
Val Phe Cys Ser Asn Arg Gly Asp Ala Arg Gly Trp Pro His Leu Phe
            420                 425                 430
His Phe Glu Leu Pro Ala Ile Tyr Thr Glu Ile Gln Gly Tyr Val Met
        435                 440                 445
Asp Arg Glu Glu Asn Pro Ile Ala Gly Ala Thr Val Arg Ile Val Gly
    450                 455                 460
Glu Arg Gly Pro Val Gly Gln Gly Phe Val Thr Thr Arg Asp Asp Gly
465                 470                 475                 480
Ser Tyr Lys Met Ser Val Gln Gly Asp Thr Arg Tyr Val Met Leu Ala
                485                 490                 495
Gly Ala Ser Gly Tyr Leu Asn Gln Tyr Val Glu Leu Lys Thr Asp Thr
            500                 505                 510
Ala Lys Gln Ser Glu Thr Tyr Tyr Val Asp Phe Phe Leu Ala Ser Arg
        515                 520                 525
Glu Lys Ala Glu Gly Leu Gln Asn Ile Phe Tyr Asp Phe Asp Lys Ala
    530                 535                 540
Thr Leu Arg Pro Glu Ser Met Lys Ser Leu Asp Glu Leu Ile Arg Ile
545                 550                 555                 560
Leu Thr Asp Asn Pro Asp Ile Arg Ile Glu Leu Gly Ser His Ala Asp
                565                 570                 575
Arg Lys Gly Pro Asp Ala Tyr Asn Leu Gly Leu Ser Asp Arg Arg Ala
            580                 585                 590
Lys Ser Val Val Asp Tyr Leu Thr Ser Arg Gly Ile Ala Ala Asp Arg
        595                 600                 605
Leu Thr Trp Lys Gly Tyr Gly Lys Ser Val Pro Lys Thr Val Thr Ala
    610                 615                 620
Lys Ile Ala Glu Arg His Asp Phe Leu Lys Glu Gly Asp Val Leu Thr
625                 630                 635                 640
Glu Glu Phe Val Ala Pro Leu Thr Glu Glu Gln Gln Ser Val Cys Asp
                645                 650                 655
Gln Leu Asn Arg Arg Thr Glu Phe Arg Val Ile Glu Glu Glu Leu Arg
            660                 665                 670

(2) INFORMATION FOR SEQ ID NO:448

    (i) SEQUENCE CHARACTERISTICS:
        (A) LENGTH: 708 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...708

    (xi) SEQUENCE DESCRIPTION: SEQ ID NO:448

Met Lys Lys Phe Phe Phe Ala Leu Leu Ser Ile Gly Ile Ser Ala Gln
1               5                   10                  15
Ala Phe Ala Lys Thr Asp Asn Val Pro Thr Asp Ser Leu Arg Val His
            20                  25                  30
Asn Leu Gln Thr Val Thr Val Tyr Ser Thr Arg Thr Ala Val Pro Leu
        35                  40                  45
Lys Lys Ile Pro Ala Lys Met Glu Leu Ile Ser Ser Arg Asn Ile Lys
    50                  55                  60
Gln Ser Gly Phe Asn Asn Met Thr Asp Ile Leu Lys Thr Gln Ser Ser
65                  70                  75                  80
Leu Asp Val Ile Gln Tyr Pro Gly Phe Ser Ser Asn Ile Gly Ile Arg
                85                  90                  95
Gly Phe Lys Pro Ser Gly Lys Tyr Val Thr Val Leu Val Asn Gly Ile
            100                 105                 110
Pro Ala Gly Thr Asp Asn Ile Ser Thr Leu Asn Thr Ser Asn Ile Glu
        115                 120                 125
Gln Ile Glu Ile Leu Lys Gly Pro Phe Ser Ser Ile Tyr Gly Thr Asn
    130                 135                 140

Ala Met Gly Gly Val Val Asn Ile Ile Thr His Lys Ser Lys Asp Lys
145                 150                 155                 160
Ile His Gly Asn Val Ser Leu Phe Gly Gly Ser Tyr Gln Thr Met Ala
                165                 170                 175
Gly Ser Phe Asn Leu Gly Gly Arg Phe Glu Asp Ile Phe Ser Phe Asp
            180                 185                 190
Leu Ser Leu Gly Leu Asp Lys Gln Asn Lys Asp Tyr Lys Thr Gly Ser
        195                 200                 205
Asn Asn Phe Leu Ser Leu Ser Lys Leu Glu Glu Ala Ile Val Asp Val
    210                 215                 220
Asn Ala Thr Lys Asn Lys Lys Met Lys Gly Ser Asp Tyr Thr Val Ala
225                 230                 235                 240
Thr Gly Arg Leu Arg Phe Gly Ile Asp Phe Thr Pro Glu Trp Ser Leu
                245                 250                 255
Asn Leu Tyr Gln Asn Val Phe Leu Gly Asp Ala Ile Pro Val Gly Gly
            260                 265                 270
Ser Ile Trp Gly Val Tyr Gly Glu Ser Lys Lys Asn Leu Asn Arg Ser
        275                 280                 285
Ser Thr Ser Phe Glu Leu Leu Gly Lys His Gly Cys His Thr Leu Gln
    290                 295                 300
Phe Ser Pro Tyr Phe Asn Ile Glu Lys Ser Glu Asn Tyr Asn Asn Ala
305                 310                 315                 320
Asp Pro Thr Gly Phe Ile Asn Tyr Lys Ser Asp Tyr Tyr Thr Tyr Gly
                325                 330                 335
Ala Leu Leu Gln Asp Lys Ile Ser Phe Gly Gly Gln Asn Ile Val Leu
            340                 345                 350
Gly Val Asp Ser Arg Asn Met Thr Met Glu Ser Glu Arg Phe Glu Gln
        355                 360                 365
Ala Gly Val Asn Thr Lys Pro Tyr Asn Pro Gly Tyr Ala Thr Asn Asn
    370                 375                 380
Ile Gly Leu Phe Gly Gln Ala Asn Phe Tyr Leu Leu Asn Asp Ala Leu
385                 390                 395                 400
Ser Ile Ser Ala Gly Ala Arg Ala Asp Phe Met Phe Phe Asp Leu Lys
                405                 410                 415
Ala Asn Glu Tyr Leu Asn Asn Glu Ala Lys Gln Glu Thr His Asn Val
            420                 425                 430
Ile Asn Pro Asn Val Gly Ile Lys Tyr Glu Phe Val Lys Gly Leu Thr
        435                 440                 445
Ala His Gly Thr Phe Gly Ser Ala Phe Ser Ala Pro Asp Ala Phe Gln
    450                 455                 460
Lys Ala Gly Gln Tyr Val Gly Pro Phe Gly Thr Thr Ile Gly Asn Pro
465                 470                 475                 480
Asp Leu Lys Pro Glu Lys Ser Met Thr Trp Asp Phe Gly Ile Gly Tyr
                485                 490                 495
Ser Asn Ala Arg Cys Gly Ile Gln Ala Asp Val Thr Leu Thr Tyr Phe
            500                 505                 510
His Thr Asp His Lys Asp Leu Ile Leu Ser Ser Pro Asp Tyr Ala Asn
        515                 520                 525
Asn Ile Thr Thr Tyr Ile Asn Ala Asp Lys Ala Arg Met Ser Gly Ile
    530                 535                 540
Glu Ala Leu Leu Ser Tyr Asp Phe Gly Ser Leu Phe Ala Asn Lys Phe
545                 550                 555                 560
Ser Leu Arg Ala Phe Ala Asn Ala Thr Ile Met Leu Asn Ser Glu Met
                565                 570                 575
Lys Lys Ser Gln Thr Asp Ala Pro Trp Ser Glu Met Tyr Tyr Val Arg
            580                 585                 590
Lys Gln Asn Ile Thr Phe Gly Ile Glu Tyr Arg Gly Lys Glu Gly Leu
        595                 600                 605
Glu Val Met Leu Asn Gly Arg Phe Met Gly Arg Arg Ile Glu Gln Asn
    610                 615                 620
Trp Tyr Ala Tyr Tyr Pro Glu Val Arg Pro Glu Leu Gln Gln Leu Leu
625                 630                 635                 640
Ala Ala Glu Glu Pro Glu Leu Ala Ala Gln Gly Leu Leu Arg His Pro
                645                 650                 655
Gln Ala Met Val Phe Asn Ala Ser Ala Tyr Tyr His Met Asn Lys Tyr
            660                 665                 670
Leu Thr Phe Gly Val Asn Leu Asn Asn Ile Leu Asp Glu Leu Tyr Thr
        675                 680                 685
Glu Lys Asp Gly Tyr His Met Pro Gly Arg Asn Ile Met Gly Lys Val
    690                 695                 700
Met Val Asn Phe
705

(2) INFORMATION FOR SEQ ID NO:449

(i) SEQUENCE CHARACTERISTICS:
(A) LENGTH: 462 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...462

(xi) SEQUENCE DESCRIPTION: SEQ ID NO:449

Met Asn Arg Phe Ser Asn His Trp Pro Cys Ile Leu Val Gly Phe Val
1               5                   10                  15
Leu Trp Phe Val Ser Ala Ser Arg Thr Val Ala Gln Asn Ala Ser Glu
            20                  25                  30
Thr Thr Val Ser Tyr Asp Thr Asp Thr Ala Val Leu Ser Glu Ala Asp
        35                  40                  45
Val Leu Arg Ile Ala Leu Ser Glu Asn Ala Thr Val Lys Val Ala Asp
    50                  55                  60
Met Asp Val Arg Lys Gln Glu Tyr Ala Arg Arg Ala Ala Arg Ala Asp
65                  70                  75                  80
Leu Phe Pro Lys Val Asp Leu Asn Gly Val Tyr Ser His Thr Leu Lys
                85                  90                  95
Lys Gln Val Leu Tyr Ile Asp Met Pro Gly Phe Ser Ser Ser Glu Gly
            100                 105                 110
Ile Glu Met Gly Arg Thr His Asn Thr Gln Gly Gly Val Asn Val Ser
        115                 120                 125
Met Pro Leu Val Ser Ala Gln Leu Trp Lys Ser Ile Ala Met Thr Gly
    130                 135                 140
Glu Gln Leu Asp Leu Ala Leu Glu Lys Ala Arg Ser Ser Arg Ile Asp
145                 150                 155                 160
Leu Val Ala Glu Val Lys Lys Ala Tyr Leu Ser Val Leu Leu Ala Glu
                165                 170                 175
Asp Ser Tyr Gly Val Phe Lys Arg Ser Tyr Asp Asn Ala Leu Ala Asn
            180                 185                 190
Tyr Lys Asn Ile Ser Asp Lys Phe Asp Arg Gly Leu Val Ala Glu Tyr
        195                 200                 205
Asp Lys Ile Arg Ala Asn Val Gln Val Arg Asn Ile Glu Pro Asn Leu
    210                 215                 220
Leu Gln Ala Gln Asn Ser Val Ala Leu Ala Leu Trp Gln Leu Lys Val
225                 230                 235                 240
Leu Met Ser Met Glu Val Glu Thr Pro Ile Arg Leu Ser Gly Ser Leu
                245                 250                 255
Ser Asp Tyr Lys Glu Gln Val Tyr Thr Gly Tyr Phe Ala Ala Asp Thr
            260                 265                 270
Leu Ile Ser Asn Asn Ser Ser Leu Arg Gln Leu Asp Ile Gln Arg Arg
        275                 280                 285
Leu Ala Val Ser Ala Asp Lys Leu Asn Lys Tyr Ser Phe Leu Pro Thr
    290                 295                 300
Leu Asn Leu Gly Gly Gln Tyr Thr Tyr Ser Leu Asn Ser Asn Asp Ile
305                 310                 315                 320
Lys Phe Trp Gly Glu Gly Gln Arg Trp Thr Pro Phe Ser Thr Ile Ser
                325                 330                 335
Leu Ser Leu Tyr Ile Pro Ile Phe Asn Gly Gly Lys Arg Leu Tyr Asn
            340                 345                 350
Val Lys Gln Ser Ala Leu Ser Ile Arg Gln Ile Asp Leu Gln Arg Arg
        355                 360                 365
His Ile Glu Gln Ser Ile Arg Met Gly Ile Lys Asn Gln Asn Asp Arg
    370                 375                 380
Leu Arg Thr Cys Met Gln Arg Phe Val Ala Ser Glu Glu Ala Val Arg
305                 390                 395                 400
Ser Ala Glu Lys Gly Tyr Gln Ile Ala Glu Lys Arg Tyr Gln Thr Gly
                405                 410                 415
Glu Gly Thr Leu Val Glu Leu Asn Asp Ala Asp Val Ala Leu Leu Gln
            420                 425                 430
Ala Arg Leu Asn Tyr Asn Gln Ala Ile Phe Asp Phe Met Thr Ala Lys
        435                 440                 445
Ala Glu Leu Asp Lys Met Asn Gly Met Gly Ile Pro Glu Gln

450                 455                 460

(2) INFORMATION FOR SEQ ID NO:450

(i) SEQUENCE CHARACTERISTICS:
(A) LENGTH: 492 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...492

(xi) SEQUENCE DESCRIPTION: SEQ ID NO:450

Met Trp Gly Asp Ser His Gly Val Ala Pro Asn Gln Val Arg Arg Thr
1               5                   10                  15
Leu Val Lys Val Ala Leu Ser Glu Ser Leu Pro Pro Gly Ala Lys Gln
            20                  25                  30
Ile Arg Ile Gly Phe Ser Leu Pro Lys Glu Thr Glu Glu Lys Val Thr
        35                  40                  45
Ala Leu Tyr Leu Leu Val Ser Asp Ser Leu Ala Val Arg Asp Leu Pro
    50                  55                  60
Asp Tyr Lys Gly Arg Val Ser Tyr Asp Ser Phe Pro Ile Ser Lys Glu
65                  70                  75                  80
Asp Arg Thr Thr Ala Leu Ser Ala Asp Ser Val Ala Gly Arg Arg Phe
                85                  90                  95
Phe Tyr Leu Ala Ala Asp Ile Gly Pro Val Ala Ser Phe Ser Arg Ser
            100                 105                 110
Asp Thr Leu Thr Ala Arg Val Glu Glu Val Ala Val Asp Gly Arg Pro
        115                 120                 125
Leu Pro Leu Lys Glu Leu Ser Pro Ala Ser Arg Arg Leu Tyr Arg Gly
    130                 135                 140
Tyr Glu Ala Leu Phe Val Pro Gly Asp Gly Gly Ser Arg Asn Tyr Arg
145                 150                 155                 160
Ile Pro Ala Ile Leu Lys Thr Ala Asn Gly Thr Leu Ile Ala Met Ala
                165                 170                 175
Asp Arg Arg Lys Tyr Asn Gln Thr Asp Leu Pro Glu Asp Ile Asp Ile
            100                 185                 190
Val Met Arg Arg Ser Thr Asp Gly Gly Lys Ser Trp Ser Asp Pro Arg
        195                 200                 205
Ile Ile Val Gln Gly Glu Gly Arg Asn His Gly Phe Gly Asp Val Ala
    210                 215                 220
Leu Val Gln Thr Gln Ala Gly Lys Leu Leu Met Ile Phe Val Gly Gly
225                 230                 235                 240
Val Gly Leu Trp Gln Ser Thr Pro Asp Arg Pro Gln Arg Thr Tyr Ile
                245                 250                 255
Ser Glu Ser Arg Asp Glu Gly Leu Thr Trp Ser Pro Pro Arg Asp Ile
            260                 265                 270
Thr His Phe Ile Phe Gly Lys Asp Cys Ala Asp Pro Gly Arg Ser Arg
        275                 280                 285
Trp Leu Ala Ser Phe Cys Ala Ser Gly Gln Gly Leu Val Leu Pro Ser
    290                 295                 300
Gly Arg Ile Thr Phe Val Ala Ala Ile Arg Glu Ser Gly Gln Glu Tyr
305                 310                 315                 320
Val Leu Asn Asn Tyr Val Leu Tyr Ser Asp Asp Glu Gly Asp Thr Trp
                325                 330                 335
Gln Leu Ser Asp Cys Ala Tyr Arg Arg Gly Asp Glu Ala Lys Leu Ser
            340                 345                 350
Leu Met Pro Asp Gly Arg Val Leu Met Ser Ile Arg Asn Gln Gly Arg
        355                 360                 365
Gln Glu Ser Arg Gln Arg Phe Phe Ala Leu Ser Ser Asp Asp Gly Leu
    370                 375                 380
Thr Trp Glu Arg Ala Lys Gln Phe Glu Gly Ile His Asp Pro Gly Cys
385                 390                 395                 400
Asn Gly Ala Met Leu Gln Val Lys Arg Asn Gly Arg Asp Gln Val Leu
                405                 410                 415
His Ser Leu Pro Leu Gly Pro Asp Gly Arg Arg Asp Gly Ala Val Tyr

420                 425                 430
Leu Phe Asp His Val Ser Gly Arg Trp Ser Ala Pro Val Val Val Asn
        435                 440                 445
Ser Gly Ser Ser Ala Tyr Ser Asp Met Thr Leu Leu Ala Asp Gly Thr
    450                 455                 460
Ile Gly Tyr Phe Val Glu Glu Gly Asp Glu Ile Ser Leu Val Phe Ile
465                 470                 475                 480
Arg Phe Val Leu Asp Asp Leu Phe Asp Val Arg Gln
                485                 490

(2) INFORMATION FOR SEQ ID NO:451

     (i) SEQUENCE CHARACTERISTICS:
          (A) LENGTH: 245 amino acids
          (B) TYPE: amino acid
          (D) TOPOLOGY: linear

    (ii) MOLECULE TYPE: protein

   (iii) HYPOTHETICAL: YES

    (vi) ORIGINAL SOURCE:
          (A) ORGANISM: Porphyromonas gingivalis

    (ix) FEATURE:
          (A) NAME/KEY: misc_feature
          (B) LOCATION 1...245

    (xi) SEQUENCE DESCRIPTION: SEQ ID NO:451

Met Lys Lys Glu Lys Leu Trp Ile Ala Ile Val Ala Gly Leu Ala Phe
1               5                   10                  15
Val Leu Gly Leu Tyr Ala Leu Gly Arg Ser Val Ala Gln Leu Arg Arg
            20                  25                  30
Ser Gln Pro Ser Val Thr Val Thr Gly Met Ala Glu Arg Asn Phe Lys
        35                  40                  45
Ser Asp Leu Ile Val Trp Thr Ala Ser Tyr Gln Leu Gln Met Met Asp
    50                  55                  60
Leu Glu Ser Ala Tyr Lys Ala Leu Lys Glu Lys Gln Ile Leu Val Ala
65                  70                  75                  80
Asp Tyr Leu Lys Asn Lys Gln Leu Pro Asp Ser Ser Tyr Ile Phe Ser
                85                  90                  95
Ser Val Ala Ile Ser Lys Glu Tyr Asn Tyr Tyr Tyr Asp Pro Arg Gln
            100                 105                 110
Glu Gln Asn Val Arg Thr Phe Ala Gly Tyr Leu Leu Ser Gln Thr Val
        115                 120                 125
Thr Val Thr Ser Gln Asp Ile Glu His Val Glu Lys Ile Ser Arg Asp
    130                 135                 140
Ile Thr Glu Leu Ile Asn Gln Gly Val Glu Ile Thr Ser Asp Arg Pro
145                 150                 155                 160
Ala Tyr Tyr Tyr Thr Lys Leu Asn Asp Leu Lys Val Glu Met Leu Arg
                165                 170                 175
Asn Ala Ser Glu Asp Ala Phe Asn Arg Ala Ser Val Ile Ala Glu Gly
            180                 185                 190
Ser Gly Ser Ser Val Gly Lys Met Leu Ser Ser Ser Met Gly Val Phe
        195                 200                 205
Gln Ile Val Gly Leu Asn Ser Asn Glu Asp Tyr Ser Trp Gly Gly Ser
    210                 215                 220
Phe Asn Thr Ser Ser Lys Met Lys Thr Ala Ser Ile Thr Val Lys Ala
225                 230                 235                 240
Ser Phe Ala Leu Lys
                245

(2) INFORMATION FOR SEQ ID NO:452

     (i) SEQUENCE CHARACTERISTICS:
          (A) LENGTH: 276 amino acids
          (B) TYPE: amino acid
          (D) TOPOLOGY: linear

    (ii) MOLECULE TYPE: protein

   (iii) HYPOTHETICAL: YES

    (vi) ORIGINAL SOURCE:

(A) ORGANISM: Porphyromonas gingivalis

(ix) FEATURE:
(A) NAME/KEY: misc_feature
(B) LOCATION 1...276

(xi) SEQUENCE DESCRIPTION: SEQ ID NO:452

Met Lys Lys Thr Ile Ala Ile Ile Ala Ser Ala Leu Leu Ala Leu Gly
1               5                   10                  15
Ala Val Gly Cys Lys Lys Asn Ala Asp Thr Thr Ala Val Ser Glu Lys
            20                  25                  30
Asp Ser Ile Ala Leu Ser Met Gly Ile Leu Tyr Gly Gln Asp Phe Ala
        35                  40                  45
Asn Gln Phe Glu Met Ser Arg Leu Gln Gly Gln Pro Ile Asp Ser Val
    50                  55                  60
Ala Phe Leu Asp Gly Phe Lys Tyr Gly Ile Asp Thr Thr Arg Phe Ser
65                  70                  75                  80
Tyr Asn Leu Gly Ala Ile Tyr Ala Ser Asn Ile Ala Arg Gln Leu Ala
                85                  90                  95
His Asp Ser Ile Asp Ile Asp Lys Phe Tyr Ala Ala Met Arg Ala Ala
            100                 105                 110
Leu Leu Lys Asp Thr Val Ser Ile Ala Met Lys Pro Ala Asp Ala Gln
        115                 120                 125
Ala Phe Met Gln Arg Ile Gln Ala Lys Lys Gln Arg Glu Asn Asn Met
    130                 135                 140
Lys Gln Phe Gly Gln Asn Ile Glu Lys Gly Asn Glu Tyr Ile Asp Thr
145                 150                 155                 160
Phe Lys Lys Glu Asp Gly Val Thr Val Thr Thr Thr Gly Leu Ala Tyr
                165                 170                 175
Lys Thr Leu Gln Glu Gly Thr Gly Ala Thr Pro Ser Leu Ala Asp Thr
            180                 185                 190
Val Arg Val Lys Tyr Val Gly Thr Leu Val Asp Gly Lys Glu Phe Asp
        195                 200                 205
Lys Asn Glu Glu Gly Ile Glu Phe Ala Val Thr Gly Val Ile Lys Gly
    210                 215                 220
Trp Thr Glu Met Leu Gln Leu Met Lys Val Gly Gln Lys Val Arg Val
225                 230                 235                 240
Val Ile Pro Gln Glu Leu Ala Tyr Gly Glu Thr Gly Asn Tyr Thr Ile
                245                 250                 255
Glu Pro Phe Ser Thr Leu Thr Phe Glu Met Glu Leu Ile Gly Ile Lys
            260                 265                 270
Pro Gly Lys Lys
        275

(2) INFORMATION FOR SEQ ID NO:453

(i) SEQUENCE CHARACTERISTICS:
(A) LENGTH: 775 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...775

(xi) SEQUENCE DESCRIPTION: SEQ ID NO:453

Met Lys Val Leu Arg Gln Val Phe Leu Pro Ile Leu Phe Val Leu Leu
1               5                   10                  15
Thr Gly Ala Cys Ser Thr Thr Lys Asn Leu Pro Glu Gly Glu Gln Leu
            20                  25                  30
Tyr Ile Gly Met Gly Lys Thr Gln Ile Leu Arg Gln Asp Lys Ser His
        35                  40                  45
Ala Gly Gln Gln Ala Leu Thr Glu Val Glu Ser Thr Leu Lys Val Thr
    50                  55                  60
Pro Asn Gly Ala Ile Phe Gly Ser Ala Ser Ala Ser Leu Pro Lys Ile
65                  70                  75                  80

Pro Phe Gly Leu Trp Leu Tyr Asn Ser Phe Val Gly Asp Ser Thr Val
                85                  90                  95
Ile Ser Lys Trp Ile Phe Asp Lys Phe Ala Ala Lys Pro Val Phe Ile
            100                 105                 110
Ser Gln Val Lys Ser Asp Ser Arg Ala Lys Val Ala Thr Asn Ile Leu
        115                 120                 125
Arg Glu His Gly Tyr Phe Asp Ala Lys Val Lys Ser Ser Val Thr Thr
    130                 135                 140
Leu Lys Lys Asp Ser Leu Lys Ala Lys Ile Ser Tyr Thr Val Asp Met
145                 150                 155                 160
Ala Ser Pro Tyr His Tyr Asp Ser Ile Ile Pro Leu Pro Ile Ser Thr
                165                 170                 175
Phe Pro Asp Ser Ile Leu Ala Tyr Arg Gln Thr Pro Ser Leu Ile Arg
            180                 185                 190
Lys Gly Asp Gln Phe Asn Leu Ala Lys Leu His Glu Glu Arg Gln Thr
        195                 200                 205
Ile Ser Ala Leu Leu Arg Asp Asn Gly Tyr Tyr Tyr Phe Arg Pro Gln
    210                 215                 220
Asp Ile Ile Tyr Glu Ala Asp Thr Leu Leu Val Arg Gly Ala Val Cys
225                 230                 235                 240
Leu Arg Ala Lys Leu Ser Glu Asp Thr Pro Pro Gln Ala Met Arg Pro
                245                 250                 255
Trp Arg Ile Gly Lys Arg Thr Ala Val Leu Leu Gly Met Asn Gly Glu
            260                 265                 270
Ser Pro Thr Asp Ser Leu Glu Val Glu Asp Met Lys Val Leu Tyr Tyr
        275                 280                 285
Arg Lys Met Pro Val Arg Pro Lys Ile Leu Ala Lys Arg Phe Arg Phe
    290                 295                 300
Phe Ser Gly Asn Leu Tyr Arg Gln Lys Asp Asp Glu Thr Thr Arg Lys
305                 310                 315                 320
Ser Leu Ala Arg Leu Gly Ala Phe Ser Val Ile Asp Leu Asn Phe Leu
                325                 330                 335
Gln Arg Asp Ser Ile Ser Gly Leu Leu Asp Val Arg Leu Leu Thr Thr
            340                 345                 350
Leu Asp Lys Pro Trp Asp Ala Ser Leu Glu Thr Leu Phe Thr Ser Lys
        355                 360                 365
Ser Asn Asp Phe Ile Gly Pro Gly Leu Asn Phe Ala Leu Ala Arg Arg
    370                 375                 380
Asn Val Phe Gly Gly Gly Glu Asn Leu Ser Trp Asn Ile Gly Gly Ser
385                 390                 395                 400
Tyr Glu Trp Glu Thr Gly Asn Arg Pro Glu Asn Ser Ser Asn Arg Leu
                405                 410                 415
Ile Asp Ile Asn Ser Tyr Asn Met Asn Thr Ala Val Asn Leu Ser Phe
            420                 425                 430
Pro Ser Ile Val Phe Pro Gly Leu Leu Asp Lys Tyr Tyr Tyr Tyr Pro
        435                 440                 445
Thr Thr Thr Thr Phe Gln Ala Ser Ala Thr Ala Leu Asn Arg Ala His
    450                 455                 460
Tyr Phe Ser Met Tyr Ser Phe Gly Phe Ser Thr Thr Tyr Glu Phe Gln
465                 470                 475                 480
Pro Ser Lys Glu His Arg His Ala Ile Phe Pro Leu Lys Leu Asn Tyr
                485                 490                 495
Asn Leu Leu Gly His Gln Thr Glu Thr Phe Gln Ala Ile Thr Ala Asn
            500                 505                 510
Asn Pro Pro Leu Leu Leu Ser Leu Gln Ser Gln Phe Leu Ala Gln Met
        515                 520                 525
Gly Tyr Ile Tyr Thr Phe Asn Lys Ser Val Ser Glu Lys Ser Pro His
    530                 535                 540
His Leu Trp Met Gln Phe Gly Leu Ser Glu Ala Gly Asn Leu Leu Asn
545                 550                 555                 560
Leu Ile Tyr Leu Ala Ala Gly Lys Lys Tyr Ser Asp Thr Lys Asn Phe
                565                 570                 575
Val Gly Val Pro Phe Ser Gln Phe Ile Lys Ala Thr Gly Glu Leu Arg
            580                 585                 590
Tyr Ser Tyr Thr Ile Asp Arg Asn Gln Ser Leu Ala Thr Arg Phe Gly
        595                 600                 605
Thr Gly Val Ile Tyr Ser Tyr Gly Asn Met Arg Val Ala Pro Tyr Ser
    610                 615                 620
Glu Gln Phe Tyr Val Gly Gly Ala Asn Ser Ile Arg Ala Phe Thr Val
625                 630                 635                 640
Arg Ser Ile Gly Pro Gly Arg Phe Asn Pro Asp Ser Asp Asn Gln Tyr
                645                 650                 655
Ser Tyr Leu Asp Gln Val Gly Glu Phe Lys Leu Glu Ala Asn Val Glu
            660                 665                 670
Tyr Arg Gly Lys Leu Phe Gly Asp Leu His Ala Ala Val Phe Leu Asp

675                 680                 685
Ala Gly Asn Val Trp Leu Leu Arg Glu Asp Ser Ser Arg Pro Gly Gly
    690                 695                 700
Ala Leu Ser Glu Val Gly Ser Val Ser Asn Phe Leu Asn Ser Ile Ala
705                 710                 715                 720
Leu Gly Thr Gly Val Gly Leu Arg Tyr Asp Leu Ala Phe Leu Val Val
                725                 730                 735
Arg Val Asp Val Gly Phe Gly Leu His Leu Pro Tyr Asn Thr Gly Lys
            740                 745                 750
Lys Gly Tyr Tyr Asn Ile Pro Arg Phe Lys Asp Ala Ile Gly Phe His
        755                 760                 765
Leu Ala Val Gly Tyr Pro Phe
    770                 775

(2) INFORMATION FOR SEQ ID NO:454

     (i) SEQUENCE CHARACTERISTICS:
          (A) LENGTH: 774 amino acids
          (B) TYPE: amino acid
          (D) TOPOLOGY: linear

    (ii) MOLECULE TYPE: protein

   (iii) HYPOTHETICAL: YES

    (vi) ORIGINAL SOURCE:
          (A) ORGANISM: Porphyromonas gingivalis

    (ix) FEATURE:
          (A) NAME/KEY: misc_feature
          (B) LOCATION 1...774

    (xi) SEQUENCE DESCRIPTION: SEQ ID NO:454

Met Ser Ser His Ser Val Arg Tyr Leu Ile Gly Ile Ala Gly Cys Leu
1               5                   10                  15
Leu Leu Met Leu Ala Ser Ser Cys Ser Val Thr Arg Tyr Val Pro Asp
            20                  25                  30
Gly Ser Arg Leu Leu Asp Arg Val Thr Ile Ala Ser Glu Thr Gly Ser
        35                  40                  45
Ile Ala Leu Pro Glu Asp Ile Arg Asp Tyr Thr Leu Gln Gln Pro Asn
    50                  55                  60
Tyr Arg Leu Phe Gly Met Thr Arg Trp Leu Leu Arg Val Tyr Ser Ser
65                  70                  75                  80
Ser Asn Pro Asn Ser Asn Ser Trp Trp Asn Arg Ser Leu Arg Lys Met
                85                  90                  95
Gly Glu Pro Pro Val Leu Ile Asp Ser Val Leu Thr Asp Arg Thr Ala
            100                 105                 110
Asn Arg Leu Ala Lys Ala Met Ala Gly Asp Gly Phe Leu Asp Ala Thr
        115                 120                 125
Ala Arg Ala Val Val Asp Thr Gly Leu Tyr Lys Lys Ala Arg Ile Thr
    130                 135                 140
Tyr Leu Ile Gln Pro Gly Ser Arg Tyr Tyr Ile Arg Asn Met Ala Leu
145                 150                 155                 160
Asp Val Lys Asn Pro Leu Leu Pro Pro Val Ala Leu Gly Asn Ser Leu
                165                 170                 175
Pro Ser Ala Tyr Lys Val Gly Ile Ser Glu Gly Ser Pro Leu Ser Pro
            180                 185                 190
Ile Val Leu Asp Glu Glu Arg Lys Ala Ile Ala Arg His Met Arg Asn
        195                 200                 205
Asn Gly Phe Trp Lys Phe Ser Ala Glu Asp Val Tyr Tyr Glu Ala Asp
    210                 215                 220
Thr Thr Val Ser Gly Gly Ser Gly Thr Lys Ser Ala Asp Leu Lys Leu
225                 230                 235                 240
Val Val Asn Gly Ile Gly Arg Tyr Pro Tyr Arg Ile Gly Arg Val Phe
                245                 250                 255
Phe His Ala Asp Tyr Asp Pro Leu Glu Ser Asp Phe Arg Val Gln Glu
            260                 265                 270
Leu Pro Arg Ile Asp Ser Ile Ser Arg Gly Asp Tyr Thr Val Tyr Tyr
        275                 280                 285
Gly Ser Arg Gly Arg Tyr Ile Arg Ala Ser Ala Leu Thr Arg Ser Val
    290                 295                 300
Ser Val Thr Pro Gly Ala Phe Phe Cys Glu Asp Asp Val Glu Arg Ser
305                 310                 315                 320
Tyr Ile Lys Leu Asn Ala Leu Pro Ile Val Arg Asn Val Asn Ile Arg

                    325                     330                     335
Phe Val Glu His Asn Gly Lys Asp Glu Ile Ala Leu Ala Asp Ser Ser
                340                     345                     350
Arg Leu Val Asp Cys Tyr Ile Leu Thr Val Pro Ala Lys Ser Lys Ser
            355                     360                     365
Phe Glu Ala Glu Val Leu Gly Thr Asn Ser Ala Gly Asp Phe Gly Ala
    370                     375                     380
Ala Leu Ser Leu Gly Phe Thr Asp Arg Asn Leu Phe Arg Gly Ala Glu
385                     390                     395                     400
Met Phe Asn Ile Lys Leu Lys Gly Ala Tyr Glu Ala Ile Arg Lys Gly
                405                     410                     415
Ser His Ser Phe Met Glu Tyr Gly Val Glu Ser Ser Leu Arg Phe Pro
                420                     425                     430
Arg Leu Leu Phe Pro Phe Ile Ser Asp Glu Thr Arg Arg Arg Leu Arg
            435                     440                     445
Ala Ser Thr Glu Trp Lys Ile Gly Tyr Asn Tyr Gln Thr Arg Pro Glu
    450                     455                     460
Phe Asp Arg Val Ile Leu Ser Ala Gln Leu Asn Tyr Ser Trp Gln Thr
465                     470                     475                     480
Tyr Leu His Asn Arg Leu Arg His Thr Ile Arg Leu Leu Asp Val Asp
                485                     490                     495
Tyr Leu His Leu Pro Tyr Ile Asp Pro Asp Phe Ala Gln Ser Leu Pro
                500                     505                     510
Pro Thr Thr Ala Leu Tyr Asn Tyr Thr Glu Gln Phe Ile Leu Gly Ser
            515                     520                     525
Ala Tyr Ile Leu Asn Tyr Thr Thr Ala Ser Ser Met Glu Arg Thr Val
    530                     535                     540
Ser Asn Pro Phe Thr Ala Arg Phe Ser Ile Gln Thr Ala Gly Asn Leu
545                     550                     555                     560
Leu Gln Ala Ile Ser Tyr Leu Thr Asp Ser Pro Lys Asp Glu His Gly
                565                     570                     575
Leu Tyr Lys Met Phe Gly Leu His Tyr Ala Gln Phe Val Lys Leu Asp
                580                     585                     590
Leu Asp Leu Ala Lys Thr Val Leu Leu Glu Lys Asp Asn Thr Leu Ala
            595                     600                     605
Leu His Leu Gly Phe Gly Leu Ala Phe Pro Tyr Gly Asn Ala Arg His
    610                     615                     620
Ile Pro Phe Glu Leu Arg Tyr Phe Ala Gly Gly Ser Asn Ser Val Arg
625                     630                     635                     640
Gly Trp Ser Val Arg Thr Leu Gly Pro Gly Ser Met Lys Met Thr Pro
                645                     650                     655
Asp Lys Thr Phe Phe Asp Gln Met Gly Asp Ile Arg Leu Asp Leu Asn
                660                     665                     670
Val Glu Tyr Arg Thr Lys Leu Phe Trp Lys Phe Arg Ala Ala Ala Phe
            675                     680                     685
Val Asp Ala Gly Asn Val Trp Thr Ile Lys Glu Tyr Glu Asn Gln Glu
    690                     695                     700
Asp Gly Leu Phe Arg Phe Asp Arg Phe Tyr Lys Glu Ile Ala Leu Ala
705                     710                     715                     720
Tyr Gly Leu Gly Leu Arg Leu Asp Phe Asp Tyr Phe Leu Val Arg Leu
                725                     730                     735
Asp Ala Gly Leu Lys Ala Tyr Asp Pro Gln Gln Thr Gly Arg Tyr Lys
                740                     745                     750
Trp Ala Ile Thr Arg Pro Asn Leu Ser Ser Asn Phe Ala Trp His Ile
            755                     760                     765
Ala Val Gly Tyr Pro Phe
    770

(2) INFORMATION FOR SEQ ID NO:455

    (i) SEQUENCE CHARACTERISTICS:
        (A) LENGTH: 867 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...867

(xi) SEQUENCE DESCRIPTION: SEQ ID NO:455

Met Arg Lys Arg Ile Leu Gln Leu Phe Leu Thr Ala Leu Leu Leu Ala
1               5                   10                  15
Leu Gly Ser Ser Leu Ala Ile Ala Gln Thr Val Val Thr Gly Lys Val
            20                  25                  30
Ile Asp Ser Glu Thr Ser Glu Pro Leu Ile Gly Val Ser Val Ser Thr
        35                  40                  45
Gly Gln Gly Ala Ser Leu Arg Gly Val Thr Thr Asp Met Asp Gly Gly
    50                  55                  60
Phe Arg Phe Glu Val Pro Ala Lys Ser Val Leu Thr Phe Arg Cys Val
65                  70                  75                  80
Gly Tyr Ala Thr Val Thr Arg Ser Ile Gly Arg Gly Ser Gln Glu Asp
                85                  90                  95
Leu Gly Thr Ile Leu Leu Asp Pro Gln Ala Ile Gly Leu Asp Glu Ile
            100                 105                 110
Gln Val Ile Ala Ser Val Val Pro Lys Asp Arg Met Thr Pro Val Pro
        115                 120                 125
Val Ser Asn Ile Arg Val Ala Asp Ile Gln Ala Ala Ser Leu Asn Val
    130                 135                 140
Glu Phe Pro Glu Leu Val Lys Ser Thr Pro Ser Thr Tyr Thr Thr Lys
145                 150                 155                 160
Gly Ser Gly Gly Phe Gly Asp Gly Arg Thr Asn Val Arg Gly Phe Asp
                165                 170                 175
Thr Tyr Asn Phe Gly Val Leu Ile Asn Gly Val Pro Val Asn Gly Met
            180                 185                 190
Glu Asp Gly Lys Val Tyr Trp Ser Asn Trp Ser Gly Leu Met Asn Gln
        195                 200                 205
Ala Ser Thr Ile Gln Ile Gln Arg Gly Leu Gly Ala Ser Lys Leu Gly
    210                 215                 220
Ile Ser Ser Val Gly Gly Thr Met Asn Ile Ile Thr Lys Thr Thr Asp
225                 230                 235                 240
Ala Asn Thr Gly Gly Ser Ala Tyr Val Gly Met Gly Asn Asp Gly Leu
                245                 250                 255
His Lys Glu Ser Phe Ser Ile Ser Thr Gly Met Asn Asp Gly Trp Ala
            260                 265                 270
Ile Thr Ile Ala Gly Ser His Met Thr Gly Leu Gly Tyr Val Lys Gly
        275                 280                 285
Leu Lys Gly Arg Ala Phe Ser Tyr Phe Phe Asn Val Ser Lys Lys Phe
    290                 295                 300
Asn Glu Arg His Thr Leu Ser Leu Thr Gly Phe Gly Ala Pro Gln Trp
305                 310                 315                 320
His Asn Gln Arg Ser Ser Lys Tyr Ser Val Ala Asp Tyr Asp Lys Tyr
                325                 330                 335
Gly Ile Arg His Asn Gln Ser Phe Gly Tyr Leu Arg Gly Glu Leu Thr
            340                 345                 350
Pro Thr Ala Tyr Ala Tyr Asn Thr Tyr His Lys Pro Gln Phe Ser Leu
        355                 360                 365
Asn His Phe Trp Lys Met Asp Glu Asn Thr Ser Leu Tyr Thr Ala Unk
    370                 375                 380
Tyr Ala Ser Leu Ala Thr Gly Gly Gly Arg Arg Ala Tyr Gly Lys Asn
385                 390                 395                 400
Ser Lys Trp Val Leu Ile Asn Tyr Asn Thr Gly Gln Pro Tyr Glu Gln
                405                 410                 415
Thr Lys Val Thr Pro Asp Gly Leu Ile Asp Tyr Asp Ala Val Leu Ala
            420                 425                 430
Ala Asn Ala Ala Ala Ser Asn Gly Ser Glu Ala Ile Phe Ala Leu Gly
        435                 440                 445
Ser Asn Ser His Lys Trp Phe Gly Leu Leu Ser Ser Phe Lys Lys Lys
    450                 455                 460
Leu Asn Ser Ser Leu Thr Leu Thr Ala Gly Tyr Asp Gly Arg Tyr Tyr
465                 470                 475                 480
Arg Gly Asp His Tyr Asp Lys Ile Thr Asp Leu Leu Gly Gly Ser Tyr
                485                 490                 495
Tyr Ile Glu Asp Pro Lys Thr Lys Leu Ala Tyr His Ala Glu Gly Gln
            500                 505                 510
Gln Leu Lys Val Gly Asp Ile Val Asn Arg Asp Tyr Thr Gly Glu Ile
        515                 520                 525
Met Trp His Gly Leu Phe Ala Gln Met Glu His Ser Ser Glu Trp Ile
    530                 535                 540
Asp Ala Phe Val Ser Gly Ser Ile Asn Tyr Glu Leu Tyr Arg Asn His
545                 550                 555                 560
Asn Tyr Gly Gly Ser Lys Ser Thr Gly Tyr Leu Pro Gly Val Ser Pro
                565                 570                 575

Trp Lys Ser Phe Leu Pro Trp Ser Gly Lys Ala Gly Leu Ser Tyr Lys
580 585 590
Phe Ala Gln Gly His Asn Val Phe Ala Asn Gly Gly Phe Phe Thr Arg
595 600 605
Ala Pro Leu Phe Gly Asn Ile Tyr Ala Ala Gly Ala Ile Ile Pro Asn
610 615 620
Asp Lys Ala Asn Met Glu Lys Val Leu Thr Gly Glu Val Gly Tyr Gly
625 630 635 640
Phe Thr Asn His Lys Asn Phe Glu Phe Asn Ile Asn Gly Tyr Tyr Thr
645 650 655
Lys Trp Met Asp Arg Val Thr Ser Lys Arg Ile Gly Asn Glu Tyr Val
660 665 670
Tyr Leu Asn Gly Val Asp Ala Val His Cys Gly Val Glu Ala Glu Val
675 680 685
Ser Tyr Arg Pro Ile Arg Gln Ile Asp Leu Arg Gly Met Phe Ser Leu
690 695 700
Gly Asp Trp Thr Trp Gln Asn Asn Val Ser Tyr Thr Ser Tyr Asp Glu
705 710 715 720
Ala Gly Asn Glu Thr Gly Gln Asp Ile Thr Tyr Ile Lys Gly Leu His
725 730 735
Val Gly Asp Ala Ala Gln Met Thr Ala Ala Val Ser Ala Asp Ile Glu
740 745 750
Leu Phe Lys Gly Phe His Val Ile Gly Lys Tyr Asn Phe Leu Gly Lys
755 760 765
Asn Tyr Ala Gly Phe Asn Pro Ala Thr Arg Asn Ala Gln Gln Tyr Glu
770 775 780
Ala Asp Gly Lys Glu Ile Val Glu Ser Trp Lys Leu Pro Asp Val Gly
785 790 795 800
Leu Phe Asp Leu Ser Ala Ser Tyr Asn Phe Lys Leu Gly Ser Leu Ser
805 810 815
Thr Thr Phe Tyr Phe Asn Met Asp Asn Val Ala Asp Lys Arg Tyr Val
820 825 830
Ser Asp Ala Asp Asp Asn Ile Ile Gly Lys Lys His Asp Glu Ala Ser
835 840 845
Ala Leu Val Trp Tyr Gly Phe Gly Arg Thr Trp Ser Thr Gly Ile Arg
850 855 860
Val Asn Phe
865

(2) INFORMATION FOR SEQ ID NO:456

(i) SEQUENCE CHARACTERISTICS:
(A) LENGTH: 431 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...431

(xi) SEQUENCE DESCRIPTION: SEQ ID NO:456

Met Lys Phe Ser Ile Arg Leu Phe Leu Cys Ile Ile Phe Leu Leu Ser
1               5                   10                  15
Ala Phe Ile Leu Pro Ala Leu Gly Gln Lys Ser Lys Gln Val Gln Arg
            20                  25                  30
Leu Glu Lys Gln Arg Lys Glu Ala Leu Lys Ala Ile Glu Lys Thr Asp
        35                  40                  45
Arg Glu Leu Arg Asn Thr Lys Lys Asp Lys Gln Asp Lys Gln Lys His
    50                  55                  60
Leu Asn Leu Leu Asn Lys Gln Val Ala Gln Arg Lys Gln Met Val Gln
65                  70                  75                  80
Leu Leu Asp Asn Glu Val Lys Glu Leu Gln Ser Asp Ile Asp Ser Met
                85                  90                  95
Thr Gly Val Cys His Gln Leu Ser Val Glu Glu Lys Ala Arg Ser Asp
            100                 105                 110
Glu Tyr Ala Gln Ala Leu Gln Ser Met Gln Lys Arg Lys Arg Ser Leu
        115                 120                 125

Asp Arg Ile Leu Phe Ile Ser Ser Ala Lys Ser Phe Asp Glu Gly Met
    130                 135                 140
Arg Arg Met Arg Phe Leu Glu Gln Tyr Ala Ser Ala Tyr Lys Leu Ala
145                 150                 155                 160
Ser Val Arg Leu Arg Asp Thr Arg Ser Lys Leu Glu Thr Glu Arg Ala
                165                 170                 175
Thr Val Glu Asp Ala Lys Lys Glu Lys Gly His Leu Leu Val Ile Arg
            180                 185                 190
Glu Glu Glu Lys Lys Lys Leu Glu Gly Gln Gln Ala Glu Gln Arg Arg
        195                 200                 205
Gln Val Gln Ala Leu Gly Ala Lys Gln Lys Asp Leu Glu Ala Gln Leu
    210                 215                 220
Arg Lys Gln Lys Lys Gln Ala Glu Ala Leu Asn Arg Lys Ile Glu Lys
225                 230                 235                 240
Gln Ile Ala Lys Glu Ile Glu Ala Ala Glu Arg Arg Ala Arg Glu Glu
                245                 250                 255
Arg Glu Arg Leu Ala Arg Glu Ala Lys Ala Lys Gly Lys Pro Val Pro
            260                 265                 270
Ala Glu Pro Glu Arg Lys Ala Glu Thr Lys Gly Gly Tyr Ala Met Asp
        275                 280                 285
Ala Ser Glu Arg Ala Leu Ser Gly Ser Phe Ala Gln Asn Lys Gly Arg
    290                 295                 300
Leu Pro Gly Pro Val Arg Gly Arg Tyr Arg Ile Val Ser Asp Phe Gly
305                 310                 315                 320
Val His Gln His Ser Glu Leu Lys Lys Val Gln Val Asn Asn Gly Gly
                325                 330                 335
Ile Asp Ile Ala Val Ala Thr Gly Ser Asp Ala Thr Ser Val Phe Asp
            340                 345                 350
Gly Val Val Ser Ser Val Phe Val Ile Pro Gly Tyr Asn Ser Ala Val
        355                 360                 365
Met Val Arg His Gly Asn Tyr Ile Thr Val Tyr Ala Asn Leu Ser Lys
    370                 375                 380
Val Tyr Val Asn Ser Gly Thr Arg Val Lys Thr Gly Gln Ala Leu Gly
385                 390                 395                 400
Arg Ala Tyr Thr Asp Pro Ser Asn Asn Gln Thr Ile Ile His Phe Glu
                405                 410                 415
Ile Trp Lys Glu Arg Ser Lys Gln Asn Pro Arg Leu Trp Leu Arg
            420                 425                 430

(2) INFORMATION FOR SEQ ID NO:457

    (i) SEQUENCE CHARACTERISTICS:
        (A) LENGTH: 333 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...333

    (xi) SEQUENCE DESCRIPTION: SEQ ID NO:457

Met Lys Lys Tyr Leu Leu Tyr Ala Ser Leu Leu Thr Ser Val Leu Leu
1               5                   10                  15
Phe Ser Cys Ser Lys Asn Asn Pro Asn Glu Pro Val Glu Asp Arg Ser
            20                  25                  30
Ile Glu Ile Ser Ile Arg Val Asp Asp Phe Thr Lys Thr Gly Glu Ala
        35                  40                  45
Val Arg Tyr Glu Arg Asn Gln Gly Ser Ala Ala Glu Arg Leu Ile Thr
    50                  55                  60
Asn Leu Tyr Leu Leu Leu Phe Asp Gln Ser Gly Ala Asn Pro Ala Lys
65                  70                  75                  80
Tyr Tyr Ile Thr Gly Asn Thr Phe Thr Gly Gly Thr Trp Leu Pro Asp
                85                  90                  95
Asp Met Lys Val Lys Leu Asp Met Thr Gln Ser Glu Ala Gly Glu Arg
            100                 105                 110
Lys Val Tyr Val Val Ala Asn Val Asp Asn Ala Val Lys Thr Ala Leu
        115                 120                 125

Asp Ala Val Ala Asn Glu Ser Asp Leu Gln Thr Val Lys Arg Thr Thr
    130                 135                 140
Ala Met Pro Trp Ser Thr Asp Ile Ala Ser Pro Phe Leu Met Ser Gly
145                 150                 155                 160
Asn Lys Thr His Asp Phe Leu Ala Asn Arg Leu Leu Asp Asn Val Pro
                165                 170                 175
Leu Val Arg Ala Ile Ala Lys Val Glu Leu Asn Ile Ser Leu Ser Glu
            180                 185                 190
Lys Phe Gln Ile Val Pro Ile Ile Val Asn Gly Ser Leu Ser Glu Phe
        195                 200                 205
Lys Phe Arg Tyr Val Asn Phe Asp Lys Glu Thr Tyr Val Val Lys Pro
    210                 215                 220
Thr Thr Lys Pro Asp Asn Leu Ile Ser Ser Ala Asn Gly Val Trp Pro
225                 230                 235                 240
Gln Ile Thr Asp Trp Thr Val Trp Gly Ala Ser Leu Asn Thr Ser Pro
                245                 250                 255
Ala Pro Asp Ala Gly Thr Gly Tyr Thr Leu Asp Ala Asn Gly Lys Val
            260                 265                 270
Thr Ala Leu Arg Ile Val Thr Tyr Leu Asn Glu Arg Asp Ser Lys Gly
        275                 280                 285
Ala Thr Val Glu Val Ala Leu Pro Arg Val Asp Asp Gly Thr Leu Pro
    290                 295                 300
Pro Pro Glu Phe Gly Pro Glu Leu Tyr Arg Leu Pro Leu Pro Asp Lys
305                 310                 315                 320
Ile Leu Arg Asn His Trp Tyr Lys Tyr Glu Val Glu Ile
                325                 330

(2) INFORMATION FOR SEQ ID NO:458

    (i) SEQUENCE CHARACTERISTICS:
        (A) LENGTH: 315 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...315

    (xi) SEQUENCE DESCRIPTION: SEQ ID NO:458

Met Ile Arg Thr Ile Leu Ser Arg Tyr Val Ser Ser Asn Phe Trp Ser
1               5                   10                  15
Arg Gly Ala Thr Phe Phe Phe Thr Ile Phe Pro Ala Phe Ile Leu Ala
            20                  25                  30
Ala Thr Ala Leu Pro Ala Cys Gly Gly Gly Thr Ala Ser Gly Ser Asp
        35                  40                  45
Arg Thr Leu Ala Val Thr Ile Glu Pro Gln Lys Tyr Phe Ile Glu Ser
    50                  55                  60
Ile Ala Asp Lys Ser Val Gln Val Val Ala Leu Val Pro Ala Gly Ser
65                  70                  75                  80
Asn Pro Glu Glu Tyr Asp Pro Ser Pro Thr Val Met Lys Arg Leu Ser
                85                  90                  95
Glu Ala Asp Ala Tyr Phe Tyr Ile Gly Gly Leu Gly Phe Glu Gln Arg
            100                 105                 110
Asn Leu Ala Ala Ile Arg Asp Asn Asn Pro Lys Leu Pro Leu Phe Glu
        115                 120                 125
Met Gly Lys Ala Leu Ala Asp Ala Gly Ser Ala Asp Leu His Gly Ser
    130                 135                 140
Cys Thr Asp His Ser His Thr Asp Leu His Ala His Asp Pro His Tyr
145                 150                 155                 160
Trp Ser Ser Val Val Gly Ala Lys Ala Leu Ser Arg Ala Ala Tyr Asp
                165                 170                 175
Ala Leu Val Glu Leu Tyr Pro Asn Glu Lys Asp Lys Trp Asp Lys Gly
            180                 185                 190
His Asp Arg Leu Asn Gly Arg Ile Asp Ser Val Lys Arg Leu Val Asp
        195                 200                 205
Thr Met Phe Ala Asn Gly Lys Ala Asp Lys Ala Phe Val Ile Tyr His
    210                 215                 220

Pro Ser Leu Ser Phe Phe Ala Gln Glu Phe Gly Leu Arg Gln Ile Val
225                 230                 235                 240
Ile Glu Glu Asp Gly Lys Glu Pro Thr Ala Ala His Leu Arg Arg Val
                245                 250                 255
Ile Asp Gln Ala Arg Ala Asp Gly Val Arg Ile Val Phe Ile Gln Pro
            260                 265                 270
Glu Phe Glu Thr Arg Gln Ala Glu Asp Ile Ala Arg Glu Ile Gly Ala
        275                 280                 285
Arg Pro Val Arg Ile Asn Pro Leu Arg Ser Ser Trp Glu Glu Glu Ile
    290                 295                 300
Leu His Ile Ala Arg Ala Leu Ala His Glu Arg
305                 310                 315

(2) INFORMATION FOR SEQ ID NO:459

(i) SEQUENCE CHARACTERISTICS:
(A) LENGTH: 848 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...848

(xi) SEQUENCE DESCRIPTION: SEQ ID NO:459

Met Ile Gly Lys Lys Ile Phe Phe Ile Leu Leu Ala Leu Ile Ala Phe
1               5                   10                  15
Ser Gly Leu Asn Ala Ala Thr Asp Thr Glu Phe Lys Tyr Pro Thr Asp
            20                  25                  30
Ala Asn Ile Ile Gly His Val Lys Asp Ser Lys Thr Gly Glu His Leu
        35                  40                  45
Val Gly Ile Thr Ile Ala Ile Lys Gly Thr Thr Phe Gly Thr Ser Thr
    50                  55                  60
Asp Ala Thr Gly His Tyr Tyr Leu Arg Asn Leu Arg Pro Gly Glu Ile
65                  70                  75                  80
Thr Leu Ile Met Arg Gly Met Gly Tyr Lys Ser Gln Glu Arg Val Val
                85                  90                  95
Arg Val Glu Lys Asp Lys Thr Ile Glu Val Asn Phe Glu Ala Glu Glu
            100                 105                 110
Asp Ala Ile Asn Leu Asp Glu Val Val Ile Ser Ala Asn Arg Glu Leu
        115                 120                 125
Thr Leu Arg Arg Leu Ala Pro Thr Leu Val Asn Val Leu Asn Glu Lys
    130                 135                 140
Val Phe Ser Gln Val Asn Ala Ser Asn Leu Ala Gln Gly Leu Ser Phe
145                 150                 155                 160
Gln Pro Gly Val Arg Val Glu Asn Asn Cys Gln Asn Cys Gly Phe Asn
                165                 170                 175
Gln Val Arg Ile Asn Gly Leu Asp Gly Arg Tyr Ala Gln Ile Leu Ile
            180                 185                 190
Asp Ser Arg Pro Ile Met Ser Ala Leu Ala Gly Val Tyr Gly Leu Glu
        195                 200                 205
Gln Ile Pro Ala Asn Met Ile Glu Arg Val Glu Val Val Arg Gly Gly
    210                 215                 220
Gly Ser Ala Leu Tyr Gly Ser Ser Ala Ile Ala Gly Val Val Asn Ile
225                 230                 235                 240
Ile Thr Lys Glu Pro Ser His Asn Ser Phe Thr Phe Asn Glu Ser Leu
                245                 250                 255
Ser Phe Thr Gly Phe Ser Lys Leu Asp Asn Asn Thr Asn Phe Asn Ala
            260                 265                 270
Ser Ile Val Ser Asp Asp Asn Arg Ala Gly Ala Met Val Phe Gly Gln
        275                 280                 285
Ala Arg Tyr Arg Asn His Trp Asp Ala Asn Asn Asp Gly Tyr Ser Glu
    290                 295                 300
Leu Gly Lys Ile Asp Ala Arg Ser Leu Gly Ala His Ser Tyr Leu Arg
305                 310                 315                 320
Leu Ser Asp Tyr Ser Lys Leu Thr Gly Glu Phe His Thr Ile Ser Glu
                325                 330                 335

Phe Arg Arg Gly Gly Asp Arg Ile Asp Leu Pro Pro His Val Val Gly
            340                 345                 350
Val Ala Glu Gln Thr Asp His Ser Val Phe Ser Gly Asn Leu Lys Tyr
        355                 360                 365
Asp Leu Phe Ser Ser Asn Tyr Lys His His Phe Gln Ala Tyr Thr Ser
    370                 375                 380
Gly Gln Ile Val Asn Arg Lys Ser Tyr Tyr Gly Gly Ile Gly Glu Ile
385                 390                 395                 400
Asp Val Asn Gly His Pro Gly Gly Thr Glu Gly Tyr Pro Ile Pro Gln
                405                 410                 415
Asp Gln Tyr Gly Asn Asn Tyr Gly Val Thr Lys Gly Lys Thr Tyr Met
            420                 425                 430
Gly Gly Ile Gln Tyr Ser Tyr Asp Leu Asp Lys Phe Leu Leu Met Pro
        435                 440                 445
Ser Gln Leu Leu Phe Gly Ala Glu Tyr Thr Arg Asp Glu Leu Asn Asp
    450                 455                 460
Val Met Pro Ile Leu Ser Trp Gln Thr Gly Glu Asp Ala Asn Gly Asn
465                 470                 475                 480
Thr Ile Pro Leu Tyr Pro Glu Leu Asp Gln Asn Ile Asn Asn Tyr Ser
                485                 490                 495
Leu Phe Gly Gln Asn Glu Trp Lys Asn Asp Arg Trp Ser Ile Leu Val
            500                 505                 510
Gly Ala Arg Leu Asp Lys His Ser Glu Val Lys Asp Met Ile Leu Ser
        515                 520                 525
Pro Arg Thr Thr Leu Arg Phe Asn Val Asn Pro Asp Ile Asn Leu Arg
    530                 535                 540
Ala Thr Tyr Ala Lys Gly Phe Arg Ala Pro Gln Val Phe Asp Glu Asp
545                 550                 555                 560
Leu His Val Gly Val Val Gly Gly Glu Ala Gln Lys Val Phe Asn Asp
                565                 570                 575
Pro Asn Leu Lys Pro Glu Ile Ser His Ala Phe Ser Leu Ser Ala Asp
            580                 585                 590
Met Tyr His Arg Phe Gly Asn Val Gln Thr Asn Phe Leu Val Glu Gly
        595                 600                 605
Phe Tyr Thr Arg Leu Leu Asp Val Phe Thr Asn Glu Glu Gln Pro Asp
    610                 615                 620
Gln His Asp Gly Ile Lys Arg Tyr Thr Arg Ile Asn Gly Ser Gly Ala
625                 630                 635                 640
Lys Val Phe Gly Leu Asn Leu Glu Gly Lys Val Ala Tyr Lys Ser Phe
                645                 650                 655
Gln Leu Gln Ala Gly Leu Thr Leu Ala Ser Asn Lys Tyr Asp Glu Ala
            660                 665                 670
Gln Glu Trp Gly Leu Asn Thr Val Lys Asp Thr Asn Gly Ala Phe Val
        675                 680                 685
Thr Glu Ala Asn Ala Asn Gly Gln Gln Glu Tyr Lys Asn Glu Ser Met
    690                 695                 700
Thr Asp Thr Gln Ile Thr Arg Thr Pro Ser Val Tyr Gly Tyr Phe Thr
705                 710                 715                 720
Leu Ala Tyr Asn Pro Ala His Ser Trp Asn Ile Ala Leu Thr Gly Ala
                725                 730                 735
Tyr Thr Gly Gln Met Tyr Val Pro His Ala Ile Glu Tyr Gly Val Lys
            740                 745                 750
Ser Ala Glu Leu Asp Ile Met Gln Asn Asn Pro Glu Ile Thr Asp Glu
        755                 760                 765
Thr Gly Lys Ala Pro Arg Ile Asp Glu Leu Lys Lys Thr Pro Ala Phe
    770                 775                 780
Phe Asp Leu Gly Leu Lys Val Gly Tyr Asp Phe His Val Phe Gln Ala
785                 790                 795                 800
Thr Glu Val Gln Leu Tyr Val Gly Met Asn Asn Ile Phe Asn Ser Phe
                805                 810                 815
Gln Lys Asp Phe Asp Arg Gly Ala Ala Arg Asp Ser Gly Tyr Ile Tyr
            820                 825                 830
Gly Pro Thr Gln Pro Arg Thr Gly Tyr Met Gly Leu Val Val Lys Phe
        835                 840                 845

(2) INFORMATION FOR SEQ ID NO:460

    (i) SEQUENCE CHARACTERISTICS:
        (A) LENGTH: 202 amino acids
        (B) TYPE: amino acid
        (D) TOPOLOGY: linear

    (ii) MOLECULE TYPE: protein

    (iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...202

(xi) SEQUENCE DESCRIPTION: SEQ ID NO:460

Met Thr Val Lys Arg Ala Val Arg Ile Ala Leu Leu Thr Leu Ile Gly
1               5                   10                  15
Ile Leu Phe Ser Ser Pro Ser Leu Val Arg Ala Gln Ser Leu Phe Ser
            20                  25                  30
Thr Glu His Val Leu Gln Leu Tyr Asn Lys Ile Leu Tyr Gly Glu Ser
        35                  40                  45
Ala Ala Asp Thr Val Ala Glu Lys Thr Ala Gly Glu Ser Ala Phe Pro
    50                  55                  60
Phe Ile Asp Lys Leu Ile Asn Leu Gly Arg Thr Phe Leu Gly Lys Pro
65                  70                  75                  80
Tyr Arg Tyr Arg Gly Pro Ser Pro Trp Pro Met Asp Cys Ser Gly Tyr
                85                  90                  95
Val Ser Tyr Leu Tyr Ser Lys Phe Asp Ile Lys Leu Pro Arg Gly Ala
            100                 105                 110
Ala Ala Gln Ser Gln Tyr Thr Asn Pro Ile Glu Arg Glu Asp Val Arg
        115                 120                 125
Pro Gly Asp Leu Leu Phe Phe Lys Gly Arg Asn Ala Arg Ser Asn Arg
    130                 135                 140
Ile Gly His Val Ala Leu Val Val Ser Val Asp Glu Asp Asp Ile Thr
145                 150                 155                 160
Met Met His Ser Arg Asn Ser Arg Gly Ile Val Ile Glu Lys Leu Asn
                165                 170                 175
Arg Ser Ala Tyr Phe Ser Arg Arg Leu Val Ser Tyr Gly Arg Val Pro
            180                 185                 190
Gly Ala Lys Arg Val Ile Pro Arg Lys Ser
        195                 200

(2) INFORMATION FOR SEQ ID NO:461

(i) SEQUENCE CHARACTERISTICS:
(A) LENGTH: 455 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...455

(xi) SEQUENCE DESCRIPTION: SEQ ID NO:461
   Met Lys Arg Thr Ile Leu Leu Thr Ala Leu Thr Val Leu Ser Ser Leu
   1               5                   10                  15
   Ser Leu Leu Arg Ala Gln Asn Glu Ser Glu Ala Ser Thr Asn Pro Met
               20                  25                  30
   Ser Gly Leu Ser Leu Glu Asp Cys Ile Arg Ile Ala Lys Glu Arg Asn
           35                  40                  45
   Leu Asn Leu Arg Arg Gln Glu Ile Glu Gln Glu Asn Arg Ile Ile Ser
       50                  55                  60
   Leu Asp Ala Ala Arg His Ser Phe Leu Pro Ser Val Asn Ala Gly Ile
   65                  70                  75                  80
   Gly His Asn Tyr Ser Phe Gly Arg Ser Lys Asp Lys Thr Gly Val Thr
                   85                  90                  95
   Val Asp Arg Ser Ser Met Asn Thr Asn Leu Ser Ile Gly Ala Ser Val
               100                 105                 110
   Glu Val Phe Ser Gly Thr Arg Arg Leu His Asp Leu Lys Gln Gln Lys
           115                 120                 125
   Tyr Asn Val Glu Asp Gly Ile Ala Arg Leu Gln Lys Ala Arg Glu Asp
       130                 135                 140
   Leu Ser Leu Gln Ile Ala Ala Leu Tyr Ile Asn Leu Leu Phe Arg Gln
   145                 150                 155                 160
   Glu Met Thr Arg Thr Ala Glu Thr Gln Leu Ala Leu Ile Arg Glu Gln
                   165                 170                 175
   Arg Asn Arg Thr Ala Glu Met Val Arg Val Gly Lys Trp Ala Glu Gly
               180                 185                 190
   Lys Leu Leu Asp Ile Asn Ala Gln Met Ala Lys Asp Glu Gln Leu Leu
           195                 200                 205
   Val Gln Tyr Arg Ser Glu Glu Glu Leu Ala Arg Leu Asp Leu Gly Gln
       210                 215                 220
   Ala Leu Glu Leu Glu His Pro Glu Ser Ile Ala Val Lys Ala Pro Asp
   225                 230                 235                 240
   Thr Asp Val Leu Val Ala Glu Arg Leu Gly Ser Leu Leu Ala Pro Glu
                   245                 250                 255
   Glu Ile Tyr Arg Thr Ala Leu Gly Leu Lys Pro Ala Leu His Ser Ser
               260                 265                 270
   Glu Leu Gln Ile Ala Ser Ala Arg Glu Gly Leu Ala Ser Ala Arg Ala
           275                 280                 285
   Ala Tyr Phe Pro Thr Leu Ser Leu Ser Ala Gly Tyr Ser Asn Gly Tyr
       290                 295                 300
   Phe Arg Asp Leu Gly Lys Glu Tyr Ala Ala Ile Asn Pro Ser Phe Ser
   305                 310                 315                 320
   Glu Gln Trp Lys Asn Asn Gly Ser Tyr Ser Ile Gly Leu Ser Leu Asn
                   325                 330                 335
   Ile Pro Ile Phe Ser Ala Met Gln Thr Gln Asp Arg Val Arg Ser Ser
               340                 345                 350
   Arg Leu Gln Ile Arg Ser Ser Glu Leu Arg Leu Val Glu Glu Lys Lys
           355                 360                 365
   Ala Leu Tyr Lys Glu Ile Arg Gln Ala Tyr Ser Asn Ala Val Ala Ala
       370                 375                 380
   Asp Lys Ala Ile Ala Ala Ala Glu Asn Ser Lys Ala Ala Thr Leu Lys
   385                 390                 395                 400
   Ala Tyr Glu Tyr Ala Arg Asp Ser Phe Glu Ala Gly Arg Leu Ser Ala
                   405                 410                 415
   Tyr Glu Tyr Ala Glu Ala Lys Thr Lys Tyr Ala Leu Ser Gln Val Glu
               420                 425                 430
   Glu Leu Arg Ala Lys Tyr Asp Phe Ile Tyr Lys Ala Lys Val Leu Asp
           435                 440                 445
   Phe Tyr Gln Gly Lys Asp Phe
       450                 455

(2) INFORMATION FOR SEQ ID NO:462

    (i) SEQUENCE CHARACTERISTICS:
        (A) LENGTH: 444 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...444

    (xi) SEQUENCE DESCRIPTION: SEQ ID NO:462

Met Arg Phe Gln His Tyr Leu Ile Cys Thr Ala Ala Val Ala Ala Leu
1               5                   10                  15
Ala Ala Asn Pro Leu Thr Gly Gln Ser Asn Met Thr Leu Glu Glu Cys
            20                  25                  30
Ile Asp Tyr Ala Arg Arg His Ser Ser Ala Val Ala Leu Ser Ala Ala
        35                  40                  45
Glu Leu Glu Gln Ser Lys Ala Asp Tyr Leu Gln Ala Val Gly Asn Phe
    50                  55                  60
Leu Pro Arg Val Ser Ala Gly Thr Gly Ala Ser Trp Asn Phe Gly Arg
65                  70                  75                  80
Gly Leu Asp Ala Glu Thr Asn Thr Tyr Thr Asp Ile Asn Ser Phe Asn
                85                  90                  95
Asn Ser Tyr Ser Ile His Ala Thr Met Thr Leu Phe Asp Gly Leu Gln
            100                 105                 110

Ser Val Tyr Arg Leu Arg Met Ala His Ala Arg Arg Glu Ala Ser Arg
        115                 120                 125
Leu Ser Val Arg Glu Gln Gln Glu Leu Ala Ala Leu Gly Thr Thr Glu
    130                 135                 140
Ala Tyr Tyr Asp Leu Val Tyr Ala Arg Gln Met Gln Glu Leu Ala Met
145                 150                 155                 160
Gln Lys Tyr Glu Glu Ser Ser Arg Leu His Arg Gln Thr Ala Arg Met
                165                 170                 175
Glu Glu Leu Gly Met Lys Ser Arg Pro Asp Val Leu Glu Met Gln Ser
            180                 185                 190
Arg Met Ala Gly Asp Arg Leu Ala Leu Thr Gln Ala Asp Asn Gln Cys
        195                 200                 205
Ile Ile Ala Leu Ile Arg Leu Lys Glu Lys Met Asn Phe Pro Ile Asp
    210                 215                 220
Asp Glu Leu Val Val Asp Asp Met Pro Ala Asp Ser Leu Ser Ala Asp
225                 230                 235                 240
Met Ala Glu Ser Asp Ser Ser Ala Gly Val Phe Ala Arg Ala Ala His
                245                 250                 255
His His Pro Val Leu Leu Arg Ala Lys Leu Asp Glu Gln Ala Ala Thr
            260                 265                 270
Asp Arg Leu Arg Ala Ala Arg Gly Ala Phe Leu Pro Ser Val Ser Val
        275                 280                 285
Ser Gly Gly Trp Asn Thr Gly Phe Ser Arg Phe Leu Asn Gly Ser Asp
    290                 295                 300
Tyr Thr Pro Phe Ser Glu Gln Phe Arg Asn Arg Arg Gly Glu Tyr Val
305                 310                 315                 320
Ser Leu Asn Leu Ser Ile Pro Ile Phe Ser Gly Phe Ser Leu Val Ser
                325                 330                 335
His Leu Arg Gln Ala Arg Ala Glu Arg Arg Ala Ala Ile Val Arg Arg
            340                 345                 350
Gly Glu Ala Glu Arg Arg Leu Tyr Ser Glu Ile Ala Gln Ala Met Ala
        355                 360                 365
Asp Arg Asp Ala Ala Leu Ala Ser Tyr Arg Gln Ala Lys Glu His Thr
    370                 375                 380
Asp Ala Met Gln Thr Ala Tyr Glu Ala Val Leu Gln Arg Tyr Glu Glu
385                 390                 395                 400
Gly Leu Asn Thr Ala Ile Asp Leu Thr Thr Gln Ala Asn Arg Leu Leu
                405                 410                 415
Asp Ala Arg Val Gln Arg Leu Arg Ala Ala Met Thr Tyr Arg Leu Lys
            420                 425                 430
Cys Lys Leu Ile Ala Tyr Tyr Gly Cys Leu Ser Asp
        435                 440

(2) INFORMATION FOR SEQ ID NO:463

(i) SEQUENCE CHARACTERISTICS:
(A) LENGTH: 940 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...940

(xi) SEQUENCE DESCRIPTION: SEQ ID NO:463

Met Asn Lys Phe Tyr Lys Ser Leu Leu Gln Ser Gly Leu Ala Ala Phe
1               5                   10                  15
Val Ser Met Ala Thr Ala Leu Thr Ala Ser Ala Gln Ile Ser Phe Gly
            20                  25                  30
Gly Glu Pro Leu Ser Phe Ser Ser Arg Ser Ala Gly Thr His Ser Phe
        35                  40                  45
Asp Asp Ala Met Thr Ile Arg Leu Thr Pro Asp Phe Asn Pro Glu Asp
    50                  55                  60
Leu Ile Ala Gln Ser Arg Trp Gln Ser Gln Arg Asp Gly Arg Pro Val
65                  70                  75                  80
Arg Ile Gly Gln Val Ile Pro Val Asp Val Asp Phe Ala Ser Lys Ala
                85                  90                  95

Ser His Ile Ser Ser Ile Gly Asp Val Asp Val Tyr Arg Leu Gln Phe
            100                 105                 110
Lys Leu Glu Gly Ala Lys Ala Ile Thr Leu Tyr Tyr Asp Ala Phe Asn
        115                 120                 125
Ile Pro Glu Gly Gly Arg Leu Tyr Ile Tyr Thr Pro Asp His Glu Ile
    130                 135                 140
Val Leu Gly Ala Tyr Thr Asn Ala Thr His Arg Arg Asn Gly Ala Phe
145                 150                 155                 160
Ala Thr Glu Pro Val Pro Gly Ser Glu Leu Ile Met Asp Tyr Glu Val
                165                 170                 175
Ser Arg Gly Gly Thr Leu Pro Asp Ile Lys Ile Ser Gly Ala Gly Tyr
            180                 185                 190
Ile Phe Asp Lys Val Gly Gly Arg Pro Val Thr Asp Asn His Tyr Gly
        195                 200                 205
Ile Gly Glu Asp Asp Ser Asp Ser Asp Cys Glu Ile Asn Ile Asn Cys
    210                 215                 220
Pro Glu Gly Ala Asp Trp Gln Ala Glu Lys Asn Gly Val Val Gln Met
225                 230                 235                 240
Ile Met Val Lys Gly Gln Tyr Ile Ser Met Cys Ser Gly Asn Leu Leu
                245                 250                 255
Asn Asn Thr Lys Gly Asp Phe Thr Pro Leu Ile Ile Ser Ala Gly His
            260                 265                 270
Cys Ala Ser Ile Thr Thr Asn Phe Gly Val Thr Gln Ser Glu Leu Asp
        275                 280                 285
Lys Trp Ile Phe Thr Phe His Tyr Glu Lys Arg Gly Cys Ser Asn Gly
    290                 295                 300
Thr Leu Ala Ile Phe Arg Gly Asn Ser Ile Ile Gly Ala Ser Met Lys
305                 310                 315                 320
Ala Phe Leu Pro Ile Lys Gly Lys Ser Asp Gly Leu Leu Leu Gln Leu
                325                 330                 335
Asn Asp Glu Val Pro Leu Arg Tyr Arg Val Tyr Tyr Asn Gly Trp Asp
            340                 345                 350
Ser Thr Pro Asp Ile Pro Ser Ser Gly Ala Gly Ile His His Pro Ala
        355                 360                 365
Gly Asp Ala Met Lys Ile Ser Ile Leu Lys Lys Thr Pro Ala Leu Asn
    370                 375                 380
Thr Trp Ile Ser Ser Ser Gly Ser Gly Gly Thr Asp Asp His Phe Tyr
385                 390                 395                 400
Phe Lys Tyr Asp Gln Gly Gly Thr Glu Gly Gly Ser Ser Gly Ser Ser
                405                 410                 415
Leu Phe Asn Gln Asn Lys His Val Val Gly Thr Leu Thr Gly Gly Ala
            420                 425                 430
Gly Asn Cys Gly Gly Thr Glu Phe Tyr Gly Arg Leu Asn Ser His Trp
        435                 440                 445
Asn Glu Tyr Ala Ser Asp Gly Asn Thr Ser Arg Met Asp Ile Tyr Leu
    450                 455                 460
Asp Pro Gln Asn Asn Gly Gln Thr Thr Ile Leu Asn Gly Thr Tyr Arg
465 470 475 460
Asp Gly Tyr Lys Pro Leu Pro Ser Val Pro Arg Leu Leu Leu Gln Ser
                485                 490                 495
Thr Gly Asp Gln Val Glu Leu Asn Trp Thr Ala Val Pro Ala Asp Gln
            500                 505                 510
Tyr Pro Ser Ser Tyr Gln Val Glu Tyr His Ile Phe Arg Asn Gly Lys
        515                 520                 525
Glu Ile Ala Thr Thr Lys Glu Leu Ser Tyr Ser Asp Ala Ile Asp Glu
    530                 535                 540
Ser Ile Ile Gly Ser Gly Ile Ile Arg Tyr Glu Val Ser Ala Arg Phe
545                 550                 555                 560
Ile Tyr Pro Ser Pro Leu Asp Gly Val Glu Ser Tyr Lys Asp Thr Asp
                565                 570                 575
Lys Thr Ser Ala Asp Leu Ala Ile Gly Asp Ile Gln Thr Lys Leu Lys
            580                 585                 590
Pro Asp Val Thr Pro Leu Pro Gly Gly Gly Val Ser Leu Ser Trp Lys
        595                 600                 605
Val Pro Phe Leu Ser Gln Leu Val Ser Arg Phe Gly Glu Ser Pro Asn
    610                 615                 620
Pro Val Phe Lys Thr Phe Glu Val Pro Tyr Val Ser Ala Ala Ala Ala
625                 630                 635                 640
Gln Thr Pro Asn Pro Pro Val Gly Val Val Ile Ala Asp Lys Phe Met
                645                 650                 655
Ala Gly Thr Tyr Pro Glu Lys Ala Ala Ile Ala Ala Val Tyr Val Met
            660                 665                 670
Pro Ser Ala Pro Asp Ser Thr Phe His Leu Phe Leu Lys Ser Asn Thr
        675                 680                 685
Asn Arg Arg Leu Gln Lys Val Thr Thr Pro Ser Asp Trp Gln Ala Gly

690                 695                 700
Thr Trp Leu Arg Ile Asn Leu Asp Lys Pro Phe Pro Val Asn Asn Asp
705                 710                 715                 720
His Met Leu Phe Ala Gly Ile Arg Met Pro Asn Lys Tyr Lys Leu Asn
                725                 730                 735
Arg Ala Ile Arg Tyr Val Arg Asn Pro Asp Asn Leu Phe Ser Ile Thr
            740                 745                 750
Gly Lys Lys Ile Ser Tyr Asn Asn Gly Val Ser Phe Glu Gly Tyr Gly
        755                 760                 765
Ile Pro Ser Leu Leu Gly Tyr Met Ala Ile Lys Tyr Leu Val Val Asn
    770                 775                 780
Thr Asp Ala Pro Lys Ile Asp Met Ser Leu Val Gln Glu Pro Tyr Ala
785                 790                 795                 800
Lys Gly Thr Asn Val Ala Pro Phe Pro Glu Leu Val Gly Ile Tyr Val
                805                 810                 815
Tyr Lys Asn Gly Thr Phe Ile Gly Thr Gln Asp Pro Ser Val Thr Thr
            820                 825                 830
Tyr Ser Val Ser Asp Gly Thr Glu Ser Asp Glu Tyr Glu Ile Lys Leu
        835                 840                 845
Val Tyr Lys Gly Ser Gly Ile Ser Asn Gly Val Ala Gln Ile Glu Asn
    850                 855                 860
Asn Asn Ala Val Val Ala Tyr Pro Ser Val Val Thr Asp Arg Phe Ser
865                 870                 875                 880
Ile Lys Asn Ala His Met Val His Ala Ala Leu Tyr Ser Leu Asp
                885                 890                 895
Gly Lys Gln Val Arg Ser Trp Asn Asn Leu Arg Asn Gly Val Thr Phe
            900                 905                 910
Ser Val Gln Gly Leu Thr Ala Gly Thr Tyr Met Leu Val Met Gln Thr
        915                 920                 925
Ala Asn Gly Pro Val Ser Gln Lys Ile Val Lys Gln
    930                 935                 940

(2) INFORMATION FOR SEQ ID NO:464

    (i) SEQUENCE CHARACTERISTICS:
        (A) LENGTH: 670 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...670

    (xi) SEQUENCE DESCRIPTION: SEQ ID NO:464

Met Lys Tyr Leu Ile Arg Leu Phe Leu Ser Leu Met Leu Leu Ser Leu
1               5                   10                  15
Trp Thr Gly Cys Thr His Glu Glu Leu Ser Ile Cys Asp Gly Glu Asn
            20                  25                  30
Thr Leu Val Leu Arg Val Glu Thr Gly Lys Ala Pro Asn Ala Arg Ala
        35                  40                  45
Thr Glu Pro Gly Gln Gly Ile Tyr Asn Glu Asn Lys Val Gly Ser Ile
    50                  55                  60
Ser Val Leu Phe Tyr Leu Glu Gly Gln Leu Arg Trp Gln Val Lys Ser
65                  70                  75                  80
Thr Asp Tyr Gln Ile His Glu Gly Ala Tyr Ile Ile Pro Val Lys Glu
                85                  90                  95
Gln Met Arg Pro Leu Phe Asn Gly Asn Asn Asn Phe Ser Ile Tyr Val
            100                 105                 110
Val Ala Asn Leu Asp Phe Asn Ala Pro Ala Thr Glu Ala Ala Leu Ser
        115                 120                 125
Gln Phe Val Val Glu Lys Ser Ile Glu Val Ser Ser Thr Thr Ala Pro
    130                 135                 140
Ala Asp Phe Val Met Leu Ala His Gly Asn Lys Gln Ile Asn Met Ala
145                 150                 155                 160
Thr Thr Glu Gly Lys Leu Leu Gly Asp Tyr Lys Leu Lys Arg Val Ala
                165                 170                 175
Ala Lys Ile Arg Met Ile Lys Pro Thr Ile Asn Val Gln Gly Tyr Glu

                180                 185                 190
Val Val Gly Asn Ile Gln Ala Lys Phe Arg Asn Ser Val Thr Lys Gly
        195                 200                 205
Phe Leu Thr Thr Glu Ala Gln Glu Ile Pro Ala Ala Ala Ser Tyr Lys
    210                 215                 220
Thr Ser Glu Tyr Leu Asp Ile Ala Glu Ser Ala Pro Ala Asn Ser Ile
225                 230                 235                 240
His Phe Tyr Ser Tyr Tyr Asn Lys Trp Thr Leu Ser Thr Pro Glu Lys
                245                 250                 255
Arg Pro Glu Phe Phe Ile Met Val Lys Phe Lys Lys Thr Gly Gln Pro
            260                 265                 270
Asp Asn Thr Ala Lys Pro Tyr Tyr Tyr Arg Val Pro Leu Glu Ser Gln
        275                 280                 285
Asp Asn Gln Val Lys Ser Asn Val Leu Tyr Asn Leu Asn Val Lys Ile
    290                 295                 300
Glu Ile Leu Gly Ser Leu Gln Glu Pro Glu Ala Val Ser Val Asn Gly
305                 310                 315                 320
Thr Leu Ala Ile Glu Glu Trp Ile Leu His Gln Asp Ala Phe Asn Leu
                325                 330                 335
Pro Ala Thr Asn Tyr Leu Ile Val Glu Gln His Glu Ile Phe Met Asn
            340                 345                 350
Asn Val Asn Thr Tyr Ser Val Lys Tyr Gln Thr Ser Gln Lys Pro Ile
        355                 360                 365
Ser Ile Ser Ile Gln Ser Val Thr Phe Ser Tyr Val Ser Ser Asp Gly
    370                 375                 380
Thr Gln His Asn Asp Leu Val Ala Ser Ser Ser Asp Gln Tyr Pro Thr
385                 390                 395                 400
Ile Thr Ser Asp Asn Thr Ser Ile Ile Ile Thr Ser Lys Ile Pro Val
                405                 410                 415
Asn Asn Val Pro Lys Lys Ile Val Phe Glu Val Thr Asn Gly Val Ala
            420                 425                 430
Gly Leu Lys Glu Thr Val Thr Val Leu Gln Tyr Pro Ala Gln Phe Ile
        435                 440                 445
Val Asn Thr Leu Gly Thr Ala Ser Ala Trp Arg Pro Asp Gly Ser Leu
    450                 455                 460
Ala Pro Gly Leu Asn Asn Lys Ala Ile Tyr His Val Val Val Leu Val
465                 470                 475                 480
Pro Pro Glu Asn Leu Phe Glu Asp Gly Thr Gln Thr Ile Ile Gly Tyr
                485                 490                 495
Pro Pro Thr Glu Thr Ile Ser Phe His Lys Lys Glu Asn Asn Thr Tyr
            500                 505                 510
Pro Ile Val Trp Ser Asp Thr Asn Thr Thr Lys Gln Asp Leu Glu Thr
        515                 520                 525
Ser Arg Met Ile Ser Pro Ser Phe Glu Leu Ala Ser Gln Leu Gly Ala
    530                 535                 540
Thr Leu Pro Met Pro Tyr Leu Glu Tyr Trp Pro Gly Thr Ser Tyr Leu
545                 550                 555                 560
Leu Asp Tyr Ser Gly Asn Tyr Asn Asn Lys Arg Tyr Ala Leu Phe Asn
                565                 570                 575
Cys Ala Phe Tyr Trp Glu Lys Arg Lys Val Asn Asn Glu Glu Ile Lys
            580                 585                 590
Phe Asp Asp Trp Arg Leu Pro Thr Glu Ala Glu Ile Lys Leu Ile Asp
        595                 600                 605
Lys Leu Gln His Asn Glu Gln Ser Ala Val Gln Ala Ile Met Thr Gly
    610                 615                 620
Asn Tyr Tyr Trp Asp Ser Tyr Ser Ala Asn Gly Ser Tyr Lys Met Gln
625                 630                 635                 640
Gly Gly Gly Gly Gln Gly Asn Ser Ser Lys Ala Tyr Val Arg Cys Val
                645                 650                 655
Arg Asp Val Lys Lys Pro Ile Arg Asp Lys Lys Ser Gly Lys
            660                 665                 670

(2) INFORMATION FOR SEQ ID NO:465

     (i) SEQUENCE CHARACTERISTICS:
          (A) LENGTH: 1282 amino acids
          (B) TYPE: amino acid
          (D) TOPOLOGY: linear

    (ii) MOLECULE TYPE: protein

   (iii) HYPOTHETICAL: YES

    (vi) ORIGINAL SOURCE:
          (A) ORGANISM: Porphyromonas gingivalis

(ix) FEATURE:
(A) NAME/KEY: misc_feature
(B) LOCATION 1...1282

(xi) SEQUENCE DESCRIPTION: SEQ ID NO:465

Met Arg Lys Ile Leu Ser Phe Leu Met Met Cys Ser Leu His Leu Gly
1               5                   10                  15
Leu Gln Ser Gln Thr Trp His Gly Asp Pro Asp Ser Val Ala Ala Leu
            20                  25                  30
Pro Ser Ile Gly Ile Gln Glu Ser Ser Cys Thr Arg Ile Thr Phe Glu
        35                  40                  45
Val Val Phe Pro Gly Phe Tyr Ser Val Glu Lys Arg Glu Gly Asn Gln
    50                  55                  60
Val Phe Gln Arg Ile Ser Met Pro Gly Cys Gly Ser Phe Gly Asn Leu
65                  70                  75                  80
Gly Glu Ala Glu Leu Pro Val Leu Lys Lys Met Ile Ala Val Pro Glu
                85                  90                  95
Phe Ser Thr Ala Asn Val Ala Val Lys Ile Lys Glu Thr Glu Thr Phe
            100                 105                 110
Asp Asn Tyr Asn Ile Tyr Pro Asn Pro Thr Tyr Val Val Glu Glu Leu
        115                 120                 125
Pro Glu Gly Gly Thr Tyr Leu Val Glu Ala Phe Ala Ile Asn Asn Asp
    130                 135                 140
Tyr Tyr Ser Gln Asn Val Ser Leu Pro Ser Thr His Tyr Val Tyr Ser
145                 150                 155                 160
Gln Asp Gly Tyr Phe Arg Ser Gln Arg Phe Ile Glu Val Thr Leu Tyr
                165                 170                 175
Pro Phe Arg Tyr Asn Pro Val Arg Gln Glu Ile Leu Phe Ala Lys Lys
            180                 185                 190
Ile Glu Val Thr Ile Thr Phe Asp Asn Pro Gln Pro Pro Leu Gln Lys
        195                 200                 205
Asn Thr Gly Ile Phe Asn Lys Val Ala Ser Ser Ala Phe Ile Asn Tyr
    210                 215                 220
Glu Ala Asp Gly Lys Ser Ala Ile Glu Asn Asp Met Val Phe Ser Arg
225                 230                 235                 240
Gly Thr Thr Thr Tyr Ile Ser Gly Asn Val Ala Ser Asn Leu Pro Gln
                245                 250                 255
Asn Cys Asp Tyr Leu Val Ile Tyr Asp Asp Met Phe Asn Val Asn Gln
            260                 265                 270
Gln Pro His Asp Glu Ile Lys Arg Leu Cys Glu His Arg Ala Phe Tyr
        275                 280                 285
Asn Gly Phe Asp Val Ala Ala Val Ser Ile Lys Asp Val Leu Asn Ser
    290                 295                 300
Phe Pro Ser Asn Ala Thr Ser Tyr Ile Asn Glu Thr Lys Leu Lys Asn
305                 310                 315                 320
Phe Ile Arg Ser Val Tyr Asn Gln Ser Asn Ala Lys Arg Thr Leu Asp
                325                 330                 335
Gly Lys Leu Gly Tyr Val Leu Leu Ile Gly Lys Pro Leu Ser Lys Tyr
            340                 345                 350
Leu Ala Asp Thr Asp Asn Thr Lys Val Pro Thr Ser Phe Ile His Asn
        355                 360                 365
Val Ser Leu Ile Pro Ser His Pro Thr Phe Gly Ser Ile Cys Ala Ser
    370                 375                 380
Asp Tyr Phe Phe Ser Cys Val Ser Pro Leu Asp Thr Val Gly Asp Leu
385                 390                 395                 400
Phe Ile Gly Arg Phe Ser Val Thr Asn Ala His Glu Leu His Asn Leu
                405                 410                 415
Ile Glu Lys Thr Ile Asn Lys Glu Ile Ser Tyr Asn Pro Ile Ala His
            420                 425                 430
Lys Asn Ile Leu Tyr Ala Glu Gly Lys Gly Cys Asp Ala Pro Ile Leu
        435                 440                 445
Arg Leu Phe Leu Lys Glu Ile Ala Ser Gly Tyr Thr Val Asn Ser Ile
    450                 455                 460
Leu Lys Ser Asn Gln Val Ser Ala Ile Asp Ser Ile Phe Asp Cys Leu
465                 470                 475                 480
Asn Asn Gly Ser His His Phe Tyr Phe Asn Thr His Gly Met Pro Thr
                485                 490                 495
Val Trp Gly Ile Gly Gln Gly Leu Asp Val Asn Thr Leu Thr Ala Arg
            500                 505                 510
Leu Asn Asn Thr Ser Ser Gln Gly Leu Cys Thr Ser Leu Ser Cys Ser
        515                 520                 525
Ser Ala Val Ala Asp Ser Thr Ile Arg Ser Leu Gly Glu Val Leu Thr
    530                 535                 540

Thr Tyr Ala Pro Asn Lys Gly Phe Ser Ala Phe Leu Gly Gly Ser Arg
545                 550                 555                 560
Ala Thr Gln Tyr Ala Val Tyr Leu Glu Gly Pro Cys Pro Pro Ser Glu
                565                 570                 575
Phe Tyr Glu Tyr Leu Pro Tyr Ser Leu Tyr His Asn Leu Ser Thr Val
            580                 585                 590
Val Gly Glu Met Leu Leu Ser Ser Ile Ile Asn Thr Asn Ser Val Asp
        595                 600                 605
Thr Tyr Ser Lys Phe Asn Phe Asn Leu Leu Gly Asp Pro Ala Leu Asn
    610                 615                 620
Ile Met Ala His Gly Met Glu Val Ser Asn Cys Ile Thr Leu Pro Asn
625                 630                 635                 640
Asn Thr Ile Ile Ser Ser Pro Ile Thr Ile Lys Asn Gly Gly Cys Leu
                645                 650                 655
Lys Ile Pro Glu Lys Gly Val Leu His Phe Thr Asn Asn Gly Ser Ile
            660                 665                 670
Gln Val Met Ser Gly Gly Thr Leu Glu Ile Gly Asn Gln Ala Lys Ile
        675                 680                 685
Ser Gly Glu Thr Gly Ala Asn Pro Thr Phe Ile Thr Val Tyr Gly Asp
    690                 695                 700
Gly Leu Ala Ile Asn Lys Gln Val Glu Ile Asp Asn Ile Asp Arg Leu
705                 710                 715                 720
Asn Leu Phe Ser Thr His Ser Val Met Pro Lys Phe His Phe Asp Ser
                725                 730                 735
Val Lys Phe Asn Ser Ala Pro Leu Tyr Thr Thr Asn Cys Ile Val Glu
            740                 745                 750
Ile Ser Asn Cys Glu Phe Thr Asn Arg Ser Asp Ile Ile Ser Lys Asn
        755                 760                 765
Cys Asp Leu Ser Val Glu Asn Ser Met Phe Ser Ser Ser Gly Ile Thr
    770                 775                 780
Val Phe Lys Pro Met Ala Thr Ser Ser Ile Thr Gly Leu Ser Thr Lys
785                 790                 795                 800
Ala Lys Ile Thr Asp Asn Thr Phe Phe Ala Thr Gly Asn Phe Ala Tyr
                805                 810                 815
His Ile Thr Asn Thr Pro Gly Leu Thr Ala Thr Ser Asn Ala Ala Ile
            820                 825                 830
Lys Leu Asp Asn Ile Pro Glu Tyr Tyr Ile Ser Gly Asn Lys Ile Val
        835                 840                 845
Asn Cys Asp Glu Ala Leu Val Leu Asn Asn Ser Gly Asn Arg Thr Asn
    850                 855                 860
Arg Leu His Asn Ile Thr Arg Asn Val Ile Lys Asn Cys Arg Ile Gly
865                 870                 875                 880
Ser Thr Leu Tyr Asn Ser Tyr Gly Ile Tyr Asn Arg Asn Lys Ile Ser
                885                 890                 895
Asn Asn His Ile Gly Val Arg Leu Leu Asn Asn Ser Cys Phe Tyr Phe
            900                 905                 910
Asp Asn Ala Pro Val Ile Asn Glu Glu Asp Lys Gln Thr Phe Ile Ser
        915                 920                 925
Asn Arg Thr Trp Gln Leu Tyr Ser Ser Asn Gly Thr Phe Pro Leu Asn
    930                 935                 940
Phe His Tyr Asn Ser Leu Gln Gly Gly Asp Thr Asp Thr Trp Ile Tyr
945                 950                 955                 960
Asn Asp Thr Tyr Thr Asn Arg Tyr Ile Asp Val Ser Asn Asn His Trp
                965                 970                 975
Gly Asn Asn Asp Leu Phe Asp Pro Asn Gln Val Phe Asn Thr Pro Asp
            980                 985                 990
Leu Phe Ile Trp Ile Pro Phe Trp Asp Gly Leu Pro Asn Gly Arg Ser
        995                 1000                1005
Gly Asn Ser Ser Ala Glu Ala Val Glu Phe Gln Thr Ala Leu Asp Cys
    1010                1015                1020
Ile Gly Asn Ser Asp Tyr Leu Ser Ala Lys Val Ala Leu Lys Met Met
1025                1030                1035                1040
Val Glu Thr Tyr Pro Glu Ser Asp Phe Ala Ile Ala Ala Leu Lys Glu
                1045                1050                1055
Leu Phe Arg Ile Glu Lys Met Ser Gly Asn Asp Tyr Glu Gly Leu Lys
            1060                1065                1070
Asp Tyr Phe Arg Ser Asn Pro Thr Ile Ile Ser Ser Gln Asn Leu Phe
        1075                1080                1085
Pro Thr Ala Asp Phe Leu Ser Ala Arg Cys Asp Ile Val Cys Glu Asn
    1090                1095                1100
Tyr Gln Ser Ala Ile Asp Trp Tyr Glu Asn Arg Leu Asn Ser Glu Ile
1105                1110                1115                1120
Ser Tyr Gln Asp Ser Val Phe Ala Val Ile Asp Leu Gly Asp Ile Tyr
                1125                1130                1135
Trp Asn Met Gln Leu Asp Ser Leu Arg Gly Thr Gly Ile Asp Leu Asn

                1140                    1145                    1150
Ile Leu Ser Cys Glu Gln Arg Lys Ser Leu Glu Ser His Gln Asn Val
        1155                    1160                    1165
Lys Asn Tyr Leu Leu Ser Thr Leu Pro Glu Ser Thr Gly Thr Leu Leu
    1170                    1175                    1180
Pro Pro Leu Glu Cys Asn Lys Ser Ser Leu Asp Lys Ser Lys Ile Ile
1185                    1190                    1195                    1200
Ser Ile Ser Pro Asn Pro Ala Lys Ala Val Val Thr Ile Ile Tyr Tyr
                1205                    1210                    1215
Thr Asp Asn Pro Ser Cys Ser Val Ile Lys Ile Tyr Gly Ile Asn Gly
            1220                    1225                    1230
Ala Ser Ala Asp Ile Thr Gly Leu Pro Lys His Leu Ser Glu Gly Tyr
        1235                    1240                    1245
Tyr Ser Ile Gln Phe Asn Thr Ser Asn Phe Asp Pro Gly Phe Tyr Leu
    1250                    1255                    1260
Val Thr Leu Asn Val Asp Gln Lys Ile Ile Asp Thr Glu Lys Leu Arg
1265                    1270                    1275                    1280
Ile Lys

(2) INFORMATION FOR SEQ ID NO:466

    (i) SEQUENCE CHARACTERISTICS:
        (A) LENGTH: 1274 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...1274

    (xi) SEQUENCE DESCRIPTION: SEQ ID NO:466

Met Met Cys Ser Leu His Leu Gly Leu Gln Ser Gln Thr Trp His Gly
1               5                   10                  15
Asp Pro Asp Ser Val Ala Ala Leu Pro Ser Ile Gly Ile Gln Glu Ser
            20                  25                  30
Ser Cys Thr Arg Ile Thr Phe Glu Val Val Phe Pro Gly Phe Tyr Ser
        35                  40                  45
Val Glu Lys Arg Glu Gly Asn Gln Val Phe Gln Arg Ile Ser Met Pro
    50                  55                  60
Gly Cys Gly Ser Phe Gly Asn Leu Gly Glu Ala Glu Leu Pro Val Leu
65                  70                  75                  80
Lys Lys Met Ile Ala Val Pro Glu Phe Ser Thr Ala Asn Val Ala Val
                85                  90                  95
Lys Ile Lys Glu Thr Glu Thr Phe Asp Asn Tyr Asn Ile Tyr Pro Asn
            100                 105                 110
Pro Thr Tyr Val Val Glu Glu Leu Pro Glu Gly Gly Thr Tyr Leu Val
        115                 120                 125
Glu Ala Phe Ala Ile Asn Asn Asp Tyr Tyr Ser Gln Asn Val Ser Leu
    130                 135                 140
Pro Ser Thr His Tyr Val Tyr Ser Gln Asp Gly Tyr Phe Arg Ser Gln
145                 150                 155                 160
Arg Phe Ile Glu Val Thr Leu Tyr Pro Phe Arg Tyr Asn Pro Val Arg
                165                 170                 175
Gln Glu Ile Leu Phe Ala Lys Lys Ile Glu Val Thr Ile Thr Phe Asp
            180                 185                 190
Asn Pro Gln Pro Pro Leu Gln Lys Asn Thr Gly Ile Phe Asn Lys Val
        195                 200                 205
Ala Ser Ser Ala Phe Ile Asn Tyr Glu Ala Asp Gly Lys Ser Ala Ile
    210                 215                 220
Glu Asn Asp Met Val Phe Ser Arg Gly Thr Thr Thr Tyr Ile Ser Gly
225                 230                 235                 240
Asn Val Ala Ser Asn Leu Pro Gln Asn Cys Asp Tyr Leu Val Ile Tyr
                245                 250                 255
Asp Asp Met Phe Asn Val Asn Gln Gln Pro His Asp Glu Ile Lys Arg
            260                 265                 270
Leu Cys Glu His Arg Ala Phe Tyr Asn Gly Phe Asp Val Ala Ala Val

        275                 280                 285
Ser Ile Lys Asp Val Leu Asn Ser Phe Pro Ser Asn Ala Thr Ser Tyr
    290                 295                 300
Ile Asn Glu Thr Lys Leu Lys Asn Phe Ile Arg Ser Val Tyr Asn Gln
305                 310                 315                 320
Ser Asn Ala Lys Arg Thr Leu Asp Gly Lys Leu Gly Tyr Val Leu Leu
                325                 330                 335
Ile Gly Lys Pro Leu Ser Lys Tyr Leu Ala Asp Thr Asp Asn Thr Lys
            340                 345                 350
Val Pro Thr Ser Phe Ile His Asn Val Ser Leu Ile Pro Ser His Pro
        355                 360                 365
Thr Phe Gly Ser Ile Cys Ala Ser Asp Tyr Phe Phe Ser Cys Val Ser
    370                 375                 380
Pro Leu Asp Thr Val Gly Asp Leu Phe Ile Gly Arg Phe Ser Val Thr
385                 390                 395                 400
Asn Ala His Glu Leu His Asn Leu Ile Glu Lys Thr Ile Asn Lys Glu
                405                 410                 415
Ile Ser Tyr Asn Pro Ile Ala His Lys Asn Ile Leu Tyr Ala Glu Gly
            420                 425                 430
Lys Gly Cys Asp Ala Pro Ile Leu Arg Leu Phe Leu Lys Glu Ile Ala
        435                 440                 445
Ser Gly Tyr Thr Val Asn Ser Ile Leu Lys Ser Asn Gln Val Ser Ala
    450                 455                 460
Ile Asp Ser Ile Phe Asp Cys Leu Asn Asn Gly Ser His His Phe Tyr
465                 470                 475                 480
Phe Asn Thr His Gly Met Pro Thr Val Trp Gly Ile Gly Gln Gly Leu
                485                 490                 495
Asp Val Asn Thr Leu Thr Ala Arg Leu Asn Asn Thr Ser Ser Gln Gly
            500                 505                 510
Leu Cys Thr Ser Leu Ser Cys Ser Ser Ala Val Ala Asp Ser Thr Ile
        515                 520                 525
Arg Ser Leu Gly Glu Val Leu Thr Thr Tyr Ala Pro Asn Lys Gly Phe
    530                 535                 540
Ser Ala Phe Leu Gly Gly Ser Arg Ala Thr Gln Tyr Ala Val Tyr Leu
545                 550                 555                 560
Glu Gly Pro Cys Pro Pro Ser Glu Phe Tyr Glu Tyr Leu Pro Tyr Ser
                565                 570                 575
Leu Tyr His Asn Leu Ser Thr Val Val Gly Glu Met Leu Leu Ser Ser
            580                 585                 590
Ile Ile Asn Thr Asn Ser Val Asp Thr Tyr Ser Lys Phe Asn Phe Asn
        595                 600                 605
Leu Leu Gly Asp Pro Ala Leu Asn Ile Met Ala His Gly Met Glu Val
    610                 615                 620
Ser Asn Cys Ile Thr Leu Pro Asn Asn Thr Ile Ile Ser Ser Pro Ile
625                 630                 635                 640
Thr Ile Lys Asn Gly Gly Cys Leu Lys Ile Pro Glu Lys Gly Val Leu
                645                 650                 655
His Phe Thr Asn Asn Gly Ser Ile Gln Val Met Ser Gly Gly Thr Leu
            660                 665                 670
Glu Ile Gly Asn Gln Ala Lys Ile Ser Gly Glu Thr Gly Ala Asn Pro
        675                 680                 685
Thr Phe Ile Thr Val Tyr Gly Asp Gly Leu Ala Ile Asn Lys Gln Val
    690                 695                 700
Glu Ile Asp Asn Ile Asp Arg Leu Asn Leu Phe Ser Thr His Ser Val
705                 710                 715                 720
Met Pro Lys Phe His Phe Asp Ser Val Lys Phe Asn Ser Ala Pro Leu
                725                 730                 735
Tyr Thr Thr Asn Cys Ile Val Glu Ile Ser Asn Cys Glu Phe Thr Asn
            740                 745                 750
Arg Ser Asp Ile Ile Ser Lys Asn Cys Asp Leu Ser Val Glu Asn Ser
        755                 760                 765
Met Phe Ser Ser Ser Gly Ile Thr Val Phe Lys Pro Met Ala Thr Ser
    770                 775                 780
Ser Ile Thr Gly Leu Ser Thr Lys Ala Lys Ile Thr Asp Asn Thr Phe
785                 790                 795                 800
Phe Ala Thr Gly Asn Phe Ala Tyr His Ile Thr Asn Thr Pro Gly Leu
                805                 810                 815
Thr Ala Thr Ser Asn Ala Ala Ile Lys Leu Asp Asn Ile Pro Glu Tyr
            820                 825                 830
Tyr Ile Ser Gly Asn Lys Ile Val Asn Cys Asp Glu Ala Leu Val Leu
        835                 840                 845
Asn Asn Ser Gly Asn Arg Thr Asn Arg Leu His Asn Ile Thr Arg Asn
    850                 855                 860
Val Ile Lys Asn Cys Arg Ile Gly Ser Thr Leu Tyr Asn Ser Tyr Gly
865                 870                 875                 880

Ile Tyr Asn Arg Asn Lys Ile Ser Asn Asn His Ile Gly Val Arg Leu
                885                 890                 895
Leu Asn Asn Ser Cys Phe Tyr Phe Asp Asn Ala Pro Val Ile Asn Glu
            900                 905                 910
Glu Asp Lys Gln Thr Phe Ile Ser Asn Arg Thr Trp Gln Leu Tyr Ser
        915                 920                 925
Ser Asn Gly Thr Phe Pro Leu Asn Phe His Tyr Asn Ser Leu Gln Gly
    930                 935                 940
Gly Asp Thr Asp Thr Trp Ile Tyr Asn Asp Thr Tyr Thr Asn Arg Tyr
945                 950                 955                 960
Ile Asp Val Ser Asn Asn His Trp Gly Asn Asn Asp Leu Phe Asp Pro
                965                 970                 975
Asn Gln Val Phe Asn Thr Pro Asp Leu Phe Ile Trp Ile Pro Phe Trp
            980                 985                 990
Asp Gly Leu Pro Asn Gly Arg Ser Gly Asn Ser Ser Ala Glu Ala Val
        995                 1000                1005
Glu Phe Gln Thr Ala Leu Asp Cys Ile Gly Asn Ser Asp Tyr Leu Ser
    1010                1015                1020
Ala Lys Val Ala Leu Lys Met Met Val Glu Thr Tyr Pro Glu Ser Asp
1025                1030                1035                1040
Phe Ala Ile Ala Ala Leu Lys Glu Leu Phe Arg Ile Glu Lys Met Ser
                1045                1050                1055
Gly Asn Asp Tyr Glu Gly Leu Lys Asp Tyr Phe Arg Ser Asn Pro Thr
            1060                1065                1070
Ile Ile Ser Ser Gln Asn Leu Phe Pro Thr Ala Asp Phe Leu Ser Ala
        1075                1080                1085
Arg Cys Asp Ile Val Cys Glu Asn Tyr Gln Ser Ala Ile Asp Trp Tyr
    1090                1095                1100
Glu Asn Arg Leu Asn Ser Glu Ile Ser Tyr Gln Asp Ser Val Phe Ala
1105                1110                1115                1120
Val Ile Asp Leu Gly Asp Ile Tyr Trp Asn Met Gln Leu Asp Ser Leu
                1125                1130                1135
Arg Gly Thr Gly Ile Asp Leu Asn Ile Leu Ser Cys Glu Gln Arg Lys
            1140                1145                1150
Ser Leu Glu Ser His Gln Asn Val Lys Asn Tyr Leu Leu Ser Thr Leu
        1155                1160                1165
Pro Glu Ser Thr Gly Thr Leu Leu Pro Pro Leu Glu Cys Asn Lys Ser
    1170                1175                1180
Ser Leu Asp Lys Ser Lys Ile Ile Ser Ile Ser Pro Asn Pro Ala Lys
1185                1190                1195                1200
Ala Val Val Thr Ile Ile Tyr Tyr Thr Asp Asn Pro Ser Cys Ser Val
                1205                1210                1215
Ile Lys Ile Tyr Gly Ile Asn Gly Ala Ser Ala Asp Ile Thr Gly Leu
            1220                1225                1230
Pro Lys His Leu Ser Glu Gly Tyr Tyr Ser Ile Gln Phe Asn Thr Ser
        1235                1240                1245
Asn Phe Asp Pro Gly Phe Tyr Leu Val Thr Leu Asn Val Asp Gln Lys
    1250                1255                1260
Ile Ile Asp Thr Glu Lys Leu Arg Ile Lys
1265                1270

(2) INFORMATION FOR SEQ ID NO:467

     (i) SEQUENCE CHARACTERISTICS:
          (A) LENGTH: 925 amino acids
          (B) TYPE: amino acid
          (D) TOPOLOGY: linear

     (ii) MOLECULE TYPE: protein

     (iii) HYPOTHETICAL: YES

     (vi) ORIGINAL SOURCE:
          (A) ORGANISM: Porphyromonas gingivalis

     (ix) FEATURE:
          (A) NAME/KEY: misc feature
          (B) LOCATION 1...925

     (xi) SEQUENCE DESCRIPTION: SEQ ID NO:467

Met Ala Ile Met Met Lys Ser Ile Val Phe Arg Ala Phe Leu Thr Ile
1               5                   10                  15
Leu Leu Ser Trp Ala Ala Ile Thr Asn Pro Thr Ala Gln Glu Ile Ser
            20                  25                  30

Gly Met Asn Ala Ser Cys Leu Ala Ala Pro Ala Gln Pro Asp Thr Ile
        35                  40                  45
Leu Tyr Glu Ser Phe Glu Asn Gly Pro Val Pro Asn Gly Trp Leu Glu
    50                  55                  60
Ile Asp Ala Asp Ala Asp Gly Ala Thr Trp Gly Ser Pro Ser Gly Ser
65                  70                  75                  80
Phe Ser Val Pro Tyr Gly His Asn Gly Leu Cys Thr Tyr Ser His Ile
                85                  90                  95
Arg Ser Gly Ile Ser Thr Ala Gly Asn Tyr Leu Ile Thr Pro Asn Ile
            100                 105                 110
Glu Gly Ala Lys Arg Val Lys Tyr Trp Val Cys Asn Gln Tyr Ser Thr
        115                 120                 125
Asn Pro Glu His Tyr Ala Val Met Val Ser Thr Thr Gly Thr Ala Ile
    130                 135                 140
Glu Asp Phe Val Leu Leu Phe Asp Asp Ser Ile Thr Gly Lys Pro Thr
145                 150                 155                 160
Pro Leu Val Trp Arg Arg Arg Ile Val Asp Leu Pro Glu Gly Thr Lys
                165                 170                 175
Tyr Ile Ala Trp Arg His Tyr Lys Val Thr Asp Ser His Thr Glu Phe
            180                 185                 190
Leu Lys Leu Asp Asp Val Thr Val Tyr Arg Ser Ile Glu Gly Pro Glu
        195                 200                 205
Pro Ala Thr Asp Phe Thr Val Ile Asn Ile Gly Gln Asn Val Gly Arg
    210                 215                 220
Leu Thr Trp Asn Tyr Pro Glu Asp Tyr Gln Pro Glu Gly Lys Gly Asn
225                 230                 235                 240
Glu Glu Leu Gln Leu Ser Gly Tyr Asn Ile Tyr Ala Asn Gly Thr Leu
                245                 250                 255
Leu Ala Gln Ile Lys Asp Val Ser Ile Leu Glu Tyr Val Asp Ser Thr
            260                 265                 270
Tyr Ser Leu Arg Asp Asn Pro Leu Gln Val Glu Tyr Cys Val Thr Ala
        275                 280                 285
Val Tyr Asp Glu Ser Ile Glu Ser Ser Thr Val Cys Gly Thr Leu His
    290                 295                 300
Tyr Ala Thr Asp Ala Ile Leu Tyr Glu Asn Phe Glu Asn Gly Pro Val
305                 310                 315                 320
Pro Asn Gly Trp Leu Val Ile Asp Ala Asp Gly Asp Gly Phe Ser Trp
                325                 330                 335
Gly His Tyr Leu Asn Ala Tyr Asp Ala Phe Pro Gly His Asn Gly Gly
            340                 345                 350
His Cys Ser Leu Ser Ala Ser Tyr Val Pro Gly Ile Gly Pro Val Thr
        355                 360                 365
Pro Asp Asn Tyr Leu Ile Thr Pro Lys Val Glu Gly Ala Lys Arg Val
    370                 375                 380
Lys Tyr Trp Val Ser Thr Gln Asp Ala Asn Trp Ala Ala Glu His Tyr
385                 390                 395                 400
Ala Val Met Ala Ser Thr Thr Gly Thr Ala Val Gly Asp Phe Val Ile
                405                 410                 415
Leu Phe Glu Glu Thr Met Thr Ala Lys Pro Thr Gly Ala Trp Tyr Glu
            420                 425                 430
Arg Thr Ile Asn Leu Pro Glu Gly Thr Lys Tyr Ile Ala Trp Arg His
        435                 440                 445
Tyr Asn Cys Thr Asp Ile Tyr Phe Leu Lys Leu Asp Asp Ile Thr Val
    450                 455                 460
Phe Gly Thr Pro Ala Ser Glu Pro Glu Pro Val Thr Asp Phe Val Val
465                 470                 475                 480
Ser Leu Ile Glu Asn Asn Lys Gly Arg Leu Lys Trp Asn Tyr Pro Asn
                485                 490                 495
Gly Tyr Glu Pro Asp Lys Thr Asp Asp Lys Asp Pro Leu Gln Leu Ala
            500                 505                 510
Gly Tyr Asn Ile Tyr Ala Asn Gly Ser Leu Leu Val His Ile Gln Asp
        515                 520                 525
Pro Thr Val Leu Glu Tyr Ile Asp Glu Thr Tyr Ser Ser Arg Asp Asp
    530                 535                 540
Gln Val Glu Val Glu Tyr Cys Val Thr Ala Val Tyr Asn Asp Asn Ile
545                 550                 555                 560
Glu Ser Gln Ser Val Cys Asp Lys Leu Ile Tyr Asp Ser Gln Ser Asp
                565                 570                 575
Ile Ile Leu Tyr Glu Gly Phe Glu Ala Gly Ser Ile Pro Glu Gly Trp
            580                 585                 590
Leu Leu Ile Asp Ala Asp Gly Asp Asn Val Asn Trp Asp Tyr Tyr Pro
        595                 600                 605
Trp Thr Met Tyr Gly His Asp Ser Glu Lys Cys Ile Ala Ser Pro Ser
    610                 615                 620
Tyr Leu Pro Met Ile Gly Val Leu Thr Pro Asp Asn Tyr Leu Val Thr

625                 630                 635                 640
Pro Arg Leu Glu Gly Ala Lys Leu Val Lys Tyr Trp Val Ser Ala Gln
                645                 650                 655
Asp Ala Val Tyr Ser Ala Glu His Tyr Ala Val Met Val Ser Thr Thr
            660                 665                 670
Gly Thr Ala Val Glu Asp Phe Val Leu Leu Phe Glu Glu Thr Met Thr
        675                 680                 685
Ala Lys Ala Asn Gly Ala Trp Tyr Glu Arg Thr Ile Thr Leu Pro Ala
    690                 695                 700
Gly Thr Lys Tyr Ile Ala Trp Arg His Tyr Asp Cys Thr Asp Met Phe
705                 710                 715                 720
Phe Leu Leu Leu Asp Asp Ile Thr Val Tyr Arg Ser Thr Glu Thr Val
                725                 730                 735
Pro Glu Pro Val Thr Asp Phe Val Val Ser Leu Ile Glu Asn Asn Lys
            740                 745                 750
Gly Arg Leu Lys Trp Asn Tyr Pro Asn Gly Tyr Glu Pro Asp Lys Thr
        755                 760                 765
Asp Asp Lys Lys Pro Leu Gln Leu Thr Gly Tyr Asn Ile Tyr Ala Asn
    770                 775                 780
Gly Ser Leu Leu Val His Ile Gln Asp Pro Thr Val Leu Glu Tyr Ile
785                 790                 795                 800
Asp Glu Thr Tyr Ser Ser Arg Asp Gly Gln Val Glu Met Glu Tyr Cys
                805                 810                 815
Val Thr Ala Val Tyr Asn Asp Asn Ile Glu Ser Gln Ser Val Cys Asp
            820                 825                 830
Lys Leu Asn Tyr Thr Ile Thr Ser Leu Asp Asn Ile Gln Ser Asp Thr
        835                 840                 845
Ser Leu Lys Ile Tyr Pro Asn Pro Ala Ser Tyr Val Val Arg Ile Glu
    850                 855                 860
Gly Leu Ser Arg Ser Lys Ser Thr Ile Glu Leu Tyr Asn Ala Leu Gly
865                 870                 875                 880
Ile Cys Ile Leu Arg Glu Glu Thr His Ser Glu Lys Thr Glu Ile Asp
                885                 890                 895
Val Ser Arg Leu Asn Asp Gly Val Tyr Leu Ile Lys Val Val Gly Gly
            900                 905                 910
Asn Lys Thr Thr Thr Glu Lys Val Glu Ile Lys Arg Pro
        915                 920                 925

(2) INFORMATION FOR SEQ ID NO:468

     (i) SEQUENCE CHARACTERISTICS:
          (A) LENGTH: 922 amino acids
          (B) TYPE: amino acid
          (D) TOPOLOGY: linear

    (ii) MOLECULE TYPE: protein

   (iii) HYPOTHETICAL: YES

    (vi) ORIGINAL SOURCE:
          (A) ORGANISM: Porphyromonas gingivalis

    (ix) FEATURE:
          (A) NAME/KEY: misc_feature
          (B) LOCATION 1...922

    (xi) SEQUENCE DESCRIPTION: SEQ ID NO:468

Met Met Lys Ser Ile Val Phe Arg Ala Phe Leu Thr Ile Leu Leu Ser
1               5                   10                  15
Trp Ala Ala Ile Thr Asn Pro Thr Ala Gln Glu Ile Ser Gly Met Asn
            20                  25                  30
Ala Ser Cys Leu Ala Ala Pro Ala Gln Pro Asp Thr Ile Leu Tyr Glu
        35                  40                  45
Ser Phe Glu Asn Gly Pro Val Pro Asn Gly Trp Leu Glu Ile Asp Ala
    50                  55                  60
Asp Ala Asp Gly Ala Thr Trp Gly Ser Pro Ser Gly Ser Phe Ser Val
65                  70                  75                  80
Pro Tyr Gly His Asn Gly Leu Cys Thr Tyr Ser His Ile Arg Ser Gly
                85                  90                  95
Ile Ser Thr Ala Gly Asn Tyr Leu Ile Thr Pro Asn Ile Glu Gly Ala
            100                 105                 110
Lys Arg Val Lys Tyr Trp Val Cys Asn Gln Tyr Ser Thr Asn Pro Glu
        115                 120                 125
His Tyr Ala Val Met Val Ser Thr Thr Gly Thr Ala Ile Glu Asp Phe

    130                 135                 140
Val Leu Leu Phe Asp Asp Ser Ile Thr Gly Lys Pro Thr Pro Leu Val
145                 150                 155                 160
Trp Arg Arg Arg Ile Val Asp Leu Pro Glu Gly Thr Lys Tyr Ile Ala
                165                 170                 175
Trp Arg His Tyr Lys Val Thr Asp Ser His Thr Glu Phe Leu Lys Leu
            180                 185                 190
Asp Asp Val Thr Val Tyr Arg Ser Ile Glu Gly Pro Glu Pro Ala Thr
        195                 200                 205
Asp Phe Thr Val Ile Asn Ile Gly Gln Asn Val Gly Arg Leu Thr Trp
    210                 215                 220
Asn Tyr Pro Glu Asp Tyr Gln Pro Glu Gly Lys Gly Asn Glu Glu Leu
225                 230                 235                 240
Gln Leu Ser Gly Tyr Asn Ile Tyr Ala Asn Gly Thr Leu Leu Ala Gln
                245                 250                 255
Ile Lys Asp Val Ser Ile Leu Glu Tyr Val Asp Ser Thr Tyr Ser Leu
            260                 265                 270
Arg Asp Asn Pro Leu Gln Val Glu Tyr Cys Val Thr Ala Val Tyr Asp
        275                 280                 285
Glu Ser Ile Glu Ser Ser Thr Val Cys Gly Thr Leu His Tyr Ala Thr
    290                 295                 300
Asp Ala Ile Leu Tyr Glu Asn Phe Glu Asn Gly Pro Val Pro Asn Gly
305                 310                 315                 320
Trp Leu Val Ile Asp Ala Asp Gly Asp Gly Phe Ser Trp Gly His Tyr
                325                 330                 335
Leu Asn Ala Tyr Asp Ala Phe Pro Gly His Asn Gly Gly His Cys Ser
            340                 345                 350
Leu Ser Ala Ser Tyr Val Pro Gly Ile Gly Pro Val Thr Pro Asp Asn
        355                 360                 365
Tyr Leu Ile Thr Pro Lys Val Glu Gly Ala Lys Arg Val Lys Tyr Trp
    370                 375                 380
Val Ser Thr Gln Asp Ala Asn Trp Ala Ala Glu His Tyr Ala Val Met
385                 390                 395                 400
Ala Ser Thr Thr Gly Thr Ala Val Gly Asp Phe Val Ile Leu Phe Glu
                405                 410                 415
Glu Thr Met Thr Ala Lys Pro Thr Gly Ala Trp Tyr Glu Arg Thr Ile
            420                 425                 430
Asn Leu Pro Glu Gly Thr Lys Tyr Ile Ala Trp Arg His Tyr Asn Cys
        435                 440                 445
Thr Asp Ile Tyr Phe Leu Lys Leu Asp Asp Ile Thr Val Phe Gly Thr
    450                 455                 460
Pro Ala Ser Glu Pro Glu Pro Val Thr Asp Phe Val Val Ser Leu Ile
465                 470                 475                 480
Glu Asn Asn Lys Gly Arg Leu Lys Trp Asn Tyr Pro Asn Gly Tyr Glu
                485                 490                 495
Pro Asp Lys Thr Asp Asp Lys Asp Pro Leu Gln Leu Ala Gly Tyr Asn
            500                 505                 510
Ile Tyr Ala Asn Gly Ser Leu Leu Val His Ile Gln Asp Pro Thr Val
        515                 520                 525
Leu Glu Tyr Ile Asp Glu Thr Tyr Ser Ser Arg Asp Asp Gln Val Glu
    530                 535                 540
Val Glu Tyr Cys Val Thr Ala Val Tyr Asn Asp Asn Ile Glu Ser Gln
545                 550                 555                 560
Ser Val Cys Asp Lys Leu Ile Tyr Asp Ser Gln Ser Asp Ile Ile Leu
                565                 570                 575
Tyr Glu Gly Phe Glu Ala Gly Ser Ile Pro Glu Gly Trp Leu Leu Ile
            580                 585                 590
Asp Ala Asp Gly Asp Asn Val Asn Trp Asp Tyr Tyr Pro Trp Thr Met
        595                 600                 605
Tyr Gly His Asp Ser Glu Lys Cys Ile Ala Ser Pro Ser Tyr Leu Pro
    610                 615                 620
Met Ile Gly Val Leu Thr Pro Asp Asn Tyr Leu Val Thr Pro Arg Leu
625                 630                 635                 640
Glu Gly Ala Lys Leu Val Lys Tyr Trp Val Ser Ala Gln Asp Ala Val
                645                 650                 655
Tyr Ser Ala Glu His Tyr Ala Val Met Val Ser Thr Thr Gly Thr Ala
            660                 665                 670
Val Glu Asp Phe Val Leu Leu Phe Glu Glu Thr Met Thr Ala Lys Ala
        675                 680                 685
Asn Gly Ala Trp Tyr Glu Arg Thr Ile Thr Leu Pro Ala Gly Thr Lys
    690                 695                 700
Tyr Ile Ala Trp Arg His Tyr Asp Cys Thr Asp Met Phe Phe Leu Leu
705                 710                 715                 720
Leu Asp Asp Ile Thr Val Tyr Arg Ser Thr Glu Thr Val Pro Glu Pro
                725                 730                 735

Val Thr Asp Phe Val Val Ser Leu Ile Glu Asn Asn Lys Gly Arg Leu
            740                 745                 750
Lys Trp Asn Tyr Pro Asn Gly Tyr Glu Pro Asp Lys Thr Asp Asp Lys
        755                 760                 765
Lys Pro Leu Gln Leu Thr Gly Tyr Asn Ile Tyr Ala Asn Gly Ser Leu
    770                 775                 780
Leu Val His Ile Gln Asp Pro Thr Val Leu Glu Tyr Ile Asp Glu Thr
785                 790                 795                 800
Tyr Ser Ser Arg Asp Gly Gln Val Glu Met Glu Tyr Cys Val Thr Ala
                805                 810                 815
Val Tyr Asn Asp Asn Ile Glu Ser Gln Ser Val Cys Asp Lys Leu Asn
            820                 825                 830
Tyr Thr Ile Thr Ser Leu Asp Asn Ile Gln Ser Asp Thr Ser Leu Lys
        835                 840                 845
Ile Tyr Pro Asn Pro Ala Ser Tyr Val Val Arg Ile Glu Gly Leu Ser
    850                 855                 860
Arg Ser Lys Ser Thr Ile Glu Leu Tyr Asn Ala Leu Gly Ile Cys Ile
865                 870                 875                 880
Leu Arg Glu Glu Thr His Ser Glu Lys Thr Glu Ile Asp Val Ser Arg
                885                 890                 895
Leu Asn Asp Gly Val Tyr Leu Ile Lys Val Val Gly Gly Asn Lys Thr
            900                 905                 910
Thr Thr Glu Lys Val Glu Ile Lys Arg Pro
        915                 920

(2) INFORMATION FOR SEQ ID NO:469

    (i) SEQUENCE CHARACTERISTICS:
        (A) LENGTH: 921 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...921

    (xi) SEQUENCE DESCRIPTION: SEQ ID NO:469

Met Lys Ser Ile Val Phe Arg Ala Phe Leu Thr Ile Leu Leu Ser Trp
1               5                   10                  15
Ala Ala Ile Thr Asn Pro Thr Ala Gln Glu Ile Ser Gly Met Asn Ala
            20                  25                  30
Ser Cys Leu Ala Ala Pro Ala Gln Pro Asp Thr Ile Leu Tyr Glu Ser
        35                  40                  45
Phe Glu Asn Gly Pro Val Pro Asn Gly Trp Leu Glu Ile Asp Ala Asp
    50                  55                  60
Ala Asp Gly Ala Thr Trp Gly Ser Pro Ser Gly Ser Phe Ser Val Pro
65                  70                  75                  80
Tyr Gly His Asn Gly Leu Cys Thr Tyr Ser His Ile Arg Ser Gly Ile
                85                  90                  95
Ser Thr Ala Gly Asn Tyr Leu Ile Thr Pro Asn Ile Glu Gly Ala Lys
            100                 105                 110
Arg Val Lys Tyr Trp Val Cys Asn Gln Tyr Ser Thr Asn Pro Glu His
        115                 120                 125
Tyr Ala Val Met Val Ser Thr Thr Gly Thr Ala Ile Glu Asp Phe Val
    130                 135                 140
Leu Leu Phe Asp Asp Ser Ile Thr Gly Lys Pro Thr Pro Leu Val Trp
145                 150                 155                 160
Arg Arg Arg Ile Val Asp Leu Pro Glu Gly Thr Lys Tyr Ile Ala Trp
                165                 170                 175
Arg His Tyr Lys Val Thr Asp Ser His Thr Glu Phe Leu Lys Leu Asp
            180                 185                 190
Asp Val Thr Val Tyr Arg Ser Ile Glu Gly Pro Glu Pro Ala Thr Asp
        195                 200                 205
Phe Thr Val Ile Asn Ile Gly Gln Asn Val Gly Arg Leu Thr Trp Asn
    210                 215                 220
Tyr Pro Glu Asp Tyr Gln Pro Glu Gly Lys Gly Asn Glu Glu Leu Gln
225                 230                 235                 240

Leu Ser Gly Tyr Asn Ile Tyr Ala Asn Gly Thr Leu Leu Ala Gln Ile
                245                 250                 255
Lys Asp Val Ser Ile Leu Glu Tyr Val Asp Ser Thr Tyr Ser Leu Arg
            260                 265                 270
Asp Asn Pro Leu Gln Val Glu Tyr Cys Val Thr Ala Val Tyr Asp Glu
        275                 280                 285
Ser Ile Glu Ser Ser Thr Val Cys Gly Thr Leu His Tyr Ala Thr Asp
    290                 295                 300
Ala Ile Leu Tyr Glu Asn Phe Glu Asn Gly Pro Val Pro Asn Gly Trp
305                 310                 315                 320
Leu Val Ile Asp Ala Asp Gly Asp Gly Phe Ser Trp Gly His Tyr Leu
                325                 330                 335
Asn Ala Tyr Asp Ala Phe Pro Gly His Asn Gly Gly His Cys Ser Leu
            340                 345                 350
Ser Ala Ser Tyr Val Pro Gly Ile Gly Pro Val Thr Pro Asp Asn Tyr
        355                 360                 365
Leu Ile Thr Pro Lys Val Glu Gly Ala Lys Arg Val Lys Tyr Trp Val
    370                 375                 380
Ser Thr Gln Asp Ala Asn Trp Ala Ala Glu His Tyr Ala Val Met Ala
385                 390                 395                 400
Ser Thr Thr Gly Thr Ala Val Gly Asp Phe Val Ile Leu Phe Glu Glu
                405                 410                 415
Thr Met Thr Ala Lys Pro Thr Gly Ala Trp Tyr Glu Arg Thr Ile Asn
            420                 425                 430
Leu Pro Glu Gly Thr Lys Tyr Ile Ala Trp Arg His Tyr Asn Cys Thr
        435                 440                 445
Asp Ile Tyr Phe Leu Lys Leu Asp Asp Ile Thr Val Phe Gly Thr Pro
    450                 455                 460
Ala Ser Glu Pro Glu Pro Val Thr Asp Phe Val Val Ser Leu Ile Glu
465                 470                 475                 480
Asn Asn Lys Gly Arg Leu Lys Trp Asn Tyr Pro Asn Gly Tyr Glu Pro
                485                 490                 495
Asp Lys Thr Asp Asp Lys Asp Pro Leu Gln Leu Ala Gly Tyr Asn Ile
            500                 505                 510
Tyr Ala Asn Gly Ser Leu Leu Val His Ile Gln Asp Pro Thr Val Leu
        515                 520                 525
Glu Tyr Ile Asp Glu Thr Tyr Ser Ser Arg Asp Asp Gln Val Glu Val
    530                 535                 540
Glu Tyr Cys Val Thr Ala Val Tyr Asn Asp Asn Ile Glu Ser Gln Ser
545                 550                 555                 560
Val Cys Asp Lys Leu Ile Tyr Asp Ser Gln Ser Asp Ile Ile Leu Tyr
                565                 570                 575
Glu Gly Phe Glu Ala Gly Ser Ile Pro Glu Gly Trp Leu Leu Ile Asp
            580                 585                 590
Ala Asp Gly Asp Asn Val Asn Trp Asp Tyr Tyr Pro Trp Thr Met Tyr
        595                 600                 605
Gly His Asp Ser Glu Lys Cys Ile Ala Ser Pro Ser Tyr Leu Pro Met
    610                 615                 620
Ile Gly Val Leu Thr Pro Asp Asn Tyr Leu Val Thr Pro Arg Leu Glu
625                 630                 635                 640
Gly Ala Lys Leu Val Lys Tyr Trp Val Ser Ala Gln Asp Ala Val Tyr
                645                 650                 655
Ser Ala Glu His Tyr Ala Val Met Val Ser Thr Thr Gly Thr Ala Val
            660                 665                 670
Glu Asp Phe Val Leu Leu Phe Glu Glu Thr Met Thr Ala Lys Ala Asn
        675                 680                 685
Gly Ala Trp Tyr Glu Arg Thr Ile Thr Leu Pro Ala Gly Thr Lys Tyr
    690                 695                 700
Ile Ala Trp Arg His Tyr Asp Cys Thr Asp Met Phe Phe Leu Leu Leu
705                 710                 715                 720
Asp Asp Ile Thr Val Tyr Arg Ser Thr Glu Thr Val Pro Glu Pro Val
                725                 730                 735
Thr Asp Phe Val Val Ser Leu Ile Glu Asn Asn Lys Gly Arg Leu Lys
            740                 745                 750
Trp Asn Tyr Pro Asn Gly Tyr Glu Pro Asp Lys Thr Asp Asp Lys Lys
        755                 760                 765
Pro Leu Gln Leu Thr Gly Tyr Asn Ile Tyr Ala Asn Gly Ser Leu Leu
    770                 775                 780
Val His Ile Gln Asp Pro Thr Val Leu Glu Tyr Ile Asp Glu Thr Tyr
785                 790                 795                 800
Ser Ser Arg Asp Gly Gln Val Glu Met Glu Tyr Cys Val Thr Ala Val
                805                 810                 815
Tyr Asn Asp Asn Ile Glu Ser Gln Ser Val Cys Asp Lys Leu Asn Tyr
            820                 825                 830
Thr Ile Thr Ser Leu Asp Asn Ile Gln Ser Asp Thr Ser Leu Lys Ile

835                 840                 845
Tyr Pro Asn Pro Ala Ser Tyr Val Val Arg Ile Glu Gly Leu Ser Arg
    850                 855                 860
Ser Lys Ser Thr Ile Glu Leu Tyr Asn Ala Leu Gly Ile Cys Ile Leu
865                 870                 875                 880
Arg Glu Glu Thr His Ser Glu Lys Thr Glu Ile Asp Val Ser Arg Leu
                885                 890                 895
Asn Asp Gly Val Tyr Leu Ile Lys Val Val Gly Gly Asn Lys Thr Thr
            900                 905                 910
Thr Glu Lys Val Glu Ile Lys Arg Pro
        915                 920

(2) INFORMATION FOR SEQ ID NO:470

    (i) SEQUENCE CHARACTERISTICS:
        (A) LENGTH: 593 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (D) LOCATION 1...593

    (xi) SEQUENCE DESCRIPTION: SEQ ID NO:470

Met Asn Ser Ile Met Lys Tyr Gln Leu Tyr Thr Ala Val Ile Met Ala
1               5                   10                  15
Leu Ser Val Ser Ser Val Cys Gly Gln Thr Pro Arg Asn Thr Glu Thr
            20                  25                  30
Lys Arg Pro Asp Thr Leu Arg Arg Glu Leu Thr Ile Val Asn Asp Gln
        35                  40                  45
Thr Val Glu Met Glu His Ala Asp Pro Leu Pro Ala Ala Tyr Lys Ala
    50                  55                  60
Ile Glu Pro Arg Leu Lys Pro Phe Arg Pro Glu Tyr Asn Lys Arg Thr
65                  70                  75                  80
Phe Gly Phe Val Pro Glu Val Ser Ser Ser Gly Arg Asn Asn Leu Pro
                85                  90                  95
Asn Ile Leu Pro Thr Glu Gly His Met Lys His Arg Gly Tyr Leu Asn
            100                 105                 110
Ile Gly Ile Gly His Thr Leu Asn Gln Arg Met Asp Ala Gly Tyr Arg
        115                 120                 125
Leu Ile Asp Ala Glu Gln Glu Arg Leu Asn Leu Phe Leu Ser Tyr Arg
    130                 135                 140
Gly Met Lys Ser Ala Phe Asn Thr Gly Asp Phe Asp Gly Asp Arg Lys
145                 150                 155                 160
Asp Arg Arg Met Met Ala Gly Val Asp Tyr Glu Gln Arg Arg Pro Ser
                165                 170                 175
Phe Val Leu Ala Thr Gly Leu Tyr Tyr Ser Asn His Tyr Phe Asn Asn
            180                 185                 190
Tyr Gly Arg Gly Ala Thr Thr Asn Val Gly Ser Ile Pro Gln Leu Ser
        195                 200                 205
Thr Pro Val Thr Pro Gln Met Asp Asn Gly Thr His Asn Val Arg Val
    210                 215                 220
Tyr Leu Gly Ala Lys Asn Asp Val Ile Asp Ala Arg Ile Asp Tyr Arg
225                 230                 235                 240
Phe Phe Arg Ser Ile Pro Tyr Leu Gly Thr Asp Pro Met Lys Ala Leu
                245                 250                 255
Thr Glu His Thr Pro Glu Leu Asn Val Thr Met Ser Asn Glu Leu Ser
            260                 265                 270
Asp Asp Ile Lys Leu Gly Val Glu Val Arg Thr Gly Gly Leu Phe Phe
        275                 280                 285
Ala Lys Asn Ser Glu Met Ile Gln Thr Gly Val Leu Ser Glu Thr Asp
    290                 295                 300
Arg Asn Leu Tyr Tyr Val Glu Gly Ala Pro Thr Ile Gly Phe Val Gly
305                 310                 315                 320
Asp Ser Asp Asn Met Gln Trp Asn Ile Gln Ala Gly Val Gly Ile Ser
                325                 330                 335
Ser His Phe Gly Ala Lys Gly Arg Leu Phe Phe Trp Pro Lys Leu Asp

340                 345                 350
Ala Ser Leu Ser Ile Phe Pro Ser Trp Arg Val Tyr Ala Lys Ala Phe
        355                 360                 365
Gly Gly Val Ile Arg Asn Gly Leu Ala Asp Val Met Gln Glu Glu Met
    370                 375                 380
Pro Tyr Leu Met Pro Asn Thr Ile Val Leu Pro Ser Arg Asn Ala Leu
385                 390                 395                 400
Thr Ala Gln Leu Gly Val Lys Gly Asn Ile Ala Asp Val Val Arg Met
                405                 410                 415
Glu Val Tyr Gly Asp Phe Ser Lys Leu Thr Gly Val Pro Phe Tyr Thr
            420                 425                 430
Pro Thr Leu Pro Leu Tyr Asn Pro Ser Asp Leu Tyr Gln Tyr Asn Val
        435                 440                 445
Ser Phe Leu Pro Ile Tyr Ala Asp Gly Ser Arg Trp Arg Ala Gly Gly
    450                 455                 460
Lys Leu Glu Tyr Ser Tyr Arg Asp Met Leu Arg Phe Leu Val Asp Ala
465                 470                 475                 480
Ser Tyr Gly Lys Trp Asn Leu Asp Gly Gly Leu Val Ala Ser Met Gln
                485                 490                 495
Pro Asp Leu Ile Leu Lys Ala Glu Val Gly Val His Pro Ile Ala Pro
            500                 505                 510
Leu Asp Val Arg Leu Arg Tyr Thr Gln Leu Asn Gly Arg Tyr Arg Tyr
        515                 520                 525
Ser Phe Gly Ser Ala Gly Ser Glu Ala Leu Gly Ile Gly Asn Val His
    530                 535                 540
Leu Leu Ser Ala Asp Val Ser Tyr Lys Leu Lys Lys Asn Leu Ser Leu
545                 550                 555                 560
Tyr Leu Lys Ile Asp Asn Met Leu Ala Glu Thr Thr Glu Leu Ile Gly
                565                 570                 575
Tyr Tyr Pro Met Gln Pro Phe His Cys Phe Ala Gly Phe Ser Trp Thr
            580                 585                 590
Phe

(2) INFORMATION FOR SEQ ID NO:471

(i) SEQUENCE CHARACTERISTICS:
(A) LENGTH: 589 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...589

(xi) SEQUENCE DESCRIPTION: SEQ ID NO:471

Met Lys Tyr Gln Leu Tyr Thr Ala Val Ile Met Ala Leu Ser Val Ser
1               5                   10                  15
Ser Val Cys Gly Gln Thr Pro Arg Asn Thr Glu Thr Lys Arg Pro Asp
            20                  25                  30
Thr Leu Arg Arg Glu Leu Thr Ile Val Asn Asp Gln Thr Val Glu Met
        35                  40                  45
Glu His Ala Asp Pro Leu Pro Ala Ala Tyr Lys Ala Ile Glu Pro Arg
    50                  55                  60
Leu Lys Pro Phe Arg Pro Glu Tyr Asn Lys Arg Thr Phe Gly Phe Val
65                  70                  75                  80
Pro Glu Val Ser Ser Ser Gly Arg Asn Asn Leu Pro Asn Ile Leu Pro
                85                  90                  95
Thr Glu Gly His Met Lys His Arg Gly Tyr Leu Asn Ile Gly Ile Gly
            100                 105                 110
His Thr Leu Asn Gln Arg Met Asp Ala Gly Tyr Arg Leu Ile Asp Ala
        115                 120                 125
Glu Gln Glu Arg Leu Asn Leu Phe Leu Ser Tyr Arg Gly Met Lys Ser
    130                 135                 140
Ala Phe Asn Thr Gly Asp Phe Asp Gly Asp Arg Lys Asp Arg Arg Met
145                 150                 155                 160
Met Ala Gly Val Asp Tyr Glu Gln Arg Arg Pro Ser Phe Val Leu Ala

                165                 170                 175
Thr Gly Leu Tyr Tyr Ser Asn His Tyr Phe Asn Asn Tyr Gly Arg Gly
            180                 185                 190
Ala Thr Thr Asn Val Gly Ser Ile Pro Gln Leu Ser Thr Pro Val Thr
        195                 200                 205
Pro Gln Met Asp Asn Gly Thr His Asn Val Arg Val Tyr Leu Gly Ala
    210                 215                 220
Lys Asn Asp Val Ile Asp Ala Arg Ile Asp Tyr Arg Phe Phe Arg Ser
225                 230                 235                 240
Ile Pro Tyr Leu Gly Thr Asp Pro Met Lys Ala Leu Thr Glu His Thr
                245                 250                 255
Pro Glu Leu Asn Val Thr Met Ser Asn Glu Leu Ser Asp Asp Ile Lys
            260                 265                 270
Leu Gly Val Glu Val Arg Thr Gly Gly Leu Phe Phe Ala Lys Asn Ser
        275                 280                 285
Glu Met Ile Gln Thr Gly Val Leu Ser Glu Thr Asp Arg Asn Leu Tyr
    290                 295                 300
Tyr Val Glu Gly Ala Pro Thr Ile Gly Phe Val Gly Asp Ser Asp Asn
305                 310                 315                 320
Met Gln Trp Asn Ile Gln Ala Gly Val Gly Ile Ser Ser His Phe Gly
                325                 330                 335
Ala Lys Gly Arg Leu Phe Phe Trp Pro Lys Leu Asp Ala Ser Leu Ser
            340                 345                 350
Ile Phe Pro Ser Trp Arg Val Tyr Ala Lys Ala Phe Gly Gly Val Ile
        355                 360                 365
Arg Asn Gly Leu Ala Asp Val Met Gln Glu Glu Met Pro Tyr Leu Met
    370                 375                 380
Pro Asn Thr Ile Val Leu Pro Ser Arg Asn Ala Leu Thr Ala Gln Leu
385                 390                 395                 400
Gly Val Lys Gly Asn Ile Ala Asp Val Val Arg Met Glu Val Tyr Gly
                405                 410                 415
Asp Phe Ser Lys Leu Thr Gly Val Pro Phe Tyr Thr Pro Thr Leu Pro
            420                 425                 430
Leu Tyr Asn Pro Ser Asp Leu Tyr Gln Tyr Asn Val Ser Phe Leu Pro
        435                 440                 445
Ile Tyr Ala Asp Gly Ser Arg Trp Arg Ala Gly Gly Lys Leu Glu Tyr
    450                 455                 460
Ser Tyr Arg Asp Met Leu Arg Phe Leu Val Asp Ala Ser Tyr Gly Lys
465                 470                 475                 480
Trp Asn Leu Asp Gly Gly Leu Val Ala Ser Met Gln Pro Asp Leu Ile
                485                 490                 495
Leu Lys Ala Glu Val Gly Val His Pro Ile Ala Pro Leu Asp Val Arg
            500                 505                 510
Leu Arg Tyr Thr Gln Leu Asn Gly Arg Tyr Arg Tyr Ser Phe Gly Ser
        515                 520                 525
Ala Gly Ser Glu Ala Leu Gly Ile Gly Asn Val His Leu Leu Ser Ala
    530                 535                 540
Asp Val Ser Tyr Lys Leu Lys Lys Asn Leu Ser Leu Tyr Leu Lys Ile
545                 550                 555                 560
Asp Asn Met Leu Ala Glu Thr Thr Glu Leu Ile Gly Tyr Tyr Pro Met
                565                 570                 575
Gln Pro Phe His Cys Phe Ala Gly Phe Ser Trp Thr Phe
            580                 585

(2) INFORMATION FOR SEQ ID NO:472

(i) SEQUENCE CHARACTERISTICS:
(A) LENGTH: 346 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...346

(xi) SEQUENCE DESCRIPTION: SEQ ID NO:472

Met Met Glu Lys Cys Ile Phe Ala His Tyr Pro His Asn Leu Val Phe

1                   5                   10                  15
Met Ile Arg Lys His Phe Gly Ile Ile Leu Gly Phe Leu Ser Leu Val
                20                  25                  30
Phe Ser Ala Gly Ala Gln Gln Glu Lys Gln Val Phe His Phe Leu Asn
        35                  40                  45
Leu Pro Ala Thr Ala Gln Ala Leu Ala Ala Gly Gly Lys Ala Ile Thr
    50                  55                  60
Ile Val Asp Asp Asn Pro Gly Leu Ala Phe Glu Asn Pro Ala Leu Leu
65                  70                  75                  80
Gly Tyr Glu Ser Gly Gly Arg Ala Phe Leu Ser Tyr Leu Tyr Tyr Met
                85                  90                  95
Ser Gly Ser His Met Gly Asn Ala Cys Tyr Ala Ser Ser Val Gly Glu
            100                 105                 110
Arg Gly Met Trp Gly Val Gly Met Arg Phe Leu Asn Tyr Gly Ser Met
        115                 120                 125
Gln Gly Tyr Asp Gln Asn Ala Ile Ala Thr Gly Ser Phe Ser Ala Ser
    130                 135                 140
Asp Ile Ala Val Gln Gly Phe Tyr Ser His Glu Leu Ser Asn His Phe
145                 150                 155                 160
Arg Gly Gly Val Ser Leu Lys Ala Leu Tyr Ser Ser Ile Glu Thr Tyr
                165                 170                 175
Ser Ser Phe Gly Leu Gly Val Asp Val Gly Ile Ser Tyr Tyr Asp Asp
            180                 185                 190
Asp Lys Gly Tyr Ser Ala Ser Ala Leu Phe Lys Asn Val Gly Ala Gln
        195                 200                 205
Leu Lys Gly Tyr Asn Glu Glu Arg Glu Pro Leu Asp Trp Asp Phe Gln
    210                 215                 220
Leu Gly Phe Ser Arg Ser Phe Ile Asn Ala Pro Phe Arg Leu His Ile
225                 230                 235                 240
Thr Leu Phe Asn Leu Asn Pro His Tyr Phe Lys Arg Leu Val Pro Arg
                245                 250                 255
Asp Leu Ser Lys Met Gln Lys Phe Leu Arg His Phe Ser Ile Gly Ala
            260                 265                 270
Glu Phe Thr Pro Ser Glu Arg Phe Trp Val Gly Leu Gly Tyr Thr Pro
        275                 280                 285
Gln Ile Ala Gln Asp Phe Glu Val Glu Gly Gly Asn Lys Trp Gly Gly
    290                 295                 300
Leu Ser Ala Gly Val Gly Phe Thr Ser Gly Val Val Arg Val Gly Val
305                 310                 315                 320
Ser Ala Ala Thr Tyr His Pro Ala Ala Leu Ser Phe Met Cys Ser Val
                325                 330                 335
Gly Ile Arg Leu Asp Asp Lys Ser Ile Phe
            340                 345

(2) INFORMATION FOR SEQ ID NO:473

    (i) SEQUENCE CHARACTERISTICS:
        (A) LENGTH: 345 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...345

    (xi) SEQUENCE DESCRIPTION: SEQ ID NO:473

Met Glu Lys Cys Ile Phe Ala His Tyr Pro His Asn Leu Val Phe Met
1               5                   10                  15
Ile Arg Lys His Phe Gly Ile Ile Leu Gly Phe Leu Ser Leu Val Phe
            20                  25                  30
Ser Ala Gly Ala Gln Gln Glu Lys Gln Val Phe His Phe Leu Asn Leu
        35                  40                  45
Pro Ala Thr Ala Gln Ala Leu Ala Ala Gly Gly Lys Ala Ile Thr Ile
    50                  55                  60
Val Asp Asp Asn Pro Gly Leu Ala Phe Glu Asn Pro Ala Leu Leu Gly
65                  70                  75                  80
Tyr Glu Ser Gly Gly Arg Ala Phe Leu Ser Tyr Leu Tyr Tyr Met Ser

                    85                  90                  95
Gly Ser His Met Gly Asn Ala Cys Tyr Ala Ser Ser Val Gly Glu Arg
                100                 105                 110
Gly Met Trp Gly Val Gly Met Arg Phe Leu Asn Tyr Gly Ser Met Gln
            115                 120                 125
Gly Tyr Asp Gln Asn Ala Ile Ala Thr Gly Ser Phe Ser Ala Ser Asp
    130                 135                 140
Ile Ala Val Gln Gly Phe Tyr Ser His Glu Leu Ser Asn His Phe Arg
145                 150                 155                 160
Gly Gly Val Ser Leu Lys Ala Leu Tyr Ser Ser Ile Glu Thr Tyr Ser
                165                 170                 175
Ser Phe Gly Leu Gly Val Asp Val Gly Ile Ser Tyr Tyr Asp Asp Asp
            180                 185                 190
Lys Gly Tyr Ser Ala Ser Ala Leu Phe Lys Asn Val Gly Ala Gln Leu
        195                 200                 205
Lys Gly Tyr Asn Glu Glu Arg Glu Pro Leu Asp Trp Asp Phe Gln Leu
    210                 215                 220
Gly Phe Ser Arg Ser Phe Ile Asn Ala Pro Phe Arg Leu His Ile Thr
225                 230                 235                 240
Leu Phe Asn Leu Asn Pro His Tyr Phe Lys Arg Leu Val Pro Arg Asp
                245                 250                 255
Leu Ser Lys Met Gln Lys Phe Leu Arg His Phe Ser Ile Gly Ala Glu
            260                 265                 270
Phe Thr Pro Ser Glu Arg Phe Trp Val Gly Leu Gly Tyr Thr Pro Gln
        275                 280                 285
Ile Ala Gln Asp Phe Glu Val Glu Gly Gly Asn Lys Trp Gly Gly Leu
    290                 295                 300
Ser Ala Gly Val Gly Phe Thr Ser Gly Val Val Arg Val Gly Val Ser
305                 310                 315                 320
Ala Ala Thr Tyr His Pro Ala Ala Leu Ser Phe Met Cys Ser Val Gly
                325                 330                 335
Ile Arg Leu Asp Asp Lys Ser Ile Phe
                340                 345

(2) INFORMATION FOR SEQ ID NO:474

    (i) SEQUENCE CHARACTERISTICS:
        (A) LENGTH: 330 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...330

    (xi) SEQUENCE DESCRIPTION: SEQ ID NO:474

Met Ile Arg Lys His Phe Gly Ile Ile Leu Gly Phe Leu Ser Leu Val
1               5                   10                  15
Phe Ser Ala Gly Ala Gln Gln Glu Lys Gln Val Phe His Phe Leu Asn
            20                  25                  30
Leu Pro Ala Thr Ala Gln Ala Leu Ala Ala Gly Gly Lys Ala Ile Thr
        35                  40                  45
Ile Val Asp Asp Asn Pro Gly Leu Ala Phe Glu Asn Pro Ala Leu Leu
    50                  55                  60
Gly Tyr Glu Ser Gly Gly Arg Ala Phe Leu Ser Tyr Leu Tyr Tyr Met
65                  70                  75                  80
Ser Gly Ser His Met Gly Asn Ala Cys Tyr Ala Ser Ser Val Gly Glu
                85                  90                  95
Arg Gly Met Trp Gly Val Gly Met Arg Phe Leu Asn Tyr Gly Ser Met
            100                 105                 110
Gln Gly Tyr Asp Gln Asn Ala Ile Ala Thr Gly Ser Phe Ser Ala Ser
        115                 120                 125
Asp Ile Ala Val Gln Gly Phe Tyr Ser His Glu Leu Ser Asn His Phe
    130                 135                 140
Arg Gly Gly Val Ser Leu Lys Ala Leu Tyr Ser Ser Ile Glu Thr Tyr
145                 150                 155                 160
Ser Ser Phe Gly Leu Gly Val Asp Val Gly Ile Ser Tyr Tyr Asp Asp

                165                 170                 175
Asp Lys Gly Tyr Ser Ala Ser Ala Leu Phe Lys Asn Val Gly Ala Gln
            180                 185                 190
Leu Lys Gly Tyr Asn Glu Glu Arg Glu Pro Leu Asp Trp Asp Phe Gln
        195                 200                 205
Leu Gly Phe Ser Arg Ser Phe Ile Asn Ala Pro Phe Arg Leu His Ile
    210                 215                 220
Thr Leu Phe Asn Leu Asn Pro His Tyr Phe Lys Arg Leu Val Pro Arg
225                 230                 235                 240
Asp Leu Ser Lys Met Gln Lys Phe Leu Arg His Phe Ser Ile Gly Ala
                245                 250                 255
Glu Phe Thr Pro Ser Glu Arg Phe Trp Val Gly Leu Gly Tyr Thr Pro
            260                 265                 270
Gln Ile Ala Gln Asp Phe Glu Val Glu Gly Gly Asn Lys Trp Gly Gly
        275                 280                 285
Leu Ser Ala Gly Val Gly Phe Thr Ser Gly Val Val Arg Val Gly Val
    290                 295                 300
Ser Ala Ala Thr Tyr His Pro Ala Ala Leu Ser Phe Met Cys Ser Val
305                 310                 315                 320
Gly Ile Arg Leu Asp Asp Lys Ser Ile Phe
                325                 330

(2) INFORMATION FOR SEQ ID NO:475

(i) SEQUENCE CHARACTERISTICS:
(A) LENGTH: 324 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...324

(xi) SEQUENCE DESCRIPTION: SEQ ID NO:475

Met Cys Leu Glu Pro Ile Ile Ala Pro Ile Ser Ser Glu Leu Leu Glu
1               5                   10                  15
Gln Glu Leu Thr Ala Asp Arg Phe Leu Arg Met Thr Asn Lys Ala Gly
            20                  25                  30
Asn Glu Ile Tyr Val Phe Thr Ala Glu Glu Ala Pro His Cys Met Lys
        35                  40                  45
Glu Val Gly Arg Leu Arg Glu Glu Ala Phe Arg His Tyr Gly Gly Gly
    50                  55                  60
Thr Gly Lys Ala Ile Asp Ile Asp Glu Phe Asp Thr Met Pro Gly Ser
65                  70                  75                  80
Tyr Lys Gln Leu Ile Val Trp Asp Pro Gln Asn Lys Ala Ile Leu Gly
                85                  90                  95
Gly Tyr Arg Phe Ile Tyr Gly Arg Asp Val Ala Phe Asp Thr Asp Gly
            100                 105                 110
Lys Pro Leu Leu Ala Thr Ala Glu Met Phe Arg Phe Ser Asp Ala Phe
        115                 120                 125
Leu His Asp Tyr Leu Pro Tyr Thr Val Glu Leu Gly Arg Ser Phe Val
    130                 135                 140
Ser Leu Gln Tyr Gln Ser Thr Arg Met Gly Thr Lys Ala Ile Phe Val
145                 150                 155                 160
Leu Asp Asn Leu Trp Asp Gly Ile Gly Ala Leu Thr Val Val Asn Pro
                165                 170                 175
Glu Ala Leu Tyr Phe Tyr Gly Lys Val Thr Met Tyr Lys Asp Tyr Asp
            180                 185                 190
Arg Arg Ala Arg Asn Leu Ile Leu Tyr Phe Leu Arg Lys His Phe Ser
        195                 200                 205
Asp Pro Glu Gly Leu Val Lys Pro Ile His Pro Leu Pro Ile Glu Ile
    210                 215                 220
Ser Ala Glu Asp Glu Ala Leu Phe Ser Ser Ser Asp Phe Asp Thr Asn
225                 230                 235                 240
Tyr Lys Thr Leu Asn Ile Glu Val Arg Lys Leu Gly Ile Asn Ile Pro
                245                 250                 255
Pro Leu Val Ser Ala Tyr Ile Ala Leu Ser Pro Glu Met Arg Val Phe

260                 265                 270
Gly Thr Ala Val Asn Glu Ser Phe Gly Glu Val Glu Glu Thr Gly Ile
        275                 280                 285
Phe Ile Ala Val Gly Lys Ile Leu Glu Glu Lys Lys Gln Arg His Ile
    290                 295                 300
Glu Ser Phe Ile Leu Ser Arg Asn Glu Lys Lys Gly Leu Asp Ser Ser
305                 310                 315                 320
Asn Gly Arg Ser

(2) INFORMATION FOR SEQ ID NO:476

(i) SEQUENCE CHARACTERISTICS:
(A) LENGTH: 547 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...547

(xi) SEQUENCE DESCRIPTION: SEQ ID NO:476

Met Lys Thr Ile Val Arg Tyr Ser Arg Leu Pro Val Ala Leu Phe Phe
1               5                   10                  15
Cys Leu Leu Gly Ala Val His Leu Ser Val Glu Ala Gln Met Leu Asn
            20                  25                  30
Thr Pro Phe Glu Leu Ser Asp Gln Ile Val Leu Ser Pro Thr Glu Arg
        35                  40                  45
Gln Tyr Arg Glu Ile Cys Val Gln Thr Lys Glu Lys Arg Gly Ala Asp
    50                  55                  60
Leu Phe Pro Leu Ser Asp Lys Leu Arg Asp Ser Ala Tyr Val Arg Phe
65                  70                  75                  80
Gly Ser Ala Tyr Gly Asp Ile Ala Gly Asp Tyr Leu Pro Tyr Asn Gly
                85                  90                  95
Asn Asn Tyr Ser Ser Leu Ser Leu Glu Ser Gly Gly Arg Ile Ser Val
            100                 105                 110
Arg Asn Tyr Gly Thr Leu Gln Gly Ser Ala Ser Tyr Ser Arg Gly Met
        115                 120                 125
His Lys Arg Ile Gly Trp Asn Ala Leu Arg Asn Ala Glu Ala Tyr Tyr
    130                 135                 140
Pro Tyr Leu Val Ser Asp Ser Thr Gly Gly Asp Tyr His Phe Glu Asp
145                 150                 155                 160
Tyr Arg Leu Ala Gly Tyr Tyr Ser Phe Arg Ala Gly Arg Leu Pro Leu
                165                 170                 175
Gly Ile Gly Phe Ser Tyr Arg Gly Glu Val Ala Tyr Arg Leu Thr Asp
            180                 185                 190
Pro Arg Thr Thr Asn Thr Thr Gly Ala Leu Glu Leu Ser Cys Ala Thr
        195                 200                 205
Ser Leu Thr Leu Pro Arg Glu Asn Arg Leu Ser Leu Ser Ala Ala Tyr
    210                 215                 220
Leu Tyr His Arg Gln His Leu Thr Gln Tyr Asn Trp Arg Pro Gly Gln
225                 230                 235                 240
Gln Asp Lys Phe Phe Val Ser Tyr Gly Phe Gly Gln Val Asp Val Ser
                245                 250                 255
Asn Ser Pro Ile Trp Phe Gly Ile Ser Arg Met Asn Tyr Val Asn Gly
            260                 265                 270
Trp Lys Leu Ser Ser Arg Leu Asp Thr Arg Arg Gly Asp Ala Ile Gly
        275                 280                 285
Leu Asp Tyr Ser Gly Tyr Phe Leu Asp Thr Glu Glu Arg Ser Ser Ile
    290                 295                 300
Asn Leu Phe Ala Leu Leu Tyr Asn Arg Leu Arg Leu Tyr Gly Ser Trp
305                 310                 315                 320
His Leu Ser Asp Phe Asp Phe Ser Phe Ser Ala Asp Tyr Ala Leu Arg
                325                 330                 335
Gln Gly Ile Glu Arg Ile Tyr Glu Asp Tyr Lys Pro Asp Asp Asn Tyr
            340                 345                 350
His Ile Tyr Asp Leu Arg Ile Leu Ala Ile Arg Arg Trp Tyr Met Leu

355                 360                 365
Asn Glu Phe Ser Ala Gln Ala Gln Ala Ser Tyr Arg Ile Arg Thr Asp
    370                 375                 380
Arg Gly Cys Ala Leu Arg Val Ser Ala Gly Ser Asp Phe Tyr Gly Tyr
385                 390                 395                 400
Asp Glu Thr Tyr Arg Lys His Gly His His Thr Met Ser Gly Met Leu
                405                 410                 415
Arg Pro Phe Ala Gly Ile Ala Tyr Asp His Ala Gly Ser Lys Leu Asp
            420                 425                 430
Phe Gly Leu Ser Leu Ser Ala Ala Tyr Arg Met Val Leu Thr His Ser
        435                 440                 445
Tyr Lys Ile Arg Thr Ile Gln Lys Glu Gln Leu Asp Tyr Gln Leu Ala
    450                 455                 460
Tyr Leu Pro Tyr Ala Tyr Arg Asn Arg Glu Gly Val Glu Val Arg Ser
465                 470                 475                 480
Ser Leu Tyr Val Ser Ile Pro Met Gln Asn Thr His Arg Leu Met Thr
                485                 490                 495
Glu Leu Arg Leu Tyr Gly Asp Leu Met Lys Arg Lys Asp Gly Ile Ala
            500                 505                 510
Tyr Gly Lys Thr Pro Gly Val Ile Ser His Ile Leu Ser Asp Pro Gln
        515                 520                 525
Ala Glu Arg Thr Ser Gly His Thr Ile Gly Ala Ile Cys Asn Ile Ser
    530                 535                 540
Tyr Leu Phe
545

(2) INFORMATION FOR SEQ ID NO:477

(i) SEQUENCE CHARACTERISTICS:
(A) LENGTH: 750 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...750

(xi) SEQUENCE DESCRIPTION: SEQ ID NO:477

Met Lys Lys Leu His Met Ile Ala Ala Leu Ala Val Leu Pro Phe Cys
1               5                   10                  15
Leu Thr Ala Gln Ala Pro Val Ser Asn Ser Glu Ile Asp Ser Leu Ser
            20                  25                  30
Asn Val Gln Leu Gln Thr Val Gln Val Val Ala Thr Arg Ala Thr Ala
        35                  40                  45
Lys Thr Pro Val Ala Tyr Thr Asn Val Arg Lys Ala Glu Leu Ser Lys
    50                  55                  60
Ser Asn Tyr Gly Arg Asp Ile Pro Tyr Leu Leu Met Leu Thr Pro Ser
65                  70                  75                  80
Val Val Ala Thr Ser Asp Ala Gly Thr Gly Ile Gly Tyr Ser Gly Phe
                85                  90                  95
Arg Val Arg Gly Thr Asp Ala Asn Arg Ile Asn Ile Thr Thr Asn Gly
            100                 105                 110
Val Pro Leu Asn Asp Ser Glu Ser Gln Ser Val Phe Trp Val Asn Met
        115                 120                 125
Pro Asp Phe Ala Ser Ser Ile Glu Asp Leu Gln Val Gln Arg Gly Val
    130                 135                 140
Gly Thr Ser Thr Asn Gly Ala Gly Ala Phe Gly Ala Ser Val Asn Met
145                 150                 155                 160
Arg Thr Asp Asn Leu Gly Leu Ala Pro Tyr Gly Arg Val Asp Leu Ser
                165                 170                 175
Gly Gly Ser Phe Gly Thr Phe Arg Arg Ser Val Lys Leu Gly Ser Gly
            180                 185                 190
Arg Ile Gly Arg His Trp Ala Val Asp Ala Arg Leu Ser Lys Ile Gly
        195                 200                 205
Ser Asp Gly Tyr Val Asp Arg Gly Ser Val Asp Leu Lys Ser Tyr Phe
    210                 215                 220
Ala Gln Val Gly Tyr Phe Gly Ser Asn Thr Ala Leu Arg Phe Ile Thr

225                 230                 235                 240
Phe Gly Gly Lys Glu Val Thr Gly Ile Ala Trp Asn Gly Leu Ser Lys
                245                 250                 255
Glu Asp Glu Ala Lys Tyr Gly Arg Arg Tyr Asn Ser Ala Gly Leu Met
            260                 265                 270
Tyr Val Asp Ala Gln Gly Val Pro His Tyr Tyr His Asn Thr Asp Asn
        275                 280                 285
Tyr Glu Gln Arg His Tyr His Ala Ile Met Thr His Ser Phe Ser Pro
    290                 295                 300
Ser Val Ile Leu Asn Leu Thr Ala His Tyr Thr Ala Gly Tyr Gly Tyr
305                 310                 315                 320
Thr Asp Glu Tyr Arg Thr Gly Arg Lys Leu Lys Glu Tyr Ala Leu Gln
                325                 330                 335
Pro Tyr Val Glu Asn Ser Val Thr Val Lys Lys Thr Asp Leu Ile Arg
            340                 345                 350
Gln Lys Tyr Leu Asp Asn Asp Phe Gly Gly Leu Ile Gly Ser Leu Asn
        355                 360                 365
Trp His Thr Gly Ala Trp Asp Leu Gln Phe Gly Ala Ser Gly Asn Ile
    370                 375                 380
Tyr Lys Gly Asp His Phe Gly Arg Ile Thr Tyr Ile Lys Lys Tyr Asn
385                 390                 395                 400
Gln Pro Leu Ala Pro Asp Phe Glu Tyr Tyr Arg Asn Arg Ala Asp Lys
                405                 410                 415
Arg Glu Gly Ala Ala Phe Ala Lys Ala Asn Trp Gln Ile Thr Pro Glu
            420                 425                 430
Leu Asn Met Tyr Ala Asp Leu Gln Tyr Arg Thr Ile Gly Tyr Thr Ile
        435                 440                 445
Asn Gly Ile Thr Asp Glu Tyr Asp Glu Val Gln Gly Ser Met Gln His
    450                 455                 460
Ile Asp Leu Asp Lys Thr Phe Arg Phe Leu Asn Pro Lys Ala Gly Leu
465                 470                 475                 480
Thr Tyr Ser Phe Asp Asp Ala His Thr Ala Tyr Ala Ser Val Ala Val
                485                 490                 495
Ala His Arg Glu Pro Asn Arg Thr Asn Tyr Thr Glu Ala Gly Ile Gly
            500                 505                 510
Gln Tyr Pro Thr Pro Glu Arg Leu Ile Asp Tyr Glu Leu Gly Tyr Arg
        515                 520                 525
Tyr Ala Ser Pro Leu Leu Ser Ala Gly Val Gly Leu Tyr Tyr Met Gln
    530                 535                 540
Tyr Lys Asp Gln Leu Val Leu Asp Gly Arg Leu Ser Asp Val Gly Gln
545                 550                 555                 560
Met Leu Thr Ser Asn Val Pro Asp Ser Tyr Arg Met Gly Leu Glu Leu
                565                 570                 575
Thr Leu Gly Trp Gln Ile Leu Pro Arg Leu Leu Arg Trp Asp Ala Ser
            580                 585                 590
Phe Thr Met Ser Arg Asn Lys Ile Asp Arg Tyr Val Gln Tyr Thr Ser
        595                 600                 605
Val Tyr Asp Ala Asp Tyr Asn Trp Leu Glu Leu Lys Glu Glu Thr Leu
    610                 615                 620
Glu Ser Thr Asp Ile Ala Tyr Ser Pro Asn Val Ile Ala Gly Ser Met
625                 630                 635                 640
Leu Thr Leu Ser His Ala Gly Phe Glu Met Ala Trp Thr Ser Arg Phe
                645                 650                 655
Val Ser Lys Gln Tyr Leu Asp Asn Thr Gln Arg Ser Asp Arg Met Leu
            660                 665                 670
Ser Ser Tyr Trp Val Asn Asp Leu Arg Leu Gly Tyr Val Leu Pro Val
        675                 680                 685
His Phe Val Lys Arg Val Ala Leu Gly Val Gln Leu Asn Asn Leu Phe
    690                 695                 700
Asn Leu Met Tyr Ala Ser Asn Ala Tyr Ile Tyr Asp Ala Gly Tyr Val
705                 710                 715                 720
Gln Ala Ser Gly Glu Leu Ser Ala Tyr Ala Asp Leu Arg Tyr Tyr Pro
                725                 730                 735
Gln Ala Gly Phe Asn Ala Leu Gly Ser Leu Thr Ile Asp Phe
            740                 745                 750

(2) INFORMATION FOR SEQ ID NO:478

     (i) SEQUENCE CHARACTERISTICS:
          (A) LENGTH: 494 amino acids
          (B) TYPE: amino acid
          (D) TOPOLOGY: linear

     (ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...494

(xi) SEQUENCE DESCRIPTION: SEQ ID NO:478

Met Lys Arg Arg Phe Leu Ser Leu Leu Leu Leu Tyr Ile Leu Ser Ser
1               5                   10                  15
Ile Ser Leu Ser Ala Gln Arg Phe Pro Met Val Gln Gly Ile Glu Leu
            20                  25                  30
Asp Thr Asp Ser Leu Phe Ser Leu Pro Lys Arg Pro Trp Arg Ala Ile
        35                  40                  45
Gly Lys Thr Ile Gly Val Asn Leu Ala Val Trp Gly Phe Asp His Phe
    50                  55                  60
Ile Met Asn Glu Asp Phe Ala Asp Ile Ser Trp Gln Thr Ile Lys Ser
65                  70                  75                  80
Asn Phe Gln Thr Gly Phe Gly Trp Asp Asn Asp Lys Phe Val Thr Asn
                85                  90                  95
Leu Phe Ala His Pro Tyr His Gly Ser Leu Tyr Phe Asn Ala Ala Arg
            100                 105                 110
Ser Asn Gly Leu Ser Phe Arg His Ser Ala Pro Phe Ala Phe Phe Gly
        115                 120                 125
Ser Leu Met Trp Glu Leu Leu Met Glu Asn Glu Pro Pro Ser Ile Asn
    130                 135                 140
Asp Leu Cys Ala Thr Thr Ile Gly Gly Ile Ala Leu Gly Glu Met Gly
145                 150                 155                 160
His Arg Leu Ser Asp Leu Leu Ile Asp Asn Arg Thr Thr Gly Trp Glu
                165                 170                 175
Arg Met Gly Arg Glu Val Ala Ile Ala Leu Ile Asn Pro Met Arg Phe
            180                 185                 190
Leu Asn Arg Leu Thr Ala Gly Glu Val Thr Ser Val Gly Ser Arg Ser
        195                 200                 205
Gly Gln Ile Phe Gln Ser Val Pro Ile Asn Ile Val Val Asp Ala Gly
    210                 215                 220
Phe Arg Phe Leu Ala Asp Lys Arg His Ala Arg Thr Gly Ala Thr Ala
225                 230                 235                 240
Leu Thr Leu Asn Leu Arg Phe Asp Tyr Gly Asp Pro Phe Arg Ser Glu
                245                 250                 255
Thr Phe Ser Pro Tyr Asp Phe Phe Gln Phe Lys Ala Gly Leu Ser Phe
            260                 265                 270
Ser Glu Ser Gln Pro Leu Leu Ser Gln Ile Asn Leu Ile Gly Ile Leu
        275                 280                 285
Ser Gly Cys Gln Leu Leu Ala His Glu Arg Thr Val Leu Val Gly Gly
    290                 295                 300
Leu Phe Gln His Phe Asp Tyr Tyr Asn Ser Glu Lys Arg Ile Ser Lys
305                 310                 315                 320
Asn Ser Glu Glu Val Leu Val Thr Pro Tyr Arg Ile Ser Gln Val Ala
                325                 330                 335
Ala Leu Gly Gly Gly Leu Ile Phe Gln His His Gly Lys Phe Arg Arg
            340                 345                 350
Arg Pro Leu Glu Leu Tyr Ala Glu Thr Tyr Leu Asn Val Val Pro Met
        355                 360                 365
Gly Ala Ser Leu Ser Asp His Tyr Asn Val Asp Asn Arg Asp Tyr Asn
    370                 375                 380
Leu Gly Ser Gly Leu Ser Gly Lys Leu Tyr Leu Gly Ala Thr Tyr Asn
385                 390                 395                 400
Asp Leu Trp Ser Trp Leu Leu Gly Val Glu Ser Tyr Arg Leu Tyr Thr
                405                 410                 415
Trp Ile Gly Tyr Glu Glu Pro His Gln Lys Asn Thr Asp Val Ser Ser
            420                 425                 430
Phe Met Val Gln Gly Asp Glu Ser Lys Ala Arg Leu Leu Val Thr Ser
        435                 440                 445
Ser Glu Phe Ala Phe His Pro Gly Pro Trp His Val Ala Ile Val Ala
    450                 455                 460
Arg Arg Phe Ile Arg Lys Thr Ala Tyr Gln Phe Tyr Pro Asn Val Ser
465                 470                 475                 480
Phe Asp Thr Gly Asp Ile Gln Leu Arg Val Gly Phe His Phe
                485                 490

(2) INFORMATION FOR SEQ ID NO:479

(i) SEQUENCE CHARACTERISTICS:
(A) LENGTH: 294 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...294

(xi) SEQUENCE DESCRIPTION: SEQ ID NO:479

Met Lys Arg Leu Ile Val Phe Leu Ala Met Gly Gly Leu Leu Phe Thr
1               5                   10                  15
Leu Ala Asn Ala Gln Glu Ala Asn Thr Ala Ser Asp Thr Pro Lys Lys
            20                  25                  30
Asp Trp Thr Ile Lys Gly Val Thr Gly Leu Asn Ala Ser Gln Thr Ser
        35                  40                  45
Leu Thr Asn Trp Ala Ala Gly Gly Glu Asn Thr Val Ala Gly Asn Leu
    50                  55                  60
Tyr Leu Asn Ile Asp Ala Asn Tyr Leu Lys Asp Lys Trp Ser Trp Asp
65                  70                  75                  80
Asn Gly Leu Arg Thr Asp Phe Gly Leu Thr Tyr Thr Thr Ala Asn Lys
                85                  90                  95
Trp Asn Lys Ser Val Asp Lys Ile Glu Leu Phe Thr Lys Ala Gly Tyr
            100                 105                 110
Glu Ile Gly Lys His Trp Tyr Gly Ser Ala Leu Phe Thr Phe Leu Ser
        115                 120                 125
Gln Tyr Ala Lys Gly Tyr Glu Lys Pro Ser Asp His Leu Thr Gly Val
    130                 135                 140
Lys His Ile Ser Asn Phe Phe Ala Pro Ala Tyr Leu Thr Leu Gly Ile
145                 150                 155                 160
Gly Ala Asp Tyr Lys Pro Asn Glu Lys Phe Ser Leu Tyr Leu Ser Pro
                165                 170                 175
Thr Thr Gly Lys Leu Thr Val Val Ala Asp Asp Tyr Leu Ser Ser Leu
            180                 185                 190
Gly Ala Phe Gly Val Lys Val Gly Glu Lys Thr Met Phe Glu Leu Gly
        195                 200                 205
Ala Leu Val Val Gly Ser Ala Asn Ile Asn Leu Met Glu Asn Val Asn
    210                 215                 220
Leu Ile Thr Lys Ala Ser Phe Phe Ser Ala Tyr Thr His Asp Phe Gly
225                 230                 235                 240
Asn Ile Asp Ile Asn Trp Glu Ala Met Leu Ala Met Lys Ile Asn Lys
                245                 250                 255
Phe Leu Thr Ala Thr Ile Ala Thr Asn Leu Ile Tyr Asp Asp Asp Val
            260                 265                 270
Lys Ile Asn Asp Gly Pro Lys Ile Gln Phe Lys Glu Val Val Gly Val
        275                 280                 285
Gly Val Ala Tyr Thr Phe
    290

(2) INFORMATION FOR SEQ ID NO:480

(i) SEQUENCE CHARACTERISTICS:
(A) LENGTH: 204 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...204

(xi) SEQUENCE DESCRIPTION: SEQ ID NO:480

Met Lys Lys Met Ile Leu Ala Ala Thr Met Leu Leu Ala Thr Ile Gly
1               5                   10                  15
Phe Ala Asn Ala Gln Ser Arg Pro Ala Leu Arg Leu Asp Ala Asn Phe
            20                  25                  30
Val Gly Ser Asn Leu Met Gln Lys Val Ala Asn Thr Ser Val Asn Asn
        35                  40                  45
Lys Met Ile Val Gly Leu Arg Val Gly Ala Ala Ala Glu Phe Ala Leu
    50                  55                  60
Ser Asn Asp Gly Phe Tyr Leu Ala Pro Gly Leu Ala Tyr Thr Met Arg
65                  70                  75                  80
Gly Ala Lys Met Glu Ser Leu Ser Glu Thr Thr Thr Arg Leu His Tyr
                85                  90                  95
Leu Gln Ile Pro Val Asn Ala Gly Met Arg Phe Ser Phe Ala Asp Asn
            100                 105                 110
Met Ala Ile Ser Leu Glu Ala Gly Pro Tyr Phe Ala Tyr Gly Val Ala
        115                 120                 125
Gly Thr Ile Lys Thr Lys Val Ala Gly Val Thr Ala Ser Val Asp Ala
    130                 135                 140
Phe Gly Asp Asn Gly Tyr Asn Arg Phe Asp Leu Gly Leu Gly Leu Ser
145                 150                 155                 160
Ala Ala Leu Ser Tyr Asp Arg Tyr Tyr Val Gln Ile Gly Tyr Glu His
                165                 170                 175
Gly Leu Leu Asn Met Leu Lys Asp Ala Pro Asp Lys Thr Ser Leu Arg
            180                 185                 190
Asn His Asp Phe Phe Val Gly Leu Gly Val Arg Phe
        195                 200

(2) INFORMATION FOR SEQ ID NO:481

    (i) SEQUENCE CHARACTERISTICS:
        (A) LENGTH: 243 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...243

    (xi) SEQUENCE DESCRIPTION: SEQ ID NO:481

Met Lys Arg Ile Phe Thr Val Ala Leu Val Leu Leu Ala Ser Val Thr
1               5                   10                  15
Met Ala Ile Gly Gln Ser Arg Pro Ala Leu Arg Val Asp Ala Asn Phe
            20                  25                  30
Val Gly Ser Asn Gln Ser Met Lys Arg Asp Gly Tyr Val Trp Asp Thr
        35                  40                  45
Lys Met Asn Val Gly Leu Arg Val Gly Ala Ala Ala Glu Phe Met Ile
    50                  55                  60
Gly Ser Arg Gly Phe Tyr Leu Ala Pro Gly Leu Asn Tyr Thr Met Lys
65                  70                  75                  80
Gly Ser Lys Thr Glu Trp Asp Ile Pro Glu Met Val Pro Gly Thr Tyr
                85                  90                  95
Ile Thr Met Val Ser Thr Arg Leu His Tyr Leu Gln Leu Pro Ile Asn
            100                 105                 110
Ala Gly Met Arg Phe Asp Leu Met Asn Asp Met Ala Val Ser Ile Glu
        115                 120                 125
Ala Gly Pro Phe Leu Ala Tyr Gly Ile Tyr Gly Thr Tyr Arg Gln Lys
    130                 135                 140
Leu Glu Gly Trp Lys Pro Asn Asn Tyr Ser Thr Glu Phe Phe Gly Pro
145                 150                 155                 160
Thr Leu Gly Gly Pro Thr Asn Ile Arg Trp Asp Ile Gly Ala Asn Ile
                165                 170                 175
Ile Ala Ala Phe His Tyr Lys Arg Tyr Tyr Ile Gln Ile Gly Tyr Glu
            180                 185                 190
His Gly Phe Val Asp Ile Val Ser Gly Gly Gly Ser Asp Ile Pro Arg

            195                     200                     205
Leu Asn Asp Asn Arg Gln Ser Ser Ser Thr Thr Ala Leu Arg Glu Lys
    210                     215                     220
Gly Asn Asn Glu Tyr Ala Tyr Asn Arg Asp Phe Phe Val Gly Ile Gly
225                     230                     235                     240
Tyr Arg Phe

(2) INFORMATION FOR SEQ ID NO:482

     (i) SEQUENCE CHARACTERISTICS:
          (A) LENGTH: 207 amino acids
          (B) TYPE: amino acid
          (D) TOPOLOGY: linear

     (ii) MOLECULE TYPE: protein

    (iii) HYPOTHETICAL: YES

     (vi) ORIGINAL SOURCE:
          (A) ORGANISM: Porphyromonas gingivalis

     (ix) FEATURE:
          (A) NAME/KEY: misc_feature
          (B) LOCATION 1...207

     (xi) SEQUENCE DESCRIPTION: SEQ ID NO:482

Met Lys Arg Met Leu Leu Leu Leu Val Val Leu Leu Tyr Gly Ile Ala
1               5                   10                  15
Gly Arg Leu Ala Ala Gln Asp Val Ile Arg Pro Trp Ser Leu Gln Val
            20                  25                  30
Gly Ala Gly Tyr Ser Asp Thr Glu Asn Ile Pro Gly Gly Phe Thr Tyr
        35                  40                  45
Gly Phe Tyr Leu Gly Lys Arg Met Gly Ser Phe Leu Glu Val Gly Leu
    50                  55                  60
Ser Met Tyr Asn Ser Thr Arg Gln Thr Ala Asn Asn Ala Asp Ser Phe
65                  70                  75                  80
Ala Ser Asn Glu Gly Asp Gly Ser Phe Gln Val Asn Met Ser Ser Pro
                85                  90                  95
Asn Glu Lys Trp Ser Phe Phe Asp Ala Gly Ser Ala Asn Cys Tyr Met
            100                 105                 110
Ile Val Val Gly Val Asn Pro Leu His Leu Phe Trp Gln Asn Ser Arg
        115                 120                 125
His Asn Leu Phe Leu Ala Val Gln Ala Gly Leu Ser Asn Lys His Asn
    130                 135                 140
Ile His Phe Ile Tyr Gly Asp Lys Gly Ala Lys Val Ser Ile Tyr Thr
145                 150                 155                 160
Asn Ser Asn Thr Tyr Ile Gly Tyr Gly Ala Arg Val Ala Tyr Glu Tyr
                165                 170                 175
Gln Ile His Lys Asn Val Gly Ala Gly Ala Ala Val Met Tyr Asp His
            180                 185                 190
Gly Asn Lys Met Leu Thr Ala Met Ala Thr Leu Ser Thr His Phe
        195                 200                 205

(2) INFORMATION FOR SEQ ID NO:483

     (i) SEQUENCE CHARACTERISTICS:
          (A) LENGTH: 951 amino acids
          (B) TYPE: amino acid
          (D) TOPOLOGY: linear

     (ii) MOLECULE TYPE: protein

    (iii) HYPOTHETICAL: YES

     (vi) ORIGINAL SOURCE:
          (A) ORGANISM: Porphyromonas gingivalis

     (ix) FEATURE:
          (A) NAME/KEY: misc_feature
          (B) LOCATION 1...951

     (xi) SEQUENCE DESCRIPTION: SEQ ID NO:483

Met Arg Val Ser Asp Leu Cys Ser Arg Leu Ser Trp Leu Leu Pro Val
1               5                   10                  15
Ile Leu Val Gly Leu Leu Cys Ala Thr Leu Val Ala Ala Glu Arg Pro
            20                  25                  30
Met Ala Gly Ala Val Gly Leu His His Arg Arg His Ala Ala Leu Ser
        35                  40                  45
Asp Ser Thr Ala Lys Asp Thr Val Pro Leu Ala Lys Pro Ile Pro Asp
    50                  55                  60
Ser Ala Phe Arg Asp Ser Leu Pro Ala Asp Ser Thr Gly Ser Met Arg
65                  70                  75                  80
Gln Asp Ser Val Tyr Asp Asp Glu Phe Glu Leu Glu Asp Ile Val Glu
                85                  90                  95
Tyr Glu Ala Ala Asp Ser Ile Val Leu Leu Gly Gln Asn Arg Ala Tyr
            100                 105                 110
Leu Phe Gly Lys Ser Tyr Val Ser Tyr Gln Lys Ser Arg Leu Glu Ala
        115                 120                 125
Asn Phe Met Tyr Leu Asn Thr Asp Ser Ser Thr Val Tyr Thr Arg Tyr
    130                 135                 140
Val Leu Asp Thr Ala Gly Tyr Pro Met Ala Phe Pro Val Phe Lys Asp
145                 150                 155                 160
Gly Glu Gln Ser Phe Glu Ala Lys Asn Phe Thr Tyr Asn Phe Arg Thr
                165                 170                 175
Glu Lys Gly Ile Ile Ser Gly Val Ile Thr Gln Gln Gly Glu Gly Tyr
            180                 185                 190
Leu Thr Ala Gly Lys Thr Lys Lys Met Pro Asp Asn Ile Met Phe Met
        195                 200                 205
Gln Gly Gly Arg Tyr Thr Thr Cys Asp Asn His Asp His Pro His Phe
    210                 215                 220
Tyr Ile Asn Leu Ser Lys Ala Lys Val His Pro Glu Lys Asp Ile Val
225                 230                 235                 240
Thr Gly Pro Val Asn Leu Val Ile Ala Asp Met Pro Leu Pro Ile Gly
                245                 250                 255
Leu Pro Phe Gly Tyr Phe Pro Phe Ser Asn Lys Tyr Ser Ser Gly Ile
            260                 265                 270
Leu Met Pro Thr Tyr Gly Glu Asp Asn Arg Tyr Gly Phe Tyr Leu Arg
        275                 280                 285
Asn Gly Gly Tyr Tyr Phe Ala Phe Ser Asp Tyr Ile Asp Leu Ala Leu
    290                 295                 300
Arg Gly Glu Ile Phe Ser Lys Gly Ser Trp Gly Ile Ser Ala Gln Ser
305                 310                 315                 320
Lys Tyr Lys Lys Arg Tyr Lys Tyr Asn Gly Ser Phe Glu Ala Asn Tyr
                325                 330                 335
Leu Val Ser Lys Ser Gly Asp Lys Tyr Val Pro Gly Asp Tyr Ser Lys
            340                 345                 350
Thr Thr Ser Leu Asn Ile Arg Trp Thr His Ser Gln Asp Pro Lys Ala
        355                 360                 365
Asn Pro Leu Gln Thr Leu Ser Ala Asn Val Asn Phe Ala Thr Gly Ser
    370                 375                 380
Tyr Phe Gln Asn Ser Leu Asn Thr Thr Tyr Asp Val Asn Ala Arg Thr
385                 390                 395                 400
Ala Thr Thr Arg Ser Ser Ala Val Ser Tyr Ser Arg Lys Phe Pro Gly
                405                 410                 415
Thr Pro Phe Ser Ile Thr Gly Ser Met Asp Ile Ser Gln Asn Met Arg
            420                 425                 430
Asp Thr Thr Val Ser Leu Thr Leu Pro Asn Leu Ser Ile Asn Met Ser
        435                 440                 445
Thr Arg Tyr Pro Phe Lys Arg Lys Thr Arg Val Gly Pro Glu Arg Trp
    450                 455                 460
Tyr Glu Lys Leu Ser Val Gly Tyr Ser Gly Gln Leu Arg Asn Ser Ile
465                 470                 475                 480
Leu Thr Lys Glu Lys Asp Leu Leu Gln Ser Asn Leu Val Arg Asp Trp
                485                 490                 495
Lys Asn Gly Met Arg His Ser Val Pro Ile Ser Leu Thr Val Pro Leu
            500                 505                 510
Leu Asp Tyr Ile Asn Leu Thr Met Gly Val Asn Tyr Asn Glu Trp Trp
        515                 520                 525
Tyr Thr Lys Gly Ile Arg Lys Ser Trp Asn Glu Asp Lys Lys Thr Phe
    530                 535                 540
Leu Pro Ser Asp Thr Thr Tyr Lys Phe Arg Arg Leu Tyr Asp Tyr Ser
545                 550                 555                 560
Leu Ser Ala Gly Leu Ser Thr Thr Leu Tyr Gly Met Phe Lys Pro Trp
                565                 570                 575
Lys Pro Phe Ser Phe Gly Gly Asn Leu Ile Met Ile Arg His Arg Phe
            580                 585                 590
Thr Pro Thr Val Ser Phe Ser Tyr Met Pro Asp Phe Thr Lys Arg Arg

595                 600                 605
Tyr Gly Phe Trp Glu Leu Leu Glu His Thr Asp Gln Asn Gly Lys Leu
    610                 615                 620
His Thr Leu Leu Tyr Ser Pro Tyr Phe Glu Gln Ile Phe Gly Ala Pro
625                 630                 635                 640
Ser Met Gly Asn Ala Gly Ser Val Asn Phe Ser Phe Asp Asn Asn Leu
                645                 650                 655
Glu Ala Lys Ile Lys Ser Lys Ser Asp Ser Thr Gly Ile Lys Lys Ile
            660                 665                 670
Ser Leu Ile Asp Gln Phe Thr Trp Ser Thr Ser Tyr Asn Met Phe Ala
        675                 680                 685
Asp Ser Ile Arg Trp Ser Asn Ile Ser Ala Ser Leu Ala Leu Arg Leu
    690                 695                 700
Ser Lys Ser Phe Thr Leu Arg Leu Ser Gly Leu Phe Asp Pro Tyr Leu
705                 710                 715                 720
Thr Lys Tyr Tyr Glu Gly Glu Asp Gly Lys Ile Ile Pro Tyr Lys Ser
                725                 730                 735
Asn Asp Leu Arg Ile Phe Asn Gly Lys Gly Leu Ala Arg Leu Ile Ser
            740                 745                 750
Thr Gly Thr Ser Phe Ser Tyr Thr Leu Asn Lys Glu Ser Leu Ser Gly
        755                 760                 765
Leu Ile Ala Leu Phe Ser Gly Lys Lys Glu Arg Arg Asp Glu Lys Lys
    770                 775                 780
Asn Thr Gly Ala Thr Pro His Glu Gly Asp Asp Ala Ala Asp Ile Leu
785                 790                 795                 800
Glu Gly Gly Arg Pro Gln Asn Glu Ser Gly Gly Ser Leu Leu Glu Arg
                805                 810                 815
Asn Arg Gln Gly Gly Ala Val Asp Gln Asp Gly Tyr Phe Ala Tyr Ser
            820                 825                 830
Ile Pro Trp Ser Leu Ser Phe Asp Tyr Ser Trp Asn Ile Ala Thr Asp
        835                 840                 845
Tyr Asn Arg Tyr Asn Val Asn Lys Met Glu His Tyr Tyr Arg Val Thr
    850                 855                 860
Gln Asn Leu Ser Phe Arg Gly Asn Ile Gln Pro Thr Pro Asn Trp Ser
865                 870                 875                 880
Phe Gly Phe Asn Ala Asn Tyr Asn Phe Asp Leu Lys Lys Ile Thr Ser
                885                 890                 895
Leu Thr Cys Asn Val Thr Arg Asp Met His Cys Trp Ala Ile Ser Ala
            900                 905                 910
Ser Phe Ile Pro Ile Gly Ala Tyr Lys Ser Tyr Asn Phe Val Ile Ser
        915                 920                 925
Val Lys Ser Ser Leu Leu Gln Asp Leu Lys Tyr Gln Gln Ser Asn Arg
    930                 935                 940
Pro Ile Thr Asn Thr Trp Tyr
945                 950

(2) INFORMATION FOR SEQ ID NO:484

     (i) SEQUENCE CHARACTERISTICS:
          (A) LENGTH: 1226 amino acids
          (B) TYPE: amino acid
          (D) TOPOLOGY: linear

    (ii) MOLECULE TYPE: protein

   (iii) HYPOTHETICAL: YES

    (vi) ORIGINAL SOURCE:
          (A) ORGANISM: Porphyromonas gingivalis

    (ix) FEATURE:
          (A) NAME/KEY: misc_feature
          (B) LOCATION 1...1226

    (xi) SEQUENCE DESCRIPTION: SEQ ID NO:484

Met Met Lys Arg Tyr Thr Ile Ile Leu Ala Val Phe Leu Leu Phe Cys
1               5                   10                  15
Thr Val Phe Thr Phe Gln Ile Lys Ala Arg Pro Tyr Glu Arg Phe Ala
            20                  25                  30
Asp Val Glu Lys Pro Trp Ile Gln Lys His Ser Met Asp Ser Lys Leu
        35                  40                  45
Val Pro Ala Asn Lys Gly Asn Leu Ile Gln Ala Glu Ile Val Tyr Gln
    50                  55                  60
Ser Val Ser Glu His Ser Asp Leu Val Ile Ser Pro Val Asn Glu Ile

65                  70                  75                  80
Arg Pro Ala Asn Arg Phe Pro Ser His Arg Lys Ser Phe Phe Ala Glu
                85                  90                  95
Asn Leu Arg Ala Ser Pro Pro Val Val Pro Val Ala Val Asp Lys Tyr
            100                 105                 110
Ala Val Pro Val Ala Asn Pro Met Asp Pro Glu Asn Pro Asn Ala Trp
        115                 120                 125
Asp Val Thr Leu Lys Ile Thr Thr Lys Ala Val Thr Val Pro Val Asp
    130                 135                 140
Val Val Met Val Ile Asp Gln Ser Ser Ser Met Gly Gly Gln Asn Ile
145                 150                 155                 160
Ala Arg Leu Lys Ser Ala Ile Ala Ser Gly Gln Arg Phe Val Lys Lys
                165                 170                 175
Met Leu Pro Lys Gly Thr Ala Thr Glu Gly Val Arg Ile Ala Leu Val
            180                 185                 190
Ser Tyr Asp His Glu Pro His Arg Leu Ser Asp Phe Thr Lys Asp Thr
        195                 200                 205
Ala Phe Leu Cys Gln Lys Ile Arg Ala Leu Thr Pro Ile Trp Gly Thr
    210                 215                 220
His Thr Gln Gly Gly Leu Lys Met Ala Arg Asn Ile Met Ala Thr Ser
225                 230                 235                 240
Thr Ala Val Asp Lys His Ile Ile Leu Met Ser Asp Gly Leu Ala Thr
                245                 250                 255
Glu Gln Tyr Pro Val Lys Asn Val Thr Thr Ala Asp Phe Ile Gly Lys
            260                 265                 270
Thr Gly Asn Ala Asn Asp Pro Ile Asp Leu Val Ile Gln Gly Ala Ile
        275                 280                 285
Asn Phe Pro Thr Asn Tyr Val Ser Asn Asn Pro Ser Thr Pro Leu Thr
    290                 295                 300
Pro Asn Tyr Pro Thr His Ser Ser Lys Val Gly Arg Arg Asn Leu Pro
305                 310                 315                 320
Glu Ser Lys Phe Asp Tyr Ser Asn Leu Ser Ala Arg Ile Thr Phe Asp
                325                 330                 335
Gly Val Ala Gly Ala Leu Val Tyr Glu Pro Arg Phe Pro His Pro Tyr
            340                 345                 350
Tyr Tyr Tyr Phe Pro Cys Asn Ala Ala Ile Asn Glu Ala Gln Phe Ala
        355                 360                 365
Lys Asn Ser Gly Tyr Thr Ile His Thr Ile Gly Tyr Asp Leu Gly Asp
    370                 375                 380
Phe Ala Leu Ala Asn Asn Ser Leu Lys Leu Thr Ala Thr Asp Glu Asn
385                 390                 395                 400
His Phe Phe Thr Ala Thr Pro Ala Asn Leu Ala Ala Ala Phe Asp Asn
                405                 410                 415
Ile Ala Gln Thr Ile Asn Ile Gly Ile Gln Arg Gly Glu Val Thr Asp
            420                 425                 430
Phe Val Ala Pro Gly Phe Ile Val Lys Asn Leu Thr Gln Ser Gly Asp
        435                 440                 445
Val Thr His Leu Leu Asn Val Ser Asn Gly Thr Val His Tyr Asp Val
    450                 455                 460
Ser Thr Lys Lys Leu Thr Trp Thr Thr Gly Thr Ile Leu Ser Ser Ser
465                 470                 475                 480
Glu Ala Thr Ile Thr Tyr Arg Ile Tyr Ala Asp Leu Asp Tyr Ile Gln
                485                 490                 495
Asn Asn Asp Ile Pro Val Asn Thr Thr Ser Ala Ile Gly Pro Asp Leu
            500                 505                 510
Gly Gly Phe Asp Thr Asn Thr Glu Ala Lys Leu Thr Tyr Thr Asn Ser
        515                 520                 525
Asn Gly Glu Pro Asn Gln Gln Leu Ile Phe Pro Arg Pro Thr Val Lys
    530                 535                 540
Leu Gly Tyr Gly Val Ile Lys Arg His Tyr Val Leu Val Asn Lys Asp
545                 550                 555                 560
Gly Gln Pro Ile Gln Ala Asn Gly Thr Val Val Ser Ser Leu Ser Glu
                565                 570                 575
Ala His Val Leu Gln Ser Gln Asp Phe Phe Leu Pro Ser Gly Gly Gly
            580                 585                 590
His Ile Val Pro Lys Trp Ile Lys Leu Asp Lys Thr Thr Glu Ala Leu
        595                 600                 605
Gln Tyr Tyr Ser Val Pro Pro Thr Asn Thr Val Ile Thr Thr Ala Asp
    610                 615                 620
Gly Lys Arg Tyr Arg Phe Val Glu Val Pro Gly Ser Thr Pro Asn Pro
625                 630                 635                 640
Gly Gln Ile Gly Ile Ser Trp Lys Lys Pro Ala Gly Asn Ala Tyr Phe
                645                 650                 655
Ala Tyr Lys Leu Leu Asn Tyr Trp Met Gly Gly Thr Thr Asp Gln Gln
            660                 665                 670

Ser Glu Trp Asp Val Thr Ser Asn Trp Thr Gly Ala Gln Val Pro Leu
        675                 680                 685
Thr Gly Glu Asp Val Glu Phe Ala Thr Thr Glu Asn Phe Gly Ser Pro
    690                 695                 700
Ala Val Ala Asp Leu His Val Pro Thr Thr Asn Pro Lys Ile Ile Gly
705                 710                 715                 720
Asn Leu Ile Asn Asn Ser Asp Lys Asp Leu Val Val Thr Thr Ser Ser
                725                 730                 735
Gln Leu Thr Ile Asn Gly Val Val Glu Asp Asn Asn Pro Asn Val Gly
            740                 745                 750
Thr Ile Val Val Lys Ser Ser Lys Asp Asn Pro Thr Gly Thr Leu Leu
        755                 760                 765
Phe Ala Asn Pro Gly Tyr Asn Gln Asn Val Gly Gly Thr Val Glu Phe
    770                 775                 780
Tyr Asn Gln Gly Tyr Asp Cys Ala Asp Cys Gly Met Tyr Arg Arg Ser
785                 790                 795                 800
Trp Gln Tyr Phe Gly Ile Pro Val Asn Glu Ser Gly Phe Pro Ile Asn
                805                 810                 815
Asp Val Gly Gly Asn Glu Thr Val Asn Gln Trp Val Glu Pro Phe Asn
            820                 825                 830
Gly Asp Lys Trp Arg Pro Ala Pro Tyr Ala Pro Asp Thr Glu Leu Gln
        835                 840                 845
Lys Phe Lys Gly Tyr Gln Ile Thr Asn Asp Val Gln Ala Gln Pro Thr
    850                 855                 860
Gly Val Tyr Ser Phe Lys Gly Met Ile Cys Val Cys Asp Ala Phe Leu
865                 870                 875                 880
Asn Leu Thr Arg Thr Ser Gly Val Asn Tyr Ser Gly Ala Asn Leu Ile
                885                 890                 895
Gly Asn Ser Tyr Thr Gly Ala Ile Asp Ile Lys Gln Gly Ile Val Phe
            900                 905                 910
Pro Pro Glu Val Glu Gln Thr Val Tyr Leu Phe Asn Thr Gly Thr Arg
        915                 920                 925
Asp Gln Trp Arg Lys Leu Asn Gly Ser Thr Val Ser Gly Tyr Arg Ala
    930                 935                 940
Gly Gln Tyr Leu Ser Val Pro Lys Asn Thr Ala Gly Gln Asp Asn Leu
945                 950                 955                 960
Pro Asp Arg Ile Pro Ser Met His Ser Phe Leu Val Lys Met Gln Asn
                965                 970                 975
Gly Ala Ser Cys Thr Leu Unk Ile Leu Tyr Asp Lys Leu Leu Lys Asn
            980                 985                 990
Thr Thr Val Asn Asn Gly Asn Gly Thr Gln Ile Thr Trp Arg Ser Gly
        995                 1000                1005
Asn Ser Gly Ser Ala Asn Met Pro Ser Leu Val Met Asp Val Leu Gly
    1010                1015                1020
Asn Glu Ser Ala Asp Arg Leu Trp Ile Phe Thr Asp Gly Gly Leu Ser
1025                1030                1035                1040
Phe Gly Phe Asp Asn Gly Trp Asp Gly Arg Lys Leu Thr Glu Lys Gly
                1045                1050                1055
Leu Ser Gln Leu Tyr Ala Met Ser Asp Ile Gly Asn Asp Lys Phe Gln
            1060                1065                1070
Val Ala Gly Val Pro Glu Leu Asn Asn Leu Leu Ile Gly Phe Asp Ala
        1075                1080                1085
Asp Lys Asp Gly Gln Tyr Thr Leu Glu Phe Ala Leu Ser Asp His Phe
    1090                1095                1100
Ala Lys Gly Ala Val Tyr Leu His Asp Leu Gln Ser Gly Ala Lys His
1105                1110                1115                1120
Arg Ile Thr Asn Ser Thr Ser Tyr Ser Phe Asp Ala Lys Arg Gly Asp
                1125                1130                1135
Ser Gly Ala Arg Phe Arg Leu Ser Tyr Gly Cys Asp Glu Asn Val Asp
            1140                1145                1150
Asp Ser His Val Val Ser Thr Asn Gly Arg Glu Ile Ile Ile Leu Asn
        1155                1160                1165
Gln Asp Ala Leu Asp Cys Thr Val Thr Leu Phe Thr Ile Glu Gly Lys
    1170                1175                1180
Leu Leu Arg Arg Leu Lys Val Leu Ala Gly His Arg Glu Val Met Lys
1185                1190                1195                1200
Val Gln Thr Gly Gly Ala Tyr Ile Val His Leu Gln Asn Ala Phe Thr
                1205                1210                1215
Asn Asp Val His Lys Val Leu Val Glu Tyr
            1220                1225

(2) INFORMATION FOR SEQ ID NO:485

    (i) SEQUENCE CHARACTERISTICS:
        (A) LENGTH: 1225 amino acids

(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...1225

(xi) SEQUENCE DESCRIPTION: SEQ ID NO:485

Met Lys Arg Tyr Thr Ile Ile Leu Ala Val Phe Leu Leu Phe Cys Thr
1               5                   10                  15
Val Phe Thr Phe Gln Ile Lys Ala Arg Pro Tyr Glu Arg Phe Ala Asp
            20                  25                  30
Val Glu Lys Pro Trp Ile Gln Lys His Ser Met Asp Ser Lys Leu Val
        35                  40                  45
Pro Ala Asn Lys Gly Asn Leu Ile Gln Ala Glu Ile Val Tyr Gln Ser
    50                  55                  60
Val Ser Glu His Ser Asp Leu Val Ile Ser Pro Val Asn Glu Ile Arg
65                  70                  75                  80
Pro Ala Asn Arg Phe Pro Ser His Arg Lys Ser Phe Phe Ala Glu Asn
                85                  90                  95
Leu Arg Ala Ser Pro Pro Val Val Pro Val Ala Val Asp Lys Tyr Ala
            100                 105                 110
Val Pro Val Ala Asn Pro Met Asp Pro Glu Asn Pro Asn Ala Trp Asp
        115                 120                 125
Val Thr Leu Lys Ile Thr Thr Lys Ala Val Thr Val Pro Val Asp Val
    130                 135                 140
Val Met Val Ile Asp Gln Ser Ser Ser Met Gly Gly Gln Asn Ile Ala
145                 150                 155                 160
Arg Leu Lys Ser Ala Ile Ala Ser Gly Gln Arg Phe Val Lys Lys Met
                165                 170                 175
Leu Pro Lys Gly Thr Ala Thr Glu Gly Val Arg Ile Ala Leu Val Ser
            180                 185                 190
Tyr Asp His Glu Pro His Arg Leu Ser Asp Phe Thr Lys Asp Thr Ala
        195                 200                 205
Phe Leu Cys Gln Lys Ile Arg Ala Leu Thr Pro Ile Trp Gly Thr His
    210                 215                 220
Thr Gln Gly Gly Leu Lys Met Ala Arg Asn Ile Met Ala Thr Ser Thr
225                 230                 235                 240
Ala Val Asp Lys His Ile Ile Leu Met Ser Asp Gly Leu Ala Thr Glu
                245                 250                 255
Gln Tyr Pro Val Lys Asn Val Thr Thr Ala Asp Phe Ile Gly Lys Thr
            260                 265                 270
Gly Asn Ala Asn Asp Pro Ile Asp Leu Val Ile Gln Gly Ala Ile Asn
        275                 280                 285
Phe Pro Thr Asn Tyr Val Ser Asn Asn Pro Ser Thr Pro Leu Thr Pro
    290                 295                 300
Asn Tyr Pro Thr His Ser Ser Lys Val Gly Arg Arg Asn Leu Pro Glu
305                 310                 315                 320
Ser Lys Phe Asp Tyr Ser Asn Leu Ser Ala Arg Ile Thr Phe Asp Gly
                325                 330                 335
Val Ala Gly Ala Leu Val Tyr Glu Pro Arg Phe Pro His Pro Tyr Tyr
            340                 345                 350
Tyr Tyr Phe Pro Cys Asn Ala Ala Ile Asn Glu Ala Gln Phe Ala Lys
        355                 360                 365
Asn Ser Gly Tyr Thr Ile His Thr Ile Gly Tyr Asp Leu Gly Asp Phe
    370                 375                 380
Ala Leu Ala Asn Asn Ser Leu Lys Leu Thr Ala Thr Asp Glu Asn His
385                 390                 395                 400
Phe Phe Thr Ala Thr Pro Ala Asn Leu Ala Ala Ala Phe Asp Asn Ile
                405                 410                 415
Ala Gln Thr Ile Asn Ile Gly Ile Gln Arg Gly Glu Val Thr Asp Phe
            420                 425                 430
Val Ala Pro Gly Phe Ile Val Lys Asn Leu Thr Gln Ser Gly Asp Val
        435                 440                 445
Thr His Leu Leu Asn Val Ser Asn Gly Thr Val His Tyr Asp Val Ser
    450                 455                 460
Thr Lys Lys Leu Thr Trp Thr Thr Gly Thr Ile Leu Ser Ser Ser Glu

465                 470                 475                 480
Ala Thr Ile Thr Tyr Arg Ile Tyr Ala Asp Leu Asp Tyr Ile Gln Asn
                485                 490                 495
Asn Asp Ile Pro Val Asn Thr Thr Ser Ala Ile Gly Pro Asp Leu Gly
            500                 505                 510
Gly Phe Asp Thr Asn Thr Glu Ala Lys Leu Thr Tyr Thr Asn Ser Asn
        515                 520                 525
Gly Glu Pro Asn Gln Gln Leu Ile Phe Pro Arg Pro Thr Val Lys Leu
    530                 535                 540
Gly Tyr Gly Val Ile Lys Arg His Tyr Val Leu Val Asn Lys Asp Gly
545                 550                 555                 560
Gln Pro Ile Gln Ala Asn Gly Thr Val Val Ser Ser Leu Ser Glu Ala
                565                 570                 575
His Val Leu Gln Ser Gln Asp Phe Phe Leu Pro Ser Gly Gly Gly His
            580                 585                 590
Ile Val Pro Lys Trp Ile Lys Leu Asp Lys Thr Thr Glu Ala Leu Gln
        595                 600                 605
Tyr Tyr Ser Val Pro Pro Thr Asn Thr Val Ile Thr Thr Ala Asp Gly
    610                 615                 620
Lys Arg Tyr Arg Phe Val Glu Val Pro Gly Ser Thr Pro Asn Pro Gly
625                 630                 635                 640
Gln Ile Gly Ile Ser Trp Lys Lys Pro Ala Gly Asn Ala Tyr Phe Ala
                645                 650                 655
Tyr Lys Leu Leu Asn Tyr Trp Met Gly Gly Thr Thr Asp Gln Gln Ser
            660                 665                 670
Glu Trp Asp Val Thr Ser Asn Trp Thr Gly Ala Gln Val Pro Leu Thr
        675                 680                 685
Gly Glu Asp Val Glu Phe Ala Thr Thr Glu Asn Phe Gly Ser Pro Ala
    690                 695                 700
Val Ala Asp Leu His Val Pro Thr Thr Asn Pro Lys Ile Ile Gly Asn
705                 710                 715                 720
Leu Ile Asn Asn Ser Asp Lys Asp Leu Val Val Thr Thr Ser Ser Gln
                725                 730                 735
Leu Thr Ile Asn Gly Val Val Glu Asp Asn Asn Pro Asn Val Gly Thr
            740                 745                 750
Ile Val Val Lys Ser Ser Lys Asp Asn Pro Thr Gly Thr Leu Leu Phe
        755                 760                 765
Ala Asn Pro Gly Tyr Asn Gln Asn Val Gly Gly Thr Val Glu Phe Tyr
    770                 775                 780
Asn Gln Gly Tyr Asp Cys Ala Asp Cys Gly Met Tyr Arg Arg Ser Trp
785                 790                 795                 800
Gln Tyr Phe Gly Ile Pro Val Asn Glu Ser Gly Phe Pro Ile Asn Asp
                805                 810                 815
Val Gly Gly Asn Glu Thr Val Asn Gln Trp Val Glu Pro Phe Asn Gly
            820                 825                 830
Asp Lys Trp Arg Pro Ala Pro Tyr Ala Pro Asp Thr Glu Leu Gln Lys
        835                 840                 845
Phe Lys Gly Tyr Gln Ile Thr Asn Asp Val Gln Ala Gln Pro Thr Gly
    850                 855                 860
Val Tyr Ser Phe Lys Gly Met Ile Cys Val Cys Asp Ala Phe Leu Asn
865                 870                 875                 880
Leu Thr Arg Thr Ser Gly Val Asn Tyr Ser Gly Ala Asn Leu Ile Gly
                885                 890                 895
Asn Ser Tyr Thr Gly Ala Ile Asp Ile Lys Gln Gly Ile Val Phe Pro
            900                 905                 910
Pro Glu Val Glu Gln Thr Val Tyr Leu Phe Asn Thr Gly Thr Arg Asp
        915                 920                 925
Gln Trp Arg Lys Leu Asn Gly Ser Thr Val Ser Gly Tyr Arg Ala Gly
    930                 935                 940
Gln Tyr Leu Ser Val Pro Lys Asn Thr Ala Gly Gln Asp Asn Leu Pro
945                 950                 955                 960
Asp Arg Ile Pro Ser Met His Ser Phe Leu Val Lys Met Gln Asn Gly
                965                 970                 975
Ala Ser Cys Thr Leu Unk Ile Leu Tyr Asp Lys Leu Leu Lys Asn Thr
            980                 985                 990
Thr Val Asn Asn Gly Asn Gly Thr Gln Ile Thr Trp Arg Ser Gly Asn
        995                 1000                1005
Ser Gly Ser Ala Asn Met Pro Ser Leu Val Met Asp Val Leu Gly Asn
    1010                1015                1020
Glu Ser Ala Asp Arg Leu Trp Ile Phe Thr Asp Gly Gly Leu Ser Phe
1025                1030                1035                1040
Gly Phe Asp Asn Gly Trp Asp Gly Arg Lys Leu Thr Glu Lys Gly Leu
                1045                1050                1055
Ser Gln Leu Tyr Ala Met Ser Asp Ile Gly Asn Asp Lys Phe Gln Val
            1060                1065                1070

Ala Gly Val Pro Glu Leu Asn Asn Leu Leu Ile Gly Phe Asp Ala Asp
        1075                1080                1085
Lys Asp Gly Gln Tyr Thr Leu Glu Phe Ala Leu Ser Asp His Phe Ala
    1090                1095                1100
Lys Gly Ala Val Tyr Leu His Asp Leu Gln Ser Gly Ala Lys His Arg
1105                1110                1115                1120
Ile Thr Asn Ser Thr Ser Tyr Ser Phe Asp Ala Lys Arg Gly Asp Ser
                1125                1130                1135
Gly Ala Arg Phe Arg Leu Ser Tyr Gly Cys Asp Glu Asn Val Asp Asp
            1140                1145                1150
Ser His Val Val Ser Thr Asn Gly Arg Glu Ile Ile Ile Leu Asn Gln
        1155                1160                1165
Asp Ala Leu Asp Cys Thr Val Thr Leu Phe Thr Ile Glu Gly Lys Leu
    1170                1175                1180
Leu Arg Arg Leu Lys Val Leu Ala Gly His Arg Glu Val Met Lys Val
1185                1190                1195                1200
Gln Thr Gly Gly Ala Tyr Ile Val His Leu Gln Asn Ala Phe Thr Asn
                1205                1210                1215
Asp Val His Lys Val Leu Val Glu Tyr
            1220                1225

(2) INFORMATION FOR SEQ ID NO:486

    (i) SEQUENCE CHARACTERISTICS:
        (A) LENGTH: 425 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...425

    (xi) SEQUENCE DESCRIPTION: SEQ ID NO:486

Met Glu Val Lys Lys Asn Thr Val Val Leu Arg Leu Leu Ile Trp Phe
1               5                   10                  15
Val Ala Ile Leu Leu Phe His Ser Ser Arg Leu Trp Gly Gln Glu Gly
            20                  25                  30
Glu Gly Ser Ala Arg Tyr Arg Phe Lys Gly Phe Val Asp Thr Tyr His
        35                  40                  45
Ala Val Arg Ser Ser Ser Pro Phe Asp Phe Met Ser Ser Arg Thr Arg
    50                  55                  60
Val Arg Gly Glu Leu Glu Arg Ser Phe Gly Asn Ser Lys Val Ala Val
65                  70                  75                  80
Ser Val Asn Ala Thr Tyr Asn Ala Leu Leu Lys Asp Glu Thr Gly Leu
                85                  90                  95
Arg Leu Arg Glu Ala Phe Phe Glu His Gln Glu Glu His Trp Gly Leu
            100                 105                 110
Arg Leu Gly Arg Gln Ile Val Ile Trp Gly Ala Ala Asp Gly Val Arg
        115                 120                 125
Ile Thr Asp Leu Ile Ser Pro Met Asp Met Thr Glu Phe Leu Ala Gln
    130                 135                 140
Asp Tyr Asp Asp Ile Arg Met Pro Val Asn Ala Leu Arg Phe Ser Val
145                 150                 155                 160
Phe Asn Glu Ser Met Lys Val Glu Val Val Val Leu Pro Val Phe Glu
                165                 170                 175
Gly Tyr Arg Leu Pro Val Asp Pro Arg Asn Pro Trp Asn Ile Phe Ser
            180                 185                 190
Leu Ser Pro Ile Ala Gln Gly Met Asn Ile Val Trp Lys Glu Glu Ala
        195                 200                 205
Gly Lys Pro Ala Phe Lys Val Ala Asn Ile Glu Tyr Gly Ala Arg Trp
    210                 215                 220
Ser Thr Thr Leu Ser Gly Ile Asp Phe Ala Leu Ala Ala Leu His Thr
225                 230                 235                 240
Trp Asn Lys Met Pro Val Ile Glu Val Gln Gly Ile Val Pro Thr Glu
                245                 250                 255
Ile Ile Val Ser Pro Arg Tyr Tyr Arg Met Gly Phe Val Gly Gly Asp
            260                 265                 270

Leu Ser Val Pro Val Gly Gln Phe Val Phe Arg Gly Glu Ala Ala Phe
        275                 280                 285
Asn Ile Asp Lys His Phe Thr Tyr Lys Ser His Ala Glu Gln Glu Gly
    290                 295                 300
Phe Gln Thr Ile Asn Trp Leu Ala Gly Ala Asp Trp Tyr Ala Pro Gly
305                 310                 315                 320
Glu Trp Met Ile Ser Gly Gln Phe Ser Met Glu Ser Ile Phe Arg Tyr
                325                 330                 335
Arg Asp Phe Ile Ser Gln Arg Gln His Ser Thr Leu Ile Thr Leu Asn
            340                 345                 350
Val Ser Lys Lys Phe Phe Gly Ser Thr Leu Gln Leu Ser Asp Phe Thr
        355                 360                 365
Tyr Tyr Asp Leu Thr Gly Lys Gly Trp Phe Ser Arg Phe Ala Ala Asp
    370                 375                 380
Tyr Ala Leu Asn Asp Gln Ile His Leu Met Ala Gly Tyr Asp Trp Phe
385                 390                 395                 400
Ser Ser Lys Gly Ser Gly Ile Phe Asp Arg Tyr Lys Asp Asn Ser Glu
                405                 410                 415
Leu Trp Phe Lys Ala Arg Tyr Ser Phe
            420                 425

(2) INFORMATION FOR SEQ ID NO:487

    (i) SEQUENCE CHARACTERISTICS:
        (A) LENGTH: 404 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...404

    (xi) SEQUENCE DESCRIPTION: SEQ ID NO:487

Met Ser Ser Cys Glu Val Ala Tyr Phe Ser Leu Lys Pro Ile Asp Leu
1               5                   10                  15
Gln Asn Ile Arg Glu Arg Asn His Ser Ser Asp Ile Ala Leu Ser Asn
            20                  25                  30
Leu Leu Asp Asn Ser Asn Gln Leu Leu Ala Thr Ile Leu Ile Gly Asn
        35                  40                  45
Asn Val Ile Asn Val Ala Ile Val Ile Leu Ser Asn Tyr Ala Ile Glu
    50                  55                  60
Gln Thr Phe Val Phe Ser Ser Pro Ile Ile Gly Phe Leu Ile Gln Thr
65                  70                  75                  80
Ile Leu Leu Thr Thr Val Leu Leu Leu Phe Gly Glu Ile Leu Pro Lys
                85                  90                  95
Val Tyr Ala Arg Lys Asn Pro Leu Gln Tyr Ser Arg Phe Ser Ala Ala
            100                 105                 110
Ala Met Ser Val Ile Tyr Lys Ile Leu Ser Pro Phe Ser Lys Leu Leu
        115                 120                 125
Val Lys Ser Thr Gly Ile Val Thr Arg Gly Ile Ser Lys Lys Lys Tyr
    130                 135                 140
Asp Met Ser Val Asp Glu Leu Ser Lys Ala Val Ala Leu Thr Thr Thr
145                 150                 155                 160
Glu Gly Glu Pro Glu Glu Lys Glu Met Ile Asn Glu Ile Ile Lys Phe
                165                 170                 175
Tyr Asn Lys Thr Ala Cys Glu Ile Met Val Pro Arg Ile Asp Ile Val
            180                 185                 190
Asp Val Asp Leu Ser Trp Pro Phe Arg Lys Met Leu Asp Phe Val Val
        195                 200                 205
Ser Ser Gly Tyr Ser Arg Leu Pro Val Ser Glu Gly Ser Glu Asp Asn
    210                 215                 220
Ile Lys Gly Val Ile Tyr Ile Lys Asp Leu Ile Pro His Met Asp Lys
225                 230                 235                 240
Gly Asp Glu Phe Asp Trp His Pro Leu Ile Arg Lys Ala Tyr Phe Val
                245                 250                 255
Pro Glu Asn Lys Arg Ile Asp Asp Leu Leu Glu Glu Phe Arg Ala Asn
            260                 265                 270

Lys Val His Val Ser Ile Val Val Asp Glu Phe Gly Gly Thr Cys Gly
        275                 280                 285
Leu Ile Thr Met Glu Asp Ile Leu Glu Glu Ile Val Gly Glu Ile Thr
    290                 295                 300
Asp Glu Tyr Asp Glu Glu Glu Leu Pro Phe Lys Val Leu Gly Asp Gly
305                 310                 315                 320
Ser Tyr Leu Phe Glu Gly Lys Thr Ser Leu Ser Asp Val Arg His Tyr
                325                 330                 335
Leu Asp Leu Pro Glu Asn Ala Phe Gly Glu Leu Gly Asp Glu Val Asp
            340                 345                 350
Thr Leu Ser Gly Leu Phe Leu Glu Ile Lys Gln Glu Leu Pro His Val
        355                 360                 365
Gly Asp Thr Ala Val Tyr Glu Pro Phe Arg Phe Gln Val Thr Gln Met
    370                 375                 380
Asp Lys Arg Arg Ile Ile Glu Ile Lys Ile Phe Pro Phe Glu Arg Thr
385                 390                 395                 400
Trp Glu Val Glu

(2) INFORMATION FOR SEQ ID NO:488

    (i) SEQUENCE CHARACTERISTICS:
        (A) LENGTH: 260 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...260

    (xi) SEQUENCE DESCRIPTION: SEQ ID NO:488

Met Lys Leu Leu Leu Tyr Leu Leu Leu Val Leu Ser Thr Leu Ser Pro
1               5                   10                  15
Met Tyr Ser Gln Met Leu Phe Ser Glu Asn Leu Thr Met Asn Ile Asp
            20                  25                  30
Ser Thr Lys Thr Ile Gln Gly Thr Ile Leu Pro Val Leu Asp Phe Lys
        35                  40                  45
Thr Glu Lys Glu Asn Val Phe Thr Phe Lys Asn Thr Ala Asn Leu Asn
    50                  55                  60
Leu Leu Ile Lys His Gly Gln Val Ile Asn Leu Ile Asn Lys Leu Glu
65                  70                  75                  80
Phe Ser Thr Tyr Gly Asn Lys Val Thr Val Ser Gly Gly Tyr Val His
                85                  90                  95
Thr Glu Tyr Arg Tyr Leu Leu His His Val Phe Glu Val Tyr Pro Tyr
            100                 105                 110
Val Glu Ser Gln Trp Ala Glu Ser Arg Gly Met Lys Tyr Lys Val Ser
        115                 120                 125
Thr Gly Leu Gln Ser Arg Tyr Arg Leu Val Asn Ser Asp Asn Cys Leu
    130                 135                 140
Met Phe Ala Thr Leu Gly Val Phe Phe Glu Phe Glu Lys Trp Glu Gln
145                 150                 155                 160
Pro Ala Thr Ser Leu Phe Ala Gly Thr Tyr Ala Tyr Ser Arg Ser Ile
                165                 170                 175
Lys Ser His Leu Ser Ile Ser Phe Arg His Arg Leu Gly Glu His Trp
            180                 185                 190
Glu Phe Thr Thr Thr Ala Ile His Gln Gly Lys Pro Asp Ser Tyr Phe
        195                 200                 205
Lys Lys Ala Arg Phe Gly Gly Ala Ile Asp Leu Lys Tyr His Ile Thr
    210                 215                 220
Pro Thr Ile Gly Ile Arg Gly Ala Tyr Arg Ile Ile Tyr Asp Thr Ala
225                 230                 235                 240
Pro Ile Val Pro Val Arg Lys Asp Tyr Asn Thr Val Asp Val Gly Ile
                245                 250                 255
Asp Ile Ser Phe
            260

(2) INFORMATION FOR SEQ ID NO:489

(i) SEQUENCE CHARACTERISTICS:
(A) LENGTH: 834 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...834

(xi) SEQUENCE DESCRIPTION: SEQ ID NO:489

Met Lys Arg Ile Val Leu Ser Ser Phe Leu Phe Val Leu Ser Ile Leu
1               5                   10                  15
Ser Leu Met Ala Gln Asn Asn Thr Leu Asp Val His Ile Ser Gly Thr
            20                  25                  30
Ile Lys Asp Ala Ser Ser Gly Glu Pro Val Pro Tyr Ala Thr Val Ser
        35                  40                  45
Ile Arg Leu Thr Gly Ala Asp Thr Thr Gln Val Phe Arg Gln Val Thr
    50                  55                  60
Asp Gly Asn Gly Tyr Phe Val Ile Gly Leu Pro Ala Ala Pro Ser Tyr
65                  70                  75                  80
His Leu Thr Ala Ser Phe Val Gly Met Lys Thr His Thr Met Gln Ile
                85                  90                  95
Ser Arg Gly Asn Gly Gln His Asp Ile Lys Ser Ile Asp Ile Ser Leu
            100                 105                 110
Glu Ser Glu Asp Lys Gln Leu Ser Thr Val Thr Val Ser Ala Ala Arg
        115                 120                 125
Pro Leu Val Lys Met Glu Ile Asp Arg Leu Ser Tyr Asn Met Lys Asp
    130                 135                 140
Asp Pro Ala Ala Lys Thr Asn Asn Leu Leu Glu Met Leu Arg Asn Val
145                 150                 155                 160
Pro Leu Val Thr Val Asp Gly Gln Gly Asn Ile Gln Val Lys Gly Ser
                165                 170                 175
Ser Asn Phe Lys Ile His Leu Asn Gly Arg Pro Ser Thr Met Val Ser
            180                 185                 190
Ser Asn Pro Lys Glu Val Phe Arg Ser Ile Pro Ala His Thr Ile Lys
        195                 200                 205
Arg Val Glu Val Ile Thr Asp Pro Gly Val Lys Tyr Asp Ala Glu Gly
    210                 215                 220
Thr Ser Ala Ile Leu Asp Ile Val Thr Glu Glu Gly Lys Lys Leu Glu
225                 230                 235                 240
Gly Tyr Ser Gly Ser Ile Thr Ala Ser Val Ser Asn Asn Pro Thr Ala
                245                 250                 255
Asn Gly Ser Ile Phe Leu Thr Ala Lys Ser Gly Lys Val Gly Leu Thr
            260                 265                 270
Thr Asn Tyr Asn Tyr Tyr Gly Gly Lys Asn Lys Gly Ser Arg Tyr Phe
        275                 280                 285
Thr Glu Arg Thr Thr Ser Met Leu Gln Thr Ile Glu Glu Gly Lys Gly
    290                 295                 300
Gln Glu Thr Phe Gly Gly His Phe Gly Asn Ala Leu Leu Ser Phe Glu
305                 310                 315                 320
Ile Asp Ser Leu Asn Leu Phe Thr Val Gly Gly Asn Val Arg Leu Trp
                325                 330                 335
Glu Met Thr Thr Asp Arg Asn Ser Val Glu Lys Ser Phe Ala Gly Ser
            340                 345                 350
Asn Leu Met Ser Tyr Ile Asp Arg Lys Leu Lys Thr Gln Met Asp Ala
        355                 360                 365
Gly Ser Tyr Glu Leu Asn Ala Asp Tyr Gln His Ser Thr Arg Leu Pro
    370                 375                 380
Gly Glu Leu Leu Thr Val Ser Tyr Arg Phe Thr His Asn Pro Asn Asn
385                 390                 395                 400
Ser Glu Thr Phe Ile Asp Gln Trp Lys Arg Asp Pro Leu Asn Thr Ala
                405                 410                 415
Asn Thr Ile Gln Tyr Ala Gly Gln His Ser Lys Ser Asp Ala Gly Met
            420                 425                 430
Asp Glu His Thr Ala Gln Val Asp Tyr Thr Arg Pro Leu Gly Gln Ala
        435                 440                 445

His Ser Leu Glu Ala Gly Leu Lys Tyr Ile Tyr Arg His Ala Thr Ser
    450                 455                 460
Asp Pro Leu Tyr Glu Ile Arg Pro Ser Glu Asp Ala Pro Trp Gln Pro
465                 470                 475                 480
Gly Ser Leu Tyr Ala Gln Asn Pro Ser Asn Gly Lys Phe Arg His Asp
                485                 490                 495
Gln Tyr Ile Gly Ala Ala Tyr Ala Gly Tyr Asn Tyr Arg Lys Asp Gln
            500                 505                 510
Tyr Ser Leu Gln Thr Gly Leu Arg Val Glu Ser Ser Arg Leu Lys Ala
        515                 520                 525
Leu Phe Pro Glu Asn Ala Ala Ala Asp Phe Ser His Asn Ser Phe Asp
    530                 535                 540
Trp Val Pro Gln Leu Thr Leu Gly Tyr Thr Pro Ser Pro Met Lys Gln
545                 550                 555                 560
Leu Lys Leu Ala Tyr Asn Phe Arg Ile Gln Arg Pro Ala Ile Gly Gln
                565                 570                 575
Leu Asn Pro Tyr Arg Leu Gln Thr Asn Asp Tyr Gln Val Gln Tyr Gly
            580                 585                 590
Asn Pro Asp Leu Lys Ser Glu Lys Arg His His Val Gly Leu Ser Tyr
        595                 600                 605
Asn Gln Tyr Gly Ala Lys Val Met Leu Thr Ala Ser Leu Asp Tyr Asp
    610                 615                 620
Phe Cys Asn Asn Ala Ile Gln Asn Tyr Thr Phe Ser Asp Pro Ala Asn
625                 630                 635                 640
Pro Asn Leu Phe His Gln Thr Tyr Gly Asn Ile Gly Arg Glu His Ser
                645                 650                 655
Phe Ser Leu Asn Thr Tyr Ala Met Tyr Thr Pro Ala Val Trp Val Arg
            660                 665                 670
Ile Met Leu Asn Gly Asn Ile Asp Arg Thr Phe Gln Lys Ser Glu Ala
        675                 680                 685
Leu Gly Ile Asp Val Asn Ser Trp Ser Gly Met Val Tyr Ser Gly Leu
    690                 695                 700
Met Phe Thr Leu Pro Lys Asp Trp Thr Val Asn Leu Phe Gly Gly Tyr
705                 710                 715                 720
Tyr His Gly Gly Arg Ser Tyr Gln Thr Lys Tyr Asp Gly Asn Val Phe
                725                 730                 735
Asn Asn Ile Gly Ile Ala Lys Gln Leu Phe Asp Lys Lys Leu Arg Val
            740                 745                 750
Ser Leu Ser Ala Asn Asn Ile His Ala Lys Tyr Ser Thr Trp Lys Ser
        755                 760                 765
Arg Thr Ile Gly Asn Gly Phe Thr Ile Tyr Ser Glu Asn Ala Gly Ile
    770                 775                 780
Gln Arg Ser Val Ser Leu Ser Leu Thr Tyr Ser Phe Gly Lys Met Asn
785                 790                 795                 800
Thr Gln Val Arg Lys Val Glu Arg Thr Ile Val Asn Asp Asp Leu Lys
                805                 810                 815
Gln Thr Ser Ser Gln Gly Gln Gln Gly Gly Gly Gln Gly Asn Pro Thr
            820                 825                 830
Gly Asn

(2) INFORMATION FOR SEQ ID NO:490

(i) SEQUENCE CHARACTERISTICS:
(A) LENGTH: 399 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...399

(xi) SEQUENCE DESCRIPTION: SEQ ID NO:490

Met Arg Leu Ser Ala Ile Leu Ile Ala Leu Ile Val Met Leu Pro Ala
1               5                   10                  15
Val Leu Ser Gly Gln His Tyr Tyr Ser Met Ala Gly Glu Arg Leu Glu
            20                  25                  30

Thr Asp Ser Ile Arg Pro Asn Glu Leu Ser Ala Ser Ile Arg Ser Ala
        35                  40                  45
Leu Phe Phe Arg Asn Asn Glu Tyr Asn Ala Arg Ser Val Lys Gly Tyr
    50                  55                  60
Thr Leu Pro Gly Ala Arg Val Ser Ala Phe Ala Ser Tyr Ser Leu Pro
65                  70                  75                  80
Ala Ala His Gly Val Lys Leu Ser Leu Gly Val Ser Thr Leu Asn Tyr
                85                  90                  95
Trp Gly Ala Ser Arg Tyr Pro Ala Gly Ile Ala Tyr Ser Asp Leu Pro
            100                 105                 110
Tyr Trp Thr Asp Tyr Asn Asp Tyr Val Arg Leu Arg Ile Leu Pro Tyr
        115                 120                 125
Val Gln Ala Met Leu Lys Pro Thr Ala Thr Thr Ala Leu Met Leu Gly
    130                 135                 140
Asn Ile Ala Gly Gly Thr Ala His Gly Leu Ile Glu Pro Ile Tyr Asn
145                 150                 155                 160
Pro Glu Leu Asp Leu Thr Ala Asp Pro Glu Ala Gly Val Gln Phe Arg
                165                 170                 175
Gly Asp Trp Thr Arg Phe Arg Met Asp Val Trp Val Asn Trp Met Ser
            180                 185                 190
Met Ile Phe Lys Asn Asp Asn His Gln Glu Ser Phe Val Phe Gly Leu
        195                 200                 205
Ser Thr Thr Ser Lys Leu Leu Ser Gly Glu Gly Lys Trp Arg Leu Glu
    210                 215                 220
Leu Pro Leu Gln Ala Ile Ala Thr His Arg Gly Gly Glu Tyr Asn Trp
225                 230                 235                 240
Ala Gln Gln Asp Thr Val His Thr Trp Val Asn Gly Ala Val Gly Leu
                245                 250                 255
Lys Leu Ser Tyr Arg Pro Arg Thr Asp Lys Pro Met Gln Ile Trp Gly
            260                 265                 270
Ser Ala Tyr Gly Val Ala Ala Leu Ser Ser Gly Gly Tyr Phe Pro Tyr
        275                 280                 285
Glu Arg Gly Trp Gly Gly Tyr Leu Ser Leu Gly Met Asp Leu Glu His
    290                 295                 300
Phe Ala Phe Arg Thr Asp Tyr Trp Tyr Gly Arg His Tyr Val Ser Pro
305                 310                 315                 320
Phe Ala Ala Pro Phe Ala Asn Ser Leu Thr Tyr Asp Lys Gln Pro Leu
                325                 330                 335
Thr Asn Gly Trp Gly Asp Tyr Ile Arg Leu Tyr Ala Asp Tyr Ser Trp
            340                 345                 350
Arg Met Ala Arg Ser Val Ser Leu Ala Ala Val Ala Arg Val Trp Phe
        355                 360                 365
Gln Pro Ser Asp Arg Phe Ala Met Ser His Ala Leu Glu Leu Thr Met
    370                 375                 380
Arg Ile Asp Pro Lys Phe Pro Ile Ala Phe Leu Lys Gly Asn His
385                 390                 395

(2) INFORMATION FOR SEQ ID NO:491

(i) SEQUENCE CHARACTERISTICS:
(A) LENGTH: 382 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...382

(xi) SEQUENCE DESCRIPTION: SEQ ID NO:491

Met Asn Lys Ser Leu Leu Ser Leu Ala Cys Leu Ile Leu Cys Gly Met
1               5                   10                  15
Pro Ala Ile Ala Gln Gln Thr Gly Pro Ala Glu Arg Ser Gly Glu Pro
            20                  25                  30
Ser Leu Ala Glu Arg Val Phe Gly Leu Glu Gln Lys Gln Lys Lys Leu
        35                  40                  45
Lys Val Tyr Leu Gly Ile Gln Ser Phe Tyr Asp Gln Pro Leu Val Asp
    50                  55                  60

Asp Glu Ser His Ile Gly His Phe Lys Val Gln Glu Leu Arg Met Ser
65                  70                  75                  80
Ala His Gly Glu Leu Asn Arg His Leu Ser Phe Asp Trp Arg Gln Arg
                85                  90                  95
Leu Asn Arg Ala Ala Asp Gly Thr Ser Phe Ala Asp Asn Leu Ser Asn
            100                 105                 110
Ala Ile Asp Ile Ala Gly Val Asp Trp His Pro Asn Asp Lys Val Ser
        115                 120                 125
Phe Phe Phe Gly Arg Gln Tyr Ala Arg Phe Gly Gly Ile Glu Tyr Asp
    130                 135                 140
Met Asn Pro Val Glu Ile Tyr Gln Tyr Ser Asp Leu Val Asp Tyr Met
145                 150                 155                 160
Thr Cys Tyr Thr Ser Gly Val Asn Phe Ala Trp Asn Phe His Pro Glu
                165                 170                 175
Gln Gln Leu Gln Leu Gln Val Leu Asn Ala Tyr Asn Asn Arg Phe Ala
            180                 185                 190
Asp Arg Tyr His Val Thr Pro Asp Val Ala Thr Ala Thr Ser Tyr Pro
        195                 200                 205
Leu Leu Tyr Ser Ala Gln Trp Asn Gly Thr Leu Leu Gly Gly Ala Leu
    210                 215                 220
His Met Arg Tyr Ala Val Ser Met Ala His Gln Ala Gln Glu Arg Asn
225                 230                 235                 240
Met Trp Tyr Phe Thr Ala Gly Asn Leu Phe Asn Pro Gly Lys Arg Ile
                245                 250                 255
Asn Gly Tyr Leu Asp Leu Thr Tyr Ser Ile Glu Gly Leu Asp Asp Lys
            260                 265                 270
Gly Ile Met Thr Ala Arg Tyr Gly Lys Gly Lys Thr Leu Thr Asp Val
        275                 280                 285
Lys Tyr Tyr Ala Leu Val Ser Lys Trp Asn Phe Arg Ile Phe Asp Gln
    290                 295                 300
Val Asn Leu Phe Leu Lys Gly Met Tyr Glu Asn Gly Tyr Ala Pro Ala
305                 310                 315                 320
Gln Tyr Gly Glu Ser Ser His Thr Arg His Ser Tyr Gly Tyr Met Gly
                325                 330                 335
Gly Val Glu Tyr Tyr Pro Thr Glu Thr Asn Phe Arg Leu Phe Val Thr
            340                 345                 350
Tyr Ile Gly Arg His Tyr Arg Tyr Ser Ala Thr Glu Thr Glu Ser Thr
        355                 360                 365
Asn Ala Leu Arg Ala Gly Leu Ile Tyr Gln Ile Pro Phe Leu
    370                 375                 380

(2) INFORMATION FOR SEQ ID NO:492

    (i) SEQUENCE CHARACTERISTICS:
        (A) LENGTH: 222 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...222

    (xi) SEQUENCE DESCRIPTION: SEQ ID NO:492

Met Lys His Leu Phe Lys Ser Thr Leu Val Leu Leu Cys Ala Leu Ser
1               5                   10                  15
Phe Ser Gly Thr Tyr Thr Phe Ala Gln Glu Asn Asn Thr Glu Lys Ser
            20                  25                  30
Arg Phe Asp Phe Ser Val Arg Leu Gly Gln Gly Tyr Ile Ala Gly Ser
        35                  40                  45
Thr Thr Asn Leu Met Tyr Gly Tyr Thr Ser Ala Asn Asp Arg Leu Leu
    50                  55                  60
Ser Gly Ala Ile Tyr Leu Gly Leu Thr Pro Ser Lys Lys Glu Asn Ala
65                  70                  75                  80
Thr Gly Val Ala Phe Arg Phe Leu Ser Pro Ser Pro Gly Tyr Tyr Val
                85                  90                  95
Asp Ile Ser Gly Lys Glu Asn Thr Leu Asn Tyr Ala Phe Tyr Val Val
            100                 105                 110

Gly Ala Tyr Asn Arg Ile Ala Ile Pro Ile Arg Pro Ile Lys Asn Phe
        115                 120                 125
Asn Phe Ile Phe Ser Thr Glu Val Gly Met Ala Trp Met Ser Arg His
    130                 135                 140
Glu Gln Ile Tyr Asn Ser Thr Ser Gln Thr Trp Asp Lys Gln Arg Lys
145                 150                 155                 160
Ser Arg Ser Gly Leu Asp Phe Gly Leu Gly Met His Leu Gln Unk His
                165                 170                 175
Ile Asn Lys Thr Val Tyr Phe Met Ala Gly Thr Asp Leu Thr Ser Cys
            180                 185                 190
Met Phe Gly Lys Arg Ile Asn Asp Tyr Gln Gln Lys Asp Arg Thr Phe
        195                 200                 205
Ile Ala Leu Ile Asp Asn Ser Ile Gly Ile Gly Leu Asn Leu
    210                 215                 220

(2) INFORMATION FOR SEQ ID NO:493

     (i) SEQUENCE CHARACTERISTICS:
          (A) LENGTH: 391 amino acids
          (B) TYPE: amino acid
          (D) TOPOLOGY: linear

    (ii) MOLECULE TYPE: protein

   (iii) HYPOTHETICAL: YES

    (vi) ORIGINAL SOURCE:
          (A) ORGANISM: Porphyromonas gingivalis

    (ix) FEATURE:
          (A) NAME/KEY: misc_feature
          (B) LOCATION 1...391

    (xi) SEQUENCE DESCRIPTION: SEQ ID NO:493

Met Ile Ile Lys Lys Met Leu Lys Asn Lys Leu Ala Pro Leu Ala Ile
1               5                   10                  15
Leu Phe Leu Phe Ala Pro Lys Ala Met Lys Ala Gln Glu Gln Leu Asn
            20                  25                  30
Val Val His Thr Ser Val Pro Ser Leu Asn Ile Ser Pro Asp Ala Arg
        35                  40                  45
Ala Ala Gly Met Gly Asp Ile Gly Val Ala Thr Thr Pro Asp Ala Tyr
    50                  55                  60
Ser Gln Tyr Trp Asn Pro Ser Lys Tyr Ala Phe Met Asp Thr Lys Ala
65                  70                  75                  80
Gly Ile Ser Phe Ser Tyr Thr Pro Trp Leu Ser Lys Leu Val Asn Asp
                85                  90                  95
Ile Ala Leu Met Gln Met Thr Gly Phe Tyr Lys Leu Gly Thr Asp Glu
            100                 105                 110
Asn Gln Ala Ile Ser Ala Ser Leu Arg Tyr Phe Thr Leu Gly Lys Leu
        115                 120                 125
Glu Thr Phe Asp Glu Leu Gly Glu Ser Met Gly Glu Ala His Pro Asn
    130                 135                 140
Glu Phe Ala Val Asp Leu Gly Tyr Ser Arg Gln Leu Ser Glu Asn Phe
145                 150                 155                 160
Ser Met Ala Val Ala Leu Arg Tyr Ile Arg Ser Asp Gln Ser Thr His
                165                 170                 175
Asn Thr Gly Glu Asn Gln Ala Gly Asn Ala Phe Ala Ala Asp Ile Ala
            180                 185                 190
Gly Tyr Leu Gln Lys Tyr Val Leu Leu Gly Asn Ala Glu Ser Leu Trp
        195                 200                 205
Ser Leu Gly Phe Asn Val Lys Asn Ile Gly Thr Lys Ile Ser Tyr Asp
    210                 215                 220
Gly Gly Val Thr Ser Phe Phe Ile Pro Thr Ser Leu Asn Leu Gly Thr
225                 230                 235                 240
Gly Leu Leu Tyr Pro Ile Asp Asp Tyr Asn Ser Ile Asn Phe Asn Leu
                245                 250                 255
Glu Leu Ser Lys Leu Leu Val Pro Thr Pro Pro Ile Met Asp Gln Asn
            260                 265                 270
Asp Gln Ala Gly Tyr Glu Ala Ala Leu Lys Lys Tyr Gln Glu Thr Ser
        275                 280                 285
Ser Ile Ser Gly Ile Phe Ser Ser Phe Gly Asp Ala Pro Gly Gly Leu
    290                 295                 300
Lys Glu Glu Phe Arg Glu Ile Thr Trp Gly Leu Gly Ala Glu Tyr Ser
305                 310                 315                 320

Tyr Asp Asp Lys Phe Phe Val Arg Ala Gly Tyr Ser Tyr Leu His Pro
                325                 330                 335
Thr Lys Gly Asn Leu Gln Tyr Phe Thr Ala Gly Ala Gly Phe Lys Met
            340                 345                 350
Asn Ile Phe Arg Ile Asp Ala Ser Tyr Leu Leu Ser Thr Ile Gln Ser
        355                 360                 365
Asn Pro Leu Asp Gln Thr Leu Arg Phe Thr Leu Ala Phe Asp Met Asp
    370                 375                 380
Gly Leu Arg Asn Leu Phe His
385                 390

(2) INFORMATION FOR SEQ ID NO:494

(i) SEQUENCE CHARACTERISTICS:
(A) LENGTH: 446 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...446

(xi) SEQUENCE DESCRIPTION: SEQ ID NO:494

Met Lys Thr Thr Val Gln Gln Ile Ile Leu Cys Leu Ala Leu Met Met
1               5                   10                  15
Ser Gly Val Leu Gly Gly Asn Ala Gln Ser Phe Trp Glu Glu Ile Ala
            20                  25                  30
Pro Pro Phe Ile Ser Asn Glu Pro Asn Val Lys Tyr Ile Ile Pro Asn
        35                  40                  45
Met Gly Ile Asp Ser Lys Gly Thr Ile Tyr Val Thr Val Thr Lys Arg
    50                  55                  60
Ile Gln Gln Gly Ala Asn Tyr Thr Ser Glu Gln Leu Gly Met Tyr Tyr
65                  70                  75                  80
Arg Pro Leu Gly Asp Asn Glu Gln Trp Trp Lys His Asp Pro Tyr Phe
                85                  90                  95
Asp Asp Lys Ile Val Ala Asp Ile Gln Thr Asp Ala Tyr Gly Arg Val
            100                 105                 110
Tyr Val Cys Thr Thr Ser Ser Arg Asp Gln Glu Tyr Gln Leu Tyr Ile
        115                 120                 125
Asn Glu Gln Asn Glu Trp Arg Cys Ile Phe Lys Thr Ser Val Ser Thr
    130                 135                 140
Tyr Glu His Gly Met Ala Val Phe Arg Ser Ser Thr Gly Val Thr Tyr
145                 150                 155                 160
Ile Gly Thr Arg His His Ile Phe Ala Ser Gly Val Asn Asp Phe Glu
                165                 170                 175
Phe Asn Thr Ile Tyr Glu Asp Ser Thr Pro Met Ser Cys Arg Phe Ala
            180                 185                 190
Glu Ala Thr Asn Ser Gly Thr Ile Tyr Leu Ala Leu Met His Glu Thr
        195                 200                 205
Thr Met Ser Thr Thr Ile Leu Thr Tyr Gln Asn Gly Glu Phe Val Asp
    210                 215                 220
Ile Ser Glu Ser Glu Leu Ser Asn Ser Ile Ile Ala Ser Met Cys Ser
225                 230                 235                 240
Asn Lys Glu Gly Asp Ile Ile Ala Leu Val Thr Ser Tyr Thr Gly Phe
                245                 250                 255
Met Ser Gly Thr Leu Ala Ile Arg Lys Ala Asp Glu Gly Lys Trp Gln
            260                 265                 270
Leu Val Gly Gly Asp Ile Gln Asn Ala Ile Val Gln Asn Ile Cys Met
        275                 280                 285
Met Asp Asp Asn Lys Ile Ala Cys Glu Val Phe Gly Thr Pro Asn Gly
    290                 295                 300
Val Asp Gly Arg Thr Arg Val Cys Val Ser Asp Ala Ser Val Phe Asp
305                 310                 315                 320
Phe Glu Trp Tyr Glu Asp Glu Ile Tyr Gly Gly Leu Ile Phe Asp Thr
                325                 330                 335
Phe Phe Tyr Ser Pro Trp Asp Lys Leu Leu Tyr Ala Lys Phe Gly Gly
            340                 345                 350

Ile Met Leu Arg Ser Lys Glu Ser Phe Ile Thr Ser Phe Ile Ser Pro
        355                 360                 365
Thr Val Val Gln Gly Val Asp Val Tyr Thr Leu Ala Gly Lys Ile Arg
    370                 375                 380
Ile Glu Ser Glu Thr Pro Val Ser Glu Val Leu Leu Phe Asp Leu Ala
385                 390                 395                 400
Gly Arg Met Val Leu Arg Gln Thr Ile Asp Asn Lys Ile Tyr Ser Asp
                405                 410                 415
Ile Asp Thr Asn Gly Leu Lys Arg Ser Gly Ile Tyr Val Val Ser Val
            420                 425                 430
Arg Leu Ser Ser Gly Gln Val Phe Ser His Lys Val Gln Val
        435                 440                 445

(2) INFORMATION FOR SEQ ID NO:495

    (i) SEQUENCE CHARACTERISTICS:
        (A) LENGTH: 308 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...308

    (xi) SEQUENCE DESCRIPTION: SEQ ID NO:495

Met Ile Ile Arg Cys Leu Ile Arg Arg Pro Arg Thr Val Leu Phe Gly
1               5                   10                  15
Leu Ile Phe Val Val Gly Leu Phe Ser Ala Met Ala Gln Glu Lys Lys
            20                  25                  30
Asp Ser Leu Ser Thr Val Gln Pro Val Pro Asn Ser Ser Met Val Glu
        35                  40                  45
Gln Thr Pro Leu Leu Ser Ile Asp His Pro Val Leu Pro Ala Ser Phe
    50                  55                  60
Gln Asn Thr Arg Thr Leu Lys Arg Phe Arg Asp Lys His Leu Ser Asp
65                  70                  75                  80
Ala Leu Leu Asn Gly Leu Lys Pro His Arg Ser Ser Leu Gln Leu Asn
                85                  90                  95
Glu Glu Leu Asn Phe Ala Ala Glu Arg Arg Asp Phe Val Ser Pro Leu
            100                 105                 110
Leu Gln Thr Arg His Ala Ala Gly Val Leu Ser Trp Arg Pro Thr Asp
        115                 120                 125
Arg Met His Phe Tyr Thr Ser Gly Asn Ile Gly Leu Gly His Asp Leu
    130                 135                 140
Leu Thr Gly Val Arg Lys Asp Phe Gly Trp Asn Ala Gly Ala Asp Phe
145                 150                 155                 160
Leu Leu Ser Gln Asn Leu Thr Ala His Val Gln Gly Gly Trp Gln Gln
                165                 170                 175
Asn Phe Gly Phe Ile Pro Met Thr Ala Val Asn Gly Gln Leu Arg Trp
            180                 185                 190
Gln Ala Thr Glu Arg Leu Ser Phe Thr Thr Gly Ile Asp Tyr Arg Gln
        195                 200                 205
Val Gln Trp Asn Ala Phe Asp Asn Arg Thr Phe Ser Leu Lys Gly Ser
    210                 215                 220
Ala Arg Tyr Glu Val Met Asp Asn Val Phe Val Asn Gly Phe Gly Ser
225                 230                 235                 240
Tyr Pro Leu Tyr Ser Ser Thr Arg Ser Gly Leu Asn Met Ala Val Pro
                245                 250                 255
Met His Gly Phe Gly Pro Gln Tyr Gly Gly Ser Leu Glu Leu Lys Val
            260                 265                 270
Ser Glu Arg Phe Gly Phe Ala Val Gly Met Glu Arg Glu Tyr Asn Ile
        275                 280                 285
Trp Thr Arg Arg Trp Glu Thr His Tyr Phe Ala Tyr Pro Val Phe Tyr
    290                 295                 300
Gly Asp Lys Lys
305

(2) INFORMATION FOR SEQ ID NO:496

(i) SEQUENCE CHARACTERISTICS:
(A) LENGTH: 315 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...315

(xi) SEQUENCE DESCRIPTION: SEQ ID NO:496

Met Lys Thr Asn Arg Arg Tyr Ala Phe Val Leu Pro Leu Leu Leu Leu
1               5                   10                  15
Thr Gly Leu Leu Ala Trp Gly Gln Asp Ser Ser His Gly Ser Asn Thr
            20                  25                  30
Ala Phe Ala Thr Asp Ser Ser Ser Arg Glu Leu Pro Thr Glu Gln Ser
        35                  40                  45
Ala Tyr Arg Ile His Ser Ala Tyr Met Val Gly Gly Gly Gly Ser Ile
    50                  55                  60
Thr Arg Asp Thr Tyr Leu Ser Pro Leu Arg Tyr Gly Gly Trp Thr Leu
65                  70                  75                  80
Asn Leu Leu Gly Glu Lys Thr Phe Pro Leu Lys Ala Ser Asp Ser Arg
                85                  90                  95
Trp Met Ile Arg Thr Gly His Glu Leu Asp Phe Ala Leu Met Asp Asn
            100                 105                 110
Pro Ala Asn Asn Ala His Phe Tyr Ser Leu Leu Tyr Asn Gly Ser Ala
        115                 120                 125
Ala Ala Leu Tyr Arg Leu Gly Ala Lys His Leu Arg Ala Ala Trp Met
    130                 135                 140
Asp Asn Leu Arg Leu Ala Phe Gly Pro Gly Leu Glu Ile Gly Leu Gly
145                 150                 155                 160
Gly Ile Tyr Ser Thr Arg Asn Gly Asn Asn Pro Ala Thr Leu Lys Leu
                165                 170                 175
Tyr Thr Asn Ala Ile Ala Gln Ala Ser Ile Gly Tyr Tyr Val Pro Ser
            180                 185                 190
Glu Thr Phe Pro Leu Tyr Phe Arg Leu Leu Ser Gln Ile Asn Leu Phe
        195                 200                 205
Gly Ile Ala Tyr Gly Asn Gly Phe Gly Glu Ser Tyr Tyr Glu Asn Phe
    210                 215                 220
Leu Leu Asn Asn Gly Ile Ala Gly Ser Leu His Phe Thr Tyr Pro Gly
225                 230                 235                 240
Lys Phe Thr Arg Phe Thr Thr Leu Ile Thr Ala Asp Ile Pro Ile Arg
                245                 250                 255
Asn Phe Cys Thr Leu Arg Val Gly Tyr Arg Tyr Ser His Leu Gly Ser
            260                 265                 270
Ser Leu Asn Ala Leu Asp Thr Arg Ile His Ser His Thr Ala Phe Ile
        275                 280                 285
Gly Phe Val Thr Glu Phe Tyr Arg Phe Arg Gly Arg Lys Ala Met Asn
    290                 295                 300
Thr Gly Arg Arg Thr Ser Leu Tyr Tyr His Asp
305                 310                 315

(2) INFORMATION FOR SEQ ID NO:497

(i) SEQUENCE CHARACTERISTICS:
(A) LENGTH: 285 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:

(A) NAME/KEY: misc_feature
(B) LOCATION 1...285

(xi) SEQUENCE DESCRIPTION: SEQ ID NO:497

Met Val Val Thr Leu Leu Val Ile Val Gly Ile Val Val Val Val Arg
1               5                   10                  15
Tyr Ser Leu Arg Val His Val His Lys Thr Gly Thr Val Val Ser Ala
            20                  25                  30
Ala Ile Phe Gly Phe Ile Leu Leu Gly Lys Thr Val Pro Cys Asp Thr
        35                  40                  45
Arg Asn Phe Phe Ser Ser Glu Ser Asp Glu Pro Glu Ser Arg Val Ala
    50                  55                  60
Thr Glu Ile Ala His Leu Cys Glu Ile Gly Phe Gln Ile His Ala Ser
65                  70                  75                  80
Ser Ile His Val Ala Val Arg Thr Asp Phe Gly Gln Ala Gly Ile His
                85                  90                  95
Cys Pro Met Ala Thr Asp Ala Ser Ala Thr Glu Phe Asp Arg Ser Ala
            100                 105                 110
Glu Cys Ala Glu Arg Thr Ser Ala Gln Ile Asp Thr Ala Ile Arg Ser
        115                 120                 125
Gln Ser Gln Ile Ile Arg Thr His Ile Asp Thr Cys Pro Lys Ser Ser
    130                 135                 140
Gly Thr Ile Gly Gly Ser Thr His Thr Ser Leu His Leu Lys Val Phe
145                 150                 155                 160
Asp Gly Arg Gly Glu Val Gly His Ile His Pro Lys Asp Gly Leu Arg
                165                 170                 175
Phe Gly Val Val Glu Gly Tyr Ser Ile Gly Ser Tyr Val Asp Ala Ile
            180                 185                 190
Gly Ile Gly Ala Thr His Ala Lys Ala Gly Ile Ser Asp Thr Arg Thr
        195                 200                 205
Gly Ile Ala Gly Gly Tyr His Gly Gly Ser Gln His Gln Gln Ile Gly
    210                 215                 220
Asp Val Thr Thr Ile Ile Gly Leu Gly Lys Phe Gly Leu Ala Asn Val
225                 230                 235                 240
Gly Val Ser Asp Arg Gly Phe Arg Arg Gly Ala Ser Ser Tyr Asp Leu
                245                 250                 255
Tyr Gly Leu Glu Leu His Ile Ala Lys Thr Ile Tyr Leu Ala Val Gly
            260                 265                 270
Asp Gly Cys Leu Cys Arg Gln Ala Glu Arg Gln Asp Gly
        275                 280                 285

(2) INFORMATION FOR SEQ ID NO:498

(i) SEQUENCE CHARACTERISTICS:
(A) LENGTH: 599 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...599

(xi) SEQUENCE DESCRIPTION: SEQ ID NO:498

Met Glu Asn Leu Lys Asn Ile Gln Pro Arg Glu Asp Phe Asn Trp Glu
1               5                   10                  15
Glu Phe Glu Ala Gly Gly Val His Ala Ala Val Ser Arg Gln Glu Gln
            20                  25                  30
Glu Ala Ala Tyr Asp Lys Thr Leu Asn Thr Ile Lys Glu Lys Glu Val
        35                  40                  45
Val Met Gly Arg Val Thr Ala Ile Asn Lys Arg Glu Val Val Ile Asn
    50                  55                  60
Val Gly Tyr Lys Ser Glu Gly Val Val Pro Ala Thr Glu Phe Arg Tyr
65                  70                  75                  80
Asn Pro Glu Leu Lys Val Gly Asp Glu Val Glu Val Tyr Ile Glu Asn
                85                  90                  95
Gln Glu Asp Lys Lys Gly Gln Leu Val Leu Ser His Arg Lys Gly Arg

                100                 105                 110
Ala Ala Arg Ser Trp Glu Arg Val Asn Glu Ala Leu Glu Lys Asp Glu
            115                 120                 125
Ile Val Lys Gly Tyr Val Lys Cys Arg Thr Lys Gly Gly Met Ile Val
    130                 135                 140
Asp Val Phe Gly Ile Glu Ala Phe Leu Pro Gly Ser Gln Ile Asp Val
145                 150                 155                 160
Arg Pro Ile Arg Asp Tyr Asp Ala Phe Val Glu Lys Thr Met Glu Phe
                165                 170                 175
Lys Ile Val Lys Ile Asn Gln Glu Tyr Lys Asn Val Val Val Ser His
            180                 185                 190
Lys Val Leu Ile Glu Ala Glu Leu Glu Gln Gln Lys Lys Glu Ile Ile
        195                 200                 205
Gly Lys Leu Glu Lys Gly Gln Val Leu Glu Gly Ile Val Lys Asn Ile
    210                 215                 220
Thr Ser Tyr Gly Val Phe Ile Asp Leu Gly Gly Val Asp Gly Leu Ile
225                 230                 235                 240
His Ile Thr Asp Leu Ser Trp Gly Arg Val Ala His Pro Glu Glu Ile
                245                 250                 255
Val Gln Leu Asp Gln Lys Ile Asn Val Val Ile Leu Asp Phe Asp Glu
            260                 265                 270
Asp Arg Lys Arg Ile Ala Leu Gly Leu Lys Gln Leu Met Pro His Pro
        275                 280                 285
Trp Asp Ala Leu Asp Ser Glu Leu Lys Val Gly Asp Lys Val Lys Gly
    290                 295                 300
Lys Val Val Val Met Ala Asp Tyr Gly Ala Phe Val Glu Ile Ala Gln
305                 310                 315                 320
Gly Val Glu Gly Leu Ile His Val Ser Glu Met Ser Trp Thr Gln His
                325                 330                 335
Leu Arg Ser Ala Gln Asp Phe Leu His Val Gly Asp Glu Val Glu Ala
            340                 345                 350
Val Ile Leu Thr Leu Asp Arg Glu Glu Arg Lys Met Ser Leu Gly Leu
        355                 360                 365
Lys Gln Leu Lys Pro Asp Pro Trp Ala Asp Ile Glu Thr Arg Phe Pro
    370                 375                 380
Val Gly Ser Arg His His Ala Arg Val Arg Asn Phe Thr Asn Phe Gly
385                 390                 395                 400
Val Phe Val Glu Ile Glu Glu Gly Val Asp Gly Leu Ile His Ile Ser
                405                 410                 415
Asp Leu Ser Trp Thr Lys Lys Ile Lys His Pro Ser Glu Phe Thr Glu
            420                 425                 430
Val Gly Ala Asp Ile Glu Val Gln Val Ile Glu Ile Asp Lys Glu Asn
        435                 440                 445
Arg Arg Leu Ser Leu Gly His Lys Gln Leu Glu Glu Asn Pro Trp Asp
    450                 455                 460
Val Phe Glu Thr Val Phe Thr Val Gly Ser Ile His Glu Gly Thr Val
465                 470                 475                 480
Ile Glu Val Met Asp Lys Gly Ala Val Val Ser Leu Pro Tyr Gly Val
                485                 490                 495
Glu Gly Phe Ala Thr Pro Lys His Met Val Lys Glu Asp Gly Ser Gln
            500                 505                 510
Ala Val Leu Glu Glu Lys Leu Pro Phe Lys Val Ile Glu Phe Asn Lys
        515                 520                 525
Asp Ala Lys Arg Ile Ile Val Ser His Ser Arg Val Phe Glu Asp Glu
    530                 535                 540
Gln Lys Met Ala Gln Arg Glu Ala Asn Ala Glu Arg Lys Ala Glu Ala
545                 550                 555                 560
Lys Ala Ala Gln Lys Glu Ala Ala Ala Glu Ala Ala Asn Pro Ala Gln
                565                 570                 575
Ala Val Glu Lys Ala Thr Leu Gly Asp Leu Gly Glu Leu Ala Ala Leu
            580                 585                 590
Lys Glu Lys Leu Ser Glu Asn
        595

(2) INFORMATION FOR SEQ ID NO:499

    (i) SEQUENCE CHARACTERISTICS:
        (A) LENGTH: 550 amino acids
        (B) TYPE: amino acid
        (D) TOPOLOGY: linear

    (ii) MOLECULE TYPE: protein

    (iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...550

(xi) SEQUENCE DESCRIPTION: SEQ ID NO:499

Met Gly Arg Val Thr Ala Ile Asn Lys Arg Glu Val Val Ile Asn Val
1               5                   10                  15
Gly Tyr Lys Ser Glu Gly Val Val Pro Ala Thr Glu Phe Arg Tyr Asn
            20                  25                  30
Pro Glu Leu Lys Val Gly Asp Glu Val Glu Val Tyr Ile Glu Asn Gln
        35                  40                  45
Glu Asp Lys Lys Gly Gln Leu Val Leu Ser His Arg Lys Gly Arg Ala
    50                  55                  60
Ala Arg Ser Trp Glu Arg Val Asn Glu Ala Leu Glu Lys Asp Glu Ile
65                  70                  75                  80
Val Lys Gly Tyr Val Lys Cys Arg Thr Lys Gly Gly Met Ile Val Asp
                85                  90                  95
Val Phe Gly Ile Glu Ala Phe Leu Pro Gly Ser Gln Ile Asp Val Arg
            100                 105                 110
Pro Ile Arg Asp Tyr Asp Ala Phe Val Glu Lys Thr Met Glu Phe Lys
        115                 120                 125
Ile Val Lys Ile Asn Gln Glu Tyr Lys Asn Val Val Val Ser His Lys
    130                 135                 140
Val Leu Ile Glu Ala Glu Leu Glu Gln Gln Lys Lys Glu Ile Ile Gly
145                 150                 155                 160
Lys Leu Glu Lys Gly Gln Val Leu Glu Gly Ile Val Lys Asn Ile Thr
                165                 170                 175
Ser Tyr Gly Val Phe Ile Asp Leu Gly Gly Val Asp Gly Leu Ile His
            180                 185                 190
Ile Thr Asp Leu Ser Trp Gly Arg Val Ala His Pro Glu Glu Ile Val
        195                 200                 205
Gln Leu Asp Gln Lys Ile Asn Val Val Ile Leu Asp Phe Asp Glu Asp
    210                 215                 220
Arg Lys Arg Ile Ala Leu Gly Leu Lys Gln Leu Met Pro His Pro Trp
225                 230                 235                 240
Asp Ala Leu Asp Ser Glu Leu Lys Val Gly Asp Lys Val Lys Gly Lys
                245                 250                 255
Val Val Val Met Ala Asp Tyr Gly Ala Phe Val Glu Ile Ala Gln Gly
            260                 265                 270
Val Glu Gly Leu Ile His Val Ser Glu Met Ser Trp Thr Gln His Leu
        275                 280                 285
Arg Ser Ala Gln Asp Phe Leu His Val Gly Asp Glu Val Glu Ala Val
    290                 295                 300
Ile Leu Thr Leu Asp Arg Glu Glu Arg Lys Met Ser Leu Gly Leu Lys
305                 310                 315                 320
Gln Leu Lys Pro Asp Pro Trp Ala Asp Ile Glu Thr Arg Phe Pro Val
                325                 330                 335
Gly Ser Arg His His Ala Arg Val Arg Asn Phe Thr Asn Phe Gly Val
            340                 345                 350
Phe Val Glu Ile Glu Glu Gly Val Asp Gly Leu Ile His Ile Ser Asp
        355                 360                 365
Leu Ser Trp Thr Lys Lys Ile Lys His Pro Ser Glu Phe Thr Glu Val
    370                 375                 380
Gly Ala Asp Ile Glu Val Gln Val Ile Glu Ile Asp Lys Glu Asn Arg
385                 390                 395                 400
Arg Leu Ser Leu Gly His Lys Gln Leu Glu Glu Asn Pro Trp Asp Val
                405                 410                 415
Phe Glu Thr Val Phe Thr Val Gly Ser Ile His Glu Gly Thr Val Ile
            420                 425                 430
Glu Val Met Asp Lys Gly Ala Val Val Ser Leu Pro Tyr Gly Val Glu
        435                 440                 445
Gly Phe Ala Thr Pro Lys His Met Val Lys Glu Asp Gly Ser Gln Ala
    450                 455                 460
Val Leu Glu Glu Lys Leu Pro Phe Lys Val Ile Glu Phe Asn Lys Asp
465                 470                 475                 480
Ala Lys Arg Ile Ile Val Ser His Ser Arg Val Phe Glu Asp Glu Gln
                485                 490                 495
Lys Met Ala Gln Arg Glu Ala Asn Ala Glu Arg Lys Ala Glu Ala Lys
            500                 505                 510
Ala Ala Gln Lys Glu Ala Ala Ala Glu Ala Ala Asn Pro Ala Gln Ala
        515                 520                 525

Val Glu Lys Ala Thr Leu Gly Asp Leu Gly Glu Leu Ala Ala Leu Lys
    530                 535                 540
Glu Lys Leu Ser Glu Asn
545                 550

(2) INFORMATION FOR SEQ ID NO:500

     (i) SEQUENCE CHARACTERISTICS:
          (A) LENGTH: 458 amino acids
          (B) TYPE: amino acid
          (D) TOPOLOGY: linear

     (ii) MOLECULE TYPE: protein

     (iii) HYPOTHETICAL: YES

     (vi) ORIGINAL SOURCE:
          (A) ORGANISM: Porphyromonas gingivalis

     (ix) FEATURE:
          (A) NAME/KEY: misc_feature
          (B) LOCATION 1...458

     (xi) SEQUENCE DESCRIPTION: SEQ ID NO:500

Met Ile Val Asp Val Phe Gly Ile Glu Ala Phe Leu Pro Gly Ser Gln
1               5                   10                  15
Ile Asp Val Arg Pro Ile Arg Asp Tyr Asp Ala Phe Val Glu Lys Thr
            20                  25                  30
Met Glu Phe Lys Ile Val Lys Ile Asn Gln Glu Tyr Lys Asn Val Val
        35                  40                  45
Val Ser His Lys Val Leu Ile Glu Ala Glu Leu Glu Gln Gln Lys Lys
    50                  55                  60
Glu Ile Ile Gly Lys Leu Glu Lys Gly Gln Val Leu Glu Gly Ile Val
65                  70                  75                  80
Lys Asn Ile Thr Ser Tyr Gly Val Phe Ile Asp Leu Gly Gly Val Asp
                85                  90                  95
Gly Leu Ile His Ile Thr Asp Leu Ser Trp Gly Arg Val Ala His Pro
            100                 105                 110
Glu Glu Ile Val Gln Leu Asp Gln Lys Ile Asn Val Val Ile Leu Asp
        115                 120                 125
Phe Asp Glu Asp Arg Lys Arg Ile Ala Leu Gly Leu Lys Gln Leu Met
    130                 135                 140
Pro His Pro Trp Asp Ala Leu Asp Ser Glu Leu Lys Val Gly Asp Lys
145                 150                 155                 160
Val Lys Gly Lys Val Val Val Met Ala Asp Tyr Gly Ala Phe Val Glu
                165                 170                 175
Ile Ala Gln Gly Val Glu Gly Leu Ile His Val Ser Glu Met Ser Trp
            180                 185                 190
Thr Gln His Leu Arg Ser Ala Gln Asp Phe Leu His Val Gly Asp Glu
        195                 200                 205
Val Glu Ala Val Ile Leu Thr Leu Asp Arg Glu Glu Arg Lys Met Ser
    210                 215                 220
Leu Gly Leu Lys Gln Leu Lys Pro Asp Pro Trp Ala Asp Ile Glu Thr
225                 230                 235                 240
Arg Phe Pro Val Gly Ser Arg His His Ala Arg Val Arg Asn Phe Thr
                245                 250                 255
Asn Phe Gly Val Phe Val Glu Ile Glu Glu Gly Val Asp Gly Leu Ile
            260                 265                 270
His Ile Ser Asp Leu Ser Trp Thr Lys Lys Ile Lys His Pro Ser Glu
        275                 280                 285
Phe Thr Glu Val Gly Ala Asp Ile Glu Val Gln Val Ile Glu Ile Asp
    290                 295                 300
Lys Glu Asn Arg Arg Leu Ser Leu Gly His Lys Gln Leu Glu Glu Asn
305                 310                 315                 320
Pro Trp Asp Val Phe Glu Thr Val Phe Thr Val Gly Ser Ile His Glu
                325                 330                 335
Gly Thr Val Ile Glu Val Met Asp Lys Gly Ala Val Val Ser Leu Pro
            340                 345                 350
Tyr Gly Val Glu Gly Phe Ala Thr Pro Lys His Met Val Lys Glu Asp
        355                 360                 365
Gly Ser Gln Ala Val Leu Glu Glu Lys Leu Pro Phe Lys Val Ile Glu
    370                 375                 380
Phe Asn Lys Asp Ala Lys Arg Ile Ile Val Ser His Ser Arg Val Phe
385                 390                 395                 400

Glu Asp Glu Gln Lys Met Ala Gln Arg Glu Ala Asn Ala Glu Arg Lys
                405                 410                 415
Ala Glu Ala Lys Ala Ala Gln Lys Glu Ala Ala Ala Glu Ala Ala Asn
            420                 425                 430
Pro Ala Gln Ala Val Glu Lys Ala Thr Leu Gly Asp Leu Gly Glu Leu
        435                 440                 445
Ala Ala Leu Lys Glu Lys Leu Ser Glu Asn
    450                 455

(2) INFORMATION FOR SEQ ID NO:501

     (i) SEQUENCE CHARACTERISTICS:
          (A) LENGTH: 426 amino acids
          (B) TYPE: amino acid
          (D) TOPOLOGY: linear

    (ii) MOLECULE TYPE: protein

   (iii) HYPOTHETICAL: YES

    (vi) ORIGINAL SOURCE:
          (A) ORGANISM: Porphyromonas gingivalis

    (ix) FEATURE:
          (A) NAME/KEY: misc_feature
          (B) LOCATION 1...426

    (xi) SEQUENCE DESCRIPTION: SEQ ID NO:501

Met Glu Phe Lys Ile Val Lys Ile Asn Gln Glu Tyr Lys Asn Val Val
1               5                   10                  15
Val Ser His Lys Val Leu Ile Glu Ala Glu Leu Glu Gln Gln Lys Lys
            20                  25                  30
Glu Ile Ile Gly Lys Leu Glu Lys Gly Gln Val Leu Glu Gly Ile Val
        35                  40                  45
Lys Asn Ile Thr Ser Tyr Gly Val Phe Ile Asp Leu Gly Gly Val Asp
    50                  55                  60
Gly Leu Ile His Ile Thr Asp Leu Ser Trp Gly Arg Val Ala His Pro
65                  70                  75                  80
Glu Glu Ile Val Gln Leu Asp Gln Lys Ile Asn Val Val Ile Leu Asp
                85                  90                  95
Phe Asp Glu Asp Arg Lys Arg Ile Ala Leu Gly Leu Lys Gln Leu Met
            100                 105                 110
Pro His Pro Trp Asp Ala Leu Asp Ser Glu Leu Lys Val Gly Asp Lys
        115                 120                 125
Val Lys Gly Lys Val Val Val Met Ala Asp Tyr Gly Ala Phe Val Glu
    130                 135                 140
Ile Ala Gln Gly Val Glu Gly Leu Ile His Val Ser Glu Met Ser Trp
145                 150                 155                 160
Thr Gln His Leu Arg Ser Ala Gln Asp Phe Leu His Val Gly Asp Glu
                165                 170                 175
Val Glu Ala Val Ile Leu Thr Leu Asp Arg Glu Glu Arg Lys Met Ser
            180                 185                 190
Leu Gly Leu Lys Gln Leu Lys Pro Asp Pro Trp Ala Asp Ile Glu Thr
        195                 200                 205
Arg Phe Pro Val Gly Ser Arg His His Ala Arg Val Arg Asn Phe Thr
    210                 215                 220
Asn Phe Gly Val Phe Val Glu Ile Glu Glu Gly Val Asp Gly Leu Ile
225                 230                 235                 240
His Ile Ser Asp Leu Ser Trp Thr Lys Lys Ile Lys His Pro Ser Glu
                245                 250                 255
Phe Thr Glu Val Gly Ala Asp Ile Glu Val Gln Val Ile Glu Ile Asp
            260                 265                 270
Lys Glu Asn Arg Arg Leu Ser Leu Gly His Lys Gln Leu Glu Glu Asn
        275                 280                 285
Pro Trp Asp Val Phe Glu Thr Val Phe Thr Val Gly Ser Ile His Glu
    290                 295                 300
Gly Thr Val Ile Glu Val Met Asp Lys Gly Ala Val Val Ser Leu Pro
305                 310                 315                 320
Tyr Gly Val Glu Gly Phe Ala Thr Pro Lys His Met Val Lys Glu Asp
                325                 330                 335
Gly Ser Gln Ala Val Leu Glu Glu Lys Leu Pro Phe Lys Val Ile Glu
            340                 345                 350
Phe Asn Lys Asp Ala Lys Arg Ile Ile Val Ser His Ser Arg Val Phe
        355                 360                 365

Glu Asp Glu Gln Lys Met Ala Gln Arg Glu Ala Asn Ala Glu Arg Lys
    370                 375                 380
Ala Glu Ala Lys Ala Ala Gln Lys Glu Ala Ala Ala Glu Ala Ala Asn
385                 390                 395                 400
Pro Ala Gln Ala Val Glu Lys Ala Thr Leu Gly Asp Leu Gly Glu Leu
                405                 410                 415
Ala Ala Leu Lys Glu Lys Leu Ser Glu Asn
            420                 425

(2) INFORMATION FOR SEQ ID NO:502

    (i) SEQUENCE CHARACTERISTICS:
        (A) LENGTH: 240 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...240

    (xi) SEQUENCE DESCRIPTION: SEQ ID NO:502

Met Lys Lys Ala Ile Leu Ser Gly Ala Ala Leu Leu Leu Gly Leu Cys
1               5                   10                  15
Ala Asn Ala Gln Asn Val Gln Leu His Tyr Asp Phe Gly His Ser Ile
            20                  25                  30
Tyr Asp Glu Leu Asp Gly Arg Pro Lys Leu Thr Thr Thr Val Glu Asn
        35                  40                  45
Phe Thr Pro Asp Lys Trp Gly Ser Thr Phe Phe Phe Ile Asp Met Asp
    50                  55                  60
Tyr Thr Gly Lys Gly Ile Gln Ser Ala Tyr Trp Glu Ile Ser Arg Glu
65                  70                  75                  80
Leu Lys Phe Trp Gln Ala Pro Val Ser Ile His Leu Glu Tyr Asn Gly
                85                  90                  95
Gly Leu Ser Thr Ser Phe Thr Phe Gly His Asp Ala Leu Ile Gly Ala
            100                 105                 110
Thr Tyr Thr Tyr Asn Asn Pro Ser Phe Thr Arg Gly Phe Thr Ile Thr
        115                 120                 125
Pro Met Tyr Lys His Leu Gly Ala His Asp Phe His Thr Tyr Gln Ile
    130                 135                 140
Thr Gly Thr Trp Tyr Met His Phe Leu Asp Gly Leu Leu Thr Phe Asn
145                 150                 155                 160
Gly Phe Leu Asp Leu Trp Gly Phe Pro Gln Glu Asn Pro Ile Gly Gly
                165                 170                 175
Pro Val Leu Lys Glu Gly Asp Lys Phe Val Phe Leu Ser Glu Pro Gln
            180                 185                 190
Phe Trp Ile Asn Leu Asn Arg Ile Lys Gly Ile Asp Lys Asp Phe Asn
        195                 200                 205
Leu Ser Ile Gly Thr Glu Met Glu Ile Ser Arg Asn Phe Ala Arg Met
    210                 215                 220
Asp Lys Phe Ser Cys Ile Pro Thr Leu Ala Val Lys Trp Thr Phe Asn
225                 230                 235                 240

(2) INFORMATION FOR SEQ ID NO:503

    (i) SEQUENCE CHARACTERISTICS:
        (A) LENGTH: 434 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:

(A) NAME/KEY: misc_feature
(B) LOCATION 1...434

(xi) SEQUENCE DESCRIPTION: SEQ ID NO:503

Met Tyr Lys Asp Tyr Lys Gly Leu Tyr Ala Ser Leu Arg Trp Tyr Ala
1               5                   10                  15
Leu Ile Ile Gly Leu Leu Phe Ala Ala Asp Gly Ile Gln Ala Gln Asn
            20                  25                  30
Asn Asn Phe Thr Glu Ser Pro Tyr Thr Arg Phe Gly Leu Gly Arg Leu
        35                  40                  45
Gly Glu Arg Thr Thr Ile Ser Gly His Ser Met Gly Gly Leu Gly Val
    50                  55                  60
Gly Leu Arg Gln Gly Thr Tyr Val Asn Ala Val Asn Pro Ala Ser Tyr
65                  70                  75                  80
Ser Ala Val Asp Ser Met Thr Phe Ile Phe Asp Phe Gly Ala Ser Thr
                85                  90                  95
Gly Ile Thr Trp Tyr Ala Glu Asn Gly Lys Lys Asp Asn Arg Lys Met
            100                 105                 110
Gly Asn Ile Glu Tyr Phe Ala Met Leu Phe Pro Ile Ser Lys Ser Ile
        115                 120                 125
Ala Met Ser Ala Gly Val Leu Pro Tyr Ser Ala Ser Gly Tyr Gln Phe
    130                 135                 140
Gly Ser Val Asp Gln Val Glu Gly Gly Ser Val Gln Tyr Thr Arg Lys
145                 150                 155                 160
Tyr Leu Gly Thr Gly Asn Leu Asn Asp Leu Tyr Val Gly Ile Gly Ala
                165                 170                 175
Thr Pro Phe Lys Asn Phe Ser Ile Gly Ala Asn Ala Ser Ser Leu Phe
            180                 185                 190
Gly Arg Phe Thr His Ser Arg Gln Val Ile Phe Ser Thr Glu Ala Pro
        195                 200                 205
Tyr Asn Pro Val His Leu Ser Thr Leu Tyr Leu Lys Ala Ala Lys Phe
    210                 215                 220
Asp Phe Gly Met Gln Tyr His Leu Leu Leu Lys Ser Asp Arg Ser Leu
225                 230                 235                 240
Val Ile Gly Ala Val Tyr Ser Pro Arg Val Lys Met His Ser Glu Leu
                245                 250                 255
Thr Gln Ile Lys Asn Gln Val Gln Asn Gly Val Val Val Glu Ser Glu
            260                 265                 270
Thr Gln Glu Tyr Ile Lys Gly Met Asp Tyr Tyr Thr Leu Pro His Thr
        275                 280                 285
Leu Gly Ile Gly Phe Ser Tyr Glu Lys Lys Asp Lys Leu Leu Leu Gly
    290                 295                 300
Ala Asp Val Gln Tyr Ser Lys Trp Lys Gly Glu Lys Phe Tyr Lys Ser
305                 310                 315                 320
Asp Cys Lys Phe Gln Asp Arg Ile Arg Val Ser Leu Gly Gly Glu Ile
                325                 330                 335
Ile Pro Asp Ile Asn Ala Val Gly Met Trp Pro Lys Val Arg Tyr Arg
            340                 345                 350
Phe Gly Leu His Gly Glu Asn Ser Tyr Leu Lys Val Pro Thr Lys Gly
        355                 360                 365
Gly Val Tyr Gln Gly Tyr His Ile Val Gly Ala Val Phe Gly Ile Gly
    370                 375                 380
Ile Pro Leu Asn Asp Arg Arg Ser Phe Val Asn Val Ser Leu Glu Tyr
385                 390                 395                 400
Asp Arg Leu Ile Pro Lys Glu Gly Met Ile Lys Glu Asn Ala Leu Lys
                405                 410                 415
Leu Thr Phe Gly Leu Thr Phe Asn Glu Ser Trp Phe Lys Lys Leu Lys
            420                 425                 430
Leu Asn

(2) INFORMATION FOR SEQ ID NO:504

(i) SEQUENCE CHARACTERISTICS:
(A) LENGTH: 926 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...926

(xi) SEQUENCE DESCRIPTION: SEQ ID NO:504

Met Arg Ser Ile Tyr Gln Leu Leu Leu Ser Ile Leu Leu Ala Ser Leu
1               5                   10                  15
Gly Phe Val Gly Leu Glu Ala Gln Gln Ala Gly Val Ala Gly Arg Val
            20                  25                  30
Leu Asp Glu Glu Gly Asn Pro Met Ile Gln Ala Asn Val Gln Leu Val
        35                  40                  45
Gln Ser Thr Gly Gln Val Ala Val Ala Ala Gly Ala Thr Asn Glu Lys
    50                  55                  60
Gly Leu Phe Ser Leu Lys Thr Ser Gln Glu Gly Asp Tyr Ile Leu Arg
65                  70                  75                  80
Val Ser Tyr Val Gly Tyr Thr Thr His Asp Glu Lys Ile Ser Leu Arg
                85                  90                  95
Asn Gly Gln Thr Ile Thr Leu Lys Asp Ile Ser Met Asn Glu Asp Ala
            100                 105                 110
Arg Leu Leu Gln Ser Val Thr Val Gln Ala Lys Ala Ala Glu Val Val
        115                 120                 125
Val Arg Asn Asp Thr Leu Glu Phe Asn Ala Gly Ser Tyr Thr Val Ala
    130                 135                 140
Gln Gly Ala Ser Ile Glu Glu Leu Ile Lys Lys Leu Pro Gly Ala Glu
145                 150                 155                 160
Ile Gly Ser Asp Gly Lys Ile Thr Ile Asn Gly Lys Asp Ile Ser Lys
                165                 170                 175
Ile Leu Val Asp Gly Lys Glu Phe Phe Ser Lys Asp Pro Gln Val Ala
            180                 185                 190
Ile Lys Asn Leu Pro Ala Asp Met Val Asn Lys Val Gln Val Leu Asn
        195                 200                 205
Lys Leu Ser Glu Leu Ser Arg Met Ser Gly Phe Asp Asp Gly Glu Glu
    210                 215                 220
Glu Thr Val Ile Asn Leu Thr Val Lys Pro Glu Lys Lys Lys Gly Leu
225                 230                 235                 240
Phe Gly Thr Leu Gln Ala Gly Tyr Gly Thr Asp Gln Arg Tyr Met Ala
                245                 250                 255
Gly Gly Asn Val Asn Arg Phe Asp Gly Asn Lys Gln Trp Thr Leu Ile
            260                 265                 270
Gly Ser Ala Asn Asn Thr Asn Asn Met Gly Phe Ser Glu Met Asp Ser
        275                 280                 285
Glu Met Gly Ser Met Thr Phe Phe Ser Pro Gln Gly Gly Gly Arg Arg
    290                 295                 300
Gly Phe Gly Asn Ser Gly Gly Val Thr Ser Ser Ser Met Leu Gly Gly
305                 310                 315                 320
Asn Phe Ser Val Glu Phe Ser Ser Ala Leu Asn Thr Gly Gly Asp Ala
                325                 330                 335
Arg Tyr Gly Tyr Asn Asp Lys Ala Ile Glu Thr Thr Lys Arg Val Glu
            340                 345                 350
Asn Ile Leu Ala Glu Gly Asn Thr Tyr Met Asp Glu Asn Ile Leu Glu
        355                 360                 365
Arg Ser Phe Ser His Asn Gly Gln Ala Arg Phe Arg Met Gln Trp Lys
    370                 375                 380
Pro Ser Glu Arg Thr Glu Val Val Phe Glu Pro Asp Leu Ser Ile Ser
385                 390                 395                 400
Lys Ile Asp Gly Phe Phe Asn Asp Thr Tyr Glu Thr Lys Asp Ala Thr
                405                 410                 415
Gly Ile Ser Ile Asn Lys Gly Ser Ile His Gln Thr Thr Gln Gly Asn
            420                 425                 430
Asn Phe Arg Leu Asn Gly Glu Leu Asp Ile Ser His Lys Leu Asn Asp
        435                 440                 445
Glu Gly Arg Thr Ile Ser Ala Ser Val Ser Gly Gly Leu Thr Asp Glu
    450                 455                 460
Asp Gly Asp Gly Ile Tyr Gln Ala Val Leu Gln Ser Val Glu Thr Asn
465                 470                 475                 480
Gln Lys Gln Phe Asn Asp Asn Ser Asn Leu Gln Tyr Arg Leu Arg Leu
                485                 490                 495
Ser Tyr Val Glu Pro Leu Gly Lys Asn Tyr Phe Ala Gln Ala Ile Leu
            500                 505                 510
Asn Arg Arg Phe Ser Arg Arg Asn Ser Asp Arg Glu Val Tyr Arg Leu
        515                 520                 525
Gly Asp Asp Gly Gln Tyr Ser Ile Leu Asp Ser Gln Tyr Gly Leu Ser
    530                 535                 540

Tyr Ser Asn Glu Phe Thr Gln Tyr Arg Ile Gly Leu Asn Leu Lys Lys
545                 550                 555                 560
Ile Ala Lys Thr Trp Asp Tyr Thr Val Gly Phe Asn Val Asp Pro Asn
                565                 570                 575
Arg Thr Val Ser Tyr Arg Ser Val Ala Gly Val Glu Gln Asp Lys Leu
            580                 585                 590
Ala Phe Asn Arg Val Asn Leu Ser Pro Met Leu Arg Ile Asn Tyr Lys
        595                 600                 605
Pro Ser Arg Thr Thr Asn Leu Arg Val Asp Tyr Arg Gly Arg Thr Thr
    610                 615                 620
Gln Pro Ser Ile Asn Gln Ile Ala Pro Val Gln Asp Ile Thr Asn Pro
625                 630                 635                 640
Leu Phe Val Thr Glu Gly Asn Pro Gly Leu Lys Pro Ser Tyr Ser Asn
                645                 650                 655
Asn Val Met Ala Met Phe Ser Asp Phe Asp Ala Lys Ser Gln Arg Ala
            660                 665                 670
Phe Asn Ile Val Phe Phe Gly Asn Tyr Thr Phe Asp Asp Ile Val Pro
        675                 680                 685
Asn Thr His Tyr Asp Pro Ser Thr Gly Ile Arg Thr Thr Arg Tyr Glu
    690                 695                 700
Asn Ala Ser Gly Thr Trp Gln Ala Asn Leu His Gly Thr Leu Ser Leu
705                 710                 715                 720
Pro Leu Lys Asn Arg Ala Phe Ser Phe Arg Met Ser Leu Phe Asn Arg
                725                 730                 735
Leu Ala Glu Gly Gln Ser Phe Ile Asn Asp Asp Lys Asn Lys Ala Leu
            740                 745                 750
Ser Phe Arg Thr Arg Glu Arg Leu Thr Leu Thr Tyr Arg Asn Asn Trp
        755                 760                 765
Ile Asp Thr Ser Ile Gly Gly Asn Ile Gly Phe Tyr Met Ala Asn Asn
    770                 775                 780
Ser Leu Ser Gly Gln Lys Asp Ser Arg Thr Tyr Asp Phe Gly Gly Asn
785                 790                 795                 800
Tyr Gln Val Ala Leu Thr Leu Pro Tyr Gly Phe Arg Ile Asp Ser Asp
                805                 810                 815
Val Glu Tyr Asn Thr Asn Ser Gly Tyr Ser Gly Gly Phe Ser Leu Asp
            820                 825                 830
Glu Trp Leu Trp Asn Ala Ser Leu Ser Tyr Ser Phe Leu Arg Asp Lys
        835                 840                 845
Ala Gly Thr Leu Arg Val Asn Gly Tyr Asp Ile Leu Gly Gln Arg Ser
    850                 855                 860
Ser Ile Ser Arg Ser Ala Ser Ala Ile Asn Ile Glu Glu Ser Met Ser
865                 870                 875                 880
Asn Thr Ile Gly Arg Tyr Val Met Val Asp Phe Ile Tyr Arg Phe Asn
                885                 890                 895
Ala Phe Ser Gly Gly Gly Ser Arg Ser Asp His Gln Arg Gly Asn Met
            900                 905                 910
Asn Arg Pro Gly Pro Pro Phe Gly Gly Gly Arg Arg Pro Ser
        915                 920                 925

(2) INFORMATION FOR SEQ ID NO:505

(i) SEQUENCE CHARACTERISTICS:
(A) LENGTH: 400 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...400

(xi) SEQUENCE DESCRIPTION: SEQ ID NO:505

Met Val Met Lys Leu Ile Lys Arg Ser Leu Leu Leu Leu Gly Ala Val
1               5                   10                  15
Leu Leu Ile Thr Leu Pro Ala Tyr Ser Gln Asn Asp Asp Ile Phe Glu
            20                  25                  30
Asp Asp Ile Tyr Thr Ser Arg Lys Glu Ile Arg Lys Gln Asn Gln Val
        35                  40                  45

Lys Asp Trp Gln Asn Gln Glu Asp Gly Tyr Gly Asp Asp Thr Glu Tyr
    50                  55                  60
Thr Val Ala Ser Asp Arg Asp Ile Asp Ala Tyr Asn Arg Arg Asp Gly
65                  70                  75                  80
Gln Ser Tyr Asp Gly Lys Lys Leu Ser Lys Asp Lys Lys Arg Asp Ser
                85                  90                  95
Thr Arg Ser Ser Val Pro Gly Arg Tyr Ser Arg Arg Leu Ala Arg Phe
            100                 105                 110
Tyr Lys Pro Asn Thr Ile Val Ile Ser Gly Ala Asp Asn Val Tyr Val
        115                 120                 125
Thr Asp Asp Gly Glu Tyr Phe Val Tyr Gly Asp Glu Tyr Tyr Asp Asp
    130                 135                 140
Ala Ser Ser Val Asn Ile Tyr Ile Asn Ser Pro Trp Cys Asp Pro Phe
145                 150                 155                 160
Pro Tyr Thr Ser Trp Tyr Pro Ser Phe Ser Gly Trp Tyr Asn Tyr Thr
                165                 170                 175
Trp Asn Tyr Pro Trp Phe Tyr Tyr Gly Ser His Ile Gly Trp Gly Gly
            180                 185                 190
Tyr Tyr Pro Gly Tyr Asn Trp Tyr Trp Ser Tyr Tyr Tyr Asp Pro Phe
        195                 200                 205
Tyr Asn Pro Tyr Gly Ile Gly Met Gly Trp Gly Tyr Pro Tyr Gly Trp
    210                 215                 220
Gly Ser Tyr Tyr Gly Trp Gly Gly Tyr Pro Gly Val Ile His His Tyr
225                 230                 235                 240
His His Tyr Pro Lys Lys Thr Tyr Ser Asn Gly Gln His Ser Gly Ala
                245                 250                 255
Tyr Tyr Ser Tyr Gly Arg Pro Asn Arg Ile Lys Gly Gly Thr Ser Gly
            260                 265                 270
Ala Lys Leu Gly Thr Gly Arg Tyr Asp Arg Ile Gln Asn Ser Ser Ser
        275                 280                 285
Gln Lys Asn Lys Phe Gly Leu Gln Ser Asn Lys Pro Asn Asn Asn Leu
    290                 295                 300
Gln Asn Val Lys Ser Gly Arg Thr Gly Arg Ala Asn Arg Asp Arg Asn
305                 310                 315                 320
Ile Glu Thr Val Thr Pro Asn Asn Gly Gln Lys Gln Asn Arg Pro Val
                325                 330                 335
Phe Gln Gln Asn Gln Ser Gly Asn Asp Arg Pro Thr Gly Arg Asn Ile
            340                 345                 350
Arg Ser Glu Arg Gln Gly Glu Asn Asn Asp Arg Thr Phe Ser Thr Pro
        355                 360                 365
Ser Arg Ser Asn Ser Asn Gly Gly Phe Ser Thr Pro Ser Arg Ser Ser
    370                 375                 380
Ser Gly Ser Met Ser Gly Gly Gly Gly Arg Ser Gly Arg Gly Arg Asn
385                 390                 395                 400

(2) INFORMATION FOR SEQ ID NO:506

    (i) SEQUENCE CHARACTERISTICS:
        (A) LENGTH: 398 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...398

    (xi) SEQUENCE DESCRIPTION: SEQ ID NO:506

Met Lys Leu Ile Lys Arg Ser Leu Leu Leu Leu Gly Ala Val Leu Leu
1               5                   10                  15
Ile Thr Leu Pro Ala Tyr Ser Gln Asn Asp Asp Ile Phe Glu Asp Asp
            20                  25                  30
Ile Tyr Thr Ser Arg Lys Glu Ile Arg Lys Gln Asn Gln Val Lys Asp
        35                  40                  45
Trp Gln Asn Gln Glu Asp Gly Tyr Gly Asp Asp Thr Glu Tyr Thr Val
    50                  55                  60
Ala Ser Asp Arg Asp Ile Asp Ala Tyr Asn Arg Arg Asp Gly Gln Ser
65                  70                  75                  80

Tyr Asp Gly Lys Lys Leu Ser Lys Asp Lys Lys Arg Asp Ser Thr Arg
                85                  90                  95
Ser Ser Val Pro Gly Arg Tyr Ser Arg Arg Leu Ala Arg Phe Tyr Lys
            100                 105                 110
Pro Asn Thr Ile Val Ile Ser Gly Ala Asp Asn Val Tyr Val Thr Asp
        115                 120                 125
Asp Gly Glu Tyr Phe Val Tyr Gly Asp Glu Tyr Tyr Asp Asp Ala Ser
    130                 135                 140
Ser Val Asn Ile Tyr Ile Asn Ser Pro Trp Cys Asp Pro Phe Pro Tyr
145                 150                 155                 160
Thr Ser Trp Tyr Pro Ser Phe Ser Gly Trp Tyr Asn Tyr Thr Trp Asn
                165                 170                 175
Tyr Pro Trp Phe Tyr Tyr Gly Ser His Ile Gly Trp Gly Gly Tyr Tyr
            180                 185                 190
Pro Gly Tyr Asn Trp Tyr Trp Ser Tyr Tyr Tyr Asp Pro Phe Tyr Asn
        195                 200                 205
Pro Tyr Gly Ile Gly Met Gly Trp Gly Tyr Pro Tyr Gly Trp Gly Ser
    210                 215                 220
Tyr Tyr Gly Trp Gly Gly Tyr Pro Gly Val Ile His His Tyr His His
225                 230                 235                 240
Tyr Pro Lys Lys Thr Tyr Ser Asn Gly Gln His Ser Gly Ala Tyr Tyr
                245                 250                 255
Ser Tyr Gly Arg Pro Asn Arg Ile Lys Gly Gly Thr Ser Gly Ala Lys
            260                 265                 270
Leu Gly Thr Gly Arg Tyr Asp Arg Ile Gln Asn Ser Ser Ser Gln Lys
        275                 280                 285
Asn Lys Phe Gly Leu Gln Ser Asn Lys Pro Asn Asn Asn Leu Gln Asn
    290                 295                 300
Val Lys Ser Gly Arg Thr Gly Arg Ala Asn Arg Asp Arg Asn Ile Glu
305                 310                 315                 320
Thr Val Thr Pro Asn Asn Gly Gln Lys Gln Asn Arg Pro Val Phe Gln
                325                 330                 335
Gln Asn Gln Ser Gly Asn Asp Arg Pro Thr Gly Arg Asn Ile Arg Ser
            340                 345                 350
Glu Arg Gln Gly Glu Asn Asn Asp Arg Thr Phe Ser Thr Pro Ser Arg
        355                 360                 365
Ser Asn Ser Asn Gly Gly Phe Ser Thr Pro Ser Arg Ser Ser Ser Gly
    370                 375                 380
Ser Met Ser Gly Gly Gly Gly Arg Ser Gly Arg Gly Arg Asn
385                 390                 395

(2) INFORMATION FOR SEQ ID NO:507

     (i) SEQUENCE CHARACTERISTICS:
          (A) LENGTH: 581 amino acids
          (B) TYPE: amino acid
          (D) TOPOLOGY: linear

    (ii) MOLECULE TYPE: protein

   (iii) HYPOTHETICAL: YES

    (vi) ORIGINAL SOURCE:
          (A) ORGANISM: Porphyromonas gingivalis

    (ix) FEATURE:
          (A) NAME/KEY: misc_feature
          (B) LOCATION 1...581

    (xi) SEQUENCE DESCRIPTION: SEQ ID NO:507

Met Ile Arg Lys Leu Ile Leu Leu Leu Ala Leu Met Pro Val Ala Ser
1               5                   10                  15
Val Ala Phe Ala Val Pro Thr Asp Ser Thr Glu Ser Lys Asp Asn Arg
            20                  25                  30
Ile Leu Thr Ser Met Gln Ser Ser Ser Leu Asn Arg Asp Asp Ala Pro
        35                  40                  45
Asp Lys Trp Gln Pro Met His Ala Asn Phe Ser Ile Gln Ser Asp Met
    50                  55                  60
Leu Leu Ser Thr Ala Gln Lys Ser Lys Asn Thr Trp Phe Gly Asn Ser
65                  70                  75                  80
Tyr Ile Met Gly Ile Ile Lys Asn Asn Tyr Leu Glu Phe Gly Ala Arg
                85                  90                  95
Phe Glu Asp Leu Tyr Lys Pro Leu Pro Gly His Glu Pro Glu Met Gly
            100                 105                 110

Arg Gly Val Pro His Met Tyr Val Lys Gly Ser Tyr His Trp Ala Glu
          115                 120                 125
Leu Thr Met Gly Asp Phe Tyr Asp Gln Phe Gly Ser Gly Met Val Phe
    130                 135                 140
Arg Thr Tyr Glu Glu Arg Asn Leu Gly Ile Asp Asn Ala Val Arg Gly
145                 150                 155                 160
Gly Arg Ile Val Leu Thr Pro Phe Asp Gly Val Arg Val Lys Gly Ile
                165                 170                 175
Ala Gly Gln Gln Arg Asn Tyr Phe Asp Arg Thr Gly Lys Val Phe Asn
            180                 185                 190
Ser Gly Arg Gly Tyr Leu Leu Gly Ser Asp Leu Glu Leu Asn Val Glu
        195                 200                 205
Arg Trp Ser Ser Ala Met Arg Asp Asn Asp Tyr His Leu Ala Ile Gly
    210                 215                 220
Gly Ser Phe Val Ser Lys His Glu Ala Asp Glu Asp Ile Phe Val Gly
225                 230                 235                 240
Val Gly Glu Asp Arg Lys Arg Leu Asn Leu Pro Leu Asn Val Pro Ile
                245                 250                 255
Met Gly Leu Arg Thr Asn Phe Gln Lys Gly Gly Leu Ala Leu Tyr Ala
            260                 265                 270
Glu Tyr Gly Tyr Lys Tyr Asn Asp Pro Ser Ala Asp Asn Asp Tyr Ile
        275                 280                 285
Tyr His Asp Gly Gln Ala Ala Leu Leu Ser Ala Ser Tyr Ser Lys Lys
    290                 295                 300
Gly Met Ser Ile Leu Leu Gln Ala Lys Arg Cys Glu Asn Phe Ala Phe
305                 310                 315                 320
Arg Ser Lys Arg Ser Ala Gln Leu Thr Pro Leu Met Ile Asn Tyr Met
                325                 330                 335
Pro Ala Phe Thr Gln Ala His Thr Tyr Thr Leu Ala Ala Ile Tyr Pro
            340                 345                 350
Tyr Ala Thr Gln Pro Gln Gly Glu Trp Ala Phe Gln Gly Glu Leu Arg
        355                 360                 365
Tyr Asn Phe Ala Arg Arg Thr Ala Leu Gly Gly Arg Tyr Gly Thr Gly
    370                 375                 380
Leu Arg Ile Asn Val Ser His Val Arg Gly Leu Asp Lys Lys Met Leu
385                 390                 395                 400
Lys Glu Asn Pro Asp Glu Leu Ile Gly Thr Asp Gly Tyr Thr Val Ser
                405                 410                 415
Phe Phe Gly Met Gly Asp Leu Tyr Tyr Ser Asp Ile Asp Val Glu Ile
            420                 425                 430
Thr Lys Lys Val Ser Pro Gly Phe Asn Phe Thr Leu Thr Tyr Leu Asn
        435                 440                 445
Gln Ile Tyr Asn Asn Lys Val Leu His Gly Ala Ala Gly Glu Lys Pro
    450                 455                 460
Glu Lys Ile Tyr Ala Asn Ile Phe Val Tyr Asp Gly Lys Tyr Lys Leu
465                 470                 475                 480
Ser Asn Lys Val Ala Leu Arg Thr Glu Leu Gln Tyr Leu His Thr Lys
                485                 490                 495
Gln Asp Gln Gly Asp Trp Ile Tyr Gly Met Ala Glu Leu Ser Ile Leu
            500                 505                 510
Pro Ser Leu Met Leu Ser Leu Ser Glu Gln Tyr Asn Ile Gly Glu Thr
        515                 520                 525
Lys Lys His Tyr Val Met Gly Ser Val Thr Tyr Thr His Gly Ala His
    530                 535                 540
Arg Val Ala Phe Ser Ala Gly Lys Thr Arg Ala Gly Met Asn Cys Ser
545                 550                 555                 560
Gly Gly Val Cys Arg Val Val Pro Glu Thr Gln Gly Phe Tyr Leu Ser
                565                 570                 575
Tyr Ser Thr Asn Leu
            580

(2) INFORMATION FOR SEQ ID NO:508

    (i) SEQUENCE CHARACTERISTICS:
        (A) LENGTH: 239 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

(ix) FEATURE:
(A) NAME/KEY: misc_feature
(B) LOCATION 1...239

(xi) SEQUENCE DESCRIPTION: SEQ ID NO:508

Met Arg Ser Leu Phe Leu Ser Ala Leu Arg Ser Ser Ser Leu His Gly
1               5                   10                  15
Ser Glu Arg Arg Ser Arg Ile Ser Ser Ser Val Val Met Ser Ile Arg
            20                  25                  30
Gln Lys Ile Arg Leu Phe His Leu Ser Val Cys Ala Gln Thr His Asp
        35                  40                  45
His Leu Ile Glu Ile His Leu Val Cys Ile Glu Phe Gly Ala Ile Asp
    50                  55                  60
Thr Asp Glu Phe Arg Leu Ser Ser His Ala Tyr Thr Thr Ser Pro Thr
65                  70                  75                  80
His Thr Gly Ala Ile His His Asn Cys Ile Glu Arg Ser Tyr Gly Arg
                85                  90                  95
Tyr Leu Val Thr Phe Gly Gln Glu Arg Asn Glu Leu His His His Ser
            100                 105                 110
Arg Pro Asp Arg Asn Ala Glu Val Tyr Arg Phe Pro Phe Asp Asn Ala
        115                 120                 125
Phe His Ser Ile Arg Tyr Glu Ala Phe Arg Pro Ile Arg Pro Ile Val
    130                 135                 140
Cys His Asp Asp His Phe Ile Ala Ile Gly Ser His Leu Phe Phe Lys
145                 150                 155                 160
Asp Asn Gln Ile Phe Ser Ser Gly Ser Gln Tyr Asp Asn Tyr Thr Val
                165                 170                 175
Ala Cys Phe Val Glu Ser Leu His Asp Arg Glu Gln Arg Ser His Thr
            180                 185                 190
His Thr Ala Ser Gly Thr Asn His Cys Ala Asp Leu Phe Asp Met Arg
        195                 200                 205
Thr Leu Ser Gln Arg Thr Tyr His Ile Arg Asp Ile Val Ala Asp Phe
    210                 215                 220
Glu Phe Gly Gln Phe Leu Gly Arg Phe Ala His Gly Leu Asn His
225                 230                 235

(2) INFORMATION FOR SEQ ID NO:509

(i) SEQUENCE CHARACTERISTICS:
(A) LENGTH: 211 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...211

(xi) SEQUENCE DESCRIPTION: SEQ ID NO:509

Met Ser Ile Arg Gln Lys Ile Arg Leu Phe His Leu Ser Val Cys Ala
1               5                   10                  15
Gln Thr His Asp His Leu Ile Glu Ile His Leu Val Cys Ile Glu Phe
            20                  25                  30
Gly Ala Ile Asp Thr Asp Glu Phe Arg Leu Ser Ser His Ala Tyr Thr
        35                  40                  45
Thr Ser Pro Thr His Thr Gly Ala Ile His His Asn Cys Ile Glu Arg
    50                  55                  60
Ser Tyr Gly Arg Tyr Leu Val Thr Phe Gly Gln Glu Arg Asn Glu Leu
65                  70                  75                  80
His His His Ser Arg Pro Asp Arg Asn Ala Glu Val Tyr Arg Phe Pro
                85                  90                  95
Phe Asp Asn Ala Phe His Ser Ile Arg Tyr Glu Ala Phe Arg Pro Ile
            100                 105                 110
Arg Pro Ile Val Cys His Asp Asp His Phe Ile Ala Ile Gly Ser His
        115                 120                 125
Leu Phe Phe Lys Asp Asn Gln Ile Phe Ser Ser Gly Ser Gln Tyr Asp
    130                 135                 140

Asn Tyr Thr Val Ala Cys Phe Val Glu Ser Leu His Asp Arg Glu Gln
145                 150                 155                 160
Arg Ser His Thr His Thr Ala Ser Gly Thr Asn His Cys Ala Asp Leu
                165                 170                 175
Phe Asp Met Arg Thr Leu Ser Gln Arg Thr Tyr His Ile Arg Asp Ile
            180                 185                 190
Val Ala Asp Phe Glu Phe Gly Gln Phe Leu Gly Arg Phe Ala His Gly
        195                 200                 205
Leu Asn His
    210

(2) INFORMATION FOR SEQ ID NO:510

(i) SEQUENCE CHARACTERISTICS:
(A) LENGTH: 781 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...781

(xi) SEQUENCE DESCRIPTION: SEQ ID NO:510

Met Asp Arg Pro Lys Pro Ser Tyr Ile Val Arg Ile Ala Ala Ile Leu
1               5                   10                  15
Cys Leu Phe Val Gly Arg Pro Leu Phe Ala Gln Ser Tyr Val Asp Tyr
            20                  25                  30
Val Asp Pro Leu Ile Gly Thr Leu Ser Ser Phe Glu Leu Ser Ala Gly
        35                  40                  45
Asn Thr Tyr Pro Val Ile Gly Leu Pro Trp Gly Met Asn Ser Trp Thr
    50                  55                  60
Pro Met Thr Gly Val Pro Gly Asp Gly Trp Gln Tyr Thr Tyr Ser Ala
65                  70                  75                  80
His Lys Ile Arg Gly Phe Lys Gln Thr His Gln Pro Ser Pro Trp Ile
                85                  90                  95
Asn Asp Tyr Gly Gln Phe Ser Leu Leu Pro Leu Thr Ala Pro Gln Lys
            100                 105                 110
Pro Ser Ser Asn Asp Ser Ile Ala Leu Thr Lys Trp Cys Lys Gln Leu
        115                 120                 125
Phe Ser Asp Glu Gln Thr Ser Trp Phe Ser His Lys Ala Glu Thr Ala
    130                 135                 140
Thr Pro Tyr Tyr Tyr Ser Val Tyr Leu Ala Asp Tyr Asp Thr Arg Val
145                 150                 155                 160
Glu Met Ala Pro Thr Glu Arg Ala Ala Ile Phe Arg Ile Arg Tyr Ser
                165                 170                 175
Gly Asn Thr Glu Ser Gly Ser Gly Arg Trp Leu Arg Leu Asp Ala Phe
            180                 185                 190
Thr Gly Gly Ser Glu Ile Ser Ile Val Asp Pro His Thr Val Val Gly
        195                 200                 205
Ile Ser Arg Lys Asn Ser Gly Gly Val Pro Ala Asn Phe Ala Cys Tyr
    210                 215                 220
Phe Ile Leu Gln Ser Asp Thr Pro Met Ala Asp Val Leu Leu Glu Thr
225                 230                 235                 240
Asp Thr Gly Lys Ser Asp Glu Gly Thr Arg Ala Trp Ala Ala Cys Arg
                245                 250                 255
Phe Asp Ser Gln Glu Val Thr Val Arg Val Ala Ser Ser Phe Ile Ser
            260                 265                 270
Val Glu Gln Ala Glu Arg Asn Leu Ala Glu Val Lys Gly Gln Ser Phe
        275                 280                 285
Asp Arg Ile Arg Leu Ala Gly Arg Glu Ala Trp Asn Lys Val Leu Gly
    290                 295                 300
Arg Ile His Val Glu Gly Gly Thr Lys Asp Glu Arg Thr Thr Phe Tyr
305                 310                 315                 320
Ser Ala Leu Tyr Arg Cys Leu Leu Phe Pro Arg Arg Phe Tyr Glu Glu
                325                 330                 335
Asp Ala Ser Gly Asn Phe Val His Tyr Ser Pro Tyr Asn Gly Glu Val
            340                 345                 350

Leu Pro Gly Tyr Leu Tyr Thr Asp Thr Gly Phe Trp Asp Thr Phe Arg
        355                 360                 365
Ala Leu Phe Pro Leu Leu Asn Leu Leu Tyr Pro Asp Glu Asn Ile Lys
    370                 375                 380
Ile Gln Glu Gly Leu Leu Asn Val Tyr Arg Glu Ser Gly Phe Phe Pro
385                 390                 395                 400
Glu Trp Ala Ser Pro Gly His Arg Asp Cys Met Ile Gly Asn Asn Ser
                405                 410                 415
Ala Ser Val Leu Ala Asp Ala Tyr Leu Lys Gly Val Arg Val Glu Asp
            420                 425                 430
Thr Arg Thr Leu Met Asn Gly Leu Leu His Ala Thr Lys Ala Val His
        435                 440                 445
Pro Lys Ile Ser Ser Thr Gly Arg Lys Gly Trp Glu Trp Tyr Asn Ser
    450                 455                 460
Leu Gly Tyr Val Pro Ala Asp Ala Gly Ile Asp Glu Ser Ala Ala Arg
465                 470                 475                 480
Thr Leu Glu Tyr Ala Tyr Asn Asp Trp Cys Ile Leu Arg Leu Gly Arg
                485                 490                 495
Thr Leu Gly Trp Asp Arg Ala Ala Leu Asp Thr Leu Ala His Arg Ser
            500                 505                 510
Met Asn Tyr Arg His Leu Phe Asp Pro Glu Thr Lys Leu Met Arg Gly
        515                 520                 525
Arg Asn Gln Asp Gly Ser Phe Arg Thr Pro Phe Ser Pro Phe Lys Trp
    530                 535                 540
Gly Asp Val Phe Thr Glu Gly Asn Ala Trp His Tyr Thr Trp Ser Val
545                 550                 555                 560
Phe His Asp Val Gln Gly Leu Ile Asp Leu Met Gly Gly Asp Arg Pro
                565                 570                 575
Phe Val Ser Met Leu Asp Ser Val Phe Asn Thr Pro Pro Met Phe Asp
            580                 585                 590
Glu Ser Tyr Tyr Gly Phe Val Ile His Glu Ile Arg Glu Met Gln Ile
        595                 600                 605
Ala Asp Met Gly Asn Tyr Ala His Gly Asn Gln Pro Ile Gln His Met
    610                 615                 620
Ile Tyr Leu Tyr Asn His Ala Gly His Pro Trp Lys Ala Gln Glu Arg
625                 630                 635                 640
Leu Arg Glu Val Met Gly Arg Leu Tyr Arg Pro Thr Pro Asp Gly Tyr
                645                 650                 655
Cys Gly Asp Glu Asp Asn Gly Gln Thr Ser Ala Trp Tyr Val Phe Ser
            660                 665                 670
Ala Leu Gly Phe Tyr Pro Val Thr Pro Ala Thr Asp Gln Tyr Val Leu
        675                 680                 685
Gly Ser Pro Ile Phe Ser Lys Val Ile Leu Ser Phe Pro Asp Gly His
    690                 695                 700
Lys Thr Val Leu His Ala Pro Ala Asn Ser Ala Asp Thr Pro Tyr Ile
705                 710                 715                 720
Arg Ser Ile Ser Val Glu Gly Lys Glu Trp Ser Cys Asn Tyr Leu Thr
                725                 730                 735
His Glu Gln Leu Arg Ser Ser Ala Ser Ile Gln Trp Met Met Asp Thr
            740                 745                 750
Lys Pro Asn Tyr Asn Arg Gly Met Lys Glu Ser Asp Arg Pro Tyr Ser
        755                 760                 765
Phe Ser Thr Glu Gln Gln Arg Arg Ala Asn His Ser Asn
    770                 775                 780

(2) INFORMATION FOR SEQ ID NO:511

    (i) SEQUENCE CHARACTERISTICS:
        (A) LENGTH: 271 amino acids
        (B) TYPE: amino acid
        (D) TOPOLOGY: linear

   (ii) MOLECULE TYPE: protein

  (iii) HYPOTHETICAL: YES

   (vi) ORIGINAL SOURCE:
        (A) ORGANISM: Porphyromonas gingivalis

   (ix) FEATURE:
        (A) NAME/KEY: misc_feature
        (B) LOCATION 1...271

   (xi) SEQUENCE DESCRIPTION: SEQ ID NO:511

Met Met Lys Ser Met Arg Ser Val Leu Leu Leu Leu Phe Pro Leu Ser
1               5                   10                  15
Leu Ile Thr Ala Leu Gly Cys Ser Asn Asn Lys Ala Ala Glu Ser Lys
            20                  25                  30
Ser Val Ser Phe Asp Ser Ala Tyr Leu Glu Arg Tyr Ile Pro Leu Arg
        35                  40                  45
Ala Asp Ile Asp Thr Pro Ser Leu His Val Met Ile Ser Tyr Val Tyr
    50                  55                  60
Pro Ser Gly Asp Asp Met Leu Thr Glu Ile Phe Asn Gly Leu Leu Phe
65                  70                  75                  80
Gly Asp Ser Leu Met Asp Ser Ser Ser Pro Glu Asn Ala Met Glu Gly
                85                  90                  95
Tyr Ala Gln Met Leu Gly Glu Asp Tyr Arg Ser Asn Asn Ala Glu Ala
            100                 105                 110
Asn Leu Gln Gly Leu Pro Ser Asp Leu Leu Asp Tyr Ile Tyr Lys Gln
        115                 120                 125
Glu Asn Thr Ile Ala Tyr Cys Asp Thr Gly Leu Ile Ser Thr Arg Ile
    130                 135                 140
Asn Thr Tyr Thr Tyr Glu Gly Gly Ala His Thr Glu Asn Thr Val Arg
145                 150                 155                 160
Phe Ala Asn Ile Leu Arg Thr Thr Gly Lys Val Leu Glu Glu Arg Asp
                165                 170                 175
Ile Phe Lys Ile Asp Tyr Ala Glu Arg Leu Ser Ala Leu Ile Ile Gly
            180                 185                 190
Gln Leu Val His Asp Phe Gly Lys Thr Thr Pro Ala Glu Leu Asp Ala
        195                 200                 205
Ile Gly Phe Phe Asn Ala Glu Glu Ile Gln Pro Asn Gly Asn Phe Met
    210                 215                 220
Ile Asp Asp Lys Gly Leu Thr Tyr Cys Phe Asn Glu Tyr Gln Ile Ala
225                 230                 235                 240
Ala Tyr Ala Arg Gly Ala Val Tyr Val Arg Leu Gly Tyr Asp Val Leu
                245                 250                 255
Ala Pro Leu Leu Arg Asp Asp Ser Pro Leu Lys Arg Tyr Leu Pro
            260                 265                 270

(2) INFORMATION FOR SEQ ID NO:512

(i) SEQUENCE CHARACTERISTICS:
(A) LENGTH: 270 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...270

(xi) SEQUENCE DESCRIPTION: SEQ ID NO:512

Met Lys Ser Met Arg Ser Val Leu Leu Leu Leu Phe Pro Leu Ser Leu
1               5                   10                  15
Ile Thr Ala Leu Gly Cys Ser Asn Asn Lys Ala Ala Glu Ser Lys Ser
            20                  25                  30
Val Ser Phe Asp Ser Ala Tyr Leu Glu Arg Tyr Ile Pro Leu Arg Ala
        35                  40                  45
Asp Ile Asp Thr Pro Ser Leu His Val Met Ile Ser Tyr Val Tyr Pro
    50                  55                  60
Ser Gly Asp Asp Met Leu Thr Glu Ile Phe Asn Gly Leu Leu Phe Gly
65                  70                  75                  80
Asp Ser Leu Met Asp Ser Ser Ser Pro Glu Asn Ala Met Glu Gly Tyr
                85                  90                  95
Ala Gln Met Leu Gly Glu Asp Tyr Arg Ser Asn Asn Ala Glu Ala Asn
            100                 105                 110
Leu Gln Gly Leu Pro Ser Asp Leu Leu Asp Tyr Ile Tyr Lys Gln Glu
        115                 120                 125
Asn Thr Ile Ala Tyr Cys Asp Thr Gly Leu Ile Ser Thr Arg Ile Asn
    130                 135                 140
Thr Tyr Thr Tyr Glu Gly Gly Ala His Thr Glu Asn Thr Val Arg Phe
145                 150                 155                 160

Ala Asn Ile Leu Arg Thr Thr Gly Lys Val Leu Glu Glu Arg Asp Ile
                165                 170                 175
Phe Lys Ile Asp Tyr Ala Glu Arg Leu Ser Ala Leu Ile Ile Gly Gln
            180                 185                 190
Leu Val His Asp Phe Gly Lys Thr Thr Pro Ala Glu Leu Asp Ala Ile
        195                 200                 205
Gly Phe Phe Asn Ala Glu Glu Ile Gln Pro Asn Gly Asn Phe Met Ile
    210                 215                 220
Asp Asp Lys Gly Leu Thr Tyr Cys Phe Asn Glu Tyr Gln Ile Ala Ala
225                 230                 235                 240
Tyr Ala Arg Gly Ala Val Tyr Val Arg Leu Gly Tyr Asp Val Leu Ala
                245                 250                 255
Pro Leu Leu Arg Asp Asp Ser Pro Leu Lys Arg Tyr Leu Pro
            260                 265                 270

(2) INFORMATION FOR SEQ ID NO:513

     (i) SEQUENCE CHARACTERISTICS:
          (A) LENGTH: 267 amino acids
          (B) TYPE: amino acid
          (D) TOPOLOGY: linear

     (ii) MOLECULE TYPE: protein

    (iii) HYPOTHETICAL: YES

     (vi) ORIGINAL SOURCE:
          (A) ORGANISM: Porphyromonas gingivalis

     (ix) FEATURE:
          (A) NAME/KEY: misc_feature
          (B) LOCATION 1...267

     (xi) SEQUENCE DESCRIPTION: SEQ ID NO:513

Met Arg Ser Val Leu Leu Leu Leu Phe Pro Leu Ser Leu Ile Thr Ala
1               5                   10                  15
Leu Gly Cys Ser Asn Asn Lys Ala Ala Glu Ser Lys Ser Val Ser Phe
            20                  25                  30
Asp Ser Ala Tyr Leu Glu Arg Tyr Ile Pro Leu Arg Ala Asp Ile Asp
        35                  40                  45
Thr Pro Ser Leu His Val Met Ile Ser Tyr Val Tyr Pro Ser Gly Asp
    50                  55                  60
Asp Met Leu Thr Glu Ile Phe Asn Gly Leu Leu Phe Gly Asp Ser Leu
65                  70                  75                  80
Met Asp Ser Ser Ser Pro Glu Asn Ala Met Glu Gly Tyr Ala Gln Met
                85                  90                  95
Leu Gly Glu Asp Tyr Arg Ser Asn Asn Ala Glu Ala Asn Leu Gln Gly
            100                 105                 110
Leu Pro Ser Asp Leu Leu Asp Tyr Ile Tyr Lys Gln Glu Asn Thr Ile
        115                 120                 125
Ala Tyr Cys Asp Thr Gly Leu Ile Ser Thr Arg Ile Asn Thr Tyr Thr
    130                 135                 140
Tyr Glu Gly Gly Ala His Thr Glu Asn Thr Val Arg Phe Ala Asn Ile
145                 150                 155                 160
Leu Arg Thr Thr Gly Lys Val Leu Glu Glu Arg Asp Ile Phe Lys Ile
                165                 170                 175
Asp Tyr Ala Glu Arg Leu Ser Ala Leu Ile Ile Gly Gln Leu Val His
            180                 185                 190
Asp Phe Gly Lys Thr Thr Pro Ala Glu Leu Asp Ala Ile Gly Phe Phe
        195                 200                 205
Asn Ala Glu Glu Ile Gln Pro Asn Gly Asn Phe Met Ile Asp Asp Lys
    210                 215                 220
Gly Leu Thr Tyr Cys Phe Asn Glu Tyr Gln Ile Ala Ala Tyr Ala Arg
225                 230                 235                 240
Gly Ala Val Tyr Val Arg Leu Gly Tyr Asp Val Leu Ala Pro Leu Leu
                245                 250                 255
Arg Asp Asp Ser Pro Leu Lys Arg Tyr Leu Pro
            260                 265

(2) INFORMATION FOR SEQ ID NO:514

     (i) SEQUENCE CHARACTERISTICS:
          (A) LENGTH: 259 amino acids
          (B) TYPE: amino acid

(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...259

(xi) SEQUENCE DESCRIPTION: SEQ ID NO:514

Met Lys Lys Thr Thr Leu Thr Gly Ser Ile Cys Ala Leu Leu Leu Phe
1               5                   10                  15
Leu Gly Leu Ser Ala Asn Ala Gln Ser Lys Leu Lys Ile Lys Ser Ile
            20                  25                  30
Glu Ala Ala Thr Thr Phe Ser Ser Ala Thr Ala Gly Asn Gly Phe Gly
        35                  40                  45
Gly Asn Ile Phe Gly Met Asp Met Ser Ile Arg Met Arg Val His His
    50                  55                  60
Ser Ile Leu Pro Glu Gly Leu Asp Phe Ser Val Gly Ile His Glu Arg
65                  70                  75                  80
Arg Ala His Trp Glu Glu Ala Gly Ser Pro Lys Leu Met Tyr Thr Asn
                85                  90                  95
Val Pro Ser Ile Ile Gly Ile Val Glu Lys Val Ile Val Phe Glu Asp
            100                 105                 110
Ala Glu Asp Phe Phe Asp Lys Lys Ala Leu Gly Arg Phe Leu Ile Ser
        115                 120                 125
Leu Gly Ile Ser Tyr Thr Lys His Leu Gly Ala Tyr Trp Gly Trp Thr
    130                 135                 140
Asn Asp Ala His Ile Leu Phe Ser Pro Ile Pro Lys Ser Lys Val His
145                 150                 155                 160
Tyr Asp Thr Tyr Thr Arg Ala Gly Ser Asp Leu Val Leu Gln Ser Glu
                165                 170                 175
Asp Val Ala Thr Val Ser Asn Gly Phe Ser Pro Gly Ile Gly Leu Lys
            180                 185                 190
Ser Ser Ile Trp Trp Lys Met Pro Ile Lys Ser Lys Tyr Asp Phe Arg
        195                 200                 205
Leu Gly Phe Ser Leu Gly Tyr Glu Tyr Leu Asn Leu Leu Tyr Pro Tyr
    210                 215                 220
Arg Asn Phe Lys Leu Asp Gly Asn Lys Pro Leu Ser Ala Leu Ser Pro
225                 230                 235                 240
Arg Met Asn His Ile Gly His Val Gly Phe Asn Phe Thr Val Gly Leu
                245                 250                 255
Trp Thr Asn

(2) INFORMATION FOR SEQ ID NO:515

(i) SEQUENCE CHARACTERISTICS:
(A) LENGTH: 1266 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...1266

(xi) SEQUENCE DESCRIPTION: SEQ ID NO:515

Met Gly Lys Tyr Lys Arg Ala Lys Tyr Arg Tyr Trp Leu Phe Pro Phe
1               5                   10                  15
Cys Ser Asp Tyr Tyr Thr Phe Glu Gly Val Thr Phe Leu Cys Ala Ser
            20                  25                  30
Asp Asp Met Thr Thr Lys Lys Pro Gln Ala Ile Leu Asp Leu Glu Lys

35                  40                  45
Ala Tyr Asn Ile Glu Ile Pro Asp Leu Ser Ser Gln Glu Gly Ile Ser
    50                  55                  60
Trp Ser Val Asn Arg Tyr Phe Lys Gln Asp Ser Ser Gly Ala Val Val
65                  70                  75                  80
Glu Leu Cys Leu Arg Glu Cys Gln Ile Glu Ser Met Thr Trp Leu Ile
                85                  90                  95
Asp Phe Pro Ala Leu Lys Lys Leu Asp Leu Ser Tyr Asn Gln Ile Ser
            100                 105                 110
Lys Leu Glu Gly Leu Glu Arg Leu Thr Ser Leu Thr Lys Leu Arg Leu
        115                 120                 125
Arg Ser Asn Gln Ile Arg Lys Leu Glu Gly Leu Asp Ser Leu Thr Ser
    130                 135                 140
Leu Thr Lys Leu Ser Leu Ser Asp Asn Gln Ile Ser Lys Leu Glu Gly
145                 150                 155                 160
Leu Glu Arg Leu Thr Ser Leu Ala Glu Leu Tyr Leu Leu Asp Asn Gln
                165                 170                 175
Ile Ser Lys Leu Glu Gly Leu Glu Arg Leu Thr Ser Leu Ala Thr Leu
            180                 185                 190
Glu Leu Ser Gly Asn Gln Ile Arg Lys Leu Glu Gly Leu Glu Arg Leu
        195                 200                 205
Thr Ser Leu Ala Thr Leu Glu Leu Ser Gly Asn Gln Ile Arg Lys Leu
    210                 215                 220
Glu Gly Leu Glu Arg Leu Thr Ser Leu Thr Lys Leu Arg Leu Arg Ser
225                 230                 235                 240
Asn Gln Ile Ser Lys Leu Glu Gly Leu Glu Arg Leu Thr Ser Leu Ala
                245                 250                 255
Thr Leu Glu Leu Ser Gly Asn Gln Ile Arg Lys Leu Glu Gly Leu Glu
            260                 265                 270
Arg Leu Thr Ser Leu Ala Thr Leu Glu Leu Ser Gly Asn Gln Ile Ser
        275                 280                 285
Lys Leu Glu Gly Leu Glu Arg Leu Ser Ser Leu Thr Lys Leu Arg Leu
    290                 295                 300
Arg Ser Asn Gln Ile Ser Lys Leu Glu Gly Leu Glu Arg Leu Thr Ser
305                 310                 315                 320
Leu Thr Lys Leu Ser Leu Ser Asp Asn Gln Ile Ser Lys Leu Glu Gly
                325                 330                 335
Leu Glu Arg Leu Thr Ser Leu Ala Glu Leu Tyr Leu Leu Asp Asn Gln
            340                 345                 350
Ile Arg Lys Leu Glu Gly Leu Glu Arg Leu Thr Ser Leu Thr Lys Leu
        355                 360                 365
Arg Leu Arg Ser Asn Gln Ile Ser Lys Leu Glu Gly Leu Asp Ser Leu
    370                 375                 380
Thr Ser Leu Thr Lys Leu Ser Leu Ser Asp Asn Gln Ile Ser Lys Leu
305                 390                 395                 400
Glu Gly Leu Glu Arg Leu Thr Ser Leu Ala Glu Leu Tyr Leu Leu Asp
                405                 410                 415
Asn Gln Ile Arg Lys Leu Glu Gly Leu Asp Gly Leu Ala Ser Leu Thr
            420                 425                 430
Arg Leu Ser Leu Arg Arg Asn Gln Ile Ser Lys Leu Glu Gly Leu Asp
        435                 440                 445
Arg Leu Lys Val Leu Arg Lys Leu Asp Val Ser Gly Asn Asp Ile Gln
    450                 455                 460
Ser Ile Asp Asp Ile Lys Leu Leu Ala Pro Ile Leu Glu Gln Thr Leu
465                 470                 475                 480
Glu Lys Leu Arg Ile His Asp Asn Pro Phe Val Ala Ser Ser Gly Leu
                485                 490                 495
Ile Leu Ser Pro Tyr Asp Asn His Leu Pro Glu Ile Lys Ala Leu Leu
            500                 505                 510
Glu Lys Glu Lys Glu Lys Gln Lys Lys Thr Ser Val Glu Tyr His Pro
        515                 520                 525
Phe Cys Lys Val Met Leu Leu Gly Asn His Ser Ser Gly Lys Thr Thr
    530                 535                 540
Phe Leu Ser Gln Tyr Asp Thr Asn Tyr Thr Tyr Gln Lys Asn Thr His
545                 550                 555                 560
Val Leu Ser Ile His Arg Ser Asn Asn Pro Asn Ala Ile Phe Tyr Asp
                565                 570                 575
Phe Gly Gly Gln Asp Tyr Tyr His Gly Ile Tyr Gln Ala Phe Phe Thr
            580                 585                 590
Thr Gln Ser Leu Tyr Leu Leu Phe Trp Asp Ala Lys Lys Asp Arg Asn
        595                 600                 605
Phe Val Ser Val Asp Asp Lys Glu Tyr Gln Thr Leu Asn Phe Asn Arg
    610                 615                 620
Pro Tyr Trp Leu Gly Gln Ile Ala Tyr Ala Cys Asn Arg Cys Met Ser
625                 630                 635                 640

Val Gly Gly Asn Pro Asp Gly Lys Asp Thr Pro Gln Thr Thr Asp Asp
                645                 650                 655
Thr Ile Ile Ile Gln Thr His Ala Asp Glu Thr Gly Ala Lys Gln Gln
            660                 665                 670
Thr Leu Gly Cys Ala Ala Glu Asn Gly Val Leu Glu Glu Ile Tyr Val
        675                 680                 685
Ser Leu Glu Pro Lys Ala Asn Ser Ala Val His Ala Leu Asn Tyr Leu
    690                 695                 700
Asn Glu Arg Val Arg Glu Val Val Ala Ser Arg Ser Lys Ser Ile Gln
705                 710                 715                 720
Ile Thr Glu Lys Asp Lys Gly Leu Tyr Glu Ala Leu Pro Thr Ile Ala
                725                 730                 735
Gly Asp Asn Lys His Ile Pro Ile Ser Leu Glu Ala Leu Ala Ala Gln
            740                 745                 750
Leu Asn Lys Gly Arg Ala Glu Asn Asp Leu Tyr Thr Ile Glu Tyr Leu
        755                 760                 765
Gln Thr Glu Leu Asn Gln Leu Ser Leu Arg Gly Glu Val Leu Tyr Tyr
    770                 775                 780
Arg Glu Asn Glu Lys Leu Asn Asn Tyr Val Trp Leu Asp Pro Ala Ala
785                 790                 795                 800
Phe Val Gln Met Ile His Gly Glu Ile Leu Gln Lys Asp Asn Ile Asn
                805                 810                 815
Arg Gly Thr Val Pro Lys Asp Ile Phe Glu Cys Lys Leu His Asn Leu
            820                 825                 830
Ser Ser Gly Ser Ile Phe Glu Glu Asp Gly Gln Asn Gly Asn Met Ile
        835                 840                 845
Leu Gln Leu Leu Leu Glu Glu Leu Ile Val Tyr Glu Asp Lys Asp Cys
    850                 855                 860
Tyr Val Ile Pro Gly Tyr Leu Pro Leu His Ser Asp Asp Glu Ala Tyr
865                 870                 875                 880
Lys Trp Leu Thr Leu Gly Phe Glu Arg Pro Asn Phe Val Leu Lys Phe
                885                 890                 895
Glu Arg Phe Ile Pro Phe Gly Leu Ile Asn Gln Ile Ile Ala Tyr Tyr
            900                 905                 910
Gly Arg Glu Glu Gly Ala Leu Lys Arg Tyr Trp Arg Asp Gln Val Ile
        915                 920                 925
Phe Thr Ala Gly Arg Glu Met Asp Arg Gln Thr Leu Glu Gln Glu Glu
    930                 935                 940
Glu Lys Glu Gly Leu Pro Lys Thr Asn Ala Glu Asp Tyr Gln Ile Trp
945                 950                 955                 960
Ile Lys Leu Asp Phe Thr Asp Leu Ala Ile Ser Val Phe Ile Lys Glu
                965                 970                 975
Gln Arg Lys Thr Ser Ala Lys Asp Met Gln Arg Lys Glu Ala Thr Ile
            980                 985                 990
Leu Ser Asp Met Leu Asp Met Tyr Trp Asn Asn Ile Pro Pro Arg Glu
        995                 1000                1005
Gln Ile Gly Asp Lys Asp Thr Glu Gln Thr Arg Ser Thr Ile Arg Glu
    1010                1015                1020
Thr Asn Arg Lys Lys Arg Pro Ile Gln Asp Leu Tyr Leu Ser Cys Ala
1025                1030                1035                1040
Gln Ala Asp Lys Asp Leu Thr Glu Ser His Tyr Ile His Leu Gly Thr
                1045                1050                1055
Leu Asp Asp Glu Ser Lys Thr Thr Ala Arg Ile Ala Ala Tyr Pro Leu
            1060                1065                1070
Lys Asn Gly Val Ile Asp Lys Glu Arg Val Arg Glu Val Ser Thr Arg
        1075                1080                1085
Pro Tyr Lys His Leu Ser Val Asn Lys Asn Leu Ala Thr Ala Lys Gln
    1090                1095                1100
Ile Phe Ile Ser Tyr Ser Lys Glu Asp Gln Thr Glu Leu Glu Thr Cys
1105                1110                1115                1120
Leu Gln Phe Phe Lys Pro Leu Glu Lys Asn Gly Gln Ile Glu Ile Tyr
                1125                1130                1135
Tyr Asp Lys Leu Thr Lys Phe Glu Thr Pro Ile His Pro Glu Ile Arg
            1140                1145                1150
Lys Arg Ile Val Glu Ala Asp Cys Ile Ile Ala Leu Ile Ser Gln Arg
        1155                1160                1165
Tyr Leu Ala Thr Asp Tyr Ile Leu Asp His Glu Leu Pro Val Phe Arg
    1170                1175                1180
Glu Tyr Asn Lys Thr Ile Val Pro Ile Leu Ile Lys Pro Cys Thr Phe
1185                1190                1195                1200
Glu Asp Asp Glu Phe Leu Arg Glu Lys Tyr Phe Ala Gln Lys Ala Gln
                1205                1210                1215
Ile Ile Asn Leu Gly Lys Glu Gly Lys Thr Ile Lys Ala Tyr Asp Ser
            1220                1225                1230
Ile Thr Ala Ser Ala His Arg Asp Glu Asn Trp Val Ala Val Val Arg

1235                1240                1245
Glu Phe Lys Glu Lys Ile Leu Arg Ile Thr Lys Gln Glu Val Asn Thr
    1250                1255                1260
Asp Glu
1265

(2) INFORMATION FOR SEQ ID NO:516

     (i) SEQUENCE CHARACTERISTICS:
          (A) LENGTH: 1232 amino acids
          (B) TYPE: amino acid
          (D) TOPOLOGY: linear

     (ii) MOLECULE TYPE: protein

     (iii) HYPOTHETICAL: YES

     (vi) ORIGINAL SOURCE:
          (A) ORGANISM: Porphyromonas gingivalis

     (ix) FEATURE:
          (A) NAME/KEY: misc_feature
          (B) LOCATION 1...1232

     (xi) SEQUENCE DESCRIPTION: SEQ ID NO:516

Met Thr Thr Lys Lys Pro Gln Ala Ile Leu Asp Leu Glu Lys Ala Tyr
1               5                   10                  15
Asn Ile Glu Ile Pro Asp Leu Ser Ser Gln Glu Gly Ile Ser Trp Ser
            20                  25                  30
Val Asn Arg Tyr Phe Lys Gln Asp Ser Ser Gly Ala Val Val Glu Leu
        35                  40                  45
Cys Leu Arg Glu Cys Gln Ile Glu Ser Met Thr Trp Leu Ile Asp Phe
    50                  55                  60
Pro Ala Leu Lys Lys Leu Asp Leu Ser Tyr Asn Gln Ile Ser Lys Leu
65                  70                  75                  80
Glu Gly Leu Glu Arg Leu Thr Ser Leu Thr Lys Leu Arg Leu Arg Ser
                85                  90                  95
Asn Gln Ile Arg Lys Leu Glu Gly Leu Asp Ser Leu Thr Ser Leu Thr
            100                 105                 110
Lys Leu Ser Leu Ser Asp Asn Gln Ile Ser Lys Leu Glu Gly Leu Glu
        115                 120                 125
Arg Leu Thr Ser Leu Ala Glu Leu Tyr Leu Leu Asp Asn Gln Ile Ser
    130                 135                 140
Lys Leu Glu Gly Leu Glu Arg Leu Thr Ser Leu Ala Thr Leu Glu Leu
145                 150                 155                 160
Ser Gly Asn Gln Ile Arg Lys Leu Glu Gly Leu Glu Arg Leu Thr Ser
                165                 170                 175
Leu Ala Thr Leu Glu Leu Ser Gly Asn Gln Ile Arg Lys Leu Glu Gly
            180                 185                 190
Leu Glu Arg Leu Thr Ser Leu Thr Lys Leu Arg Leu Arg Ser Asn Gln
        195                 200                 205
Ile Ser Lys Leu Glu Gly Leu Glu Arg Leu Thr Ser Leu Ala Thr Leu
    210                 215                 220
Glu Leu Ser Gly Asn Gln Ile Arg Lys Leu Glu Gly Leu Glu Arg Leu
225                 230                 235                 240
Thr Ser Leu Ala Thr Leu Glu Leu Ser Gly Asn Gln Ile Ser Lys Leu
                245                 250                 255
Glu Gly Leu Glu Arg Leu Ser Ser Leu Thr Lys Leu Arg Leu Arg Ser
            260                 265                 270
Asn Gln Ile Ser Lys Leu Glu Gly Leu Glu Arg Leu Thr Ser Leu Thr
        275                 280                 285
Lys Leu Ser Leu Ser Asp Asn Gln Ile Ser Lys Leu Glu Gly Leu Glu
    290                 295                 300
Arg Leu Thr Ser Leu Ala Glu Leu Tyr Leu Leu Asp Asn Gln Ile Arg
305                 310                 315                 320
Lys Leu Glu Gly Leu Glu Arg Leu Thr Ser Leu Thr Lys Leu Arg Leu
                325                 330                 335
Arg Ser Asn Gln Ile Ser Lys Leu Glu Gly Leu Asp Ser Leu Thr Ser
            340                 345                 350
Leu Thr Lys Leu Ser Leu Ser Asp Asn Gln Ile Ser Lys Leu Glu Gly
        355                 360                 365
Leu Glu Arg Leu Thr Ser Leu Ala Glu Leu Tyr Leu Leu Asp Asn Gln
    370                 375                 380
Ile Arg Lys Leu Glu Gly Leu Asp Gly Leu Ala Ser Leu Thr Arg Leu

385                 390                 395                 400
Ser Leu Arg Arg Asn Gln Ile Ser Lys Leu Glu Gly Leu Asp Arg Leu
                405                 410                 415
Lys Val Leu Arg Lys Leu Asp Val Ser Gly Asn Asp Ile Gln Ser Ile
            420                 425                 430
Asp Asp Ile Lys Leu Leu Ala Pro Ile Leu Glu Gln Thr Leu Glu Lys
        435                 440                 445
Leu Arg Ile His Asp Asn Pro Phe Val Ala Ser Ser Gly Leu Ile Leu
    450                 455                 460
Ser Pro Tyr Asp Asn His Leu Pro Glu Ile Lys Ala Leu Leu Glu Lys
465                 470                 475                 480
Glu Lys Glu Lys Gln Lys Lys Thr Ser Val Glu Tyr His Pro Phe Cys
                485                 490                 495
Lys Val Met Leu Leu Gly Asn His Ser Ser Gly Lys Thr Thr Phe Leu
            500                 505                 510
Ser Gln Tyr Asp Thr Asn Tyr Thr Tyr Gln Lys Asn Thr His Val Leu
        515                 520                 525
Ser Ile His Arg Ser Asn Asn Pro Asn Ala Ile Phe Tyr Asp Phe Gly
    530                 535                 540
Gly Gln Asp Tyr Tyr His Gly Ile Tyr Gln Ala Phe Phe Thr Thr Gln
545                 550                 555                 560
Ser Leu Tyr Leu Leu Phe Trp Asp Ala Lys Lys Asp Arg Asn Phe Val
                565                 570                 575
Ser Val Asp Asp Lys Glu Tyr Gln Thr Leu Asn Phe Asn Arg Pro Tyr
            580                 585                 590
Trp Leu Gly Gln Ile Ala Tyr Ala Cys Asn Arg Cys Met Ser Val Gly
        595                 600                 605
Gly Asn Pro Asp Gly Lys Asp Thr Pro Gln Thr Thr Asp Asp Thr Ile
    610                 615                 620
Ile Ile Gln Thr His Ala Asp Glu Thr Gly Ala Lys Gln Gln Thr Leu
625                 630                 635                 640
Gly Cys Ala Ala Glu Asn Gly Val Leu Glu Glu Ile Tyr Val Ser Leu
                645                 650                 655
Glu Pro Lys Ala Asn Ser Ala Val His Ala Leu Asn Tyr Leu Asn Glu
            660                 665                 670
Arg Val Arg Glu Val Val Ala Ser Arg Ser Lys Ser Ile Gln Ile Thr
        675                 680                 685
Glu Lys Asp Lys Gly Leu Tyr Glu Ala Leu Pro Thr Ile Ala Gly Asp
    690                 695                 700
Asn Lys His Ile Pro Ile Ser Leu Glu Ala Leu Ala Ala Gln Leu Asn
705                 710                 715                 720
Lys Gly Arg Ala Glu Asn Asp Leu Tyr Thr Ile Glu Tyr Leu Gln Thr
                725                 730                 735
Glu Leu Asn Gln Leu Ser Leu Arg Gly Glu Val Leu Tyr Tyr Arg Glu
            740                 745                 750
Asn Glu Lys Leu Asn Asn Tyr Val Trp Leu Asp Pro Ala Ala Phe Val
        755                 760                 765
Gln Met Ile His Gly Glu Ile Leu Gln Lys Asp Asn Ile Asn Arg Gly
    770                 775                 780
Thr Val Pro Lys Asp Ile Phe Glu Cys Lys Leu His Asn Leu Ser Ser
785                 790                 795                 800
Gly Ser Ile Phe Glu Glu Asp Gly Gln Asn Gly Asn Met Ile Leu Gln
                805                 810                 815
Leu Leu Leu Glu Glu Leu Ile Val Tyr Glu Asp Lys Asp Cys Tyr Val
            820                 825                 830
Ile Pro Gly Tyr Leu Pro Leu His Ser Asp Asp Glu Ala Tyr Lys Trp
        835                 840                 845
Leu Thr Leu Gly Phe Glu Arg Pro Asn Phe Val Leu Lys Phe Glu Arg
    850                 855                 860
Phe Ile Pro Phe Gly Leu Ile Asn Gln Ile Ile Ala Tyr Tyr Gly Arg
865                 870                 875                 880
Glu Glu Gly Ala Leu Lys Arg Tyr Trp Arg Asp Gln Val Ile Phe Thr
                885                 890                 895
Ala Gly Arg Glu Met Asp Arg Gln Thr Leu Glu Gln Glu Glu Glu Lys
            900                 905                 910
Glu Gly Leu Pro Lys Thr Asn Ala Glu Asp Tyr Gln Ile Trp Ile Lys
        915                 920                 925
Leu Asp Phe Thr Asp Leu Ala Ile Ser Val Phe Ile Lys Glu Gln Arg
    930                 935                 940
Lys Thr Ser Ala Lys Asp Met Gln Arg Lys Glu Ala Thr Ile Leu Ser
945                 950                 955                 960
Asp Met Leu Asp Met Tyr Trp Asn Asn Ile Pro Pro Arg Glu Gln Ile
                965                 970                 975
Gly Asp Lys Asp Thr Glu Gln Thr Arg Ser Thr Ile Arg Glu Thr Asn
            980                 985                 990

Arg Lys Lys Arg Pro Ile Gln Asp Leu Tyr Leu Ser Cys Ala Gln Ala
        995                 1000                1005
Asp Lys Asp Leu Thr Glu Ser His Tyr Ile His Leu Gly Thr Leu Asp
    1010                1015                1020
Asp Glu Ser Lys Thr Thr Ala Arg Ile Ala Ala Tyr Pro Leu Lys Asn
1025                1030                1035                1040
Gly Val Ile Asp Lys Glu Arg Val Arg Glu Val Ser Thr Arg Pro Tyr
                1045                1050                1055
Lys His Leu Ser Val Asn Lys Asn Leu Ala Thr Ala Lys Gln Ile Phe
            1060                1065                1070
Ile Ser Tyr Ser Lys Glu Asp Gln Thr Glu Leu Glu Thr Cys Leu Gln
        1075                1080                1085
Phe Phe Lys Pro Leu Glu Lys Asn Gly Gln Ile Glu Ile Tyr Tyr Asp
    1090                1095                1100
Lys Leu Thr Lys Phe Glu Thr Pro Ile His Pro Glu Ile Arg Lys Arg
1105                1110                1115                1120
Ile Val Glu Ala Asp Cys Ile Ile Ala Leu Ile Ser Gln Arg Tyr Leu
                1125                1130                1135
Ala Thr Asp Tyr Ile Leu Asp His Glu Leu Pro Val Phe Arg Glu Tyr
            1140                1145                1150
Asn Lys Thr Ile Val Pro Ile Leu Ile Lys Pro Cys Thr Phe Glu Asp
        1155                1160                1165
Asp Glu Phe Leu Arg Glu Lys Tyr Phe Ala Gln Lys Ala Gln Ile Ile
    1170                1175                1180
Asn Leu Gly Lys Glu Gly Lys Thr Ile Lys Ala Tyr Asp Ser Ile Thr
1185                1190                1195                1200
Ala Ser Ala His Arg Asp Glu Asn Trp Val Ala Val Val Arg Glu Phe
                1205                1210                1215
Lys Glu Lys Ile Leu Arg Ile Thr Lys Gln Glu Val Asn Thr Asp Glu
            1220                1225                1230

(2) INFORMATION FOR SEQ ID NO:517

      (i) SEQUENCE CHARACTERISTICS:
           (A) LENGTH: 1175 amino acids
           (B) TYPE: amino acid
           (D) TOPOLOGY: linear

     (ii) MOLECULE TYPE: protein

    (iii) HYPOTHETICAL: YES

     (vi) ORIGINAL SOURCE:
           (A) ORGANISM: Porphyromonas gingivalis

     (ix) FEATURE:
           (A) NAME/KEY: misc_feature
           (B) LOCATION 1...1175

     (xi) SEQUENCE DESCRIPTION: SEQ ID NO:517

Met Thr Trp Leu Ile Asp Phe Pro Ala Leu Lys Lys Leu Asp Leu Ser
1               5                   10                  15
Tyr Asn Gln Ile Ser Lys Leu Glu Gly Leu Glu Arg Leu Thr Ser Leu
            20                  25                  30
Thr Lys Leu Arg Leu Arg Ser Asn Gln Ile Arg Lys Leu Glu Gly Leu
        35                  40                  45
Asp Ser Leu Thr Ser Leu Thr Lys Leu Ser Leu Ser Asp Asn Gln Ile
    50                  55                  60
Ser Lys Leu Glu Gly Leu Glu Arg Leu Thr Ser Leu Ala Glu Leu Tyr
65                  70                  75                  80
Leu Leu Asp Asn Gln Ile Ser Lys Leu Glu Gly Leu Glu Arg Leu Thr
                85                  90                  95
Ser Leu Ala Thr Leu Glu Leu Ser Gly Asn Gln Ile Arg Lys Leu Glu
            100                 105                 110
Gly Leu Glu Arg Leu Thr Ser Leu Ala Thr Leu Glu Leu Ser Gly Asn
        115                 120                 125
Gln Ile Arg Lys Leu Glu Gly Leu Glu Arg Leu Thr Ser Leu Thr Lys
    130                 135                 140
Leu Arg Leu Arg Ser Asn Gln Ile Ser Lys Leu Glu Gly Leu Glu Arg
145                 150                 155                 160
Leu Thr Ser Leu Ala Thr Leu Glu Leu Ser Gly Asn Gln Ile Arg Lys
                165                 170                 175
Leu Glu Gly Leu Glu Arg Leu Thr Ser Leu Ala Thr Leu Glu Leu Ser
            180                 185                 190

Gly Asn Gln Ile Ser Lys Leu Glu Gly Leu Glu Arg Leu Ser Ser Leu
        195                 200                 205
Thr Lys Leu Arg Leu Arg Ser Asn Gln Ile Ser Lys Leu Glu Gly Leu
    210                 215                 220
Glu Arg Leu Thr Ser Leu Thr Lys Leu Ser Leu Ser Asp Asn Gln Ile
225                 230                 235                 240
Ser Lys Leu Glu Gly Leu Glu Arg Leu Thr Ser Leu Ala Glu Leu Tyr
                245                 250                 255
Leu Leu Asp Asn Gln Ile Arg Lys Leu Glu Gly Leu Glu Arg Leu Thr
            260                 265                 270
Ser Leu Thr Lys Leu Arg Leu Arg Ser Asn Gln Ile Ser Lys Leu Glu
        275                 280                 285
Gly Leu Asp Ser Leu Thr Ser Leu Thr Lys Leu Ser Leu Ser Asp Asn
    290                 295                 300
Gln Ile Ser Lys Leu Glu Gly Leu Glu Arg Leu Thr Ser Leu Ala Glu
305                 310                 315                 320
Leu Tyr Leu Leu Asp Asn Gln Ile Arg Lys Leu Glu Gly Leu Asp Gly
                325                 330                 335
Leu Ala Ser Leu Thr Arg Leu Ser Leu Arg Arg Asn Gln Ile Ser Lys
            340                 345                 350
Leu Glu Gly Leu Asp Arg Leu Lys Val Leu Arg Lys Leu Asp Val Ser
        355                 360                 365
Gly Asn Asp Ile Gln Ser Ile Asp Asp Ile Lys Leu Leu Ala Pro Ile
    370                 375                 380
Leu Glu Gln Thr Leu Glu Lys Leu Arg Ile His Asp Asn Pro Phe Val
385                 390                 395                 400
Ala Ser Ser Gly Leu Ile Leu Ser Pro Tyr Asp Asn His Leu Pro Glu
                405                 410                 415
Ile Lys Ala Leu Leu Glu Lys Glu Lys Glu Lys Gln Lys Lys Thr Ser
            420                 425                 430
Val Glu Tyr His Pro Phe Cys Lys Val Met Leu Leu Gly Asn His Ser
        435                 440                 445
Ser Gly Lys Thr Thr Phe Leu Ser Gln Tyr Asp Thr Asn Tyr Thr Tyr
    450                 455                 460
Gln Lys Asn Thr His Val Leu Ser Ile His Arg Ser Asn Asn Pro Asn
465                 470                 475                 480
Ala Ile Phe Tyr Asp Phe Gly Gly Gln Asp Tyr Tyr His Gly Ile Tyr
                485                 490                 495
Gln Ala Phe Phe Thr Thr Gln Ser Leu Tyr Leu Leu Phe Trp Asp Ala
            500                 505                 510
Lys Lys Asp Arg Asn Phe Val Ser Val Asp Asp Lys Glu Tyr Gln Thr
        515                 520                 525
Leu Asn Phe Asn Arg Pro Tyr Trp Leu Gly Gln Ile Ala Tyr Ala Cys
    530                 535                 540
Asn Arg Cys Met Ser Val Gly Gly Asn Pro Asp Gly Lys Asp Thr Pro
545                 550                 555                 560
Gln Thr Thr Asp Asp Thr Ile Ile Ile Gln Thr His Ala Asp Glu Thr
                565                 570                 575
Gly Ala Lys Gln Gln Thr Leu Gly Cys Ala Ala Glu Asn Gly Val Leu
            580                 585                 590
Glu Glu Ile Tyr Val Ser Leu Glu Pro Lys Ala Asn Ser Ala Val His
        595                 600                 605
Ala Leu Asn Tyr Leu Asn Glu Arg Val Arg Glu Val Val Ala Ser Arg
    610                 615                 620
Ser Lys Ser Ile Gln Ile Thr Glu Lys Asp Lys Gly Leu Tyr Glu Ala
625                 630                 635                 640
Leu Pro Thr Ile Ala Gly Asp Asn Lys His Ile Pro Ile Ser Leu Glu
                645                 650                 655
Ala Leu Ala Ala Gln Leu Asn Lys Gly Arg Ala Glu Asn Asp Leu Tyr
            660                 665                 670
Thr Ile Glu Tyr Leu Gln Thr Glu Leu Asn Gln Leu Ser Leu Arg Gly
        675                 680                 685
Glu Val Leu Tyr Tyr Arg Glu Asn Glu Lys Leu Asn Asn Tyr Val Trp
    690                 695                 700
Leu Asp Pro Ala Ala Phe Val Gln Met Ile His Gly Glu Ile Leu Gln
705                 710                 715                 720
Lys Asp Asn Ile Asn Arg Gly Thr Val Pro Lys Asp Ile Phe Glu Cys
                725                 730                 735
Lys Leu His Asn Leu Ser Ser Gly Ser Ile Phe Glu Glu Asp Gly Gln
            740                 745                 750
Asn Gly Asn Met Ile Leu Gln Leu Leu Leu Glu Glu Leu Ile Val Tyr
        755                 760                 765
Glu Asp Lys Asp Cys Tyr Val Ile Pro Gly Tyr Leu Pro Leu His Ser
    770                 775                 780
Asp Asp Glu Ala Tyr Lys Trp Leu Thr Leu Gly Phe Glu Arg Pro Asn

785                 790                 795                 800
Phe Val Leu Lys Phe Glu Arg Phe Ile Pro Phe Gly Leu Ile Asn Gln
                805                 810                 815
Ile Ile Ala Tyr Tyr Gly Arg Glu Glu Gly Ala Leu Lys Arg Tyr Trp
            820                 825                 830
Arg Asp Gln Val Ile Phe Thr Ala Gly Arg Glu Met Asp Arg Gln Thr
        835                 840                 845
Leu Glu Gln Glu Glu Glu Lys Glu Gly Leu Pro Lys Thr Asn Ala Glu
    850                 855                 860
Asp Tyr Gln Ile Trp Ile Lys Leu Asp Phe Thr Asp Leu Ala Ile Ser
865                 870                 875                 880
Val Phe Ile Lys Glu Gln Arg Lys Thr Ser Ala Lys Asp Met Gln Arg
                885                 890                 895
Lys Glu Ala Thr Ile Leu Ser Asp Met Leu Asp Met Tyr Trp Asn Asn
            900                 905                 910
Ile Pro Pro Arg Glu Gln Ile Gly Asp Lys Asp Thr Glu Gln Thr Arg
        915                 920                 925
Ser Thr Ile Arg Glu Thr Asn Arg Lys Lys Arg Pro Ile Gln Asp Leu
    930                 935                 940
Tyr Leu Ser Cys Ala Gln Ala Asp Lys Asp Leu Thr Glu Ser His Tyr
945                 950                 955                 960
Ile His Leu Gly Thr Leu Asp Asp Glu Ser Lys Thr Thr Ala Arg Ile
                965                 970                 975
Ala Ala Tyr Pro Leu Lys Asn Gly Val Ile Asp Lys Glu Arg Val Arg
            980                 985                 990
Glu Val Ser Thr Arg Pro Tyr Lys His Leu Ser Val Asn Lys Asn Leu
        995                 1000                1005
Ala Thr Ala Lys Gln Ile Phe Ile Ser Tyr Ser Lys Glu Asp Gln Thr
    1010                1015                1020
Glu Leu Glu Thr Cys Leu Gln Phe Phe Lys Pro Leu Glu Lys Asn Gly
1025                1030                1035                1040
Gln Ile Glu Ile Tyr Tyr Asp Lys Leu Thr Lys Phe Glu Thr Pro Ile
                1045                1050                1055
His Pro Glu Ile Arg Lys Arg Ile Val Glu Ala Asp Cys Ile Ile Ala
            1060                1065                1070
Leu Ile Ser Gln Arg Tyr Leu Ala Thr Asp Tyr Ile Leu Asp His Glu
        1075                1080                1085
Leu Pro Val Phe Arg Glu Tyr Asn Lys Thr Ile Val Pro Ile Leu Ile
    1090                1095                1100
Lys Pro Cys Thr Phe Glu Asp Asp Glu Phe Leu Arg Glu Lys Tyr Phe
1105                1110                1115                1120
Ala Gln Lys Ala Gln Ile Ile Asn Leu Gly Lys Glu Gly Lys Thr Ile
                1125                1130                1135
Lys Ala Tyr Asp Ser Ile Thr Ala Ser Ala His Arg Asp Glu Asn Trp
            1140                1145                1150
Val Ala Val Val Arg Glu Phe Lys Glu Lys Ile Leu Arg Ile Thr Lys
        1155                1160                1165
Gln Glu Val Asn Thr Asp Glu
    1170                1175

(2) INFORMATION FOR SEQ ID NO:518

     (i) SEQUENCE CHARACTERISTICS:
          (A) LENGTH: 229 amino acids
          (B) TYPE: amino acid
          (D) TOPOLOGY: linear

    (ii) MOLECULE TYPE: protein

   (iii) HYPOTHETICAL: YES

    (vi) ORIGINAL SOURCE:
          (A) ORGANISM: Porphyromonas gingivalis

    (ix) FEATURE:
          (A) NAME/KEY: misc_feature
          (B) LOCATTON 1...229

    (xi) SEQUENCE DESCRIPTION: SEQ ID NO:518

Met Met Lys Lys Ala Phe Val Phe Val Leu Leu Val Cys Leu Phe Ser
1               5                   10                  15
Ser Phe Ser Ser Ser Ala Gln Thr Thr Thr Asn Ser Ser Arg Ser Tyr
            20                  25                  30
Phe Thr Gly Arg Ile Glu Lys Val Ser Leu Asn Leu Gly Val Pro Pro

        35                  40                  45
Val Ser Thr Glu Val Trp Gly Met Thr His Asp Ala Asn Gly Leu Pro
    50                  55                  60
Phe Glu Ile Pro Ile Ser Phe Ser Arg Phe Asn Ser Gln Gly Asp Ile
65                  70                  75                  80
Ala Thr Thr Tyr Tyr Ile Ala Asn Ser Glu Ala Thr Leu Asn Glu Trp
                85                  90                  95
Cys Asp Tyr Ala His Pro Gly Gly Ile Val Arg Val Glu Gly Arg Phe
            100                 105                 110
Trp Lys Met Thr Tyr Asn Ile Pro Thr Tyr Asn Ala Val Cys Thr Arg
        115                 120                 125
Ile Thr Phe Glu Asn Gln Glu Ile Glu Gly Thr Ile Val Leu Ile Pro
    130                 135                 140
Lys Pro Lys Val Ser Leu Pro His Val Ser Glu Ser Val Pro Cys Ile
145                 150                 155                 160
Arg Thr Glu Ala Gly Arg Glu Phe Ile Leu Cys Glu Glu Asp Asp Thr
                165                 170                 175
Phe Val Ser His Asp Gly Asn Glu Val Thr Ile Gly Gly Lys Pro Phe
            180                 185                 190
Leu Leu Asn Thr Asn Val Lys Ile Val Gly Asp Val Ser Gln Lys Tyr
        195                 200                 205
Ala Val Gly Val Gly Glu Ile Arg Phe Leu Gln Ile Cys Ala Gln Thr
    210                 215                 220
Val Ser Gln Gln Lys
225

(2) INFORMATION FOR SEQ ID NO:519

    (i) SEQUENCE CHARACTERISTICS:
        (A) LENGTH: 228 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...228

    (xi) SEQUENCE DESCRIPTION: SEQ ID NO:519

Met Lys Lys Ala Phe Val Phe Val Leu Leu Val Cys Leu Phe Ser Ser
1               5                   10                  15
Phe Ser Ser Ser Ala Gln Thr Thr Thr Asn Ser Ser Arg Ser Tyr Phe
            20                  25                  30
Thr Gly Arg Ile Glu Lys Val Ser Leu Asn Leu Gly Val Pro Pro Val
        35                  40                  45
Ser Thr Glu Val Trp Gly Met Thr His Asp Ala Asn Gly Leu Pro Phe
    50                  55                  60
Glu Ile Pro Ile Ser Phe Ser Arg Phe Asn Ser Gln Gly Asp Ile Ala
65                  70                  75                  80
Thr Thr Tyr Tyr Ile Ala Asn Ser Glu Ala Thr Leu Asn Glu Trp Cys
                85                  90                  95
Asp Tyr Ala His Pro Gly Gly Ile Val Arg Val Glu Gly Arg Phe Trp
            100                 105                 110
Lys Met Thr Tyr Asn Ile Pro Thr Tyr Asn Ala Val Cys Thr Arg Ile
        115                 120                 125
Thr Phe Glu Asn Gln Glu Ile Glu Gly Thr Ile Val Leu Ile Pro Lys
    130                 135                 140
Pro Lys Val Ser Leu Pro His Val Ser Glu Ser Val Pro Cys Ile Arg
145                 150                 155                 160
Thr Glu Ala Gly Arg Glu Phe Ile Leu Cys Glu Glu Asp Asp Thr Phe
                165                 170                 175
Val Ser His Asp Gly Asn Glu Val Thr Ile Gly Gly Lys Pro Phe Leu
            180                 185                 190
Leu Asn Thr Asn Val Lys Ile Val Gly Asp Val Ser Gln Lys Tyr Ala
        195                 200                 205
Val Gly Val Gly Glu Ile Arg Phe Leu Gln Ile Cys Ala Gln Thr Val
    210                 215                 220
Ser Gln Gln Lys

225

(2) INFORMATION FOR SEQ ID NO:520

(i) SEQUENCE CHARACTERISTICS:
(A) LENGTH: 540 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...540

(xi) SEQUENCE DESCRIPTION: SEQ ID NO:520

Met Lys Thr Lys Val Leu Arg Lys Phe Val Val Ala Ala Phe Ala Val
1               5                   10                  15
Ala Thr Leu Cys Pro Leu Ala Gln Ala Gln Thr Met Gly Gly Asp Asp
            20                  25                  30
Val Lys Val Val Gln Tyr Asn Gln Glu Lys Leu Val Gln Thr Arg Met
        35                  40                  45
Ser Val Ala Asp Asn Gly Trp Ile Tyr Val Met Thr His Ser Gly Tyr
    50                  55                  60
Asp Thr Gly Asn Ser Asn Val Lys Ile Phe Arg Ser Lys Asp Gln Gly
65                  70                  75                  80
Ala Thr Tyr Gln Lys Leu Arg Asp Trp Asp Pro Ser Asp Asp Tyr Gln
                85                  90                  95
Phe Gln Asp Phe Asp Ile Val Val Thr Gly Lys Asn Glu Ser Asp Ile
            100                 105                 110
Lys Ile Trp Ser Val Glu Leu Met Asn Lys Pro Gly Gly Tyr Lys Ser
        115                 120                 125
Arg Val Ala Val Phe Ser Arg Asp Ala Asn Ala Gln Asn Ala Lys Leu
    130                 135                 140
Val Tyr Lys Glu Asp Phe Ser Asn Val Gln Leu Tyr Asp Val Asp Ile
145                 150                 155                 160
Ala Ser Asn Tyr Arg Ser Pro Ser Ser Leu Asn Asn Gly Gly Asn Pro
                165                 170                 175
Phe Ala Leu Ala Phe Ala Tyr Thr Gly Phe Asn Asn Thr His Lys Ile
            180                 185                 190
Ser Phe Val Asp Tyr Val Phe Ser Leu Asn Gly Gly Gln Asn Phe Asn
        195                 200                 205
Lys Asn Leu Leu Phe Ser Gln Asp Gly Glu Lys Lys Ile Asp Lys Val
    210                 215                 220
Asp Leu Ser Leu Gly Ser Thr Ser Glu Ser Met Gly His Asn Ala Trp
225                 230                 235                 240
Pro Leu Met Gly Val Val Phe Glu Met Asn Lys Gln Gly Gly Lys Ser
                245                 250                 255
Asp Ile Gly Phe Leu Ser Asn Phe Val Asp Asn Asp Pro Glu Phe Gln
            260                 265                 270
Trp Ser Gly Pro Ile Lys Val Ser Glu Ser Asp Met Ser Phe Ser Pro
        275                 280                 285
Lys Ile Gln Met Leu Leu Asp Glu Asp Asn Asn Thr Ile Asn Gly Glu
    290                 295                 300
Ser Cys His Asn Phe Met Ile Thr Tyr Ser Asp Tyr Asp Ser Glu Tyr
305                 310                 315                 320
Ser Asp Trp Asp Ile Arg Tyr Val Tyr Pro Lys Lys Ser Phe Lys Tyr
                325                 330                 335
Glu Lys Gly Lys Thr Pro Thr Met Asp Asp Leu Val Glu Ala Phe Leu
            340                 345                 350
Thr Ala Ser Tyr Gln Ser Glu Thr Asn Ser Gly Leu Gly Tyr Asp Lys
        355                 360                 365
Asn Ala Asn His Tyr Leu Ile Thr Tyr Ala Lys Lys Glu Glu Asn Gly
    370                 375                 380
Thr Asn Thr Leu Lys Tyr Arg Trp Ala Asn Tyr Asp Lys Ile His Asn
385                 390                 395                 400
Lys Asp Leu Trp Ser Asp Thr Phe Thr Tyr Thr Ser Ser Ala Asn Ala
                405                 410                 415
Leu Tyr Thr Pro Gln Val Asp Ile Asn Pro Thr Lys Gly Leu Val Cys

            420                 425                 430
Trp Ser Trp Val Glu Tyr Leu Pro Gly Lys Arg Ile Val Trp Ser Asp
        435                 440                 445
Thr Gln Trp Thr His Ala Asn Gly Val Glu Asp Ile Val Met Gln Glu
    450                 455                 460
Gly Ser Met Lys Leu Tyr Pro Asn Pro Ala Gln Glu Tyr Ala Val Ile
465 470 475 400
Ser Leu Pro Thr Ala Ala Asn Cys Lys Ala Val Val Tyr Asp Met Gln
                485                 490                 495
Gly Arg Val Val Ala Glu Ala Ser Phe Ser Gly Asn Glu Tyr Arg Leu
            500                 505                 510
Asn Val Gln His Leu Ala Lys Gly Thr Tyr Ile Leu Lys Val Val Ser
        515                 520                 525
Asp Thr Glu Arg Phe Val Glu Lys Leu Ile Val Glu
    530                 535                 540

(2) INFORMATION FOR SEQ ID NO:521

(i) SEQUENCE CHARACTERISTICS:
(A) LENGTH: 771 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...771

(xi) SEQUENCE DESCRIPTION: SEQ ID NO:521

Met Leu Thr Ile Arg Asn Phe Leu Leu Phe Cys Cys Leu Ser Leu Ile
1               5                   10                  15
Ala Phe Ala Ala Asp Ala Gln Ser Ser Val Ser Ser Gly Arg Arg Leu
            20                  25                  30
Thr Glu Tyr Val Asn Pro Phe Ile Gly Thr Ala Asn Tyr Gly Thr Thr
        35                  40                  45
Asn Pro Gly Ala Val Leu Pro Asn Gly Leu Met Ser Val Thr Pro Phe
    50                  55                  60
Asn Val Ser Gly Ser Thr Glu Asn Arg Phe Asp Lys Asp Ser Arg Trp
65                  70                  75                  80
Trp Ser Ala Pro Tyr Ser Ala Asp Asn Ser Tyr Cys Ile Gly Phe Ser
                85                  90                  95
His Val Asn Leu Ser Gly Val Gly Cys Pro Glu Leu Ser Gly Ile Leu
            100                 105                 110
Leu Met Ala Thr Ser Gly Thr Phe Asp Pro Asp Tyr Cys Cys Tyr Gly
        115                 120                 125
Ser Ser Leu Ser Arg Glu Tyr Ala Arg Pro Gly Glu Tyr Lys Ala Val
    130                 135                 140
Leu Asp Lys Tyr Gly Ile Asp Ala Ala Val Thr Val Thr Glu Arg Thr
145                 150                 155                 160
Ala Leu Thr Glu Phe Ala Phe Pro Glu Gly Glu Gly His Ile Leu Leu
                165                 170                 175
Asn Leu Gly Gln Ala Leu Ser Asn Glu Ser Gly Ala Ser Val Arg Phe
            180                 185                 190
Leu Asn Asp Ser Thr Val Val Gly Ser Arg Leu Met Gly Thr Phe Cys
        195                 200                 205
Tyr Asn Pro Gln Ala Val Phe Arg Gln Tyr Phe Val Leu Gln Val Ser
    210                 215                 220
Arg Arg Pro Ile Ser Ala Gly Tyr Trp Lys Lys Gln Pro Pro Met Thr
225                 230                 235                 240
Val Glu Ala Gln Trp Asp Ser Thr Ala Gly Lys Tyr Lys Gln Tyr Asp
                245                 250                 255
Gly Tyr Lys Arg Glu Met Ser Gly Asp Asp Ile Gly Val Arg Phe Ser
            260                 265                 270
Phe Asn Cys Asp Gln Gly Glu Lys Ile Tyr Val Arg Ser Ala Val Ser
        275                 280                 285
Phe Val Ser Glu Ala Asn Ala Leu Tyr Asn Leu Glu Ala Glu Gln Glu
    290                 295                 300
Glu Val Phe Lys Ser Val Gly Gly Asn Pro Ala Lys Ala Phe Ser Ala

305                 310                 315                 320
Ile Arg Ser Arg Ala Ile Glu Arg Trp Glu Glu Ala Leu Gly Thr Val
                325                 330                 335
Glu Val Glu Gly Gly Thr Pro Asp Glu Lys Thr Ile Phe Tyr Thr Ala
            340                 345                 350
Leu Tyr His Leu Leu Ile His Pro Asn Ile Leu Gln Asp Ala Asn Gly
        355                 360                 365
Glu Tyr Pro Met Met Gly Ser Gly Lys Thr Gly Asn Thr Ala His Asp
    370                 375                 380
Arg Tyr Thr Val Phe Ser Leu Trp Asp Thr Tyr Arg Asn Val His Pro
385                 390                 395                 400
Leu Leu Cys Leu Leu Tyr Pro Glu Lys Gln Leu Asp Met Val Arg Thr
                405                 410                 415
Leu Ile Asp Met Tyr Arg Glu Ser Gly Trp Leu Pro Arg Trp Glu Leu
            420                 425                 430
Tyr Gly Gln Glu Thr Leu Thr Met Glu Gly Asp Pro Ser Leu Ile Val
        435                 440                 445
Ile Asn Asp Thr Trp Gln Arg Gly Leu Arg Ala Phe Asp Thr Ala Thr
    450                 455                 460
Ala Tyr Glu Ala Met Lys Lys Asn Ala Ser Ser Ala Gly Ala Thr His
465                 470                 475                 480
Pro Ile Arg Pro Asp Asn Asp Asp Tyr Leu Thr Leu Gly Phe Val Pro
                485                 490                 495
Leu Arg Glu Gln Tyr Asp Asn Ser Val Ser His Ala Leu Glu Tyr Tyr
            500                 505                 510
Leu Ala Asp Trp Asn Leu Ser Arg Phe Ala His Ala Leu Gly His Lys
        515                 520                 525
Glu Asp Ala Ala Leu Phe Gly Lys Arg Ser Leu Gly Tyr Arg His Tyr
    530                 535                 540
Tyr Asn Lys Glu Tyr Gly Met Leu Cys Pro Leu Leu Pro Asp Gly Ser
545                 550                 555                 560
Phe Leu Thr Pro Phe Asp Pro Lys Gln Gly Glu Asn Phe Glu Pro Asn
                565                 570                 575
Pro Gly Phe His Glu Gly Ser Ala Tyr Asn Tyr Ala Phe Phe Val Pro
            580                 585                 590
His Asp Ile Gln Gly Leu Ala Arg Leu Met Gly Gly Ala Lys Val Phe
        595                 600                 605
Ser Glu Arg Leu Gln Lys Val Phe Asp Glu Gly Tyr Tyr Asp Pro Thr
    610                 615                 620
Asn Glu Pro Asp Ile Ala Tyr Pro Tyr Leu Phe Ser Tyr Phe Pro Lys
625                 630                 635                 640
Glu Ala Trp Arg Thr Gln Lys Leu Thr Arg Glu Leu Ile Asp Lys His
                645                 650                 655
Phe Cys Asn Ala Pro Asn Gly Leu Pro Gly Asn Asp Asp Ala Gly Thr
            660                 665                 670
Met Ser Ala Trp Leu Val Tyr Ser Met Leu Gly Phe Tyr Pro Asp Cys
        675                 680                 685
Pro Gly Ser Pro Thr Tyr Thr Leu Thr Ser Pro Val Phe Pro Arg Val
    690                 695                 700
Arg Ile Arg Leu Asn Pro Gln Tyr Tyr Pro Gln Gly Glu Leu Ile Ile
705                 710                 715                 720
Thr Thr Asn Thr Glu Asn Gln Pro Thr Asp Ser Ile Tyr Ile His Thr
                725                 730                 735
Val Ser Leu Gly Asn Lys Thr Leu Pro His Gly Thr Arg His Ile Ser
            740                 745                 750
His Ala Asp Leu Val Arg Cys Gly His Leu Arg Tyr Glu Leu Ser Asn
        755                 760                 765
Arg Pro Arg
    770

(2) INFORMATION FOR SEQ ID NO:522

     (i) SEQUENCE CHARACTERISTICS:
          (A) LENGTH: 776 amino acids
          (B) TYPE: amino acid
          (D) TOPOLOGY: linear

     (ii) MOLECULE TYPE: protein

    (iii) HYPOTHETICAL: YES

     (vi) ORIGINAL SOURCE:
          (A) ORGANISM: Porphyromonas gingivalis

     (ix) FEATURE:

(A) NAME/KEY: misc_feature
(B) LOCATION 1...776

(xi) SEQUENCE DESCRIPTION: SEQ ID NO:522

Met Cys Lys Ile Arg Phe Ser Leu Leu Gln Ala Leu Val Val Cys Leu
1               5                   10                  15
Leu Phe Thr Ser Phe Ser Leu Gln Ala Gln Glu Glu Gly Ile Trp Asn
            20                  25                  30
Thr Leu Leu Ala Ile His Lys Thr Glu Lys Ala Val Glu Thr Pro Lys
        35                  40                  45
Lys Val Phe Ala Val Ala Asn Gly Val Leu Tyr Ser Val Gly Lys Glu
    50                  55                  60
Ala Pro His Glu Ala Lys Ile Phe Asp Arg Ile Ser Gly Leu Ser Asp
65                  70                  75                  80
Thr Ser Val Ser Ser Ile Ala Tyr Ser Glu Gln Leu Lys Ser Leu Val
                85                  90                  95
Ile Tyr Tyr Ala Ser Gly Asn Ile Asp Ile Leu Asp Glu Ala Gly Arg
            100                 105                 110
Val Thr Asn Val Pro Ala Leu Lys Asp Asn Ile Asp Leu Ile Asp Lys
        115                 120                 125
Thr Leu Asn Arg Leu Leu Ile Val Gly Asn Arg Ala Tyr Leu Ala Gly
    130                 135                 140
Gly Phe Gly Leu Ser Val Leu Asp Val Ala Glu Ala Arg Ile Pro Ala
145                 150                 155                 160
Thr Tyr Ala Lys Gly Thr Lys Val Thr Asp Val Ala Lys Leu Asp Asn
                165                 170                 175
Asp Arg Leu Leu Met Leu Lys Glu Gly Gln Leu Phe Ile Gly Lys Glu
            180                 185                 190
Thr Asp Asn Leu Gln Asp Pro Ala Ala Trp Thr Ala Leu Ser Leu Asn
        195                 200                 205
Leu Pro Met Gly Ser Val Thr Gly Leu Gly Ile Val Gly Glu Asp Ile
    210                 215                 220
Cys Phe Leu Leu Ala Asp Gly Arg Val Tyr Val Ala Ala Asn Gln Ser
225                 230                 235                 240
Phe Glu Pro Glu Leu Leu Leu Ser Ser Ser Ala Asp Ser Arg Leu Tyr
                245                 250                 255
Val Thr Asp Arg Gly Leu Phe Ile Cys Ala Glu Asn Arg Ile Tyr Phe
            260                 265                 270
Ile Glu Lys Gly Arg Lys Thr Thr Gln Phe Pro Ile Ala Asp Val Leu
        275                 280                 285
Gly Val Gly Ala Met Asn Glu Ser Asn Thr Ala Tyr Ile Ala Leu Gly
    290                 295                 300
Glu Glu Gly Leu Ala Ser Leu Leu Leu Ala Glu Gly Ser Thr Ala Glu
305                 310                 315                 320
Ala Met Pro Val Ala Phe Asp Gly Pro Gly Asp Asn Asp Phe Tyr Glu
                325                 330                 335
Met Arg Phe Ser His Gly Arg Leu Tyr Ala Ala Ser Gly Leu Trp Gly
            340                 345                 350
Thr Asn Leu Met Gly His Ala Gly Met Val Lys Leu Tyr Asp Gly Asn
        355                 360                 365
Arg Trp Thr Asn Phe Asp Lys Lys Thr Val Gln Glu Gln Leu Gly Gly
    370                 375                 380
Gly Phe Ser Phe Asn Asp Ala Ile Asp Ile Ala Val Ser Asn Gly Asp
385                 390                 395                 400
Pro Asp His Phe Phe Val Gly Thr Trp Gly Asn Gly Leu Phe Glu Phe
                405                 410                 415
Lys Asp Gly Lys Ala Ile Ala Arg Tyr Ser Gly Asn Glu Thr Ala Ile
            420                 425                 430
Ala Glu Cys Asn Pro Gly Asp Ala Arg Val Lys Ala Ile Ala Phe Asp
        435                 440                 445
Asn Lys Gly Asn Leu Trp Gly Thr Leu Gly Ala Val Gly Lys Asn Ile
    450                 455                 460
Phe Met Tyr Asp Pro Gln Ser Ser Thr Trp His Ser Phe Ser Tyr Pro
465                 470                 475                 480
Asp Val Ala Asn Leu Ala Ser Phe Gly Asn Met Ile Ile Leu Pro Asn
                485                 490                 495
Gly Asp Lys Trp Val Asn Ile Leu His Arg Ser Gly Gly Ser Thr Arg
            500                 505                 510
Lys Gly Val Leu Ile Phe Asn Asp Arg Gly Thr Pro Glu Thr Thr Ser
        515                 520                 525
Asp Asp Ser His Leu Tyr Val Glu Gln Phe Val Asn Arg Leu Gly Ala
    530                 535                 540
Ala Ile Gly His Lys Thr Ile Tyr Ala Met Ala Val Asp His Asn Gly
545                 550                 555                 560

Ser Val Trp Met Gly Ser Asp Ile Gly Ile Phe Gly Val Tyr Asn Ala
                565                 570                 575
Ala Gly Val Leu Ser Ser Thr Ser Thr Pro Ile Ala Val Arg Pro Val
            580                 585                 590
Gly Gly Glu Glu Pro Asn Leu Tyr Tyr Val Leu Asp Lys Val Thr Val
        595                 600                 605
Thr Asp Ile Val Val Asp Lys Leu Asn His Lys Trp Val Ala Thr Gln
    610                 615                 620
Gly Thr Gly Leu Tyr Leu Leu Ser Glu Asp Cys Ser Lys Ile Leu Ala
625                 630                 635                 640
Gln Phe Thr Val Glu Asn Ser Pro Leu Leu Ser Asn Asn Ile Leu Ser
                645                 650                 655
Leu Ala Leu Asn Asp Asp Asn Gly Leu Leu Tyr Ile Gly Thr Ala Asp
            660                 665                 670
Gly Leu Met Thr Phe Gln Thr Gly Thr Gly Ser Gly Ser Ala Ser Glu
        675                 680                 685
Leu Asp Gly Val Tyr Val Tyr Pro Asn Pro Leu Arg Pro Glu Tyr Pro
    690                 695                 700
Asp Gly Val Thr Ile Ala Gly Leu Gln Ala Gly Cys Ser Val Lys Ile
705                 710                 715                 720
Thr Asp Thr Thr Gly Arg Leu Leu Tyr Gln Thr Glu Ser Val Thr Thr
                725                 730                 735
Glu Val Lys Trp Asn Ala Arg Gly Ala Asp Gly Asn Arg Val Ala Ser
            740                 745                 750
Gly Val Tyr Ala Val Ala Val Tyr Asp Pro Val Ser Lys Lys Ser Lys
        755                 760                 765
Leu Ile Arg Phe Ala Val Ile Arg
    770                 775

(2) INFORMATION FOR SEQ ID NO:523

     (i) SEQUENCE CHARACTERISTICS:
          (A) LENGTH: 1158 amino acids
          (B) TYPE: amino acid
          (D) TOPOLOGY: linear

    (ii) MOLECULE TYPE: protein

   (iii) HYPOTHETICAL: YES

    (vi) ORIGINAL SOURCE:
          (A) ORGANISM: Porphyromonas gingivalis

    (ix) FEATURE:
          (A) NAME/KEY: misc_feature
          (B) LOCATION 1...1158

    (xi) SEQUENCE DESCRIPTION: SEQ ID NO:523
   Met Lys Arg Ile Leu Pro Ile Val Ala Phe Leu Ser Leu Phe Leu Ala
   1               5                   10                  15
   Leu Ala Leu Pro Ala Lys Ala Gln Arg Ala Met Gly Lys Thr Ala Asp
               20                  25                  30
   Arg Ser Leu Met Ala Ser Gly His Trp Val Lys Ile Arg Val Asp Ala
           35                  40                  45
   Ser Gly Val Tyr Arg Leu Thr Asp Glu Gln Leu Arg Ala Asn Gly Phe
       50                  55                  60
   Ser Asp Pro Ser Lys Val Gly Val Phe Gly Tyr Gly Gly Gly Val Leu
   65                  70                  75                  80
   Pro Glu Asp Leu Ser Arg Ile Thr Thr Asp Asp Leu Pro Pro Val Pro
                   85                  90                  95
   Val Leu Arg Gln Gly Asn Ala Leu Tyr Phe Tyr Ala Val Gly Pro Val
               100                 105                 110
   Thr Trp Phe Tyr Asn Pro Ala Lys Thr Thr Met Glu His Thr Val Asn
           115                 120                 125
   Thr Tyr Ser Thr His Gly Tyr Tyr Phe Leu Ser Asp Ala Ala Gly Ala
       130                 135                 140
   Pro Leu Gln Met Ser Gln Tyr Thr Gly Gly Gly Ala Ser Ala Glu Ala
   145                 150                 155                 160
   Leu Ile Asp Tyr Tyr Asp Glu Leu Met Leu His Glu Gln Glu Leu Tyr
                   165                 170                 175
   Ser Pro Lys Glu Ser Gly Arg Asp Leu Tyr Gly Glu Ser Phe Ser Ala
               180                 185                 190
   Val Asn Thr Arg Thr Val Lys Phe Pro Leu Arg Gly Asn Thr Arg Ser
           195                 200                 205
   Ser Gly Glu Leu Gly Thr Val Phe Ser Tyr Ile Ala Lys Ala Arg Ser
   210 215 220
   Ala Gly Gly Gly Arg Glu Met Ser Leu Ser Ala Asn Gly Ile Leu Ile
   225 230 235 240
   Phe Ser Asp Pro Phe Ser Met Thr Ser Asn Glu Val Ser Asn Ser Tyr
   245 250 255
   Leu Ala Gly Lys Lys Arg Arg Leu Tyr His Ser Thr Pro Met Asn Ser
   260 265 270
   Leu Val Asn Glu Leu Arg Leu Asp Ala Asn Tyr Ser Met Thr Gly Asp
   275 280 285
   Ala Val Asn Leu Asp Phe Ile Glu Val Ala Thr Gln Asn Asp Leu Arg
   290 295 300
   Tyr Asp Gly Ala Pro Met His Ile Arg Arg Phe Ser Asn Leu Pro Val
   305 310 315 320
   Leu Gly Gly Glu Ser Cys Arg Phe Val Ile Ser Glu Val Pro Glu Ser
   325 330 335
   Leu Val Val Leu Gln Ala Asn Ser Ser Leu Thr Ala Ser Leu Val Pro
   340 345 350
   Val Lys Thr Val Gly Asp Lys Thr Ile Glu Phe Val Ala Pro Pro Lys
   355 360 365
   Gly Gln Asp Arg Arg Thr Ile Asn Thr Phe Tyr Ala Val Asp Leu Ser
   370 375 380
   Gln Ala Ser Ala Pro Glu Ile Leu Gly Ala Val Pro Asn Gln Asn Leu
   385 390 395 400
   His Gly Glu Glu Ile Pro Asp Leu Ile Ile Val Ser Thr Gln Ala Leu
   405 410 415
   Leu Leu Glu Ala Asp Arg Leu Ala Thr Tyr Arg Arg Glu Lys Asn Gly
   420 425 430
   Leu Lys Val Leu Val Val Leu Gln Glu Gln Val Phe Asn Glu Phe Ser
   435 440 445
   Gly Gly Thr Pro Asp Ala Thr Ala Tyr Arg Leu Phe Ala Lys Met Phe
   450 455 460
   Tyr Asp Arg Trp Lys Ala Asn Ala Pro Val Gly Glu Thr Phe Pro Met
   465 470 475 480
   Gln Met Leu Leu Phe Gly Asp Gly Ala His Asp Asn Arg Lys Val Ser
   485 490 495
   Val Ala Trp Gln Lys Pro Tyr Leu Gln Gln Thr Glu Phe Leu Leu Thr
   500 505 510
   Phe Gln Ala Val Asn Ser Thr Asn Val Asn Ser Tyr Val Thr Asp Asp
   515 520 525
   Tyr Phe Gly Leu Leu Asp Asp Gln Pro Ala Ser Val Asn Ile Gly Trp
   530 535 540
   Arg Asn Tyr Asn Met Ala Val Gly Arg Phe Pro Val Arg Thr Pro Ala
   545 550 555 560
   Glu Ala Arg Ile Ala Val Asp Lys Thr Ile Arg Tyr Glu Glu Asp Arg
   565 570 575
   Glu Ser Gly Ala Trp Arg Ile Arg Ala Cys Phe Ala Ala Asp Asn Gly
   580 585 590
   Asp Lys His Ala Thr Glu Thr Ser Arg Leu Ile Asp Thr Val Lys Arg
   595 600 605
   Tyr Ala Pro Ala Ile Met Pro Val Arg Ala Phe Gln Asp Val Tyr Pro
   610 615 620
   His Val Ile Glu Asn Gly Leu His Ser Ile Pro Gly Ala Lys Lys Lys
   625 630 635 640
   Met Leu Glu Thr Leu Gln Ser Gly Ile Ile Leu Leu Asn Tyr Ala Gly
   645 650 655
   His Gly Gly Pro Ala Gly Trp Ser Asp Glu His Leu Leu Thr Leu Asn
   660 665 670
   Asp Ile His Lys Phe Asn Tyr Lys His Met Pro Ile Trp Ile Thr Ala
   675 680 685
   Thr Cys Asp Phe Ala Asn Tyr Asp Ser Gln Thr Thr Ser Ala Gly Glu
   690 695 700
   Glu Val Phe Leu His Glu Lys Ser Gly Thr Pro Ile Met Phe Ser Thr
   705 710 715 720
   Thr Arg Val Val Tyr Asn Thr Gln Asn Glu Lys Ile Asn Gly Phe Met
   725 730 735
   Leu Arg Arg Met Phe Glu Lys Ala Lys Asp Gly Arg Tyr Arg Thr Met
   740 745 750
   Gly Glu Ile Ile Arg Ser Ala Lys Gln Gly Met Leu Ser Thr Val Phe
   755 760 765
   Pro Asp Ser Ile Asn Gln Leu Ser Phe Phe Leu Met Gly Asp Pro Ser
   770 775 780
   Val Arg Met Asn Leu Pro Thr His Lys Val Gln Leu Thr Ala Ile Asn
   785 790 795 800
   Gly Gln Asp Pro Glu Gly Gln Tyr Gly Thr Ile Met Leu Lys Ser Leu
   805 810 815
   Glu Arg Val Ala Leu Lys Gly Lys Val Thr Asp Glu Lys Gly Thr Phe
   820 825 830
   Asp Glu Thr Phe Ser Gly Lys Val Phe Leu Thr Val Phe Asp Gly Arg
   835 840 845
   Lys Lys Met Thr Ala Leu Glu Glu Glu Gly Asn Asp Leu Ser Leu Val
   850 855 860
   Tyr Tyr Asp Tyr Pro Asn Val Met Tyr Ala Gly Ile Ala Glu Val Lys
   865 870 875 880
   Asp Gly Leu Phe Glu Thr Ser Phe Ile Val Pro Lys Asp Val Asn Tyr
   885 890 895
   Ser Glu His Glu Gly Arg Ile Asn Leu Tyr Ala Tyr Asn Glu Ser Thr
   900 905 910
   Lys Ala Glu Ala Met Gly Val Asp Phe Ser Ile Arg Val Gln Pro Gly
   915 920 925
   Ile Pro Asp Glu Val Thr Glu Asp Asn Thr Pro Pro Glu Ile Ile Ser
   930 935 940
   Cys Phe Leu Asn Asp Ser Thr Phe Arg Ser Gly Asp Glu Val Asn Pro
   945 950 955 960
   Thr Pro Leu Phe Met Ala Glu Val Phe Asp Leu Asn Gly Ile Asn Ile
   965 970 975
   Thr Gly Ser Gly Val Gly His Asp Ile Thr Leu Cys Ile Asp Gly Arg
   980 985 990
   Ala Asp Leu Thr Tyr Asn Leu Asn Ala Tyr Phe Thr Ser Ser Ala Thr
   995 1000 1005
   Asp Ala Gly Val Gly Thr Ile Leu Phe Met Ile Pro Ala Leu Ala Glu
   1010 1015 1020
   Gly Asp His Thr Ala Arg Leu Thr Val Trp Asp Ile Phe Asn Asn Ala
   1025 1030 1035 1040
   Val His His Asp Phe Ser Phe Arg Val Val Asp Gly Ile Ala Pro Asp
   1045 1050 1055
   Val Ala Asp Val Ile Leu Phe Pro Asn Pro Val Arg Glu Ser Ala Thr
   1060 1065 1070
   Phe Arg Ile Phe His Asn Arg Pro Gly Ser Asp Leu Asn Val Ala Val
   1075 1080 1085
   Glu Ile Tyr Asp Phe Thr Gly Arg Leu Val Asn Ser Leu Pro Val Lys
   1090 1095 1100
   Thr Tyr Ser Ser Ser Tyr Gly Glu Pro Ile Glu Ile Lys Trp Asp Leu
   1105 1110 1115 1120
   Thr Ser Lys Tyr Gly Val Lys Ile Gly Asn Gly Phe Tyr Leu Tyr Arg
   1125 1130 1135
   Cys Val Val Asn Ser Pro Gly Gly Gln Thr Ala Ser Met Ala Lys Lys
   1140 1145 1150
   Met Ile Val Val Gly Gln
   1155

(2) INFORMATION FOR SEQ ID NO:524

(i) SEQUENCE CHARACTERISTICS:
(A) LENGTH: 961 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...961

(xi) SEQUENCE DESCRIPTION: SEQ ID NO:524

Met Lys Lys Leu Phe Pro Leu Leu Leu Leu Ile Leu Ser Ile Leu Val
1               5                   10                  15
Gly Cys Gly Lys Lys Glu Lys His Ser Val Thr Glu Ile Ala Arg Glu
            20                  25                  30
Lys Lys Arg Ile Thr Ala Leu Leu Tyr Glu Lys Glu Leu Pro Thr Asp
        35                  40                  45
Ser Val Lys Gln Leu Tyr Glu Asn Ser Val Gln Asn Lys Asn Leu Val
    50                  55                  60
Gly Gln Met Leu Phe Ala Ile Glu Val Gly Lys Arg Met Arg Asn Met

65                  70                  75                  80
Ser Gln Tyr Thr Asp Ala Met Leu Tyr His Gln Lys Gly Leu Asn Ala
                85                  90                  95
Ala Leu Asn Leu Arg Asp Thr Ile Val Ala Ala Gln Ala Trp Asn His
            100                 105                 110
Leu Gly Thr Asp Ser Arg Arg Ile Gly Ala Leu Ala Glu Ala Ser Asp
        115                 120                 125
Tyr His Tyr Lys Ala Leu Ser Leu Ile Glu Ser Phe Ser Gly Asn Gln
    130                 135                 140
Asn Arg Pro Ala Ile Lys Ala Arg Ser Ala Ala Leu Asn Gly Ile Gly
145                 150                 155                 160
Asn Ile Asn Leu Glu Leu Gly Tyr His Asp Glu Ala Glu Lys Asn Phe
                165                 170                 175
Leu Lys Ala Leu Gln Gly Glu Lys Glu Leu Asp Ser Pro Leu Gly Gln
            180                 185                 190
Ala Ile Asn Tyr Ala Asn Leu Gly Arg Ile Tyr Arg Gln Arg Lys Glu
        195                 200                 205
Tyr Asp Lys Ala Arg Thr Tyr Phe Leu Leu Ser Leu Glu Gln Asn Asn
    210                 215                 220
Met Ala Glu Asn Leu Met Gly Ile Gly Leu Cys Ser Ile Asn Leu Gly
225                 230                 235                 240
Glu Val Asp Glu Glu Lys Gly Asp Tyr Gln Lys Ala Leu Gln Glu Tyr
                245                 250                 255
Ala Thr Ala Tyr Lys Leu Met Glu Gln Leu Ser Asp Arg Trp His Trp
            260                 265                 270
Leu Asn Ser Cys Ile Pro Met Ala Arg Ile Asn Leu Lys Gln Gly Asn
        275                 280                 285
Glu Arg Leu Tyr Gln His Phe Ile Ser Leu Ala Glu Gly Thr Ala Lys
    290                 295                 300
Glu Ile Asn Ser Thr Ser His Leu Ile Glu Ile Tyr Asn Leu Gln Tyr
305                 310                 315                 320
Glu Asn Leu Glu Arg Lys Lys Glu Tyr Lys Gln Ala Leu Glu Ala Phe
                325                 330                 335
Cys Leu Ser Lys Thr Leu Ser Asp Ser Met Ser Ile Ala His Lys Val
            340                 345                 350
Ser Ser Ile Gln Glu Thr Arg Phe Asn Tyr Glu Arg Asn Lys Ser Gln
        355                 360                 365
Lys Glu Leu Glu Glu Ile Gln Gln Val Ser Lys Ala Lys Gln Glu Lys
    370                 375                 380
Ser Lys Phe Ile Leu Leu Ser Thr Leu Phe Ala Leu Phe Ile Ser Ile
385                 390                 395                 400
Leu Leu Ile Ser Val Leu Thr Tyr Ala Tyr Arg Gln Gly Lys Lys His
                405                 410                 415
Asn Lys Leu Ile Lys Glu Thr Asp Lys Leu Arg Ser Gly Phe Phe Thr
            420                 425                 430
Gly Ile Thr His Glu Phe Arg Thr Pro Ile Thr Val Ile Gln Gly Leu
        435                 440                 445
Asn Glu Lys Met Ser Ser Ser Pro Asp Leu Gln Ala Ser Asp Arg Thr
    450                 455                 460
Glu Leu His Lys Ile Ile Asp Arg Gln Ser Ser His Met Leu Asn Leu
465                 470                 475                 480
Val Asn Gln Leu Leu Asp Ile Cys Lys Ile Arg Ser Gly Val Ser Thr
                485                 490                 495
Pro Glu Trp Arg Asn Gly Asp Ile Val Ser Phe Val Gln Ile Leu Ile
            500                 505                 510
Asp Ser Phe Ala Pro Tyr Ala Gln Ala Gln Asp Ile Thr Leu Glu Leu
        515                 520                 525
Gln Pro Glu Ser Lys Pro Ile Val Val Asp Phe Val Pro Ser Tyr Leu
    530                 535                 540
Gln Lys Ile Ile Ser Asn Leu Leu Ser Asn Ala Ile Lys Tyr Ser Leu
545                 550                 555                 560
Ala Gly Gly Arg Val Val Ile Ser Leu Ala Lys Thr Lys Asn Glu Lys
                565                 570                 575
Asn Leu Ile Ile Arg Val Ala Asp Asn Gly Ile Gly Ile Asp Lys Thr
            580                 585                 590
Asp Gln Ala His Ile Phe Asp Ile Phe Tyr Arg Gly Gln Ser Ala Thr
        595                 600                 605
Glu Lys His Gly Ser Gly Val Gly Leu Ser Phe Thr Asn Ile Leu Val
    610                 615                 620
Glu Asn Leu Arg Gly Thr Ile Lys Val Glu Ser Gln Pro Gly Lys Gly
625                 630                 635                 640
Ser Ala Phe Thr Ile Ser Ile Pro Thr Gln Asn Gln Ser Ser Ser Ala
                645                 650                 655
Glu Ile Leu Pro Trp Leu Pro Ser Ser Asp Asp Ile Val Met Pro Val
            660                 665                 670

His Ile Ala Pro Asp Asp Ser Pro Thr Ser Pro Met Val Ala Ala Leu
            675                 680                 685
Asn His Arg Phe Glu Asp Glu Arg Pro Thr Ile Leu Leu Val Glu Asp
        690                 695                 700
Asn Lys Asp Ile Asn Leu Leu Val Lys Leu Leu Leu Cys Asp Arg Tyr
705                 710                 715                 720
Asn Val Leu Ser Ala Ala Asn Gly Lys Glu Gly Ile Ala Leu Ala Thr
                725                 730                 735
Glu His Ile Pro Asp Ile Ile Ile Thr Asp Ile Met Met Pro Ile Met
            740                 745                 750
Asp Gly Ile Glu Met Thr Ile Arg Met Lys Gln Ser Pro Leu Leu Cys
        755                 760                 765
His Ile Pro Ile Val Ala Leu Thr Ala Lys Ser Thr Glu Gln Asp Arg
    770                 775                 780
Leu Glu Gly Ile Lys Ser Gly Val Val Ser Tyr Leu Cys Lys Pro Phe
785                 790                 795                 800
Ser Pro Glu Glu Leu Leu Met Arg Ile Glu Gln Leu Leu Lys Asp Arg
                805                 810                 815
Glu Leu Leu Lys Lys Phe Tyr Met Gln Lys Leu Met Leu Asp Arg Lys
            820                 825                 830
Pro Glu Glu Glu Pro Gln Pro Ile Asp Asp Ser Ser Met Gln Phe Leu
        835                 840                 845
Leu Ala Ala Lys Asp Ala Val Ser Gly Gly Ile Lys Gln Asn Pro Asp
    850                 855                 860
Phe Ser Ala Gln Asp Leu Ala Glu Lys Met Cys Met Ser Pro Ser Gln
865                 870                 875                 880
Leu Asn Arg Lys Leu Thr Ser Val Val Gly Cys Ser Thr Ile Gly Tyr
                885                 890                 895
Ile Gln Gln Ile Lys Ile Lys Leu Ala Cys Lys Leu Leu Ala Asp Glu
            900                 905                 910
Ser Lys Asn Ile Ser Asp Ile Ser Ile Glu Ala Gly Phe Ser Asp Pro
        915                 920                 925
Ala Tyr Phe Ser Arg Thr Phe Lys Arg Tyr Met Asn Cys Ser Pro Ser
    930                 935                 940
Gln Tyr Arg Gln Lys Leu Leu Ala Met Pro Gly Ser Asp Lys Glu Thr
945                 950                 955                 960
Val

(2) INFORMATION FOR SEQ ID NO:525

    (i) SEQUENCE CHARACTERISTICS:
        (A) LENGTH: 556 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...556

    (xi) SEQUENCE DESCRIPTION: SEQ ID NO:525

Met Lys Lys Leu Leu Gln Ala Lys Ala Leu Ile Leu Ala Leu Gly Leu
1               5                   10                  15
Phe Gln Leu Pro Ala Ile Ala Gln Thr Gln Met Gln Ala Asp Arg Thr
            20                  25                  30
Asn Gly Gln Phe Ala Thr Glu Glu Met Gln Arg Ala Phe Gln Glu Thr
        35                  40                  45
Asn Pro Pro Ala Gly Pro Val Arg Ala Ile Ala Glu Tyr Glu Arg Ser
    50                  55                  60
Ala Ala Val Leu Val Arg Tyr Pro Phe Gly Ile Pro Met Glu Leu Ile
65                  70                  75                  80
Lys Glu Leu Ala Lys Asn Asp Lys Val Ile Thr Ile Val Ala Ser Glu
                85                  90                  95
Ser Gln Lys Asn Thr Val Ile Thr Gln Tyr Thr Gln Ser Gly Val Asn
            100                 105                 110
Leu Ser Asn Cys Asp Phe Ile Ile Ala Lys Thr Asp Ser Tyr Trp Thr
        115                 120                 125

Arg Asp Tyr Thr Gly Trp Phe Ala Met Tyr Asp Thr Asn Lys Val Gly
    130                 135                 140
Leu Val Asp Phe Ile Tyr Asn Arg Pro Arg Pro Asn Asp Asp Glu Phe
145                 150                 155                 160
Pro Lys Tyr Glu Ala Gln Tyr Leu Gly Ile Glu Met Phe Gly Met Lys
                165                 170                 175
Leu Lys Gln Thr Gly Gly Asn Tyr Met Thr Asp Gly Tyr Gly Ser Ala
            180                 185                 190
Val Gln Ser His Ile Ala Tyr Thr Glu Asn Ser Ser Leu Ser Gln Ala
        195                 200                 205
Gln Val Asn Gln Lys Met Lys Asp Tyr Leu Gly Ile Thr His His Asp
    210                 215                 220
Val Val Gln Asp Pro Asn Gly Glu Tyr Ile Asn His Val Asp Cys Trp
225                 230                 235                 240
Gly Lys Tyr Leu Ala Pro Asn Lys Ile Leu Ile Arg Lys Val Pro Asp
                245                 250                 255
Asn His Pro Gln His Gln Ala Leu Glu Asp Met Ala Ala Tyr Phe Ala
            260                 265                 270
Ala Gln Thr Cys Ala Trp Gly Thr Lys Tyr Glu Val Tyr Arg Ala Leu
        275                 280                 285
Ala Thr Asn Glu Gln Pro Tyr Thr Asn Ser Leu Ile Leu Asn Asn Arg
    290                 295                 300
Val Phe Val Pro Val Asn Gly Pro Ala Ser Val Asp Asn Asp Ala Leu
305                 310                 315                 320
Asn Val Tyr Lys Thr Ala Met Pro Gly Tyr Glu Ile Ile Gly Val Lys
                325                 330                 335
Gly Ala Ser Gly Thr Pro Trp Leu Gly Thr Asp Ala Leu His Cys Arg
            340                 345                 350
Thr His Glu Val Ala Asp Lys Gly Tyr Leu Tyr Ile Lys His Tyr Pro
        355                 360                 365
Ile Leu Gly Glu Gln Ala Gly Pro Asp Tyr Lys Ile Glu Ala Asp Val
    370                 375                 380
Val Ser Cys Ala Asn Ala Thr Ile Ser Pro Val Gln Cys Tyr Tyr Arg
385                 390                 395                 400
Ile Asn Gly Ser Gly Ser Phe Lys Ala Ala Asp Met Thr Met Glu Ser
                405                 410                 415
Thr Gly His Tyr Thr Tyr Ser Phe Thr Gly Leu Asn Lys Asn Asp Lys
            420                 425                 430
Val Glu Tyr Tyr Ile Ser Ala Ala Asp Asn Ser Gly Arg Lys Glu Thr
        435                 440                 445
Tyr Pro Phe Ile Gly Glu Pro Asp Pro Phe Lys Phe Thr Cys Met Asn
    450                 455                 460
Glu Thr Asn Thr Cys Thr Val Thr Gly Ala Ala Lys Ala Leu Arg Ala
465                 470                 475                 480
Trp Phe Asn Ala Gly Arg Ser Glu Leu Ala Val Ser Val Ser Leu Asn
                485                 490                 495
Ile Ala Gly Thr Tyr Arg Ile Lys Leu Tyr Asn Thr Ala Gly Glu Glu
            500                 505                 510
Val Ala Ala Met Thr Lys Glu Leu Val Ala Gly Thr Ser Val Phe Ser
        515                 520                 525
Met Asp Val Tyr Ser Gln Ala Pro Gly Thr Tyr Val Leu Val Val Glu
    530                 535                 540
Gly Asn Gly Ile Arg Glu Thr Met Lys Ile Leu Lys
545                 550                 555

(2) INFORMATION FOR SEQ ID NO:526

    (i) SEQUENCE CHARACTERISTICS:
        (A) LENGTH: 428 amino acids
        (B) TYPE: amino acid
        (D) TOPOLOGY: linear

    (ii) MOLECULE TYPE: protein

    (iii) HYPOTHETICAL: YES

    (vi) ORIGINAL SOURCE:
        (A) ORGANISM: Porphyromonas gingivalis

    (ix) FEATURE:
        (A) NAME/KEY: misc_feature
        (B) LOCATION 1...428

    (xi) SEQUENCE DESCRIPTION: SEQ ID NO:526

Met Lys Leu Ser Ser Lys Lys Ile Leu Ala Ile Ile Ala Leu Leu Thr
1               5                   10                  15
Met Gly His Ala Val Gln Ala Gln Phe Val Pro Ala Pro Thr Thr Gly
            20                  25                  30
Ile Arg Met Ser Val Thr Thr Thr Lys Ala Val Gly Glu Lys Ile Glu
        35                  40                  45
Leu Leu Val His Ser Ile Glu Lys Lys Gly Ile Trp Ile Asp Leu Asn
    50                  55                  60
Gly Asp Ala Thr Tyr Gln Gln Gly Glu Glu Ile Thr Val Phe Asp Glu
65                  70                  75                  80
Ala Tyr His Glu Tyr Thr Ile Gly Thr Gln Thr Leu Thr Ile Tyr Gly
                85                  90                  95
Asn Thr Thr Arg Leu Gly Cys Arg Ser Thr Gly Ala Thr Ala Val Asp
            100                 105                 110
Val Thr Lys Asn Pro Asn Leu Thr Tyr Leu Ala Cys Pro Lys Asn Asn
        115                 120                 125
Leu Lys Ser Leu Asp Leu Thr Gln Asn Pro Lys Leu Leu Arg Val Trp
    130                 135                 140
Cys Asp Ser Asn Glu Ile Glu Ser Leu Asp Leu Ser Gly Asn Pro Ala
145                 150                 155                 160
Leu Ile Ile Leu Gly Cys Asp Arg Asn Lys Leu Thr Glu Leu Lys Thr
                165                 170                 175
Asp Asn Asn Pro Lys Leu Ala Ser Leu Trp Cys Ser Asp Asn Asn Leu
            180                 185                 190
Thr Glu Leu Glu Leu Ser Ala Asn Pro Arg Leu Asn Asp Leu Trp Cys
        195                 200                 205
Phe Gly Asn Arg Ile Thr Lys Leu Asp Leu Ser Ala Asn Pro Leu Leu
    210                 215                 220
Val Thr Leu Trp Cys Ser Asp Asn Glu Leu Ser Thr Leu Asp Leu Ser
225                 230                 235                 240
Lys Asn Ser Asp Val Ala Tyr Leu Trp Cys Ser Ser Asn Lys Leu Thr
                245                 250                 255
Ser Leu Asn Leu Ser Gly Val Lys Gly Leu Ser Val Leu Val Cys His
            260                 265                 270
Ser Asn Gln Ile Ala Gly Glu Glu Met Thr Lys Val Val Asn Ala Leu
        275                 280                 285
Pro Thr Leu Ser Pro Gly Ala Gly Ala Gln Ser Lys Phe Val Val Val
    290                 295                 300
Asp Leu Lys Asp Thr Asp Glu Lys Asn Ile Cys Thr Val Lys Asp Val
305                 310                 315                 320
Glu Lys Ala Lys Ser Lys Asn Trp Arg Val Phe Asp Phe Asn Gly Asp
                325                 330                 335
Ser Asp Asn Met Leu Pro Tyr Glu Gly Ser Pro Thr Ser Asn Leu Ala
            340                 345                 350
Val Asp Ala Pro Thr Val Arg Ile Tyr Pro Asn Pro Val Gly Arg Tyr
        355                 360                 365
Ala Leu Val Glu Ile Pro Glu Ser Leu Leu Gly Gln Glu Ala Ala Leu
    370                 375                 380
Tyr Asp Met Asn Gly Val Lys Val Tyr Ser Phe Ala Val Glu Ser Leu
385                 390                 395                 400
Arg Gln Asn Ile Asp Leu Thr His Leu Pro Asp Gly Thr Tyr Phe Phe
                405                 410                 415
Arg Leu Asp Asn Tyr Thr Thr Lys Leu Ile Lys Gln
            420                 425

(2) INFORMATION FOR SEQ ID NO:527

(i) SEQUENCE CHARACTERISTICS:
(A) LENGTH: 310 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...310

(xi) SEQUENCE DESCRIPTION: SEQ ID NO:527

Met Arg Lys Thr Ile Ile Phe Cys Leu Leu Leu Ala Leu Phe Gly Cys
1               5                   10                  15
Ser Trp Ala Gln Glu Arg Val Asp Glu Lys Val Phe Ser Ala Gly Thr
            20                  25                  30
Ser Ile Phe Arg Gly Ile Leu Glu Lys Val Lys Ala Pro Leu Met Tyr
        35                  40                  45
Gly Asp Arg Glu Val Trp Gly Met Ala Arg Ala Ser Glu Asp Phe Phe
    50                  55                  60
Phe Ile Leu Pro Val Thr Asp Asp Leu Thr Pro Val Leu Phe Tyr Asn
65                  70                  75                  80
Arg Leu Thr Asn Glu Pro Cys Phe Val Ser Asp Gln Gly Ile Thr Glu
                85                  90                  95
Tyr Phe Lys Phe Ala Gln Glu Gly Asp Tyr Ile Glu Val Glu Gly Ser
            100                 105                 110
Ser Val Phe Met Ala Asn Leu Leu Tyr Tyr Arg Phe Phe Pro Thr Arg
        115                 120                 125
Ile Thr Ser Tyr Asn Ala Pro Ile Glu Gly Val Val Ser Lys Thr Gly
    130                 135                 140
Asn Pro Ala Phe Thr Ile Pro Met Leu Pro Gly Val Ser Asp Cys Ile
145                 150                 155                 160
Glu Ile Ser Asn Asn Arg Lys Val Phe Leu Thr Asn Gln Leu Gly Val
                165                 170                 175
Val Asn Ile Thr Asp Gly Met Glu Pro Pro Ile Ile Ala Gly Val Ser
            180                 185                 190
Ala Ser Tyr Gly Ser Ser Val Arg Val Tyr Gly His Val Ser Gln Arg
        195                 200                 205
Trp Asp Ile Ile Gly His Cys Tyr Leu Asp Ile Tyr Pro Thr Asn Cys
    210                 215                 220
Tyr Pro Leu Ser Thr Lys Pro Val Ala Gly Asp Asp Glu Val Phe Val
225                 230                 235                 240
Lys Gln Gln Gly Arg Gln Ile Glu Ile Asp Ser Asn Ser Pro Ile Val
                245                 250                 255
Gln Val Val Val Tyr Asp Leu Glu Gly Lys Ser Val Phe Arg Lys Arg
            260                 265                 270
Met Thr Glu Asn Ala Tyr Thr Leu Ser Phe Arg Ala Pro Met Leu Gly
        275                 280                 285
Phe Met Thr Ile Met Ile Glu Thr Gln Asn Ser Ile Ile Asn Lys Lys
    290                 295                 300
Leu Asn Val Thr Gln Leu
305                 310

(2) INFORMATION FOR SEQ ID NO:528

(i) SEQUENCE CHARACTERISTICS:
(A) LENGTH: 405 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...405

(xi) SEQUENCE DESCRIPTION: SEQ ID NO:528

Met Lys Lys Thr Thr Ile Ile Ser Leu Ile Val Phe Gly Ala Phe Phe
1               5                   10                  15
Ala Ala Val Gly Gln Thr Lys Asp Asn Ser Ser Tyr Lys Pro Phe Ser
            20                  25                  30
Lys Glu Asp Ile Ala Gly Gly Val Tyr Ser Leu Pro Thr Gln Asn Arg
        35                  40                  45
Ala Gln Lys Asp Asn Ala Glu Trp Leu Leu Thr Ala Thr Val Ser Thr
    50                  55                  60
Asn Gln Ser Ala Asp Thr His Phe Ile Phe Asp Glu Asn Asn Arg Tyr
65                  70                  75                  80
Ile Ala Arg Asp Ile Lys Ala Asn Gly Val Arg Lys Ser Thr Asp Ser
                85                  90                  95
Ile Tyr Tyr Asp Ala Asn Gly Arg Ile Ser His Val Asp Leu Tyr Ile
            100                 105                 110

Ser Phe Ser Gly Gly Glu Pro Ala Leu Asp Thr Arg Phe Lys Tyr Thr
        115                 120                 125
Tyr Asp Asp Glu Gly Lys Met Thr Val Arg Glu Val Phe Met Leu Val
    130                 135                 140
Met Asp Pro Asn Thr Pro Ile Ser Arg Leu Glu Tyr His Tyr Asp Ala
145                 150                 155                 160
Gln Gly Arg Leu Thr His Trp Ile Ser Phe Ala Phe Gly Ala Glu Ser
                165                 170                 175
Gln Lys Asn Thr Tyr His Tyr Asn Glu Lys Gly Leu Leu Val Ser Glu
            180                 185                 190
Val Leu Ser Asn Ala Met Gly Thr Thr Tyr Ser Asp Thr Gly Lys Thr
        195                 200                 205
Glu Tyr Ser Tyr Asp Asp Ala Asp Asn Met Val Lys Ala Glu Tyr Phe
    210                 215                 220
Val Val Gln Gln Gly Lys Ala Trp Gln Val Leu Lys Arg Glu Glu Tyr
225                 230                 235                 240
Thr Tyr Glu Asp Asn Ile Cys Ile Gln Tyr Leu Ala Ile Asn Gly Thr
                245                 250                 255
Asp Thr Lys Val Tyr Lys Arg Asp Ile Glu Ser Asp Lys Ser Ile Ser
            260                 265                 270
Ala Asn Val Ile Asp Ile Pro Ser Met Pro Glu Gln Thr Trp Pro Asn
        275                 280                 285
Met Tyr Gly Phe Asn Ala Lys Arg Leu Lys Glu Thr Tyr Ser Ser Tyr
    290                 295                 300
Glu Gly Asp Val Ala Thr Pro Ile Phe Asp Tyr Ile Tyr Thr Tyr Lys
305                 310                 315                 320
Ala Leu Thr Ser Met Ala Thr Pro Ser Thr Glu Ala Gln Val Ala Val
                325                 330                 335
Tyr Leu Asn Pro Ser Thr Asp Arg Leu Val Ile Leu Ala Asn Gly Ile
            340                 345                 350
Thr His Leu Ser Met Tyr Asp Leu Gln Gly Lys Leu Ile Arg Asp Cys
        355                 360                 365
Ala Leu Ser Gly Asp Lys Val Glu Met Gly Val Gly Ser Leu Thr Lys
    370                 375                 380
Gly Thr Tyr Leu Leu Lys Val Asn Thr Asp Gln Gly Ala Phe Val Arg
385                 390                 395                 400
Lys Val Val Ile Arg
                405

(2) INFORMATION FOR SEQ ID NO:529

    (i) SEQUENCE CHARACTERISTICS:
        (A) LENGTH: 2037 base pairs
        (B) TYPE: nucleic acid
        (C) STRANDEDNESS: double
        (D) TOPOLOGY: circular

    (ii) MOLECULE TYPE: DNA (genomic)

    (iii) HYPOTHETICAL: NO

    (iv) ANTI-SENSE: NO

    (vi) ORIGINAL SOURCE:
        (A) ORGANISM: Porphyromonas gingivalis

    (ix) FEATURE:
        (A) NAME/KEY: misc_feature
        (B) LOCATION 1...2037

    (xi) SEQUENCE DESCRIPTION: SEQ ID NO:529

GTAATGGACT ACAAACTCAC TTCTCGATTC AAGCCCACGG GCGACCAGCC GGAAGCCATT 60
CGCCAACTCG TACAGGGCAT CAACGAAGGG ATGCCGGCTC AGACGCTGCT CGGCGTAACG 120
GGTTCGGGCA AAACTTTTAC GGTGGCTAAC GTGGTGGCGG CGGTCAATCG TCCGACCCTT 180
GTCCTGAGTC ACAACAAGAC CTTGGCAGCA CAGCTATACG GAGAGTTCAA AGCCTTCTTC 240
CCCGAGAATG CGGTGGAGTA TTTCGTCAGC TACTACGACT ACTATCAGCC CGAGGCCTAC 300
CTCCCCGTCA CAGACACCTA TATCGAAAAG GACATGGCCA TCAACGCGGA GATCGAAAAA 360
CTGCGATTGA GGGCCACGGC TTCGCTCCTG TCAGGGCGGA AAGATGTGCT TGTGGTCAGC 420
TCCGTATCCT GTCTCTACGG TATGGCCAAT CCTGAAGCTT TTTCCGAAAA GGTGATCAGC 480
CTGCACACGG GACAAAGGGC AGACAGGGAT CATTTTATCC GCCTGCTGGT AGAGAGCTAC 540
TACACGAACA ATAAAGTAGA GTTCGAGAGC GGCAACTTCC GTGTCAAAGG CGACAGCGTG 600
GACATATTCC CCGCCGTAGA AGGTTATGAC GGCGTGGCAT ACAGGGTGGA GTTTTGGGAT 660
GGAGAGGTCG AGCGGCTGAG TACCTTCGAT CCGCGAACGG GACGGGAATA CGGCCTGCTG 720
TCGGAGCTGA AGATATATCC GGCCAATCTC TTCGTGACGA CTAAGGAGCA GGTGGATCGG 780

GCAGTAGGGA AAATCGATGT GGATCTGGGC GCACAGGTCG ATTTTCTGAA AGAAATCGGC 840
AAACCATATG AAGCCAAACG CTTGTATGAG CGGGTCACGT ATGACTTGGA AATGATCCGT 900
GAGTTGGGTT ATTGTTCCGG TATAGAGAAC TATTCGCGCT ACTTCGACGG CCGTGACGCG 960
GGCGAACGTC CTTTCTGTCT GTTGGATTAT TTCCCGGAGG ATTTCCTGTT GGTCATAGAC 1020
GAAAGCCATG TAACGATACC GCAGATACGT GCCATGTACG GAGGCGATCG TTCGCGCAAG 1080
GAGAATCTGG TCGAATACGG ATTCCGCCTG CCTGCCGCTC TCGACAATCG GCCGCTTCGC 1140
TTCGACGAGT TCGAAGCTCT CACCCCCCGG ACCCTTTATA TCAGTGCCAC GCCTGCCGAC 1200
TATGAGCTGA ACAGAAGCGA AGGCGTGATC GTCGAGCAGC TGATCCGTCC GACCGGACTG 1260
CTGGATCCCA TCATCGACGT CAAGCCGACG GCAAACCAAG TGGACGATCT GATGGAGGAG 1320
ATAGCACGCT GCATCGAAAA GAAAGAGCGC GTACTGGTAA CGACCCTGAC CAAACGTATG 1380
GCAGAGGAGC TTAGCGAATA CCTGCTACGC CACGGTATCA GCACCGGCTA CATACACAGC 1440
GATGTGGACA CGCTGGAGCG TGTGCGTATC ATGGAAGACC TGCGCAAGGG GGTCTACGAT 1500
GCACTCATCG GGGTGAATCT GCTCCGCGAA GGATTGGACT TGCCGGAAGT TTCGCTTGTG 1560
GCTATTCTGG ATGCGGATAA GGAAGGATTC CTGCGCTCGC ATCGTTCGCT CACGCAGACT 1620
GCAGGACGTG CCGCCCGGCA CATTCATGGG CGTGTCATCT TCTACGCGGA CAAGATCACC 1680
GACAGTATGC AGCTCACCAT GGACGAGACT GCACGCCGAC GCGCAAAGCA ACTGGCCTAC 1740
AACGAAGCGC ACGGCATCAC CCCCCAACAG ATAGTGAAGA ACAGTGCTGC CATTTGGGGA 1800
GAAGGCGATG TGTCGGCCTT GCAATCCGAT ACAGAATCCG GTGCGTACAT AGAAGAGAGC 1860
AGCATGGTGG CTGCCGATCC TTTGGCCGAC TATCTGAGCA AACCCAAGCT GGAAGCACTC 1920
ATTGCTTCGA CCAAGAAGCA AATGCTGGCA GCAGCCAAAG AGCTGGACTT TCTGGAAGCG 1980
GCACGACTTC GGGACGAAGC CGCACGATTG GAAAAGAAAC TGGAGCAACT CACAGCC 2037

(2) INFORMATION FOR SEQ ID NO:530

(i) SEQUENCE CHARACTERISTICS:
(A) LENGTH: 2034 base pairs
(B) TYPE: nucleic acid
(C) STRANDEDNESS: double
(D) TOPOLOGY: circular

(ii) MOLECULE TYPE: DNA (genomic)

(iii) HYPOTHETICAL: NO

(iv) ANTI-SENSE: NO

(vi) ORIGINAL SOURCE:
(A) ORGANISM: Porphyromonas gingivalis

(ix) FEATURE:
(A) NAME/KEY: misc_feature
(B) LOCATION 1...2034

(xi) SEQUENCE DESCRIPTION: SEQ ID NO:530

ATGGACTACA AACTCACTTC TCGATTCAAG CCCACGGGCG ACCAGCCGGA AGCCATTCGC 60
CAACTCGTAC AGGGCATCAA CGAAGGGATG CCGGCTCAGA CGCTGCTCGG CGTAACGGGT 120
TCGGGCAAAA CTTTTACGGT GGCTAACGTG GTGGCGGCGG TCAATCGTCC GACCCTTGTC 180
CTGAGTCACA ACAAGACCTT GGCAGCACAG CTATACGGAG AGTTCAAAGC CTTCTTCCCC 240
GAGAATGCGG TGGAGTATTT CGTCAGCTAC TACGACTACT ATCAGCCCGA GGCCTACCTC 300
CCCGTCACAG ACACCTATAT CGAAAAGGAC ATGGCCATCA ACGCGGAGAT CGAAAAACTG 360
CGATTGAGGG CCACGGCTTC GCTCCTGTCA GGGCGGAAAG ATGTGCTTGT GGTCAGCTCC 420
GTATCCTGTC TCTACGGTAT GGCCAATCCT GAAGCTTTTT CCGAAAAGGT GATCAGCCTG 480
CACACGGGAC AAAGGGCAGA CAGGGATCAT TTTATCCGCC TGCTGGTAGA GAGCTACTAC 540
ACGAACAATA AAGTAGAGTT CGAGAGCGGC AACTTCCGTG TCAAAGGCGA CAGCGTGGAC 600
ATATTCCCCG CCGTAGAAGG TTATGACGGC GTGGCATACA GGGTGGAGTT TTGGGATGGA 660
GAGGTCGAGC GGCTGAGTAC CTTCGATCCG CGAACGGGAC GGGAATACGG CCTGCTGTCG 720
GAGCTGAAGA TATATCCGGC CAATCTCTTC GTGACGACTA AGGAGCAGGT GGATCGGGCA 780
GTAGGGAAAA TCGATGTGGA TCTGGGCGCA CAGGTCGATT TTCTGAAAGA AATCGGCAAA 840
CCATATGAAG CCAAACGCTT GTATGAGCGG GTCACGTATG ACTTGGAAAT GATCCGTGAG 900
TTGGGTTATT GTTCCGGTAT AGAGAACTAT TCGCGCTACT TCGACGGCCG TGACGCGGGC 960
GAACGTCCTT TCTGTCTGTT GGATTATTTC CCGGAGGATT TCCTGTTGGT CATAGACGAA 1020
AGCCATGTAA CGATACCGCA GATACGTGCC ATGTACGGAG GCGATCGTTC GCGCAAGGAG 1080
AATCTGGTCG AATACGGATT CCGCCTGCCT GCCGCTCTCG ACAATCGGCC GCTTCGCTTC 1140
GACGAGTTCG AAGCTCTCAC CCCCCGGACC CTTTATATCA GTGCCACGCC TGCCGACTAT 1200
GAGCTGAACA GAAGCGAAGG CGTGATCGTC GAGCAGCTGA TCCGTCCGAC CGGACTGCTG 1260
GATCCCATCA TCGACGTCAA GCCGACGGCA AACCAAGTGG ACGATCTGAT GGAGGAGATA 1320
GCACGCTGCA TCGAAAAGAA AGAGCGCGTA CTGGTAACGA CCCTGACCAA ACGTATGGCA 1380
GAGGAGCTTA GCGAATACCT GCTACGCCAC GGTATCAGCA CCGGCTACAT ACACAGCGAT 1440
GTGGACACGC TGGAGCGTGT GCGTATCATG GAAGACCTGC GCAAGGGGGT CTACGATGCA 1500
CTCATCGGGG TGAATCTGCT CCGCGAAGGA TTGGACTTGC CGGAAGTTTC GCTTGTGGCT 1560
ATTCTGGATG CGGATAAGGA AGGATTCCTG CGCTCGCATC GTTCGCTCAC GCAGACTGCA 1620
GGACGTGCCG CCCGGCACAT TCATGGGCGT GTCATCTTCT ACGCGGACAA GATCACCGAC 1680
AGTATGCAGC TCACCATGGA CGAGACTGCA CGCCGACGCG CAAAGCAACT GGCCTACAAC 1740

GAAGCGCACG GCATCACCCC CCAACAGATA GTGAAGAACA GTGCTGCCAT TTGGGGAGAA 1800
GGCGATGTGT CGGCCTTGCA ATCCGATACA GAATCCGGTG CGTACATAGA AGAGAGCAGC 1860
ATGGTGGCTG CCGATCCTTT GGCCGACTAT CTGAGCAAAC CCAAGCTGGA AGCACTCATT 1920
GCTTCGACCA AGAAGCAAAT GCTGGCAGCA GCCAAAGAGC TGGACTTTCT GGAAGCGGCA 1980
CGACTTCGGG ACGAAGCCGC ACGATTGGAA AAGAAACTGG AGCAACTCAC AGCC 2034

(2) INFORMATION FOR SEQ ID NO:531

(i) SEQUENCE CHARACTERISTICS:
(A) LENGTH: 679 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...679

(xi) SEQUENCE DESCRIPTION: SEQ ID NO:531

Val Met Asp Tyr Lys Leu Thr Ser Arg Phe Lys Pro Thr Gly Asp Gln
1               5                   10                  15
Pro Glu Ala Ile Arg Gln Leu Val Gln Gly Ile Asn Glu Gly Met Pro
            20                  25                  30
Ala Gln Thr Leu Leu Gly Val Thr Gly Ser Gly Lys Thr Phe Thr Val
        35                  40                  45
Ala Asn Val Val Ala Ala Val Asn Arg Pro Thr Leu Val Leu Ser His
    50                  55                  60
Asn Lys Thr Leu Ala Ala Gln Leu Tyr Gly Glu Phe Lys Ala Phe Phe
65                  70                  75                  80
Pro Glu Asn Ala Val Glu Tyr Phe Val Ser Tyr Tyr Asp Tyr Tyr Gln
                85                  90                  95
Pro Glu Ala Tyr Leu Pro Val Thr Asp Thr Tyr Ile Glu Lys Asp Met
            100                 105                 110
Ala Ile Asn Ala Glu Ile Glu Lys Leu Arg Leu Arg Ala Thr Ala Ser
        115                 120                 125
Leu Leu Ser Gly Arg Lys Asp Val Leu Val Val Ser Ser Val Ser Cys
    130                 135                 140
Leu Tyr Gly Met Ala Asn Pro Glu Ala Phe Ser Glu Lys Val Ile Ser
145                 150                 155                 160
Leu His Thr Gly Gln Arg Ala Asp Arg Asp His Phe Ile Arg Leu Leu
                165                 170                 175
Val Glu Ser Tyr Tyr Thr Asn Asn Lys Val Glu Phe Glu Ser Gly Asn
            180                 185                 190
Phe Arg Val Lys Gly Asp Ser Val Asp Ile Phe Pro Ala Val Glu Gly
        195                 200                 205
Tyr Asp Gly Val Ala Tyr Arg Val Glu Phe Trp Asp Gly Glu Val Glu
    210                 215                 220
Arg Leu Ser Thr Phe Asp Pro Arg Thr Gly Arg Glu Tyr Gly Leu Leu
225                 230                 235                 240
Ser Glu Leu Lys Ile Tyr Pro Ala Asn Leu Phe Val Thr Thr Lys Glu
                245                 250                 255
Gln Val Asp Arg Ala Val Gly Lys Ile Asp Val Asp Leu Gly Ala Gln
            260                 265                 270
Val Asp Phe Leu Lys Glu Ile Gly Lys Pro Tyr Glu Ala Lys Arg Leu
        275                 280                 285
Tyr Glu Arg Val Thr Tyr Asp Leu Glu Met Ile Arg Glu Leu Gly Tyr
    290                 295                 300
Cys Ser Gly Ile Glu Asn Tyr Ser Arg Tyr Phe Asp Gly Arg Asp Ala
305                 310                 315                 320
Gly Glu Arg Pro Phe Cys Leu Leu Asp Tyr Phe Pro Glu Asp Phe Leu
                325                 330                 335
Leu Val Ile Asp Glu Ser His Val Thr Ile Pro Gln Ile Arg Ala Met
            340                 345                 350
Tyr Gly Gly Asp Arg Ser Arg Lys Glu Asn Leu Val Glu Tyr Gly Phe
        355                 360                 365
Arg Leu Pro Ala Ala Leu Asp Asn Arg Pro Leu Arg Phe Asp Glu Phe
    370                 375                 380

Glu Ala Leu Thr Pro Arg Thr Leu Tyr Ile Ser Ala Thr Pro Ala Asp
385                 390                 395                 400
Tyr Glu Leu Asn Arg Ser Glu Gly Val Ile Val Glu Gln Leu Ile Arg
                405                 410                 415
Pro Thr Gly Leu Leu Asp Pro Ile Ile Asp Val Lys Pro Thr Ala Asn
            420                 425                 430
Gln Val Asp Asp Leu Met Glu Glu Ile Ala Arg Cys Ile Glu Lys Lys
        435                 440                 445
Glu Arg Val Leu Val Thr Thr Leu Thr Lys Arg Met Ala Glu Glu Leu
    450                 455                 460
Ser Glu Tyr Leu Leu Arg His Gly Ile Ser Thr Gly Tyr Ile His Ser
465                 470                 475                 480
Asp Val Asp Thr Leu Glu Arg Val Arg Ile Met Glu Asp Leu Arg Lys
                485                 490                 495
Gly Val Tyr Asp Ala Leu Ile Gly Val Asn Leu Leu Arg Glu Gly Leu
            500                 505                 510
Asp Leu Pro Glu Val Ser Leu Val Ala Ile Leu Asp Ala Asp Lys Glu
        515                 520                 525
Gly Phe Leu Arg Ser His Arg Ser Leu Thr Gln Thr Ala Gly Arg Ala
    530                 535                 540
Ala Arg His Ile His Gly Arg Val Ile Phe Tyr Ala Asp Lys Ile Thr
545                 550                 555                 560
Asp Ser Met Gln Leu Thr Met Asp Glu Thr Ala Arg Arg Arg Ala Lys
                565                 570                 575
Gln Leu Ala Tyr Asn Glu Ala His Gly Ile Thr Pro Gln Gln Ile Val
            580                 585                 590
Lys Asn Ser Ala Ala Ile Trp Gly Glu Gly Asp Val Ser Ala Leu Gln
        595                 600                 605
Ser Asp Thr Glu Ser Gly Ala Tyr Ile Glu Glu Ser Ser Met Val Ala
    610                 615                 620
Ala Asp Pro Leu Ala Asp Tyr Leu Ser Lys Pro Lys Leu Glu Ala Leu
625                 630                 635                 640
Ile Ala Ser Thr Lys Lys Gln Met Leu Ala Ala Ala Lys Glu Leu Asp
                645                 650                 655
Phe Leu Glu Ala Ala Arg Leu Arg Asp Glu Ala Ala Arg Leu Glu Lys
            660                 665                 670
Lys Leu Glu Gln Leu Thr Ala
        675

(2) INFORMATION FOR SEQ ID NO:532

(i) SEQUENCE CHARACTERISTICS:
(A) LENGTH: 678 amino acids
(B) TYPE: amino acid
(D) TOPOLOGY: linear

(ii) MOLECULE TYPE: protein

(iii) HYPOTHETICAL: YES

(vi) ORIGINAL SOURCE:
(A) ORGANISM: Porphyromonas gingivalis

(ix) FEATURE:
(A) NAME/KEY: misc_feature
(B) LOCATION 1...678

(xi) SEQUENCE DESCRIPTION: SEQ ID NO:532

Met Asp Tyr Lys Leu Thr Ser Arg Phe Lys Pro Thr Gly Asp Gln Pro
1               5                   10                  15
Glu Ala Ile Arg Gln Leu Val Gln Gly Ile Asn Glu Gly Met Pro Ala
            20                  25                  30
Gln Thr Leu Leu Gly Val Thr Gly Ser Gly Lys Thr Phe Thr Val Ala
        35                  40                  45
Asn Val Val Ala Ala Val Asn Arg Pro Thr Leu Val Leu Ser His Asn
    50                  55                  60
Lys Thr Leu Ala Ala Gln Leu Tyr Gly Glu Phe Lys Ala Phe Phe Pro
65                  70                  75                  80
Glu Asn Ala Val Glu Tyr Phe Val Ser Tyr Tyr Asp Tyr Tyr Gln Pro
                85                  90                  95
Glu Ala Tyr Leu Pro Val Thr Asp Thr Tyr Ile Glu Lys Asp Met Ala
            100                 105                 110
Ile Asn Ala Glu Ile Glu Lys Leu Arg Leu Arg Ala Thr Ala Ser Leu
        115                 120                 125

Leu Ser Gly Arg Lys Asp Val Leu Val Val Ser Ser Val Ser Cys Leu
    130                 135                 140
Tyr Gly Met Ala Asn Pro Glu Ala Phe Ser Glu Lys Val Ile Ser Leu
145                 150                 155                 160
His Thr Gly Gln Arg Ala Asp Arg Asp His Phe Ile Arg Leu Leu Val
                165                 170                 175
Glu Ser Tyr Tyr Thr Asn Asn Lys Val Glu Phe Glu Ser Gly Asn Phe
            180                 185                 190
Arg Val Lys Gly Asp Ser Val Asp Ile Phe Pro Ala Val Glu Gly Tyr
        195                 200                 205
Asp Gly Val Ala Tyr Arg Val Glu Phe Trp Asp Gly Glu Val Glu Arg
    210                 215                 220
Leu Ser Thr Phe Asp Pro Arg Thr Gly Arg Glu Tyr Gly Leu Leu Ser
225                 230                 235                 240
Glu Leu Lys Ile Tyr Pro Ala Asn Leu Phe Val Thr Thr Lys Glu Gln
                245                 250                 255
Val Asp Arg Ala Val Gly Lys Ile Asp Val Asp Leu Gly Ala Gln Val
            260                 265                 270
Asp Phe Leu Lys Glu Ile Gly Lys Pro Tyr Glu Ala Lys Arg Leu Tyr
        275                 280                 285
Glu Arg Val Thr Tyr Asp Leu Glu Met Ile Arg Glu Leu Gly Tyr Cys
    290                 295                 300
Ser Gly Ile Glu Asn Tyr Ser Arg Tyr Phe Asp Gly Arg Asp Ala Gly
305                 310                 315                 320
Glu Arg Pro Phe Cys Leu Leu Asp Tyr Phe Pro Glu Asp Phe Leu Leu
                325                 330                 335
Val Ile Asp Glu Ser His Val Thr Ile Pro Gln Ile Arg Ala Met Tyr
            340                 345                 350
Gly Gly Asp Arg Ser Arg Lys Glu Asn Leu Val Glu Tyr Gly Phe Arg
        355                 360                 365
Leu Pro Ala Ala Leu Asp Asn Arg Pro Leu Arg Phe Asp Glu Phe Glu
    370                 375                 380
Ala Leu Thr Pro Arg Thr Leu Tyr Ile Ser Ala Thr Pro Ala Asp Tyr
385                 390                 395                 400
Glu Leu Asn Arg Ser Glu Gly Val Ile Val Glu Gln Leu Ile Arg Pro
                405                 410                 415
Thr Gly Leu Leu Asp Pro Ile Ile Asp Val Lys Pro Thr Ala Asn Gln
            420                 425                 430
Val Asp Asp Leu Met Glu Glu Ile Ala Arg Cys Ile Glu Lys Lys Glu
        435                 440                 445
Arg Val Leu Val Thr Thr Leu Thr Lys Arg Met Ala Glu Glu Leu Ser
    450                 455                 460
Glu Tyr Leu Leu Arg His Gly Ile Ser Thr Gly Tyr Ile His Ser Asp
465                 470                 475                 480
Val Asp Thr Leu Glu Arg Val Arg Ile Met Glu Asp Leu Arg Lys Gly
                485                 490                 495
Val Tyr Asp Ala Leu Ile Gly Val Asn Leu Leu Arg Glu Gly Leu Asp
            500                 505                 510
Leu Pro Glu Val Ser Leu Val Ala Ile Leu Asp Ala Asp Lys Glu Gly
        515                 520                 525
Phe Leu Arg Ser His Arg Ser Leu Thr Gln Thr Ala Gly Arg Ala Ala
    530                 535                 540
Arg His Ile His Gly Arg Val Ile Phe Tyr Ala Asp Lys Ile Thr Asp
545                 550                 555                 560
Ser Met Gln Leu Thr Met Asp Glu Thr Ala Arg Arg Arg Ala Lys Gln
                565                 570                 575
Leu Ala Tyr Asn Glu Ala His Gly Ile Thr Pro Gln Gln Ile Val Lys
            580                 585                 590
Asn Ser Ala Ala Ile Trp Gly Glu Gly Asp Val Ser Ala Leu Gln Ser
        595                 600                 605
Asp Thr Glu Ser Gly Ala Tyr Ile Glu Glu Ser Ser Met Val Ala Ala
    610                 615                 620
Asp Pro Leu Ala Asp Tyr Leu Ser Lys Pro Lys Leu Glu Ala Leu Ile
625                 630                 635                 640
Ala Ser Thr Lys Lys Gln Met Leu Ala Ala Ala Lys Glu Leu Asp Phe
                645                 650                 655
Leu Glu Ala Ala Arg Leu Arg Asp Glu Ala Ala Arg Leu Glu Lys Lys
            660                 665                 670
Leu Glu Gln Leu Thr Ala
        675

## Claims

1. An isolated antigenic *Porphorymonas gingivalis* polypeptide, the polypeptide comprising:
(i) an amino acid sequence selected from the group consisting of SEQ ID NO. 424, SEQ ID NO. 265 to SEQ ID NO. 423, SEQ ID NO. 425 to SEQ ID NO. 528, SEQ ID NO. 531 and SEQ ID NO. 532; or
(ii) an amino acid sequence at least 85%, preferably at least 95%, identical to an amino acid sequence of (i); or
(iii) at least 40 amino acids having a contiguous sequence of at least 40 amino acids identical to a contiguous amino acid sequence of (i).

2. A polypeptide as claimed in claim 1 in which the polypeptide comprises;
(i) an amino acid sequence selected from the group consisting of SEQ ID NO. 424, SEQ ID NO. 386 to SEQ ID NO. 423, SEQ ID NO. 425 to SEQ ID NO. 528, and SEQ ID NO. 532; or
(ii) an amino acid sequence at least 85%, preferably at least 95%, identical to an amino acid sequence of (i); or
(iii) at least 40 amino acids having a contiguous sequence of at least 40 amino acids identical to a contiguous amino acid sequence of (i).

3. A polypeptide as claimed in claim 2 in which the polypeptide comprises an amino acid sequence selected from the group consisting of SEQ ID NO. 424, SEQ ID NO. 386, SEQ ID NO. 425, SEQ ID NO. 434, SEQ ID NO. 447, SEQ ID NO. 458, SEQ ID NO. 475, SEQ ID NO. 498, SEQ ID NO. 499, SEQ ID NO. 500, SEQ ID NO. 501, SEQ ID NO. 387, SEQ ID NO. 400, SEQ ID NO. 411, SEQ ID NO. 419, SEQ ID NO. 420, SEQ ID NO. 427, SEQ ID NO. 429, SEQ ID NO. 433, SEQ ID NO. 437, SEQ ID NO. 438, SEQ ID NO. 443, SEQ ID NO. 444, SEQ ID NO. 448, SEQ ID NO. 449, SEQ ID NO. 452, SEQ ID NO. 455, SEQ ID NO. 457, SEQ ID NO. 459, SEQ ID NO. 461, SEQ ID NO. 462, SEQ ID NO. 463, SEQ ID NO. 467, SEQ ID NO. 468, SEQ ID NO. 469, SEQ ID NO. 482, SEQ ID NO. 484, SEQ ID NO. 485, SEQ ID. NO 494, SEQ ID NO. 508, SEQ ID NO. 509, SEQ ID NO. 510, SEQ ID NO. 520, SEQ ID NO. 521, SEQ ID NO. 522, SEQ ID NO. 525, SEQ ID NO. 526, SEQ ID NO. 528, SEQ ID NO. 389, SEQ ID NO. 390 and SEQ ID NO. 391.

4. An isolated antigenic *Porphorymonas gingivalis* polypeptide, the polypeptide comprising an amino acid sequence selected from the group consisting of SEQ ID NO. 424, SEQ ID NO. 386 to SEQ ID NO. 423, SEQ ID NO. 425 to SEQ ID NO. 528 and SEQ ID NO. 532 less the leader sequence set out in Table 3.

5. An isolated DNA molecule, the DNA molecule comprising a nucleotide sequence which encodes the polypeptide as claimed in preceding claim or a sequence which hybridises thereto under conditions of high stringency.

6. An isolated DNA molecule as claimed in claim 5 in which the DNA molecule comprises a nucleotide sequence selected from the group consisting of SEQ ID NO. 160, SEQ ID NO. 1 to SEQ ID NO. 159, SEQ ID NO. 161 to SEQ ID NO. 264, SEQ ID. NO 529 and SEQ ID NO. 530.

7. A recombinant expression vector comprising the DNA molecule as claimed in claim 5 or claim 6 operably linked to a transcription regulatory element.

8. A cell comprising the recombinant expression vector as claimed in claim 7.

9. A method for producing a *P. gingivalis* polypeptide comprising culturing the cell as claimed in claim 8 under conditions that permit expression of the polypeptide.

10. A composition for use in raising an immune response directed against P. *gingivalis* in a subject, the composition comprising
(i) an effective amount of at least one polypeptide as claimed in any one of claims 1 to 4; and/or
(ii) an effective amount of at least one DNA molecule as claimed in claim 5 or claim 6; and
(iii) a pharmaceutically acceptable carrier.

11. A composition as claimed in claim 10 in which the pharmaceutically acceptable carrier is an adjuvant.

12. The use of a composition as claimed in claim 10 or claim 11 in the manufacture of a medicament for treating a subject for *P. gingivalis* infection.

13. The use as claimed in claim 12, wherein the treatment is a prophylactic treatment or a therapeutic treatment.

14. An antibody raised against a polypeptide as claimed in any one of claims 1 to 4.

15. An antibody as claimed in claim 14 in which the antibody is polyclonal or monoclonal.

16. A composition comprising at least one antibody as claimed in claim 14 or claim 15.

17. A composition as claimed in claim 16 which is adapted for oral use.

18. A nucleotide probe comprising at least 18 nucleotides and having a contiguous sequence of at least 18 nucleotides identical to a contiguous nucleotide sequence selected from the group consisting of SEQ ID NO. 1 to SEQ ID NO. 121, SEQ ID NO. 529 and sequences complementary thereto.

19. A nucleotide probe as claimed in claim 18 in which the probe further comprises a detectable label.

20. A method for detecting the presence of *P. gingivalis* nucleic acid in a sample comprising:
(a) contacting a sample with the nucleotide probe as claimed in claim 18 or claim 19 under conditions in which a hybrid can form between the probe and a P. *gingivalis* nucleic acid in the sample; and
(b) detecting the hybrid formed in step (a), wherein detection of a hybrid indicates the presence of a *P. gingivalis* nucleic acid in the sample.
